# EUROPEAN PATENT APPLICATION

(11) **EP 1 847 620 A2**
(43) Date of publication of application: **24.10.2007**
(21) Application number: 07075380.1
(22) Date of filing: 12.10.2001
(51) Int. Cl.: C12Q 1/68, C07H 21/02, C12N 15/85

(54) **Methods of diagnosis of prostate cancer, compositions and methods of screening for modulators of prostate cancer**

(30) Priority: 13.10.2000 US 687576; 08.12.2000 US 733742; 08.12.2000 US 733288; 24.01.2001 US 263957 P; 16.03.2001 US 276888 P; 16.03.2001 US 276791 P; 06.04.2001 US 281922 P; 24.04.2001 US 286214 P; 30.04.2001 US 847046; 04.05.2001 US 288589 P
(62) Divisional of application: 01983958.8
(71) Applicant: Cedars-Sinai Medical Center, Los Angeles, California 90048-1865 (US); GARVAN INSTITUTE OF MEDICAL RESEARCH, Darlinghurst Sydney NSW 2010 (AU)
(72) Inventor: Gish, Kurt C., San Francisco, CA 94131 (US); Afar, Daniel, Brisbane, CA 94005 (US); Mack, David H., Menlow Park, CA 94025 (US); Hevezi, Peter, San Francisco, CA 94122 (US); Wilson, Keith E., Redwood City, CA 94062 (US); Agus, David B., Beverly Hills, CA 90210 (US)
(74) Representative: Banford, Paul Clifford

(57) **Abstract**

Described herein are genes whose expression are up-regulated or down-regulated in prostate cancer. Also described are such genes whose expression is further up-regulated or down-regulated in drug-resistant prostate cancer cells. Related methods and compositions that can be used for diagnosis and treatment of prostate cancer are disclosed. Also described herein are methods that can be used to identify modulators of prostate cancer.

## Description

### CROSS-REFERENCES TO RELATED APPLICATIONS

This application claims priority from the following applications: USSN 09/687,576 filed October 13, 2000, USSN 60/276,791 filed March 16, 2001; USSN 60/288,589, filed May 4,2001; USSN 09/733,742, filed December 8, 2000; USSN 09/733,288, filed December 8, 2000; USSN 09/847,046, filed April 30, 2001; USSN 60/276,888, filed March 16,2001; USSN 60/286,214, filed April 24, 2001; USSN 60/281,922, filed April 6, 2001; USSN 60/263,957, filed January 24, 2001, which are incorporated herein by reference in their entirety.

### FIELD OF THE INVENTION

The invention relates to the identification of nucleic acid and protein expression profiles and nucleic acids, products, and antibodies thereto that are involved in prostate cancer; and to the use of such expression profiles and compositions in the diagnosis, prognosis and therapy of prostate cancer. The invention further relates to methods for identifying and using agents and/or targets that inhibit prostate cancer.

### BACKGROUND OF THE INVENTION

Prostate cancer is the most commonly diagnosed internal malignancy and second most common cause of cancer death in men in the U.S., resulting in approximately 40,000 deaths each year ( Landis et al., CA Cancer J. Clin. 48:6-29 (1998); Greenlee et al., CA Cancer J. Clin. 50(1):7-13 (2000)), and incidence of prostate cancer has been increasing rapidly over the past 20 years in many parts of the world (Nakata et al., Int. J. Urol. 7(7):254-257 (2000); Majeed et al., BJU Int. 85(9):1058-1062 (2000)). It develops as the result of a pathologic transformation of normal prostate cells. In tumorigenesis, the cancer cell undergoes initiation, proliferation and loss of contact inhibition, culminating in invasion of surrounding tissue and, ultimately, metastasis.

Deaths from prostate cancer are a result of metastasis of a prostate tumor. Therefore, early detection of the development of prostate cancer is critical in reducing mortality from this disease. Measuring levels of prostate-specific antigen (PSA) has become a very common method for early detection and screening, and may have contributed to the slight decrease in the mortality rate from prostate cancer in recent years (Nowroozi et al., Cancer Control 5(6):522-531 (1998)). However, many cases are not diagnosed until the disease has progressed to an advanced stage.

Treatments such as surgery (prostatectomy), radiation therapy, and cryotherapy are potentially curative when the cancer remains localized to the prostate. Therefore, early detection of prostate cancer is important for a positive prognosis for treatment. Systemic treatment for metastatic prostate cancer is limited to hormone therapy and chemotherapy. Chemical or surgical castration has been the primary treatment for symptomatic metastatic prostate cancer for over 50 years. This testicular androgen deprivation therapy usually results in stabilization or regression of the disease (in 80% of patients), but progression of metastatic prostate cancer eventually develops (Panvichian et al., Cancer Control 3(6):493-500 (1996)). Metastatic disease is currently considered incurable, and the primary goals of treatment are to prolong survival and improve quality of life (Rago, Cancer Control 5(6):513-521 (1998)).

Thus, methods that can be used for diagnosis and prognosis of prostate cancer and effective treatment of prostate cancer, and including particularly metastatic prostate cancer, would be desirable. Accordingly, provided herein are methods that can be used in diagnosis and prognosis of prostate cancer. Further provided are methods that can be used to screen candidate bioactive agents for the ability to modulate, e.g., treat, prostate cancer. Additionally, provided herein are molecular targets and compositions for therapeutic intervention in prostate cancer and other cancers.

### SUMMARY OF THE INVENTION

The present invention therefore provides nucleotide sequences of genes that are up- and down-regulated in prostate cancer cells. Such genes are useful for diagnostic purposes, and also as targets for screening for therapeutic compounds that modulate prostate cancer, such as hormones or antibodies. Other aspects of the invention will become apparent to the skilled artisan by the following description of the invention.

In one aspect, the present invention provides a method of detecting a prostate cancer-associated transcript in a cell from a patient, the method comprising contacting a biological sample from the patient with a polynucleotide that selectively hybridizes to a sequence at least 80% identical to a sequence as shown in Tables 1-16.

In one embodiment, the present invention provides a method of determining the level of a prostate cancer associated transcript in a cell from a patient.

In one embodiment, the present invention provides a method of detecting a prostate cancer-associated transcript in a cell from a patient, the method comprising contacting a biological sample from the patient with a polynucleotide that selectively hybridizes to a sequence at least 80% identical to a sequence as shown in Tables 1-16.

In one embodiment, the polynucleotide selectively hybridizes to a sequence at least 95% identical to a sequence as shown in Tables 1-16. In another embodiment, the polynucleotide comprises a sequence as shown in Tables 1-16.

In one embodiment, the biological sample is a tissue sample. In another embodiment, the biological sample comprises isolated nucleic acids, e.g., mRNA.

In one embodiment, the polynucleotide is labeled, e.g., with a fluorescent label.

In one embodiment, the polynucleotide is immobilized on a solid surface.

In one embodiment, the patient is undergoing a therapeutic regimen to treat prostate cancer. In another embodiment, the patient is suspected of having metastatic prostate cancer.

In one embodiment, the patient is a human.

In one embodiment, the patient is suspected of having a taxol-resistant cancer.

In one embodiment, the prostate cancer associated transcript is mRNA.

In one embodiment, the method further comprises the step of amplifying nucleic acids before the step of contacting the biological sample with the polynucleotide.

In another aspect, the present invention provides a method of monitoring the efficacy of a therapeutic treatment of prostate cancer, the method comprising the steps of: (i) providing a biological sample from a patient undergoing the therapeutic treatment; and (ii) determining the level of a prostate cancer-associated transcript in the biological sample by contacting the biological sample with a polynucleotide that selectively hybridizes to a sequence at least 80% identical to a sequence as shown in Tables 1-16, thereby monitoring the efficacy of the therapy. In a further embodiment, the patient has metastatic prostate cancer. In a further embodiment, the patient has a drug resistant (e.g., taxol resistant) form of prostate cancer.

In one embodiment, the method further comprises the step of: (iii) comparing the level of the prostate cancer-associated transcript to a level of the prostate cancer-associated transcript in a biological sample from the patient prior to, or earlier in, the therapeutic treatment.

Additionally, provided herein is a method of evaluating the effect of a candidate prostate cancer drug comprising administering the drug to a patient and removing a cell sample from the patient. The expression profile of the cell is then determined. This method may further comprise comparing the expression profile to an expression profile of a healthy individual. In a preferred embodiment, said expression profile includes a gene of Tables 1-16.

In one aspect, the present invention provides an isolated nucleic acid molecule consisting of a polynucleotide sequence as shown in Tables 1-16.

In one embodiment, an expression vector or cell comprises the isolated nucleic acid.

In one aspect, the present invention provides an isolated polypeptide which is encoded by a nucleic acid molecule having polynucleotide sequence as shown in Tables 1-16.

In another aspect, the present invention provides an antibody that specifically binds to an isolated polypeptide which is encoded by a nucleic acid molecule having polynucleotide sequence as shown in Tables 1-16.

In one embodiment, the antibody is conjugated to an effector component, e.g., a fluorescent label, a radioisotope or a cytotoxic chemical.

In one embodiment, the antibody is an antibody fragment. In another embodiment, the antibody is humanized.

In one aspect, the present invention provides a method of detecting a prostate cancer cell in a biological sample from a patient, the method comprising contacting the biological sample with an antibody as described herein.

In another aspect, the present invention provides a method of detecting antibodies specific to prostate cancer in a patient, the method comprising contacting a biological sample from the patient with a polypeptide encoded by a nucleic acid comprising a sequence from Tables 1-16.

In another aspect, the present invention provides a method for identifying a compound that modulates a prostate cancer-associated polypeptide, the method comprising the steps of: (i) contacting the compound with a prostate cancer-associated polypeptide, the polypeptide encoded by a polynucleotide that selectively hybridizes to a sequence at least 80% identical to a sequence as shown in Tables 1-16; and (ii) determining the functional effect of the compound upon the polypeptide.

In one embodiment, the functional effect is a physical effect, an enzymatic effect, or a chemical effect.

In one embodiment, the polypeptide is expressed in a eukaryotic host cell or cell membrane. In another embodiment, the polypeptide is recombinant.

In one embodiment, the functional effect is determined by measuring ligand binding to the polypeptide.

In another aspect, the present invention provides a method of inhibiting proliferation of a prostate cancer-associated cell to treat prostate cancer in a patient, the method comprising the step of administering to the subject a therapeutically effective amount of a compound identified as described herein.

In one embodiment, the compound is an antibody.

In another aspect, the present invention provides a drug screening assay comprising the steps of: (i) administering a test compound to a mammal having prostate cancer or to a cell sample isolated therefrom; (ii) comparing the level of gene expression of a polynucleotide that selectively hybridizes to a sequence at least 80% identical to a sequence as shown in Tables 1-16 in a treated cell or mammal with the level of gene expression of the polynucleotide in a control cell sample or mammal, wherein a test compound that modulates the level of expression of the polynucleotide is a candidate for the treatment of prostate cancer.

In one embodiment, the control is a mammal with prostate cancer or a cell sample therefrom that has not been treated with the test compound. In another embodiment, the control is a normal cell or mammal.

In one embodiment, the test compound is administered in varying amounts or concentrations. In another embodiment, the test compound is administered for varying time periods. In another embodiment, the comparison can occur after addition or removal of the drug candidate.

In one embodiment, the levels of a plurality of polynucleotides that selectively hybridize to a sequence at least 80% identical to a sequence as shown in Tables 1-16 are individually compared to their respective levels in a control cell sample or mammal. In a preferred embodiment the plurality of polynucleotides is from three to ten.

In another aspect, the present invention provides a method for treating a mammal having prostate cancer comprising administering a compound identified by the assay described herein.

In another aspect, the present invention provides a pharmaceutical composition for treating a mammal having prostate cancer, the composition comprising a compound identified by the assay described herein and a physiologically acceptable excipient.

In one aspect, the present invention provides a method of screening drug candidates by providing a cell expressing a gene that is up- and down-regulated as in a prostate cancer. In one embodiment, a gene is selected from Tables 1-16. The method further includes adding a drug candidate to the cell and determining the effect of the drug candidate on the expression of the expression profile gene.

In one embodiment, the method of screening drug candidates includes comparing the level of expression in the absence of the drug candidate to the level of expression in the presence of the drug candidate, wherein the concentration of the drug candidate can vary when present, and wherein the comparison can occur after addition or removal of the drug candidate. In a preferred embodiment, the cell expresses at least two expression profile genes. The profile genes may show an increase or decrease.

Also provided is a method of evaluating the effect of a candidate prostate cancer drug comprising administering the drug to a transgenic animal expressing or over-expressing the prostate cancer modulatory protein, or an animal lacking the prostate cancer modulatory protein, for example as a result of a gene knockout.

Moreover, provided herein is a biochip comprising one or more nucleic acid segments of Tables 1-16, wherein the biochip comprises fewer than 1000 nucleic acid probes. Preferably, at least two nucleic acid segments are included. More preferably, at least three nucleic acid segments are included.

Furthermore, a method of diagnosing a disorder associated with prostate cancer is provided. The method comprises determining the expression of a gene of Tables 1-16, in a first tissue type of a first individual, and comparing the distribution to the expression of the gene from a second normal tissue type from the first individual or a second unaffected individual. A difference in the expression indicates that the first individual has a disorder associated with prostate cancer.

In a further embodiment, the biochip also includes a polynucleotide sequence of a gene that is not up- and down-regulated in prostate cancer.

In one embodiment a method for screening for a bioactive agent capable of interfering with the binding of a prostate cancer modulating protein (prostate cancer modulatory protein) or a fragment thereof and an antibody which binds to said prostate cancer modulatory protein or fragment thereof. In a preferred embodiment, the method comprises combining a prostate cancer modulatory protein or fragment thereof, a candidate bioactive agent and an antibody which binds to said prostate cancer modulatory protein or fragment thereof. The method further includes determining the binding of said prostate cancer modulatory protein or fragment thereof and said antibody. Wherein there is a change in binding, an agent is identified as an interfering agent. The interfering agent can be an agonist or an antagonist. Preferably, the agent inhibits prostate cancer.

Also provided herein are methods of eliciting an immune response in an individual. In one embodiment a method provided herein comprises administering to an individual a composition comprising a prostate cancer modulating protein, or a fragment thereof. In another embodiment, the protein is encoded by a nucleic acid selected from those of Tables 1-16.

Further provided herein are compositions capable of eliciting an immune response in an individual. In one embodiment, a composition provided herein comprises a prostate cancer modulating protein, preferably encoded by a nucleic acid of Tables 1-16, or a fragment thereof, and a pharmaceutically acceptable carrier. In another embodiment, said composition comprises a nucleic acid comprising a sequence encoding a prostate cancer modulating protein, preferably selected from the nucleic acids of Tables 1-16, and a pharmaceutically acceptable carrier.

Also provided are methods of neutralizing the effect of a prostate cancer protein, or a fragment thereof, comprising contacting an agent specific for said protein with said protein in an amount sufficient to effect neutralization. In another embodiment, the protein is encoded by a nucleic acid selected from those of Tables 1-16.

In another aspect of the invention, a method of treating an individual for prostate cancer is provided. In one embodiment, the method comprises administering to said individual an inhibitor of a prostate cancer modulating protein. In another embodiment, the method comprises administering to a patient having prostate cancer an antibody to a prostate cancer modulating protein conjugated to a therapeutic moiety. Such a therapeutic moiety can be a cytotoxic agent or a radioisotope.

### DETAILED DESCRIPTION OF THE INVENTION

In accordance with the objects outlined above, the present invention provides novel methods for diagnosis and prognosis evaluation for prostate cancer (PC), including metastatic prostate cancer, as well as methods for screening for compositions which modulate prostate cancer. Also provided are methods for treating prostate cancer.

In addition to the other nucleic acid and peptide sequences, the present invention also relates to the identification of PAA2 as a gene that is highly over expressed in prostate cancer patient tissues. PAA2 sequence is identical to the zinc transporter ZNT4. Results presented herein demonstrate that PAA2/ZNT4 is highly expressed in prostate cancer cells. The prostate gland is unique in that it has the highest capacity of any organ in the body to accumulate zinc. Zinc uptake is regulated by prolactin and testosterone, which induce the expression of a member of the ZIP family of zinc transporters (Costello et al., 1999, J. Biol. Chem. 274:17499-17504). Zinc accumulation in the prostate functions to inhibit citrate oxidation, which results in a decrease in cellular ATP production (Costello and Franklin, 1998, Prostate 35:285-296). Cancer cells are more sensitive to decreased ATP production and have evolved to prevent zinc accumulation. Without wishing to be bound by theory, the up-regulation of ZNT4 in prostate cancer cells may result in protection of the cells from high zinc levels by its ability to pump accumulated zinc out of the cells.

The present invention also relates to nucleic acid sequencess encoding PBH1. PBH1 is related to human TRPC7 (transient receptor potential-related channels, NP_003298), a putative calcium channel highly expressed in brain (Nagamine et al., Genomics 54:124-131 (1998)). Trp is related to melastatin, a gene down-regulated in metastatic melanomas (Duncan et al., Cancer Res. 58:1515-1520 (1998)), and MTR1, a gene locallized to within the Beckwith-Wiedemann syndrome/Wilm's tumor susceptability region (Prawitt et al., Hum. Mol. Genet. 9:203-216 (2000)). Without wishing to be bound by theory, it is believed that PBH1 functions as a calcium channel.

As a calcium channel, PBH1 is an ideal target for a small molecule therapeutic, or a therapeutic antibody that disrupts channel function. CD20, the target of Rituximab in non-Hodgekin's lymphoma (Maloney et al., Blood 90:2188-2195 (1997); Leget and Czuczman, Curr. Opin. Oncol. 10:548-551 (1998)), is a plasma membrane calcium channel expressed in B cells (Tedder and Engel, Immunol. Today 15:450-454 (1994)). Similarly, a small molecule, or antibody that inhibits or alters a calcium signal mediated by PBH1, will result in the death of prostate cancer cells.

PBH1, and other genes of the invention, are also be useful as targets for cytotoxic T-lymphocytes. Genes that are tumor specific, or that are expressed in immune-privileged organs, are currently being used as potential vaccine targets (Van den Eynde and Boon, Int. J. Clin. Lab. Res. 27:81-86 (1997)). The expression pattern of PBH1 indicates that it is an ideal target for cytotoxic T-lymphocytes. Thus, therapies that utilize PBH1-specific cytotoxic T-lymphocytes to induce prostate cancer cell death are also provided by this invention. See, e.g., U.S. Patent No. 6,051,227 and WO 00/32231, the disclosures of which are herein incorporated by reference.

The present invention is also related to the identification of PAA3 as a gene that is important in the modulation of prostate cancer and or breast cancer.

Tables 1-16 provide unigene cluster identification numbers, exemplar accession numbers, or genomic nucleotide position numbers for the nucleotide sequence of genes that exhibit increased or decreased expression in prostate cancer samples.

### Definitions

The term "prostate cancer protein" or "prostate cancer polynucleotide" or "prostate cancer-associated transcript" refers to nucleic acid and polypeptide polymorphic variants, alleles, mutants, and interspecies homologues that: (1) have a nucleotide sequence that has greater than about 60% nucleotide sequence identity, 65%, 70%, 75%, 80%, 85%, 90%, preferably 91%, 92%, 93%, 94%, 95%, 96%, 97%, 98% or 99% or greater nucleotide sequence identity, preferably over a region of over a region of at least about 25, 50, 100, 200, 500, 1000, or more nucleotides, to a nucleotide sequence of or associated with a unigene cluster of Tables 1-16; (2) bind to antibodies, e.g., polyclonal antibodies, raised against an immunogen comprising an amino acid sequence encoded by a nucleotide sequence of or associated with a unigene cluster of Tables 1-16, and conservatively modified variants thereof; (3) specifically hybridize under stringent hybridization conditions to a nucleic acid sequence, or the complement thereof of Tables 1-16 and conservatively modified variants thereof or (4) have an amino acid sequence that has greater than about 60% amino acid sequence identity, 65%, 70%, 75%, 80%, 85%, 90%, preferably 91%, 92%, 93%, 94%, 95%, 96%, 97%, 98% or 99% or greater amino sequence identity, preferably over a region of over a region of at least about 25, 50, 100, 200, 500, 1000, or more amino acid, to an amino acid sequence encoded by a nucleotide sequence of or associated with a unigene cluster of Tables 1-16. A polynucleotide or polypeptide sequence is typically from a mammal including, but not limited to, primate, e.g., human; rodent, e.g., rat, mouse, hamster; cow, pig, horse, sheep, or other mammal. A "prostate cancer polypeptide" and a "prostate cancer polynucleotide," include both naturally occurring or recombinant forms.

A "full length" prostate cancer protein or nucleic acid refers to a prostate cancer polypeptide or polynucleotide sequence, or a variant thereof, that contains all of the elements normally contained in one or more naturally occurring, wild type prostate cancer polynucleotide or polypeptide sequences. For example, a full length prostate cancer nucleic acid will typically comprise all of the exons that encode for the full length, naturally ocurring protein. The "full length" may be prior to, or after, various stages of post-translation processing or splicing, including alternative splicing.

"Biological sample" as used herein is a sample of biological tissue or fluid that contains nucleic acids or polypeptides, e.g., of a prostate cancer protein, polynucleotide or transcript. Such samples include, but are not limited to, tissue isolated from primates, e.g., humans, or rodents, e.g., mice, and rats. Biological samples may also include sections of tissues such as biopsy and autopsy samples, frozen sections taken for histologic purposes, blood, plasma, serum, sputum, stool, tears, mucus, hair, skin, etc. Biological samples also include explants and primary and/or transformed cell cultures derived from patient tissues. A biological sample is typically obtained from a eukaryotic organism, most preferably a mammal such as a primate e.g., chimpanzee or human; cow; dog; cat; a rodent, e.g., guinea pig, rat, mouse; rabbit; or a bird; reptile; or fish.

"Providing a biological sample" means to obtain a biological sample for use in methods described in this invention. Most often, this will be done by removing a sample of cells from an animal, but can also be accomplished by using previously isolated cells (*e.g.*, isolated by another person, at another time, and/or for another purpose), or by performing the methods of the invention *in vivo.* Archival tissues, having treatment or outcome history, will be particularly useful.

The terms "identical" or percent "identity," in the context of two or more nucleic acids or polypeptide sequences, refer to two or more sequences or subsequences that are the same or have a specified percentage of amino acid residues or nucleotides that are the same (i.e., about 60% identity, preferably 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters described below, or by manual alignment and visual inspection *(see, e.g.,* NCBI web site http://www.ncbi.nlm.nih.gov/BLAST/ or the like). Such sequences are then said to be "substantially identical." This definition also refers to, or may be applied to, the compliment of a test sequence. The definition also includes sequences that have deletions and/or additions, as well as those that have substitutions, as well as naturally occurring, e.g., polymorphic or allelic variants, and man-made variants. As described below, the preferred algorithms can account for gaps and the like. Preferably, identity exists over a region that is at least about 25 amino acids or nucleotides in length, or more preferably over a region that is 50-100 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of one of the number of contiguous positions selected from the group consisting typically of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (*see, e.g., Current Protocols in Molecular Biology* (Ausubel *et al.,* eds. 1995 supplement)).

Preferred examples of algorithms that are suitable for determining percent sequence identity and sequence similarity include the BLAST and BLAST 2.0 algorithms, which are described in Altschul et al., Nuc. Acids Res. 25:3389-3402 (1977) and Altschul et al., J. Mol. Biol. 215:403-410 (1990). BLAST and BLAST 2.0 are used, with the parameters described herein, to determine percent sequence identity for the nucleic acids and proteins of the invention. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are extended in both directions along each sequence for as far as the cumulative alignment score can be increased. Cumulative scores are calculated using, e.g., for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (*see* Henikoff & Henikoff, Proc. Natl. Acad. Sci. USA 89:10915 (1989)) alignments (B) of 50, expectation (E) of 10, M=5, N=-4, and a comparison of both strands.

The BLAST algorithm also performs a statistical analysis of the similarity between two sequences (*see, e.g.,* Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001. Log values may be large negative numbers, e.g., 5, 10, 20, 30, 40, 40, 70, 90, 110, 150, 170, etc.

An indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, e.g., where the two peptides differ only by conservative substitutions. Another indication that two nucleic acid sequences are substantially identical is that the two molecules or their complements hybridize to each other under stringent conditions, as described below. Yet another indication that two nucleic acid sequences are substantially identical is that the same primers can be used to amplify the sequences.

A "host cell" is a naturally occurring cell or a transformed cell that contains an expression vector and supports the replication or expression of the expression vector. Host cells may be cultured cells, explants, cells *in vivo,* and the like. Host cells may be prokaryotic cells such as *E*. *coli,* or eukaryotic cells such as yeast, insect, amphibian, or mammalian cells such as CHO, HeLa, and the like (*see, e.g.,* the American Type Culture Collection catalog or web site, www.atcc.org).

The terms "isolated," "purified," or "biologically pure" refer to material that is substantially or essentially free from components that normally accompany it as found in its native state. Purity and homogeneity are typically determined using analytical chemistry techniques such as polyacrylamide gel electrophoresis or high performance liquid chromatography. A protein or nucleic acid that is the predominant species present in a preparation is substantially purified. In particular, an isolated nucleic acid is separated from some open reading frames that naturally flank the gene and encode proteins other than protein encoded by the gene. The term "purified" in some embodiments denotes that a nucleic acid or protein gives rise to essentially one band in an electrophoretic gel. Preferably, it means that the nucleic acid or protein is at least 85% pure, more preferably at least 95% pure, and most preferably at least 99% pure. "Purify" or "purification" in other embodiments means removing at least one contaminant from the composition to be purified. In this sense, purification does not require that the purified compound be homogenous, e.g., 100% pure.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers, those containing modified residues, and non-naturally occurring amino acid polymer.

The term "amino acid" refers to naturally occurring and synthetic amino acids, as well as amino acid analogs and amino acid mimetics that function similarly to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, e.g., an α carbon that is bound to a hydrogen, a carboxyl group, an amino group, and an R group, e.g., homoserine, norleucine, methionine sulfoxide, methionine methyl sulfonium. Such analogs may have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that functions similarly to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical or associated, e.g., naturally contiguous, sequences. Because of the degeneracy of the genetic code, a large number of functionally identical nucleic acids encode most proteins. For instance, the codons GCA, GCC, GCG and GCU all encode the amino acid alanine. Thus, at every position where an alanine is specified by a codon, the codon can be altered to another of the corresponding codons described without altering the encoded polypeptide. Such nucleic acid variations are "silent variations," which are one species of conservatively modified variations. Every nucleic acid sequence herein which encodes a polypeptide also describes silent variations of the nucleic acid. One of skill will recognize that in certain contexts each codon in a nucleic acid (except AUG, which is ordinarily the only codon for methionine, and TGG, which is ordinarily the only codon for tryptophan) can be modified to yield a functionally identical molecule. Accordingly, often silent variations of a nucleic acid which encodes a polypeptide is implicit in a described sequence with respect to the expression product, but not with respect to actual probe sequences.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention.typically conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (*see, e.g.,* Creighton, Proteins (1984)).

Macromolecular structures such as polypeptide structures can be described in terms of various levels of organization. For a general discussion of this organization, *see, e.g.,* Alberts et al., Molecular Biology of the Cell (3rd ed.,1994) and Cantor & Schimmel, Biophysical Chemistry Part I: The Conformation of Biological Macromolecules (1980). "Primary structure" refers to the amino acid sequence of a particular peptide. "Secondary structure" refers to locally ordered, three dimensional structures within a polypeptide. These structures are commonly known as domains. Domains are portions of a polypeptide that often form a compact unit of the polypeptide and are typically 25 to approximately 500 amino acids long. Typical domains are made up of sections of lesser organization such as stretches of β-sheet and α-helices. "Tertiary structure" refers to the complete three dimensional structure of a polypeptide monomer. "Quaternary structure" refers to the three dimensional structure formed, usually by the noncovalent association of independent tertiary units. Anisotropic terms are also known as energy terms.

"Nucleic acid" or "oligonucleotide" or "polynucleotide" or grammatical equivalents used herein means at least two nucleotides covalently linked together. Oligonucleotides are typically from about 5, 6, 7, 8, 9, 10, 12, 15, 25, 30, 40, 50 or more nucleotides in length, up to about 100 nucleotides in length. Nucleic acids and polynucleotides are a polymers of any length, including longer lengths, e.g., 200, 300, 500, 1000, 2000, 3000, 5000, 7000,10,000, etc. A nucleic acid of the present invention will generally contain phosphodiester bonds, although in some cases, nucleic acid analogs are included that may have alternate backbones, comprising, e.g., phosphoramidate, phosphorothioate, phosphorodithioate, or O-methylphophoroamidite linkages (see Eckstein, Oligonucleotides and Analogues: A Practical Approach, Oxford University Press); and peptide nucleic acid backbones and linkages. Other analog nucleic acids include those with positive backbones; non-ionic backbones, and non-ribose backbones, including those described in U.S. Patent Nos. 5,235,033 and 5,034,506, and Chapters 6 and 7, ASC Symposium Series 580, Carbohydrate Modifications in Antisense Research, Sanghui & Cook, eds.. Nucleic acids containing one or more carbocyclic sugars are also included within one definition of nucleic acids. Modifications of the ribose-phosphate backbone may be done for a variety of reasons, e.g. to increase the stability and half-life of such molecules in physiological environments or as probes on a biochip. Mixtures of naturally occurring nucleic acids and analogs can be made; alternatively, mixtures of different nucleic acid analogs, and mixtures of naturally occurring nucleic acids and analogs may be made.

A variety of references disclose such nucleic acid analogs, including, for example, phosphoramidate (Beaucage et al., Tetrahedron 49(10):1925 (1993) and references therein; Letsinger, J. Org. Chem. 35:3800 (1970); Sprinzl et al., Eur. J. Biochem. 81:579 (1977); Letsinger et al., Nucl. Acids Res. 14:3487 (1986); Sawai et al, Chem. Lett. 805 (1984), Letsinger et al., J. Am. Chem. Soc. 110:4470 (1988); and Pauwels et al., Chemica Scripta 26:141 91986)), phosphorothioate (Mag et al., Nucleic Acids Res. 19:1437 (1991); and U.S. Patent No. 5,644,048), phosphorodithioate (Briu et al., J. Am. Chem. Soc. 111:2321 (1989), O-methylphophoroamidite linkages (see Eckstein, Oligonucleotides and Analogues: A Practical Approach, Oxford University Press), and peptide nucleic acid backbones and linkages (see Egholm, J. Am. Chem. Soc. 114:1895 (1992); Meier et al., Chem. Int. Ed. Engl. 31:1008 (1992); Nielsen, Nature, 365:566 (1993); Carlsson et al., Nature 380:207 (1996), all of which are incorporated by reference). Other analog nucleic acids include those with positive backbones (Denpcy et al., Proc. Natl. Acad. Sci. USA 92:6097 (1995); non-ionic backbones (U.S. Patent Nos. 5,386,023, 5,637,684, 5,602,240, 5,216,141 and 4,469,863; Kiedrowshi et al., Angew. Chem. Intl. Ed. English 30:423 (1991); Letsinger et al., J. Am. Chem. Soc. 110:4470 (1988); Letsinger et al., Nucleoside & Nucleotide 13:1597 (1994); Chapters 2 and 3, ASC Symposium Series 580, "Carbohydrate Modifications in Antisense Research", Ed. Y.S. Sanghui and P. Dan Cook; Mesmaeker et al., Bioorganic & Medicinal Chem. Lett. 4:395 (1994); Jeffs et al., J. Biomolecular NMR 34:17 (1994); Tetrahedron Lett. 37:743 (1996)) and non-ribose backbones, including those described in U.S. Patent Nos. 5,235,033 and 5,034,506, and Chapters 6 and 7, ASC Symposium Series 580, "Carbohydrate Modifications in Antisense Research", Ed. Y.S. Sanghui and P. Dan Cook. Nucleic acids containing one or more carbocyclic sugars are also included within one definition of nucleic acids (see Jenkins et al., Chem. Soc. Rev. (1995) pp 169-176). Several nucleic acid analogs are described in Rawls, C & E News June 2, 1997 page 35. All of these references are hereby expressly incorporated by reference.

Particularly preferred are peptide nucleic acids (PNA) which includes peptide nucleic acid analogs. These backbones are substantially non-ionic under neutral conditions, in contrast to the highly charged phosphodiester backbone of naturally occurring nucleic acids. This results in two advantages. First, the PNA backbone exhibits improved hybridization kinetics. PNAs have larger changes in the melting temperature (Tₘ) for mismatched versus perfectly matched basepairs. DNA and RNA typically exhibit a 2-4°C drop in Tₘ for an internal mismatch. With the non-ionic PNA backbone, the drop is closer to 7-9°C. Similarly, due to their non-ionic nature, hybridization of the bases attached to these backbones is relatively insensitive to salt concentration. In addition, PNAs are not degraded by cellular enzymes, and thus can be more stable.

The nucleic acids may be single stranded or double stranded, as specified, or contain portions of both double stranded or single stranded sequence. As will be appreciated by those in the art, the depiction of a single strand also defines the sequence of the complementary strand; thus the sequences described herein also provide the complement of the sequence. The nucleic acid may be DNA, both genomic and cDNA, RNA or a hybrid, where the nucleic acid may contain combinations of deoxyribo- and ribo-nucleotides, and combinations of bases, including uracil, adenine, thymine, cytosine, guanine, inosine, xanthine hypoxanthine, isocytosine, isoguanine, etc. "Transcript" typically refers to a naturally occurring RNA, e.g., a pre-mRNA, hnRNA, or mRNA. As used herein, the term "nucleoside" includes nucleotides and nucleoside and nucleotide analogs, and modified nucleosides such as amino modified nucleosides. In addition, "nucleoside" includes non-naturally occurring analog structures. Thus, e.g. the individual units of a peptide nucleic acid, each containing a base, are referred to herein as a nucleoside.

A "label" or a "detectable moiety" is a composition detectable by spectroscopic, photochemical, biochemical, immunochemical, chemical, or other physical means. For example, useful labels include fluorescent dyes, electron-dense reagents, enzymes (*e.g.,* as commonly used in an ELISA), biotin, digoxigenin, or haptens and proteins or other entities which can be made detectable, *e.g.,* by incorporating a radiolabel into the peptide or used to detect antibodies specifically reactive with the peptide. The radioisotope may be, for example, 3H, 14C, 32P, 35S, or 125I. In some cases, particularly using antibodies against the proteins of the invention, the radioisotopes are used as toxic moieties, as described below. The labels may be incorporated into the prostate cancer nucleic acids, proteins and antibodies at any position. Any method known in the art for conjugating the antibody to the label may be employed, including those methods described by Hunter et al., Nature, 144:945 (1962); David et al., Biochemistry, 13:1014 (1974); Pain et al., J. Immunol. Meth., 40:219 (1981); and Nygren, J. Histochem. and Cytochem., 30:407 (1982). The lifetime of radiolabeled peptides or radiolabeled antibody compositions may extended by the addition of substances that stablize the radiolabeled peptide or antibody and protect it from degradation. Any substance or combination of substances that stablize the radiolabeled peptide or antibody may be used including those substances disclosed in US Patent No. 5,961,955.

An "effector" or "effector moiety" or "effector component" is a molecule that is bound (or linked, or conjugated), either covalently, through a linker or a chemical bond, or noncovalently, through ionic, van der Waals, electrostatic, or hydrogen bonds, to an antibody. The "effector" can be a variety of molecules including, e.g., detection moieties including radioactive compounds, fluorescent compounds, an enzyme or substrate, tags such as epitope tags, a toxin; activatable moieties, a chemotherapeutic agent; a lipase; an antibiotic; or a radioisotope emitting "hard" e.g., beta radiation.

A "labeled nucleic acid probe or oligonucleotide" is one that is bound, either covalently, through a linker or a chemical bond, or noncovalently, through ionic, van der Waals, electrostatic, or hydrogen bonds to a label such that the presence of the probe may be detected by detecting the presence of the label bound to the probe. Alternatively, method using high affinity interactions may achieve the same results where one of a pair of binding partners binds to the other, e.g., biotin, streptavidin.

As used herein a "nucleic acid probe or oligonucleotide" is defined as a nucleic acid capable of binding to a target nucleic acid of complementary sequence through one or more types of chemical bonds, usually through complementary base pairing, usually through hydrogen bond formation. As used herein, a probe may include natural (i.e., A, G, C, or T) or modified bases (7-deazaguanosine, inosine, etc.). In addition, the bases in a probe may be joined by a linkage other than a phosphodiester bond, so long as it does not functionally interfere with hybridization. Thus, e.g., probes may be peptide nucleic acids in which the constituent bases are joined by peptide bonds rather than phosphodiester linkages. It will be understood by one of skill in the art that probes may bind target sequences lacking complete complementarity with the probe sequence depending upon the stringency of the hybridization conditions. The probes are preferably directly labeled as with isotopes, chromophores, lumiphores, chromogens, or indirectly labeled such as with biotin to which a streptavidin complex may later bind. By assaying for the presence or absence of the probe, one can detect the presence or absence of the select sequence or subsequence. Diagnosis or prognosis may be based at the genomic level, or at the level of RNA or protein expression.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, e.g., recombinant cells express genes that are not found within the native (non-recombinant) form of the cell or express native genes that are otherwise abnormally expressed, under expressed or not expressed at all. By the term "recombinant nucleic acid" herein is meant nucleic acid, originally formed *in vitro,* in general, by the manipulation of nucleic acid, e.g., using polymerases and endonucleases, in a form not normally found in nature. In this manner, operably linkage of different sequences is achieved. Thus an isolated nucleic acid, in a linear form, or an expression vector formed *in vitro* by ligating DNA molecules that are not normally joined, are both considered recombinant for the purposes of this invention. It is understood that once a recombinant nucleic acid is made and reintroduced into a host cell or organism, it will replicate non-recombinantly, i.e., using the *in vivo* cellular machinery of the host cell rather than *in vitro* manipulations; however, such nucleic acids, once produced recombinantly, although subsequently replicated non-recombinantly, are still considered recombinant for the purposes of the invention. Similarly, a "recombinant protein" is a protein made using recombinant techniques, i.e., through the expression of a recombinant nucleic acid as depicted above.

The term "heterologous" when used with reference to portions of a nucleic acid indicates that the nucleic acid comprises two or more subsequences that are not normally found in the same relationship to each other in nature. For instance, the nucleic acid is typically recombinantly produced, having two or more sequences, e.g., from unrelated genes arranged to make a new functional nucleic acid, e.g., a promoter from one source and a coding region from another source. Similarly, a heterologous protein will often refer to two or more subsequences that are not found in the same relationship to each other in nature (e.g., a fusion protein).

A "promoter" is defined as an array of nucleic acid control sequences that direct transcription of a nucleic acid. As used herein, a promoter includes necessary nucleic acid sequences near the start site of transcription, such as, in the case of a polymerase II type promoter, a TATA element. A promoter also optionally includes distal enhancer or repressor elements, which can be located as much as several thousand base pairs from the start site of transcription. A "constitutive" promoter is a promoter that is active under most environmental and developmental conditions. An "inducible" promoter is a promoter that is active under environmental or developmental regulation. The term "operably linked" refers to a functional linkage between a nucleic acid expression control sequence (such as a promoter, or array of transcription factor binding sites) and a second nucleic acid sequence, wherein the expression control sequence directs transcription of the nucleic acid corresponding to the second sequence.

An "expression vector" is a nucleic acid construct, generated recombinantly or synthetically, with a series of specified nucleic acid elements that permit transcription of a particular nucleic acid in a host cell. The expression vector can be part of a plasmid, virus, or nucleic acid fragment. Typically, the expression vector includes a nucleic acid to be transcribed operably linked to a promoter.

The phrase "selectively (or specifically) hybridizes to" refers to the binding, duplexing, or hybridizing of a molecule only to a particular nucleotide sequence that is determinative of the presence of the nucleotide sequence, in a heterogeneous population of nucleic acids and other biologics (e.g., total cellular or library DNA or RNA). Similarly, the phrase "specifically (or selectively) binds" to an antibody or "specifically (or selectively) immunoreactive with," when referring to a protein or peptide, refers to a binding reaction that is determinative of the presence of the protein, in a heterogeneous population of proteins and other biologics. Thus, under designated immunoassay or nucleic acid hybridization conditions, the specified antibodies or nucleic acid probes bind to a particular protein nucleotide sequences at least two times the background and more typically more than 10 to 100 times background.

Specific binding to an antibody under such conditions requires an antibody that is selected for its specificity for a particular protein. For example, polyclonal antibodies raised to a particular protein, polymorphic variants, alleles, orthologs, and conservatively modified variants, or splice variants, or portions thereof, can be selected to obtain only those polyclonal antibodies that are specifically immunoreactive with the desired prostact cancer protein and not with other proteins. This selection may be achieved by subtracting out antibodies that cross-react with other molecules. A variety of immunoassay formats may be used to select antibodies specifically immunoreactive with a particular protein. For example, solid-phase ELISA immunoassays are routinely used to select antibodies specifically immunoreactive with a protein (*see, e.g.,* Harlow & Lane, Antibodies, A Laboratory Manual (1988) for a description of immunoassay formats and conditions that can be used to determine specific immunoreactivity).

The phrase "stringent hybridization conditions" refers to conditions under which a probe will hybridize to its target subsequence, typically in a complex mixture of nucleic acids, but to no other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, stringent conditions are selected to be about 5-10°C lower than the thermal melting point (Tₘ) for the specific sequence at a defined ionic strength pH. The Tₘ is the temperature (under defined ionic strength, pH, and nucleic concentration) at which 50% of the probes complementary to the target hybridize to the target sequence at equilibrium (as the target sequences are present in excess, at Tₘ, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. For selective or specific hybridization, a positive signal is at least two times background; preferably 10 times background hybridization. Exemplary stringent hybridization conditions can be as following: 50% formamide, 5x SSC, and 1% SDS, incubating at 42°C, or, 5x SSC, 1% SDS, incubating at 65°C, with wash in 0.2x SSC, and 0.1% SDS at 65°C. For PCR, a temperature of about 36°C is typical for low stringency amplification, although annealing temperatures may vary between about 32°C and 48°C depending on primer length. For high stringency PCR amplification, a temperature of about 62°C is typical, although high stringency annealing temperatures can range from about 50°C to about 65°C, depending on the primer length and specificity. Typical cycle conditions for both high and low stringency amplifications include a denaturation phase of 90°C - 95°C for 30 sec - 2 min., an annealing phase lasting 30 sec. - 2 min., and an extension phase of about 72°C for 1 - 2 min. Protocols and guidelines for low and high stringency amplification reactions are provided, e.g., in Innis et al. (1990) PCR Protocols, A Guide to Methods and Applications, Academic Press, Inc. N.Y.).

Nucleic acids that do not hybridize to each other under stringent conditions are still substantially identical if the polypeptides which they encode are substantially identical. This occurs, e.g., when a copy of a nucleic acid is created using the maximum codon degeneracy permitted by the genetic code. In such cases, the nucleic acids typically hybridize under moderately stringent hybridization conditions. Exemplary "moderately stringent hybridization conditions" include a hybridization in a buffer of 40% formamide, 1 M NaCl, 1% SDS at 37°C, and a wash in 1X SSC at 45°C. A positive hybridization is at least twice background. Those of ordinary skill will readily recognize that alternative hybridization and wash conditions can be utilized to provide conditions of similar stringency. Additional guidelines for determining hybridization parameters are provided in numerous reference, e.g., and Current Protocols in Molecular Biology, ed. Ausubel, *et al.*

The phrase "functional effects" in the context of assays for testing compounds that modulate activity of a prostate cancer protein includes the determination of a parameter that is indirectly or directly under the influence of the prostate cancer protein or nucleic acid, e.g., a functional, physical, or chemical effect, such as the ability to decrease prostate cancer. It includes ligand binding activity; cell growth on soft agar; anchorage dependence; contact inhibition and density limitation of growth; cellular proliferation; cellular transformation; growth factor or serum dependence; tumor specific marker levels; invasiveness into Matrigel; tumor growth and metastasis *in vivo*; mRNA and protein expression in cells undergoing metastasis, and other characteristics of prostate cancer cells. "Functional effects" include *in vitro, in vivo,* and *ex vivo* activities.

By "determining the functional effect" is meant assaying for a compound that increases or decreases a parameter that is indirectly or directly under the influence of a prostate cancer protein sequence, e.g., functional, enzymatic, physical and chemical effects. Such functional effects can be measured by any means known to those skilled in the art, e.g., changes in spectroscopic characteristics (e.g., fluorescence, absorbance, refractive index), hydrodynamic (e.g., shape), chromatographic, or solubility properties for the protein, measuring inducible markers or transcriptional activation of the prostate cancer protein; measuring binding activity or binding assays, e.g. binding to antibodies or other ligands, and measuring cellular proliferation. Determination of the functional effect of a compound on prostate cancer can also be performed using prostate cancer assays known to those of skill in the art such as an *in vitro* assays, e.g., cell growth on soft agar; anchorage dependence; contact inhibition and density limitation of growth; cellular proliferation; cellular transformation; growth factor or serum dependence; tumor specific marker levels; invasiveness into Matrigel; tumor growth and metastasis *in vivo;* mRNA and protein expression in cells undergoing metastasis, and other characteristics of prostate cancer cells. The functional effects can be evaluated by many means known to those skilled in the art, e.g., microscopy for quantitative or qualitative measures of alterations in morphological features, measurement of changes in RNA or protein levels for prostate cancer-associated sequences, measurement of RNA stability, identification of downstream or reporter gene expression (CAT, luciferase, β-gal, GFP and the like), e.g., via chemiluminescence, fluorescence, colorimetric reactions, antibody binding, inducible markers, and ligand binding assays.

"Inhibitors", "activators", and "modulators" of prostate cancer polynucleotide and polypeptide sequences are used to refer to activating, inhibitory, or modulating molecules or compounds identified using *in vitro* and *in vivo* assays of prostate cancer polynucleotide and polypeptide sequences. Inhibitors are compounds that, e.g., bind to, partially or totally block activity, decrease, prevent, delay activation, inactivate, desensitize, or down regulate the activity or expression of prostate cancer proteins, e.g., antagonists. Antisense nucleic acids may seem to inhibit expression and subsequent function of the protein. "Activators" are compounds that increase, open, activate, facilitate, enhance activation, sensitize, agonize, or up regulate prostate cancer protein activity. Inhibitors, activators, or modulators also include genetically modified versions of prostate cancer proteins, e.g., versions with altered activity, as well as naturally occurring and synthetic ligands, antagonists, agonists, antibodies, small chemical molecules and the like. Such assays for inhibitors and activators include, e.g., expressing the prostate cancer protein *in vitro,* in cells, or cell membranes, applying putative modulator compounds, and then determining the functional effects on activity, as described above. Activators and inhibitors of prostate cancer can also be identified by incubating prostate cancer cells with the test compound and determining increases or decreases in the expression of 1 or more prostate cancer proteins, e.g., 1, 2, 3, 4, 5, 10, 15, 20, 25, 30, 40, 50 or more prostate cancer proteins, such as prostate cancer proteins encoded by the sequences set out in Tables 1-16.

Samples or assays comprising prostate cancer proteins that are treated with a potential activator, inhibitor, or modulator are compared to control samples without the inhibitor, activator, or modulator to examine the extent of inhibition. Control samples (untreated with inhibitors) are assigned a relative protein activity value of 100%. Inhibition of a polypeptide is achieved when the activity value relative to the control is about 80%, preferably 50%, more preferably 25-0%. Activation of a prostate cancer polypeptide is achieved when the activity value relative to the control (untreated with activators) is 110%, more preferably 150%, more preferably 200-500% (i.e., two to five fold higher relative to the control), more preferably 1000-3000% higher.

The phrase "changes in cell growth" refers to any change in cell growth and proliferation characteristics *in vitro* or *in vivo,* such as formation of foci, anchorage independence, semi-solid or soft agar growth, changes in contact inhibition and density limitation of growth, loss of growth factor or serum requirements, changes in cell morphology, gaining or losing immortalization, gaining or losing tumor specific markers, ability to form or suppress tumors when injected into suitable animal hosts, and/or immortalization of the cell. *See, e.g.,* Freshney, Culture of Animal Cells a Manual of Basic Technique pp. 231-241 (3rd ed. 1994).

"Tumor cell" refers to precancerous, cancerous, and normal cells in a tumor.

"Cancer cells," "transformed" cells or "transformation" in tissue culture, refers to spontaneous or induced phenotypic changes that do not necessarily involve the uptake of new genetic material. Although transformation can arise from infection with a transforming virus and incorporation of new genomic DNA, or uptake of exogenous DNA, it can also arise spontaneously or following exposure to a carcinogen, thereby mutating an endogenous gene. Transformation is associated with phenotypic changes, such as immortalization of cells, aberrant growth control, nonmorphological changes, and/or malignancy *(see,* Freshney, Culture of Animal Cells a Manual of Basic Technique (3rd ed. 1994)).

"Antibody" refers to a polypeptide comprising a framework region from an immunoglobulin gene or fragments thereof that specifically binds and recognizes an antigen. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Typically, the antigen-binding region of an antibody or its functional equivalent will be most critical in specificity and affinity of binding. See Paul, Fundamental Immunology.

An exemplary immunoglobulin (antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

Antibodies exist, e.g., as intact immunoglobulins or as a number of well-characterized fragments produced by digestion with various peptidases. Thus, e.g., pepsin digests an antibody below the disulfide linkages in the hinge region to produce F(ab)'₂, a dimer of Fab which itself is a light chain joined to V_{H}-C_{H}1 by a disulfide bond. The F(ab)'₂ may be reduced under mild conditions to break the disulfide linkage in the hinge region, thereby converting the F(ab)'₂ dimer into an Fab' monomer. The Fab' monomer is essentially Fab with part of the hinge region (*see* Fundamental Immunology (Paul ed., 3d ed. 1993). While various antibody fragments are defined in terms of the digestion of an intact antibody, one of skill will appreciate that such fragments may be synthesized *de novo* either chemically or by using recombinant DNA methodology. Thus, the term antibody, as used herein, also includes antibody fragments either produced by the modification of whole antibodies, or those synthesized *de novo* using recombinant DNA methodologies (e.g., single chain Fv) or those identified using phage display libraries (*see, e.g.,* McCafferty et al., Nature 348:552-554 (1990))

For preparation of antibodies, e.g., recombinant, monoclonal, or polyclonal antibodies, many technique known in the art can be used (*see, e.g.,* Kohler & Milstein, Nature 256:495-497 (1975); Kozbor et al., Immunology Today 4:72 (1983); Cole et al., pp. 77-96 in Monoclonal Antibodies and Cancer Therapy (1985); Coligan, Current Protocols in Immunology (1991); Harlow & Lane, Antibodies, A Laboratory Manual (1988); and Goding, Monoclonal Antibodies: Principles and Practice (2d ed. 1986)). Techniques for the production of single chain antibodies (U.S. Patent 4,946,778) can be adapted to produce antibodies to polypeptides of this invention. Also, transgenic mice, or other organisms such as other mammals, may be used to express humanized antibodies. Alternatively, phage display technology can be used to identify antibodies and heteromeric Fab fragments that specifically bind to selected antigens (*see, e.g.,* McCafferty et al., Nature 348:552-554 (1990); Marks et al., Biotechnology 10:779-783 (1992)).

A "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, *e.g.*, an enzyme, toxin, hormone, growth factor, drug, etc.; or (b) the variable region, or a portion thereof, is altered, replaced or exchanged with a variable region having a different or altered antigen specificity.

### Identification of prostate cancer-associated sequences

In one aspect, the expression levels of genes are determined in different patient samples for which diagnosis information is desired, to provide expression profiles. An expression profile of a particular sample is essentially a "fingerprint" of the state of the sample; while two states may have any particular gene similarly expressed, the evaluation of a number of genes simultaneously allows the generation of a gene expression profile that is characteristic of the state of the cell. That is, normal tissue (e.g., normal prostate or other tissue) may be distinguished from cancerous or metastatic cancerous tissue of the prostate, or prostate cancer tissue or metastatic prostate cancerous tissue can be compared with tissue samples of prostate and other tissues from surviving cancer patients. By comparing expression profiles of tissue in known different prostate cancer states, information regarding which genes are important (including both up- and down-regulation of genes) in each of these states is obtained.

The identification of sequences that are differentially expressed in prostate cancer versus non-prostate cancer tissue allows the use of this information in a number of ways. For example, a particular treatment regime may be evaluated: does a chemotherapeutic drug act to down-regulate prostate cancer, and thus tumor growth or recurrence, in a particular patient. Similarly, diagnosis and treatment outcomes may be done or confirmed by comparing patient samples with the known expression profiles. Metastatic tissue can also be analyzed to determine the stage of prostate cancer in the tissue. Furthermore, these gene expression profiles (or individual genes) allow screening of drug candidates with an eye to mimicking or altering a particular expression profile; e.g., screening can be done for drugs that suppress the prostate cancer expression profile. This may be done by making biochips comprising sets of the important prostate cancer genes, which can then be used in these screens. These methods can also be done on the protein basis; that is, protein expression levels of the prostate cancer proteins can be evaluated for diagnostic purposes or to screen candidate agents. In addition, the prostate cancer nucleic acid sequences can be administered for gene therapy purposes, including the administration of antisense nucleic acids, or the prostate cancer proteins (including antibodies and other modulators thereof) administered as therapeutic drugs.

Thus the present invention provides nucleic acid and protein sequences that are differentially expressed in prostate cancer, herein termed "prostate cancer sequences." As outlined below, prostate cancer sequences include those that are up-regulated (i.e., expressed at a higher level) in prostate cancer, as well as those that are down-regulated (i.e., expressed at a lower level). In a preferred embodiment, the prostate cancer sequences are from humans; however, as will be appreciated by those in the art, prostate cancer sequences from other organisms may be useful in animal models of disease and drug evaluation; thus, other prostate cancer sequences are provided, from vertebrates, including mammals, including rodents (rats, mice, hamsters, guinea pigs, etc.), primates, farm animals (including sheep, goats, pigs, cows, horses, etc.) and pets, e.g., (dogs, cats, etc.). Prostate cancer sequences from other organisms may be obtained using the techniques outlined below.

Prostate cancer sequences can include both nucleic acid and amino acid sequences. As will be appreciated by those in the art and is more fully outlined below, prostate cancer nucleic acid sequences are useful in a variety of applications, including diagnostic applications, which will detect naturally occurring nucleic acids, as well as screening applications; e.g., biochips comprising nucleic acid probes or PCR microtiter plates with selected probes to the prostate cancer sequences can be generated.

A prostate cancer sequence can be initially identified by substantial nucleic acid and/or amino acid sequence homology to the prostate cancer sequences outlined herein. Such homology can be based upon the overall nucleic acid or amino acid sequence, and is generally determined as outlined below, using either homology programs or hybridization conditions.

For identifying prostate cancer-associated sequences, the prostate cancer screen typically includes comparing genes identified in different tissues, e.g., normal and cancerous tissues, or tumor tissue samples from patients who have metastatic disease vs. non metastatic tissue. Other suitable tissue comparisons include comparing prostate cancer samples with metastatic cancer samples from other cancers, such as lung, breast, gastrointestinal cancers, ovarian, etc. Samples of different stages of prostate cancer, e.g., survivor tissue, drug resistant states, and tissue undergoing metastasis, are applied to biochips comprising nucleic acid probes. The samples are first microdissected, if applicable, and treated as is known in the art for the preparation of mRNA. Suitable biochips are commercially available, e.g. from Affymetrix. Gene expression profiles as described herein are generated and the data analyzed.

In one embodiment, the genes showing changes in expression as between normal and disease states are compared to genes expressed in other normal tissues, preferably normal prostate, but also including, and not limited to lung, heart, brain, liver, breast, kidney, muscle, colon, small intestine, large intestine, spleen, bone and placenta. In a preferred embodiment, those genes identified during the prostate cancer screen that are expressed in any significant amount in other tissues are removed from the profile, although in some embodiments, this is not necessary. That is, when screening for drugs, it is usually preferable that the target be disease specific, to minimize possible side effects.

In a preferred embodiment, prostate cancer sequences are those that are up-regulated in prostate cancer; that is, the expression of these genes is higher in the prostate cancer tissue as compared to non-cancerous tissue. "Up-regulation" as used herein often means at least about a two-fold change, preferably at least about a three fold change, with at least about five-fold or higher being preferred. All unigene cluster identification numbers and accession numbers herein are for the GenBank sequence database and the sequences of the accession numbers are hereby expressly incorporated by reference. GenBank is known in the art, see, *e.g.,* Benson, DA, et al., Nucleic Acids Research 26:1-7 (1998) and http://www.ncbi.nlm.nih.gov/. Sequences are also available in other databases, e.g., European Molecular Biology Laboratory (EMBL) and DNA Database of Japan (DDBJ).

In another preferred embodiment, prostate cancer sequences are those that are down-regulated in prostate cancer; that is, the expression of these genes is lower in prostate cancer tissue as compared to non-cancerous tissue (*see, e.g.,* Tables 8, 12 and 14). "Down-regulation" as used herein often means at least about a 1.5-fold change more preferrably a two-fold change, preferably at least about a three fold change, with at least about five-fold or higher being most preferred.

### Informatics

The ability to identify genes that are over or under expressed in prostate cancer can additionally provide high-resolution, high-sensitivity datasets which can be used in the areas of diagnostics, therapeutics, drug development, pharmacogenetics, protein structure, biosensor development, and other related areas. For example, the expression profiles can be used in diagnostic or prognostic evaluation of patients with prostate cancer. Or as another example, subcellular toxicological information can be generated to better direct drug structure and activity correlation (*see* Anderson, Pharmaceutical Proteomics: Targets, Mechanism, and Function, paper presented at the IBC Proteomics conference, Coronado, CA (June 11-12, 1998)). Subcellular toxicological information can also be utilized in a biological sensor device to predict the likely toxicological effect of chemical exposures and likely tolerable exposure thresholds (*see* U.S. Patent No. 5,811,231). Similar advantages accrue from datasets relevant to other biomolecules and bioactive agents (e.g., nucleic acids, saccharides, lipids, drugs, and the like).

Thus, in another embodiment, the present invention provides a database that includes at least one set of assay data. The data contained in the database is acquired, e.g., using array analysis either singly or in a library format. The database can be in substantially any form in which data can be maintained and transmitted, but is preferably an electronic database. The electronic database of the invention can be maintained on any electronic device allowing for the storage of and access to the database, such as a personal computer, but is preferably distributed on a wide area network, such as the World Wide Web.

The focus of the present section on databases that include peptide sequence data is for clarity of illustration only. It will be apparent to those of skill in the art that similar databases can be assembled for any assay data acquired using an assay of the invention.

The compositions and methods for identifying and/or quantitating the relative and/or absolute abundance of a variety of molecular and macromolecular species from a biological sample undergoing prostate cancer, i.e., the identification of prostate cancer-associated sequences described herein, provide an abundance of information, which can be correlated with pathological conditions, predisposition to disease, drug testing, therapeutic monitoring, gene-disease causal linkages, identification of correlates of immunity and physiological status, among others. Although the data generated from the assays of the invention is suited for manual review and analysis, in a preferred embodiment, prior data processing using high-speed computers is utilized.

An array of methods for indexing and retrieving biomolecular information is known in the art. For example, U.S. Patents 6,023,659 and 5,966,712 disclose a relational database system for storing biomolecular sequence information in a manner that allows sequences to be catalogued and searched according to one or more protein function hierarchies. U.S. Patent 5,953,727 discloses a relational database having sequence records containing information in a format that allows a collection of partial-length DNA sequences to be catalogued and searched according to association with one or more sequencing projects for obtaining full-length sequences from the collection of partial length sequences. U.S. Patent 5,706,498 discloses a gene database retrieval system for making a retrieval of a gene sequence similar to a sequence data item in a gene database based on the degree of similarity between a key sequence and a target sequence. U.S. Patent 5,538,897 discloses a method using mass spectroscopy fragmentation patterns of peptides to identify amino acid sequences in computer databases by comparison of predicted mass spectra with experimentally-derived mass spectra using a closeness-of-fit measure. U.S. Patent 5,926,818 discloses a multi-dimensional database comprising a functionality for multi-dimensional data analysis described as on-line analytical processing (OLAP), which entails the consolidation of projected and actual data according to more than one consolidation path or dimension. U.S. Patent 5,295,261 reports a hybrid database structure in which the fields of each database record are divided into two classes, navigational and informational data, with navigational fields stored in a hierarchical topological map which can be viewed as a tree structure or as the merger of two or more such tree structures.

See also Mount et al., Bioinformatics (2001); Biological Sequence Analysis: Probabilistic Models of Proteins and Nucleic Acids (Durbin et al., eds., 1999); Bioinformatics: A Practical Guide to the Analysis of Genes and Proteins (Baxevanis & Oeullette eds., 1998)); Rashidi & Buehler, Bioinformatics: Basic Applications in Biological Science and Medicine (1999); Introduction to Computational Molecular Biology (Setubal et al., eds 1997); Bioinformatics: Methods and Protocols (Misener & Krawetz, eds, 2000); Bioinformatics: Sequence, Structure, and Databanks: A Practical Approach (Higgins & Taylor, eds., 2000); Brown, Bioinformatics: A Biologist's Guide to Biocomputing and the Internet (2001); Han & Kamber, Data Mining: Concepts and Techniques (2000); and Waterman, Introduction to Computational Biology: Maps, Sequences, and Genomes (1995).

The present invention provides a computer database comprising a computer and software for storing in computer-retrievable form assay data records cross-tabulated, e.g., with data specifying the source of the target-containing sample from which each sequence specificity record was obtained.

In an exemplary embodiment, at least one of the sources of target-containing sample is from a control tissue sample known to be free of pathological disorders. In a variation, at least one of the sources is a known pathological tissue specimen, e.g., a neoplastic lesion or another tissue specimen to be analyzed for prostate cancer. In another variation, the assay records cross-tabulate one or more of the following parameters for each target species in a sample: (1) a unique identification code, which can include, e.g., a target molecular structure and/or characteristic separation coordinate (e.g., electrophoretic coordinates); (2) sample source; and (3) absolute and/or relative quantity of the target species present in the sample.

The invention also provides for the storage and retrieval of a collection of target data in a computer data storage apparatus, which can include magnetic disks, optical disks, magneto-optical disks, DRAM, SRAM, SGRAM, SDRAM, RDRAM, DDR RAM, magnetic bubble memory devices, and other data storage devices, including CPU registers and on-CPU data storage arrays. Typically, the target data records are stored as a bit pattern in an array of magnetic domains on a magnetizable medium or as an array of charge states or transistor gate states, such as an array of cells in a DRAM device (e.g., each cell comprised of a transistor and a charge storage area, which may be on the transistor). In one embodiment, the invention provides such storage devices, and computer systems built therewith, comprising a bit pattern encoding a protein expression fingerprint record comprising unique identifiers for at least 10 target data records cross-tabulated with target source.

When the target is a peptide or nucleic acid, the invention preferably provides a method for identifying related peptide or nucleic acid sequences, comprising performing a computerized comparison between a peptide or nucleic acid sequence assay record stored in or retrieved from a computer storage device or database and at least one other sequence. The comparison can include a sequence analysis or comparison algorithm or computer program embodiment thereof (e.g., FASTA, TFASTA, GAP, BESTFIT) and/or the comparison may be of the relative amount of a peptide or nucleic acid sequence in a pool of sequences determined from a polypeptide or nucleic acid sample of a specimen.

The invention also preferably provides a magnetic disk, such as an IBM-compatible (DOS, Windows, Windows95/98/2000, Windows NT, OS/2) or other format (*e*.g., Linux, SunOS, Solaris, AIX, SCO Unix, VMS, MV, Macintosh, etc.) floppy diskette or hard (fixed, Winchester) disk drive, comprising a bit pattern encoding data from an assay of the invention in a file format suitable for retrieval and processing in a computerized sequence analysis, comparison, or relative quantitation method.

The invention also provides a network, comprising a plurality of computing devices linked via a data link, such as an Ethernet cable (coax or 10BaseT), telephone line, ISDN line, wireless network, optical fiber, or other suitable signal transmission medium, whereby at least one network device (e.g., computer, disk array, etc.) comprises a pattern of magnetic domains (e.g., magnetic disk) and/or charge domains (e.g., an array of DRAM cells) composing a bit pattern encoding data acquired from an assay of the invention.

The invention also provides a method for transmitting assay data that includes generating an electronic signal on an electronic communications device, such as a modem, ISDN terminal adapter, DSL, cable modem, ATM switch, or the like, wherein the signal includes (in native or encrypted format) a bit pattern encoding data from an assay or a database comprising a plurality of assay results obtained by the method of the invention.

In a preferred embodiment, the invention provides a computer system for comparing a query target to a database containing an array of data structures, such as an assay result obtained by the method of the invention, and ranking database targets based on the degree of identity and gap weight to the target data. A central processor is preferably initialized to load and execute the computer program for alignment and/or comparison of the assay results. Data for a query target is entered into the central processor via an I/O device. Execution of the computer program results in the central processor retrieving the assay data from the data file, which comprises a binary description of an assay result.

The target data or record and the computer program can be transferred to secondary memory, which is typically random access memory (e.g., DRAM, SRAM, SGRAM, or SDRAM). Targets are ranked according to the degree of correspondence between a selected assay characteristic (e.g., binding to a selected affinity moiety) and the same characteristic of the query target and results are output via an I/O device. For example, a central processor can be a conventional computer (e.g., Intel Pentium, PowerPC, Alpha, PA-8000, SPARC, MIPS 4400, MIPS 10000, VAX, etc.); a program can be a commercial or public domain molecular biology software package (e.g., UWGCG Sequence Analysis Software, Darwin); a data file can be an optical or magnetic disk, a data server, a memory device (e.g., DRAM, SRAM, SGRAM, SDRAM, EPROM, bubble memory, flash memory, *etc.*); an I/O device can be a terminal comprising a video display and a keyboard, a modem, an ISDN terminal adapter, an Ethernet port, a punched card reader, a magnetic strip reader, or other suitable I/O device.

The invention also preferably provides the use of a computer system, such as that described above, which comprises: (1) a computer; (2) a stored bit pattern encoding a collection of peptide sequence specificity records obtained by the methods of the invention, which may be stored in the computer; (3) a comparison target, such as a query target; and (4) a program for alignment and comparison, typically with rank-ordering of comparison results on the basis of computed similarity values.

### Characteristics of prostate cancer-associated proteins

Prostate cancer proteins of the present invention may be classified as secreted proteins, transmembrane proteins or intracellular proteins. In one embodiment, the prostate cancer protein is an intracellular protein. Intracellular proteins may be found in the cytoplasm and/or in the nucleus. Intracellular proteins are involved in all aspects of cellular function and replication (including, e.g., signaling pathways); aberrant expression of such proteins often results in unregulated or disregulated cellular processes (*see, e.g.,* Molecular Biology of the Cell (Alberts, ed., 3rd ed., 1994). For example, many intracellular proteins have enzymatic activity such as protein kinase activity, protein phosphatase activity, protease activity, nucleotide cyclase activity, polymerase activity and the like. Intracellular proteins also serve as docking proteins that are involved in organizing complexes of proteins, or targeting proteins to various subcellular localizations, and are involved in maintaining the structural integrity of organelles.

An increasingly appreciated concept in characterizing proteins is the presence in the proteins of one or more motifs for which defined functions have been attributed. In addition to the highly conserved sequences found in the enzymatic domain of proteins, highly conserved sequences have been identified in proteins that are involved in protein-protein interaction. For example, Src-homology-2 (SH2) domains bind tyrosine-phosphorylated targets in a sequence dependent manner. PTB domains, which are distinct from SH2 domains, also bind tyrosine phosphorylated targets. SH3 domains bind to proline-rich targets. In addition, PH domains, tetratricopeptide repeats and WD domains to name only a few, have been shown to mediate protein-protein interactions. Some of these may also be involved in binding to phospholipids or other second messengers. As will be appreciated by one of ordinary skill in the art, these motifs can be identified on the basis of primary sequence; thus, an analysis of the sequence of proteins may provide insight into both the enzymatic potential of the molecule and/or molecules with which the protein may associate. One useful database is Pfam (protein families), which is a large collection of multiple sequence alignments and hidden Markov models covering many common protein domains. Versions are available via the internet from Washington University in St. Louis, the Sanger Center in England, and the Karolinska Institute in Sweden (*see, e.g.,* Bateman et al., Nuc. Acids Res. 28:263-266 (2000); Sonnhammer et al., Proteins 28:405-420 (1997); Bateman et al., Nuc. Acids Res. 27:260-262 (1999); and Sonnhammer et al., Nuc. Acids Res. 26:320-322-(1998)).

In another embodiment, the prostate cancer sequences are transmembrane proteins. Transmembrane proteins are molecules that span a phospholipid bilayer of a cell. They may have an intracellular domain, an extracellular domain, or both. The intracellular domains of such proteins may have a number of functions including those already described for intracellular proteins. For example, the intracellular domain may have enzymatic activity and/or may serve as a binding site for additional proteins. Frequently the intracellular domain of transmembrane proteins serves both roles. For example certain receptor tyrosine kinases have both protein kinase activity and SH2 domains. In addition, autophosphorylation of tyrosines on the receptor molecule itself, creates binding sites for additional SH2 domain containing proteins.

Transmembrane proteins may contain from one to many transmembrane domains. For example, receptor tyrosine kinases, certain cytokine receptors, receptor guanylyl cyclases and receptor serine/threonine protein kinases contain a single transmembrane domain. However, various other proteins including channels and adenylyl cyclases contain numerous transmembrane domains. Many important cell surface receptors such as G protein coupled receptors (GPCRs) are classified as "seven transmembrane domain" proteins, as they contain 7 membrane spanning regions. Characteristics of transmembrane domains include approximately 20 consecutive hydrophobic amino acids that may be followed by charged amino acids. Therefore, upon analysis of the amino acid sequence of a particular protein, the localization and number of transmembrane domains within the protein may be predicted (*see, e.g.* PSORT web site http://psort.nibb.ac.jp/). Important transmembrane protein receptors include, but are not limited to the insulin receptor, insulin-like growth factor receptor, human growth hormone receptor, glucose transporters, transferrin receptor, epidermal growth factor receptor, low density lipoprotein receptor, epidermal growth factor receptor, leptin receptor, interleukin receptors, e.g. IL-1 receptor, IL-2 receptor,

The extracellular domains of transmembrane proteins are diverse; however, conserved motifs are found repeatedly among various extracellular domains. Conserved structure and/or functions have been ascribed to different extracellular motifs. Many extracellular domains are involved in binding to other molecules. In one aspect, extracellular domains are found on receptors. Factors that bind the receptor domain include circulating ligands, which may be peptides, proteins, or small molecules such as adenosine and the like. For example, growth factors such as EGF, FGF and PDGF are circulating growth factors that bind to their cognate receptors to initiate a variety of cellular responses. Other factors include cytokines, mitogenic factors, neurotrophic factors and the like. Extracellular domains also bind to cell-associated molecules. In this respect, they mediate cell-cell interactions. Cell-associated ligands can be tethered to the cell, e.g., via a glycosylphosphatidylinositol (GPI) anchor, or may themselves be transmembrane proteins. Extracellular domains also associate with the extracellular matrix and contribute to the maintenance of the cell structure.

Prostate cancer proteins that are transmembrane are particularly preferred in the present invention as they are readily accessible targets for immunotherapeutics, as are described herein. In addition, as outlined below, transmembrane proteins can be also useful in imaging modalities. Antibodies may be used to label such readily accessible proteins *in situ*. Alternatively, antibodies can also label intracellular proteins, in which case samples are typically permeablized to provide access to intracellular proteins.

It will also be appreciated by those in the art that a transmembrane protein can be made soluble by removing transmembrane sequences, e.g., through recombinant methods. Furthermore, transmembrane proteins that have been made soluble can be made to be secreted through recombinant means by adding an appropriate signal sequence.

In another embodiment, the prostate cancer proteins are secreted proteins; the secretion of which can be either constitutive or regulated. These proteins have a signal peptide or signal sequence that targets the molecule to the secretory pathway. Secreted proteins are involved in numerous physiological events; by virtue of their circulating nature, they serve to transmit signals to various other cell types. The secreted protein may function in an autocrine manner (acting on the cell that secreted the factor), a paracrine manner (acting on cells in close proximity to the cell that secreted the factor) or an endocrine manner (acting on cells at a distance). Thus secreted molecules find use in modulating or altering numerous aspects of physiology. Prostate cancer proteins that are secreted proteins are particularly preferred in the present invention as they serve as good targets for diagnostic markers, *e.g.*, for blood, plasma, serum, or stool tests.

### Use of prostate cancer nucleic acids

As described above, prostate cancer sequence is initially identified by substantial nucleic acid and/or amino acid sequence homology or linkage to the prostate cancer sequences outlined herein. Such homology can be based upon the overall nucleic acid or amino acid sequence, and is generally determined as outlined below, using either homology programs or hybridization conditions. Typically, linked sequences on a mRNA are found on the same molecule.

The prostate cancer nucleic acid sequences of the invention, e.g., the sequences in Tables 1-16, can be fragments of larger genes, i.e., they are nucleic acid segments. "Genes" in this context includes coding regions, non-coding regions, and mixtures of coding and non-coding regions. Accordingly, as will be appreciated by those in the art, using the sequences provided herein, extended sequences, in either direction, of the prostate cancer genes can be obtained, using techniques well known in the art for cloning either longer sequences or the full length sequences; see Ausubel, *et al., supra.* Much can be done by informatics and many sequences can be clustered to include multiple sequences corresponding to a single gene, e.g., systems such as UniGene (see, http://www.ncbi.nlm.nih.gov/UniGene/).

Once the prostate cancer nucleic acid is identified, it can be cloned and, if necessary, its constituent parts recombined to form the entire prostate cancer nucleic acid coding regions or the entire mRNA sequence. Once isolated from its natural source, e.g., contained within a plasmid or other vector or excised therefrom as a linear nucleic acid segment, the recombinant prostate cancer nucleic acid can be further-used as a probe to identify and isolate other prostate cancer nucleic acids, e.g., extended coding regions. It can also be used as a "precursor" nucleic acid to make modified or variant prostate cancer nucleic acids and proteins.

The prostate cancer nucleic acids of the present invention are used in several ways. In a first embodiment, nucleic acid probes to the prostate cancer nucleic acids are made and attached to biochips to be used in screening and diagnostic methods, as outlined below, or for administration, e.g., for gene therapy, vaccine, and/or antisense applications. Alternatively, the prostate cancer nucleic acids that include coding regions of prostate cancer proteins can be put into expression vectors for the expression of prostate cancer proteins, again for screening purposes or for administration to a patient.

In a preferred embodiment, nucleic acid probes to prostate cancer nucleic acids (both the nucleic acid sequences outlined in the figures and/or the complements thereof) are made. The nucleic acid probes attached to the biochip are designed to be substantially complementary to the prostate cancer nucleic acids, *i.e.* the target sequence (either the target sequence of the sample or to other probe sequences, e.g., in sandwich assays), such that hybridization of the target sequence and the probes of the present invention occurs. As outlined below, this complementarity need not be perfect; there may be any number of base pair mismatches which will interfere with hybridization between the target sequence and the single stranded nucleic acids of the present invention. However, if the number of mutations is so great that no hybridization can occur under even the least stringent of hybridization conditions, the sequence is not a complementary target sequence. Thus, by "substantially complementary" herein is meant that the probes are sufficiently complementary to the target sequences to hybridize under normal reaction conditions, particularly high stringency conditions, as outlined herein.

A nucleic acid probe is generally single stranded but can be partially single and partially double stranded. The strandedness of the probe is dictated by the structure, composition, and properties of the target sequence. In general, the nucleic acid probes range from about 8 to about 100 bases long, with from about 10 to about 80 bases being preferred, and from about 30 to about 50 bases being particularly preferred. That is, generally whole genes are not used. In some embodiments, much longer nucleic acids can be used, up to hundreds of bases.

In a preferred embodiment, more than one probe per sequence is used, with either overlapping probes or probes to different sections of the target being used. That is, two, three, four or more probes, with three being preferred, are used to build in a redundancy for a particular target. The probes can be overlapping (i.e., have some sequence in common), or separate. In some cases, PCR primers may be used to amplify signal for higher sensitivity.

As will be appreciated by those in the art, nucleic acids can be attached or immobilized to a solid support in a wide variety of ways. By "immobilized" and grammatical equivalents herein is meant the association or binding between the nucleic acid probe and the solid support is sufficient to be stable under the conditions of binding, washing, analysis, and removal as outlined below. The binding can typically be covalent or non-covalent. By "non-covalent binding" and grammatical equivalents herein is meant one or more of electrostatic, hydrophilic, and hydrophobic interactions. Included in non-covalent binding is the covalent attachment of a molecule, such as, streptavidin to the support and the non-covalent binding of the biotinylated probe to the streptavidin. By "covalent binding" and grammatical equivalents herein is meant that the two moieties, the solid support and the probe, are attached by at least one bond, including sigma bonds, pi bonds and coordination bonds. Covalent bonds can be formed directly between the probe and the solid support or can be formed by a cross linker or by inclusion of a specific reactive group on either the solid support or the probe or both molecules. Immobilization may also involve a combination of covalent and non-covalent interactions.

In general, the probes are attached to the biochip in a wide variety of ways, as will be appreciated by those in the art. As described herein, the nucleic acids can either be synthesized first, with subsequent attachment to the biochip, or can be directly synthesized on the biochip.

The biochip comprises a suitable solid substrate. By "substrate" or "solid support" or other grammatical equivalents herein is meant a material that can be modified to contain discrete individual sites appropriate for the attachment or association of the nucleic acid probes and is amenable to at least one detection method. As will be appreciated by those in the art, the number of possible substrates are very large, and include, but are not limited to, glass and modified or functionalized glass, plastics (including acrylics, polystyrene and copolymers of styrene and other materials, polypropylene, polyethylene, polybutylene, polyurethanes, TeflonJ, etc.), polysaccharides, nylon or nitrocellulose, resins, silica or silica-based materials including silicon and modified silicon, carbon, metals, inorganic glasses, plastics, etc. In general, the substrates allow optical detection and do not appreciably fluoresce. A preferred substrate is described in copending application entitled Reusable Low Fluorescent Plastic Biochip, U.S. Application Serial No. 09/270,214, filed March 15,1999, herein incorporated by reference in its entirety.

Generally the substrate is planar, although as will be appreciated by those in the art, other configurations of substrates may be used as well. For example, the probes may be placed on the inside surface of a tube, for flow-through sample analysis to minimize sample volume. Similarly, the substrate may be flexible, such as a flexible foam, including closed cell foams made of particular plastics.

In a preferred embodiment, the surface of the biochip and the probe may be derivatized with chemical functional groups for subsequent attachment of the two. Thus, e.g., the biochip is derivatized with a chemical functional group including, but not limited to, amino groups, carboxy groups, oxo groups and thiol groups, with amino groups being particularly preferred. Using these functional groups, the probes can be attached using functional groups on the probes. For example, nucleic acids containing amino groups can be attached to surfaces comprising amino groups, e.g. using linkers as are known in the art; e.g., homo-or hetero-bifunctional linkers as are well known (*see* 1994 Pierce Chemical Company catalog, technical section on cross-linkers, pages 155-200). In addition, in some cases, additional linkers, such as alkyl groups (including substituted and heteroalkyl groups) may be used.

In this embodiment, oligonucleotides are synthesized as is known in the art, and then attached to the surface of the solid support. As will be appreciated by those skilled in the art, either the 5' or 3' terminus may be attached to the solid support, or attachment may be via an internal nucleoside.

In another embodiment, the immobilization to the solid support may be very strong, yet non-covalent. For example, biotinylated oligonucleotides can be made, which bind to surfaces covalently coated with streptavidin, resulting in attachment.

Alternatively, the oligonucleotides may be synthesized on the surface, as is known in the art. For example, photoactivation techniques utilizing photopolymerization compounds and techniques are used. In a preferred embodiment, the nucleic acids can be synthesized in situ, using well known photolithographic techniques, such as those described in WO 95/25116; WO 95/35505; U.S. Patent Nos. 5,700,637 and 5,445,934; and references cited within, all of which are expressly incorporated by reference; these methods of attachment form the basis of the Affimetrix GeneChip^{™} technology.

Often, amplification-based assays are performed to measure the expression level of prostate cancer-associated sequences. These assays are typically performed in conjunction with reverse transcription. In such assays, a prostate cancer-associated nucleic acid sequence acts as a template in an amplification reaction (e.g., Polymerase Chain Reaction, or PCR). In a quantitative amplification, the amount of amplification product will be proportional to the amount of template in the original sample. Comparison to appropriate controls provides a measure of the amount of prostate cancer-associated RNA. Methods of quantitative amplification are well known to those of skill in the art. Detailed protocols for quantitative PCR are provided, e.g., in Innis et al., PCR Protocols, A Guide to Methods and Applications (1990).

In some embodiments, a TaqMan based assay is used to measure expression. TaqMan based assays use a fluorogenic oligonucleotide probe that contains a 5' fluorescent dye and a 3' quenching agent. The probe hybridizes to a PCR product, but cannot itself be extended due to a blocking agent at the 3' end. When the PCR product is amplified in subsequent cycles, the 5' nuclease activity of the polymerase, *e.g.*, AmpliTaq, results in the cleavage of the TaqMan probe. This cleavage separates the 5' fluorescent dye and the 3' quenching agent, thereby resulting in an increase in fluorescence as a function of amplification (*see,* e.g., literature provided by Perkin-Elmer, *e.g.*, www2.perkin-elmer.com).

Other suitable amplification methods include, but are not limited to, ligase chain reaction (LCR) (*see* Wu & Wallace, Genomics 4:560 (1989), Landegren et al., Science 241:1077 (1988), and Barringer et al., Gene 89:117 (1990)), transcription amplification (Kwoh et al., Proc. Natl. Acad Sci. USA 86:1173 (1989)), self-sustained sequence replication (Guatelli et al., Proc. Nat. Acad Sci. USA 87:1874 (1990)), dot PCR, and linker adapter PCR, etc.

### Expression of prostate cancer proteins from nucleic acids

In a preferred embodiment, prostate cancer nucleic acids, e.g., encoding prostate cancer proteins are used to make a variety of expression vectors to express prostate cancer proteins which can then be used in screening assays, as described below. Expression vectors and recombinant DNA technology are well known to those of skill in the art (*see, e.g.,* Ausubel, *supra,* and Gene Expression Systems (Fernandez & Hoeffler, eds, 1999)) and are used to express proteins. The expression vectors may be either self-replicating extrachromosomal vectors or vectors which integrate into a host genome. Generally, these expression vectors include transcriptional and translational regulatory nucleic acid operably linked to the nucleic acid encoding the prostate cancer protein. The term "control sequences" refers to DNA sequences used for the expression of an operably linked coding sequence in a particular host organism. Control sequences that are suitable for prokaryotes, e.g., include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is typically accomplished by ligation at convenient restriction sites. If such sites do not exist, synthetic oligonucleotide adaptors or linkers are used in accordance with conventional practice. Transcriptional and translational regulatory nucleic acid will generally be appropriate to the host cell used to express the prostate cancer protein. Numerous types of appropriate expression vectors, and suitable regulatory sequences are known in the art for a variety of host cells.

In general, transcriptional and translational regulatory sequences may include, but are not limited to, promoter sequences, ribosomal binding sites, transcriptional start and stop sequences, translational start and stop sequences, and enhancer or activator sequences. In a preferred embodiment, the regulatory sequences include a promoter and transcriptional start and stop sequences.

Promoter sequences encode either constitutive or inducible promoters. The promoters may be either naturally occurring promoters or hybrid promoters. Hybrid promoters, which combine elements of more than one promoter, are also known in the art, and are useful in the present invention.

In addition, an expression vector may comprise additional elements. For example, the expression vector may have two replication systems, thus allowing it to be maintained in two organisms, e.g. in mammalian or insect cells for expression and in a procaryotic host for cloning and amplification. Furthermore, for integrating expression vectors, the expression vector contains at least one sequence homologous to the host cell genome, and preferably two homologous sequences which flank the expression construct. The integrating vector may be directed to a specific locus in the host cell by selecting the appropriate homologous sequence for inclusion in the vector. Constructs for integrating vectors are well known in the art (e.g., Fernandez & Hoeffler, *supra*)*.*

In addition, in a preferred embodiment, the expression vector contains a selectable marker gene to allow the selection of transformed host cells. Selection genes are well known in the art and will vary with the host cell used.

The prostate cancer proteins of the present invention are produced by culturing a host cell transformed with an expression vector containing nucleic acid encoding a prostate cancer protein, under the appropriate conditions to induce or cause expression of the prostate cancer protein. Conditions appropriate for prostate cancer protein expression will vary with the choice of the expression vector and the host cell, and will be easily ascertained by one skilled in the art through routine experimentation or optimization. For example, the use of constitutive promoters in the expression vector will require optimizing the growth and proliferation of the host cell, while the use of an inducible promoter requires the appropriate growth conditions for induction. In addition, in some embodiments, the timing of the harvest is important. For example, the baculoviral systems used in insect cell expression are lytic viruses, and thus harvest time selection can be crucial for product yield.

Appropriate host cells include yeast, bacteria, archaebacteria, fungi, and insect and animal cells, including mammalian cells. Of particular interest are *Saccharomyces cerevisiae* and other yeasts, *E. coli, Bacillus subtilis,* Sf9 cells, C129 cells, 293 cells, *Neurospora,* BHK, CHO, COS, HeLa cells, HCTVEC (human umbilical vein endothelial cells), THP1 cells (a macrophage cell line) and various other human cells and cell lines.

In a preferred embodiment, the prostate cancer proteins are expressed in mammalian cells. Mammalian expression systems are also known in the art, and include retroviral and adenoviral systems. One expression vector system is a retroviral vector system such as is generally described in PCT/US97/01019 and PCT/US97/01048, both of which are hereby expressly incorporated by reference. Of particular use as mammalian promoters are the promoters from mammalian viral genes, since the viral genes are often highly expressed and have a broad host range. Examples include the SV40 early promoter, mouse mammary tumor virus LTR promoter, adenovirus major late promoter, herpes simplex virus promoter, and the CMV promoter (*see, e.g.,* Fernandez & Hoeffler, *supra*). Typically, transcription termination and polyadenylation sequences recognized by mammalian cells are regulatory regions located 3' to the translation stop codon and thus, together with the promoter elements, flank the coding sequence. Examples of transcription terminator and polyadenlyation signals include those derived form SV40.

The methods of introducing exogenous nucleic acid into mammalian hosts, as well as other hosts, is well known in the art, and will vary with the host cell used. Techniques include dextran-mediated transfection, calcium phosphate precipitation, polybrene mediated transfection, protoplast fusion, electroporation, viral infection, encapsulation of the polynucleotide(s) in liposomes, and direct microinjection of the DNA into nuclei.

In a preferred embodiment, prostate cancer proteins are expressed in bacterial systems. Bacterial expression systems are well known in the art. Promoters from bacteriophage may also be used and are known in the art. In addition, synthetic promoters and hybrid promoters are also useful; e.g., the tac promoter is a hybrid of the trp and lac promoter sequences. Furthermore, a bacterial promoter can include naturally occurring promoters of non-bacterial origin that have the ability to bind bacterial RNA polymerase and initiate transcription. In addition to a functioning promoter sequence, an efficient ribosome binding site is desirable. The expression vector may also include a signal peptide sequence that provides for secretion of the prostate cancer protein in bacteria. The protein is either secreted into the growth media (gram-positive bacteria) or into the periplasmic space, located between the inner and outer membrane of the cell (gram-negative bacteria). The bacterial expression vector may also include a selectable marker gene to allow for the selection of bacterial strains that have been transformed. Suitable selection genes include genes which render the bacteria resistant to drugs such as ampicillin, chloramphenicol, erythromycin, kanamycin, neomycin and tetracycline. Selectable markers also include biosynthetic genes, such as those in the histidine, tryptophan and leucine biosynthetic pathways. These components are assembled into expression vectors. Expression vectors for bacteria are well known in the art, and include vectors for *Bacillus subtilis, E. coli, Streptococcus cremoris,* and *Streptococcus lividans,* among others (e.g., Fernandez & Hoeffler, *supra*). The bacterial expression vectors are transformed into bacterial host cells using techniques well known in the art, such as calcium chloride treatment, electroporation, and others.

In one embodiment, prostate cancer proteins are produced in insect cells. Expression vectors for the transformation of insect cells, and in particular, baculovirus-based expression vectors, are well known in the art.

In a preferred embodiment, prostate cancer protein is produced in yeast cells. Yeast expression systems are well known in the art, and include expression vectors for *Saccharomyces cerevisiae, Candida albicans and C. maltosa, Hansenula polymorpha, Kluyveromyces fragilis* and *K. lactis, Pichia guillerimondii and P. pastoris, Schizosaccharomyces pombe*, and *Yarrowia lipolytica.*

The prostate cancer protein may also be made as a fusion protein, using techniques well known in the art. Thus, e.g., for the creation of monoclonal antibodies, if the desired epitope is small, the prostate cancer protein may be fused to a carrier protein to form an immunogen. Alternatively, the prostate cancer protein may be made as a fusion protein to increase expression, or for other reasons. For example, when the prostate cancer protein is a prostate cancer peptide, the nucleic acid encoding the peptide may be linked to other nucleic acid for expression purposes.

In a preferred embodiment, the prostate cancer protein is purified or isolated after expression. Prostate cancer proteins may be isolated or purified in a variety of ways known to those skilled in the art depending on what other components are present in the sample. Standard purification methods include electrophoretic, molecular, immunological and chromatographic techniques, including ion exchange, hydrophobic, affinity, and reverse-phase HPLC chromatography, and chromatofocusing. For example, the prostate cancer protein may be purified using a standard anti-prostate cancer protein antibody column. Ultrafiltration and diafiltration techniques, in conjunction with protein concentration, are also useful. For general guidance in suitable purification techniques, see Scopes, Protein Purification (1982). The degree of purification necessary will vary depending on the use of the prostate cancer protein. In some instances no purification will be necessary.

Once expressed and purified if necessary, the prostate cancer proteins and nucleic acids are useful in a number of applications. They may be used as immunoselection reagents, as vaccine reagents, as screening agents, etc.

### Variants of prostate cancer proteins

In one embodiment, the prostate cancer proteins are derivative or variant prostate cancer proteins as compared to the wild-type sequence. That is, as outlined more fully below, the derivative prostate cancer peptide will often contain at least one amino acid substitution, deletion or insertion, with amino acid substitutions being particularly preferred. The amino acid substitution, insertion or deletion may occur at any residue within the prostate cancer peptide.

Also included within one embodiment of prostate cancer proteins of the present invention are amino acid sequence variants. These variants typically fall into one or more of three classes: substitutional, insertional or deletional variants. These variants ordinarily are prepared by site specific mutagenesis of nucleotides in the DNA encoding the prostate cancer protein, using cassette or PCR mutagenesis or other techniques well known in the art, to produce DNA encoding the variant, and thereafter expressing the DNA in recombinant cell culture as outlined above. However, variant prostate cancer protein fragments having up to about 100-150 residues may be prepared by in vitro synthesis using established techniques. Amino acid sequence variants are characterized by the predetermined nature of the variation, a feature that sets them apart from naturally occurring allelic or interspecies variation of the prostate cancer protein amino acid sequence. The variants typically exhibit the same qualitative biological activity as the naturally occurring analogue, although variants can also be selected which have modified characteristics as will be more fully outlined below.

While the site or region for introducing an amino acid sequence variation is predetermined, the mutation per se need not be predetermined. For example, in order to optimize the performance of a mutation at a given site, random mutagenesis may be conducted at the target codon or region and the expressed prostate cancer variants screened for the optimal combination of desired activity. Techniques for making substitution mutations at predetermined sites in DNA having a known sequence are well known, e.g., M13 primer mutagenesis and PCR mutagenesis. Screening of the mutants is done using assays of prostate cancer protein activities.

Amino acid substitutions are typically of single residues; insertions usually will be on the order of from about 1 to 20 amino acids, although considerably larger insertions may be tolerated. Deletions range from about 1 to about 20 residues, although in some cases deletions may be much larger.

Substitutions, deletions, insertions or any combination thereof may be used to arrive at a final derivative. Generally these changes are done on a few amino acids to minimize the alteration of the molecule. However, larger changes may be tolerated in certain circumstances. When small alterations in the characteristics of the prostate cancer protein are desired, substitutions are generally made in accordance with the amino acid substitution relationships provided in the definition section.

The variants typically exhibit the same qualitative biological activity and will elicit the same immune response as the naturally-occurring analog, although variants also are selected to modify the characteristics of the prostate cancer proteins as needed. Alternatively, the variant may be designed such that the biological activity of the prostate cancer protein is altered. For example, glycosylation sites may be altered or removed.

Substantial changes in function or immunological identity are made by selecting substitutions that are less conservative than those described above. For example, substitutions may be made which more significantly affect: the structure of the polypeptide backbone in the area of the alteration, for example the alpha-helical or beta-sheet structure; the charge or hydrophobicity of the molecule at the target site; or the bulk of the side chain. The substitutions which in general are expected to produce the greatest changes in the polypeptide's properties are those in which (a) a hydrophilic residue, e.g. seryl or threonyl is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g. lysyl, arginyl, or histidyl, is substituted for (or by) an electronegative residue, e.g. glutamyl or aspartyl; or (d) a residue having a bulky side chain, e.g. phenylalanine, is substituted for (or by) one not having a side chain, e.g. glycine.

Covalent modifications of prostate cancer polypeptides are included within the scope of this invention. One type of covalent modification includes reacting targeted amino acid residues of a prostate cancer polypeptide with an organic derivatizing agent that is capable of reacting with selected side chains or the N-or C-terminal residues of a prostate cancer polypeptide. Derivatization with bifunctional agents is useful, for instance, for crosslinking prostate cancer polypeptides to a water-insoluble support matrix or surface for use in the method for purifying anti-prostate cancer polypeptide antibodies or screening assays, as is more fully described below. Commonly used crosslinking agents include, e.g., 1,1-bis(diazoacetyl)-2-phenylethane, glutaraldehyde, N-hydroxysuccinimide esters, e.g., esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-((p-azidophenyl)dithio)propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl, threonyl or tyrosyl residues, methylation of the amino groups of the lysine, arginine, and histidine side chains (Creighton, Proteins: Structure and Molecular Properties, pp. 79-86 (1983)), acetylation of the N-terminal amine, and amidation of any C-terminal carboxyl group.

Another type of covalent modification of the prostate cancer polypeptide included within the scope of this invention comprises altering the native glycosylation pattern of the polypeptide. "Altering the native glycosylation pattern" is intended for purposes herein to mean deleting one or more carbohydrate moieties found in native sequence prostate cancer polypeptide, and/or adding one or more glycosylation sites that are not present in the native sequence prostate cancer polypeptide. Glycosylation patterns can be altered in many ways. For example the use of different cell types to express prostate cancer-associated sequences can result in different glycosylation patterns.

Addition of glycosylation sites to prostate cancer polypeptides may also be accomplished by altering the amino acid sequence thereof. The alteration may be made, e.g., by the addition of, or substitution by, one or more serine or threonine residues to the native sequence prostate cancer polypeptide (for O-linked glycosylation sites). The prostate cancer amino acid sequence may optionally be altered through changes at the DNA level, particularly by mutating the DNA encoding the prostate cancer polypeptide at preselected bases such that codons are generated that will translate into the desired amino acids.

Another means of increasing the number of carbohydrate moieties on the prostate cancer polypeptide is by chemical or enzymatic coupling of glycosides to the polypeptide. Such methods are described in the art, e.g., in WO 87/05330, and in Aplin & Wriston, CRC Crit. Rev. Biochem., pp. 259-306 (1981).

Removal of carbohydrate moieties present on the prostate cancer polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo-and exo-glycosidases as described by Thotakura et al., Meth. Enzymol., 138:350 (1987).

Another type of covalent modification of prostate cancer comprises linking the prostate cancer polypeptide to one of a variety of nonproteinaceous polymers, e.g., polyethylene glycol, polypropylene glycol, or polyoxyalkylenes, in the manner set forth in U.S. Patent Nos. 4,640,835; 4,496,689; 4,301,144; 4,670,417; 4,791,192 or 4,179,337.

Prostate cancer polypeptides of the present invention may also be modified in a way to form chimeric molecules comprising a prostate cancer polypeptide fused to another, heterologous polypeptide or amino acid sequence. In one embodiment,_such a chimeric molecule comprises a fusion of a prostate cancer polypeptide with a tag polypeptide which provides an epitope to which an anti-tag antibody can selectively bind. The epitope tag is generally placed at the amino-or carboxyl-terminus of the prostate cancer polypeptide. The presence of such epitope-tagged forms of a prostate cancer polypeptide can be detected using an antibody against the tag polypeptide. Also, provision of the epitope tag enables the prostate cancer polypeptide to be readily purified by affinity purification using an anti-tag antibody or another type of affinity matrix that binds to the epitope tag. In an alternative embodiment, the chimeric molecule may comprise a fusion of a prostate cancer polypeptide with an immunoglobulin or a particular region of an immunoglobulin. For a bivalent form of the chimeric molecule, such a fusion could be to the Fc region of an IgG molecule.

Various tag polypeptides and their respective antibodies are well known in the art. Examples include poly-histidine (poly-his) or poly-histidine-glycine (poly-his-gly) tags; HIS6 and metal chelation tags, the flu HA tag polypeptide and its antibody 12CA5 (Field et al., Mol. Cell. Biol. 8:2159-2165 (1988)); the c-myc tag and the 8F9, 3C7, 6E10, G4, B7 and 9E10 antibodies thereto (Evan et al., Molecular and Cellular Biology 5:3610-3616 (1985)); and the Herpes Simplex virus glycoprotein D (gD) tag and its antibody ( Paborsky et al., Protein Engineering 3(6):547-553 (1990)). Other tag polypeptides include the Flag-peptide (Hopp et al., BioTechnology 6:1204-1210 (1988)); the KT3 epitope peptide (Martin et al., Science 255:192-194 (1992)); tubulin epitope peptide (Skinner et al., J. Biol. Chem. 266:15163-15166 (1991)); and the T7 gene 10 protein peptide tag (Lutz-Freyermuth et al., Proc. Natl. Acad. Sci. USA 87:6393-6397 (1990)).

Also included are other prostate cancer proteins of the prostate cancer family, and prostate cancer proteins from other organisms, which are cloned and expressed as outlined below. Thus, probe or degenerate polymerase chain reaction (PCR) primer sequences may be used to find other related prostate cancer proteins from humans or other organisms. As will be appreciated by those in the art, particularly useful probe and/or PCR primer sequences include the unique areas of the prostate cancer nucleic acid sequence. As is generally known in the art, preferred PCR primers are from about 15 to about 35 nucleotides in length, with from about 20 to about 30 being preferred, and may contain inosine as needed. The conditions for the PCR reaction are well known in the art (e.g., Innis, PCR Protocols, *supra*).

### Antibodies to prostate cancer proteins

In a preferred embodiment, when the prostate cancer protein is to be used to generate antibodies, e.g., for immunotherapy or immunodiagnosis, the prostate cancer protein should share at least one epitope or determinant with the full length protein. By "epitope" or "determinant" herein is typically meant a portion of a protein which will generate and/or bind an antibody or T-cell receptor in the context of MHC. Thus, in most instances, antibodies made to a smaller prostate cancer protein will be able to bind to the full-length protein, particularly linear epitopes. In a preferred embodiment; the epitope is unique; that is, antibodies generated to a unique epitope show little or no cross-reactivity.

Methods of preparing polyclonal antibodies are known to the skilled artisan (e.g., Coligan, *supra;* and Harlow & Lane, *supra*). Polyclonal antibodies can be raised in a mammal, e.g., by one or more injections of an immunizing agent and, if desired, an adjuvant. Typically, the immunizing agent and/or adjuvant will be injected in the mammal by multiple subcutaneous or intraperitoneal injections. The immunizing agent may include a protein encoded by a nucleic acid of the figures or fragment thereof or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

The antibodies may, alternatively, be monoclonal antibodies. Monoclonal antibodies may be prepared using hybridoma methods, such as those described by Kohler & Milstein, Nature 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized in vitro. The immunizing agent will typically include a polypeptide encoded by a nucleic acid of Tables 1-16 fragment thereof, or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, Monoclonal Antibodies. Principles and Practice, pp. 59-103 (1986)). Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

In one embodiment, the antibodies are bispecific antibodies. Bispecific antibodies are monoclonal, preferably human or humanized, antibodies that have binding specificities for at least two different antigens or that have binding specificities for two epitopes on the same antigen. In one embodiment, one of the binding specificities is for a protein encoded by a nucleic acid Tables 1-16 or a fragment thereof, the other one is for any other antigen, and preferably for a cell-surface protein or receptor or receptor subunit, preferably one that is tumor specific. Alternatively, tetramer-type technology may create multivalent reagents.

In a preferred embodiment, the antibodies to prostate cancer protein are capable of reducing or eliminating a biological function of a prostate cancer protein, as is described below. That is, the addition of anti-prostate cancer protein antibodies (either polyclonal or preferably monoclonal) to prostate cancer tissue (or cells containing prostate cancer) may reduce or eliminate the prostate cancer. Generally, at least a 25% decrease in activity, growth, size or the like is preferred, with at least about 50% being particularly preferred and about a 95-100% decrease being especially preferred.

In a preferred embodiment the antibodies to the prostate cancer proteins are humanized antibodies (e.g., Xenerex Biosciences, Mederex, Inc., Abgenix, Inc., Protein Design Labs,Inc.) Humanized forms of non-human (e.g., murine) antibodies are chimeric molecules of immunoglobulins, immunoglobulin chains or fragments thereof (such as Fv, Fab, Fab', F(ab')2 or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, a humanized antibody will comprise substantially all of at least one, and typically two, variable domains, in which all or substantially all of the CDR regions correspond to those of a non-human immunoglobulin and all or substantially all of the framework (FR) regions are those of a human immunoglobulin consensus sequence. The humanized antibody optimally also will comprise at least a portion of an immunoglobulin constant region (Fc), typically that of a human immunoglobulin (Jones et al., Nature 321:522-525 (1986); Riechmann et al., Nature 332:323-329 (1988); and Presta, Curr. Op. Struct. Biol. 2:593-596 (1992)). Humanization can be essentially performed following the method of Winter and co-workers (Jones et al., Nature 321:522-525 (1986); Riechmann et al., Nature 332:323-327 (1988); Verhoeyen et al., Science 239:1534-1536 (1988)), by substituting rodent CDRs or CDR sequences for the corresponding sequences of a human antibody. Accordingly, such humanized antibodies are chimeric antibodies (U.S. Patent No. 4,816,567), wherein substantially less than an intact human variable domain has been substituted by the corresponding sequence from a non-human species.

Human antibodies can also be produced using various techniques known in the art, including phage display libraries (Hoogenboom & Winter, J. Mol. Biol. 227:381 (1991); Marks et al., J. Mol. Biol. 222:581 (1991)). The techniques of Cole *et al.* and Boerner *et al.* are also available for the preparation of human monoclonal antibodies (Cole et al., Monoclonal Antibodies and Cancer Therapy, p. 77 (1985) and Boerner et al., J. Immunol. 147(1):86-95 (1991)). Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, e.g., in U.S. Patent Nos. 5,545,807; 5,545,806; 5,569,825; 5,625,126; 5,633,425; 5,661,016, and in the following scientific publications: Marks et al., Bio/Technology 10:779-783 (1992); Lonberg et al., Nature 368:856-859 (1994); Morrison, Nature 368:812-13 (1994); Fishwild et al., Nature Biotechnology 14:845-51 (1996); Neuberger, Nature Biotechnology 14:826 (1996); Lonberg & Huszar, Intern. Rev. Immunol. 13:65-93 (1995).

By immunotherapy is meant treatment of prostate cancer with an antibody raised against prostate cancer proteins. As used herein, immunotherapy can be passive or active. Passive immunotherapy as defined herein is the passive transfer of antibody to a recipient (patient). Active immunization is the induction of antibody and/or T-cell responses in a recipient (patient). Induction of an immune response is the result of providing the recipient with an antigen to which antibodies are raised. As appreciated by one of ordinary skill in the art, the antigen may be provided by injecting a polypeptide against which antibodies are desired to be raised into a recipient, or contacting the recipient with a nucleic acid capable of expressing the antigen and under conditions for expression of the antigen, leading to an immune response.

In a preferred embodiment the prostate cancer proteins against which antibodies are raised are secreted proteins as described above. Without being bound by theory, antibodies used for treatment, bind and prevent the secreted protein from binding to its receptor, thereby inactivating the secreted prostate cancer protein.

In another preferred embodiment, the prostate cancer protein to which antibodies are raised is a transmembrane protein. Without being bound by theory, antibodies used for treatment, bind the extracellular domain of the prostate cancer protein and prevent it from binding to other proteins, such as circulating ligands or cell-associated molecules. The antibody may cause down-regulation of the transmembrane prostate cancer protein. As will be appreciated by one of ordinary skill in the art, the antibody may be a competitive, non-competitive or uncompetitive inhibitor of protein binding to the extracellular domain of the prostate cancer protein. The antibody is also an antagonist of the prostate cancer protein. Further, the antibody prevents activation of the transmembrane prostate cancer protein. In one aspect, when the antibody prevents the binding of other molecules to the prostate cancer protein, the antibody prevents growth of the cell. The antibody may also be used to target or sensitize the cell to cytotoxic agents, including, but not limited to TNF-α, TNF-β, IL-1, INF-γ and IL-2, or chemotherapeutic agents including 5FU, vinblastine, actinomycin D, cisplatin, methotrexate, and the like. In some instances the antibody belongs to a sub-type that activates serum complement when complexed with the transmembrane protein thereby mediating cytotoxicity or antigen-dependent cytotoxicity (ADCC). Thus, prostate cancer is treated by administering to a patient antibodies directed against the transmembrane prostate cancer protein. Antibody-labeling may activate a co-toxin, localize a toxin payload, or otherwise provide means to locally ablate cells.

In another preferred embodiment, the antibody is conjugated to an effector moiety. The effector moiety can be any number of molecules, including labelling moieties such as radioactive labels or fluorescent labels, or can be a therapeutic moiety. In one aspect the therapeutic moiety is a small molecule that modulates the activity of the prostate cancer protein. In another aspect the therapeutic moiety modulates the activity of molecules associated with or in close proximity to the prostate cancer protein. The therapeutic moiety may inhibit enzymatic activity such as protease or collagenase or protein kinase activity associated with prostate cancer.

In a preferred embodiment, the therapeutic moiety can also be a cytotoxic agent. In this method, targeting the cytotoxic agent to prostate cancer tissue or cells, results in a reduction in the number of afflicted cells, thereby reducing symptoms associated with prostate cancer. Cytotoxic agents are numerous and varied and include, but are not limited to, cytotoxic drugs or toxins or active fragments of such toxins. Suitable toxins and their corresponding fragments include diphtheria A chain, exotoxin A chain, ricin A chain, abrin A chain, curcin, crotin, phenomycin, enomycin and the like. Cytotoxic agents also include radiochemicals made by conjugating radioisotopes to antibodies raised against prostate cancer proteins, or binding of a radionuclide to a chelating agent that has been covalently attached to the antibody. Targeting the therapeutic moiety to transmembrane prostate cancer proteins not only serves to increase the local concentration of therapeutic moiety in the prostate cancer afflicted area, but also serves to reduce deleterious side effects that may be associated with the therapeutic moiety.

In another preferred embodiment, the prostate cancer protein against which the antibodies are raised is an intracellular protein. In this case, the antibody may be conjugated to a protein which facilitates entry into the cell. In one case, the antibody enters the cell by endocytosis. In another embodiment, a nucleic acid encoding the antibody is administered to the individual or cell. Moreover, wherein the prostate cancer protein can be targeted within a cell, i.e., the nucleus, an antibody thereto contains a signal for that target localization, i.e., a nuclear localization signal.

The prostate cancer antibodies of the invention specifically bind to prostate cancer proteins. By "specifically bind" herein is meant that the antibodies bind to the protein with a K_{d} of at least about 0.1 mM, more usually at least about 1 µM, preferably at least about 0.1 µM or better, and most preferably, 0.01 µM or better. Selectivity of binding is also important.

### Detection of prostate cancer sequence for diagnostic and therapeutic applications

In one aspect, the RNA expression levels of genes are determined for different cellular states in the prostate cancer phenotype. Expression levels of genes in normal tissue (i.e., not undergoing prostate cancer) and in prostate cancer tissue (and in some cases, for varying severities of prostate cancer that relate to prognosis, as outlined below) are evaluated to provide expression profiles. An expression profile of a particular cell state or point of development is essentially a "fingerprint" of the state. While two states may have any particular gene similarly expressed, the evaluation of a number of genes simultaneously allows the generation of a gene expression profile that is reflective of the state of the cell. By comparing expression profiles of cells in different states, information regarding which genes are important (including both up- and down-regulation of genes) in each of these states is obtained. Then, diagnosis may be performed or confirmed to determine whether a tissue sample has the gene expression profile of normal or cancerous tissue. This will provide for molecular diagnosis of related conditions.

"Differential expression," or grammatical equivalents as used herein, refers to qualitative or quantitative differences in the temporal and/or cellular gene expression patterns within and among cells and tissue. Thus, a differentially expressed gene can qualitatively have its expression altered, including an activation or inactivation, in, e.g., normal versus prostate cancer tissue. Genes may be turned on or turned off in a particular state, relative to another state thus permitting comparison of two or more states. A qualitatively regulated gene will exhibit an expression pattern within a state or cell type which is detectable by standard techniques. Some genes will be expressed in one state or cell type, but not in both. Alternatively, the difference in expression may be quantitative, e.g., in that expression is increased or decreased; i.e., gene expression is either upregulated, resulting in an increased amount of transcript, or downregulated, resulting in a decreased amount of transcript. The degree to which expression differs need only be large enough to quantify via standard characterization techniques as outlined below, such as by use of Affymetrix GeneChip^{™} expression arrays, Lockhart, Nature Biotechnology 14:1675-1680 (1996), hereby expressly incorporated by reference. Other techniques include, but are not limited to, quantitative reverse transcriptase PCR, northern analysis and RNase protection. As outlined above, preferably the change in expression (i.e., upregulation or downregulation) is at least about 50%, more preferably at least about 100%, more preferably at least about 150%, more preferably at least about 200%, with from 300 to at least 1000% being especially preferred.

Evaluation may be at the gene transcript, or the protein level. The amount of gene expression may be monitored using nucleic acid probes to the DNA or RNA equivalent of the gene transcript, and the quantification of gene expression levels, or, alternatively, the final gene product itself (protein) can be monitored, e.g., with antibodies to the prostate cancer protein and standard immunoassays (ELISAs, etc.) or other techniques, including mass spectroscopy assays, 2D gel electrophoresis assays, etc. Proteins corresponding to prostate cancer genes, i.e., those identified as being important in a prostate cancer phenotype, can be evaluated in a prostate cancer diagnostic test.

In a preferred embodiment, gene expression monitoring is performed simultaneously on a number of genes. Multiple protein expression monitoring can be performed as well. Similarly, these assays may be performed on an individual basis as well.

In this embodiment, the prostate cancer nucleic acid probes are attached to biochips as outlined herein for the detection and quantification of prostate cancer sequences in a particular cell. The assays are further described below in the example. PCR techniques can be used to provide greater sensitivity.

In a preferred embodiment nucleic acids encoding the prostate cancer protein are detected. Although DNA or RNA encoding the prostate cancer protein may be detected, of particular interest are methods wherein an mRNA encoding a prostate cancer protein is detected. Probes to detect mRNA can be a nucleotide/deoxynucleotide probe that is complementary to and hybridizes with the mRNA and includes, but is not limited to, oligonucleotides, cDNA or RNA. Probes also should contain a detectable label, as defined herein. In one method the mRNA is detected after immobilizing the nucleic acid to be examined on a solid support such as nylon membranes and hybridizing the probe with the sample. Following washing to remove the non-specifically bound probe, the label is detected. In another method detection of the mRNA is performed in situ. In this method permeabilized cells or tissue samples are contacted with a detectably labeled nucleic acid probe for sufficient time to allow the probe to hybridize with the target mRNA. Following washing to remove the non-specifically bound probe, the label is detected. For example a digoxygenin labeled riboprobe (RNA probe) that is complementary to the mRNA encoding a prostate cancer protein is detected by binding the digoxygenin with an anti-digoxygenin secondary antibody and developed with nitro blue tetrazolium and 5-bromo-4-chloro-3-indoyl phosphate.

In a preferred embodiment, various proteins from the three classes of proteins as described herein (secreted, transmembrane or intracellular proteins) are used in diagnostic assays. The prostate cancer proteins, antibodies, nucleic acids, modified proteins and cells containing prostate cancer sequences are used in diagnostic assays. This can be performed on an individual gene or corresponding polypeptide level. In a preferred embodiment, the expression profiles are used, preferably in conjunction with high throughput screening techniques to allow monitoring for expression profile genes and/or corresponding polypeptides.

As described and defined herein, prostate cancer proteins, including intracellular, transmembrane or secreted proteins, find use as markers of prostate cancer. Detection of these proteins in putative prostate cancer tissue allows for detection or diagnosis of prostate cancer. In one embodiment, antibodies are used to detect prostate cancer proteins. A preferred method separates proteins from a sample by electrophoresis on a gel (typically a denaturing and reducing protein gel, but may be another type of gel, including isoelectric focusing gels and the like). Following separation of proteins, the prostate cancer protein is detected, e.g., by immunoblotting with antibodies raised against the prostate cancer protein. Methods of immunoblotting are well known to those of ordinary skill in the art.

In another preferred method, antibodies to the prostate cancer protein find use in *in situ* imaging techniques, e.g., in histology (e.g., Methods in Cell Biology: Antibodies in Cell Biology, volume 37 (Asai, ed. 1993)). In this method cells are contacted with from one to many antibodies to the prostate cancer protein(s). Following washing to remove non-specific antibody binding, the presence of the antibody or antibodies is detected. In one embodiment the antibody is detected by incubating with a secondary antibody that contains a detectable label. In another method the primary antibody to the prostate cancer protein(s) contains a detectable label, e.g. an enzyme marker that can act on a substrate. In another preferred embodiment each one of multiple primary antibodies contains a distinct and detectable label. This method finds particular use in simultaneous screening for a plurality of prostate cancer proteins. As will be appreciated by one of ordinary skill in the art, many other histological imaging techniques are also provided by the invention.

In a preferred embodiment the label is detected in a fluorometer which has the ability to detect and distinguish emissions of different wavelengths. In addition, a fluorescence activated cell sorter (FACS) can be used in the method.

In another preferred embodiment, antibodies find use in diagnosing prostate cancer from blood, serum, plasma, stool, and other samples. Such samples, therefore, are useful as samples to be probed or tested for the presence of prostate cancer proteins. Antibodies can be used to detect a prostate cancer protein by previously described immunoassay techniques including ELISA, immunoblotting (western blotting), immunoprecipitation, BIACORE technology and the like. Conversely, the presence of antibodies may indicate an immune response against an endogenous prostate cancer protein.

In a preferred embodiment, *in situ* hybridization of labeled prostate cancer nucleic acid probes to tissue arrays is done. For example, arrays of tissue samples, including prostate cancer tissue and/or normal tissue, are made. *In situ* hybridization (*see, e.g.,* Ausubel, *supra*) is then performed. When comparing the fingerprints between an individual and a standard, the skilled artisan can_make a diagnosis, a prognosis, or a prediction based on the findings. It is further understood that the genes which indicate the diagnosis may differ from those which indicate the prognosis and molecular profiling of the condition of the cells may lead to distinctions between responsive or refractory conditions or may be predictive of outcomes.

In a preferred embodiment, the prostate cancer proteins, antibodies, nucleic acids, modified proteins and cells containing prostate cancer sequences are used in prognosis assays. As above, gene expression profiles can be generated that correlate to prostate cancer, in terms of long term prognosis. Again, this may be done on either a protein or gene level, with the use of genes being preferred. As above, prostate cancer probes may be attached to biochips for the detection and quantification of prostate cancer sequences in a tissue or patient. The assays proceed as outlined above for diagnosis. PCR method may provide more sensitive and accurate quantification.

### Assays for therapeutic compounds

In a preferred embodiment members of the proteins, nucleic acids, and antibodies as described herein are used in drug screening assays. The prostate cancer proteins, antibodies, nucleic acids, modified proteins and cells containing prostate cancer sequences are used in drug screening assays or by evaluating the effect of drug candidates on a "gene expression profile" or expression profile of polypeptides. In a preferred embodiment, the expression profiles are used, preferably in conjunction with high throughput screening techniques to allow monitoring for expression profile genes after treatment with a candidate agent (e.g., Zlokarnik, et al., Science 279:84-8 (1998); Heid, Genome Res 6:986-94, 1996).

In a preferred embodiment, the prostate cancer proteins, antibodies, nucleic acids, modified proteins and cells containing the native or modified prostate cancer proteins are used in screening assays. That is, the present invention provides novel methods for screening for compositions which modulate the prostate cancer phenotype or an identified physiological function of a prostate cancer protein. As above, this can be done on an individual gene level or by evaluating the effect of drug candidates on a "gene expression profile". In a preferred embodiment, the expression profiles are used, preferably in conjunction with high throughput screening techniques to allow monitoring for expression profile genes after treatment with a candidate agent, see Zlokarnik, *supra.*

Having identified the differentially expressed genes herein, a variety of assays may be executed. In a preferred embodiment, assays may be run on an individual gene or protein level. That is, having identified a particular gene as up regulated in prostate cancer, test compounds can be screened for the ability to modulate gene expression or for binding to the prostate cancer protein. "Modulation" thus includes both an increase and a decrease in gene expression. The preferred amount of modulation will depend on the original change of the gene expression in normal versus tissue undergoing prostate cancer, with changes of at least 10%, preferably 50%, more preferably 100-300%, and in some embodiments 300-1000% or greater. Thus, if a gene exhibits a 4-fold increase in prostate cancer tissue compared to normal tissue, a decrease of about four-fold is often desired; similarly, a 10-fold decrease in prostate cancer tissue compared to normal tissue often provides a target value of a 10-fold increase in expression to be induced by the test compound.

The amount of gene expression may be monitored using nucleic acid probes and the quantification of gene expression levels, or, alternatively, the gene product itself can be monitored, e.g., through the use of antibodies to the prostate cancer protein and standard immunoassays. Proteomics and separation techniques may also allow quantification of expression.

In a preferred embodiment, gene expression or protein monitoring of a number of entities, i.e., an expression profile, is monitored simultaneously. Such profiles will typically involve a plurality of those entities described herein..

In this embodiment, the prostate cancer nucleic acid probes are attached to biochips as outlined herein for the detection and quantification of prostate cancer sequences in a particular cell. Alternatively, PCR may be used. Thus, a series, e.g., of microtiter plate, may be used with dispensed primers in desired wells. A PCR reaction can then be performed and analyzed for each well.

Expression monitoring can be performed to identify compounds that modify the expression of one or more prostate cancer-associated sequences, e.g., a polynucleotide sequence set out in Tables 1-16. Generally, in a preferred embodiment, a test modulator is added to the cells prior to analysis. Moreover, screens are also provided to identify agents that modulate prostate cancer, modulate prostate cancer proteins, bind to a prostate cancer protein, or interfere with the binding of a prostate cancer protein and an antibody or other binding partner.

The term "test compound" or "drug candidate" or "modulator" or grammatical equivalents as used herein describes any molecule, e.g., protein, oligopeptide, small organic molecule, polysaccharide, polynucleotide, etc., to be tested for the capacity to directly or indirectly alter the prostate cancer phenotype or the expression of a prostate cancer sequence, e.g., a nucleic acid or protein sequence. In preferred embodiments, modulators alter expression profiles, or expression profile nucleic acids or proteins provided herein. In one embodiment, the modulator suppresses a prostate cancer phenotype, e.g. to a normal tissue fingerprint. In another embodiment, a modulator induced a prostate cancer phenotype. Generally, a plurality of assay mixtures are run in parallel with different agent concentrations to obtain a differential response to the various concentrations. Typically, one of these concentrations serves as a negative control, i.e., at zero concentration or below the level of detection.

Drug candidates encompass numerous chemical classes, though typically they are organic molecules, preferably small organic compounds having a molecular weight of more than 100 and less than about 2,500 daltons. Preferred small molecules are less than 2000, or less than 1500 or less than 1000 or less than 500 D. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof. Particularly preferred are peptides.

In one aspect, a modulator will neutralize the effect of a prostate cancer protein. By "neutralize" is meant that activity of a protein is inhibited or blocked and the consequent effect on the cell.

In certain embodiments, combinatorial libraries of potential modulators will be screened for an ability to bind to a prostate cancer polypeptide or to modulate activity. Conventionally, new chemical entities with useful properties are generated by identifying a chemical compound (called a "lead compound") with some desirable property or activity, e.g., inhibiting activity, creating variants of the lead compound, and evaluating the property and activity of those variant compounds. Often, high throughput screening (HTS) methods are employed for such an analysis.

In one preferred embodiment, high throughput screening methods involve providing a library containing a large number of potential therapeutic compounds (candidate compounds). Such "combinatorial chemical libraries" are then screened in one or more assays to identify those library members (particular chemical species or subclasses) that display a desired characteristic activity. The compounds thus identified can serve as conventional "lead compounds" or can themselves be used as potential or actual therapeutics.

A combinatorial chemical library is a collection of diverse chemical compounds generated by either chemical synthesis or biological synthesis by combining a number of chemical "building blocks" such as reagents. For example, a linear combinatorial chemical library, such as a polypeptide (e.g., mutein) library, is formed by combining a set of chemical building blocks called amino acids in every possible way for a given compound length (i.e., the number of amino acids in a polypeptide compound). Millions of chemical compounds can be synthesized through such combinatorial mixing of chemical building blocks (Gallop et al., J. Med. Chem. 37(9):1233-1251 (1994)).

Preparation and screening of combinatorial chemical libraries is well known to those of skill in the art. Such combinatorial chemical libraries include, but are not limited to, peptide libraries (*see, e.g.,* U.S. Patent No. 5,010,175, Furka, Pept. Prot. Res. 37:487-493 (1991), Houghton et al., Nature, 354:84-88 (1991)), peptoids (PCT Publication No WO 91/19735), encoded peptides (PCT Publication WO 93/20242), random bio-oligomers (PCT Publication WO 92/00091), benzodiazepines (U.S. Pat. No. 5,288,514), diversomers such as hydantoins, benzodiazepines and dipeptides (Hobbs et al., Proc. Nat. Acad. Sci. USA 90:6909-6913 (1993)), vinylogous polypeptides (Hagihara et al., J. Amer. Chem. Soc. 114:6568 (1992)), nonpeptidal peptidomimetics with a Beta-D-Glucose scaffolding (Hirschmann et al., J. Amer. Chem. Soc. 114:9217-9218 (1992)), analogous organic syntheses of small compound libraries (Chen et al., J. Amer. Chem. Soc. 116:2661 (1994)), oligocarbamates (Cho, et al., Science 261:1303 (1993)), and/or peptidyl phosphonates (Campbell et al., J. Org. Chem. 59:658 (1994)). *See, generally,* Gordon et al., J. Med. Chem. 37:1385 (1994), nucleic acid libraries (*see, e.g.,* Strategene, Corp.), peptide nucleic acid libraries (*see, e.g.,* U.S. Patent 5,539,083), antibody libraries (*see, e.g.,* Vaughn et al., Nature Biotechnology 14(3):309-314 (1996), and PCT/CTS96/10287), carbohydrate libraries (*see, e.g.,* Liang et al., Science 274:1520-1522 (1996), and U.S. Patent No. 5,593,853), and small organic molecule libraries (*see, e.g.,* benzodiazepines, Baum, C&EN, Jan 18, page 33 (1993); isoprenoids, U.S. Patent No. 5,569,588; thiazolidinones and metathiazanones, U.S. Patent No. 5,549,974; pyrrolidines, U.S. Patent Nos. 5,525,735 and 5,519,134; morpholino compounds, U.S. Patent No. 5,506,337; benzodiazepines, U.S. Patent No. 5,288,514; and the like).

Devices for the preparation of combinatorial libraries are commercially available (*see, e.g.,* 357 MPS, 390 MPS, Advanced Chem Tech, Louisville KY, Symphony, Rainin, Woburn, MA, 433A Applied Biosystems, Foster City, CA, 9050 Plus, Millipore, Bedford, MA).

A number of well known robotic systems have also been developed for solution phase chemistries. These systems include automated workstations like the automated synthesis apparatus developed by Takeda Chemical Industries, LTD. (Osaka, Japan) and many robotic systems utilizing robotic arms (Zymate II, Zymark Corporation, Hopkinton, Mass.; Orca, Hewlett-Packard, Palo Alto, Calif.), which mimic the manual synthetic operations performed by a chemist. Any of the above devices are suitable for use with the present invention. The nature and implementation of modifications to these devices (if any) so that they can operate as discussed herein will be apparent to persons skilled in the relevant art. In addition, numerous combinatorial libraries are themselves commercially available *(see, e.g.,* ComGenex, Princeton, N.J., Asinex, Moscow, Ru, Tripos, Inc., St. Louis, MO, ChemStar, Ltd, Moscow, RU, 3D Pharmaceuticals, Exton, PA, Martek Biosciences, Columbia, MD, *etc.*).

The assays to identify modulators are amenable to high throughput screening. Preferred assays thus detect enhancement or inhibition of prostate cancer gene transcription, inhibition or enhancement of polypeptide expression, and inhibition or enhancement of polypeptide activity.

High throughput assays for the presence, absence, quantification, or other properties of particular nucleic acids or protein products are well known to those of skill in the art. Similarly, binding assays and reporter gene assays are similarly well known. Thus, e.g., U.S. Patent No. 5,559,410 discloses high throughput screening methods for proteins, U.S. Patent No. 5,585,639 discloses high throughput screening methods for nucleic acid binding (i.e., in arrays), while U.S. Patent Nos. 5,576,220 and 5,541,061 disclose high throughput methods of screening for ligand/antibody binding.

In addition, high throughput screening systems are commercially available (*see, e.g.,* Zymark Corp., Hopkinton, MA; Air Technical Industries, Mentor, OH; Beckman Instruments, Inc. Fullerton, CA; Precision Systems, Inc., Natick, MA, *etc.*). These systems typically automate entire procedures, including all sample and reagent pipetting, liquid dispensing, timed incubations, and final readings of the microplate in detector(s) appropriate for the assay. These configurable systems provide high throughput and rapid start up as well as a high degree of flexibility and customization. The manufacturers of such systems provide detailed protocols for various high throughput systems. Thus, e.g., Zymark Corp. provides technical bulletins describing screening systems for detecting the modulation of gene transcription, ligand binding, and the like.

In one embodiment, modulators are proteins, often naturally occurring proteins or fragments of naturally occurring proteins. Thus, *e.g.*, cellular extracts containing proteins, or random or directed digests of proteinaceous cellular extracts, may be used. In this way libraries of proteins may be made for screening in the methods of the invention. Particularly preferred in this embodiment are libraries of bacterial, fungal, viral, and mammalian proteins, with the latter being preferred, and human proteins being especially preferred. Particularly useful test compound will be directed to the class of proteins to which the target belongs, *e.g.*, substrates for enzymes or ligands and receptors.

In a preferred embodiment, modulators are peptides of from about 5 to about 30 amino acids, with from about 5 to about 20 amino acids being preferred, and from about 7 to about 15 being particularly preferred. The peptides may be digests of naturally occurring proteins as is outlined above, random peptides, or "biased" random peptides. By "randomized" or grammatical equivalents herein is meant that each nucleic acid and peptide consists of essentially random nucleotides and amino acids, respectively. Since generally these random peptides (or nucleic acids, discussed below) are chemically synthesized, they may incorporate any nucleotide or amino acid at any position. The synthetic process can be designed to generate randomized proteins or nucleic acids, to allow the formation of all or most of the possible combinations over the length of the sequence, thus forming a library of randomized candidate bioactive proteinaceous agents.

In one embodiment, the library is fully randomized, with no sequence preferences or constants at any position. In a preferred embodiment, the library is biased. That is, some positions within the sequence are either held constant, or are selected from a limited number of possibilities. For example, in a preferred embodiment, the nucleotides or amino acid residues are randomized within a defined class, e.g., of hydrophobic amino acids, hydrophilic residues, sterically biased (either small or large) residues, towards the creation of nucleic acid binding domains, the creation of cysteines, for cross-linking, prolines for SH-3 domains, serines, threonines, tyrosines or histidines for phosphorylation sites, etc., or to purines, etc.

Modulators of prostate cancer can also be nucleic acids, as defined below. As described above generally for proteins, nucleic acid modulating agents may be naturally occurring nucleic acids, random nucleic acids, or "biased" random nucleic acids. For example, digests of procaryotic or eucaryotic genomes may be used as is outlined above for proteins.

In certain embodiments, the activity of a prostate cancer-associated protein is down-regulated, or entirely inhibited, by the use of antisense polynucleotide, *i.e.*, a nucleic acid complementary to, and which can preferably hybridize specifically to, a coding mRNA nucleic acid sequence, e.g., a prostate cancer protein mRNA, or a subsequence thereof. Binding of the antisense polynucleotide to the mRNA reduces the translation and/or stability of the mRNA.

In the context of this invention, antisense polynucleotides can comprise naturally-occurring nucleotides, or synthetic species formed from naturally-occurring subunits or their close homologs. Antisense polynucleotides may also have altered sugar moieties or inter-sugar linkages. Exemplary among these are the phosphorothioate and other sulfur containing species which are known for use in the art. Analogs are comprehended by this invention so long as they function effectively to hybridize with the prostate cancer protein mRNA. *See, e.g.,* Isis Pharmaceuticals, Carlsbad, CA; Sequitor, Inc., Natick, MA.

Such antisense polynucleotides can readily be synthesized using recombinant means, or can be synthesized *in vitro.* Equipment for such synthesis is sold by several vendors, including Applied Biosystems. The preparation of other oligonucleotides such as phosphorothioates and alkylated derivatives is also well known to those of skill in the art.

Antisense molecules as used herein include antisense or sense oligonucleotides. Sense oligonucleotides can, e.g., be employed to block transcription by binding to the anti-sense strand. The antisense and sense oligonucleotide comprise a single-stranded nucleic acid sequence (either RNA or DNA) capable of binding to target mRNA (sense) or DNA (antisense) sequences for prostate cancer molecules. Antisense or sense oligonucleotides, according to the present invention, comprise a fragment generally at least about 14 nucleotides, preferably from about 14 to 30 nucleotides. The ability to derive an antisense or a sense oligonucleotide, based upon a cDNA sequence encoding a given protein is described in, e.g., Stein & Cohen (Cancer Res. 48:2659 (1988 and van der Krol et al. (BioTechniques 6:958 (1988)).

In addition to antisense polynucleotides, ribozymes can be used to target and inhibit transcription of prostate cancer-associated nucleotide sequences. A ribozyme is an RNA molecule that catalytically cleaves other RNA molecules. Different kinds of ribozymes have been described, including group I ribozymes, hammerhead ribozymes, hairpin ribozymes, RNase P, and axhead ribozymes (*see, e.g.,* Castanotto et al., Adv. in Pharmacology 25: 289-317 (1994) for a general review of the properties of different ribozymes).

The general features of hairpin ribozymes are described, e.g., in Hampel et al., Nucl. Acids Res. 18:299-304 (1990); European Patent Publication No. 0 360 257; U.S. Patent No. 5,254,678. Methods of preparing are well known to those of skill in the art (*see, e.g.,* WO 94/26877; Ojwang et al., Proc. Natl. Acad Sci. USA 90:6340-6344 (1993); Yamada et al., Human Gene Therapy 1:39-45 (1994); Leavitt et al., Proc. Natl. Acad. Sci. USA 92:699-703 (1995); Leavitt et al., Human Gene Therapy 5:1151-120 (1994); and Yamada et al., Virology 205: 121-126 (1994)).

Polynucleotide modulators of prostate cancer may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell. Alternatively, a polynucleotide modulator of prostate cancer may be introduced into a cell containing the target nucleic acid sequence, e.g., by formation of an polynucleotide-lipid complex, as described in WO 90/10448. It is understood that the use of antisense molecules or knock out and knock in models may also be used in screening assays as discussed above, in addition to methods of treatment.

As noted above, gene expression monitoring is conveniently used to test candidate modultors (*e.g.*, protein, nucleic acid or small molecule). After the candidate agent has been added and the cells allowed to incubate for some period of time, the sample containing a target sequence to be analyzed is added to the biochip. If required, the target sequence is prepared using known techniques. For example, the sample may be treated to lyse the cells, using known lysis buffers, electroporation, etc., with purification and/or amplification such as PCR performed as appropriate. For example, an *in vitro* transcription with labels covalently attached to the nucleotides is performed. Generally, the nucleic acids are labeled with biotin-FITC or PE, or with cy3 or cy5.

In a preferred embodiment, the target sequence is labeled with, e.g., a fluorescent, a chemiluminescent, a chemical, or a radioactive signal, to provide a means of detecting the target sequence's specific binding to a probe. The label also can be an enzyme, such as, alkaline phosphatase or horseradish peroxidase, which when provided with an appropriate substrate produces a product that can be detected. Alternatively, the label can be a labeled compound or small molecule, such as an enzyme inhibitor, that binds but is not catalyzed or altered by the enzyme. The label also can be a moiety or compound, such as, an epitope tag or biotin which specifically binds to streptavidin. For the example of biotin, the streptavidin is labeled as described above, thereby, providing a detectable signal for the bound target sequence. Unbound labeled streptavidin is typically removed prior to analysis.

As will be appreciated by those in the art, these assays can be direct hybridization assays or can comprise "sandwich assays", which include the use of multiple probes, as is generally outlined in U.S. Patent Nos. 5,681,702, 5,597,909, 5,545,730, 5,594,117,5,591,584,5,571,670,5,580,731, 5,571,670,5,591,584,5,624,802,5,635,352, 5,594,118, 5,359,100, 5,124,246 and 5,681,697, all of which are hereby incorporated by reference. In this embodiment, in general, the target nucleic acid is prepared as outlined above, and then added to the biochip comprising a plurality of nucleic acid probes, under conditions that allow the formation of a hybridization complex.

A variety of hybridization conditions may be used in the present invention, including high, moderate and low stringency conditions as outlined above. The assays are generally run under stringency conditions which allows formation of the label probe hybridization complex only in the presence of target. Stringency can be controlled by altering a step parameter that is a thermodynamic variable, including, but not limited to, temperature, formamide concentration, salt concentration, chaotropic salt concentration pH, organic solvent concentration, etc.

These parameters may also be used to control non-specific binding, as is generally outlined in U.S. Patent No. 5,681,697. Thus it may be desirable to perform certain steps at higher stringency conditions to reduce non-specific binding.

The reactions outlined herein may be accomplished in a variety of ways. Components of the reaction may be added simultaneously, or sequentially, in different orders, with preferred embodiments outlined below. In addition, the reaction may include a variety of other reagents. These include salts, buffers, neutral proteins, e.g. albumin, detergents, etc. which may be used to facilitate optimal hybridization and detection, and/or reduce non-specific or background interactions. Reagents that otherwise improve the efficiency of the assay, such as protease inhibitors, nuclease inhibitors, anti-microbial agents, *etc.,* may also be used as appropriate, depending on the sample preparation methods and purity of the target.

The assay data are analyzed to determine the expression levels, and changes in expression levels as between states, of individual genes, forming a gene expression profile.

Screens are performed to identify modulators of the prostate cancer phenotype. In one embodiment, screening is performed to identify modulators that can induce or suppress a particular expression profile, thus preferably generating the associated phenotype. In another embodiment, *e.g.*, for diagnostic applications, having identified differentially expressed genes important in a particular state, screens can be performed to identify modulators that alter expression of individual genes. In an another embodiment, screening is performed to identify modulators that alter a biological function of the expression product of a differentially expressed gene. Again, having identified the importance of a gene in a particular state, screens are performed to identify agents that bind and/or modulate the biological activity of the gene product.

In addition screens can be done for genes that are induced in response to a candidate agent. After identifying a modulator based upon its ability to suppress a prostate cancer expression pattern leading to a normal expression pattern, or to modulate a single prostate cancer gene expression profile so as to mimic the expression of the gene from normal tissue, a screen as described above can be performed to identify genes that are specifically modulated in response to the agent. Comparing expression profiles between normal tissue and agent treated prostate cancer tissue reveals genes that are not expressed in normal tissue or prostate cancer tissue, but are expressed in agent treated tissue. These agent-specific sequences can be identified and used by methods described herein for prostate cancer genes or proteins. In particular these sequences and the proteins they encode find use in marking or identifying agent treated cells. In addition, antibodies can be raised against the agent induced proteins and used to target novel therapeutics to the treated prostate cancer tissue sample.

Thus, in one embodiment, a test compound is administered to a population of prostate cancer cells, that have an associated prostate cancer expression profile. By "administration" or "contacting" herein is meant that the candidate agent is added to the cells in such a manner as to allow the agent to act upon the cell, whether by uptake and intracellular action, or by action at the cell surface. In some embodiments, nucleic acid encoding a proteinaceous candidate agent (i.e., a peptide) may be put into a viral construct such as an adenoviral or retroviral construct, and added to the cell, such that expression of the peptide agent is accomplished, e.g., PCT US97/01019. Regulatable gene therapy systems can also be used.

Once the test compound has been administered to the cells, the cells can be washed if desired and are allowed to incubate under preferably physiological conditions for some period of time. The cells are then harvested and a new gene expression profile is generated, as outlined herein.

Thus, e.g., prostate cancer tissue may be screened for agents that modulate, e.g., induce or suppress the prostate cancer phenotype. A change in at least one gene, preferably many, of the expression profile indicates that the agent has an effect on prostate cancer activity. By defining such a signature for the prostate cancer phenotype, screens for new drugs that alter the phenotype can be devised. With this approach, the drug target need not be known and need not be represented in the original expression screening platform, nor does the level of transcript for the target protein need to change.

In a preferred embodiment, as outlined above, screens may be done on individual genes and gene products (proteins). That is, having identified a particular differentially expressed gene as important in a particular state, screening of modulators of either the expression of the gene or the gene product itself can be done. The gene products of differentially expressed genes are sometimes referred to herein as "prostate cancer proteins" or a "prostate cancer modulatory protein". The prostate cancer modulatory protein may be a fragment, or alternatively, be the full length protein to the fragment encoded by the nucleic acids of Tables 1-16. Preferably, the prostate cancer modulatory protein is a fragment. In a preferred embodiment, the prostate cancer amino acid sequence which is used to determine sequence identity or similarity is encoded by a nucleic acid of Tables 1-16. In another embodiment, the sequences are naturally occurring allelic variants of a protein encoded by a nucleic acid of Tables 1-16. In another embodiment, the sequences are sequence variants as further described herein.

Preferably, the prostate cancer modulatory protein is a fragment of approximately 14 to 24 amino acids long. More preferably the fragment is a soluble fragment. Preferably, the fragment includes a non-transmembrane region. In a preferred embodiment, the fragment has an N-terminal Cys to aid in solubility. In one embodiment, the C-terminus of the fragment is kept as a free acid and the N-terminus is a free amine to aid in coupling, i.e., to cysteine.

In one embodiment the prostate cancer proteins are conjugated to an immunogenic agent as discussed herein. In one embodiment the prostate cancer protein is conjugated to BSA.

Measurements of prostate cancer polypeptide activity, or of prostate cancer or the prostate cancer phenotype can be performed using a variety of assays. For example, the effects of the test compounds upon the function of the prostate cancer polypeptides can be measured by examining parameters described above. A suitable physiological change that affects activity can be used to assess the influence of a test compound on the polypeptides of this invention. When the functional consequences are determined using intact cells or animals, one can also measure a variety of effects such as, in the case of prostate cancer associated with tumors, tumor growth, tumor metastasis, neovascularization, hormone release, transcriptional changes to both known and uncharacterized genetic markers (e.g., northern blots), changes in cell metabolism such as cell growth or pH changes, and changes in intracellular second messengers such as cGMP. In the assays of the invention, mammalian prostate cancer polypeptide is typically used, e.g., mouse, preferably human.

Assays to identify compounds with modulating activity can be performed *in vitro.* For example, a prostate cancer polypeptide is first contacted with a potential modulator and incubated for a suitable amount of time, e.g., from 0.5 to 48 hours. In one embodiment, the prostate cancer polypeptide levels are determined *in vitro* by measuring the level of protein or mRNA. The level of protein is measured using immunoassays such as western blotting, ELISA and the like with an antibody that selectively binds to the prostate cancer polypeptide or a fragment thereof. For measurement of mRNA, amplification, e.g., using PCR, LCR, or hybridization assays, e.g., northern hybridization, RNAse protection, dot blotting, are preferred. The level of protein or mRNA is detected using directly or indirectly labeled detection agents, e.g., fluorescently or radioactively labeled nucleic acids, radioactively or enzymatically labeled antibodies, and the like, as described herein.

Alternatively, a reporter gene system can be devised using the prostate cancer protein promoter operably linked to a reporter gene such as luciferase, green fluorescent protein, CAT, or β-gal. The reporter construct is typically transfected into a cell. After treatment with a potential modulator, the amount of reporter gene transcription, translation, or activity is measured according to standard techniques known to those of skill in the art.

In a preferred embodiment, as outlined above, screens may be done on individual genes and gene products (proteins). That is, having identified a particular differentially expressed gene as important in a particular state, screening of modulators of the expression of the gene or the gene product itself can be done. The gene products of differentially expressed genes are sometimes referred to herein as "prostate cancer proteins." The prostate cancer protein may be a fragment, or alternatively, be the full length protein to a fragment shown herein.

In one embodiment, screening for modulators of expression of specific genes is performed. Typically, the expression of only one or a few genes are evaluated. In another embodiment, screens are designed to first find compounds that bind to differentially expressed proteins. These compounds are then evaluated for the ability to modulate differentially expressed activity. Moreover, once initial candidate compounds are identified, variants can be further screened to better evaluate structure activity relationships.

In a preferred embodiment, binding assays are done. In general, purified or isolated gene product is used; that is, the gene products of one or more differentially expressed nucleic acids are made. For example, antibodies are generated to the protein gene products, and standard immunoassays are run to determine the amount of protein present. Alternatively, cells comprising the prostate cancer proteins can be used in the assays.

Thus, in a preferred embodiment, the methods comprise combining a prostate cancer protein and a candidate compound, and determining the binding of the compound to the prostate cancer protein. Preferred embodiments utilize the human prostate cancer protein, although other mammalian proteins may also be used, e.g. for the development of animal models of human disease. In some embodiments, as outlined herein, variant or derivative prostate cancer proteins may be used.

Generally, in a preferred embodiment of the methods herein, the prostate cancer protein or the candidate agent is non-diffusably bound to an insoluble support having isolated sample receiving areas (e.g. a microtiter plate, an array, etc.). The insoluble supports may be made of any composition to which the compositions can be bound, is readily separated from soluble material, and is otherwise compatible with the overall method of screening. The surface of such supports may be solid or porous and of any convenient shape. Examples of suitable insoluble supports include microtiter plates, arrays, membranes and beads. These are typically made of glass, plastic (e.g., polystyrene), polysaccharides, nylon or nitrocellulose, teflon^{™}, etc. Microtiter plates and arrays are especially convenient because a large number of assays can be carried out simultaneously, using small amounts of reagents and samples. The particular manner of binding of the composition is not crucial so long as it is compatible with the reagents and overall methods of the invention, maintains the activity of the composition and is nondiffusable. Preferred methods of binding include the use of antibodies (which do not sterically block either the ligand binding site or activation sequence when the protein is bound to the support), direct binding to "sticky" or ionic supports, chemical crosslinking, the synthesis of the protein or agent on the surface, etc. Following binding of the protein or agent, excess unbound material is removed by washing. The sample receiving areas may then be blocked through incubation with bovine serum albumin (BSA), casein or other innocuous protein or other moiety.

In a preferred embodiment, the prostate cancer protein is bound to the support, and a test compound is added to the assay. Alternatively, the candidate agent is bound to the support and the prostate cancer protein is added. Novel binding agents include specific antibodies, non-natural binding agents identified in screens of chemical libraries, peptide analogs, etc. Of particular interest are screening assays for agents that have a low toxicity for human cells. A wide variety of assays may be used for this purpose, including labeled in vitro protein-protein binding assays, electrophoretic mobility shift assays, immunoassays for protein binding, functional assays (phosphorylation assays, etc.) and the like.

The determination of the binding of the test modulating compound to the prostate cancer protein may be done in a number of ways. In a preferred embodiment, the compound is labeled, and binding determined directly, e.g., by attaching all or a portion of the prostate cancer protein to a solid support, adding a labeled candidate agent (e.g., a fluorescent label), washing off excess reagent, and determining whether the label is present on the solid support. Various blocking and washing steps may be utilized as appropriate.

In some embodiments, only one of the components is labeled, e.g., the proteins (or proteinaceous candidate compounds) can be labeled. Alternatively, more than one component can be labeled with different labels, e.g., ¹²⁵I for the proteins and a fluorophor for the compound. Proximity reagents, e.g., quenching or energy transfer reagents are also useful.

In one embodiment, the binding of the test compound is determined by competitive binding assay. The competitor is a binding moiety known to bind to the target molecule (i.e., a prostate cancer protein), such as an antibody, peptide, binding partner, ligand, etc. Under certain circumstances, there may be competitive binding between the compound and the binding moiety, with the binding moiety displacing the compound. In one embodiment, the test compound is labeled. Either the compound, or the competitor, or both, is added first to the protein for a time sufficient to allow binding, if present. Incubations may be performed at a temperature which facilitates optimal activity, typically between 4 and 40°C. Incubation periods are typically optimized, e.g., to facilitate rapid high throughput screening. Typically between 0.1 and 1 hour will be sufficient. Excess reagent is generally removed or washed away. The second component is then added, and the presence or absence of the labeled component is followed, to indicate binding.

In a preferred embodiment, the competitor is added first, followed by the test compound. Displacement of the competitor is an indication that the test compound is binding to the prostate cancer protein and thus is capable of binding to, and potentially modulating, the activity of the prostate cancer protein. In this embodiment, either component can be labeled. Thus, e.g., if the competitor is labeled, the presence of label in the wash solution indicates displacement by the agent. Alternatively, if the test compound is labeled, the presence of the label on the support indicates displacement.

In an alternative embodiment, the test compound is added first, with incubation and washing, followed by the competitor. The absence of binding by the competitor may indicate that the test compound is bound to the prostate cancer protein with a higher affinity. Thus, if the test compound is labeled, the presence of the label on the support, coupled with a lack of competitor binding, may indicate that the test compound is capable of binding to the prostate cancer protein.

In a preferred embodiment, the methods comprise differential screening to identity agents that are capable of modulating the activity of the prostate cancer proteins. In this embodiment, the methods comprise combining a prostate cancer protein and a competitor in a first sample. A second sample comprises a test compound, a prostate cancer protein, and a competitor. The binding of the competitor is determined for both samples, and a change, or difference in binding between the two samples indicates the presence of an agent capable of binding to the prostate cancer protein and potentially modulating its activity. That is, if the binding of the competitor is different in the second sample relative to the first sample, the agent is capable of binding to the prostate cancer protein.

Alternatively, differential screening is used to identify drug candidates that bind to the native prostate cancer protein, but cannot bind to modified prostate cancer proteins. The structure of the prostate cancer protein may be modeled, and used in rational drug design to synthesize agents that interact with that site. Drug candidates that affect the activity of a prostate cancer protein are also identified by screening drugs for the ability to either enhance or reduce the activity of the protein.

Positive controls and negative controls may be used in the assays. Preferably control and test samples are performed in at least triplicate to obtain statistically significant results. Incubation of all samples is for a time sufficient for the binding of the agent to the protein. Following incubation, samples are washed free of non-specifically bound material and the amount of bound, generally labeled agent determined. For example, where a radiolabel is employed, the samples may be counted in a scintillation counter to determine the amount of bound compound.

A variety of other reagents may be included in the screening assays. These include reagents like salts, neutral proteins, e.g. albumin, detergents, etc. which may be used to facilitate optimal protein-protein binding and/or reduce non-specific or background interactions. Also reagents that otherwise improve the efficiency of the assay, such as protease inhibitors, nuclease inhibitors, anti-microbial agents, etc., may be used. The mixture of components may be added in an order that provides for the requisite binding.

In a preferred embodiment, the invention provides methods for screening for a compound capable of modulating the activity of a prostate cancer protein. The methods comprise adding a test compound, as defined above, to a cell comprising prostate cancer proteins. Preferred cell types include almost any cell. The cells contain a recombinant nucleic acid that encodes a prostate cancer protein. In a preferred embodiment, a library of candidate agents are tested on a plurality of cells.

In one aspect, the assays are evaluated in the presence or absence or previous or subsequent exposure of physiological signals, e.g. hormones, antibodies, peptides, antigens, cytokines, growth factors, action potentials, pharmacological agents including chemotherapeutics, radiation, carcinogenics, or other cells (i.e. cell-cell contacts). In another example, the determinations are determined at different stages of the cell cycle process.

In this way, compounds that modulate prostate cancer agents are identified. Compounds with pharmacological activity are able to enhance or interfere with the activity of the prostate cancer protein. Once identified, similar structures are evaluated to identify critical structural feature of the compound.

In one embodiment, a method of inhibiting prostate cancer cell division is provided. The method comprises administration of a prostate cancer inhibitor. In another embodiment, a method of inhibiting prostate cancer is provided. The method comprises administration of a prostate cancer inhibitor. In a further embodiment, methods of treating cells or individuals with prostate cancer are provided. The method comprises administration of a prostate cancer inhibitor.

In one embodiment, a prostate cancer inhibitor is an antibody as discussed above. In another embodiment, the prostate cancer inhibitor is an antisense molecule.

A variety of cell growth, proliferation, and metastasis assays are known to those of skill in the art, as described below.

### Soft agar growth or colony formation in suspension

Normal cells require a solid substrate to attach and grow. When the cells are transformed, they lose this phenotype and grow detached from the substrate. For example, transformed cells can grow in stirred suspension culture or suspended in semi-solid media, such as semi-solid or soft agar. The transformed cells, when transfected with tumor suppressor genes, regenerate normal phenotype and require a solid substrate to attach and grow. Soft agar growth or colony formation in suspension assays can be used to identify modulators of prostate cancer sequences, which when expressed in host cells, inhibit abnormal cellular proliferation and transformation. A therapeutic compound would reduce or eliminate the host cells' ability to grow in stirred suspension culture or suspended in semi-solid media, such as semi-solid or soft.

Techniques for soft agar growth or colony formation in suspension assays are described in Freshney, Culture of Animal Cells a Manual of Basic Technique (3rd ed., 1994), herein incorporated by reference. *See also,* the methods section of Garkavtsev *et al.* (1996), *supra,* herein incorporated by reference.

### Contact inhibition and density limitation of growth

Normal cells typically grow in a flat and organized pattern in a petri dish until they touch other cells. When the cells touch one another, they are contact inhibited and stop growing. When cells are transformed, however, the cells are not contact inhibited and continue to grow to high densities in disorganized foci. Thus, the transformed cells grow to a higher saturation density than normal cells. This can be detected morphologically by the formation of a disoriented monolayer of cells or rounded cells in foci within the regular pattern of normal surrounding cells. Alternatively, labeling index with (³H)-thymidine at saturation density can be used to measure density limitation of growth. *See* Freshney (1994), *supra.* The transformed cells, when transfected with tumor suppressor genes, regenerate a normal phenotype and become contact inhibited and would grow to a lower density.

In this assay, labeling index with (³H)-thymidine at saturation density is a preferred method of measuring density limitation of growth. Transformed host cells are transfected with a prostate cancer-associated sequence and are grown for 24 hours at saturation density in non-limiting medium conditions. The percentage of cells labeling with (³H)-thymidine is determined autoradiographically. *See,* Freshney (1994), *supra.*

### Growth factor or serum dependence

Transformed cells have a lower serum dependence than their normal counterparts (*see, e.g.,* Temin, J. Natl. Cancer Insti. 37:167-175 (1966); Eagle et al., J. Exp. Med. 131:836-879 (1970)); Freshney, *supra.* This is in part due to release of various growth factors by the transformed cells. Growth factor or serum dependence of transformed host cells can be compared with that of control.

### Tumor specific markers levels

Tumor cells release an increased amount of certain factors (hereinafter "tumor specific markers") than their normal counterparts. For example, plasminogen activator (PA) is released from human glioma at a higher level than from normal brain cells (*see, e.g.,* Gullino, Angiogenesis, tumor vascularization, and potential interference with tumor growth. in Biological Responses in Cancer, pp. 178-184 (Mihich (ed.) 1985)). Similarly, Tumor angiogenesis factor (TAF) is released at a higher level in tumor cells than their normal counterparts. *See, e.g.,* Folkman, Angiogenesis and Cancer, Sem Cancer Biol. (1992)).

Various techniques which measure the release of these factors are described in Freshney (1994), *supra.* Also, *see,* Unkless et al. , J. Biol. Chem. 249:4295-4305 (1974); Strickland & Beers, J. Biol. Chem. 251:5694-5702 (1976); Whur et al., Br. J. Cancer 42:305-312 (1980); Gullino, Angiogenesis, tumor vascularization, and potential interference with tumor growth. in Biological Responses in Cancer, pp. 178-184 (Mihich (ed.) 1985); Freshney Anticancer Res. 5:111-130 (1985).

### Invasiveness into Matrigel

The degree of invasiveness into Matrigel-or some other extracellular matrix constituent can be used as an assay to identify compounds that modulate prostate cancer-associated sequences. Tumor cells exhibit a good correlation between malignancy and invasiveness of cells into Matrigel or some other extracellular matrix constituent. In this assay, tumorigenic cells are typically used as host cells. Expression of a tumor suppressor gene in these host cells would decrease invasiveness of the host cells.

Techniques described in Freshney (1994), *supra,* can be used. Briefly, the level of invasion of host cells can be measured by using filters coated with Matrigel or some other extracellular matrix constituent. Penetration into the gel, or through to the distal side of the filter, is rated as invasiveness, and rated histologically by number of cells and distance moved, or by prelabeling the cells with ¹²⁵I and counting the radioactivity on the distal side of the filter or bottom of the dish. *See, e.g.,* Freshney (1984), *supra.*

### Tumor growth in vivo

Effects of prostate cancer-associated sequences on cell growth can be tested in transgenic or immune-suppressed mice. Knock-out transgenic mice can be made, in which the prostate cancer gene is disrupted or in which a prostate cancer gene is inserted. Knock-out transgenic mice can be made by insertion of a marker gene or other heterologous gene into the endogenous prostate cancer gene site in the mouse genome via homologous recombination. Such mice can also be made by substituting the endogenous prostate cancer gene with a mutated version of the prostate cancer gene, or by mutating the endogenous prostate cancer gene, e.g., by exposure to carcinogens.

A DNA construct is introduced into the nuclei of embryonic stem cells. Cells containing the newly engineered genetic lesion are injected into a host mouse embryo, which is re-implanted into a recipient female. Some of these embryos develop into chimeric mice that possess germ cells partially derived from the mutant cell line. Therefore, by breeding the chimeric mice it is possible to obtain a new line of mice containing the introduced genetic lesion (*see, e.g.*, Capecchi et al., Science 244:1288 (1989)). Chimeric targeted mice can be derived according to Hogan et al., Manipulating the Mouse Embryo: A Laboratory Manual, Cold Spring Harbor Laboratory (1988) and Teratocarcinomas and Embryonic Stem Cells: A Practical Approach, Robertson, ed., IRL Press, Washington, D.C., (1987).

Alternatively, various immune-suppressed or immune-deficient host animals can be used. For example, genetically athymic "nude" mouse (*see, e.g.,* Giovanella et al., J. Natl. Cancer Inst. 52:921 (1974)), a SCID mouse, a thymectomized mouse, or an irradiated mouse (*see, e.g.,* Bradley et al., Br. J. Cancer 38:263 (1978); Selby et al., Br. J. Cancer 41:52 (1980)) can be used as a host. Transplantable tumor cells (typically about 10⁶ cells) injected into isogenic hosts will produce invasive tumors in a high proportions of cases, while normal cells of similar origin will not. In hosts which developed invasive tumors, cells expressing a prostate cancer-associated sequences are injected subcutaneously. After a suitable length of time, preferably 4-8 weeks, tumor growth is measured (e.g., by volume or by its two largest dimensions) and compared to the control. Tumors that have statistically significant reduction (using, e.g., Student's T test) are said to have inhibited growth.

### Methods of identifying variant prostate cancer-associated sequences

Without being bound by theory, expression of various prostate cancer sequences is correlated with prostate cancer. Accordingly, disorders based on mutant or variant prostate cancer genes may be determined. In one embodiment, the invention provides methods for identifying cells containing variant prostate cancer genes, e.g., determining all or part of the sequence of at least one endogenous prostate cancer genes in a cell. This may be accomplished using any number of sequencing techniques. In a preferred embodiment, the invention provides methods of identifying the prostate cancer genotype of an individual, e.g., determining all or part of the sequence of at least one prostate cancer gene of the individual. This is generally done in at least one tissue of the individual, and may include the evaluation of a number of tissues or different samples of the same tissue. The method may include comparing the sequence of the sequenced prostate cancer gene to a known prostate cancer gene, i.e., a wild-type gene.

The sequence of all or part of the prostate cancer gene can then be compared to the sequence of a known prostate cancer gene to determine if any differences exist. This can be done using any number of known homology programs, such as Bestfit, etc. In a preferred embodiment, the presence of a difference in the sequence between the prostate cancer gene of the patient and the known prostate cancer gene correlates with a disease state or a propensity for a disease state, as outlined herein.

In a preferred embodiment, the prostate cancer genes are used as probes to determine the number of copies of the prostate cancer gene in the genome.

In another preferred embodiment, the prostate cancer genes are used as probes to determine the chromosomal localization of the prostate cancer genes. Information such as chromosomal localization finds use in providing a diagnosis or prognosis in particular when chromosomal abnormalities such as translocations, and the like are identified in the prostate cancer gene locus.

### Administration of pharmaceutical and vaccine compositions

In one embodiment, a therapeutically effective dose of a prostate cancer protein or modulator thereof, is administered to a patient. By "therapeutically effective dose" herein is meant a dose that produces effects for which it is administered. The exact dose will depend on the purpose of the treatment, and will be ascertainable by one skilled in the art using known techniques (e.g., Ansel et al., Pharmaceutical Dosage Forms and Drug Delivery*;* Lieberman, Pharmaceutical Dosage Forms (vols. 1-3, 1992), Dekker, ISBN 0824770846, 082476918X, 0824712692, 0824716981; Lloyd, The Art, Science and Technology of Pharmaceutical Compounding (1999); and Pickar, Dosage Calculations (1999)). As is known in the art, adjustments for prostate cancer degradation, systemic versus localized delivery, and rate of new protease synthesis, as well as the age, body weight, general health, sex, diet, time of administration, drug interaction and the severity of the condition may be necessary, and will be ascertainable with routine experimentation by those skilled in the art. U.S. Patent Application N. 09/687,576, further discloses the use of compositions and methods of diagnosis and treatment in prostate cancer is hereby expressly incorporated by reference.

A "patient" for the purposes of the present invention includes both humans and other animals, particularly mammals. Thus the methods are applicable to both human therapy and veterinary applications. In the preferred embodiment the patient is a mammal, preferably a primate, and in the most preferred embodiment the patient is human.

The administration of the prostate cancer proteins and modulators thereof of the present invention can be done in a variety of ways as discussed above, including, but not limited to, orally, subcutaneously, intravenously, intranasally, transdermally, intraperitoneally, intramuscularly, intrapulmonary, vaginally, rectally, or intraocularly. In some instances, e.g., in the treatment of wounds and inflammation, the prostate cancer proteins and modulators may be directly applied as a solution or spray.

The pharmaceutical compositions of the present invention comprise a prostate cancer protein in a form suitable for administration to a patient. In the preferred embodiment, the pharmaceutical compositions are in a water soluble form, such as being present as pharmaceutically acceptable salts, which is meant to include both acid and base addition salts. "Pharmaceutically acceptable acid addition salt" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like. "Pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium, and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine.

The pharmaceutical compositions may also include one or more of the following: carrier proteins such as serum albumin; buffers; fillers such as microcrystalline cellulose, lactose, corn and other starches; binding agents; sweeteners and other flavoring agents; coloring agents; and polyethylene glycol.

The pharmaceutical compositions can be administered in a variety of unit dosage forms depending upon the method of administration. For example, unit dosage forms suitable for oral administration include, but are not limited to, powder, tablets, pills, capsules and lozenges. It is recognized that prostate cancer protein modulators (e.g., antibodies, antisense constructs, ribozymes, small organic molecules, *etc.*) when administered orally, should be protected from digestion. This is typically accomplished either by complexing the molecule(s) with a composition to render it resistant to acidic and enzymatic hydrolysis, or by packaging the molecule(s) in an appropriately resistant carrier, such as a liposome or a protection barrier. Means of protecting agents from digestion are well known in the art.

The compositions for administration will commonly comprise a prostate cancer protein modulator dissolved in a pharmaceutically acceptable carrier, preferably an aqueous carrier. A variety of aqueous carriers can be used, e.g., buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, e.g., sodium acetate, sodium chloride, potassium chloride, calcium chloride, sodium lactate and the like. The concentration of active agent in these formulations can vary widely, and will be selected primarily based on fluid volumes, viscosities, body weight and the like in accordance with the particular mode of administration selected and the patient's needs (e.g., Remington's Pharmaceutical Science (15th ed., 1980) and Goodman & Gillman, The Pharmacologial Basis of Therapeutics (Hardman et al.,eds., 1996)).

Thus, a typical pharmaceutical composition for intravenous administration would be about 0.1 to 10 mg per patient per day. Dosages from 0.1 up to about 100 mg per patient per day may be used, particularly when the drug is administered to a secluded site and not into the blood stream, such as into a body cavity or into a lumen of an organ. Substantially higher dosages are possible in topical administration. Actual methods for preparing parenterally administrable compositions will be known or apparent to those skilled in the art, *e.g.,* Remington's Pharmaceutical Science and Goodman and Gillman, The Pharmacologial Basis of Therapeutics*, supra.*

The compositions containing modulators of prostate cancer proteins can be administered for therapeutic or prophylactic treatments. In therapeutic applications, compositions are administered to a patient suffering from a disease (e.g., a cancer) in an amount sufficient to cure or at least partially arrest the disease and its complications. An amount adequate to accomplish this is defined as a "therapeutically effective dose." Amounts effective for this use will depend upon the severity of the disease and the general state of the patient's health. Single or multiple administrations of the compositions may be administered depending on the dosage and frequency as required and tolerated by the patient. In any event, the composition should provide a sufficient quantity of the agents of this invention to effectively treat the patient. An amount of modulator that is capable of preventing or slowing the development of cancer in a mammal is referred to as a "prophylactically effective dose." The particular dose required for a prophylactic treatment will depend upon the medical condition and history of the mammal, the particular cancer being prevented, as well as other factors such as age, weight, gender, administration route, efficiency, *etc.* Such prophylactic treatments may be used, *e.g.,* in a mammal who has previously had cancer to prevent a recurrence of the cancer, or in a mammal who is suspected of having a significant likelihood of developing cancer.

It will be appreciated that the present prostate cancer protein-modulating compounds can be administered alone or in combination with additional prostate cancer modulating compounds or with other therapeutic agent, *e.g.*, other anti-cancer agents or treatments.

In numerous embodiments, one or more nucleic acids, *e.g.*, polynucleotides comprising nucleic acid sequences set forth in Tables 1-16, such as antisense polynucleotides or ribozymes, will be introduced into cells, *in vitro* or *in vivo.* The present invention provides methods, reagents, vectors, and cells useful for expression of prostate cancer-associated polypeptides and nucleic acids using *in vitro* (cell-free), *ex vivo* or *in vivo* (cell or organism-based) recombinant expression systems.

The particular procedure used to introduce the nucleic acids into a host cell for expression of a-protein or nucleic acid is application specific. Many procedures for introducing foreign nucleotide sequences into host cells may be used. These include the use of calcium phosphate transfection, spheroplasts, electroporation, liposomes, microinjection, plasma vectors, viral vectors and any of the other well known methods for introducing cloned genomic DNA, cDNA, synthetic DNA or other foreign genetic material into a host cell (*see, e.g.*, Berger & Kimmel, Guide to Molecular Cloning Techniques, Methods in Enzymology volume 152 (Berger), Ausubel et al., eds., Current Protocols (supplemented through 1999), and Sambrook et al., Molecular Cloning - A Laboratory Manual (2nd ed., Vol. 1-3, 1989.

In a preferred embodiment, prostate cancer proteins and modulators are administered as therapeutic agents, and can be formulated as outlined above. Similarly, prostate cancer genes (including both the full-length sequence, partial sequences, or regulatory sequences of the prostate cancer coding regions) can be administered in a gene therapy application. These prostate cancer genes can include antisense applications, either as gene therapy (i.e. for incorporation into the genome) or as antisense compositions, as will be appreciated by those in the art.

Prostate cancer polypeptides and polynucleotides can also be administered as vaccine compositions to stimulate HTL, CTL and antibody responses.. Such vaccine compositions can include, e.g., lipidated peptides (*see, e.g.*,Vitiello, A. et al., J. Clin. Invest. 95:341 (1995)), peptide compositions encapsulated in poly(DL-lactide-co-glycolide) ("PLG") microspheres (*see, e.g.,* Eldridge, et al., Molec. Immunol. 28:287-294, (1991); Alonso et al., Vaccine 12:299-306 (1994); Jones et al., Vaccine 13:675-681 (1995)), peptide compositions contained in immune stimulating complexes (ISCOMS) (*see, e.g.,* Takahashi et al., Nature 344:873-875 (1990); Hu et al., Clin Exp Immunol. 113:235-243 (1998)), multiple antigen peptide systems (MAPs) (*see, e.g.,* Tam, Proc. Natl. Acad Sci. U.S.A. 85:5409-5413 (1988); Tam, J. Immunol. Methods 196:17-32 (1996)), peptides formulated as multivalent peptides; peptides for use in ballistic delivery systems, typically crystallized peptides, viral delivery vectors (Perkus, et al., In: Concepts in vaccine development (Kaufmann, ed., p. 379, 1996); Chakrabarti, et al., Nature 320:535 (1986); Hu et al., Nature 320:537 (1986); Kieny, et al., AIDS Bio/Technology 4:790 (1986); Top et al., J. Infect. Dis. 124:148 (1971); Chanda et al., Virology 175:535 (1990)), particles of viral or synthetic origin (*see, e.g.,* Kofler et al., J. Immunol. Methods. 192:25 (1996); Eldridge et al., Sem. Hematol. 30:16 (1993); Falo et al., Nature Med. 7:649 (1995)), adjuvants (Warren et al., Annu. Rev. Immunol. 4:369 (1986); Gupta et al., Vaccine 11:293 (1993)), liposomes (Reddy et al., J. Immunol. 148:1585 (1992); Rock, Immunol. Today 17:131 (1996)), or, naked or particle absorbed cDNA (Ulmer, et al., Science 259:1745 (1993); Robinson et al., Vaccine 11:957 (1993); Shiver et al., In: Concepts in vaccine development (Kaufmann, ed., p. 423,1996); Cease & Berzofsky, Annu. Rev. Immunol. 12:923 (1994) and Eldridge et al., Sem. Hematol. 30:16 (1993)). Toxin-targeted delivery technologies, also known as receptor mediated targeting, such as those of Avant Immunotherapeutics, Inc. (Needham, Massachusetts) may also be used.

Vaccine compositions often include adjuvants. Many adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Certain adjuvants are commercially available as, e.g., Freund's Incomplete Adjuvant and Complete Adjuvant (Difco Laboratories, Detroit, MI); Merck Adjuvant 65 (Merck and Company, Inc., Rahway, NJ); AS-2 (SmithKline Beecham, Philadelphia, PA); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF, interleukin-2, -7, -12, and other like growth factors, may also be used as adjuvants.

Vaccines can be administered as nucleic acid compositions wherein DNA or RNA encoding one or more of the polypeptides, or a fragment thereof, is administered to a patient. This approach is described, for instance, in Wolff et. al., Science 247:1465 (1990) as well as U.S. Patent Nos. 5,580,859; 5,589,466; 5,804,566; 5,739,118; 5,736,524; 5,679,647; WO 98/04720; and in more detail below. Examples of DNA-based delivery technologies include "naked DNA", facilitated (bupivicaine, polymers, peptide-mediated) delivery, cationic lipid complexes, and particle-mediated ("gene gun") or pressure-mediated delivery (*see, e.g.,* U.S. Patent No. 5,922,687).

For therapeutic or prophylactic immunization purposes, the peptides of the invention can be expressed by viral or bacterial vectors. Examples of expression vectors include attenuated viral hosts, such as vaccinia or fowlpox. This approach involves the use of vaccinia virus, e.g., as a vector to express nucleotide sequences that encode prostate cancer polypeptides or polypeptide fragments. Upon introduction into a host, the recombinant vaccinia virus expresses the immunogenic peptide, and thereby elicits an immune response. Vaccinia vectors and methods useful in immunization protocols are described in, *e.g.*, U.S. Patent No. 4,722,848. Another vector is BCG (Bacille Calmette Guerin). BCG vectors are described in Stover et al., Nature 351:456-460 (1991). A wide variety of other vectors useful for therapeutic administration or immunization e.g. adeno and adeno-associated virus vectors, retroviral vectors, *Salmonella typhi* vectors, detoxified anthrax toxin vectors, and the like, will be apparent to those skilled in the art from the description herein (*see, e.g.,* Shata et al., Mol Med Today 6:66-71 (2000); Shedlock et al., J Leukoc Biol 68:793-806 (2000); Hipp et al., In Vivo 14:571-85 (2000)).

Methods for the use of genes as DNA vaccines are well known, and include placing a prostate cancer gene or portion of a prostate cancer gene under the control of a regulatable promoter or a tissue-specific promoter for expression in a prostate cancer patient. The prostate cancer gene used for DNA vaccines can encode full-length prostate cancer proteins, but more preferably encodes portions of the prostate cancer proteins including peptides derived from the prostate cancer protein. In one embodiment, a patient is immunized with a DNA vaccine comprising a plurality of nucleotide sequences derived from a prostate cancer gene. For example, prostate cancer-associated genes or sequence encoding subfragments of a prostate cancer protein are introduced into expression vectors and tested for their immunogenicity in the context of Class I MHC and an ability to generate cytotoxic T cell responses. This procedure provides for production of cytotoxic T cell responses against cells which present antigen, including intracellular epitopes.

In a preferred embodiment, the DNA vaccines include a gene encoding an adjuvant molecule with the DNA vaccine. Such adjuvant molecules include cytokines that increase the immunogenic response to the prostate cancer polypeptide encoded by the DNA vaccine. Additional or alternative adjuvants are available.

In another preferred embodiment prostate cancer genes find use in generating animal models of prostate cancer. When the prostate cancer gene identified is repressed or diminished in cancer tissue, gene therapy technology, e.g., wherein antisense RNA directed to the prostate cancer gene will also diminish or repress expression of the gene. Animal models of prostate cancer find use in screening for modulators of a prostate cancer-associated sequence or modulators of prostate cancer. Similarly, transgenic animal technology including gene knockout technology, e.g. as a result of homologous recombination with an appropriate gene targeting vector, will result in the absence or increased expression of the prostate cancer protein. When desired, tissue-specific expression or knockout of the prostate cancer protein may be necessary.

It is also possible that the prostate cancer protein is overexpressed in prostate cancer. As such, transgenic animals can be generated that overexpress the prostate cancer protein. Depending on the desired expression level, promoters of various strengths can be employed to express the transgene. Also, the number of copies of the integrated transgene can be determined and compared for a determination of the expression level of the transgene. Animals generated by such methods find use as animal models of prostate cancer and are additionally useful in screening for modulators to treat prostate cancer.

### Kits for Use in Diagnostic and/or Prognostic Applications

For use in diagnostic, research, and therapeutic applications suggested above, kits are also provided by the invention. In the diagnostic and research applications such kits may include any or all of the following: assay reagents, buffers, prostate cancer-specific nucleic acids or antibodies, hybridization probes and/or primers, antisense polynucleotides, ribozymes, dominant negative prostate cancer polypeptides or polynucleotides, small molecules inhibitors of prostate cancer-associated sequences etc. A therapeutic product may include sterile saline or another pharmaceutically acceptable emulsion and suspension base.

In addition, the kits may include instructional materials containing directions (i.e., protocols) for the practice of the methods of this invention. While the instructional materials typically comprise written or printed materials they are not limited to such. Any medium capable of storing such instructions and communicating them to an end user is contemplated by this invention. Such media include, but are not limited to electronic storage media (e.g., magnetic discs, tapes, cartridges, chips), optical media (e.g., CD ROM), and the like. Such media may include addresses to internet sites that provide such instructional materials.

The present invention also provides for kits for screening for modulators of prostate cancer-associated sequences. Such kits can be prepared from readily available materials and reagents. For example, such kits can comprise one or more of the following materials: a prostate cancer-associated polypeptide or polynucleotide, reaction tubes, and instructions for testing prostate cancer-associated activity. Optionally, the kit contains biologically active prostate cancer protein. A wide variety of kits and components can be prepared according to the present invention, depending upon the intended user of the kit and the particular needs of the user. Diagnosis would typically involve evaluation of a plurality of genes or products. The genes will be selected based on correlations with important parameters in disease which may be identified in historical or outcome data.

### EXAMPLES

### Example 1: Tissue Preparation, Labeling Chips, and Fingerprints

### Purifying total RNA from tissue sample using TRIzol Reagent

The sample weight is first estimated. The tissue samples are homogenized in 1 ml of TRIzol per 50 mg of tissue using a homogenizer (e.g., Polytron 3100). The size of the generator/probe used depends upon the sample amount. A generator that is too large for the amount of tissue to be homogenized will cause a loss of sample and lower RNA yield. A larger generator (e.g., 20 mm) is suitable for tissue samples weighing more than 0.6 g. Fill tubes should not be overfilled. If the working volume is greater than 2 ml and no greater than 10 ml, a 15 ml polypropylene tube (Falcon 2059) is suitable for homogenization.

Tissues should be kept frozen until homogenized. The TRIzol is added directly to the frozen tissue before homogenization. Following homogenization, the insoluble material is removed from the homogenate by centrifugation at 7500 x g for 15 min. in a Sorvall superspeed or 12,000 x g for 10 min. in an Eppendorf centrifuge at 4°C. The cleared homogenate is then transferred to a new tube(s). Samples may be frozen and stored at -60 to -70°C for at least one month or else continue with the purification.

The next process is phase separation. The homogenized samples are incubated for 5 minutes at room temperature. Then, 0.2 ml of chloroform per 1ml of TRIzol reagent is added to the homogenization mixture. The tubes are securely capped and shaken vigorously by hand (do not vortex) for 15 seconds. The samples are then incubated at room temp. for 2-3 minutes and next centrifuged at 6500 rpm in a Sorvall superspeed for 30 min. at 4oC.

The next process is RNA Precipitation. The aqueous phase is transferred to a fresh tube. The organic phase can be saved if isolation of DNA or protein is desired. Then 0.5 ml of isopropyl alcohol is added per 1ml of TRIzol reagent used in the original homogenization. Then, the tubes are securely capped and inverted to mix. The samples are then incubated at room temp. for 10 minutes an centrifuged at 6500 rpm in Sorvall for 20 min. at 4°C.

The RNA is then washed. The supernatant is poured off and the pellet washed with cold 75% ethanol. 1 ml of 75% ethanol is used per 1 ml of the TRIzol reagent used in the initial homogenization. The tubes are capped securely and inverted several times to loosen pellet without vortexing. They are next centrifuged at <8000 rpm (<7500 x g) for 5 minutes at 4°C.

The RNA wash is decanted. The pellet is carefully transferred to an Eppendorf tube (sliding down the tube into the new tube by use of a pipet tip to help guide it in if necessary). Tube(s) sizes for precipitating the RNA depending on the working volumes. Larger tubes may take too long to dry. Dry pellet. The RNA is then resuspended in an appropriate volume (e.g., 2 -5 ug/ul) of DEPC H₂O. The absorbance is then measured.

The poly A+ mRNA may next be purified from total RNA by other methods such as Qiagen' s RNeasy kit. The poly A⁺ mRNA is purified from total RNA by adding the oligotex suspension which has been heated to 37°C and mixing prior to adding to RNA. The Elution Buffer is incubated at 70°C. If there is precipitate in the buffer, warm up the 2 x Binding Buffer at 65°C. The the total RNA is mixed with DEPC-treated water, 2 x Binding Buffer, and Oligotex according to Table 2 on page 16 of the Oligotex Handbook and next incubated for 3 minutes at 65°C and 10 minutes at room temperature.

The preparation is centrifuged for 2 minutes at 14,000 to 18,000 g, preferably, at a "soft setting," The supernatant is removed without disturbing Oligotex pellet. A little bit of solution can be left behind to reduce the loss of Oligotex. The supernatant is saved until satisfactory binding and elution of poly A⁺ mRNA has been found.

Then, the preparation is gently resuspended in Wash Buffer OW2 and pipetted onto the spin column and centrifuged at full speed (soft setting if possible) for 1 minute.

Next, the spin column is transferred to a new collection tube and gently resuspended in Wash Buffer OW2 and centrifuged as described herein.

Then, the spin column is transferred to a new tube and eluted with 20 to 100 ul of preheated (70°C) Elution Buffer. The Oligotex resin is gently resuspended by pipetting up and down. The centrifugation is repeated as above and the elution repeated with fresh elution buffer or first eluate to keep the elution volume low.

The absorbance is next read to determine the yield, using diluted Elution Buffer as the blank.

Before proceeding with cDNA synthesis, the mRNA is precipitated before proceeding with cDNA synthesis, as components leftover or in the Elution Buffer from the Oligotex purification procedure will inhibit downstream enzymatic reactions of the mRNA. 0.4 vol. of 7.5 M NH4OAc + 2.5 vol. of cold 100% ethanol is added and the preparation precipitated at -20°C 1 hour to overnight (or 20-30 min. at -70°C), and centrifuged at 14,000-16,000 x g for 30 minutes at 4°C. Next, the pellet is washed with 0.5 ml of 80% ethanol (-20°C) and then centrifuged at 14,000-16,000 x g for 5 minutes at room temperature. The80% ethanol wash is then repeated. The last bit of ethanol from the pellet is then dried without use of a speed vacuum and the pellet is then resuspended in DEPC H₂0 at 1ug/ul concentration.

### Alternatively the RNA may be purified using other methods (e.g., Qiagen's RNeasy kit).

No more than 100 ug is added to the RNeasy column. The sample volume is adjusted to 100 ul with RNase-free water. 350 ul Buffer RLT and then 250 ul ethanol (100%) are added to the sample. The preparation is then mixed by pipetting and applied to an RNeasy mini spin column for centrifugation (15 sec at >10,000 rpm). If yield is low, reapply the flowthrough to the column and centrifuge again.

Then, transfer column to a new 2 ml collection tube and add 500 ul Buffer RPE and centrifuge for 15 sec at >10,000 rpm. The flowthrough is discarded. 500 ul Buffer RPE and is then added and the preparation is centriuged for 15 sec at >10,000 rpm. The flowthrough is discarded. and the column membrane dried by centrifuging for 2 min at maximum speed. The column is transferred to a new 1.5-ml collection tube. 30-50 ul of RNase-free water is applied directly onto column membrane. The column is then centrifuged for 1 min at >10,000 rpm and the elution step repeated.

The absorbance is then read to determine yield. If necessary, the material may be ethanol precipitated with ammonium acetate and 2.5X volume 100% ethanol.

### First Strand cDNA Synthesis

The first strand can be make using using Gibco's "SuperScript Choice System for cDNA Synthesis" kit. The starting material is 5 ug of total RNA or 1 ug of polyA+ mRNAl. For total RNA, 2 ul of Superscript RT is used; for polyA+ mRNA, 1 ul of Superscript RT is used. The final volume of first strand synthesis mix is 20 ul. The RNA should be in a volume no greater than 10 ul. The RNA is incubated with 1 ul of 100 pmol T7-T24 oligo for 10 min at 70°C followed by addition on ice of 7 ul of: 4ul 5X 1^{st} Strand Buffer, 2 ul of 0.1M DTT, and 1 ul of 10mM dNTP mix. The preparation is then incubated at 37°C for 2 min before addition of the SuperScript RT followed by incubation at 37°C for 1 hour.

### Second Strand Synthesis

For the second strand synthesis, place 1st strand reactions on ice and add: 91 ul DEPC H₂O; 30 ul 5X 2nd Strand Buffer; 3 ul 10mM dNTP mix; 1 ul 10 U/ul E.coli DNA Ligase; 4 ul 10 U/ul E.coli DNA Polymerase; and 1 ul 2 U/ul RNase H. Mix and incubate 2 hours at 16°C. Add 2 ul T4 DNA Polymerase. Incubate 5 min at 16°C. Add 10 ul of 0.5M EDTA.

### Cleaning up cDNA

The cDNA is purified using Phenol:Chloroform:Isoamyl Alcohol (25:24:1) and Phase-Lock gel tubes. The PLG tubes are centrifuged for 30 sec at maximum speed. The cDNA mix is then transferred to PLG tube. An equal volume of phenol:chloroform:isamyl alcohol is then added, the preparation shaken vigorously (no vortexing), and centrifuged for 5 minutes at maximum speed. The top aqueous solution is transferred to a new tube and ethanol precipitated by adding 7.5X 5M NH40Ac and 2.5X volume of 100% ethanol. Next, it is centrifuged immediately at room temperature for 20 min, maximum speed. The supernatant is removed, and the pellet washed with 2X with cold 80% ethanol. As much ethanol wash as possible should be removed before air drying the pellet; and resuspending it in 3 ul RNase-free water.

### In vitro Transcription (IVT) and labeling with biotin

In vitro Transcription (IVT) and labeling with biotin is performed as follows: Pipet 1.5 ul of cDNA into a thin-wall PCR tube. Make NTP labeling mix by combining 2 ul T7 10xATP (75 mM) (Ambion); 2 ul T7 10xGTP (75 mM) (Ambion); 1.5 ul T7 10xCTP (75 mM) (Ambion); 1.5 ul T7 10xUTP (75 mM) (Ambion); 3.75 ul 10 mM Bio-11-UTP (Boehringer-Mannheim/Roche or Enzo); 3.75 ul 10 mM Bio-16-CTP (Enzo); 2 ul 10x T7 transcription buffer (Ambion); and 2 ul 10x T7 enzyme mix (Ambion). The final volume is 20 ul. Incubate 6 hours at 37°C in a PCR machine. The RNA can be furthered cleaned. Clean-up follows the previous instructions for RNeasy columns or Qiagen's RNeasy protocol handbook The cRNA often needs to be ethanol precipitated by resuspension in a volume compatible with the fragmentation step.

Fragmentation is performed as follows. 15 ug of labeled RNA is usually fragmented. Try to minimize the fragmentation reaction volume; a 10 ul volume is recommended but 20 ul is all right. Do not go higher than 20 ul because the magnesium in the fragmentation buffer contributes to precipitation in the hybridization buffer. Fragment RNA by incubation at 94 C for 35 minutes in 1 x Fragmentation buffer (5 x Fragmentation buffer is 200 mM Tris-acetate, pH 8.1; 500 mM KOAc; 150 mM MgOAc). The labeled RNA transcript can be analyzed before and after fragmentation. Samples can be heated to 65°C for 15 minutes and electrophoresed on 1% agarose/TBE gels to get an approximate idea of the transcript size range.

For hybridization, 200 ul (10 ug cRNA) of a hybridization mix is put on the chip. If multiple hybridizations are to be done (such as cycling through a 5 chip set), then it is recommended that an initial hybridization mix of 300 ul or more be made. The hybridization mix is: fragment labeled RNA (50 ng/ul final conc.); 50 pM 948-b control oligo; 1.5 pM BioB; 5 pM BioC; 25 pM BioD; 100 pM CRE; 0.1 mg/ml herring sperm DNA; 0.5 mg/ml acetylated BSA; and 300 ul with lxMES hyb buffer.

The hybridization reaction is conducted with non-biotinylated IVT (purified by RNeasy columns) (see example 1 for steps from tissue to IVT): The following mixture is prepared:

| | |
|---|---|
| IVT antisense RNA; 4 µg: | µl |
| Random Hexamers (1 µg/µl): | 4 µl |
| H₂O: | µl |
| | 14 µl |

Incubate the above 14 µl mixture at 70°C for 10 min.; then put on ice.

The Reverse transcription procedure uses the following mixture:

| | |
|---|---|
| 0.1 M DTT: | 3 µl |
| 50X dNTP mix: | 0.6 µl |
| H₂O: | 2.4 µl |
| Cy3 or Cy5 dUTP (1mM): | 3 µl |
| SS RT II (BRL): | 1 µl |
| | 16 µl |

The above solution is added to the hybridization reaction and incubated for 30 min., 42°C. Then, 1 µl SSII is added and incubated for another hour before being placed on ice.

The 50X dNTP mix contains 25mM of cold dATP, dCTP, and dGTP, 10mM of dTTP and is made by adding 25 µl each of 100mM dATP, dCTP, and dGTP; 10 µl of 100mM dTTP to 15 µl H₂O.]

RNA degradation is performed as follows. Add 86 µl H2O, 1.5 µl 1M NaOH/ 2 mM EDTA and incubate at 65°C, 10 min.. For U-Con 30, 500 µl TE/sample spin at 7000 g for 10 min, save flow through for purification. For Qiagen purification, suspend u-con recovered material in 500 µl buffer PB and proceed using Qiagen protocol. For DNAse digestion, add 1 ul of 1/100 dilution of DNAse/30 ul Rx and incubate at 37°C for 15 min. Incubate at 5 min 95°C to denature the DNAse.

### Sample preparation

For sample preparation, add Cot-1 DNA, 10 µl; 50X dNTPs, 1 µl; 20X SSC, 2.3 µl; Na pyro phosphate, 7.5 µl; 10 mg/ml Herring sperm DNA; 1 ul of 1/10 dilution to 21.8 final vol. Dry in speed vac. Resuspend in 15 µl H₂O. Add 0.38 µl 10% SDS. Heat 95°C, 2 min and slow cool at room temp. for 20 min. Put on slide and hybridize overnight at 64°C. Washing after the hybridization: 3X SSC/0.03% SDS: 2 min., 37.5 ml 20X SSC+0.75m110% SDS in 250ml H₂O; 1X SSC: 5 min., 12.5 ml 20X SSC in 250ml H₂O; 0.2X SSC: 5 min., 2.5 ml 20X SSC in 250ml H₂O. Dry slides and scan at appropiate PMT's and channels.

### Example 2: Taxol resistant Xenograft Model of Human Prostate Cancer

Treatment regimens that include paclitaxel (Taxol; Bristol-Myers Squibb Company, Princeton, NJ) have been particularly successful in treating hormone-refractory prostate cancer in the phase II setting (Smith et al., Semin. Oncol. 26(1 Suppl 2):109-11 (1999)). However, many patients develop tumors which are initially, or later become, resistant to taxol. To identify genes that may be involved with resistance to taxol, or are regulated in response to taxol resistance, and therefore may be used to treat, or identify, taxol resistance in patients, the following experiments were carried out.

The androgen-independent human cell line CWR22R was grown as a xenograft in nude mice (Nagabhushan et al., Cancer Res. 56(13):3042-3046 (1996); Agus et al., J. Natl. Cancer Inst.91(21):1869-1876 (1999); Bubendorf et al., J. Natl. Cancer Inst. 91(20):1758-1764 (1999)). Initially, these xenograft tumors were sensitive to therapeutic doses of taxol. The mice were treated continuously with sub-therapeutic doses, and the tumors were allowed to grow for 3-4 weeks, before surgical removal of the tumors. The tumor from an individual mouse was then minced, and a small portion was then injected into a healthy nude mouse, establishing a second passage of the tumor. This mouse was then treated continuously with the same sub-therapeutic dose of taxol. This process was repeated 14 times, and a portion of each generation of xenograft tumor was collected. There was increasing resistance to therapeutic doses of taxol with each generation. Bythe end of the process, the tumors were fully resistant to therapeutic doses of taxol. RNA from each generation of tumor was then isolated, and individual mRNA species were quantified using a custom Affymetrix GeneChip® oligonucleotide microarray, with probes to interrogate approximately 35,000 unique mRNA transcripts. Genes were selected that showed a statistically significant up-regulation, or down-regulation, during the subsequent generations of increasingly taxol-resistant tumors. Only one gene was significantly up-regulated, whereas 24 genes were down-regulated; these are presented in Table 10.

The gene sequences identified to be overexpressed in prostate cancer may be used to identify coding regions from the public DNA database. The sequences may be used to either identify genes that encode known proteins, or they may be used to predict the coding regions from genomic DNA using exon prediction algorithms, such as FGENESH (Salamov and Solovyev, 2000, Genome Res. 10:516-522). In addition, one of ordinary skill in the art would understand how to obtain the unigene cluster identification and sequence information according to the exemplar accession numbers provided in Tables 1-16. (see, http://www.ncbi.nlm.nih.gov/UniGene/).

**TABLE1: shows genes, including expression sequence tags, differentially expressed in prostate tumor tissue compared to normal tissue as analyzed using the Affymetrix/Eos Hu01 GeneChip array. Shown are the relative amounts of each gene expressed in prostate tumor samples and various normal tissue samples showing the highest expression of the gene.**

| | | | | |
|---|---|---|---|---|
| Pkey: | | Unique Eos probeset identifier number | | |
| ExAccn: | | Exemplar Accession number, Genbank accession number | | |
| UnigeneID: | | Unigene number | | |
| Unigene Title: | | Unigene gene title | | |
| R1: | | Ratio of tumor to normal body tissue | | |

| **Pkey** | **UnigeneID** | **ExAccn** | **Uningene Title** | **R1** |
|---|---|---|---|---|
| 131919 | Hs.272458 | AA121266 | ESTs | 37.2 |
| 120328 | Hs.290905 | AA196979 | ESTs; Weakly similar to (defline not ava | 32.6 |
| 105201 | Hs.31412 | AA195626 | ESTs | 30.1 |
| 101486 | Hs.1852 | M24902 | acid phosphatase; prostate | 25.2 |
| 119073 | Hs.279477 | R32894 | ESTs | 24.8 |
| 133428 | Hs.183752 | M34376 | microseminoprotein; beta- | 23.8 |
| 128180 | Hs.171995 | AA595348 | kallikrein 3; (prostate specific antigen | 21.4 |
| 104080 | Hs.57771 | AA402971 | Homo sapiens mRNA for serine protease (T | 18.9 |
| 127537 | Hs.162859 | AA569531 | ESTs | 18.6 |
| 131665 | Hs.30343 | R22139 | ESTs | 17.4 |
| 101050 | Hs.1832 | K01911 | neuropeptide Y | 17.3 |
| 130771 | Hs.1915 | N48056 | folate hydrolase (prostate-specific memb | 17 |
| 108153 | Hs.40808 | AA054237 | ESTs | 16.9 |
| 107485 | Hs.262476 | W63793 | S-adenosylmethionine decarboxylase 1 | 16.7 |
| 106155 | Hs.33287 | AA425309 | ESTs | 16.5 |
| 129534 | Hs.11260 | R73640 | ESTs | 16.4 |
| 100569 | Hs.171995 | HG2261-HT2351 | Antigen, Prostate Specific, Alt. Splice | 16 |
| 101889 | Hs.181350 | S39329 | kallikrein 2; prostatic | 15.4 |
| 135389 | Hs.99872 | U05237 | fetal Alzheimer antigen | 15 |
| 101506 | Hs.62192 | M27436 | coagulation factor III (thromboplastin; | 13.9 |
| 134374 | Hs.8236 | D62633 | ESTs | 12.7 |
| 133944 | Hs.7780 | AA045870 | ESTs | 12.5 |
| 109141 | Hs.193380 | AA176428 | ESTs | 12.3 |
| 130974 | Hs.2178 | X57985 | H2B histone family; member Q | 11.8 |
| 114768 | Hs.182339 | AA149007 | ESTs | 11.8 |
| 104394 | Hs.172129 | H46617 | yp19h1.r1 Soares breast 3NbHBst Homo sap | 11.8 |
| 125299 | Hs.102720 | Z39436 | ESTs | 11.6 |
| 104660 | Hs.14846 | AA007160 | ESTs | 11.4 |
| 100116 | Hs.78045 | D00654 | actin; gamma 2; smooth muscle; enteric | 11 |
| 131061 | Hs.268744 | N64328 | ESTs; Moderately similar to KIAA0273 [H. | 10.9 |
| 126645 | 126645 | AI167942 | Homo sapiens BAC clone RG041D11 from 7q2 | 10.7 |
| 135153 | Hs.95420 | N40141 | Homo sapiens mRNA for JM27 protein; comp | 10.6 |
| 107033 | Hs.113314 | AA599629 | ESTs | 10.6 |
| 118417 | | N66048 | ESTs; Weakly similar to polymerase [H.sa | 10.5 |
| 126758 | Hs.293960 | W37145 | ESTs | 10.2 |
| 115674 | Hs.8364 | AA406542 | ESTs | 10.1 |
| 134989 | Hs.92381 | AA236324 | ESTs; Weakly similar to !!!! ALU CLASS A | 10.1 |
| 107102 | Hs.30652 | AA609723 | ESTs | 10.1 |
| 116787 | Hs.15641 | H28581 | ESTs | 10.1 |
| 115719 | Hs.59622 | AA416997 | ESTs | 10 |
| 123209 | Hs.203270 | AA489711 | ESTs | 9.9 |
| 101664 | Hs.121017 | M60752 | H2A histone family; member A | 9.8 |
| 112971 | Hs.83883 | T17185 | ESTs | 9.7 |
| 102519 | Hs.80296 | U52969 | Purkinje cell protein 4 | 9.7 |
| 117984 | Hs.106778 | N51919 | ESTs | 9.7 |
| 105840 | Hs.22209 | AA398533 | ESTs | 9.4 |
| 129523 | Hs.274509 | M30894 | T-cell receptor; gamma cluster | 9.4 |
| 132964 | Hs.167133 | AA031360 | ESTs | 9.2 |
| 121853 | Hs.98502 | AA425887 | ESTs | 9 |
| 115764 | Hs.91011 | AA421562 | anterior gradient 2 (Xenopus laevis; sec | 8.9 |
| 119617 | Hs.55999 | W47380 | ESTs | 8.9 |
| 100552 | Hs.301946 | HG2167-HT2237 | Protein Kinase Ht31, Camp-Dependent | 8.9 |
| 105627 | Hs.23317 | AA281245 | ESTs | 8.8 |
| 101461 | Hs.76422 | M22430 | phospholipase A2; group IIA (platelets; | 8.7 |
| 131725 | Hs.31146 | AA456264 | ESTs; Highly similar to (defline not ava | 8.5 |
| 124526 | Hs.293185 | N62096 | yz61c5.s1 Soares_multiple_sclerosis_2NbH | 8.5 |
| 118528 | Hs.49397 | N67889 | ESTs | 8.2 |
| 133845 | Hs.76704 | T68510 | ESTs | 8.2 |
| 133354 | Hs.334762 | AA055552 | ESTs; Weakly similar to KIAA0319 [H.sapi | 8.1 |
| 105912 | Hs.20415 | AA402000 | ESTs; Weakly similar to GS3786 [H.sapien | 8 |
| 119018 | Hs.278695 | N95796 | ESTs | 8 |
| 100394 | Hs.66052 | D84276 | CD38 antigen (p45) | 8 |
| 114132 | Hs.24192 | Z38688 | ESTs | 7.9 |
| 116786 | Hs.301527 | H25836 | tumor necrosis factor (ligand) superfami | 7.7 |
| 106579 | Hs.23023 | AA456135 | ESTs | 7.6 |
| 128790 | Hs.105700 | AA291725 | secreted frizzled-related protein 4 | 7.5 |
| 114965 | Hs.72472 | AA250737 | ESTs | 7.4 |
| 112033 | Hs.22627 | R43162 | ESTs | 7.1 |
| 102398 | | U42359 | Human N33 protein form 1 (N33) gene, exo | 7 |
| 101201 | Hs.2256 | L22524 | matrix metalloproteinase 7 (matrilysin; | 6.9 |
| 109272 | Hs.288462 | AA195718 | ESTs | 6.9 |
| 103145 | Hs.169849 | X66276 | myosin-binding protein C; slow-type | 6.9 |
| 101803 | Hs.155691 | M86546 | pre-B-cell leukemia transcription factor | 6.8 |
| 120562 | Hs.302267 | AA280036 | ESTs; Weakly similar to W01A6.c [C.elega | 6.8 |
| 109112 | Hs.257924 | AA169379 | ESTs | 6.8 |
| 109795 | Hs.326416 | F10707 | ESTs | 6.7 |
| 107532 | Hs.173684 | Z19643 | ESTs; Weakly similar to (defline not ava | 6.7 |
| 130336 | Hs.171995 | X07730 | kallikrein 3; (prostate specific antigen | 6.6 |
| 131425 | Hs.26691 | AA219134 | ESTs | 6.6 |
| 120588 | Hs.16193 | AA281591 | Homo sapiens mRNA; cDNA DKFZp586B211 (fr | 6.6 |
| 132902 | Hs.59838 | AA490969 | ESTs | 6.6 |
| 125674 | Hs.323378 | W28078 | H.sapiens mRNA for transmembrane protein | 6.6 |
| 133724 | Hs.75746 | U07919 | aldehyde dehydrogenase 6 | 6.5 |
| 130343 | Hs.278628 | AA490262 | ESTs; Moderately similar to APXL gene pr | 6.5 |
| 120215 | Hs.108787 | Z41050 | Homo sapiens Mcd4p homolog mRNA; complet | 6.5 |
| 129215 | Hs.126085 | AA176867 | ESTs | 6.5 |
| 131881 | Hs.3383 | AA010163 | upstream regulatory element binding prot | 6.5 |
| 133376 | Hs.7232 | T23670 | ESTs | 6.4 |
| 105376 | Hs.8768 | AA236559 | ESTs; Weakly similar to neuronal thread | 6.4 |
| 104674 | Hs.26289 | AA009527 | ESTs | 6.4 |
| 100727 | Hs.334786 | X07290 | Human HF.12 gene mRNA | 6.3 |
| 130150 | Hs.15113 | AF000573 | homogentisate 1;2-dioxygenase (homogenti | 6.3 |
| 121770 | Hs.278428 | AA421714 | Homo sapiens mRNA for KIAA0896 protein; | 6.3 |
| 123475 | Hs.250528 | AA599267 | ESTs; Weakly similar to ANKYRIN; BRAIN V | 6.3 |
| 133061 | Hs.296638 | AB000584 | prostate differentiation factor | 6.3 |
| 116429 | Hs.279923 | AA609710 | ESTs; Weakly similar to similar to GTP-b | 6.2 |
| 101233 | Hs.878 | L29008 | sorbitol dehydrogenase | 6.2 |
| 104691 | Hs.37744 | AA011176 | ESTs | 6.2 |
| 127248 | | AA325029 | EST27953 Cerebellum II Homo sapiens cDNA | 6.2 |
| 127775 | Hs.179902 | H04106 | ESTs; Weakly similar to (defline not ava | 6.2 |
| 105500 | Hs.222399 | AA256485 | ESTs | 6.1 |
| 131463 | Hs.2714 | X74142 | forkhead (Drosophila)-like 1 | 6.1 |
| 132116 | Hs.40289 | AA234767 | ESTs | 6 |
| 130828 | Hs.203213 | AA053400 | ESTs | 5.9 |
| 115357 | Hs.72988 | AA281793 | ESTs | 5.8 |
| 105496 | Hs.301997 | AA256323 | ESTs | 5.7 |
| 116334 | Hs.48948 | AA491457 | ESTs | 5.7 |
| 107968 | Hs.61539 | AA034020 | ESTs | 5.7 |
| 120132 | Hs.125019 | Z38839 | ESTs; Weakly similar to !!!! ALU SUBFAMI | 5.6 |
| 106375 | Hs.289072 | AA443993 | ESTs | 5.6 |
| 132550 | Hs.170195 | AA029597 | bone morphogenetic protein 7 (osteogenic | 5.6 |
| 124777 | Hs.140237 | R41933 | ESTs; Weakly similar to neuronal thread | 5.6 |
| 100311 | Hs.337616 | D50640 | phosphodiesterase 3B; cGMP-inhibited | 5.6 |
| 101791 | Hs.62354 | M83822 | Human beige-like protein (BGL) mRNA; par | 5.5 |
| 117698 | Hs.45107 | N41002 | ESTs | 5.5 |
| 132387 | Hs.281434 | R70914 | heat shock 70kD protein 1 | 5.5 |
| 122041 | Hs.98732 | AA431407 | Homo sapiens Chromosome 16 BAC clone CIT | 5.5 |
| 133723 | Hs.262476 | AA088851 | S-adenosylmethionine decarboxylase 1 | 5.5 |
| 113938 | | W81598 | ESTs | 5.4 |
| 133015 | Hs.246315 | AA047036 | ESTs | 5.4 |
| 125745 | Hs.75722 | AI283493 | ribophorin II | 5.4 |
| 107295 | Hs.80120 | T34527 | UDP-N-acetyl-alpha-D-gatactosamine:polyp | 5.4 |
| 108186 | Hs.7780 | AA056482 | ESTs | 5.3 |
| 100184 | Hs.21223 | D17408 | calponin 1; basic; smooth muscle | 5.3 |
| 104466 | Hs.326392 | N25110 | Human guanine nucleotide exchange factor | 5.3 |
| 104033 | Hs.98944 | AA365031 | ESTs | 5.3 |
| 110844 | Hs.167531 | N31952 | ESTs; Weakly similar to (defline not ava | 5.3 |
| 129056 | Hs.108336 | H70627 | ESTs; Weakly similar to !!!! ALU SUBFAMI | 5.3 |
| 102805 | Hs.25351 | U90304 | iroquois-class homeodomain protein | 5.3 |
| 133493 | Hs.194369 | AA284143 | Homo sapiens chromosome 1 atrophin-1 rel | 5.3 |
| 129184 | Hs.109201 | W26769 | ESTs; Highly similar to (defline not ava | 5.2 |
| 134158 | Hs.79428 | U15174 | BCL2/adenovirus E1B 19kD-interacting pro | 5.2 |
| 107240 | Hs.159872 | D59368 | ESTs | 5.2 |
| 104787 | | AA027317 | ESTs; Weakly similar to !!!! ALU SUBFAMI | 5.2 |
| 123527 | Hs.108327 | AA608679 | damage-specific DNA binding protein 1 (1 | 5.2 |
| 116646 | Hs.194228 | F03048 | ESTs; Moderately similar to !!!! ALU SUB | 5.2 |
| 101448 | Hs.195850 | M21389 | keratin 5 (epidermolysis bullosa simplex | 5.1 |
| 116188 | Hs.184598 | AA464728 | ESTs; Weakly similar to !!!! ALU SUBFAMI | 5.1 |
| 126259 | Hs.281428 | Z21472 | ESTs; Moderately similar to !!!! ALU SUB | 5.1 |
| 105921 | Hs.169119 | AA402613 | ESTs | 5.1 |
| 103375 | Hs.54416 | X91868 | sine oculis homeobox (Drosophila) homolo | 5.1 |
| 128871 | Hs.106778 | AA400271 | ESTs; Highly similar to (defline not ava | 5.1 |
| 112681 | Hs.148932 | R87331 | ESTs; Moderately similar to semaphorin V | 5.1 |
| 105784 | Hs.226434 | AA350771 | ESTs | 5.1 |
| 116238 | Hs.47144 | AA479362 | ESTs | 5 |
| 102913 | Hs.80342 | X07696 | keratin 15 | 5 |
| 103011 | Hs.326035 | X52541 | early growth response 1 | 5 |
| 126023 | | H58881 | yr36d09.r1 Soares fetal liver spleen 1NF | 5 |
| 103709 | Hs.13804 | AA037316 | ESTs | 5 |
| 118981 | Hs.39288 | N93839 | ESTs; Weakly similar to !!!! ALU SUBFAMI | 5 |
| 134807 | Hs.89732 | X78932 | zinc finger protein 273 | 5 |
| 100079 | Hs.23311 | AB002365 | Human mRNA for KIAA0367 gene; partial cd | 4.9 |
| 132047 | Hs.3796 | D83492 | EphB6 | 4.9 |
| 132880 | Hs.177537 | AA444369 | ESTs | 4.9 |
| 124049 | Hs.74519 | F10523 | primase; polypeptide 2A (58kD) | 4.8 |
| 133330 | Hs.71119 | U42360 | Human N33 mRNA; complete cds | 4.8 |
| 104776 | | AA026349 | ESTs | 4.8 |
| 122593 | Hs.128749 | AA453310 | Homo sapiens alpha-methylacyl-CoA racema | 4.8 |
| 103912 | Hs.143087 | AA251078 | ESTs | 4.8 |
| 113961 | Hs.26009 | W86307 | Homo sapiens mRNA for KIAA0860 protein; | 4.8 |
| 105288 | Hs.3585 | AA233168 | ESTs; Weakly similar to coded for by C. | 4.8 |
| 135035 | Hs.284186 | H89575 | ESTs | 4.8 |
| 104144 | Hs.183390 | AA447439 | ESTs; Weakly similar to ZINC FINGER PROT | 4.8 |
| 129389 | Hs.288126 | AA621604 | ESTs | 4.8 |
| 125982 | | R98091 | RAE1 (RNA export 1; S.pombe) homolog | 4.8 |
| 125162 | Hs.26243 | W44682 | ESTs | 4.8 |
| 103023 | Hs.117950 | X53793 | multifunctional polypeptide similar to S | 4.7 |
| 129735 | | W80701 | ESTs; Weakly similar to HERV-E envelope | 4.7 |
| 104479 | Hs.106390 | N36040 | ESTs | 4.7 |
| 103731 | | AA070545 | zm7c3.r1 Stratagene neuroepithelium (#93 | 4.7 |
| 126575 | Hs.127602 | W72416 | ESTs | 4.7 |
| 124578 | Hs.231500 | N68321 | Human glucose transporter-like protein-l | 4.7 |
| 130617 | Hs.1674 | M90516 | glutamine-fructose-6-phosphate transamin | 4.7 |
| 116752 | Hs.91622 | H06373 | Homo sapiens clone 24456 mRNA sequence | 4.7 |
| 100279 | Hs.82007 | D42084 | Human mRNA for KIAA0094 gene; partial cd | 4.7 |
| 126288 | Hs.89576 | AI479264 | ESTs | 4.7 |
| 131836 | Hs.32990 | AA610086 | ESTs | 4.7 |
| 106717 | Hs.239489 | AA465093 | TIA1 cytotoxic granule-associated RNA-bi | 4.7 |
| 114542 | Hs.91011 | AA055768 | ESTs | 4.6 |
| 103806 | | AA130614 | zo1f2.r1 Stratagene neuroepithelium NT2R | 4.6 |
| 130529 | | AA173238 | small inducible cytokine A5 (RANTES) | 4.6 |
| 115675 | Hs.82065 | AA406546 | ESTs | 4.6 |
| 111386 | Hs.293798 | N95326 | ESTs | 4.6 |
| 106503 | Hs.29679 | AA452411 | ESTs | 4.6 |
| 119943 | Hs.14158 | W86835 | copine III | 4.6 |
| 104459 | Hs.100070 | M91493 | EST | 4.6 |
| 100774 | Hs.89603 | HG371-HT1063 | Mucin 1, Epithelial, Alt. Splice 6 | 4.6 |
| 100652 | Hs.142653 | HG2825-HT2949 | Ret Transforming Gene | 4.6 |
| 132015 | Hs.3731 | D11900 | ESTs | 4.6 |
| 126086 | | H70975 | yr73g01.r1 Soares fetal liver spleen 1NF | 4.6 |
| 130888 | Hs.173094 | F03819 | ESTs | 4.6 |
| 106390 | Hs.20166 | AA446964 | Prostate stem cell antigen | 4.6 |
| 126959 | | AA199853 | ESTs; Moderately similar to !!!! ALU SUB | 4.5 |
| 131584 | Hs.29117 | X91648 | H.sapiens mRNA for pur alpha extended 3' | 4.5 |
| 104838 | Hs.20953 | AA039481 | ESTs | 4.5 |
| 125661 | | R50319 | ESTs | 4.5 |
| 103171 | Hs.234726 | X68733 | alpha-1-antichymotrypsin | 4.5 |
| 103928 | Hs.199160 | AA280085 | ESTs | 4.5 |
| 102899 | Hs.75730 | X06272 | signal recognition particle receptor (d | 4.5 |
| 100892 | Hs.180789 | HG4557-HT4962 | Small Nuclear Ribonucleoprotein U1, 1snr | 4.5 |
| 106167 | Hs.7956 | AA425906 | ESTs | 4.5 |
| 129404 | Hs.317584 | AA172056 | ESTs | 4.5 |
| 106990 | Hs.24758 | AA521354 | ESTs | 4.5 |
| 132316 | Hs.44566 | U28831 | Human protein immuno-reactive with anti- | 4.4 |
| 132056 | Hs.38176 | T89386 | Homo sapiens mRNA for KIAA0606 protein; | 4.4 |
| 133718 | Hs.198760 | X15306 | neurofilament; heavy polypeptide (200kD) | 4.4 |
| 101470 | Hs.1846 | M22898 | tumor protein p53 (Li-Fraumenl syndrome) | 4.4 |
| 131904 | Hs.284296 | AA143019 | ESTs; Highly similar to surface 4 integr | 4.4 |
| 105804 | Hs.22514 | AA383142 | ESTs | 4.4 |
| 122861 | Hs.119394 | AA464428 | ESTs | 4.4 |
| 111336 | Hs.29894 | N79565 | ESTs | 4.4 |
| 121944 | Hs.98518 | AA429278 | ESTs | 4.4 |
| 134401 | Hs.211577 | AA243746 | ESTs; Highly similar to CG1 protein [H.s | 4.4 |
| 126458 | Hs.288969 | AA815252 | ESTs; Weakly similar to !!!! ALU SUBFAMI | 4.4 |
| 133435 | Hs.323966 | T23983 | ESTs; Moderately similar to !!!! ALU SUB | 4.4 |
| 105178 | Hs.21941 | AA187490 | ESTs | 4.3 |
| 127315 | | AA640834 | nr27b06.r1 NCl_CGAP_Pr3 Homo sapiens cDN | 4.3 |
| 132645 | Hs.54424 | X87870 | H.sapiens mRNA for hepatocyte nuclear fa | 4.3 |
| 116162 | Hs.282990 | AA461487 | ESTs; Weakly similar to F52C12.2 [C.eleg | 4.3 |
| 118040 | Hs.47567 | N52876 | EST | 4.3 |
| 130008 | Hs.278427 | M31423 | cerebellar degeneration-related protein | 4.3 |
| 126607 | Hs.114688 | W87424 | ESTs | 4.3 |
| 123061 | Hs.105130 | AA482030 | EST | 4.3 |
| 109391 | Hs.184245 | AA219699 | ESTs | 4.3 |
| 109175 | | AA180496 | ESTs | 4.3 |
| 127003 | Hs.173540 | AA550806 | ESTs; Weakly similar to (defline not ava | 4.3 |
| 102547 | Hs.46638 | U57911 | chromosome 11 open reading frame 8 | 4.3 |
| 134208 | Hs.79993 | U88871 | peroxisomal biogenesis factor 7 | 4.3 |
| 104258 | Hs.5462 | AF007216 | solute carrier family 4; sodium bicarbon | 4.3 |
| 130759 | Hs.18946 | AA094720 | ESTs; Weakly similar to (defline not ava | 4.3 |
| 132160 | Hs.295923 | AA281770 | seven in absentia (Drosophila) homolog 1 | 4.3 |
| 135062 | Hs.93872 | AA174183 | ESTs | 4.3 |
| 126510 | Hs.334762 | R49702 | ESTs; Weakly similar to KIAA0319 [H.sapi | 4.2 |
| 122055 | Hs.98747 | AA431732 | EST | 4.2 |
| 133136 | Hs.6574 | AF007165 | suppressin (nuclear deformed epidermal a | 4.2 |
| 109890 | Hs.20843 | H04649 | ESTs | 4.2 |
| 133294 | Hs.69997 | R79723 | H.sapiens mRNA for translin associated z | 4.2 |
| 134436 | Hs.83190 | S80437 | fatty acid synthase (3' region) [human, | 4.2 |
| 107375 | Hs.251064 | U88573 | NBR2 | 4.2 |
| 122223 | Hs.27413 | AA436158 | ESTs | 4.2 |
| 103044 | Hs.248210 | X55777 | H.sapiens Mahlavu hepatocellular carcino | 4.2 |
| 120125 | Hs.59815 | W99362 | EST | 4.2 |
| 128969 | Hs.283978 | T65327 | ESTs; Highly similar to (defline not ava | 4.2 |
| 129637 | Hs.1179 | D90359 | TATA box binding protein (TBP)-associate | 4.2 |
| 106566 | | AA455921 | ESTs; Weakly similar to !!!! ALU SUBFAMI | 4.2 |
| 112605 | Hs.29852 | R79220 | ESTs | 4.2 |
| 103364 | Hs.279929 | X90872 | H.sapiens mRNA for gp25L2 protein | 4.2 |
| 132811 | Hs.57419 | U25435 | transcriptional repressor | 4.2 |
| 126570 | Hs.326292 | T79274 | ESTs | 4.2 |
| 116298 | Hs.94109 | AA489046 | ESTs | 4.2 |
| 103024 | Hs.105938 | X53961 | lactotransferrin | 4.1 |
| 129133 | Hs.108850 | R56728 | yg95c6.r1 Soares infant brain 1NIB Homo | 4.1 |
| 133167 | Hs.6641 | N98707 | kinesin family member 5C | 4.1 |
| 126871 | Hs.14051 | AA351779 | ESTs | 4.1 |
| 132333 | Hs.45032 | AA192157 | ESTs | 4.1 |
| 107376 | Hs.327179 | U90545 | solute carrier family 17 (sodium phospha | 4.1 |
| 128517 | Hs.100861 | AA280617 | ESTs; Weakly similar to p60 katanin [H.s | 4.1 |
| 130555 | Hs.116774 | AA450324 | ESTs | 4.1 |
| 105765 | Hs.24183 | AA343514 | ESTs | 4.1 |
| 126529 | Hs.26369 | AA133237 | ESTs | 4.1 |
| 125928 | Hs.181889 | H29730 | ESTs | 4.1 |
| 117280 | Hs.172129 | N22107 | ESTs; Moderately similar to !!!! ALU SUB | 4.1 |
| 100234 | Hs.3085 | D29677 | KIAA0054 gene product | 4.1 |
| 100959 | Hs.118127 | J00073 | actin; alpha; cardiac muscle | 4.1 |
| 107130 | Hs.12913 | AA620582 | ESTs; Weakly similar to (defline not ava | 4.1 |
| 105035 | Hs.8859 | AA128486 | ESTs | 4.1 |
| 126735 | Hs.226795 | AA808949 | glutathione S-transferase pi | 4.1 |
| 113056 | Hs.8036 | T26471 | ESTs; Moderately similar to !!!! ALU SUB | 4 |
| 102460 | Hs.211582 | U48959 | Homo sapiens myosin light chain kinase ( | 4 |
| 106968 | Hs.26813 | AA504631 | ESTs; Weakly similar to (defline not ava | 4 |
| 123107 | Hs.104207 | AA486071 | ESTs | 4 |
| 127256 | Hs267967 | AA327550 | ESTs; Weakly similar to !!!! ALU SUBFAMI | 4 |
| 105329 | Hs.22862 | AA234561 | ESTs | 4 |
| 115504 | Hs.42736 | AA291946 | ESTs | 4 |
| 120726 | Hs.97293 | AA293656 | ESTs | 4 |
| 103576 | Hs.94560 | Z26317 | desmoglein 2 | 4 |
| 127889 | Hs.144941 | AI147408 | ESTs | 4 |
| 106394 | Hs.25320 | AA447223 | ESTs | 4 |
| 128046 | | AA873285 | ESTs | 4 |
| 103391 | Hs.114366 | X94453 | pyrroline-5-carboxylate synthetase (glut | 4 |
| 106448 | Hs.27004 | AA449455 | ESTs | 4 |
| 126513 | Hs.86276 | W27601 | ESTs; Moderately similar to (defline not | 4 |
| 129593 | Hs.98314 | AA487015 | ESTs; Weakly similar to !!!! ALU SUBFAMI | 3.9 |
| 110151 | Hs.31608 | H18836 | ESTs | 3.9 |
| 105344 | Hs.8645 | AA235303 | ESTs | 3.9 |
| 104791 | Hs.301871 | AA029046 | ESTs | 3.9 |
| 123442 | Hs.111496 | AA598803 | ESTs | 3.9 |
| 127800 | Hs.79428 | AA521047 | BCL2/adenovirus E1B 19kD-interacting pro | 3.9 |
| 114555 | Hs.167904 | AA058594 | ESTs | 3.9 |
| 122138 | Hs.163960 | AA435549 | ESTs | 3.9 |
| 129565 | Hs.198726 | X77777 | vasoactive intestinal peptide receptor 1 | 3.9 |
| 103471 | Hs.75216 | Y00815 | protein tyrosine phosphatase; receptor t | 3.9 |
| 133908 | Hs.325474 | M83216 | caldesmon 1 | 3.9 |
| 105635 | Hs.301985 | AA281508 | ESTs | 3.9 |
| 134285 | Hs.81086 | AA460012 | solute carrier family 22 (organic cation | 3.9 |
| 134125 | Hs.50421 | R38102 | KIAA0203 gene product | 3.9 |
| 125628 | Hs.241493 | AA418069 | natural killer-tumor recognition sequenc | 3.9 |
| 103695 | Hs.186600 | AA018758 | ESTs | 3.9 |
| 100642 | Hs.182183 | HG2743-HT3926 | Caldesmon 1, Alt. Splice 6, Non-Muscle | 3.9 |
| 104334 | Hs.78771 | D82614 | ESTs | 3.9 |
| 110242 | Hs.19978 | H26417 | ESTs | 3.9 |
| 125298 | Hs.289008 | Z39255 | ESTs | 3.9 |
| 104060 | Hs.303193 | AA397968 | zt87a9.r1 Soares_testis_NHT Homo sapiens | 3.9 |
| 105823 | Hs.293960 | AA398197 | ESTs | 3.9 |
| 126499 | Hs.110445 | AA315671 | ESTs; Moderately similar to unknown [M.m | 3.9 |
| 130752 | Hs.18895 | D50927 | KIAA0137 gene product | 3.8 |
| 123494 | Hs.112110 | AA599786 | ESTs | 3.8 |
| 104846 | Hs.32478 | AA040154 | ESTs | 3.8 |
| 108921 | Hs.71721 | AA142913 | ESTs | 3.8 |
| 115506 | Hs.45207 | AA292537 | ESTs | 3.8 |
| 100452 | Hs.241552 | D87742 | Human mRNA for KIAA0268 gene; partial cd | 3.8 |
| 104454 | Hs.129228 | M84443 | galactokinase 2 | 3.8 |
| 108730 | Hs.102859 | AA126254 | ESTs | 3.8 |
| 131223 | Hs.24427 | AA247788 | ESTs; Highly similar to (defline not ava | 3.8 |
| 104784 | Hs.269228 | AA027055 | ESTs | 3.8 |
| 104946 | Hs.73848 | AA069549 | ESTs | 3.8 |
| 106932 | Hs.9394 | AA495926 | ESTs | 3.8 |
| 101724 | Hs.620 | M69225 | bullous pemphigoid antigen 1 (230/240kD) | 3.8 |
| 106140 | Hs.14912 | AA424524 | Homo sapiens mRNA for KIAA0286 gene; par | 3.8 |
| 128135 | Hs.269721 | AA913491 | ESTs | 3.8 |
| 120030 | Hs.58694 | W92051 | ESTs | 3.8 |
| 126457 | Hs.50382 | AA007489 | zh98g04.r1 Soares_fetal_liver_spleen_1NF | 3.8 |
| 123917 | Hs.112969 | AA621311 | EST | 3.7 |
| 110714 | Hs.17752 | H95978 | Homo sapiens phosphatidylserine-specific | 3.7 |
| 130577 | Hs.162 | M35410 | insulin-like growth factor binding prote | 3.7 |
| 117667 | Hs.44708 | N39214 | ser-Thr protein kinase related to the my | 3.7 |
| 126104 | Hs.39712 | N77278 | ESTs; Weakly similar to BONE/CARTILAGE P | 3.7 |
| 100379 | Hs.278721 | D82060 | Homo sapiens mRNA for membrane protein w | 3.7 |
| 115646 | Hs.305971 | AA404352 | ESTs | 3.7 |
| 125792 | Hs.193700 | AI005388 | ESTs; Moderately similar to !!!! ALU SUB | 3.7 |
| 102162 | Hs.1592 | U18291 | CDC16 (cell division cycle 16; S. cerevi | 3.7 |
| 128530 | Hs.183475 | AA504343 | ESTs; Moderately similar to !!!! ALU SUB | 3.7 |
| 119940 | Hs.272531 | W86779 | EST | 3.7 |
| 110769 | Hs.23837 | N22222 | yw34b06.s1 Morton Fetal Cochlea Homo sap | 3.7 |
| 132914 | Hs.60293 | AA496037 | ESTs | 3.7 |
| 113594 | Hs.15683 | T92030 | ESTs | 3.7 |
| 103702 | Hs.279952 | AA027793 | ESTs; Highly similar to (defline not ava | 3.7 |
| 130780 | Hs.19347 | AA248406 | ESTs | 3.7 |
| 123288 | Hs.291025 | AA495836 | EST | 3.7 |
| 120691 | Hs.22380 | AA291173 | ESTs | 3.7 |
| 103153 | Hs.75295 | X66534 | guanylate cyclase 1; soluble; alpha 3 | 3.7 |
| 129201 | Hs.109390 | H19969 | ESTs | 3.7 |
| 114798 | Hs.54900 | AA159181 | ESTs | 3.7 |
| 126801 | Hs.7337 | AA512902 | ESTs | 3.7 |
| 105503 | Hs.31707 | AA256616 | ESTs | 3.7 |
| 104260 | Hs.194283 | AF008192 | Homo sapiens putative GR6 protein (GR6) | 3.7 |
| 125930 | Hs.35699 | R97219 | ESTs | 3.7 |
| 123255 | Hs.105273 | AA490890 | ESTs | 3.6 |
| 103862 | Hs.6363 | AA206625 | ESTs | 3.6 |
| 100696 | Hs.121686 | HG3162-HT3339 | Transcription Factor lia | 3.6 |
| 134917 | Hs.166994 | X87241 | FAT tumor suppressor (Drosophila) homolo | 3.6 |
| 103520 | | Y10511 | H.sapiens mRNA for CD176 protein | 3.6 |
| 113778 | Hs.302738 | W15263 | ESTs | 3.6 |
| 101838 | Hs.75511 | M92934 | connective tissue growth factor | 3.6 |
| 113702 | | T97307 | ESTs; Moderately similar to !!!! ALU SUB | 3.6 |
| 118201 | Hs.48428 | N59800 | EST | 3.6 |
| 116519 | Hs.68554 | C20780 | EST | 3.6 |
| 105886 | Hs.22983 | AA400517 | ESTs; Moderately similar to UDP-GLUCOSE: | 3.6 |
| 106709 | Hs.170291 | AA464696 | ESTs | 3.6 |
| 127858 | Hs.27973 | AA806365 | oc26h07.s1 NCI_CGAP_GCB1 Homo sapiens cD | 3.6 |
| 101964 | | S81578 | dioxin-responsive gene {putative polyade | 3.6 |
| 105508 | Hs.326416 | AA256680 | ESTs | 3.6 |
| 116844 | Hs.337434 | H64938 | ESTs | 3.6 |
| 105372 | Hs.142296 | AA236481 | ESTs | 3.6 |
| 100745 | Hs.144630 | HG3510-HT3704 | V-Erba Related Ear-3 Protein | 3.6 |
| 127521 | Hs.164018 | AA809982 | ESTs | 3.6 |
| 110758 | Hs.274265 | N21385 | talin | 3.6 |
| 107307 | Hs.44155 | T52099 | creatine kinase; mitochondrial 2 (sarcom | 3.6 |
| 133200 | Hs.183639 | AA432248 | ESTs | 3.6 |
| 114774 | Hs.184325 | AA150043 | ESTs | 3.6 |
| 120265 | Hs.270696 | AA173759 | ESTs; Moderately similar to !!!! ALU SUB | 3.6 |
| 134359 | Hs.199067 | M34309 | v-erb-b2 avian erythroblastic leukemia v | 3.6 |
| 116250 | Hs.44829 | AA480975 | ESTs; Moderately similar to !!!! ALU SUB | 3.6 |
| 106313 | Hs.35841 | AA436459 | nuclear factor I/X (CCAAT-binding transc | 3.6 |
| 131898 | Hs.279780 | N52232 | ESTs | 3.6 |
| 133444 | Hs.73793 | M27281 | vascular endothelial growth factor | 3.6 |
| 128232 | Hs.334641 | H06296 | ESTs | 3.6 |
| 135357 | Hs.79572 | AA235803 | ESTs | 3.5 |
| 457951 | | AI369384 | arylsulfatase D | 3.5 |
| 108407 | | AA075519 | zm87h9.s1 Stratagene ovarian cancer (#93 | 3.5 |
| 126659 | | T16245 | a disintegrin and metalloproteinase doma | 3.5 |
| 104189 | Hs.301804 | AA485805 | ESTs | 3.5 |
| 125956 | Hs.129014 | N53276 | ESTs | 3.5 |
| 103026 | Hs.79386 | X54162 | Human mRNA for a 64 Kd autoantigen expre | 3.5 |
| 133011 | Hs.171921 | AA042990 | sema domain; immunoglobulin domain (Ig); | 3.5 |
| 131379 | Hs.26176 | R49035 | ESTs | 3.5 |
| 126742 | Hs.169359 | H64106 | yr57e06.r1 Soares fetal liver spleen 1NF | 3.5 |
| 105560 | Hs.306915 | AA262783 | ESTs | 3.5 |
| 118472 | Hs.42179 | N66818 | ESTs | 3.5 |
| 105623 | Hs.30127 | AA280895 | ESTs; Highly similar to !!!! ALU SUBFAMI | 3.5 |
| 120262 | Hs.145807 | AA172076 | ESTs; Moderately similar to !!!! ALU SUB | 3.5 |
| 105027 | Hs.26771 | AA126472 | ESTs | 3.5 |
| 130760 | Hs.18953 | AA128997 | phosphodiesterase 9A | 3.5 |
| 117473 | Hs.155560 | N30157 | ESTs | 3.5 |
| 102663 | Hs.168075 | U70322 | karyopherin (importin) beta 2 | 3.5 |
| 126349 | Hs.13531 | AA442868 | ESTs; Weakly similar to (defline not ava | 3.5 |
| 132154 | Hs.41119 | N67179 | ESTs | 3.5 |
| 131689 | Hs.30696 | AA599653 | transcription factor-like 5 (basic helix | 3.5 |
| 127862 | Hs.163191 | AA765305 | EST | 3.5 |
| 126995 | Hs.189810 | W26950 | Human DNA sequence from PAC 388M5 on chr | 3.5 |
| 119071 | | R31180 | ESTs | 3.5 |
| 103941 | Hs.96593 | AA282978 | ESTs | 3.5 |
| 110721 | Hs.31319 | H97678 | ESTs | 3.5 |
| 126586 | Hs.43086 | AA011247 | ESTs | 3.5 |
| 103106 | Hs.1857 | X62025 | phosphodiesterase 6G; cGMP-specific; rod | 3.5 |
| 116357 | Hs.90797 | AA504806 | Homo sapiens clone 23620 mRNA sequence | 3.5 |
| 105309 | Hs.4104 | AA233790 | ESTs | 3.5 |
| 130796 | Hs.19525 | R39390 | ESTs | 3.5 |
| 109101 | Hs.52184 | AA167708 | ESTs | 3.5 |
| 103134 | Hs.2839 | X65724 | Norrie disease (pseudoglioma) | 3.5 |
| 131798 | Hs.301449 | X86098 | adenovirus 5 E1A binding protein | 3.5 |
| 118535 | Hs.49418 | N67968 | ESTs | 3.5 |
| 102592 | Hs.11223 | U62389 | Human putative cytosolic NADP-dependent | 3.4 |
| 125905 | Hs.6456 | T69868 | chaperonin containing TCP1; subunit 2 (b | 3.4 |
| 109160 | Hs.301997 | AA179387 | ESTs | 3.4 |
| 105327 | Hs.211593 | AA234440 | ESTs | 3.4 |
| 106586 | Hs.57787 | AA456598 | ESTs | 3.4 |
| 122635 | | AA454085 | EST | 3.4 |
| 132413 | Hs.260116 | AA132969 | metalloprotease 1 (pitrilysin family) | 3.4 |
| 131938 | Hs.34956 | AA283620 | ESTs | 3.4 |
| 133871 | Hs.182793 | AA454597 | ESTs | 3.4 |
| 107175 | Hs.292503 | AA621751 | ESTs; Weakly similar to KIAA0601 protein | 3.4 |
| 101188 | Hs.184298 | L20320 | cyclin-dependent kinase 7 (homolog of Xe | 3.4 |
| 126422 | Hs.237658 | H48518 | ESTs; Highly similar to apolipoprotein A | 3.4 |
| 118475 | | N66845 | ESTs; Weakly similar to !!!! ALU CLASS B | 3.4 |
| 104558 | Hs.88959 | R56678 | ESTs; Weakly similar to !!!! ALU SUBFAMI | 3.4 |
| 128307 | Hs.132005 | AI453794 | ESTs | 3.4 |
| 112254 | Hs.25829 | R51831 | ESTs | 3.4 |
| 125408 | Hs.89578 | N72353 | yv37e12.r1 Soares fetal liver spleen 1NF | 3.4 |
| 109834 | Hs.175955 | H00604 | ESTs | 3.4 |
| 130844 | Hs.20191 | D12122 | seven in absentia (Drosophila) homolog 2 | 3.4 |
| 127143 | Hs.20843 | AA533553 | nj68h04.s1 NCI_CGAP_Pr10 Homo sapiens cD | 3.4 |
| 135309 | Hs.42500 | D25984 | ESTs | 3.4 |
| 125724 | Hs.295978 | AA083407 | stimulated trans-acting factor (50 kDa) | 3.4 |
| 127692 | Hs.187983 | AI021912 | ESTs | 3.4 |
| 116674 | Hs.92127 | F04816 | ESTs | 3.4 |
| 134700 | Hs.8868 | AA481414 | golgi SNAP receptor complex member 1 | 3.4 |
| 114846 | Hs.166196 | AA234929 | ESTs | 3.4 |
| 103649 | Hs.155983 | Z70219 | H.sapiens mRNA for 5'UTR for unknown pro | 3.4 |
| 134835 | Hs.89925 | L04569 | calcium channel; voltage-dependent; L ty | 3.4 |
| 130568 | Hs.16085 | AA232535 | ESTs; Highly similar to (defline not ava | 3.4 |
| 111331 | Hs.15978 | N78773 | ESTs | 3.4 |
| 106036 | Hs.10653 | AA412505 | ESTs | 3.4 |
| 130987 | Hs.21893 | R45698 | ESTs | 3.4 |
| 112814 | Hs.35828 | R98192 | ESTs | 3.4 |
| 127815 | Hs.255015 | AA876009 | ob93c10.s1 NCI_CGAP_GCB1 Homo sapiens cD | 3.4 |
| 100144 | Hs.75616 | D13643 | KIAA0018 gene product | 3.4 |
| 101129 | Hs.247992 | L10405 | Homo sapiens DNA binding protein for sur | 3.4 |
| 130874 | Hs.20621 | T08287 | ESTs | 3.4 |
| 106882 | Hs.26994 | AA489009 | ESTs | 3.4 |
| 103855 | Hs.302267 | AA195179 | ESTs | 3.4 |
| 125957 | | H45213 | yo03b08.r1 Soares adult brain N2b5HB55Y | 3.3 |
| 114048 | Hs.146085 | W94613 | ESTs | 3.3 |
| 109826 | Hs.75354 | F13702 | ESTs | 3.3 |
| 125355 | Hs.170098 | R45630 | ESTs; Highly similar to KIAA0372 [H.sapi | 3.3 |
| 104182 | Hs.143792 | AA479990 | ESTs; Weakly similar to glioma amplified | 3.3 |
| 100294 | Hs.75454 | D49396 | Human mRNA for Apo1_Human (MER5(Aop1-Mou | 3.3 |
| 131688 | Hs.30692 | U24153 | p21 (CDKN1A)-activated kinase 2 | 3.3 |
| 116256 | Hs.88201 | AA481256 | ESTs; Weakly similar to (defline not ava | 3.3 |
| 102034 | Hs.230 | U05291 | fibromodulin | 3.3 |
| 130072 | Hs.14658 | R99606 | Human chromosome 5q13.1 clone 5G8 mRNA | 3.3 |
| 114615 | Hs.159456 | AA083812 | ESTs; Highly similar to (defline not ava | 3.3 |
| 128707 | Hs.104105 | AA136474 | Meis (mouse) homolog 2 | 3.3 |
| 115048 | Hs.190057 | AA252668 | ESTs | 3.3 |
| 125862 | Hs.31110 | H12084 | ESTs | 3.3 |
| 135142 | Hs.24192 | R31679 | ESTs | 3.3 |
| 103119 | Hs.2877 | X63629 | cadherin 3; P-cadherin (placental) | 3.3 |
| 104460 | Hs.62604 | M91504 | ESTs | 3.3 |
| 100365 | Hs.79284 | D78611 | mesoderm specific transcript (mouse) hom | 3.3 |
| 131524 | Hs.301804 | N39152 | ESTs | 3.3 |
| 102165 | Hs.159627 | U18321 | Death associated protein 3 | 3.3 |
| 126966 | Hs.182575 | R38438 | solute carrier family 15 (H+/peptide tra | 3.3 |
| 124839 | Hs.140942 | R55784 | ESTs | 3.3 |
| 100709 | Hs.100469 | HG3264-HT3441 | Af-6 (Gb:U02478) | 3.3 |
| 132967 | Hs.61635 | AA032221 | Homo sapiens BAC clone RG041D11 from 7q2 | 3.3 |
| 102927 | Hs.65114 | X12876 | keratin 18 | 3.3 |
| 132616 | Hs.283558 | AA386264 | ESTs | 3.3 |
| 125132 | Hs.129781 | W15495 | ESTs | 3.3 |
| 111225 | Hs.31652 | N68989 | ESTs | 3.3 |
| 114956 | Hs.87113 | AA243681 | ESTs | 3.3 |
| 122235 | Hs.112227 | AA436475 | ESTs | 3.3 |
| 112325 | Hs.12315 | R56055 | ESTs | 3.3 |
| 123360 | Hs.178604 | AA504784 | ESTs | 3.3 |
| 105150 | Hs.155995 | AA169640 | Homo sapiens mRNA for KIAA0643 protein; | 3.3 |
| 107391 | Hs.284294 | W02877 | ESTs | 3.3 |
| 113058 | Hs.7569 | T26893 | EST | 3.3 |
| 134371 | Hs.82318 | S69790 | Brush-1 | 3.3 |
| 125669 | Hs.333256 | R51308 | ESTs; Moderately similar to !!!! ALU SUB | 3.3 |
| 111506 | Hs.294105 | R07726 | ESTs | 3.3 |
| 122974 | Hs.194215 | AA478625 | ESTs | 3.3 |
| 102369 | Hs.299867 | U39840 | hepatocyte nuclear factor 3; alpha | 3.3 |
| 120408 | Hs.190151 | AA235045 | ESTs | 3.3 |
| 117993 | Hs.47402 | N52039 | ESTs; Weakly similar to !!!! ALU SUBFAMI | 3.3 |
| 129586 | Hs.11500 | AA437118 | ESTs | 3.3 |
| 128138 | Hs.126494 | AI200825 | ESTs | 3.3 |
| 127265 | | AA332751 | EST37214 Embryo, 8 week I Homo sapiens c | 3.3 |
| 107674 | Hs.41143 | AA011027 | Homo sapiens mRNA for KIAA0581 protein, | 3.2 |
| 104866 | Hs.293691 | AA045342 | ESTs | 3.2 |
| 103427 | Hs.250655 | X97303 | H.sapiens mRNA for Ptg-12 protein | 3.2 |
| 132990 | Hs.334334 | AA458761 | ESTs | 3.2 |
| 127017 | Hs.251946 | AA740146 | ESTs | 3.2 |
| 132313 | Hs.44481 | U13220 | forkhead (Drosophila)-like 6 | 3.2 |
| 106880 | Hs.32425 | AA488889 | ESTs | 3.2 |
| 107039 | Hs.169780 | AA599751 | homologous to yeast nitrogen permease (c | 3.2 |
| 120870 | Hs.292581 | AA357172 | ESTs | 3.2 |
| 107920 | Hs.284207 | AA027951 | ESTs | 3.2 |
| 104165 | Hs.105116 | AA459160 | EST | 3.2 |
| 107012 | Hs.63908 | AA598745 | ESTs | 3.2 |
| 103605 | Hs.194657 | Z35402 | H.sapiens gene encoding E-cadherin, exon | 3.2 |
| 124006 | Hs.270016 | D60302 | ESTs | 3.2 |
| 101300 | Hs.74137 | L40391 | Homo sapiens (clone s153) mRNA fragment | 3.2 |
| 101183 | Hs.795 | L19779 | H2A histone family; member O | 3.2 |
| 125596 | | R25698 | yg44h11.r2 Soares infant brain 1NIB Homo | 3.2 |
| 127261 | | AA661567 | nu86b02.s1 NCI_CGAP_Alv1 Homo sapiens cD | 3.2 |
| 120090 | Hs.59554 | W94591 | ESTs | 3.2 |
| 129393 | Hs.166982 | D13435 | phosphatidylinositol glycan; class F | 3.2 |
| 120923 | Hs.97129 | AA382283 | ESTs | 3.2 |
| 118907 | Hs.274256 | N91003 | ESTs | 3.2 |
| 111552 | Hs.191185 | R09411 | ESTs | 3.2 |
| 104431 | Hs.99913 | J03019 | adrenergic; beta-1-; receptor | 3.2 |
| 133551 | Hs.278634 | D63480 | Human mRNA for KIAA0146 gene; partial cd | 3.2 |
| 131615 | Hs.192803 | D14533 | xeroderma pigmentosum; complementation g | 3.2 |
| 126547 | Hs.84072 | U47732 | transmembrane 4 superfamily member 3 | 3.2 |
| 103172 | Hs.116774 | X68742 | integrin; alpha 1 | 3.2 |
| 113867 | Hs.24095 | W68845 | ESTs | 3.2 |
| 133323 | Hs.70937 | Z83735 | H3 histone family, member K | 3.2 |
| 111597 | Hs.189716 | R11499 | ESTs | 3.2 |
| 121515 | Hs.104696 | AA412133 | ESTs | 3.2 |
| 107445 | Hs.6639 | W28406 | ESTs | 3.2 |
| 106887 | Hs.334335 | AA489091 | ESTs | 3.2 |
| 123052 | Hs.185766 | AA481806 | ESTs | 3.2 |
| 107072 | Hs.130760 | AA609113 | Homo sapiens mRNA; cDNA DKFZp586N0318 (f | 3.2 |
| 102214 | Hs.32964 | U23752 | SRY (sex-determining region Y)-box 11 | 3.2 |
| 123147 | | AA487961 | ab11h6.s1 Stratagene lung (#93721) Homo | 3.2 |
| 125435 | Hs.272138 | R00940 | ye87g03.r1 Soares fetal liver spleen 1NF | 3.2 |
| 116246 | Hs.250646 | AA479961 | ESTs; Highly similar to ubiquitin-conjug | 3.2 |
| 105169 | Hs.180789 | AA180321 | Homo sapiens (clone S164) mRNA; 3' end o | 3.2 |
| 134001 | Hs.78344 | AF001548 | myosin; heavy polypeptide 11; smooth mus | 3.2 |
| 124866 | Hs.304389 | R68571 | ESTs | 3.2 |
| 133205 | Hs.67619 | AA089559 | Homo sapiens mRNA; chromosome 1 specific | 3.2 |
| 102986 | Hs.182378 | X17648 | colony stimulating factor 1 (macrophage) | 3.2 |
| 101232 | Hs.242894 | L28997 | ADP-ribosylation factor-like 1 | 3.1 |
| 132906 | Hs.234896 | AA142857 | ESTs; Highly similar to geminin [H.sapie | 3.1 |
| 104281 | Hs.5669 | C14290 | ESTs | 3.1 |
| 123926 | Hs.227933 | AA621348 | ESTs; Highly similar to (defline not ava | 3.1 |
| 134464 | Hs.239720 | N79354 | ESTs; Weakly similar to Rga [D.melanogas | 3.1 |
| 105322 | Hs.16346 | AA234100 | ESTs | 3.1 |
| 100631 | Hs.48332 | HG2709-HT2805 | Serine/Threonine Kinase (Gb:Z25431) | 3.1 |
| 130791 | Hs.199263 | AA259102 | ESTs; Highly similar to (defline not ava | 3.1 |
| 131220 | Hs.300855 | R77200 | ESTs | 3.1 |
| 113237 | Hs.123642 | T62857 | ESTs | 3.1 |
| 125562 | Hs.98968 | AI494372 | ESTs | 3.1 |
| 134110 | Hs.79136 | U41060 | Human breast cancer; estrogen regulated | 3.1 |
| 132393 | Hs.47334 | W85888 | ESTs; Moderately similar to !!!! ALU SUB | 3.1 |
| 107439 | Hs.296842 | W27995 | ESTs; Moderately similar to non-muscle m | 3.1 |
| 125863 | Hs.40719 | AA299096 | Homo sapiens mRNA; cDNA DKFZp564M0916 (f | 3.1 |
| 105811 | Hs.286192 | AA394121 | ESTs | 3.1 |
| 129284 | Hs.296141 | AA104023 | ESTs | 3.1 |
| 125321 | Hs.178294 | T86652 | ESTs | 3.1 |
| 107332 | Hs.183297 | T87750 | ESTs | 3.1 |
| 123570 | Hs.109653 | AA608955 | ESTs | 3.1 |
| 100384 | Hs.90800 | D83646 | matrix metalloproteinase 16 (membrane-in | 3.1 |
| 109063 | Hs.38972 | AA161043 | tetraspan 1 | 3.1 |
| 133284 | Hs.182828 | U09367 | zinc finger protein 136 (clone pHZ-20) | 3.1 |
| 131839 | Hs.33010 | H80622 | Homo sapiens mRNA for KIAA0633 protein; | 3.1 |
| 117606 | Hs.44698 | N35115 | ESTs | 3.1 |
| 418998 | Hs.287849 | F13215 | ESTs | 3.1 |
| 125180 | Hs.103120 | W58344 | ESTs | 3.1 |
| 100789 | | HG3893-HT4163 | Phosphoglucomutase 1, Alt. Splice | 3.1 |
| 126017 | Hs.159440 | H60487 | ESTs | 3.1 |
| 132452 | Hs.247324 | AA005262 | Homo sapiens DNA sequence from PAC 262D1 | 3.1 |
| 129077 | Hs.108479 | H78886 | ESTs | 3.1 |
| 126563 | Hs.181368 | W26247 | U5 snRNP-specific protein (220 kD); orth | 3.1 |
| 129650 | Hs.118258 | N52554 | ESTs | 3.1 |
| 123465 | | AA599033 | ESTs | 3.1 |
| 126486 | Hs.152316 | AA345339 | EST51345 Gall bladder II Homo sapiens cD | 3.1 |
| 126460 | Hs.167031 | W01616 | za36d05.r1 Soares fetal liver spleen 1NF | 3.1 |
| 118697 | Hs.43234 | N72094 | ESTs | 3.1 |
| 103860 | Hs.38057 | AA203742 | ESTs | 3.1 |
| 127968 | Hs.124347 | AA971439 | ESTs | 3.1 |
| 124984 | Hs.223241 | T47566 | yb15c11.s1 Stratagene placenta (#937225) | 3.1 |
| 103903 | Hs.15220 | AA249334 | j312.seq.F Human fetal heart, Lambda ZAP | 3.1 |
| 106697 | Hs.22242 | AA463737 | ESTs | 3.1 |
| 130892 | Hs.20993 | AA442604 | ESTs; Weakly similar to Ydr374cp [S.cere | 3 |
| 114032 | Hs.35014 | W92779 | ESTs | 3 |
| 128835 | Hs.106390 | W15528 | ESTs | 3 |
| 103667 | Hs.247815 | Z80788 | H.sapiens H4/l gene | 3 |
| 126264 | Hs.250614 | N42897 | yy13h06.r1 Soares melanocyte 2NbHM Homo | 3 |
| 132626 | Hs.21275 | D25755 | ESTs | 3 |
| 131107 | Hs.75354 | N87590 | ESTs | 3 |
| 126780 | Hs.5811 | R12421 | ESTs | 3 |
| 127363 | Hs.22116 | AA307744 | Homo sapiens Cdc14B1 phosphatase mRNA; c | 3 |
| 103690 | Hs.84063 | AA016186 | ESTs | 3 |
| 102589 | Hs.8867 | U62015 | Homo sapiens Cyr61 mRNA, complete cds | 3 |
| 125144 | Hs.24336 | W37999 | ESTs | 3 |
| 132977 | Hs.301404 | U28686 | RNA binding motif protein 3 | 3 |
| 120714 | Hs.146170 | AA292689 | ESTs | 3 |
| 101038 | Hs.79411 | J05249 | replication protein A2 (32kD) | 3 |
| 102856 | Hs.248177 | X00090 | Human histone H3 gene | 3 |
| 105516 | Hs.30738 | AA257971 | ESTs | 3 |
| 131137 | Hs.33287 | U85193 | nuclear factor I/B | 3 |
| 127221 | Hs.241551 | AI354332 | ESTs | 3 |
| 411888 | Hs.24104 | R26708 | ESTs | 3 |
| 131684 | Hs.3066 | U26174 | granzyme K (serine protease; granzyme 3; | 3 |
| 100629 | Hs.21291 | HG2706-HT2802 | Serine/Threonine Kinase (Gb:Z25428) | 3 |
| 119944 | Hs.58915 | W86838 | EST | 3 |
| 113801 | Hs.118281 | W38418 | zinc finger protein 266 | 3 |
| 133780 | Hs.76152 | M14219 | decorin | 3 |
| 104690 | Hs.14449 | AA010889 | ESTs | 3 |
| 126371 | Hs.304139 | N57645 | EST | 3 |
| 127635 | Hs.116346 | AA766903 | ESTs | 3 |
| 128434 | Hs.143880 | AI190914 | ESTs | 3 |
| 435761 | Hs.187555 | AA701941 | ESTs | 3 |
| 125025 | Hs.50748 | T71561 | ESTs | 3 |
| 124940 | Hs.103804 | R99599 | heterogeneous nuclear ribonucleoprotein | 3 |
| 128742 | Hs.251531 | D00763 | proteasome (prosome; macropain) subunit; | 3 |
| 107147 | Hs.10450 | AA621125 | Homo sapiens chromosome 2; 10 repeat reg | 3 |
| 112068 | Hs.22545 | R43910 | ESTs | 3 |
| 105346 | Hs.263727 | AA235465 | ESTs; Moderately similar to !!!! ALU SUB | 3 |
| 130972 | Hs.21739 | AA370302 | Homo sapiens mRNA; cDNA DKFZp586l1518 (f | 3 |
| 131230 | Hs.274407 | AA149987 | thymus specific serine peptidase | 3 |
| 133743 | Hs.75847 | N79435 | ESTs | 3 |
| 127402 | Hs.227949 | AA358869 | ESTs; Highly similar to SEC13-RELATED PR | 3 |
| 117483 | Hs.44189 | N30426 | ESTs | 3 |
| 123659 | Hs.112699 | AA609368 | ESTs | 3 |
| 103963 | Hs.63290 | AA298588 | EST114219 HSC172 cells II Homo sapiens c | 3 |
| 103795 | Hs.7367 | AA112222 | ESTs; Moderately similar to (defline not | 3 |
| 115092 | Hs.80975 | AA255903 | CD39-like 4 | 2.9 |
| 134831 | Hs.89890 | S72370 | pyruvate carboxylase | 2.9 |
| 128579 | Hs.101810 | AA093378 | ESTs; Weakly similar to !!!! ALU SUBFAMI | 2.9 |
| 134193 | Hs.7980 | F09570 | ESTs | 2.9 |
| 123522 | Hs.112575 | AA608577 | ESTs | 2.9 |
| 107109 | Hs.32793 | AA609943 | ESTs | 2.9 |
| 134694 | Hs.88556 | D50405 | histone deacetylase 1 | 2.9 |
| 134399 | Hs.82689 | H99801 | tumor rejection antigen (gp96) 1 | 2.9 |
| 134632 | Hs.174139 | AA398710 | H. sapiens RNA for CLCN3 | 2.9 |
| 106683 | Hs.14512 | AA461495 | ESTs | 2.9 |
| 108555 | | AA084963 | zn13e12.s1 Stratagene hNT neuron (#93723 | 2.9 |
| 100953 | Hs.2110 | HG945-HT945 | Nucleic Acid-Binding Protein (Gb:L12693) | 2.9 |
| 130597 | Hs.16492 | AA173998 | ESTs; Weakly similar to weakly similar t | 2.9 |
| 101813 | Hs.139226 | M87338 | replication factor (activator 1) 2 (40 | 2.9 |
| 106636 | Hs.286 | AA459950 | ESTs | 2.9 |
| 129109 | Hs.108708 | AA491295 | calcium/calmodulin-dependent protein kin | 2.9 |
| 125819 | Hs.251871 | AA044840 | stromal cell-derived factor 1 | 2.9 |
| 106282 | Hs.9857 | AA433946 | ESTs; Weakly similar to (defline not ava | 2.9 |
| 100386 | Hs.301636 | D83703 | peroxisomal biogenesis factor 6 | 2.9 |
| 114546 | Hs.98074 | AA056263 | ESTs; Moderately similar to !!!! ALU SUB | 2.9 |
| 105914 | Hs.9701 | AA402224 | Homo sapiens growth arrest and DNA-damag | 2.9 |
| 108552 | | AA084912 | zn11c7.s1 Stratagene hNT neuron (#937233 | 2.9 |
| 126505 | Hs.190057 | W26894 | 16a11 Human retina cDNA randomly primed | 2.9 |
| 134098 | Hs.79086 | X06323 | Human MRL3 mRNA for ribosomal protein L3 | 2.9 |
| 129721 | Hs.211539 | L19161 | eukaryotic translation initiation factor | 2.9 |
| 100076 | Hs.277422 | AB000897 | Homo sapiens mRNA for cadherin FIB3, par | 2.9 |
| 117466 | Hs.44104 | N29862 | ESTs | 2.9 |
| 106335 | Hs.36688 | AA437258 | ESTs; Moderately similar to WAP four-dis | 2.9 |
| 134510 | Hs.250870 | U25265 | protein kinase; mitogen-activated; kinas | 2.9 |
| 105835 | Hs.32995 | AA398412 | ESTs | 2.9 |
| 106611 | Hs.26267 | AA458904 | ESTs; Weakly similar to torsinA [H.sapie | 2.9 |
| 134087 | Hs.173824 | U51166 | thymine-DNA glycosylase | 2.9 |
| 100641 | Hs.182183 | HG2743-HT2846 | Caldesmon 1, Alt. Splice 4, Non-Muscle | 2.9 |
| 104602 | | R86920 | ESTs | 2.9 |
| 117203 | Hs.42738 | H99799 | ESTs | 2.9 |
| 131889 | Hs.34073 | AA401912 | BH-protocadherin (brain-heart) | 2.9 |
| 101707 | Hs.155212 | M65131 | methylmalonyl Coenzyme A mutase | 2.9 |
| 115271 | Hs.5724 | AA279422 | ESTs | 2.9 |
| 125812 | Hs.287912 | H73420 | lectin; mannose-binding; 1 | 2.9 |
| 110740 | Hs.19762 | H99675 | ESTs | 2.9 |
| 103406 | Hs.285728 | X95677 | H.sapiens mRNA for ArgBPIB protein | 2.9 |
| 104577 | Hs.132390 | R71539 | ESTs | 2.9 |
| 102772 | Hs.161002 | U83115 | absent in melanoma 1 | 2.9 |
| 131710 | Hs.30985 | AA233225 | ESTs; Highly similar to (defline not ava | 2.9 |
| 125231 | Hs.268903 | W84714 | ESTs | 2.9 |
| 127380 | Hs.15535 | AI417137 | Homo sapiens clone 24582 mRNA sequence | 2.9 |
| 104229 | Hs.61289 | AB002346 | inositol phosphate 5'-phosphatase 2 (syn | 2.9 |
| 126600 | Hs.191385 | AA699949 | ESTs | 2.9 |
| 125175 | Hs.303030 | W52355 | EST | 2.9 |
| 103849 | Hs.34578 | AA187045 | ESTs; Weakly similar to !!!! ALU SUBFAMI | 2.9 |
| 102126 | Hs.78961 | U14575 | protein phosphatase 1; regulatory (inhib | 2.9 |
| 124906 | Hs.107815 | R87647 | ESTs | 2.9 |
| 131148 | Hs.303125 | C00038 | ESTs | 2.9 |
| 123158 | Hs.218329 | AA488658 | heat shock 70kD protein 1 | 2.9 |
| 133667 | Hs.75462 | U72649 | Human BTG2 (BTG2) mRNA; complete cds | 2.9 |
| 105182 | Hs.18271 | AA191014 | ESTs; Weakly similar to Ydr372cp [S.cere | 2.9 |
| 133968 | Hs.232068 | D15050 | Human mRNA for transcription factor AREB | 2.9 |
| 117425 | Hs.336901 | N27154 | ESTs | 2.9 |
| 111087 | Hs.37637 | N59645 | ESTs | 2.9 |
| 129641 | Hs.11805 | N66066 | ESTs | 2.9 |
| 128639 | Hs.102897 | N91246 | ESTs | 2.9 |
| 133209 | Hs.79265 | AA114183 | ESTs; Moderately similar to glutamate py | 2.9 |
| 135154 | Hs.267812 | AA126433 | sorting nexin 4 | 2.9 |
| 126838 | Hs.279609 | AA858097 | pigment epithelium-derived factor | 2.9 |
| 103803 | Hs.106149 | AA127696 | ESTs | 2.9 |
| 102139 | Hs.2128 | U15932 | dual specificity phosphatase 5 | 2.9 |
| 128104 | | AA971000 | op67g11.s1 Soares_NFL_T_GBC_S1 Homo sapi | 2.8 |
| 127834 | Hs.337631 | AA761415 | nz22d08.s1 NCI_CGAP_GCB1 Homo sapiens cD | 2.8 |
| 133101 | Hs.180952 | AA488230 | ESTs | 2.8 |
| 127250 | Hs.217916 | AI023717 | ESTs | 2.8 |
| 135063 | Hs.93883 | D10537 | myelin protein zero (Charcot-Marie-Tooth | 2.8 |
| 126323 | Hs.68644 | N45014 | yy80g06.r1 Soares_multiple_sclerosis_2Nb | 2.8 |
| 121873 | Hs.145696 | AA426270 | ESTs | 2.8 |
| 122090 | Hs.98684 | AA432141 | ESTs | 2.8 |
| 118728 | Hs.322645 | N73705 | ESTs | 2.8 |
| 135400 | Hs.99915 | M23263 | androgen receptor (dihydrotestosterone r | 2.8 |
| 125278 | Hs.129998 | W93523 | ESTs | 2.8 |
| 124387 | Hs.109019 | N27637 | ESTs | 2.8 |
| 124803 | Hs.12186 | R45480 | cyclin K | 2.8 |
| H45968 | Hs.32149 | H45968 | ESTs | 2.8 |
| 104261 | Hs.5409 | AF008442 | RNA polymerase I subunit | 2.8 |
| 105366 | Hs.282093 | AA236356 | ESTs | 2.8 |
| 106070 | Hs.5957 | AA417761 | Homo sapiens clone 24416 mRNA sequence | 2.8 |
| 131356 | Hs.25960 | M13241 | v-myc avian myelocytomatosis viral relat | 2.8 |
| 112009 | Hs.26255 | R42714 | EST | 2.8 |
| 133199 | Hs.250176 | AA609773 | Homo sapiens clone 23904 mRNA sequence | 2.8 |
| 110379 | Hs.33130 | H44825 | ESTs | 2.8 |
| 103890 | Hs.72085 | AA236843 | ESTs; Weakly similar to unknown [S.cerev | 2.8 |
| 128152 | | R20353 | yg20f10.r1 Soares infant brain 1NIB Homo | 2.8 |
| 107008 | Hs.23740 | AA598710 | ESTs | 2.8 |
| 135243 | Hs.97101 | AA215333 | ESTs | 2.8 |
| 103058 | Hs.184510 | X57348 | stratifin | 2.8 |
| 132020 | Hs.293845 | AA428990 | ESTs | 2.8 |
| 116354 | Hs.292566 | AA504262 | ESTs | 2.8 |
| 125867 | Hs.12372 | H98141 | ESTs | 2.8 |
| 120603 | Hs.98541 | AA282787 | ESTs; Highly similar to (defline not ava | 2.8 |
| 115119 | Hs.46847 | AA256524 | Human DNA sequence from clone 30M3 on ch | 2.8 |
| 133865 | Hs.170290 | F09315 | discs; large (Drosophila) homolog 5 | 2.8 |
| 109415 | Hs.110826 | AA227219 | Homo sapiens CAGF9 mRNA; partial cds | 2.8 |
| 128687 | Hs.23767 | Z38910 | ESTs | 2.8 |
| 109984 | Hs.10299 | H09594 | ESTs; Moderately similar to !!!! ALU SUB | 2.8 |
| 133179 | Hs.66731 | U81599 | homeo box B13 | 2.8 |
| 115998 | Hs.336629 | AA448488 | ESTs; Weakly similar to zinc finger prot | 2.8 |
| 112180 | Hs.25067 | R49116 | EST | 2.8 |
| 120428 | Hs.173694 | AA236822 | ESTs; Moderately similar to (defline not | 2.8 |
| 106241 | Hs.6019 | AA430108 | ESTs | 2.8 |
| 131060 | Hs.22564 | AA160890 | myosin VI | 2.8 |
| 111383 | Hs.40919 | N94527 | ESTs | 2.8 |
| 102123 | Hs.1594 | U14518 | centromere protein A (17kD) | 2.8 |
| 102722 | Hs.79981 | U79242 | Human clone 23560 mRNA sequence | 2.8 |
| 129887 | Hs.274324 | W92041 | PCAF associated factor 65 alpha | 2.8 |
| 126663 | Hs.181297 | AA714635 | ESTs | 2.8 |
| 104367 | Hs.134342 | H17438 | ESTs; Weakly similar to seventransmembra | 2.8 |
| 107316 | Hs.193700 | T63174 | ESTs; Moderately similar to !!!! ALU SUB | 2.8 |
| 128059 | Hs.145096 | AA972446 | ESTs | 2.8 |
| 124447 | | N48000 | ESTs | 2.8 |
| 111398 | Hs.125565 | R00086 | deafness; X-linked 1; progressive | 2.8 |
| 134085 | Hs.79018 | U20979 | chromatin assembly factor I (150 kDa) | 2.8 |
| 124788 | Hs.100912 | R43543 | ESTs | 2.8 |
| 112248 | Hs.326416 | R51361 | ESTs | 2.8 |
| 121309 | Hs.97312 | AA402482 | ESTs | 2.8 |
| 103076 | Hs.75319 | X59618 | ribonucleotide reductase M2 polypeptide | 2.8 |
| 107071 | Hs.35198 | AA609053 | ESTs | 2.8 |
| 104425 | Hs.35380 | H88496 | ESTs | 2.8 |
| 132991 | Hs.62245 | AA446906 | solute carrier family 25 (mitochondrial | 2.8 |
| 104968 | Hs.29669 | AA084602 | ESTs | 2.8 |
| 121153 | Hs.97694 | AA399640 | ESTs | 2.8 |
| 131216 | Hs.243901 | D31058 | ESTs | 2.8 |
| 109682 | Hs.22869 | F09299 | ESTs | 2.8 |
| 131990 | Hs.168818 | H77734 | ESTs; Moderately similar to roundabout 1 | 2.8 |
| 132027 | Hs.181444 | N78844 | ESTs; Weakly similar to R12C12.6 [C.eleg | 2.8 |
| 127383 | Hs.190478 | AA447990 | ESTs | 2.8 |
| 132598 | Hs.530 | M81379 | collagen; type IV; alpha 3 (Goodpasture | 2.8 |
| 101121 | Hs.1313 | L09753 | tumor necrosis factor (ligand) superfami | 2.8 |
| 123000 | Hs.105640 | AA479347 | ESTs | 2.8 |
| 121329 | Hs.1755 | AA404324 | ESTs | 2.8 |
| 100481 | Hs.121489 | HG1098-HT1098 | Cystatin D | 2.7 |
| 113803 | Hs.283683 | W42789 | ESTs | 2.7 |
| 110934 | Hs.169001 | N48708 | ESTs; Weakly similar to cytochrome P-450 | 2.7 |
| 432888 | | T86823 | ESTs | 2.7 |
| 121802 | Hs.188898 | AA424328 | ESTs | 2.7 |
| 130396 | Hs.155313 | AB002331 | Human mRNA for KIAA0333 gene; partial cd | 2.7 |
| 121103 | Hs.97697 | AA398936 | ESTs; Weakly similar to (defline not ava | 2.7 |
| 131129 | Hs.23240 | R27296 | ESTs | 2.7 |
| 130943 | Hs.272429 | D50855 | calcium-sensing receptor (hypocalciuric | 2.7 |
| 134676 | Hs.87819 | W28051 | ESTs; Weakly similar to keratin 9; cytos | 2.7 |
| 111900 | Hs.25318 | R39044 | ESTs | 2.7 |
| 106025 | Hs.173334 | AA412063 | ESTs | 2.7 |
| 126144 | Hs.40639 | N39696 | yx92a07.r1 Soares melanocyte 2NbHM Homo | 2.7 |
| 103248 | Hs.75262 | X77383 | cathepsin O | 2.7 |
| 127230 | Hs.274170 | H30501 | Homo sapiens Opa-interacting protein OIP | 2.7 |
| 101584 | Hs.84072 | M35252 | transmembrane 4 superfamily member 3 | 2.7 |
| 124131 | Hs.167489 | H19980 | ESTs | 2.7 |
| 129689 | Hs.77873 | AA130156 | ESTs | 2.7 |
| 132892 | Hs.9973 | W92797 | ESTs | 2.7 |
| 120827 | Hs.132967 | AA347717 | ESTs | 2.7 |
| 134579 | Hs.85963 | N23222 | ESTs; Moderately similar to !!!! ALU SUB | 2.7 |
| 106149 | Hs.256301 | AA424881 | ESTs | 2.7 |
| 132037 | Hs.332541 | AA203649 | ESTs; Weakly similar to HEM45 [H.sapiens | 2.7 |
| 130542 | Hs.179825 | U64675 | Human sperm membrane protein BS-63 mRNA, | 2.7 |
| 122851 | Hs.99598 | AA463627 | ESTs | 2.7 |
| 134983 | Hs.196384 | D28235 | prostaglandin-endoperoxide synthase 2 (p | 2.7 |
| 120537 | Hs.160422 | AA262790 | ESTs | 2.7 |
| 131036 | Hs.174140 | X64330 | ATP citrate lyase | 2.7 |
| 133889 | Hs.211582 | AA099391 | ESTs | 2.7 |
| 128847 | Hs.106529 | AA424199 | zv81e01.r1 Soares_total_fetus_Nb2HF8_9w | 2.7 |
| 112755 | Hs.306044 | R93802 | ESTs | 2.7 |
| 423239 | | AA323591 | EST26392 Cerebellum II Homo sapiens cDNA | 2.7 |
| 105031 | Hs.12321 | AA127240 | ESTs | 2.7 |
| 126021 | Hs.187516 | AA775894 | ESTs | 2.7 |
| 102116 | | U13706 | Human ELAV-like neuronal protein 1 isofo | 2.7 |
| 133394 | Hs.237225 | R16759 | ESTs, Weakly similar to (defline not ava | 2.7 |
| 104267 | Hs.278439 | C00358 | ESTs | 2.7 |
| 107614 | Hs.40241 | AA004878 | ESTs, Highly similar to (defline not ava | 2.7 |
| 129809 | Hs.1259 | X55283 | aslaloglycoprotein receptor 2 | 2.7 |
| 112109 | Hs.283309 | R45221 | ESTs; Weakly similar to !!!! ALU SUBFAMI | 2.7 |
| 128422 | | T85681 | yd60c06.r1 Soares fetal liver spleen 1NF | 2.7 |
| 109494 | Hs.43899 | AA233702 | ESTs | 2.7 |
| 118696 | Hs.292284 | N72086 | Homo sapiens RNA polymerase III largest | 2.7 |
| 106053 | Hs.36727 | AA416963 | ESTs; Highly similar to histone H2A [H.s | 2.7 |
| 104440 | Hs.284380 | L20492 | gamma-glutamyltransferase 1 | 2.7 |
| 129426 | Hs.111323 | AA412087 | EST;Highly similar to (defline not aval | 2.7 |
| 123798 | | AA620411 | small inducible cytokine A5 (RANTES) | 2.7 |
| 106716 | Hs.238928 | AA464962 | ESTs | 2.7 |
| 103663 | | Z78291 | Z78291 Homo sapiens brain fetus Homo sap | 2.7 |
| 114162 | Hs.22265 | Z38909 | ESTs | 2.7 |
| 113063 | Hs.5027 | T32438 | ESTs | 2.7 |
| 127897 | | AA773857 | af80c09.r1 Soares_NhHMPu_S1 Homo sapiens | 2.7 |
| 130621 | Hs.16803 | AA621718 | ESTs; Weakly similar to (defline not ava | 2.7 |
| 116245 | Hs.42796 | AA479958 | ESTs; Highly similar to (defline not ava | 2.7 |
| 125499 | | R11878 | yf49d11.r1 Soares infant brain 1NIB Homo | 2.7 |
| 133960 | Hs.77899 | M19267 | tropomyosin 1 (alpha) | 2.7 |
| 104470 | Hs.246358 | N28843 | ESTs; Weakly similar to Similar to colla | 2.7 |
| 134982 | Hs.92308 | N46086 | ESTs | 2.7 |
| 106803 | Hs.284295 | AA479114 | ESTs | 2.7 |
| 104899 | Hs.285574 | AA054726 | ESTs | 2.7 |
| 125401 | Hs.337585 | AI204637 | ESTs; Moderately similar to KIAA0350 [H. | 2.7 |
| 111253 | Hs.15768 | N70042 | ESTs; Moderately similar to !!!! ALU SUB | 2.7 |
| 118449 | Hs.164478 | N66413 | ESTs; Weakly similar to (defline not ava | 2.7 |
| 134507 | Hs.84318 | M63488 | replication protein A1 (70kD) | 2.7 |
| 121609 | Hs.98185 | AA416867 | EST | 2.7 |
| 113835 | Hs.27475 | W56590 | ESTs | 2.7 |
| 113962 | Hs.285290 | W86375 | ESTs; Highly similar to (defline not ava | 2.7 |
| 121913 | Hs.98558 | AA428062 | ESTs | 2.7 |
| 108194 | Hs.216717 | AA057250 | ESTs | 2.7 |
| 130799 | Hs.12696 | AA464273 | ESTs | 2.7 |
| 123184 | Hs.18166 | AA489072 | Homo sapiens mRNA for KIAA0870 protein; | 2.7 |
| 103420 | Hs.173497 | X97065 | SEC23-like protein B | 2.7 |
| 106186 | Hs.6315 | AA427398 | acetylserotonin N-methyltransferase-like | 2.7 |
| 101349 | | L77559 | Homo sapiens DGS-B partial mRNA | 2.7 |
| 112954 | Hs.6655 | T16559 | ESTs | 2.7 |
| 133054 | Hs.291079 | R07876 | ESTs; Weakly similar to unknown [S.cerev | 2.7 |
| 128131 | Hs.25640 | AI283162 | claudin 3 | 2.6 |
| 101864 | Hs.75777 | M95787 | transgelin | 2.6 |
| 111948 | Hs.26303 | R40752 | ESTs | 2.6 |
| 130145 | Hs.151051 | U07620 | protein kinase mitogen-activated 10 (MAP | 2.6 |
| 126507 | Hs.23964 | AI362218 | ESTs | 2.6 |
| 117903 | Hs.47111 | N50740 | ESTs | 2.6 |
| 116345 | Hs.199067 | AA496981 | ESTs | 2.6 |
| 132227 | Hs.4248 | AA412620 | ESTs | 2.6 |
| 125746 | Hs.274256 | H03574 | yj42b06.r1 Soares placenta Nb2HP Homo sa | 2.6 |
| 105073 | Hs.89463 | AA137034 | ESTs | 2.6 |
| 102764 | | U82310 | Homo sapiens unknown protein mRNA, parti | 2.6 |
| 131367 | Hs.173933 | AA456687 | ESTs | 2.6 |
| 130792 | Hs.19500 | AA307896 | nuclear localization signal deleted in v | 2.6 |
| 107427 | Hs.46736 | W26975 | ESTs | 2.6 |
| 117477 | Hs.44175 | N30328 | ESTs | 2.6 |
| 106290 | Hs.16364 | AA435542 | ESTs | 2.6 |
| 126829 | Hs.7910 | R11547 | ESTs | 2.6 |
| 118836 | Hs.173001 | N79820 | ESTs | 2.6 |
| 100147 | Hs.136348 | D13666 | osteoblast specific factor 2 (fasciclin | 2.6 |
| 104278 | Hs.109253 | C02582 | ESTs; Highly similar to (defline not ava | 2.6 |
| 135051 | Hs.83484 | C15324 | ESTs | 2.6 |
| 126081 | Hs.227835 | AI346024 | collagen; type I; alpha 1 | 2.6 |
| 123579 | | AA608983 | af5d4.s1 Soares_testis_NHT Homo sapiens | 2.6 |
| 130115 | Hs.149923 | M31627 | X-box binding protein 1 | 2.6 |
| 101434 | Hs.1430 | M20218 | coagulation factor XI (plasma thrombopla | 2.6 |
| 122962 | Hs.104720 | AA478429 | ESTs; Moderately similar to !!!! ALU SUB | 2.6 |
| 126151 | Hs.40808 | AA324743 | ESTs | 2.6 |
| 128925 | Hs.21851 | D61676 | Homo sapiens mRNA; cDNA DKFZp586J2118 (f | 2.6 |
| 128919 | Hs.103391 | L27559 | insulin-like growth factor binding prote | 2.6 |
| 130296 | Hs.154103 | R09286 | LIM protein (similar to rat protein kina | 2.6 |
| 128402 | Hs.191637 | AA457244 | ESTs | 2.6 |
| 129273 | Hs.109968 | W63783 | ESTs | 2.6 |
| 125483 | Hs.7788 | F07759 | ESTs | 2.6 |
| 132953 | Hs.321264 | AA029927 | ESTs | 2.6 |
| 130963 | Hs.21639 | U57099 | nuclear protein; marker for differential | 2.6 |
| 120614 | Hs.194154 | AA284281 | ESTs; Weakly similar to !!!! ALU SUBFAMI | 2.6 |
| 123251 | Hs.103267 | AA490858 | ESTs; Moderately similar to Rabin3 [R.no | 2.6 |
| 121710 | Hs.96744 | AA419011 | ESTs | 2.6 |
| 125428 | Hs.851 | W74608 | ESTs; Highly similar to (defline not ava | 2.6 |
| 115906 | Hs.82302 | AA436616 | ESTs | 2.6 |
| 108432 | | AA076626 | Homo sapiens clone 23851 mRNA sequence | 2.6 |
| 126191 | Hs.191911 | H97728 | ESTs | 2.6 |
| 106164 | Hs.281434 | AA425773 | ESTs | 2.6 |
| 111519 | Hs.268615 | R08165 | ESTs | 2.6 |
| 134590 | Hs.173840 | W58612 | ESTs | 2.6 |
| 102565 | | U59748 | Human desert hedgehog (hDHH) mRNA, parti | 2.6 |
| 129879 | Hs.13109 | AA194973 | ESTs | 2.6 |
| 114264 | Hs.334609 | Z40074 | ESTs | 2.6 |
| 106236 | Hs.21104 | AA429951 | ESTs | 2.6 |
| 135192 | Hs.321709 | AF000234 | purinergic receptor P2X; ligand-gated io | 2.6 |
| 109833 | Hs.29889 | H00580 | ESTs | 2.6 |
| 105756 | Hs.8535 | AA303088 | ESTs; Weakly similar to transformation-r | 2.6 |
| 121422 | Hs.97967 | AA406210 | ESTs | 2.6 |
| 130417 | Hs.155485 | U58522 | Human huntingtin interacting protein (Hl | 2.6 |
| 124312 | Hs.102329 | H94647 | ESTs | 2.6 |
| 108998 | Hs.97199 | AA156058 | ESTs | 2.6 |
| 127081 | Hs.180591 | R88362 | ESTs; Weakly similar to weak similarity | 2.6 |
| 129574 | Hs.11463 | AA458603 | ESTs; Weakly similar to (defline not ava | 2.6 |
| 112410 | Hs.26904 | R61680 | ESTs | 2.6 |
| 123929 | Hs.112981 | AA621364 | ESTs | 2.6 |
| 122905 | Hs.104835 | AA470070 | ESTs | 2.6 |
| 116399 | Hs.110637 | AA599729 | Homo sapiens homeobox protein A10 (HOXA1 | 2.6 |
| 130279 | Hs.153934 | AA424044 | core-binding factor, runt domain; alpha | 2.6 |
| 130021 | Hs.1435 | M24470 | guanosine monophosphate reductase | 2.6 |
| 100585 | Hs.199160 | HG2367-HT2463 | Trithorax Homolog Hrx | 2.6 |
| 104965 | Hs.30177 | AA084104 | ESTs | 2.6 |
| 117711 | Hs.46485 | N45201 | EST | 2.6 |
| 124792 | Hs.48712 | R44357 | ESTs | 2.6 |
| 111299 | Hs.74313 | N73808 | ESTs | 2.6 |
| 103616 | Hs.32971 | Z46973 | phosphoinositide-3-kinase; class 3 | 2.6 |
| 133629 | Hs.195614 | D13642 | KIAA0017 gene product | 2.6 |
| 126484 | Hs.169977 | AI086782 | ESTs | 2.6 |
| 100858 | | HG4245-HT4515 | Forkhead Family Afx1 | 2.6 |
| 133547 | Hs.301927 | X02883 | T-cell receptor, alpha (V;D;J;C) | 2.6 |
| 126680 | Hs.133865 | F07097 | ESTs | 2.6 |
| 125739 | Hs.92137 | AA428557 | v-myc avian myelocytomatosis viral oncog | 2.6 |
| 102276 | Hs.10247 | U30999 | Human (memc) mRNA, 3'UTR | 2.6 |
| 105586 | Hs.191538 | AA279137 | ESTs | 2.6 |
| 103978 | Hs.34136 | AA307443 | ESTs | 2.6 |
| 125054 | Hs.268601 | T80622 | ESTs; Weakly similar to (defline not ava | 2.6 |
| 114212 | Hs.21201 | Z39338 | ESTs; Highly similar to (defline not ava | 2.6 |
| 116959 | Hs.40022 | H79310 | EST | 2.6 |
| 109228 | Hs.306995 | AA193366 | ESTs | 2.6 |
| 133989 | Hs.78202 | U29175 | SWI/SNF related; matrix associated; acti | 2.6 |
| 100640 | Hs.182183 | HG2743-HT2845 | Caldesmon 1, Alt Splice 3, Non-Muscle | 2.6 |
| 133093 | Hs.285996 | AA598749 | ESTs | 2.6 |
| 114306 | Hs.6540 | Z40861 | ESTs | 2.6 |
| 106060 | Hs.171391 | AA417287 | C-terminal binding protein 2 | 2.5 |
| 107748 | Hs.60772 | AA017258 | EST | 2.5 |
| 100134 | Hs.49 | D13264 | macrophage scavenger receptor 1 | 2.5 |
| 133969 | Hs.78 | U13044 | GA-binding protein transcription factor; | 2.5 |
| 130992 | Hs.74316 | AA455001 | ESTs | 2.5 |
| 127493 | Hs.291701 | AA808081 | oc39a08.s1 NCI_CGAP_GCB1 Homo sapiens cD | 2.5 |
| 132869 | Hs.203961 | N26855 | ESTs | 2.5 |
| 117570 | Hs.44583 | N34415 | EST | 2.5 |
| 124644 | Hs.109654 | N91279 | ESTs | 2.5 |
| 103558 | Hs.2785 | Z19574 | keratin 17 | 2.5 |
| 132883 | Hs.5897 | AA047151 | ESTs | 2.5 |
| 102009 | Hs.82643 | U02680 | protein tyrosine kinase 9 | 2.5 |
| 116058 | Hs.20159 | AA454156 | ESTs | 2.5 |
| 121989 | Hs.193784 | AA430044 | ESTs | 2.5 |
| 131257 | Hs.24908 | AA256042 | ESTs | 2.5 |
| 100320 | Hs.75275 | D50916 | homolog of yeast (S. cerevisiae) ufd2 | 2.5 |
| 102959 | Hs.121524 | X15722 | glutathione reductase | 2.5 |
| 132969 | Hs.6166 | AA047616 | ESTs | 2.5 |
| 130869 | Hs.2057 | AA128100 | uridine monophosphate synthetase (orotat | 2.5 |
| 129645 | Hs.118131 | L38928 | 5;10-methenyltetrahydrofolate synthetase | 2.5 |
| 126399 | Hs.83883 | AA128075 | zl16d08.r1 Soares_pregnant_uterus_NbHPU | 2.5 |
| 134069 | Hs.78935 | U29607 | Homo sapiens elF-2-associated p67 homolo | 2.5 |
| 109816 | Hs.61960 | F11013 | ESTs; Weakly similar to KIAA0176 [H.sapi | 2.5 |
| 134801 | Hs.89695 | X02160 | insulin receptor | 2.5 |
| 104232 | Hs.10587 | AB002351 | Human mRNA for KIAA0353 gene; partial cd | 2.5 |
| 107361 | Hs.159486 | U72513 | Human RPL13-2 pseudogene mRNA; complete | 2.5 |
| 106057 | Hs.289074 | AA417067 | ESTs | 2.5 |
| 134252 | Hs.80720 | AA031782 | Homo sapiens mRNA; cDNA DKFZp586B1722 (f | 2.5 |
| 128062 | Hs.105547 | AA379500 | ESTs | 2.5 |
| 110009 | Hs.6614 | H10933 | ESTs | 2.5 |
| 111375 | Hs.20432 | N93696 | ESTs | 2.5 |
| 122642 | Hs.99361 | AA454186 | ESTs | 2.5 |
| 127999 | Hs.69851 | AA837495 | ESTs; Weakly similar to Wiskott-Aldrich | 2.5 |
| 105029 | Hs.13268 | AA126855 | ESTs | 2.5 |
| 105082 | Hs.26765 | AA143763 | ESTs; Weakly similar to Similarity to S. | 2.5 |

**TABLE 1A show the accession numbers for those primekeys lacking unigeneID's for Table 1. For each probeset we have listed the gene cluster number from which the oligonucleotides were designed. Gene clusters were compiled using sequences derived from Genbank ESTs and mRNAs. These sequences were clustered based on sequence similarity using Clustering and Alignment Tools (DoubleTwist, Oakland California). The Genbank accession numbers for sequences comprising each cluster are listed in the "Accession" column.**

| | | |
|---|---|---|
| Pkey: | Unique Eos probeset identifier number | |
| CAT number: | Gene cluster number | |
| Accession: | Genbank accession numbers | |
| | | |

| **Pkey** | **CAT number** | **Accessions** |
|---|---|---|
| | | |
| 108552 | 111555_1 | AA071210 AA069899 AA071438 AA084912 AA084803 AA079371 AA079370 |
| 126023 | 1596090_1 | H57661 H58881 |
| 126086 | 1606216_1 | H75681 H70975 |
| 102565 | 32479_1 | AB010994 U59748 AA064660 |
| 101964 | 48158_-7 | S81578 |
| 125499 | 1562851_1 | H10543 R11878 |
| 125596 | 1708455_1 | R25698 R56582 R56018 |
| 118417 | 37186_1 | AF080229 AF080231 AF080230 AF080232 AF080233 AF080234 BE550633 AI636743 AW614951 BE467547 AI680833 |
| | | AI633818 N29986 U87592 U87593 U87590 U87591 S46404 U87587 AA463992 AW206802 AI970376 Al583718 AI672574 |
| | | N25695 AW665466 AI818326 AA126128 AI480345 AW013827 AA248638 AI214968 AA204735 AA207155 AA206262 |
| | | AA204833 AW003247 AW496808 AI080480 AI631703 AI651023 AI867418 AW818140 AA502500 AI206199 AI671282 |
| | | AI352545 BE501030 AI652535 BE465762 AA206331 AW451866 AA471088 AA206342 AA204834 AA206100 AW021661 |
| | | AA332922 N66048 AA703396 H92278 AW139734 H92683 U87589 U87595 H69001 U87594 BE466420 AI624817 |
| | | BE466611 AI206344 AA574397 AA348354 AI493192 |
| 125661 | 327827_1 | AA491830 R50173 R55192 R50320 AI732306 AI732305 AI820727 AI820728 R55191 R50319 R50227 |
| 125957 | 1583542_1 | H41694 H45213 |
| 125982 | 1766315_1 | R98091 W92898 |
| 127248 | 227560_1 | AA364195 AA325029 AW962050 |
| 103731 | 112052_1 | AA070595 AA131490 AA131373 |
| 127261 | 231687_1 | AA330501 AA661567 |
| 127265 | 232391_1 | AA331503 AA332751 AW962542 |
| 126659 | 1541209_1 | T16245 R19694 F13545 H10299 T66048 T65279 H18006 |
| 127315 | 37938_1 | AF116622 AI114507 AA640834 AA377999 |
| 103806 | 112618_1 | AA130614 AA071410 |
| 128104 | 502608_1 | AA906093 AA971000 |
| 104602 | 524482_2 | H47610 R86920 |
| 128152 | 297868_1 | F07973 R20353 AA442660 |
| 128422 | 1811283_1 | T77794 T85681 |
| 127897 | 446527_1 | AA773681 AA773857 |
| 106566 | 120358_1 | BE298210 AI672315 AW086489 BE298417 AA455921 AA902537 BE327124 R14963 AA085210 AW274273 AI333584 |
| | | AI369742 AI039658 AI885095 AI476470 AI287650 AI885299 AI985381 AW592624 AW340136 AI266556 AA456390 |
| | | AI310815 AA484951 |
| 129735 | 44573_2 | AI950087 N70208 R97040 N36809 AI308119 AW967677 N35320 AI251473 H59397 AW971573 R97278 W01059 |
| | | AW967671 AA908598 AA251875 AI820501 AI820532 W87891 T85904 U71456 T82391 BE328571 T75102 R34725 |
| | | AA884922 BE328517 AI219788 AA884444 N92578 F13493 AA927794 AI560251 AW874068 AL134043 AW235363 |
| | | AA663345 AW008282 AA488964 AA283144 AI890387 AI950344 AI741346 AI689062 AA282915 AW102898 AI872193 |
| | | AI763273 AW173586 AW150329 AI653832 AI762688 AA988777 AA488892 AI356394 AW103813 AI539642 AA642789 |
| | | AA856975 AW505512 AI961530 AW629970 BE612881 AW276997 AW513601 AW512843 AA044209 AW856538 |
| | | AA180009 AA337499 AW961101 AA251669 AA251874 AI819225 AW205862 AI683338 AI858509 AW276905 AI633006 |
| | | AA972584 AA908741 AW072629 AW513996 AA293273 AA969759 N75628 N22388 H84729 H60052 T92487 AI022058 |
| | | AA780419 AA551005 W80701 AW613456 AI373032 AI564269 F00531 H83488 W37181 W78802 R66056 AI002839 |
| | | R67840 AA300207 AW959581 T63226 F04005 |
| 123147 | 219802_-2 | AA487961 |
| 130529 | 158447_1 | AA178953 AA192740 |
| 123579 | genbank_AA608983 | AA608983 |
| 109175 | genbank_AA180496 | AA180496 |
| 100789 | tigr_HT4163 | S67998 |
| 100858 | tigr_HT4515 | U10072 |
| 123798 | 579959_1 | AA620411 AA287491 |
| 102116 | entrez_U13706 | U13706 |
| 102398 | entrez_U42359 | U42359 |
| 102764 | entrez_U82310 | U82310 |
| 118475 | genbank_N66845 | N66845 |
| 104776 | genbank_AA026349 | AA026349 |
| 104787 | genbank_AA027317 | AA027317 |
| 113702 | genbank_T97307 | T97307 |
| 113938 | genbank_W81598 | W81598 |
| 122635 | genbank_AA454085 | AA454085 |
| 108407 | genbank_AA075519 | AA075519 |
| 108432 | genbank_AA076626 | AA076626 |
| 108555 | genbank_AA084963 | AA084963 |
| 101349 | entrez_L77559 | L77559 |
| 124447 | genbank_N48000 | N48000 |
| 119071 | genbank_R31180 | R31180 |
| 103520 | entrez_Y10511 | Y10511 |
| 103663 | genbank_Z78291 | Z78291 |
| 128046 | 877605_1 | AA873285 AI025762 |
| 126959 | 546044_1 | AA199853 AA206355 |
| 123465 | genbank_AA599033 | AA599033 |

### MISSING AT THE TIME OF PUBLICATION

**TABLE 2: shows a preferred subset of the Accession numbers for genes found in Table 1 which are differentially expressed in prostate tumor tissue compared to normal prostate tissue.**

| | | | | |
|---|---|---|---|---|
| Pkey: | Unique Eos probeset identifier number | | | |
| ExAccn: | Exemplar Accession number, Genbank accession number | | | |
| UnigeneID: | Unigene number | | | |
| Unigene Title: | Unigene gene title | | | |
| R1: | Ratio of tumor to normal body tissue (Relaxed ratio (87/70) | | | |
| | | | | |

| **Pkey** | **ExAccn** | **UnigeneID** | **Unigene Title** | **R1** |
|---|---|---|---|---|
| | | | | |
| 131919 | AA121266 | Hs.272458 | ESTs | 37.2 |
| 120328 | AA196979 | Hs.290905 | ESTs; Weakly similar to (defline not ava | 32.6 |
| 101486 | M24902 | Hs.1852 | acid phosphatase; prostate | 25.2 |
| 119073 | R32894 | Hs.279477 | ESTs | 24.8 |
| 133428 | M34376 | Hs.183752 | microseminoprotein; beta- | 23.8 |
| 128180 | AA595348 | Hs.171995 | kallikrein 3; (prostate specific antigen | 21.4 |
| 104080 | AA402971 | Hs.57771 | Homo sapiens mRNA for serine protease (T | 18.9 |
| 127537 | AA569531 | Hs.162859 | ESTs | 18.6 |
| 131665 | R22139 | Hs.30343 | ESTs | 17.4 |
| 101050 | K01911 | Hs.1832 | neuropeptide Y | 17.3 |
| 130771 | N48056 | Hs.1915 | folate hydrolase (prostate-specific memb | 17 |
| 107485 | W63793 | Hs.262476 | S-adenosylmethionine decarboxylase 1 | 16.7 |
| 106155 | AA425309 | Hs.33287 | ESTs | 16.5 |
| 129534 | R73640 | Hs.11260 | ESTs | 16.4 |
| 100569 | HG2261-HT2351 | | | Antigen, Prostate Specific. Alt Splice 16 |
| 101889 | S39329 | Hs.181350 | kallikrein 2; prostatic | 15.4 |
| 135389 | U05237 | Hs.99872 | fetal Alzheimer antigen | 15 |
| 133944 | AA045870 | Hs.7780 | ESTs | 12.5 |
| 130974 | X57985 | Hs.2178 | H2B histone family; member Q | 11.8 |
| 114768 | AA149007 | Hs.182339 | ESTs | 11.8 |
| 104660 | AA007160 | Hs.14846 | ESTs | 11.4 |
| 131061 | N64328 | Hs.268744 | ESTs; Moderately similar to KIAA0273 [H. | 10.9 |
| 126645 | AI167942 | Hs.61635 | Homo sapiens BAC clone RG041D11 from 7q2 | 10.7 |
| 135153 | N40141 | Hs.95420 | Homo sapiens mRNA for JM27 protein; comp | 10.6 |
| 107033 | AA599629 | Hs.113314 | ESTs | 10.6 |
| 118417 | N66048 | | ESTs; Weakly similar to polymerase [H.sa | 10.5 |
| 126758 | W37145 | Hs.293960 | ESTs | 10.2 |
| 107102 | AA609723 | Hs.30652 | ESTs | 10.1 |
| 116787 | H28581 | Hs.15641 | ESTs | 10.1 |
| 115719 | AA416997 | Hs.59622 | ESTs | 10 |
| 123209 | AA489711 | Hs.203270 | ESTs | 9.9 |
| 101664 | M60752 | Hs.121017 | H2A histone family; member A | 9.8 |
| 112971 | T17185 | Hs.83883 | ESTs | 9.7 |
| 117984 | N51919 | Hs.106778 | ESTs | 9.7 |
| 129523 | M30894 | Hs.274509 | T-cell receptor, gamma cluster | 9.4 |
| 132964 | AA031360 | Hs.167133 | ESTs | 9.2 |
| 121853 | AA425887 | Hs.98502 | ESTs | 9 |
| 119617 | W47380 | Hs.55999 | ESTs | 8.9 |
| 105627 | AA281245 | Hs.23317 | ESTs | 8.8 |
| 101461 | M22430 | Hs.76422 | phospholipase A2; group IIA (platelets; | 8.7 |
| 124526 | N62096 | Hs.293185 | yz61c5.s1 Soares_multiple_sclerosis_2NbH | 8.5 |
| 133845 | T68510 | Hs.76704 | ESTs | 8.2 |
| 133354 | AA055552 | Hs.334762 | ESTs; Weakly similar to KIAA0319 [H.sapi | 8.1 |
| 119018 | N95796 | Hs.278695 | ESTs | 8 |
| 100394 | D84276 | Hs.66052 | CD38 antigen (p45) | 8 |
| 106579 | AA456135 | Hs.23023 | ESTs | 7.6 |
| 114965 | AA250737 | Hs.72472 | ESTs | 7.4 |
| 112033 | R43162 | Hs.22627 | ESTs | 7.1 |
| 102398 | U42359 | | Human N33 protein form 1 (N33) gene, exo | 7 |
| 101201 | L22524 | Hs.2256 | matrix metalloproteinase 7 (matrilysin; | 6.9 |
| 101803 | M86546 | Hs.155691 | pre-B-cell leukemia transcription factor | 6.8 |
| 120562 | AA280036 | Hs.302267 | ESTs; Weakly similar to W01A6.c [C.elega | 6.8 |
| 109112 | AA169379 | Hs.257924 | ESTs | 6.8 |
| 109795 | F10707 | Hs.326416 | ESTs | 6.7 |
| 130336 | X07730 | Hs.171995 | kallikrein 3; (prostate specific antigen | 6.6 |
| 131425 | AA219134 | Hs.26691 | ESTs | 6.6 |
| 132902 | AA490969 | Hs.59838 | ESTs | 6.6 |
| 133724 | U07919 | Hs.75746 | aldehyde dehydrogenase 6 | 6.5 |
| 120215 | Z41050 | Hs.108787 | Homo sapiens Mod4p homolog mRNA; complet | 6.5 |
| 131881 | AA010163 | Hs.3383 | upstream regulatory element binding prot | 6.5 |
| 100727 | X07290 | Hs.334786 | Human HF.12 gene mRNA | 6.3 |
| 121770 | AA421714 | Hs.278428 | Homo sapiens mRNA for KIAA0896 protein; | 6.3 |
| 123475 | AA599267 | Hs.250528 | ESTs; Weakly similar to ANKYRIN; BRAIN V | 6.3 |
| 133061 | AB000584 | Hs.296638 | prostate differentiation factor | 6.3 |
| 116429 | AA609710 | Hs.279923 | ESTs; Weakly similar to similar to GTP-b | 6.2 |
| 101233 | L29008 | Hs.878 | sorbitol dehydrogenase | 6.2 |
| 104691 | AA011176 | Hs.37744 | ESTs | 6.2 |
| 127248 | AA325029 | | EST27953 Cerebellum II Homo sapiens cDNA | 6.2 |
| 105500 | AA256485 | Hs.222399 | ESTs | 6.1 |
| 130828 | AA053400 | Hs.203213 | ESTs | 5.9 |
| 115357 | AA281793 | Hs.72988 | ESTs | 5.8 |
| 116334 | AA491457 | Hs.48948 | ESTs | 5.7 |
| 120132 | Z38839 | Hs.125019 | ESTs; Weakly similar to !!!! ALU SUBFAMI | 5.6 |
| 106375 | AA443993 | Hs.289072 | ESTs | 5.6 |
| 124777 | R41933 | Hs.140237 | ESTs; Weakly similar to neuronal thread | 5.6 |
| 101791 | M83822 | Hs.62354 | Human beige-like protein (BGL) mRNA; par | 5.5 |
| 117698 | N41002 | Hs.45107 | ESTs | 5.5 |
| 122041 | AA431407 | Hs.98732 | Homo sapiens Chromosome 16 BAC clone CIT | 5.5 |
| 133723 | AA088851 | Hs.262476 | S-adenosylmethionine decarboxylase 1 | 5.5 |
| 113938 | W81598 | | ESTs | 5.4 |
| 133015 | AA047036 | Hs.246315 | ESTs | 5.4 |
| 108186 | AA056482 | Hs.7780 | ESTs | 5.3 |
| 104466 | N25110 | Hs.326392 | Human guanine nucleotide exchange factor | 5.3 |
| 104033 | AA365031 | Hs.98944 | ESTs | 5.3 |
| 110844 | N31952 | Hs.167531 | ESTs; Weakly similar to (defline not ava | 5.3 |
| 129056 | H70627 | Hs.108336 | ESTs; Weakly similar to !!!! ALU SUBFAMI | 5.3 |
| 133493 | AA284143 | Hs.194369 | Homo sapiens chromosome 1 atrophin-1 rel | 5.3 |
| 129184 | W26769 | Hs.109201 | ESTs; Highly similar to (defline not ava | 5.2 |
| 101448 | M21389 | Hs.195850 | keratin 5 (epidermolysis bullosa simplex | 5.1 |
| 116188 | AA464728 | Hs.184598 | ESTs; Weakly similar to !!!! ALU SUBFAMI | 5.1 |
| 105921 | AA402613 | Hs.169119 | ESTs | 5.1 |
| 103375 | X91868 | Hs.54416 | sine oculis homeobox (Drosophila) homolo | 5.1 |
| 128871 | AA400271 | Hs.106778 | ESTs; Highly similar to (defline not ava | 5.1 |
| 116238 | AA479362 | Hs.47144 | ESTs | 5 |
| 102913 | X07696 | Hs.80342 | keratin 15 | 5 |
| 103011 | X52541 | Hs.326035 | early growth response 1 | 5 |
| 118981 | N93839 | Hs.39288 | ESTs; Weakly slmilar to !!!! ALU SUBFAMI | 5 |

**TABLE 2A shows the accession numbers for those primekeys lacking unigeneID's for Table 2. For each probeset we have listed the gene cluster number from which the oligonucleotides were designed. Gene clusters were compiled using sequences derived from Genbank ESTs and mRNAs. These sequences were clustered based on sequence similarity using Clustering and Alignment Tools (Double Twist, Oakland California). The Genbank accession numbers for sequences comprising each cluster are listed in the "Accession" column.**

| | | |
|---|---|---|
| Pkey: | Unique Eos probeset identifier number | |
| CAT number: | Gene cluster number | |
| Accession: | Genbank accession numbers | |
| | | |

| **Pkey** | **CAT number** | **Accession** |
|---|---|---|
| | | |
| 118417 | 37186_1 | AF080229 AF080231 AF080230 AF080232 AF080233 AF080234 BE550633 AI636743 AW614951 BE467547 AI680833 |
| | | AI633818 N29986 U87592 U87593 U87590 U97591 S46404 U87587 AA463992 AW206802 AI970376 AI583718 AI672574 |
| | | N25695 AW665466 AI818326 AA126128 AI480345 AW013827 AA248638 AI214968 AA204735 AA207155 AA206262 |
| | | AA204833 AW003247 AW496808 AI080480 AI631703 AI651023 AI867418 AW818140 AA502500 AI206199 AI671282 |
| | | AI352545 BE501030 AI652535 BE465762 AA206331 AW451866 AA471088 AA206342 AA204834 AA206100 AW021661 |
| | | AA332922 N66048 AA703396 H92278 AW139734 H92683 U87589 U87595 H69001 U87594 BE466420 AI624817 |
| | | BE466611 AI206344 AA574397 AA348354 AI493192 |
| 127248 | 227560_1 | AA364195 AA325029 AW962050 |
| 107033 | 235652_1 | AI141999 AA730176 R44544 R41778 AW300793 AW966157 AA918501 AA599629 AI082195 AI198537 AW006520 |
| | | AW236663 AW151420 AI826987 AI810832 AI669102 AI201981 N27331 AA335566 T84622 BE085347 BE085269 |
| 102398 | entrez_U42359 | U42359 |
| 113938 | genbank_W81598W81598 | |

**TABLE 3: shows genes, including expression sequence tags, differentially expressed in prostate tumor tissue compared to normal tissue as analyzed using the Affymetrix/Eos Hu02 GeneChip array. Shown are the relative amounts of each gene expressed in prostate tumor samples and various normal tissue samples showing the highest expression of the gene.**

| | | | | |
|---|---|---|---|---|
| Pkey: | | Unique Eos probeset identifier number | | |
| ExAccn: | | Exemplar Accession number, Genbank accession number | | |
| UnigeneID: | | Unigene number | | |
| Unigene Title: | | Unigene gene title | | |
| R1: | | Ratio of tumor to normal body tissue | | |
| | | | | |

| **Pkey** | **ExAccn** | **UnigeneID** | **Unigene Title** | **R1** |
|---|---|---|---|---|
| | | | | |
| 100131 | D12485 | Hs.11951 | phosphodiesterase l/nucleotide pyrophosp | 6.3 |
| 100235 | D29954 | Hs.13421 | KIAA0056 protein | 5.1 |
| 100570 | HG2261-HT2352 | Hs.171995 | Antigen, Prostate Specific, Alt. Splice | 9 |
| 100819 | HG4020-HT4290 | Hs.2387 | Transglutaminase | 10.5 |
| 101063 | L00354 | Hs.80247 | cholecystokinin | 8.5 |
| 101247 | L33801 | Hs.78802 | glycogen synthase kinase 3 beta | 4.7 |
| 101416 | M17254 | Hs.279477 | v-ets avian erythroblastosis virus E26 o | 4.7 |
| 101447 | M21305 | | Human alpha satellite and satellite 3 ju | 11 |
| 101485 | M24736 | Hs.89546 | selectin E (endothelial adhesion molecul | 9.8 |
| 101514 | M28214 | Hs.123072 | RAB3B; member RAS oncogene family | 6.2 |
| 101626 | M57399 | Hs.44 | pleiotrophin (heparin binding growth fac | 8.4 |
| 101663 | M60750 | Hs.2178 | H2B histone family; member A | 4.9 |
| 101758 | M77836 | Hs.79217 | pyrroline-5-carboxylate reductase 1 | 5.4 |
| 101768 | M81118 | Hs.78989 | | 7.5 |
| 101817 | M88163 | Hs.152292 | SWI/SNF related; matrix associated; acti | 5.5 |
| 101888 | M99701 | Hs.95243 | transcription elongation factor A (SII)- | 5.7 |
| 102031 | U04898 | Hs.2156 | RAR-related orphan receptor A | 13.2 |
| 102052 | U07559 | Hs.505 | ISL1 transcription factor; LIM/homeodoma | 8.9 |
| 102221 | U24576 | Hs.3844 | LIM domain only 4 | 5.6 |
| 102233 | U26173 | Hs.79334 | nuclear factor; interleukin 3 regulated | 7.4 |
| 102302 | U33052 | Hs.69171 | protein kinase C-like 2 | 8.2 |
| 102348 | U37519 | Hs.87539 | aldehyde dehydrogenase 8 | 5.9 |
| 102457 | U48807 | Hs.2359 | dual specificity phosphatase 4 | 5.1 |
| 102473 | U49957 | Hs.180398 | LIM domain-containing preferred transloc | 5.7 |
| 102669 | U71207 | Hs.29279 | eyes absent (Drosophila) homolog 2 | 9 |
| 102698 | U75272 | Hs.1867 | progastricsin (pepsinogen C) | 10.6 |
| 102751 | U80034 | Hs.68583 | mitochondrial intermediate peptidase | 15.6 |
| 102823 | U90914 | Hs.5057 | carboxypeptidase D | 4.9 |
| 102869 | X02544 | Hs.572 | orosomucoid 1 | 22.6 |
| 103031 | X54667 | Hs.123114 | cystatin S | 4.7 |
| 103043 | X55733 | Hs.93379 | eukaryotic translation initiation factor | 4.9 |
| 103093 | X60708 | Hs.44926 | dipeptidylpeptidase IV (CD26; adenosine | 5.8 |
| 103376 | X92098 | Hs.323378 | coated vesicle membrane protein | 5.2 |
| 103401 | X95240 | Hs.54431 | specific granule protein (28 kDa); cyste | 7.4 |
| 103613 | Z46629 | Hs.2316 | SRY (sex-determining region Y)-box 9 (ca | 5.2 |
| 103677 | Z83806 | | H.sapiens mRNA for axonemal dynein heavy | 4.9 |
| 103962 | AA298180 | Hs.83243 | ESTs | 6 |
| 104084 | AA410529 | Hs.30732 | ESTs | 6.4 |
| 104257 | AF006265 | Hs.9222 | estrogen receptor-binding fragment-assoc | 6.8 |
| 104301 | D45332 | Hs.6783 | ESTs | 10.5 |
| 104769 | AA025887 | Hs.293943 | ESTs; Weakly similar to !!!! ALU SUBFAMI | 6.3 |
| 104851 | AA040882 | Hs.10290 | U5 snRNP-specific 40 kDa protein (hPrp8- | 4.9 |
| 104896 | AA054228 | Hs.23165 | ESTs | 5.8 |
| 104956 | AA074880 | Hs.20509 | ESTs; Weakly similar to hypothetical pro | 6.4 |
| 104957 | AA074919 | Hs.10026 | ESTs; Weakly similar to ORF YJL063c [S.c | 4.8 |
| 104967 | AA084506 | Hs.291000 | ESTs | 6.5 |
| 105099 | AA150776 | Hs.23729 | Homo sapiens clone 24405 mRNA sequence | 7 |
| 105298 | AA233459 | Hs.26369 | ESTs | 5.1 |
| 105304 | AA233553 | Hs.190325 | ESTs | 4.7 |
| 105370 | AA236476 | Hs.22791 | ESTs; Weakly similar to transmembrane pr | 10.3 |
| 105427 | AA251330 | Hs.28248 | ESTs | 5 |
| 105542 | AA261858 | Hs.266957 | ESTs; Weakly similar to heat shock prote | 8.8 |
| 105628 | AA281251 | Hs.79828 | ESTs; Weakly similar to putative zinc fi | 5.5 |
| 105640 | AA281623 | Hs.6685 | ESTs; Weakly similar to KIAA0742 protein | 8 |
| 105645 | AA282138 | Hs.11325 | ESTs | 14 |
| 105691 | AA287097 | Hs.289068 | transcription factor 4 | 6.3 |
| 105730 | AA292701 | Hs.5364 | DKFZP564l052 protein | 4.9 |
| 105808 | AA393808 | Hs.286131 | KIAA0438 gene product | 7 |
| 105826 | AA398243 | Hs.194477 | ESTs; Moderately similar to similar to N | 5 |
| 105903 | AA401433 | Hs.200016 | ESTs; Weakly similar to diphosphoinosito | 9.9 |
| 105906 | AA401633 | Hs.22380 | ESTs | 11.5 |
| 106065 | AA417558 | Hs.25206 | ESTs | 5.1 |
| 106094 | AA419461 | Hs.23317 | ESTs | 10.9 |
| 106157 | AA425367 | Hs.34892 | ESTs | 6.6 |
| 106184 | AA426643 | Hs.10762 | ESTs | 8.5 |
| 106211 | AA428240 | Hs.126083 | ESTs | 8.4 |
| 106213 | AA428258 | Hs.8769 | Homo sapiens mRNA; cDNA DKFZp564E153 (fr | 5.7 |
| 106272 | AA432074 | Hs.323099 | ESTs | 5.8 |
| 106369 | AA443828 | Hs.288856 | ESTs | 6.3 |
| 106400 | AA447621 | Hs.94109 | ESTs | 5.4 |
| 106474 | AA450212 | Hs.42484 | Homo sapiens mRNA; cDNA DKFZp564C053 (fr | 9.2 |
| 106507 | AA452584 | Hs.267819 | protein phosphatase 1; regulatory (inhib | 5.6 |
| 106523 | AA453441 | Hs.31511 | ESTs | 4.7 |
| 106532 | AA453628 | Hs.37443 | ESTs | 4.7 |
| 106557 | AA455087 | Hs.22247 | ESTs | 5.7 |
| 106575 | AA456039 | Hs.105421 | ESTs | 7.2 |
| 106618 | AA459249 | Hs.8715 | ESTs; Weakly similar to Similarity with | 5.6 |
| 106820 | AA481037 | Hs.12592 | ESTs | 5.4 |
| 106846 | AA485223 | Hs.34892 | ESTs | 5.3 |
| 106973 | AA505141 | Hs.11923 | Human DNA sequence from clone 167A19 on | 7.5 |
| 107110 | AA609952 | Hs.12784 | KIAA0293 protein | 6.1 |
| 107127 | AA620504 | Hs.179898 | ESTs | 7.1 |
| 107159 | AA621340 | Hs.10600 | ESTs; Weakly similar to ORF YKR081c [S.c | 5.2 |
| 107217 | D51095 | Hs.35861 | DKFZP586E1621 protein | 15.1 |
| 107365 | U78294 | Hs.111256 | arachidonate 15-lipoxygenase; second typ | 4.7 |
| 107630 | AA007218 | Hs.60178 | ESTs | 5.3 |
| 107734 | AA016225 | Hs.7517 | ESTs | 4.8 |
| 107760 | AA018042 | Hs.252085 | EST | 7.6 |
| 107997 | AA037388 | Hs.82223 | Human DNA sequence from clone 141H5 on c | 10.5 |
| 108012 | AA039616 | Hs.173334 | ESTs | 6.5 |
| 108520 | AA084138 | Hs.46786 | ESTs | 7.9 |
| 108583 | AA088276 | Hs.68826 | ESTs | 5.8 |
| 108613 | AA100967 | Hs.69165 | ESTs | 6 |
| 108664 | AA113349 | Hs.69588 | EST | 6.3 |
| 108677 | AA115629 | Hs.118531 | ESTs | 5.9 |
| 108807 | AA129968 | Hs.49376 | ESTs; Weakly similar to PROTEIN PHOSPHAT | 5.8 |
| 108910 | AA136590 | | ESTs | 5 |
| 108933 | AA147224 | Hs.337232 | ESTs | 12.7 |
| 108948 | AA149579 | Hs.118258 | ESTs | 6.8 |
| 109014 | AA156790 | Hs.262036 | ESTs | 15.3 |
| 109124 | AA171529 | Hs.183887 | ESTs | 6.1 |
| 109142 | AA176438 | Hs.41295 | ESTs | 5.1 |
| 109277 | AA196332 | Hs.86043 | ESTs | 5.5 |
| 109342 | AA213620 | | Homo sapiens mRNA; cDNA DKFZp586M1418 (f 6 | |
| 109562 | F01811 | Hs.187931 | ESTs; Moderately similar to voltage-gate | 10.8 |
| 109565 | F01930 | Hs.23648 | ESTs | 7 |
| 109648 | F04600 | Hs.7154 | ESTs | 9.9 |
| 109799 | F10770 | Hs.180378 | Homo sapiens clone 669 unknown mRNA; com | 6.4 |
| 109859 | H02308 | Hs.20792 | ESTs | 5.3 |
| 110181 | H20276 | Hs.31742 | ESTs | 16.8 |
| 110854 | N32919 | Hs.27931 | ESTs | 10 |
| 110924 | N47938 | Hs.12940 | yy84a09.s1 Soares_multiple_sclerosis_2Nb | 5.6 |
| 111046 | N55514 | Hs.318584 | ESTs | 6.9 |
| 111091 | N59858 | Hs.33032 | Homo sapiens mRNA; cDNA DKFZp434N185 (fr | 5.2 |
| 111157 | N66613 | Hs.99364 | ESTs | 5 |
| 111164 | N66857 | Hs.122489 | ESTs; Weakly similar to !!!! ALU CLASS C | 5.6 |
| 111221 | N68869 | Hs.15119 | ESTs | 6.2 |
| 111348 | N90041 | Hs.9585 | ESTs | 5.4 |
| 111353 | N90430 | Hs.6616 | ESTs | 5.3 |
| 111495 | R07210 | Hs.9683 | ESTs | 5.8 |
| 111540 | R08850 | Hs.9786 | ESTs | 6 |
| 111579 | R10657 | Hs.167115 | KIAA0830 protein | 12.6 |
| 111581 | R10684 | Hs.5794 | ESTs | 7.1 |
| 111734 | R25375 | Hs.128749 | ESTs | 6.2 |
| 111861 | R37460 | Hs.25231 | ESTs | 9.4 |
| 111870 | R37778 | Hs.18685 | ESTs; Weakly similar to hypothetical pro | 6.5 |
| 111937 | R40431 | Hs.14846 | Homo sapiens mRNA; cDNA DKFZp564D016 (fr | 4.8 |
| 111987 | R42036 | Hs.6763 | KIAA0942 protein | 6.4 |
| 112184 | R49173 | Hs.330242 | ESTs | 5.6 |
| 112286 | R53765 | Hs.158135 | KIAA0981 protein | 9.3 |
| 112380 | R59740 | Hs.5740 | ESTs | 4.7 |
| 112452 | R63841 | Hs.157461 | ESTs | 6 |
| 112601 | R79111 | Hs.78225 | annexin A1 | 5.4 |
| 112753 | R93696 | Hs.169882 | ESTs | 5.8 |
| 112902 | T09262 | Hs.129190 | ESTs | 5.1 |
| 112984 | T23457 | Hs.289014 | ESTs | 4.9 |
| 113021 | T23855 | Hs.129836 | KIAA1028 protein | 10.8 |
| 113083 | T40530 | Hs.266957 | ESTs; Weakly similar to heat shock prote | 5.7 |
| 113200 | T57773 | Hs.10263 | ESTs | 7.3 |
| 113494 | T88878 | Hs.86538 | ESTs | 8.7 |
| 113849 | W60439 | Hs.8858 | ESTs; Moderately similar to cbp146 [M.mu | 4.9 |
| 113883 | W72382 | Hs.11958 | oxidative 3 alpha hydroxysteroid dehydro | 4.7 |
| 113950 | W85765 | Hs.30504 | Homo sapiens mRNA; cDNA DKFZp434E082 (fr | 6.7 |
| 113986 | W87462 | Hs.21894 | ESTs | 5.9 |
| 113989 | W87544 | Hs.268828 | ESTs | 4.7 |
| 114124 | Z38595 | Hs.125019 | ESTs; Highly similar to KIAA0886 protein | 21.3 |
| 114340 | Z41395 | Hs.143611 | ESTs | 9.6 |
| 114346 | Z41450 | Hs.130489 | ESTs | 5.2 |
| 114435 | AA018216 | Hs.164975 | Bicaudal D (Drosophila) homolog 1 | 7.4 |
| 114463 | AA025370 | Hs.40109 | KIAA0872 protein | 8.2 |
| 114652 | AA101416 | Hs.107149 | ESTs; Weakly similar to PTB-ASSOCIATED S | 5.4 |
| 114721 | AA131450 | Hs.103822 | ESTs | 4.8 |
| 114730 | AA133527 | Hs.331328 | ESTs; Weakly similar to The KIAA0138 gen | 5.1 |
| 114833 | AA234362 | Hs.87159 | ESTs; Moderately similar to CGI-66 prote | 5.5 |
| 114860 | AA235112 | Hs.42179 | ESTs; Moderately similar to similar to m | 6.3 |
| 114884 | AA235811 | Hs.293672 | ESTs | 5.2 |
| 114895 | AA236177 | Hs.76591 | KIAA0887 protein | 4.7 |
| 114908 | AA236545 | Hs.54973 | ESTs | 5.2 |
| 114932 | AA242751 | Hs.16218 | KIAA0903 protein | 5.7 |
| 115084 | AA255566 | Hs.42484 | Homo sapiens mRNA; cDNA DKFZp564C053 (fr | 5.2 |
| 115140 | AA258030 | Hs.279938 | ESTs; Weakly similar to supported by GEN | 5.9 |
| 115468 | AA287061 | Hs.48499 | ESTs; Highly similar to Bdeight protein | 4.7 |
| 115583 | AA398913 | Hs.45231 | LDOC1 protein | 7.6 |
| 115709 | AA412519 | Hs.58279 | ESTs | 4.8 |
| 115772 | AA423972 | Hs.131740 | ESTs | 5 |
| 115774 | AA424029 | Hs.288390 | ESTs; Moderately similar to dynamin; int | 5.4 |
| 115776 | AA424038 | Hs.81897 | ESTs | 5 |
| 115821 | AA427528 | Hs.130965 | ESTs; Weakly similar to ZINC FINGER PROT | 13.7 |
| 115955 | AA446121 | Hs.44198 | Homo sapiens BAC clone RG054D04 from 7q3 | 10.6 |
| 116024 | AA451748 | Hs.83883 | Human DNA sequence from clone 718J7 on c | 6.8 |
| 116108 | AA457566 | Hs.28777 | ESTs | 6 |
| 116117 | AA459117 | Hs.31575 | SEC63; endoplasmic reticulum translocon | 7.3 |
| 116146 | AA460701 | Hs.15423 | ESTs | 5.5 |
| 116296 | AA489033 | Hs.62601 | Homo sapiens mRNA; cDNA DKFZp586K1318 | (f 5.7 |
| 116379 | AA521472 | Hs.71252 | ESTs | 5.9 |
| 116393 | AA599463 | Hs.306051 | protein phosphatase 2 (formerly 2A); reg | 5.9 |
| 116401 | AA599963 | Hs.59698 | ESTs | 7.9 |
| 116416 | AA609219 | Hs.39982 | ESTs | 9.2 |
| 116587 | D59325 | Hs.121429 | ESTs | 5.2 |
| 116601 | D80055 | Hs.45140 | ESTs | 4.9 |
| 116684 | F09156 | Hs.66095 | ESTs | 7.2 |
| 116722 | F13654 | | HSFIH32 Stratagene cat#937212 (1992) Hom | 5.5 |
| 116766 | H13260 | Hs.95097 | ESTs | 5.9 |
| 117453 | N29568 | Hs.108319 | thyroid hormone receptor-associated prot | 6.9 |
| 117557 | N33920 | Hs.44532 | diubiquitin | 4.8 |
| 117708 | N45114 | Hs.126280 | ESTs | 6.3 |
| 118001 | N52151 | Hs.47447 | ESTs | 11.4 |
| 118229 | N62339 | Hs.166254 | heat shock 90kD protein 1; alpha | 6.2 |
| 118599 | N69207 | Hs.203697 | ESTs | 5.8 |
| 118645 | N70358 | Hs.125180 | growth hormone receptor | 7.1 |
| 118873 | N89881 | Hs.44577 | ESTs | 6 |
| 118985 | N94303 | Hs.55028 | ESTs | 9.3 |
| 119107 | R42424 | Hs.63841 | ESTs | 6 |
| 119126 | R45175 | Hs.117183 | ESTs | 17.9 |
| 119271 | T16387 | Hs.65328 | ESTs | 6 |
| 119367 | T78324 | Hs.250895 | ESTs | 5 |
| 119721 | W69440 | Hs.48376 | ESTs | 15.4 |
| 119741 | W70205 | Hs.43670 | kinesin family member 3A | 10.1 |
| 119780 | W72967 | Hs.191381 | ESTs; Weakly similar to hypothetical pro | 5.3 |
| 120217 | Z41078 | Hs.66035 | ESTs | 4.8 |
| 120266 | AA173939 | Hs.205442 | ESTs; Weakly similar to inner centromere | 8.8 |
| 120294 | AA190888 | Hs.153881 | ESTs; Highly similar to NY-REN-62 antige | 4.9 |
| 120418 | AA236010 | Hs.26613 | Homo sapiens mRNA; cDNA DKFZp586F1323 | (f 4.7 |
| 120486 | AA253400 | Hs.137569 | tumor protein 63 kDa with strong homolog | 5.6 |
| 120524 | AA261852 | Hs.192905 | ESTs | 4.9 |
| 120571 | AA280738 | Hs.34892 | ESTs | 8.8 |
| 120596 | AA282074 | Hs.237323 | ESTs | 6.2 |
| 120713 | AA292655 | Hs.96557 | ESTs | 9.9 |
| 120992 | AA398246 | Hs.97594 | ESTs | 16.4 |
| 121429 | AA406293 | Hs.41167 | ESTs | 6.9 |
| 121503 | AA412049 | Hs.290347 | ESTs | 7.6 |
| 121512 | AA412105 | Hs.193736 | ESTs | 5.8 |
| 121816 | AA424814 | Hs.48827 | ESTs | 4.6 |
| 122027 | AA431302 | Hs.98721 | EST; Weakly similar to N-copine [H.sapie | 5.6 |
| 122294 | AA437311 | Hs.98927 | ESTs | 5.7 |
| 122411 | AA446859 | Hs.99083 | ESTs | 6.5 |
| 122791 | AA460158 | Hs.129836 | KIAA1028 protein | 12.4 |
| 122792 | AA460225 | Hs.99519 | ESTs | 5.1 |
| 122969 | AA478539 | Hs.104336 | ESTs | 4.9 |
| 123095 | AA485724 | Hs.27413 | ESTs | 5.4 |
| 123100 | AA485957 | Hs.306219 | Homo sapiens clone 25032 mRNA sequence | 5 |
| 123295 | AA495981 | Hs.250830 | ESTs | 4.7 |
| 123311 | AA496252 | Hs.105069 | ESTs | 7.4 |
| 123583 | AA609006 | Hs.111240 | ESTs | 9.1 |
| 123619 | AA609200 | | ESTs | 4.7 |
| 123645 | AA609310 | Hs.188691 | ESTs | 4.8 |
| 123709 | AA609651 | Hs.112742 | ESTs | 7 |
| 123968 | C14333 | Hs.108327 | damage-specific DNA binding protein 1 (1 | 5 |
| 124178 | H45996 | Hs.97101 | putative G protein-coupled receptor | 6.8 |
| 124352 | N21626 | Hs.102406 | ESTs | 10.2 |
| 124357 | N22401 | | yw37g07.s1 Morton Fetal Cochlea Homo sap | 10.6 |
| 124515 | N58172 | Hs.109370 | ESTs | 14.2 |
| 124911 | R88992 | Hs.174195 | ESTs | 4.8 |
| 125154 | W38419 | | ESTs | 4.7 |
| 125992 | W01626 | | za36e07.r1 Soares fetal liver spleen 1NF | 5.1 |
| 126802 | AA947601 | Hs.97056 | ESTs | 5.1 |
| 126812 | Z36290 | Hs.173933 | ESTs; Weakly similar to NUCLEAR FACTOR 1 | 4.6 |
| 127080 | AA662913 | Hs.190173 | ESTs | 5 |
| 127308 | AA507628 | Hs.334390 | ESTs | 4.8 |
| 127370 | AI024352 | Hs.70337 | immunoglobulin superfamily; member 4 | 4.7 |
| 127386 | AI457411 | Hs.106728 | ESTs | 4.8 |
| 127965 | AA828760 | Hs.292059 | ESTs | 4.8 |
| 128172 | AI400862 | Hs.265130 | ESTs | 5 |
| 128305 | AI039722 | Hs.279009 | ESTs | 5.8 |
| 128420 | AI088155 | Hs.41296 | ESTs; Weakly similar to unknown [H.sapie | 17 |
| 128467 | AA176446 | Hs.180428 | ESTs; Weakly similar to hypothetical 43. | 4.8 |
| 128610 | L38606 | Hs.10247 | activated leucocyte cell adhesion molecu | 7.9 |
| 128625 | AA242816 | Hs.102652 | ESTs; Weakly similar to KIAA0437 [H.sapi | 8.1 |
| 128651 | AA446990 | Hs.103135 | ESTs | 6.5 |
| 129088 | AA215971 | Hs.194431 | KIAA0992 protein | 5.2 |
| 129136 | N26391 | Hs.250723 | ESTs | 5.1 |
| 129171 | AA234048 | Hs.7753 | calumenin | 5.8 |
| 129229 | AA211941 | Hs.109643 | polyadenylate binding protein-interactin | 5.8 |
| 129386 | N27524 | Hs.260024 | Cdc42 effector protein 3 | 5.2 |
| 129467 | AA410311 | Hs.44208 | ESTs | 5.1 |
| 129564 | H22136 | Hs.75295 | guanylate cyclase 1; soluble; alpha 3 | 16.3 |
| 129699 | AA458578 | Hs.12017 | KIAA0439 protein; homolog of yeast ubiqu | 9.2 |
| 129821 | F11019 | Hs.12696 | cortactin SH3 domain-binding protein | 8.6 |
| 129823 | X00948 | Hs.105314 | relaxin 2 (H2) | 9.1 |
| 129847 | W46767 | Hs.296178 | ESTs; Weakly similar to RNA POLYMERASE I | 5.4 |
| 129912 | AA047344 | Hs.107213 | ESTs; Highly similar to NY-REN-6 antigen | 6.5 |
| 129958 | L20591 | Hs.1378 | annexin A3 | 5.1 |
| 129977 | J04076 | Hs.1395 | early growth response 2 (Krox-20 (Drosop | 8.6 |
| 130061 | U82256 | Hs.172851 | arginase; type II | 7.4 |
| 130241 | U78313 | Hs.153203 | MyoD family inhibitor | 4.9 |
| 130466 | N21679 | Hs.180059 | ESTs | 5.8 |
| 130541 | X05608 | Hs.211584 | neurofilament; light polypeptide (68kD) | 6.7 |
| 130619 | AA477739 | Hs.12532 | ESTs | 6.4 |
| 130925 | N71935 | Hs.169378 | multiple PDZ domain protein | 7.9 |
| 130938 | AA013250 | Hs.21398 | ESTs; Moderately similar to PUTATIVE GLU | 6.2 |
| 130971 | H20332 | Hs.301444 | signal sequence receptor, gamma (transio | 6.4 |
| 131066 | F09006 | Hs.22588 | ESTs | 5 |
| 131126 | F09012 | Hs.181326 | myotubularin related protein 2 | 6.4 |
| 131310 | J02960 | Hs.2551 | adrenergic; beta-2-; receptor, surface | 7.9 |
| 131487 | AA253220 | Hs.27373 | Homo sapiens mRNA; cDNA DKFZp564O1763 (f | 5.9 |
| 131561 | X59841 | Hs.294101 | pre-B-cell leukemia transcription factor | 7.6 |
| 131562 | U90551 | Hs.28777 | H2A histone family; member L | 5.1 |
| 131579 | N62922 | Hs.29088 | ESTs | 11 |
| 131629 | AA442119 | Hs.238809 | ESTs | 4.9 |
| 131682 | AA428368 | Hs.30654 | ESTs | 4.8 |
| 131699 | R68657 | Hs.90421 | ESTs; Moderately similar to !!!! ALU SUB | 6.5 |
| 131795 | N32724 | Hs.32317 | Sox-like transcriptional factor | 5.6 |
| 132053 | H93381 | Hs.38085 | ESTs; Weakly similar to putative glycine | 7.2 |
| 132122 | U65092 | Hs.40403 | Cbp/p300-interacting transactivator; wit | 5.6 |
| 132191 | AA449431 | Hs.288361 | KIAA0741 gene product | 8 |
| 132256 | AA608856 | Hs.431 | murine leukemia viral (bmi-1) oncogene h | 5.5 |
| 132482 | AA429478 | Hs.238126 | ESTs; Highly similar to CGI-49 protein [ | 6.6 |
| 132533 | AA021608 | Hs.172510 | ESTs | 5.8 |
| 132572 | AA448297 | Hs.237825 | signal recognition particle 72kD | 6.2 |
| 132581 | R42266 | Hs.52256 | ESTs; Weakly similar to beta-TrCP protei | 16 |
| 132700 | N47109 | Hs.5521 | ESTs | 6.8 |
| 132701 | AA279359 | Hs.55220 | BCL2-associated athanogene 2 | 5.3 |
| 132725 | L41887 | Hs.184167 | splicing factor; arginine/serine-rich 7 | 7.8 |
| 132783 | N74897 | Hs.278894 | DEAD/H (Asp-Glu-Ala-Asp/His) box polypep | 5.9 |
| 132790 | X75535 | Hs.168670 | peroxisomal famesylated protein | 8 |
| 132939 | U76189 | Hs.61152 | exostoses (multiple)-like 2 | 5.2 |
| 133142 | F03321 | Hs.65874 | ESTs | 5.2 |
| 133342 | U29589 | Hs.7138 | cholinergic receptor; muscarinic 3 | 10.3 |
| 133434 | AA278852 | Hs.30212 | ESTs | 5.8 |
| 133453 | M68941 | Hs.73826 | protein tyrosine phosphatase; non-recept | 4.9 |
| 133520 | X74331 | Hs.74519 | primase; polypeptide 2A (58kD) | 13.1 |
| 133544 | T33873 | Hs.74624 | protein tyrosine phosphatase; receptor t | 4.6 |
| 133608 | D13315 | Hs.75207 | glyoxalase I | 4.8 |
| 133626 | H75939 | Hs.75277 | Homo sapiens mRNA; cDNA DKFZp586M141 (fr | 5 |
| 133633 | D21262 | Hs.75337 | nucleolar phosphoprotein p130 | 6.3 |
| 133797 | S66431 | Hs.76272 | retinoblastoma-binding protein 2 | 6 |
| 133928 | N34096 | Hs.7766 | ubiquitin-conjugating enzyme E2E 1 (homo | 5.4 |
| 134095 | U47414 | Hs.79069 | cyclin G2 | 5.2. |
| 134249 | N89827 | Hs.80667 | RALBP1 associated Eps domain containing | 6.5 |
| 134321 | AA418230 | Hs.8172 | ESTs | 7 |
| 134453 | X70683 | Hs.83484 | SRY (sex determining region Y)-box 4 | 4.7 |
| 134542 | X57025 | Hs.85112 | insulin-like growth factor 1 (somatomedi | 7.7 |
| 134570 | U66615 | Hs.172280 | SWI/SNF related; matrix associated; acti | 6.4 |
| 134592 | U82613 | Hs.289104 | Alu-binding protein with zinc finger dom | 5.4 |
| 134654 | W23625 | Hs.8739 | ESTs; Weakly similar to ORF YGR200c [S.c | 5 |
| 134666 | AA482319 | Hs.8752 | putative type II membrane protein | 5.4 |
| 134806 | Z49099 | Hs.89718 | spermine synthase | 6.7 |
| 134951 | AA431480 | Hs.169358 | ESTs | 9.8 |
| 135066 | X04602 | Hs.93913 | interleukin 6 (interferon; beta 2) | 5.7 |
| 135155 | AA358268 | Hs.166556 | ESTs; Moderately similar to transcriptio | 4.9 |
| 135411 | L10333 | Hs.99947 | reticulon 1 | 5.3 |
| 300023 | M10098 | | AFFX control: 18S ribosomal RNA | 4.6 |
| 300254 | AW079607 | Hs.55610 | ESTs; Weakly similar to ZnT-3 [H.sapiens | 7.8 |
| 300273 | AW013907 | Hs.167531 | ESTs; Moderately similar to predicted us | 11.5 |
| 300319 | AW157646 | Hs.153506 | ESTs; Weakly similar to microtubule-acti | 8.5 |
| 300566 | H86709 | Hs.326392 | son of sevenless (Drosophila) homolog 1 | 5.8 |
| 300578 | AI989417 | Hs.134289 | ESTs | 4.4 |
| 300671 | AI239706 | Hs.93810 | ESTs | 7.9 |
| 300675 | AA039352 | Hs.125034 | ESTs; Weakly similar to ORF YDL040c [S.c | 4.5 |
| 300680 | AW468066 | Hs.24817 | ESTs; Weakly similar to KIAA0986 protein | 5.2 |
| 300762 | AI497778 | Hs.20509 | ESTs | 6.4 |
| 300810 | AI076890 | Hs.146847 | ESTs | 5.8 |
| 300813 | AA406411 | Hs.208341 | ESTs; Weakly similar to KIAA0989 protein | 10.6 |
| 300823 | AI863068 | Hs.106823 | ESTs; Weakly similar to putative zinc fi | 5.6 |
| 300834 | AF109300 | Hs.147924 | ESTs | 6.7 |
| 300923 | AW136372 | Hs.1852 | ESTs | 7.6 |
| 300962 | AA593373 | Hs.293744 | ESTs | 5.5 |
| 301015 | AA947682 | Hs.20252 | ESTs; Weakly similar to Chain A; Cdc42hs | 7 |
| 301042 | AI659131 | Hs.197733 | ESTs | 24.9 |
| 301242 | AW161535 | Hs.23782 | ESTs | 11.8 |
| 301254 | AI049624 | Hs.283390 | EST cluster (not in UniGene) with exon h | 4.3 |
| 301262 | H29500 | Hs.7130 | ESTs; Moderately similar to N-copine [H. | 4.3 |
| 301388 | AA156879 | Hs.262036 | ESTs; Weakly similar to ZINC FINGER PROT | 6.6 |
| 301563 | AI802946 | Hs.44208 | ESTs; Weakly similar to match to ESTs AA | 5.7 |
| 301656 | AW008475 | Hs.151258 | EST cluster (not in UniGene) with exon h | 6.8 |
| 301689 | Z44810 | Hs.301789 | ESTs; Weakly similar to similar to C.ele | 6.3 |
| 301783 | AL046347 | Hs.83937 | Homo sapiens PAC clone DJ1159O04 from 7p | 6.2 |
| 301805 | AI800004 | Hs.142846 | ESTs; Weakly similar to MesP1 [M.musculu | 8.5 |
| 301846 | R20002 | Hs.6823 | ESTs; Weakly similar to intrinsic factor | 4.6 |
| 301891 | AF131855 | Hs.279591 | Homo sapiens clone 25056 mRNA sequence | 6.3 |
| 302005 | AI869666 | Hs.123119 | ESTs | 36.8 |
| 302056 | AI457532 | Hs.30488 | ESTs; Moderately similar to ROSA26AS [M. | 9.5 |
| 302067 | H05698 | Hs.222399 | ESTs; Weakly similar to protein-tyrosine | 5.8 |
| 302099 | AL021397 | Hs.137576 | ribosomal protein L34 pseudogene 1 | 8.8 |
| 302147 | AB022660 | Hs.151717 | KIAA0437 protein | 5.9 |
| 302214 | AJ001454 | Hs.159425 | Homo sapiens mRNA for testican-3 | 4.3 |
| 302236 | AI128606 | Hs.6557 | zinc finger protein 161 | 4.3 |
| 302358 | D81150 | Hs.322848 | EST cluster (not in UniGene) with exon h | 5.5 |
| 302410 | NM_004917 | Hs.218366 | EST cluster (not in UniGene) with exon h | 26.8 |
| 302486 | AC003682 | Hs.183512 | multiple UniGene matches | 8.2 |
| 302582 | NM_000522 | Hs.249195 | EST cluster (not in UniGene) with exon h | 6.4 |
| 302785 | AA425562 | Hs.11065 | EST cluster (not in UniGene) with exon h | 5 |
| 302792 | AA343696 | Hs.46821 | ESTs; Weakly similar to putative [H.sapi | 4.8 |
| 302881 | AA508353 | Hs.105314 | relaxin 1 (H1) | 78.8 |
| 302892 | N58545 | Hs.42346 | histone deacetylase 3 | 8.5 |
| 302970 | AW118352 | Hs.312679 | EST cluster (not in UniGene) with exon h | 7.4 |
| 302977 | AW263124 | Hs.315111 | EST cluster (not in UniGene) with exon h | 5.5 |
| 303029 | AF199613 | | EST cluster (not in UniGene) with exon h | 4.6 |
| 303125 | AF161352 | Hs.111782 | EST cluster (not in UniGene) with exon h | 5.8 |
| 303280 | AI571580 | Hs.170307 | ESTs | 4.3 |
| 303306 | AA215297 | Hs.61441 | EST cluster (not in UniGene) with exon h | 6.4 |
| 303309 | AL134164 | Hs.145416 | ESTs | 6.6 |
| 303344 | AA255977 | Hs.250646 | ESTs; Highly similar to ubiquitin-conjug | 19.5 |
| 303380 | AA298471 | Hs.326567 | EST cluster (not in UniGene) with exon h | 6.6 |
| 303401 | AA758552 | Hs.309497 | ESTs | 6.8 |
| 303525 | AW516519 | Hs.273294 | ESTs | 4.8 |
| 303526 | AA348111 | Hs.96900 | ESTs | 12.1 |
| 303540 | AA355607 | Hs.309490 | ESTs; Weakly similar to MMSET type I [H. | 8.2 |
| 303572 | AW338520 | Hs.242540 | ESTs | 8.4 |
| 303685 | AW500106 | Hs.23643 | EST cluster (not in UniGene) with exon h | 4.9 |
| 303699 | D30891 | Hs.19525 | EST cluster (not in UniGene) with exon h | 15.7 |
| 303702 | AW500748 | Hs.224961 | ESTs; Weakly similar to 73 kDA subunit o | 6.3 |
| 303718 | AI741397 | Hs.114658 | ESTs | 4.6 |
| 303722 | AA521510 | Hs.145010 | ESTs | 12.5 |
| 303732 | AW502405 | Hs.125759 | ESTs; Weakly similar to tumor suppressor | 4.3 |
| 303735 | AA707750 | Hs.169055 | ESTs; Weakly similar to cis-Golgi matrix | 5.4 |
| 303752 | AI017286 | Hs.5957 | EST cluster (not in UniGene) with exon h | 5.3 |
| 303753 | AW503733 | Hs.9414 | ESTs | 13 |
| 303813 | AI275850 | Hs.114658 | EST cluster (not in UniGene) with exon h | 7.8 |
| 304053 | R00493 | Hs.125565 | translocase of inner mitochondrial membr | 4.8 |
| 304218 | N66373 | Hs.27973 | ESTs; Weakly similar to ZK354.7 [C.elega | 6 |
| 305200 | AA668128 | Hs.45207 | EST singleton (not in UniGene) with exon | 5.7 |
| 306716 | AI024916 | Hs.251354 | ESTs | 5.7 |
| 307848 | AI364186 | | EST singleton (not in UniGene) with exon | 7.3 |
| 307871 | AI368665 | Hs.31476 | EST singleton (not in UniGene) with exon | 5.4 |
| 308050 | AI460004 | Hs.31608 | EST singleton (not in UniGene) with exon | 8.1 |
| 308362 | AI613519 | Hs.105749 | EST singleton (not in UniGene) with exon | 5.5 |
| 308923 | AI863051 | Hs.279815 | ESTs | 4.4 |
| 309116 | AI927149 | Hs.29797 | ribosomal protein L10 | 4.5 |
| 309375 | AW075342 | Hs.9271 | EST singleton (not in UniGene) with exon | 7.4 |
| 309674 | AW205604 | Hs.266009 | ESTs; Weakly similar to !!!! ALU SUBFAMI | 5 |
| 310095 | AI921750 | Hs.144871 | ESTs | 5 |
| 310098 | AI685841 | Hs.161354 | ESTs | 11.6 |
| 310250 | AI478629 | Hs.158465 | ESTs | 5.8 |
| 310365 | AI262148 | Hs.145569 | ESTs | 9.7 |
| 310382 | AI734009 | Hs.127699 | EST cluster (not in UniGene) | 10.4 |
| 310409 | AI612775 | Hs.145710 | ESTs | 4.6 |
| 310431 | AI420227 | Hs.149358 | ESTs | 72.9 |
| 310573 | AW292180 | Hs.156142 | ESTs | 7.6 |
| 310598 | AI338013 | Hs.140546 | ESTs | 9.2 |
| 310639 | AW269082 | Hs.175162 | ESTs | 4.5 |
| 310787 | AW262580 | Hs.147674 | ESTs | 4.9 |
| 310816 | AI973051 | Hs.224965 | ESTs | 7.6 |
| 311251 | AI655662 | Hs.197698 | ESTs | 41.3 |
| 311280 | AI767957 | Hs.198248 | ESTs; Weakly similar to Y38A8.1 gene pro | 4.5 |
| 311330 | AI679524 | Hs.201629 | ESTs; Moderately similar to !!!! ALU SUB | 4.6 |
| 311515 | AW136713 | Hs.23862 | ESTs | 5.9 |
| 311574 | AI824863 | Hs.211420 | ESTs | 4.8 |
| 311587 | AI828254 | Hs.271019 | ESTs | 5.8 |
| 311596 | AI682088 | Hs.79375 | ESTs | 26.4 |
| 311631 | AI809519 | Hs.27133 | ESTs | 6.4 |
| 311688 | AW025661 | Hs.240090 | ESTs | 7.4 |
| 311783 | AI682478 | Hs.13528 | EST | 4.6 |
| 311826 | AA765470 | Hs.85092 | ESTs | 6.7 |
| 311853 | AW014013 | Hs.107056 | ESTs | 5.3 |
| 311901 | R16890 | Hs.137135 | ESTs | 5.6 |
| 311932 | AW451654 | Hs.257482 | ESTs | 4.3 |
| 312153 | AA759250 | Hs.118625 | cytochrome b-561 | 11 |
| 312182 | AA834800 | Hs.326263 | EST cluster (not in UniGene) | 16.9 |
| 312242 | AI380207 | Hs.125276 | ESTs | 4.7 |
| 312296 | C01367 | Hs.127128 | ESTs | 5.3 |
| 312407 | R46180 | Hs.153485 | ESTs | 6.2 |
| 312424 | AA847398 | Hs.291997 | ESTs | 4.8 |
| 312425 | R49353 | Hs.293892 | ESTs | 5.2 |
| 312480 | R68651 | Hs.144997 | ESTs | 9.5 |
| 312518 | C17785 | Hs.182738 | ESTs | 6.3 |
| 312521 | AA033609 | Hs.239884 | ESTs | 11.2 |
| 312527 | AI695522 | Hs.191271 | ESTs | 4.7 |
| 312539 | AI004377 | Hs.200360 | ESTs | 7 |
| 312546 | AI623511 | Hs.118567 | ESTs | 5.1 |
| 312563 | AA976064 | Hs.180842 | ESTs | 6.5 |
| 312623 | AA694607 | Hs.176956 | EST cluster (not in UniGene) | 10.8 |
| 312857 | AA772279 | Hs.126914 | ESTs | 5 |
| 312890 | AI813654 | Hs.5957 | ESTs | 5.8 |
| 312903 | AA939266 | Hs.276826 | ESTs | 7.7 |
| 312905 | H92571 | Hs.234478 | ESTs | 6.5 |
| 312976 | AA836271 | Hs.125830 | ESTs | 4.6 |
| 312983 | AI079278 | Hs.269899 | ESTs | 5.1 |
| 312996 | AA249018 | Hs.154331 | EST cluster (not in UniGene) | 7 |
| 313035 | N36417 | Hs.144928 | ESTs | 6.3 |
| 313166 | AI801098 | Hs.151500 | ESTs | 4.3 |
| 313188 | AI039702 | Hs.179573 | collagen; type 1; alpha 2 | 4.8 |
| 313218 | AA827805 | Hs.124296 | ESTs | 5 |
| 313226 | AI200281 | Hs.123910 | ESTs | 5.9 |
| 313325 | AI420611 | Hs.127832 | ESTs | 4.6 |
| 313326 | AI088120 | Hs.122329 | ESTs | 7.4 |
| 313425 | AA745689 | Hs.186838 | ESTs; Weakly similar to similar to zinc | 6.3 |
| 313499 | AI261390 | Hs.146085 | ESTs | 5.6 |
| 313540 | AI797301 | Hs.5740 | ESTs | 5.9 |
| 313568 | AW467376 | Hs.129640 | ESTs | 4.3 |
| 313569 | AI273419 | Hs.135146 | ESTs; Weakly similar to ZK1058.5 [C.eleg | 4.6 |
| 313603 | AW468119 | Hs.287631 | EST cluster (not in UniGene) | 6.8 |
| 313615 | AW295194 | Hs.301997 | DKFZP434N126 protein | 5.2 |
| 313625 | AW468402 | Hs.254020 | ESTs | 7.8 |
| 313634 | AA688292 | Hs.337786 | ESTs | 4.4 |
| 313635 | AA507227 | Hs.6390 | ESTs | 8.1 |
| 313638 | AI753075 | Hs.104627 | ESTs | 6.7 |
| 313670 | C16690 | Hs.23767 | EST cluster (not in UniGene) | 4.4 |
| 313671 | W49823 | Hs.104613 | ESTs | 4.4 |
| 313676 | AA861697 | Hs.120591 | EST cluster (not in UniGene) | 13.4 |
| 313703 | AI161293 | Hs.280380 | ESTs; Weakly similar to KIAA0525 protein | 10 |
| 313712 | AA768553 | Hs.74170 | ESTs | 5.2 |
| 313800 | AW296132 | Hs.55098 | ESTs | 5.4 |
| 313979 | AI535895 | Hs.221024 | ESTs | 4.3 |
| 314121 | AI732100 | Hs.187619 | ESTs | 13.6 |
| 314123 | AW245993 | Hs.223394 | ESTs | 6.4 |
| 314171 | AI821895 | Hs.193481 | ESTs | 29.4 |
| 314188 | AL138431 | Hs.164243 | ESTs | 4.6 |
| 314219 | AL036001 | Hs.48376 | ESTs | 5.7 |
| 314236 | AA743396 | Hs.189023 | ESTs | 4.9 |
| 314237 | AA732359 | Hs.96264 | ESTs | 4.4 |
| 314284 | AA731431 | Hs.293464 | EST cluster (not in UniGene) | 6.4 |
| 314305 | AI280112 | Hs.125232 | ESTs | 5.3 |
| 314343 | AI754701 | Hs.328476 | ESTs; Weakly similar to alternatively sp | 6.2 |
| 314530 | AI052358 | Hs.193726 | ESTs | 4.5 |
| 314691 | AW207206 | Hs.136319 | ESTs | 17 |
| 314695 | AW502698 | Hs.118152 | ESTs | 8.9 |
| 314785 | AI538226 | Hs.32976 | ESTs | 9.4 |
| 314801 | AA481027 | Hs.109045 | ESTs; Weakly similar to ORF YGR245c [S.c | 8 |
| 314864 | AA493811 | Hs.294068 | ESTs | 6 |
| 314907 | AI672225 | Hs.222886 | ESTs | 19.3 |
| 314916 | AA548906 | Hs.122244 | ESTs | 4.5 |
| 314954 | AA521381 | Hs.187726 | ESTs | 5.3 |
| 314981 | AA524953 | Hs.293334 | ESTs | 4.6 |
| 315021 | AA533447 | Hs.312989 | EST cluster (not in UniGene) | 5.1 |
| 315051 | AW292425 | Hs.163484 | EST | 15.5 |
| 315052 | AA876910 | Hs.134427 | ESTs | 20 |
| 315073 | AW452948 | Hs.257631 | ESTs | 5.3 |
| 315084 | AI821085 | | ESTs | 8.2 |
| 315214 | AI915927 | Hs.34771 | ESTs | 5.4 |
| 315220 | AI420753 | Hs.66731 | ESTs | 5.1 |
| 315278 | AI985544 | Hs.12450 | ESTs | 5.8 |
| 315282 | AI222165 | Hs.144923 | ESTs | 4.5 |
| 315368 | AW291563 | Hs.104696 | ESTs | 8 |
| 315369 | AA764918 | Hs.256531 | ESTs | 4.8 |
| 315378 | AI263393 | Hs.145008 | ESTs | 6.2 |
| 315379 | AI378329 | Hs.126629 | ESTs | 5.4 |
| 315402 | AW293424 | Hs.75354 | ESTs | 5.1 |
| 315442 | AA977935 | Hs.127274 | ESTs | 6.6 |
| 315443 | AW003416 | Hs.160604 | ESTs | 5.5 |
| 315528 | R37257 | Hs.184780 | ESTs | 8.1 |
| 315593 | AW198103 | Hs.158154 | ESTs | 9.9 |
| 315634 | AA837085 | Hs.220585 | ESTs | 7.8 |
| 315705 | AW449285 | Hs.313636 | ESTs | 8.9 |
| 315707 | AI418055 | Hs.161160 | ESTs | 5.1 |
| 315714 | AA744015 | Hs.298138 | EST cluster (not in UniGene) | 6.1 |
| 315740 | T05558 | Hs.156880 | EST cluster (not in UniGene) | 6.8 |
| 315762 | AI391470 | Hs.158618 | ESTs | 5.3 |
| 315769 | AA744875 | Hs.189413 | ESTs | 5 |
| 315843 | AA679430 | Hs.191897 | ESTs | 5.7 |
| 315990 | AI800041 | Hs.190555 | ESTs | 9.2 |
| 316012 | AA764950 | Hs.119898 | ESTs | 4.3 |
| 316036 | AA708016 | Hs.190389 | ESTs | 5.9 |
| 316055 | AA693880 | Hs.6947 | EST cluster (not in UniGene) | 6.7 |
| 316074 | AW517542 | Hs.293273 | ESTs | 5.5 |
| 316100 | AW203986 | Hs.213003 | ESTs | 5.1 |
| 316169 | AI127483 | Hs.120451 | ESTs | 8.2 |
| 316442 | AA760894 | Hs.153023 | ESTs | 17.1 |
| 316491 | AA766025 | Hs.186854 | EST | 4.6 |
| 316504 | AW135854 | Hs.132458 | ESTs | 4.3 |
| 316667 | AW015940 | Hs.232234 | ESTs | 7.6 |
| 316854 | AA831215 | Hs.159066 | ESTs; Weakly similar to predicted using | 5.1 |
| 316905 | AW138241 | Hs.210846 | ESTs | 6.4 |
| 317008 | AW051597 | Hs.143707 | ESTs | 4.4 |
| 317019 | AA864968 | Hs.127699 | ESTs | 11 |
| 317194 | AW445167 | Hs.126036 | ESTs | 13.5 |
| 317224 | D56760 | Hs.93029 | ESTs | 8.7 |
| 317404 | AI806867 | Hs.126594 | ESTs | 8.7 |
| 317501 | AA931245 | Hs.137097 | ESTs | 11.1 |
| 317548 | AI654187 | Hs.195704 | ESTs | 14.2 |
| 317651 | AW292779 | Hs.169799 | ESTs | 5.8 |
| 317758 | AI733277 | Hs.128321 | ESTs | 5.4 |
| 317850 | N29974 | Hs.152982 | EST cluster (not in UniGene) | 11.4 |
| 317869 | AW295184 | Hs.129142 | ESTs; Weakly similar to DEOXYRIBONUCLEAS | 13.8 |
| 317902 | AI828602 | Hs.211265 | ESTs | 5.3 |
| 317916 | AI565071 | Hs.159983 | ESTs | 7.7 |
| 318239 | AI085198 | Hs.164226 | ESTs | 13.1 |
| 318268 | AI817736 | Hs.182490 | ESTs | 6.2 |
| 318327 | AW294013 | Hs.200942 | ESTs | 4.6 |
| 318363 | R45530 | Hs.1440 | gamma-aminobutyric acid (GABA) A recepto | 6 |
| 318428 | AI949409 | Hs.194591 | ESTs | 12.3 |
| 318464 | AI151010 | Hs.157774 | ESTs | 4.3 |
| 318524 | AW291511 | Hs.159065 | ESTs | 25.9 |
| 318540 | T30280 | Hs.274803 | EST cluster (not in UniGene) | 7 |
| 318591 | AW206806 | Hs.115325 | ESTs | 4.8 |
| 318615 | AI133617 | Hs.10177 | ESTs | 5.5 |
| 318646 | AW175665 | Hs.278695 | ESTs | 5.7 |
| 318667 | AI493742 | Hs.165210 | ESTs | 11 |
| 318668 | W26276 | Hs.136075 | ESTs | 5.9 |
| 318753 | AA578265 | Hs.7130 | copine IV | 5.5 |
| 319080 | Z45131 | Hs.23023 | ESTs | 16.9 |
| 319181 | F06504 | Hs.27384 | EST cluster (not in UniGene) | 4.6 |
| 319191 | AF071538 | Hs.79414 | prostate epithelium-specific Ets transcr | 6.6 |
| 319233 | R21054 | Hs.180532 | ESTs | 4.9 |
| 319586 | D78808 | Hs.283683 | ESTs | 8.2 |
| 319750 | AA621606 | Hs.117956 | ESTs | 9.3 |
| 319763 | AA460775 | Hs.6295 | ESTs | 14.3 |
| 319824 | AA424266 | Hs.123642 | EST cluster (not in UniGene) | 12.8 |
| 319838 | AA337642 | Hs.95262 | nuclear factor related to kappa B bindin | 5.1 |
| 319913 | AA179304 | Hs.271586 | ESTs; Moderately similar to !!!! ALU SUB | 4.3 |
| 319964 | T80579 | Hs.290270 | ESTs | 5.8 |
| 320076 | AI653733 | Hs.271593 | ESTs | 8.5 |
| 320102 | AW296219 | Hs.115325 | RAB7; member RAS oncogene family-like 1 | 9.8 |
| 320187 | T99949 | Hs.303428 | EST cluster (not in UniGene) | 9.8 |
| 320211 | AL039402 | Hs.125783 | DEME-6 protein | 7.9 |
| 320324 | AF071202 | Hs.139336 | ATP-binding cassette; sub-family C(CFTR | 56.2 |
| 320455 | R49889 | Hs.24144 | EST cluster (not in UniGene) | 8.3 |
| 320464 | AI089817 | Hs.237146 | ESTs | 5.4 |
| 320561 | NM_006953 | Hs.159330 | EST cluster (not in UniGene) | 7 |
| 320574 | AL049443 | Hs.161283 | Homo sapiens mRNA; cDNA DKFZp586N2020 (f | 4.4 |
| 320576 | AL049977 | Hs.162209 | Homo sapiens mRNA; cDNA DKFZp564C122 (fr | 6.7 |
| 320654 | AW263086 | Hs.118112 | ESTs | 6 |
| 320796 | AF038966 | Hs.31218 | secretory carrier membrane protein 1 | 13.5 |
| 320800 | AI681006 | Hs.71721 | ESTs | 6.2 |
| 320813 | AW360847 | Hs.16578 | ESTs | 9.3 |
| 320853 | AI473796 | Hs.135904 | ESTs | 8.1 |
| 320856 | D59945 | Hs.65366 | EST cluster (not in UniGene) | 6 |
| 320899 | AA633772 | Hs.116798 | ESTs | 9.2 |
| 320918 | AW195012 | Hs.293970 | ESTs | 5 |
| 320973 | H19732 | Hs.247917 | ESTs | 5.9 |
| 321099 | AA018386 | Hs.64341 | ESTs | 4.6 |
| 321190 | H52462 | Hs.163872 | EST cluster (not in UniGene) | 5.8 |
| 321318 | AB033041 | Hs.137507 | EST cluster (not in UniGene) | 8.4 |
| 321382 | AW372449 | Hs.175982 | EST cluster (not in UniGene) | 7.3 |
| 321441 | AW297633 | Hs.118498 | ESTs | 14.7 |
| 321538 | H80483 | Hs.46903 | EST cluster (not in UniGene) | 9.2 |
| 321609 | H86021 | Hs.182538 | ESTs; Weakly similar to hMmTRA1b [H.sapi | 4.8 |
| 321636 | AI791838 | Hs.193465 | ESTs | 5.5 |
| 321638 | AI356352 | Hs.108932 | ESTs | 4.6 |
| 321644 | AI204177 | Hs.237396 | ESTs | 6.6 |
| 321681 | AA233821 | Hs.190173 | EST cluster (not in UniGene) | 4.6 |
| 321726 | X91221 | Hs.144465 | EST cluster (not in UniGene) | 5 |
| 321758 | U29112 | Hs.196151 | EST cluster (not in UniGene) | 6.2 |
| 321877 | AL109784 | Hs.189222 | EST cluster (not in UniGene) | 4.6 |
| 321899 | N55158 | Hs.29468 | ESTs | 4.6 |
| 321902 | AA746374 | Hs.145010 | ESTs | 8.2 |
| 322007 | AW410646 | Hs.164649 | ESTs | 5.1 |
| 322055 | AL137646 | Hs.146001 | EST cluster (not in UniGene) | 4.3 |
| 322092 | AF085833 | Hs.135624 | EST cluster (not in UniGene) | 4.3 |
| 322221 | AI890619 | Hs.179662 | nucleosome assembly protein 1-like 1 | 4.4 |
| 322278 | AF086283 | | EST cluster (not in UniGene) | 5.8 |
| 322303 | W07459 | Hs.157601 | EST cluster (not in UniGene) | 22 |
| 322437 | AW393804 | Hs.170253 | ESTs; Weakly similar to rabaptin-4 [H.sa | 4.4 |
| 322493 | AF143235 | Hs.279819 | EST cluster (not in UniGene) | 7.2 |
| 322782 | AA056060 | Hs.202577 | EST cluster (not in UniGene) | 18.4 |
| 322811 | AA782292 | Hs.105872 | ESTs | 6.9 |
| 322818 | AW043782 | Hs.293616 | ESTs | 10.7 |
| 322826 | AI807883 | Hs.180059 | ESTs | 5 |
| 322887 | AI986306 | Hs.86149 | ESTs; Weakly similar to KIAA0969 protein | 11.9 |
| 322889 | AA081924 | Hs.124918 | ESTs | 7.1 |
| 322924 | AA669253 | Hs.136075 | ESTs | 4.5 |
| 322982 | AI351191 | Hs.128430 | ESTs | 6.6 |
| 322994 | AA422116 | Hs.191461 | ESTs | 4.7 |
| 323040 | AA336609 | Hs.10862 | ESTs | 6.9 |
| 323041 | AL118747 | Hs.26691 | EST cluster (not in UniGene) | 8.3 |
| 323045 | AA148950 | Hs.188836 | ESTs | 4.6 |
| 323048 | AL118923 | Hs.175110 | EST cluster (not in UniGene) | 7.5 |
| 323070 | AA157726 | Hs.264330 | ESTs | 7.5 |
| 323071 | AA157867 | Hs.5722 | ESTs | 4.7 |
| 323097 | Z44354 | Hs.296261 | guanine nucleotide binding protein (G pr | 4.9 |
| 323131 | AA176982 | Hs.270124 | EST cluster (not in UniGene) | 6.1 |
| 323136 | AL120351 | Hs.30177 | EST cluster (not in UniGene) | 4.3 |
| 323175 | AI827137 | Hs.336454 | ESTs | 6.2 |
| 323218 | AF131846 | Hs.13396 | Homo sapiens clone 25028 mRNA sequence | 6.3 |
| 323226 | AF055019 | Hs.21906 | Homo sapiens clone 24670 mRNA sequence | 12.6 |
| 323236 | AA363148 | Hs.293960 | ESTs | 10.9 |
| 323262 | AI829770 | Hs.190642 | ESTs | 7.6 |
| 323276 | AA836452 | Hs.323822 | ESTs | 7.6 |
| 323287 | AA639902 | Hs.104215 | ESTs | 24.7 |
| 323335 | AI655499 | Hs.161712 | ESTs | 14.1 |
| 323341 | AL134875 | Hs.108646 | ESTs | 5.3 |
| 323362 | AL135067 | Hs.117182 | ESTs | 6.1 |
| 323486 | C05278 | Hs.299221 | ESTs; Moderately similar to [PYRUVATE DE | 8.5 |
| 323496 | AI826801 | Hs.300700 | ESTs | 4.5 |
| 323507 | H71721 | Hs.128387 | ESTs | 4.4 |
| 323545 | AI814405 | Hs.224569 | ESTs | 5.8 |
| 323623 | AA314280 | Hs.146589 | EST cluster (not in UniGene) | 5 |
| 323663 | AW263526 | Hs.243023 | ESTs | 7.7 |
| 323691 | AA317561 | Hs.145599 | EST cluster (not in UniGene) | 5.9 |
| 323810 | AA740405 | Hs.108806 | ESTs | 6.2 |
| 323846 | AA337621 | Hs.137635 | ESTs | 6 |
| 323929 | AA354940 | Hs.145958 | ESTs | 10.7 |
| 323959 | AI636775 | Hs.6831 | ESTs | 5.4 |
| 323996 | AA367032 | Hs.217882 | ESTs | 5.8 |
| 323997 | AA844907 | Hs.274454 | EST cluster (not in UniGene) | 4.4 |
| 324019 | AW177009 | | EST cluster (not in UniGene) | 4.6 |
| 324130 | AL046575 | Hs.130198 | ESTs | 11 |
| 324295 | AI146686 | Hs.143691 | ESTs | 13.7 |
| 324296 | AI524039 | Hs.192524 | ESTs | 6.8 |
| 324307 | AA627642 | Hs.4994 | transducer of ERBB2; 2 (TOB2) | 4.9 |
| 324330 | AA884766 | | EST cluster (not in UniGene) | 4.3 |
| 324385 | F28212 | Hs.284247 | EST cluster (not in UniGene) | 4.7 |
| 324430 | AA464018 | Hs.184598 | EST cluster (not in UniGene) | 13.6 |
| 324452 | AW014022 | Hs.170953 | ESTs | 7.6 |
| 324547 | AW501974 | Hs.74170 | ESTs | 5.6 |
| 324603 | AW016378 | Hs.292934 | ESTs | 24.2 |
| 324617 | AA508552 | Hs.195839 | ESTs | 54 |
| 324618 | AI346282 | Hs.87159 | ESTs | 4.6 |
| 324620 | AA448021 | Hs.94109 | EST cluster (not in UniGene) | 5.7 |
| 324626 | AI685464 | | ESTs | 9 |
| 324658 | AI694767 | Hs.129179 | ESTs | 22 |
| 324676 | AW503943 | Hs.112451 | ESTs | 4.9 |
| 324691 | AI217963 | Hs.293341 | ESTs; Weakly similar to Pro-a2(XI) [H.sa | 10.6 |
| 324696 | AA641092 | Hs.257339 | ESTs | 10.2 |
| 324713 | AW340249 | Hs.163440 | ESTs | 5.5 |
| 324715 | AI739168 | Hs.131798 | EST cluster (not in UniGene) | 7.2 |
| 324718 | AI557019 | Hs.116467 | ESTs | 34.4 |
| 324720 | AA578904 | Hs.292437 | ESTs | 4.8 |
| 324752 | AI279919 | Hs.272072 | ESTs; Moderately similar to !!!! ALU SUB | 7.9 |
| 324753 | AA612626 | Hs.144871 | EST cluster (not in UniGene) | 5.2 |
| 324790 | AI334367 | Hs.159337 | ESTs | 7.6 |
| 324801 | AI819924 | Hs.14553 | ESTs | 12.6 |
| 324804 | AI692552 | | ESTs | 6.5 |
| 324845 | AA361016 | Hs.337533 | ESTs | 4.5 |
| 324888 | AI564134 | Hs.136102 | KIAA0853 protein | 4.4 |
| 324929 | AI741633 | Hs.125350 | ESTs | 6.5 |
| 324961 | AA613792 | | EST cluster (not in UniGene) | 5.1 |
| 325108 | AA401863 | Hs.22380 | ESTs | 7.1 |
| 326816 | | | CH.20_hs gi\|6552458 | 9.6 |
| 326997 | | | CH.21_hs gi\|5867660 | 4.8 |
| 327098 | | | CH.21_hs gi\|6682516 | 4.3 |
| 328492 | | | CH.07_hs gi\|5868455 | 5.8 |
| 329362 | | | CH.X_hs gi\|5868837 | 4.3 |
| 329929 | | | CH.16_p2 gi\|6165201 | 5.5 |
| 329960 | | | CH.16_p2 gi\|5091594 | 7.6 |
| 330020 | | | CH.16_p2 gi\|6671887 | 6 |
| 330211 | | | CH.05_p2 gi\|6013592 | 12.6 |
| 330384 | M23263 | | androgen receptor (dihydrotestosterone r | 9 |
| 330430 | HG2261-HT2352 | Hs.321110 | Antigen, Prostate Specific, Alt Splice | 13.8 |
| 330546 | U31382 | Hs.299867 | guanine nucleotide binding protein 4 | 6 |
| 330551 | U39840 | | hepatocyte nuclear factor 3; alpha | 4.9 |
| 330658 | AA319514 | Hs.30732 | ESTs | 6 |
| 330700 | AA037415 | Hs.20999 | ESTs | 5.5 |
| 330704 | AA056557 | Hs.6759 | ESTs | 5.1 |
| 330705 | AA102571 | Hs.157078 | ESTs | 11.7 |
| 330706 | AA121140 | Hs.177576 | ESTs; Moderately similar to kynurenine a | 14.5 |
| 330712 | AA167269 | Hs.52620 | ESTs | 5 |
| 330725 | AA252033 | Hs.24052 | ESTs; Weakly similar to !!!! ALU SUBFAMI | 7.2 |
| 330732 | AA281092 | Hs.35254 | ESTs | 4.9 |
| 330762 | AA449677 | Hs.15251 | Human DNA sequence from clone 437M21 on | 18.5 |
| 330763 | AA450200 | Hs.143187 | FK506-binding protein 3 (25kD) | 4.3 |
| 330772 | AA479114 | Hs.11356 | ESTs | 5.8 |
| 330786 | D60374 | | EST | 4.6 |
| 330892 | AA149579 | Hs.91202 | ESTs | 15.3 |
| 330949 | H01458 | Hs.142896 | ESTs | 10.3 |
| 330977 | H20826 | Hs.315181 | ESTs | 4.4 |
| 331017 | N24619 | Hs.108920 | ESTs | 11.8 |
| 331099 | R36671 | Hs.14846 | ESTs | 11.6 |
| 331128 | R51361 | Hs.268714 | ESTs | 4.8 |
| 331151 | R82331 | Hs.268838 | ESTs | 13 |
| 331195 | T64447 | Hs.168439 | ESTs | 4.9 |
| 331320 | AA262999 | Hs.300141 | ESTs | 4.8 |
| 331321 | AA278355 | Hs.87929 | ESTs | 6.1 |
| 331337 | AA287662 | Hs.118630 | ESTs | 9.2 |
| 331348 | AA400596 | Hs.88143 | ESTs | 9.9 |
| 331359 | AA416979 | Hs.81897 | ESTs | 4.3 |
| 331383 | AA454543 | Hs.43543 | ESTs | 4.6 |
| 331422 | F10802 | Hs.237339 | ESTs; Moderately similar to !!!! ALU SUB | 4.9 |
| 331442 | H77381 | Hs.41223 | ESTs | 7.5 |
| 331466 | N21680 | Hs.43455 | ESTs | 5.4 |
| 331479 | N27154 | Hs.44076 | ESTs | 6.5 |
| 331490 | N32912 | Hs.291039 | ESTs; Weakly similar to hypothetical 43. | 12.5 |
| 331493 | N34357 | Hs.93817 | ESTs | 4.6 |
| 331561 | N62780 | Hs.48703 | ESTs | 9.2 |
| 331615 | N92352 | Hs.5472 | ESTs | 4.6 |
| 331659 | W48868 | Hs.334305 | ESTs | 8.7 |
| 331696 | Z38907 | Hs.65949 | KIAA0888 protein | 10.3 |
| 331811 | AA404500 | Hs.187958 | ESTs | 4.8 |
| 331848 | AA417039 | Hs.98268 | signal recognition particle 72kD | 7.5 |
| 331873 | AA429445 | Hs.98640 | ESTs | 6.5 |
| 331889 | AA431407 | Hs.98802 | Homo sapiens Chromosome 16 BAC clone CIT | 33.6 |
| 331967 | AA460158 | Hs.99589 | KIAA1028 protein | 6.8 |
| 331974 | AA464518 | Hs.105322 | ESTs | 5.3 |
| 332043 | AA490831 | Hs.201591 | ESTs | 10.8 |
| 332076 | AA599477 | Hs.291156 | ESTs | 4.4 |
| 332173 | F09281 | Hs.100725 | ESTs | 5.5 |
| 332247 | N58172 | | ESTs | 14.2 |
| 332249 | N62096 | Hs.194140 | ESTs | 7.2 |
| 332325 | T79428 | Hs.339667 | ESTs | 5.6 |
| 332396 | AA340504 | | ESTs; Weakly similar to similarto human | 21.2 |
| 332434 | N75542 | Hs.237731 | transcription factor 4 | 15.3 |
| 332493 | N95495 | Hs.56729 | ESTs; Highly similar to GTP-binding prot | 7.1 |
| 332522 | L38503 | Hs.178357 | glutathione S-transferase theta 2 | 6.6 |
| 332526 | AA281753 | Hs.17731 | inositol 1;4;5-triphosphate receptor; ty | 5.8 |
| 332530 | M31682 | Hs.19280 | inhibin; beta B (activin AB beta polypep | 5.5 |
| 332533 | M99487 | Hs.325825 | folate hydrolase (prostate-specific memb | 38.1 |
| 332538 | N48715 | Hs.20991 | ESTs | 6.5 |
| 332546 | D84454 | Hs.22587 | solute carrier family 35 (UDP-galactose | 4.8 |
| 332594 | AA279313 | Hs.32951 | methyl CpG binding protein 2 | 5.6 |
| 332610 | AA412405 | Hs.40513 | ESTs; Weakly similar to BETA GALACTOSIDA | 5.6 |
| 332661 | N95742 | Hs.6390 | ESTs | 6.9 |
| 332697 | T94885 | Hs.75725 | carboxypeptidase E | 24.3 |
| 332712 | D26070 | Hs.79306 | inositol 1;4;5-triphosphate receptor; ty | 9.9 |
| 332716 | L00058 | Hs.79630 | v-myc avian myelocytomatosis viral oncog | 5.6 |
| 332726 | R72029 | Hs.83428 | synaptophysin-like protein | 5 |
| 332781 | AA233258 | | ESTs; Weakly similar to D1007.5 [C.elega | 4.5 |
| 332797 | | | CH22_FGENES.6_2 | 30.8 |
| 332798 | | | CH22_FGENES.6_5 | 66.8 |
| 332799 | | | CH22_FGENES.6_6 | 19.8 |
| 332933 | | | CH22_FGENES.38_7 | 5.6 |
| 332980 | | | CH22_FGENES.54_1 | 5.5 |
| 332984 | | | CH22_FGENES.54_6 | 4.9 |
| 333168 | | | CH22_FGENES.94_1 | 4.7 |
| 333169 | | | CH22_FGENES.94_2 | 4.4 |
| 333452 | | | CH22_FGENES.157_1 | 4.8 |
| 333456 | | | CH22_FGENES.157_5 | 4.3 |
| 333458 | | | CH22_FGENES.157_7 | 4.6 |
| 333611 | | | CH22_FGENES.217_6 | 4.7 |
| 333621 | | | CH22_FGENES.219_5 | 5.5 |
| 333814 | | | CH22_FGENES.282_2 | 7.1 |
| 333849 | | | CH22_FGENES.290_8 | 6.2 |
| 333949 | | | CH22_FGENES.303_5 | 4.3 |
| 333951 | | | CH22_FGENES.303_7 | 4.9 |
| 333955 | | | CH22_FGENES.303_11 | 5.6 |
| 334150 | | | CH22_FGENES.339_1 | 5.1 |
| 334223 | | | CH22_FGENES.360_4 | 20.3 |
| 334297 | | | CH22_FGENES.372_3 | 9.4 |
| 334443 | | | CH22_FGENES.387_2 | 4.6 |
| 334444 | | | CH22_FGENES.387_4 | 5.6 |
| 334447 | | | CH22_FGENES.387_7 | 13.1 |
| 334570 | | | CH22_FGENES.405_11 | 5.4 |
| 334749 | | | CH22_FGENES.427_1 | 5.3 |
| 334777 | | | CH22_FGENES.430_9 | 4.7 |
| 334960 | | | CH22_FGENES.465_29 | 5.2 |
| 335179 | | | CH22_FGENES.504_9 | 8.8 |
| 335293 | | | CH22_FGENES.527_6 | 4.7 |
| 335550 | | | CH22_FGENES.576_11 | 5.1 |
| 335581 | | | CH22_FGENES.581_19 | 5.7 |
| 335586 | | | CH22_FGENES.581_25 | 4.3 |
| 335809 | | | CH22_FGENES.617_6 | 6.2 |
| 335810 | | | CH22_FGENES.617_7 | 5.8 |
| 335822 | | | CH22_FGENES.619_7 | 7.1 |
| 335824 | | | CH22_FGENES.619_11 | 8.5 |
| 335853 | | | CH22_FGENES.626_5 | 4.3 |
| 335886 | | | CH22_FGENES.632_4 | 4.3 |
| 336034 | | | CH22_FGENES.678_5 | 6.8 |
| 336441 | | | CH22_FGENES.827_7 | 7.6 |
| 336624 | | | CH22_FGENES.6-3 | 43.3 |
| 336625 | | | CH22_FGENES.6-4 | 37.9 |
| 336679 | | | CH22_FGENES.43-7 | 5.3 |
| 337577 | | | CH22_C65E1.GENSCAN.8-1 | 4.9 |
| 338255 | | | CH22_EM:AC005500.GENSCAN.276-3 | 13.4 |
| 338260 | | | CH22_EM:AC005500.GENSCAN.279-10 | 4.6 |
| 338561 | | | CH22_EM:AC005500.GENSCAN.421-5 | 4.6 |
| 338562 | | | CH22_EM:AC005500.GENSCAN.421-6 | 4.3 |
| 338759 | | | CH22_EM:AC005500.GENSCAN.517-6 | 5.1 |
| 338763 | | | CH22_EM:AC005500.GENSCAN.517-16 | 5.5 |
| 338764 | | | CH22_EM:AC005500.GENSCAN.517-17 | 7.1 |

**TABLE 3A shows the accession numbers for those primekeys lacking unigeneID's for Table 3. For each probeset we have listed the gene cluster number from which the oligonucleotides were designed. Gene clusters were compiled using sequences derived from Genbank ESTs and mRNAs. These sequences were clustered based on sequence similarity using Clustering and Alignment Tools (DoubleTwist, Oakland California). The Genbank accession numbers for sequences comprising each cluster are listed in the "Accession" column.**

| | | |
|---|---|---|
| Pkey. | Unique Eos probeset identifier number | |
| CAT number: | Gene cluster number | |
| Accession: | Genbank accession numbers | |
| | | |

| **Pkey** | **CAT number** | **Accession** |
|---|---|---|
| | | |
| 123619 | 371681_1 | AA602964 AA609200 |
| 116722 | 143512_1 | Z24878 AA494098 F13654 AA494040 AA143127 |
| 103677 | 41847_1 | Z83806 AJ132091 AJ132090 |
| 125992 | 1589048_1 | H48372 W01626 |
| 109342 | genbank_AA213620 | AA213620 |
| 125154 | genbank_W38419 | W38419 |
| 101447 | entrez_M21305 | M21305 |
| 124357 | genbank_N22401 | N22401 |
| 108910 | genbank_AA136590 | AA136590 |
| 322278 | 47271_1 | W69304 AF086283 W69200 |
| 315084 | 350959_1 | AI821085 AW973464 AA554802 AI821831 AA657438 AA640756 AA650339 |
| 324019 | 262792_1 | AW177009 AI381610 |
| 324330 | 300543_1 | AA884766 AW974271 AA592975 AA447312 |
| 324626 | 336411_1 | AI685464 AW971336 AA513587 AA525142 |
| 303029 | 37699_1 | AF199613 AF108756 |
| 324804 | 398093_1 | AI692552 AI393343 AI800510 AI377711 F24263 AA661876 |
| 324961 | 376239_1 | AA613792 AW182329 T05304 AW858385 |
| 329362 | c_x_hs | |
| 336624 | CH22_4071FG_6_3_ | |
| 336625 | CH22_4072FG_6_4_ | |
| 336679 | CH22_4157FG_43_7_ | |
| 338255 | CH22_6856FG_LINK_EM:AC00 | |
| 338260 | CH22_6863FG_LINK_EM:AC00 | |
| 329929 | c16_p2 | |
| 329960 | c16_p2 | |
| 338561 | CH22_7294FG_LINK_EM:AC00 | |
| 338562 | CH22_7295FG_LINK_EM:AC00 | |
| 338759 | CH22_7581FG_LINK_EM:AC00 | |
| 338763 | CH22_7585FG_LINK_EM:AC00 | |
| 338764 | CH22_7586FG_LINK_EM:AC00 | |
| 333168 | CH22_400FG_94_1_LINK_EM:A | |
| 333169 | CH22_401FG_94_2_LINK_EM:A | |
| 333452 | CH22_702FG_157_1_LINK_EM: | |
| 333456 | CH22_706FG_157_5_LINK_EM: | |
| 333458 | CH22_708FG_157_7_LlNK_EM: | |
| 333611 | CH22_872FG_217_6_LINK_EM: | |
| 333621 | CH22_882FG_219_5_LINK_EM: | |
| 333814 | CH22_1083FG_282_2_LINK_EM | |
| 333849 | CH22_1118FG_290_8_LINK_EM | |
| 335179 | CH22_2515FG_504_9_LINK_EM | |
| 333949 | CH22_1225FG_303_5_LINK_EM | |
| 333951 | CH22_1227FG_303_7_LINK_EM | |
| 333955 | CH22_1231FG_303_11_LINK_E | |
| 335293 | CH22_2635FG_527_6_LINK_EM | |
| 326816 | c20_hs | |
| 326997 | c21_hs | |
| 335550 | CH22_2905FG_576_11_LINK_E | |
| 335581 | CH22_2938FG_581_19_LINK_E | |
| 335586 | CH22_2944FG_581_25_LINK_E | |
| 328492 | c_7_hs | |
| 335809 | CH22_3181FG_617_6_LlNK_EM | |
| 335810 | CH22_3182FG_617_7_LlNK_EM | |
| 335822 | CH22_3195FG_619_7_LINK_EM | |
| 335824 | CH22_3197FG_619_11_LlNK_E | |
| 335853 | CH22_3228FG_626_5_LINK_EM | |
| 335886 | CH22_3261FG_632_4_LINK_EM | |
| 330020 | c16_p2 | |
| 330211 | c_5_p2 | |
| 337577 | CH22_5864FG_LlNK_C65E1.G | |
| 307848 | AI364186 | |
| 332797 | CH22_13FG_6_2_LINK_C4G1.G | |
| 332798 | CH22_14FG_6_5_LINK_C4G1.G | |
| 332799 | CH22_15FG_6_6_LINK_C4G1.G | |
| 334150 | CH22_1429FG_339_1_LINK_EM | |
| 332933 | CH22_154FG_38_7_LINK_C20H | |
| 332980 | CH22_204FG_54_1_LINK_EM:A | |
| 332984 | CH22_208FG_54_6_LINK_EM:A | |
| 334223 | CH22_1507FG_360_4_LINK_EM | |
| 334297 | CH22_1588FG_372_3_LINK_EM | |
| 327098 | c21_hs | |
| 334443 | CH22_1742FG_387_2_LINK_EM | |
| 334444 | CH22_1743FG_387_4_LINK_EM | |
| 334447 | CH22_1746FG_387_7_LINK_EM | |
| 334570 | CH22_1875FG_405_11_LINK_E | |
| 334749 | CH22_2061FG_427_1_LINK_EM | |
| 334777 | CH22_2089FG_430_9_LINK_EM | |
| 336034 | CH22_3419FG_678_5_LINK_DJ | |
| 334960 | CH22_2281FG_465_29_LINK_E | |
| 336441 | CH22_3861FG_827_7_LINK_DJ | |
| 330551 | 9851_2 | U39840 NM_004496 AW135607 BE087458 BE087567 AA177116 AW195705 AW750756 AI811008 AI694151 |
| | | BE348594 AW971075 AI347950 AI201455 AI073898 AA652680 AA613671 AI318364 AA507550 AA693692 |
| | | AI032599 AA991871 AI269801 AW948974 T74639 AA532907 AW949173 |
| 330786 | 53973_3 | BE379594 AI192455 AL039862 AI744012 AI761735 AW243181 AI743687 AI928223 AI423022 AI627855 |
| | | AI636059 AI651571 AW802044 AI826995 AI431733 AI539125 AA863056 AW270910 AI768930 AW008835 |
| | | AW615183 AW591147 AI695294 AI672106 AA506358 AI308060 AA011556 AA962437 AI935488 BE219625 |
| | | AI004356 AW151394 AI218466 N66178 AI419784 AW242519 AW946907 D60374 AA989263 AI698799 |
| | | AA470460 AI824167 |
| 332247 | 372959_1 | AA669097 AA513815 AA026798 AA676526 AA704429 AA704269 AW118292 AA579216 N58172 |
| 332396 | 20265_1 | AW579842 BE156562 BE156690 BE156489 BE081033 AK001559 BE149402 M85387 AW367811 AW367798 |
| | | R17370 AI908947 AA382932 R58449 H18732 AA371231 AW962899 AA713530 AW892946 R53463 H11063 |
| | | AW068542 Z40761 BE176212 BE176155 W23952 W92188 AW374883 AA303497 AW954769 AA036808 |
| | | BE168063 AW382073 AW382085 AL041475 H80748 AI078161 BE463983 AI805213 AI761264 W94885 |
| | | N94502 AI623772 AI419532 AI810302 AI634190 AW002516 AW150777 AI352312 AI367474 AW204807 |
| | | AI675502 AI337026 AW134715 BE328451 AI123157 AI560020 AI300745 AI608631 AI248873 AA742484 |
| | | AW051635 H18646 AI245045 AA507111 AI640510 AI925594 AA115747 AA143035 AA151106 |
| 332781 | 32044_1 | AK001764 BE313896 AA380199 AA380151 AA184996 AW118089 AA495871 AW975219 AW085598 |
| | | AI378909 AW992310 AW992409 AI911857 AA657643 AI804471 AI242589 AI623968 R09556 AI129100 |
| | | AI206500 AA680094 AA677784 AI023178 AI277519 AA424742 AI240654 AA232846 AI804273 AI382376 |
| | | AA001729 W90790 BE090656 AW295015 AI674596 AI431734 AI420517 AW769185 AI128355 AI192474 |
| | | AI820001 AA001929 AA706925 AI076676 AI499119 AI200493 AI695919 AI376217 W69195 W69261 |
| | | AW305099 W90320 BE048357 AI658856 AA838534 AA233258 AI753393 AA709227 AI674387 AI872616 |

**TABLE 3B shows the genomic positioning for those primekeys lacking unigene ID's and accession numbers in Table 3. For each predicted exon, we have listed the genomic sequence source used for prediction. Nucleotide locations of each predicted exon are also listed.**

| | | | |
|---|---|---|---|
| Pkey. | Unique number corresponding to an Eos probeset | | |
| Ref: | Sequence source. The 7 digit numbers in this column are Genbank Identifier (GI) numbers. "Dunham I. et al." refers to the publication entitled "The DNA sequence of human chromosome 22." Dunham I. et al., Nature (1999) 402:489-495. | | |
| Strand: | Indicates DNA strand from which exons were predicted. | | |
| Nt_position: | Indicates nucleotide positions of predicted exons. | | |
| | | | |

| Pkey | Ref | Strand | Nt_position |
|---|---|---|---|
| | | | |
| 333611 | Dunham, I. et.al. | Plus | 6548368-6548507 |
| 333621 | Dunham, I. et.al. | Plus | 8597414-8597560 |
| 333814 | Dunham, I. et.al. | Plus | 7894165-7894252 |
| 333849 | Dunham, I. et.al. | Plus | 8018323-8018472 |
| 333949 | Dunham, I. et.al. | Plus | 8589634-8589791 |
| 333951 | Dunham, I. et.al. | Plus | 8592501-8592637 |
| 333955 | Dunham, I. et.al. | Plus | 8597414-8597560 |
| 334150 | Dunham, I. et.al. | Plus | 10529221-10529854 |
| 334297 | Dunham, I. et.al. | Plus | 13420934-13421058 |
| 334443 | Dunham, I. et.al. | Plus | 14298981-14299056 |
| 334444 | Dunham, I. et.al. | Plus | 14306433-14306492 |
| 334447 | Dunham, I. et.al. | Plus | 14308764-14308824 |
| 334570 | Dunham, I. et.al. | Plus | 14994868-14994943 |
| 334777 | Dunham, I. et.al. | Plus | 16259586-16260166 |
| 335179 | Dunham, I. et.al. | Plus | 21634405-21634526 |
| 335581 | Dunham, I. et.al. | Plus | 24976198-24976334 |
| 335586 | Dunham, I. et.al. | Plus | 24990333-24990497 |
| 335809 | Dunham, I. et.al. | Plus | 26310772-26310909 |
| 335810 | Dunham, I. et.al. | Plus | 26314767-26314849 |
| 335822 | Dunham, I. et.al. | Plus | 26364087-26364196 |
| 335824 | Dunham, I. et.al. | Plus | 26376860-26376942 |
| 335886 | Dunham, I. et.al. | Plus | 26934235-26934364 |
| 336034 | Dunham, I. et.al. | Plus | 29014404-29014590 |
| 336441 | Dunham, I. et.al. | Plus | 34187606-34187663 |
| 337577 | Dunham, I. et.al. | Plus | 595377-595678 |
| 338260 | Dunham, I. et.al. | Plus | 15458919-15459257 |
| 332797 | Dunham, I. et.al. | Minus | 216964-216798 |
| 332798 | Dunham, I. et.al. | Minus | 232147-231974 |
| 332799 | Dunham, I. et.al. | Minus | 232421-232307 |
| 332933 | Dunham, I. et.al. | Minus | 2035790-2035681 |
| 332980 | Dunham, I. et.al. | Minus | 5136165-5136019 |
| 332984 | Dunham, I. et.al. | Minus | 2632606-2632457 |
| 333168 | Dunham, I. et.al. | Minus | 3729896-3729788 |
| 333169 | Dunham, I. et.al. | Minus | 3730864-3730767 |
| 333452 | Dunham, I. et.al. | Minus | 5136165-5136019 |
| 333456 | Dunham, I. et.al. | Minus | 2631933-2631797 |
| 333458 | Dunham, I. et.al. | Minus | 5143942-5143806 |
| 334223 | Dunham, I. et.al. | Minus | 12734365-12734269 |
| 334749 | Dunham, I. et.al. | Minus | 16090686-16090106 |
| 334960 | Dunham, I. et.al. | Minus | 20160968-20160795 |
| 335293 | Dunham, I. et.al. | Minus | 22316408-22316275 |
| 335550 | Dunham, I. et.al. | Minus | 24668714-24668658 |
| 335853 | Dunham, I. et.al. | Minus | 26614629-26614506 |
| 336624 | Dunham, I. et.al. | Minus | 227714-227577 |
| 336625 | Dunham, I. et.al. | Minus | 229124-229024 |
| 336679 | Dunham, I. et.al. | Minus | 2035790-2035681 |
| 338255 | Dunham, I. et.al. | Minus | 15242294-15242231 |
| 338561 | Dunham, I. et.al. | Minus | 22311966-22311856 |
| 338562 | Dunham, I. et.al. | Minus | 22312594-22312465 |
| 338759 | Dunham, I. et.al. | Minus | 26582475-26582199 |
| 338763 | Dunham, I. et.al. | Minus | 26628148-26628009 |
| 338764 | Dunham, I. et.al. | Minus | 26641232-26641101 |
| 329960 | 5091594 | Minus | 1031-1162 |
| 329929 | 6165201 | Minus | 156410-156553 |
| 330020 | 6671887 | Plus | 172397-172491 |
| 326816 | 6552458 | Plus | 198354-198436 |
| 326997 | 5867660 | Minus | 71389-72147 |
| 327098 | 6682516 | Minus | 1061684-1062361 |
| 330211 | 6013592 | Plus | 59158-59215 |
| 328492 | 5868455 | Minus | 46094-46241 |
| 329362 | 5868837 | Minus | 65688-68173 |

**TABLE 4: shows a preferred subset of the Accession numbers for genes found in Table 3 which are differentially expressed in prostate tumor tissue compared to normal prostate tissue.**

| | | | | |
|---|---|---|---|---|
| Pkey: | | Unique Eos probeset identifier number | | |
| ExAccn: | | Exemplar Accession number, Genbank accession number | | |
| UnigeneID: | | Unigene number | | |
| Unigene Title: | | Unigene gene title | | |
| R1: | | Ratio of tumor to normal body tissue | | |
| | | | | |

| **Pkey** | **ExAccn** | **UnigeneID** | **Unigene Title** | **R1** |
|---|---|---|---|---|
| | | | | |
| 100819 | HG4020-HT4290 | Hs.2387 | Transglutaminase | 10.5 |
| 102698 | U75272 | Hs.1867 | progastricsin (pepsinogen C) | 10.6 |
| 102869 | X02544 | Hs.572 | orosomucoid 1 | 22.6 |
| 105370 | AA236476 | Hs.22791 | ESTs; Weakly similar to transmembrane pr | 10.3 |
| 105645 | AA282138 | Hs.11325 | ESTs | 14 |
| 106094 | AA419461 | Hs.23317 | ESTs | 10.9 |
| 109014 | AA156790 | Hs.262036 | ESTs | 15.3 |
| 109562 | F01811 | Hs.187931 | ESTs; Moderately similar to voltage-gate | 10.8 |
| 113021 | T23855 | Hs.129836 | KIAA1028 protein | 10.8 |
| 114124 | Z38595 | Hs.125019 | ESTs; Highly similar to KIAA0886 protein | 21.3 |
| 122791 | AA460158 | Hs.129836 | KIAA1028 protein | 12.4 |
| 124352 | N21626 | Hs.102406 | ESTs | 10.2 |
| 301042 | AI659131 | Hs.197733 | ESTs | 24.9 |
| 302005 | AI869666 | Hs.123119 | ESTs | 36.8 |
| 302410 | NM_004917 | Hs.218366 | EST cluster (not in UniGene) with exon h | 26.8 |
| 302881 | AA508353 | Hs.105314 | relaxin 1 (H1) | 78.8 |
| 303344 | AA255977 | Hs.250646 | ESTs; Highly similar to ubiquitin-conjug | 19.5 |
| 303753 | AW503733 | Hs.9414 | ESTs | 13 |
| 310431 | AI420227 | Hs.149358 | ESTs | 72.9 |
| 311251 | AI655662 | Hs.197698 | ESTs | 41.3 |
| 311596 | AI682088 | Hs.79375 | ESTs | 26.4 |
| 312153 | AA759250 | Hs.118625 | cytochrome b-561 | 11 |
| 312521 | AA033609 | Hs.239884 | ESTs | 11.2 |
| 313676 | AA861697 | Hs.120591 | EST cluster (not in UniGene) | 13.4 |
| 314171 | AI821895 | Hs.193481 | ESTs | 29.4 |
| 314907 | AI672225 | Hs.222886 | ESTs | 19.3 |
| 315051 | AW292425 | Hs.163484 | EST | 15.5 |
| 315052 | AA876910 | Hs.134427 | ESTs | 20 |
| 317548 | AI654187 | Hs.195704 | ESTs | 14.2 |
| 317869 | AW295184 | Hs.129142 | ESTs; Weakly similar to DEOXYRIBONUCLEAS | 13.8 |
| 318428 | AI949409 | Hs.194591 | ESTs | 12.3 |
| 318524 | AW291511 | Hs.159066 | ESTs | 25.9 |
| 319080 | Z45131 | Hs.23023 | ESTs | 16.9 |
| 319763 | AA460775 | Hs.6295 | ESTs | 14.3 |
| 320324 | AF071202 | Hs.139336 | ATP-binding cassette; sub-family C (CFTR | 56.2 |
| 321441 | AW297633 | Hs.118498 | ESTs | 14.7 |
| 322303 | W07459 | Hs.157601 | EST cluster (not in UniGene) | 22 |
| 322782 | AA056060 | Hs.202577 | EST cluster (not in UniGene) | 18.4 |
| 322818 | AW043782 | Hs.293616 | ESTs | 10.7 |
| 323287 | AA639902 | Hs.104215 | ESTs | 24.7 |
| 324603 | AW016378 | Hs.292934 | ESTs | 24.2 |
| 324617 | AA508552 | Hs.195839 | ESTs | 54 |
| 324658 | AI694767 | Hs.129179 | ESTs | 22 |
| 324691 | AI217963 | Hs.293341 | ESTs; Weakly similar to Pro-a2(XI) [H.sa | 10.6 |
| 324696 | AA641092 | Hs.257339 | ESTs | 10.2 |
| 324718 | AI557019 | Hs.116467 | ESTs | 34.4 |
| 330211 | | | CH.05_p2 gi\|6013592 | 12.6 |
| 330430 | HG2261-HT2352 Hs.321110 | | Antigen, Prostate Specific, Alt. Splice | 13.8 |
| 330706 | AA121140 | Hs.177576 | ESTs; Moderately similar to kynurenine a | 14.5 |
| 330762 | AA449677 | Hs.15251 | Human DNA sequence from clone 437M21 on | 18.5 |
| 330892 | AA149579 | Hs.91202 | ESTs | 15.3 |
| 330949 | H01458 | Hs.142896 | ESTs | 10.3 |
| 331099 | R36671 | Hs.14846 | ESTs | 11.6 |
| 331151 | R82331 | Hs.268838 | ESTs | 13 |
| 331889 | AA431407 | Hs.98802 | Homo sapiens Chromosome 16 BAC clone CIT | 33.6 |
| 332247 | N58172 | | ESTs | 14.2 |
| 332396 | AA340504 | | ESTs; Weakly similar to similarto human | 21.2 |
| 332533 | M99487 | Hs.325825 | folate hydrolase (prostate-specific memb | 38.1 |
| 332697 | T94885 | Hs.75725 | carboxypeptidase E | 24.3 |
| 332797 | | | CH22_FGENES.6_2 | 30.8 |
| 332798 | | | CH22_FGENES.6_5 | 66.8 |
| 332799 | | | CH22_FGENES.6_6 | 19.8 |
| 334223 | | | CH22_FGENES.360_4 | 20.3 |
| 336624 | | | CH22_FGENES.6-3 | 43.3 |
| 336625 | | | CH22_FGENES.6-4 | 37.9 |

**TABLE 4A shows the accession numbers for those primekeys lacking unigeneID's for Table 4. For each probeset we have listed the gene cluster number from which the oligonucleotides were designed. Gene clusters were compiled using sequences derived from Genbank ESTs and mRNAs. These sequences were clustered based on sequence similarity using Clustering and Alignment Tools (DoubleTwist, Oakland California). The Genbank accession numbers for sequences comprising each cluster are listed in the "Accession" column.**

| | | |
|---|---|---|
| Pkey: | Unique Eos probeset identifier number | |
| CAT number: | Gene cluster number | |
| Accession: | Genbank accession numbers | |
| | | |

| **Pkey** | **CAT number** | **Accession** |
|---|---|---|
| | | |
| 336624 | CH22_4071FG_6_3_ | |
| 336625 | CH22_4072FG_6_4_ | |
| 330211 | c_5_p2 | |
| 332797 | CH22_13FG_6_2_LINK_C4G1.G | |
| 332798 | CH22_14FG_6_5_LINK_C4G1.G | |
| 332799 | CH22_15FG_6_6_LINK_C4G1.G | |
| 334223 | CH22_1507FG_360_4_LINK_EM | |
| 332247 | 372969_1 | AA669097 AA513815 AA026798 AA676526 AA704429 AA704269 AW118292 AA579216 N58172 |
| 332396 | 20265_1 | AW579842 BE156562 BE156690 BE156489 BE081033 AK001559 BE149402 M85387 AW367811 |
| | | AW367798 R17370 AI908947 AA382932 R58449 H18732 AA371231 AW962899 AA713530 AW892946 |
| | | R53463 H11063 AW068542 Z40761 BE176212 BE176155 W23952 W92188 AW374883 AA303497 |
| | | AW954769 AA036808 BE168063 AW382073 AW382085 AL041475 H80748 AI078161 BE463983 |
| | | AI805213 AI761264 W94885 N94502 AI623772 AI419532 AI810302 AI634190 AW002516 AW150777 |
| | | AI352312 AI367474 AW204807 AI675502 AI337026 AW134715 BE328451 AI123157 AI560020 |
| | | AI300745 AI608631 AI248873 AA742484 AW051635 H18646 AI245045 AA507111 AI640510 AI925594 |
| | | AA115747 AA143035 AA151106 |

**TABLE 4B shows the genomic positioning for those primekeys lacking unigene ID's and accession numbers in Table 4. For each predicted exon, we have listed the genomic sequence source used for prediction. Nucleotide locations of each predicted exon are also listed.**

| | | | |
|---|---|---|---|
| Pkey: | Unique number corresponding to an Eos probeset | | |
| Ref: | Sequence source. The 7 digit numbers in this column are Genbank Identifier (GI) numbers. "Dunham I. et al." refers to the publication entitled "The DNA sequence of human chromosome 22." Dunham I. et al., Nature (1999) 402:489-495. | | |
| Strand: | Indicates DNA strand from which exons were predicted. | | |
| Nt_position: | Indicates nucleotide positions of predicted exons. | | |
| | | | |

| Pkey | Ref | Strand | Nt_position |
|---|---|---|---|
| | | | |
| 332797 | Dunham, I. et.al. | Minus | 216964-216798 |
| 332798 | Dunham, I. et.al. | Minus | 232147-231974 |
| 332799 | Dunham, I. et.al. | Minus | 232421-232307 |
| 334223 | Dunham, I. et.al. | Minus | 12734365-12734269 |
| 336624 | Dunham, I. et.al. | Minus | 227714-227577 |
| 336625 | Dunham, I. et.al. | Minus | 229124-229024 |
| 330211 | 6013592 | Plus | 59158-59215 |

**TABLE 5: 1170 GENES UP-REGULATED IN PROSTATE CANCER COMPARED TO NORMAL ADULT TISSUES**

| Table 5 shows 1170 genes up-regulated in prostate cancer compared to normal adult tissues. These were selected from 59680 probesets on the Affymetrix/Eos Hu03 GeneChip array such that the ratio of "average" prostate cancer to "average" normal adult tissues was greater than or equal to 3.44. The "average" prostate cancer level was set to the 85^{th} percentile amongst 73 prostate cancers. The "average" normal adult tissue level was set to the 85^{th} percentile amongst 162 non-malignant tissues. In order to remove gene-specific background levels of non-specific hybridization, the 7.5^{th} percentile value amongst the 162 non-malignant tissues was subtracted from both the numerator and the denominator before the ratio was evaluated. | | | | |
|---|---|---|---|---|
| Pkey: | | Unique Eos probeset identifier number | | |
| ExAccn: | | Exemplar Accession number, Genbank accession number | | |
| UnigeneID: | | Unigene number | | |
| Unigene Title: | | Unigene gene title | | |
| R1: | | Ratio of tumor to normal tissue | | |
| | | | | |

| **Pkey** | **ExAccn** | **UnigeneID** | **Unigene Title** | **R1** |
|---|---|---|---|---|
| | | | | |
| 446057 | AI420227 | Hs.149358 | ESTs, Weakly similar to A46010 X-linked | 86.42 |
| 400302 | N48056 | Hs.1915 | folate hydrolase (prostate-specific memb | 66.46 |
| 414569 | AF109298 | Hs.118258 | prostate cancer associated protein 1 | 58.36 |
| 417407 | AA923278 | Hs.290905 | ESTs, Weakly similar to protease [H.sapi | 56.16 |
| 431579 | AW971082 | Hs.222886 | ESTs, Weakly similar to TRHY_HUMAN TRICH | 53.38 |
| 409361 | NM_005982 | Hs.54416 | sine oculis homeobox (Drosophila) homolo | 48.28 |
| 409731 | AA125985 | Hs.56145 | thymosin, beta, identified in neuroblast | 45.24 |
| 400298 | AA032279 | Hs.61635 | six transmembrane epithelial antigen of | 43.48 |
| 420154 | AI093155 | Hs.95420 | JM27 protein | 41.12 |
| 433466 | AA508353 | Hs.105314 | relaxin 1 (H1) | 39.88 |
| 400296 | AA305627 | Hs.139336 | ATP-binding cassette, sub-family C (CFTR | 38.42 |
| 400292 | AA250737 | Hs.72472 | ESTs | 38.00 |
| 432887 | AI926047 | Hs.162859 | ESTs | 36.48 |
| 439176 | AI446444 | Hs.190394 | ESTs, Weakly similar to B28096 line-1 pr | 36.45 |
| 430722 | AW968543 | Hs.203270 | ESTs, Weakly similar to ALU1_HUMAN ALU S | 33.20 |
| 437052 | AA861697 | Hs.120591 | ESTs | 33.02 |
| 418396 | AI765805 | Hs.26691 | ESTs | 32.68 |
| 434036 | AI659131 | Hs.197733 | hypothetical protein MGC2849 | 32.44 |
| 407709 | AA456135 | Hs.23023 | ESTs | 32.10 |
| 426747 | AA535210 | Hs.171995 | kallikrein 3, (prostate specific antigen | 31.80 |
| 407168 | R45175 | | ESTs | 31.72 |
| 440260 | AI972867 | Hs.7130 | copine IV | 30.52 |
| 421513 | X00949 | Hs.105314 | relaxin 1 (H1) | 30.10 |
| 416370 | N90470 | Hs.203697 | ESTs, Weakly similar to l38022 hypotheti | 29.68 |
| 407122 | H20276 | Hs.31742 | ESTs | 29.24 |
| 400287 | S39329 | Hs.181350 | kallikrein 2, prostatic | 28.90 |
| 432244 | AI669973 | Hs.200574 | ESTs | 28.74 |
| 451939 | U80456 | Hs.27311 | single-minded (Drosophila) homolog 2 | 28.74 |
| 415989 | AI267700 | Hs.111128 | ESTs | 28.34 |
| 418961 | AW967646 | Hs.23023 | ESTs | 27.34 |
| 425628 | NM_004476 | Hs.1915 | folate hydrolase (prostate-specific memb | 27.32 |
| 458509 | AA654650 | Hs.282906 | ESTs | 27.24 |
| 448290 | AK002107 | Hs.20843 | Homo sapiens cDNA FLJ11245 fis, clone PL | 27.16 |
| 428336 | AA503115 | Hs.183752 | microseminoprotein, beta- | 26.17 |
| 450096 | AI682088 | Hs.223368 | holocarboxylase synthetase (biotin-[prop | 25.60 |
| 400299 | X07730 | Hs.171995 | kallikrein 3, (prostate specific antigen | 24.91 |
| 437571 | AA760894 | Hs.153023 | ESTs | 24.74 |
| 453160 | AI263307 | Hs.146228 | H2B histone family, member L | 24.66 |
| 453096 | AW294631 | Hs.11325 | ESTs | 24.46 |
| 425075 | AA506324 | Hs.1852 | acid phosphatase, prostate | 24.23 |
| 407202 | N58172 | Hs.109370 | ESTs | 24.18 |
| 424846 | AU077324 | Hs.1832 | neuropeptide Y | 23.57 |
| 453370 | AI470523 | Hs.182356 | ATP-binding cassette, sub-family C (CFTR | 23.16 |
| 422805 | AA436989 | Hs.121017 | H2A histone family, member A | 22.52 |
| 444917 | R68651 | Hs.144997 | ESTs | 22.26 |
| 408826 | AF216077 | Hs.48376 | Homo sapiens clone HB-2 mRNA sequence | 22.02 |
| 413597 | AW302885 | Hs.117183 | ESTs | 21.76 |
| 426429 | X73114 | Hs.169849 | myosin-binding protein C, slow-type | 21.32 |
| 435981 | H74319 | Hs.188620 | ESTs | 21.12 |
| 432966 | AA650114 | | ESTs | 21.07 |
| 418848 | AI820961 | Hs.193465 | ESTs | 21.06 |
| 405685 | | | | 20.90 |
| 443271 | BE568568 | Hs.195704 | ESTs | 19.98 |
| 418819 | AA228776 | Hs.191721 | ESTs | 19.94 |
| 420757 | X78592 | Hs.99915 | androgen receptor (dihydrotestosterone r | 19.72 |
| 418994 | AA296520 | Hs.89546 | selectin E (endothelial adhesion molecul | 19.56 |
| 429918 | AW873986 | Hs.119383 | ESTs | 19.04 |
| 415539 | AI733881 | Hs.72472 | ESTs | 18.43 |
| 450382 | AA397658 | Hs.60257 | Homo sapiens cDNA FLJ13598 fis, clone PL | 18.34 |
| 418829 | AA516531 | Hs.55999 | NK homeobox (Drosophila), family 3,A | 18.28 |
| 429984 | AL050102 | Hs.227209 | hypothetical protein FLJ21617 | 17.82 |
| 443822 | AI087412 | Hs.143611 | ESTs, Weakly similar to 2004399A chromos | 17.66 |
| 431676 | AI685464 | Hs.292638 | gb:tt88f04.x1 NCI_CGAP_Pr28 Homo sapiens | 17.64 |
| 410330 | AW023630 | Hs.46786 | ESTs | 17.52 |
| 432441 | AW292425 | Hs.163484 | ESTs | 17.41 |
| 452792 | AB037765 | Hs.30652 | KIAA1344 protein | 17.39 |
| 445472 | AB006631 | Hs.12764 | Homo sapiens mRNA for KIAA0293 gene, par | 17.00 |
| 414565 | AA502972 | Hs.183390 | hypothetical protein FLJ13590 | 16.82 |
| 430487 | D87742 | Hs.241552 | KIAA0268 protein | 16.72 |
| 431716 | D89053 | Hs.268012 | fatty-acid-Coenzyme A ligase, long-chain | 16.60 |
| 419536 | AA603305 | | gb:np 12d11.s1 NCI_CGAP_Pr3 Homo sapiens | 16.50 |
| 439677 | R82331 | Hs.164599 | ESTs | 16.46 |
| 449625 | NM_014253 | Hs.23796 | odz (odd Oz/ten-m, Drosophila) homolog 1 | 16.32 |
| 408430 | S79876 | Hs.44926 | dipeptidylpeptidase IV (CD26, adenosine | 16.28 |
| 447033 | AI357412 | Hs.157601 | ESTs | 16.02 |
| 453006 | AI362575 | Hs.167133 | ESTs | 15.74 |
| 431474 | AL133990 | Hs.190642 | ESTs | 15.70 |
| 420218 | AW958037 | Hs.22437 | ribosomal protein L4 | 15.64 |
| 408000 | L11690 | Hs.620 | bullous pemphigoid antigen 1 (230/240kD) | 15.54 |
| 416208 | AW291168 | Hs.41295 | ESTs, Weakly similar to MUC2_HUMAN MUCIN | 15.48 |
| 430226 | BE245562 | Hs.2551 | adrenergic, beta-2-, receptor, surface | 15.40 |
| 415263 | AA948033 | Hs.130853 | ESTs | 15.38 |
| 432437 | W07088 | Hs.293685 | ESTs | 15.26 |
| 428398 | AI249368 | Hs.98558 | ESTs | 15.21 |
| 429900 | AA460421 | Hs.30875 | ESTs | 14.90 |
| 449156 | AF103907 | Hs.171353 | prostate cancer antigen 3 | 14.89 |
| 411096 | U80034 | Hs.68583 | mitochondrial intermediate peptidase | 14.81 |
| 435974 | U29690 | Hs.37744 | Homo sapiens beta-1 adrenergic receptor | 14.76 |
| 444484 | AK002126 | Hs.11260 | hypothetical protein FLJ11264 | 14.76 |
| 422728 | AW937826 | Hs.103262 | ESTs, Weakly similar to ZN91_HUMAN ZINC | 14.60 |
| 418601 | AA279490 | Hs.86368 | calmegin | 14.56 |
| 448999 | AF179274 | Hs.22791 | transmembrane protein with EGF-like and | 14.55 |
| 445885 | AI734009 | Hs.127699 | KIAA1603 protein | 14.44 |
| 452712 | AW838616 | | gb:RC5-LT0054-140200-013-D01 LT0054 Homo⁻ | 14.22 |
| 432189 | AA527941 | | gb:nh30c04.s1 NCl_CGAP_Pr3 Homo sapiens | 14.12 |
| 424565 | AW102723 | Hs.75295 | guanylate cyclase 1, soluble, alpha 3 | 13.78 |
| 429290 | AF203032 | Hs.198760 | neurofilament, heavy polypeptide (200kD) | 13.57 |
| 419264 | AA877104 | Hs.293672 | ESTs, Weakly similar to ALUB_HUMAN !!!! | 13.40 |
| 416445 | AL043004 | Hs.300678 | KIAA0135 protein | 13.32 |
| 407275 | AI364186 | | gb:qw34h07.x1 NCI_CGAP_Ut4 Homo sapiens | 13.24 |
| 408369 | R38438 | Hs.182575 | solute carrier family 15 (H+/peptide tra | 13.21 |
| 446720 | AI439136 | Hs.140546 | ESTs | 13.06 |
| 434988 | AI418055 | Hs.161160 | ESTs | 13.02 |
| 448172 | N75276 | Hs.135904 | ESTs | 12.98 |
| 416182 | NM_004354 | Hs.79069 | cyclin G2 | 12.94 |
| 420544 | AA677577 | Hs.98732 | Homo sapiens Chromosome 16 BAC clone CIT | 12.79 |
| 445413 | AA151342 | Hs.12677 | CGI-147 protein | 12.64 |
| 452588 | AA889120 | Hs.110637 | homeo box A10 | 12.62 |
| 407819 | R42185 | Hs.274803 | ESTs | 12.60 |
| 433444 | AW975324 | Hs.129816 | ESTs | 12.60 |
| 421059 | AI654133 | Hs.30212 | thyroid receptor interacting protein 15 | 12.30 |
| 420077 | AW512260 | Hs.87767 | ESTs | 12.24 |
| 453930 | AA419466 | Hs.36727 | hypothetical protein FLJ10903 | 12.22 |
| 441610 | AW576148 | Hs.148376 | ESTs | 12.20 |
| 451009 | AA013140 | Hs.115707 | ESTs | 12.18 |
| 433764 | AW753676 | Hs.39982 | ESTs | 12.16 |
| 440286 | U29589 | Hs.7138 | cholinergic receptor, muscarinic 3 | 12.04 |
| 443912 | R37257 | Hs.184780 | ESTs | 11.92 |
| 419526 | AI821895 | Hs.193481 | ESTs | 11.91 |
| 423073 | BE252922 | Hs.123119 | MAD (mothers against decapentaplegic, Dr | 11.87 |
| 452784 | BE463857 | Hs.151258 | hypothetical protein FLJ21062 | 11.86 |
| 414422 | AA147224 | Hs.71814 | ESTs | 11.76 |
| 450203 | AF097994 | Hs.301528 | L-kynurenine/alpha-aminoadipate aminotra | 11.68 |
| 436679 | AI127483 | Hs.120451 | ESTs, Weakly similar to unnamed protein | 11.60 |
| 440901 | AA909358 | Hs.128612 | ESTs | 11.60 |
| 448045 | AJ297436 | Hs.20166 | prostate stem cell antigen | 11.51 |
| 433887 | AW204232 | Hs.279522 | ESTs | 11.50 |
| 434980 | AW770553 | Hs.293640 | sterol O-acyltransferase (acyl-Coenzyme | 11.38 |
| 425905 | AB032959 | Hs.161700 | novel C3HC4 type Zinc finger (ring finge | 11.33 |
| 434680 | T11738 | Hs.127574 | ESTs | 11.32 |
| 449650 | AF055575 | Hs.297647 | calcium channel, voltage-dependent, L ty | 11.18 |
| 431173 | AW971198 | Hs.294068 | ESTs | 11.16 |
| 434539 | AW748078 | Hs.214410 | ESTs, Weakly similar to MUC2_HUMAN MUCIN | 11.16 |
| 410037 | AB020725 | Hs.58009 | KIAA0918 protein | 11.14 |
| 417708 | N74392 | Hs.50495 | ESTs | 11.14 |
| 458332 | AI000341 | Hs.220491 | ESTs | 11.12 |
| 420381 | D50640 | Hs.301782 | phosphodiesterase 3B, cGMP-inhibited | 11.10 |
| 425665 | AK001050 | Hs.159066 | hypothetical protein FLJ10188 | 11.08 |
| 425710 | AF030880 | Hs.159275 | solute carrier family, member 4 | 11.08 |
| 428728 | NM_016625 | Hs.191381 | hypothetical protein | 11.04 |
| 407021 | U52077 | | gb:Human mariner1 transposase gene, comp | 11.02 |
| 410733 | D84284 | Hs.66052 | CD38 antigen (p45) | 11.02 |
| 401714 | | | | 10.90 |
| 434485 | AI623511 | Hs.118567 | ESTs | 10.89 |
| 415786 | AW419196 | Hs.257924 | hypothetical protein FLJ13782 | 10.87 |
| 452340 | NM_002202 | Hs.505 | ISL1 transcription factor, LIM/homeodoma | 10.85 |
| 453628 | AW243307 | Hs.170187 | hypothetical protein | 10.72 |
| 408063 | BE086548 | Hs.42346 | calcineurin-binding protein calsarcin-1 | 10.67 |
| 417687 | AI828596 | Hs.250691 | ESTs | 10.64 |
| 434666 | AF151103 | Hs.112259 | T cell receptor gamma locus | 10.53 |
| 432374 | W68815 | Hs.301885 | Homo sapiens cDNA FLJ11346 fis, clone PL | 10.50 |
| 428819 | AL135623 | Hs.193914 | KIAA0575 gene product | 10.48 |
| 413409 | AI638418 | Hs.21745 | DEAD/H (Asp-Glu-Ala-Asp/His) box polypep | 10.44 |
| 428775 | AA434579 | Hs.143691 | ESTs | 10.21 |
| 436556 | AI364997 | Hs.7572 | ESTs | 10.20 |
| 441690 | R81733 | Hs.33106 | ESTs | 10.14 |
| 419852 | AW503756 | Hs.286184 | hypothetical protein dJ551D2.5 | 10.10 |
| 421991 | NM_014918 | Hs.110488 | KIAA0990 protein | 10.04 |
| 423698 | AA329796 | Hs.1098 | DKFZp434J1813 protein | 10.02 |
| 452039 | AI922988 | Hs.172510 | ESTs | 10.00 |
| 433043 | W57554 | Hs.125019 | ESTs | 9.98 |
| 433927 | AI557019 | Hs.116467 | small nuclear protein PRAC | 9.97 |
| 445424 | AB028945 | Hs.12696 | cortactin SH3 domain-binding protein | 9.96 |
| 432240 | AI694767 | Hs.129179 | Homo sapiens cDNA FLJ13581 fis, clone PL | 9.88 |
| 433104 | AL043002 | Hs.128246 | ESTs, Moderately similar to unnamed prot | 9.84 |
| 452744 | AI267652 | Hs.30504 | Homo sapiens mRNA; cDNA DKFZp434E082 (fr | 9.82 |
| 431217 | NM_013427 | Hs.250830 | Rho GTPase activating protein 6 | 9.75 |
| 427398 | AW390020 | Hs.20415 | chromosome 21 open reading frame 11 | 9.70 |
| 446896 | T15767 | Hs.22452 | Homo sapiens mRNA for KIAA1737 protein, | 9.70 |
| 421470 | R27496 | Hs.1378 | annexin A3 | 9.64 |
| 406554 | | | | 9.60 |
| 401424 | | | | 9.58 |
| 407902 | AL117474 | Hs.41181 | Homo sapiens mRNA; cDNA DKFZp727C191 (fr | 9.56 |
| 423545 | AP000692 | Hs.129781 | chromosome 21 open reading frame 5 | 9.54 |
| 439024 | R96696 | Hs.35598 | ESTs | 9.51 |
| 431548 | AI834273 | Hs.9711 | novel protein | 9.48 |
| 409262 | AK000631 | Hs.52256 | hypothetical protein FLJ20624 | 9.45 |
| 446271 | D82484 | Hs.100469 | ESTs | 9.42 |
| 448692 | AW013907 | Hs.224276 | methylcrotonoyl-Coenzyme A carboxylase 2 | 9.26 |
| 414140 | AA281279 | Hs.23317 | hypothetical protein FLJ14681 | 9.24 |
| 435980 | AF274571 | Hs.129142 | deoxyribonuclease II beta | 9.24 |
| 421246 | AW582962 | Hs.300961 | CGI-47 protein | 9.20 |
| 427304 | AA761526 | Hs.163853 | ESTs | 9.16 |
| 442914 | AW188551 | Hs.99519 | hypothetical protein FLJ14007 | 9.16 |
| 413627 | BE182082 | Hs.246973 | ESTs | 9.14 |
| 439699 | AF086534 | Hs.187561 | ESTs, Moderately similar to ALU1_HUMAN A | 9.10 |
| 437718 | AI927288 | Hs.196779 | ESTs | 9.07 |
| 439820 | AL360204 | Hs.283853 | Homo sapiens mRNA full length insert cDN | 9.06 |
| 447342 | AI199268 | Hs.19322 | Homo sapiens, Similar to RIKEN cDNA 2010 | 9.05 |
| 446223 | BE300091 | Hs.119699 | hypothetical protein FLJ12969 | 9.04 |
| 410001 | AB041036 | Hs.57771 | kallikrein 11 | 9.03 |
| 424012 | AW368377 | Hs.137569 | tumor protein 63 kDa with strong homolog | 9.03 |
| 441791 | AW372449 | Hs.175982 | hypothetical protein FLJ21159 | 9.02 |
| 448206 | BE622585 | Hs.3731 | ESTs, Moderately similar to 138022 hypot | 9.02 |
| 414269 | AA298489 | | olfactory receptor, family 51, subfamily | 8.99 |
| 442081 | AA401863 | Hs.22380 | ESTs | 8.98 |
| 420092 | AA814043 | Hs.88045 | ESTs | 8.85 |
| 411630 | U42349 | Hs.71119 | Putative prostate cancer tumor suppresso | 8.80 |
| 421863 | AI952677 | Hs.108972 | Homo sapiens mRNA; cDNA DKFZp434P228 (fr | 8.80 |
| 454141 | AW138413 | Hs.182356 | ATP-binding cassette, sub-family C (CFTR | 8.80 |
| 418278 | AI088489 | Hs.83937 | hypothetical protein | 8.78 |
| 428330 | L22524 | Hs.2256 | matrix metalloproteinase 7 (matrilysin, | 8.76 |
| 432415 | T16971 | Hs.289014 | ESTs, Weakly similar to A43932 mucin 2 p | 8.75 |
| 424906 | AI566086 | Hs.153716 | Homo sapiens mRNA for Hmob33 protein, 3' | 8.74 |
| 415245 | N59650 | Hs.27252 | ESTs | 8.72 |
| 442409 | BE208843 | Hs.129544 | hypothetical protein MGC15438 | 8.70 |
| 404571 | | | | 8.66 |
| 418033 | W68180 | Hs.259855 | elongation factor-2 kinase | 8.64 |
| 456497 | AW967956 | Hs.123648 | ESTs, Weakly similar to AF108460 1 ubinu | 8.56 |
| 405876 | | | | 8.54 |
| 448807 | AI571940 | Hs.7549 | ESTs | 8.52 |
| 445372 | N36417 | Hs.144928 | ESTs | 8.48 |
| 425171 | AW732240 | Hs.300615 | ESTs | 8.44 |
| 419968 | X04430 | Hs.93913 | interleukin 6 (interferon,beta 2) | 8.36 |
| 407385 | AA610150 | Hs.272072 | ESTs,Weakly similar to l38022 hypotheti | 8.31 |
| 433172 | AB037841 | Hs.102652 | hypothetical protein ASH1 | 8.30 |
| 422631 | BE218919 | Hs.118793 | hypothetical protein FLJ10688 | 8.27 |
| 412719 | AW016610 | Hs.129911 | ESTs | 8.24 |
| 418849 | AW474547 | Hs.53565 | Homo sapiens PIG-M mRNA for mannosyltran | 8.22 |
| 444922 | AI921750 | Hs.144871 | Homo sapiens cDNA FLJ13752 fis, clone PL | 8.22 |
| 427674 | NM_003528 | Hs.2178 | H2B histone family, member Q | 8.20 |
| 432101 | AI918950 | Hs.11092 | EphA3 | 8.17 |
| 416288 | H51299 | | gb:yp07c06.s1 Soares breast 3NbHBst Homo | 8.15 |
| 404915 | | | | 8.08 |
| 440106 | AA864968 | Hs.127699 | KIAA1603 protein | 8.07 |
| 442861 | AA243837 | Hs.57787 | ESTs | 8.06 |
| 452259 | AA317439 | Hs.28707 | signal sequence receptor, gamma (transio | 8.06 |
| 443250 | AI041530 | Hs.132107 | ESTs | 8.06 |
| 437267 | AW511443 | Hs.258110 | ESTs | 8.04 |
| 452891 | N75582 | Hs.212875 | ESTs, Weakly similar to DYH9_HUMAN CIL1 | 8.02 |
| 422219 | AW978073 | | regulator of mitotic spindle assembly 1 | 8.00 |
| 453049 | BE537217 | Hs.30343 | ESTs | 8.00 |
| 439731 | AI953135 | Hs.45140 | hypothetical protein FLJ14084 | 7.98 |
| 408554 | AA836381 | Hs.7323 | nuclear receptor co-repressor/HDAC3 comp | 7.94 |
| 421154 | AA284333 | Hs.287631 | Homo sapiens cDNA FLJ14269 fis, clone PL | 7.94 |
| 430107 | AA465293 | Hs.105069 | ESTs | 7.94 |
| 433404 | T32982 | Hs.102720 | ESTs | 7.93 |
| 450813 | AI739625 | Hs.203376 | ESTs | 7.90 |
| 416239 | AL038450 | Hs.48948 | ESTs | 7.85 |
| 448212 | AI475858 | | gb:tc87d07.x1 NCI_CGAP_CLL1 Homo sapiens | 7.82 |
| 449532 | W74653 | Hs.271593 | ESTs, Moderately similar to A47582 B-cel | 7.82 |
| 413930 | M86153 | Hs.75618 | RAB11A, member RAS oncogene family | 7.80 |
| 458191 | AI420611 | Hs.127832 | ESTs | 7.80 |
| 444858 | AI199738 | Hs.208275 | ESTs, Weakly similar to ALUA_HUMAN !!!! | 7.78 |
| 457498 | AI732230 | Hs.191737 | ESTs | 7.78 |
| 407235 | D20569 | Hs.169407 | SAC2 (suppressor of actin mutations 2, y | 7.76 |
| 433759 | AA680003 | Hs.109363 | Homo sapiens cDNA: FLJ23603 fis, clone L | 7.74 |
| 433805 | AA706910 | Hs.112742 | ESTs | 7.74 |
| 426485 | NM_006207 | Hs.170040 | platelet-derived growth factor receptor. | 7.72 |
| 446028 | R44714 | Hs.106795 | Homo sapiens cDNA FLJ13136 fis, clone NT | 7.72 |
| 418555 | AI417215 | Hs.87159 | hypothetical protein FLJ12577 | 7.70 |
| 447499 | AW262580 | Hs.147674 | protocadherin beta 16 | 7.70 |
| 419839 | U24577 | Hs.93304 | phospholipase A2, group VII (platelet-ac | 7.68 |
| 416857 | AA188775 | Hs.292453 | ESTs | 7.68 |
| 413801 | M62246 | Hs.35406 | ESTs, Highly similar to unnamed protein | 7.66 |
| 425480 | AB023198 | Hs.158135 | KIAA0981 protein | 7.66 |
| 420120 | AL049610 | Hs.95243 | transcription elongation factor A (SII)- | 7.64 |
| 424099 | AF071202 | Hs.139336 | ATP-binding cassette, sub-family C (CFTR | 7.64 |
| 446307 | T50083 | Hs.9094 | ESTs | 7.63 |
| 429220 | AW207206 | Hs.136319 | ESTs | 7.59 |
| 420345 | AW295230 | Hs.25231 | ESTs | 7.54 |
| 429208 | AA447990 | Hs.190478 | ESTs | 7.54 |
| 447247 | AW369351 | Hs.287955 | Homo sapiens cDNA FLJ13090 fis, clone NT | 7.53 |
| 440995 | T57773 | Hs.10263 | ESTs | 7.53 |
| 448706 | AW291095 | Hs.21814 | interleukin 20 receptor, alpha | 7.52 |
| 410227 | AB009284 | Hs.61152 | exostoses (multiple)-like 2 | 7.49 |
| 431616 | AA508552 | Hs.195839 | ESTs, Weakly similar to l38022 hypotheti | 7.46 |
| 434217 | AW014795 | Hs.23349 | ESTs | 7.44 |
| 431467 | N71831 | Hs.256398 | Homo sapiens mRNA; cDNA DKFZp434E0528 (f | 7.42 |
| 448519 | AW175665 | Hs.244334 | Homo sapiens prostein mRNA, complete cds | 7.42 |
| 446791 | AI632278 | Hs.34981 | ESTs | 7.40 |
| 419743 | AW408762 | Hs.127478 | Homo sapiens clone 24416 mRNA sequence | 7.39 |
| 445855 | BE247129 | Hs.145569 | ESTs | 7.36 |
| 425211 | M18667 | Hs.1867 | progastricsin (pepsinogen C) | 7.35 |
| 419131 | AA406293 | Hs.301622 | ESTs | 7.34 |
| 400294 | N95796 | Hs.179809 | Homo sapiens prostein mRNA, complete cds | 7.33 |
| 441736 | AW292779 | Hs.169799 | ESTs | 7.28 |
| 427701 | AA411101 | Hs.221750 | nuclear autoantigenic sperm protein (his | 7.24 |
| 457733 | AW974812 | Hs.291971 | ESTs | 7.24 |
| 418432 | M14156 | Hs.85112 | insulin-like growth factor 1 (somatomedi | 7.22 |
| 441201 | AW118822 | Hs.128757 | ESTs | 7.21 |
| 419953 | BE267154 | Hs.125752 | ESTs | 7.20 |
| 419991 | AJ000098 | Hs.94210 | eyes absent (Drosophila) homolog 1 | 7.20 |
| 425018 | BE245277 | Hs.154196 | E4F transcription factor 1 | 7.20 |
| 424560 | AA158727 | Hs.150555 | protein predicted by clone 23733 | 7.18 |
| 435380 | AA679001 | Hs.192221 | ESTs | 7.14 |
| 420658 | AW965215 | Hs.130707 | ESTs | 7.12 |
| 408291 | AB023191 | Hs.44131 | KIAA0974 protein | 7.10 |
| 409110 | AA191493 | Hs.48778 | niban protein | 7.10 |
| 414485 | W27026 | Hs.182625 | VAMP (vesicle-associated membrane protei | 7.10 |
| 430039 | BE253012 | Hs.153400 | ESTs, Weakly similar to ALU1_HUMAN ALU S | 7.10 |
| 450832 | AW970602 | Hs.105421 | ESTs | 7.10 |
| 417153 | X57010 | Hs.81343 | collagen, type II, alpha 1 (primary oste | 7.08 |
| 412446 | AI768015 | Hs.92127 | ESTs | 7.07 |
| 412953 | Z45794 | Hs.238809 | ESTs | 7.06 |
| 418051 | AW192535 | Hs.19479 | ESTs | 7.06 |
| 421566 | NM_000399 | Hs.1395 | early growth response 2 (Krox-20 (Drosop | 7.04 |
| 446999 | AA151520 | Hs.279525 | hypothetical protein MGC4485 | 7.04 |
| 440529 | AW207640 | Hs.16478 | Homo sapiens cDNA:FLJ21718 fis, clone C | 7.04 |
| 441111 | AI806867 | Hs.126594 | ESTs | 7.01 |
| 451027 | AW519204 | Hs.40808 | ESTs | 7.00 |
| 408432 | AW195262 | | gb:xn67b05.x1 NCI_CGAP_CML1 Homo sapiens | 7.00 |
| 432223 | AA333283 | Hs.285336 | Homo sapiens, clone IMAGE:3460280, mRNA | 7.00 |
| 444805 | AB007899 | Hs.12017 | homolog of yeast ubiquitin-protein ligas | 6.99 |
| 414212 | AA136569 | Hs.295940 | KIAA0187 gene product | 6.98 |
| 431725 | X65724 | Hs.2839 | Norrie disease (pseudoglioma) | 6.98 |
| 449685 | AW296669 | Hs.66095 | ESTs | 6.97 |
| 447313 | U92981 | Hs.18081 | Homo sapiens clone DT1P1B6 mRNA, CAG rep | 6.96 |
| 424590 | AW966399 | Hs.46821 | hypothetical protein FLJ20086 | 6.94 |
| 449655 | AI021987 | Hs.59970 | ESTs | 6.92 |
| 419563 | AA526235 | Hs.193162 | Homo sapiens cDNA FLJ11983 fis, clone HE | 6.90 |
| 434163 | AW974720 | Hs.25206 | group XII secreted phospholipase A2 | 6.89 |
| 415809 | Z32789 | Hs.46601 | ESTs | 6.86 |
| 425782 | U66468 | Hs.159525 | cell growth regulatory with EF-hand doma | 6.85 |
| 417958 | AA767382 | Hs.193417 | ESTs | 6.84 |
| 427408 | AA583206 | Hs.2156 | RAR-related orphan receptor A | 6.79 |
| 445873 | AA250970 | Hs.251946 | poly(A)-binding protein, cytoplasmic 1-l | 6.74 |
| 410718 | AI920783 | Hs.191435 | ESTs | 6.74 |
| 432363 | AA534489 | | gb:nf76g11.s1 NCI_CGAP_Co3 Homo sapiens | 6.74 |
| 436521 | AW203986 | Hs.213003 | ESTs | 6.73 |
| 435604 | AA625279 | Hs.26892 | uncharacterized bone marrow protein BM04 | 6.73 |
| 419083 | AI479560 | Hs.98613 | Homo sapiens cDNA FLJ12292 fis, clone MA | 6.72 |
| 418245 | AA088767 | Hs.83883 | transmembrane, prostate androgen induced | 6.70 |
| 420714 | BE172704 | Hs.222746 | KIAA1610 protein | 6.70 |
| 412707 | AW206373 | Hs.16443 | Homo sapiens cDNA:FLJ21721 fis, clone C | 6.67 |
| 421896 | N62293 | Hs.45107 | ESTs | 6.66 |
| 411078 | AI222020 | Hs.182364 | CocoaCrisp | 6.66 |
| 452465 | AA610211 | Hs.34244 | ESTs | 6.66 |
| 422763 | AA033699 | Hs.83938 | ESTs, Moderately similar to MAS2_HUMAN M | 6.66 |
| 444618 | AV653785 | Hs.300171 | ELL-RELATED RNA POLYMERASE II, ELONGATIO | 6.64 |
| 450164 | AI239923 | Hs.30098 | ESTs | 6.63 |
| 431060 | AF039307 | Hs.249171 | homeo box A11 | 6.62 |
| 408031 | AA081395 | Hs.42173 | Homo sapiens cDNA FLJ10366 fis, clone NT | 6.62 |
| 420285 | AA258124 | Hs.293878 | ESTs, Moderately similar to ZN91_HUMAN Z | 6.62 |
| 444670 | H58373 | Hs.37494 | hypothetical protein MGC5370 | 6.62 |
| 444489 | AI151010 | Hs.157774 | ESTs | 6.60 |
| 445685 | AW779829 | Hs.263436 | gb:hn88a05.x1 NCI_CGAP_Kid11 Homo sapien | 6.60 |
| 435677 | AA694142 | Hs.293726 | ESTs, Weakly similar to TSGA RAT TESTIS | 6.59 |
| 452221 | C21322 | Hs.11577 | hypothetical protein FLJ22242 | 6.59 |
| 431510 | AA580082 | Hs.112264 | ESTs | 6.56 |
| 415874 | AF091622 | Hs.78893 | KIAA0244 protein | 6.54 |
| 418405 | AI868282 | Hs.11898 | ESTs, Highly similar to KIAA1370 protein | 6.54 |
| 452768 | AW069459 | Hs.61539 | ESTs | 6.54 |
| 401451 | | | | 6.52 |
| 416289 | W26333 | | ESTs | 6.52 |
| 431778 | AL080276 | Hs.268562 | regulator of G-protein signalling 17 | 6.51 |
| 409089 | NM_014781 | Hs.50421 | KIAA0203 gene product | 6.50 |
| 442833 | AA328153 | Hs.88201 | ESTs, Weakly similar to A Chain A, Cryst | 6.50 |
| 431992 | NM_002742 | Hs.2891 | protein kinase C, mu | 6.49 |
| 418833 | AW974899 | Hs.292776 | ESTs | 6.48 |
| 429163 | AA884766 | | gb:am20a10.s1 Soares_NFL_T_GBC_S1 Homo s | 6.46 |
| 430403 | AF039390 | Hs.241382 | tumor necrosis factor (ligand) superfami | 6.46 |
| 443058 | AW451642 | Hs.16732 | ESTs | 6.46 |
| 418564 | AA631143 | Hs.179809 | Homo sapiens prostein mRNA, complete cds | 6.44 |
| 432674 | AA641092 | Hs.257339 | ESTs, Weakly similar to I38022 hypotheti | 6.44 |
| 423600 | AI633559 | Hs.29076 | ESTs | 6.44 |
| 404253 | | | | 6.42 |
| 433610 | AA806822 | Hs.112547 | ESTs | 6.42 |
| 421552 | AF026692 | Hs.105700 | secreted frizzled-related protein 4 | 6.41 |
| 407118 | AA156790 | Hs.262036 | ESTs, Weakly similar to Z223_HUMAN ZINC | 6.40 |
| 408608 | N79738 | Hs.136102 | KIAA0853 protein | 6.40 |
| 421452 | AI925946 | Hs.104530 | fetal hypothetical protein | 6.40 |
| 433285 | AW975944 | Hs.237396 | ESTs | 6.40 |
| 434926 | BE543269 | Hs.50252 | mitochondrial ribosomal protein L32 | 6.40 |
| 446189 | H85224 | Hs.214013 | ESTs | 6.40 |
| 416806 | NM_000288 | Hs.79993 | peroxisomal biogenesis factor 7 | 6.38 |
| 416467 | H57585 | Hs.37467 | ESTs | 6.36 |
| 453403 | BE466639 | Hs.61779 | Homo sapiens cDNA FLJ13591 fis, clone PL | 6.34 |
| 429769 | NM_004917 | Hs.218366 | kallikrein 4 (prostase, enamel matrix, p | 6.34 |
| 423642 | AW452650 | Hs.157148 | hypothetical protein MGC13204 | 6.32 |
| 425843 | BE313280 | Hs.159627 | death associated protein 3 | 6.32 |
| 439221 | AA737106 | Hs.32250 | ESTs, Moderately similar to L78885 serin | 6.32 |
| 428194 | AA765603 | Hs.180877 | H3 histone, family 3B (H3.3B) | 6.30 |
| 431958 | X63629 | Hs.2877 | cadherin 3, type 1, P-cadherin (placenta | 6.30 |
| 439366 | AF100143 | Hs.6540 | fibroblast growth factor 13 | 6.30 |
| 452789 | AW081626 | Hs.242561 | ESTs | 6.30 |
| 416836 | D54745 | Hs.80247 | cholecystotokinin | 6.30 |
| 436962 | AW377314 | Hs.5364 | DKFZP564l052 protein | 6.29 |
| 433383 | AF034837 | Hs.192731 | double-stranded RNA specific adenosine d | 6.29 |
| 418636 | AW749855 | | gb:QV4-BT0534-281299-053-c05 BT0534 Homo | 6.26 |
| 450728 | AW162923 | Hs.25363 | presenllin 2 (Alzheimer disease 4) | 6.25 |
| 440293 | AI004193 | Hs.22123 | ESTs | 6.24 |
| 453745 | AA952989 | Hs.63908 | hypothetical protein MGC14726 | 6.24 |
| 426595 | AW971980 | Hs.62402 | p21/Cdc42/Rac1-activated kinase 1 (yeast | 6.24 |
| 444412 | AI147652 | Hs.216381 | Homo sapiens clone HH409 unknown mRNA | 6.24 |
| 413384 | NM_000401 | Hs.75334 | exostoses (multiple) 2 | 6.22 |
| 426320 | W47595 | Hs.169300 | transforming growth factor, beta 2 | 6.22 |
| 423349 | AF010258 | Hs.127428 | homeo box A9 | 6.20 |
| 429165 | AW009886 | Hs.118258 | prostate cancer associated protein 1 | 6.18 |
| 424800 | AL035588 | Hs.153203 | MyoD family inhibitor | 6.18 |
| 409564 | AA045857 | Hs.54943 | fracture callus 1 (rat) homolog | 6.16 |
| 438796 | W67821 | Hs.109590 | genethonin 1 | 6.16 |
| 425451 | AF242769 | Hs.157461 | mesenchymal stem cell protein DSC54 | 6.14 |
| 451663 | AI872360 | Hs.209293 | ESTs | 6.14 |
| 413623 | AA825721 | Hs.246973 | ESTs | 6.12 |
| 452232 | AW020603 | Hs.271698 | radial spoke protein 3 | 6.12 |
| 453390 | AA862496 | Hs.28482 | ESTs | 6.12 |
| 435542 | AA687376 | Hs.269533 | ESTs | 6.12 |
| 420424 | AB033036 | Hs.97594 | KIAA1210 protein | 6.11 |
| 407103 | AA424881 | Hs.256301 | hypothetical protein MGC13170 | 6.10 |
| 409734 | BE161664 | Hs.56155 | hypothetical protein | 6.10 |
| 432686 | BE223007 | Hs.152460 | Homo sapiens cDNA FLJ12909 fis, clone NT | 6.10 |
| 438361 | AA805666 | Hs.146217 | Homo sapiens cDNA:FLJ23077 fis, clone L | 6.10 |
| 411479 | AW848047 | | gb:IL3-CT0214-291299-052-A12 CT0214 Homo | 6.10 |
| 438849 | W28948 | Hs.10762 | ESTs | 6.08 |
| 452726 | AF188527 | Hs.61661 | ESTs, Weakly similar to AF174605 1 F-box | 6.08 |
| 445895 | D29954 | Hs.13421 | KIAA0056 protein | 6.08 |
| 440774 | AI420611 | Hs.127832 | ESTs | 6.07 |
| 422583 | AA410506 | Hs.118578 | KIAA0874 protein | 6.06 |
| 427500 | AW970017 | Hs.293948 | ESTs, Weakly similar to S65657 alpha-1C- | 6.04 |
| 443646 | AI085198 | Hs.298699 | ESTs | 6.04 |
| 410566 | AA373210 | Hs.43047 | Homo sapiens cDNA FLJ13585 fis, clone PL | 6.02 |
| 417845 | AL117461 | Hs.82719 | Homo sapiens mRNA;cDNA DKFZp586F1822 (f | 6.02 |
| 430273 | AI311127 | Hs.125522 | ESTs | 6.02 |
| 434792 | AA649253 | Hs.132458 | ESTs | 6.01 |
| 442490 | AW965078 | Hs.30212 | thyroid receptor interacting protein 15 | 6.01 |
| 420026 | AI831190 | Hs.166676 | ESTs | 6.00 |
| 437782 | AI370876 | Hs.123163 | exportin 1 (CRM1, yeast, homolog) | 6.00 |
| 447359 | NM_012093 | Hs.18268 | adenylate kinase 5 | 6.00 |
| 447713 | AI420733 | Hs.207083 | ESTs | 6.00 |
| 451073 | AI758905 | Hs.206063 | ESTs | 6.00 |
| 451640 | AA195601 | Hs.26771 | Human DNA sequence from clone 747H23 on | 6.00 |
| 410889 | X91662 | Hs.66744 | twist (Drosophila) homolog (acrocephalos | 5.97 |
| 441222 | AI277237 | Hs.44208 | hypothetical protein FLJ23153 | 5.96 |
| 447732 | AI758398 | Hs.161318 | ESTs | 5.96 |
| 437756 | AA767537 | Hs.197096 | ESTs | 5.95 |
| 408829 | NM_006042 | Hs.48384 | heparan sulfate (glucosamine) 3-O-sulfot | 5.94 |
| 453911 | AW503857 | Hs.4007 | Sarcolemmal-associated protein | 5.94 |
| 414085 | AA114016 | Hs.75746 | aldehyde dehydrogenase 1 family, member | 5.93 |
| 408875 | NM_015434 | Hs.48604 | DKFZP434B168 protein | 5.92 |
| 439451 | AF086270 | Hs.278554 | heterochromatin-like protein 1 | 5.92 |
| 423853 | AB011537 | Hs.133466 | slit (Drosophila) homolog 1 | 5.91 |
| 453060 | AW294092 | Hs.21594 | hypothetical protein MGC15754 | 5.91 |
| 420407 | AA814732 | Hs.145010 | lipopolysaccaride-specific response 5-li | 5.91 |
| 450480 | X82125 | Hs.25040 | zinc finger protein 239 | 5.90 |
| 408446 | AW450669 | Hs.45068 | hypothetical protein DKFZp434l143 | 5.88 |
| 421039 | NM_003478 | Hs.101299 | cullin 5 | 5.88 |
| 451684 | AF216751 | Hs.26813 | CDA14 | 5.88 |
| 436063 | AK000028 | Hs.250867 | ribosomal protein S24 | 5.86 |
| 410507 | AA355288 | Hs.271408 | transitional epithelia response protein | 5.86 |
| 420179 | N74530 | Hs.21168 | ESTs | 5.84 |
| 453878 | AW964440 | Hs.19025 | DC32 | 5.84 |
| 452270 | AW975014 | Hs.26 | ferrocholatase (protoporphyria) | 5.83 |
| 435867 | AA954229 | Hs.114052 | ESTs | 5.82 |
| 417683 | AW566008 | Hs.239154 | ankyrin repeat, family A (RFXANK-like), | 5.82 |
| 432005 | AA524190 | Hs.120777 | ESTs, Weakly similar to ELL2_HUMAN RNA P | 5.81 |
| 406815 | AA833930 | Hs.288036 | tRNA isopentenylpyrophosphate transferas | 5.80 |
| 437980 | R50393 | Hs.278436 | KIAA1474 protein | 5.80 |
| 425856 | AA364908 | Hs.98927 | hypothetical protein FLJ13993 | 5.79 |
| 400301 | X03635 | Hs.1657 | estrogen receptor 1 | 5.78 |
| 446261 | AA313893 | Hs.13399 | hypothetical protein FLJ12615 similar to | 5.78 |
| 410141 | R07775 | Hs.287657 | Homo sapiens cDNA:FLJ21291 fis, clone C | 5.77 |
| 427258 | AA400091 | Hs.39421 | ESTs | 5.76 |
| 419108 | AA389724 | Hs.191264 | ESTs, Weakly similar to ALU7_HUMAN ALU S | 5.76 |
| 442029 | AW956698 | Hs.14456 | neural precursor cell expressed, develop | 5.76 |
| 407783 | AW996872 | Hs.172028 | a disintegrin and metalloproteinase doma | 5.75 |
| 434408 | AI031771 | Hs.132586 | ESTs | 5.74 |
| 415077 | L41607 | Hs.934 | glucosaminyl (N-acetyl) transferase 2, l | 5.74 |
| 432435 | BE218886 | Hs.282070 | ESTs | 5.74 |
| 433313 | W20128 | Hs.296039 | ESTs | 5.73 |
| 431740 | N75450 | Hs.183412 | ESTs, Moderately similar to AF116721 67 | 5.73 |
| 412991 | AW949013 | | gb:QV4-FT0005-110500-201-e12 FT0005 Homo | 5.72 |
| 418852 | BE537037 | Hs.273294 | hypothetical protein FLJ20069 | 5.72 |
| 418882 | NM_004996 | Hs.89433 | ATP-binding cassette, sub-family C (CFTR | 5.72 |
| 446867 | AB007891 | Hs.16349 | KIAA0431 protein | 5.72 |
| 437866 | AA156781 | Hs.83992 | metallothionein 1E (functional) | 5.72 |
| 410232 | AW372451 | Hs.61184 | CGI-79 protein | 5.70 |
| 414452 | AA454038 | Hs.29032 | ESTs | 5.70 |
| 422762 | AL031320 | Hs.119976 | Human DNA sequence from clone RP1-20N2 o | 5.70 |
| 428730 | AA625947 | Hs.25750 | ESTs | 5.70 |
| 431571 | AW500486 | Hs.180610 | splicing factor proline/glutamine rich ( | 5.70 |
| 433393 | AF038564 | Hs.98074 | itchy (mouse homolog) E3 ubiquitin prote | 5.70 |
| 450616 | AL133067 | Hs.25214 | hypothetical protein | 5.70 |
| 443774 | AL117428 | Hs.9740 | DKFZP434A236 protein | 5.69 |
| 446100 | AW967109 | Hs.13804 | hypothetical protein dJ462O23.2 | 5.69 |
| 419168 | AI336132 | Hs.33718 | Homo sapiens cDNA FLJ12641 fis, clone NT | 5.68 |
| 416653 | AA768553 | Hs.77496 | metallothionein 1E (functional) | 5.67 |
| 452679 | Z42387 | Hs.4299 | transmembrane, prostate androgen induced | 5.66 |
| 450244 | AA007534 | Hs.125062 | ESTs | 5.66 |
| 408621 | AI970672 | Hs.46638 | chromosome 11 open reading frame 8 | 5.65 |
| 450325 | AI935962 | Hs.26289 | ESTs | 5.65 |
| 439671 | AW162840 | Hs.6641 | kinesin family member 5C | 5.64 |
| 452387 | AI680772 | Hs.4316 | trinucleotide repeat containing 12 | 5.64 |
| 413992 | W26276 | Hs.136075 | RNA, U2 small nuclear | 5.63 |
| 444151 | AW972917 | Hs.128749 | alpha-methylacyl-CoA racemase | 5.63 |
| 417791 | AW965339 | Hs.111471 | ESTs | 5.62 |
| 410196 | AI936442 | Hs.59838 | hypothetical protein FLJ10808 | 5.60 |
| 415123 | D60925 | | ESTs | 5.60 |
| 429170 | NM_001394 | Hs.2359 | dual specificity phosphatase 4 | 5.60 |
| 434415 | BE177494 | | gb:RC6-HT0596-270300-011-C05 HT0596 Homo | 5.60 |
| 440738 | AI004650 | Hs.225674 | WD repeat domain 9 | 5.60 |
| 443830 | AI142095 | Hs.143273 | ESTs | 5.60 |
| 449603 | AI655662 | Hs.197698 | ESTs | 5.60 |
| 414342 | AA742181 | Hs.75912 | KIAA0257 protein | 5.59 |
| 422634 | NM_016010 | Hs.118821 | CGI-62 protein | 5.56 |
| 435047 | AA454985 | Hs.54973 | cadherin-like protein VR20 | 5.55 |
| 400268 | | | | 5.55 |
| 452055 | AI377431 | Hs.293772 | hypothetical protein MGC10858 | 5.54 |
| 437073 | AI885608 | Hs.94122 | ESTs | 5.54 |
| 434072 | H70854 | Hs.283059 | Homo sapiens PRO1082 mRNA, complete cds | 5.53 |
| 418339 | AA639902 | Hs.104215 | ESTs, Moderately similar to SPCN_HUMAN S | 5.52 |
| 434551 | BE387162 | Hs.280858 | ESTs, Highly similar to A35661 DNA excis | 5.52 |
| 439569 | AW602166 | Hs.222399 | CEGP1 protein | 5.51 |
| 441102 | AA973905 | Hs.16003 | intermediate filament protein syncoilin | 5.50 |
| 448310 | AI480316 | | gb:tm26h09.x1 Soares_NFL_T_GBC_S1 Homo s | 5.50 |
| 413173 | BE076928 | Hs.70980 | ESTs | 5.48 |
| 436246 | AW450963 | Hs.119991 | ESTs | 5.48 |
| 449300 | AI656959 | Hs.222165 | ESTs | 5.48 |
| 452823 | AB012124 | Hs.30696 | transcription factor-like 5 (basic helix | 5.48 |
| 451403 | AA885569 | Hs.15727 | Homo sapiens cDNA FLJ14511 fis, clone NT | 5.46 |
| 417061 | AI675944 | Hs.188691 | Homo sapiens cDNA FLJ12033 fis, clone HE | 5.44 |
| 429126 | AW172356 | Hs.99083 | ESTs | 5.44 |
| 431316 | AA502663 | Hs.145037 | ESTs | 5.44 |
| 439192 | AW970536 | Hs.105413 | ESTs | 5.44 |
| 431938 | AA938471 | Hs.115242 | specific granule protein (28 kDa); cyste | 5.44 |
| 451552 | AA047233 | Hs.33810 | ESTs | 5.43 |
| 416991 | N36389 | Hs.295091 | KIAA0226 gene product | 5.42 |
| 427638 | AA406411 | Hs.208341 | ESTs, Weakly similar to KIAA0989 protein | 5.42 |
| 427718 | AI798680 | Hs.25933 | ESTs | 5.42 |
| 438710 | AA833907 | Hs.178724 | ESTs, Weakly similar to ALU1_HUMAN ALU S | 5.42 |
| 406076 | AL390179 | Hs.137011 | Homo sapiens mRNA; cDNA DKFZp547P134 (fr | 5.40 |
| 431263 | AW129203 | Hs.13743 | ESTs | 5.40 |
| 421264 | AL039123 | Hs.103042 | microtubule-associated protein 1B | 5.38 |
| 421685 | AF189723 | Hs.106778 | ATPase, Ca++ transporting, type 2C, memb | 5.37 |
| 408460 | AA054726 | Hs.285574 | ESTs | 5.36 |
| 409091 | AW970386 | Hs.269423 | ESTs | 5.36 |
| 421987 | AI133161 | Hs.286131 | CGI-101 protein | 5.36 |
| 428002 | AA418703 | | gb:zv98c03.s1 Soares_NhHMPu_S1 Homo sapi | 5.36 |
| 441217 | AI922183 | Hs.213246 | ESTs | 5.36 |
| 426006 | R49031 | Hs.22627 | ESTs | 5.35 |
| 422806 | BE314767 | Hs.1581 | glutathione S-transferase theta 2 | 5.34 |
| 432281 | AK001239 | Hs.274263 | hypothetical protein FLJ10377 | 5.32 |
| 451982 | F13036 | Hs.27373 | Homo sapiens mRNA; cDNA DKFZp564O1763 (f | 5.32 |
| 421129 | BE439899 | Hs.89271 | ESTs | 5.31 |
| 444042 | NM_004915 | Hs.10237 | ATP-binding cassette, sub-family G (WHIT | 5.31 |
| 410150 | AW382942 | Hs.6774 | ESTs | 5.30 |
| 423952 | AW877787 | Hs.136102 | KIAA0853 protein | 5.30 |
| 452822 | X85689 | Hs.288617 | hypothetical protein FLJ22621 | 5.30 |
| 447752 | M73700 | Hs.347 | lactotransferrin | 5.29 |
| 441766 | R53790 | Hs.23294 | hypothetical protein FLJ14393 | 5.29 |
| 431359 | AW993522 | Hs.292934 | ESTs | 5.27 |
| 427212 | AW293849 | Hs.58279 | ESTs, Weakly similar to ALU7_HUMAN ALU S | 5.27 |
| 449916 | T60525 | Hs.299221 | pyruvate dehydrogenase kinase, isoenzyme | 5.27 |
| 454014 | AW016670 | Hs.233275 | ESTs | 5.27 |
| 419714 | AA758751 | Hs.98216 | ESTs | 5.26 |
| 428845 | AL157579 | Hs.153610 | KIAA0751 gene product | 5.26 |
| 417333 | AL157545 | Hs.42179 | bromodomain and PHD finger containing, 3 | 5.24 |
| 419986 | AI345455 | Hs.78915 | GA-binding protein transcription factor, | 5.24 |
| 407182 | AA312551 | Hs.230157 | ESTs | 5.22 |
| 420111 | AA255652 | | gb:zs21h11.r1 NCI_CGAP_GCB1 Homo sapiens | 5.22 |
| 428058 | AI821625 | Hs.191602 | ESTs | 5.22 |
| 459551 | AI472808 | | gb:tj70e07.x1 Soares_NSF_F8_9W_OT_PA_P_S | 5.22 |
| 432524 | AI458020 | Hs.293287 | ESTs | 5.22 |
| 436207 | AA334774 | Hs.12845 | hypothetical protein MGC13159 | 5.22 |
| 410870 | U81599 | Hs.66731 | homeo box B13 | 5.22 |
| 451418 | BE387790 | Hs.26369 | hypothetical protein FLJ20287 | 5.22 |
| 409757 | NM_001898 | Hs.123114 | cystatin SN | 5.21 |
| 441124 | T97717 | Hs.119563 | ESTs | 5.21 |
| 428593 | AW207440 | Hs.185973 | degenerative spermatocyte (homolog Droso | 5.21 |
| 436401 | AI087958 | Hs.29088 | ESTs | 5.20 |
| 437113 | AA744693 | | gb:ny26c10.s1 NCI_CGAP_GCB1 Homo sapiens | 5.20 |
| 450947 | AI745400 | Hs.204662 | ESTs | 5.20 |
| 453279 | AW893940 | Hs.59698 | ESTs | 5.20 |
| 445467 | AI239832 | Hs.15617 | ESTs, Weakly similar to ALU4_HUMAN ALU S | 5.19 |
| 448944 | AB014605 | Hs.22599 | atrophin-1 interacting protein 1;activi | 5.19 |
| 412198 | AA937111 | Hs.69165 | ESTs | 5.18 |
| 422646 | H87863 | Hs.151380 | ESTs, Weakly similar to T16584 hypotheti | 5.18 |
| 438986 | AF085888 | Hs.269307 | ESTs | 5.18 |
| 453954 | AW118336 | Hs.75251 | DEAD/H (Asp-Glu-Ala-Asp/His) box binding | 5.18 |
| 447541 | AK000288 | Hs.18800 | hypothetical protein FLJ20281 | 5.18 |
| 434029 | AA621763 | Hs.170434 | Homo sapiens cDNA FLJ14242 fis, clone OV | 5.16 |
| 459294 | AW977286 | Hs.169531 | RBP1-like protein | 5.16 |
| 429441 | AJ224172 | Hs.204096 | lipophilin B (uteroglobin family member) | 5.16 |
| 424692 | AA429834 | Hs.151791 | KIAA0092 gene product | 5.15 |
| 427359 | AW020782 | Hs.79881 | Homo sapiens cDNA:FLJ23006 fis, clone L | 5.15 |
| 419872 | AI422951 | Hs.146162 | ESTs | 5.15 |
| 429422 | AK001494 | Hs.202596 | Homo sapiens cDNA FLJ10632 fis, clone NT | 5.14 |
| 448902 | Z45998 | Hs.22543 | Homo sapiens mRNA; cDNA DKFZp761l1912 (f | 5.14 |
| 459055 | N23235 | Hs.30567 | ESTs, Weakly similar to B34087 hypotheti | 5.14 |
| 431318 | AA502700 | Hs.293147 | ESTs, Moderately similar to A46010 X-lin | 5.14 |
| 452953 | AI932884 | Hs.271741 | ESTs, Weakly similar to A46010 X-linked | 5.13 |
| 428372 | AK000684 | Hs.183887 | hypothetical protein FLJ22104 | 5.12 |
| 434401 | AI864131 | Hs.71119 | Putative prostate cancer tumor suppresso | 5.12 |
| 416434 | AW163045 | Hs.79334 | nuclear factor, interleukin 3 regulated | 5.11 |
| 410268 | AA316181 | Hs.61635 | six transmembrane epithelial antigen of | 5.10 |
| 417517 | AF001176 | Hs.82238 | POP4 (processing of precursor, S. cerev | 5.10 |
| 453616 | NM_003462 | Hs.33846 | dynein, axonemal, light intermediate pol | 5.10 |
| 427958 | AA418000 | Hs.98280 | potassium intermediate/small conductance | 5.09 |
| 407945 | X69208 | Hs.606 | ATPase.Cu++ transporting, alpha polypep | 5.08 |
| 425154 | NM_001851 | Hs.154850 | collagen, type IX, alpha 1 | 5.08 |
| 412863 | AA121673 | Hs.59757 | zinc finger protein 281 | 5.06 |
| 420807 | AA280627 | Hs.57846 | ESTs | 5.06 |
| 430568 | AA769221 | Hs.270847 | delta-tubulin | 5.06 |
| 433687 | AA743991 | | gb:ny57g01.s1 NCI_CGAP_Pr18 Homo sapiens | 5.06 |
| 438375 | AW015940 | Hs.232234 | ESTs | 5.06 |
| 418092 | R45154 | Hs.106604 | ESTs | 5.06 |
| 418576 | AW968159 | Hs.289104 | Alu-binding protein with zinc finger dom | 5.05 |
| 413328 | Y15723 | Hs.75295 | guanylate cyclase 1, soluble, alpha 3 | 5.04 |
| 414271 | AK000275 | Hs.75871 | protein kinase C binding protein 1 | 5.04 |
| 432729 | AK000292 | Hs.278732 | hypothetical protein FLJ20285 | 5.04 |
| 433433 | AI692623 | Hs.121513 | Homo sapiens clone Z'3-1 placenta expres | 5.04 |
| 439662 | H97552 | Hs.269060 | ESTs | 5.04 |
| 439743 | AL389956 | Hs.283858 | Homo sapiens mRNA full length insert cDN | 5.04 |
| 417511 | AL049176 | Hs.82223 | chordin-like | 5.02 |
| 437814 | AI088192 | Hs.135474 | ESTs, Weakly similar to DDX9_HUMAN ATP-D | 5.02 |
| 426342 | AF093419 | Hs.169378 | multiple PDZ domain protein | 5.02 |
| 429782 | NM_005754 | Hs.220689 | Ras-GTPase-activating protein SH3-domain | 5.02 |
| 429975 | AI167145 | Hs.165538 | ESTs | 5.02 |
| 436209 | AW850417 | Hs.254020 | ESTs, Moderately similar to unnamed prot | 5.02 |
| 438571 | AW020775 | Hs.56022 | ESTs | 5.02 |
| 450223 | AA418204 | Hs.241493 | natural killer-tumor recognition sequenc | 5.02 |
| 408267 | AW380525 | Hs.267705 | tubulin-specific chaperone e | 5.01 |
| 417730 | Z44761 | | gb:HSC28F061 normalized infant brain cDN | 5.00 |
| 425465 | L18964 | Hs.1904 | protein kinase C, iota | 5.00 |
| 430599 | NM_004855 | Hs.247118 | phosphatidylinositol glycan, class B | 5.00 |
| 450961 | AW978813 | Hs.250867 | metallothionein 1E (functional) | 5.00 |
| 451386 | AB029006 | Hs.26334 | spastic paraplegia 4 (autosomal dominant | 5.00 |
| 420380 | AA640891 | Hs.102406 | ESTs | 4.99 |
| 424947 | R77952 | Hs.239625 | ESTs, Weakly similar to alternatively sp | 4.99 |
| 442653 | BE269247 | Hs.170226 | gb:601185486F1 NIH_MGC_8 Homo sapiens cD | 4.98 |
| 457211 | AW972565 | Hs.32399 | ESTs, Weakly similar to S51797 vasodilat | 4.97 |
| 425851 | NM_001490 | Hs.159642 | glucosaminyl (N-acetyl) transferase 1, c | 4.97 |
| 446279 | AA490770 | Hs.182382 | ESTs | 4.96 |
| 433377 | AI752713 | Hs.43845 | ESTs | 4.96 |
| 450218 | R02018 | Hs.168640 | ankylosis, progressive (mouse) homolog | 4.96 |
| 412715 | NM_000947 | Hs.74519 | primase, polypeptide 2A (58kD) | 4.94 |
| 448164 | R61680 | Hs.26904 | ESTs, Moderately similar to Z195_HUMANZ Z | 4.94 |
| 420121 | AW968271 | Hs.191534 | ESTs, Weakly similar to ALU1_HUMAN ALU S | 4.94 |
| 421689 | N87820 | Hs.106826 | KIAA1696 protein | 4.93 |
| 445808 | AV655234 | Hs.298083 | ESTs, Moderately similar to PC4259 ferri | 4.92 |
| 416533 | BE244053 | Hs.79362 | retinoblastoma-like 2 (p130) | 4.92 |
| 418049 | AA211467 | Hs.190488 | Homo sapiens, Similar to nuclear localiz | 4.92 |
| 436039 | AW023323 | Hs.121070 | ESTs | 4.92 |
| 432653 | N62096 | Hs.293185 | ESTs, Weakly similar to JC7328 amino aci | 4.91 |
| 420324 | AF163474 | Hs.96744 | prostate androgen-regulated transcript 1 | 4.91 |
| 403047 | | | | 4.91 |
| 436899 | AA764852 | Hs.291567 | ESTs | 4.90 |
| 431117 | AF003522 | Hs.250500 | delta (Drosophila)-like 1 | 4.90 |
| 427617 | D42063 | Hs.179825 | RAN binding protein 2 | 4.88 |
| 428804 | AK000713 | Hs.193736 | hypothetical protein FLJ20706 | 4.88 |
| 433050 | AI093930 | Hs.163440 | Homo sapiens cDNA: FLJ21000 fis, clone C | 4.88 |
| 418575 | AA225313 | Hs.222886 | ESTs, Weakly similar to TRHY_HUMAN TRICH | 4.86 |
| 432615 | AA557191 | Hs.55028 | ESTs, Weakly similar to l54374 gene NF2 | 4.86 |
| 412652 | AI801777 | Hs.6774 | ESTs | 4.86 |
| 432473 | AI202703 | Hs.152414 | ESTs | 4.86 |
| 449071 | NM_005872 | Hs.22960 | breast carcinoma amplified sequence 2 | 4.86 |
| 450654 | AJ245587 | Hs.25275 | Kruppel-type zinc finger protein | 4.85 |
| 418866 | T65754 | Hs.100489 | gb:yc11c07.s1 Stratagene lung (937210) H | 4.85 |
| 407596 | R86913 | | gb:yq30f05.r1 Soares fetal liver spleen | 4.84 |
| 456516 | BE172704 | Hs.222746 | KIAA1610 protein | 4.84 |
| 426501 | AW043782 | Hs.293616 | ESTs | 4.84 |
| 448730 | AB032983 | Hs.21894 | KIAA1157 protein | 4.84 |
| 458339 | AW976853 | Hs.172843 | ESTs | 4.83 |
| 422083 | NM_001141 | Hs.111256 | arachidonate 15-lipoxygenase, second typ | 4.82 |
| 420159 | AI572490 | Hs.99785 | Homo sapiens cDNA: FLJ21245 fis, clone C | 4.82 |
| 424103 | NM_001918 | Hs.139410 | dihydrolipoamide branched chain transacy | 4.82 |
| 449535 | W15267 | Hs.23672 | low density lipoprotein receptor-related | 4.82 |
| 422048 | NM_012445 | Hs.288126 | spondin 2, extracellular matrix protein | 4.82 |
| 416737 | AF154335 | Hs.79691 | LIM domain protein | 4.82 |
| 419972 | AL041465 | Hs.294038 | golgin-67 | 4.81 |
| 420235 | AA256756 | Hs.31178 | ESTs | 4.81 |
| 423412 | AF109300 | Hs.147924 | prostate cancer associated protein 5 | 4.80 |
| 429598 | AA811257 | Hs.269710 | ESTs | 4.80 |
| 457114 | AI821625 | Hs.191602 | ESTs | 4.80 |
| 421828 | AW891965 | Hs.289109 | histone deacetylase 3 | 4.79 |
| 424602 | AK002055 | Hs.301129 | hypothetical protein FLJ11193 | 4.78 |
| 428364 | AA426565 | Hs.160541 | ESTs, Moderately similar to ALU1_HUMAN A | 4.78 |
| 452335 | AW188944 | Hs.61272 | ESTs | 4.78 |
| 410765 | AI694972 | Hs.66180 | nucleosome assembly protein 1-like 2 | 4.77 |
| 421040 | AA715026 | Hs.135280 | ESTs | 4.76 |
| 421518 | AI056392 | Hs.208819 | ESTs | 4.76 |
| 452560 | BE077084 | | ESTs | 4.76 |
| 409752 | AW963990 | | gb:EST376063 MAGE resequences, MAGH Homo | 4.75 |
| 439703 | AF086538 | Hs.196245 | ESTs | 4.75 |
| 418836 | AI655499 | Hs.161712 | ESTs | 4.74 |
| 450642 | R39773 | Hs.7130 | copine IV | 4.74 |
| 419879 | Z17805 | Hs.93564 | Homer, neuronal immediate early gene, 2 | 4.74 |
| 411440 | AW749402 | | gb:QV4-BT0383-28129-061-c06 BT0383 Homo | 4.74 |
| 450649 | NM_001429 | Hs.297722 | E1A binding protein p300 | 4.74 |
| 408738 | NM_014785 | Hs.47313 | KIAA0258 gene product | 4.73 |
| 435020 | AW505076 | Hs.301855 | DiGeorge syndrome critical region gene 8 | 4.72 |
| 411624 | BE145964 | | KIAA0594 protein | 4.72 |
| 439360 | AA448488 | Hs.55346 | ribosomal protein L44 | 4.72 |
| 440491 | R35252 | Hs.24944 | ESTs, Weakly similar to 2109260A B cell | 4.72 |
| 442611 | BE077155 | Hs.177537 | hypothetical protein DKFZp761B1514 | 4.72 |
| 443555 | N71710 | Hs.21398 | ESTs, Moderately similar to A Chain A, H | 4.72 |
| 453800 | BE300741 | Hs.288416 | hypothetical protein FLJ13340 | 4.72 |
| 457528 | AW973791 | Hs.292784 | ESTs | 4.72 |
| 416795 | AI497778 | Hs.168053 | HBV pX associated protein-8 | 4.71 |
| 407302 | R74206 | Hs.268755 | ESTs, Weakly similar to l78885 serine/th | 4.71 |
| 404721 | | | | 4.70 |
| 426261 | AW242243 | Hs.168670 | peroxisomal famesylated protein | 4.70 |
| 431924 | AK000850 | Hs.272203 | Homo sapiens cDNA FLJ20843 fis, clone AD | 4.70 |
| 435256 | AF193766 | Hs.13872 | cytokine-like protein C17 | 4.70 |
| 438295 | AI394151 | Hs.37932 | ESTs | 4.70 |
| 442655 | AW027457 | Hs.30323 | ESTs, Weakly similar to B34087 hypotheti | 4.70 |
| 415788 | AW628686 | Hs.78851 | KIAA0217 protein | 4.69 |
| 442760 | BE075297 | Hs.10067 | ESTs, Weakly similar to A43932 mucin 2 p | 4.69 |
| 432432 | AA541323 | Hs.115831 | ESTs | 4.68 |
| 454398 | AA463437 | Hs.11556 | Homo sapiens cDNA FLJ12566 fis, clone NT | 4.68 |
| 452741 | BE392914 | Hs.30503 | Homo sapiens cDNA FLJ11344 fis, clone PL | 4.67 |
| 424853 | BE549737 | Hs.132967 | Human EST clone 122887 mariner transposo | 4.67 |
| 419706 | C04649 | Hs.77899 | tropomyosin 1 (alpha) | 4.66 |
| 412088 | AI689496 | Hs.108932 | ESTs | 4.65 |
| 416276 | U41060 | Hs.79136 | LIV-1 protein, estrogen regulated | 4.64 |
| 429281 | AA830856 | Hs.29808 | Homo sapiens cDNA:FLJ21122 fis, clone C | 4.64 |
| 448207 | AI475490 | Hs.170577 | ESTs | 4.64 |
| 408374 | AW025430 | Hs.155591 | forkhead box F1 | 4.64 |
| 447162 | BE328091 | Hs.157396 | ESTs, Weakly similar to A46010 X-linked | 4.64 |
| 451900 | AB023199 | Hs.27207 | KIAA0982 protein | 4.63 |
| 421437 | AW821252 | Hs.104336 | hypothetical protein | 4.63 |
| 418624 | AI734080 | Hs.104211 | ESTs | 4.63 |
| 426172 | AA371307 | Hs.125056 | ESTs | 4.62 |
| 439831 | AW136488 | Hs.25545 | ESTs | 4.61 |
| 452994 | AW962597 | Hs.31305 | KIAA1547 protein | 4.61 |
| 457726 | AI217477 | Hs.194591 | ESTs | 4.60 |
| 434629 | AA789081 | Hs.4029 | glioma-amplified sequence-41 | 4.60 |
| 403764 | | | | 4.58 |
| 410659 | AI080175 | Hs.68826 | ESTs | 4.58 |
| 432383 | AK000144 | Hs.274449 | Homo sapiens cDNA FLJ20137 fis, clone CO | 4.58 |
| 451246 | AW189232 | Hs.39140 | cutaneous T-cell lymphoma tumor antigen | 4.58 |
| 433234 | AB040928 | Hs.65366 | KIAA1495 protein | 4.57 |
| 424983 | AI742434 | Hs.169911 | ESTs | 4.56 |
| 437812 | AI582291 | Hs.16846 | ESTs, Weakly similar to O4HUD1 debrisoqu | 4.56 |
| 438447 | AI082883 | Hs.167593 | hypothetical protein FLJ13409; KIAA1711 | 4.55 |
| 434715 | BE005346 | Hs.116410 | ESTs | 4.55 |
| 447673 | AI823987 | Hs.182285 | ESTs | 4.54 |
| 408897 | N50204 | Hs.283709 | lipopolysaccharide specific response-7 p | 4.54 |
| 436645 | AW023424 | Hs.156520 | ESTs | 4.54 |
| 421247 | BE391727 | Hs.102910 | general transcription factor IIH, polype | 4.53 |
| 450377 | AB033091 | Hs.24936 | KIAA1265 protein | 4.53 |
| 433644 | AW342028 | Hs.256112 | gb:hb75d03.x1 NCI_CGAP_Ut2 Homo sapiens | 4.53 |
| 408321 | AW405882 | Hs.44205 | cortistatin | 4.53 |
| 439225 | AA192669 | Hs.45032 | ESTs | 4.52 |
| 440348 | AW015802 | Hs.47023 | ESTs | 4.52 |
| 446351 | AW444551 | Hs.258532 | x001 protein | 4.52 |
| 451212 | AW902672 | Hs.287334 | ESTs | 4.52 |
| 430294 | AI538226 | Hs.135184 | guanine nucleotide binding protein 4 | 4.52 |
| 435005 | U80743 | Hs.4316 | trinucleotide repeat containing 12 | 4.52 |
| 448072 | AI459306 | Hs.24908 | ESTs | 4.50 |
| 403721 | | | | 4.50 |
| 451018 | AW965599 | Hs.247324 | mitochondrial ribosomal protein S14 | 4.50 |
| 453070 | AK001465 | Hs.31575 | SEC63, endoplasmic reticulum translocon | 4.49 |
| 417412 | X16896 | Hs.82112 | interleukin 1 receptor, type I | 4.48 |
| 439735 | AI635386 | Hs.142846 | hypothetical protein | 4.48 |
| 435663 | AI023707 | Hs.134273 | ESTs | 4.48 |
| 424036 | AA770688 | Hs.81946 | H2A histone family, member L | 4.48 |
| 426386 | AA748850 | Hs.174877 | bladder cancer overexpressed protein | 4.48 |
| 408622 | AA056060 | Hs.202577 | Homo sapiens cDNA FLJ12166 fis, clone MA | 4.47 |
| 444269 | AI590346 | Hs.146220 | ESTs | 4.47 |
| 430187 | AI799909 | Hs.158989 | ESTs | 4.46 |
| 427761 | AA412205 | Hs.140996 | ESTs | 4.46 |
| 430261 | AA305127 | Hs.237225 | hypothetical protein HT023 | 4.46 |
| 444169 | AV648170 | Hs.58756 | ESTs | 4.44 |
| 430598 | AK001764 | Hs.247112 | hypothetical protein FLJ10902 | 4.44 |
| 412903 | BE007967 | Hs.155795 | ESTs | 4.44 |
| 417048 | AI088775 | Hs.55498 | geranylgeranyl diphosphate synthase 1 | 4.44 |
| 442710 | AI015631 | Hs.23210 | ESTs | 4.44 |
| 457413 | AA743462 | Hs.165337 | ESTs | 4.44 |
| 400303 | AA242758 | Hs.79136 | LIV-1 protein, estrogen regulated | 4.42 |
| 443268 | AI800271 | Hs.129445 | hypothetical protein FLJ12496 | 4.42 |
| 438209 | AL120659 | Hs.6111 | aryl-hydrocarbon receptor nuclear transl | 4.42 |
| 431724 | AA514535 | Hs.283704 | ESTs | 4.41 |
| 412280 | AW205116 | Hs.272814 | hypothetical protein DKFZp434E1723 | 4.40 |
| 440801 | AA906366 | Hs.190535 | ESTs | 4.40 |
| 452959 | AI933416 | Hs.189674 | ESTs | 4.40 |
| 453861 | AI026838 | Hs.30120 | ESTs, Weakly similar to NUCL_HUMAN NUCLE | 4.40 |
| 417421 | AL138201 | Hs.82120 | nuclear receptor subfamily 4, group A, m | 4.40 |
| 447270 | AC002551 | Hs.331 | general transcription factor IIIC, polyp | 4.38 |
| 433641 | AF080229 | | gb:Human endogenous retrovirus K clone 1 | 4.38 |
| 447078 | AW885727 | Hs.301570 | ESTs | 4.38 |
| 424242 | AA337476 | | hypothetical protein MGC13102 | 4.37 |
| 408170 | AW204516 | Hs.31835 | ESTs | 4.36 |
| 448757 | AI366784 | Hs.48820 | TATA box binding protein (TBP)-associate | 4.36 |
| 420021 | AA252848 | Hs.293557 | ESTs | 4.36 |
| 449694 | AI659790 | Hs.253302 | ESTs | 4.36 |
| 453867 | AI929383 | Hs.108196 | hypothetical protein DKFZp434N185 | 4.36 |
| 458712 | AI347502 | Hs.173066 | hypothetical protein FLJ20761 | 4.36 |
| 417251 | AW015242 | Hs.99488 | ESTs, Weakly similar to YK54_YEAST HYPOT | 4.35 |
| 434423 | NM_006769 | Hs.3844 | LIM domain only 4 | 4.35 |
| 423427 | AL137612 | Hs.285848 | KIAA1454 protein | 4.34 |
| 415715 | F30364 | | ESTs | 4.33 |
| 404561 | | | | 4.32 |
| 422969 | AA782536 | Hs.122647 | N-myristoyltransferase 2 | 4.32 |
| 423685 | BE350494 | Hs.49753 | uveal autoantigen with coiled coil domai | 4.32 |
| 443977 | AL120986 | Hs.150627 | ESTs, Weakly similar to l38022 hypotheti | 4.32 |
| 425071 | NM_013989 | Hs.154424 | deiodinase, iodothyronine, type II | 4.32 |
| 431583 | AL042613 | Hs.262476 | S-adenosylmethionine decarboxylase 1 | 4.31 |
| 411379 | AI816344 | Hs.12554 | ESTs, Weakly similar to NPL4_HUMAN NUCLE | 4.30 |
| 421476 | AW953805 | Hs.21887 | ESTs | 4.30 |
| 425178 | H16097 | Hs.161027 | ESTs | 4.30 |
| 439262 | AA832333 | Hs.124399 | ESTs | 4.30 |
| 442818 | AK001741 | Hs.8739 | hypothetical protein FLJ10879 | 4.30 |
| 421977 | W94197 | Hs.110165 | ribosomal protein L26 homolog | 4.29 |
| 437114 | AA836641 | Hs.163085 | ESTs | 4.28 |
| 420195 | N44348 | Hs.300794 | Homo sapiens cDNA FLJ11177 fis, clone PL | 4.28 |
| 418330 | BE409405 | Hs.94722 | ESTs | 4.27 |
| 419750 | AL079741 | Hs.183114 | Homo sapiens cDNA FLJ14236 fis, clone NT | 4.26 |
| 437065 | AL036450 | Hs.103238 | ESTs | 4.26 |
| 455276 | BE176479 | | gb:RC3-HT0585-16030-022-b09 HT0585 Homo | 4.24 |
| 416292 | AA179233 | Hs.42390 | nasopharyngeal carcinoma susceptibility | 4.24 |
| 423740 | Y07701 | Hs.132243 | aminopeptidase puromycin sensitive | 4.24 |
| 442023 | AI187878 | Hs.144549 | ESTs | 4.24 |
| 426764 | AA732524 | Hs.151464 | ESTs, Weakly similar to ALUC_HUMAN !!!! | 4.23 |
| 454058 | AI273419 | Hs.135146 | hypothetical protein FLJ13984 | 4.23 |
| 456511 | AA282330 | Hs.145668 | ESTs | 4.22 |
| 448330 | AL036449 | Hs.207163 | ESTs | 4.22 |
| 424701 | NM_005923 | Hs.151988 | mitogen-activated protein kinase kinase | 4.21 |
| 432621 | AI298501 | Hs.12807 | ESTs, Weakly similar to T46428 hypotheti | 4.20 |
| 445707 | AI248720 | Hs.114390 | ESTs | 4.20 |
| 419910 | AA662913 | Hs.190173 | ESTs, Weakly similar to A46010 X-linked | 4.20 |
| 424085 | NM_002914 | Hs.139226 | replication factor C (activator 1) 2 (40 | 4.20 |
| 440749 | W22335 | Hs.7392 | hypothetical protein MGC3199 | 4.20 |
| 442787 | W93048 | Hs.227203 | hypothetical protein MGC2747 | 4.20 |
| 443414 | R54594 | Hs.25209 | ESTs | 4.20 |
| 443556 | AA256769 | Hs.94949 | methylmalonyl-CoA epimerase | 4.20 |
| 444170 | AW613879 | Hs.102408 | ESTs | 4.20 |
| 446751 | AA766998 | Hs.85874 | Human DNA sequence from clone RP11-16L21 | 4.20 |
| 421041 | N36914 | Hs.14691 | ESTs, Moderately similar to l38022 hypot | 4.19 |
| 447476 | BE293466 | Hs.20880 | ESTs, Weakly similar to l38022 hypotheti | 4.19 |
| 448543 | AW897741 | Hs.21380 | Homo sapiens mRNA; cDNA DKFZp586P1124 (f | 4.18 |
| 410294 | AB014515 | Hs.288891 | KIAA0615 gene product | 4.18 |
| 433607 | AA602004 | Hs.23260 | ESTs | 4.18 |
| 435552 | AI668636 | Hs.193480 | ESTs, Moderately similar to ALU6_HUMAN A | 4.18 |
| 447124 | AW976438 | Hs.17428 | RBP1-like protein | 4.18 |
| 453308 | AW959731 | Hs.32538 | ESTs | 4.17 |
| 439328 | W07411 | Hs.118212 | ESTs, Moderately similar to ALU3_HUMAN A | 4.16 |
| 430473 | AW130690 | Hs.299842 | ESTs | 4.16 |
| 437257 | AI283085 | Hs.290931 | ESTs, Weakly similar to YFJ7_YEAST HYPOT | 4.16 |
| 438018 | AK001160 | Hs.5999 | hypothetical protein FLJ10298 | 4.16 |
| 443857 | AI089292 | Hs.287621 | hypothetical protein FLJ14069 | 4.15 |
| 446711 | AF169692 | Hs.12450 | protocadherin 9 | 4.15 |
| 419103 | Z40229 | Hs.96423 | hypothetical protein FLJ23033 | 4.14 |
| 405403 | | | | 4.14 |
| 407378 | AA299264 | | ESTs, Moderately similar to l38022 hypot | 4.14 |
| 408986 | AW298602 | Hs.197687 | ESTs | 4.14 |
| 418727 | AA227609 | Hs.94834 | ESTs | 4.14 |
| 434400 | AI478211 | Hs.186896 | Homo sapiens cDNA FLJ11417 fis, clone HE | 4.14 |
| 438578 | AA811244 | Hs.164168 | ESTs | 4.14 |
| 450459 | AI697193 | Hs.299254 | Homo sapiens cDNA:FLJ23597 fis, clone L | 4.14 |
| 429887 | AW366286 | Hs.145696 | splicing factor (CC1.3) | 4.13 |
| 448148 | NM_016578 | Hs.20509 | HBV pX associated protein-8 | 4.13 |
| 450316 | W84446 | Hs.17850 | hypothetical protein MGC4643 | 4.12 |
| 417531 | NM_003157 | Hs.1087 | serine/threonine kinase 2 | 4.12 |
| 431592 | R69016 | Hs.293871 | hypothetical protein MGC10895s | 4.12 |
| 432463 | AA548518 | Hs.186733 | ESTs | 4.12 |
| 433613 | AA836126 | Hs.5669 | ESTs | 4.12 |
| 434739 | AA804487 | Hs.144130 | ESTs | 4.12 |
| 438259 | AW205969 | Hs.131808 | ESTs | 4.12 |
| 425810 | AI923627 | Hs.31903 | ESTs | 4.10 |
| 432672 | AW973775 | Hs.130760 | myosin phosphatase, target subunit 2 | 4.10 |
| 433345 | AI681545 | Hs.152982 | hypothetical protein FLJ13117 | 4.10 |
| 432712 | AB016247 | Hs.288031 | sterol-C5-desaturase (fungal ERG3, delta | 4.09 |
| 453020 | AL162039 | Hs.31422 | Homo sapiens mRNA; cDNA DKFZp434M229 (fr | 4.09 |
| 412045 | AA099802 | Hs.4299 | transmembrane, prostate androgen induced | 4.09 |
| 435114 | AA775483 | Hs.288936 | mitochondrial ribosomal protein L9 | 4.08 |
| 443204 | AW205878 | Hs.29643 | Homo sapiens cDNA FLJ13103 fis, clone NT | 4.08 |
| 445459 | AI478629 | Hs.158465 | likely ortholog of mouse putative IKK re | 4.08 |
| 438938 | H46212 | Hs.137221 | ESTs | 4.07 |
| 454119 | BE549773 | Hs.40510 | uncoupling protein 4 | 4.06 |
| 411000 | N40449 | Hs.201619 | ESTs, Weakly similar to S38383 SEB4B pro | 4.06 |
| 418926 | AA232658 | Hs.87070 | UDP-glucose:glycoprotein glucosyltransfe | 4.06 |
| 424432 | AB037821 | Hs.146858 | protocadherin 10 | 4.06 |
| 449673 | AA002064 | Hs.18920 | ESTs | 4.06 |
| 429299 | AI620463 | Hs.99197 | hypothetical protein MGC13102 | 4.06 |
| 422174 | AL049325 | Hs.112493 | Homo sapiens mRNA;cDNA DKFZp564D036 (fr | 4.05 |
| 455497 | AA112573 | Hs.285691 | Homo sapiens prostein mRNA, complete cds | 4.05 |
| 415138 | C18356 | Hs.78045 | tissue factor pathway inhibitor 2 | 4.04 |
| 402791 | | | | 4.04 |
| 426792 | AL044854 | Hs.172329 | KIAA0576 protein | 4.04 |
| 438660 | U95740 | Hs.6349 | Homo sapiens, clone IMAGE:3010666, mRNA, | 4.04 |
| 442768 | AL048534 | Hs.48458 | ESTs, Weakly similar to ALU8_HUMAN ALU S | 4.04 |
| 447568 | AF155655 | Hs.18885 | CGI-116 protein | 4.04 |
| 428342 | AI739168 | Hs.131798 | Homo sapiens cDNA FLJ13458 fis, clone PL | 4.04 |
| 453439 | AI572438 | Hs.32976 | guanine nucleotide binding protein 4 | 4.02 |
| 453857 | AL080235 | Hs.35861 | DKFZP586E1621 protein | 4.02 |
| 428249 | AA130914 | Hs.183291 | zinc finger protein 268 | 4.02 |
| 432015 | AL157504 | Hs.159115 | Homo sapiens mRNA; cDNA DKFZp586O0724 (f | 4.02 |
| 445495 | BE622641 | Hs.38489 | ESTs, Weakly similar to l38022 hypotheti | 4.02 |
| 451746 | M86178 | | ESTs | 4.02 |
| 452211 | AI985513 | Hs.233420 | ESTs | 4.02 |
| 453046 | AA284040 | Hs.219441 | ESTs, Highly similar to CA5B_HUMAN CARBO | 4.02 |
| 456038 | AA203285 | Hs.294141 | ESTs, Weakly similar to alternatively sp | 4.02 |
| 452449 | AW068658 | Hs.20943 | ESTs | 4.02 |
| 407204 | R41933 | Hs.140237 | ESTs, Weakly similar to ALU1_HUMAN ALU S | 4.01 |
| 428046 | AW812795 | Hs.155381 | ESTs, Moderately similar to l38022 hypot | 4.01 |
| 438520 | AA706319 | Hs.98416 | ESTs | 4.01 |
| 443292 | AK000213 | Hs.9196 | hypothetical protein | 4.01 |
| 432715 | AA247152 | Hs.200483 | ESTs, Weakly similar to KIAA1074 protein | 4.00 |
| 403797 | | | | 4.00 |
| 418347 | AA216419 | Hs.269295 | gb:nc16e03.s1 NCI_CGAP_Pr1 Homo sapiens | 4.00 |
| 419459 | AW291128 | Hs.278422 | DKFZP586G1122 protein | 4.00 |
| 420911 | U77413 | Hs.100293 | O-linked N-acetylglucosamine (GlcNAc) tr | 4.00 |
| 425176 | AW015644 | Hs.301430 | TEA domain family member 1 (SV40 transcr | 4.00 |
| 447505 | AL049266 | Hs.18724 | Homo sapiens mRNA; cDNA DKFZp564F093 (fr | 4.00 |
| 453773 | AL133761 | | gb:DKFZp761C1413_r1 761 (synonym: hamy2) | 4.00 |
| 434384 | AA631910 | Hs.162849 | ESTs | 3.99 |
| 422471 | AA311027 | Hs.271894 | ESTs, Weakly similar to l38022 hypotheti | 3.99 |
| 427386 | AW836261 | Hs.177486 | ESTs | 3.98 |
| 433394 | AI907753 | Hs.93810 | cerebral cavernous malformations 1 | 3.98 |
| 441269 | AW015206 | Hs.178784 | ESTs | 3.97 |
| 419629 | AB020695 | Hs.91662 | KIAA0888 protein | 3.96 |
| 435008 | AF150262 | Hs.162898 | ESTs | 3.96 |
| 456649 | R74441 | Hs.117176 | poly(A)-binding protein, nuclear 1 | 3.96 |
| 418723 | AA504428 | Hs.10487 | Homo sapiens, clone IMAGE:3954132, mRNA, | 3.96 |
| 428738 | NM_000380 | Hs.192803 | xeroderma pigmentosum, complementation g | 3.95 |
| 430456 | AA314998 | Hs.241503 | hypothetical protein | 3.95 |
| 422017 | NM_003877 | Hs.110776 | STAT induced STAT inhibitor-2 | 3.95 |
| 409960 | BE261944 | Hs.153028 | hexokinase 1 | 3.95 |
| 455309 | AW894017 | | gb:RC4-NN0027-150400-012-g04 NN0027 Homo | 3.95 |
| 450295 | AI766732 | Hs.201194 | ESTs | 3.94 |
| 456660 | AA909249 | Hs.112282 | solute carrier family 30 (zinc transport | 3.94 |
| 410908 | AA121686 | Hs.10592 | ESTs | 3.94 |
| 447145 | AA761073 | Hs.192943 | TRAF family member-associated NFKB activ | 3.94 |
| 449318 | AW236021 | Hs.108788 | Homo sapiens, Similar to RIKEN cDNA 5730 | 3.94 |
| 449869 | W57990 | Hs.60059 | Homo sapiens cDNA FLJ11478 fis, clone HE | 3.94 |
| 411887 | AW182924 | Hs.128790 | ESTs | 3.93 |
| 437531 | AI400752 | Hs.112259 | T cell receptor gamma locus | 3.93 |
| 452238 | F01811 | Hs.187931 | ESTs | 3.93 |
| 410486 | AW235094 | Hs.193424 | zinc finger protein | 3.92 |
| 424882 | AI379461 | Hs.153636 | far upstream element (FUSE) binding prot | 3.92 |
| 426269 | H15302 | Hs.168950 | Homo sapiens mRNA; cDNA DKPZp566A1046 (f | 3.92 |
| 427043 | AA397679 | Hs.298460 | ESTs | 3.92 |
| 440404 | AI015881 | Hs.125616 | mitochondrial ribosomal protein S5 | 3.92 |
| 452762 | AW501435 | Hs.171409 | v-akt murine thymoma viral oncogene homo | 3.92 |
| 453058 | AW612293 | Hs.288684 | Homo sapiens cDNA FLJ11750 fis, clone HE | 3.92 |
| 423583 | AL122055 | Hs.129836 | KIAA1028 protein | 3.92 |
| 408001 | AA046458 | Hs.95296 | ESTs | 3.92 |
| 419197 | N48921 | Hs.27441 | KIAA1615 protein | 3.91 |
| 428695 | AI355647 | Hs.189999 | purinergic receptor (family A group 5) | 3.91 |
| 401747 | | | | 3.91 |
| 410011 | AB020641 | Hs.57856 | PFTAIRE protein kinase 1 | 3.91 |
| 432205 | AI806583 | Hs.125291 | ESTs | 3.91 |
| 447857 | AA081218 | Hs.58608 | Homo sapiens cDNA FLJ14206 fis, clone NT | 3.91 |
| 446494 | AA463276 | Hs.288906 | WW Domain-Containing Gene | 3.91 |
| 409928 | AL137163 | Hs.57549 | hypothetical protein dJ473B4 | 3.90 |
| 411598 | BE336654 | Hs.70937 | H3 histone family, member A | 3.90 |
| 424790 | AL119344 | Hs.13326 | ESTs, Weakly similar to 2004399A chromos | 3.90 |
| 425707 | AF115402 | Hs.11713 | E74-like factor 5 (ets domain transcript | 3.90 |
| 431325 | AW026751 | Hs.5794 | ESTs, Weakly similar to 2109260A B cell | 3.89 |
| 451806 | NK_003729 | Hs.27076 | RNA 3'-terminal phosphate cyclase | 3.89 |
| 401045 | | | | 3.89 |
| 433023 | AW864793 | Hs.34161 | thrombospondin 1 | 3.89 |
| 452160 | BE378541 | Hs.279815 | cysteine sulfinic acid decarboxylase-rel | 3.89 |
| 437372 | AA323968 | Hs.283631 | hypothetical protein DKFZp547G183 | 3.89 |
| 417067 | AJ001417 | Hs.81086 | solute carrier family 22 (extraneuronal | 3.88 |
| 410467 | AF102546 | Hs.63931 | dachshund (Drosophila) homolog | 3.88 |
| 422660 | AW297582 | Hs.237062 | hypothetical protein FLJ22548 similar to | 3.88 |
| 431930 | AB035301 | Hs.272211 | cadherin 7, type 2 | 3.88 |
| 453047 | AW023798 | Hs.286025 | ESTs | 3.88 |
| 433891 | AA613792 | | gb:no97h03.s1 NCI_CGAP_Pr2 Homo sapiens | 3.88 |
| 401785 | | | | 3.88 |
| 431088 | AA491824 | Hs.196881 | ESTs | 3.88 |
| 451952 | AL120173 | Hs.301663 | ESTs | 3.87 |
| 422089 | AA523172 | Hs.103135 | ESTs, Weakly similar to SFR4_HUMAN SPLIC | 3.87 |
| 452277 | AL049013 | Hs.28783 | KIAA1223 protein | 3.87 |
| 438279 | AA805166 | Hs.165165 | HIV-1 rev binding protein 2 | 3.86 |
| 458229 | AI929602 | Hs.177 | phosphatidylinositol glycan, class H | 3.86 |
| 406414 | | | | 3.86 |
| 417193 | AI922189 | Hs.288390 | hypothetical protein FLJ22795 | 3.85 |
| 413174 | AA723564 | Hs.191343 | ESTs | 3.85 |
| 433332 | AI367347 | Hs.127809 | Homo sapiens clone TCCCTA00151 mRNA sequ | 3.85 |
| 411089 | AA456454 | Hs.118637 | cell division cycle 2-like 1 (PITSLRE pr | 3.85 |
| 412494 | AL133900 | Hs.792 | ADP-ribosylation factor domain protein 1 | 3.84 |
| 413530 | AA130158 | Hs.19977 | ESTs, Moderately similar to ALU8_HUMAN A | 3.84 |
| 459592 | AL037421 | Hs.208746 | ESTs, Moderately similar to pot. ORFl [ | 3.84 |
| 418329 | AW247430 | Hs.84152 | cystathionine-beta-synthase | 3.83 |
| 451468 | AW503398 | Hs.210047 | ESTs, Moderately similar to l38022 hypot | 3.83 |
| 434804 | AA649530 | | gb:ns44f05.s1 NCI_CGAP_Alv1 Homo sapiens | 3.83 |
| 401819 | | | | 3.82 |
| 424179 | F30712 | | Homo sapiens, clone IMAGE:4285740, mRNA | 3.82 |
| 424850 | AA151057 | Hs.153498 | chromosome 18 open reading frame 1 | 3.82 |
| 426472 | BE246138 | Hs.30853 | ESTs | 3.82 |
| 426625 | T78300 | Hs.171409 | serologically defined colon cancer antig | 3.82 |
| 427585 | D31152 | Hs.179729 | collagen, type X, alpha 1 (Schmid metaph | 3.82 |
| 427756 | AI376540 | Hs.15574 | ESTs | 3.82 |
| 444701 | AI916512 | Hs.198394 | ESTs | 3.82 |
| 423052 | M28214 | Hs.123072 | RAB3B, member RAS oncogene family | 3.82 |
| 429259 | AA420450 | Hs.292911 | ESTs, Highly similar to S60712 band-6-pr | 3.82 |
| 416111 | AA033813 | Hs.79018 | chromatin assembly factor 1, subunit A ( | 3.82 |
| 433586 | T85301 | | gb:yd78d06.s1 Soares fetal liver spleen | 3.81 |
| 438527 | AI969251 | Hs.143237 | RAB7, member RAS oncogene family-like 1 | 3.81 |
| 410297 | AA148710 | Hs.159441 | lumican | 3.81 |
| 429898 | AW117322 | Hs.42366 | ESTs | 3.81 |
| 409079 | W87707 | Hs.82065 | interleukin 6 signal transducer (gp130, | 3.80 |
| 419423 | D26488 | Hs.90315 | KIAA0007 protein | 3.80 |
| 429643 | AA455889 | Hs.187548 | FYVE-finger-containing Rab5 effector pro | 3.80 |
| 431499 | NM_001514 | Hs.258561 | general transcription factor IIB | 3.80 |
| 445060 | AA830811 | Hs.88808 | ESTs | 3.80 |
| 449419 | R34910 | Hs.119172 | ESTs | 3.80 |
| 450584 | AA040403 | Hs.60371 | ESTs | 3.80 |
| 426137 | AL040683 | Hs.167031 | DKFZP566D133 protein | 3.79 |
| 420185 | AL044056 | Hs.158047 | ESTs | 3.79 |
| 410076 | T05387 | Hs.7991 | ESTs | 3.78 |
| 444078 | BE246919 | Hs.10290 | U5 snRNP-specific 40 kDa protein (hPrp8- | 3.78 |
| 417318 | AW953937 | Hs.12891 | ESTs | 3.78 |
| 414664 | AA587775 | Hs.66295 | multi-PDZ-domain-containing protein | 3.78 |
| 410275 | U85658 | Hs.61796 | transcription factor AP-2 gamma (activat | 3.77 |
| 410503 | AW975746 | Hs.188662 | KIAA1702 protein | 3.77 |
| 434170 | AA626509 | Hs.122329 | ESTs | 3.77 |
| 421838 | AW881089 | Hs.108806 | Homo sapiens mRNA; cDNA DKFZp566M0947 (f | 3.77 |
| 425268 | AI807883 | Hs.156932 | Homo sapiens cDNA FLJ20653 fis, clone KA | 3.76 |
| 431696 | AA259068 | Hs.267819 | protein phosphatase 1, regulatory (inhib | 3.76 |
| 411990 | AW963624 | Hs.31707 | ESTs, Weakly similar to YEW4_YEAST HYPOT | 3.76 |
| 430291 | AV660345 | Hs.238126 | CGI-49 protein | 3.76 |
| 448779 | BE042877 | Hs.177135 | ESTs | 3.76 |
| 452682 | AA456193 | Hs.155606 | progesterone membrane binding protein | 3.75 |
| 452598 | AI831594 | Hs.68647 | ESTs, Weakly similar to ALU7_HUMAN ALU S | 3.75 |
| 439498 | AA908731 | Hs.58297 | CLLL8 protein | 3.75 |
| 440258 | AI741633 | Hs.125350 | ESTs | 3.74 |
| 456848 | AL121087 | Hs.296406 | KIAA0685 gene product | 3.74 |
| 415082 | AA160000 | Hs.137396 | ESTs, Weakly similar to JC5238 galactosy | 3.74 |
| 420653 | AI224532 | Hs.88550 | ESTs | 3.74 |
| 431637 | AI879330 | Hs.265960 | hypothetical protein FLJ10563 | 3.74 |
| 440411 | N30256 | Hs.156971 | hypothetical protein DKFZp434G1415 | 3.74 |
| 405917 | | | | 3.74 |
| 419440 | AB020689 | Hs.90419 | KIAA0882 protein | 3.74 |
| 451230 | BE546208 | Hs.26090 | hypothetical protein FLJ20272 | 3.73 |
| 429597 | NM_003816 | Hs.2442 | a disintegrin and metalloproteinase doma | 3.73 |
| 430144 | AI732722 | Hs.187694 | ERGL protein; ERGIC-53-like protein | 3.72 |
| 438394 | BE379623 | Hs.27693 | peptidylprolyl isomerase (cyclophilin)-l | 3.72 |
| 440527 | AV657117 | Hs.184164 | ESTs, Moderately similar to S65657 alpha | 3.72 |
| 449433 | AI672096 | Hs.9012 | ESTs, Weakly similar to S26650 DNA-bindi | 3.72 |
| 456228 | BE503227 | Hs.134759 | ESTs | 3.72 |
| 448663 | BE614599 | Hs.106823 | hypothetical protein MGC14797 | 3.72 |
| 415075 | L27479 | Hs.77889 | Friedreich ataxia region gene X123 | 3.72 |
| 433544 | AI793211 | Hs.165372 | ESTs, Moderately similar to ALU1_HUMAN A | 3.71 |
| 418293 | AI224483 | Hs.16063 | hypothetical protein FLJ21877 | 3.71 |
| 449897 | AW819642 | Hs.24135 | transmembrane protein vezatin; hypotheti | 3.71 |
| 420297 | AI628272 | Hs.88323 | ESTs, Weakly similar to ALU1_HUMAN ALU S | 3.70 |
| 423065 | R96158 | Hs.194606 | Homo sapiens, clone MGC:5406, mRNA, comp | 3.70 |
| 429340 | N35938 | Hs.199429 | Homo sapiens mRNA; cDNA DKFZp434M2216 (f | 3.70 |
| 437777 | AA768098 | Hs.189079 | ESTs | 3.70 |
| 440351 | AF030933 | Hs.7179 | RAD1 (S. pombe) homolog | 3.70 |
| 443603 | BE502601 | Hs.134289 | ESTs, Weakly similar to KIAA1063 protein | 3.70 |
| 446965 | BE242873 | Hs.16677 | WD repeat domain 15 | 3.70 |
| 412350 | AI659306 | Hs.73826 | protein tyrosine phosphatase, non-recept | 3.70 |
| 433852 | AI378329 | Hs.126629 | ESTs | 3.70 |
| 433142 | AL120697 | Hs.110640 | ESTs | 3.69 |
| 419994 | AA282881 | Hs.190057 | ESTs | 3.69 |
| 412628 | AI972402 | Hs.173902 | hypothetical protein MGC2648 | 3.69 |
| 431416 | AA532718 | Hs.178604 | ESTs | 3.69 |
| 439444 | AI277652 | Hs.54578 | ESTs, Weakly similar to l38022 hypotheti | 3.68 |
| 414709 | AA704703 | Hs.77031 | Sp2 transcription factor | 3.68 |
| 447397 | BE247676 | Hs.18442 | E-1 enzyme | 3.68 |
| 405718 | | | | 3.68 |
| 425217 | AU076696 | Hs.155174 | CDC5 (cell division cycle 5, S. pombe, h | 3.68 |
| 442242 | AV647908 | Hs.90424 | Homo sapiens cDNA:FLJ23285 fis, clone H | 3.68 |
| 424690 | BE538356 | Hs.151777 | eukaryotic translation initiation factor | 3.68 |
| 421734 | AI318624 | Hs.107444 | Homo sapiens cDNA FLJ20562 fis, clone KA | 3.67 |
| 427221 | L15409 | Hs.174007 | von Hippel-Lindau syndrome | 3.67 |
| 439864 | AI720078 | Hs.291997 | ESTs, Weakly similar to A47582 B-cell gr | 3.66 |
| 402408 | | | | 3.66 |
| 426327 | W03242 | Hs.44898 | Homo sapiens clone TCCCTA00151 mRNA sequ | 3.66 |
| 427119 | AW880562 | Hs.114574 | ESTs | 3.66 |
| 427356 | AW023482 | Hs.97849 | ESTs | 3.66 |
| 452946 | X95425 | Hs.31092 | EphA5 | 3.66 |
| 419078 | M93119 | Hs.89584 | insulinoma-associated 1 | 3.66 |
| 416295 | AI064824 | Hs.193385 | ESTs | 3.65 |
| 427144 | X95097 | Hs.2126 | vasoactive intestinal peptide receptor 2 | 3.65 |
| 447500 | AI381900 | Hs.159212 | ESTs | 3.65 |
| 453127 | AI696671 | Hs.294110 | ESTs | 3.65 |
| 423396 | AI382555 | Hs.127950 | bromodomain-containing 1 | 3.65 |
| 419346 | AI830417 | | polybromo 1 | 3.64 |
| 441540 | C01367 | Hs.127128 | ESTs | 3.64 |
| 446501 | AI302616 | Hs.150819 | ESTs | 3.64 |
| 459527 | AW977556 | Hs.291735 | ESTs, Weakly similar to l78885 serine/th | 3.63 |
| 446320 | AF126245 | Hs.14791 | acyl-Coenzyme A dehydrogenase family, me | 3.63 |
| 435706 | W31254 | Hs.7045 | GL004 protein | 3.63 |
| 400110 | | | | 3.62 |
| 410313 | R10305 | Hs.185683 | ESTs | 3.62 |
| 414713 | BE465243 | Hs.12664 | ESTs | 3.62 |
| 436279 | AW900372 | Hs.180793 | ESTs, Weakly similar to S65657 alpha-1C- | 3.62 |
| 439818 | AL360137 | Hs.19934 | Homo sapiens mRNA full length insert cDN | 3.62 |
| 451797 | AW663858 | Hs.56120 | small inducible cytokine subfamily E, me | 3.62 |
| 451294 | AI457338 | Hs.29894 | ESTs | 3.62 |
| 434194 | AF119847 | Hs.283940 | Homo sapiens PRO1550 mRNA, partial cds | 3.62 |
| 404939 | | | | 3.62 |
| 408101 | AW968504 | Hs.123073 | CDC2-related protein kinase 7 | 3.62 |
| 435846 | AA700870 | Hs.14304 | ESTs | 3.61 |
| 432833 | N51075 | Hs.47191 | ESTs | 3.61 |
| 427276 | AA400269 | Hs.49598 | ESTs | 3.61 |
| 433495 | AW373784 | Hs.71 | alpha-2-glycoprotein 1, zinc | 3.60 |
| 403137 | | | | 3.60 |
| 404165 | | | | 3.60 |
| 409571 | AA504249 | Hs.187585 | ESTs | 3.60 |
| 410561 | BE540255 | Hs.6994 | Homo sapiens cDNA: FLJ22044 fis, clone H | 3.60 |
| 412924 | BE018422 | Hs.75258 | H2A histone family, member Y | 3.60 |
| 434228 | Z42047 | Hs.283978 | Homo sapiens PRO2751 mRNA, complete cds | 3.60 |
| 436797 | AA731491 | Hs.178518 | hypothetical protein MGC14879 | 3.60 |
| 437162 | AW005505 | Hs.5464 | thyroid hormone receptor coactivating pr | 3.60 |
| 437444 | H46008 | Hs.31518 | ESTs | 3.60 |
| 404210 | | | | 3.59 |
| 446157 | BE270828 | Hs.131740 | Homo sapiens cDNA: FLJ22562 fis, clone H | 3.59 |
| 437587 | AI591222 | Hs.122421 | Human DNA sequence from clone RP1-187J11 | 3.58 |
| 423147 | AA987927 | Hs.131740 | Homo sapiens cDNA: FLJ22562 fis, clone H | 3.57 |
| 452226 | AA024898 | Hs.296002 | ESTs | 3.56 |
| 443775 | AF291664 | Hs.204732 | matrix metalloproteinase 26 | 3.56 |
| 452501 | AB037791 | Hs.29716 | hypothetical protein FLJ10980 | 3.56 |
| 428647 | AA830050 | Hs.124344 | ESTs | 3.56 |
| 422443 | NM_014707 | Hs.116753 | histone deacetylase 78 | 3.55 |
| 447966 | AA340605 | Hs.105887 | ESTs, Weakly similar to Homolog of rat Z | 3.55 |
| 420892 | AW975076 | Hs.172589 | nuclear phosphoprotein similar to S. cer | 3.55 |
| 420230 | AL034344 | Hs.298020 | forkhead box C1 | 3.55 |
| 418428 | Y12490 | Hs.85092 | thyroid hormone receptor interactor 11 | 3.54 |
| 428949 | AA442153 | Hs.104744 | hypothetical protein DKFZp434J0617 | 3.54 |
| 444929 | AI685841 | Hs.161354 | ESTs | 3.54 |
| 433339 | AF019226 | Hs.8036 | glioblastoma overexpressed | 3.54 |
| 424369 | R87622 | Hs.26714 | KIAA1831 protein | 3.54 |
| 433002 | AF048730 | Hs.279906 | cyclin T1 | 3.53 |
| 435425 | H16263 | Hs.31416 | ESTs | 3.53 |
| 415621 | AI648602 | Hs.131189 | ESTs | 3.53 |
| 416974 | AF010233 | Hs.80667 | RALBP1 associated Eps domain containing | 3.53 |
| 405793 | | | | 3.52 |
| 409770 | AW499536 | | gb:Ul-HF-BR0p-aji-c-12-0-Ul.r1 NIH_MGC_5 | 3.52 |
| 425305 | AA363025 | Hs.155572 | Human clone 23801 mRNA sequence | 3.52 |
| 428939 | AW236550 | Hs.131914 | ESTs | 3.52 |
| 438388 | AA806349 | Hs.44698 | ESTs | 3.52 |
| 443703 | AV646177 | Hs.213021 | ESTs | 3.52 |
| 457940 | AL360159 | Hs.30445 | Homo sapiens TRIpartite motif protein ps | 3.52 |
| 402444 | | | | 3.52 |
| 409643 | AW450866 | Hs.257359 | ESTs | 3.51 |
| 418250 | U29926 | Hs.83918 | adenosine monophosphate deaminase (isofo | 3.51 |
| 432745 | AI821926 | Hs.269507 | gb:nt78f05.x5 NCI_CGAP_Pr3 Homo sapiens | 3.51 |
| 414222 | AL135173 | Hs.878 | sorbitol dehydrogenase | 3.51 |
| 430061 | AB037817 | Hs.230188 | KIAA1396 protein | 3.51 |
| 421491 | H99999 | Hs.42736 | ESTs | 3.50 |
| 422384 | AA224077 | Hs.42438 | Sm protein F | 3.50 |
| 434565 | T52172 | | ESTs | 3.50 |
| 438379 | N23018 | Hs.171391 | C-terminal binding protein 2 | 3.50 |
| 439741 | BE379646 | Hs.6904 | Homo sapiens mRNA full length insert cDN | 3.50 |
| 447311 | R37010 | Hs.33417 | Homo sapiens cDNA:FLJ22806 fis, clone K | 3.50 |
| 447805 | AW627932 | Hs.19614 | gemin4 | 3.50 |
| 454265 | H03556 | Hs.300949 | ESTs, Weakly similar to thyroid hormone | 3.50 |
| 418838 | AW385224 | Hs.35198 | ectonucleotide pyrophosphatase/phosphodi | 3.50 |
| 448804 | AW512213 | Hs.42500 | ADP-ribosylation factor-like 5 | 3.50 |
| 409617 | BE003760 | Hs.55209 | Homo sapiens mRNA; cDNA DKFZp434K0514 (f | 3.49 |
| 434075 | AW003416 | Hs.160604 | ESTs | 3.49 |
| 444190 | AI878918 | Hs.10526 | cysteine and glycine-rich protein 2 | 3.49 |
| 435017 | AA336522 | Hs.12854 | angiotensin II, type I receptor-associat | 3.48 |
| 423445 | NM_014324 | Hs.128749 | alpha-methylacyl-CoA racemase | 3.48 |
| 420271 | AI954365 | Hs.42892 | ESTs | 3.48 |
| 443684 | AI681307 | Hs.166674 | ESTs | 3.48 |
| 444168 | AW379879 | | gb:RC1-HT0256-081199-011-f01 HT0256 Homo | 3.48 |
| 446074 | AA079799 | Hs.29263 | hypothetical protein FLJ11896 | 3.48 |
| 452582 | AL137407 | Hs.29911 | Homo sapiens mRNA;cDNA DKFZp434M232 (fr | 3.48 |
| 431542 | H63010 | Hs.5740 | ESTs | 3.48 |
| 432697 | AW975050 | Hs.293892 | ESTs, Weakly similar to ALU4_HUMAN ALU S | 3.48 |
| 435572 | AW975339 | Hs.239828 | ESTs, Weakly similar to GAG2_HUMAN RETRO | 3.47 |
| 407192 | AA609200 | | gb:af12e02.s1 Soares_testis_NHT Homo sap | 3.47 |
| 413435 | X51405 | Hs.75360 | carboxypeptidase E | 3.46 |
| 447210 | AF035269 | Hs.17752 | phosphatidylserine-specific phospholipas | 3.46 |
| 447958 | AW796524 | Hs.68644 | Homo sapiens microsomal signal peptidase | 3.46 |
| 425312 | AA354940 | Hs.145958 | ESTs | 3.46 |
| 442007 | AA301116 | Hs.142838 | nucleolar phosphoprotein Nopp34 | 3.46 |
| 417455 | AW007066 | Hs.18949 | ESTs, Weakly similar to CA2B_HUMAN COLLA | 3.45 |
| 426931 | NM_003416 | Hs.2076 | zinc finger protein 7 (KOX 4, clone HF.1 | 3.45 |
| 408739 | W01556 | Hs.238797 | ESTs, Moderately similar to l38022 hypot | 3.45 |
| 436024 | AI800041 | Hs.190555 | ESTs | 3.45 |
| 408418 | AW963897 | Hs.44743 | KIAA1435 protein | 3.45 |
| 409151 | AA306105 | Hs.50785 | SEC22, vesicle trafficking protein (S.c | 3.44 |
| 418626 | AW299508 | Hs.135230 | ESTs | 3.44 |
| 420560 | AW207748 | Hs.59115 | ESTs | 3.44 |
| 420686 | AI950339 | Hs.40782 | ESTs | 3.44 |
| 428870 | AA436831 | Hs.36049 | ESTs | 3.44 |
| 436754 | AI061288 | Hs.133437 | ESTs | 3.44 |
| 437960 | AI669586 | Hs.222194 | ESTs | 3.44 |
| 452300 | AW628045 | Hs.28896 | Homo sapiens mRNA full length insert cDN | 3.44 |
| 421887 | AW161450 | Hs.109201 | CGI-86 protein | 3.44 |

**TABLE 5A shows the accession numbers for those primekeys lacking a unigeneID in Tables 5, 6, and 7. For each probeset we have listed the gene cluster number from which the oligonucleotides were designed. Gene clusters were compiled using sequences derived from Genbank ESTs and mRNAs. These sequences were clustered based on sequence similarity using Clustering and Alignment Tools (DoubleTwist, Oakland California). The Genbank accession numbers for sequences comprising each cluster are listed in the "Accession" column.**

| | | |
|---|---|---|
| Pkey: | | Unique Eos probeset identifier number |
| CAT number: | | Gene cluster number |
| Accession: | | Genbank accession numbers |
| | | |

| **Pkey** | **CAT number** | **Accession** |
|---|---|---|
| | | |
| 407596 | 1003489_1 | R86913 R86901 H25352 R01370 H43764 AW044451 W21298 |
| 408432 | 1058667_1 | AW195262 R27868 AW811262 |
| 409752 | 115301_1 | AW963990 AA078196 AW749482 AA077468 BE151571 AA376917 |
| 409770 | 1154048_1 | AW499536 AW499553 AW502138 AW499537 AW502136 AW501743 |
| 411440 | 124577_1 | AW749402 AW749403 Z45743 R80376 AA093358 |
| 411479 | 1247077_1 | AW848047 AW848202 AW848631 AW848142 AW848702 AW848121 AW848632 AW848140 AW848571 |
| | | AW848009 AW848067 AW848069 AW848905 AW848214 |
| 411624 | 1252166_1 | BE145964 BE146286 AW854564 |
| 412991 | 134248_1 | AW949013 AA126111 |
| 414269 | 143133_1 | AA298489 AA137165 |
| 415123 | 1523390_1 | D60925 D60828 D80787 |
| 415715 | 1548818_1 | F30364 F36559 T15435 |
| 416288 | 1585983_1 | H51299 H44619 H46391 R86024 H51892 T72744 |
| 416289 | 1586037_1 | W26333 R05358 H44682 |
| 417730 | 1695795_1 | Z44761 R25801 R11926 R35604 |
| 418636 | 177402_1 | AW749855 AA225995 AW750208 AW750206 |
| 419346 | 184129_1 | AI830417 AA236612 |
| 419536 | 185688_1 | AA603305 AA244095 AA244183 |
| 420111 | 190755_1 | AA255652 AA280911 AW967920 AA262684 |
| 422219 | 213547_1 | AW978073 AW978072 AA807550 AA306567 |
| 424179 | 236389_1 | F30712 F35665 AW263888 AI904014 AI904018 AA336927 AA336502 |
| 424242 | 237181_1 | AA337476 AW966227 AA450376 AW960222 AA381051 |
| 428002 | 285602_1 | AA418703 AA418711 BE071915 BE071920 BE071912 |
| 429163 | 300543_1 | AA884766 AW974271 AA592975 AA447312 |
| 432189 | 342819_1 | AA527941 AI810608 AI620190 AA635266 |
| 432340 | 345248_1 | AA534222 AA632632 T81234 |
| 432363 | 345469_1 | AA534489 AW970240 AW970323 |
| 432966 | 356839_1 | AA650114 AW974148 AA572946 |
| 433586 | 370470_1 | T85301 AW517087 AA601054 BE073959 |
| 433641 | 37186_1 | AF080229 AF080231 AF080230 AF080232 AF080233 AF080234 BE550633 AI636743 AW614951 BE467547 |
| | | AI680833 AI633818 N29986 U87592 U87593 U87590 U87591 S46404 U87587 AA463992 AW206802 AI970376 |
| | | AI583718 AI672574 N25695 AW665466 AI818326 AA126128 AI480345 AW013827 AA248638 AI214968 |
| | | AA204735 AA207155 AA206262 AA204833 AW003247 AW496808 AI080480 AI631703 AI651023 AI867418 |
| | | AW818140 AA502500 AI206199 AI671282 AI352545 BE501030 AI652535 BE465762 AA206331 AW451866 |
| | | AA471088 AA206342 AA204834 AA206100 AW021661 AA332922 N66048 AA703396 H92278 AW139734 |
| | | H92683 U87569 U87595 H69001 U87594 BE466420 AI624817 BE466611 AI206344 AA574397 AA348354 |
| | | AI493192 |
| 433687 | 373061_1 | AA743991 AA604852 AW272737 |
| 433891 | 376239_1 | AA613792 AW182329 T05304 AW858385 |
| 434415 | 385931_1 | BE177494 AW276909 AA632849 |
| 434565 | 38898_1 | T52172 AF147324 T52248 |
| 434804 | 393481_1 | AA649530 AA659316 H64973 |
| 437113 | 433234_1 | AA744693 AW750059 |
| 444168 | 593829_1 | AW379879 AI126285 H12014 |
| 448212 | 755099_1 | AI475858 AW969013 |
| 448310 | 757918_1 | AI480316 AW847535 |
| 451746 | 883303_1 | M86178 AI813822 D56993 |
| 452560 | 922216_1 | BE077084 AW139963 AW863127 AW806209 AW806204 AW806205 AW806206 AW806211 AW806212 |
| | | AW806207 AW806208 AW806210 AI907497 |
| 452712 | 928309_1 | AW838616 AW838660 BE144343 AI914520 AW888910 BE184854 BE184784 |
| 453773 | 980699_1 | AL133761 AL133767 |
| 455276 | 1272541_1 | BE176479 BE176678 BE176357 BE176550 AW886079 BE176676 BE176615 BE176555 BE176489 BE176610 |
| | | BE176362 |
| 455309 | 1278153_1 | AW894017 AW893956 AW894032 |

**TABLE 5B shows the genomic positioning for those primekeys lacking unigene ID's and accession numbers in Tables 5, 6, and 7. For each predicted exon, we have listed the genomic sequence source used for prediction. Nucleotide locations of each predicted exon are also listed.**

| | | | |
|---|---|---|---|
| Pkey. | | Unique number corresponding to an Eos probeset | |
| Ref: | | Sequence source. The 7 digit numbers in this column are Genbank Identifier (GI) numbers. "Dunham I. et al." refers to the publication entitled "The DNA sequence of human chromosome 22." Dunham I. et al., Nature (1999) 402:489-495. | |
| Strand: | | Indicates DNA strand from which exons were predicted. | |
| Nt_position: | | Indicates nucleotide positions of predicted exons. | |
| | | | |

| **Pkey** | **Ref** | **Strand** | **Nt_position** |
|---|---|---|---|
| | | | |
| 401045 | 8117619 | Plus | 90044-90184,91111-91345 |
| 401424 | 8176894 | Plus | 24223-24428 |
| 401451 | 6634068 | Minus | 119926-121272 |
| 401714 | 6715702 | Plus | 96484-96681 |
| 401747 | 9789672 | Minus | 118596-118816,119119-119244,119609-119761,120422-120990,130161-130381,130468-130593,131097- |
| | | | 131258,131866-131932,132451-132575,133580-134011 |
| 401785 | 7249190 | Minus | 165176-165996,166189-166314, 166408-166569,167112-167268,167387-167469,168634-168942 |
| 401819 | 7467933 | Minus | 28217-28486 |
| 402408 | 9796239 | Minus | 110326-110491 |
| 402444 | 9796614 | Plus | 28391-28517 |
| 402791 | 6137008 | Minus | 51036-51207 |
| 403047 | 3540153 | Minus | 59793-59968 |
| 403137 | 9211494 | Minus | 92349-92572,92958-93084,93579-93712,93949-94072,94591-94748,95214-95337 |
| 403721 | 7528046 | Minus | 156647-157366 |
| 403764 | 7717105 | Minus | 118692-118853 |
| 403797 | 8099896 | Minus | 123065-125008 |
| 404165 | 9926489 | Minus | 69025-69128 |
| 404210 | 5006246 | Plus | 169926-170121 |
| 404253 | 9367202 | Minus | 55675-56055 |
| 404561 | 9795980 | Minus | 69039-70100 |
| 404571 | 7249169 | Minus | 112450-112648 |
| 404721 | 9856648 | Minus | 173763-174294 |
| 404915 | 7341766 | Minus | 100915-101087 |
| 404939 | 6862697 | Plus | 175318-175476 |
| 405403 | 6850244 | Minus | 37491-37670,40951-41031 |
| 405685 | 4508129 | Minus | 37956-38097 |
| 405718 | 9795467 | Plus | 113080-113266 |
| 405793 | 1405887 | Minus | 89197-89453 |
| 405876 | 6758747 | Plus | 39694-40031 |
| 405917 | 7712162 | Minus | 106829-107213 |
| 406414 | 9256407 | Plus | 49593-49850 |
| 406554 | 7711566 | Plus | 106956-107121 |

**TABLE 6:286 GENES ENCODING EXTRACELLULAR OR CELL SURFACE PROTEINS UP-REGULATED IN PROSTATE CANCER COMPARED TO NORMAL ADULT TISSUES**

| Table 6 shows 286 genes up-regulated in prostate cancer compared to normal adult tissues that are likely to be extracellular or cell-surface proteins. These were selected as for Table 5 and the predicted protein contained a structural domain that is indicative of extracellular localization (e.g. egf, 7tm domains). | | | | |
|---|---|---|---|---|
| Pkey: | | Unique Eos probeset Identifier number | | |
| ExAccn: | | Exemplar Accession number, Genbank accession number | | |
| UnigeneID: | | Unigene number | | |
| Unigene Title: | | Unigene gene title | | |
| R1: | | Ratio of tumor to normal tissue | | |
| | | | | |

| Pkey | ExAccn | UnigeneID | Uningene Title | R1 |
|---|---|---|---|---|
| | | | | |
| 409361 | NM_005982 | Hs.54416 | sine oculis homeobok (Drosophila) homolo | 48.28 |
| 409731 | AA125985 | Hs.56145 | thymosin, beta, identified in neuroblast | 45.24 |
| 400298 | AA032279 | Hs.61635 | six transmembrane epithelial antigen of | 43.48 |
| 420154 | AI093155 | Hs.95420 | JM27 protein | 41.12 |
| 426747 | AA535210 | Hs.171995 | kallikrein 3, (prostate specific antigen | 31.80 |
| 400299 | X07730 | Hs.171995 | kallikrein 3, (prostate specific antigen | 24.91 |
| 425075 | AA506324 | Hs.1852 | acid phosphatase, prostate | 24.23 |
| 424846 | AU077324 | Hs.1832 | neuropeptide Y | 23.57 |
| 405685 | | | | 20.90 |
| 420757 | X78592 | Hs.99915 | androgen receptor (dihydrotestosterone r | 19.72 |
| 418994 | AA296520 | Hs.89546 | selectin E (endothelial adhesion molecul | 19.56 |
| 452792 | AB037765 Hs.30652 | | KIAA1344 protein | 17.39 |
| 445472 | AB006631 | Hs.12784 | Homo sapiens mRNA for KIAA0293 gene, par | 17.00 |
| 414565 | AA502972 | Hs.183390 | hypothetical protein FLJ13590 | 16.82 |
| 431716 | D89053 | Hs.268012 | fatty-acid-Coenzyme A ligase, long-chain | 16.60 |
| 408430 | S79876 | Hs.44926 | dipeptidylpeptidase IV (CD26, adenosine | 16.28 |
| 408000 | L11690 | Hs.620 | bullous pemphigoid antigen 1 (230/240kD) | 15.54 |
| 430226 | BE245562 | Hs.2551 | adrenergic, beta-2-, receptor, surface | 15.40 |
| 444484 | AK002126 | Hs.11260 | hypothetical protein FLJ11264 | 14.76 |
| 418601 | AA279490 | Hs.86368 | calmegin | 14.56 |
| 448999 | AF179274 | Hs.22791 | transmembrane protein with EGF-like and | 14.55 |
| 416182 | NM_004354 | Hs.79069 | cyclin G2 | 12.94 |
| 420544 | AA677577 | Hs.98732 | Homo sapiens Chromosome 16 BAC clone CIT | 12.79 |
| 445413 | AA151342 | Hs.12677 | CGI-147 protein | 12.64 |
| 453930 | AA419466 | Hs.36727 | hypothetical protein FLJ10903 | 12.22 |
| 440286 | U29589 | Hs.7138 | cholinergic receptor, muscarinic 3 | 12.04 |
| 452784 | BE463857 | Hs.151258 | hypothetical protein FLJ21062 | 11.86 |
| 450203 | AF097994 | Hs.301528 | L-kynurenine/alpha-aminoadipate aminotra | 11.68 |
| 448045 | AJ297436 | Hs.20166 | prostate stem cell antigen | 11.51 |
| 449650 | AF055575 | Hs.23838 | calcium channel voltage-dependent. L ty | 11.18 |
| 420381 | D50640 | Hs.337616 | phosphodiesterase 3B, cGMP-inhibited | 11.10 |
| 425665 | AK001050 | Hs.159066 | hypothetical protein FLJ10188 | 11.08 |
| 425710 | AF030880 | Hs.159275 | solute carrier family, member 4 | 11.08 |
| 428728 | NM_016625 | Hs.191381 | hypothetical protein | 11.04 |
| 407021 | U52077 | | gb:Human marinert transposase gene, comp | 11.02 |
| 410733 | D84284 | Hs.66052 | CD38 antigen (p45) | 11.02 |
| 452340 | NM_002202 | Hs.505 | ISL1 transcription factor, LIM/homeodoma | 10.85 |
| 428819 | AL135623 | Hs.193914 | KIAA0575 gene product | 10.48 |
| 421991 | NM_014918 | Hs.110488 | KIAA0990 protein | 10.04 |
| 431217 | NM_013427 | Hs.250830 | Rho GTPase activating protein 6 | 9.75 |
| 421470 | R27496 | Hs.1378 | annexin A3 | 9.64 |
| 409262 | AK000631 | Hs.52256 | hypothetical protein FLJ20624 | 9.45 |
| 435980 | AF274571 | Hs.129142 | deoxyribonuclease II beta | 9.24 |
| 421246 | AW582962 | Hs.102897 | CGI-47 protein | 9.20 |
| 410001 | AB041036 | Hs.57771 | kallikrein 11 | 9.03 |
| 441791 | AW372449 | Hs.175982 | hypothetical protein FLJ21159 | 9.02 |
| 404571 | | | | 8.66 |
| 456497 | AW967956 | Hs.123648 | ESTs, Weakly similar to AF108460 1 ubinu | 8.56 |
| 419968 | X04430 | Hs.93913 | interleukin 6 (interferon, beta 2) | 8.36 |
| 433172 | AB037841 | Hs.102652 | hypothetical protein ASH1 | 8.30 |
| 422631 | BE218919 | Hs.118793 | hypothetical protein FLJ10688 | 8.27 |
| 427674 | NM_003528 | Hs.2178 | H28 histone family, member Q | 8.20 |
| 404915 | | | | 8.08 |
| 452259 | AA317439 | Hs.28707 | signal sequence receptor, gamma (translo | 8.06 |
| 452891 | N75582 | Hs.212875 | ESTs, Weakly similar to DYH9_HUMAN CILIA | 8.02 |
| 439731 | AI953135 | Hs.45140 | hypothetical protein FLJ14084 | 7.98 |
| 419839 | U24577 | Hs.93304 | phospholipase A2, group VII (platelet-ac | 7.68 |
| 420120 | AL049610 | Hs.95243 | transcription elongation factor A (SII)- | 7.64 |
| 424099 | AF071202 | Hs.139336 | ATP-binding cassette, sub-family C (CFTR | 7.64 |
| 448706 | AW291095 | Hs.21814 | interleukin 20 receptor, alpha | 7.52 |
| 410227 | AB009284 | Hs.61152 | exostoses (multiple)-like 2 | 7.49 |
| 425211 | M18667 | Hs.1867 | progastricsin (pepsinogen C) | 7.35 |
| 441736 | AW292779 | Hs.169799 | ESTs | 7.28 |
| 419991 | AJ000098 | Hs.94210 | eyes absent (Drosophila) homolog 1 | 7.20 |
| 425018 | BE245277 | Hs.154196 | E4F transcription factor 1 | 7.20 |
| 424560 | AA158727 | Hs.150555 | protein predicted by clone 23733 | 7.18 |
| 409110 | AA191493 | Hs.48778 | niban protein | 7.10 |
| 421566 | NM_000399 | Hs.1395 | early growth response 2 (Krox-20 (Drosop | 7.04 |
| 431725 | X65724 | Hs.2839 | Norrie disease (pseudoglioma) | 6.98 |
| 425782 | U66468 | Hs.159525 | cell growth regulatory with EF-hand doma | 6.85 |
| 427408 | AA583206 | Hs.2156 | RAR-related orphan receptor A | 6.79 |
| 435604 | AA625279 | Hs.26892 | uncharacterized bone marrow protein BM04 | 6.73 |
| 415874 | AF091622 | Hs.78893 | KIAA0244 protein | 6.54 |
| 401451 | | | | 6.52 |
| 431778 | AL080276 | Hs.268562 | regulator of G-protein signalling 17 | 6.51 |
| 409089 | NM_014781 | Hs.50421 | KIAA0203 gene product | 6.50 |
| 431992 | NM_002742 | Hs.2891 | protein kinase C, mu | 6.49 |
| 404253 | | | | 6.42 |
| 421552 | AF026692 | Hs.105700 | secreted frizzled-related protein 4 | 6.41 |
| 416806 | NM_000288 | Hs.79993 | peroxisomal biogenesis factor 7 | 6.38 |
| 431958 | X63629 | Hs.2877 | cadherin 3, type 1, P-cadherin (placenta | 6.30 |
| 439366 | AF100143 | Hs.6540 | fibroblast growth factor 13 | 6.30 |
| 416836 | D54745 | Hs.80247 | cholecystokinin | 6.30 |
| 433383 | AF034837 | Hs.192731 | double-stranded RNA specific adenosine d | 6.29 |
| 450728 | AW162923 | Hs.25363 | presenilin 2 (Alzheimer disease 4) | 6.25 |
| 413384 | NM_000401 | Hs.75334 | exostoses (multiple) 2 | 6.22 |
| 423349 | AF010258 | Hs.127428 | homeo box A9 | 6.20 |
| 424800 | AL035588 | Hs.153203 | MyoD family inhibitor | 6.18 |
| 425451 | AF242769 | Hs.157461 | mesenchymal stem cell protein DSC54 | 6.14 |
| 447359 | NM_012093 | Hs.18268 | adenylate kinase 5 | 6.00 |
| 410889 | X91662 | Hs.66744 | twist (Drosophila) homolog (acrocephalos | 5.97 |
| 408829 | NM_006042 | Hs.48384 | heparan sulfate (glucosamine) 3-O-sulfot | 5.94 |
| 453911 | AW503857 | Hs.4007 | Sarcolemmal-associated protein | 5.94 |
| 408875 | NM_015434 | Hs.48604 | DKFZP434B168 protein | 5.92 |
| 450480 | X82125 | Hs.25040 | zinc finger protein 239 | 5.90 |
| 451684 | AF216751 | Hs.26813 | CDA14 | 5.88 |
| 400301 | X03635 | Hs.1657 | estrogen receptor 1 | 5.78 |
| 415077 | L41607 | Hs.934 | glucosaminyl (N-acetyl) transferase 2, l | 5.74 |
| 418852 | BE537037 | Hs.273294 | hypothetical protein FLJ20069 | 5.72 |
| 446867 | AB007891 | Hs.16349 | KIAA0431 protein | 5.72 |
| 410232 | AW372451 | Hs.61184 | CGI-79 protein | 5.70 |
| 422762 | AL031320 | Hs.119976 | Human DNA sequence from clone RP1-20N2 o | 5.70 |
| 450616 | AL133067 | Hs.302689 | hypothetical protein | 5.70 |
| 408621 | AI970672 | Hs.46638 | chromosome 11 open reading frame 8 | 5.65 |
| 439671 | AW162840 | Hs.6641 | kinesin family member 5C | 5.64 |
| 410196 | AI936442 | Hs.59838 | hypothetical protein FLJ10808 | 5.60 |
| 429170 | NM_001394 | Hs.2359 | dual specificity phosphatase 4 | 5.60 |
| 440738 | AI004650 | Hs.225674 | WD repeat domain 9 | 5.60 |
| 414342 | AA742181 | Hs.75912 | KIAA0257 protein | 5.59 |
| 422634 | NM_016010 | Hs.118821 | CGI-62 protein | 5.56 |
| 400268 | | | | 5.55 |
| 439569 | AW602166 | Hs.222399 | CEGP1 protein | 5.51 |
| 452823 | AB012124 | Hs.30696 | transcription factor-like 5 (basic helix | 5.48 |
| 431938 | AA938471 | Hs.54431 | specific granule protein (28 kDa); cyste | 5.44 |
| 427638 | AA406411 | Hs.208341 | ESTs, Weakly similar to KIAA0989 protein | 5.42 |
| 421264 | AL039123 | Hs.103042 | microtubule-associated protein 1B | 5.38 |
| 421685 | AF189723 | Hs.106778 | ATPase, Ca++ transporting, type 2C, memb | 5.37 |
| 421987 | AI133161 | Hs.286131 | CGI-101 protein | 5.36 |
| 422806 | BE314767 | Hs.1581 | glutathione S-transferase theta 2 | 5.34 |
| 432281 | AK001239 | Hs.274263 | hypothetical protein FLJ10377 | 5.32 |
| 451982 | F13036 | Hs.27373 | Homo sapiens mRNA; cDNA DKFZp564O1763 (f | 5.32 |
| 444042 | NM_004915 | Hs.10237 | ATP-binding cassette, sub-family G (WHIT | 5.31 |
| 447752 | M73700 | Hs.105938 | lactotransferrin | 5.29 |
| 451418 | BE387790 | Hs.26369 | hypothetical protein FLJ20287 | 5.22 |
| 428593 | AW207440 | Hs.185973 | degenerative spermatocyte (homolog Droso | 5.21 |
| 447541 | AK000288 | Hs.18800 | hypothetical protein FLJ20281 | 5.18 |
| 459294 | AW977286 | Hs.17428 | RBP1-like protein | 5.16 |
| 424692 | AA429834 | Hs.151791 | KIAA0092 gene product | 5.15 |
| 416434 | AW163045 | Hs.79334 | nuclear factor, interleukin 3 regulated | 5.11 |
| 410268 | AA316181 | Hs.61635 | six transmembrane epithelial antigen of | 5.10 |
| 417517 | AF001176 | Hs.82238 | POP4 (processing of precursor, S. cerev | 5.10 |
| 453616 | NM_003462 | Hs.33846 | dynein, axonemal, light intermediate pol | 5.10 |
| 427958 | AA418000 | Hs.98280 | potassium intermediate/small conductance | 5.09 |
| 407945 | X69208 | Hs.606 | ATPase, Cu++ transporting, alpha polypep | 5.08 |
| 418576 | AW968159 | Hs.289104 | Alu-binding protein with zinc finger dom | 5.05 |
| 413328 | Y15723 | Hs.75295 | guanylate cyclase 1, soluble, alpha 3 | 5.04 |
| 432729 | AK000292 | Hs.278732 | hypothetical protein FLJ20285 | 5.04 |
| 426342 | AF093419 | Hs.169378 | multiple PDZ domain protein | 5.02 |
| 429782 | NM_005754 | Hs.220689 | Ras-GTPase-activating protein SH3-domain | 5.02 |
| 436209 | AW850417 | Hs.254020 | ESTs, Moderately similar to unnamed prot | 5.02 |
| 430599 | NM_004855 | Hs.247118 | phosphatidylinositol glycan, class B | 5.00 |
| 451386 | AB029006 | Hs.26334 | spastic paraplegia 4 (autosomal dominant | 5.00 |
| 457211 | AW972565 | Hs.32399 | ESTs, Weakly similar to S51797 vasodilat | 4.97 |
| 425851 | NM_001490 | Hs.159642 | glucosaminyl (N-acetyl) transferase 1, c | 4.97 |
| 421689 | N87820 | Hs.106826 | KIAA1696 protein | 4.93 |
| 416533 | BE244053 | Hs.79362 | retinoblastoma-like 2 (p130) | 4.92 |
| 432653 | N62096 | Hs.293185 | ESTs, Weakly similar to JC7328 amino aci | 4.91 |
| 403047 | | | | 4.91 |
| 431117 | AF003522 | Hs.250500 | delta (Drosophila)-like 1 | 4.90 |
| 427617 | D42063 | Hs.199179 | RAN binding protein 2 | 4.88 |
| 428804 | AK000713 | Hs.193736 | hypothetical protein FLJ20706 | 4.88 |
| 449071 | NM_005872 | Hs.22960 | breast carcinoma amplified sequence 2 | 4.86 |
| 407596 | R86913 | | gb:yq30f05.r1 Soares fetal liver spleen | 4.84 |
| 456516 | BE172704 | Hs.222746 | KIAA1610 protein | 4.84 |
| 458339 | AW976853 | Hs.172843 | ESTs | 4.83 |
| 422083 | NM_001141 | Hs.111256 | arachidonate 15-lipoxygenase, second typ | 4.82 |
| 449535 | W15267 | Hs.23672 | low density lipoprotein receptor-related | 4.82 |
| 422048 | NM_012445 | Hs.288126 | spondin 2, extracellular matrix protein | 4.82 |
| 424602 | AK002055 | Hs.151046 | hypothetical protein FLJ11193 | 4.78 |
| 410765 | AI694972 | Hs.66180 | nucleosome assembly protein 1-like 2 | 4.77 |
| 419879 | Z17805 | Hs.93564 | Homer, neuronal immediate early gene, 2 | 4.74 |
| 450649 | NM_001429 | Hs.25272 | E1A binding protein p300 | 4.74 |
| 411624 | BE145964 | Hs.103283 | KIAA0594 protein | 4.72 |
| 404721 | | | | 4.70 |
| 426261 | AW242243 | Hs.168670 | peroxisomal famesylated protein | 4.70 |
| 416276 | U41060 | Hs.79136 | LIV-1 protein, estrogen regulated | 4.64 |
| 408374 | AW025430 | Hs.155591 | forkhead box F1 | 4.64 |
| 451900 | AB023199 | Hs.27207 | KIAA0982 protein | 4.63 |
| 421437 | AW821252 | Hs.104336 | hypothetical protein | 4.63 |
| 434629 | AA789081 | Hs.4029 | glioma-amplified sequence-41 | 4.60 |
| 403764 | | | | 4.58 |
| 421247 | BE391727 | Hs.102910 | general transcription factor IIH, polype | 4.53 |
| 403721 | | | | 4.50 |
| 453070 | AK001465 | Hs.31575 | SEC63, endoplasmic reticulum translocon | 4.49 |
| 417412 | X16896 | Hs.82112 | interleukin 1 receptor, type I | 4.48 |
| 439735 | AI635386 | Hs.142846 | hypothetical protein | 4.48 |
| 430261 | AA305127 | Hs.237225 | hypothetical protein HT023 | 4.46 |
| 430598 | AK001764 | Hs.247112 | hypothetical protein FLJ10902 | 4.44 |
| 400303 | AA242758 | Hs.79136 | UV-1 protein, estrogen regulated | 4.42 |
| 438209 | AL120659 | Hs.6111 | aryl-hydrocarbon receptor nuclear transl | 4.42 |
| 417421 | AL138201 | Hs.82120 | nuclear receptor subfamily 4, group A, m | 4.40 |
| 447270 | AC002551 | Hs.331 | general transcription factor IIIC, polyp | 4.38 |
| 434423 | NM_006769 | Hs.3844 | LIM domain only 4 | 4.35 |
| 404561 | | | | 4.32 |
| 422969 | AA782536 | Hs.122647 | N-myristoyltransferase 2 | 4.32 |
| 423685 | BE350494 | Hs.49753 | uveal autoantigen with coiled coil domai | 4.32 |
| 425071 | NM_013989 | Hs.154424 | deiodinase, iodothyronine, type II | 4.32 |
| 431583 | AL042613 | Hs.262476 | S-adenosylmethionine decarboxylase 1 | 4.31 |
| 442818 | AK001741 | Hs.8739 | hypothetical protein FLJ10879 | 4.30 |
| 423740 | Y07701 | Hs.293007 | aminopeptidase puromycin sensitive | 4.24 |
| 424701 | NM_005923 | Hs.151988 | mitogen-activated protein kinase kinase | 4.21 |
| 424085 | NM_002914 | Hs.139226 | replication factor C (activator 1) 2 (40 | 4.20 |
| 410294 | AB014515 | Hs.323712 | KIAA0615 gene product | 4.18 |
| 447124 | AW976438 | Hs.17428 | RBP1-like protein | 4.18 |
| 438018 | AK001160 | Hs.5999 | hypothetical protein FLJ10298 | 4.16 |
| 443857 | AI089292 | Hs.287621 | hypothetical protein FLJ14069 | 4.15 |
| 446711 | AF169692 | Hs.12450 | protocadherin 9 | 4.15 |
| 405403 | | | | 4.14 |
| 448148 | NM_016578 | Hs.20509 | HBV pX associated protein-8 | 4.13 |
| 417531 | NM_003157 | Hs.1087 | serine/threonine kinase 2 | 4.12 |
| 433345 | AI681545 | Hs.152982 | hypothetical protein FLJ13117 | 4.10 |
| 432712 | AB016247 | Hs.288031 | sterol-C5-desaturase (fungal ERG3, delta | 4.09 |
| 435114 | AA775483 | Hs.288936 | mitochondrial ribosomal protein L9 | 4.08 |
| 445459 | AI478629 | Hs.158465 | likely ortholog of mouse putative IKK re | 4.08 |
| 402791 | | | | 4.04 |
| 438660 | U95740 | Hs.6349 | Homo sapiens, clone IMAGE:3010666, mRNA, | 4.04 |
| 447568 | AF155655 | Hs.18885 | CGI-116 protein | 4.04 |
| 452211 | AI985513 | Hs.233420 | ESTs | 4.02 |
| 443292 | AK000213 | Hs.9196 | hypothetical protein | 4.01 |
| 420911 | U77413 | Hs.100293 | O-linked N-acetylglucosamine (GlcNAc) tr | 4.00 |
| 428738 | NM_000380 | Hs.192803 | xeroderma pigmentosum, complementation g | 3.95 |
| 430456 | AA314998 | Hs.241503 | hypothetical protein | 3.95 |
| 437531 | AI400752 | Hs.112259 | T cell receptor gamma locus | 3.93 |
| 428695 | AI355647 | Hs.189999 | purinergic receptor (family A group 5) | 3.91 |
| 410011 | AB020641 | Hs.57856 | PFTAIRE protein kinase 1 | 3.91 |
| 446494 | AA463276 | Hs.288906 | WW Domain-Containing Gene | 3.91 |
| 409928 | AL137163 | Hs.57549 | hypothetical protein dJ473B4 | 3.90 |
| 411598 | BE336654 | Hs.70937 | H3 histone family, member A | 3.90 |
| 425707 | AF115402 | Hs.11713 | E74-like factor 5 (ets domain transcript | 3.90 |
| 451806 | NM_003729 | Hs.27076 | RNA 3'-terminal phosphate cyclase | 3.89 |
| 401045 | | | | 3.89 |
| 437372 | AA323968 | Hs.283631 | hypothetical protein DKFZp547G183 | 3.89 |
| 417067 | AJ001417 | Hs.81086 | solute carrier family 22 (extraneuronal | 3.88 |
| 410467 | AF102546 | Hs.63931 | dachshund (Drosophila) homolog | 3.88 |
| 431930 | AB035301 | Hs.272211 | cadherin 7, type 2 | 3.88 |
| 453047 | AW023798 | Hs.286025 | ESTs | 3.88 |
| 401785 | | | | 3.88 |
| 458229 | AI929602 | Hs.177 | phosphatidylinositol glycan, class H | 3.86 |
| 406414 | | | | 3.86 |
| 412494 | AL133900 | Hs.792 | ADP-ribosylation factor domain protein 1 | 3.84 |
| 418329 | AW247430 | Hs.84152 | cystathionine-beta-synthase | 3.83 |
| 424850 | AA151057 | Hs.153498 | chromosome 18 open reading frame 1 | 3.82 |
| 427585 | D31152 | Hs.179729 | collagen, type X, alpha 1 (Schmid metaph | 3.82 |
| 423052 | M28214 | Hs.123072 | RAB3B, member RAS oncogene family | 3.82 |
| 416111 | AA033813 | Hs.79018 | chromatin assembly factor 1, subunit A ( | 3.82 |
| 419423 | D26488 | Hs.90315 | KIAA0007 protein | 3.80 |
| 429643 | AA455889 | Hs.167279 | FYVE-finger-containing Rab5 effector pro | 3.80 |
| 431499 | NM_001514 | Hs.258561 | general transcription factor IIB | 3.80 |
| 444078 | BE246919 | Hs.10290 | U5 snRNP-specific 40 kDa protein (hPrp8- | 3.78 |
| 430291 | AV660345 | Hs.238126 | CGI-49 protein | 3.76 |
| 431637 | AI879330 | Hs.265960 | hypothetical protein FLJ10563 | 3.74 |
| 440411 | N30256 | Hs.151093 | hypothetical protein DKFZp434G1415 | 3.74 |
| 405917 | | | | 3.74 |
| 451230 | BE546208 | Hs.26090 | hypothetical protein FLJ20272 | 3.73 |
| 429597 | NM_003816 | Hs.2442 | a disintegrin and metalloproteinase doma | 3.73 |
| 415075 | L27479 | Hs.77889 | Friedreich ataxia region gene X123 | 3.72 |
| 440351 | AF030933 | Hs.7179 | RAD1 (S.pombe) homolog | 3.70 |
| 443603 | BE502601 | Hs.134289 | ESTs, Weakly similar to KIAA1063 protein | 3.70 |
| 446965 | BE242873 | Hs.16677 | WD repeat domain 15 | 3.70 |
| 412350 | AI659306 | Hs.73826 | protein tyrosine phosphatase, non-recept | 3.70 |
| 433852 | AI378329 | Hs.126629 | ESTs | 3.70 |
| 447397 | BE247676 | Hs.18442 | E-1 enzyme | 3.68 |
| 405718 | | | | 3.68 |
| 425217 | AU076696 | Hs.155174 | CDC5 (cell division cycle 5, S. pombe, h | 3.68 |
| 421734 | AI318624 | Hs.107444 | Homo sapiens cDNA FLJ20562 fis, clone KA | 3.67 |
| 427221 | L15409 | Hs.174007 | von Hippel-Lindau syndrome | 3.67 |
| 402408 | | | | 3.66 |
| 452946 | X95425 | Hs.31092 | EphA5 | 3.66 |
| 419078 | M93119 | Hs.89584 | insulinoma-associated 1 | 3.66 |
| 427144 | X95097 | Hs.2126 | vasoactive intestinal peptide receptor 2 | 3.65 |
| 423396 | AI382555 | Hs.127950 | bromodomain-containing 1 | 3.65 |
| 446320 | AF126245 | Hs.14791 | acyl-Coenzyme A dehydrogenase family, me | 3.63 |
| 404939 | | | | 3.62 |
| 403137 | | | | 3.60 |
| 437162 | AW005505 | Hs.5464 | thyroid hormone receptor coactivating pr | 3.60 |
| 404210 | | | | 3.59 |
| 443775 | AF291664 | Hs.204732 | matrix metalloproteinase 26 | 3.56 |
| 452501 | AB037791 | Hs.29716 | hypothetical protein FLJ10980 | 3.56 |
| 422443 | NM_014707 | Hs.116753 | histone deacetylase 7B | 3.55 |
| 420230 | AL034344 | Hs.284186 | forkhead box C1 | 3.55 |
| 418428 | Y12490 | Hs.85092 | thyroid hormone receptor interactor 11 | 3.54 |
| 433002 | AF048730 | Hs.279906 | cyclin T1 | 3.53 |
| 405793 | | | | 3.52 |
| 457940 | AL360159 | Hs.306517 | Homo sapiens TRIpartite motif protein ps | 3.52 |
| 402444 | | | | 3.52 |
| 418250 | U29926 | Hs.83918 | adenosine monophosphate deaminase (isofo | 3.51 |
| 414222 | AL135173 | Hs.878 | sorbitol dehydrogenase | 3.51 |
| 422384 | AA224077 | Hs.42438 | Sm protein F | 3.50 |
| 447805 | AW627932 | Hs.19614 | gemin4 | 3.50 |
| 454265 | H03556 | Hs.300949 | ESTs, Weakly simitar to thyroid hormone | 3.50 |
| 423445 | NK_014324 | Hs.128749 | alpha-methylacyl-CoA racemase | 3.48 |
| 413435 | X51405 | Hs.75360 | carboxypeptidase E | 3.46 |
| 447210 | AF035269 | Hs.17752 | phosphatidylserine-specific phospholipas | 3.46 |
| 426931 | NM_003416 | Hs.2076 | zinc finger protein 7 (KOX 4, clone HF.1 | 3.45 |
| 408418 | AW963897 | Hs.44743 | KIAA1435 protein | 3.45 |
| 421887 | AW161450 | Hs.109201 | CGI-86 protein | 3.44 |

**Table 7: 42 GENES ENCODING SMALL MOLECULE TARGETS UP-REGULATED IN PROSTATE CANCER COMPARED TO NORMAL ADULT TISSUES**

| Table 7 shows 42 genes up-regulated in prostate cancer compared to normal adult tissues that are likely to be small molecule targets. These were selected as for Table 5 and the predicted protein contained a structural domain that is indicative of a drugable structure (e.g. protease, kinase, phosphatase, receptor). The functional domain is indicated for each gene. | | | | | |
|---|---|---|---|---|---|
| Pkey: | | Unique Eos probeset identifier number | | | |
| ExAccn: | | Exemplar Accession number, Genbank accession number | | | |
| UnigeneID: | | Unigene number | | | |
| Unigene Title: | | Unigene gene title | | | |
| PSDomain: | | Protein Structural Domain | | | |
| R1: | | Ratio of tumor vs. normal tissue | | | |
| | | | | | |

| **Pkey** | **ExAccn** | **UnigeneID** | **Unigene Title** | **PSDomaln** | **R1** |
|---|---|---|---|---|---|
| | | | | | |
| 426747 | AA535210 | Hs.171995 | kallikrein 3, (prostate specific antigen | trypsin | 31.80 |
| 400299 | X07730 | Hs.171995 | kallikrein 3, (prostate specific antigen | trypsin | 24.91 |
| 420757 | X78592 | Hs.99915 | androgen receptor (dihydrotestosterone r | Androgen_recep, hormone_rec,zf-C4 | 19.72 |
| 408430 | S79876 | Hs.44926 | dipeptidylpeptidase IV (CD26, adenosine | DPPIV_N_term, Peptidase_S9 | 16.28 |
| 430226 | BE245562 | Hs.2551 | adrenergic, beta-2-, receptor, surface | 7tm_1 | 15.40 |
| 411096 | U80034 | Hs.68583 | mitochondrial intermediate peptidase | Peptidase_M3 | 14.81 |
| 440286 | U29589 | Hs.7138 | cholinergic receptor, muscarinic 3 | 7tm_1 | 12.04 |
| 420381 | D50640 | Hs.337616 | phosphodiesterase 3B, cGMP-inhibited | PDEase | 11.10 |
| 407021 | U52077 | | gb:Human mariner1 transposase gene, comp | SET, Transposase_1 | 11.02 |
| 401424 | | | | arginase | 9.58 |
| 410001 | AB041036 | Hs.57771 | kallikrein 11 | trypsin | 9.03 |
| 428330 | L22524 | Hs.2256 | matrix metalloproteinase 7 (matrilysin, | Peptidase_M10 | 8.76 |
| 424099 | AF071202 | Hs.139336 | ATP-binding cassette, sub-family C (CFTR | ABC_tran,ABC_membrane | 7.64 |
| 419991 | AJ000098 | Hs.94210 | eyes absent (Drosophila) homolog 1 | Hydrolase | 7.20 |
| 431992 | NM_002742 | Hs.2891 | protein kinase C, mu | pkinase,DAG_PE-bind,PH | 6.49 |
| 447359 | NM_012093 | Hs.18268 | adenylate kinase 5 | adenylatekinase | 6.00 |
| 400301 | X03635 | Hs.1657 | estrogen receptor 1 | Oest_recep,zf-C4,hormone_rec | 5.78 |
| 421685 | AF189723 | Hs.106778 | ATPase, Ca++ transporting, type 2C, memb | E1-E2_ATPase,Hydrolase | 5.37 |
| 444042 | NM_004915 | Hs.10237 | ATP-binding cassette, sub-family G (WHIT | ABC_tran | 5.31 |
| 447752 | M73700 | Hs.105938 | lactotransferrin | transferrin,7tm_1 | 5.29 |
| 407945 | X69208 | Hs.606 | ATPase, Cu++ transporting, alpha polypep | E1-E2_ATPase,Hydrolase, HMA | 5.08 |
| 403047 | | | | trypsin | 4.91 |
| 427617 | D42063 | Hs.199179 | RAN binding protein 2 | Ran_BP1,zf-RanBP,TPR,pro_isomerase | 4.88 |
| 422083 | NM_001141 | Hs.111256 | arachidonate 15-lipoxygenase, second typ | lipoxygenase,PLAT | 4.82 |
| 449535 | W15267 | Hs.23672 | low density lipoprotein receptor-related | ldl_recept_b,ldl_recept_a,EGF | 4.82 |
| 425071 | NM_013989 | Hs.154424 | deiodinase, iodothyronine, type II | T4_deiodinase | 4.32 |
| 423740 | Y07701 | Hs.293007 | aminopeptidase puromycin sensitive | Peptidase_M1 | 4.24 |
| 424701 | NM_005923 | Hs.151988 | mitogen-activated protein kinase kinase | pkinase | 4.21 |
| 424085 | NM_002914 | Hs.139226 | replication factor C (activator 1) 2 (40 | AAA,Viral_helicase1 | 4.20 |
| 417531 | NM_003157 | Hs.1087 | serine/threonine kinase 2 | pkinase | 4.12 |
| 428695 | AI355647 | Hs.189999 | purinergic receptor (family A group 5) | 7tm_1 | 3.91 |
| 410011 | AB020641 | Hs.57856 | PFTAIRE protein kinase 1 | pkinase | 3.91 |
| 424850 | AA151057 | Hs.153498 | chromosome 18 open reading frame 1 | ldl_recept_a | 3.82 |
| 412350 | AI659306 | Hs.73826 | protein tyrosine phosphatase, non-recept | Y_phosphatase,8and_41,PDZ | 3.70 |
| 447397 | BE247676 | Hs.18442 | E-1 enzyme | Hydrolase | 3.68 |
| 452946 | X95425 | Hs.31092 | EphA5 | EPH_lbd,fn3,pkinase,SAM | 3.66 |
| 427144 | X95097 | Hs.2126 | vasoactive intestinal peptide receptor 2 | 7tm_2 | 3.65 |
| 443775 | AF291664 | Hs.204732 | matrix metalloproteinase 26 | Peptidase_M10 | 3.56 |
| 457940 | AL360159 | Hs.306517 | Homo sapiens TRIpartite motif protein ps | SPRY,7tm_1 | 3.52 |
| 418250 | U29926 | Hs.83918 | adenosine monophosphate deaminase (isofo | A_deaminase | 3.51 |
| 413435 | X51405 | Hs.75360 | carboxypeptidase E | Zn_carbOpept | 3.46 |
| 447210 | AF035269 | Hs.17752 | phosphatidylserine-specific phospholipas | lipase | 3.46 |

**TABLE 8: 136 GENES SIGNIFICANTLY DOWN-REGULATED IN PROSTATE CANCER COMPARED TO NORMAL PROSTATE**

| Table 8 shows 136 genes significantly down-regulated in prostate cancer compared to normal prostate. These were selected from 59680 probesets on the Affymetrix/Eos Hu03 GeneChip array such that the ratio of "average" normal prostate to "average" prostate cancer tissues was greater than or equal to 2. The "average" normal prostate level was set to the mean amongst 4 normal prostate tissues. The "average" prostate cancer level was set to the 85^{th} percentile amongst 73 tumor samples. In order to remove gene-specific background levels of non-specific hybridization, the 10^{th} percentile value amongst all the tissues was subtracted from both the numerator and the denominator before the ratio was evaluated. | | | | |
|---|---|---|---|---|
| Pkey: | | Unique Eos probeset identifier number | | |
| ExAccn: | | Exemplar Accession number, Genbank accession number | | |
| UnigeneID: | | Unigene number | | |
| Unigene Title: | | Unigene gene title | | |
| R1: | | Ratio of normal prostate to prostate cancer | | |
| | | | | |

| **Pkey** | **ExAccn** | **UnigeneID** | **Unigene Title** | **R1** |
|---|---|---|---|---|
| | | | | |
| 425932 | M81650 | Hs.1968 | semenogelin l | 57.69 |
| 425545 | N98529 | Hs.158295 | Human mRNA for myosin light chain 3 (MLC | 19.70 |
| 426752 | X69490 | Hs.172004 | titin | 15.25 |
| 442082 | R41823 | Hs.7413 | ESTs; calsyntenin-2 | 10.05 |
| 407245 | X90568 | Hs.172004 | titin | 9.38 |
| 422711 | D60641 | Hs.21739 | Homo sapiens mRNA;cDNA DKFZp586l1518 (f | 9.05 |
| 420813 | X51501 | Hs.99949 | prolactin-induced protein | 8.18 |
| 411987 | AA375975 | Hs.183380 | "ESTs, Moderately similar to ALU7_HUMAN | 7.45 |
| 404567 | | | | 5.62 |
| 416030 | H15261 | Hs.21948 | ESTs | 5.51 |
| 444892 | AI620617 | Hs.148565 | ESTs | 5.27 |
| 444573 | AW043590 | Hs.225023 | ESTs | 5.20 |
| 428068 | AW016437 | Hs.233462 | ESTs | 5.08 |
| 437440 | AA846804 | Hs.123694 | ESTs | 4.95 |
| 404113 | | | | 4.75 |
| 452279 | AA286844 | Hs.61260 | hypothetical protein FLJ13164 | 4.75 |
| 421058 | AW297967 | Hs.188181 | ESTs | 4.63 |
| 445592 | AV654382 | Hs.17947 | "ESTs, Weakly similar to K02F3.10 [C.ele | 4.53 |
| 405163 | | | | 4.49 |
| 405227 | | | | 4.45 |
| 454059 | NM_003154 | Hs.37048 | statherin | 4.45 |
| 450152 | AI138635 | Hs.22968 | ESTs | 4.40 |
| 407013 | U35637 | | "gb:Human nebulin mRNA, partial cds" | 4.03 |
| 403612 | | | | 4.02 |
| 440089 | AA864468 | Hs.135646 | ESTs | 4.00 |
| 408988 | AL119844 | Hs.49476 | Homo sapiens clone TUA8 Cri-du-chat regi | 3.98 |
| 436726 | AA324975 | Hs.128993 | "ESTs, Weakly similar to KIAA0465 protei | 3.95 |
| 459367 | BE148877 | | "gb:CM4-HT0244-111199-040-h12 HT0244 Hom | 3.95 |
| 427318 | AF186081 | Hs.175783 | zinc transporter | 3.92 |
| 411762 | AW860972 | | "gb:QV0-CT0387-180300-167-h07 CT0387 Hom | 3.85 |
| 418668 | AW407987 | Hs.87150 | Human clone A9A2BR11 (CAC)n/(GTG)n repea | 3.75 |
| 458311 | AF069478 | | "gb:AF069478 Homo sapiens astrocytoma li | 3.61 |
| 403649 | | | | 3.60 |
| 419682 | H13139 | Hs.92282 | paired-like homeodomain transcription fa | 3.58 |
| 412519 | AA196241 | Hs.73980 | "troponin T1, skeletal, slow" | 3.51 |
| 414206 | AW276887 | Hs.46609 | ESTs | 3.45 |
| 427419 | NM_000200 | Hs.177888 | histatin 3 | 3.37 |
| 420777 | AA280223 | Hs.130865 | ESTs | 3.35 |
| 428134 | AA421773 | Hs.161008 | ESTs | 3.31 |
| 450218 | R02018 | Hs.168640 | "Ank, mouse, homolog of" | 3.30 |
| 433474 | AI192195 | Hs.147174 | "EST, Highly similar to ubiquitin-protei | 3.30 |
| 418833 | AW974899 | Hs.292776 | ESTs | 3.26 |
| 400440 | X83957 | Hs.83870 | nebulin | 3.16 |
| 413778 | AA090235 | Hs.75535 | "myosin, light polypeptide 2, regulatory | 3.06 |
| 423151 | AW838068 | | "gb:QV3-LT0048-10300-109-f02 LT0048 Hom | 3.05 |
| 445060 | AA830811 | Hs.88808 | ESTs | 2.98 |
| 457065 | AI476318 | Hs.192480 | ESTs | 2.95 |
| 432456 | H00093 | | "gb:ph8f12u_19/1TV Outward Alu-primed hn | 2.92 |
| 405678 | | | | 2.85 |
| 406707 | S73840 | Hs.931 | "myosin, heavy polypeptide 2, skeletal m | 2.81 |
| 444105 | AW189097 | Hs.166597 | ESTs | 2.78 |
| 433968 | AL157518 | Hs.90421 | PRO2463 protein | 2.73 |
| 438522 | AA809431 | Hs.258886 | ESTs | 2.73 |
| 436562 | H71937 | Hs.169756 | "complement component 1, s subcomponent" | 2.68 |
| 412417 | AA102268 | Hs.42175 | ESTs | 2.67 |
| 455590 | BE072259 | | "gb:QV4-BT0536-271299-059-g04 BT0536 Hom | 2.65 |
| 415380 | F07953 | Hs.16085 | putative G-protein coupled receptor | 2.65 |
| 428729 | AL162331 | Hs.191436 | hypothetical protein FLJ10619 | 2.64 |
| 408537 | AW207734 | | "gb:Ul-H-BI2-age-h-01-0-Ul.s1 NCI_CGAP_S | 2.63 |
| 424706 | AA741336 | Hs.152108 | transcriptional unit N143 | 2.63 |
| 413212 | BE072092 | | "gb:PM4-BT0532-160200-003-b11 BT0532 Hom | 2.63 |
| 406704 | M21665 | Hs.929 | "myosin, heavy polypeptide 7, cardiac mu | 2.62 |
| 437507 | AA758538 | Hs.246882 | ESTs | 2.60 |
| 410384 | AI933794 | Hs.42745 | ESTs | 2.58 |
| 408074 | R20723 | Hs.124764 | ESTs | 2.58 |
| 436653 | AA829828 | Hs.292402 | ESTs | 2.52 |
| 458090 | AI282149 | Hs.56213 | "ESTs, Highly similar to FXD3_HUMAN FORK | 2.51 |
| 432003 | AI689154 | Hs.122972 | ESTs | 2.50 |
| 436915 | AA737400 | Hs.142230 | ESTs | 2.50 |
| 410028 | AW576454 | Hs.258553 | ESTs | 2.46 |
| 448920 | AW408009 | Hs.22580 | alkylglycerone phosphate synthase | 2.45 |
| 422046 | AI638562 | | "gb:ts50a10.x1 NCI_CGAP_Ut1 Homo sapiens | 2.44 |
| 451122 | AA015767 | Hs.193587 | ESTs | 2.40 |
| 422646 | H87863 | Hs.151380 | ESTs | 2.36 |
| 451237 | AW600293 | | "gb:EST00049 pGEM-T library Homo sapiens | 2.36 |
| 400001 | | | AFFX control: BioB-3 | 2.36 |
| 415835 | Z45365 | | "gb:HSC2NF061 normalized infant brain cD | 2.36 |
| 439706 | AW872527 | Hs.59761 | ESTs | 2.36 |
| 423341 | AW242394 | Hs.252495 | ESTs | 2.36 |
| 436486 | AA742221 | Hs.120633 | ESTs | 2.35 |
| 407449 | AJ002784 | | gb:Homo sapiens mRNA; fetal brain cDNA 5 | 2.33 |
| 430573 | AA744550 | Hs.136345 | ESTs | 2.32 |
| 401974 | | | | 2.31 |
| 443356 | AL044498 | Hs.133262 | "ESTs, Weakly similar to PH0217 reverse | 2.31 |
| 430751 | NM_012471 | Hs.247868 | transient receptor potential channel 5 | 2.25 |
| 439128 | AI949371 | Hs.153089 | ESTs | 2.25 |
| 448765 | R15337 | Hs.21958 | "Homo sapiens cDNA FLJ10532 fis, clone N | 2.25 |
| 451130 | AI762250 | Hs.211347 | ESTs | 2.24 |
| 405420 | | | | 2.23 |
| 455029 | AW851258 | | "gb:IL3-CT0220-160200-066-H06 CT0220 Hom | 2.23 |
| 438224 | AA933999 | | "gb:on91f04.s1 Soares_NFL_T_GBC_S1 Homo | 2.23 |
| 407764 | BE008347 | | "gb:CM0-BN0154-080400-325-h04 BN0154 Hom | 2.23 |
| 413549 | BE252470 | | "gb:601108292F1 NIH_MGC_16 Homo sapiens | 2.23 |
| 437010 | AA741368 | Hs.291434 | ESTs | 2.23 |
| 435111 | AI914279 | Hs.213740 | ESTs | 2.22 |
| 403375 | | | | 2.21 |
| 455060 | AW853441 | | "gb:RC1-CT0252-030100-023-g09 CT0252 Hom | 2.21 |
| 409792 | AW854153 | | "gb:RC3-CT0254-060400-029-d03 CT0254 Hom | 2.20 |
| 421154 | AA284333 | Hs.287631 | "Homo sapiens cDNA FLJ14269 fis, clone P | 2.19 |
| 401963 | | | | 2.18 |
| 435034 | AF168711 | Hs.159397 | x 010 protein | 2.18 |
| 448996 | AW998989 | Hs.105749 | KIAA0553 protein | 2.18 |
| 436816 | AW297599 | Hs.255667 | ESTs | 2.17 |
| 442252 | AI733395 | Hs.129124 | ESTs | 2.17 |
| 419310 | AA236233 | Hs.188716 | ESTs | 2.16 |
| 418579 | H91800 | Hs.124156 | ESTs | 2.16 |
| 423315 | R54109 | Hs.26096 | ESTs | 2.16 |
| 432744 | AA988835 | Hs.38664 | ESTs | 2.15 |
| 424492 | AI133482 | Hs.165210 | ESTs | 2.15 |
| 424770 | AA425562 | | "gb:zw46e05.r1 Soares_total_fetus_Nb2HF8 | 2.15 |
| 437101 | AA744518 | Hs.120610 | ESTs | 2.15 |
| 428793 | AC004957 | Hs.298975 | "ESTs, Highly similar to collapsin-2-lik | 2.15 |
| 415708 | H56475 | | "gb.yt87d11.r1 Soares_pineal_gland_N3HPG | 2.13 |
| 459619 | | | | 2.12 |
| 427506 | AK000134 | Hs.179100 | hypothetical protein FLJ20127 | 2.12 |
| 452508 | AA804174 | Hs.184354 | ESTs | 2.10 |
| 410881 | AW809157 | | "gb:RC0-ST0118-041099-031-c07_1 ST0118 Homo sapiens cDNA, mRNA sequence" | 2.10 |
| 403087 | | | | 2.10 |
| 403869 | | | | 2.10 |
| 445028 | D81194 | Hs.282499 | ESTs | 2.10 |
| 447884 | H29505 | | "gb:ym60d10.r1 Soares infant brain 1NIB Homo sapiens cDNA clone 5', mRNA sequence" | 2.10 |
| 414575 | H11257 | Hs.295233 | ESTs | 2.09 |
| 420351 | BE218221 | Hs.190044 | ESTs | 2.08 |
| 426998 | BE274360 | | "gb:601121O68F1 NIH_MGC_20 Homo sapiens cDNA clone 5', mRNA sequence" | 2.08 |
| 405455 | | | | 2.08 |
| 423843 | AA332652 | | "gb:EST36627 Embryo, 8 week 1 Homo sapiens cDNA 5' end similar to similar to | |
| | | | monoamine oxidase B, mRNA sequence" | 2.08 |
| 406135 | | | | 2.07 |
| 427046 | BE246180 | Hs.121385 | ESTs | 2.07 |
| 403493 | | | | 2.05 |
| 444514 | AI682905 | Hs.270431 | "ESTs, Weakly similar to ALU1_HUMAN ALU SUBFAMILY J SEQUENCE | |
| | | | CONTAMINATION WARNING ENTRY [H.sapiens]" | 2.05 |
| 435884 | AA701443 | Hs.192868 | ESTs | 2.05 |
| 419629 | AB020695 | Hs.91662 | KIAA0888 protein | 2.03 |
| 405900 | | | | 2.03 |
| 457350 | AW974438 | Hs.194136 | "ESTs, Moderately similar to AF091457 1 zinc finger protein RIN ZF [R.norvegicus]" | 2.02 |
| 400007 | | | AFFX control: BioDn-5 | 2.01 |
| 406978 | M64358 | | "gb:Human rhom-3 gene, exon." | 2.00 |

**TABLE 8A shows the accession numbers for those primekeys lacking a unigeneID in Table 8. For each probeset we have listed the gene cluster number from which the oligonucleotides were designed. Gene clusters were compiled using sequences derived from Genbank ESTs and mRNAs. These sequences were clustered based on sequence similarity using Clustering and Alignment Tools (DoubleTwist, Oakland California). The Genbank accession numbers for sequences comprising each cluster are listed in the "Accession" column.**

| | | |
|---|---|---|
| Pkey: | | Unique Eos probeset identifier number |
| CAT number: | | Gene cluster number |
| Accession: | | Genbank accession numbers |
| | | |

| **Pkey** | **CAT number** | **Accessions** |
|---|---|---|
| | | |
| 407764 | 1014849_1 | BE008347 BE008320 BE083307 BE083311 AW075968 |
| 408537 | 1064753_1 | AW207734 D60164 D81150 D81078 D61356 AW996804 |
| 409792 | 1154677_1 | AW854153 AW500210 BE145772 AW501310 |
| 410881 | 1225682_1 | AW809157 AW812181 AW812175 AW812172 AW812161 AW812165 |
| 411762 | 1256906_1 | AW860972 AW862598 AW862599 AW860988 AW860983 AW860898 AW860925 AW860922 AW860986 AW860984 AW860989 |
| 413212 | 1353792_1 | BE072092 BE072106 BE072086 BE072098 BE072103 |
| 413549 | 1375933_2 | BE252470 BE147573 |
| 415708 | 1548209_1 | H56475 F29401 F34552 |
| 415835 | 1558511_1 | Z45365 R25905 H05203 T77496 |
| 422046 | 210744_1 | AI638562 T16929 H13401 F07773 R55836 |
| 423151 | 225415_1 | AW838068 AW837986 AW838067 AA322487 AW837936 |
| 423843 | 232510_1 | AA332652 AA331633 AW999369 AW902993 BE170475 AA378845 AW964175 AI475221 |
| 424770 | 243504_1 | AA425562 AI880208 AA346646 N22655 AW811775 AW811786 |
| 426998 | 274259_-1 | BE274360 |
| 432456 | 347718_2 | H00093 H00079 H00070 H00054 H00049 H00063 AW905306 AW905241 AW905410 AW905307 AW905411 AW905240 AW905210 |
| | | AW905352 AW905304 AW905239 AW905242 AW905243 H00087 |
| 438224 | 452656_1 | AA933999 AA781181 |
| 447884 | 740749_1 | H29505 R18575 Z43580 T48738 AI435454 BE004683 |
| 451237 | 863269_1 | AW600293 AI767468 |
| 455029 | 1249374_1 | AW851258 AW851435 AW851106 AW851421 |
| 455060 | 1251259_1 | AW853441 BE145228 BE14218 BE145162 BE145283 |
| 455590 | 1335127_1 | BE072259 BE072230 BE007911 |
| 458311 | 543550_1 | AF069478 AF069479 AF069480 |

**TABLE 8B shows the genomic positioning for those primekeys lacking unigene ID's and accession numbers in table 8. For each predicted exon, we have listed the genomic sequence source used for prediction. Nucleotide locations of each predicted exon are also listed.**

| | | | |
|---|---|---|---|
| Pkey: | | Unique number corresponding to an Eos probeset | |
| Ref: | | Sequence source. The 7 digit numbers in this column are Genbank Identifier (GI) numbers. "Dunham I. et al." refers to the publication entitled "The DNA sequence of human chromosome 22." Dunham I. et al., Nature (1999) 402:489-495. | |
| Strand: | | Indicates DNA strand from which exons were predicted. | |
| Nt_position: | | Indicates nucleotide positions of predicted exons. | |
| | | | |

| **Pkey** | **Ref** | **Strand** | **Nt_position** |
|---|---|---|---|
| | | | |
| 401963 | 3126783 | Plus | 51382-51521 |
| 401974 | 3126777 | Plus | 85330-85683 |
| 403087 | 8954241 | Plus | 169511-169795 |
| 403375 | 9255944 | Minus | 92554-92795 |
| 403493 | 7341425 | Plus | 157568-159084 |
| 403612 | 8469060 | Minus | 94723-94859 |
| 403649 | 8705159 | Minus | 27141-27247 |
| 403869 | 7280046 | Minus | 34379-34583 |
| 404113 | 9588571 | Minus | 13446-13646 |
| 404567 | 7249169 | Minus | 101320-101501 |
| 405163 | 9966267 | Minus | 161171-161299 |
| 405227 | 6731245 | Minus | 22550-22802 |
| 405420 | 7211837 | Minus | 13428-13582 |
| 405455 | 7656675 | Plus | 134112-134671 |
| 405678 | 4079670 | Plus | 151821-152027 |
| 405900 | 6758795 | Minus | 71181-71535 |
| 406135 | 9164918 | Minus | 65489-65715 |

**TABLE 9: 1001 GENES SIGNIFICANTLY UP-REGULATED IN NORMAL PROSTATE COMPATED TO PROSTATE CANCER**

| Table 9 shows 1001 genes significantly up-regulated in prostate cancer compared to normal prostate. These were selected from 59680 probesets on the Affymetrix/Eos Hu03 GeneChip array such that the ratio of "average" normal prostate to "average" prostate cancer tissues was greater than or equal to 8.14. The "average" normal prostate level was set to the mean amongst 4 normal prostate tissues. The "average" prostate cancer level was set to the 85^{th} percentile amongst 73 tumor samples. In order to remove gene-specific background levels of non-specific hybridization, the 10^{th} percentile value amongst all the tissues was subtracted from both the numerator and the denominator before the ratio was evaluated. | | | | |
|---|---|---|---|---|
| Pkey. | | Unique Eos probeset identifier number | | |
| ExAccn: | | Exemplar Accession number, Genbank accession number | | |
| UnigeneID: | | Unigene number | | |
| Unigene Title: | | Unigene gene title | | |
| R1: | | Ratio of prostate cancer to normal prostate | | |
| | | | | |

| **Pkey** | **ExAccn** | **UnigeneID** | **Unigene Title** | **R1** |
|---|---|---|---|---|
| | | | | |
| 451002 | AA013299 | Hs.8018 | ESTs, Weakly similar to ALU3_HUMAN ALU S | 1684.00 |
| 435596 | AA689465 | Hs.188999 | ESTs | 738.00 |
| 443576 | AI078027 | Hs.169338 | ESTs | 246.86 |
| 434247 | AA928116 | Hs.272065 | ESTs | 245.20 |
| 400452 | AK000185 | | gb:Homo sapiens cDNA FLJ20178 fis, clone | 222.00 |
| 405932 427906 | AA864330 | Hs.166520 | ESTs | 221.33 212.00 |
| 443685 | AI686550 | Hs.174481 | ESTs | 163.20 |
| 451554 | AI474866 | Hs.193237 | ESTs | 149.45 |
| 418323 | NM_002118 | Hs.1162 | major histocompatibility complex, class | 126.11 |
| 429480 | M36860 | Hs.9295 | elastin (supravalvular aortic stenosis, | 123.27 |
| 426025 | AW138330 | Hs.233778 | ESTs | 120.00 |
| 418917 | X02994 | Hs.1217 | adenosine deaminase | 106.75 |
| 404407 | | | | 105.71 |
| 442027 | AI652926 | Hs.128395 | ESTs | 100.53 |
| 433704 | AA608684 | Hs.121705 | ESTs, Moderately similar to ALUC_HUMAN1 | 94.00 |
| 453758 | U83527 | | gb:HSU83527 Human fetal brain (M.Lovett) | 89.18 |
| 415354 | F06495 | | gb:HSC1AB051 normalized infant brain cDN | 87.73 |
| 424239 | M67439 | Hs.143526 | dopamine receptor D5 | 86.82 |
| 444143 | AW747996 | Hs.160999 | ESTs | 86.43 |
| 401672 | | | | 77.26 |
| 430590 | AW383947 | Hs.246381 | CD68 antigen | 68.47 |
| 411972 | BE074959 | | gb:PM0-BT0582-310100-001-f08 BT0582 Homo | 68.00 |
| 448992 | AI766053 | Hs.188346 | ESTs | 61.26 |
| 408828 | BE540279 | | gb:601059857F1 NIH_MGC_10 Homo sapiens c | 57.71 |
| 409653 | AW451693 | Hs.220826 | ESTs | 56.40 |
| 402964 | | | | 54.67 |
| 422673 | N59027 | | gb:yv59d11.r1 Soares fetal liver spleen | 54.00 |
| 422568 | AA372275 | Hs.279800 | Homo sapiens cDNA FLJ11383 fis, clone HE | 54.00 |
| 438907 | R32704 | Hs.301298 | ESTs | 52.96 |
| 405172 | | | | 52.96 |
| 444897 | AW137088 | Hs.144857 | ESTs | 52.32 |
| 458019 | AW592931 | Hs.256298 | ESTs | 51.63 |
| 405275 | AB028989 | Hs.88500 | mitogen-activated protein kinase 8 inter | 50.98 |
| 457815 | AA703679 | Hs.106999 | ESTs, Weakly similar to SYT5_HUMAN SYNAP | 49.60 |
| 424385 | AA339666 | | gb:EST44776 Fetal brain I Homo sapiens c | 48.90 |
| 407172 | T54095 | | gb:ya92c05.s1 Stratagene placenta (93722 | 47.98 |
| 428202 | AA424163 | Hs.156895 | ESTs | 46.83 |
| 435672 | AI700148 | Hs.283626 | ESTs | 43.57 |
| 420283 | AA485224 | Hs.57734 | G proteln-coupled receptor kinase-intera | 43.00 |
| 417016 | AA837098 | Hs.269933 | ESTs | 42.70 |
| 438854 | AF074994 | Hs.24240 | ESTs | 42.67 |
| 406134 | | | | 42.43 |
| 457319 | AA480895 | Hs.201552 | ESTs, Weakly similar to T17288 hypotheti | 42.31 |
| 409314 | AA070266 | | gb:zm69d04.r1 Stratagene neuroepithelium | 42.25 |
| 401124 | | | | 41.61 |
| 429316 | AI371157 | Hs.178538 | ESTs | 40.00 |
| 420317 | AB006628 | Hs.96485 | KIAA0290 protein | 39.64 |
| 457586 | AW062439 | | gb:MR0-CT0060-120899-001-f08 CT0060 Homo | 39.60 |
| 417407 | AA923278 | Hs.290905 | ESTs, Weakly similar to protease [H.sapi | 38.73 |
| 430269 | BE221682 | Hs.178364 | ESTs | 38.06 |
| 439602 | W79114 | Hs.58558 | ESTs | 36.69 |
| 433686 | AA604799 | Hs.136528 | ESTs, Moderately similar to ALU1_HUMAN A | 36.29 |
| 417993 | AW963705 | Hs.295806 | ESTs, Weakly similar to ALU7_HUMAN ALU S | 36.18 |
| 428214 | AA936282 | Hs.120397 | ESTs | 36.10 |
| 416908 | AA333990 | Hs.80424 | coagulation factor XIII, A1 polypeptide | 36.08 |
| 426264 | BE314852 | Hs.168694 | hypothetical protein FLJ10257 | 36.00 |
| 415911 | H08796 | Hs.124952 | ESTs | 36.00 |
| 457502 | AA076049 | Hs.274415 | Homo sapiens cDNA FLJ10229 fis, clone HE | 35.23 |
| 421566 | NM_000399 | Hs.1395 | early growth response 2 (Krox-20 (Drosop | 35.20 |
| 401468 | | | | 34.89 |
| 458561 | AI220150 | Hs.211195 | ESTs | 34.60 |
| 433601 | BE350738 | Hs.123993 | ESTs, Weakly similar to T00366 hypotheti | 33.24 |
| 454977 | AW848032 | | gb:IL3-CT0214-231299-053-D11 CT0214 Homo | 32.96 |
| 402828 | | | | 32.93 |
| 414522 | AW518944 | Hs.76325 | Homo sapiens cDNA:FLJ23125 fis, clone L | 31.76 |
| 402842 | | | | 31.68 |
| 421245 | AA285363 | | gb:HTH280 HTCDL1 Homo sapiens cDNA 5'/3' | 31.59 |
| 401631 | F05183 | Hs.1799 | CD1D antigen, d polypeptide | 31.26 |
| 408057 | AW139565 | | gb:Ul-H-Bl1-aea-d-04-0-Ul.s1 NCI_CGAP_Su | 31.24 |
| 408069 | H81795 | | gb:ys68a10.r1 Soares retina N2b4HR Homo | 31.20 |
| 438694 | T87479 | Hs.291797 | ESTs | 31.09 |
| 449156 | AF103907 | Hs.171353 | prostate cancer antigen 3 | 29.78 |
| 428796 | AU076734 | Hs.193665 | solute carrier family 28 (sodium-coupled | 29.76 |
| 452549 | AI907039 | | gb:PM-BT134-020499-566 BT134 Homo sapien | 29.59 |
| 410129 | BE244074 | Hs.285531 | regulator of Fas-induced apoptosis | 29.53 |
| 414464 | AI870175 | Hs.13957 | ESTs | 29.47 |
| 412326 | R07566 | Hs.73817 | Small inducible cytokine A3 (homologous | 29.22 |
| 459081 | W07808 | | gb:zb03a12.r1 Soares_fetal_lung_NbHL19W | 29.20 |
| 448702 | AW102670 | Hs.122464 | ESTs | 29.13 |
| 451939 | U80456 | Hs.27311 | single-minded (Drosophila) homolog 2 | 28.74 |
| 443412 | W84893 | Hs.9305 | angiotensin receptor-like 1 | 28.61 |
| 457324 | AB028990 | Hs.243901 | KIAA1067 protein | 28.24 |
| 424247 | X14008 | Hs.234734 | lysozyme (renal amyloidosis) | 28.18 |
| 457140 | AI279960 | Hs.178140 | ESTs | 28.12 |
| 444151 | AW972917 | Hs.128749 | alpha-methylacyl-CoA racemase | 28.06 |
| 457669 | AW104257 | Hs.123426 | ESTs, Weakly similar to putative serine/ | 27.61 |
| 412429 | AV650262 | Hs.75765 | GRO2 oncogene | 27.36 |
| 405495 | | | | 27.33 |
| 406516 | | | | 27.25 |
| 407997 | AW135429 | Hs.243577 | ESTs | 26.96 |
| 442115 | AW452332 | Hs.257554 | ESTs | 26.36 |
| 409038 | T97490 | Hs.50002 | small inducible cytokine subfamily A (Cy | 26.34 |
| 402838 | | | | 26.32 |
| 449846 | AI979284 | Hs.200552 | ESTs | 26.21 |
| 417153 | X57010 | Hs.81343 | collagen, type II, alpha 1 (primary oste | 26.20 |
| 439792 | NM_014856 | Hs.6684 | KIAA0476 gene product | 25.91 |
| 450096 | AI682088 | Hs.223368 | ESTs | 25.60 |
| 424196 | AL133660 | Hs.142926 | Homo sapiens mRNA; cDNA DKFZp434M0927 (f | 25.57 |
| 414246 | BE391090 | Hs.280278 | EST | 25.57 |
| 420848 | NM_005188 | Hs.99980 | Cas-Br-M (murine) ecotropic retroviral t | 25.48 |
| 424778 | AA251048 | Hs.153042 | lymphocyte antigen 9 | 25.42 |
| 409126 | AA063426 | | gb:zf70c08.s1 Soares_pineal_gland_N3HPG | 25.25 |
| 443936 | AW083491 | Hs.31196 | ESTs | 25.22 |
| 419392 | W28573 | | gb:51f10 Human retina cDNA randomly prim | 25.01 |
| 411201 | T74588 | Hs.8509 | ESTs, Weakly similar to CO3_HUMAN COMPLE | 24.85 |
| 422940 | BE077458 | | gb:RC1-BT0606-090500-015-b04 BT0606 Homo | 24.76 |
| 437571 | AA760894 | Hs.153023 | ESTs | 24.74 |
| 433973 | AI014723 | Hs.131770 | ESTs | 24.57 |
| 422416 | BE019557 | Hs.11900 | Human DNA sequence from clone RP4-583P15 | 24.53 |
| 421552 | AF026692 | Hs.105700 | secreted frizzled-related protein 4 | 24.49 |
| 443668 | U25758 | Hs.134584 | ESTs | 24.49 |
| 424800 | AL035588 | Hs.153203 | MyoD family inhibitor | 24.10 |
| 453633 | AA357001 | Hs.34045 | hypothetical protein FLJ20764 | 24.04 |
| 430565 | AL 122081 | Hs.244343 | cadherin related 23 | 24.00 |
| 433694 | AI208611 | Hs.12066 | Homo sapiens cDNA FLJ11720 fis, clone HE | 23.89 |
| 451045 | AA215672 | | gb:zr96e09.s1 NCI_CGAP_GCB1 Homo sapiens | 23.83 |
| 408583 | AW449674 | Hs.47359 | ESTs | 23.73 |
| 444040 | AF204231 | Hs.182982 | golgin-67 | 23.62 |
| 414182 | AA136301 | | gb:zk93g04.s1 Soares_pregnant_uterus_NbH | 23.39 |
| 418678 | NM_001327 | Hs.167379 | cancer/testis antigen | 23.20 |
| 408380 | AF123050 | Hs.44532 | diubiquitin | 22.68 |
| 456076 | BE243877 | Hs.76941 | ATPase, Na+/K+ transporting, beta 3 poly | 22.65 |
| 418299 | AA279530 | Hs.83968 | integrin, beta 2 (antigen CD18 (p95), ly | 22.38 |
| 444917 | R68651 | Hs.144997 | ESTs | 22.26 |
| 444381 | BE387335 | Hs.283713 | ESTs | 22.08 |
| 415788 | AW628686 | Hs.78851 | KIAA0217 protein | 22.04 |
| 410896 | AW809637 | | gb:MR4-ST0124-261099-015-b07 ST0124 Homo | 22.00 |
| 412978 | AI431708 | Hs.820 | homeo box C6 | 21.95 |
| 458418 | AV653846 | Hs.126261 | Homo sapiens Chromosome 16 BAC clone CIT | 21.94 |
| 454791 | BE071874 | | gb:RC2-BT0522-120200-014-a06 BT0522 Homo | 21.84 |
| 408748 | J05500 | Hs.47431 | spectrin, beta, erythrocytic (includes s | 21.26 |
| 416011 | H14487 | | gb:ym18c10.r1 Soares infant brain 1NIB H | 21.24 |
| 440474 | AI207936 | Hs.7195 | gamma-aminobutyric acid (GABA) A recepto | 21.14 |
| 447047 | AI623698 | Hs.246306 | Homo sapiens cDNA:FLJ23529 fis, clone L | 21.11 |
| 426793 | X89887 | Hs.172350 | HIR (histone cell cycle regulation defec | 21.10 |
| 409841 | AW502139 | | gb:UI-HF-BR0p-ajr-e-05-0-Ul.r1 NIH_MGC_5 | 21.07 |
| 405685 | | | | 20.90 |
| 457359 | AI983207 | Hs.192481 | ESTs, Weakly similar to SYPH_HUMAN SYNAP | 20.84 |
| 423067 | AA321355 | Hs.285401 | ESTs | 20.74 |
| 422355 | AW403724 | Hs.140 | immunoglobulin heavy constant gamma 3 (G | 20.73 |
| 401201 | | | | 20.73 |
| 458278 | W28912 | Hs.129019 | ESTs | 20.68 |
| 439097 | H66948 | | gb:yr86d10.r1 Soares fetal liver spleen | 20.67 |
| 414875 | H42679 | Hs.77522 | major histocompatibility complex, class | 20.66 |
| 400926 | | | | 20.66 |
| 451355 | NM_004197 | Hs.444 | serine/threonine kinase 19 | 20.64 |
| 446982 | AW500221 | Hs.43616 | Homo sapiens mRNA for FLJ00029 protein, | 20.61 |
| 417105 | X60992 | Hs.81226 | CD6 antigen | 20.61 |
| 405777 | | | | 20.51 |
| 424123 | AW966158 | Hs.58582 | Homo sapiens cDNA FLJ12702 fis, clone NT | 20.20 |
| 425009 | X58288 | Hs.154151 | protein tyrosine phosphatase, receptor t | 20.10 |
| 443271 | BE568568 | Hs.195704 | ESTs | 19.98 |
| 421064 | AI245432 | Hs.101382 | tumor necrosis factor, alpha-induced pro | 19.98 |
| 418819 | AA228776 | Hs.191721 | ESTs | 19.94 |
| 457595 | AA584854 | | gb:no09h11.s1 NCI_CGAP_Phe1 Homo sapiens | 19.90 |
| 404426 | | | | 19.84 |
| 412571 | U43143 | Hs.74049 | fms-related tyrosine kinase 4 | 19.79 |
| 431457 | NM_012211 | Hs.256297 | integrin, alpha 11 | 19.62 |
| 414002 | NM_006732 | Hs.75678 | FBJ murine osteosarcoma viral oncogene h | 19.57 |
| 418994 | AA296520 | Hs.89546 | Selectin E (endothelial adhesion molecul | 19.56 |
| 437158 | AW090198 | Hs.4779 | KIAA1150 protein | 19.52 |
| 437866 | AA156781 | Hs.83992 | ESTs | 19.44 |
| 417421 | AL138201 | Hs.82120 | nuclear receptor subfamily 4, group A, m | 19.34 |
| 433057 | X15675 | Hs.296832 | Human pTR7 mRNA for repetitive sequence | 19.22 |
| 421730 | AW449808 | Hs.164036 | glucosamine (N-acetyl)-6-sulfatase (Sanf | 19.21 |
| 456557 | AA284477 | Hs.96618 | ESTs | 18.77 |
| 440806 | AI247422 | Hs.129966 | ESTs | 18.76 |
| 439845 | AL355743 | Hs.56663 | Homo sapiens EST from clone 41214, full | 18.65 |
| 416155 | AI807264 | Hs.205442 | ESTs, Weakly similar to AF117610 1 inner | 18.64 |
| 437820 | AA769062 | Hs.16029 | ESTs, Weakly similar to alternatively sp | 18.62 |
| 450923 | AW043951 | Hs.38449 | ESTs | 18.59 |
| 418329 | AW247430 | Hs.84152 | cystathionine-beta-synthase | 18.58 |
| 424537 | AI673027 | Hs.143271 | ESTs | 18.55 |
| 447742 | AF113925 | Hs.19405 | caspase recruitment domain 4 | 18.52 |
| 415251 | R42863 | Hs.7124 | ESTs | 18.47 |
| 440770 | AA912815 | Hs.222078 | ESTs | 18.40 |
| 407711 | AI085846 | Hs.25522 | ESTs | 18.32 |
| 427157 | U51166 | Hs.173824 | thymine-DNA glycosylase | 18.28 |
| 409847 | AW501751 | Hs.279733 | ESTs | 18.15 |
| 417240 | N57568 | Hs.176028 | EST | 18.13 |
| 435732 | AF229178 | Hs.123136 | leucine rich repeat and death domain con | 18.12 |
| 436896 | AW977385 | Hs.278615 | ESTs | 18.12 |
| 432485 | N90866 | Hs.276770 | CDW52 antigen (CAMPATH-1 antigen) | 17.90 |
| 429490 | AI971131 | Hs.293684 | ESTs, Weakly similar to alternatively sp | 17.82 |
| 429984 | AL050102 | Hs.227209 | DKFZP586F1019 protein | 17.82 |
| 449214 | AI889114 | Hs.195663 | ESTs | 17.75 |
| 433867 | AK000596 | Hs.3618 | hippocalcin-like 1 | 17.72 |
| 431735 | AW977724 | Hs.75968 | thymosin, beta 4, X chromosome | 17.71 |
| 401515 | | | | 17.67 |
| 444045 | AI097439 | Hs.135548 | ESTs | 17.58 |
| 442754 | AL045825 | Hs.210197 | ESTs | 17.55 |
| 426559 | AB001914 | Hs.170414 | paired basic amino acid cleaving system | 17.54 |
| 432415 | T16971 | Hs.289014 | ESTs | 17.50 |
| 427829 | AI188225 | Hs.127462 | ESTs | 17.50 |
| 432516 | R08003 | Hs.188013 | ESTs | 17.44 |
| 435259 | AA152106 | Hs.4859 | cyclin L ania-6a | 17.36 |
| 414989 | T81668 | | gb:yd29c04.r1 Soares fetal liver spleen | 17.31 |
| 444880 | AW118683 | Hs.154150 | ESTs | 17.30 |
| 417651 | R06874 | Hs.268628 | ESTs | 17.27 |
| 453457 | AL037103 | Hs.270599 | ESTs, Weakly similar to unnamed protein | 17.22 |
| 424246 | AW452533 | Hs.143604 | Kaiso | 17.22 |
| 419078 | M93119 | Hs.89584 | insulinoma-associated 1 | 17.18 |
| 417696 | BE241624 | Hs.82401 | CD69 antigen (p60, early T-cell activati | 17.14 |
| 431117 | AF003522 | Hs.250500 | delta (Drosophila)-like 1 | 17.14 |
| 455254 | AW877015 | | gb:QV2-PT0010-250300-096-f12 PT0010 Homo | 17.14 |
| 425782 | U66468 | Hs.159525 | cell growth regulatory with EF-hand doma | 17.12 |
| 426678 | H08170 | Hs.113755 | ESTs | 17.12 |
| 426403 | NM_000361 | Hs.2030 | thrombomodulin | 17.01 |
| 425905 | AB032959 | Hs.161700 | KIAA1133 protein | 17.00 |
| 438867 | AW451157 | Hs.181157 | ESTs | 16.98 |
| 420940 | AA830664 | Hs.143974 | ESTs | 16.94 |
| 459234 | AI940425 | | gb:CM0-CT0052-150799-024-c04 CT0052 Homo | 16.92 |
| 404756 | | | | 16.91 |
| 422247 | U18244 | Hs.113602 | solute carrier family 1 (high affinity a | 16.90 |
| 420568 | F09247 | Hs.167399 | protocadherin alpha 5 | 16.88 |
| 443559 | AI076765 | Hs.269899 | ESTs | 16.80 |
| 438703 | AI803373 | Hs.31599 | ESTs | 16.78 |
| 411424 | AW845985 | | gb:RC2-CT0163-200999-002-H08 CT0163Homo | 16.70 |
| 402895 | | | | 16.69 |
| 422538 | NM_006441 | Hs.118131 | 5,10-methenyltetrahydrofolate synthetase | 16.68 |
| 447108 | AW449602 | Hs.217953 | ESTs, Moderately similar to NK-TUMOR REC | 16.65 |
| 448520 | AB002367 | Hs.21355 | doublecortin and CaM kinase-like 1 | 16.54 |
| 438567 | AW451955 | Hs.153065 | ESTs | 16.52 |
| 407811 | AW190902 | Hs.40098 | cysteine knot superfamily 1, BMP antagon | 16.50 |
| 410721 | R23534 | Hs.2730 | heterogeneous nuclear ribonucleoprotein | 16.50 |
| 437133 | AB018319 | Hs.5460 | KIAA0776 protein | 16.40 |
| 408182 | AA047854 | | gb:zf49g04.r1 Soares retina N2b4HR Homo | 16.32 |
| 417315 | AI080042 | Hs.180450 | ribosomal protein S24 | 16.30 |
| 431840 | AA534908 | Hs.2860 | POU domain, class 5, transcription facto | 16.28 |
| 439882 | AA847856 | Hs.124565 | ESTs | 16.20 |
| 418277 | AW135221 | Hs.130812 | ESTs | 16.09 |
| 410688 | AW796342 | | gb:PM2-UM0027-230200-002-h02 UM0027 Homo | 16.04 |
| 420120 | AL049610 | Hs.95243 | transcription elongation factor A (SII)- | 16.04 |
| 429597 | NM_003816 | Hs.2442 | a disintegrin and metalloproteinase doma | 16.02 |
| 447033 | AI357412 | Hs.157601 | EST - not in UniGene | 16.02 |
| 421684 | BE281591 | Hs.106768 | hypothetical protein FLJ10511 | 15.94 |
| 408599 | AA055800 | Hs.222933 | ESTs | 15.93 |
| 446012 | AV656098 | Hs.172382 | hypothetical protein FLJ20001 | 15.86 |
| 409671 | AA076769 | | gb:7B02B10 Chromosome 7 Fetal Brain cDNA | 15.85 |
| 405934 | | | | 15.84 |
| 426108 | AA622037 | Hs.166468 | programmed cell death 5 | 15.84 |
| 416208 | AW291168 | Hs.41295 | ESTs | 15.48 |
| 410708 | AA534370 | Hs.154088 | Homo sapiens cDNA:FLJ22756 fis, clone K | 15.42 |
| 447342 | AI199268 | Hs.19322 | ESTs; Weakly similar to !!!! ALU SUBFAMI | 15.38 |
| 454563 | AW807530 | | gb:CM0-ST0081-130999-054-d02 ST0081 Homo | 15.37 |
| 411507 | AW850140 | | gb:IL3-CT0219-261099-023-D11 CT0219 Homo | 15.36 |
| 438170 | AI916685 | Hs.194601 | ESTs | 15.29 |
| 416292 | AA179233 | Hs.42390 | nasopharyngeal carcinoma susceptibility | 15.26 |
| 406638 | M13861 | | gb:Human T-cell receptor active beta-cha | 15.26 |
| 446686 | AW138043 | Hs.156307 | ESTs | 15.25 |
| 434485 | AI623511 | Hs.118567 | ESTs | 15.24 |
| 441188 | AW292830 | Hs.255609 | ESTs | 15.22 |
| 444172 | BE147740 | Hs.104558 | ESTs | 15.22 |
| 409521 | BE244854 | Hs.159578 | Homo sapiens mRNA for FLJ00020 protein, | 15.16 |
| 420748 | AA279956 | Hs.88672 | ESTs | 15.14 |
| 422583 | AA410506 | Hs.118578 | H.sapiens mRNA for ribosomal protein L18 | 15.14 |
| 424240 | AB023185 | Hs.143535 | calcium/calmodulin-dependent protein kin | 15.12 |
| 451118 | AI862096 | Hs.60640 | ESTs | 15.12 |
| 437495 | BE177778 | | gb:RC1-HT0598-310300-012-f07 HT0598 Homo | 15.12 |
| 445467 | AI239832 | Hs.15617 | ESTs, Weakly similar to ALU4_HUMAN ALU S | 15.06 |
| 418305 | AW006783 | Hs.6686 | ESTs | 15.03 |
| 402812 | | | | 15.02 |
| 436851 | AA732480 | Hs.293581 | ESTs | 15.00 |
| 400991 | | | | 15.00 |
| 415752 | BE314524 | Hs.78776 | Human putative transmembrane protein (nm | 14.96 |
| 429900 | AA460421 | Hs.30875 | ESTs | 14.90 |
| 403683 | | | | 14.84 |
| 430315 | NM_004293 | Hs.239147 | guanine deaminase | 14.80 |
| 451952 | AL120173 | Hs.301663 | ESTs | 14.72 |
| 424687 | J05070 | Hs.151738 | matrix metalloproteinase 9 (gelatinase B | 14.69 |
| 447229 | BE617135 | | gb:601441677F1 NIH_MGC_65 Homo sapiens c | 14.67 |
| 425818 | AB021225 | Hs.159581 | matrix metalloproteinase 17 (membrane-in | 14.65 |
| 448553 | AI638449 | Hs.173031 | ESTs | 14.63 |
| 431089 | BE041395 | Hs.283676 | ESTs, Weakly similar to unknown protein | 14.60 |
| 459145 | AI903354 | | gb:RC-BT029-100199-117 BT029 Homo sapien | 14.55 |
| 449650 | AF055575 | Hs.297647 | ESTs, Moderately similar to calcium chan | 14.54 |
| 400952 | | | | 14.46 |
| 445885 | AI734009 | Hs.127699 | EST cluster (not in UniGene) | 14.44 |
| 407938 | AA905097 | Hs.85050 | phospholamban | 14.42 |
| 431676 | AI685464 | Hs.292638 | ESTs | 14.40 |
| 437210 | AA311443 | Hs.293563 | Homo sapiens mRNA; cDNA DKFZp586E2317 (f | 14.36 |
| 451900 | AB023199 | Hs.27207 | KIAA0982 protein | 14.36 |
| 445800 | AA126419 | Hs.301632 | ESTs | 14.32 |
| 412368 | AW945992 | Hs.181125 | immunoglobulin lambda locus | 14.31 |
| 409055 | AW304028 | Hs.300578 | ESTs | 14.23 |
| 408763 | W57550 | Hs.301526 | Homo sapiens cDNA FLJ13181 fis, clone NT | 14.22 |
| 446734 | AL049278 | Hs.16074 | Homo sapiens mRNA; cDNA DKFZp564l153 (fr | 14.22 |
| 413551 | BE242639 | Hs.75425 | ubiquitin associated protein | 14.22 |
| 421913 | AI934365 | Hs.109439 | osteoglycin (osteoinductive factor, mime | 14.22 |
| 452712 | AW838616 | | gb:RC5-LT0054-140200-013-D01 LT0054 Homo | 14.22 |
| 451468 | AW503398 | Hs.210047 | ESTs | 14.16 |
| 406038 | Y14443 | Hs.88219 | zinc finger protein 200 | 14.14 |
| 424909 | S78187 | Hs.153752 | cell division cycle 25B | 14.07 |
| 434078 | AW880709 | Hs.283683 | EST | 14.07 |
| 415254 | AI815831 | Hs.184378 | ESTs | 14.05 |
| 418196 | AI745649 | Hs.26549 | ESTs, Weakly similar to T00066 hypotheti | 14.02 |
| 410020 | T86315 | Hs.728 | ribonuclease, RNase A family, 2 (liver, | 13.98 |
| 411352 | NM_002890 | Hs.758 | RAS p21 protein activator (GTPase activa | 13.98 |
| 429848 | AF145439 | Hs.225946 | chemokine (C-C motif) receptor 9 | 13.95 |
| 413729 | BE159999 | | gb:QV1-HT0412-270300-123-d10 HT0412 Homo | 13.90 |
| 400125 | | | | 13.88 |
| 420319 | AW406289 | Hs.96593 | hypothetical protein | 13.85 |
| 448272 | AI479094 | Hs.170786 | ESTs | 13.80 |
| 422695 | AA315158 | | gb:EST186956 HCC cell line (matastasis t | 13.80 |
| 424565 | AW102723 | Hs.75295 | guanylate cyclase 1, soluble, alpha 3 | 13.78 |
| 458048 | H30340 | Hs.173705 | Homo sapiens cDNA: FLJ22050 fis, clone H | 13.78 |
| 408894 | AI935400 | Hs.217286 | ESTs | 13.76 |
| 454093 | AW860158 | | gb:RC0-CT0379-290100-032-b04 CT0379 Homo | 13.75 |
| 410889 | X91662 | Hs.66744 | twist (Drosophila) homolog (acrocephalos | 13.74 |
| 457751 | AI908236 | | gb:IL-BT166-180399-010 BT166 Homo sapien | 13.72 |
| 455131 | AW857913 | | gb:RC0-CT0323-231199-031-b05 CT0323 Homo | 13.69 |
| 408364 | AW015238 | Hs.128453 | ESTs | 13.67 |
| 425907 | AA365752 | Hs.155965 | ESTs | 13.62 |
| 402359 | | | | 13.60 |
| 401044 | | | | 13.53 |
| 409877 | AW502498 | Hs.157150 | ESTs, Weakly similar to zinc finger prot | 13.53 |
| 423690 | AA329648 | Hs.23804 | ESTs | 13.49 |
| 430685 | AI690234 | Hs.191666 | ESTs, Weakly similar to reverse transcri | 13.47 |
| 414052 | AW578849 | Hs.283552 | ESTs, Weakly similar to unnamed protein | 13.46 |
| 447858 | AW080339 | Hs.211911 | ESTs | 13.44 |
| 435716 | AI573283 | Hs.38458 | ESTs | 13.44 |
| 439120 | H56389 | | gb:yt87c03.r1 Soares_pineal_gland_N3HPG | 13.43 |
| 402788 | | | | 13.40 |
| 451591 | AA886446 | Hs.146278 | ESTs | 13.40 |
| 405411 | | | | 13.38 |
| 426558 | AW188574 | Hs.24218 | ESTs | 13.34 |
| 453506 | AA132818 | Hs.110407 | ESTs, Weakly similar to coded for by C. | 13.33 |
| 416445 | AL043004 | Hs.300678 | Human serine/threonine kinase mRNA, part | 13.32 |
| 457084 | AI074149 | Hs.150905 | ESTs, Weakly similar to chondroitin 4-su | 13.32 |
| 403838 | | | | 13.32 |
| 427337 | Z46223 | Hs.176663 | Fc fragment of IgG, low affinity IIIb, r | 13.30 |
| 434318 | AW207552 | Hs.116328 | ESTs, Weakly similar to dJ134E15.1 [H.sa | 13.28 |
| 435193 | N41359 | Hs.218107 | ESTs | 13.28 |
| 414756 | AW451101 | Hs.159489 | ESTs, Moderately similar to hexokinase I | 13.27 |
| 420626 | AF043722 | Hs.99491 | RAS guanyl releasing protein 2 (calcium | 13.26 |
| 420052 | AA418850 | Hs.44410 | ESTs | 13.25 |
| 414020 | NM_002984 | Hs.75703 | small inducible cytokine A4 (homologous | 13.25 |
| 403851 | | | | 13.24 |
| 422647 | W07492 | Hs.157101 | ESTs | 13.21 |
| 433598 | AI762836 | Hs.271433 | ESTs, Moderately similar to ALU2_HUMAN A | 13.21 |
| 409065 | AB033113 | Hs.50187 | KIAA1287 protein | 13.20 |
| 435063 | R21966 | Hs.57734 | G protein-coupled receptor kinase-intera | 13.19 |
| 439367 | BE386844 | Hs.248746 | ESTs | 13.17 |
| 451957 | AI796320 | Hs.10299 | Homo sapiens cDNA FLJ13545 fis, clone PL | 13.16 |
| 420569 | AA278362 | Hs.289062 | Homo sapiens cDNA FLJ12334 fis, clone MA | 13.14 |
| 447883 | BE262802 | Hs.4909 | dickkopf (Xenopus laevis) homolog 3 | 13.07 |
| 426490 | NM_001621 | Hs.170087 | aryl hydrocarbon receptor | 13.06 |
| 414789 | AA155859 | Hs.79708 | ESTs | 13.05 |
| 451418 | BE387790 | Hs.26369 | ESTs | 13.04 |
| 443494 | T99719 | Hs.270404 | Homo sapiens cDNA:FLJ22389 fis, clone H | 13.03 |
| 425878 | AW964806 | Hs.38085 | ESTs, Weakly similar to putative glycine | 13.02 |
| 431912 | AI660552 | Hs.154903 | ESTs, Weakly similar to A56154 Abl subst | 13.00 |
| 407122 | H20276 | Hs.31742 | ESTs | 13.00 |
| 456491 | AL137466 | Hs.97277 | Homo sapiens mRNA; cDNA DKFZp434H1322 (f | 12.99 |
| 448172 | N75276 | Hs.135904 | ESTs | 12.98 |
| 452144 | AA032197 | Hs.102558 | ESTs | 12.96 |
| 419953 | BE267154 | Hs.125752 | ESTs | 12.96 |
| 416182 | NM_004354 | Hs.79069 | cyclin G2 | 12.94 |
| 451154 | AA015879 | Hs.33536 | ESTs | 12.93 |
| 412257 | AW903830 | | gb:CM4-NN1037-250400-155-h04 NN1037 Homo | 12.93 |
| 449784 | AW161319 | Hs.12915 | ESTs | 12.92 |
| 432695 | D63480 | Hs.278634 | KIAA0146 protein | 12.92 |
| 454105 | NM_001259 | Hs.38481 | cyclin-dependent kinase 6 | 12.92 |
| 439093 | AA534163 | Hs.5476 | serine protease inhibitor, Kazal type, 5 | 12.90 |
| 416098 | H41324 | Hs.31581 | ESTs, Moderately similar to ST1B_HUMAN S | 12.88 |
| 424897 | D63216 | Hs.153684 | frizzled-related protein | 12.88 |
| 414604 | AU076649 | Hs.76556 | growth arrest and DNA-damage-inducible 3 | 12.88 |
| 414664 | AA587775 | Hs.66295 | Homo sapiens HSPC311 mRNA, partial cds | 12.84 |
| 452560 | BE077084 | | gb:RC5-BT0603-220200-013-C07 BT0603 Homo | 12.84 |
| 413869 | NM_000878 | Hs.75596 | interleukin 2 receptor, beta | 12.80 |
| 452359 | BE167229 | Hs.29206 | Homo sapiens clone 24659 mRNA sequence | 12.80 |
| 435886 | BE265839 | Hs.12126 | hepatocellular carcinoma-associated anti | 12.78 |
| 445230 | U97018 | Hs.12451 | echinoderm microtubule-associated protei | 12.78 |
| 412226 | W26786 | | gb:15d7 Human retina cDNA randomly prime | 12.77 |
| 446619 | AU076643 | Hs.313 | secreted phosphoprotein 1 (osteopontin, | 12.76 |
| 447769 | AW873704 | Hs.48764 | ESTs | 12.76 |
| 414478 | AI306389 | Hs.76240 | adenylate kinase 1 | 12.76 |
| 425383 | D83407 | Hs.156007 | Down syndrome critical region gene 1-lik | 12.68 |
| 450704 | H85157 | Hs.40696 | ESTs | 12.66 |
| 405856 | | | | 12.66 |
| 412935 | BE267045 | Hs.75064 | tubulin-specific chaperone c | 12.65 |
| 402802 | | | | 12.62 |
| 452588 | AA889120 | Hs.110637 | Homeo box A10 | 12.62 |
| 419978 | NM_001454 | Hs.93974 | forkhead box J1 | 12.62 |
| 403137 | | | | 12.60 |
| 430226 | BE245562 | Hs.2551 | adrenergic,beta-2-, receptor, surface | 12.57 |
| 448076 | AJ133123 | Hs.20196 | adenylate cyclase 9 | 12.56 |
| 450462 | F07097 | Hs.300828 | Homo sapiens mRNA full length insert cDN | 12.54 |
| 405236 | | | | 12.52 |
| 409292 | AA071051 | | gb:zm58e05.s1 Stratagene fibroblast (937 | 12.47 |
| 421540 | AA797669 | Hs.10242 | ESTs | 12.47 |
| 425840 | AW978731 | Hs.301824 | ESTs | 12.44 |
| 443181 | AI039201 | Hs.54548 | ESTs | 12.42 |
| 452436 | BE077546 | Hs.31447 | ESTs | 12.42 |
| 455183 | AW984111 | | gb:RC0-HN0007-160300-011-f09 HN0007 Homo | 12.40 |
| 432887 | AI926047 | Hs.162859 | ESTs | 12.37 |
| 410494 | M36564 | Hs.64016 | protein S (alpha) | 12.36 |
| 439024 | R96696 | Hs.35598 | ESTs | 12.36 |
| 451246 | AW189232 | Hs.39140 | cutaneous T-cell lymphoma tumor antigen | 12.36 |
| 432892 | AL042615 | Hs.15995 | ESTs | 12.35 |
| 418982 | AI348838 | Hs.13073 | ESTs | 12.35 |
| 414516 | AI307802 | Hs.279551 | ESTs | 12.34 |
| 440134 | BE410734 | | gb:601301619F1 NIH_MGC_21 Homo sapiens c | 12.29 |
| 443873 | AL048542 | Hs.16291 | ESTs | 12.28 |
| 401286 | | | | 12.26 |
| 454020 | AW962845 | Hs.256527 | ESTs | 12.24 |
| 420077 | AW512260 | Hs.87767 | ESTs | 12.24 |
| 443837 | AI984625 | Hs.9884 | spindle pole body protein | 12.24 |
| 407519 | X64979 | | gb:H.sapiens mRNA HTPCRX01 for olfactory | 12.23 |
| 435839 | AF249744 | Hs.25951 | Rho guanine nucleotide exchange factor ( | 12.22 |
| 448552 | AW973653 | Hs.20104 | hypothetical protein FLJ00052 | 12.20 |
| 405325 | | | | 12.20 |
| 451009 | AA013140 | Hs.115707 | ESTs | 12.18 |
| 423066 | Y18264 | Hs.120171 | ESTs | 12.17 |
| 439556 | AI623752 | Hs.163603 | ESTs | 12.16 |
| 443062 | N77999 | Hs.8963 | Homo sapiens mRNA full length insert cDN | 12.15 |
| 445873 | AA250970 | Hs.251946 | Homo sapiens cDNA: FLJ23107 fis, clone L | 12.14 |
| 453542 | AW836724 | Hs.33190 | Homo sapiens mRNA expressed only in plac | 12.11 |
| 440106 | AA864968 | Hs.127699 | ESTs | 12.10 |
| 417605 | AF006609 | Hs.82294 | regulator of G-protein signalling 3 | 12.10 |
| 440286 | U29589 | Hs.7138 | cholinergic receptor, muscarinic 3 | 12.04 |
| 420061 | AW024937 | Hs.29410 | ESTs | 12.02 |
| 458727 | AI022813 | Hs.92679 | Homo sapiens clone CDABP0014 mRNA sequen | 11.96 |
| 445407 | AI222658 | Hs.221889 | ESTs, Weakly similar to la costa [D.mela | 11.95 |
| 418250 | U29926 | Hs.83918 | adenosine monophosphate deaminase (isoto | 11.94 |
| 414129 | AI990287 | Hs.270798 | ESTs | 11.93 |
| 409799 | D11928 | Hs.76845 | phosphoserine phosphatase-like | 11.92 |
| 438461 | AW075485 | Hs.286049 | phosphoserine aminotransferase | 11.92 |
| 443912 | R37257 | Hs.184780 | ESTs | 11.92 |
| 424606 | AA343936 | | gb:EST49786 Gall bladder Homo sapiens | 11.90 |
| 434217 | AW014795 | Hs.23349 | ESTs | 11.90 |
| 451533 | NM_004657 | Hs.26530 | serum deprivation response (phosphatidyl | 11.90 |
| 422423 | AF283777 | Hs.116481 | CD72 antigen | 11.89 |
| 409398 | AW386461 | | gb:PM4-PT0019-121299-004-F02 PT0019 Homo | 11.89 |
| 423853 | AB011537 | Hs.133466 | slit (Drosophila) homolog 1 | 11.82 |
| 446180 | AI074413 | Hs.14220 | hypothetical protein FLJ20450 | 11.80 |
| 414341 | D80004 | Hs.75909 | KIAA0182 protein | 11.80 |
| 406538 | | | | 11.79 |
| 433253 | AW450502 | Hs.24218 | ESTs | 11.79 |
| 447397 | BE247676 | Hs.18442 | E-1 enzyme | 11.78 |
| 451684 | AF216751 | Hs.26813 | CDA14 | 11.76 |
| 416862 | R23765 | Hs.23575 | ESTs | 11.74 |
| 425770 | NM_014363 | Hs.159492 | spastic ataxia of Charlevoix-Saguenay (s | 11.72 |
| 428826 | AL048842 | Hs.194019 | attractin | 11.72 |
| 433037 | NM_014158 | Hs.279938 | HSPC067 protein | 11.72 |
| 447476 | BE293466 | Hs.20880 | ESTs | 11.72 |
| 452092 | BE245374 | Hs.27842 | hypothetical protein FLJ11210 | 11.72 |
| 412922 | M60721 | Hs.74870 | H2.0 (Drosophila)-like homeo box 1 | 11.72 |
| 401680 | NK_005578 | Hs.180398 | LIM domain-containing preferred transloc | 11.69 |
| 422576 | BE548555 | Hs.118554 | CGI-83 protein | 11.68 |
| 450203 | AF097994 | Hs.301528 | L-kynurenine/alpha-aminoadipate aminotra | 11.68 |
| 410531 | AW752953 | | gb:QV0-CT0224-261099-035-g02 CT0224 Homo | 11.67 |
| 425917 | W28517 | Hs.117167 | Homo sapiens cDNA:FLJ23067 fis, clone L | 11.66 |
| 418693 | AI750878 | Hs.87409 | thrombospondin 1 | 11.64 |
| 400557 | | | | 11.62 |
| 416188 | BE157260 | Hs.79070 | v-myc avian myelocytomatosis viral oncog | 11.60 |
| 419047 | AW952771 | Hs.90043 | ESTs | 11.59 |
| 420441 | AI986160 | Hs.88446 | ESTs | 11.59 |
| 400885 | | | | 11.57 |
| 409853 | AW502327 | | gb:UI-HF-BR0p-aka-a-07-0-Ul.r1 NIH_MGC_5 | 11.56 |
| 400802 | | | | 11.56 |
| 434540 | NM_016045 | Hs.5184 | TH1 drosophila homolog | 11.55 |
| 431449 | M55994 | Hs.256278 | tumor necrosis factor receptor superfami | 11.55 |
| 425928 | S55736 | Hs.238852 | ESTs, Weakly similar to hypothetical pro | 11.54 |
| 434701 | AA460479 | Hs.4096 | KIAA0742 protein | 11.53 |
| 434228 | Z42047 | Hs.283978 | ESTs; KIAA0738 gene product | 11.52 |
| 420729 | AW964897 | Hs.290825 | ESTs | 11.52 |
| 428328 | AA426080 | Hs.98489 | ESTs | 11.50 |
| 433887 | AW204232 | Hs.279522 | ESTs | 11.50 |
| 414812 | X72755 | Hs.77367 | monokine induced by gamma interferon | 11.46 |
| 457718 | F18572 | Hs.22978 | ESTs | 11.44 |
| 452260 | AA453208 | Hs.28726 | RAB9, member RAS oncogene family | 11.42 |
| 459029 | AA131376 | Hs.285203 | fibroblast growth factor 12 | 11.42 |
| 456267 | AI127958 | Hs.83393 | cystatin E/M | 11.39 |
| 433285 | AW975944 | Hs.237396 | ESTs | 11.38 |
| 449186 | AW291876 | Hs.196986 | ESTs | 11.37 |
| 447861 | AI434593 | Hs.164294 | ESTs | 11.37 |
| 456023 | R00028 | | gb:ye70a06_s1 Soares fetal river spleen | 11.36 |
| 439444 | AI277652 | Hs.54578 | ESTs | 11.31 |
| 401163 | | | | 11.31 |
| 430886 | L36149 | Hs.248116 | chemokine (C motif) XC receptor 1 | 11.28 |
| 450784 | AW246803 | Hs.47289 | ESTs | 11.28 |
| 452391 | AL044829 | Hs.29331 | carnitine palmitoyltransferase l, muscle | 11.27 |
| 449625 | NM_014253 | Hs.23796 | odz (odd Oz/ten-m, Drosophila) homolog 1 | 11.26 |
| 456827 | AA075687 | Hs.147176 | epidermal growth factor receptor substra | 11.24 |
| 439328 | W07411 | Hs.118212 | ESTs, Moderately similar to ALU3_HUMAN A | 11.24 |
| 432093 | H28383 | | gb:yl152c03.r1 Soares breast 3NbHBst Homo | 11.24 |
| 407335 | AA631047 | Hs.158761 | Homo sapiens cDNA FLJ13054 fis, clone NT | 11.23 |
| 442501 | AA315267 | Hs.23128 | ESTs | 11.22 |
| 429746 | AJ237672 | Hs.214142 | 5,10-methylenetetrahydrofolate reductase | 11.21 |
| 422858 | R35398 | | gb:yg64g10.r1 Soares infant brain 1NIB H | 11.20 |
| 415156 | X84908 | Hs.78060 | phosphorylase kinase, beta | 11.20 |
| 446713 | AV660122 | Hs.282675 | ESTs | 11.20 |
| 452221 | C21322 | Hs.11577 | ESTs | 11.20 |
| 418261 | W78902 | Hs.293297 | ESTs | 11.17 |
| 433332 | AI367347 | Hs.127809 | ESTs | 11.16 |
| 434539 | AW748078 | Hs.214410 | ESTs | 11.16 |
| 413471 | BE142098 | | gb:CM4-HT0137-220999-017-d11 HT0137 Homo | 11.14 |
| 410037 | AB020725 | Hs.58009 | KIAA0918 protein | 11.14 |
| 405601 | | | | 11.13 |
| 458332 | AI000341 | Hs.220491 | ESTs | 11.12 |
| 427654 | AA410183 | Hs.137475 | ESTs | 11.12 |
| 427138 | N77624 | Hs.173717 | phosphatidic acid phosphatase type 2B | 11.10 |
| 431475 | AI567669 | Hs.287316 | ESTs | 11.10 |
| 425710 | AF030880 | Hs.159275 | solute carrier family, member 4 | 11.08 |
| 413748 | AW104057 | Hs.19193 | ESTs | 11.07 |
| 409208 | Y00093 | Hs.51077 | integrin, alpha X (antigen CD11C (p150), | 11.07 |
| 457278 | W92745 | Hs.193324 | ESTs | 11.03 |
| 407021 | U52077 | | gb:Human mariner1 transposase gene, comp | 11.02 |
| 445701 | AF055581 | Hs.13131 | lymphocyte adaptor protein | 11.02 |
| 408338 | AW867079 | | gb:MR1-SN0033-120400-002-c10 SN0033 Homo | 10.95 |
| 401030 | BE382701 | Hs.25960 | v-myc avian myelocytomatosis viral relat | 10.95 |
| 437891 | AW006969 | Hs.6311 | hypothetical protein FLJ20859 | 10.94 |
| 453874 | AW591783 | Hs.36131 | collagen, type XIV, alpha 1 (undulin) | 10.94 |
| 421562 | AA530994 | Hs.105803 | ghrelin precursor | 10.92 |
| 413431 | AW246428 | Hs.75355 | ubiquitin-conjugating enzyme E2N (homolo | 10.92 |
| 400132 | | | | 10.92 |
| 436420 | AA443966 | Hs.31595 | ESTs | 10.90 |
| 424880 | NM_000328 | Hs.153614 | retinitis pigmentosa GTPase regulator | 10.88 |
| 433264 | D85782 | Hs.3229 | cysteine dioxygenase, type I | 10.88 |
| 429842 | AI366213 | Hs.173422 | KIAA1605 protein | 10.87 |
| 412405 | AW948126 | | gb:RC0-MT0013-280300-031-a12 MT0013 Homo | 10.85 |
| 400615 | | | | 10.80 |
| 425018 | BE245277 | Hs.154196 | E4F transcription factor 1 | 10.80 |
| 456011 | BE243628 | | gb:TCBAP1D1053 Pediatric pre-B cell acut | 10.79 |
| 455982 | BE176862 | | gb:RC4-HT0587-170300-012-a04 HT0587 Homo | 10.74 |
| 450418 | BE218418 | Hs.201802 | ESTs | 10.73 |
| 412490 | AW803564 | Hs.288850 | ESTs | 10.72 |
| 436962 | AW377314 | Hs.5364 | DKFZP564l052 protein | 10.70 |
| 437743 | AI383497 | Hs.131811 | ESTs, Weakly similar to ALU1_HUMAN ALU S | 10.70 |
| 449967 | R40978 | Hs.271498 | ESTs, Moderately similar to ALU1_HUMAN A | 10.70 |
| 449590 | AA694070 | Hs.268835 | ESTs | 10.68 |
| 446035 | NM_006558 | Hs.13565 | Sam68-like phosphotyroslne protein, T-ST | 10.68 |
| 426530 | U24578 | Hs.170250 | complement component 4A | 10.66 |
| 428600 | AW863261 | Hs.15036 | ESTs, Highly similar to AF161358 1 HSPC0 | 10.64 |
| 420090 | AA220238 | Hs.94986 | ribonuclease P (38kD) | 10.64 |
| 451593 | AF151879 | Hs.26706 | CGI-121 protein | 10.62 |
| 438893 | AF075031 | Hs.29327 | ESTs | 10.62 |
| 459324 | AW080953 | | gb:xc28c12.x1 NCI_CGAP_Co18 Homo sapiens | 10.61 |
| 439883 | AL359652 | Hs.171096 | Homo sapiens EST from clone DKFZp434A041 | 10.58 |
| 406513 | AA715328 | Hs.291205 | ESTs | 10.57 |
| 407826 | AA128423 | Hs.40300 | calpain 3, (p94) | 10.57 |
| 419550 | D50918 | Hs.90998 | KIAA0128 protein; septin 2 | 10.56 |
| 428522 | R10184 | Hs.191987 | ESTs, Weakly similar to ALU1_HUMAN ALU S | 10.56 |
| 459526 | AI142350 | Hs.146735 | EST | 10.55 |
| 411448 | AA178955 | Hs.271439 | ESTs | 10.54 |
| 410102 | AW248508 | Hs.279727 | ESTs; | 10.52 |
| 406577 | | | | 10.52 |
| 408405 | AK001332 | Hs.44672 | hypothetical protein FLJ10470 | 10.51 |
| 428966 | AF059214 | Hs.194687 | cholesterol 25-hydroxylase | 10.50 |
| 400880 | | | | 10.48 |
| 415875 | AA894876 | Hs.5687 | protein phosphatase 1B (formerly 2C), ma | 10.48 |
| 434715 | BE005346 | Hs.116410 | ESTs | 10.46 |
| 406851 | AA609784 | Hs.180255 | major histocompatibility complex, class | 10.44 |
| 413409 | AI638418 | Hs.21745 | ESTs | 10.44 |
| 418489 | U76421 | Hs.85302 | adenosine deaminase, RNA-specific, B1 (h | 10.44 |
| 419465 | AW500239 | Hs.21187 | Homo sapiens cDNA: FLJ23068 fis, clone L | 10.44 |
| 419544 | AI909154 | | gb:QV-BT200-010499-007 BT200 Homo sapien | 10.44 |
| 432180 | Y18418 | Hs.272822 | RuvB (E coli homolog)-like 1 | 10.44 |
| 413822 | R08950 | Hs.272044 | ESTs, Weakly similar to ALU1_HUMAN ALU S | 10.42 |
| 437446 | AA788946 | Hs.16869 | ESTs, Moderately similar to CA1C RAT COL | 10.41 |
| 415701 | NM_003878 | Hs.78619 | gamma-glutamyl hydrolase (conjugase, fol | 10.41 |
| 443790 | NM_003500 | Hs.9795 | acyl-Coenzyme A oxidase 2, branched chai | 10.40 |
| 458873 | AW150717 | Hs.296176 | STAT induced STAT inhibitor 3 | 10.38 |
| 415082 | AA160000 | Hs.137396 | ESTs | 10.37 |
| 429124 | AW505086 | Hs.196914 | minor histocompatibility antigen HA-1 | 10.36 |
| 417187 | AB011151 | Hs.81505 | KIAA0579 protein | 10.34 |
| 426827 | AW067805 | Hs.172665 | methylenetetrahydrofolate dehydrogenase | 10.34 |
| 424280 | NM_000030 | Hs.271366 | alanine-glyoxylate aminotransferase homo | 10.33 |
| 446099 | T93096 | Hs.17126 | ESTs | 10.32 |
| 423445 | NM_014324 | Hs.128749 | alpha-methylacyl-CoA racemase | 10.31 |
| 409995 | AW960597 | Hs.30164 | ESTs | 10.30 |
| 432242 | AW022715 | Hs.162160 | ESTs, Weakly similar to ALU4_HUMAN ALU S | 10.30 |
| 406394 | AA172106 | Hs.110950 | Rag C protein | 10.30 |
| 406189 | | | | 10.29 |
| 422283 | AW411307 | Hs.114311 | CDC45 (cell division cycle 45, S.cerevis | 10.26 |
| 401598 | AA172106 | Hs.110950 | Rag C protein | 10.26 |
| 456995 | T89832 | Hs.170278 | ESTs | 10.26 |
| 416511 | NM_006762 | Hs.79356 | Lysosomal-associated multispanning membr | 10.24 |
| 427274 | NM_005211 | Hs.174142 | colony stimulating factor 1 receptor, fo | 10.24 |
| 401384 | | | | 10.23 |
| 456226 | D13168 | Hs.82002 | endothelin receptor type B | 10.22 |
| 426928 | AF037062 | Hs.172914 | retinol dehydrogenase 5 (11-cisand 9-cis | 10.21 |
| 423032 | AI684746 | Hs.119274 | ESTs | 10.20 |
| 436556 | AI364997 | Hs.7572 | ESTs | 10.20 |
| 418400 | BE243026 | Hs.301989 | KIAA0246 protein | 10.19 |
| 437401 | AA757196 | Hs.121190 | ESTs | 10.19 |
| 403690 | | | | 10.17 |
| 423790 | BE152393 | | gb:CM2-HT0323-171199-033-a08 HT0323 Homo | 10.16 |
| 434094 | AA305599 | Hs.238205 | hypothetical protein PRO2013 | 10.16 |
| 434967 | AW975009 | Hs.292274 | ESTs | 10.16 |
| 432827 | Z68128 | Hs.3109 | Rho GTPase activating protein 4 | 10.16 |
| 432660 | AI288430 | Hs.64004 | ESTs | 10.14 |
| 452234 | AW084176 | Hs.223296 | ESTs | 10.14 |
| 445629 | AI245701 | | gb:qk31f05.x1 NCI_CGAP_Kid3 Homo sapiens | 10.13 |
| 457236 | AA626142 | Hs.179991 | ESTs, Weakly similar to KPCE_HUMAN PROTE | 10.13 |
| 444605 | AI174603 | Hs.254105 | enolase 1, (alpha) | 10.12 |
| 450313 | AI038989 | Hs.24809 | hypothetical protein FLJ10826 | 10.12 |
| 407482 | NM_006056 | | | 10.12 |
| 449971 | AA807346 | Hs.288581 | Homo sapiens cDNA FLJ14296 fis, clone PL | 10.11 |
| 441201 | AW118822 | Hs.128757 | ESTs | 10.10 |
| 435157 | AW014605 | Hs.179872 | ESTs | 10.10 |
| 417308 | H60720 | Hs.81892 | KIAA0101 gene product | 10.09 |
| 442582 | AI204266 | Hs.179303 | ESTs | 10.05 |
| 437252 | AI433833 | Hs.164159 | ESTs, Weakly similar to ALU1_HUMAN ALU S | 10.04 |
| 448663 | BE614599 | Hs.106823 | H.sapiens gene from PAC 426l6, similar t | 10.04 |
| 434467 | BE552368 | Hs.231853 | Homo sapiens cDNA FLJ13445 fis, clone PL | 10.04 |
| 423698 | AA329796 | Hs.1098 | DKFZp434J1813 protein | 10.02 |
| 412707 | AW206373 | Hs.16443 | Homo sapiens cDNA: FLJ21721 fis, clone C | 10.00 |
| 414658 | X58528 | Hs.76781 | ATP-binding cassette, sub-family D (ALD) | 10.00 |
| 421832 | NM_016098 | Hs.108725 | HSPC040 protein | 10.00 |
| 423554 | M90516 | Hs.1674 | glutamine-fructose-6-phosphate transamin | 10.00 |
| 452039 | AI922988 | Hs.172510 | ESTs | 10.00 |
| 434673 | AW137442 | Hs.136965 | ESTs | 10.00 |
| 427978 | AA418280 | Hs.180040 | Homo sapiens cDNA:FLJ22439 fis, clone H | 10.00 |
| 457803 | BE501815 | Hs.198011 | ESTs | 9.99 |
| 428279 | AA425310 | Hs.155766 | ESTs | 9.98 |
| 444412 | AI147652 | Hs.216381 | Homo sapiens clone HH409 unknown mRNA | 9.98 |
| 417049 | N72394 | Hs.44862 | ESTs | 9.96 |
| 427509 | M62505 | Hs.2161 | complement component 5 receptor 1 (C5a l | 9.96 |
| 445424 | AB028945 | Hs.12696 | contactin SH3 domain-binding protein | 9.96 |
| 443678 | AW009605 | Hs.231923 | ESTs | 9.96 |
| 447567 | AW474513 | Hs.224397 | ESTs, Weakly similar to B48013 proline-r | 9.94 |
| 414709 | AA704703 | Hs.77031 | Sp2 transcription factor | 9.94 |
| 434596 | T59538 | | gb:yb65g12.s1 Stratagene ovary (937217) | 9.94 |
| 427630 | BE276115 | Hs.144980 | ESTs, Weakly similar to CA13_HUMAN COLLA | 9.93 |
| 416111 | AA033813 | Hs.79018 | chromatin assembly factor 1, subunit A ( | 9.92 |
| 423349 | AF010258 | Hs.127428 | homeo box A9 | 9.92 |
| 424308 | AW975531 | Hs.154443 | minichromosome maintenance deficient (S. | 9.92 |
| 416814 | AW192307 | Hs.80042 | dolichyl-P-Glc:Man9GlcNAc2-PP-dolichylgl | 9.90 |
| 417986 | AA481003 | Hs.97128 | ESTs | 9.90 |
| 425174 | D87450 | Hs.154978 | KIAA0261 protein | 9.90 |
| 438171 | AW976507 | Hs.293515 | ESTs | 9.90 |
| 421984 | AW972187 | Hs.110443 | hypothetical protein FLJ22215 | 9.89 |
| 408597 | NM_005291 | Hs.46453 | G protein-coupled receptor 17 | 9.88 |
| 413907 | AI097570 | Hs.71222 | ESTs | 9.87 |
| 451296 | AW801383 | Hs.118578 | H.sapiens mRNA for ribosomal protein L18 | 9.86 |
| 433409 | AI278802 | Hs.25661 | ESTs | 9.85 |
| 450360 | AW117416 | Hs.245484 | ESTs | 9.85 |
| 433104 | AL043002 | Hs.128246 | ESTs, Moderately similar to unnamed prot | 9.84 |
| 449824 | AI962552 | Hs.226765 | ESTs | 9.84 |
| 452744 | AI267652 | Hs.30504 | Homo sapiens mRNA; cDNA DKFZp434E082 (fr | 9.82 |
| 431066 | AF026273 | Hs.249175 | interleukin-1 receptor-associated kinase | 9.82 |
| 426457 | AW894667 | Hs. 169965 | chimerin (chimaerin) 1 | 9.80 |
| 443371 | AI792888 | Hs.145489 | ESTs | 9.80 |
| 437159 | AL050072 | | gb:Homo sapiens mRNA; cDNA DKFZp566E1346 | 9.75 |
| 425242 | D13635 | Hs.155287 | KIAA0010 gene product | 9.74 |
| 447498 | N67619 | Hs.43687 | ESTs | 9.74 |
| 426759 | AI590401 | Hs.21213 | ESTs | 9.73 |
| 435129 | AI381659 | Hs.267086 | ESTs | 9.72 |
| 437672 | AW748265 | Hs.5741 | flavohemoprotein b5+b5R | 9.72 |
| 438209 | AL120659 | Hs.6111 | KIAA0307 gene product | 9.72 |
| 438440 | AA807228 | Hs.225161 | ESTs | 9.72 |
| 449720 | AA311152 | Hs.288708 | ESTs; Weakly similar to KIAA0226 [H.sapi | 9.72 |
| 414291 | AI289619 | Hs.13040 | ESTs | 9.72 |
| 436206 | AK001451 | Hs.265561 | CD2-associated protein | 9.70 |
| 446896 | T15767 | Hs.22452 | Homo sapiens cDNA:FLJ21084 fis, clone C | 9.70 |
| 412667 | AW977540 | Hs.269254 | ESTs | 9.70 |
| 423301 | S67580 | Hs.1645 | cytochrome P450, subfamily IVA, polypept | 9.67 |
| 440757 | AW118645 | Hs.160004 | ESTs | 9.67 |
| 441412 | AI393657 | Hs.159750 | ESTs | 9.66 |
| 421044 | AF061871 | Hs.101302 | collagen, type XII, alpha 1 | 9.66 |
| 414726 | BE466863 | Hs.280099 | ESTs | 9.66 |
| 418485 | R91679 | Hs.124981 | ESTs | 9.66 |
| 433480 | X02422 | Hs.181125 | immunoglobulin lambda locus | 9.65 |
| 441530 | AI248301 | Hs.127112 | ESTs | 9.65 |
| 433533 | D53304 | Hs.65394 | ESTs | 9.65 |
| 421470 | R27496 | Hs.1378 | annexin A3 | 9.64 |
| 438613 | C05569 | Hs.243122 | hypothetical protein FLJ13057 similar to | 9.64 |
| 429324 | AA488101 | Hs.199245 | inactivation escape 1 | 9.62 |
| 450244 | AA007534 | Hs.125062 | ESTs | 9.62 |
| 407660 | AW063190 | Hs.279101 | ESTs | 9.61 |
| 406554 | | | | 9.60 |
| 426404 | AA377607 | Hs.273138 | ESTs | 9.58 |
| 447045 | AW392394 | Hs.278569 | KIAA0064 gene product | 9.58 |
| 449894 | AK001578 | Hs.24129 | hypothetical protein FLJ10716 | 9.58 |
| 448376 | AI494332 | Hs.196963 | ESTs | 9.58 |
| 407902 | AL117474 | Hs.41181 | Homo sapiens mRNA; cDNA DKFZp727C191 (fr | 9.56 |
| 446572 | AV659151 | Hs.282961 | ESTs | 9.56 |
| 459245 | BE242623 | Hs.31939 | manic fringe (Drosophila) homolog | 9.55 |
| 423545 | AP000692 | Hs.129781 | chromosome 21 open reading frame 5 | 9.54 |
| 414697 | BE266134 | Hs.76927 | translocase of outer mitochondrial membr | 9.54 |
| 410846 | AW807057 | | gb:MR4-ST0062-031199-018-b03 ST0062 Homo | 9.52 |
| 421181 | NM_005574 | Hs.184585 | LIM domain only 2 (rhombotin-like 1) | 9.52 |
| 427308 | D26067 | Hs.174905 | KIAA0033 protein | 9.52 |
| 415995 | NM_004573 | Hs.994 | phospholipase C, beta 2 | 9.51 |
| 434846 | AW295389 | Hs.119768 | ESTs | 9.51 |
| 414342 | AA742181 | Hs.75912 | Homo sapiens cDNA: FLJ22199 fis, clone H | 9.50 |
| 416959 | D28459 | Hs.80612 | ubiquitin-conjugating enzyme E2A (RAD6 h | 9.50 |
| 443123 | AA094538 | Hs.6588 | ESTs | 9.50 |
| 439312 | AA833902 | Hs.270745 | ESTs | 9.48 |
| 449375 | R07114 | Hs.271224 | ESTs | 9.48 |
| 436357 | AJ132085 | | gb:Homo sapiens mRNA for axonemal dynein | 9.44 |
| 458723 | AW137726 | Hs.244352 | ESTs, Moderately similar to laminin alph | 9.44 |
| 457526 | AW450584 | Hs.192131 | ESTs, Weakly similar to RIBB [H.sapiens] | 9.43 |
| 404741 | | | | 9.43 |
| 422409 | NM_005428 | Hs.116237 | vav 1 oncogene | 9.43 |
| 403708 | | | | 9.42 |
| 408806 | AW847814 | Hs.289005 | Homo sapiens cDNA:FLJ21532 fis, clone C | 9.42 |
| 417380 | T06809 | | gb:EST04698 Fetal brain, Stratagene (cat | 9.42 |
| 422501 | AA354690 | Hs.144967 | ESTs | 9.42 |
| 426197 | AA004410 | Hs.167835 | acyl-Coenzyme A oxidase 1, palmitoyl | 9.42 |
| 452624 | AU076606 | Hs.30054 | coagulation factor V (proaccelerin, labi | 9.42 |
| 412110 | AW893569 | | gb:RC0-NN0021-040400-021-c10 NN0021 Homo | 9.41 |
| 414158 | AA361623 | Hs.288775 | Homo sapiens cDNA FLJ13900 fis, clone TH | 9.41 |
| 408101 | AW968504 | Hs.123073 | CDC2-related protein kinase 7 | 9.40 |
| 414171 | AA360328 | Hs.865 | RAP1A, member of RAS oncogene family | 9.40 |
| 415947 | U04045 | Hs.78934 | mutS (E. coli) homolog 2 (colon cancer, | 9.40 |
| 426959 | BE262745 | | gb:601153869F1 NIH_MGC_19 Homo sapiens c | 9.39 |
| 417519 | AI689987 | Hs.177669 | ESTs, Weakly similar to RMS1_HUMAN REGUL | 9.39 |
| 457181 | BE514362 | Hs.296422 | FK506-binding protein 3 (25kD) | 9.39 |
| 402835 | | | | 9.38 |
| 404632 | | | | 9.38 |
| 446566 | H95741 | Hs.17914 | Homo sapiens cDNA: FLJ22801 fis, clone K | 9.37 |
| 455369 | AW903533 | | gb:CM1-NN1031-060400-178-d05 NN1031 Homo | 9.37 |
| 444001 | AI095087 | Hs.152299 | ESTs, Moderately similar to ALU5_HUMAN A | 9.36 |
| 458191 | AI420611 | Hs.127832 | ESTs | 9.36 |
| 431374 | BE258532 | Hs.251871 | CTP synthase | 9.34 |
| 429327 | AA283981 | Hs.199248 | prostaglandin E receptor 4 (subtype EP4) | 9.33 |
| 407061 | X97748 | | gb:H.sapiens PTX3 gene promotor region. | 9.33 |
| 416967 | BE616731 | Hs.80645 | interferon regulatory factor 1 | 9.33 |
| 423013 | AW875443 | Hs.22209 | secreted modular calcium-binding protein | 9.33 |
| 439461 | AA693960 | Hs.103158 | ESTs | 9.33 |
| 418830 | BE513731 | Hs.88959 | Human DNA sequence from clone 967N21 on | 9.32 |
| 422763 | AA033699 | Hs.83938 | ESTs, Moderately,similar to MASP-2 [H.sa | 9.32 |
| 442739 | NM_007274 | Hs.8679 | cytosolic acyl coenzyme A thioester hydr | 9.32 |
| 452859 | AI300555 | Hs.288158 | Homo sapiens cDNA: FLJ23591 fis, clone L | 9.32 |
| 403237 | | | | 9.32 |
| 415000 | AW025529 | Hs.239812 | ESTs, Weakly similar to CALM_HUMAN CALMO | 9.31 |
| 417951 | AW976410 | Hs.289069 | Homo sapiens cDNA: FLJ21016 fis, clone C | 9.30 |
| 419066 | Z98492 | Hs.6975 | PR01073 protein | 9.30 |
| 448443 | AW167128 | Hs.231934 | ESTs | 9.30 |
| 405125 | | | | 9.30 |
| 409768 | AW499566 | | gb:UI-HF-BR0p-aji-h-03-0-UI.r1 NIH_MGC_5 | 9.28 |
| 453708 | AI191811 | Hs.54629 | ESTs | 9.28 |
| 442271 | AF000652 | Hs.8180 | syndecan binding protein (syntenin) | 9.27 |
| 410055 | AJ250839 | Hs.58241 | gene for serine/threonine protein kinase | 9.26 |
| 448692 | AW013907 | Hs.224276 | ESTs, Moderately similar to predicted us | 9.26 |
| 417381 | AF164142 | Hs.82042 | solute carrier family 23 (nucleobase tra | 9.25 |
| 422497 | D29642 | Hs.1528 | KIAA0053 gene product | 9.25 |
| 414140 | AA281279 | Hs.23317 | ESTs | 9.24 |
| 435980 | AF274571 | Hs.129142 | ESTs; Weakly similar to DEOXYRIBONUCLEAS | 9.24 |
| 458530 | BE395035 | Hs.199889 | ESTs, Weakly similar to KIAA0874 protein | 9.24 |
| 402585 | | | | 9.24 |
| 420819 | AA280700 | | gb:zs95h11.s1 NCI_CGAP_GCB1 Homo sapiens | 9.23 |
| 444755 | AA431791 | Hs.183001 | ESTs | 9.22 |
| 411630 | U42349 | Hs.71119 | Putative prostate cancer tumor suppresso | 9.22 |
| 421246 | AW582962 | Hs.300961 | ESTs, Highly similar to AF151805 1 CGI-4 | 9.20 |
| 421924 | BE514514 | Hs.109606 | coronin, aclin-binding protein, 1A | 9.19 |
| 414888 | AL039185 | Hs.77558 | thyroid hormone receptor interactor 7 | 9.18 |
| 434267 | AI206589 | Hs.116243 | ESTs | 9.17 |
| 409213 | U61412 | Hs.51133 | PTK6 protein tyrosine kinase 6 | 9.17 |
| 428242 | H55709 | Hs.2250 | leukemia inhibitory factor (cholinergic | 9.16 |
| 451736 | AW080356 | Hs.293684 | ESTs, Weakly similar to alternatively sp | 9.15 |
| 413627 | BE182082 | Hs.246973 | ESTs | 9.14 |
| 416134 | AA528402 | Hs.74861 | activated RNA polymerase II transcriptio | 9.14 |
| 449251 | AW151660 | Hs.31444 | ESTs | 9.14 |
| 452813 | U54727 | Hs.191445 | ESTs | 9.14 |
| 443622 | AI911527 | Hs.11805 | ESTs | 9.14 |
| 413260 | BE075281 | | gb:PM1-BT0585-290200-005-d07 BT0585 Homo | 9.12 |
| 413450 | Z99716 | Hs.75372 | N-acetylgalactosaminidase, alpha- | 9.12 |
| 446442 | BE221533 | Hs.257858 | ESTs | 9.12 |
| 438540 | AA810021 | Hs.136906 | ESTs | 9.12 |
| 426251 | M24283 | Hs.168383 | Intercellular adhesion molecule 1 (CD54) | 9.11 |
| 410290 | AA402307 | Hs.73818 | ubiquinol-cytochrome c reductase hinge p | 9.10 |
| 437398 | AA913736 | Hs.126715 | ESTs | 9.10 |
| 421559 | NM_014720 | Hs.105751 | Ste20-related serine/threonine kinase | 9.10 |
| 439699 | AF086534 | Hs.187561 | ESTs, Moderately similar to ALU1_HUMAN A | 9.10 |
| 430799 | C19035 | Hs.164259 | ESTs | 9.09 |
| 424544 | M88700 | Hs.150403 | dopa decarboxylase (aromatic L-amino aci | 9.08 |
| 453942 | AW190920 | Hs.19928 | ESTs | 9.08 |
| 425844 | T68073 | Hs.159628 | serine (or cysteine) proteinase inhibito | 9.08 |
| 434658 | AI624436 | Hs.194488 | ESTs | 9.07 |
| 453999 | BE328153 | Hs.240087 | ESTs | 9.06 |
| 436490 | R71543 | Hs.18713 | ESTs | 9.05 |
| 409192 | AA065131 | Hs.233439 | ESTs, Weakly similar to ALU7_HUMAN ALU S | 9.05 |
| 446223 | BE300091 | Hs.119699 | hypothetical protein FLJ12969 | 9.04 |
| 447247 | AW369351 | Hs.287955 | Homo sapiens cDNA FLJ13090 fis, clone NT | 9.04 |
| 450094 | AI174947 | Hs.295789 | Homo sapiens mRNA; cDNA DKFZp564D1164 (f | 9.04 |
| 432012 | AW301344 | Hs.195969 | ESTs | 9.04 |
| 422520 | AU076730 | Hs.117977 | kinesin 2 (60-70kD) | 9.02 |
| 418650 | BE386750 | Hs.86978 | prolyl endopeptidase | 9.02 |
| 423008 | M81590 | Hs.123016 | 5-hydroxytryptamine (serotonin) receptor | 9.02 |
| 436476 | AA326108 | Hs.53631 | ESTs | 9.02 |
| 448206 | BE622585 | Hs.3731 | ESTs | 9.02 |
| 431574 | AW572659 | Hs.261373 | adenosine A2b receptor pseudogene | 9.01 |
| 443453 | R99876 | Hs.269882 | ESTs | 9.01 |
| 435472 | AW972330 | Hs.283022 | triggering receptor expressed on myeloid | 9.01 |
| 420337 | AW295840 | Hs.14555 | Homo sapiens cDNA: FLJ21513 fis, clone C | 9.00 |
| 449810 | AB008681 | Hs.23994 | activin A receptor, type IIB | 9.00 |
| 406780 | AA902386 | Hs.286 | ribosomal protein L4 | 8.99 |
| 429169 | AW341130 | Hs.197757 | ESTs, Moderately similar to FGFE_HUMAN F | 8.99 |
| 421326 | AF051428 | Hs.103504 | estrogen receptor 2 (ER beta) | 8.97 |
| 425491 | AA883316 | Hs.255221 | ESTs | 8.96 |
| 425516 | BE000707 | Hs.29567 | ESTs | 8.96 |
| 439773 | AI051313 | Hs.143315 | ESTs | 8.96 |
| 443247 | BE614387 | Hs.47378 | ESTs | 8.96 |
| 456623 | AI084125 | Hs.108106 | transcription factor | 8.95 |
| 438707 | L08239 | Hs.5326 | porcupine | 8.95 |
| 402240 | | | | 8.95 |
| 444152 | AI125694 | Hs.149305 | Homo sapiens cDNA FLJ14264 fis, clone PL | 8.95 |
| 409842 | AW501756 | | gb:Ul-HF-BR0p-ajm-c-09-0-Ul.r1 NIH_MGC_5 | 8.94 |
| 416277 | W78765 | Hs.73580 | ESTs | 8.94 |
| 456697 | AI908006 | Hs.111334 | ferritin, light polypeptide | 8.94 |
| 410762 | AF226053 | Hs.66170 | HSKM-B protein | 8.92 |
| 412942 | AL120344 | Hs.75074 | mitogen-activated protein kinase-activat | 8.92 |
| 442320 | AI287817 | Hs.129636 | ESTs | 8.92 |
| 449673 | AA002064 | Hs.18920 | ESTs | 8.91 |
| 411486 | N85785 | Hs.181165 | eukaryotic translation elongation factor | 8.90 |
| 437916 | BE566249 | Hs.20999 | Homo sapiens cDNA:FLJ23142 fis, clone L | 8.90 |
| 442732 | AA257161 | Hs.8658 | hypothetical protein DKFZp434E0321 | 8.89 |
| 419741 | NM_007019 | Hs.93002 | ubiquitin carrier protein E2-C | 8.89 |
| 411499 | AW849292 | | gb:IL3-CT0215-020300-090-E06 CT0215 Homo | 8.89 |
| 431154 | AW971228 | Hs.290259 | ESTs | 8.89 |
| 414922 | D00723 | Hs.77631 | glycine cleavage system protein H (amino | 8.88 |
| 418036 | Z37976 | Hs.83337 | latent transforming growth factor beta b | 8.87 |
| 406422 | | | | 8.87 |
| 422926 | NM_016102 | Hs.121748 | ring finger protein 16 | 8.87 |
| 435220 | D50030 | Hs.104 | HGF activator | 8.86 |
| 418203 | X54942 | Hs.83758 | CDC28 protein kinase 2 | 8.86 |
| 418613 | AA744529 | Hs.86575 | mitogen-activated protein kinase kinase | 8.85 |
| 439250 | H66566 | Hs.271711 | ESTs | 8.85 |
| 432359 | AA076049 | Hs.274415 | Homo sapiens cDNA FLJ10229 fis, clone HE | 8.84 |
| 450000 | AI952797 | Hs.10888 | Homo sapiens cDNA: FLJ21559 fis, clone C | 8.83 |
| 425657 | T89839 | Hs.119471 | ESTs | 8.83 |
| 425694 | U51333 | Hs.159237 | hexokinase 3 (white cell) | 8.82 |
| 419972 | AL041465 | Hs.294038 | ESTs,Moderately similar to ALU2_HUMAN A | 8.82 |
| 436396 | AI683487 | Hs.299112 | Homo sapiens cDNA FLJ11441 fis, clone HE | 8.82 |
| 413413 | D82520 | Hs.301834 | Homo sapiens cDNA FLJ10952 fis, clone PL | 8.82 |
| 428807 | AA435997 | Hs.104930 | ESTs | 8.82 |
| 415839 | R40611 | Hs.137565 | ESTs | 8.81 |
| 419553 | N34145 | Hs.250614 | ESTs | 8.80 |
| 420309 | AW043637 | Hs.21766 | ESTs | 8.80 |
| 421863 | AI952677 | Hs.108972 | Homo sapiens mRNA; cDNA DKFZp434P228 (fr | 8.80 |
| 447965 | AW292577 | Hs.94445 | ESTs | 8.80 |
| 459172 | BE063380 | | gb:PM0-BT0275-291099-002-g10 BT0275 Homo | 8.80 |
| 403259 | | | | 8.78 |
| 411534 | AW850473 | | gb:IL3-CT0219-280100-061-B11 CT0219 Homo | 8.78 |
| 456161 | BE264645 | Hs.282093 | Homo sapiens cDNA: FLJ21918 fis, clone H | 8.77 |
| 413654 | AA331881 | Hs.75454 | peroxiredoxin 3 | 8.76 |
| 401744 | | | | 8.76 |
| 425348 | AL137477 | Hs.155912 | cadherin-like 24 | 8.76 |
| 423396 | AI382555 | Hs.127950 | bromodomain-containing 1 | 8.75 |
| 450649 | NM_001429 | Hs.297722 | Human DNA sequence from clone RP1-85F18 | 8.75 |
| 408331 | NM_007240 | Hs.44229 | dual specificity phosphatase 12 | 8.74 |
| 423872 | AB020316 | Hs.134015 | uronyl 2-sulfotransferase | 8.74 |
| 424906 | AI566086 | Hs.153716 | Homo sapiens mRNA for Hmob33 protein, 3' | 8.74 |
| 427596 | AA449506 | Hs.179765 | Homo sapiens mRNA; cDNA DKFZp586H1921 (f | 8.73 |
| 432488 | AA551010 | Hs.216640 | ESTs | 8.72 |
| 448980 | AL137527 | Hs.22703 | Homo sapiens mRNA; cDNA DKFZp434P1018 (f | 8.72 |
| 429455 | AI472111 | Hs.292507 | ESTs | 8.71 |
| 429855 | AW385597 | Hs.138902 | ESTs, Weakly similar to B34087 hypotheti | 8.71 |
| 441746 | H59955 | Hs.127829 | ESTs | 8.70 |
| 411945 | AL033527 | Hs.92137 | v-myc avian myelocytomatosis viral oncog | 8.70 |
| 413492 | D87470 | Hs.75400 | KIAA0280 protein | 8.70 |
| 435706 | W31254 | Hs.7045 | GL004 protein | 8.70 |
| 433741 | AA609019 | Hs.159343 | ESTs | 8.70 |
| 426340 | Z97989 | Hs.169370 | FYN oncogene related to SRC, FGR, YES | 8.69 |
| 422779 | AA317036 | Hs.41989 | ESTs | 8.67 |
| 449785 | AI225235 | Hs.288300 | Homo sapiens cDNA: FLJ23231 fis, clone C | 8.67 |
| 420144 | AA811813 | Hs.119421 | ESTs | 8.66 |
| 420235 | AA256756 | Hs.31178 | ESTs | 8.66 |
| 432606 | NM_002104 | Hs.3066 | granzyme K (serine protease, granzyme 3; | 8.66 |
| 425762 | BE244076 | Hs.159578 | Homo sapiens mRNA for FLJ00020 protein, | 8.65 |
| 427448 | BE246449 | Hs.2157 | Wiskott-Aldrich syndrome (eczema-thrombo | 8.64 |
| 418033 | W68180 | Hs.259855 | Homo sapiens cDNA FLJ12507 fis, clone NT | 8.64 |
| 429084 | AJ001443 | Hs.195614 | splicing factor 3b, subunit 3, 130kD | 8.64 |
| 417094 | NM_006895 | Hs.81182 | histamine N-methyltransferase | 8.64 |
| 457277 | NM_004736 | Hs.227656 | xenotroplc and polytroplc retrovirus rec | 8.63 |
| 422631 | BE218919 | Hs.118793 | hypothetical protein FLJ10688 | 8.63 |
| 410679 | AW795196 | Hs.215857 | ring finger protein 14 | 8.63 |
| 431585 | BE242803 | Hs.262823 | hypothetical protein FLJ10326 | 8.62 |
| 401851 | | | | 8.62 |
| 401866 | | | | 8.62 |
| 407783 | AW996872 | Hs.172028 | a disintegrin and metalloproteinase doma | 8.62 |
| 408242 | AA251594 | Hs.43913 | PIBF1 gene product | 8.62 |
| 422250 | AW408530 | Hs.113823 | ClpX (caseinolytic protease X, E. coli) | 8.62 |
| 430259 | BE550182 | Hs.127826 | RalGEF-like protein 3, mouse homolog | 8.62 |
| 452598 | AI831594 | Hs.68647 | ESTs, Weakly similar to ALU7_HUMAN ALU S | 8.62 |
| 419541 | AW749617 | | gb:RC3-BT0502-130100-012-g07 BT0502 Homo | 8.60 |
| 428839 | AI767756 | Hs.82302 | ESTs | 8.60 |
| 429328 | AA829402 | Hs.47939 | ESTs | 8.60 |
| 451491 | AI972094 | Hs.286221 | Homo sapiens cDNA FLJ13741 fis, clone PL | 8.60 |
| 452561 | AI692181 | Hs.49169 | KIAA1634 protein | 8.60 |
| 420027 | AF009746 | Hs.94395 | ATP-binding cassette, sub-family D (ALD) | 8.60 |
| 435205 | X54136 | Hs.181125 | immunoglobulin lambda locus | 8.60 |
| 430900 | U91939 | Hs.248123 | G protein-coupled receptor 25 | 8.60 |
| 405074 | | | | 8.59 |
| 437991 | AI479773 | Hs.181679 | ESTs | 8.59 |
| 436346 | BE328882 | Hs.193096 | ESTs, Moderately similar to U119_HUMAN U | 8.58 |
| 411079 | AA091228 | | gb:cchn2152.seq.F Human fetal heart, Lam | 8.57 |
| 418452 | BE379749 | Hs.85201 | C-type (calcium dependent, carbohydrate- | 8.56 |
| 429109 | AL008637 | Hs.196352 | neutrophil cytosolic factor 4 (40kD) | 8.56 |
| 448019 | AW947164 | Hs.195641 | ESTs | 8.56 |
| 449865 | AW204272 | Hs.199371 | ESTs | 8.55 |
| 431180 | H55883 | | gb:yq94h03.r1 Soares fetal liver spleen | 8.54 |
| 445988 | BE007663 | Hs.13503 | inactivation escape 2 | 8.54 |
| 405876 | | | | 8.54 |
| 407235 | D20569 | Hs.169407 | SAC2 (suppressor of actin mutations 2, y | 8.54 |
| 414807 | AI738616 | Hs.77348 | hydroxyprostaglandin dehydrogenase 15-(N | 8.54 |
| 425671 | AF193612 | Hs.159142 | lunatic fringe (Drosophila) homolog | 8.54 |
| 452413 | AW082633 | Hs.212715 | ESTs | 8.54 |
| 421620 | AA446183 | Hs.91885 | ESTs | 8.53 |
| 444539 | AI955765 | Hs.146907 | ESTs | 8.52 |
| 415102 | M31899 | Hs.77929 | excision repair cross-complementing rode | 8.51 |
| 405552 | | | | 8.51 |
| 418068 | AW971155 | Hs.293902 | ESTs, Weakly similar to prolyl 4-hydroxy | 8.50 |
| 420133 | AA426117 | Hs.14373 | ESTs | 8.50 |
| 438887 | R68857 | Hs.265499 | ESTs | 8.50 |
| 446468 | AI765890 | Hs.16341 | ESTs; Moderately similar to !!!! ALU SUB | 8.50 |
| 446585 | AV659397 | Hs.282948 | ESTs | 8.50 |
| 441896 | AW891873 | | gb:CM3-NT0090-040500-173-b02 NT0090 Homo | 8.50 |
| 437718 | AI927288 | Hs.196779 | ESTs | 8.48 |
| 420656 | AA279098 | Hs.187636 | ESTs | 8.48 |
| 429303 | AW137635 | Hs.44238 | ESTs | 8.48 |
| 450624 | AL043983 | Hs.125063 | Homo sapiens cDNA FLJ3825 fis, clone TH | 8.48 |
| 452573 | AI907957 | Hs.287622 | Homo sapiens cDNA FLJ14082 fis, clone HE | 8.48 |
| 456341 | AA229126 | Hs.122647 | N-myristoyltransferase 2 | 8.48 |
| 423024 | AA593731 | Hs.75613 | CD36 antigen (collagen type I receptor, | 8.47 |
| 446985 | AL038704 | Hs.156827 | ESTs, Weakly similar to ALU1_HUMAN ALU S | 8.46 |
| 431778 | AL080276 | Hs.268562 | regulator of G-protein signalling 17 | 8.46 |
| 400268 | | | | 8.46 |
| 421828 | AW891965 | Hs.289109 | dimethylarginine dimethylaminohydrolase | 8.45 |
| 417022 | NM_014737 | Hs.80905 | Ras association (RalGDS/AF-6) domain fam | 8.44 |
| 421029 | AW057782 | Hs.293053 | ESTs | 8.44 |
| 425171 | AW732240 | Hs.300615 | ESTs | 8.44 |
| 459070 | AI814302 | | gb:wj71c12.x1 NCI_CGAP_Lu19 Homo sapiens | 8.42 |
| 406006 | | | | 8.42 |
| 412643 | AW971239 | Hs.293982 | ESTs | 8.42 |
| 424775 | AB014540 | Hs.153026 | SWAP-70 protein | 8.42 |
| 446848 | AW136083 | Hs.195266 | ESTs; Weakly similar to S59501 interfero | 8.42 |
| 448043 | AI458653 | Hs.201881 | ESTs | 8.41 |
| 407183 | AA358015 | | gb:EST66864 Fetal lung III Homo sapiens | 8.40 |
| 412324 | AW978439 | Hs.69504 | ESTs | 8.40 |
| 419594 | AA013051 | Hs.91417 | topoisomerase (DNA) II binding protein | 8.40 |
| 430968 | AW972830 | | gb:EST384925 MAGE resequences, MAGL Homo | 8.40 |
| 431689 | AA305688 | Hs.267695 | UDP.Gal:betaGlcNAc beta 1,3-galactosyltr | 8.40 |
| 438582 | AI521310 | Hs.283365 | ESTs.Weakly similar to ALU5_HUMAN ALU S | 8.40 |
| 447685 | AL122043 | Hs.19221 | hypothetical protein DKFZp566G1424 | 8.40 |
| 459119 | AW844498 | Hs.289052 | Homo sapiens LENG8 mRNA, variant C, part | 8.38 |
| 400817 | | | | 8.37 |
| 425265 | BE245297 | | gb:TCBAP1E2482 Pediatric pre-B cell acut | 8.37 |
| 409385 | AA071267 | | gb:zm61g01.r1 Stratagene fibroblast (937 | 8.36 |
| 439121 | BE047779 | Hs.44701 | ESTs | 8.36 |
| 419968 | X04430 | Hs.93913 | interleukin 6 (interferon, beta 2) | 8.36 |
| 408327 | AW182309 | Hs.249963 | ESTs, Highly similar to dJ1170K4.4 [H.sa | 8.35 |
| 403976 | | | | 8.34 |
| 448064 | AA379036 | | gb:EST91809 Synovial sarcoma Homo sapien | 8.33 |
| 442914 | AW188551 | Hs.99519 | Homo sapiens cDNA FLJ14007 fis, clone Y7 | 8.33 |
| 428032 | AW997704 | Hs.11493 | Homo sapiens cDNA FLJ13536 fis, clone PL | 8.32 |
| 434194 | AF119847 | Hs.283940 | Homo sapiens PRO1550 mRNA, partial cds | 8.32 |
| 458677 | AW937670 | Hs.254379 | ESTs | 8.32 |
| 420925 | NM_015698 | Hs.100391 | T54 protein | 8.30 |
| 416475 | T70298 | | gb:yd26g02.s1 Soares fetal liver spleen | 8.30 |
| 416852 | AF283776 | Hs.80285 | Homo sapiens mRNA; cDNA DKFZp586C1723 (f | 8.30 |
| 430676 | AF084866 | | gb:Homo sapiens envelope protein RIC-3 ( | 8.30 |
| 428455 | AI732694 | Hs.98520 | ESTs | 8.29 |
| 435343 | AW194962 | Hs.199028 | ESTs | 8.29 |
| 450783 | BE266695 | | gb:601190242F1 NIH_MGC_7 Homo sapiens cD | 8.29 |
| 404946 | | | | 8.28 |
| 422942 | AF054839 | Hs.122540 | tetraspan 2 | 8.28 |
| 453716 | AA037675 | Hs.152675 | ESTs | 8.28 |
| 437098 | AA744488 | Hs.132842 | ESTs, Moderately similar to ALU1_HUMAN A | 8.28 |
| 443907 | AU076484 | Hs.9963 | TYRO protein tyrosine kinase binding pro | 8.27 |
| 401930 | AF106069 | Hs.23168 | ubiquitin specific protease 15 | 8.26 |
| 446554 | AA151730 | Hs.301789 | ESTs, Weakly similar to similar to C.ele | 8.26 |
| 426290 | AB007918 | Hs.169182 | KIAA0449 protein | 8.25 |
| 419904 | AA974411 | Hs.18672 | ESTs | 8.25 |
| 413886 | AW958264 | Hs.103832 | ESTs, Weakly similar to TRHY_HUMAN TRlCH | .824 |
| 424738 | AI963740 | Hs.46826 | ESTs | 8.24 |
| 427359 | AW020782 | Hs.79881 | Homo sapiens cDNA: FLJ23006 fis, clone L | 8.24 |
| 424534 | D87682 | Hs.150275 | KIAA0241 protein | 8.24 |
| 424429 | U63830 | Hs.146847 | TRAF family member-associated NFKB activ | 8.24 |
| 442604 | BE263710 | Hs.279904 | ESTs | 8.22 |
| 442992 | AI914699 | Hs.13297 | ESTs | 8.22 |
| 427210 | BE396283 | Hs.173987 | eukaryotic translation initiation factor | 8.22 |
| 457229 | BE222450 | Hs.266390 | ESTs | 8.21 |
| 423730 | AA330214 | | gb:EST33935 Embryo, 12 week II Homo sapi | 8.21 |
| 411928 | AA888624 | Hs.19121 | adaptor-related protein complex 2, alpha | 8.20 |
| 416051 | AA835868 | Hs.25253 | Homo sapiens cDNA: FLJ20935 fis, clone A | 8.20 |
| 417231 | R40739 | Hs.21326 | ESTs | 8.20 |
| 422049 | W25760 | Hs.77631 | glycine cleavage system protein H (amino | 8.20 |
| 427528 | AU077143 | Hs.179565 | minichromosome maintenance deficient (S. | 8.20 |
| 458776 | AV654978 | Hs.19904 | cystathionase (cystathionine gamma-lyase | 8.19 |
| 417687 | AI828596 | Hs.250691 | ESTs | 8.18 |
| 423218 | NM_015896 | Hs.167380 | BLu protein | 8.18 |
| 425397 | J04088 | Hs.156346 | topoisomerase (DNA) II alpha (170kD) | 8.18 |
| 406964 | M21305 | Hs.247946 | Human alpha satellite and satellite 3 ju | 8.18 |
| 402401 | U42349 | Hs.71119 | Putative prostate cancer tumor suppresso | 8.18 |
| 423397 | NM_001838 | Hs.1652 | chemokine (C-C motif) receptor 7 | 8.18 |
| 427857 | AL133017 | Hs.2210 | thyroid hormone receptor interactor 3 | 8.17 |
| 401519 | | | | 8.17 |
| 447188 | H65423 | Hs.17631 | Homo sapiens cDNA FLJ20118 fis, clone CO | 8.16 |
| 424704 | AI263293 | Hs.152096 | cytochrome P450, subfamily IIJ (arachido | 8.16 |
| 435854 | AJ278120 | Hs.4996 | DKFZP564D166 protein | 8.14 |
| 448556 | AW885606 | Hs.5064 | ESTs | 8.14 |
| 449217 | AA278536 | Hs.23262 | ribonuclease, RNase A family, k6 | 8.14 |
| 453124 | AI139058 | Hs.23296 | ESTs | 8.14 |
| 442812 | AI018406 | Hs.131284 | ESTs | 8.14 |
| 421129 | BE439899 | Hs.89271 | ESTs | 8.14 |

**TABLE 9A shows the accession numbers for those primekeys lacking a unigeneID in Table 9. For each probeset we have listed the gene cluster number from which the oligonucleotides were designed. Gene clusters were compiled using sequences derived from Genbank ESTs and mRNAs. These sequences were clustered based on sequence similarity using Clustering and Alignment Tools (DoubleTwist, Oakland California). The Genbank accession numbers for sequences comprising each cluster are listed in the "Accession" column.**

| | | |
|---|---|---|
| Pkey: | | Unique Eos probeset identifier number |
| CAT number: | | Gene cluster number |
| Accession: | | Genbank accession numbers |
| | | |

| **Pkey** | **CAT number** | **Accession** |
|---|---|---|
| | | |
| 408057 1035720_-1 | | AW139565 |
| 408069 | 103655_1 | H81795 Z42291 R20973 AA046920 |
| 408182 | 104479_1 | AA047854 AA057506 AA053841 |
| 408338 | 1052148_1 | AW867079 AW867086 AW182772 |
| 408828 | 108463_1 | BE540279 AW410659 AA057857 R77693 BE278674 |
| 409126 | 110159_1 | AA063426 AW962323 AW408063 AA063503 AA772927 AW753492 BE175371 AA311147 |
| 409292 | 111586_1 | AA071051 AA070584 AA069938 AA102136 AA074430 |
| 409314 | 111841_1 | AA070266 AA084967 AA126998 |
| 409385 | 112523_1 | AA071267 T65940 T64515 AA071334 |
| 409398 | 1126716_1 | AW386461 AW876408 AW386672 AW386599 AW876258 AW386619 AW386289 AW876136 AW876203 AW876213 AW876301 |
| | | AW876295 AW876349 AW876365 AW876160 AW876369 AW876352 AW876271 |
| 409671 | 114731_1 | AA076769 AA076781 AI087968 |
| 409768 | 1154035_1 | AW499566 AW502378 AW499522 AW502046 AW502671 AW501917 AW501868 AW501721 AW502813 |
| 409841 | 156088_1 | AW502139 AW502432 AW502235 AW501683 AW502647 |
| 409842 | 1156119_1 | AW501756 AW502096 AW502465 AW501715 |
| 409853 | 1156226_1 | AW502327 AW502488 AW501829 AW502625 AW502687 |
| 410531 | 1207200_1 | AW752953 H88044 BE156092 |
| 410688 | 1216101_1 | AW796342 AW796356 BE161430 |
| 410846 | 1223902_1 | AW807057 AW807054 AW807189 AW807193 AW807369 AW807429 AW807364 AW807365 AW807078 AW807256 AW807180 |
| | | AW807331 |
| 410896 | 1226053_1 | AW809637 AW809697 AW810554 AW809707 AW809885 AW810000 AW810088 AW809742 AW809816 AW809749 AW809639 |
| | | AW809722 AW809836 AW809774 AW810023 AW810013 AW809813 AW809660 AW809728 AW809768 AW809951 AW809657 |
| | | AW809954 |
| 411079 | 123128_1 | AA091228 H71860 H71073 |
| 411424 | 1245497_1 | AW845985 AW845991 AW845962 |
| 411499 | 1248105_1 | AW849292 AW849431 AW849422 AW849428 AW849420 AW849424 AW849427 |
| 411507 | 1248607_1 | AW850140 AW850195 AW850192 |
| 411534 | 1248827_1 | AW850473 AW850471 AW850431 AW850523 |
| 411972 | 1268491_1 | BE074959 AW880160 |
| 412110 | 1277844_1 | AW893569 AW893571 AW893588 AW893593 |
| 412226 | 1284289_1 | W26786 AW998612 AW902272 |
| 412257 | 1285376_1 | AW903830 BE071916 |
| 412405 | 1293012_1 | AW948126 AW948139 AW948196 AW948145 AW948162 AW948134 AW948127 AW948124 AW948153 AW948157 AW948125 |
| | | AW948131 AW948158 AW948164 AW948151 |
| 413260 | 1356003_1 | BE075281 BE075219 BE075123 BE075119 BE075046 |
| 413471 | 1371778_1 | BE142098 BE142092 |
| 413729 | 1385114_1 | BE159999 BE160056 BE160107 BE160139 |
| 414182 | 142409_1 | AA136301 AI381776 AA136321 |
| 414989 | 1511339_1 | T81668 C19040 C17569 |
| 415354 | 1534763_1 | F06495 R24336 R13046 |
| 416011 | 1566439_1 | H14487 R50911 Z43216 |
| 416475 | 1596398_1 | T70298 H58072 R02750 |
| 417380 | 1672461_1 | T06809 N75735 |
| 419392 | 1843934_-1 | W28573 |
| 419541 | 185724_1 | AW749617 R64714 AA244138 AA244137 BE094019 |
| 419544 | 185760_2 | AI909154 AA526337 AA244193 AI909153 |
| 420819 | 196721_1 | AA280700 AW975494 AA687385 |
| 421245 | 200620_1 | AA285363 AA285333 AA285359 AA285326 AA285350 |
| 422673 | 219674_1 | N59027 AA314694 N53937 R08100 |
| 422695 | 219996_1 | AA315158 AW961298 N76067 AW802759 AI858495 W04474 |
| 422858 | 222209_1 | R35398 BE252178 AA318153 |
| 422940 | 223106_1 | BE077458 AA337277 AA319285 |
| 423730 | 231462_1 | AA330214 AW962519 T54709 |
| 423790 | 232031_1 | BE152393 AA330984 BE073904 |
| 424385 | 238731_1 | AA339666 AW952809 AA349119 |
| 424606 | 241409_1 | AA343936 AA344060 AW963081 |
| 425265 | 249175_1 | BE245297 AA353976 AW505023 |
| 426959 | 273830_-1 | BE262745 |
| 430676 | 32168_1 | AF084866 AF084870 AF084864 AF084867 AF084869 AF084865 AF084868 AW818206 AW812038 BE144813 BE144812 |
| | | AW812041 AW812040 AW812067 BE061583 BE061604 T05808 AI352469 AA580921 BE141783 BE141782 BE061601 |
| | | AW814393 AW885029 |
| 430968 | 326269_1 | AW972830 AA527647 AA489820 AA570362 |
| 431180 | 328906_1 | H55883 AW971249 AA493900 H55788 |
| 432093 | 341283_1 | H28383 AW972670 H28359 AA525808 |
| 434596 | 38937_1 | T59538 T59589 T59598 T59542 AF147374 |
| 436357 | 41842_1 | AJ132085 Z83805 |
| 437159 | 43393_1 | AL050072 AW900148 |
| 437495 | 43765_1 | BE177778 BE177779 AL390180 AA359908 |
| 439097 | 46858_1 | H66948 AF085954 H66949 |
| 439120 | 46879_1 | H56389 AF085977 H56173 |
| 440134 | 48675_1 | BE410734 BE560117 BE270054 BE296330 BE267957 AI003007 BE545259 |
| 441896 | 52842_1 | AW891873 AW891897 BE564764 |
| 445629 | 645767_1 | AI245701 BE272724 |
| 447229 | 71288_1 | BE617135 AW504051 AW504283 |
| 448064 | 74761_1 | AA379036 AA150589 AI696854 BE621316 |
| 450783 | 84655_1 | BE266695 BE265474 N53200 BE267333 |
| 451045 | 85673_1 | AA215672 AI696628 AA013335 H86334 AA017006 |
| 452549 | 921802_1 | AI907039 AI907081 |
| 452560 | 922216_1 | BE077084 AW139963 AW863127 AW806209 AW806204 AW806205 AW806206 AW606211 AW806212 AW806207 AW806208 |
| | | AW806210 AI907497 |
| 452712 | 928309_1 | AW838616 AW838660 BE144343 AI914520 AW886910 BE184854 BE184784 |
| 453758 | 980026_1 | U83527 AL120938 U83522 |
| 454093 | 1007366_1 | AW860158 AW862385 AW860159 AW862386 AW862341 AW821869 AW821893 AW062660 AW062656 |
| 454563 | 1224342_1 | AW807530 AW807540 AW807537 AW846086 BE141634 AW846089 AW807499 AW807533 AW838499 |
| 454791 | 1234759_1 | BE071874 BE071882 AW820782 AW821007 |
| 454977 | 1247099_1 | AW848032 AW848630 AW848478 AW848623 AW848484 AW848169 AW848830 AW848149 AW848119 AW848893 AW848903 |
| | | AW848407 |
| 455131 | 1254674_1 | AW857913 AW857916 AW857914 AW861627 AW861626 AW861624 |
| 455183 | 1259023_1 | AW984111 AW863918 AW863856 |
| 455254 | 1266449_1 | AW877015 AW877133 AW876978 AW877071 AW876988 AW877069 AW877063 AW877013 |
| 455369 | 1285173_16 | AW903533 AW903516 AW903562 BE085202 BE085215 BE085214 BE085209 BE085172 BE085175 BE085193 BE085211 |
| | | BE085199 |
| 455982 | 1396849_1 | BE176862 BE176876 BE176947 BE176878 |
| 456011 | 1410860_1 | BE243628 BE246081 BE247016 BE241984 BE241534 BE246091 BE245679 BE243620 BE245998 BE242329 BE241417 |
| | | BE241457 BE242522 BE241989 BE241464 |
| 456023 | 1416335_1 | R00028 BE247630 |
| 457586 | 360505_1 | AW062439 AW751554 AA579463 |
| 457595 | 364225_-1 | AA584854 |
| 457751 | 399422_1 | AI908236 AA663731 |
| 459070 | 883688_1 | AI814302 AI814428 |
| 459081 | 889426_1 | W07808 AI822066 |
| 459145 | 918957_1 | AI903354 AI903489 AI903488 |
| 459172 | 921149_1 | BE063380 BE063346 AI906097 |
| 459234 | 945240_-1 | AI940425 |

**TABLE 9B shows the genomic positioning for those primekeys lacking unigene ID's and accession numbers in Table 9. For each predicted exon, we have listed the genomic sequence source used for prediction. Nucleotide locations of each predicted exon are also listed.**

| | | | |
|---|---|---|---|
| Pkey: | | Unique number corresponding to an Eos probeset | |
| Ref: | | Sequence source. The 7 digit numbers in this column are Genbank Identifier (GI) numbers. "Dunham I. et al." refers to the publication entitled "The DNA sequence of human chromosome 22." Dunham I. et al., Nature (1999) 402:489-495. | |
| Strand: | | Indicates DNA strand from which exons were predicted. | |
| Nt_position: | | Indicates nucleotide positions of predicted exons. | |
| | | | |

| **Pkey** | **Ref** | **Strand** | **Nt_position** |
|---|---|---|---|
| | | | |
| 400452 | 8113550 | Minus | 90308-90505 |
| 400557 | 9801261 | Plus | 208453-208528,209633-209813 |
| 400615 | 9908994 | Plus | 118036-118166,118681-118807 |
| 400802 | 8567867 | Minus | 174571-174856 |
| 400817 | 8569994 | Plus | 170793-170948 |
| 400880 | 9931121 | Plus | 29235-29336,36363-36580 |
| 400885 | 9958187 | Minus | 58242-58733 |
| 400926 | 7651921 | Minus | 52033-52158,53956-54120,54957-55052,55420-55480,56452-56666,57221-57718 |
| 400952 | 7658481 | Plus | 192667-192826,194387-194876 |
| 400991 | 8096825 | Plus | 159197-159320 |
| 401044 | 8117619 | Plus | 73501-73674 |
| 401124 | 8570296 | Minus | 124181-124391 |
| 401163 | 6981820 | Plus | 5302-5545 |
| 401201 | 9743387 | Minus | 138534-138629,139234-139294,140121-140335,142033-142479 |
| 401286 | 9801342 | Minus | 147036-147318 |
| 401384 | 6850939 | Minus | 58360-58545 |
| 401468 | 6433826 | Plus | 13056-13482 |
| 401515 | 7630851 | Plus | 29929-30126 |
| 401519 | 6649315 | Plus | 157315-157950 |
| 401672 | 9838136 | Plus | 128526-128704,130755-130860 |
| 401744 | 2576349 | Plus | 14595-14751 |
| 401851 | 7770425 | Minus | 146443-146664,147794-147971,148351-148480,148980-149111,149801-149949 |
| 401866 | 8018106 | Plus | 73126-73623 |
| 402240 | 7690131 | Plus | 104382-104527,106136-106372 |
| 402359 | 9211204 | Minus | 40403-41961 |
| 402585 | 9908890 | Minus | 174893-175050,183210-183435 |
| 402788 | 9796102 | Plus | 98273-101430 |
| 402802 | 3287156 | Minus | 53242-53432 |
| 402812 | 6010110 | Plus | 25026-25091,25844-25920 |
| 402828 | 8918414 | Plus | 69071-69642 |
| 402835 | 9187337 | Plus | 26961-27101 |
| 402838 | 9369121 | Minus | 32589-32735,35478-35666 |
| 402842 | 9369121 | Minus | 76355-76479 |
| 402895 | 9967547 | Plus | 85537-85671,86379-86469 |
| 402964 | 9581599 | Minus | 46624-46784 |
| 403137 | 9211494 | Minus | 92349-92572,92958-93084,93579-93712,93949-94072,94591-94748,95214-95337 |
| 403237 | 7637807 | Plus | 7271-7527 |
| 403259 | 7770585 | Plus | 4693-4857 |
| 403683 | 7331517 | Plus | 217175-217446 |
| 403690 | 7387384 | Minus | 78627-79583 |
| 403708 | 5705981 | Minus | 134394-134812 |
| 403838 | 4176355 | Plus | 19197-19502 |
| 403851 | 7708872 | Plus | 22733-23007 |
| 403976 | 7657840 | Plus | 24755-24969 |
| 404407 | 7329316 | Minus | 48154-48499 |
| 404426 | 7407959 | Plus | 77842-77954 |
| 404632 | 9796668 | Plus | 45096-45229 |
| 404741 | 8574139 | Plus | 143025-143467 |
| 404756 | 7706327 | Plus | 82849-83627 |
| 404946 | 7382189 | Plus | 134445-134750 |
| 405074 | 7770440 | Plus | 44340-44559,44790-45059 |
| 405125 | 8247873 | Plus | 137113-137814 |
| 405172 | 9966752 | Plus | 153027-153262 |
| 405236 | 7249076 | Minus | 151699-151915 |
| 405325 | 6094661 | Minus | 25818-26380 |
| 405411 | 3451356 | Minus | 17503-17778,18021-18290 |
| 405495 | 8050952 | Minus | 72182-72373 |
| 405552 | 1552506 | Plus | 45199-45647 |
| 405601 | 5815493 | Minus | 147835-147935,149220-149299 |
| 405685 | 4508129 | Minus | 37956-38097 |
| 405777 | 7263187 | Minus | 104773-105051 |
| 405856 | 7653009 | Plus | 101777-102043 |
| 405876 | 6758747 | Plus | 39694-40031 |
| 405932 | 7767812 | Minus | 123525-123713 |
| 405934 | 6758795 | Plus | 159913-160605 |
| 406006 | 8247801 | Minus | 42640-42776 |
| 406134 | 9163473 | Plus | 153291-153452 |
| 406189 | 7289992 | Minus | 22007-22234 |
| 406422 | 9256411 | Plus | 163003-163311 |
| 406516 | 7711422 | Minus | 128375-128449,128560-128784 |
| 406538 | 7711478 | Plus | 35196-35367,38229-38476,40080-40216,43522-43840 |
| 406554 | 7711566 | Plus | 106956-107121 |
| 406577 | 7711730 | Plus | 11377-11509 |

**TABLE 10: shows genes, including expression sequence tags differentially expressed in taxol resistant prostate tumor xenografts as compared to taxol sensitive prostate tumor xenografts. The genes are indicated as either being upregulated or downregulated during the induction of taxol resistance in sequential passages of the grafts.**

| | | | | | | | | | | | | | | | | |
|---|---|---|---|---|---|---|---|---|---|---|---|---|---|---|---|---|
| Pkey: | | Unique Eos probeset identifier number | | | | | | | | | | | | | | |
| ExAccn: | | Exemplar Accession number, Genbank accession number | | | | | | | | | | | | | | |
| UnigeneID: | | Unigene number | | | | | | | | | | | | | | |
| Unigene Title: | | Unigene gene title | | | | | | | | | | | | | | |
| Eos: | | Internal Eos name | | | | | | | | | | | | | | |
| F00-F14: | | passage number | | | | | | | | | | | | | | |
| | | | | | | | | | | | | | | | | |

| Pkey | ExAccn | UnigeneID | Unigen Title | Eos | Resp.F00 | F00 | F02 | F02 | F05 | F05 | F07 | F09 | F10 | F11 | F13 | F14 |
|---|---|---|---|---|---|---|---|---|---|---|---|---|---|---|---|---|
| | | | | | | | | | | | | | | | | |
| 117921 | N51002 | Hs.47170 | Liprin A2 | PM28UP | 1 | 9 | 8 | 9 | 32 | 20 | 34 | 122 | 105 | 82 | 71 | 111 |
| 112971 | T17185 | Hs.4299 | ESTs | CHA1 down | 290 | 281 | 267 | 335 | 270 | 284 | 150 | 157 | 83 | 89 | 49 | 75 |
| 126645 | AI167942 | Hs.61635 | STEAP | PAA5 down | 106 | 111 | 103 | 71 | 34 | 67 | 33 | 14 | 2 | 1 | 1 | 1 |
| 119018 | N95796 | Hs.179809 | ESTs | PAB2 down | 765 | 841 | 757 | 909 | 742 | 704 | 478 | 428 | 253 | 175 | 228 | 238 |
| 110844 | N31952 | Hs.167531 | ESTs | PAV7 down | 175 | 192 | 147 | 141 | 123 | 129 | 73 | 65 | 55 | 48 | 54 | 84 |
| 100654 | HG2841-HT2969 | Hs.75442 | Albumin, A | PM01 down | 666 | 605 | 504 | 728 | 357 | 445 | 602 | 187 | 117 | 127 | 117 | 113 |
| 100655 | HG2841-HT2970 | Hs.75442 | Albumin, A | PM02 down | 620 | 653 | 486 | 688 | 368 | 386 | 606 | 175 | 101 | 95 | 115 | 97 |
| 102076 | U09579 | Hs.252437 | cyclin-dep | PM03 down | 101 | 94 | 143 | 190 | 105 | 107 | 88 | 40 | 34 | 31 | 46 | 22 |
| 102208 | U22961 | Hs.75442 | albumin | PM04 down | 495 | 424 | 323 | 518 | 252 | 296 | 467 | 188 | 169 | 143 | 165 | 145 |
| 103739 | AA075779 | -- | mitochondr | PM05 down | 75 | 190 | 606 | 230 | 378 | 106 | 218 | 88 | 69 | 192 | 69 | 99 |
| 107036 | AA599690 | Hs.15725 | SBBl48 | PM06 down | 87 | 124 | 115 | 188 | 132 | 111 | 66 | 71 | 49 | 70 | 38 | 50 |
| 108242 | AA062746 | -- | ESTs | PM07 down | 14 | 20 | 252 | 13 | 22 | 43 | 193 | 10 | 10 | 104 | 21 | 18 |
| 108282 | AA065143 | -- | solute car | PM08 down | 27 | 54 | 178 | 73 | 108 | 37 | 53 | 24 | 14 | 53 | 15 | 34 |
| 108679 | AA115963 | -- | beta-1-glo | PM09 down | 680 | 893 | 1292 | 656 | 869 | 389 | 1 | 74 | 118 | 662 | 359 | 409 |
| 108731 | AA126313 | Hs. 107476 | ATP syntha | PM10 down | 10 | 19 | 185 | 25 | 60 | 1 | 32 | 3 | 7 | 14 | 1 | 1 |
| 110675 | H89355 | Hs.6598 | adrenergic | PM11 down | 207 | 334 | 237 | 239 | 231 | 220 | 119 | 145 | 93 | 64 | 56 | 124 |
| 115412 | AA283804 | Hs.193552 | ESTs | PM12 down | 146 | 316 | 282 | 271 | 340 | 334 | 115 | 238 | 100 | 196 | 83 | 207 |
| 115844 | AA430124 | Hs.234607 | MDM2 | PM13 down | 49 | 93 | 94 | 154 | 132 | 91 | 23 | 54 | 23 | 76 | 14 | 41 |
| 120588 | AA281591 | Hs.16193 | ESTs | PM14 down | 80 | 157 | 58 | 141 | 159 | 127 | 39 | 83 | 35 | 37 | 16 | 46 |
| 132349 | Y00705 | Hs.181286 | serine pro | PM15 down | 146 | 217 | 214 | 150 | 106 | 128 | 177 | 85 | 54 | 63 | 66 | 56 |
| 132888 | AA490775 | Hs.5920 | N-acetylma | PM16 down | 92 | 150 | 132 | 178 | 126 | 139 | 53 | 94 | 48 | 67 | 41 | 80 |
| 132967 | AA032221 | Hs.61635 | STEAP | PM17 down | 224 | 208 | 203 | 215 | 205 | 180 | 132 | 65 | 68 | 50 | 48 | 63 |
| 133063 | AA283085 | Hs.64065 | ESTs | PM18 down | 85 | 148 | 161 | 150 | 92 | 108 | 42 | 99 | 42 | 65 | 29 | 126 |
| 134374 | D62633 | Hs.8236 | ESTs | PM19 down | 230 | 240 | 194 | 212 | 231 | 189 | 89 | 123 | 107 | 95 | 68 | 91 |
| 135400 | M23263 | Hs.99915 | androgen r | PM20 down | 36 | 167 | 99 | 178 | 132 | 101 | 23 | 71 | 26 | 122 | 14 | 44 |

**TABLE 11: shows genes, including expression sequence tags that are up-regulated in prostate tumor tissue compared to normal prostate tissue as analyzed using Affymetrix/Eos Hu01 GeneChip array. Shown are the ratios of "average" normal prostate to "average" prostate cancer tissues.**

| | | | | |
|---|---|---|---|---|
| Pkey: | Unique Eos probeset identifier number | | | |
| ExAccn: | Exemplar Accession number, Genbank accession number | | | |
| UnigeneID: | Unigene number | | | |
| Unigene Title: | Unigene gene title | | | |
| R1: | Background subtracted normal prostate : prostate tumor tissue | | | |
| | | | | |

| Pkey | ExAccn | UnigeneID | Unigene Title | R1 |
|---|---|---|---|---|
| | | | | |
| 101336 | L49169 | Hs.75678 | FBJ murine osteosarcoma viral oncogene homolog B | 0.012 |
| 130642 | M63438 | Hs.156110 | Immunoglobulin kappa variable 1D-8 | 0.015 |
| 133512 | X01677 | Hs.195188 | glyceraldehyde-3-phosphate dehydrogenase | 0.017 |
| 133436 | H44631 | Hs.737 | immediate early protein | 0.017 |
| 129292 | X13810 | Hs.1101 | POU domain; class 2; transcription factor 2 | 0.019 |
| 100610 | HG2566-HT4792 | | Microtubule-Associated Protein Tau, Alt. Splice 3, Exon 8 | 0.02 |
| 133448 | M34516 | Hs.170116 | immunoglobulin lambda-like polypeptide 3 | 0.021 |
| 125193 | W67577 | Hs.84298 | CD74 antigen (invariant polypeptide of major histocompatibility complex; class II antigen-associated) | 0.022 |
| 133456 | T49257 | Hs.183704 | ubiquitin C | 0.022 |
| 134546 | AA459310 | Hs.8518 | Homo sapiens mRNA; cDNA DKFZp586L1722 (from clone DKFZp586L1722) | 0.023 |
| 102131 | U15085 | Hs.1162 | major histocompatibility complex; class II; DM beta | 0.023 |
| 101375 | M13560 | Hs.84298 | CD74 antigen (invariant polypeptide of major histocompatibility complex; class II antigen-associated) | 0.023 |
| 100674 | HG3033-HT3194 | | Spliceosomal Protein Sap 62 | 0.024 |
| 134365 | R32377 | Hs.82240 | syntaxin 3A | 0.027 |
| 132335 | D60387 | Hs.189885 | ESTs | 0.027 |
| 110303 | H37901 | Hs.32706 | ESTs | 0.028 |
| 131678 | N59162 | Hs.30542 | ESTs | 0.028 |
| 116599 | D80046 | Hs.250879 | ESTs | 0.029 |
| 133769 | M17733 | Hs.75968 | thymosin; beta 4; X chromosome | 0.029 |
| 107904 | AA026648 | Hs.61389 | ESTs | 0.03 |
| 129427 | T80746 | Hs.111334 | ferritin; light polypeptide | 0.03 |
| 105987 | AA406631 | Hs.110299 | mitogen-activated protein kinase kinase 7 | 0.03 |
| 131466 | F03233 | Hs.27189 | ESTs | 0.032 |
| 102859 | X00274 | Hs.76807 | Human HLA-DR alpha-chain mRNA | 0.032 |
| 134626 | S82198 | Hs.8709 | caldecrin (serum calcium decreasing factor, elastase IV) | 0.032 |
| 134170 | M63138 | Hs.79572 | cathepsin D (lysosomal aspartyl protease) | 0.033 |
| 131713 | X57809 | Hs.181125 | immunoglobulin lambda gene cluster | 0.034 |
| 100748 | HG3517-HT3711 | | Alpha-1-Antitrypsin, 5' End | 0.034 |
| 118769 | N74496 | | ESTs | 0.034 |
| 111734 | R25375 | Hs.126916 | ESTs | 0.036 |
| 109221 | AA192755 | Hs.85840 | ESTs; Weakly similar to stac [H.sapiens] | 0.036 |
| 133846 | AA480073 | Hs.76719 | U6 snRNA-associated Sm-like protein | 0.036 |
| 135281 | AA401575 | Hs.97757 | ESTs | 0.037 |
| 119073 | R32894 | Hs.45514 | v-ets avian erythroblastosis virus E26 oncogene related | 0.037 |
| 100760 | HG3576-HT3779 | | Major Histocompatibility Complex, Class li Beta W52 | 0.037 |
| 101426 | M19483 | Hs.25 | ATP synthase; H+ transprtng; mitochndrl F1 complex; beta polypept | 0.038 |
| 129568 | AA428025 | Hs.114360 | transforming growth factor beta-stimulated protein TSC-22 | 0.038 |
| 130900 | Z38468 | Hs.21036 | ESTs; Moderately similar to F25965_3 [H.sapiens] | 0.039 |
| 133879 | M13829 | Hs.77183 | v-raf murine sarcoma 3611 viral oncogene homolog 1 | 0.039 |
| 100627 | HG2702-HT2798 | | Serine/Threonine Kinase (Gb:Z25424) | 0.039 |
| 129424 | M55593 | Hs.111301 | matrix metalloproteinase 2 (gelatinase A; 72kD gelatinase; 72kD type IV collagenase) | 0.039 |
| 128652 | AA621245 | Hs.103147 | ESTs; Weakly similar to similar to SP:YR40_BACSU [C.elegans] | 0.039 |
| 129979 | T72635 | Hs.13956 | ESTs | 0.039 |
| 133468 | X03068 | Hs.73931 | major histocompatibility complex; class II; DQ beta 1 | 0.04 |
| 102636 | U67092 | | Human ataxia-telangiectasia locus protein (ATM) gene, exons 1a, 1b, 2, 3 and 4, partial cds | 0.04 |
| 129536 | M33493 | Hs.184504 | tryptase; alpha | 0.04 |
| 133599 | M64788 | Hs.75151 | RAP1; GTPase activating protein 1 | 0.041 |
| 102104 | U12139 | | Human alpha1(XI) collagen (COL11A1) gene, 5' region and exon 1 | 0.041 |
| 131340 | AA478305 | Hs.25817 | Homo sapiens chromosome 19; cosmid R27216 | 0.041 |
| 130446 | X79510 | Hs.155693 | protein tyrosine phosphatase; non-receptor type 21 | 0.042 |
| 101352 | L77701 | Hs.16297 | COX17 (yeast) homolog; cytochrome c oxidase assembly protein | 0.042 |
| 122593 | AA453310 | Hs.128749 | alpha-methylacyl-CoA racemase | 0.042 |
| 130181 | R39552 | Hs.151608 | Homo sapiens clone 23622 mRNA sequence | 0.042 |
| 134071 | Z14093 | Hs.78950 | branched chain keto acid dehydrogenase E1; alpha polypeptide (maple syrup urine disease) | 0.042 |
| 108129 | AA053252 | Hs.185848 | ESTs; Weakly similar to !! ALU SUBFAMILY J WARNING ENTRY !! [H.sapiens] | 0.043 |
| 130511 | L32137 | Hs.1584 | cartilage oligomeric matrix protein (pseudoachondroplasia; epiphyseal dysplasia 1; multiple) | 0.043 |
| 133336 | AA291456 | Hs.71190 | ESTs | 0.043 |
| 132982 | L02326 | Hs.198118 | immunoglobulin lambda-like polypeptide 2 | 0.044 |
| 131880 | AA047034 | Hs.33818 | RecQ protein-like 5 | 0.044 |
| 130540 | U35234 | Hs.159534 | protein tyrosine phosphatase; receptor type; S | 0.044 |
| 133467 | AA258595 | Hs.73931 | major histocompatibility complex; class II; DQ beta 1 | 0.044 |
| 101191 | L20688 | Hs.83656 | Rho GDP dissociation inhibitor (GDI) beta | 0.044 |
| 101860 | M95610 | Hs.37165 | collagen; type IX; alpha 2 | 0.044 |
| 102799 | U88898 | | Human endogenous retroviral H protease/integrase-derived ORF1 mRNA, complete cds, and putative envelope prot mRNA, partial cds | 0.044 |
| 107200 | D20350 | Hs.5628 | ESTs | 0.044 |
| 101166 | L14927 | Hs.2099 | lipocalin 1 (protein migrating faster than albumin; tear prealbumin) | 0.044 |
| 134289 | M54915 | Hs.81170 | pim-1 oncogene | 0.044 |
| 135329 | AA436026 | Hs.98858 | ESTs | 0.044 |
| 124950 | T03786 | Hs.151531 | protein phosphatase 3 (formerly 2B); catalytic subunit; beta isoform (calcineurin A beta) | 0.044 |
| 102919 | X12447 | Hs.183760 | aldolase A; fructose-bisphosphate | 0.044 |
| 100574 | HG2279-HT2375 | | Triosephosphate Isomerase | 0.045 |
| 131286 | AA450092 | Hs.25300 | Homo sapiens clones 24718 and 24825 mRNA sequence | 0.045 |
| 102675 | U72512 | | Human B-cell receptor associated protein (hBAP) alternatively spliced mRNA, partial 3'UTR | 0.045 |
| 131332 | R50487 | Hs.25717 | ESTs | 0.045 |
| 101634 | M57731 | Hs.75765 | GRO2 oncogene | 0.046 |
| 113118 | T47906 | Hs.220512 | ESTs | 0.046 |
| 124884 | R77276 | Hs.120911 | ESTs | 0.046 |
| 130523 | W76097 | Hs.214507 | ESTs | 0.046 |
| 110244 | H26742 | Hs.25367 | ESTs; Weakly similar to ALR [H.sapiens] | 0.046 |
| 131932 | AA454980 | Hs.25601 | chromodomain helicase DNA binding protein 3 | 0.046 |
| 132509 | H09751 | Hs.5038 | neuropathy target esterase | 0.046 |
| 133372 | AA291139 | Hs.72242 | ESTs | 0.046 |
| 100817 | HG4011-HT4804 | | Dystrophin-Associated Glycoprotein,50 Kda, Alt. Splice 2 | 0.047 |
| 106746 | AA476438 | Hs.7991 | ESTs | 0.047 |
| 135401 | L14813 | Hs.169271 | carboxyl ester lipase-like (bile salt-stimulated lipase-like) | 0.047 |
| 130479 | R44163 | Hs.12457 | Homo sapiens clone 23770 mRNA sequence | 0.047 |
| 102589 | U62015 | Hs.8667 | cysteine-rich; angiogenic inducer; 61 | 0.047 |
| 121521 | AA412165 | Hs.97358 | EST | 0.048 |
| 135340 | AA425137 | Hs.99093 | Homo sapiens chromosome 19; cosmid R28379 | 0.048 |
| 132336 | AA342422 | Hs.45073 | ESTs | 0.048 |
| 115368 | AA282133 | Hs.88960 | ESTs; Weakly similar to similar to collagen [C.elegans] | 0.048 |
| 101278 | L38487 | Hs.110849 | estrogen-related receptor alpha | 0.048 |
| 103284 | X80200 | Hs.8375 | TNF receptor-associated factor 4 | 0.048 |
| 100564 | HG2239-HT2324 | | Potassium Channel Protein (Gb:Z11585) | 0.048 |
| 133132 | Z40883 | Hs.65588 | ESTs; Weakly similar to dJ393P12.2 [H.sapiens] | 0.048 |
| 121811 | AA424535 | Hs.98416 | ESTs | 0.048 |
| 129613 | AA279481 | Hs.238831 | ESTs; Weakly similar to collagen alpha 1 (XVIII) chain [M.musculus] | 0.049 |
| 132468 | S79854 | Hs.49322 | deiodinase; iodothyronine; type III | 0.049 |
| 120111 | W95841 | Hs.136031 | ESTs | 0.049 |
| 103668 | Z83741 | Hs.248174 | H2A histone family; member M | 0.049 |
| 130386 | F10874 | Hs.234249 | mitogen-activated protein kinase 8 interacting protein 1 | 0.049 |
| 104275 | C02170 | Hs.39387 | ESTs; Weakly smlr to weak smlrity to ribosomal prot L 14 [C.elegans] | 0.049 |
| 106305 | AA436146 | Hs.12828 | ESTs | 0.05 |
| 116431 | AA609878 | Hs.55289 | ESTs; Weakly smlr to 110 KD CELL MEMBRANE GLYCOPROTEIN [H.sapiens] | 0.813 |
| 120339 | AA206465 | Hs.256470 | EST | 0.05 |
| 114427 | AA017063 | | ESTs; Highly similar to Miz-1 protein [H.sapiens] | 0.05 |
| 118821 | N79070 | Hs.94789 | ESTs | 0.05 |
| 118979 | N93798 | Hs.43666 | protein tyrosine phosphatase type IVA; member 3 | 0.05 |
| 107495 | W78776 | Hs.90375 | ESTs | 0.051 |
| 120240 | Z41732 | Hs.66049 | ESTs | 0.051 |
| 114331 | Z41309 | Hs.12400 | ESTs | 0.051 |
| 130947 | R40037 | Hs.21506 | ESTs | 0.052 |
| 129242 | W81679 | Hs.5174 | ribosomal protein S17 | 0.052 |
| 131413 | AA482390 | Hs.26510 | ESTs; Modly smlr to vacuolar prot sorting homolog r-vps33b [R.norvegicus] | 0.052 |
| 112304 | R54798 | Hs.26239 | ESTs | 0.052 |
| 101416 | M17254 | Hs.45514 | v-ets avian erythroblastosis virus E26 oncogene related | 0.052 |
| 131201 | AA426304 | Hs.24174 | ESTs | 0.052 |
| 101054 | K02405 | Hs.73933 | Human MHC class II HLA-DQ-beta mRNA (DR7 DQw2); complete cds | 0.052 |
| 101306 | L41143 | Hs.232069 | T-cell leukemia translocation altered gene | 0.053 |
| 129311 | T55087 | | yb45c08.r1 Stratagene fetal spleen (#937205) Homo sapiens cDNA | |
| | | | clone IMAGE:74126 5', mRNA sequence. | 0.053 |
| 129942 | U95301 | Hs.144442 | phospholipase A2; group X | 0.053 |
| 119210 | R93340 | Hs.92995 | ESTs | 0.053 |
| 101046 | K01160 | | Accession not listed in Genbank | 0.053 |
| 114086 | Z38266 | Hs.12770 | Homo sapiens PAC clone DJ0777O23 from 7p14-p15 | 0.053 |
| 110171 | H19964 | Hs.31709 | ESTs | 0.053 |
| 101004 | J04101 | Hs.248109 | v-ets avian erythroblastosis virus E26 oncogene homolog 1 | 0.053 |
| 129715 | N58479 | Hs.12126 | ESTs; Weakly similar to LR8 [H.sapiens] | 0.053 |
| 101581 | M34996 | Hs.198253 | major histocompatibility complex; class II; DQ alpha 1 | 0.053 |
| 113285 | T66830 | Hs.182712 | ESTs | 0.053 |
| 127537 | AA569531 | Hs.162859 | ESTs | 0.054 |
| 100813 | HG3995-HT4265 | | Cpg-Enriched Dna, Clone S19 | 0.054 |
| 101841 | M93107 | Hs.76893 | 3-hydroxybutyrate dehydrogenase (heart; mitochondrial) | 0.054 |
| 135053 | R77159 | Hs.93678 | ESTs | 0.054 |
| 101419 | M17886 | Hs.177592 | ribosomal protein; large; P1 | 0.054 |
| 119724 | W69468 | Hs.47622 | ESTs | 0.055 |
| 102673 | U72509 | | Human alternatively spliced B8 (B7) mRNA, partial sequence | 0.055 |
| 129877 | AA248589 | Hs.13094 | ESTs; Weakly similar to ORFYGR101w [S.cerevisiae] | 0.055 |
| 114788 | AA156737 | Hs.103904 | EST | 0.055 |
| 123812 | AA620607 | Hs.111591 | ESTs | 0.055 |
| 117669 | N39237 | Hs.44977 | ESTs | 0.055 |
| 123782 | AA610111 | Hs.162695 | EST | 0.055 |
| 102395 | U41767 | Hs.92208 | a disintegrin and metalloproteinase domain 15 (metargidin) | 0.055 |
| 133795 | M12529 | Hs.169401 | apolipoprotein E | 0.055 |
| 123193 | AA489228 | Hs.136956 | ESTs | 0.056 |
| 132595 | AA253369 | Hs.155742 | glyoxylate reductase/hydroxypyruvate reductase | 0.056 |
| 104161 | AA456471 | Hs.7724 | KIAA0963 protein | 0.056 |
| 115330 | AA281145 | Hs.88827 | ESTs | 0.056 |
| 112893 | T08000 | Hs.194684 | bassoon (presynaptic cytomatrix protein) | 0.056 |
| 133475 | L29217 | Hs.73987 | CDC-like kinase 3 | 0.056 |
| 128699 | K03207 | Hs.103972 | proline-rich protein BstNl subfamily 4 | 0.056 |
| 102940 | X13956 | Hs.24998 | Hu 12S RNA induced by poly(rI); poly(rC) and Newcastle disease virus | 0.056 |
| 131299 | AA431464 | Hs.25426 | ESTs; Weakly similar to unknown [H,sapiens] | 0.057 |
| 102495 | U51240 | Hs.79356 | Lysosomal-associated multispanning membrane protein-5 | 0.057 |
| 129594 | R70379 | Hs.115396 | Human germline IgD chain gene; C-region; C-delta-1 domain | 0.057 |
| 118593 | N69020 | Hs.207689 | EST | 0.057 |
| 126702 | U54602 | Hs.2785 | keratin 17 | 0.057 |
| 124386 | N27368 | Hs.212414 | sema domain; immunoglobulin domain (Ig); short basic domain; | |
| | | | secreted; (semaphorin) 3E | 0.057 |
| 130538 | M20786 | Hs.159509 | alpha-2-plasmin inhibitor | 0.057 |
| 114299 | Z40782 | Hs.22920 | similar to S68401 (cattle) glucose induced gene | 0.057 |
| 115604 | AA400378 | Hs.49391 | ESTs | 0.057 |
| 106052 | AA416947 | Hs.6382 | ESTs; Highly similar to KIAA0612 protein [H.sapiens] | 0.057 |
| 131730 | U05681 | Hs.31210 | B-cell CLL/lymphoma 3 | 0.057 |
| 131285 | AA479498 | Hs.25274 | ESTs; Modly smlr to putative seven pass transmembrane prot [H.sapiens] | 0.058 |
| 129705 | X78706 | Hs.12068 | carnitine acetyltransferase | 0.058 |
| 123175 | AA489010 | Hs.178400 | ESTs | 0.058 |
| 103592 | Z30644 | Hs.123059 | chloride channel Kb | 0.058 |
| 118196 | N59478 | Hs.48396 | ESTs; Moderately similar to tumor necrosis factor-alpha | |
| | | | -induced protein B12 [H.sapiens] | 0.058 |
| 104886 | AA053348 | Hs.144626 | growth differentiation factor 11 | 0.058 |
| 104250 | AF000575 | Hs.105928 | leukocyte immunoglobulin-like receptor, subfamily B (with TM | |
| | | | and ITIM domains); member 3 | 0.058 |
| 113301 | T67452 | Hs.13104 | EST | 0.058 |
| 110441 | H50302 | Hs.19845 | ESTs; Highly smlr to prot phosphatase 2A BR gamma subunit [H.sapiens] | 0.058 |
| 125297 | Z39215 | Hs.159409 | ESTs | 0.058 |
| 135258 | AA292423 | Hs.97272 | ESTs; Weakly similar to dJ281H8.2 [H.sapiens] | 0.058 |
| 130633 | T92363 | Hs.178703 | ESTs | 0.058 |
| 112006 | R42607 | Hs.22241 | hypothetical protein | 0.058 |
| 130805 | U12194 | Hs.170238 | sodium channel; voltage-gated; type I; beta polypeptide | 0.058 |
| 134907 | D80002 | Hs.178292 | KIAA0180 protein | 0.058 |
| 132619 | AA404565 | Hs.53447 | ESTs; Moderately similar to kinesin light chain 1 [M.musculus] | 0.058 |
| 135115 | N35489 | Hs.94653 | neurochondrin | 0.058 |
| 100531 | HG1872-HT1907 | | Major Histocompatibility Complex, Dg | 0.058 |
| 124530 | N62256 | Hs.102727 | EST | 0.058 |
| 119960 | W87533 | Hs.32699 | ESTs; Moderately similar to LIV-1 protein [H.sapiens] | 0.058 |
| 132793 | AA478999 | Hs.56966 | KIAA0906 protein | 0.058 |
| 101076 | L04270 | Hs.1116 | lymphotoxin beta receptor (TNFR superfamily; member 3 | 0.058 |
| 130655 | N92934 | Hs.17409 | cysteine-rich protein 1 (intestinal) | 0.058 |
| 134458 | AA192614 | Hs.83577 | cysteine and glycine-rich protein 3 (cardiac LIM protein) | 0.058 |
| 105904 | AA401452 | Hs.32060 | ESTs | 0.059 |
| 132878 | AA026793 | Hs.58679 | ESTs; Weakly similar to 4F2/CD98 light chain [M.musculus] | 0.059 |
| 121828 | AA425166 | Hs.98497 | ESTs | 0.059 |
| 133418 | U76366 | Hs.172727 | Treacher Collins-Franceschetti syndrome 1 | 0.059 |
| 129317 | N46244 | Hs.110373 | ESTs | 0.059 |
| 130153 | D85815 | Hs.15114 | ras homolog gene family; member D | 0.059 |
| 124403 | N31745 | Hs.102493 | ESTs | 0.059 |
| 127683 | AA668123 | Hs.134170 | ESTs | 0.059 |
| 129814 | W20070 | Hs.168625 | KIAA0979 protein | 0.059 |
| 131770 | D59682 | Hs.31833 | ESTs | 0.06 |
| 117557 | N33920 | Hs.44532 | diubiquitin | 0.06 |
| 103522 | Y10514 | | H.sapiens mRNA for CD152 protein | 0.06 |
| 120029 | W91960 | Hs.250640 | sequence-specific single-stranded-DNA-binding protein | 0.06 |
| 102135 | U15460 | Hs.41691 | activating transcription factor B | 0.06 |
| 123617 | AA609183 | Hs.181131 | ESTs | 0.06 |
| 112136 | R46100 | Hs.9739 | ESTs | 0.061 |
| 133725 | V00563 | Hs.179543 | immunoglobulin mu | 0.061 |
| 102069 | U09196 | Hs.82520 | Hu 1.1 kb mRNA upregltd in retinoic acid treated HL-60 neutrophilic cells | 0.061 |
| 106555 | AA455000 | Hs.16725 | ESTs | 0.061 |
| 123269 | AA491226 | Hs.105280 | ESTs; Weakly similar to dJ963K23.2 [H.sapiens] | 0.061 |
| 109088 | AA166837 | Hs.72620 | DKFZP434l114 protein | 0.061 |
| 129399 | AA263028 | Hs.111076 | malate dehydrogenase 2; NAD (mitochondrial) | 0.061 |
| 129375 | W79850 | Hs.11081 | ESTs; Weakly similar to HPBRll-7 protein [H.sapiens] | 0.061 |
| 135271 | AA397763 | Hs.97562 | ESTs | 0.061 |
| 132958 | W90398 | Hs.6147 | KIAA1075 protein | 0.061 |
| 129364 | AA477106 | Hs.110757 | DNA segment on chromosome 21 (unique) 2056 expressed sequence | 0.061 |
| 123427 | AA598548 | Hs.112471 | ESTs | 0.061 |
| 105236 | AA219179 | Hs.19105 | translocase of inner mitochondrial membrane 17 (yeast) homolog B | 0.061 |
| 101012 | J04444 | Hs.697 | cytochrome c-1 | 0.062 |
| 134791 | L18983 | Hs.89655 | protein tyrosine phosphatase; receptor type; N | 0.062 |
| 133700 | K01396 | Hs.75621 | protease inhibitor 1 (anti-elastase); alpha-1-antitrypsin | 0.062 |
| 123887 | AA621065 | Hs.112943 | ESTs | 0.062 |
| 129363 | H05704 | Hs.110746 | H sapiens HCR (a-helix coiled-coil rod homologue) mRNA; complete cds | 0.062 |
| 105719 | AA291644 | Hs.36793 | ESTs | 0.062 |
| 124226 | H62396 | Hs.190266 | ESTs | 0.062 |
| 117437 | N27645 | | yw5e3.s1 Weizmann Olfactory Epithelium H sapiens cDNA clone | |
| | | | IMAGE:255676 3' smlr to contains L1.t3 L1 repetitive element ;, mRNA seq | 0.062 |
| 132741 | AA394133 | Hs.55898 | ESTs; Highly similar to OASIS protein [M.musculus] | 0.062 |
| 134437 | M26041 | Hs.198253 | major histocompatibility complex; class II; DQ alpha 1 | 0.062 |
| 107664 | AA010594 | Hs.5326 | ESTs; Moderately similar to pim-1 protein [H.sapiens] | 0.062 |
| 120844 | AA349417 | Hs.96917 | ESTs | 0.062 |
| 101574 | M34182 | Hs.158029 | protein kinase; cAMP-dependent; catalytic; gamma | 0.062 |
| 131219 | C00476 | Hs.24395 | small inducible cytokine subfamily B (Cys-X-Cys); member 14 (BRAK) | 0.062 |
| 103495 | Y09022 | Hs.153591 | Not56 (D.melanogaster)-like protein | 0.062 |
| 129607 | AA404594 | Hs.11607 | ESTs | 0.062 |
| 106467 | AA450040 | Hs.154162 | ADP-ribosylation factor-like 2 | 0.062 |
| 128841 | T16358 | Hs.106443 | ESTs | 0.062 |
| 100515 | HG1723-HT1729 | | Macrophage Scavenger Receptor, Alt. Splice 2 | 0.062 |
| 119332 | T54095 | | ESTs; Weakly similar to !! ALU SUBFAMILY J WARNING ENTRY !! [H.sapiens] | 0.062 |
| 134516 | AA171939 | Hs.23413 | ESTs | 0.062 |
| 135012 | X73608 | Hs.93029 | sparc/osteonectin; cwcv and kazal-like domains proteoglycan (testican) | 0.063 |
| 103575 | Z26256 | | H.sapiens isoform 1 gene for L-type calcium channel, exon 1 | 0.063 |
| 115514 | AA297739 | Hs.55609 | ESTs; Weakly similar to ISOLEUCYL-TRNA SYNTHETASE; | |
| | | | CYTOPLASMIC [H.sapiens] | 0.063 |
| 103996 | AA321355 | | EST2393 Bone marrow Homo sapiens cDNA 5' end, mRNA sequence | 0.063 |
| 110505 | H55992 | Hs.20495 | DKFZP434F011 protein | 0.063 |
| 133912 | X62744 | Hs.77522 | major histocompatibility complex; class II; DM alpha | 0.063 |
| 129581 | M33600 | Hs.180255 | major histocompatibility complex; class II; DR beta 1 | 0.063 |
| 130139 | R38280 | Hs.150922 | BCS1 (yeast homolog)-like | 0.064 |
| 105817 | AA397825 | Hs.5307 | synaptopodin | 0.064 |
| 134658 | AA410617 | Hs.178009 | ESTs | 0.064 |
| 100306 | D50495 | Hs.80598 | transcription elongation factor A (SII); 2 | 0.064 |
| 100277 | D42053 | Hs.75890 | site-1 protease (subtilisin-like; sterol-regulated; cleaves sterol regulatory | |
| | | | element binding proteins) | 0.064 |
| 133116 | D61259 | Hs.6529 | ESTs | 0.064 |
| 134909 | AA521488 | Hs.90998 | KIAA0128 protein | 0.064 |
| 130319 | X74794 | Hs.154443 | minichromosome maintenance deficient (S. cerevisiae) 4 | 0.064 |
| 132057 | AA102489 | Hs.173484 | ESTs | 0.064 |
| 108334 | AA070473 | | zm7c8.s1 Stratagene neuroepithelium (#937231) Homo sapiens cDNA | |
| | | | clone IMAGE:5399 3', mRNA sequence | 0.064 |
| 129763 | F10815 | Hs.12373 | KIAA0422 protein | 0.064 |
| 135112 | T67464 | Hs.94617 | ESTs; Weakly similar to predicted using Genefinder [C.elegans] | 0.064 |
| 122269 | AA436856 | Hs.98910 | ESTs | 0.064 |
| 133082 | AA457129 | Hs.6455 | RuvB (E coli homolog)-like 2 | 0.064 |
| 113213 | T58607 | | ya94a02.s1 Stratagene placenta (#937225) Homo sapiens cDNA clone | |
| | | | IMAGE:69290 3', mRNA sequence. | 0.065 |
| 106228 | AA429290 | Hs.17719 | ESTs | 0.065 |
| 130192 | Y12661 | Hs.171014 | VGF nerve growth factor inducible | 0.065 |
| 104894 | AA054087 | Hs.18858 | phospholipase A2; group IVC (cytosolic; calcium-independent) | 0.065 |
| 103508 | Y10141 | | H.sapiens DAT1 gene, partial, VNTR | 0.065 |
| 128474 | U40671 | Hs.100299 | ligase III; DNA; ATP-dependent | 0.065 |
| 134012 | AA417821 | Hs.237924 | ESTs; Highly similar to CGI-69 protein [H.sapiens] | 0.065 |
| 134536 | AA457735 | Hs.850 | IMP (inosine monophosphate) dehydrogenase 1 | 0.065 |
| 111714 | R23146 | Hs.23466 | ESTs | 0.065 |
| 110521 | H57060 | Hs.108268 | ESTs | 0.065 |
| 103282 | X80198 | Hs.77628 | steroidogenic acute regulatory protein related | 0.065 |
| 113921 | W80730 | Hs.28355 | ESTs | 0.065 |
| 129331 | N93465 | Hs.110453 | ESTs; Highly similar to CGI-38 protein [H.sapiens] | 0.065 |
| 111316 | N74597 | Hs.180535 | ESTs; Weakly similar to mitogen inducible gene mig-2 [H.sapiens] | 0.065 |
| 135138 | AA036794 | Hs.95196 | ESTs; Weakly similar to T20B12.3 [C.elegans] | 0.065 |
| 107289 | T10792 | Hs.172098 | ESTs | 0.065 |
| 121405 | AA406083 | Hs.98007 | ESTs | 0.065 |
| 124965 | T16275 | Hs.106359 | ESTs | 0.065 |
| 106595 | AA456933 | Hs.174481 | ESTs | 0.066 |
| 100106 | AF015910 | | Homo sapiens unknown protein mRNA, partial cds | 0.066 |
| 134715 | AA282757 | Hs.89040 | prepronociceptin | 0.066 |
| 135367 | AA480109 | Hs.9963 | TYRO protein tyrosine kinase binding protein | 0.066 |
| 111533 | R08548 | Hs.251651 | EST | 0.066 |
| 128509 | R53109 | Hs.247362 | dimethylarginine dimethylaminohydrolase 2 | 0.066 |
| 101030 | J05037 | Hs.76751 | serine dehydratase | 0.066 |
| 102753 | U80226 | | Human gamma-aminobutyric acid transaminase mRNA, partial cds | 0.067 |
| 126991 | R31652 | Hs.821 | biglycan | 0.067 |
| 109583 | F02322 | Hs.26135 | ESTs | 0.067 |
| 119241 | T12559 | Hs.221382 | ESTs | 0.067 |
| 130569 | AA156597 | Hs.256441 | EST; Moderately similar to CGl-136 protein [H.sapiens] | 0.067 |
| 112926 | T10316 | Hs.4302 | ESTs | 0.067 |
| 120495 | AA256073 | Hs.190626 | ESTs | 0.067 |
| 130931 | AA278412 | Hs.21346 | ESTs; Weakly similar to F42C5.7 gene product [C.elegans] | 0.067 |
| 129982 | M87789 | Hs.140 | immunoglobulin gamma 3 (Gm marker) | 0.067 |
| 133832 | H03387 | Hs.241305 | estrogen-responsive B box protein | 0.067 |
| 110697 | H93721 | Hs.20798 | ESTs | 0.067 |
| 121183 | AA400138 | Hs.97703 | ESTs | 0.067 |
| 130953 | U12707 | Hs.2157 | Wiskott-Aldrich syndrome (ecezema-thrombocytopenia) | 0.067 |
| 102218 | U24183 | Hs.75160 | phosphofructokinase; muscle | 0.067 |
| 114181 | Z39079 | Hs.8021 | KIAA1058 protein | 0.067 |
| 116581 | D51287 | Hs.82148 | ribosomal protein S12 | 0.067 |
| 132498 | T87708 | Hs.50098 | ESTs | 0.068 |
| 103788 | AA096014 | Hs.9527 | ESTs; Highly similar to HSPC013 [H.sapiens] | 0.068 |
| 102459 | U48936 | | Human amiloride-sensitive epithelial sodium channel gamma subunit mRNA, | |
| | | | 5' end, partial cds | 0.068 |
| 100373 | D79999 | Hs.77225 | ADP-ribosyltransferase (NAD+; poly (ADP-ribose) polymerase)-like 1 | 0.068 |
| 132717 | AA203321 | Hs.151696 | DKFZP727G051 protein | 0.068 |
| 128863 | D87462 | Hs.106674 | BRCA1 associated protein-1 (ubiquitin carboxy-terminal hydrolase) | 0.068 |
| 115193 | AA262029 | Hs.88218 | ESTs | 0.068 |
| 124558 | N66046 | Hs.141605 | ESTs | 0.069 |
| 117225 | N20392 | Hs.42846 | ESTs | 0.069 |
| 110665 | H83380 | Hs.32757 | ESTs | 0.069 |
| 132905 | U70663 | Hs.182965 | Kruppel-like factor 4 (gut) | 0.069 |
| 105778 | AA348910 | Hs.153299 | DOM-3(C.elegans) homolog Z | 0.069 |
| 134770 | R72079 | Hs.89575 | CD79B antigen (immunoglobulin-associated beta) | 0.069 |
| 123097 | AA485869 | Hs.105671 | ESTs | 0.069 |
| 100750 | HG3523-HT4899 | | Proto-Oncogene C-Myc, Alt. Splice 3, Orf 114 | 0.069 |
| 125091 | T91518 | | ye20f05.s1 Stratagene lung (#937210) H sapiens cDNA clone IMAGE: | |
| | | | 3' similar to contains Alu repetitive element;contains MER12 repetitive element; | |
| | | | mRNA sequence. | 0.069 |
| 100756 | HG3565-HT3768 | | Zinc Finger Protein (Gb:M88357) | 0.069 |
| 113483 | T87768 | Hs.16439 | ESTs | 0.069 |
| 101119 | L09708 | Hs.2253 | complement component 2 | 0.069 |
| 102286 | U31628 | Hs.12503 | interleukin 15 receptor; alpha | 0.07 |
| 135349 | D83174 | Hs.9930 | collagen-binding protein 2 (colligen 2) | 0.07 |
| 100991 | J03764 | Hs.82085 | plasminogen activator inhibitor; type I | 0.07 |
| 133675 | AA443720 | Hs.7551 | ESTs; Weakly similar to T25G3.1 [C.elegans] | 0.07 |
| 105422 | AA251014 | Hs.12210 | ESTs | 0.07 |
| 102932 | X13334 | Hs.75627 | CD14 antigen | 0.07 |
| 119147 | R58878 | Hs.65739 | ESTs | 0.07 |
| 104900 | AA055048 | Hs.180481 | ESTs; Weakly similar to ACROSIN PRECURSOR [H.sapiens] | 0.07 |
| 133185 | AA481404 | Hs.6686 | ESTs | 0.07 |
| 115496 | AA290674 | Hs.71819 | eukaryotic translation initiation factor 4E binding protein 1 | 0.07 |
| 121005 | AA398332 | Hs.97613 | ESTs | 0.07 |
| 124869 | R69088 | Hs.28728 | ESTs; Weakly similar to F55A12.9 [C.elegans] | 0.071 |
| 129154 | N23673 | Hs.108969 | mannosidase; alpha; class 2B; member 1 | 0.071 |
| 112161 | R48295 | | ESTs; Wkly smlr to !! ALU SUBFAMILY J WARNING ENTRY !! [H.sapiens] | 0.071 |
| 125251 | W87486 | Hs.141464 | ESTs | 0.071 |
| 134298 | J00116 | Hs.81343 | collagen; type II; alpha 1 (primary osteoarthritis; spondyloepiphyseal | |
| | | | dysplasia; congenital) | 0.071 |
| 119745 | W70264 | Hs.58093 | ESTs | 0.071 |
| 131306 | AA232686 | Hs.25489 | ESTs | 0.071 |
| 107776 | AA018820 | Hs.221147 | ESTs | 0.071 |
| 134271 | AA199630 | Hs.184456 | ESTs; Wkly smlr to !! ALU SUBFAMILY SX WARNING ENTRY !! [H.sapiens] | 0.071 |
| 101798 | M85220 | | Accession not listed in Genbank | 0.071 |
| 135402 | S76942 | Hs.99922 | dopamine receptor D4 | 0.071 |
| 118742 | N74052 | Hs.50424 | EST | 0.071 |
| 131867 | N64656 | Hs.3353 | Homo sapiens clone 24940 mRMA sequence | 0.071 |
| 102923 | X12517 | Hs.1063 | small nuclear ribonucleoprotein polypeptide C | 0.072 |
| 100775 | HG371-HT26388 | | Mucin 1, Epithelial, Alt. Splice 9 | 0.072 |
| 111020 | N54361 | Hs.185726 | ESTs | 0.072 |
| 134224 | X80822 | Hs.163593 | ribosomal protein L18a | 0.072 |
| 124059 | F13673 | Hs.99769 | ESTs | 0.072 |
| 133972 | AA160743 | Hs.78019 | Homo sapiens clone 24432 mRNA sequence | 0.072 |
| 129681 | AA436009 | Hs.178186 | ESTs; Weakly similar to WASP-family protein [H.sapiens] | 0.072 |
| 103065 | X58399 | Hs.81221 | Human L2-9 transcript of unrearranged immunoglobulin V(H)5 pseudogene | 0.072 |
| 124966 | T19271 | Hs.155560 | calnexin | 0.072 |
| 112270 | R53021 | Hs.203358 | ESTs | 0.072 |
| 116704 | F10183 | Hs.66140 | EST | 0.072 |
| 129890 | M13699 | Hs.111461 | ceruloplasmin (ferroxidase) | 0.072 |
| 127345 | AA972008 | Hs.166253 | ESTs; Highly similar to KIAA0476 protein [H.sapiens] | 0.072 |
| 112436 | R63090 | Hs.28391 | ESTs | 0.072 |
| 114531 | AA053033 | Hs.203330 | ESTs | 0.072 |
| 135122 | H99080 | Hs.94814 | ESTs | 0.072 |
| 103934 | AA281338 | Hs.134200 | Homo sapiens mRNA; cDNA DKFZp564C186 (from clone DKFZp564C186) | 0.072 |
| 109363 | AA215369 | Hs.185764 | ESTs; Weakly similar to hypothetical protein [H.sapiens] | 0.072 |
| 112647 | R83329 | Hs.33403 | ESTs | 0.073 |
| 127083 | Z44079 | Hs.91608 | otoferlin | 0.073 |
| 133027 | AA402624 | Hs.63236 | synuclein; gamma (breast cancer-specific protein 1) | 0.073 |
| 122086 | AA432121 | Hs.250986 | EST | 0.073 |
| 110405 | H47542 | Hs.33962 | ESTs | 0.073 |
| 128697 | AB002344 | Hs.103915 | KIAA0346 protein | 0.073 |
| 112221 | R50380 | Hs.25670 | ESTs | 0.073 |
| 100478 | HG1067-HT1067 | | Mucin (Gb:M22406) | 0.073 |
| 115598 | AA400129 | Hs.65735 | ESTs | 0.073 |
| 132491 | AA227137 | Hs.4984 | KIAA0828 protein | 0.073 |
| 101655 | M60299 | | Human alpha-1 collagen type II gene, exons 1,2 and 3 | 0.073 |
| 106018 | AA411887 | Hs.34737 | ESTs | 0.073 |
| 129683 | W05348 | Hs.158196 | DKFZP434B103 protein | 0.073 |
| 134137 | F10045 | Hs.79347 | KIAA0211 gene product | 0.073 |
| 114008 | W89128 | Hs.19872 | ESTs | 0.073 |
| 107653 | AA010210 | Hs.47041 | ESTs | 0.073 |
| 104798 | AA029462 | Hs.17235 | ESTs | 0.073 |
| 134082 | L16991 | Hs.79006 | deoxythymidylate kinase | 0.073 |
| 119180 | R80413 | Hs.92520 | ESTs | 0.073 |
| 107741 | AA016982 | Hs.64341 | ESTs | 0.073 |
| 133683 | AA335223 | Hs.75558 | pepsinogen 5; group I (pepsinogen A) | 0.073 |
| 111694 | R22035 | Hs.23331 | ESTs | 0.073 |
| 120764 | AA338729 | Hs.133096 | ESTs | 0.073 |
| 119389 | T88826 | Hs.90973 | ESTs | 0.074 |
| 100929 | HG688-HT688 | | Major Histocompatibility Complex, Class li, Dr Beta 2 (Gb:X65561) | 0.074 |
| 119388 | T88798 | | plasminogen activator inhibitor, type I | 0.074 |
| 133019 | AF009674 | Hs.184434 | axin | 0.074 |
| 105185 | AA191495 | Hs.189937 | ESTs | 0.074 |
| 133413 | S72043 | Hs.73133 | metallothionein 3 (growth inhibitory factor (neurotrophic)) | 0.074 |
| 101017 | J04599 | Hs.821 | biglycan | 0.074 |
| 132865 | K02765 | Hs.251972 | complement component 3 | 0.074 |
| 110882 | N36001 | Hs.17348 | ESTs; Wkly smlr to !! ALU SUBFAMILY SQ WARNING ENTRY !! [H.sapiens] | 0.074 |
| 129197 | T90303 | Hs.109308 | ESTs; Wkly smlr to leucine-rich glioma-inactivated prot precursor [H.sapiens] | 0.074 |
| 101184 | L19871 | Hs.460 | activating transcription factor 3 | 0.075 |
| 134910 | AA431320 | Hs.9100 | ESTs | 0.075 |
| 119411 | T96621 | Hs.203656 | EST | 0.075 |
| 102000 | U01824 | Hs.380 | solute carrier family 1 (glial high affinity glutamate transporter); member 2 | 0.075 |
| 114691 | AA121893 | Hs.103779 | ESTs; Weakly similar to envelope protein [H.sapiens] | 0.075 |
| 134179 | U53204 | Hs.79706 | pleclin 1; intermediate filament binding protein; 500kD | 0.075 |
| 134503 | U34880 | Hs.84183 | diptheria toxin resistance protein required for diphthamide | |
| | | | biosynthesis (Saccharomyces)-like 1 | 0.075 |
| 129719 | N66396 | Hs.167766 | ESTs; Moderately similar to Pro-a2(XI) [H.sapiens] | 0.075 |
| 113916 | W80464 | Hs.31928 | ESTs; Wkly smlr to alternatively spliced product using exon 13A [H.sapiens] | 0.075 |
| 113897 | W73926 | Hs.4947 | ESTs | 0.075 |
| 129697 | R00841 | Hs.172069 | DKFZP434C212 protein | 0.075 |
| 112078 | R44155 | Hs.112218 | ESTs | 0.075 |
| 121980 | AA429886 | Hs.110407 | ESTs; Weakly similar to coded for by C. elegans cDNA yk173c12.5 [C.elegans] | 0.075 |
| 100898 | HG4638-HT5050 | | Spliceosomal Protein Sap 49 | 0.075 |
| 121626 | AA416974 | Hs.98174 | ESTs | 0.075 |
| 133670 | AA243416 | Hs.75470 | hypothetical protein; expressed in osteoblast | 0.075 |
| 131879 | AA017161 | Hs.33792 | ESTs | 0.075 |
| 100254 | D38037 | Hs.77643 | FK506-binding protein 1B (12.6 kD) | 0.075 |
| 133194 | AA291726 | Hs.67201 | ESTs | 0.075 |
| 106081 | AA418394 | Hs.25354 | ESTs | 0.075 |
| 115544 | AA351433 | Hs.66187 | Homo sapiens clone 23700 mRNA sequence | 0.076 |
| 119955 | W87460 | Hs.58989 | ESTs | 0.076 |
| 104407 | H61361 | Hs.102171 | immunoglobulin superfamily containing leucine-rich repeat | 0.076 |
| 135019 | X58431 | Hs.98428 | Human Hox2.2 gene for a homeobox protein | 0.076 |
| 114815 | AA161488 | Hs.103931 | DKFZP434B0335 protein | 0.076 |
| 119471 | W31352 | Hs.55445 | ESTs | 0.076 |
| 117788 | N48292 | Hs.46849 | ESTs | 0.076 |
| 119406 | T95064 | Hs.193771 | EST | 0.076 |
| 130777 | R61742 | Hs.256554 | ESTs | 0.076 |
| 130494 | L13197 | Hs.75874 | pregnancy-associated plasma protein A | 0.076 |
| 104107 | AA424111 | Hs.12598 | T-cell lymphoma invasion and metastasis 2 | 0.076 |
| 121483 | AA411981 | Hs.25274 | ESTs; Modly smlr to putative seven pass transmembrane prot [H.sapiens] | 0.076 |
| 104451 | M13299 | Hs.102119 | blue cone pigment | 0.076 |
| 118027 | N52770 | Hs.75968 | thymosin; beta 4; X chromosome | 0.076 |
| 109419 | AA227560 | Hs.86987 | receptor-interacting serine-threonine kinase 3 | 0.076 |
| 115783 | AA424487 | Hs.72289 | ESTs; Weakly similar to LIV-1 protein [H.sapiens] | 0.076 |
| 110585 | H62223 | Hs.133526 | ESTs; Wkly smlr to !!!ALU SUBFAMILY SB1 WARNING ENTRY !!![H.sapiens] | 0.076 |
| 123165 | AA488863 | Hs.105216 | ESTs; Weakly smlr to !!ALU SUBFAMILY J WARNING ENTRY !! [H.sapiens] | 0.077 |
| 103966 | AA303166 | Hs.127270 | ESTs | 0.077 |
| 109549 | F01528 | Hs.21192 | Homo sapiens clone 25155 mRNA sequence | 0.077 |
| 106730 | AA465520 | Hs.22313 | ESTs | 0.077 |
| 120310 | AA193676 | Hs.118926 | DKFZP586K0919 protein | 0.077 |
| 104078 | AA402801 | Hs.222010 | ESTs | 0.077 |
| 117624 | N35978 | Hs.82364 | ESTs | 0.077 |
| 112421 | R62441 | Hs.23127 | ESTs | 0.077 |
| 106958 | AA497026 | Hs.22059 | ESTs | 0.077 |
| 129984 | W92811 | Hs.183927 | ESTs; Weakly similar to !! ALU SUBFAMILY J WARNING ENTRY !! [H.sapiens] | 0.077 |
| 122044 | AA431456 | Hs.98736 | EST | 0.077 |
| 123280 | AA491285 | Hs.175144 | ESTs | 0.077 |
| 115710 | AA412535 | Hs.55235 | sphingomyelin phosphodiesterase 2; neutra | |
| | | | I membrane (neutral sphingomyelinase) | 0.077 |
| 134129 | D87444 | Hs.79305 | KIAA0255 gene product | 0.077 |
| 129321 | AA224502 | Hs.206501 | Homo sapiens clone 643 unknown mRNA; complete sequence | 0.078 |
| 130513 | AA460257 | Hs.15866 | ESTs | 0.078 |
| 100996 | J03909 | Hs.14623 | Interferon; gamma-inducible protein 30 | 0.078 |
| 128358 | AI095718 | Hs.135015 | ESTs | 0.078 |
| 128544 | R59352 | Hs.119273 | KIAA0296 gene product | 0.078 |
| 106040 | AA412681 | Hs.125139 | ESTs | 0.078 |
| 106495 | AA452113 | Hs.32454 | ESTs; Moderately similar to KIAA0544 protein [H.sapiens] | 0.078 |
| 131833 | R40899 | Hs.32973 | glycine receptor, beta | 0.078 |
| 119219 | R97176 | Hs.110783 | ESTs | 0.078 |
| 135415 | X60655 | Hs.99967 | even-skipped homeo box 1 (homolog of Drosophila) | 0.078 |
| 109457 | AA232646 | Hs.68061 | ESTs; Weakly similar to sphingosine kinase [M.musculus] | 0.078 |
| 117137 | H96670 | Hs.42221 | ESTs | 0.078 |
| 107094 | AA609614 | Hs.5241 | ESTs | 0.078 |
| 130165 | T90529 | Hs.251613 | EST | 0.078 |
| 124072 | H05252 | Hs.101637 | EST; Weakly similar to hypothetical protein [H.sapiens] | 0.078 |
| 126151 | AA324743 | Hs.40808 | ESTs | 0.078 |
| 119035 | R01779 | Hs.7740 | ESTs | 0.078 |
| 110157 | H18987 | Hs.169731 | ESTs | 0.078 |
| 128515 | AA149044 | Hs.10086 | ESTs; Highly similar to HYPOTHETICAL PROTEIN KIAA0195 [H.sapiens] | 0.078 |
| 133069 | U94836 | Hs.6430 | protein with polyglutamine repeat | 0.078 |
| 112209 | R49644 | Hs.24865 | ESTs | 0.078 |
| 133361 | R28279 | Hs.71848 | Human clone 23548 mRNA sequence | 0.078 |
| 134714 | U89922 | Hs.890 | lymphotoxin beta (TNF superfamily; member 3) | 0.078 |
| 129905 | T86796 | Hs.132875 | ESTs; Weakly similar to predicted using Genefinder [C.elegans] | 0.079 |
| 120421 | AA236166 | Hs.132957 | ESTs; Weakly similar to chondromodulin-I precursor [H.sapiens] | 0.079 |
| 100885 | HG4490-HT4876 | | Proline-Rich Protein Prb4, Allele | 0.079 |
| 102789 | U86759 | Hs.158336 | netrin 2 (chicken)-like | 0.079 |
| 120139 | Z39273 | Hs.77876 | Human DNA from chromosome 19-specific cosmid R30923; genomic sequence | 0.079 |
| 135238 | U76343 | Hs.96970 | Human liver GABA transport protein mRNA; 3' end | 0.079 |
| 129618 | N54845 | Hs.173030 | ESTs | 0.079 |
| 132960 | AA609742 | Hs.6150 | KIAA0521 protein | 0.079 |
| 108751 | AA127063 | Hs.203717 | ESTs | 0.079 |
| 134060 | D42039 | Hs.78871 | KIAA0081 protein | 0.079 |
| 111338 | N79778 | Hs.35094 | extracellular matrix protein 2; female organ and adipocyte specific | 0.079 |
| 112345 | R56880 | Hs.26563 | ESTs | 0.079 |
| 126456 | W00881 | | za56d02.r1 Soares fetal liver spleen 1NFLS Homo sapiens cDNA clone | |
| | | | IMAGE:296547 5', mRNA sequence. | 0.079 |
| 128937 | Z39939 | Hs.10726 | ESTs | 0.079 |
| 103485 | Y08409 | Hs.248415 | thyroid hormone responsive SPOT14 (rat) homolog | 0.079 |
| 111202 | N68280 | Hs.107922 | ESTs | 0.079 |
| 132625 | AA429890 | Hs.166066 | cisplatin resistance associated | 0.079 |
| 103434 | X98085 | Hs.54433 | tenascin R (restrictin; janusin) | 0.079 |
| 102616 | U65581 | Hs.159191 | ribosomal protein L3-like | 0.079 |
| 102667 | U70867 | Hs.83974 | solute carrier family 21 (prostaglandin transporter); member 2 | 0.079 |
| 111422 | R01127 | Hs.19104 | ESTs | 0.079 |
| 101411 | M16938 | Hs.820 | homeo box C8 | 0.08 |
| 113267 | T65058 | Hs.12725 | ESTs; Weakly similar to !! ALU SUBFAMILY J WARNING ENTRY !! [H.sapiens] | 0.08 |
| 103559 | Z19585 | Hs.75774 | thrombospondin 4 | 0.08 |
| 131588 | AA258613 | Hs.29189 | KIAA1021 protein | 0.08 |
| 107821 | AA020991 | Hs.172856 | ESTs | 0.08 |
| 134278 | H82839 | Hs.81001 | ESTs; Weakly similar to DY3.6 [C.elegans] | 0.08 |
| 120893 | AA369800 | Hs.97058 | EST; Highly similar to CMP-N-acetylneuraminic acid hydroxylase [H.sapiens] | 0.08 |
| 108786 | AA128999 | | zo8f12.s1 Stratagene neuroepithelium NT2RAMI 937234 Homo sapiens | |
| | | | cDNA clone IMAGE:567119 3', mRNA sequence | 0.08 |
| 106890 | AA489245 | Hs.88500 | KIAA1066 protein; JSAP1 homolog (mouse); JIP3 homolog (mouse) | 0.08 |
| 119760 | W72267 | Hs.58219 | ESTs | 0.08 |
| 132999 | Y00787 | Hs.624 | interleukin 8 | 0.08 |
| 129156 | AA028195 | Hs.108973 | dolichyl-phosphate mannosyltransferase polypeptide 2; regulatory subunit | 0.08 |
| 121171 | AA400008 | Hs.161814 | ESTs | 0.08 |
| 103864 | AA207264 | Hs.181077 | ESTs; Weakly similar to Miller-Dieker lissencephaly gene [H.sapiens] | 0.08 |
| 128591 | AA255537 | Hs.102057 | ESTs; Weakly similar to O-linked GlcNAc transferase [H.sapiens] | 0.08 |
| 122172 | AA435753 | Hs.161854 | EST | 0.08 |
| 112802 | R97647 | Hs.174855 | EST | 0.08 |
| 107723 | AA015967 | Hs.60680 | EST | 0.08 |
| 113011 | T23737 | Hs.1600 | chaperonin containing TCP1; subunit 5 (epsilon) | 0.081 |
| 131279 | AA089853 | Hs.25197 | STIP1 homology and U-Box containing protein 1 | 0.081 |
| 103190 | X70083 | Hs.58414 | filamin C; gamma (actin-binding protein-280) | 0.081 |
| 103956 | AA292411 | Hs.233348 | ESTs | 0.081 |
| 112706 | R89828 | Hs.138493 | ESTs | 0.081 |
| 126126 | M85370 | | EST01884 Fetal brain, Stratagene (cat#936206) Homo sapiens cDNA | |
| | | | clone HFBCH10, mRNA sequence. | 0.081 |
| 130094 | H43286 | Hs.167017 | gamma-aminobutyric acid (GABA) B receptor; 1 | 0.081 |
| 100800 | HG3945-HT4215 | | Phospholipid Transfer Protein | 0.081 |
| 108675 | AA115240 | Hs.61816 | ESTs | 0.081 |
| 129420 | AA234259 | Hs.99816 | ESTs | 0.081 |
| 129666 | M77349 | Hs.118787 | transforming growth factor; beta-induced; 68kD | 0.081 |
| 101645 | M59807 | Hs.943 | natural killer cell transcript 4 | 0.081 |
| 130536 | T17045 | Hs.159492 | spastic ataxia of Charlevoix-Saguenay (sacsin) | 0.081 |
| 107732 | AA016181 | Hs.59752 | ESTs | 0.081 |
| 123071 | AA482593 | Hs.104285 | ESTs | 0.081 |
| 113537 | T90457 | Hs.191293 | ESTs | 0.081 |
| 101250 | L34060 | Hs.79133 | cadherin 8 | 0.081 |
| 122521 | AA449433 | Hs.149227 | ESTs; Weakly to PROLINE-RICH PROTEIN MP-3 [M.musculus] | 0.081 |
| 133914 | N32811 | Hs.77542 | ESTs | 0.081 |
| 102038 | U05659 | Hs.477 | hydroxysteroid (17-beta) dehydrogenase 3 | 0.081 |
| 110336 | H40338 | Hs.174094 | ESTs; Weakly similar to !! ALU SUBFAMILY J WARNING ENTRY !! [H.sapiens] | 0.081 |
| 118637 | N70274 | Hs.49822 | ESTs | 0.081 |
| 117966 | N51589 | Hs.94012 | ESTs | 0.082 |
| 104424 | H87671 | Hs.182320 | ESTs; Weakly similar to Mouse 19.5 mRNA; complete cds [M.musculus] | 0.082 |
| 100361 | D78361 | Hs.125078 | Human mRNA for ornithine decarboxylase antizyme; ORF 1 an ORF 2 | 0.082 |
| 112974 | T17291 | Hs.101174 | microtubule-associated protein tau | 0.082 |
| 132832 | D63482 | Hs.57734 | KIAA0148 gene product | 0.082 |
| 132039 | Z39489 | Hs.3781 | Homo sapiens BAC clone RG118D07 from 7q31 | 0.082 |
| 113272 | T65383 | Hs.12807 | ESTs | 0.082 |
| 104924 | AA058532 | Hs.28774 | ESTs | 0.082 |
| 111061 | N56054 | Hs.36859 | ESTs | 0.082 |
| 129269 | R45977 | Hs.163593 | ribosomal protein L18a | 0.082 |
| 102453 | U48437 | Hs.74565 | amyloid beta (A4) precursor-like protein 1 | 0.082 |
| 126204 | AI080388 | Hs.134296 | ESTs | 0.082 |
| 116615 | D80666 | Hs.45203 | ESTs | 0.082 |
| 128856 | AA219552 | Hs.204144 | ESTs; Modly smlr to tumor necrosis factor-alpha-induced prot B12 [H.sapiens] | 0.082 |
| 112776 | R95850 | Hs.34494 | ESTs | 0.082 |
| 105494 | AA256273 | Hs.29288 | Homo sapiens mRNA; cDNA DKFZp434P174 (from clone DKFZp434P174) | 0.082 |
| 117000 | H84718 | Hs.112236 | ESTs; Weakly similar to repressor protein [H.sapiens] | 0.082 |
| 112656 | R85260 | Hs.133151 | transient receptor potential channel 7 | 0.082 |
| 128963 | J03890 | Hs.1074 | surfactant pulmonary-associated protein C | 0.083 |
| 116957 | H79292 | Hs.39960 | ESTs | 0.083 |
| 101057 | K03430 | | Human complement C1q B-chain gene, exon A+1 | 0.083 |
| 121948 | AA429452 | Hs.98582 | ESTs | 0.083 |
| 130822 | M80647 | Hs.2001 | thromboxane A synthase 1(platelet; cytochrome P450; subfamily V) | 0.083 |
| 122743 | AA458674 | Hs.99478 | EST | 0.083 |
| 114569 | AA063316 | | zm2d1.s1 Stratagene corneal stroma (#937222) Homo sapiens cDNA clone | |
| | | | IMAGE:512947 3' similar to TR:E198281 E198281 THIOREDOXIN | |
| | | | REDUCTASE ;contains Alu repetitive element;, mRNA sequence | 0.083 |
| 132270 | U70671 | Hs.43509 | ataxin 2 related protein | 0.083 |
| 108126 | AA052951 | Hs.47413 | ESTs | 0.083 |
| 102880 | X04325 | Hs.2679 | gap junction protein; beta 1; 32kD (connexin 32; Charcot-Marie-Tooth | |
| | | | neuropathy; X-linked) | 0.083 |
| 115365 | AA282089 | Hs.88599 | ESTs | 0.083 |
| 114529 | AA052980 | Hs.206704 | ESTs | 0.083 |
| 135017 | AA249586 | Hs.9315 | ESTs; Weakly similar to NEURONAL OLFACTOMEDIN-RELATED | |
| | | | ER LOCALIZED PROTEIN [H.sapiens] | 0.083 |
| 123776 | AA610071 | Hs.112813 | ESTs | 0.083 |
| 114454 | AA021091 | Hs.226208 | ESTs | 0.083 |
| 101246 | L33799 | Hs.202097 | procollagen C-endopeptidase enhancer | 0.083 |
| 107366 | U78310 | Hs.13501 | pescadillo (zebrafish) homolog 1; containing BRCT domain | 0.083 |
| 132779 | T89601 | Hs.95497 | ESTs; Weakly similar to GLUCOSE TRANSPORTER TYPE 5; | |
| | | | SMALL INTESTINE [H.sapiens] | 0.083 |
| 129709 | AA112209 | Hs.1209 | acyl-Coenzyme A dehydrogenase; long chain | 0.083 |
| 115244 | AA278767 | Hs.914 | Human mRNA for SB classII histocompatibility antigen alpha-chain | 0.083 |
| 123253 | AA490878 | Hs.111334 | ferritin; light polypeptide | 0.083 |
| 128469 | T23724 | Hs.258677 | EST | 0.083 |
| 132220 | AA431847 | Hs.42409 | ESTs; Highly similar to CGI-146 protein [H.sapiens] | 0.083 |
| 111664 | R17939 | Hs.22344 | ESTs | 0.083 |
| 102354 | U38268 | | Human cytochrome b pseudogene, partial cds | 0.084 |
| 112828 | R98774 | Hs.194338 | ESTs | 0.084 |
| 110410 | H47868 | Hs.34024 | ESTs | 0.084 |
| 102620 | U66052 | | Human clone W2-6 mRNA from chromosome X | 0.084 |
| 102550 | U58087 | Hs.14541 | cullin 1 | 0.084 |
| 108417 | AA075716 | | zm89e5.s1 Stratagene ovarian cancer (#937219) H sapiens cDNA clone | |
| | | | IMAGE:54512 3' similar to gb:X14723 CLUSTERIN PRECURSOR | |
| | | | (HUMAN);, mRNA sequence | 0.084 |
| 113299 | T67285 | Hs.13089 | ESTs | 0.084 |
| 117869 | N49947 | Hs.46990 | ESTs | 0.084 |
| 113734 | T98484 | Hs.18377 | EST | 0.084 |
| 133325 | C00424 | Hs.7101 | periodontal ligament fibroblast protein | 0.084 |
| 123368 | AA505022 | Hs.124838 | ESTs | 0.084 |
| 101615 | M55153 | Hs.8265 | transglutaminase 2 (C polypeptide; protein-glutamine | |
| | | | -gamma-glutamyltransferase) | 0.084 |
| 119352 | T65972 | Hs.193365 | ESTs; Moderately similar to alternatively spliced product | |
| | | | using exon 13A [H.sapiens] | 0.084 |
| 123828 | AA620686 | Hs.112884 | EST | 0.084 |
| 103611 | Z38133 | Hs.113973 | myosin; heavy polypeptide 8; skeletal muscle; perinatal | 0.084 |
| 131289 | AA485697 | Hs.25334 | ESTs; Weakly similar to ION CHANNEL HOMOLOG RIC | |
| | | | PRECURSOR [M.musculus] | 0.084 |
| 128678 | T15896 | Hs.103535 | ESTs | 0.084 |
| 130814 | AA256695 | Hs.19813 | ESTs | 0.084 |
| 133391 | X57579 | Hs.727 | inhibin; beta A (activin A; activin AB alpha polypeptide) | 0.084 |
| 129322 | AA437153 | Hs.110407 | ESTs; Weakly similar to coded for by C. elegans cDNA yk173c12.5 [C.elegans] | 0.084 |
| 109284 | AA196995 | Hs.86092 | ESTs | 0.084 |
| 116689 | F09222 | Hs.66099 | ESTs | 0.085 |
| 100545 | HG2147-HT2217 | | Mucin 3, Intestinal (Gb:M55405) | 0.085 |
| 102634 | U66711 | Hs.77667 | lymphocyte antigen 6 complex; locus E | 0.085 |
| 111735 | R25389 | Hs.23856 | ESTs; Weakly similar to FAST kinase [H.sapiens] | 0.085 |
| 105181 | AA190676 | Hs.10974 | ESTs; Moderately similar to unknown [R.norvegicus] | 0.085 |
| 122681 | AA455350 | Hs.99401 | EST | 0.085 |
| 114543 | AA056121 | Hs.158419 | ESTs | 0.085 |
| 133597 | AA425908 | Hs.75139 | partner of RAC1 (arfaptin 2) | 0.085 |
| 121064 | AA398647 | Hs.97406 | ESTs | 0.085 |
| 122231 | AA436369 | Hs.197728 | ESTs; Weakly similar to ZINC FINGER PROTEIN 135 [H.sapiens] | 0.085 |
| 100309 | D50550 | Hs.95659 | lethal giant larvae (Drosophila) homolog 1 | 0.085 |
| 101727 | M73481 | Hs.73883 | gastrin-releasing peptide receptor | 0.085 |
| 131226 | AA165400 | Hs.24476 | ESTs | 0.085 |
| 133580 | AA095041 | Hs.181073 | ESTs | 0.085 |
| 102792 | U87964 | Hs.227576 | GTP binding protein 1 | 0.085 |
| 104976 | AA086480 | Hs.183669 | ESTs; Weakly similar to !! ALU SUBFAMILY J WARNING ENTRY !! [H.sapiens] | 0.085 |
| 120865 | AA350631 | Hs.96963 | EST | 0.085 |
| 106080 | AA418046 | Hs.35124 | ESTs | 0.085 |
| 128571 | AA416819 | Hs.101661 | ESTs | 0.085 |
| 101838 | M92934 | Hs.75511 | connective tissue growth factor | 0.085 |
| 128514 | H84261 | Hs.100843 | ESTs; Weakly similar to similar to GTP-binding protein [C.elegans] | 0.085 |
| 123099 | AA485931 | Hs.79 | aminoacylase 1 | 0.085 |
| 134067 | Y08200 | Hs.78920 | Rab geranylgeranyltransferase; alpha subunit | 0.085 |
| 116967 | H80336 | Hs.40124 | EST | 0.085 |
| 110053 | H12586 | Hs.89563 | nuclear cap binding protein 1; 80kD | 0.085 |
| 114395 | AA007313 | Hs.110155 | ESTs | 0.085 |
| 107465 | W44681 | Hs.251385 | murine retrovirus integration site 1 homolog | 0.085 |
| 101983 | S85655 | Hs.75323 | prohibitin | 0.085 |
| 112544 | R70948 | Hs.29153 | ESTs | 0.086 |
| 111423 | R01165 | Hs.188507 | ESTs | 0.086 |
| 127918 | AA806043 | Hs.115396 | Human germline IgD chain gene; C-region; C-delta-1 domain | 0.086 |
| 107300 | T40348 | Hs.90488 | ESTs | 0.086 |
| 134947 | R51194 | | yj71a08.r1 Soares breast 2NbHBst Homo sapiens cDNA clone IMAGE:154166 | |
| | | | 5' similar to gb:L11284 DUAL SPECIFICITY MITOGEN-ACTIVATED PROTEIN | |
| | | | KINASE KINASE 1 (HUMAN);, mRNA sequence. | 0.086 |
| 124579 | N68345 | Hs.127179 | ESTs; Weakly similar to TERATOCARCINOMA-DERIVED GROWTH | |
| | | | FACTOR 1 [H.sapiens] | 0.086 |
| 130471 | Z68280 | Hs.183706 | adducin 1 (alpha) | 0.086 |
| 116596 | D60755 | Hs.92955 | ESTs | 0.086 |
| 105069 | AA136345 | Hs.23617 | ESTs; Weakly similar to ZFOC1 gene product [H.sapiens] | 0.086 |
| 102491 | U51010 | | Human nicotinamide N-methyltransferase gene, exon 1 and 5' flanking region | 0.086 |
| 130069 | AA055896 | Hs.146428 | collagen; type V; alpha 1 | 0.086 |
| 130234 | AA280413 | Hs.157441 | spleen focus forming virus (SFFV) proviral integration oncogene spi1 | 0.086 |
| 120540 | AA262992 | Hs.96417 | ESTs | 0.086 |
| 122508 | AA449221 | Hs.20432 | ESTs | 0.086 |
| 128054 | AI205718 | Hs.125416 | ESTs | 0.086 |
| 133020 | AA053248 | Hs.185182 | ESTs; Highly similar to 40S RIBOSOMAL PROTEIN S10 [H.sapiens] | 0.086 |
| 130056 | AA017356 | Hs.171900 | armadillo repeat gene deletes in velocardiofacial syndrome | 0.086 |
| 130504 | U48865 | Hs.158323 | CCAAT/enhancer binding protein (C/EBP); epsilon | 0.086 |
| 133978 | W73859 | Hs.78061 | transcription factor 21 | 0.086 |
| 105265 | AA227941 | Hs.26088 | ESTs | 0.086 |
| 133035 | T15965 | Hs.6333 | ESTs | 0.086 |
| 100768 | HG3636-HT3846 | | Myosin, Heavy Polypeptide 9, Non-Muscle | 0.086 |
| 129338 | T56800 | Hs.47274 | Homo sapiens mRNA; cDNA DKFZp564B176 (from clone DKFZp564B176) | 0.086 |
| 132789 | W23761 | Hs.56876 | ESTs | 0.086 |
| 116099 | AA456309 | Hs.58831 | regulator of Fas-induced apoptosis | 0.086 |
| 100721 | HG3355-HT3532 | | Peroxisome Proliferator Activated Receptor (Gb:Z30972) | 0.087 |
| 112569 | R73150 | Hs.75270 | GTP-binding protein homologous to Saccharomyces cerevisiae SEC4 | 0.087 |
| 130645 | AA020942 | Hs.17200 | STAM-like protein containing SH3 and ITAM domains 2 | 0.087 |
| 100751 | HG3527-HT3721 | | Luteinizing Hormone, Beta Subunit | 0.087 |
| 134550 | M27161 | Hs.85258 | CD8 antigen; alpha polypeptide (p32) | 0.087 |
| 130885 | AA338646 | Hs.20912 | adenomatous polyposis coli like | 0.087 |
| 101446 | M21302 | Hs.56306 | small proline-rich protein 2A | 0.087 |
| 116287 | AA487856 | Hs.155829 | KIAA0676 protein | 0.087 |
| 134034 | X89267 | Hs.78601 | uroporphyrinogen decarboxylase | 0.087 |
| 130860 | U66061 | Hs.241395 | protease; serine; 1 (trypsin 1) | 0.087 |
| 109901 | H04992 | Hs.30499 | ESTs | 0.087 |
| 107537 | Z20777 | Hs.9857 | ESTs; Weakly similar to peroxisomal short-chain alcohol | |
| | | | dehydrogenase [H.sapiens] | 0.087 |
| 133232 | AA496030 | Hs.6845 | ESTs | 0.087 |
| 108559 | AA085161 | | zn12c5.s1 Stratagene hNT neuron (#937233)H sapiens cDNA clone | |
| | | | IMAGE:54728 3' similar to TR:G1151228 G1151228 LPG1P.; mRNA seq | 0.087 |
| 121288 | AA401735 | Hs.97340 | EST | 0.087 |
| 108844 | AA132916 | Hs.177961 | Human Chromosome 16 BAC clone CIT987SK-A-388D4 | 0.087 |
| 129874 | AA406488 | Hs.181551 | ESTs | 0.087 |
| 105139 | AA164543 | Hs.110082 | ESTs | 0.088 |
| 124789 | R43803 | Hs.78110 | ESTs; Weakly similar to F17A9.2 [C.elegans] | 0.088 |
| 115923 | AA441929 | Hs.38205 | ESTs | 0.088 |
| 123640 | AA609292 | Hs.112681 | ESTs | 0.088 |
| 131607 | AA351409 | Hs.172740 | microtubule-associated protein; RP/EB family; member 3 | 0.088 |
| 130064 | T67053 | Hs.181125 | immunoglobulin lambda gene cluster | 0.088 |
| 108752 | AA127070 | Hs.71055 | ESTs | 0.088 |
| 124249 | H68077 | Hs.108211 | ESTs | 0.088 |
| 100109 | AJ000480 | Hs.143513 | phosphoprotein regulated by mitogenic pathways | 0.088 |
| 104642 | AA004662 | Hs.184245 | KIAA0929 protein Msx2 interacting nuclear target (MINT) homolog | 0.088 |
| 131752 | AA453311 | Hs.31566 | ESTs | 0.088 |
| 114727 | AA132545 | Hs.190202 | ESTs | 0.088 |
| 120965 | AA398089 | Hs.179715 | ESTs | 0.088 |
| 100396 | D84361 | Hs.151123 | Human mRMA for p52 and p64 isoforms of N-Shc; complete cds | 0.088 |
| 106218 | AA428451 | Hs.91146 | DKFZP586E0820 protein | 0.088 |
| 111562 | R09567 | Hs.187569 | ESTs | 0.088 |
| 121219 | AA400606 | Hs.144344 | EST | 0.088 |
| 101187 | L20316 | Hs.208 | glucagon receptor | 0.088 |
| 101513 | M28210 | Hs.27744 | RAB3A; member RAS oncogene family | 0.088 |
| 116454 | AA621071 | Hs.42034 | ESTs; Moderately similar to T-complex protein 10A [H.sapiens] | 0.088 |
| 116171 | AA463434 | Hs.42658 | ESTs | 0.089 |
| 117500 | N31909 | Hs.44278 | ESTs | 0.089 |
| 119978 | W88623 | Hs.59190 | EST | 0.089 |
| 132005 | D58231 | Hs.173091 | DKFZP434K151 protein | 0.089 |
| 109914 | H05529 | Hs.194704 | leucine-rich; glioma inactivated 1 | 0.089 |
| 130370 | M55265 | Hs.155140 | casein kinase 2; alpha 1 polypeptide | 0.089 |
| 104262 | AF009801 | Hs.105941 | bagpipe homeobox (Drosophila) homolog 1 | 0.089 |
| 129708 | AA417181 | Hs.120858 | ESTs | 0.089 |
| 106398 | AA447545 | Hs.18268 | adenylate kinase 5 | 0.089 |
| 120884 | AA365356 | Hs.97041 | ESTs | 0.089 |
| 130404 | X72012 | Hs.76753 | endoglin (Osler-Rendu-Weber syndrome 1) | 0.089 |
| 114072 | Z38184 | Hs.123633 | ESTs | 0.089 |
| 131470 | X54938 | Hs.2722 | inositol 1;4;5-trisphosphate 3-kinase A | 0.089 |
| 124573 | N67935 | Hs.194703 | adaptor-related protein complex 4; mu 1 subunit | 0.089 |
| 114717 | AA131240 | Hs.252014 | EST | 0.089 |
| 133806 | M12759 | Hs.76325 | Human Ig J chain gene | 0.09 |
| 130470 | AA398552 | Hs.15711 | KIAA0639 protein | 0.09 |
| 133182 | Z80787 | Hs.240135 | H4 histone family; member J | 0.09 |
| 116036 | AA452672 | Hs.43866 | ESTs | 0.09 |
| 132404 | AA393903 | Hs.4768 | ESTs | 0.09 |
| 122695 | AA456048 | Hs.99403 | ESTs; Moderately similar to undulin 2 [H.sapiens] | 0.09 |
| 125975 | AA495891 | Hs.152290 | ESTs; Highly similar to PACAP type-3/VIP type-2 receptor [H.saplens] | 0.09 |
| 110783 | N23669 | Hs.26407 | ESTs | 0.09 |
| 129860 | AA410343 | Hs.129826 | tetraspan transmembrane 4 super family | 0.09 |
| 120740 | AA302650 | Hs.96654 | EST | 0.09 |
| 119564 | W38206 | | Accession not listed in Genbank | 0.09 |
| 134474 | AA054746 | Hs.8379 | ESTs | 0.09 |
| 119014 | N95435 | Hs.55144 | ESTs | 0.09 |
| 109791 | F10669 | Hs.13228 | DRE-antagonist modulator; calsenllin | 0.09 |
| 117605 | N35073 | Hs.44433 | ESTs | 0.09 |
| 121589 | AA416627 | Hs.191598 | ESTs | 0.09 |
| 104326 | D81655 | Hs.143067 | ESTs | 0.09 |
| 129861 | N69507 | Hs.129849 | DKFZP564M182 protein | 0.09 |
| 102795 | U88667 | Hs.198396 | ATP-binding cassette; sub-family A (ABC1); member 4 | 0.09 |
| 119626 | W49499 | Hs.184456 | ESTs; Wkly smlr to !! ALU SUBFAMILY SX WARNING ENTRY !! [H.sapiens] | 0.09 |
| 110516 | H56894 | Hs.37368 | EST | 0.09 |
| 105382 | AA236853 | Hs.111801 | Homo sapiens mRNA; cDNA DKFZp564H2023 (from clone DKFZp564H2023) | 0.09 |
| 123754 | AA609964 | Hs.102021 | ESTs | 0.09 |
| 108008 | AA039430 | Hs.61920 | ESTs | 0.09 |
| 121057 | AA398619 | Hs.142375 | ESTs; Moderately similar to putative envelope protein [H.sapiens] | 0.091 |
| 123675 | AA609474 | Hs.112713 | EST | 0.091 |
| 135194 | C20975 | Hs.9613 | ESTs; Highly similar to angiopoietin-related protein [H.sapiens] | 0.091 |
| 127070 | AA641812 | Hs.190037 | ESTs | 0.091 |
| 134051 | S67070 | Hs.78846 | heat shock 27kD protein 2 | 0.091 |
| 133382 | AA112532 | Hs.7247 | ESTs | 0.091 |
| 103615 | Z46967 | Hs.115460 | calicin | 0.091 |
| 118457 | N66593 | Hs.49230 | EST | 0.091 |
| 118504 | N67334 | Hs.50158 | ESTs | 0.091 |
| 112915 | T10176 | Hs.4254 | ESTs | 0.091 |
| 132088 | AA470121 | Hs.243980 | HLA-B associated transcript-3 | 0.091 |
| 101504 | M27288 | Hs.248156 | oncostatin M | 0.091 |
| 112550 | R71391 | Hs.29074 | ESTs | 0.091 |
| 128551 | H09058 | Hs.237323 | N-acetylglucosamine-phosphate mutase; DKFZP434B187 protein | 0.091 |
| 112879 | T03541 | Hs.115960 | ESTs | 0.091 |
| 127079 | AI364691 | Hs.128628 | ESTs; Moderately similar to CL3BC [R.norvegicus] | 0.091 |
| 101993 | U01062 | Hs.77515 | inositol 1;4;5-triphosphate receptor; type 3 | 0.091 |
| 113020 | T23830 | Hs.7303 | ESTs; Weakly similar to PROHIBITIN [H.sapiens] | 0.091 |
| 120465 | AA251505 | Hs.130861 | ESTs | 0.091 |
| 130152 | U32645 | Hs.151139 | E74-like factor 4 (ets domain transcription factor) | 0.091 |
| 104941 | AA065169 | Hs.17805 | ESTs | 0.091 |
| 110090 | H16076 | Hs.6915 | ESTs | 0.091 |
| 135375 | AA480888 | Hs.99741 | ESTs; Weakly similar to BRAIN PROTEIN H5 [H.sapiens] | 0.091 |
| 123799 | AA620418 | Hs.112861 | ESTs | 0.092 |
| 118966 | N93438 | Hs.76907 | ESTs; Highly similar to HSPC002 [H.sapiens] | 0.092 |
| 116969 | H80633 | Hs.143038 | ESTs | 0.092 |
| 125147 | W38150 | | Accession not listed in Genbank | 0.092 |
| 100836 | HG4113-HT4383 | | Olfactory Receptor Or17-201 | 0.092 |
| 114726 | AA132509 | Hs.103827 | EST | 0.092 |
| 107311 | T57738 | Hs.174112 | ESTs | 0.092 |
| 112863 | T03148 | Hs.4610 | EST | 0.092 |
| 129290 | AA521407 | Hs.110095 | ESTs | 0.092 |
| 103384 | X92762 | Hs.79021 | tafazzin (cardiomyopathy; dilated 3A (X-linked); endocardial | |
| | | | fibroelastosis 2; Barth syndrome) | 0.092 |
| 112508 | R68213 | Hs.28847 | ESTs | 0.092 |
| 111863 | R37495 | Hs.23578 | ESTs | 0.092 |
| 131184 | AA452705 | Hs.23954 | ESTs; Weakly similar to KIAA0584 protein [H.sapiens] | 0.092 |
| 107420 | W26567 | Hs.4775 | ESTs | 0.092 |
| 111768 | R27606 | Hs.24185 | ESTs | 0.092 |
| 112290 | R53940 | Hs.26016 | ESTs | 0.092 |
| 130581 | AA481982 | Hs.16258 | ESTs; Weakly similar to RAS-RELATED PROTEIN RAB-5A [H.sapiens] | 0.092 |
| 120744 | AA302772 | Hs.228649 | EST | 0.093 |
| 112226 | R50761 | Hs.25738 | ESTs | 0.093 |
| 116154 | AA460951 | Hs.57100 | ESTs | 0.093 |
| 102640 | U67674 | Hs.194783 | solute carrier family 10 (sodium/bile acid cotransporter family); member 2 | 0.093 |
| 129797 | X53595 | Hs.1252 | apolipoprotein H (beta-2-glycoprotein I) | 0.093 |
| 102705 | U77180 | Hs.50002 | small inducible cytokine subfamily A (Cys-Cys); member 19 | 0.093 |
| 132408 | AA035547 | Hs.47822 | KIAA0380 gene product; RhoA-specific guanine nucleotide exchange factor | 0.093 |
| 108441 | AA079079 | | zm97c9.s1 Stratagene colon HT29 (#937221) Homo sapiens cDNA clone | |
| | | | IMAGE:545872 3' similar to contains element MER22 MER22 repetitive | |
| | | | element;, mRNA sequence | 0.093 |
| 108145 | AA054133 | Hs.63085 | ESTs | 0.093 |
| 106466 | AA449990 | Hs.76057 | lysophospholipase II | 0.093 |
| 101697 | M64358 | | Human rhom-3 gene, exon | 0.093 |
| 121294 | AA401958 | Hs.240170 | ESTs; Moderately similar to alternatively spliced product using | |
| | | | exon 13A [H.sapiens] | 0.093 |
| 117824 | N49065 | Hs.125201 | ESTs; Weakly similar to B7 [M.musculus] | 0.093 |
| 115771 | AA422049 | Hs.40780 | ESTs | 0.093 |
| 102303 | U33053 | Hs.2499 | protein kinase C-like 1 | 0.093 |
| 131405 | U79255 | Hs.26468 | amyloid beta (A4) precursor protein-binding; family A; member 2 (X11-like) | 0.093 |
| 112909 | T10069 | Hs.101094 | ESTs | 0.093 |
| 124173 | H41281 | Hs.107619 | ESTs | 0.093 |
| 112488 | R66896 | Hs.28788 | ESTs | 0.093 |
| 130554 | X59303 | Hs.159637 | valyHRNA synthetase 2 | 0.093 |
| 106413 | AA447964 | Hs.6311 | ESTs | 0.093 |
| 111711 | R22891 | Hs.7093 | ESTs | 0.094 |
| 117595 | N34933 | Hs.44664 | EST | 0.094 |
| 113813 | W45174 | Hs.31382 | ESTs | 0.094 |
| 107769 | AA018449 | Hs.125220 | Homo sapiens DNA from chromosome 19-cosmids R30102:R29350:R27740 | |
| | | | containing MEF2B;genomic sequence | 0.094 |
| 114966 | AA250743 | Hs.92198 | ESTs; Highly similar to calcium-regulated heat stable protein | |
| | | | CRHSP-24 [H.sapiens] | 0.094 |
| 130297 | H94949 | Hs.171955 | trophinin-assisting protein (tastin) | 0.094 |
| 109589 | F02429 | Hs.6581 | ESTs | 0.094 |
| 112592 | R77631 | Hs.29126 | ESTs | 0.094 |
| 102314 | U34038 | Hs.154299 | coagulation factor II (thrombin) receptor-like 1 | 0.094 |
| 116128 | AA459915 | Hs.112193 | mutS (E. coli) homolog 5 | 0.094 |
| 106809 | AA479704 | Hs.220324 | Human DNA sequence from clone 283E3 on chromosome 1p36.21-36.33. | |
| | | | Contains the alternatively spliced gene for Matrix Metalloproteinase in the | |
| | | | Female Reproductive tract MIFR1; -2; MMP21/22A; -B and -C; a novel gene; | |
| | | | the alternatively spliced CDC2L2 gene for | 0.094 |
| 130607 | AA043894 | Hs.16603 | ESTs | 0.094 |
| 120592 | AA281929 | Hs.143974 | ESTs | 0.094 |
| 117230 | N20535 | Hs.43265 | melastatin 1 | 0.094 |
| 105948 | AA404597 | Hs.7133 | ESTs | 0.094 |
| 101333 | L47738 | Hs.80313 | p53 inducible protein | 0.094 |
| 101909 | S69265 | | Homo sapiens mRNA for PLE21 protein; complete cds | 0.094 |
| 106959 | AA497031 | Hs.8657 | ESTs; Highly similar to CTG7a [H.sapiens] | 0.094 |
| 127034 | AA352389 | | ESTs; Wkly smlr to glucose-6-phosphatase catalytic subunit [R.norvegicus] | 0.095 |
| 134430 | H52105 | Hs.8309 | KIAA0747 protein | 0.095 |
| 120342 | AA207105 | Hs.45068 | Homo sapiens mRNA; cDNA DKFZp434l143 (from clone DKFZp434l143) | 0.095 |
| 104450 | L77664 | Hs.103978 | serine/threonine kinase 22B (spermiogenesis associated) | 0.095 |
| 130902 | AA424530 | Hs.21061 | ESTs | 0.095 |
| 102708 | U77594 | Hs.37682 | retinoic acid receptor responder (tazarotene induced) 2 | 0.095 |
| 107373 | U85773 | Hs.154695 | phosphomannomutase 2 | 0.095 |
| 123569 | AA608952 | Hs.195292 | ESTs; Weakly similar to RNA helicase HDB/DICE1 [H.sapiens] | 0.095 |
| 102687 | U73379 | Hs.93002 | ubiquitin carrier protein E2-C | 0.095 |
| 128888 | AA034951 | Hs.106893 | ESTs | 0.095 |
| 100283 | D43642 | Hs.2430 | transcription factor-like 1 | 0.095 |
| 102747 | U79303 | Hs.82482 | protein predicted by clone 23882 | 0.095 |
| 107798 | AA019346 | Hs.60918 | EST | 0.095 |
| 123565 | AA608907 | Hs.112614 | EST | 0.095 |
| 116010 | AA449450 | Hs.56421 | ESTs; Weakly similar to Similarity to H.influenza ribonuclease PH [C.elegans] | 0.095 |
| 117155 | H97536 | Hs.42391 | EST | 0.095 |
| 133094 | AA115572 | Hs.64746 | chloride intracellular channel 3 | 0.095 |
| 113174 | T54659 | Hs.9779 | ESTs | 0.095 |
| 102016 | U03270 | Hs.122511 | centrin; EF-hand protein; 1 | 0.095 |
| 130126 | AB002318 | Hs.150443 | KIAA0320 protein | 0.095 |
| 134813 | X14767 | Hs.89768 | gamma-aminobutyric acid (GABA) A receptor, beta 1 | 0.095 |
| 132055 | N69440 | Hs.38132 | ESTs | 0.095 |
| 122229 | AA436198 | Hs.103902 | ESTs | 0.096 |
| 127574 | AA907314 | Hs.188905 | ESTs | 0.096 |
| 134432 | AA053022 | Hs.8312 | ESTs | 0.096 |
| 128052 | AA878398 | Hs.190491 | ESTs | 0.096 |
| 101637 | M58285 | Hs.132634 | hematopoietic protein 1 | 0.096 |
| 103386 | X92972 | Hs.80324 | protein phosphatase 6; catalytic subunit | 0.096 |
| 133079 | AM77561 | Hs.6449 | ESTs | 0.096 |
| 120328 | AA196979 | Hs.104129 | ESTs; Weakly similar to protease [H.sapiens] | 0.096 |
| 107640 | AA009615 | Hs.257808 | ESTs | 0.096 |
| 123389 | AA521176 | Hs.221231 | ESTs | 0.096 |
| 103222 | X74795 | Hs.77171 | minichromosome maintenance deficient (S.cerevisiae) 5 (cell division cycle 46) | 0.096 |
| 111704 | R22450 | Hs.23396 | ESTs; Highly similar to ZINC FINGER PROTEIN 140 [H.sapiens] | 0.096 |
| 126856 | AA306523 | | EST177475 Jurkat T-cells VI Homo sapiens cDNA 5'end, mRNA sequence. | 0.733 |
| 127071 | AA250806 | | ESTs | 0.096 |
| 114550 | AA056755 | Hs.151714 | ESTs | 0.096 |
| 125955 | AI356943 | Hs.143761 | ESTs | 0.096 |
| 134363 | M37033 | Hs.82212 | CD53 antigen | 0.096 |
| 128550 | W76492 | Hs.170142 | ESTs | 0.096 |
| 122598 | AA453465 | Hs.99329 | ESTs | 0.096 |
| 118898 | N90703 | Hs.4236 | KIAA0478 gene product | 0.096 |
| 117661 | N39092 | Hs.44940 | ESTs | 0.096 |
| 120996 | AA398281 | Hs.143684 | ESTs | 0.096 |
| 123388 | AA521172 | Hs.134417 | ESTs | 0.096 |
| 106700 | AA463929 | Hs.28701 | ESTs | 0.096 |
| 112962 | T16814 | Hs.6828 | ESTs | 0.096 |
| 121262 | AA401372 | Hs.97723 | ESTs | 0.096 |
| 134551 | R44839 | Hs.8526 | i-beta-1;3-N-acetylglucosaminyltransferase | 0.096 |
| 112060 | R43754 | Hs.21164 | ESTs | 0.096 |
| 134678 | AA039935 | Hs.182595 | dynein; axonemal; light polypeptide 4 | 0.096 |
| 100855 | HG4234-HT4504 | | Methylenetetrahydrofolate Reductase | 0.097 |
| 132414 | N91193 | Hs.48145 | ESTs | 0.097 |
| 112900 | T08758 | Hs.3813 | ESTs | 0.097 |
| 115989 | AA447777 | Hs.93135 | ESTs | 0.097 |
| 103561 | Z21488 | Hs.143434 | contactin 1 | 0.097 |
| 131087 | AM09738 | Hs.22824 | ESTs; Weakly similar to p160 myb-binding protein [M.musculus] | 0.097 |
| 120293 | AA190859 | Hs.191428 | ESTs | 0.097 |
| 111830 | R36081 | Hs.25085 | EST | 0.097 |
| 113654 | T95770 | Hs.17666 | ESTs | 0.097 |
| 132675 | AA179338 | Hs.5476 | serine proteinase inhibitor | 0.097 |
| 120182 | Z40125 | Hs.91968 | ESTs | 0.097 |
| 132879 | U16282 | Hs.5881 | ELL gene (11-19 lysine-rich leukemia gene) | 0.097 |
| 134211 | AA056681 | Hs.80021 | ESTs; Weakly similar to 62D9.p [D.melanogaster] | 0.097 |
| 115448 | AA284845 | Hs.165051 | ESTs | 0.097 |
| 118118 | N56901 | Hs.47995 | ESTs | 0.097 |
| 107598 | AA004528 | Hs.169444 | ESTs | 0.097 |
| 128933 | H01824 | Hs.760 | GATA-binding protein 2 | 0.097 |
| 114892 | AA235988 | Hs.86024 | ESTs | 0.097 |
| 101922 | S75168 | Hs.274 | megakaryocyte-associated tyrosine kinase | 0.097 |
| 105444 | AA252374 | Hs.19333 | ESTs; Weakly similar to ATP(GTP)-binding protein [H.sapiens] | 0.097 |
| 128155 | AA926843 | Hs.143302 | ESTs | 0.097 |
| 116276 | AA485870 | Hs.44914 | ESTs | 0.097 |
| 111964 | R41227 | Hs.21860 | ESTs | 0.097 |
| 135100 | AA398926 | Hs.251108 | Homo sapiens mRNA; chromosome 1 specific transcript KIAA0493 | 0.097 |
| 124872 | R69251 | Hs.101506 | EST | 0.097 |
| 103084 | X59932 | Hs.77793 | c-src tyrosine kinase | 0.097 |
| 124138 | H23199 | Hs.107010 | ESTs | 0.098 |
| 130048 | R31745 | Hs.211612 | SEC24 (S. cerevisiae) related gene family, member A | 0.098 |
| 100208 | D26129 | Hs.78224 | ribonuclease; RNase A family; 1 (pancreatic) | 0.098 |
| 123537 | AA608775 | Hs.112589 | ESTs | 0.098 |
| 118999 | N95019 | Hs.55092 | ESTs | 0.098 |
| 119847 | W80384 | Hs.9853 | ESTs | 0.098 |
| 112819 | R98618 | Hs.35984 | ESTs | 0.098 |
| 131080 | J05008 | Hs.2271 | endothelin 1 | 0.098 |
| 127353 | AA190853 | Hs.155360 | ESTs | 0.098 |
| 132068 | X66365 | Hs.38481 | cyclin-dependent kinase 6 | 0.098 |
| 105744 | AA293436 | Hs.12909 | ESTs | 0.098 |
| 133680 | M92357 | Hs.101382 | tumor necrosis factor; alpha-induced protein 2 | 0.098 |
| 122899 | AA469960 | Hs.178420 | ESTs; Highly slmilar to WASP interacting protein [H.sapiens] | 0.098 |
| 128700 | U59286 | Hs.103982 | small inducible cytokine subfamily B (Cys-X-Cys); member 11 | 0.098 |
| 104393 | H46486 | Hs.226499 | nesca protein | 0.098 |
| 123320 | AA496792 | Hs.139572 | EST | 0.098 |
| 129169 | N31641 | Hs.109058 | ribosomal protein S6 kinase; 90kD; polypeptide 5 | 0.098 |
| 135093 | U51333 | Hs.159237 | hexokinase 3 (white cell) | 0.098 |
| 113269 | T65159 | Hs.85044 | ESTs | 0.098 |
| 124283 | H86783 | Hs.194136 | ESTs; Moderately similar to zinc finger protein RIN ZF [R.norvegicus] | 0.098 |
| 114376 | GMCSF | | Accession not listed in Genbank | 0.099 |
| 100881 | HG4458-HT4727 | | Immunoglobulin Heavy Chain, Vdjc Regions (Gb:L23563) | 0.099 |
| 116572 | D45654 | Hs.65582 | DKFZP586C1324 protein | 0.099 |
| 123956 | AA621747 | Hs.112847 | EST | 0.099 |
| 100818 | HG4018-HT4288 | | Oploid-Binding Cell Adhesion Molecule | 0.099 |
| 132754 | W47419 | Hs.56007 | Human DNA from chromosome 19-specific cosmid F25965; genomic sequence | 0.099 |
| 112741 | R93080 | Hs.35035 | ESTs | 0.099 |
| 112748 | R93299 | Hs.166492 | ESTs | 0.099 |
| 130858 | S57235 | Hs.246381 | CD68 antigen | 0.099 |
| 124870 | R69233 | Hs.101504 | ESTs | 0.099 |
| 125304 | Z39833 | Hs.124940 | GTP-binding protein | 0.099 |
| 121297 | AA401995 | Hs.97860 | ESTs | 0.099 |
| 128602 | AA046103 | Hs.102367 | ESTs | 0.099 |
| 124062 | H00440 | Hs.144524 | ESTs; Weakly similar to signal transducer and activator of | |
| | | | transcription 2 [M.musculus] | 0.099 |
| 100547 | HG2149-HT2219 | | Mucin (Gb:M57417) | 0.099 |
| 105652 | AA282505 | Hs.19015 | ESTs | 0.099 |
| 133390 | AA459945 | Hs.72660 | KIAA0585 protein | 0.099 |
| 133503 | M33195 | Hs.743 | Fc fragment of IgE; high affinity I; receptor for; gamma polypeptide | 0.099 |
| 109461 | AA232667 | Hs.58210 | ESTs | 0.099 |
| 102068 | U09117 | Hs.80776 | phospholipase C; delta 1 | 0.099 |
| 113464 | T86931 | Hs.16295 | ESTs | 0.099 |
| 104240 | AB002368 | Hs.70500 | KIAA0370 protein | 0.099 |
| 121113 | AA399109 | Hs.161813 | ESTs | 0.1 |
| 122896 | AA469952 | Hs.97899 | ESTs; Weakly similar to dal2; len:343; CAl:0.17; ALC_YEAST P25335 | |
| | | | ALLANTOICASE [S.cerevisiae] | 0.1 |
| 102405 | U43148 | Hs.159526 | patched (Drosophila) homolog | 0.1 |
| 103599 | Z33905 | Hs.81218 | receptor-associated protein of the synapse; 43kD | 0.1 |
| 121079 | AA398719 | Hs.14169 | ESTs; Weakly similar to CREB-binding protein [H.sapiens] | 0.1 |
| 115820 | AA427487 | Hs.39619 | ESTs; Weakly similar to RETICULOCALBIN 1 PRECURSOR [H.sapiens] | 0.781 |
| 125106 | T95766 | Hs.189760 | ESTs | 0.1 |
| 131373 | N68116 | Hs.26148 | Down syndrome critical region gene 3 | 0.1 |
| 120224 | Z41239 | Hs.106960 | ESTs | 0.1 |
| 133090 | AA448228 | Hs.6468 | ESTs | 0.1 |
| 132300 | AA133244 | Hs.44234 | ESTs | 0.1 |
| 113129 | T49384 | Hs.8988 | EST | 0.1 |
| 110638 | H73197 | Hs.17241 | ESTs | 0.1 |
| 131364 | R53255 | Hs.26010 | ESTs | 0.1 |
| 105370 | AA236476 | Hs.22791 | ESTs; Weakly similar to transmembrane protein with EGF-like and two | |
| | | | follistatin-like domains 1 [H.sapiens] | 0.238 |

**TABLE 11A shows the accession numbers for those primekeys lacking unigeneID's for Table 11. For each probeset we have listed the gene cluster number from which the oligonucleotides were designed. Gene clusters were compiled using sequences derived from Genbank ESTs and mRNAs. These sequences were clustered based on sequence similarity using Clustering and Alignment Tools (DoubleTwist, Oakland California). The Genbank accession numbers for sequences comprising each cluster are listed in the "Accession" column.**

| | | |
|---|---|---|
| Pkey: | | Unique Eos probeset identifier number |
| CAT number: | | Gene cluster number |
| Accession: | | Genbank accession numbers |
| | | |

| **Pkey** | **CAT number** | **Accession** |
|---|---|---|
| | | |
| 100610 | 19864_1 | AW161357 AI879062 AI928938 AW161097 AW161167 BE314465 AA351715 F07096 AA179034 F08510 F00653 AI936671 AA476718 AW772454 AI807703 R44253 AA976667 AI985186 AI650254 H38942 R84829 AA018724 AA001000 H85934 AA019126 H85609 AA017000 AA339355 AW950556 D51397 AA213981 BE548002 AI056359 AA001560 AW952113 AA317769 AI857477 AI857475 AW249771 AW162661 H38943 AA018628 R85885 AI984613 AI934765 |
| | | AI796172 AW157488 AI929191 R85523 D51221 D53851 H85610 AI749674 F21582 AA323145 AA019127 AA687444 T06745 AI699293 H29532 AA214029 AA223656 NM_016834 X14474 R19697 H09695 R17455 R13812 R19056 AI681231 AI590200 R37671 AA861828 AI990023 AI935669 AW005821 AA324581 H17335 R37659 R42802 R46242 R60936 R59731 H28993 AA479907 R44570 AI890696 AA308884 AA507078 R41274 AI365507 T16348 AI560453 F03259 F04722 T16312 AA016081 AW073061 BE314824 W28930 R44098 R51045 |
| 100674 | 21517_2 | AW403342 AW248986 BE561709 AA357312 BE311834 BE389496 BE294887 AW732696 BE047868 AI702383 BE019155 AI702367 BE408966 BE280458 BE313759 BE513492 BE535404 BE280258 AC005263 NM_007165 L21990 AW732711 AI564920 AW249094 BE265365 AW607186 AW607346 BE05217 H27211 U46230 BE260066 BE207043 BE546782 AW248659 |
| 108559 | 41469_9 | AA085228 AA085161 |
| 100721 | 19818_1 | L40904 NM_005037 X90563 AB005526 H21596 AA088517 |
| 100748 | 41861_1 | X06096 X05826 |
| 100750 | 15759_1 | BE157260 BE157265 R48118 H43827 Z17877 AW379070 AW291778 M20605 J03253 M14206 V00568 AI860465 AW296022 M13930 AL047400 J00120 BE018476 AW675223 T26980 F06694 R22709 R24720 H22753 AI903100 AI903094 AW937823 X00364 D10493 K01904 K01906 K00535 L00058 AA410662 AW384760 AA304930 AI680985 X00198 H58025 AW998901 AV653447 N31654 AW610357 AW610369 AW862480 |
| | | BE223010 AW384172 AW384219 AW384171 AW384218 AA298522 BE140421 AW945162 AW751711 AA514409 AW747912 AI214214 W87741 AA972406 AA554513 BE302087 AI249030 AA477850 AV653129 AI281360 AI274110 W87861 AA641366 X66258 AI051600 AA877139 AA527483 AA857219 AI250782 AA625531 AA807892 AI278811 AI224033 H24033 AA593396 AW129709 R45453 N22772 AA235530 |
| | | T29737 AI016409 AI688907 AA568370 AA722760 AI39329 AA550843 AW674698 AI538452 AI538453 AI337957 AA477744 AA464600 AI140319 AW949294 AI339781 AI828736 AA923634 AA344094 AI278350 AA975567 AA908416 AA857170 AW023520 R43413 R48004 F02958 AI989439 R11207 AA737307 D10493 AW950652 AI093842 AI474024 AA703369 R11264 M13930 M13930 M13930 M13930 M13930 J00120 M13930 M13930 X00364 J00120 R19507 AA639812 |
| 100751 | 24700_1 | N32759 N29730 N30831 N32604 N31955 AI206390 H87574 R23494 AI186215 N30036 AI741512 J00117 NM_000737 AI453626 AA330974 AI188729 AI188604 AI188964 N30276 AI188947 AI188830 AI188303 AI200457 AI219166 AI192459 AI183280 AI189275 AI188639 AI186353 AI189616 AI184224 AI130720 AI188454 AI188391 |
| | | AI148857 AI192447 AI209155 AI190013 AI206355 AI188721 AI189429 AI189364 AI186330 AI431595 AI189595 AI188781 AI148647 AI200022 AI221552 AI220923 AI188728 AA233034 AI189807 AI189641 AI219044 AI148774 AI200658 W71989 AI207360 AI188824 AI200559 AI200270 AA644163 AI199943 AI151301 AI189555 AI262724 AI148590 AI148695 AI126906 AI149163 K03183 K03189 |
| | | AI189842 AI221014 N30608 AI186465 AI220865 AI188498 AI138226 Al189968 AI221019 AI138197 AI149426 AI148904 AI186218 AI188348 AI160579 AI198460 AI149039 AI160936 AI219055 AI184784 AI221580 AI161082 AI160814 AI123896 AI417614 AI126101 AI188872 AI149571 AI168533 AI149072 AI149467 AI131286 N30684 AI160705 AI160692 AI149559 |
| | | AI273580 AI189442 AI138448 AI149591 N27302 AA400910 AI138431 AI138435 AI128407 N30216 AI128296 AI219589 AI188492 AI149447 AI168482 H95374 AI219009 N31616 AI276216 N32233 AI291937 N30741 AI188689 N27111 R23214 AI221605 AI184348 Al200375 H94451 N26397 AI871881 AA232905 N30833 AI220780 H94446 N30822 H87464 R68815 N30290 AI128424 H12587 T47334 H87631 H87156 AI219133 |
| | | AI868741 AA330859 H86993 AA330413 H93656 N30817 T90191 H93668 AI200054 H95207 T47316 H95381 T49170 R00880 T49171 N27381 H94107 R63352 T85053 AW451899 H95142 N30313 H94015 H86987 T28278 N29701 C18834 AA331267 AA330939 AI654493 N27073 N29831 R68113 N30758 R26086 N32108 H95135 AA330414 AA330978 AI219422 AI189453 AI199951 X00264 NM_000894 AA371909 AA063496 T29543 AA371971 AA372026 AA371978 AA371346 AI051683 AI186418 AI220659 AI189068 AI219266 AI186552 AI188715 AI149156 |
| 100760 | 1334_7 | AW794626 M27126 M27014 |
| 100775 | 18179_3 | J05581 M61170 T27692 M34088 M34089 AW860335 AW579047 AW610437 AW610386 AW610422 AW610473 AW579078 AW604897 AW860163 AW579067 AW862410 AI816584 AW177757 AW602769 AI909790 AW860331 AI909787 AI909811 |
| | | AI909813 AW845083 AI905920 AW387919 BE140766 AI909279 AW369405 AA429321 AA429320 AA367451 AA847972 AW001137 AI567905 T84561 AI631295 AA151351 H02932 AI884519 AA367457 AW369421 AI678846 AW391803 AI610869 AW192838 AI922289 AI952140 AI910233 AI479474 AW001395 AA488073 |
| | | AI985760 AW130017 AI858369 AA627845 AW081805 AA158865 AI624443 AA344985 AA569793 R72486 AI589329 AI903204 AI269893 AA641284 AI279932 AA149270 AI697120 AA729146 AI589353 AA480067 AI923310 AA530908 AI275395 AA425062 AA580280 AA889527 AA158866 AW131341 AA573028 AA877326 T29335 AW951288 H04235 |
| | | AA099243 AA994659 AI659618 AA887919 AI299297 AW001116 AW263844 AI270578 AA970828 AW572126 AA775299 AW369449 AW369398 AW369452 AI933677 AI870710 AI092911 AI582464 AI497674 AA937026 AA885865 |
| | | L38597 AA908325 AW369432 AW026623 AA627778 AI264942 AA932409 AI187328 AI672970 AI886098 AW440471 AW138860 AI866858 AI802528 AI926172 AW243914 AI933690 AA996114 AA536189 AW009937 AI918060 AI270379 AI973169 AW175638 AW369413 |
| 100800 | 24735_1 | NM_006227 L26232 R50649 AU077024 AL008726 AA411079 R35151 BE278153 BE278139 AI459777 R88036 Z43210 F07326 AF052157 R17844 BE615476 T82160 R71985 H21963 AA299158 AW368246 R48123 R50628 R70441 H27245 H72015 R72345 R39392 |
| | | AI909738 BE612778 BE613234 D52116 D52136 D52132 D52067 D51922 D51995 D51905 N34249 N25459 AA464436 AA297350 AA297466 R81736 H02737 AW582505 R27523 AI834241 AW130867 W72668 W76426 AA358363 R50262 AW473860 H52335 H43953 H21964 T39505 AI887517 AW156925 AW839850 |
| | | H02628 AW007705 AI561008 F22392 R71279 AA995433 R50725 W24462 R71931 AA464437 AW591731 R25667 R52695 R50810 AI560805 AI089266 H68386 H41353 H28590 AW001860 AI141623 AA250773 AI284778 AW511412 AW083975 AA130377 AW026047 R50551 R81494 AI357668 |
| | | AI078272 F32666 F36981 AW304865 H43906 AA931068 R48010 AI540217 AI017339 AI291812 AI741954 AA458490 AI088378 AA298764 H61168 AA358362 AA298725 AA298515 AA464148 AA443538 R43046 AA084314 T40641 T47608 T48940 AI082477 AW470145 N92284 AI758958 AA298512 AA284586 AI597777 AA480277 |
| | | AI932559 AI869081 AA476615 AA503651 AI656024 AW168522 AI682051 AI689106 AI274592 AI520917 BE258916 BE615861 BE280282 R53386 BE278255 BE278398 T47607 AA477662 H68385 100817 19648_1 L34355 L46810 NM_000023 U08895 AA424260 AI097272 AA424162 N79764 F19290 F25278 AI479385 AA460662 AA432059 AW016935 F25770 F32549 F36677 F33016 F35992 F36010 AW172497 AA835076 F28727 AA211643 AA453282 |
| 100818 | 19604_3 | U79251 AA843851 R38201 R66461 R44908 AA683289 H17477 R37364 R52832 AW298336 AA351391 NM_002545 L34774 AA296866 AW967001 T28889 R13451 T77331 AL119196 AL118830 H08459 AW892812 AW905838 H17685 R52878 |
| 100881 | 458_127 | BE561958 BE561728 BE397612 BE514391 BE269037 BE514207 BE562381 BE514256 BE514403 BE514250 BE397832 BE269598 BE559865 BE396881 BE560031 BE514199 BE560037 BE560454 |
| 100885 | 12707_3 | X07881 NM_006249 X07637 AA376715 AA376677 X07715 X07704 S80916 |
| 100898 | 8542_1 | BE387614 R51501 AA199714 AW674779 F08178 BE269071 AA376313 H08264 AA380420 H18785 AL042151 BE277758 BE267438 NM_005850 L35013 BE540833 BE390902 BE391494 BE277459 BE385592 BE390612 BE384263 BE387779 BE388647 BE537373 BE547158 AW409585 AW374033 AW602185 AA355725 AW577548 AW935015 AW935160 W40232 AW938647 AW374332 |
| | | AA434040 BE293488 AL138361 BE560260 AI745075 AA317980 AW949382 AI834311 AI653582 AI831042 AI361878 AA618606 AA729052 AI424969 AA199715 AW769374 AI828422 AW044307 AI862816 AI203583 AW084461 AW514655 AA831883 AA290672 AA831286 AA578510 AW089965 |
| | | AW150746 AA292743 H22232 AI469275 AW439312 AA292744 AW471443 AI473989 AA593336 AA464070 AI678937 AW069451 AA970763 AA610480 AA593328 AA464009 AA768985 AI298928 AA436600 AA464718 AA699361 D61482 D55935 AI369591 AA470695 AI809135 AA640627 |
| | | AI568446 R51502 W45467 AI655316 AA463934 AW168609 AW518663 BE045525 Z41251 AI868091 AA908160 AI026697 AI886259 AI612932 AA215437 AI956014 BE541087 BE255652 BE265878 BE394102 W27502 |
| 102459 | 3556_1 | U48936 L36592 X87160 NM_001039 AL036606 AL036420 U35630 AW298574 |
| 126126 | 1630017_1 | W80551 M85370 |
| 102620 | 16821_37 | AA976427 U66052 |
| 102673 | 24986_6 | AI457548 U72509 |
| 102675 | 5145_4 | U72512 T98357 R31335 F18090 |
| 102753 | 2226_1 | L32961 NM_000663 U80226 S75578 AA425061 AA429317 AI815143 AA910669 AI286022 AI286019 |
| 102799 | 34624_4 | U88896 U88898 AA916056 T03285 AI341594 AI359534 AI634031 U88897 |
| 127034 | 51148_2 | BE397750 AA232171 BE562900 BE384894 BE242228 BE206819 BE261742 AA296468 AW959763 BE276164 BE264109 BE392626 BE256735 AA301453 N55872 H01676 AA292746 AA427485 AA496400 AA352389 |
| 103522 | 21640_1 | Y10518 Y10514 Z83935 Y10508 AK000055 Y10519 AI142012 AI681175 BE222219 AA890586 BE504347 BE328064 N63044 N51226 AI151248 AI521996 AI924777 AW375954 AI860275 W00549 AI742673 AW612288 AI763062 AA632510 AI087347 AI088070 AI214349 AA890297 AI494156 AI698598 AA631658 AA504593 AA860733 AI266761 AW663214 AW771231 AA639610 AI769806 AI769746 AW014326 AI288611 |
| 127071 | 188097_1 | AA250806 AA459220 |
| 126456 | 291965_1 | AA429212 W00881 |
| 119388 | 1762256_1 | T88798 R92430 |
| 126856 | 20669_1 | AI084125 AI083773 AI479687 AI939609 AI968662 AF129507 NM_013282 AW971840 AW298508 AA744240 AA811217 AA827671 AA811055 AA806567 AA488977 AA908902 AI637637 AA927056 AI870139 AW340492 AA488755 AA129794 AA306523 AA354253 BE256277 AC053467 AW962084 |
| 103996 | 224545_1 | AA321355 AW964592 R23284 H73883 R23382 N47914 C01377 H04668 AW606248 R34447 AA847136 AI684489 AI523112 AW044269 AI379138 N29366 AA761543 N79248 AA960845 AA768316 AI147926 AI718599 AI880620 R67467 AI216016 AI738663 H04648 |
| 113213 | 23798_1 | NM_001395 Y08302 AI434619 AI470328 AI261807 AW024965 AI806537 AI830549 AI640337 AI219065 AW271700 AW028488 AI133339 AI859205 R51175 U87167 BE379324 BE392008 AA340819 AA343110 T57275 D59164 AW299312 AI434422 AI936390 AW024975 R40262 AW269126 R09430 T56590 AI367247 AI253132 BE464248 T58658 AW207785 T58607 |
| 134947 | 844579_1 | R51194 AI732276 R53587 AI820697 |
| 129311 | 16078_1 | AK000526 BE550084 W30689 AW271859 AA411456 AI341551 AA242990 AA243027 H87046 D20360 AI184053 AA146956 AI721023 AI718944 AA146955 F18215 AA903890 AI700355 AI075430 AA411584 AA878210 AI476760 AW945637 AA630596 |
| | | AA431522 AA301989 AI909058 D12149 N41960 BE222214 AA609922 AA828176 AA393359 AA398693 AW024956 BE467805 AW298623 AW264085 AI024454 AI024719 AI431927 T55087 AI611014 T54920 AA131253 AI436344 |
| 114427 | 9724_2 | AA017176 AI359979 AA047836 AA017063 AA016303 AA001545 |
| 114569 | 110077_1 | AA063315 AA063316 |
| 100106 | 15621_-5 | AF015910 |
| 100515 | 342_1 | AA305746 D90187 T63943 AW951154 T29182 AI734941 D13264 AI299239 Z18812 AW299859 W24476 AA933064 AA489759 |
| 100531 | 46038_1 | AW888554 AW607282 AA319986 M28590 |
| 100545 | 22955_11 | M55405 AW752552 |
| 100574 | 17320_2 | AA326895 M10036 NM_000365 N84665 H69414 N84657 AA380453 AA329743 AA357367 AA188770 AA376532 AA353653 AA158953 AA083176 BE537313 AA181433 D53373 R57376 AA206698 R14807 H18899 H11191 H93892 R25593 T61134 N93285 AA083081 AA831789 |
| | | H13137 AA497014 AA079330 AA182861 H13138 W47161 R62913 AA687089 AA211112 AA429237 AL035923 AA100070 AW392898 AI566433 AA866006 AA214002 AW392865 N79454 AA197181 AI680371 AA176501 AA737967 AI089225 F34874 AW571437 AI620620 AA573489 AA423816 AA164917 AA458455 T47072 AI569087 AI261656 |
| | | AA730919 AI633441 AW195182 AI351622 AW243465 AI872649 AI359227 AA987941 AI693770 T47073 AW779948 AW510580 AI635626 AW627601 AA864326 AA953578 AI341418 BE222853 AI241963 AI094663 AA928380 AA493373 AW043762 AI377783 AW958987 BE619760 AA385240 BE277975 BE280095 AW631443 AA581048 BE618715 BE299610 |
| | | C14874 BE559858 BE378455 BE618290 BE544585 AI525575 BE548897 BE267110 AA804738 BE269821 AA918133 BE277647 AA599947 BE280735 BE390239 N74150 T12504 AI208197 AW955527 AA113897 N40081 H73835 H70393 AI434041 W22950 AI192661 BE264461 W26486 AA626424 AA196694 T69209 AA857976 AI540287 |
| | | AA410599 AA864287 AW950564 AA013320 T49283 AI541438 AW804703 AA335534 AA335659 BE562269 BE618802 BE277850 BE546413 BE280994 AA204813 BE561694 BE543524 BE253647 AW001452 W19116 BE542508 AA205894 BE254875 BE270033 AI525906 BE251792 AA975700 BE272138 AW607671 N87686 M10036 BE515060 BE298607 AI745178 U47924 H03193 |
| 100627 | tigr_HT2798 | Z25424 |
| 100756 | tigr_HT3768 | M88357 |
| 100768 | tigr_HT3846 | L29141 M69180 M81105 |
| 100813 | tigr_HT4265 | L33999 |
| 100836 | tigr_HT4383 | U04688 |
| 100855 | tigr_HT4504 | U09806 |
| 102104 | entrez_U12139 | U12139 |
| 125091 | genbank_T91518 | T91518 |
| 100929 | tigr_HT688 | X65561 |
| 125147 | _entrez_W38150 | W38150 |
| 102354 | entrez_U38268 | U38268 |
| 102491 | entrez_U51010 | U51010 |
| 102636 | entrez_U67092 | U67092 |
| 118769 | genbank_N74496 | N74496 |
| 101046 | entrez_K01160 | K01160 |
| 101057 | entrez_K03430 | K03430 |
| 108334 | genbank_AA070473 | AA070473 |
| 108417 | 483241_1 | AA070853 AA075749 AA075716 |
| 108441 | genbank_AA079079 | AA079079 |
| 108786 | genbank_AA128999 | AA128999 |
| 101655 | entrez_M60299 | M60299 |
| 101697 | entrez_M64358 | M64358 |
| 117437 | genbank_N27645 | N27645 |
| 101798 | entrez_M85220 | M85220 |
| 101909 | entrez_S69265 | S69265 |
| 103508 | entrez_Y10141 | Y10141 |
| 103575 | entrez_Z26256 | Z26256 |
| 119332 | genbank_T54095 | T54095 |
| 112161 | genbank_R48295 | R48295 |
| 119564 | NOT_FOUND_entrez_W38206 | W38206 |
| 114376 | NOT_FOUND_entrez_GMCSF | GMCSF |
| 100478 | tigr_HT1067 | M22406 |
| 100547 | tigr_HT2219 | M57417 |
| 100564 | tigr_HT2324 | Z11585 |

**TABLE 12: shows genes, including expression sequence tags, that are down-regulated in prostate tumor tissue compared to normal prostate tissue as analyzed using Affymetrix/Eos Hu01 GeneChip array. Shown are the ratios of "average" normal prostate to "average" prostate cancer tissues.**

| | | | | |
|---|---|---|---|---|
| Pkey: | | Unique Eos probeset identifier number | | |
| ExAccn: | | Exemplar Accession number, Genbank accession number | | |
| UnigeneID: | | Unigene number | | |
| Unigene Title: | | Unigene gene title | | |
| R1: | | Background subtracted normal prostate : prostate tumor tissue | | |
| | | | | |

| **Pkey** | **ExAccn** | **UnigeneID** | **Unigene Titel** | **R1** |
|---|---|---|---|---|
| | | | | |
| 100522 | HG1763-HT1780 | | Prolactin-Induced Protein | 17.4 |
| 130803 | M81650 | Hs.1968 | semenogelin I | 16.785 |
| 118068 | N53943 | Hs.13743 | ESTs | 13.225 |
| 114251 | Z39898 | Hs.21948 | ESTs | 12.7 |
| 112134 | R46025 | Hs.7413 | ESTs | 8.735 |
| 101436 | M20642 | Hs.158295 | Human alkali myosin light chain 3 mRNA; complete cds | 8.175 |
| 104028 | AA361094 | Hs.221128 | ESTs | 8.15 |
| 108944 | AA149204 | Hs.175783 | ESTs; Highly similar to growth arrest inducible gene product [H.sapiens] | 7.535 |
| 103838 | AA174173 | Hs.12622 | ESTs | 7.212 |
| 120469 | AA251741 | Hs.25882 | DKFZP586M1824 protein | 7.175 |
| 110279 | H29231 | Hs.27384 | ESTs | 6.701 |
| 127472 | AA761378 | Hs.192013 | ESTs | 6.642 |
| 133301 | N35229 | Hs.7037 | pallid (mouse) homolog;pallidin | 6.411 |
| 102457 | U48807 | Hs.2359 | dual specificity phosphatase 4 | 6.395 |
| 114011 | W90385 | Hs.15082 | ESTs | 6.15 |
| 101249 | L33881 | Hs.1904 | protein kinase C; iota | 6 |
| 123265 | AA491209 | Hs.105265 | ESTs; Weakly similar to reverse transcriptase [M.musculus] | 6 |
| 119322 | T49655 | Hs.241569 | ESTs; Modly smlr to !! ALU SUBFAMILY SQ WARNING ENTRY !! [H.sapiens] | 5.95 |
| 101673 | M61906 | Hs.6241 | phosphoinositide-3-kinase; regulatory subunit: polypeptide 1 (p85 alpha) | 5.925 |
| 115586 | AA399218 | Hs.92423 | ESTs | 5.7 |
| 120590 | AA281780 | Hs.111441 | ESTs; Weakly similar to similar to Kruppel-like zinc finger protein [C.elegans] | 5.7 |
| 109748 | F10192 | Hs.248323 | Tubulin; alpha; brain-specific | 5.625 |
| 134727 | X80507 | Hs.8939 | yes-associated protein 65 kDa | 5.5 |
| 129171 | AA234048 | Hs.7753 | calumenin | 5.486 |
| 120390 | AA233122 | Hs.111460 | ESTs; Highly similar to multifunctional calcium/calmodulin-dependent protein kinase II delta2 isoform [H.sapiens] | 5.4 |
| 131699 | R68657 | Hs.90421 | ESTs; Modly smlr to !! ALU SUBFAMILY SX WARNING ENTRY !! [H.sapiens] | 5.279 |
| 104490 | N71503 | Hs.43087 | ESTs; Weakly similar to dysfertin [H.sapiens] | 5.266 |
| 102124 | U14528 | Hs.29981 | solute carrier family 26 (sulfate transporter); member 2 | 5.151 |
| 109280 | AA196635 | Hs.86081 | ESTs | 5.134 |
| 109707 | F09739 | Hs.185701 | Homo sapiens mRNA full length insert cDNA clone EUROIMAGE 21920 | 5.075 |
| 108087 | AA045709 | Hs.40545 | ESTs | 5.075 |
| 135006 | M21665 | Hs.929 | myosin; heavy polypeptide 7; cardiac muscle; beta | 5.055 |
| 119182 | R80664 | Hs.77067 | ESTs | 5.033 |
| 129806 | R62444 | Hs.173373 | KIAA0931 protein | 4.675 |
| 101435 | M20543 | Hs.1288 | actin; alpha 1; skeletal muscle | 4.626 |
| 125954 | R93943 | | yt72c12.r1 Soares retina N2b4HR Homo sapiens cDNA clone IMAGE:275735 5', | 4.6 |
| 113989 | W87544 | Hs.221184 | ESTs | 4.559 |
| 104432 | J03460 | Hs.99949 | prolactin-induced protein | 4.451 |
| 112326 | R56068 | Hs.4268 | ESTs | 4.45 |
| 119063 | R16833 | Hs.53106 | ESTs; Weakly similar to !! ALU SUBFAMILY J WARNING ENTRY !! [H.sapiens] | 4.45 |
| 130376 | R40873 | Hs.155174 | KIAA0432 gene product | 4.301 |
| 122484 | AA448286 | Hs.98074 | ESTs; Highly similar to atrophin-1 interacting protein 4 [H.sapiens] | 4.2 |
| 104142 | AA447006 | | ESTs; Moderately similar to !! ALU SUBFAMILY SQ WARNING | 4.175 |
| 129413 | N32787 | Hs.11123 | ESTs; Moderately similar to hypothetical protein 2 [H.sapiens] | 4.1 |
| 103678 | Z84483 | | Human DNA sequence from PAC 46H23, BRCA2 gene region chromosome | 13q12-134.05 |
| 114266 | Z40186 | Hs.26409 | ESTs | 4.05 |
| 115206 | AA262491 | Hs.186572 | ESTs | 4.048 |
| 123723 | AA609749 | Hs.112759 | ESTs; Highly similar to unknown protein [R.norvegicus] | 4.041 |
| 129130 | H97993 | Hs.172788 | ESTs; Weakly similar to KIAA0512 protein [H.sapiens] | 4.028 |
| 120217 | Z41078 | Hs.66035 | ESTs | 4.028 |
| 108536 | AA084524 | | zn19d8.s1 Stratagene neuroepithelium NT2RAMI 937234 Homo sapiens cDNA | 4.023 |
| 134460 | AA400030 | Hs.8360 | ESTs; Weakly similar to !! ALU CLASS B WARNING ENTRY !! [H.sapiens] | 3.925 |
| 120418 | AA236010 | Hs.26613 | Homo sapiens mRNA; cDNA DKFZp586F1323 (from clone DKFZp586F1323) | 3.91 |
| 132783 | N74897 | Hs.5683 | DEAD/H (Asp-Glu-Ala-Asp/His) box polypeptide 15 | 3.889 |
| 125052 | T80174 | Hs.222779 | ESTs; Moderately similar to similar to NEDD-4 [H.sapiens] | 3.85 |
| 108600 | AA099585 | Hs.41175 | ESTs | 3.833 |
| 103099 | X61100 | Hs.8248 | NADH dehydrogenase (ubiquinone) Fe-S protein 1 (75kD) (NADH-coenzyme | 3.818 |
| 134948 | H06773 | Hs.93850 | protein kinase; AMP-activated; gamma 2 non-catalytic subunit | 3.792 |
| 120511 | AA258144 | Hs.221576 | ESTs | 3.779 |
| 111861 | R37460 | Hs.25231 | ESTs | 3.768 |
| 113966 | W86600 | Hs.9842 | ESTs | 3.75 |
| 131649 | AA481254 | Hs.30120 | ESTs | 3.708 |
| 129775 | R94659 | Hs.12420 | ESTs | 3.707 |
| 110191 | H20568 | Hs.27182 | phospholipase A2-activating protein | 3.7 |
| 112678 | R87160 | Hs.33665 | ESTs | 3.7 |
| 127115 | AA375791 | Hs.131894 | ESTs | 3.674 |
| 132892 | W92797 | Hs.59378 | DKFZP434G162 protein | 3.653 |
| 115023 | AA252079 | Hs.63931 | dachshund (Drosophila) homolog | 3.625 |
| 114932 | AA242751 | Hs.16218 | KIAA0903 protein | 3.62 |
| 106865 | AA487228 | Hs.19479 | ESTs | 3.614 |
| 134480 | AA024664 | Hs.83916 | NADH dehydrogenase (ubiquinone) 1 alpha subcomplex; 5 (13kD; B13) | 3.613 |
| 124780 | R42493 | Hs.220839 | ESTs | 3.6 |
| 130631 | AA025399 | Hs.169737 | ESTs | 3.592 |
| 134154 | AA211320 | Hs.79404 | neuron-specific protein | 3.568 |
| 104160 | AA455706 | Hs.99722 | ESTs; Weakly similar to 78 KD GLUCOSE REGULATED PROTEIN | |
| | | | PRECURSOR | 3.559 |
| 105524 | AA258158 | Hs.22153 | ESTs; Weakly similar to KIAA0352 [H.sapiens] | 3.542 |
| 110168 | H19673 | Hs.176586 | ESTs | 3.525 |
| 109480 | AA233299 | Hs.72158 | ESTs | 3.522 |
| 109585 | F02367 | Hs.27252 | ESTs | 3.5 |
| 115134 | AA257107 | Hs.194331 | ESTs | 3.5 |
| 116083 | AA455653 | Hs.44581 | ESTs; Weakly similar to HEAT SHOCK 70 KD PROTEIN 6 [H.sapiens] | 3.459 |
| 120524 | AA261852 | Hs.192905 | ESTs | 3.45 |
| 116932 | H74330 | Hs.150000 | ESTs | 3.425 |
| 130746 | AA256976 | Hs.18800 | ESTs; Weakly similar to KIAA0579 protein [H.sapiens] | 3.42 |
| 107513 | X05451 | Hs.158295 | Human alkali myosin light chain 3 mRNA; complete cds | 3.417 |
| 118641 | N70298 | Hs.49829 | ESTs | 3.407 |
| 126584 | AI028384 | Hs.127331 | ESTs | 3.399 |
| 105134 | AA159953 | Hs.22895 | ESTs; Weakly similar to arylsulfatase B precursor [H.sapiens] | 3.325 |
| 123502 | AA600116 | Hs.112526 | ESTs | 3.318 |
| 132389 | N50866 | Hs.47135 | ESTs | 3.317 |
| 105691 | AA287097 | Hs.75356 | transcription factor 4 | 3.315 |
| 131505 | H85897 | Hs.27755 | ESTs | 3.309 |
| 120775 | AA342104 | Hs.96777 | EST | 3.3 |
| 105579 | AA278824 | Hs.19218 | ESTs | 3.295 |
| 128190 | AA946876 | Hs.148376 | ESTs | 3.292 |
| 100819 | HG4020-HT4290 | | Transglutaminase | 3.288 |
| 130217 | D29956 | Hs.152818 | ubiquitin specific protease 8 | 3.273 |
| 130068 | AA608903 | Hs.106220 | KIAA0336 gene product | 3.269 |
| 134719 | L07515 | Hs.89232 | chromobox homolog 5 (Drosophila HP1 alpha) | 3.266 |
| 110277 | H29209 | Hs.151231 | ESTs; Highly similar to FYVE finger-containing phospholnositide kinase [M.musculus] | 3.26 |
| 127354 | AA418880 | Hs.185797 | ESTs | 3.212 |
| 129173 | R60523 | Hs.109087 | ESTs | 3.197 |
| 127464 | AA970504 | Hs.146103 | ESTs | 3.179 |
| 124923 | R94500 | Hs.108046 | ESTs | 3.175 |
| 122465 | AA448164 | Hs.99153 | ESTs; Highly similar to CGI-73 protein [H.sapiens] | 3.151 |
| 122027 | AA431302 | Hs.98721 | EST; Weakly similar to N-copine [H.sapiens] | 3.151 |
| 103329 | X85134 | Hs.72984 | retinoblastoma-binding protein 5 | 3.15 |
| 129937 | M95767 | Hs.135578 | chitoblase;di-N-acetyl- | 3.15 |
| 134197 | AA057341 | Hs.87889 | helicase-mol | 3.15 |
| 107764 | AA018219 | Hs.226923 | ESTs | 3.125 |
| 121775 | AA421773 | Hs.161008 | ESTs | 3.125 |
| 114768 | AA149007 | Hs.182339 | Ets homologous factor | 3.12 |
| 132381 | N48818 | Hs.46884 | ESTs | 3.11 |
| 123105 | AA485973 | Hs.143947 | ESTs | 3.104 |
| 121176 | AA400080 | Hs.97774 | ESTs | 3.1 |
| 125053 | T80620 | Hs.186473 | ESTs | 3.075 |
| 105909 | AA401739 | Hs.5111 | ESTs | 3.066 |
| 119767 | W72562 | Hs.58119 | ESTs | 3.057 |
| 115776 | AA424038 | Hs.58197 | ESTs | 3.056 |
| 111713 | R22988 | Hs.220950 | ESTs | 3.05 |
| 115301 | AA280047 | Hs.43948 | ESTs | 3.05 |
| 118448 | N66412 | Hs.49189 | ESTs | 3 |
| 106586 | AA456598 | Hs.256269 | ESTs | 2.995 |
| 110415 | H48239 | Hs.29739 | ESTs; Weakly similar to RAS-RELATED PROTEIN RAB-3A [H.sapiens] | 2.979 |
| 105173 | AA182030 | Hs.8364 | ESTs | 2.978 |
| 101102 | L07594 | Hs.79059 | transforming growth factor; beta receptor III (betaglycan; 300kD) | 2.976 |
| 110543 | H58383 | Hs.258544 | ESTs | 2.976 |
| 125593 | R24464 | Hs.202949 | KIAA1102 protein | 2.964 |
| 100824 | HG4058-HT4328 | | Oncogene Aml1-Evi-1, Fusion Activated | 2.957 |
| 106822 | AA481068 | Hs.31835 | ESTs | 2.95 |
| 131963 | D11930 | Hs.3592 | ESTs | 2.95 |
| 111221 | N68869 | Hs.15119 | ESTs | 2.936 |
| 113620 | T93795 | Hs.17252 | EST | 2.917 |
| 105220 | AA210695 | Hs.17212 | ESTs | 2.917 |
| 123234 | AA490227 | Hs.105252 | ESTs | 2.904 |
| 125250 | W87465 | Hs.222926 | ESTs; Weakly similar to D2092.2 [C.elegans] | 2.9 |
| 116196 | AA465160 | Hs.63386 | ESTs | 2.9 |
| 122100 | AA432243 | Hs.41086 | ESTs; Weakly similar to OXYSTEROL-BINDING PROTEIN [H.sapiens] | 2.896 |
| 111712 | R22905 | Hs.113716 | ESTs | 2.895 |
| 126589 | W78107 | Hs.187698 | ESTs; Weakly similar to Yer140wp [S.cerevisiae] | 2.895 |
| 111132 | N64378 | Hs.13149 | ESTs; Highly similar to unknown function [H.sapiens] | 2.894 |
| 115307 | AA280300 | Hs.191346 | ESTs | 2.886 |
| 108989 | AA152263 | Hs.18827 | KIAA0849 protein | 2.883 |
| 129486 | H03686 | Hs.220689 | Ras-GTPase-activating protein SH3-domain-binding protein | 2.879 |
| 119805 | W73788 | Hs.43213 | ESTs | 2.875 |
| 125721 | R59881 | Hs.7503 | ESTs | 2.871 |
| 103704 | AA028171 | Hs.153688 | ESTs | 2.868 |
| 128420 | AI088155 | Hs.14146 | ESTs; Weakly similar to unknown [H.sapiens] | 2.866 |
| 120571 | AA280738 | Hs.128679 | ESTs | 2.863 |
| 123059 | AA482019 | Hs.238202 | EST | 2.86 |
| 129462 | D84239 | Hs.111732 | IgG Fc binding protein | 2.856 |
| 125166 | W45491 | Hs.172609 | nucleobindin 1 | 2.854 |
| 125992 | W01626 | | za36e07.r1 Soares fetal liver spleen 1NFLS Homo sapiens cDNA clone | 2.852 |
| 109431 | AA227972 | Hs.43635 | ESTs | 2.85 |
| 105077 | AA142919 | Hs.5558 | ESTs | 2.847 |
| 131388 | R34531 | Hs.92200 | KIAA0480 gene product | 2.846 |
| 121080 | AA398720 | Hs.177953 | ESTs | 2.838 |
| 112575 | R73816 | Hs.17385 | ESTs | 2.836 |
| 130244 | R26206 | Hs.153293 | KIAA0701 protein | 2.825 |
| 134698 | AA427783 | Hs.77910 | 3-hydroxy-3-methylglutaryl-Coenzyme A synthase 1 (soluble) | 2.816 |
| 116355 | AA504356 | Hs.88650 | ESTs | 2.813 |
| 115316 | AA280627 | Hs.57846 | ESTs | 2.806 |
| 129677 | U48736 | Hs.198891 | serine/threonine-protein kinase PRP4 homolog | 2.8 |
| 130971 | H20332 | Hs.28707 | signal sequence receptor, gamma (translocon-associated protein gamma) | 2.799 |
| 115054 | AA252863 | Hs.87729 | ESTs | 2.795 |
| 130285 | AA063546 | Hs.202968 | ESTs | 2.792 |
| 124308 | H93575 | Hs.227146 | Homo sapiens mRNA; cDNA DKFZp564J142 (from clone DKFZp564J142) | 2.783 |
| 125502 | AA732329 | Hs.191959 | ESTs | 2.778 |
| 114800 | AA159825 | Hs.131887 | ESTs; Weakly similar to ORF YNL227c [S.cerevisiae] | 2.768 |
| 128625 | AA242816 | Hs.102652 | ESTs; Weakly similar to KIAA0437 [H.sapiens] | 2.766 |
| 130159 | H51098 | Hs.151310 | PDZ domain protein (Drosophila inaD-like) | 2.75 |
| 107127 | AA620504 | Hs.22119 | ESTs | 2.742 |
| 113547 | T90746 | Hs.15233 | ESTs | 2.734 |
| 104639 | AA004622 | Hs.18214 | ESTs | 2.727 |
| 127609 | AA622559 | Hs.150318 | ESTs | 2.726 |
| 106922 | AA490964 | Hs.10056 | ESTs | 2.725 |
| 124825 | R52088 | | yg85c3.s1 Soares infant brain 1NIB Homo sapiens cDNA clone | 2.725 |
| 124333 | H98683 | Hs.154054 | ESTs | 2.708 |
| 117634 | N36421 | Hs.107854 | ESTs; Weakly similar to SODIUM- AND CHLORIDE-DEPENDENTGLYCINE | |
| | | TRANSP | | 2.706 |
| 101609 | M54927 | Hs.1787 | proteolipid protein 1(Pelizaeus-Merzbacher disease; spastic paraplegia 2; | |
| | | uncomplicated) | | 2.704 |
| 117142 | H96908 | Hs.42251 | ESTs | 2.7 |
| 112602 | R79147 | Hs.203365 | ESTs | 2.695 |
| 106828 | AA481505 | Hs.13797 | ESTs | 2.68 |
| 124377 | N25996 | Hs.179833 | ESTs | 2.675 |
| 101026 | J04970 | | carboxypeptidase M | 2.675 |
| 124560 | N66393 | Hs.102754 | ESTs | 2.675 |
| 124066 | H02494 | Hs.101615 | ESTs | 2.671 |
| 130281 | R12777 | Hs.15395 | ESTs; Weakly similar to ARGINYL-TRNA SYNTHETASE [H.sapiens] | 2.66 |
| 110949 | N49602 | Hs.13308 | ESTs | 2.65 |
| 111031 | N54839 | Hs.221085 | ESTs; Highly similar to mediator [H.sapiens] | 2.633 |
| 121770 | AA421714 | Hs.11469 | KIAA0896 protein | 2.63 |
| 134132 | U32519 | Hs.220689 | Ras-GTPase-activating protein SH3-domain-binding protein | 2.626 |
| 112424 | R62452 | Hs.191265 | ESTs | 2.625 |
| 122544 | AA451679 | Hs.194410 | ESTs | 2.625 |
| 134425 | X90568 | Hs.172004 | titin | 2.624 |
| 111114 | N63391 | Hs.9238 | ESTs | 2.619 |
| 116119 | AA459242 | Hs.44445 | ESTs; Weakly similar to Kelch motif containing protein [H.sapiens] | 2.615 |
| 112079 | R44164 | Hs.23014 | ESTs | 2.6 |
| 123033 | AA481271 | Hs.193945 | ESTs | 2.591 |
| 124196 | H52617 | Hs.144167 | ESTs | 2.586 |
| 125873 | H14437 | | yl25a04.r1 Soares breast 3NbHBst Homo sapiens cDNA clone | 2.58 |
| 117684 | N40184 | Hs.45050 | ESTs | 2.575 |
| 134938 | D30037 | Hs.168326 | phosphotidylinositol transfer protein; beta | 2.575 |
| 131822 | AA215647 | Hs.200332 | ESTs | 2.568 |
| 135185 | U71203 | Hs.96038 | Ric (Drosophila)-like; expressed in many tissues | 2.564 |
| 117690 | N40467 | Hs.93834 | ESTs | 2.557 |
| 118807 | N78582 | Hs.50732 | protein kinase; AMP-activated; beta 2 non-catalytic subunit | 2.552 |
| 121369 | AA405657 | Hs.128791 | Human DNA sequence from clone 967N21 on chromosome 20p12.3-13. Contains | 2.55 |
| 114860 | AA235112 | Hs.106227 | ESTs; Moderately similar to similar to murine RNA-binding protein [H.sapiens] | 2.549 |
| 121857 | AA426017 | Hs.62694 | ESTs; Highly similar to DNA-REPAIR PROTEIN COMPLEMENTING | 2.548 |
| 110190 | H20560 | Hs.244624 | ESTs | 2.548 |
| 132573 | AA045333 | Hs.51743 | ESTs; Weakly similar to !! ALU SUBFAMILY SB2 WARNING ENTRY !! [H.sapiens] | 2.542 |
| 109706 | F09729 | Hs.12780 | ESTs | 2.537 |
| 135109 | AA410391 | Hs.94592 | klotho | 2.525 |
| 132810 | R37027 | Hs.5737 | KIAA0475 gene product | 2.525 |
| 124879 | R73588 | Hs.101533 | ESTs | 2.525 |
| 103840 | AA174190 | Hs.50932 | ESTs | 2.525 |
| 119066 | R22196 | Hs.34492 | ESTs | 2.519 |
| 114833 | AA234362 | Hs.87310 | ESTs; Moderately similar to CGI-66 protein [H.sapiens] | 2.507 |
| 112998 | T23555 | Hs.103288 | ESTs | 2.5 |
| 123312 | AA496258 | Hs.99601 | ESTs | 2.499 |
| 121873 | AA426270 | Hs.145696 | splicing factor (CC1.3) | 2.491 |
| 123321 | AA496884 | Hs.23972 | ESTs | 2.491 |
| 107760 | AA018042 | Hs.95078 | EST | 2.483 |
| 102580 | U60808 | Hs.152981 | CDP-diacylglycerol synthase (phosphatidate cytidylyltransferase) 1 | 2.481 |
| 103053 | X56741 | Hs.5947 | met transforming oncogene (derived from cell line NK14)-RAB8 homolog | 2.475 |
| 124756 | R38100 | Hs.106294 | ESTs | 2.475 |
| 112936 | T15665 | Hs.6185 | ESTs; Weakly similar to BcDNA.GH12174 [D.melanogaster] | 2.475 |
| 125178 | W58202 | Hs.125731 | ESTs | 2.475 |
| 112423 | R62447 | Hs.22123 | ESTs | 2.471 |
| 123515 | AA600323 | Hs.112535 | EST | 2.462 |
| 102842 | U95020 | Hs.21903 | calcium channel; voltage-dependent; beta 4 subunit | 2.457 |
| 102400 | U42390 | Hs.171957 | triple functional domain (PTPRF interacting) | 2.455 |
| 113187 | T56056 | Hs.9992 | ESTs | 2.452 |
| 131687 | L11066 | Hs.3069 | heat shock 70kD protein 9B (mortalin-2) | 2.448 |
| 115314 | AA280583 | Hs.256501 | ESTs | 2.437 |
| 128211 | AI206427 | Hs.166707 | ESTs; Highly similar to Ran-binding protein 2 [H.sapiens] | 2.43 |
| 134281 | L11005 | Hs.81047 | aldehyde oxidase 1 | 2.425 |
| 115985 | AA447709 | Hs.132094 | ESTs; Moderately similar to putative transcription factor CA150 [H.sapiens] | 2.425 |
| 111348 | N90041 | Hs.9585 | ESTs | 2.418 |
| 129430 | AA258842 | Hs.197877 | Homo sapiens clone 23777 putative transmembrane GTPase mRNA; partial cds | 2.418 |
| 133863 | C13990 | Hs.76930 | synuclein; alpha (non A4 component of amyloid precursor) | 2.417 |
| 111164 | N66857 | Hs.14808 | ESTs; Weakly similar to !! ALU CLASS C WARNING ENTRY !! [H.sapiens] | 2.416 |
| 132143 | AA257056 | Hs.7972 | KIAA0871 protein | 2.412 |
| 130330 | M55047 | Hs.154679 | synaptotagmin 1 | 2.408 |
| 114219 | Z39451 | Hs.27389 | ESTs | 2.406 |
| 117101 | H94043 | Hs.24341 | DKFZP586l1419 protein | 2.403 |
| 125433 | AA034325 | Hs.54320 | ESTs | 2.4 |
| 111099 | N62506 | Hs.21958 | ESTs | 2.4 |
| 120323 | AA195405 | Hs.110347 | Homo sapiens mRNA for alpha integrin binding protein 80; partial | 2.397 |
| 118624 | N69998 | Hs.21801 | ESTs | 2.394 |
| 123570 | AA608955 | Hs.109653 | ESTs | 2.389 |
| 123562 | AA608893 | Hs.190065 | ESTs | 2.388 |
| 131546 | AA262821 | Hs.28578 | muscleblind (Drosophila)-like | 2.385 |
| 103143 | X66141 | Hs.75535 | myosin; light polypeptide 2; regulatory; cardiac; slow | 2.384 |
| 123645 | AA609310 | Hs.188691 | ESTs | 2.383 |
| 130123 | AA001835 | Hs.150390 | zinc finger protein 262 | 2.379 |
| 131682 | AA428368 | Hs.30654 | ESTs | 2.378 |
| 115909 | AA436666 | Hs.59761 | ESTs | 2.375 |
| 125168 | W45574 | Hs.252497 | ESTs | 2.372 |
| 123973 | C14805 | Hs.182151 | ESTs | 2.361 |
| 135197 | U76456 | | Homo sapiens tissue inhibitor of metalloproteinase 4 mRNA complete cds | 2.357 |
| 118689 | N71545 | Hs.184544 | ESTs | 2.357 |
| 107734 | AA016225 | Hs.93386 | ESTs | 2.354 |
| 124590 | N69220 | Hs.41381 | ESTs; Weakly similar to ubiquitin hydrolyzing enzyme I [H.sapiens] | 2.35 |
| 111163 | N66850 | Hs.17606 | ESTs | 2.348 |
| 112349 | R58877 | Hs.22665 | ESTs; Moderately similar to dJ83L6.1 [H.sapiens] | 2.345 |
| 129076 | AA262179 | Hs.169343 | ESTs | 2.345 |
| 134238 | R81509 | Hs.184571 | splicing factor, arginine/serine-rich 11 | 2.341 |
| 116766 | H13260 | Hs.95097 | ESTs | 2.336 |
| 106331 | AA436853 | Hs.34795 | ESTs | 2.333 |
| 129003 | AA443752 | Hs.10784 | ESTs | 2.332 |
| 132368 | AA599814 | Hs.46637 | ESTs; Weakly similar to cDNA EST yk289g5.5 comes form this gene [C.elegans] | 2.332 |
| 124697 | R06273 | Hs.186467 | ESTs; Modly smlr to !! ALU SUBFAMILY J WARNING ENTRY !! [H.sapiens] | 2.322 |
| 120273 | AA176688 | Hs.221139 | ESTs | 2.313 |
| 127110 | AA304993 | Hs.100861 | ESTs; Weakly similar to p60 katanin [H.sapiens] | 2.307 |
| 105450 | AA252621 | Hs.93842 | ESTs | 2.301 |
| 119819 | W74371 | Hs.58383 | ESTs | 2.297 |
| 102302 | U33052 | Hs.69171 | protein kinase C-like 2 | 2.288 |
| 130596 | N74353 | Hs.16475 | ESTs | 2.282 |
| 114161 | Z38904 | Hs.22385 | ESTs; Weakly similar to KIAA0970 protein [H.sapiens] | 2.278 |
| 130542 | U64675 | | Human sperm membrane protein BS-63 mRNA, complete cds | 2.277 |
| 104491 | N71513 | Hs.39328 | ESTs | 22.75 |
| 116988 | H82527 | | ys69e12.s1 Soares retina N2b4HR Homo sapiens cDNA clone | 2.275 |
| 126823 | AA370120 | Hs.7870 | ESTs; Weakly similar to Ylr350wp [S.cerevisiae] | 2.273 |
| 108800 | AA129731 | Hs.90424 | ESTs | 2.273 |
| 101310 | L41607 | Hs.934 | glucosaminyl (N-acetyl) transferase 2; l-branching enzyme | 2.269 |
| 126842 | W19498 | Hs.21085 | ESTs | 2.255 |
| 127251 | AA936428 | Hs.128638 | ESTs | 2.251 |
| 124647 | N91947 | Hs.125033 | ESTs | 2.249 |
| 127112 | AI143906 | Hs.125103 | ESTs | 2.247 |
| 101973 | S82597 | Hs.80120 | UDP-N-acetyl-alpha-D-galactosamine:polypeptide | 2.246 |
| 120999 | AA398302 | Hs.127437 | ESTs | 2.245 |
| 130225 | AA599583 | Hs.15299 | HMBA-inducible | 2.243 |
| 119980 | W88678 | Hs.249247 | heterogeneous nuclear protein similar to rat helix destabilizing protein | 2.243 |
| 124222 | H61053 | Hs.222844 | ESTs | 2.24 |
| 129199 | H90914 | Hs.128629 | ESTs | 2.236 |
| 106802 | AA479101 | Hs.16570 | ESTs; Weakly similar to !! ALU SUBFAMILY SQ WARNING ENTRY !! [H.sapiens] | 2.231 |
| 126160 | N90960 | Hs.247277 | ESTs; Weakly similar to transformation-related protein [H.sapiens] | 2.229 |
| 104627 | AA001976 | Hs.19603 | ESTs | 2.228 |
| 106474 | AA450212 | Hs.42484 | Homo sapiens mRNA;cDNA DKFZp564C053 (from clone DKFZp564C053) | 2.226 |
| 113096 | T40927 | Hs.8345 | ESTs | 2.225 |
| 135336 | AA452822 | Hs.99027 | ESTs | 2.225 |
| 135344 | R62976 | Hs.168491 | ESTs; Moderately similar to TRF1-interacting ankyrin-related | 2.225 |
| 126156 | AA508354 | Hs.118448 | ESTs; Moderately similar to AKT3 protein kinase [H.sapiens] | 2.222 |
| 128885 | AA397841 | Hs.180141 | cofilin 2 (muscle) | 2.218 |
| 107900 | AA026385 | Hs.176600 | ESTs; Moderately similar to !! ALU SUBFAMILY SB2 WARNING | 2.217 |
| 114481 | AA033562 | Hs.151572 | ESTs | 2.212 |
| 109292 | AA199828 | Hs.188662 | ESTs | 2.212 |
| 104257 | AF006265 | Hs.9222 | estrogen receptor-binding fragment-associated gene 9 | 2.209 |
| 132932 | T15482 | Hs.6093 | ESTs | 2.204 |
| 127392 | AA262728 | Hs.14896 | Homo sapiens clone 24590 mRNA sequence | 2.204 |
| 104641 | AA004652 | Hs.18564 | ESTs | 2.2 |
| 122529 | AA449828 | Hs.99229 | ESTs | 2.195 |
| 124307 | H93562 | Hs.162395 | proline synthetase co-transcribed (bacterial homolog) | 2.193 |
| 133601 | S95936 | Hs.75155 | transferrin | 2.193 |
| 119904 | W85709 | Hs.128927 | ESTs; Weakly similar to !! ALU SUBFAMILY SP WARNING ENTRY !! [H.sapiens] | 2.192 |
| 100348 | D64109 | Hs.4994 | transducer of ERBB2; 2 (TOB2) | 2.185 |
| 126871 | AA351779 | Hs.200334 | ESTs | 2.18 |
| 127793 | AI298835 | Hs.30445 | ESTs; Weakly similar to transcription regulator Staf-50 [H.sapiens] | 2.178 |
| 105149 | AA169253 | Hs.8958 | ESTs | 2.177 |
| 121367 | AA405648 | | zw39g8.s1 Soares_total_fetus_Nb2HF8_9w H sapiens cDNA clone IMAGE:772478 | 2.177 |
| 111836 | R36228 | Hs.25119 | ESTs | 2.175 |
| 133394 | R16759 | Hs.237225 | ribosomal protein S5 pseudogene 1 | 2.175 |
| 123207 | AA489697 | Hs.145053 | ESTs | 2.175 |
| 129801 | F11087 | Hs.239666 | ESTs | 2.175 |
| 103393 | X94612 | Hs.41749 | protein kinase; cGMP-dependent; type II | 2.161 |
| 132415 | AA043223 | Hs.4815 | nudix (nucleoside diphosphate linked moiety X)-type motif 3 | 2.157 |
| 106369 | AA443828 | Hs.25324 | ESTs | 2.157 |
| 122963 | AA478446 | Hs.69559 | KIAA1096 protein | 2.156 |
| 133473 | M19309 | Hs.73980 | troponin T1; skeletal; slow | 2.155 |
| 134257 | C06270 | Hs.8078 | Homo sapiens mRNA; cDNA DKFZp586L081 (from clone DPFZp586L081) | 2.155 |
| 135156 | AA056012 | Hs.9552 | binder of Arl Two | 2.151 |
| 104055 | AA393755 | Hs.117211 | ESTs; Highly similar to CGI-62 protein [H.sapiens] | 2.15 |
| 102313 | U33921 | | HSU33921 Clontech adult lung cDNA library (HL1158a) Homo sapiens cDNA | 2.15 |
| 109788 | F10638 | Hs.12432 | Homo sapiens clone 24407 mRNA sequence | 2.15 |
| 103507 | Y10032 | Hs.159640 | serum/glucocorticoid regulated kinase | 2.15 |
| 116000 | AA448710 | Hs.41327 | ESTs | 2.15 |
| 105858 | AA399164 | Hs.227676 | ESTs; Moderately similar to !! ALU SUBFAMILY SQ | 2.137 |
| 103153 | X66534 | Hs.75295 | guanylate cyclase 1; soluble: alpha 3 | 2.137 |
| 126202 | AA652238 | Hs.199726 | ESTs | 2.135 |
| 115955 | AA446121 | Hs.44198 | Homo sapiens BAC clone RG054D04 from 7q31 | 2.134 |
| 104164 | AA458770 | Hs.27023 | KIAA0917 protein | 2.132 |
| 108692 | AA121270 | Hs.82960 | ESTs | 2.128 |
| 122878 | AA465341 | Hs.99640 | ESTs | 2.126 |
| 134771 | L13939 | Hs.89576 | adaptor-related protein complex 1; beta 1 subunit | 2.125 |
| 104298 | D31120 | Hs.40368 | adaptor-related protein complex 1; sigma 2 subunit | 2.125 |
| 104840 | AA039595 | Hs.42458 | Homo sapiens mRMA; cDNA DKFZp586C1817 (from clone DKFZp586C1817) | 2.125 |
| 122180 | AA435798 | Hs.98835 | ESTs; Moderately similar to putative ring zinc finger protein | 2.125 |
| 131012 | H01992 | Hs.202949 | KIAA1102 protein | 2.125 |
| 134092 | H17490 | Hs.7905 | ESTs; Highly similar to sorting nexin 9 [H.sapiens] | 2.123 |
| 118617 | N69666 | Hs.183413 | ESTs; Modtly smlr to !! ALU SUBFAMILY J WARNING ENTRY !! [H.sapiens] | 2.123 |
| 107155 | AA621202 | Hs.7946 | DKFZP586D1519 protein | 2.12 |
| 130925 | N71935 | Hs.169378 | multiple PDZ domain protein | 2.12 |
| 135167 | U63717 | Hs.95821 | osteoclast stimulating factor 1 | 2.118 |
| 105952 | AA405263 | Hs.181400 | ESTs | 2.109 |
| 110308 | H38148 | Hs.32775 | ESTs | 2.108 |
| 116368 | AA521186 | Hs.94217 | ESTs | 2.107 |
| 132939 | U76189 | Hs.61152 | exostoses(multiple)-like 2 | 2.102 |
| 117881 | N50073 | Hs.84926 | ESTs; Highly similar to B-IND1 protein [M.musculus] | 2.1 |
| 121723 | AA419622 | Hs.104800 | ESTs; Weakly similar to Mouse 19.5 mRNA; complete cds [M.musculus] | 2.096 |
| 103500 | Y09443 | Hs.22580 | alkylglycerone phosphate synthase | 2.094 |
| 121429 | AA406293 | Hs.193498 | ESTs | 2.093 |
| 134632 | AA398710 | Hs.174139 | chloride channel 3 | 2.091 |
| 129785 | F10980 | Hs.184780 | ESTs | 2.09 |
| 111065 | N58193 | Hs.18740 | ESTs; Weakly similar to 1-evidence | 2.089 |
| 114710 | AA129931 | Hs.79081 | protein phosphatase 1; catalytic subunit; gamma isoform | 2.083 |
| 132711 | N73702 | Hs.238927 | ESTs | 2.083 |
| 133377 | R05490 | Hs.7239 | SEC24 (S.cerevisiae) related gene family member B | 2.079 |
| 124773 | R40923 | Hs.106604 | ESTs | 2.078 |
| 117759 | N47587 | Hs.97345 | ESTs; Weakly similar to TROPOMODULIN [H.sapiens] | 2.076 |
| 127386 | AI457411 | Hs.106728 | ESTs | 2.076 |
| 101167 | L15309 | Hs.193677 | zinc finger protein 141 (clone pHZ-44) | 2.075 |
| 109597 | F02582 | Hs.14474 | ESTs | 2.074 |
| 124390 | N29325 | Hs.7535 | ESTs; Highly similar to COBW-like placental protein [H.sapiens] | 2.07 |
| 116225 | AA478609 | Hs.47278 | Human Chromosome 16 BAC clone CIT987SK-A-735G6 | 2.07 |
| 131243 | R16667 | Hs.24752 | spectrin SH3 domain binding protein 1 | 2.069 |
| 130557 | T90830 | Hs.15981 | ESTs; Weakly similar to line-1 protein ORF2 [H.sapiens] | 2.067 |
| 134103 | D14826 | Hs.155924 | cAMP responsive element modulator | 2.064 |
| 108833 | AA131866 | Hs.61661 | ESTs; Weakly similar to DY3.6 [C.elegans] | 2.063 |
| 112286 | R53765 | Hs.158135 | KIAA0981 protein | 2.063 |
| 125624 | AA165411 | | zq49a01.r1 Stratagene hNT neuron (#937233) Homo sapiens cDNA clone | 2.061 |
| 124612 | N72200 | Hs.13913 | ESTs | 2.058 |
| 116335 | AA495830 | Hs.87013 | ESTs | 2.057 |
| 112248 | R51361 | Hs.23423 | ESTs | 2.056 |
| 115789 | AA424754 | Hs.43149 | ESTs | 2.056 |
| 107029 | AA599219 | Hs.187492 | ESTs; Weakly similar to ALR [H.sapiens] | 2.056 |
| 110294 | H30270 | Hs.165062 | ESTs | 2.054 |
| 120532 | AA262354 | Hs.186648 | ESTs | 2.054 |
| 118180 | N59249 | Hs.48349 | ESTs | 2.052 |
| 132018 | AA293194 | Hs.3737 | ESTs | 2.052 |
| 132617 | AA171913 | Hs.5338 | carbonic anhydrase XII | 2.05 |
| 131526 | N36167 | Hs.28274 | ESTs | 2.05 |
| 113254 | T64438 | Hs.11449 | DKFZP564O123 protein | 2.05 |
| 122785 | AA459978 | Hs.99508 | ESTs | 2.05 |
| 107203 | D20426 | Hs.5656 | EST | 2.05 |
| 105713 | AA291321 | Hs.184319 | ESTs; Modorately similar to KIAA1006 protein [H.sapiens] | 2.046 |
| 129385 | D82675 | Hs.110950 | Homo sapiens clone 25007 mRNA sequence | 2.042 |
| 119116 | R43845 | Hs.64595 | DKFZP566E2346 protein | 2.04 |
| 116405 | AA600253 | Hs.55601 | ESTs; Highly similar to host cell factor 2 [H.sapiens] | 2.04 |
| 125924 | AA526849 | Hs.82109 | syndecan 1 | 2.039 |
| 105599 | AA279442 | Hs.143460 | protein kinase C; nu | 2.037 |
| 119741 | W70205 | Hs.43670 | kinesin family member 3A | 2.037 |
| 101449 | M21494 | Hs.118843 | creatine kinase; muscle | 2.036 |
| 107109 | AA609943 | Hs.32793 | ESTs | 2.034 |
| 117040 | H89112 | | yw25e5.s1 Morton Fetal Cochlea Homo sapiens cDNA clone IMAGE:25328 | 2.034 |
| 132906 | AA142857 | Hs.234896 | ESTs; Highly similar to geminin [H.sapiens] | 2.031 |
| 105479 | AA255546 | Hs.23467 | ESTs | 2.027 |
| 102031 | U04898 | Hs.2156 | RAR-related orphan receptor A | 2.027 |
| 119846 | W80363 | Hs.58446 | ESTs | 2.024 |
| 124809 | R46482 | Hs.106875 | ESTs | 2.024 |
| 130286 | AA041548 | Hs.154023 | KIAA0573 protein | 2.023 |
| 124457 | N50114 | Hs.128704 | ESTs | 2.017 |
| 125144 | W37999 | Hs.24336 | ESTs | 2.017 |
| 120581 | AA281257 | Hs.125868 | ESTs | 2.014 |
| 104931 | AA062731 | Hs.108319 | thyroid hormone receptor-associated protein; 150 kDa subunit | 2.012 |
| 120548 | AA278846 | Hs.187634 | ESTs | 2.011 |
| 113933 | W81362 | Hs.30567 | ESTs | 2.011 |
| 123072 | AA485041 | Hs.104308 | ESTs | 2.009 |
| 123648 | AA609323 | Hs.112689 | ESTs | 2.008 |
| 116875 | H67749 | Hs.161022 | EST | 2.003 |
| 103179 | X69398 | Hs.82685 | CD47 antigen (Rh-related antigen; integrin-associated signal transducer) | 1.995 |
| 103478 | Y07755 | Hs.38991 | S100 calcium-binding protein A2 | 1.995 |
| 111007 | N53378 | Hs.22543 | ESTs | 1.995 |
| 120470 | AA251797 | | zs11l3.s1 NCI_CGAP_GCB1 Homo sapiens cDNA clone | 1.989 |
| 112280 | R53457 | Hs.26040 | ESTs; Weakly similar to fatty acid omega-hydroxylase [H.sapiens] | 1.989 |
| 114127 | Z38652 | Hs.106961 | ESTs; Weakly similar to TYL [H.sapiens] | 1.988 |
| 129863 | AA151005 | Hs.129872 | sperm surface protein | 1.988 |
| 106320 | AA436608 | | ESTs | 1.988 |
| 108933 | AA147224 | Hs.71814 | ESTs | 1.986 |
| 105906 | AA401633 | Hs.22380 | ESTs | 1.982 |
| 109029 | AA157911 | Hs.72200 | ESTs | 1.982 |
| 118470 | N66769 | Hs.82781 | ESTs | 1.975 |
| 115358 | AA281886 | Hs.88923 | ESTs | 1.975 |
| 115257 | AA279060 | Hs.193516 | B-cell CLL/lymphoma 10 | 1.974 |
| 126879 | AA719776 | | zh38g04.s1 Soares_pineal_gland_N3HPG Homo sapiens cDNA clone IMAGE:414390 | 1.974 |
| 109547 | F01479 | Hs.26966 | ESTs | 1.973 |
| 127111 | AA805726 | Hs.220509 | ESTs | 1.969 |
| 101266 | L36645 | Hs.73964 | EphA4 | 1.966 |
| 129319 | AA037467 | Hs.30340 | ESTs | 1.965 |
| 106211 | AA428240 | Hs.126083 | ESTs | 1.962 |
| 112753 | R93696 | Hs.169882 | ESTs | 1.961 |
| 120489 | AA255538 | Hs.190504 | ESTs | 1.959 |
| 129699 | AA458578 | Hs.12017 | KIAA0439 protein; homolog of yeast ubiquitin-protein ligase Rsp5 | 1.956 |
| 105425 | AA251129 | Hs.24416 | ESTs | 1.953 |
| 134740 | L37362 | Hs.89455 | oploid receptor; kappa 1 | 1.95 |
| 109324 | AA210700 | Hs.86405 | Homo sapiens mRNA; cDNA DKFZp564P056 (from clone DKFZp564P056) | 1.95 |
| 124303 | H93043 | Hs.107070 | ESTs | 1.95 |
| 102337 | U36922 | | Human fork head domain protein (FKHR) mRNA, 3' end | 1.948 |
| 109441 | AA228100 | Hs.86998 | nuclear factor of activated T-cells 5 | 1.946 |
| 127364 | AA179573 | Hs.90061 | progesterone binding protein | 1.942 |
| 105255 | AA227498 | Hs.3623 | ESTs | 1.942 |
| 130672 | L19783 | Hs.177 | phosphatidylinositol glycan; class H | 1.942 |
| 104301 | D45332 | Hs.6783 | ESTs | 1.94 |
| 132442 | R62589 | Hs.167419 | ESTs | 1.939 |
| 105519 | AA258063 | Hs.23438 | ESTs | 1.937 |
| 132902 | AA490969 | Hs.168147 | ESTs | 1.936 |
| 118873 | N89881 | Hs.44577 | ESTs | 1.936 |
| 114124 | Z38595 | Hs.125019 | ESTs; Highly similar to KIAA0886 protein [H.sapiens] | 1.934 |
| 115075 | AA255486 | Hs.88045 | ESTs | 1.933 |
| 110695 | H93463 | Hs.124777 | ESTs | 1.931 |
| 105360 | AA236209 | Hs.187626 | ESTs | 1.931 |
| 124998 | T56013 | Hs.77910 | 3-hydroxy-3-methylglutaryl-Coenzyme A synthase 1 (soluble) | 1.929 |
| 121816 | AA424814 | Hs.187509 | ESTs | 1.927 |
| 111717 | R23241 | Hs.110776 | STAT induced STAT inhibitor-2 | 1.925 |
| 128874 | H06245 | Hs.106801 | ESTs | 1.925 |
| 109391 | AA219699 | Hs.184245 | KIAA0929 protein Msx2 interacting nuclear target (MINT) homolog | 1.913 |
| 126129 | H82165 | Hs.40334 | ESTs | 1.911 |
| 115553 | AA369027 | Hs.71414 | ESTs | 1.905 |
| 113811 | W44928 | Hs.4878 | ESTs | 1.905 |
| 108345 | AA070906 | | zm66d1.s1 Stratagene neuroepithelium (#937231) Homo sapiens cDNA clone | 1.904 |
| 120472 | AA251875 | Hs.104472 | ESTs; Weakly similar to Gag-Pol polyprotein [M.musculus] | 1.903 |
| 116602 | D80063 | Hs.241673 | EST | 1.901 |
| 121121 | AA399371 | Hs.189095 | ESTs; Weakly similar to zinc finger protein SALL1 [H.sapiens] | 1.9 |
| 125330 | AA401804 | Hs.114574 | ESTs | 1.896 |
| 130095 | F01831 | Hs.14838 | ESTs | 1.894 |
| 119782 | W72982 | Hs.58262 | ESTs | 1.894 |
| 104115 | AA428090 | Hs.26102 | ESTs | 1.893 |
| 131313 | C17938 | Hs.22370 | Homo sapiens mRNA;cDNA DKFZp564O0122 (from clone DKFZp564O0122) | 1.891 |
| 105583 | AA278907 | Hs.24549 | ESTs | 1.891 |
| 122825 | AA461195 | Hs.99580 | ESTs | 1.887 |
| 119495 | W35390 | Hs.55533 | ESTs | 1.886 |
| 130309 | AA134289 | Hs.15423 | Homo sapiens BAC clone RG114B19 from 7q31.1 | 1.886 |
| 125628 | AA418069 | Hs.241493 | natural killer-tumor recognition sequence | 1.886 |
| 110611 | H66947 | Hs.14671 | ESTs; Highly simliar to gene ERCC5 protein [H.sapiens] | 1.885 |
| 117301 | N22569 | Hs.43215 | ESTs | 1.884 |
| 131406 | N92239 | Hs.26471 | Wnt inhibitory factor-1 | 1.881 |
| 126428 | AA013312 | Hs.64988 | ESTs | 1.881 |
| 120285 | AA182882 | Hs.111110 | titin-cap (telethonin) | 1.878 |
| 112724 | R91753 | Hs.17757 | ESTs | 1.878 |
| 103121 | X63679 | Hs.4147 | translocating chain-associating membrane protein | 1.875 |
| 124381 | N26765 | Hs.109008 | ESTs | 1.875 |
| 117226 | N20468 | Hs.177322 | ESTs; Weakly similar to putative p150 [H.sapiens] | 1.875 |
| 105610 | AA279991 | Hs.124691 | ESTs; Weakly similar to trithorax homologue 2 [H.sapiens] | 1.875 |
| 111229 | N69113 | Hs.110855 | ESTs | 1.875 |
| 120627 | AA285079 | Hs.190474 | ESTs | 1.873 |
| 107048 | AA600012 | Hs.10669 | ESTs; Moderately similar to KIAA0400 [H.sapiens] | 1.872 |
| 104041 | AA381902 | Hs.197114 | RNA binding protein | 1.872 |
| 115162 | AA258366 | Hs.227806 | ras GTPase activating protein-like | 1.872 |
| 102239 | U26726 | Hs.1376 | hydroxysteroid (11-beta) dehydrogenase 2 | 1.87 |
| 100043 | M10098 | AFFX control: 18S ribosomal RNA | | 1.868 |
| 120296 | AA191353 | Hs.22385 | ESTs; Weakly similar to KIAA0970 protein [H.sapiens] | 1.867 |
| 129011 | S72869 | Hs.107932 | DNA segment; single copy; probe pH4 (transforming sequence; thyroid-1; | 1.867 |
| 134851 | R44479 | Hs.90232 | KIAA0552 gene product | 1.866 |
| 117392 | N26175 | Hs.93405 | ESTs | 1.864 |
| 114530 | AA053027 | Hs.191797 | ESTs | 1.863 |
| 123541 | AA608794 | Hs.112592 | ESTs | 1.863 |
| 124890 | R78618 | Hs.34145 | ESTs; Weakly similar to RAS-RELATED PROTEIN RAB-8 [H.sapiens] | 1.862 |
| 105299 | AA233511 | Hs.194720 | ATP-binding cassette; sub-family G (WHITE); member 2 | 1.861 |
| 103560 | Z20656 | Hs.182787 | myosin; heavy polypept 6; cardiac muscle; alpha (cardiomyopathy, hypertrophic 1) | 1.861 |
| 113073 | T33637 | Hs.6841 | ESTs | 1.86 |
| 120407 | AA235040 | Hs.107283 | ESTs | 1.859 |
| 103892 | AA243523 | Hs.17155 | ESTs | 1.858 |
| 123795 | AA620381 | Hs.70488 | ESTs | 1.857 |
| 108524 | AA084323 | Hs.68138 | ESTs | 1.857 |
| 113953 | W85812 | Hs.187554 | ESTs | 1.856 |
| 110721 | H97678 | Hs.31319 | ESTs | 1.856 |
| 129426 | AA412087 | Hs.168272 | EST; Highly smlr to prot inhibitor of activated STAT prot PlASx-alpha [H.sapiens] | 1.853 |
| 112102 | R44840 | Hs.21303 | ESTs | 1.852 |
| 118502 | N67317 | Hs.50150 | ESTs | 1.852 |
| 107619 | AA004955 | Hs.60015 | ESTs | 1.851 |
| 100436 | D87446 | Hs.75912 | KIAA0257 protein | 1.85 |
| 120652 | AA287312 | Hs.191648 | ESTs | 1.85 |
| 121643 | AA417078 | Hs.193767 | ESTs | 1.843 |
| 117387 | N26011 | Hs.53810 | ESTs | 1.843 |
| 132084 | Y12394 | Hs.3886 | karyopherin alpha 3 (importin alpha 4) | 1.843 |
| 124449 | N48593 | Hs.121820 | ESTs | 1.841 |
| 120263 | AA173440 | Hs.193919 | ESTs | 1.838 |
| 127226 | AA731036 | Hs.3463 | ribosomal protein S23 | 1.838 |
| 111837 | R36447 | Hs.24453 | ESTs | 1.835 |
| 128727 | M64174 | Hs.50651 | Janus kinase 1 (a protein tyrosine kinase) | 1.834 |
| 114439 | AA018937 | Hs.128629 | ESTs | 1.833 |
| 102332 | U35637 | | Human nebulin mRNA, partial cds | 1.83 |
| 126579 | W72979 | Hs.146082 | ESTs | 1.83 |
| 102341 | U37122 | Hs.8110 | adducin 3 (gamma) | 1.83 |
| 114246 | Z39848 | Hs.12079 | ESTs | 1.828 |
| 131757 | D17532 | Hs.316 | DEAD/H(Asp-Glu-Ala-Asp/His) box polypeptide 6 (RNA helicase: 54kD) | 1.823 |
| 108904 | AA136521 | Hs.71148 | ESTs; Weakly similar to putative p150 [H.sapiens] | 1.823 |
| 115084 | AA255566 | Hs.42484 | Homo sapiens mRNA; cDNA DKFZp564C053 (from clone DKFZp564C053) | 1.823 |
| 131957 | AA609008 | Hs.183232 | ESTs | 1.822 |
| 100131 | D12485 | Hs.11951 | phosphodiesterase I/nucleotide pyrophosphatase | |
| | | | 1 (homologous to mouse Ly-41 antigen) | 1.822 |
| 124163 | H30539 | Hs.189838 | ESTs | 1.821 |
| 118204 | N59859 | Hs.48443 | ESTs | 1.821 |
| 107727 | AA016021 | Hs.173091 | DKFZP434K151 protein | 1.82 |
| 100357 | D78156 | Hs.241548 | RAS p21 protein activator 2 | 1.82 |
| 116295 | AA489016 | Hs.91216 | ESTs; Highly similar to partial CDS; human putative tumor suppressor [H.sapiens] | 1.82 |
| 124833 | R54112 | Hs.128697 | ESTs | 1.817 |
| 122587 | AA453255 | Hs.6968 | ESTs | 1.817 |
| 114359 | Z41589 | Hs.153483 | ESTs; Moderately similar to H1 chloride channel [H.sapiens] | 1.815 |
| 111289 | N72253 | Hs.238246 | ESTs | 1.813 |
| 110826 | N30068 | Hs.15347 | ESTs | 1.812 |
| 104106 | AA422123 | Hs.42457 | ESTs | 1.811 |
| 130043 | AA055404 | Hs.193953 | ESTs; Weakly similar to !! ALU SUBFAMILY J WARNING ENTRY !! [H.sapiens] | 1.253 |
| 115864 | AA432080 | Hs.81200 | ESTs | 1.81 |
| 129737 | AA056140 | Hs.122684 | ESTs | 1.81 |
| 124477 | N53158 | Hs.102682 | ESTs | 1.809 |
| 100782 | HG3740-HT4010 | | Basic Transcription Factor 2,34 Kda Subunit | 1.806 |
| 106101 | AA421053 | Hs.34395 | ESTs | 1.806 |
| 115479 | AA287596 | | zs52h09.s1 NCI_CGAP_GCB1 H sapiens cDNA clone IMAGE:701153 | 1.804 |
| 116104 | AA456635 | Hs.78524 | ESTs | 1.804 |
| 114173 | Z39050 | Hs.21963 | ESTs | 1.804 |
| 132632 | N59764 | Hs.5398 | guanine-monophosphate synthetase | 1.803 |
| 119135 | R49548 | Hs.169681 | death effector domain-containing | 1.802 |
| 131559 | N91087 | Hs.28728 | ESTs; Weakly similar to F55A12.9 [C.elegans] | 1.801 |
| 126922 | AA177138 | Hs.161671 | ESTs | 1.8 |
| 117375 | N25427 | Hs.108812 | ESTs | 1.8 |
| 103571 | Z25535 | Hs.211608 | nucleoporin 153kD | 1.8 |
| 105978 | AA406367 | Hs.15973 | ESTs | 1.8 |
| 125904 | H22372 | Hs.163586 | ESTs | 1.799 |
| 133883 | AA397915 | Hs.77221 | choline kinase | 1.798 |
| 105777 | AA348412 | Hs.23096 | ESTs | 1.797 |
| 110166 | H19480 | Hs.174309 | ESTs | 1.796 |
| 105038 | AA130273 | Hs.7584 | ESTs; Weakly similar to hypothetical protein; similar to [H.sapiens] | 1.796 |
| 105427 | AA251330 | Hs.28248 | ESTs | 1.795 |
| 115278 | AA279757 | Hs.67466 | ESTs; Weakly similar to BACN32G11.d [D.melanogaster] | 1.794 |
| 133104 | L13698 | Hs.65029 | growth arrest-specific 1 | 1.794 |
| 131170 | N48674 | Hs.23796 | Human DNA sequence from clone 1052M9 on chromosome Xq25. Contains the | 1.792 |
| 100136 | D13540 | Hs.22868 | protein tyrosine phosphatase; non-receptor type 11 | 1.791 |
| 127263 | AA331157 | | EST35035 Embryo, 6 week, subtracted (total cDNA) I Homo sapiens cDNA | 1.79 |
| 114157 | Z38878 | Hs.24979 | ESTs | 1.79 |
| 125601 | AI096717 | Hs.247043 | KIAA0525 protein | 1.788 |
| 118472 | N66818 | Hs.42179 | ESTs | 1.787 |
| 112456 | R63925 | Hs.28464 | ESTs | 1.787 |
| 130236 | N69682 | Hs.51957 | SC35-interacting protein 1 | 1.786 |
| 133297 | AA600057 | Hs.70266 | KIAA0905 protein | 1.784 |
| 125650 | R40096 | Hs.176578 | ESTs | 1.784 |
| 132056 | T89386 | Hs.38176 | KIAA0606 protein; SCN Circadian Oscillatory Protein (SCOP) | 1.783 |
| 129093 | AA262710 | Hs.108614 | KIAA0627 protein | 1.783 |
| 123176 | AA489020 | Hs.193424 | ESTs | 1.782 |
| 106340 | AA441792 | Hs.22857 | chord domain-containing protein 1 | 1.781 |
| 100598 | HG2463-HT2559 | | Guanine Nucleotide-Binding Protein G25k | 1.779 |
| 104038 | AA374532 | | EST86676 HSC172 cells I Homo sapiens cDNA 5' end, mRNA sequence | 1.778 |
| 122235 | AA436475 | Hs.190104 | ESTs | 1.777 |
| 105104 | AA151771 | Hs.76941 | ATPase; Na+/K+ transporting; beta 3 polypeptide | 1.776 |
| 107601 | AA004636 | Hs.50223 | ESTs | 1.776 |
| 131467 | W68255 | Hs.27194 | DKFZP434K171 protein | 1.776 |
| 118449 | N66413 | Hs.172466 | ESTs; Weakly similar to KIAA0775 protein [H.sapiens] | 1.776 |
| 107969 | AA034030 | Hs.155212 | methylmalonyl Coenzyme A mutase | 1.775 |
| 115527 | AA342079 | Hs.252055 | ESTs | 1.775 |
| 132471 | T16305 | Hs.49349 | beta-site APP-cleaving enzyme | 1.775 |
| 105966 | AA406105 | Hs.5344 | adaptor-related protein complex 1; gamma 1 subunit | 1.774 |
| 127548 | AA373091 | Hs.93832 | Homo sapiens clone 24483 unknown mRNA; parital cds | 1.774 |
| 106217 | AA428379 | Hs.24870 | ESTs | 1.773 |
| 131214 | N26777 | Hs.172635 | ESTs | 1.773 |
| 106295 | AA435664 | Hs.8583 | similar to APOBEC1 | 1.773 |
| 106328 | AA436705 | Hs.28020 | KIAA0766 gene product | 1.772 |
| 124661 | N93797 | Hs.3090 | EphB1 | 1.772 |
| 122988 | AA479166 | Hs.105633 | ESTs | 1.772 |
| 115504 | AA291946 | Hs.42736 | ESTs | 1.771 |
| 105168 | AA180208 | Hs.16606 | ESTs; Highly similar to CGI-32 protein [H.sapiens] | 1.767 |
| 129153 | AA188618 | Hs.181461 | ariadne; Drosophila; homolog of | 1.766 |
| 105829 | AA398290 | Hs.21965 | ESTs | 1.764 |
| 101811 | M86917 | Hs.24734 | oxysterol binding protein | 1.764 |
| 100138 | D13628 | Hs.2463 | angiopoietin 1 | 1.764 |
| 124704 | R07335 | | ye96c1.s1 Soares fetal liver spleen 1NFLS Homo sapiens cDNA clone | 1.763 |
| 122314 | AA442257 | Hs.192076 | ESTs | 1.762 |
| 109865 | H02566 | Hs.191268 | Homo sapiens mRNA; cDNA DKFZp434N174 (from clone DKFZp434N174) | 1.761 |
| 106206 | AA428069 | Hs.89519 | KIAA1046 protein | 1.758 |
| 107135 | AA620782 | Hs.23247 | ESTs | 1.757 |
| 105760 | AA338960 | Hs.28170 | ESTs | 1.756 |
| 106288 | AA435536 | Hs.24336 | ESTs | 1.756 |
| 103968 | AA304566 | Hs.3542 | ESTs | 1.756 |
| 129559 | AA234945 | Hs.11360 | ESTs | 1.756 |
| 117885 | N50112 | Hs.47023 | ESTs | 1.754 |
| 107032 | AA599472 | Hs.247309 | succinate-CoA ligase; GDP-forming; beta subunit | 1.754 |
| 124807 | R45963 | Hs.233811 | ESTs; Weakly similar to ORF2 [M.musculus] | 1.753 |
| 100276 | D42047 | Hs.82432 | KIAA0089 protein | 1.753 |
| 110924 | N47938 | | yy84a09.s1 Soares_multiple_sclerosis_2NbHMSP Homo sapiens cDNA clone | 1.751 |
| 133002 | AF006082 | Hs.62461 | ARP2 (actin-related protein 2; yeast) homolog | 1.751 |
| 132530 | AA455917 | Hs.50785 | SEC22; vesicle trafficking protein (S.cerevisiae)-like 1 | 1.75 |
| 110759 | N21671 | Hs.19025 | ESTs | 1.75 |
| 106138 | AA424515 | Hs.33264 | ESTs | 1.75 |
| 107348 | U43701 | Hs.184776 | ribosomal protein L23a | 1.75 |
| 115867 | AA432162 | Hs.165986 | DKFZP586B2022 protein | 1.749 |
| 135398 | AA194075 | Hs.99908 | nuclear receptor coactivator 4 | 1.747 |
| 113783 | W19222 | Hs.7041 | ESTs; Weakly similar to !! ALU SUBFAMILY SQ WARNING ENTRY !! [H.sapiens] | 1.747 |
| 134898 | X98330 | Hs.90821 | ryanodine receptor 2 (cardiac) | 1.745 |
| 132215 | T10132 | Hs.4236 | KIAA0478 gene product | 1.744 |
| 104229 | AB002346 | Hs.61289 | synaptojanin 2 | 1.743 |
| 116166 | AA461556 | Hs.202949 | KIAA1102 protein | 1.743 |
| 115433 | AA284252 | Hs.58372 | ESTs | 1.743 |
| 114908 | AA236545 | Hs.54973 | ESTs | 1.742 |
| 127425 | AA470941 | Hs.143162 | ESTs | 1.741 |
| 131089 | Z38807 | Hs.22870 | ESTs | 1.739 |
| 113498 | T88908 | Hs.189746 | ESTs | 1.738 |
| 116710 | F10577 | Hs.70312 | ESTs | 1.735 |
| 127210 | R51476 | | yg76f04.r1 Soares infant brain 1NIB Homo sapiens cDNA clone | 1.733 |
| 120554 | AA279654 | Hs.194524 | ESTs | 1.733 |
| 129940 | U18242 | Hs.13572 | calcium modulating ligand | 1.732 |
| 117023 | H88157 | Hs.41105 | ESTs | 1.731 |
| 111700 | R22212 | Hs.23361 | ESTs | 1.731 |
| 116911 | H72240 | Hs.39292 | ESTs; Moderately similar to KIAA0745 protein [H.sapiens] | 1.731 |
| 106025 | AA412063 | Hs.6065 | ESTs | 1.728 |
| 108626 | AA101984 | Hs.61697 | G-protein coupled receptor | 1.726 |
| 111614 | R12581 | Hs.191146 | ESTs | 1.726 |
| 134134 | L76703 | Hs.173328 | protein phosphatase 2; regulatory subunit B (B56); epsilon isoform | 1.725 |
| 106886 | AA489086 | Hs.36545 | ESTs | 1.725 |
| 117998 | N52136 | Hs.93828 | ESTs | 1.725 |
| 121204 | AA400422 | Hs.55896 | ESTs | 1.725 |
| 121342 | AA404995 | Hs.192480 | ESTs | 1.725 |
| 131129 | R27296 | Hs.23240 | ESTs | 1.725 |
| 116235 | AA479181 | Hs.186726 | ESTs | 1.725 |
| 102423 | U44754 | Hs.179312 | small nuclear RNA activating complex; polypeptide 1; 43kD | 1.724 |
| 110273 | H29050 | Hs.24096 | ESTs | 1.722 |
| 108758 | AA127395 | Hs.222414 | ESTs | 1.722 |
| 110672 | H88477 | Hs.191178 | ESTs | 1.721 |
| 120271 | AA176404 | Hs.111092 | ESTs; Weakly similar to ZINC FINGER PROTEIN 136 [H.sapiens] | 1.72 |
| 100227 | D28915 | Hs.82316 | interferon-induced; hepatitis C-associated microtubular aggregate prot (44kD) | 1.719 |
| 129232 | W69459 | Hs.109655 | sex comb on midleg (Drosophila)-like 1 | 1.719 |
| 134663 | W73367 | Hs.8750 | ESTs | 1.717 |
| 104902 | AA055475 | Hs.104143 | clathrin; light polypeptide (Lca) | 1.717 |
| 120582 | AA281290 | Hs.125287 | ESTs; Weakly similar to BC331191_1 [H.sapiens] | 1.717 |
| 134891 | F03517 | Hs.90787 | ESTs | 1.716 |
| 106219 | AA428567 | Hs.26613 | Homo sapiens mRNA; cDNA DKFZp586F1323 (from clone DKFZp586F1323) | 1.715 |
| 116372 | AA521311 | Hs.13854 | ESTs | 1.713 |
| 107570 | AA001870 | Hs.237323 | N-acetylglucosamine-phosphate mutase; DKFZP434B187 protein | 1.713 |
| 106198 | AA427816 | Hs.11803 | ESTs | 1.712 |
| 125136 | W31479 | Hs.129051 | ESTs | 1.712 |
| 104973 | AA085676 | Hs.6763 | KIAA0942 protein | 1.712 |
| 128710 | J04813 | Hs.104117 | cytochrome P450; subfamily IIIA (niphedipine oxidase); polypeptide 5 | 1.711 |
| 123994 | D20899 | Hs.107127 | Homo sapiens mRNA; cDNA DKFZp564G022 (from clone DKFZp564G022) | 1.711 |
| 127871 | AA766511 | Hs.128848 | ESTs | 1.71 |
| 116089 | AA455933 | Hs.41324 | ESTs | 1.709 |
| 123337 | AA504153 | Hs.132797 | ESTs; Weakly similar to ORF YGL050w [S.cerevisiae] | 1.708 |
| 123619 | AA609200 | Hs.162686 | ESTs | 1.708 |
| 104781 | AA026617 | Hs.21610 | ESTs; Highly similar to BAl1-associated protein 1 [H.sapiens] | 1.707 |
| 115114 | AA256468 | Hs.88148 | ESTs | 1.705 |
| 117852 | N49408 | Hs.136102 | KIAA0853 protein | 1.705 |
| 127644 | T57570 | Hs.77039 | ribosomal protein S3A | 1.704 |
| 111359 | N91273 | Hs.27179 | ESTs | 1.702 |
| 131721 | L36644 | Hs.31092 | EphA5 | 1.7 |
| 132438 | F08925 | Hs.48610 | ESTs | 1.7 |
| 132476 | N67192 | Hs.49476 | Homo sapiens clone TUA8 Cri-du-chat region mRNA | 1.7 |
| 130990 | F02488 | Hs.21917 | KIAA0768 protein | 1.7 |
| 128499 | AA487503 | Hs.100636 | ESTs | 1.698 |
| 120780 | AA342337 | Hs.241569 | ESTs; Modtly smlr to !! ALU SUBFAMILY SQ WARNING ENTRY !! [H.sapiens] | 1.697 |
| 132920 | L06133 | Hs.606 | ATPase; Cu++ transporting; alpha polypeptide (Menkes syndrome) | 1.696 |
| 135037 | U77948 | Hs.184122 | general transcription factor II; l | 1.696 |
| 110024 | H11297 | Hs.31050 | ESTs | 1.695 |
| 134415 | AA329274 | Hs.82911 | protein tyrosine phosphatase type IVA; member 2 | 1.694 |
| 102223 | U24685 | Hs.148226 | Human anti-B cell autoantibody IgM heavy chain variable V-D-J region (VH4) | |
| | | | gene; clone E11; VH4-63 non-productive rearrangement | 1.694 |
| 126712 | AA205862 | Hs.7942 | ESTs | 1.694 |
| 101507 | M27492 | Hs.82112 | interleukin 1 receptor; type | I 692 |
| 106291 | AA435551 | Hs.30824 | ESTs | 1.691 |
| 116826 | H58691 | Hs.8215 | ESTs; Weakly similar to double-stranded RNA-binding nuclear | |
| | | | protein DRSBP76 [H.sapiens] | 1.69 |
| 135339 | D59269 | Hs.127842 | Homo sapiens mRNA full length insert cDNA clone EUROIMAGE 783648 | 1.69 |
| 118250 | N62602 | | yz75b6.s1 Soares_multiple_sclerosis_2NbHMSP Homo sapiens cDNA clone | |
| | | | IMAGE:288851 3' similar to contains Alu repetitive element;, mRNA sequence | 1.689 |
| 106470 | AA450116 | Hs.186180 | ESTs | 1.688 |
| 108203 | AA057678 | Hs.63408 | ESTs | 1.687 |
| 119748 | W70313 | Hs.126906 | ESTs | 1.686 |
| 116576 | D51228 | Hs.79404 | neuron-specific protein | 1.683 |
| 123035 | AA481392 | Hs.105166 | ESTs | 1.683 |
| 126668 | AA011616 | Hs.184086 | ESTs | 1.681 |
| 101512 | M28209 | Hs.250716 | RAB1; member RAS oncogene family | 1.678 |
| 102704 | U76638 | Hs.54089 | BRCA1 associated RING domain 1 | 1.677 |
| 126218 | AA256386 | Hs.13649 | Novel human gene mapping to chomosome 13; similarto rat RhoGAP | 1.676 |
| 111180 | N67277 | Hs.9403 | ESTs | 1.676 |
| 105937 | AA404342 | Hs.173531 | ESTs | 1.675 |
| 114118 | Z38520 | Hs.175930 | ESTs | 1.675 |
| 109203 | AA190634 | Hs.108787 | endoplasmic reticulum membrane protein | 1.675 |
| 125245 | W86608 | Hs.7243 | ubiquitin specific protease 24 | 1.675 |
| 102906 | X06956 | Hs.75318 | tubulin; alpha 1(testis specific) | 1.675 |
| 125914 | AA262925 | Hs.180034 | cleavage stimulation factor; 3' pre-RNA; subunit 3; 77kD | 1.674 |
| 134294 | U63289 | Hs.81248 | CUG triplet repeat; RNA-binding protein 1 | 1.674 |
| 109742 | F10108 | Hs.183333 | ESTs | 1.673 |
| 134674 | D63876 | Hs.87726 | KIAA0154 protein | 1.673 |
| 104079 | AA402937 | Hs.103238 | ESTs | 1.671 |
| 107554 | AA001386 | Hs.59844 | ESTs | 1.671 |
| 132439 | AA243139 | Hs.4863 | Homo sapiens clone 25088 mRNA sequence | 1.669 |
| 124515 | N58172 | Hs.109370 | ESTs | 1.668 |
| 124300 | H92575 | Hs.105959 | ESTs; Weakly similar to !! ALU SUBFAMILY SQ WARNING ENTRY !! [H.sapiens] | 1.668 |
| 126809 | AA743475 | Hs.171693 | ESTs | 1.667 |
| 106095 | AA419547 | Hs.11713 | ESTs | 1.664 |
| 101754 | M77142 | Hs.239489 | TIA1 cytotoxic granule-associated RNA-binding protein | 1.663 |
| 105188 | AA192306 | Hs.23926 | ESTs | 1.663 |
| 113582 | T91371 | Hs.16824 | EST | 1.661 |
| 119559 | W38197 | | Accession not listed in Genbank | 1.661 |
| 119961 | W87535 | Hs.59015 | ring finger protein 9 | 1.657 |
| 123255 | AA490890 | Hs.105273 | ESTs | 1.657 |
| 111078 | N59230 | Hs.186574 | ESTs | 1.655 |
| 113082 | T40528 | Hs.8246 | ESTs | 1.654 |
| 119589 | W44692 | Hs.124177 | ESTs | 1.652 |
| 104308 | D53639 | Hs.77904 | ribosomal protein S26 | 1.65 |
| 103073 | X59417 | Hs.74077 | proteasome (prosome; macropain) subunit alpha type; 6 | 1.65 |
| 124424 | N35314 | Hs.107265 | ESTs | 1.65 |
| 128890 | AA096157 | Hs.182364 | ESTs; Weakly similar to 25 kDa trypsin inhibitor [H.sapiens] | 1.65 |
| 119400 | T92767 | | ye27d06.s1 Stratagene lung (#937210) Homo sapiens cDNA clone | |
| | | | IMAGE:118955 3', mRNA sequence. | 1.65 |
| 131631 | AA486868 | Hs.29802 | slit (Drosophila) homolog 2 | 1.65 |
| 118229 | N62339 | Hs.180532 | heat shock 90kD protein 1; alpha | 1.649 |
| 118533 | N67954 | Hs.49413 | ESTs | 1.648 |
| 130666 | AA476307 | Hs.194035 | KIAA0737 gene product | 1.647 |
| 103093 | X60708 | Hs.44926 | dipeptidylpeptidase IV (CD26; adenosine deaminase complexing protein 2) | 1.647 |
| 128667 | U69140 | Hs.103419 | fasciculation and elongation protein zeta 2 (zygin II) | 1.646 |
| 112933 | T15530 | Hs.221439 | ESTs | 1.646 |
| 114546 | AA056263 | Hs.132747 | ESTs | 1.645 |
| 126705 | AA579377 | Hs.180532 | heat shock 90kD protein 1; alpha | 1.644 |
| 114399 | AA007595 | Hs.220937 | ESTs | 1.642 |
| 118836 | N79820 | Hs.50854 | ESTs | 1.64 |
| 100401 | D85423 | | Homo sapiens mRNA for Cdc5, partial cds | 1.64 |
| 105681 | AA284865 | Hs.171228 | KIAA1040 protein | 1.639 |
| 132526 | AA460128 | Hs.5074 | similar to S. pombe dim1+ | 1.639 |
| 133809 | AA034002 | Hs.76359 | catalase | 1.639 |
| 115968 | AA447083 | Hs.134522 | ESTs | 1.637 |
| 116370 | AA521256 | Hs.236204 | ESTs; Moderately similar to NUCLEAR PORE COMPLEX | |
| | | | PROTEIN NUP107 [R.norvegicus] | 1.631 |
| 109644 | F04477 | Hs.204802 | ESTs; Moderately similar to GLYCERALDEHYDE 3-PHOSPHATE | |
| | | | DEHYDROGENASE; LIVER [H.sapiens] | 1.627 |
| 103427 | X97303 | | H.sapiens mRNA for Ptg-12 protein | 1.627 |
| 132186 | T33888 | Hs.221040 | KIAA1038 protein | 1.626 |
| 131428 | U17838 | Hs.26719 | PR domain containing 2; with ZNF domain | 1.626 |
| 126638 | AA649257 | Hs.188602 | ESTs | 1.625 |
| 114503 | AA039568 | Hs.188083 | ESTs | 1.625 |
| 121242 | AA400857 | Hs.97509 | EST | 1.625 |
| 122414 | AA446885 | Hs.99087 | ESTs; Moderately similar to ZINC FINGER PROTEIN 141 [H.sapiens] | 1.625 |
| 110632 | H72344 | Hs.171635 | ESTs | 1.624 |
| 111389 | N95837 | Hs.169111 | ESTs; Weakly similar to L82A [D.melanogaster] | 1.624 |
| 112449 | R63802 | Hs.124186 | ring finger protein 2 | 1.623 |
| 113070 | T33464 | Hs.6298 | ESTs | 1.622 |
| 107229 | D59284 | Hs.34644 | ESTs | 1.618 |
| 132710 | W93726 | Hs.55279 | protease inhibitor 5 (maspin) | 1.617 |
| 124664 | N94814 | Hs.33540 | ESTs; Weakly similar to KIAA0765 protein [H.sapiens] | 1.617 |
| 130166 | AA350690 | Hs.151411 | KIAA0916 protein | 1.616 |
| 125040 | T78451 | Hs.199961 | ESTs | 1.615 |
| 132972 | H39627 | Hs.164967 | ESTs; Weakly similar to !! ALU SUBFAMILY SB WARNING ENTRY !! [H.sapiens] | 1.615 |
| 115873 | AA433916 | Hs.90093 | heat shock 70kD protein 4 | 1.611 |
| 120408 | AA235045 | Hs.190151 | ESTs | 1.61 |
| 120934 | AA383773 | Hs.191500 | ESTs | 1.61 |
| 115259 | AA279071 | Hs.13453 | splicing factor 3b; subunit 1; 155kD | 1.609 |
| 134330 | D20113 | Hs.8185 | ESTs; Highly similar to CGI-44 protein [H.sapiens] | 1.607 |
| 115117 | AA256492 | Hs.49007 | poly(A) polymerase | 1.606 |
| 125162 | W44682 | Hs.109896 | ESTs | 1.605 |
| 103946 | AA285246 | Hs.111650 | ESTs; Weakly similar to Prt1 homolog [H.sapiens] | 1.604 |
| 133389 | AA166917 | Hs.72639 | ESTs | 1.603 |
| 115528 | AA342301 | Hs.53929 | ESTs; Weakly similar to !! ALU CLASS B WARNING ENTRY !! [H.sapiens] | 1.602 |
| 129704 | W81301 | Hs.12064 | ubiquitin specific protease 22 | 1.602 |
| 109313 | AA206800 | Hs.86276 | ESTs; Moderately similar to zinc finger protein dp [H.sapiens] | 1.601 |
| 130457 | U58091 | Hs.155976 | cullin 4B | 1.6 |
| 123076 | AA485211 | Hs.190046 | ESTs | 1.6 |
| 115113 | AA256460 | Hs.44610 | ESTs | 1.6 |
| 117731 | N46433 | Hs.46609 | ESTs | 1.6 |
| 123344 | AA504338 | Hs.171857 | ESTs | 1.599 |
| 131798 | X86098 | Hs.3238 | adenovirus 5 E1A binding protein | 1.597 |
| 125370 | AA256743 | Hs.151791 | KIAA0092 gene product | 1.596 |
| 114918 | AA236813 | Hs.72324 | ESTs; Highly similar to unknown [H.sapiens] | 1.596 |
| 114807 | AA160805 | Hs.199832 | ESTs | 1.596 |
| 105103 | AA151593 | Hs.10130 | ESTs | 1.594 |
| 125004 | T60120 | | yb68f02.s1 Stratagene ovary (#937217) Homo sapiens cDNA clone | |
| | | | IMAGE:76347 3', mRNA sequence. | 1.592 |
| 105658 | AA282914 | Hs.10176 | ESTs | 1.589 |
| 110455 | H52172 | | yt85e8.s1 Soares_pineal_gland_N3HPG Homo sapiens cDNA clone | |
| | | | IMAGE:23111 3'similar to contains Alu repetitive element, mRNA sequence | 1.589 |
| 119780 | W72967 | Hs.191381 | ESTs; Weakly similar to hypothetical protein [H.sapiens] | 1.587 |
| 126983 | AA211537 | | zn55d01.r1 Stratagene muscle 937209 Homo sapiens cDNA clone | |
| | | | IMAGE:562081 5', mRNA sequence. | 1.586 |
| 134675 | AA250745 | Hs.87773 | protein kinase; cAMP-dependent; catalytic; beta | 1.584 |
| 105431 | AA252033 | Hs.15036 | ESTs; Weakly similar to !! ALU SUBFAMILY J WARNING ENTRY !! [H. sapiens] | 1.584 |
| 120187 | Z40251 | Hs.56974 | ESTs | 1.584 |
| 115830 | AA428137 | Hs.86434 | ESTs | 1.581 |
| 135069 | AA456311 | Hs.93961 | ESTs; Weakly similar to !! ALU CLASS A WARNING ENTRY !! [H.sapiens] | 1.581 |
| 122997 | AA479295 | Hs.106290 | Kelch motif containing protein | 1.581 |
| 119707 | W67569 | Hs.44143 | ESTs; Weakly similar to SNF2alpha protein [H.sapiens] | 1.58 |
| 131934 | D80948 | Hs.34922 | ESTs | 1.58 |
| 106141 | AA424558 | Hs.9302 | phosducin-like | 1.58 |
| 115271 | AA279422 | Hs.5724 | ESTs | 1.579 |
| 131468 | R27598 | Hs.27197 | KIAA0797 protein | 1.577 |
| 131165 | R98173 | Hs.23763 | Max-interacting protein | 1.575 |
| 117273 | N21680 | Hs.43047 | ESTs | 1.575 |
| 101569 | M33772 | Hs.182421 | troponin C2; fast | 1.575 |
| 116127 | AA459703 | Hs.79070 | v-myc avian myelocytomatosis viral oncogene homolog | 1.575 |
| 120022 | W90625 | Hs.58432 | ESTs | 1.575 |
| 117512 | N32157 | Hs.82207 | ESTs | 1.574 |
| 106511 | AA452865 | Hs.206713 | UDP-Gal:betaGlcNAc beta 1;4- galactosyltransferase; polypeptide2 | 1.573 |
| 116415 | AA609204 | Hs.27973 | KIAA0874 protein | 1.573 |
| 127879 | AA810215 | Hs.189079 | ESTs | 1.571 |
| 125211 | W72798 | Hs.103177 | ESTs; Wkly smlr to cDNA EST EMBL:D32579 comes from this gene [C.elegans] | 1.571 |
| 114746 | AA135638 | Hs.223756 | ESTs | 1.571 |
| 122698 | AA456112 | Hs.99410 | ESTs | 1.57 |
| 116765 | H12636 | Hs.121585 | ESTs; Weakly similar to reverse transcriptase [H.sapiens] | 1.568 |
| 130895 | AA609828 | Hs.21015 | ESTs; Highly similar to tetracycline transporter-like protein [M.musculus] | 1.568 |
| 114338 | Z41366 | Hs.40109 | KIAA0872 protein | 1.567 |
| 111005 | N53076 | Hs.5996 | ESTs | 1.567 |
| 128135 | AA913491 | Hs.189143 | ESTs; Modrtly smlr to !! ALU SUBFAMILY J WARNING ENTRY !! [H.sapiens] | 1.567 |
| 112046 | R43365 | Hs.22273 | ESTs | 1.566 |
| 132160 | AA281770 | Hs.184081 | seven in absentia (Drosophila) homolog 1 | 1.566 |
| 111568 | R10153 | Hs.20561 | ESTs | 1.566 |
| 127775 | H04106 | Hs.179902 | ESTs; Weakly similar to NG22 [H.sapiens] | 1.566 |
| 115359 | AA281936 | Hs.88914 | ESTs | 1.566 |
| 121845 | AA425734 | Hs.165066 | ESTs; Weakly similar to hypothetical protein [H.sapiens] | 1.565 |
| 127854 | AA769520 | | ESTs; Weakly similar to REGULATOR OF MITOTIC SPINDLE | |
| | | | ASSEMBLY 1 [H.sapiens] | 1.564 |
| 120287 | AA187679 | Hs.111114 | ESTs | 1.563 |
| 114940 | AA243012 | Hs.75928 | ESTs | 1.562 |
| 126716 | AA031700 | Hs.251962 | ESTs | 1.562 |
| 134161 | U97188 | Hs.79440 | IGF-II mRNA-binding protein 3 | 1.561 |
| 125390 | H95094 | Hs.75187 | translocase of outer mitochondrial membrane 20 (yeast) homolog | 1.561 |
| 115334 | AA281244 | Hs.65300 | ESTs | 1.559 |
| 113721 | T97931 | Hs.18190 | EST | 1.558 |
| 114895 | AA236177 | Hs.76591 | KIAA0887 protein | 1.558 |
| 119341 | T62571 | Hs.146388 | microtubule-associated protein 7 | 1.558 |
| 108012 | AA039616 | Hs.61933 | ESTs | 1.558 |
| 130335 | AA156499 | Hs.8454 | protein kinase; cAMP-dependent; regulatory; type II; alpha | 1.557 |
| 134351 | R82074 | Hs.82109 | syndecan 1 | 1.557 |
| 133300 | D51401 | Hs.70333 | ESTs | 1.553 |
| 106920 | AA490899 | Hs.24462 | ESTs | 1.553 |
| 118744 | N74075 | Hs.94293 | EST | 1.552 |
| 126489 | W20016 | Hs.144228 | ESTs; Weakly similar to ZINC FINGER PROTEIN 83 [H.sapiens] | 1.55 |
| 115913 | AA436720 | Hs.65487 | ESTs | 1.55 |
| 107868 | AA025234 | Hs.61260 | ESTs | 1.55 |
| 134520 | N21407 | Hs.257325 | ESTs | 1.55 |
| 109703 | F09684 | Hs.24792 | ESTs; Weakly similar to ORF YOR283w [S.cerevisiae] | 1.55 |
| 120288 | AA187938 | Hs.55189 | ESTs; Weakly similar to F25B5.3 [C.elegans] | 1.548 |
| 106356 | AA443277 | Hs.31034 | peroxisomal biogenesis factor 11A | 1.548 |
| 129460 | AA235627 | Hs.11171 | APG5 (autophagy 5; S. cerevisiae)-like | 1.547 |
| 133950 | D11961 | Hs.77823 | ESTs | 1.546 |
| 128172 | AI400862 | Hs.142607 | ESTs | 1.546 |
| 114162 | Z38909 | Hs.22265 | ESTs | 1.545 |
| 101803 | M86546 | Hs.155691 | pre-B-cell leukemia transcription factor 1 | 1.544 |
| 113617 | T93630 | Hs.17207 | ESTs | 1.542 |
| 104896 | AA054228 | Hs.23165 | ESTs | 1.541 |
| 114477 | AA032013 | Hs.144260 | EST | 1.54 |
| 110731 | H98653 | Hs.188006 | KIAA0878 protein | 1.54 |
| 130367 | Z38501 | Hs.8768 | ESTs; Wkly smlr to !! ALU SUBFAMILY SQ WARNING ENTRY !! [H.sapiens] | 1.538 |
| 130539 | L07044 | Hs.250857 | Homo sapiens calcium/calmodulin-dependent protein kinase II mRNA; partial cds | 1.538 |
| 134921 | W60186 | Hs.169487 | Kreisler (mouse) maf-related leucine zipper homolog | 1.537 |
| 130583 | W24957 | Hs.16281 | ESTs; Moderately similar to similar to C.elegans protein | |
| | | | encoded in cosmid T20D3 [H.sapiens] | 1.537 |
| 133723 | AA088851 | Hs.75744 | S-adenosylmethionine decarboxylase 1 | 1.537 |
| 106450 | AA449469 | Hs.11859 | ESTs | 1.536 |
| 104120 | AA429838 | Hs.89519 | KIAA1046 protein | 1.536 |
| 100533 | HG1879-HT1919 | | Ras-Like Protein Tc10 | 1.535 |
| 130664 | R09049 | Hs.17625 | ESTs | 1.535 |
| 127122 | AA279153 | Hs.190049 | ESTs | 1.535 |
| 134264 | T03391 | Hs.8087 | ESTs | 1.535 |
| 132319 | AA418662 | Hs.44625 | ESTs | 1.535 |
| 115465 | AA286941 | Hs.43691 | ESTs | 1.533 |
| 125003 | T59442 | Hs.100445 | ESTs | 1.532 |
| 102273 | U30888 | Hs.75981 | ubiquitin specific protease 14 (tRNA-guanine transglycosylase) | 1.532 |
| 121875 | AA426299 | Hs.98510 | ESTs | 1.532 |
| 114366 | Z41747 | Hs.469 | succinate dehydrogenase complex; subunit A; flavoprotein (Fp) | 1.531 |
| 132944 | AA054515 | Hs.6127 | ESTs; Weakly similar to prostate-specific transglutaminase [H.sapiens] | 1.53 |
| 111199 | N68210 | Hs.29822 | ESTs | 1.53 |
| 113494 | T88878 | Hs.258738 | ESTs | 1.529 |
| 129515 | AA490882 | Hs.112227 | ESTs | 1.528 |
| 133124 | AA156049 | Hs.65490 | ESTs | 1.528 |
| 104785 | AA027163 | Hs.7942 | ESTs | 1.526 |
| 105595 | AA279408 | Hs.25866 | ESTs | 1.526 |
| 130198 | U67156 | Hs.151988 | mitogen-activated protein kinase kinase kinase 5 | 1.526 |
| 114297 | Z40758 | Hs.173091 | DKFZP434K151 protein | 1.525 |
| 112876 | T03488 | Hs.4842 | ESTs | 1.525 |
| 127500 | AA525014 | Hs.162115 | ESTs | 1.525 |
| 120519 | AA258585 | Hs.129887 | cadherin 19 (NOTE: redefinition of symbol) | 1.525 |
| 119859 | W80702 | Hs.58461 | ESTs | 1.525 |
| 129944 | L00389 | Hs.1361 | cytochrome P450; subfamily I (aromatic compound-inducible); polypeptide 2 | 1.524 |
| 118864 | N89670 | Hs.42148 | ESTs; Weakly similar to Su(P)[D.melanogaster] | 1.523 |
| 123964 | C13961 | Hs.210115 | EST | 1.523 |
| 111676 | R19414 | Hs.166459 | ESTs | 1.522 |
| 128332 | AI079523 | Hs.134173 | ESTs | 1.522 |
| 130455 | X17059 | Hs.155956 | N-acetyltransferase 1 (arylamine N-acetyltransferase) | 1.521 |
| 125181 | W58461 | Hs.12396 | ESTs | 1.521 |
| 127093 | AA768241 | | oa72d02.s1 NCI_CGAP_GCB1 Homo sapiens cDNA clone | |
| | | | IMAGE:1317795 3', mRNA sequence. | 1.521 |
| 132156 | AA157401 | Hs.4113 | S-adenosylhomocysteine hydrolase-like 1 | 1.521 |
| 125303 | Z39821 | Hs.107295 | ESTs | 1.52 |
| 132697 | AA281951 | Hs.5518 | Homo sapiens mRNA;cDNA DKFZp566J2146 (from clone DKFZp566J2146) | 1.52 |
| 117086 | H93135 | Hs.41840 | ESTs | 1.519 |
| 113355 | T79203 | Hs.14480 | ESTs | 1.518 |
| 108621 | AA101811 | Hs.69506 | ESTs | 1.518 |
| 109384 | AA219172 | Hs.86849 | EST | 1.518 |
| 128510 | X94703 | Hs.100816 | RAB28; member RAS oncogene family | 1.517 |
| 132968 | N77151 | Hs.61638 | myosin X | 1.515 |
| 117035 | H88798 | Hs.41182 | ESTs | 1.515 |
| 116781 | H22985 | Hs.52132 | ESTs | 1.513 |
| 108677 | AA115629 | Hs.118531 | ESTs | 1.513 |
| 130214 | H78003 | Hs.15266 | ESTs | 1.513 |
| 134700 | AA481414 | Hs.8868 | golgi SNAP receptor complex member 1 | 1.512 |
| 116618 | D80783 | Hs.45224 | ESTs | 1.508 |
| 126257 | N99638 | | tumor necrosis factor receptor superfamily; member 10b | 1.508 |
| 125859 | AA806808 | Hs.118797 | ubiquitin-conjugating enzyme E2D 3 (homologous to yeast UBC4/5) | 1.508 |
| 113837 | W57698 | Hs.8888 | ESTs | 1.507 |
| 114317 | Z41038 | Hs.469 | succinate dehydrogenase complex; subunit A; flavoprotein (Fp) | 1.507 |
| 100311 | D50640 | Hs.184653 | phosphodiesterase 3B; cGMP-inhibited | 1.507 |
| 126802 | AA947601 | Hs.97056 | ESTs | 1.506 |
| 128661 | R82837 | Hs.103329 | KIAA0970 protein | 1.506 |
| 134194 | AA233231 | Hs.79828 | ESTs | 1.506 |
| 108953 | AA149652 | Hs.42128 | ESTs | 1.504 |
| 133240 | D31161 | Hs.68613 | ESTs | 1.502 |
| 132671 | X76302 | Hs.54649 | putative nucleic acid binding protein RY-1 | 1.501 |
| 132609 | Z48923 | Hs.53250 | bone morphogenetic protein receptor; type II (serine/threonine kinase) | 1.501 |
| 105574 | AA278678 | Hs.258567 | ESTs | 1.5 |
| 113718 | T97782 | Hs.256268 | ESTs | 1.5 |
| 127824 | AI208365 | Hs.127811 | ESTs | 1.5 |
| 130132 | U55936 | Hs.184376 | synaptosomal-associated protein; 23kD | 1.5 |
| 127394 | AA453224 | | ESTs; Weakly similar to !! ALU SUBFAMILY J WARNING ENTRY !! [H.sapiens] | 1.5 |
| 100485 | HG1111-HT1111 | | Ras-Like Protein Tc21 | 1.5 |
| 101078 | L04510 | Hs.792 | ADP-ribosylation factor domain protein 1; 64kD | 1.5 |
| 128611 | AA456845 | Hs.102471 | KIAA0680 gene product | 1.5 |

**TABLE 12A shows the accession numbers for those primekeys lacking unigeneID's for Table 12. For each probeset we have listed the gene cluster number from which the oligonucleotides were designed. Gene clusters were compiled using sequences derived from Genbank ESTs and mRNAs. These sequences were clustered based on sequence similarity using Clustering and Alignment Tools (DoubleTwist, Oakland California). The Genbank accession numbers for sequences comprising each cluster are listed in the "Accession" column.**

| | | |
|---|---|---|
| Pkey: | | Unique Eos probeset identifier number |
| CAT number. | | Gene cluster number |
| Accession: | | Genbank accession numbers |
| | | |

| **Pkey** | **CAT number** | **Accession** |
|---|---|---|
| | | |
| 108536 | 119811_1 | AA084524 AA339253 AW966289 |
| 117040 | 46956_1 | AW970600 AA503323 H89218 AF086031 H89112 |
| 100782 | 18457_1 | AA355435 NM_001516 Z30093 T28405 AW949486 AA461142 AA410532 AI652073 AA521208 AI970141 AI968234 AI026102 AA713583 AW135876 AA936614 AA770300 AI242635 AA377033 AW960263 AW607683 AI273603 AA410287 AI040513 AA460838 AI803916 AW294095 AW449680 AW798677 AW675048 BE542116 AL120521 |
| 100819 | 3022_1 | L34840 NM_003241 U31905 AI546931 AI791616 AI973065 AI792321 AI546937 AI685880 AI732835 AI682360 AA420653 AA564047 AI682323 AI824614 AI659889 AI680052 AI970887 AI623108 AA420692 AI418074 AA631018 AI810595 AW291463 AW449930 AI668908 AI970818 |
| 100824 | 5_36 | AI393237 AI521317 AI761348 AF025841 D43968 AW994987 L34598 AF025841 D89789 D89788 D89790 AW998932 AI971742 AI310238 X90976 AW139668 AW674280 AI365552 AA877452 AV657554 C75229 AA376077 AI798056 AW609213 |
| | | W25586 H30149 BE075089 BE075190 AW580858 H99598 AA425238 AA133916 AW363478 BE158121 BE158127 AW467960 BE158135 BE158126 BE158145 N92860 AA847246 AI961688 AI361423 AA878154 AA043767 AI863712 AI559226 AW339007 AI371266 AI368901 AA046624 AA134739 AW449154 AA130232 AI458720 AA962511 AI700627 |
| | | R70437 AW004008 AA045229 AI671572 H99599 AA043768 AI685454 AI871685 N29937 X90977 AA524240 AI142114 AI825750 AI567805 AI631365 AI347893 AA134740 F20669 AA046707 AW793216 AW963298 AW959380 AA363265 AI784593 AI268201 R69451 AV657618 AI695588 |
| 125004 | 264197_1 | BE312163 AJ230798 AA374482 AI926059 AA622653 AI860704 BE139185 AW296884 T60238 T60120 |
| 102313 | 27608_1 | U33921 AI190489 AA573311 |
| 102337 | 553_1 | AI814663 AA806761 AA765241 AA019317 AA092255 AA035405 T85079 AA890151 AI373959 T85080 BE153728 AA740848 BE080682 AL048137 AW182316 AI699468 AW274481 AW407538 AA306562 AW950024 AW949943 AL045703 AW843196 W25132 BE612794 AA304266 AW958054 H25673 AV646563 AV646573 |
| | | BE172990 AW593488 AA385181 AA164998 AI246476 AA345406 AI277554 AA134749 AA856624 BE613247 AA299003 AL048138 AA028121 T92510 AI923835 AW020440 AI401594 AI889401 N93290 AA044247 AA028100 AI582845 AA811151 AI741811 AI925878 AA448277 AA172221 AI214783 BE220793 AA022746 AI082882 AA022849 AI928385 AA573472 AI420686 AW072902 |
| | | AI799493 AI873506 AI468977 AI192079 AI468976 AA044272 AW015701 AW316979 AA933042 AA609017 AI318393 AI424571 AI934945 AA172023 AW050917 AA846180 AA134748 AI003947 AI766769 AW006697 AA653517 AW575680 AI474214 AA401478 U36922 AA927064 AA868000 D62654 T91745 AW500202 AA194764 AA746346 AA130464 AW117498 AA054526 N26432 |
| | | H02534 H04964 AW303367 BE300931 AI218049 AI208073 AW182749 AA983630 AI147585 AA194765 AA054534 AA922720 AI436585 AI346535 AA134269 AA280923 AA897422 AA019559 AW274010 AA035406 AA917879 H99327 W32908 AI216046 AW496823 AA019414 H82288 W35284 AI936621 AI767113 |
| | | AA866177 AW367874 H82398 AF032885 AW300151 AW467069 AA809346 AI188507 AI494178 AA872752 AI631631 U02310 NM_002015 AA815006 AI382453 AW197658 AI761654 AI804396 AI382221 AI813640 AI439635 AI523901 AW517242 AI221705 AW298104 AW204560 AW573095 AW028783 AW014650 AI766744 AI808294 AI698758 AI041809 AI766667 AI479103 AA872797 AA769305 AA765080 AA334166 AI472322 |
| 124704 | 292319_1 | R07335 R07640 |
| 116988 | 185904_1 | AW953679 AW953680 AA244436 H82527 AA361046 AA244483 H82526 |
| 124825 | 330773_1 | AA501669 R52088 |
| 110455 | 46874_1 | H52576 AF085971 H52172 |
| 126257 | 182217_1 | N99638 AW973750 AA328271 H90994 AA558020 AA234435 N59599 R94815 |
| 125624 | 154135_1 | AW968363 AA465492 R34539 AA165411 |
| 104038 | 264235_1 | AA374532 AA421255 |
| 103427 | 43892_1 | BE514383 AA071273 AW247987 AW673286 BE312102 AW749824 BE071985 AW577383 BE071945 BE072005 AW577355 BE071965 AW239231 BE072000 BE071960 AW577360 AW749830 AW373020 X97303 AW999522 BE000192 BE562219 BE266655 BE264970 |
| 104142 | 113242 1 | AA074713 AA447006 |
| 127093 | 47721_1 | AW977549 AA256038 AL365415 AW500455 AA768241 AW968097 Z17849 AA256104 |
| 125873 | 10492_1 | AW271838 AL133605 C01646 H29959 AA999896 D60676 AW999454 AW961176 AA315244 H14437 AW386118 N46512 AW272021 AI768516 BE466421 AI082809 AI804454 AA905101 AW173368 N38942 AW614169 AI080483 N29489 AI500550 AA994475 AA614464 AA707368 AA593145 AA569473 AW627815 AI828244 N63226 N42300 |
| 125954 | 4457_1 | NM_016353 AB023584 W44753 R09585 AA382865 R23772 AI814257 AA974046 AK001608 AI935638 AW440609 AI420022 AA777386 AA806969 AI554876 AI584006 AI688556 AI688634 AI697997 AI014540 AI806683 AI741202 AW263154 AW297238 AI149951 AI589076 AW082158 AW614265 AA931887 AA781969 R09490 AA484643 AI207121 AI088390 AI538065 AI619547 AI741925 AI702846 H40846 R93943 AW747979 AA461348 U30163 AA326023 AI535992 AW242870 AI244025 AI222558 W38425 AW473630 AI624599 AI921226 AI683152 AI096458 AI123822 AW170802 C16447 AI337674 D25726 AW339366 AW771259 AA461174 |
| 125992 | 1589048_1 | H48372 W01626 |
| 127210 | 15307_6 | AA305278 AA223833 110924 6443_1 AW058463 AF195766 AA680145 T86901 W60373 W60281 NM_007222 AF106862 AI000795 AA167188 AW884503 AW891313 AW891332 AW891312 AI984924 AI123518 N75170 AA131614 H25330 AI913358 A1742277 W25576 R58771 AW445159 AW888628 AW888627 AW274674 AI088482 N52314 N34282 AW001769 AI338943 T66784 AI288963 AW468676 AW237528 H25289 N71690 AA610128 AI143458 AI082599 N49144 AA854773 AW663411 AW610151 N47938 AW601626 AA167189 AA918304 AA805205 BE069496 AA652836 BE069499 AI699298 AW249926 AW888578 BE567635 T10726 AW604715 D54245 D53062 D55610 D55555 AA301376 AI133498 N77788 AI936320 AW090734 AI269977 N50828 AA550814 AI421993 AI005384 N50813 D60292 D59349 AA131710 D81698 D81699 |
| 127263 | 232161_1 | AA331156 AA331157 AA331155 |
| 135197 | 29440_1 | U76456 NM_003256 AF057532 AA193414 AW293304 AW963378 AA313095 AI359841 AI969312 AI080163 AW448926 AI671136 BE466399 AI637967 AI671873 AW196583 AW071635 AI634427 AW296872 AW292470 AA193650 |
| 127394 | 304844_1 | BE161832 AA453224 AA485772 |
| 126879 | 1860_2 | D90391 M55575 AI652268 AA719776 |
| 126983 | 171841_1 | AA524886 AW971347 AA211537 |
| 120470 | 188975_1 | AW971327 AA524988 AW628653 AA251797 |
| 127854 | 443883_1 | AW976796 AA769520 |
| 121367 | 280429_1 | AA432071 AA405648 AW000908 T16347 |
| 106320 | 6435_1 | AB028957 AL120001 AI267678 H10928 R19844 AW970334 AA393182 F05472 F11711 H09908 N50250 AI815411 BE463679 D61468 AW970253 D60889 C15548 D61011 D60867 AI815795 AA534831 D81386 AW235039 AI382158 D81174 AA416899 AA852310 H09789 H10929 H09813 F09369 R44721 D51515 Z38456 R14004 T66255 F12148 F12139 AW351702 M85350 AI018713 AW972450 AW972645 AA514964 T66172 F09785 F09776 AA436608 T05327 T07118 AA339352 |
| 115479 | 201515_1 | AW301608 N46706 AA649093 AA287595 AW811753 AA287596 N39260 |
| 101026 | 11075_1 | NM_001874 J04970 T91426 AW205201 T84979 AA255727 AA847837 R02164 T91339 AV651884 AV651835 AV651350 AV650118 AV651338 AI272002 AI367796 AA830651 AA262112 AW151198 |
| 100401 | 24827_1 | AU076696 AA219720 AL135197 AA305877 N56376 AA318063 AA130725 AW954903 BE541230 AW383312 U86753 D85423 AI679458 AI122932 AB007892 AI583919 BE160134 F08104 R34903 F13440 AA095444 AA262453 AA191036 R17895 T81266 BE149776 AI279537 AI143113 AA361072 AW959030 |
| | | AW268817 AA811533 BE275179 AI221677 T65147 R49293 AA249176 BE000290 AA768053 F09494 BE092645 BE172099 Z41177 AA044750 AI909768 BE140795 BE140574 AW845210 AW752452 BE243244 AA843664 AI300080 BE169032 AW189979 BE004869 AA621872 AI951772 AI678897 AI926598 N62813 |
| | | AI350912 AW608791 AI309602 AI983138 AW875592 AI655073 AW875626 AA130606 AI370827 C75528 C75554 AW263335 AI344426 BE004788 AA576220 AA604824 AI431405 AA749378 R38882 AW955075 AA173821 C75657 AA219672 AW768408 R43141 AI431414 AA483343 AI673792 T17294 AW770187 N74285 AI476404 AI088288 AA654152 AW974864 BE617311 BE243328 BE168049 |
| 130542 | 28089_3 | U64675 AW167507 AW167508 BE218568 AA779360 W85722 AL044843 BE159404 AF012086 AW898611 AW898610 BE159405 BE092191 AW890826 AW369841 AW368064 AW606702 AL044731 R82691 AA419346 AA416558 H96045 AL040450 AI640531 AI808434 AL046613 AW855784 AW362469 AL048881 AL049015 AA094272 AA888908 AA417294 AW237786 R59793 AL044916 |
| | | D82402 AI216854 AI079342 H96406 AL037845 AI915900 AA972133 AI478783 T31074 Z21135 Z21396 AA352182 R13918 AA430178 C17811 AI371824 AI742256 AA926801 N79156 AA350610 AA081971 N83639 R35544 AA312292 AW952080 N42322 AA171957 AA565297 R89207 AA504106 AI630782 AA826482 AI301579 T36241 AW966618 Z28426 |
| | | AL043480 AI124636 AA393449 T19504 AW887823 AI289814 N53979 AL043571 AI632764 AI859613 AI986308 AI683212 AI984499 AI133258 C05898 AW512761 AI041260 BE466240 Z19161 AI351190 N67549 AI373374 AA400873 AW440914 AW514879 AA770146 AI358754 R51113 AI283773 AA649886 T30543 D54358 R37750 T03358 T15451 T15880 AA999689 N67396 AI056289 T85597 N62441 R89099 R00035 T85596 R61335 R00128 N63359 AI535964 |
| 100485 | 30576_2 | AI207768 M31468 NM_012250 W01322 AA253280 AA253233 AA293148 AW582106 R79880 AA459547 AA363459 AA234396 N31669 H44468 AA434587 AW363088 AW993541 |
| 108345 | 112277_6 | AA070906 AA070934 |
| 100522 | 19669_1 | X51501 NM_002652 Y10179 J03460 AI791618 AI821473 AA916588 AA564296 AA9161110 AI972286 AI420470 AI568790 AI597724 AW205207 AI659305 AI791620 AA532383 AI821475 AA526498 |
| 100533 | 32905_1 | NM_012249 M31470 AL043108 AA262561 AA178883 T29433 AA313329 W48807 AW404323 AA453560 AW403227 H94816 W17101 AA165152 W23989 AA091310 |
| 100598 | 23902_2 | AL121734 D54896 AA424269 BE242906 AA382118 BE018454 AI280348 AL048769 M35543 AA757734 AI128865 H20289 H23728 AI203445 H41481 H18237 H44081 H92839 AI928621 H75675 D51148 AI796198 AW390453 D55579 D54145 D53996 D54015 R37664 H17541 AA668681 T65061 R15867 AW468123 R16049 H69030 AA054226 H16070 F09655 R92144 T03521 R05473 H92840 AA018186 R91707 |
| 102332 | 14745_3 | U35637 AA112989 Z19308 |
| 118250 | genbank_N62602 | N62602 |
| 103678 | entrez_Z84483 | Z84483 |
| 119400 | genbank_T92767 | T92767 |
| 119559 | entrez_W38197 | W38197 |

### MISSING AT THE TIME OF PUBLICATION

**TABLE 13: shows genes, including expression sequence tags, up-regulated in prostate tumor tissue compared to normal prostate tissue as analyzed using Affymetrix/Eos Hu02 GeneChip array. Shown are the ratios of "average" normal prostate to "average" prostate cancer tissues.**

| | | | | |
|---|---|---|---|---|
| Pkey: | | Unique Eos probeset identifier number | | |
| ExAccn: | | Exemplar Accession number, Genbank accession number | | |
| UnigeneID: | | Unigene number | | |
| Unigene Title: | | Unigene gene title | | |
| R1: | | Background subtracted normal prostate : prostate tumor tissue | | |
| | | | | |

| **Pkey** | **ExAccn** | **UnigeneID** | **Unigene Title** | **R1** |
|---|---|---|---|---|
| | | | | |
| 333516 | | | CH22_FGENES.173_1 | 0.028 |
| 337954 | | | CH22_EM:AC005500.GENSCAN.96-3 | 0.029 |
| 332496 | R73299 | Hs.204354 | ras homolog gene family; member B | 0.03 |
| 337944 | | | CH22_EM:AC005500.GENSCAN.89-7 | 0.033 |
| 334111 | | | CH22_FGENES.330_10 | 0.033 |
| 333657 | | | CH22_FGENES.241_2 | 0.034 |
| 327718 | | | CH.04_hs gi\|6525284 | 0.034 |
| 336355 | | | CH22_FGENES.817_5 | 0.035 |
| 322011 | AL137354 | | EST cluster (not in UniGene) | 0.035 |
| 336377 | | | CH22_FGENES.821_5 | 0.036 |
| 300254 | AW079607 | Hs.188417 | ESTs; Weakly similar to ZnT-3 [H.sapiens] | 0.037 |
| 330096 | | | CH.19_p2 gi\|6015278 | 0.037 |
| 335191 | | | CH22_FGENES.507_6 | 0.038 |
| 334040 | | | CH22_FGENES.322_8 | 0.039 |
| 333586 | | | CH22_FGENES.204_2 | 0.04 |
| 333295 | | | CH22_FGENES.132_2 | 0.042 |
| 313326 | AI086120 | Hs.122329 | ESTs | 0.043 |
| 329517 | | | CH.10_p2 gi\|3983513 | 0.043 |
| 333403 | | | CH22_FGENES.144_21 | 0.043 |
| 335226 | | | CH22_FGENES.513_11 | 0.044 |
| 335976 | | | CH22_FGENES.652_11 | 0.045 |
| 333637 | | | CH22_FGENES.229_2 | 0.046 |
| 334582 | | | CH22_FGENES.407_5 | 0.046 |
| 336437 | | | CH22_FGENES.826_4 | 0.047 |
| 337461 | | | CH22_FGENES.782-1 | 0.047 |
| 302892 | N58545 | Hs.6975 | histone deacetylase 3 | 0.049 |
| 338689 | | | CH22_EM:AC005500.GENSCAN.475-3 | 0.049 |
| 334721 | | | CH22_FGENES.421_32 | 0.049 |
| 305867 | AA864572 | | EST singleton (not in UniGene) with exon hit | 0.049 |
| 335498 | | | CH22_FGENES.571_7 | 0.05 |
| 311596 | AI682088 | Hs.223368 | ESTs | 0.05 |
| 326959 | | | CH.21_hs gi\|6469836 | 0.051 |
| 311688 | AW025661 | Hs.240090 | ESTs | 0.052 |
| 317298 | AI922374 | Hs.158549 | ESTs | 0.052 |
| 332984 | | | CH22_FGENES.54_6 | 0.052 |
| 321039 | AW247083 | | EST cluster (not in UniGene) | 0.053 |
| 335844 | | | CH22_FGENES.623_4 | 0.053 |
| 325371 | | | CH.12_hs gi\|5866920 | 0.054 |
| 335667 | | | CH22_FGENES.590_18 | 0.054 |
| 333635 | | | CH22_FGENES.228_2 | 0.054 |
| 336736 | | | CH22_FGENES.110-2 | 0.055 |
| 335893 | | | CH22_FGENES.635_1 | 0.055 |
| 333170 | | | CH22_FGENES.94_5 | 0.055 |
| 329768 | | | CH.14_p2 gi\|6015501 | 0.055 |
| 334030 | | | CH22_FGENES.320_2 | 0.055 |
| 323359 | AA234172 | Hs.137418 | ESTs | 0.055 |
| 300453 | AW051431 | Hs.113029 | ribosomal protein S25 | 0.055 |
| 334262 | | | CH22_FGENES.367_12 | 0.055 |
| 306590 | AI000246 | | EST singleton (not in UniGene) with exon hit | 0.055 |
| 331087 | R22520 | Hs.23398 | ESTs | 0.055 |
| 338620 | | | CH22_EM:AC005500.GENSCAN.450-18 | 0.056 |
| 339045 | | | CH22_DA59H18.GENSCAN.28-5 | 0.056 |
| 308023 | AI452732 | | EST singleton (not in UniGene) with exon hit | 0.057 |
| 339067 | | | CH22_DA59H18.GENSCAN.33-3 | 0.057 |
| 335689 | | | CH22_FGENES.596_4 | 0.057 |
| 339069 | | | CH22_DA59H18.GENSCAN.33-5 | 0.057 |
| 338176 | | | CH22_EM:AC005500.GENSCAN.219-4 | 0.057 |
| 328159 | | | CH.06_hs gi\|5868065 | 0.058 |
| 335655 | | | CH22_FGENES.590_6 | 0.058 |
| 336371 | | | CH22_FGENES.820_1 | 0.058 |
| 336558 | | | CH22_FGENES.842_3 | 0.059 |
| 337738 | | | CH22_EM:AC000097.GENSCAN.100-4 | 0.059 |
| 334273 | | | CH22_FGENES.369_2 | 0.059 |
| 335889 | | | CH22_FGENES.633_3 | 0.059 |
| 327807 | | | CH.05_hs gi\|5867968 | 0.059 |
| 333315 | | | CH22_FGENES.138_7 | 0.059 |
| 338825 | | | CH22_DJ246D7.GENSCAN.4-6 | 0.06 |
| 337612 | | | CH22_C20H12.GENSCAN.22-5 | 0.06 |
| 333897 | | | CH22_FGENES.293_4 | 0.06 |
| 335990 | | | CH22_FGENES.655_4 | 0.06 |
| 334264 | | | CH22_FGENES.367_15 | 0.06 |
| 338653 | | | CH22_EM:AC005500.GENSCAN.460-39 | 0.061 |
| 322303 | W07459 | | EST cluster (not in UniGene) | 0.061 |
| 333498 | | | CH22_FGENES.168_8 | 0.061 |
| 336522 | | | CH22_FGENES.839_3 | 0.061 |
| 301357 | AW295677 | Hs.137840 | ESTs; Moderately similar to HOMEOBOX | |
| | | | PROTEIN SIX1 [H.sapiens] | 0.062 |
| 305917 | AA876469 | Hs.181357 | laminin receptor 1 (67kD; ribosomal protein SA) | 0.062 |
| 336143 | | | CH22_FGENES.705_5 | 0.063 |
| 333493 | | | CH22_FGENES.168_2 | 0.063 |
| 332533 | M99487 | Hs.1915 | folate hydrolase (prostate-specific membrane antigen) 1 | 0.063 |
| 325844 | | | CH.16_hs gi\|6552453 | 0.063 |
| 336402 | | | CH22_FGENES.823_17 | 0.063 |
| 335767 | | | CH22_FGENES.607_1 | 0.064 |
| 301893 | T80334 | | EST cluster (not in UniGene) with exon hit | 0.064 |
| 324019 | AW177009 | | EST cluster (not in UniGene) | 0.064 |
| 305801 | AA845997 | | EST singleton (not in UniGene) with exon hit | 0.064 |
| 335188 | | | CH22_FGENES.507_3 | 0.065 |
| 337533 | | | CH22_FGENES.828-2 | 0.065 |
| 333311 | | | CH22_FGENES.138_3 | 0.065 |
| 335668 | | | CH22_FGENES.590_19 | 0.065 |
| 306786 | AI041589 | | EST singleton (not in UniGene) with exon hit | 0.066 |
| 306365 | AA962086 | | EST singleton (not in UniGene) with exon hit | 0.066 |
| 306249 | AA933840 | | EST singleton (not in UniGene) with exon hit | 0.066 |
| 335018 | | | CH22_FGENES.474_6 | 0.066 |
| 333594 | | | CH22_FGENES.210_3 | 0.066 |
| 333900 | | | CH22_FGENES.293_7 | 0.066 |
| 325207 | | | CH.10_hs gi\|6552430 | 0.067 |
| 329888 | | | CH.15_p2 gi\|6067149 | 0.067 |
| 326238 | | | CH.17_hs gi\|5867260 | 0.067 |
| 333658 | | | CH22_FGENES.241_4 | 0.067 |
| 335809 | | | CH22_FGENES.617_6 | 0.068 |
| 307427 | AI243437 | | EST singleton (not in UniGene) with exon hit | 0.068 |
| 318428 | AI949409 | Hs.224583 | ESTs | 0.069 |
| 327005 | | | CH.21_hs gi\|5867664 | 0.069 |
| 330463 | HG998-HT998 | | Sulfotransferase, Phenol-Preferring | 0.069 |
| 333318 | | | CH22_FGENES.138_10 | 0.07 |
| 333313 | | | CH22_FGENES.138_5 | 0.07 |
| 325937 | | | CH.16_hs gi\|5867132 | 0.07 |
| 335663 | | | CH22_FGENES.590_14 | 0.07 |
| 335349 | | | CH22_FGENES.539_2 | 0.07 |
| 303396 | AA224470 | Hs.25426 | ESTs; Weakly similar to unknown [H.sapiens] | 0.07 |
| 332603 | N66681 | Hs.33470 | ESTs | 0.07 |
| 333310 | | | CH22_FGENES.138_2 | 0.071 |
| 309924 | AW340812 | | EST singleton (not in UniGens) with exon hit | 0.071 |
| 336340 | | | CH22_FGENES.814_15 | 0.071 |
| 308025 | AI453365 | Hs.172928 | collagen; type I; alpha 1 | 0.071 |
| 306805 | AI055966 | | EST singleton (not in UniGene) with exon hit | 0.071 |
| 335499 | | | CH22_FGENES.571_8 | 0.071 |
| 329669 | | | CH.14_p2 gi\|6272129 | 0.071 |
| 321666 | D28390 | | EST cluster (not in UniGene) | 0.071 |
| 338174 | | | CH22_EM:AC005500.GENSCAN.219-2 | 0.072 |
| 336556 | | | CH22_FGENES.842_1 | 0.072 |
| 305451 | AA738105 | Hs.140 | immunoglobulin gamma 3 (Gm marker) | 0.072 |
| 336684 | | | CH22_FGENES.46-1 | 0.072 |
| 326943 | | | CH.21_hs gi\|6004446 | 0.073 |
| 333947 | | | CH22_FGENES.303_1 | 0.074 |
| 333214 | | | CH22_FGENES.104_5 | 0.074 |
| 331917 | AA446572 | Hs.174007 | ESTs; Moderately similar to !!!! ALU SUBFAMILY J WARNING | 0.074 |
| 339102 | | | CH22_DA59H18.GENSCAN.44-9 | 0.074 |
| 328122 | | | CH.06_hs gi\|5868031 | 0.075 |
| 332250 | N62712 | Hs.226223 | KIAA0618 gene product | 0.075 |
| 328506 | | | CH.07_hs gi\|5868471 | 0.075 |
| 331756 | AA291468 | Hs.98504 | ESTs | 0.075 |
| 335193 | | | CH22_FGENES.507_8 | 0.076 |
| 317729 | AA971718 | Hs.128141 | ESTs | 0.076 |
| 304515 | AA458708 | Hs.251577 | hemoglobin; alpha 2 | 0.076 |
| 313644 | AI565766 | Hs.124960 | ESTs | 0.076 |
| 326145 | | | CH.17_hs gi\|5867204 | 0.076 |
| 336394 | | | CH22_FGENES.823_6 | 0.077 |
| 306516 | AA989542 | | EST singleton (not in UniGene) with exon hit | 0.077 |
| 300629 | AA152119 | Hs.155101 | ATP synthase; H+ transporting; mitochondrial F1 complex; alpha subunit; | |
| | | | isoform 1; cardiac muscle | 0.077 |
| 333160 | | | CH22_FGENES.91_2 | 0.077 |
| 337490 | | | CH22_FGENES.799-5 | 0.077 |
| 305403 | AA723748 | | EST singleton (not in UniGene) with exon hit | 0.077 |
| 331747 | AA281765 | Hs.193689 | ESTs | 0.077 |
| 332792 | | | CH22_FGENES.3_2 | 0.078 |
| 330513 | M81057 | Hs.180884 carboxypeptidase B1 | (tissue) | 0.078 |
| 308905 | AI859636 | Hs.8102 | ribosomal protein S20 | 0.078 |
| 337419 | | | CH22_FGENES.759-4 | 0.078 |
| 333459 | | | CH22_FGENES.157_8 | 0.078 |
| 334851 | | | CH22_FGENES.440_3 | 0.078 |
| 329046 | | | CH.X_hs gi\|5868569 | 0.078 |
| 327879 | | | CH.06_hs gi\|5868142 | 0.079 |
| 305830 | AA857665 | | EST singleton (not in UniGene) with exon hit | 0.079 |
| 302928 | AL137719 | | EST cluster (not UniGene) with exon hit | 0.079 |
| 304321 | AA136698 | Hs.113029 | ribosomal protein S25 | 0.079 |
| 326390 | | | CH.19_hs gi\|5867340 | 0.079 |
| 335230 | | | CH22_FGENES.514_2 | 0.08 |
| 334622 | | | CH.22_FGENES.412_6 | 0.08 |
| 335331 | | | CH22_FGENES.535_4 | 0.08 |
| 304753 | AA578840 | Hs.77961 | major histocompatibility complex; class I; B | 0.08 |
| 301863 | AI418863 | | EST cluster (not in UniGene) with exon hit | 0.081 |
| 336561 | | | CH22_FGENES.842_6 | 0.081 |
| 335611 | | | CH22_FGENES.583_5 | 0.081 |
| 305060 | AA635771 | | EST singleton (not in UniGene) with exon hit | 0.081 |
| 306051 | AA905130 | | EST singleton (not in UniGene) with exon hit | 0.082 |
| 308289 | AI571211 | | EST singleton (not in UniGene) with exon hit | 0.082 |
| 334365 | | | CH22_FGENES.378_13 | 0.082 |
| 335496 | | | CH22_FGENES.571_4 | 0.082 |
| 332634 | S38953 | | Human unidentified gene complementary to P450c21 | |
| | | | gene; partial cds | 0.082 |
| 337824 | | | CH22_EM:AC005500.GENSCAN.13-18 | 0.082 |
| 335822 | | | CH22 FGENES.619_7 | 0.082 |
| 334758 | | | CH22_FGENES.428_7 | 0.082 |
| 309641 | AW194230 | Hs.253100 | EST | 0.082 |
| 333064 | | | CH22_FGENES.75_7 | 0.083 |
| 338695 | | | CH22_EM:AC005500.GENSCAN.477.25 | 0.083 |
| 331809 | AA402482 | Hs.97312 | ESTs | 0.083 |
| 326138 | | | CH.17_hs gi\|5867203 | 0.083 |
| 328304 | | | CH.07_hs gi\|6004478 | 0.083 |
| 330570 | U60276 | Hs.165439 | arsA (bacterial) arsenite transporter; ATP-binding; homolog 1 | 0.083 |
| 334305 | | | CH22_FGENES.373_8 | 0.083 |
| 335885 | | | CH22_FGENES.632_3 | 0.083 |
| 325839 | | | CH.16_hs gi\|6552452 | 0.083 |
| 333531 | | | CH22_FGENES.175_18 | 0.084 |
| 330385 | AA449749 | Hs.31386 | ESTs; Highly similar to secreted apoptosis related protein | |
| | | | 1 [H.sapiens] | 0.084 |
| 323305 | AA811351 | Hs.25307 | Homo sapiens clone 24812 mRNA sequence | 0.084 |
| 331698 | Z39929 | Hs.65843 | ESTs | 0.084 |
| 335888 | | | CH22_FGENES.633_2 | 0.084 |
| 306008 | AA894390 | | EST singleton (not in UniGene) with exon hit | 0.084 |
| 334249 | | | CH22_FGENES.365_15 | 0.084 |
| 318303 | AW451197 | Hs.113418 | ESTs | 0.084 |
| 330171 | | | CH.02_p2 gi\|6648220 | 0.084 |
| 336662 | | | CH22_FGENES.41-1 | 0.085 |
| 320506 | AI815668 | Hs.157476 | suc1-associated neurotrophic factor target 2 | |
| | | | (FGFR signalling adaptor) | 0.085 |
| 316974 | AI740721 | Hs.128292 | ESTs | 0.085 |
| 336492 | | | CH22_FGENES.832_9 | 0.085 |
| 335750 | | | CH22_FGENES.602_4 | 0.085 |
| 335676 | | | CH22_FGENES.594_1 | 0.086 |
| 336093 | | | CH22_FGENES.691_2 | 0.086 |
| 310932 | AI933861 | Hs.222852 | ESTs | 0.086 |
| 335160 | | | CH22_FGENES.502_4 | 0.086 |
| 334306 | | | CH22_FGENES.373_9 | 0.086 |
| 334793 | | | CH22_FGENES.433_5 | 0.086 |
| 333936 | | | CH22_FGENES.301_2 | 0.087 |
| 336413 | | | CH22_FGENES.823_35 | 0.087 |
| 333775 | | | CH22_FGENES.272_6 | 0.087 |
| 335971 | | | CH22_FGENES.652_4 | 0.087 |
| 301737 | AI815981 | | EST cluster (not in UniGene) with exon hit | 0.087 |
| 339101 | | | CH22_DA59H18.GENSCAN.44-6 | 0.087 |
| 327612 | | | CH.04_hs gi\|6525283 | 0.087 |
| 326241 | | | CH.17_hs gi\|5867260 | 0.088 |
| 338386 | | | CH22_EM:AC005500.GENSCAN.331-4 | 0.088 |
| 327762 | | | CH.05_hs gi\|5867961 | 0.088 |
| 305266 | AA679772 | | EST singleton (not in UniGene) with exon hit | 0.088 |
| 334359 | | | CH22_FGENES.378_4 | 0.088 |
| 335500 | | | CH22_FGENES.571_10 | 0.088 |
| 329687 | | | CH.14_p2 gi\|6117856 | 0.088 |
| 333654 | | | CH22_FGENES.240_2 | 0.088 |
| 324430 | AA464018 | | EST cluster (not in UniGene) | 0.088 |
| 325999 | | | CH.16_hs gi\|5867073 | 0.089 |
| 334832 | | | CH22_FGENES.439_1 | 0.089 |
| 339115 | | | CH22_DA59H18.GENSCAN.49-3 | 0.089 |
| 300896 | AI916902 | Hs.213882 | ESTs | 0.089 |
| 328784 | | | CH.07_hs gi\|5868309 | 0.089 |
| 335044 | | | CH22_FGENES.480_1 | 0.089 |
| 329791 | | | CH.14_p2gi\|6469354 | 0.089 |
| 333656 | | | CH22_FGENES.240_4 | 0.089 |
| 326180 | | | CH.17_hs gi\|5867211 | 0.089 |
| 333391 | | | CH22_FGENES.144_6 | 0.089 |
| 338324 | | | CH22_EM:AC005500.GENSCAN.306-3 | 0.089 |
| 305396 | AA721052 | | EST singleton (not in UniGene) with exon hit | 0.089 |
| 337483 | | | CH22_FGENES.795-7 | 0.09 |
| 326424 | | | CH.19_hs gi\|5867369 | 0.09 |
| 306454 | AA977992 | | EST singleton (not in UniGene) with exon hit | 0.09 |
| 338893 | | | CH22_DJ32l10.GENSCAN.7-6 | 0.09 |
| 327470 | | | CH.02_hs gi\|5867772 | 0.09 |
| 333165 | | | CH22_FGENES.91_7 | 0.09 |
| 307155 | AI186738 | Hs.182426 | ribosomal protein S2 | 0.09 |
| 330717 | AA233926 | Hs.23635 | ESTs | 0.09 |
| 335334 | | | CH22_FGENES.535_10 | 0.09 |
| 335907 | | | CH22_FGENES.636_2 | 0.09 |
| 333885 | | | CH22_FGENES.292_7 | 0.09 |
| 331034 | N51868 | Hs.31965 | ESTs; Moderately similar to 40S RIBOSOMAL | |
| | | | PROTEIN S20 [H.sapiens] | 0.09 |
| 304660 | AA534416 | Hs.162185 | ESTs | 0.09 |
| 328217 | | | CH.06_hs gi\|5868096 | 0.091 |
| 336068 | | | CH22_FGENES.684_13 | 0.091 |
| 302833 | AA295381 | Hs.44423 | ESTs | 0.091 |
| 328668 | | | CH.07_hs gi\|5868254 | 0.091 |
| 335309 | | | CH22_FGENES.532_2 | 0.091 |
| 338481 | | | CH22_EM:AC005500.GENSCAN.377-5 | 0.091 |
| 306286 | AA936892 | | EST singleton (not in UniGene) with exon hit | 0.091 |
| 305070 | AA639783 | | EST singleton (not in UniGene) with exon hit | 0.091 |
| 304870 | AA594811 | Hs.119122 | ribosomal protein L13a | 0.091 |
| 303856 | AA968589 | Hs.944 | glucose phosphate isomerase | 0.091 |
| 323789 | AI459812 | Hs.170460 | ESTs;Weakly similar to KIAA0990 protein [H.sapiens] | 0.092 |
| 334910 | | | CH22_FGENES.455_3 | 0.092 |
| 326382 | | | CH.19_hs gi\|5867327 | 0.092 |
| 332467 | AA489630 | Hs.119004 | KIAA0665 gene product | 0.092 |
| 338534 | | | CH22_EM:AC005500.GENSCAN.402-7 | 0.092 |
| 336449 | | | CH22_FGENES.829_6 | 0.092 |
| 333709 | | | CH22_FGENES.250_24 | 0.092 |
| 336559 | | | CH22_FGENES.842_4 | 0.092 |
| 333230 | | | CH22_FGENES.107_10 | 0.093 |
| 333133 | | | CH22_FGENES.83_9 | 0.093 |
| 334885 | | | CH22_FGENES.451_11 | 0.093 |
| 330605 | X02419 | Hs.77274 | plasminogen activator; urokinase | 0.093 |
| 336392 | | | CH22_FGENES.823_4 | 0.093 |
| 334083 | | | CH22_FGENES.327_38 | 0.093 |
| 325469 | | | CH.12_hs gi\|6017034 | 0.093 |
| 331077 | R09531 | Hs.19039 | ESTs | 0.093 |
| 303701 | AW500732 | | EST cluster (not in UniGene) with exon hit | 0.093 |
| 334218 | | | CH22_FGENES.358_3 | 0.093 |
| 336542 | | | CH22_FGENES.840_6 | 0.093 |
| 337151 | | | CH22_FGENES.546-1 | 0.093 |
| 333642 | | | CH22_FGENES.231_2 | 0.093 |
| 336863 | | | CH22_FGENES.297-4 | 0.093 |
| 334680 | | | CH22_FGENES.419_2 | 0.093 |
| 326365 | | | CH.18_hs gi\|5867297 | 0.093 |
| 338952 | | | CH22_DJ32l10.GENSCAN.23-22 | 0.093 |
| 337539 | | | CH22_FGENES.832-4 | 0.094 |
| 333546 | | | CH22_FGENES.180_2 | 0.094 |
| 335258 | | | CH22_FGENES.518_3 | 0.094 |
| 336786 | | | CH22_FGENES.168-19 | 0.094 |
| 321644 | AI204177 | Hs.237396 | ESTs | 0.094 |
| 335943 | | | CH22_FGENES.646_17 | 0.094 |
| 327918 | | | CH.06_hs gi\|5868165 | 0.094 |
| 306398 | AA970548 | | EST singleton (not in UniGene) with exon hit | 0.094 |
| 335671 | | | CH22_FGENE5.592_3 | 0.094 |
| 335033 | | | CH22_FGENES.475_11 | 0.094 |
| 338277 | | | CH22_EM:AC005500.GENSCAN.290-2 | 0.094 |
| 332061 | AA504812 | Hs.192824 | early B-cell factor | 0.094 |
| 305153 | AA654582 | Hs.77039 | ribosomal protein S3A | 0.094 |
| 333880 | | | CH22_FGENES.292_2 | 0.094 |
| 323940 | AI864428 | Hs.170880 | ESTs | 0.094 |
| 313779 | AA648796 | Hs.129771 | ESTs | 0.095 |
| 323109 | AA169345 | | EST cluster (not in UniGene) | 0.095 |
| 332930 | | | CH22_FGENES.38_4 | 0.095 |
| 335368 | | | CH22_FGENES.543_6 | 0.095 |
| 303887 | R72672 | Hs.193484 | ESTs; Weakly similar to Similarity with yeast gene | |
| | | | L3502.1 [C.elegans] | 0.095 |
| 336223 | | | CH22_FGENES.727 _3 | 0.095 |
| 311280 | AI767957 | Hs.197737 | ESTs; Weakly similar to Y38A8.1 gene product [C. elegans] | 0.095 |
| 337256 | | | CH22_FGENES.648-3 | 0.095 |
| 308814 | AI819263 | | EST singleton (not in UniGene) with exon hit | 0.095 |
| 334659 | | | CH22_FGENES.416_7 | 0.095 |
| 335895 | | | CH22_FGENES.635_3 | 0.095 |
| 321697 | AW388061 | Hs.4953 | golgi autoantigen; golgin subfamily a; 3 | 0.095 |
| 336010 | | | CH22_FGENES.668_8 | 0.096 |
| 302824 | U21260 | | EST cluster (not in UniGene) with exon hit | 0.096 |
| 333612 | | | CH22_FGENES.217_7 | 0.096 |
| 304823 | AA584837 | | EST singleton (not in UniGene) with exon hit | 0.096 |
| 335665 | | | CH22_FGENES.590_16 | 0.096 |
| 306518 | AA989598 | | EST singleton (not in UniGene) with exon hit | 0.096 |
| 335243 | | | CH22_FGENES.516_4 | 0.096 |
| 335436 | | | CH22_FGENES.559_5 | 0.096 |
| 300243 | AI420256 | Hs.161271 | ESTs | 0.096 |
| 332810 | | | CH22_FGENES.7_12 | 0.097 |
| 308612 | AI735634 | | EST singleton (not in UniGene) with exon hit | 0.097 |
| 335818 | | | CH22_FGENES.618_6 | 0.097 |
| 325838 | | | CH.16_hs gi\|6552452 | 0.097 |
| 337482 | | | CH22_FGENES.795-6 | 0.097 |
| 336645 | | | CH22_FGENES.26-1 | 0.097 |
| 337293 | | | CH22_FGENES.675-1 | 0.098 |
| 329893 | | | CH.15_p2 gi\|6525313 | 0.098 |
| 326533 | | | CH.19_hs gi\|5867441 | 0.098 |
| 334905 | | | CH22_FGENES.452_20 | 0.098 |
| 306347 | AA961144 | | EST singleton (not in UniGene) with exon hit | 0.098 |
| 336676 | | | CH22_FGENES.43-4 | 0.098 |
| 339166 | | | CH22_DA59H18.GENSCAN.69-7 | 0.098 |
| 335774 | | | CH22_FGENES.607_10 | 0.098 |
| 339216 | | | CH22_FF113D11.GENSCAN.6-11 | 0.098 |
| 335311 | | | CH22_FGENES.532_4 | 0.098 |
| 329632 | | | CH.11_p2 gi\|6729060 | 0.098 |
| 328595 | | | CH.07_hs gi\|5868224 | 0.098 |
| 326928 | | | CH.21_hs gi\|6456782 | 0.098 |
| 315234 | AI079680 | Hs.120770 | ESTs | 0.098 |
| 306082 | AA908508 | | EST singleton (not in UniGene) with exon hit | 0.098 |
| 305710 | AA826544 | | EST singleton (not in UniGene) with exon hit | 0.098 |
| 318540 | T30280 | | EST cluster (not in UniGene) | 0.099 |
| 337553 | | | CH22_C4G1.GENSCAN.2-1 | 0.099 |
| 320951 | AA344069 | Hs.202699 | neurexophilin 4 | 0.099 |
| 303845 | T08033 | | EST cluster (not in UniGene) with exon hit | 0.099 |
| 338981 | | | CH22_DA59H18.GENSCAN2-5 | 0.099 |
| 321313 | R87365 | Hs.26058 | ESTs; Weakly similar to p532 [H.sapiens] | 0.099 |
| 328348 | | | CH.07_hs gi\|5868383 | 0.099 |
| 332203 | H49388 | Hs.102082 | EST | 0.099 |
| 301780 | R07064 | | EST cluster (not in UniGene) with exon hit | 0.099 |
| 332095 | AA608838 | Hs.162681 | EST | 0.099 |
| 333227 | | | CH22_FGENES.107_5 | 0.099 |
| 316442 | AA760894 | Hs.153023 | ESTs | 0.099 |
| 326001 | | | CH.16_hs gi\|5867073 | 0.099 |
| 334363 | | | CH22_FGENES.378_11 | 0.099 |
| 338895 | | | CH22_DJ32l10.GENSCAN.9-2 | 0.099 |
| 327460 | | | CH.02_hs gi\|6004455 | 0.099 |
| 332705 | T59161 | Hs.76293 | thymosin; beta 10 | 0.1 |
| 307806 | AI351739 | | EST singleton (not in UniGene) with exon hit | 0.1 |
| 322800 | F25037 | Hs.225175 | ESTs | 0.1 |
| 304918 | AA602697 | | EST singleton (not in UniGene) with exon hit | 0.1 |
| 334327 | | | CH22_FGENES.375_4 | 0.1 |
| 318359 | AI097439 | Hs.135548 | ESTs | 0.1 |
| 326644 | | | CH.20_hs gi\|5867559 | 0.1 |
| 334454 | | | CH22_FGENES.388_3 | 0.1 |
| 327959 | | | CH.06_hs gi\|5868210 | 0.1 |
| 323783 | AA330586 | Hs.131819 | ESTs | 0.1 |
| 309198 | AI955915 | Hs.248038 | major histocompatibility complex; class I; C | 0.1 |
| 339265 | | | CH22_BA354l12.GENSCAN.10-3 | 0.1 |
| 320576 | AL049977 | Hs.162209 | Homo sapiens mRNA; cDNA DKFZp564C122 | |
| | | | (from clone DKFZp564C122) | 0.1 |
| 338132 | | | CH22_EM:AC005500.GENSCAN.200-2 | 0.1 |
| 333163 | | | CH22_FGENES.91_5 | 0.101 |
| 337584 | | | CH22 C20H12.GENSCAN.5-1 | 0.101 |
| 307588 | AI285535 | | EST singleton (not in UniGene) with exon hit | 0.101 |
| 336969 | | | CH22_FGENES.378-2 | 0.101 |
| 327535 | | | CH.02_hs gi\|6525279 | 0.101 |
| 328732 | | | CH.07_hs gi\|5868289 | 0.101 |
| 336686 | | | CH22_FGENES.46-3 | 0.101 |
| 335777 | | | CH22_FGENES.607_13 | 0.101 |
| 332944 | | | CH22_FGENES.47_3 | 0.101 |
| 333174 | | | CH22_FGENES.95_1 | 0.101 |
| 336380 | | | CH22_FGENES.821_8 | 0.101 |
| 330571 | U60800 | Hs.79089 | sema domain; immunoglobulin domain (Ig); | |
| | | | cytoplasmic domain; (semaphorin) 4D | 0.101 |
| 331789 | AA398721 | Hs.186749 | ESTs | 0.101 |
| 338915 | | | CH22_DJ32l10.GENSCAN.12-1 | 0.101 |
| 334844 | | | CH22_FGENES.439_24 | 0.101 |
| 336642 | | | CH22_FGENES.23-4 | 0.101 |
| 334906 | | | CH22_FGENES.452_21 | 0.101 |
| 333188 | | | CH22_FGENES.98_8 | 0.101 |
| 300088 | AW299993 | | EST cluster (not in UniGene) with exon hit | 0.101 |
| 329373 | | | CH.X_hs gi\|6682537 | 0.102 |
| 331120 | R46576 | Hs.23239 | ESTs | 0.102 |
| 335856 | | | CH22_FGENES.628_1 | 0.102 |
| 331888 | AA431337 | Hs.98017 | ESTs | 0.102 |
| 333154 | | | CH22_FGENES.89_4 | 0.102 |
| 335989 | | | CH22_FGENES.655_2 | 0.102 |
| 304385 | AA235602 | | EST singleton (not in UniGene) with exon hit | 0.102 |
| 338016 | | | CH22_EM:AC005500.GENSCAN.133-1 | 0.102 |
| 335190 | | | CH22_FGENES.507_5 | 0.102 |
| 318595 | T39486 | Hs.6137 | ESTs | 0.102 |
| 333697 | | | CH22_FGENES.250_11 | 0.102 |
| 306526 | AA989713 | | EST singleton (not in UniGene) with exon hit | 0.103 |
| 328734 | | | CH.07_hs gi\|5868289 | 0.103 |
| 307294 | AI205612 | Hs.73742 | ribosomal protein: large; P0 | 0.103 |
| 327424 | | | CH.02_hs gi\|5867751 | 0.103 |
| 335872 | | | CH22_FGENES.630_3 | 0.103 |
| 333572 | | | CH22_FGENES.189_1 | 0.103 |
| 334774 | | | CH22_FGENES.430_6 | 0.103 |
| 338660 | | | CH22_EM:AC005500.GENSCAN.462-1 | 0.103 |
| 326713 | | | CH20_hs gi\|5867595 | 0.103 |
| 333994 | | | CH22_FGENES.310_18 | 0.103 |
| 335800 | | | CH22_FGENES.613_4 | 0.103 |
| 318113 | AI187943 | Hs.132322 | ESTs | 0.103 |
| 337278 | | | CH22_FGENES.665-1 | 0.103 |
| 336386 | | | CH22_FGENES.822_6 | 0.103 |
| 334790 | | | CH22_FGENES.432_15 | 0.103 |
| 303778 | AW505368 | | EST cluster (not in UniGene) with exon hit | 0.104 |
| 336524 | | | CH22_FGENES.839_5 | 0.104 |
| 328936 | | | CH.08_hs gi\|5868500 | 0.104 |
| 335102 | | | CH22_FGENES.494_7 | 0.104 |
| 300935 | AA513644 | Hs.222815 | ESTs; Weakly similar to Wiskott-Aldrich Syndrome | |
| | | | protein [H.sapiens] | 0.104 |
| 307581 | AI284415 | | EST singleton (not in UniGene) with exon hit | 0.104 |
| 317301 | AW291683 | Hs.226056 | ESTs | 0.104 |
| 335330 | | | CH22_FGENES.535_3 | 0.104 |
| 337968 | | | CH22_EM:AC005500.GENSCAN.103-2 | 0.104 |
| 335627 | | | CH22_FGENES.584_7 | 0.104 |
| 336274 | | | CH22_FGENES.762_2 | 0.104 |
| 334730 | | | CH22_FGENES.424_5 | 0.105 |
| 334409 | | | CH22_FGENES.383_6 | 0.105 |
| 327237 | | | CH.01_hs gi\|5867544 | 0.105 |
| 333321 | | | CH22_FGENES.138_13 | 0.105 |
| 303181 | AA452366 | | EST cluster (not in UniGene) with exon hit | 0.105 |
| 333738 | | | CH22_FGENES.261_2 | 0.105 |
| 338255 | | | CH22_EM:AC005500.GENSCAN.276-3 | 0.105 |
| 334282 | | | CH22_FGENES.369_12 | 0.105 |
| 330190 | | | CH.05_p2 gi\|6165182 | 0.105 |
| 310748 | AW014249 | Hs.158698 | ESTs | 0.105 |
| 338150 | | | CH22_EM:AC005500.GENSCAN.207-2 | 0.105 |
| 336719 | | | CH22_FGENES.82-6 | 0.105 |
| 330228 | | | CH.05_p2 gi\|6013527 | 0.105 |
| 327801 | | | CH.05_hs gi\|5867924 | 0.105 |
| 330525 | S75168 | Hs.274 | megakaryocyte-associated tyrosine kinase | 0.105 |
| 334972 | | | CH22_FGENES.468_2 | 0.105 |
| 335111 | | | CH22_FGENES.494_19 | 0.106 |
| 334483 | | | CH22_FGENES.395_5 - | 0.106 |
| 328829 | | | CH.07_hs gi\|5868337 | 0.106 |
| 302753 | M74299 | | EST cluster (not in UniGene) with exon hit | 0.106 |
| 334512 | | | CH22_FGENES.398_10 | 0.106 |
| 330024 | | | CH.16_p2 gi\|6671908 | 0.106 |
| 321030 | AI769930 | Hs.233617 | Homo sapiens (clone B3B3E13)Huntington's | |
| | | | disease candidate region | 0.107 |
| 338410 | | | CH22_EM:AC005500.GENSCAN.341-6 | 0.107 |
| 334353 | | | CH22_FGENES.376_5 | 0.107 |
| 338276 | | | CH22_EM:AC005500.GENSCAN.288-9 | 0.107 |
| 329053 | | | CH.X_hs gi\|5868574 | 0.107 |
| 336560 | | | CH22_FGENES.842_5 | 0.107 |
| 332158 | AA621363 | Hs.112980 | EST | 0.107 |
| 336447 | | | CH22_FGENES.829_4 | 0.107 |
| 333703 | | | CH22_FGENES.250_17 | 0.107 |
| 326207 | | | CH.17_hs gi\|5867222 | 0.107 |
| 333232 | | | CH22_FGENES.108_1 | 0.107 |
| 334802 | | | CH22_FGENES.435_1 | 0.107 |
| 303784 | AA704983 | | EST cluster (not in UniGene) with exon hit | 0.107 |
| 338847 | | | CH22_DJ246D7.GENSCAN.10-2 | 0.107 |
| 339407 | | | CH22_DJ579N16.GENSCAN.1-9 | 0.108 |
| 337635 | | | CH22_C20H12.GENSCAN.32-8 | 0.108 |
| 334650 | | | CH22_FGENES.417_17 | 0.108 |
| 308511 | AI687580 | | EST singleton (not in UniGene) with exon hit | 0.108 |
| 333392 | | | CH22_FGENES.144_8 | 0.108 |
| 325840 | | | CH.16_hs gi\|6552452 | 0.108 |
| 315044 | AW205664 | Hs.129568 | ESTs | 0.108 |
| 333298 | | | CH22_FGENES.133_4 | 0.108 |
| 335157 | | | CH22_FGENES.501_7 | 0.108 |
| 333305 | | | CH22_FGENES.137_2 | 0.108 |
| 326379 | | | CH.19_hs gi\|5867327 | 0.108 |
| 335050 | | | CH22_FGENES.482_1 | 0.108 |
| 305185 | AA663985 | Hs.248038 | major histocompatibility complex; class I; C | 0.108 |
| 335658 | | | CH22_FGENES.590_9 | 0.108 |
| 323040 | AA336609 | Hs.10862 | ESTs | 0.108 |
| 337326 | | | CH22_FGENES.699-6 | 0.108 |
| 339262 | | | CH22_BA354l12.GENSCAN.9-6 | 0.108 |
| 321202 | H54052 | Hs.163639 | ESTs; Weakly similar to INTERCELLULAR ADHESION | |
| | | | MOLECULE-1 PRECURSOR [H.sapiens] | 0.109 |
| 331792 | AA398968 | Hs.97548 | EST | 0.109 |
| 333806 | | | CH22_FGENES.278_2 | 0.109 |
| 321325 | AB033100 | | EST cluster (not in UniGene) | 0.109 |
| 331373 | AA435513 | Hs.178170 | ESTs; Weakly similar to DUAL SPECIFICITY | |
| | | | PROTEIN PHOSPHATASE 3 | 0.87 |
| 328775 | | | CH.07_hs gi\|5868309 | 0.109 |
| 335105 | | | CH22_FGENES.494_10 | 0.109 |
| 300975 | AI283548 | Hs.149668 | ESTs | 0.109 |
| 324893 | T31940 | | EST cluster (not in UniGene) | 0.109 |
| 333397 | | | CH22_FGENES.144_15 | 0.109 |
| 336484 | | | CH22_FGENES.831_3 | 0.109 |
| 335507 | | | CH22_FGENES.571_22 | 0.109 |
| 336373 | | | CH22_FGENES.820_3 | 0.109 |
| 336188 | | | CH22_FGENES.717_12 | 0.109 |
| 313455 | AW081702 | Hs.137329 | ESTs | 0.109 |
| 335185 | | | CH22_FGENES.506_4 | 0.109 |
| 306814 | AI066577 | | EST singleton (not in UniGene) with exon hit | 0.109 |
| 311130 | AI632322 | Hs.195306 | ESTs | 0.109 |
| 310882 | AW080339 | Hs.211911 | ESTs | 0.109 |
| 323383 | AI346359 | Hs.135209 | ESTs | 0.11 |
| 300212 | AW135925 | Hs.184552 | biphenylhydrolase-like (serine hydrolase; breast epithelial | |
| | | | mucin-assoc. | 0.11 |
| 325675 | | | CH.14_hs gi\|5867014 | 0.11 |
| 330095 | | | CH.19_p2 gi\|6015278 | 0.11 |
| 331942 | AA453261 | Hs.99309 | ESTs | 0.11 |
| 334723 | | | CH22_FGENES.421_34 | 0.11 |
| 333614 | | | CH22_FGENES.217_9 | 0.11 |
| 337316 | | | CH22_FGENES.692-1 | 0.11 |
| 305057 | AA635626 | Hs.62954 | ferritin; heavy polypeptide 1 | 0.11 |
| 338704 | | | CH22_EM:AC005500.GENSCAN.480-3 | 0.11 |
| 335385 | | | CH22_FGENES.543_27 | 0.11 |
| 338012 | | | CH22_EM:AC005500.GENSCAN.128-10 | 0.11 |
| 329449 | | | CH.Y_hs gi\|5868886 | 0.11 |
| 338980 | | | CH22_DA59H18.GENSCAN.2-4 | 0.11 |
| 336553 | | | CH22_FGENES.841_10 | 0.111 |
| 330021 | | | CH.16_p2 gi\|6671889 | 0.111 |
| 327579 | | | CH.03_hs gi\|5867824 | 0.111 |
| 333099 | | | CH22_FGENES.79_4 | 0.111 |
| 337076 | | | CH22_FGENES.453-4 | 0.111 |
| 331388 | AA456852 | Hs.43543 | suppressor of white apricot homolog 2 | 0.111 |
| 306674 | AI005542 | Hs.180414 | heat shock 70kD protein 10 (HSC71) | 0.111 |
| 305949 | AA884409 | | EST singleton (not in UniGene) with exon hit | 0.111 |
| 330748 | AA419217 | Hs.15911 | DKFZP586E1422 protein | 0.111 |
| 333780 | | | CH22_FGENES.273_2 | 0.111 |
| 323676 | AI702835 | | EST cluster (not in UniGene) | 0.111 |
| 308952 | AI868157 | Hs.224226 | EST | 0.111 |
| 309338 | AW026946 | Hs.181165 | eukaryotic translation elongation factor 1 alpha 1 | 0.111 |
| 329317 | | | CH.X_hs gi\|6381976 | 0.112 |
| 333518 | | | CH22_FGENES.173_3 | 0.112 |
| 306982 | AI127883 | | EST singleton (not in UniGene) with exon hit | 0.112 |
| 336225 | | | CH22_FGENES.728_2 | 0.112 |
| 333698 | | | CH22_FGENES.250_12 | 0.112 |
| 302173 | AI417947 | Hs.14068 | ESTs | 0.112 |
| 335510 | | | CH22_FGENES.571_25 | 0.112 |
| 328042 | | | CH.06_hs gi\|5902482 | 0.112 |
| 336512 | | | CH22_FGENES.834_7 | 0.112 |
| 328541 | | | CH.07_hs gi\|5868486 | 0.112 |
| 311265 | AW205118 | Hs.199214 | ESTs | 0.112 |
| 323218 | AF131846 | Hs.13396 | Homo sapiens clone 25028 mRNA sequence | 0.112 |
| 302002 | AF013956 | Hs.123085 | chromobox homolog 4 (Drosophila Pc class) | 0.112 |
| 315088 | AA557351 | Hs.152448 | ESTs;Moderately similar to MULTIFUNCTIONAL PROTEIN ADE2 | 0.112 |
| 312581 | AI937242 | Hs.176590 | ESTs | 0.112 |
| 322246 | AW384710 | Hs.125258 | ESTs | 0.112 |
| 333659 | | | CH22_FGENES.241_5 | 0.113 |
| 327510 | | | CH.02_hs gi\|6117815 | 0.113 |
| 336520 | | | CH22_FGENES.839_1 | 0.113 |
| 338682 | | | CH22_EM:AC005500.GENSCAN.472-1 | 0.113 |
| 334508 | | | CH22_FGENES.398_6 | 0.113 |
| 322533 | T59538 | | EST cluster (not in UniGene) | 0.113 |
| 306873 | AI086929 | | EST singleton (not in UniGene) with exon hit | 0.113 |
| 336040 | | | CH22_FGENES.679_2 | 0.113 |
| 303898 | T23215 | | EST cluster (not in UniGene) with exon hit | 0.113 |
| 312011 | AW294868 | Hs.187226 | ESTs | 0.113 |
| 335186 | | | CH22_FGENES.506_5 | 0.113 |
| 333607 | | | CH22_FGENES.216_2 | 0.113 |
| 305549 | AA773530 | | EST singleton (not in UniGene) with exon hit | 0.113 |
| 333686 | | | CH22_FGENES.249_4 | 0.113 |
| 334352 | | | CH22_FGENES.376_3 | 0.113 |
| 338195 | | | CH22_EM:AC005500.GENSCAN.233-18 | 0.114 |
| 333588 | | | CH22_FGENES.206_2 | 0.114 |
| 339233 | | | CH22_BA354l12.GENSCAN.2-3 | 0.114 |
| 337455 | | | CH22_FGENES.777-1 | 0.114 |
| 309101 | AI925108 | | EST singleton (not in UniGene) with exon hit | 0.114 |
| 328522 | | | CH.07_hs gi\|5868477 | 0.114 |
| 323999 | AI537333 | Hs.252782 | ESTs | 0.114 |
| 333517 | | | CH22_FGENES.173_2 | 0.114 |
| 329935 | | | CH.16_p2 gi\|6165200 | 0.114 |
| 326226 | | | CH.17_hs gi\|5867230 | 0.114 |
| 335890 | | | CH22_FGENES.633_4 | 0.114 |
| 336715 | | | CH22_FGENES.77-1 | 0.114 |
| 327640 | | | CH.04_hs gi\|5867890 | 0.114 |
| 338842 | | | CH22_DJ246D7.GENSCAN.7-1 | 0.114 |
| 306534 | AA991487 | | EST singleton (not in UniGene) with exon hit | 0.114 |
| 336597 | | | CH22_FGENES.266_1 | 0.114 |
| 321010 | Y17456 | Hs.227150 | Homo sapiens LSFR2 gene; last exon | 0.114 |
| 302294 | AA159213 | Hs.5337 | isocitrate dehydrogenase 2 (NADP+); mitochondrial | 0.114 |
| 324895 | N44238 | Hs.77515 | inositol 1;4;5-triphosphate receptor, type 3 | 0.114 |
| 327358 | | | CH.01_hs gi\|6552411 | 0.114 |
| 308792 | AI815153 | Hs.195188 | glyceraldehyde-3-phosphate dehydrogenase | 0.115 |
| 325886 | | | CH.16_hs gi\|5867087 - | 0.115 |
| 336850 | | | CH22_FGENES.272-11 | 0.115 |
| 305858 | AAB63103 | | EST singleton (not in UniGene) with exon hit | 0.115 |
| 302569 | AC004472 | | multiple UniGene matches | 0.115 |
| 336158 | | | CH22_FGENES.707_2 | 0.115 |
| 327866 | | | CH.06_hs gi\|5868131 | 0.115 |
| 339157 | | | CH22_DA59H18.GENSCAN.67-3 | 0.115 |
| 339258 | | | CH22_BA354l12.GENSCAN.8-3 | 0.115 |
| 336129 | | | CH22_FGENES.701_17 | 0.115 |
| 333684 | | | CH22_FGENES.249_2 | 0.115 |
| 309618 | AW190162 | Hs.184776 | ribosomal protein L23a | 0.115 |
| 312928 | AA954097 | Hs.127523 | ESTs | 0.115 |
| 302640 | AB035698 | | EST cluster (not in UniGene) with exon hit | 0.115 |
| 328968 | | | CH.08_hs gi\|6456775 | 0.115 |
| 327902 | | | CH.06_hs gi\|5868158 | 0.115 |
| 321927 | AJ223366 | | EST cluster (not in UniGene) | 0.115 |
| 335962 | | | CH22_FGENES.651_4 | 0.115 |
| 334927 | | | CH22_FGENES.460_1 | 0.115 |
| 330535 | U11872 | | Human interleukin-8 receptor type B (IL8RB) mRNA, | |
| | | | splice variant IL8RB1 | 0.856 |
| 328591 | | | CH.07_hs gi\|5868227 | 0.115 |
| 334902 | | | CH22_FGENES.452_16 | 0.115 |
| 328525 | | | CH.07_hs gi\|5868482 | 0.115 |
| 325870 | | | CH.16_hs gi\|6682492 | 0.116 |
| 337522 | | | CH22_FGENES.819-1 | 0.116 |
| 305079 | AA641329 | | EST singleton (not in UniGene) with exon hit | 0.116 |
| 327343 | | | CH.01_hs gi\|6017017 | 0.116 |
| 333918 | | | CH22_FGENES.296_7 | 0.116 |
| 333600 | | | CH22_FGENES.213_2 | 0.116 |
| 335846 | | | CH22_FGENES.623_6 | 0.116 |
| 333510 | | | CH22_FGENES.171_4 | 0.116 |
| 327629 | | | CH.04_hs gi\|5867872 | 0.116 |
| 333470 | | | CH22_FGENES.161_6 | 0.116 |
| 326855 | | | CH.20_hs gi\|6552460 | 0.116 |
| 327008 | | | CH.21_hs gi\|5867664 | 0.117 |
| 337480 | | | CH22_FGENES.795-3 | 0.117 |
| 336425 | | | CH22_FGENES.824_10 | 0.117 |
| 321964 | AL079687 | Hs.171065 | ESTs | 0.117 |
| 335651 | | | CH22_FGENES.590_2 | 0.117 |
| 308164 | AI521574 | Hs.181165 | eukaryotic translation elongation factor 1 alpha 1 | 0.117 |
| 337927 | | | CH22_EM:AC005500.GENSCAN.80-3 | 0.117 |
| 300341 | H45095 | Hs.153524 | ESTs | 0.117 |
| 300154 | AI245127 | Hs.179331 | ESTs | 0.117 |
| 306295 | AA937331 | | EST singleton (not in UniGene) with exon hit | 0.117 |
| 329670 | | | CH.14_p2 gi\|6272129 | 0.117 |
| 335612 | | | CH22_FGENES.583_6 | 0.117 |
| 307845 | AI363450 | | EST singleton (not in UniGene) with exon hit | 0.117 |
| 330401 | D28383 | | Human mRNA for ATP synthase B chain, 5'UTR (sequence from | the |
| | | | 5'cap to the start codon) | 0.117 |
| 327127 | | | CH.21_hs gi\|6682520 | 0.117 |
| 333843 | | | CH22_FGENES.290_1 | 0.117 |
| 331083 | R17762 | Hs.22292 | ESTs | 0.117 |
| 329140 | | | CH.X_hs gi\|6017060 | 0.117 |
| 339338 | | | CH22_BA354l12.GENSCAN.27-3 | 0.117 |
| 331974 | AA464518 | Hs.99616 | ESTs | 0.117 |
| 338631 | | | CH22_EM:AC005500.GENSCAN.454-2 | 0.117 |
| 330299 | | | CH.06_p2 gi\|2905881 | 0.117 |
| 330351 | | | CH.09_p2 gi\|3056622 | 0.117 |
| 305377 | AA715714 | Hs.181357 | laminin receptor 1 (67kD; ribosomal protein SA) | 0.117 |
| 333106 | | | CH22_FGENES.79_12 | 0.117 |
| 338514 | | | CH22_EM:AC005500.GENSCAN.392-4 | 0.117 |
| 327335 | | | CH.01_hs gi\|5902477 | 0.117 |
| 301970 | AB028962 | Hs.120245 | KIAA1039 protein | 0.118 |
| 326339 | | | CH.17_hs gi\|6056311 | 0.118 |
| 330612 | X15673 | Hs.93174 | Human endogenous retrovirus pHE.1 (ERV9) | 0.118 |
| 334178 | | | CH22_ FGENES.350_6 | 0.118 |
| 328008 | | | CH.06_hs gi\|5902482 | 0.118 |
| 329976 | | | CH.16_p2 gi\|4878063 | 0.118 |
| 320952 | AA897432 | Hs.130411 | ESTs | 0.118 |
| 305621 | AA789095 | | EST singleton (not in UniGene) with exon hit | 0.118 |
| 337850 | | | CH22_EM:AC005500.GENSCAN.34-3 | 0.118 |
| 333626 | | | CH22_FGENES.224_2 | 0.118 |
| 337672 | | | CH22_EM:AC000097.GENSCAN.67-1 | 0.118 |
| 328803 | | | CH.07_hs gi\|6004475 | 0.118 |
| 325922 | | | CH.16_hs gi\|5867122 | 0.118 |
| 334489 | | | CH22_FGENES.397_1 | 0.118 |
| 320638 | R54766 | Hs.101120 | ESTs | 0.118 |
| 321932 | AA569229 | | EST cluster (not in UniGene) | 0.118 |
| 336958 | | | CH22_FGENES.367-1 | 0.118 |
| 332082 | AA600176 | Hs.112345 | ESTs | 0.118 |
| 306004 | AA889992 | | EST singleton (not in UniGene) with exon hit | 0.118 |
| 336803 | | | CH22_FGENES.194-1 | 0.118 |
| 309107 | AI925823 | | EST singleton (not in UniGene) with exon hit | 0.118 |
| 336859 | | | CH22_FGENES.293-9 | 0.118 |
| 337935 | | | CH22_EM:AC005500.GENSCAN.85-6 | 0.118 |
| 326492 | | | CH.19_hs gi\|5867422 | 0.118 |
| 327289 | | | CH.01_hs gi\|5867481 | 0.119 |
| 325818 | | | CH.14_hs gi\|6682490 | 0.119 |
| 310787 | AW262580 | Hs.159040 | ESTs | 0.119 |
| 330028 | | | CH.16_p2 gi\|6671908 | 0.119 |
| 325317 | | | CH.11_hs gi\|5866878 | 0.119 |
| 335279 | | | CH22_FGENES.523_7 | 0.119 |
| 331720 | AA192173 | Hs.221530 | ESTs | 0.119 |
| 329186 | | | CH.X_hs gi\|5868711 | 0.119 |
| 316012 | AA764950 | Hs.119898 | ESTs | 0.119 |
| 338316 | | | CH22_EM:AC005500.GENSCAN.304-2 | 0.119 |
| 326033 | | | CH.17_hs gi\|5867178 | 0.119 |
| 334745 | | | CH22_FGENES.426_3 | 0.119 |
| 333051 | | | CH22_FGENES.73_5 | 0.119 |
| 301763 | R01279 | | EST cluster (not in UniGene) with exon hit | 0.12 |
| 304502 | AA454809 | Hs.172928 | collagen; type I; alpha 1 | 0.12 |
| 335680 | | | CH22_FGENES.594_5 | 0.12 |
| 304678 | AA548556 | | EST singleton (not in UniGene) with exon hit | 0.12 |
| 335441 | | | CH22_FGENES.560_4 | 0.12 |
| 336187 | | | CH22_FGENES.717_11 | 0.12 |
| 309422 | AW087175 | | EST singleton (not in UniGene) with exon hit | 0.12 |
| 336047 | | | CH22_FGENES.679_9 | 0.12 |
| 309651 | AW195850 | | EST singleton (not in UniGene) with exon hit | 0.12 |
| 308547 | AI695385 | Hs.201903 | EST | 0.12 |
| 304443 | AA399444 | | EST singleton (not in UniGene) with exon hit | 0.12 |
| 336245 | | | CH22_FGENES.746_3 | 0.12 |
| 302703 | H72333 | | EST cluster (not in UniGene) with exon hit | 0.12 |
| 335690 | | | CH22_FGENES.596_5 | 0.12 |
| 328941 | | | CH.08_hs gi\|6456765 | 0.12 |
| 333873 | | | CH22_FGENES291_9 | 0.12 |
| 317246 | AW105092 | Hs.155690 | ESTs | 0.12 |
| 339288 | | | CH22_BA354l12.GENSCAN.16-6 | 0.12 |
| 337996 | | | CH22_EM:AC005500.GENSCAN.116-3 | 0.12 |
| 333304 | | | CH22_FGENES.137_1 | 0.121 |
| 308332 | AI591235 | | EST singleton (not in UniGene) with exon hit | 0.121 |
| 329319 | | | CH.X_hs gi\|6381976 | 0.121 |
| 302086 | X57138 | | multiple UniGene matches | 0.121 |
| 333290 | | | CH22_FGENES.129_2 | 0.121 |
| 323825 | AI793080 | Hs.123525 | ESTs; Weakly similar to NEUTROPHIL GELATINASE-ASSOCIATED | |
| | | | UPOCALIN PRECURSOR [R.norvegicus] | 0.121 |
| 330575 | U64105 | Hs.252280 | Rho guanine nucleotide exchange factor (GEF) 1 | 0.121 |
| 305274 | AA679990 | Hs.181165 | eukaryotic translation elongation factor 1 alpha 1 | 0.121 |
| 333647 | | | CH22_FGENES.235_2 | 0.121 |
| 302251 | AA333340 | | EST cluster (not in UniGene) with exon hit | 0.121 |
| 329777 | | | CH.14_p2 gi\|6002090 | 0.121 |
| 333155 | | | CH22_FGENES.89_5 | 0.121 |
| 326122 | | | CH.17_hs gi\|5867194 | 0.121 |
| 335310 | | | CH22_FGENES.532_3 | 0.121 |
| 335453 | | | CH22_FGENES.562_13 | 0.122 |
| 305103 | AA643329 | Hs.111334 | ferritin; light polypeptide | 0.122 |
| 337284 | | | CH22_FGENES.667.2 | 0.122 |
| 337418 | | | CH22_FGENES.758-4 | 0.122 |
| 313073 | AI963740 | Hs.46826 | ESTs | 0.122 |
| 303759 | AW504164 | | EST cluster (not in UniGene) with exon hit - | 0.122 |
| 300017 | | | | |
| | M33197 | | AFFX control: GAPDH | 0.122 |
| 316725 | AW135084 | Hs.127264 | ESTs | 0.122 |
| 330738 | AA293153 | Hs.120980 | nuclear receptor co-repressor 2 | 0.122 |
| 336466 | | | CH22_FGENES.829_25 | 0.122 |
| 335956 | | | CH22_FGENES.647_3 | 0.122 |
| 315308 | AA780564 | Hs.189053 | ESTs | 0.122 |
| 338925 | | | CH22_DJ32l10.GENSCAN.14-3 | 0.122 |
| 334969 | | | CH22_FGENES.466_2 | 0.122 |
| 322050 | AL137589 | | EST cluster (not in UniGene) | 0.122 |
| 339084 | | | CH22_DA59H18.GENSCAN.38-2 | 0.122 |
| 338323 | | | CH22_EM:AC005500.GENSCAN.306-2 | 0.122 |
| 337003 | | | CH22_FGENES.419-7 | 0.122 |
| 325470 | | | CH.12_hs gi\|6017034 | 0.123 |
| 336503 | | | CH22_FGENES.833_10 | 0.123 |
| 330786 | D60374 | Hs.258712 | EST | 0.123 |
| 329446 | | | CH.Y_hs gi\|5868886 | 0.123 |
| 303326 | AA229433 | Hs.222634 | ESTs; Moderately similar to ubiquitin-like protein / | |
| | | | ribosomal protein S30 | 0.123 |
| 309067 | AI916313 | Hs.212788 | EST | 0.123 |
| 317464 | AA968472 | Hs.130463 | ESTs | 0.123 |
| 328755 | | | CH.07_hs gi\|5868301 | 0.123 |
| 326036 | | | CH.17_hs gi\|5867178 | 0.123 |
| 327208 | | | CH.01_hs gi\|5867447 | 0.123 |
| 326124 | | | CH.17_hs gi\|5916395 | 0.123 |
| 327509 | | | CH.02_hs gi\|6117815 | 0.123 |
| 338398 | | | CH22_EM:AC005500.GENSCAN.336-5 | 0.123 |
| 304652 | AA527782 | Hs.84298 | CD74 antigen (invariant polypaptide of major | |
| | | | histocompatibility complex; class II antigen-associated) | 0.123 |
| 335797 | | | CH22_FGENES.612_6 | 0.124 |
| 336714 | | | CH22_FGENES.76-29 | 0.124 |
| 327204 | | | CH.01_hs gi\|5867447 | 0.124 |
| 331881 | AA430672 | Hs.123778 | ESTs | 0.124 |
| 306971 | AI126509 | | EST singleton (not in UniGene) with exon hit | 0.124 |
| 336174 | | | CH22_FGENES.710_1 | 0.124 |
| 336126 | | | CH22_FGENES.701_13 | 0.124 |
| 329129 | | | CH.X_hs gi\|6588026 | 0.124 |
| 303049 | AW407562 | | EST cluster (not in UniGene) with exon hit | 0.124 |
| 335778 | | | CH22_FGENES.607_14 | 0.124 |
| 336601 | | | CH22_FGENES.369_2 | 0.124 |
| 334340 | | | CH22_FGENES.375_17 | 0.124 |
| 337436 | | | CH22_FGENES.767-1 | 0.124 |
| 306013 | AA896990 | | EST singleton (not in UniGene) with exon hit | 0.124 |
| 339213 | | | CH22_FF113D11.GENSCAN.6-8 | 0.124 |
| 335355 | | | CH22_FGENES.541_2 | 0.124 |
| 336552 | | | CH22_FGENES.841_9 | 0.124 |
| 336384 | | | CH22_FGENES.822_4 | 0.124 |
| 310485 | AI286202 | Hs.149800 | ESTs | 0.125 |
| 335840 | | | CH22_FGENES.622_3 | 0.125 |
| 336444 | | | CH22_FGENES.827_10 | 0.125 |
| 315703 | N36070 | | EST cluster (not in UniGene) | 0.125 |
| 327763 | | | CH.05_hs gi\|5867961 | 0.125 |
| 336383 | | | CH22_FGENES.822_3 | 0.125 |
| 333496 | | | CH22_FGENES.168_6 | 0.125 |
| 328662 | | | CH.07_hs gi\|6004473 | 0.125 |
| 338986 | | | CH22_DA59H18.GENSCAN.5-1 | 0.125 |
| 328311 | | | CH.07_hs gi\|5868371 | 0.125 |
| 337241 | | | CH22_FGENES.644-2 | 0.125 |
| 336933 | | | CH22_FGENES.350-7 | 0.125 |
| 313483 | AW294432 | Hs.144252 | ESTs | 0.125 |
| 326116 | | | CH.17_hs gi\|5867193 | 0.125 |
| 330450 | HG363-HT363 | | Epidermal Growth Factor Receptor-Related Protein | 0.125 |
| 307491 | AI268539 | | EST singleton (not in UniGene) with exon hit | 0.125 |
| 331852 | AA418988 | Hs.98314 | Homo sapiens mRNA; cDNA DKFZp586L0120 | |
| | | | (from clone DKFZp586L0120) | 0.125 |
| 330462 | HG944-HT944 | | Dopamine Receptor D4 | 0.125 |
| 304410 | AA284508 | | EST singleton (not in UniGene) with exon hit | 0.125 |
| 336395 | | | CH22_FGENES.822_5 | 0.125 |
| 336793 | | | CH22_FGENES.176-3 | 0.125 |
| 326243 | | | CH.17_hs gi\|5867261 | 0.125 |
| 327266 | | | CH.01_hs gi\|5867462 | 0.125 |
| 320753 | AF070579 | Hs.181544 | Homo sapiens clone 24487 mRNA sequence | 0.125 |
| 336960 | | | CH22_FGENES.369-5 | 0.125 |
| 329667 | | | CH.14_p2 gi\|6272129 | 0.125 |
| 328168 | | | CH.06_hs gi\|5868071 | 0.125 |
| 336534 | | | CH22_FGENES.839_16 | 0.125 |
| 339289 | | | CH22_BA354l12.GENSCAN.16-9 | 0.126 |
| 309230 | AI970747 | | EST singleton (not in UniGene) with exon hit | 0.126 |
| 339190 | | | CH22_FF113D11.GENSCAN.1-2 | 0.126 |
| 337086 | | | CH22_FGENES.458-14 | 0.126 |
| 319233 | R21054 | Hs.211522 | ESTs | 0.126 |
| 339396 | | | CH22..BA232E17.GENSCAN.6-8 | 0.126 |
| 331930 | AA449077 | Hs.179765 | Homo sapiens mRNA; cDNA DKFZp586H1921 | |
| | | | (from clone DKFZp586H192 | 0.126 |
| 308099 | AI475914 | | EST singleton (not in UniGene) with exon hit | 0.126 |
| 338477 | | | CH22_EM:AC005500.GENSCAN.373-5 | 0.126 |
| 334286 | | | CH22_FGENES.369_16 | 0.126 |
| 317245 | AI025039 | Hs.131732 | ESTs | 0.126 |
| 335249 | | | CH22_FGENES.516_10 | 0.126 |
| 333327 | | | CH22_FGENES.138_20 | 0.126 |
| 304240 | AA009802 | | EST singleton (not in UniGene) with exon hit | 0.126 |
| 335464 | | | CH22_FGENES.562_26 | 0.126 |
| 335236 | | | CH22_FGENES.515_8 | 0.126 |
| 334154 | | | CH22_FGENES.340_4 | 0.126 |
| 309257 | AI984183 | | EST singleton (not in UniGene) with exon hit | 0.126 |
| 310015 | AI220122 | Hs.201981 | ESTs; Weakly similar to breast carcinoma-associated antigen | |
| | | | [H.sapiens] | 0.126 |
| 328280 | | | CH.07_hs gi\|5868352 | 0.126 |
| 305744 | AA831819 | | EST singleton (not in UniGene) with exon hit | 0.126 |
| 327430 | | | CH.02_hs gi\|5867754 | 0.126 |
| 328323 | | | CH.07_hs gi\|5868373 | 0.126 |
| 333274 | | | CH22_FGENES.123_2 | 0.126 |
| 337193 | | | CH22_FGENES.575-3 | 0.127 |
| 334820 | | | CH22_FGENES.437_2 | 0.127 |
| 328706 | | | CH.07_hs gi\|5868270 | 0.127 |
| 331228 | W67267 | Hs.174911 | ESTs | 0.127 |
| 307205 | AI192479 | | EST singleton (not in UniGene) with exon hit | 0.127 |
| 337123 | | | CH22_FGENES.519-3 | 0.127 |
| 326201 | | | CH.17_hs gi\|5867216 | 0.127 |
| 335276 | | | CH22_FGENES.523_2 | 0.127 |
| 331202 | T81115 | Hs.191136 | ESTs | 0.127 |
| 330532 | U03187 | Hs.121544 | interleukin 12 receptor, beta 1 | 0.127 |
| 321235 | N49521 | | EST cluster (not in UniGene) | 0.127 |
| 301743 | F12605 | Hs.204529 | ESTs; Weakly similar to reverse transcriptase [H.sapiens] | 0.127 |
| 328175 | | | CH.06_hs gi\|5868073 | 0.127 |
| 306407 | AA971985 | | EST singleton (not in UniGene) with exon hit | 0.127 |
| 327145 | | | CH.01_hs gi\|5867548 | 0.127 |
| 327649 | | | CH.04_hs gi\|5867899 | 0.127 |
| 335142 | | | CH22_FGENES.498_12 | 0.127 |
| 333909 | | | CH22_FGENES.295_2 | 0.127 |
| 330608 | X04325 | Hs.2679 | gap junction protein; beta 1; 32kD (connexin 32; | |
| | | | Charcot-Marie-Tooth neuropathy, X-linked) | 0.127 |
| 330158 | | | CH.21_p2 gi\|6580367 | 0.127 |
| 320153 | AF064594 | Hs.120360 | phospholipase A2; group VI | 0.127 |
| 314407 | AA098835 | Hs.224432 | ESTs | 0.127 |
| 333383 | | | CH22_FGENES.143_22 | 0.127 |
| 320663 | AI734242 | Hs.244473 | ESTs | 0.128 |
| 326233 | | | CH.17_hs gi\|5867232 | 0.128 |
| 326598 | | | CH.20_hs gi\|5867634 | 0.128 |
| 335174 | | | CH22_FGENES.504_4 | 0.128 |
| 319843 | H29920 | Hs.99486 | ESTs; Weakly similar to aralar1 [H.sapiens] | 0.128 |
| 335458 | | | CH22_FGENES.562_18 | 0.128 |
| 332997 | | | CH22_FGENES.58_4 | 0.128 |
| 334188 | | | CH22_FGENES.352_3 | 0.128 |
| 329759 | | | CH.14_p2 gi\|6048280 | 0.128 |
| 330348 | | | CH.D9_p2 gi\|4544475 | 0.128 |
| 326958 | | | CH.21_hs gi\|6469836 | 0.128 |
| 305263 | AA679467 | | EST singleton (not in UniGene) with exon hit | 0.128 |
| 337693 | | | CH22_EM:AC000097.GENSCAN.78-14 | 0.128 |
| 326812 | | | CH.20_hs gi\|6682504 | 0.128 |
| 333237 | | | CH22_FGENES.108_7 | 0.128 |
| 333699 | | | CH22_FGENES.250_13 | 0.128 |
| 311496 | AI768677 | Hs.209888 | ESTs; Weakly similar to phosphatidylserine | |
| | | | synthase-2 [M.musculus] | 0.128 |
| 336499 | | | CH22_FGENES.833_4 | 0.128 |
| 320087 | AF032387 | Hs.113265 | small nuclear RNA activating complex; polypeptide 4; 190kD | 0.128 |
| 309989 | AI184186 | Hs.197813 | ESTs | 0.128 |
| 301490 | AW298468 | Hs.250461 | ESTs | 0.128 |
| 337011 | | | CH22_FGENES.427-6 | 0.128 |
| 315052 | AA876910 | Hs.134427 | ESTs | 0.128 |
| 301611 | W22172 | Hs.59038 | ESTs | 0.128 |
| 336497 | | | CH22_FGENES.833_2 | 0.129 |
| 302068 | Y16280 | Hs.132049 | endothelin type b receptor-like protein 2 | 0.129 |
| 334502 | | | CH22_FGENES.397_18 | 0.129 |
| 304332 | AA158884 | | EST singleton (not in UniGene) with exon hit | 0.129 |
| 304522 | AA465405 | | EST singleton (not in UniGene) with exon hit | 0.129 |
| 312407 | R46180 | Hs.153485 | ESTs | 0.129 |
| 310098 | AI685841 | Hs.161354 | ESTs | 0.129 |
| 301119 | AF142579 | | EST cluster (not in UniGene) with exon hit | 0.129 |
| 309268 | AI985821 | Hs.62954 | ferritin; heavy polypeptide 1 | 0.129 |
| 330989 | H42142 | Hs.226396 | DEAD/H (Asp-Glu-Ala-Asp/His) box polypeptide 19 | |
| | | | (Dbp5; yeast; homolog) | 0.129 |
| 336949 | | | CH22_FGENES.361-4 | 0.129 |
| 330115 | | | CH.19_p2 gi\|6015202 | 0.129 |
| 339212 | | | CH22_FF113D11.GENSCAN.6-7 | 0.129 |
| 326951 | | | CH.21_hs gi\|6004446 | 0.129 |
| 305165 | AA662939 | | EST singleton (not in UniGene) with exon hit | 0.129 |
| 308238 | AI559492 | | EST singleton (not in UniGene) with exon hit | 0.129 |
| 337140 | | | CH22_FGENES.537-5 | 0.13 |
| 321758 | U29112 | | EST cluster (not in UniGene) | 0.13 |
| 304619 | AA515554 | Hs.119598 | ribosomal protein L3 | 0.13 |
| 312469 | AA745289 | Hs.173088 | ESTs | 0.13 |
| 339017 | | | CH22_DA59H18.GENSCAN.20-6 | 0.13 |
| 330116 | | | CH.19_p2 gi\|6015202 | 0.13 |
| 333312 | | | CH22_FGENES.138_4 | 0.13 |
| 338004 | | | CH22_EM:AC005500.GENSCAN.121-1 | 0.13 |
| 314141 | AA232134 | Hs.190028 | ESTs | 0.13 |
| 300509 | AI239845 | Hs.128494 | ESTs; Weakly similar to EG:95B7.2 [D.melanogaster] | 0.13 |
| 338530 | | | CH22_EM:AC005500.GENSCAN.398-11 | 0.13 |
| 335968 | | | CH22_FGENES.652_1 | 0.13 |
| 314121 | AI732100 | Hs.187619 | ESTs | 0.13 |
| 337593 | | | CH22_C20H12.GENSCAN.6-8 | 0.13 |
| 332881 | | | CH22_FGENES.33_ 1 | 0.13 |
| 305836 | AA858043 | | EST singleton (not in UniGene) with exon hit | 0.13 |
| 339059 | | | CH22_DA59H18.GENSCAN.30-5 | 0.13 |
| 305610 | AA782319 | | EST singleton (not in UniGene) with exon hit | 0.13 |
| 305852 | AA862455 | | EST singleton (not in UniGene) with exon hit | 0.13 |
| 327409 | | | CH.02_hs gi\|5867750 | 0.13 |
| 312751 | AI613089 | Hs.164178 | ESTs | 0.13 |
| 308726 | AI799268 | Hs.209929 | EST | 0.13 |
| 325961 | | | CH.16_hs gi\|5867147 | 0.13 |
| 311159 | AW025919 | Hs.197636 | ESTs | 0.13 |
| 322715 | AA057230 | Hs.182135 | ESTs | 0.13 |
| 336441 | | | CH22_FGENES.827_7 | 0.13 |
| 336339 | | | CH22_FGENES.814_12 | 0.13 |
| 306911 | AI095365 | | EST singleton (not in UniGene) with exon hit | 0.13 |
| 333613 | | | CH22_FGENES.217_8 | 0.13 |
| 338489 | | | CH22_EM:AC005500.GENSCAN.384-17 | 0.131 |
| 326904 | | | CH.21_hs gi\|5867684 | 0.131 |
| 337337 | | | CH22_FGENES.717-1 | 0.131 |
| 326752 | | | CH.20_hs gi\|5867615 | 0.131 |
| 303977 | AW512978 | | EST singleton (not in UniGene) with exon hit | 0.131 |
| 301373 | AA595235 | | EST cluster (not in UniGene) with exon hit | 0.131 |
| 338448 | | | CH22_EM:AC005500.GENSCAN.359-22 | 0.131 |
| 333774 | | | CH22_FGENES.272_5 | 0.131 |
| 332986 | | | CH22_FGENES.54_8 | 0.131 |
| 335362 | | | CH22_FGENES.541_12 | 0.131 |
| 335896 | | | CH22_FGENES.635_4 | 0.131 |
| 337825 | | | CH22_EM:AC005500.GENSCAN.13-19 | 0.131 |
| 325257 | | | CH.11_hs gi\|5866895 | 0.131 |
| 331188 | T50240 | Hs.167837 | ESTs | 0.131 |
| 330645 | Y08302 | Hs.144879 | dual specificity phosphatase 9 | 0.131 |
| 331760 | AA292721 | Hs.154434 | ESTs; Weakly similar to unknown [H.sapiens] | 0.131 |
| 322995 | AA513829 | Hs.29797 | ribosomal protein L10 | 0.131 |
| 335497 | | | CH22_FGENES.571_5 | 0.131 |
| 334824 | | | CH22_FGENES.437_6 | 0.131 |
| 319480 | R06933 | Hs.184221 | ESTs | 0.131 |
| 334842 | | | CH22_FGENES.439_21 | 0.131 |
| 333335 | | | CH22_FGENES.139_4 | 0.131 |
| 317252 | AA905178 | Hs.130124 | ESTs | 0.131 |
| 329034 | | | CH.X_hs gi\|5868561 | 0.131 |
| 305186 | AA664230 | | EST singleton (not in UniGene) with exon hit | 0.131 |
| 335755 | | | CH22_FGENES.604_4 | 0.131 |
| 302143 | H15270 | Hs.189847 | putative neuronal cell adhesion molecule | 0.131 |
| 334939 | | | CH22_FGENES.465_3 | 0.131 |
| 318994 | C15110 | Hs.17802 | ESTs | 0.131 |
| 334498 | | | CH22_FGENES.397_14 | 0.131 |
| 333413 | | | CH22_FGENES.146_2 | 0.132 |
| 329676 | | | CH.14_p2 gi\|6272128 | 0.132 |
| 327277 | | | CH.01_hs gi\|5867473 | 0.132 |
| 305022 | AA627416 | | EST singleton (not in UniGene) with exon hit | 0.132 |
| 336805 | | | CH22_FGENES.196-3 | 0.132 |
| 320121 | T93657 | | EST cluster (not in UniGene) | 0.132 |
| 334761 | | | CH22_FGENES.428_10 | 0.132 |
| 339400 | | | CH22_BA232E17.GENSCAN.7-6 | 0.132 |
| 330301 | | | CH.06_p2 gi\|2905862 | 0.132 |
| 316822 | AA827691 | Hs.129967 | ESTs; Weakly similar to neuronal thread protein | |
| | | | AD7c-NTP [H.sapiens] | 0.132 |
| 328020 | | | CH.06_hs gi\|5902482 | 0.132 |
| 325327 | | | CH.11_hs gi\|5866875 | 0.132 |
| 321163 | AA209530 | | EST cluster (not in UniGene) | 0.132 |
| 336393 | | | CH22_FGENES.823_5 | 0.132 |
| 325905 | | | CH.16_hs gi\|5867104 | 0.132 |
| 305237 | AA676286 | Hs.2186 | eukaryotic translation elongation factor 1 gamma | 0.132 |
| 339046 | | | CH22_DA59H18.GENSCAN.28-6 | 0.132 |
| 325375 | | | CH.12_hs gi\|5866920 | 0.132 |
| 333961 | | | CH22_FGENES.304_7 | 0.132 |
| 335450 | | | CH22_FGENES.562_8 | 0.133 |
| 302286 | R58438 | | EST cluster (not in UniGene) with exon hit | 0.133 |
| 335116 | | | CH22_FGENES.496_3 | 0.133 |
| 327333 | | | CH.01_hs gi\|5902477 | 0.133 |
| 308070 | AI470948 | | EST singleton (not in UniGene) with exon hit | 0.133 |
| 308311 | AI581855 | | EST singleton (not in UniGene) with exon hit | 0.133 |
| 320813 | AW360847 | Hs.208839 | ESTs | 0.133 |
| 323665 | AW248307 | | EST cluster (not in UniGene) | 0.133 |
| 328318 | | | CH.07_hs gi\|5868373 | 0.133 |
| 320603 | R51419 | | EST cluster (not in UniGene) | 0.133 |
| 332791 | | | CH22_FGENES.3_1 | 0.133 |
| 314976 | AA524725 | Hs.162108 | ESTs | 0.133 |
| 303309 | AL134164 | Hs.224868 | ESTs | 0.133 |
| 320581 | R39753 | Hs.170187 | ESTs | 0.133 |
| 333944 | | | CH22_FGENES.302_2 | 0.133 |
| 317992 | AI733512 | Hs.130901 | ESTs | 0.133 |
| 330935 | F02383 | Hs.26492 | beta-1;3-glucuronyltransferase 3 (glucuronosyltransferase I) | 0.133 |
| 336659 | | | CH22_FGENES.36-5 | 0.133 |
| 338887 | | | CH22_DJ32l10.GENSCAN.6-10 | 0.133 |
| 305273 | AA679979 | Hs.181165 | eukaryotic translation elongation factor 1 alpha 1 | 0.133 |
| 333566 | | | CH22_FGENES.183_2 | 0.134 |
| 316952 | AW450033 | Hs.163312 | ESTs | 0.134 |
| 333818 | | | CH22_FGENES.283_1 | 0.134 |
| 328687 | | | CH.07_hs gi\|5868262 | 0.134 |
| 302879 | H11802 | | EST cluster (not in UniGene) with exon hit | 0.134 |
| 336557 | | | CH22_FGENES.842_2 | 0.134 |
| 335222 | | | CH22_FGENES.513_5 | 0.134 |
| 338094 | | | CH22_EM:AC005500.GENSCAN.179-3 | 0.134 |
| 337384 | | | CH22_FGENES.745-1 | 0.134 |
| 327360 | | | CH.01_hs gi\|6552411 | 0.134 |
| 328132 | | | CH.06_hs gi\|5868038 | 0.134 |
| 323604 | AI751438 | Hs.182827 | ESTs; Weakly similar to !!!! ALU SUBFAMILY SQ | |
| | | | WARNING ENTRY !!!! | 0.134 |
| 337591 | | | CH22_C20H12.GENSCAN.6-6 | 0.134 |
| 307018 | AI140639 | | EST singleton (not in UniGene) with exon hit | 0.134 |
| 326896 | | | CH.21_hs gi\|5867680 | 0.134 |
| 333479 | | | CH22_FGENES.163_5 | 0.134 |
| 337915 | | | CH22_EM:AC005500.GENSCAN.61-3 | 0.134 |
| 335110 | | | CH22_FGENES.494_18 | 0.134 |
| 333481 | | | CH22_FGENES.163_9 | 0.134 |
| 327512 | | | CH.02_hs gi\|6117815 | 0.134 |
| 300096 | AW328639 | Hs.83575 | ESTs; Weakly similar to ZC328.3 [C.elegans] | 0.134 |
| 330163 | | | CH.02_p2 gi\|6042042 | 0.135 |
| 335752 | | | CH22_FGENES.604_1 | 0.135 |
| 334857 | | | CH22_FGENES.443_1 | 0.135 |
| 301872 | H84730 | | EST cluster (not in UniGene) with exon hit | 0.135 |
| 337529 | | | CH22_FGENES.823-29 | 0.135 |
| 335734 | | | CH22_FGENES.601_4 | 0.135 |
| 337551 | | | CH22_FGENES.847-8 | 0.135 |
| 309078 | AI920965 | Hs.77961 | major histocompatibility complex; class I; B | 0.135 |
| 335513 | | | CH22_FGENES.571_28 | 0.135 |
| 339078 | | | CH22_DA59H18.GENSCAN.37-6 | 0.135 |
| 321907 | N56660 | Hs.148722 | ESTs; Weakly similar to large tumor suppressor 1 [H.sapiens] | 0.135 |
| 337189 | | | CH22_FGENES.571-32 | 0.135 |
| 329635 | | | CH.12_p2 gi\|5302817 | 0.135 |
| 308601 | AI719930 | | EST singleton (not in UniGene) with exon hit | 0.135 |
| 305020 | AA627248 | Hs.2064 | vimentin | 0.135 |
| 333894 | | | CH22_FGENES.293_1 | 0.135 |
| 322465 | AA137152 | Hs.3784 | ESTs; Highly similar to phosphoserine aminotransferase | |
| | | | [H.sapiens] | 0.135 |
| 305601 | AA780975 | | EST singleton (not in UniGene) with exon hit | 0.135 |
| 332186 | H10781 | Hs.141051 | ESTs; Moderately similar to !!! ALU SUBFAMILY SB | |
| | | | WARNING ENTRY | 0.135 |
| 327822 | | | CH.05_hs gi\|5867968 | 0.135 |
| 310087 | AI393914 | Hs.160624 | ESTs; Weakly similar to similar to CR16; SH3 domain | |
| | | | binding protein | 0.135 |
| 328752 | | | CH.07_hs gi\|5868298 | 0.135 |
| 337611 | | | CH22_C20H12.GENSCAN.19-4 | 0.135 |
| 334470 | | | CH22_FGENES.394_1 | 0.136 |
| 335115 | | | CH22_FGENES.496_2 | 0.136 |
| 328730 | | | CH.07_hs gi\|5868289 | 0.136 |
| 330350 | | | CH.09_p2 gi\|3056622 | 0.136 |
| 336971 | | | CH22_FGENES.378-6 | 0.136 |
| 308258 | AI565612 | | EST singleton (not in UniGene) with exon hit | 0.136 |
| 326745 | | | CH.20_hs gi\|5867611 | 0.136 |
| 335440 | | | CH22_FGENES.560_3 | 0.136 |
| 320257 | AA330746 | | EST cluster (not in UniGene) | 0.136 |
| 328677 | | | CH.07_hs gi\|5868256 | 0.136 |
| 329731 | | | CH.14_p2 gi\|6065763 | 0.136 |
| 315950 | AA700553 | Hs.206974 | ESTs | 0.136 |
| 330049 | | | CH.17_p2 gi\|4567182 | 0.136 |
| 337070 | | | CH22_FGENES.448-3 | 0.136 |
| 304095 | H11324 | Hs.31059 | EST | 0.136 |
| 309304 | AW005527 | Hs.232820 | EST | 0.136 |
| 333458 | | | CH22_FGENES.157_7 | 0.136 |
| 329899 | | | CH.15_p2 gi\|6563505 | 0.136 |
| 322202 | AI275056 | Hs.200133 | ESTs | 0.136 |
| 333991 | | | CH22_FGENES.310_15 | 0.136 |
| 318617 | AW247252 | Hs.75514 | nucleoside phosphorylase | 0.136 |
| 310623 | AI341586 | Hs.195588 | ESTs | 0.136 |
| 330489 | M23323 | Hs.3003 | CD3E antigen; epsilon polypeptide (TiT3 complex) | 0.136 |
| 309646 | AW194694 | | EST singleton (not in UniGene) with exon hit | 0.136 |
| 331068 | R00071 | Hs.191199 | ESTs | 0.136 |
| 334285 | | | CH22_FGENES.369_15 | 0.136 |
| 332178 | F13689 | Hs.100725 | EST | 0.136 |
| 305724 | AA827608 | | EST singleton (not in UniGene) with exon hit | 0.136 |
| 303158 | AL138110 | Hs.8594 | Homo sapiens mRNA containing (CAG)4 repeat; clone CZ-CAG-7 | 0.136 |
| 334543 | | | CH22_FGENES.403_8 | 0.136 |
| 335384 | | | CH22_FGENES.543_26 | 0.136 |
| 336527 | | | CH22_FGENES.839_8 | 0.136 |
| 334951 | | | CH22_FGENES.465_20 | 0.136 |
| 325882 | | | CH.16_hs gi\|5867087 | 0.137 |
| 305134 | AA653159 | | EST singleton (not in UniGene) with exon hit | 0.137 |
| 307058 | AI148709 | | EST singleton (not in UniGene) with exon hit | 0.137 |
| 331943 | AA453418 | Hs.178272 | ESTs | 0.137 |
| 331116 | R44780 | Hs.22634 | ESTs | 0.137 |
| 306094 | AA908877 | | EST singleton (not in UniGene) with exon hit | 0.137 |
| 333561 | | | CH22_FGENES.180_18 | 0.137 |
| 321439 | H61962 | | EST cluster (not in UniGene) | 0.137 |
| 324594 | AA497090 | | EST cluster (not in UniGene) | 0.137 |
| 337926 | | | CH22_EM:AC005500.GENSCAN.77-4 | 0.137 |
| 337353 | | | CH22_FGENES.726-1 | 0.137 |
| 331836 | AA412295 | Hs.104774 | EST | 0.137 |
| 308981 | AI873242 | | EST singleton (not in UniGene) with exon hit | 0.137 |
| 329424 | | | CH.Y_hs gi\|5868879 | 0.137 |
| 325829 | | | CH.15_hs gi\|5867052 | 0.137 |
| 331845 | AA416863 | Hs.98183 | ESTs | 0.137 |
| 333854 | | | CH22_FGENES.290_13 | 0.137 |
| 306591 | AI000248 | | EST singleton (not in UniGene) with exon hit | 0.137 |
| 328948 | | | CH.08_hs gi\|6456765 | 0.137 |
| 338935 | | | CH22_DJ32\|10.GENSCAN.18-12 | 0.137 |
| 325960 | | | CH.16_hs gi\|5867147 | 0.137 |
| 328377 | | | CH.07_hs gi\|5868390 | 0.138 |
| 308851 | AI829820 | | EST singleton (not in UniGene) with exon hit | 0.138 |
| 314620 | AA424352 | Hs.210586 | ESTs | 0.138 |
| 337592 | | | CH22_C20H12.GENSCAN.6-7 | 0.138 |
| 338684 | | | CH22_EM:AC005500.GENSCAN.472-3 | 0.138 |
| 331800 | AA400498 | Hs.97543 | ESTs | 0.138 |
| 304587 | AA505535 | | EST singleton (not in UniGene) with exon hit | 0.138 |
| 333981 | | | CH22_FGENES.310_4 | 0.138 |
| 332452 | AA040369 | Hs.11170 | SYT interacting protein | 0.138 |
| 305752 | AA835278 | | EST singleton (not in UniGene) with exon hit | 0.138 |
| 311947 | T65554 | Hs.251591 | EST | 0.138 |
| 333783 | | | CH22_FGENES.273_5 | 0.138 |
| 337406 | | | CH22_FGENES.754-14 | 0.138 |
| 327976 | | | CH.06_hs gi\|5868212 | 0.138 |
| 325593 | | | CH.13_hs gi\|5866992 | 0.138 |
| 339425 | | | CH22_DJ579N16.GENSCAN.14-4 | 0.138 |
| 304475 | AA428879 | | EST singleton (not in UniGene) with exon hit | 0.138 |
| 309488 | AW131104 | | EST singleton (not in UniGene) with exon hit | 0.138 |
| 337532 | | | CH22_FGENES.827-6 | 0.138 |
| 317234 | AA904448 | Hs.126368 | ESTs | 0.138 |
| 312261 | AA854425 | Hs.144455 | ESTs | 0.138 |
| 328927 | | | CH.08_hs gi\|5868500 | 0.138 |
| 336424 | | | CH22_FGENES.824_9 | 0.138 |
| 326667 | | | CH.20_hs gi\|6552455 | 0.138 |
| 325988 | | | CH.16_hs gi\|5867064 | 0.138 |
| 318446 | AW300287 | | EST cluster (not in UniGene) | 0.139 |
| 336511 | | | CH22_FGENES.834_6 | 0.139 |
| 335204 | | | CH22_FGENES.508_13 | 0.139 |
| 303244 | AA147472 | | EST cluster (not in UniGene) with exon hit | 0.139 |
| 330870 | AA115804 | Hs.187593 | ESTs | 0.139 |
| 329376 | | | CHX_hs gi\|5868859 | 0.139 |
| 304703 | AA563898 | | EST singleton (not in UniGene) with exon hit | 0.139 |
| 333653 | | | CH22_FGENES.239_2 | 0.139 |
| 306799 | AI051696 | | EST singleton (not in UniGene) with exon hit | 0.139 |
| 304872 | AA595289 | | EST singleton (not in UniGene) with exon hit | 0.139 |
| 330812 | AA013001 | Hs.60563 | ESTs | 0.139 |
| 329568 | | | CH.10_p2 gi\|3962490 | 0.139 |
| 319210 | AA253074 | Hs.146261 | ESTs | 0.139 |
| 334320 | | | CH22_FGENES.374_5 | 0.139 |
| 300860 | A1916949 | Hs.149748 | ESTs;Weakly similar to weak similarity to collagens [C.elegans] | 0.139 |
| 305866 | AA864533 | | EST singleton (not in UniGene) with exon hit | 0.139 |
| 312943 | AA984364 | Hs.119064 | ESTs | 0.139 |
| 330523 | M99439 | Hs.83958 | transducin-like enhancer of split 4; homolog of Drosophila E(sp1) | 0.139 |
| 312708 | AI076204 | Hs.135440 | ESTs | 0.139 |
| 309366 | AW072970 | | EST singleton (not in UniGene) with exon hit | 0.139 |
| 303273 | AA316069 | | EST cluster (not in UniGene) with exon hit | 0.139 |
| 317484 | AW274696 | Hs.143921 | ESTs | 0.139 |
| 333239 | | | CH22_ FGENES.111_1 | 0.139 |
| 307126 | AI184951 | | EST singleton (not in UniGene) with exon hit | 0.139 |
| 316813 | AA826505 | Hs.124517 | ESTs | 0.139 |
| 331746 | AA281365 | Hs.121640 | ESTs; Weakly similar to KIAA0386 [H.sapiens] | 0.139 |
| 308558 | AI700145 | Hs.172182 | poly(A)-binding protein; cytoplasmic 1 | 0.139 |
| 310784 | AW086142 | Hs.159017 | ESTs | 0.139 |
| 323831 | AA335715 | Hs.200299 | ESTs | 0.139 |
| 307692 | AI318342 | | EST singleton (not in UniGene) with exon hit | 0.139 |
| 310570 | AI318327 | | EST cluster (not in UniGene) | 0.139 |
| 327934 | | | CH.06_hs gi\|5868164 | 0.139 |
| 305232 | AA670052 | Hs.195188 | glyceraldehyde-3-phosphate dehydrogenase | 0.139 |
| 334756 | | | CH22_FGENES.428_5 | 0.139 |
| 331938 | AA451867 | Hs.99255 | ESTs | 0.139 |
| 301393 | AI474722 | Hs.150898 | ESTs; Weakly similar to KIAA0644 protein [H.sapiens] | 0.139 |
| 312005 | T78450 | Hs.13941 | ESTs | 0.139 |
| 338431 | | | CH22_EM:AC005500.GENSCAN.351-4 | 0.14 |
| 331214 | T90496 | Hs.16757 | ESTs | 0.14 |
| 333601 | | | CH22_FGENES.213_4 | 0.14 |
| 323481 | AA278449 | Hs.137429 | ESTs | 0.14 |
| 336911 | | | CH22_FGENES.344-4 | 0.14 |
| 338157 | | | CH22_EM:AC005500.GENSCAN.209-5 | 0.14 |
| 327845 | | | CH.05_hs gi\|6531962 | 0.14 |
| 319109 | Z45662 | Hs.90797 | Homo sapiens clone 23620 mRNA sequence | 0.14 |
| 334763 | | | CH22_FGENES.428_12 | 0.14 |
| 329384 | | | CH.X_hs gi\|5868869 | 0.14 |
| 302996 | AF054663 | | EST cluster (not in UniGene) with exon hit | 0.14 |
| 323751 | AW452656 | Hs.209824 | ESTs | 0.14 |
| 329916 | | | CH.16_p2 gi\|6223624 | 0.14 |
| 301993 | N49826 | Hs.18602 | ESTs | 0.14 |
| 338129 | | | CH22_EM:AC005500.GENSCAN.197-2 | 0.14 |
| 325704 | | | CH.14_hs gi\|5867028 | 0.14 |
| 335656 | | | CH22_FGENES.590_7 | 0.14 |
| 331673 | W72366 | Hs.40033 | ESTs | 0.14 |
| 316807 | AI018331 | Hs.172444 | ESTs; Highly similar to transcription regulator [M.musculus] | 0.14 |
| 310743 | AW449754 | Hs.158665 | ESTs | 0.14 |
| 326941 | | | CH.21_hs gi\|6004446 | 0.14 |
| 328809 | | | CH.07_hs gi\|5868327 | 0.14 |
| 323855 | AI653164 | Hs.128665 | ESTs | 0.14 |
| 304705 | AA564064 | | EST singleton (not in UniGene) with exon hit | 0.14 |
| 325666 | | | CH.14_hs gi\|6469822 | 0.14 |
| 333747 | | | CH22_FGENES.265_6 | 0.14 |
| 318287 | AW015616 | Hs.143321 | ESTs | 0.141 |
| 332972 | | | CH22_FGENES.51_5 | 0.141 |
| 305704 | AA825266 | | EST singleton (not in UniGene) with exon hit | 0.141 |
| 315699 | AW182805 | Hs.189183 | ESTs; Weakly similar to Nod1 [H.sapiens] | 0.141 |
| 327296 | | | CH.01_hs gi\|5867492 | 0.141 |
| 336400 | | | CH22_FGENES.823_15 | 0.141 |
| 321033 | H26214 | Hs.20733 | ESTs; Weakly similar to !!!! ALU SUBFAMILY SX | |
| | | | WARNING ENTRY | 0.141 |
| 316522 | AI475995 | Hs.122910 | ESTs | 0.141 |
| 335715 | | | CH22_FGENES.599_15 | 0.141 |
| 335959 | | | CH22_FGENES.650_2 | 0.141 |
| 333259 | | | CH22_FGENES.118_7 | 0.141 |
| 337382 | | | CH22_FGENES.744-8 | 0.141 |
| 322346 | AA227618 | Hs.10882 | HMG-box containing protein 1 | 0.141 |
| 325378 | | | CH.12_hs gil5866920 | 0.141 |
| 338500 | | | CH22_EM:AC005500.GENSCAN.390-1 | 0.141 |
| 338460 | | | CH22_EM:AC005500.GENSCAN.362-5 | 0.141 |
| 315279 | AW511138 | Hs.256581 | ESTs | 0.141 |
| 314439 | AI539443 | Hs.137447 | ESTs | 0.141 |
| 333624 | | | CH22_FGENES.222_3 | 0.141 |
| 329237 | | | CH.X_hs gi\|5868729 | 0.141 |
| 330117 | | | CH.19_p2 gi\|6015201 | 0.141 |
| 338017 | | | CH22_EM:AC005500.GENSCAN.134-1 | 0.141 |
| 337854 | | | CH22_EM:AC005500.GENSCAN.38-12 | 0.142 |
| 329984 | | | CH.16_p2 gi\|4646193 | 0.142 |
| 305004 | AA622328 | Hs.162762 | EST - | 0.142 |
| 302815 | N40373 | | EST cluster (not in UniGene) with exon hit | 0.142 |
| 327823 | | | CH.05_hs gi\|5867968 | 0.142 |
| 326753 | | | CH.20_hs gi\|5867616 | 0.142 |
| 301201 | AA904482 | Hs.197775 | ESTs | 0.142 |
| 334303 | | | CH22_FGENES.373_6 | 0.142 |
| 326453 | | | CH.19_hs gi\|5867399 | 0.142 |
| 311050 | AI864581 | Hs.215477 | ESTs | 0.142 |
| 308740 | AI802711 | Hs.210337 | EST; Weakly similar to aldolase A [H.sapiens] | 0.142 |
| 331003 | H63959 | Hs.142722 | ESTs | 0.142 |
| 338010 | | | CH22_EM:AC005500.GENSCAN.128-8 | 0.142 |
| 336326 | | | CH22_FGENES.812_4 | 0.142 |
| 318100 | R44308 | Hs.242302 | ESTs | 0.142 |
| 320641 | R55421 | | EST cluster (not in UniGene) | 0.142 |
| 325855 | | | CH.16_hs gi\|5867067 | 0.142 |
| 330425 | HG1728-HT1734 | | Non-Specific Cross Reacting Antigen (Gb:D90277). | |
| | | | Alt. Splice Form 2 | 0.142 |
| 324583 | AA425411 | Hs.22581 | ESTs | 0.142 |
| 326268 | | | CH.17_hs gi\|5867267 | 0.142 |
| 331390 | AA460341 | Hs.45008 | ESTs | 0.142 |
| 338904 | | | CH22_DJ32l10.GENSCAN.10-16 | 0.143 |
| 333096 | | | CH22_FGENES.79_1 | 0.143 |
| 331919 | AA446869 | Hs.119316 | ESTs | 0.143 |
| 312214 | AI248004 | Hs.125187 | ESTs | 0.143 |
| 323198 | AW179174 | Hs.7984 | ESTs | 0.143 |
| 316107 | AI204001 | Hs.184014 | ribosomal protein L31 | 0.143 |
| 301335 | AA885317 | Hs.190511 | ESTs | 0.143 |
| 337392 | | | CH22_FGENES.747-3 | 0.143 |
| 325543 | | | CH.12_hs gi\|6682452 | 0.143 |
| 305903 | AA873085 | | EST singleton (not in UniGene) with exon hit | 0.143 |
| 332707 | L35594 | Hs.174185 | phosphodiesterase l/nucleotide pyrophosphatase 2 (autotaxin) | 0.143 |
| 337913 | | | CH22_EM:AC005500.GENSCAN.59-10 | 0.143 |
| 301436 | AA961061 | Hs.131696 | ESTs | 0.143 |
| 335078 | | | CH22_FGENES.486_5 | 0.143 |
| 338451 | | | CH22_EM:AC005500.GENSCAN.359-39 | 0.143 |
| 302777 | AJ230640 | | EST cluster (not in UniGene) with exon hit | 0.143 |
| 330464 | J03068 | Hs.78223 | N-acylaminoacyl-peptide hydrolase | 0.143 |
| 330988 | H41411 | Hs.33855 | ESTs | 0.143 |
| 328939 | | | CH.08_hs gi\|6004481 | 0.143 |
| 308015 | AI440174 | Hs.228907 | EST; Weakly similar to GUANINE NUCLEOTIDE-BINDING | |
| | | | PROTEIN BETA SUBUNIT-LIKE PROTEIN | |
| | | | 12.3 [H.sapiens] | 0.143 |
| 328504 | | | CH.07_hs gi\|5868471 | 0.143 |
| 332599 | AA402891 | Hs.32951 | solute carrier family 29 (nucleoside transporters); member 2 | 0.143 |
| 335744 | | | CH22_FGENES.601_15 | 0.143 |
| 322394 | AF077208 | | EST cluster (not in UniGene) | 0.143 |
| 323892 | AL042661 | | EST cluster (not in UniGene) | 0.143 |
| 318443 | AI939323 | Hs.157714 | ESTs;Weakly similar to NEURONAL ACETYLCHOLINE | |
| | | | RECEPTOR PROTEIN; ALPHA-5 CHAIN PRECURSOR | |
| | | | [H.sapiens] | 0.143 |
| 336568 | | | CH22_FGENES.843_7 | 0.143 |
| 330958 | H08815 | Hs.159824 | EST | 0.143 |
| 327672 | | | CH.04_hs gi\|5867843 | 0.143 |
| 335900 | | | CH22_FGENES.635_ 8 | 0.144 |
| 336044 | | | CH22_FGENES.679_6 | 0.144 |
| 318845 | AI815951 | Hs.33183 | ESTs; Weakly similar to estrogen-responsive finger protein; | |
| | | efp [H.sapiens] | | 0.144 |
| 333483 | | | CH22_GENES.165_2 | 0.144 |
| 333337 | | | CH22_FGENES.139_6 | 0.144 |
| 305993 | AA889197 | | EST singleton (not in UniGene) with exon hit | 0.144 |
| 335719 | | | CH22_FGENES.599_22 | 0.144 |
| 325682 | | | CH.14_hs gi\|6138923 | 0.144 |
| 327350 | | | CH.01_hs gi\|6249563 | 0.144 |
| 339291 | | | CH22_BA354l12.GENSCAN.18-1 | 0.144 |
| 326358 | | | CH.18_hs gi\|5867293 | 0.144 |
| 330316 | | | CH.08_p2 gi\|6007576 | 0.144 |
| 308150 | AI499346 | Hs.174131 | ribosomal protein L6 | 0.144 |
| 338065 | | | CH22_EM:AC005500.GENSCAN.164-1 | 0.144 |
| 339009 | | | CH22_DA59H18.GENSCAN.18-7 | 0.144 |
| 327776 | | | CH.05_hs gi\|5867964 - | 0.145 |
| 336664 | | | CH22_FGENES.41-8 | 0.145 |
| 321921 | AF070619 | | EST cluster (not in UniGene) | 0.145 |
| 319346 | T70147 | Hs.12024 | ESTs | 0.145 |
| 304265 | AA062892 | | EST singleton (not in UniGene) with exon hit | 0.145 |
| 303818 | Z45986 | Hs.250178 | copine II | 0.145 |
| 327498 | | | CH.02_hs gi\|6017023 | 0.145 |
| 335227 | | | CH22_FGENES.513_13 | 0.145 |
| 339022 | | | CH22_DA59H18.GENSCAN.22-1 | 0.145 |
| 302597 | H55661 | Hs.33026 | ESTs; Weakly similar to similar to Enterococcus faecalis | |
| | | TRAB [C.elegans] | | 0.145 |
| 308550 | AI697008 | Hs.201811 | EST | 0.145 |
| 302175 | AA262760 | Hs.156015 | Homo sapiens chromosome 19; cosmid R29381 | 0.145 |
| 303252 | AA156760 | | EST cluster (not in UniGene) with exon hit | 0.145 |
| 337414 | | | CH22_FGENES.757-2 | 0.145 |
| 310382 | AI734009 | | EST cluster (not in UniGene) | 0.145 |
| 329333 | | | CH.X_hs gi\|5868806 | 0.145 |
| 336857 | | | CH22_FGENES.291-7 | 0.145 |
| 332565 | AA234896 | Hs.25272 | E1A binding protein p300 | 0.145 |
| 318634 | AI928098 | Hs.156832 | ESTs | 0.145 |
| 336318 | | | CH22_FGENES.801_1 | 0.145 |
| 310960 | AI923551 | Hs.170843 | ESTs | 0.145 |
| 335346 | | | CH22_FGENES.537_2 | 0.145 |
| 331196 | T65416 | Hs.12826 | ESTs | 0.145 |
| 337607 | | | CH22_C20H12.GENSCAN.17-3 | 0.146 |
| 331206 | T84096 | Hs.15284 | ESTs | 0.146 |
| 301793 | T80698 | | EST cluster (not in UniGene) with exon hit | 0.146 |
| 319590 | AA210878 | | EST cluster (not in UniGene) | 0.146 |
| 311394 | AI695374 | Hs.256231 | ESTs | 0.146 |
| 324773 | AA632554 | Hs.163401 | ESTs | 0.146 |
| 324841 | AI142359 | Hs.155316 | ESTs | 0.146 |
| 332260 | N70088 | Hs.138467 | ESTs | 0.146 |
| 329276 | | | CH.X_hs gi\|5868762 | 0.146 |
| 335887 | | | CH22_FGENES.633_1 | 0.146 |
| 338294 | | | CH22_EM:AC005500.GENSCAN.297-1 | 0.146 |
| 336993 | | | CH22_FGENES.409-4 | 0.146 |
| 334135 | | | CH22_FGENES.336_2 | 0.146 |
| 326251 | | | CH.17_hs gi\|5867263 | 0.146 |
| 337396 | | | CH22_FGENES.749-1 | 0.146 |
| 339167 | | | CH22_DA59H18.GENSCAN.69-8 | 0.146 |
| 316838 | AW135418 | Hs.161210 | ESTs | 0.146 |
| 325313 | | | CH.11_hs gi\|5866865 | 0.146 |
| 331047 | N66918 | Hs.32205 | ESTs | 0.146 |
| 323915 | AL043362 | | EST cluster (not in UniGene) | 0.146 |
| 302747 | AF062275 | | EST cluster (not in UniGene) with exon hit | 0.146 |
| 306317 | AA947909 | | EST singleton (not in UniGene) with exon hit | 0.146 |
| 334399 | | | CH22_FGENES.382_5 | 0.146 |
| 326472 | | | CH.19_hs gi\|5867404 | 0.146 |
| 333061 | | | CH22_FGENES.75_4 | 0.146 |
| 337072 | | | CH22_FGENES.448-5 | 0.146 |
| 334328 | | | CH22_FGENES.375_5 | 0.146 |
| 327039 | | | CH.21_hs gi\|6531965 | 0.146 |
| 325576 | | | CH.12_hs gi\|6552443 | 0.147 |
| 315935 | AI075804 | Hs.132660 | ESTs | 0.147 |
| 319638 | AA323758 | | EST cluster (not in UniGene) | 0.147 |
| 334501 | | | CH22_FGENES.397_17 | 0.147 |
| 338238 | | | CH22_EM:AC005500.GENSCAN.264-4 | 0.147 |
| 308636 | AI744063 | | EST singleton (not in UniGene) with exon hit | 0.147 |
| 336567 | | | CH22_FGENES.843_6 | 0.147 |
| 335819 | | | CH22_FGENES.619_2 | 0.147 |
| 336950 | | | CH22_FGENES.361-8 | 0.147 |
| 307055 | AI148477 | | EST singleton (not in UniGene) with exon hit | 0.147 |
| 315134 | AW504854 | Hs.126714 | ESTs | 0.147 |
| 335834 | | | CH22_FGENES.621_1 | 0.147 |
| 327870 | | | CH.06_hs gi\|5868131 | 0.147 |
| 323802 | AA332011 | Hs.250138 | protein phosphatase 2C; magnesium-dependent; catalytic subunit | 0.147 |
| 329412 | | | CH.X_hs gi\|6682553 | 0.147 |
| 323791 | AA333068 | | EST cluster (not in UniGene) | 0.147 |
| 324126 | AA385315 | | EST cluster (not in UniGene) | 0.147 |
| 327865 | | | CH.06_hs gi\|5868130 | 0.147 |
| 333445 | | | CH22_FGENES.154_2 | 0.147 |
| 321302 | AA021351 | Hs.158497 | KIAA0724 gene product | 0.147 |
| 336744 | | | CH22_FGENES.118-9 | 0.147 |
| 323731 | AA323414 | | EST cluster (not in UniGene) | 0.148 |
| 320289 | H07989 | | EST cluster (not in UniGene) | 0.148 |
| 305488 | AA749000 | | EST singleton (not in UniGene) with exon hit | 0.148 |
| 305592 | AA780594 | Hs.62954 | ferritin; heavy polypeptide 1 | 0.148 |
| 304094 | H11295 | | EST singleton (not in UniGene) with exon hit | 0.148 |
| 325040 | AW296368 | | EST cluster (not in UniGene) | 0.148 |
| 339034 | | | CH22_DA59H18.GENSCAN.26-2 | 0.148 |
| 334504 | | | CH22_FGENES.398_2 | 0.148 |
| 334778 | | | CH22_FGENES.431_2 | 0.148 |
| 320148 | U77494 | Hs.119687 | RAN binding protein 8 | 0.148 |
| 303584 | AW173759 | Hs.203401 | ESTs | 0.148 |
| 325826 | | | CH.15_hs gi\|5867048 | 0.148 |
| 331192 | T55182 | Hs.152571 | ESTs; Highly similar to IGF-II mRNA-binding protein 2 [H.sapiens] | 0.148 |
| 325785 | | | CH.14_hs gi\|6381957 | 0.148 |
| 333166 | | | CH22_FGENES.91_8 | 0.148 |
| 336548 | | | CH22_FGENES.841_5 | 0.148 |
| 337552 | | | CH22_C4G1.GENSCAN.1-4 | 0.148 |
| 331775 | AA382742 | Hs.97151 | EST | 0.148 |
| 338936 | | | CH22_DJ32l10.GENSCAN.19-6 | 0.148 |
| 331869 | AA428554 | Hs.104894 | ESTs; Weakly similar to fibronectin precursor [H.sapiens] | 0.148 |
| 332865 | | | CH22_FGENES.28_5 | 0.148 |
| 328663 | | | CH.07_hs gi\|6004473 | 0.148 |
| 328436 | | | CH.07_hs gi\|5868417 | 0.148 |
| 311158 | AI634864 | Hs.250789 | ESTs; Highly similar to similar to NEDD-4 [H.sapiens] | 0.148 |
| 336942 | | | CH22_FGENES.354-2 | 0.148 |
| 302262 | R53169 | Hs.246091 | ESTs | 0.149 |
| 333296 | | | CH22_FGENES.132_3 | 0.149 |
| 333365 | | | CH22_FGENES.142_2 | 0.149 |
| 311706 | AW452392 | Hs.252854 | ESTs | 0.149 |
| 337109 | | | CH22_FGENES.489-2 | 0.149 |
| 315062 | AW173300 | Hs.190201 | ESTs | 0.149 |
| 333454 | | | CH22_FGENES.157_3 | 0.149 |
| 334784 | | | CH22_FGENES.432_9 | 0.149 |
| 333255 | | | CH22_FGENES.118_3 | 0.149 |
| 337518 | | | CH22_FGENES.814-7 | 0.149 |
| 320651 | AA489268 | | EST cluster (not in UniGene) | 0.149 |
| 323437 | AA287567 | | EST cluster (not in UniGene) | 0.149 |
| 328761 | | | CH.07_hs gi\|5868302 | 0.149 |
| 328787 | | | CH.07_hs gi\|5868309 | 0.149 |
| 335261 | | | CH22_FGENES.520_2 | 0.149 |
| 300827 | R16689 | Hs.106004 | ESTs | 0.149 |
| 339263 | | | CH22_BA354l12.GENSCAN.10-1 | 0.149 |
| 337412 | | | CH22_FGENES.756-6 | 0.149 |
| 334414 | | | CH22_FGENES.384_1 | 0.149 |
| 332931 | | | CH22_FGENES.38_5 | 0.149 |
| 310801 | AW270980 | Hs.1063 | 46 novel centrosomal protein RanBPM | 0.149 |
| 305216 | AA669056 | | EST singleton (not in UniGene) with exon hit | 0.149 |
| 314779 | AA470122 | Hs.190261 | ESTs | 0.149 |
| 338414 | | | CH22_EM:AC005500.GENSCAN.341-27 | 0.149 |
| 303342 | AW247361 | | EST cluster (not in UniGene) with exon hit | 0.149 |
| 337509 | | | CH22_FGENES.806-4 | 0.149 |
| 306631 | AI001149 | | EST singleton (not in UniGene) with exon hit | 0.149 |
| 302533 | L36149 | Hs.248116 | chemokine (C motif) XC receptor 1 | 0.149 |
| 336536 | | | CH22_FGENES.839_18 | 0.149 |
| 324666 | T32458 | Hs.14285 | ESTs | 0.149 |
| 310173 | AI767433 | Hs.170013 | ESTs | 0.149 |
| 333595 | | | CH22_FGENES.211_2 | 0.149 |
| 335975 | | | CH22_FGENES.652_9 | 0.15 |
| 306654 | AI003654 | | EST singleton (not in UniGene) with exon hit | 0.15 |
| 335025 | | | CH22_FGENES.475_3 | 0.15 |
| 328711 | | | CH.07_hs gi\|5868271 | 0.15 |
| 328274 | | | CH.07_hs gi\|5868219 | 0.15 |
| 325505 | | | CH.12_hs gi\|6682451 | 0.15 |
| 329641 | | | CH.14_p2 gi\|6468233 | 0.15 |
| 304955 | AA613504 | | EST singleton (not in UniGene) with exon hit | 0.15 |
| 339103 | | | CH22_DA59H18.GENSCAN.44-10 | 0.15 |
| 329636 | | | CH.12_p2 gi\|5302817 | 0.15 |
| 310118 | AI203293 | Hs.157489 | ESTs | 0.15 |
| 326056 | | | CH.17_hs gi\|5867184 | 0.15 |
| 303773 | AA769074 | | EST cluster (not in UniGene) with exon hit | 0.15 |
| 303153 | U09759 | Hs.8325 | mitogen-activated protein kinase 9 | 0.15 |

**TABLE 13A shows the accession numbers for those primekeys lacking unigeneID's for Table 13. For each probeset we have listed the gene cluster number from which the oligonucleotides were designed. Gene clusters were compiled using sequences derived from Genbank ESTs and mRNAs. These sequences were clustered based on sequence similarity using Clustering and Alignment Tools (DoubleTwist, Oakland California). The Genbank accession numbers for sequences comprising each cluster are listed in the "Accession" column.**

| | | |
|---|---|---|
| Pkey: | | Unique Eos probeset identifier number |
| CAT number: | | Gene cluster number |
| Accession: | | Genbank accession numbers |
| | | |

| **Pkey** | **CAT number** | **Accession** |
|---|---|---|
| | | |
| 322050 | 24275_1 | AL137589 AA423949 BE222949 BE222694 AI199615 AW873116 AI277950 AW044290 AW630096 |
| 321439 | 1599424_1 | H61962 W01567 N75711 |
| 321666 | 13653_22 | BE259906 AA232518 AA013359 AL035788 AW160822 BE387134 BE002954 BE391839 AW161565 AI878841 BE616458 BE409981 BE387308 BE297436 BE315536 AA206924 R12012 AA214169 BE312812 BE387093 H11710 BE312009 BE260569 AA343566 AA219526 R34757 AA219749 BE336733 AA219751 AW411099 AA232408 BE018716 BE398089 AA206253 AA053487 AA114224 AV655868 AW732566 BE394087 AW732574 AA313442 BE336875 AA070548 BE259840 BE019828 AW732341 AA299916 BE019253 BE018238 BE387109 AA232304 BE255589 AW732585 AA181436 AA308777 AA075802 AW732521 AA314526 AA226747 BE409513 AA206168 BE388292 BE298782 BE387086 AA305310 AV652723 AA314918 BE615510 AW951763 BE398104 BE385195 |
| | | BE407165 BE391336 BE390187 BE389189 BE540650 BE249884 BE385985 BE274245 BE391124 BE260080 AA182600 BE512821 BE390090 BE279398 BE279589 BE263454 BE515194 BE293569 BE272531 BE388814 BE384659 BE271685 BE561043 BE278449 BE302572 AW239076 AI750583 AA376179 AA112632 BE266324 BE266614 R13105 AA132286 BE296305 AI220355 AA205606 AA219527 AA219519 AW804310 AA083286 BE171208 T19693 AA338328 BE185868 AA903024 T92162 AA330119 BE410404 BE314668 |
| 300088 | 622937_1 | AW576245 BE207878 AW299993 AI199558 AI285442 AW299994 AW394242 AW394184 |
| 322303 | 704603_1 | AI357412 AI870708 AI590539 W07459 |
| 322394 | 27492_1 | AW068287 AA310079 BE336702 AA356318 AA306059 AA346785 AW402633 AA311210 AW402909 N76879 AW402913 AW401920 AA321636 AA354474 C17297 C16938 AA311774 M29871 NM_002872 Z82188 AW405674 H94176 R89281 AA214723 AI014482 AW949347 T27749 AW804226 AW796964 AW404581 AF077208 NM_014029 W68830 W79652 AA353375 AW575218 AA552192 AA521232 AA702695 AA033975 AW407827 AA829948 N94402 AW628604 AI523308 N57605 AA641662 H42477 N52784 AI753478 AA768493 AA845729 W47391 N55270 AI090117 R89282 BE206172 AA076650 AA595650 AI218931 BE049397 AI433110 |
| | | W74114 H94277 AI358627 AI085221 AI862818 AA835967 AW103905 AI640644 AA835507 AA856887 AA694392 AW337542 AI524410 BE045500 AI440060 AI358801 AW028238 AW205248 AI718264 R48618 AA357358 AI695002 AA897549 AW081065 AI433360 AI810783 AI620963 Z82188 AA360224 |
| 321758 | 44275_1 | U29112 AI656540 AI364875 AI656246 AI990940 |
| 323109 | 155498_1 | AA169345 AI762857 AI949997 AI809601 AI681948 AI221079 AW167404 AI347614 AI611090 AI023472 AI347683 AI027467 AW591788 AI380665 AA835735 AA836654 AI244028 AW193159 AI500112 AI918722 AI738693 AI702308 AA805365 AI766842 |
| 322533 | 38937_1 | T59538 T59589 T59598 T59542 AF147374 |
| 321921 | 34680_1 | AF070619 R20302 T80358 |
| 321927 | 21620_1 | AJ223366 BE305086 AW820106 AA621983 BE305208 AI738475 AI380189 AW590847 AI127232 AA622706 AI380858 AA621975 AI587036 AA665743 AW204003 AI692234 AI002242 AI692219 AW137282 AW268783 AW295910 AI308015 AW301462 AI318288 AI318575 AI318117 AI345591 AI249650 AI246934 AI246864 AI246971 AW268311 AI249654 BE041907 AW732776 |
| 321932 | 265316_1 | N72324 N52825 W19526 BE143464 AA376060 |
| 306971 | 14694_7 | M83667 NM_005195 S63168 M83667 AW068039 AW630649 AI338577 AI018125 AI269878 AW242440 AI887823 AI342581 BE222416 AI582847 AI651011 AI660815 AI699574 BE550201 AI926996 AW665855 AI827752 AI761857 BE328168 BE222451 AI762201 AW000929 AW007207 BE042962 BE551843 BE465373 AI279179 AI949945 BE551862 AW051667 BE328076 BE222296 |
| | | AW007229 AW772332 AI279801 AI934526 AI631938 AI770103 BE041412 AI417900 AI692655 AI869943 AW270119 AI431739 AI703347 AW770568 AW025473 AI701497 AI128026 BE328147 AW203980 BE046793 AW087704 AI674597 AI650732 AI813691 AI472092 AI695224 AI241217 AW207746 AI206840 AI271362 AI631788 AI911883 AI914619 AI380585 AI767501 AI823759 AI564116 AI190991 AI377369 AI814122 AI221623 AI354793 AI081988 AI391740 AI337435 BE467366 AI824347 AI565326 AI280038 AI640455 AI819744 |
| | | BE467803 BE327524 AI149402 AI313187 BE219684 AW611948 AW665821 AI091260 AW044492 BE220366 AW025381 AW183264 AI694865 AI498474 AI129780 AI202028 AI566792 BE220659 AI928040 AI830696 AI493021 AW612488 AI913152 BE042965 AI631837 AI693873 AI498925 AI768668 AI401544 BE327023 |
| | | AI693383 AI769874 AI744003 AW082273 AI686501 AI798177 AI985196 AI090033 AI432342 AI689918 AI638308 BE468080 BE219588 AI912119 BE219787 AW005392 BE326564 AI589039 AI860187 AI758143 AI338168 AI702936 BE221985 AI498727 AI918196 AI279735 AW771497 AI860133 AW237834 AW661759 AW028111 BE503416 AI360180 AW611715 AI871777 BE045447 BE326444 AI266547 AI800237 AI823315 AI478368 AI264281 AI675841 AI690041 |
| | | AI498018 AI554124 AI239893 AI864054 AI280099 AI192815 AI620465 AI080201 AW002057 BE500986 AI341131 AI818991 AI566137 AI123403 BE219192 AW183844 AI499842 AW137971 AW138720 AW015526 AW138160 AW243163 AW138705 AW139927 AW140006 AW138810 AW137450 AW206970 AW135419 AW205974 AA043494 BE465106 AW139955 AI741112 BE326942 AA043506 AI079957 AI942432 AI392902 AI097047 AI470599 AA514553 |
| | | AA984008 N47949 AI664114 AA884832 AI796752 AI765290 AI301155 AW470358 BE222764 AI823569 AI651188 AI692695 AI476643 BE504307 AI767573 BE219719 AI932249 AW467075 AI913633 BE221966 AI091025 AA969215 AI799810 AA931170 BE048559 AI809606 AI138614 AI739456 AI674605 AW772068 AI089286 AI625787 AI263418 AW008638 AI928389 AW628997 AI470010 AI914168 AI760003 AI203050 AI334069 AI694788 BE045337 AI948659 AI912982 AI867131 AI192102 |
| | | AI767583 AI347518 AI566005 AI625884 AI215888 AI633904 AW182265 AW614357 AI128030 AI343685 AI914283 AI985003 AI823578 AI493053 AI380285 AI633895 AI267880 AI538162 AI991552 BE219479 BE219296 AI302178 AW779296 AI913805 AI631644 AI566772 AI985498 AI942289 AI935659 AI339092 AI247432 AI686472 AI766886 AI017228 AI333272 AW301668 AI972218 AW082027 AI632974 |
| | | AI474761 AI766127 AW236578 AW000966 AI870734 AI222399 AI871249 AI703448 BE464210 AI768037 AI871585 AI767871 AI738757 AI220732 AI681633 AI768783 AI684463 AI307339 AI263203 AW665264 BE463969 AI768786 AI439118 AI127913 BE218324 AI672342 BE220052 AI796163 AI221662 AW197672 AW025300 AI769681 AW612448 BE219757 AW072420 AI669980 AI830418 AW204353 AA047011 AA913868 AI739146 AI669954 AW470507 AW614835 AW302151 AW772372 AI762427 AW339902 AW303370 BE464775 |
| | | AW299818 AW236072 AW195060 AW274737 AW263062 AW183846 AI868894 AW300493 AW172509 AW516876 AW593773 AW299474 AW303546 AI817323 Al823624 AI694005 AI934589 AI343479 AI861825 AI962726 AI765845 AW080318 AI640227 AI763042 AI768903 AW235386 AA738489 AW341293 AA588585 BE221732 AI914179 AW611669 AI572789 AW194735 AW236122 AW236007 AW612789 AW197501 AW195046 AI797145 AI864423 AI458934 |
| | | AI342848 AI693227 AI912642 AI689993 AA932572 AA740269 AW470392 AW086020 AI221701 T69326 T70461 AI765579 AI338263 AI431721 AI394249 AI186462 AI823571 AI953665 AI497954 AI761057 AI678228 AI640302 AI948742 AA594626 AA883155 AI972682 AI804774 AI300407 AI433524 AA897341 AI401175 AI291071 AA021213 AI126509 AI948955 AI218835 AA903938 AA502610 AI498320 AA584267 AA935285 AI476253 AA489658 AA975053 AA715326 AA557139 AA126417 |
| | | AA971455 AA557319 AI499738 AA911438 AI913637 AA494506 N90793 AI990724 AA131667 AA128164 AA046840 AI262557 AA131729 AA594926 T59467 AA436907 AA044630 AI589177 AI279237 AI880498 AI431822 AA708934 AW612558 AI634069 W03610 AI192272 BE550862 AI400879 AA708507 AI128003 |
| | | AI375308 AI271423 AI199552 AA125977 AI366498 AA458662 AI694382 AA044627 AI636263 AI796270 T90146 AW014724 AI870812 AI948781 AA369965 AI094721 AW271817 AI262898 AI244680 T69252 AI934148 AA046357 W19109 AA028157 AW021924 AA253491 AI189397 AI934388 D58282 W21323 W24288 AI682972 AA293683 AA284566 AV659511 AA434184 H87089 AA040038 N57464 AA343709 AW805815 R89837 |
| 301119 | 33384_1 | BE621320 BE266806 BE276582 AW516729 AF142579 AW451687 AK000069 AA325236 BE168997 W73105 AA715365 BE278873 AA808894 AA386371 AW517942 AW750993 BE140314 BE392384 BE621757 AA318192 BE548173 AW152607 AW166898 AA352215 AW841506 T59802 AF147378 AA335719 AW956069 T59668 AA826362 AI961329 AI290469 AW197375 AI805651 AA160748 AA581089 AI968889 AA581100 AA501478 AI621069 AA468534 AA503715 AA658457 AI144504 BE387827 AA159880 |
| 324019 | 262792_1 | AW177009 AI381610 |
| 323437 | 189513_1 | AA287567 AA252404 AW967735 AA287568 AA761222 AA865644 AA831245 |
| 307845 | 19804_10 | BE514807 R43224 AI363450 AA450226 AF030942 |
| 324126 | 272259_1 | AA385315 AI627453 AI050695 AI348281 |
| 309101 | 7570_1 | AI340462 AI583268 AA079086 AI950777 AI301866 AI925108 AW876954 AW877000 AA525418 AA888549 AI934220 AW380220 AA804858 AI927576 T61151 AW384053 BE391691 AA533856 AA248400 T48202 N57156 R68346 R26020 AL050332 W30806 H61369 AA092592 AA230324 BE271217 AW372903 T48772 AA358002 AA094302 AA559856 AW373308 AW373315 AW373297 AW373311 AW373314 AW373309 AW877055 AW770140 AW379805 AI581609 AW364144 AA078921 AA715432 AA654210 AI004899 AA602209 W47464 AA506588 R26822 AU076528 AI535743 AI535704 AI535681 |
| 315703 | 119175_1 | AA402307 D60405 D61237 D59891 AW964877 AA325215 AI459739 N36070 N25658 AA083684 AW293368 AI761958 AI741205 AI693175 AW873603 AI143269 AI187124 N25199 H19323 AI650842 AW316825 AA083842 AA935650 AW298404 AI472001 AI648568 R17676 R41625 AI123237 R17677 AI206866 F36920 AI654713 F34084 AA618029 AI915139 AW275194 AW514577 D80420 AW149850 Z40953 AI867861 AA927547 AA974344 AI825793 AI635565 AI652157 BE504748 AW295759 F16800 AW839796 F01781 AA909730 AA984010 |
| 301373 | 368214_1 | AA595235 AW973839 T03040 |
| 323665 | 54093_1 | AW248307 AA313452 AW951927 AA355961 BE566080 |
| 323676 | 220254_1 | AI702835 AI758919 AI685405 AI952108 AI299207 AI400767 AW105389 AI952710 AA845312 AI784118 AI537315 |
| 302086 | 23306_1 | X57138 NM_003514 Z98744 BE253911 BE256314 AI095013 AI138475 |
| 323731 | 226193_1 | AA323414 AW664013 AI809377 AI276041 AW296883 AI798340 |
| 323791 | 232336_1 | AA333068 AA331863 AA331838 AW962531 AA331442 |
| 325040 | 23854_1 | AW296368 AA247632 AK002030 R15304 T08775 AW975664 AI186801 AA730688 AW190918 AI141176 AW513211 AI276071 AA988601 BE042933 BE045713 AW087176 |
| 324430 | 312113_1 | AA464018 AA464079 AA468142 |
| 323892 | 477253_1 | AA846318 W15478 AL042661 |
| 309488 | 1030131_1 | AW131104 BE246610 |
| 302251 | 27216_4 | AA333340 AW955834 R49755 U33428 |
| 302286 | 22717_6 | R58438 AA358612 |
| 323915 | 110063_1 | AL043362 AA350031 AW751972 BE549118 |
| 324594 | 330528_1 | AA497090 AI351879 AI350914 |
| 301737 | 65_1 | AI815981 AF287269 BE260960 BE263991 AA311733 F12145 F07345 Z43604 T29948 H64102 Z43611 T35364 N40667 AI909783 AW751045 AA160594 AI816064 AI307240 AI951554 AA641031 AA293045 AI942492 AI687077 R78689 H12368 AA894728 AI124930 AI423498 AA777759 AA614585 AW071822 T66288 AI418558 H21480 AI335011 AI051728 AA293436 AW302233 AW188628 N26393 AI076557 AI311022 AW451505 H62593 Z39666 H12315 AI761351 AI364142 F02935 AW571491 T35366 AI240745 H64151 AA503793 AA831948 AI627686 AI761531 F03591 F09782 |
| 301763 | 1688575_1 | R01279 R05896 T86522 |
| 301780 | 18597_2 | R05735 BE349600 R37388 N79751 R10115 AA702039 AA836147 AA505716 AI049661 AI499239 R54072 AI023394 AA827710 W60285 W500038 AI884786 AA827191 AA810075 AW005088 R70248 AI858560 AW078678 AA631306 H52839 AW085835 AI656182 AA737178 AW136923 AA281028 AA570316 AA722871 AA362737 AI217268 BE242373 R01113 AA628946 AI394527 AW402308 AI361110 AI917585 T99639 AA805326 N44577 AI394021 AW403385 T23949 AI497766 T96602 AA834947 AI693908 Z33450 T92127 BE541896 AI933301 BE251540 BE252269 N50968 AI695531 AW575523 AW296889 N93796 N89924 AB61804 AI085251 AA810694 BE303011 AA743784 R13478 AA358771 AA325294 AW964880 BE258953 R54116 AW881039 AW602593 |
| 301793 | 239325_1 | BE265837 AA340632 T96304 T96075 T72780 H51978 R09868 |
| 303049 | 102592_1 | AW408042 AW407562 BE172835 BE396893 BE269184 AA045741 BE004187 AW751261 W74283 |
| 301863 | 19477_1 | BE263301 AI418863 NM_005194 X52560 AW328683 BE298869 D63161 |
| 301872 | 27494_4 | H84730 T73262 |
| 301893 | 6561_1 | T80334 BE292758 AK000854 H16996 BE253691 R88508 AA357663 AW955288 AW579550 N98864 AA595201 AI742967 AA602658 AI091433 AA813367 AI983217 AW298007 AI628490 AI708037 AI560654 AI032983 R88509 R38972 AI687783 AI560153 AW874581 N69891 AA993617 H51180 AI269042 AI281358 AW591213 AI017724 AI262859 H16997 R38991 AI804355 AI868988 AI669525 AW023081 AL047848 |
| 310382 | 653318_1 | AI734009 AI263076 AW272255 AI792912 |
| 303181 | 74060_2 | AA452366 AA351338 BE262590 BE262591 AA074050 AA389667 BE161346 |
| 302569 | 17513_2 | AC004472 BE312721 BE273942 F11928 T65358 BE612432 BE261576 BE179884 |
| 324893 | 4670_1 | AA324119 AW246199 BE395368 BE261676 BE382334 BE394701 BE304548 T31940 BE398128 BE398019 BE296693 BE379564 BE269460 BE397065 Z42029 BE305028 AK000549 BE536182 BE314372 AW393349 T50987 AA069735 BE386997 AW381699 T51050 W95025 AA477678 AA348306 AW956831 AW062919 AL040397 BE305160 AA315419 AW249929 AA295944 AI635946 AI870259 AI951125 AW028250 AI885184 AW873113 AI077544 AW025091 AI817594 AI401718 AW008245 AI499064 AA599687 AI016890 AA765638 W93340 AA588708 AW519173 R51917 AA676778 AI084871 AA687684 AI860840 AI811921 AW514730 AA477561 N78845 AA779894 AA778559 AI968953 T16188 T32828 AA991426 AI474472 AI473542 AI828972 AW247906 AA977415 AW591489 AA876008 AW191893 AA074278 AW874099 Z40196 AW083615 F01544 T55984 AI290413 AI972167 AI365049 T36028 AI042568 BE560076 W17119 AA196376 T47999 R54309 |
| 303244 | 9334_1 | AK001269 AL354613 AA147472 AA490803 BE207628 AW816113 AA085574 AW503392 AA299910 AW750305 BE079539 BE079484 BE512838 AK001593 AW968772 AW967440 AW206280 AA251270 AI627886 AA303599 AA147473 BE206616 AA490611 AA715039 AW590866 AW590447 AI864512 AA204731 AA894490 BE001136 AA612785 AA237035 AA149960 Z44257 R12986 AA448446 |
| | | AI734041 AA422167 BE220551 R66041 R32927 R32942 AA258773 AW386142 R53730 N54624 AW880296 AA253485 AW954441 H98989 AW614348 AI654838 AA779793 AW237213 N66635 AI186812 AA947479 BE158011 AI859480 AW805579 N52010 AA806305 AI628445 AW270990 AA778165 AA149949 AI650728 AA749108 AA687257 AI261661 AA747442 AA481351 AA206339 AA903407 |
| | | AW473306 AI688930 AA262281 AA448310 AA748820 AI347430 BE465692 R32839 AW510564 AA436408 AA257971 AA253362 AA938330 AA513150 AA976840 AA687117 AI281547 AA046243 R32825 AI631554 AW139818 AI244536 R52946 AW235443 R40183 AA299909 AA811958 AI302918 Z40213 BE158047 BE158060 AA767245 AW748159 AW500735 AA094074 |
| 303252 | 149690_3 | AW393348 AW393350 AW386713 AW384705 |
| 303273 | 67758_1 | AA316069 BE274224 AL120803 BE170052 BE170039 AI906340 BE091310 AA491506 AW836675 AW863111 |
| 302640 | 21194_1 | AW973784 AW843642 AA557573 AA578088 AI125161 AA349349 AI372794 BE312586 BE312777 T32148 AW239077 AI905357 Z42685 AW298772 R18578 AA780425 AA325971 AI372793 R10658 AA295021 AW885349 AW885288 BE271987 AW366519 AA349350 AA233207 R88464 AA434299 R02058 R00019 R54563 Z44886 R20150 AW368328 AW368321 AW802152 W79803 H12809 AA028951 AW367382 AA295247 H46355 AA905620 R54564 H12765 AW950608 AA028952 AA366908 AI085652 R43207 R77954 AI672848 T28547 AA427734 AA572853 AA769934 AI242108 R00020 R02059 R10659 AI185270 AI041890 NM_000080 X66403 F03854 AI652442 AI766431 AA976913 AI989882 AA471024 AI802727 AI824112 F02169 AI890843 BE250876 BE252859 AL157418 R78326 |
| 303342 | 189722_1 | AW068570 AW247361 AA252638 AI751982 BE260758 BE293073 AW293303 |
| 302703 | 7075_1 | AB040951 AK002094 AA676593 W44644 N42376 Z45942 AW841844 BE541378 AA358274 AA213391 T88771 NM_015493 AL117489 N88248 N31714 N36273 N31721 AW576263 AA449380 AI366135 AA551576 AW149789 Z17418 AW474331 AA056181 AI753611 AA046428 AA488007 AA300764 N44732 AA377697 AA346752 AA485787 AA894546 AA115295 AA299914 R88096 AA367342 AW884666 W84522 AA426325 AI983849 AA873315 AA873307 AI355170 AA534678 AA969227 AI127202 AW083323 AI338244 AW020877 AA780019 N33426 AW069314 N63079 AI926527 AA115270 AW886601 AI357402 AA599312 AI460358 AI926969 AA429402 N33197 AW886733 R88205 N52803 AW021988 AA213392 BE139656 AI142383 AA427844 AA954743 AA233622 AW073382 AA426326 |
| | | AA493560 AA425133 N24819 AI419516 AI571515 AI147373 AI628677 AI214877 AA992123 H71599 AA029095 AA622262 AW117398 AW275286 AI911337 AA864950 T94173 AI475634 AI701411 AI287696 T94091 AA505746 AI184310 AI350967 AI083596 W74274 AI954381 AI832767 AI368443 AA195578 AW874416 AI005421 AW014339 AA908660 AI350791 AW241382 AI473104 AI275186 AA515528 AA194897 AA782901 AW069414 F20248 AA426011 AI305169 AI832109 AI570082 AW072984 AI492474 AA919076 AL049024 W79889 N42400 AA625435 AW963887 AA233420 |
| 318446 | 604736_1 | AW779971 AW300287 AW152002 AW069505 AI866447 AI298231 AI146920 AI692267 AI872876 |
| 302815 | 42200_1 | BE397032 AJ292529 N40373 N34073 AA321112 AW959902 AA258103 AW860213 BE549059 BE296027 BE296657 AA300789 AI971491 AW513665 AA909530 AI951045 AW058103 AI971506 AI061239 AA600054 AI000807 AA969975 AA281492 AW593654 AA321111 AW298633 AI278754 AI863862 AI285506 AA989727 F33114 T16079 AI762625 AI492103 AW770346 AW026768 AI468710 AI499987 AA310412 AA622784 AA642297 AI866427 |
| 302879 | 36555_1 | H11802 T66097 AF042831 |
| 318540 | 1018709_2 | R42185 AW939055 T30280 Z43366 R54166 |
| 302928 | 22118_1 | AA938905 AA574056 AA714466 AI805592 AI123431 AA229723 AA620759 AI004450 AW299820 AI949299 AW874308 AA626037 A974112 AA931563 AF073924 AA995769 AI766441 AI367730 AI081342 AA235800 AA235801 AI138970 |
| | | AA719797 AA759343 X89673 AA759344 AA312909 X87825 Y10529 AC006271 AA758739 BE501015 AA909905 AF065857 AC006271 AA970044 |
| 304332 | 28696_41 | AA158883 AA171835 AA187049 AA143546 BE299538 BE614280 BE621705 BE299684 BE619550 BE613099 BE619558 BE514331 BE617716 BE612920 BE615742 BE258739 BE621539 AI434511 BE546696 BE614324 BE379359 BE250106 BE250681 BE299592 BE300272 BE616805 BE397385 BE562024 BE271246 BE250556 BE280311 BE561995 BE618755 BE276126 BE546275 BE311547 BE262155 BE281082 BE513087 |
| | | BE546891 BE514289 BE397389 BE267442 BE545455 BE614483 BE293447 BE270710 BE281071 BE267458 BE542095 BE262701 BE513634 BE548116 BE299546 BE619604 BE512885 BE616638 BE266173 BE258933 BE259710 BE268569 BE563861 BE614871 BE537509 BE250108 BE515323 BE538868 BE250081 BE277706 BE410127 BE619445 BE250753 BE304969 BE616348 BE546878 BE544962 BE410346 BE267256 X17206 NM_002952 BE304541 BE619171 BE259655 BE549186 BE314944 BE613101 BE378069 BE621110 BE542752 BE257029 |
| | | BE531315 BE619306 BE267328 BE259439 BE297093 BE280651 BE407684 BE250201 BE312819 BE535432 BE279917 BE312626 BE531118 BE378744 BE275370 BE250195 BE409980 BE274432 BE266637 BE279321 BE622382 BE280232 BE263816 BE378977 BE300145 BE250204 BE547609 BE264377 BE266688 BE259746 BE260829 BE619517 BE388097 BE264025 BE618945 BE614758 BE312249 BE294359 BE531121 |
| | | BE622300 BE615109 BE544354 BE614998 BE393239 BE297520 BE393221 BE278818 BE279309 BE265476 BE618772 BE615185 BE265144 BE249837 BE312230 BE407843 BE253884 BE407645 BE615804 BE619058 BE559512 BE383249 BE613497 BE294351 BE295062 BE622385 BE390654 BE535438 BE563186 BE396374 BE270842 BE386110 BE260368 BE250186 BE265875 BE537229 BE253369 BE256997 BE269482 BE264959 BE279072 AA662160 BE280733 AA858428 BE561308 BE267285 BE561422 BE563181 BE304614 BE295437 BE619424 |
| | | BE275863 BE394315 BE408109 BE541866 BE253772 BE618236 BE535261 BE296490 BE278212 BE563154 BE257245 BE262274 BE513032 BE378567 BE394152 BE618947 BE269302 BE546516 BE536792 BE615187 BE261186 BE615367 BE619289 BE261184 T49376 AL031671 BE273400 BE563457 BE545597 BE615169 AA150323 AA158723 AA079033 BE313333 AA160100 BE271115 BE294302 BE273051 BE273048 BE622390 AA837947 BE387721 |
| | | AW973277 AA808731 BE280792 AA160444 BE256723 AI745420 AA643017 BE549441 BE293858 AW975249 AI620819 AW089494 AI434549 BE305231 AA081262 BE280101 AA522507 AI950880 AA187460 BE386860 AW859229 BE170489 BE620149 BE548218 AA316696 AA484426 AI567740 AA160605 AW939805 AA089573 BE300194 BE391331 AW975419 H26808 BE545544 BE615974 AW800241 BE616222 W17343 BE387865 T53697 C03943 BE617637 BE315130 T52942 T50588 N74693 AA187107 T59919 AW797397 |
| | | AA206447 AA854619 T57175 AI570296 AW517964 AA158269 AI282220 W25297 AI580710 BE262453 AI185868 AA526485 AI288051 AI582513 AA100675 AW615567 BE395354 AI472725 BE314881 BE621281 N99921 AI282689 AI432725 AW732011 AA872254 BE205807 T59435 AI282712 AA650505 AI004374 AA725260 BE313161 T60173 AI371260 BE385641 AW751812 AA078827 AI491858 AI433622 |
| | | AA219118 AI002092 AA996003 AA064604 AI250287 AI304397 AI453213 AA653630 AI524573 AI440306 H48802 AA157843 AA715629 AW973788 AA932493 AI347563 AA181309 T67880 AA643033 AW467498 AA115904 AA935410 AA483032 AA084568 W25246 AI567588 AA155732 AA158614 AA888319 AA158568 AA188422 AA309183 AA084817 AA157995 AI859659 AA188008 AI287379 AI540675 AA085212 AW028391 AA173297 BE256792 AA182854 BE378771 BE538571 AA079037 BE281597 AA643926 W81011 AA159344 AA320691 |
| | | AA877597 T57107 AW263819 AI690413 AI619605 AI687579 AA970560 AI368942 AI927104 AW419220 AI620051 AA128490 AA120825 AA079520 AA199648 AW188403 BE045224 AW265533 AA074338 AA102685 AW779399 AA192451 AA182771 AW366812 BE281418 AA211094 AA131073 AA487924 AW674848 AI568103 AA171934 F30349 AW088785 AA581370 AA205482 AW352296 |
| | | AW517565 AI376249 AA158884 AI340509 T59965 AA085193 AA071570 AI874045 AA852755 BE045217 AW189428 AA211141 AA652134 AI497729 AA994817 AI811459 BE535857 AW769697 AW167892 AW149305 AI864981 AW272126 AW023245 AI439266 AI953196 AA160912 AI718560 BE537547 AA501448 AA069308 L07393 AA353007 AA079235 AI539140 AA740154 W58341 AA888403 BE299000 AA196413 BE613327 BE261523 AA866599 AW844713 AI691159 AI079975 AW327479 BE180731 AA984805 |
| 303701 | 1155179_1 | AW500732 AW504061 |
| 303759 | 447287_1 | AA774672 AW504164 |
| 303773 | 356632_1 | AA769074 AA570769 AA808585 AA808682 |
| 303778 | 174437_1 | AW505368 AA218610 F11852 T65345 AA397806 |
| 303784 | 414659_1 | BE297711 AW505574 AA704983 |
| 303845 | 50211_2 | F07942 T08033 |
| 303898 | 162688_3 | BE386266 BE148823 T23215 AI906290 AA299906 BE207197 AW074114 AI760368 AI005358 AW662201 AA188988 AI690711 AA775103 AW072931 AI684269 AW129364 AW615634 AI049941 AW874040 AI352633 AA188989 AI287775 AA868774 AA599660 |
| 20121 | 452027_1 | AA780365 AA909233 AI275542 |
| 319590 | 171338_1 | AA210878 AA215684 R11101 |
| 305186 | 17456_1 | M13560 AA336951 AA161015 R72814 T69687 R75705 T61319 AA158454 R50579 T56649 AI214156 T70375 R31655 H64997 AW800487 H49110 AA634206 H42384 H21783 AI560152 AA664230 H42302 R48708 AA013277 T61901 T92417 AA875985 T61962 T63055 AA430725 AA458964 AA578746 AI582385 T63000 AI499875 H64998 AA022538 AI364804 AI865211 AI439714 AI224059 AI249917 T59258 AA477806 AA715834 AA916120 R38304 R35899 R82985 H25524 H82984 AW516728 T54642 AA079866 H27555 AA455820 T63919 R79450 AI431241 AA937349 AA127213 AA421729 H61196 T63894 AA013050 AA079133 W98364 AA487926 AI762796 H26377 AI433386 AI865423 AW371475 R98189 AA643978 AI718204 AW381954 AI862735 |
| 319638 | 226485_1 | AA323758 R12731 R14082 |
| 320257 | 163534_1 | R17531 AW960899 AA338366 AW673294 BE047729 BE047722 AA330746 AW841797 H05030 AI142105 R12654 |
| 320289 | 115941_1 | H07989 AJ239462 H24544 AA078369 R74153 |
| 304703 | 33971_42 | BE512926 BE304794 AA129140 AA052922 AA092258 BE378058 BE615391 BE615218 BE616188 AI214126 H05675 W56857 AI028525 BE617241 BE531271 AW856227 T56489 AA322005 AW794148 AF170577 BE615738 AA005138 L76930 L76932 L76933 X95410 AW389462 BE563092 AW997937 AA263158 AI520992 AW947350 AA522535 AW945921 AV653776 AW884835 AW947338 AI687178 AW945799 AI905627 AW948449 AV653751 AW945924 AA563898 AW945810 AW945832 AW371449 AW945864 AW948447 AW945910 AA643002 AA522680 AA522715 AA578840 AA523279 AA826150 AW945809 AW405998 AA551909 R23173 AA595545 AW389497 AI933770 AI125053 AI471803 AW795856 AW796937 W30675 H70317 |
| | | H68296 T59240 AA397650 H59852 AA938072 AA978010 R35643 T89735 AW361585 AW196153 AI538069 AA604540 AI434259 R49181 T58717 AW062486 AW796966 AI648384 R77733 AI623502 BE171342 BE171303 R35658 AW974883 AW149898 AI500045 AI540710 AI540392 AW009172 AW277199 AI371312 AI500096 AI470297 AW372940 AW844562 AW844560 AW797965 AI691146 X07062 AW799199 H60666 AA837684 AF130734 T25952 AI933771 AI914860 AW391925 AW793843 AW795012 AW366709 AW750987 AW750985 R35765 AW844942 AW750986 H64920 R34651 X86703 |
| 321039 | 26338_2 | BE018103 BE018083 BE293253 AW247083 BE207643 BE514793 BE183238 AA376427 AW273850 AW043786 BE439973 AL045428 AI889050 AA026496 AI422924 AI884485 W96068 AA020872 F37119 AA714378 AA021107 AA011141 AI554001 AI375841 AI469097 AA335219 AW967315 AI692177 AA410448 AI568858 AA582647 AA026419 AA281639 AW515248 AW007777 AA010840 AW188439 AI805423 AI148210 BE301590 AA744414 AA745392 AW167423 AA622659 AW000878 AI432387 AA760930 BE047189 AA021605 AV658045 AI093347 AA588594 H63143 AA639556 AI308976 AA379270 AA633407 AI874329 AI206484 AI493895 AI694103 AI249682 AA973765 AA872445 AI125446 AA287272 AW069761 AA682569 AW009712 BE542774 R50167 BE301574 AA991202 AA502006 AI219819 AW074373 AA617996 AI621242 F25241 AW615812 R16774 AA335218 AW673800 H26778 AI468557 AI886986 AI560759 AI460075 AA502968 |
| | | AA503273 AA610680 AA287274 AA554020 AA284889 AA916636 AW469457 AW273250 AW673708 AW512948 AL041071 AI446042 AA903535 BE172441 AI282411 AW265021 AA810799 AI559865 AA729332 AW004611 AW129451 AA659019 BE208239 AA610825 H03511 BE383995 R16474 AA281701 AW009244 AA287424 AA558139 AW364081 |
| 306051 | 19085_3 | F08147 AW408359 AW949429 R23785 AW247442 AA305512 T29095 AA905130 BE246361 BE244981 AA220199 BE504058 X80878 AA533727 AA608601 AW005964 AI811627 AI367037 AI277985 AI493719 AI277848 AA854982 AW247298 AI216345 AI041295 AI887378 AA781241 AI674270 AW628959 AI383083 BE504391 AA729421 AA552188 AA373387 AW880360 AW875262 AW875369 AW581540 AW875358 AW581568 R23735 AW134768 |
| 321163 | 171122_1 | W03912 AW971410 AA506385 AA209530 H73495 H48629 W56149 |
| 321235 | 1102181_1 | H56752 AW340384 N49521 |
| 320603 | 4297_1 | AA853680 AK001668 BE386425 BE563549 BE296124 BE298950 R51419 U46295 BE147292 AA360056 R48018 AW845348 N47383 AI817280 AI671902 AA988104 AA479464 N56996 AI192374 AI927558 AA659888 AI799903 AA548397 AI161167 AI656333 AI418829 AW592671 BE327906 AW513346 AI888579 AW469410 AW512809 D25682 AA576079 AA479354 T30342 R51307 T16044 H29063 AW079357 AI339477 R47914 AI986068 AI870065 AI868489 AI521099 AI582732 AA995540 AW957299 AA352608 AA676752 AA410510 AA358874 AI865724 AA853679 AI699265 AW188789 N47380 AA233715 |
| 320641 | 185591_1 | BE258194 R55421 R55643 H42362 AA243884 |
| 320651 | 58648_1 | AW886407 AA489268 R57015 R58094 BE077459 BE077423 BE546995 AW849216 T69383 AW938111 H60337 BE221073 |
| 321325 | 28266_1 | AB033100 AA347036 BE260325 AW961669 AL047207 AA347037 AI766894 AA601045 AI559897 AW139033 AW274622 AW172884 AW089070 AA804340 AW798925 |
| 305704 | 464759_-1 | AA825266 |
| 322011 | 23158_1 | AL137354 AL043375 |
| 306407 | | AA971985 |
| 306454 | | AA977992 |
| 306516 | | AA989542 |
| 306518 | | AA989598 |
| 306526 | | AA989713 |
| 306534 | | AA991487 |
| 306590 | | AI000246 |
| 306591 | | AI000248 |
| 306631 | | AI001149 |
| 306654 | | AI003654 |
| 306786 | | AI041589 |
| 306799 | | AI051696 |
| 308023 | | AI452732 |
| 308070 | | AI470948 |
| 308099 | | AI475914 |
| 306805 | | AI055966 |
| 306814 | | AI066577 |
| 306873 | | AI086929 |
| 306911 | | AI095365 |
| 306982 | | AI127883 |
| 308238 | | AI559492 |
| 308258 | | AI565612 |
| 308289 | | AI571211 |
| 308311 | | AI581855 |
| 308332 | | AI591235 |
| 308511 | | AI687580 |
| 308601 | | AI719930 |
| 308612 | | AI735634 |
| 308636 | | AI744063 |
| 308814 | | AI819263 |
| 308851 | | AI829820 |
| 308981 | | AI873242 |
| 310570 | 1071946_1 | AI318327 AI318328 AI318495 |
| 305022 | | AA627416 |
| 305060 | | AA635771 |
| 305070 | | AA639783 |
| 305079 | | AA641329 |
| 305134 | | AA653159 |
| 303977 | | AW512978 |
| 305216 | | AA669056 |
| 305263 | | AA679467 |
| 305266 | | AA679772 |
| 305396 | | AA721052 |
| 305403 | | AA723748 |
| 305488 | | AA749000 |
| 305549 | | AA773530 |
| 305601 | | AA780975 |
| 305610 | | AA782319 |
| 305621 | | AA789095 |
| 305710 | | AA826544 |
| 305724 | | AA827608 |
| 305744 | | AA831819 |
| 305752 | | AA835278 |
| 307018 | | AI140639 |
| 307055 | | AI148477 |
| 307058 | | AI148709 |
| 305801 | | AA845997 |
| 305830 | | AA857665 |
| 305836 | | AA858043 |
| 305852 | | AA862455 |
| 305858 | | AA863103 |
| 305866 | | AA864533 |
| 305867 | | AA864572 |
| 307126 | | AI184951 |
| 305903 | | AA873085 |
| 328803 | c_7_hs | |
| 328809 | c_7_hs | |
| 305949 | AA884409 | |
| 328829 | c_7_hs | |
| 330021 | c16_p2 | |
| 330024 | c16_p2 | |
| 330028 | c16_p2 | |
| 330049 | c17_p2 | |
| 305993 | AA889197 | |
| 330095 | c19_p2 | |
| 330096 | c19_p2 | |
| 307205 | | AI192479 |
| 307427 | | AI243437 |
| 307491 | | AI268539 |
| 307581 | | AI284415 |
| 307588 | | AI285535 |
| 337672 | CH22_6002FG_LINK_EM:AC00 | |
| 337693 | CH22_6030FG_LINK_EM:AC00 | |
| 337738 | CH22_6083FG_LINK_EM:AC00 | |
| 307692 | | AI318342 |
| 307806 | | AI351739 |
| 309107 | | AI925823 |
| 309230 | | AI970747 |
| 339338 | CH22_8300FG_LINK_BA354l1 | |
| 309257 | | AI984183 |
| 309366 | | AW072970 |
| 309422 | | AW087175 |
| 325207 | c10_hs | |
| 325257 | c11_hs | |
| 309646 | | AW194694 |
| 309651 | | AW195850 |
| 325313 | c11_hs | |
| 309924 | | AW340812 |
| 334030 | CH22_1308FG_320_2_LINK_EM | |
| 334040 | CH22_1318FG_322_8_LINK_EM | |
| 334083 | CH22_1361FG_327_38_LINK_E | |
| 332810 | CH22_26FG_7_12_LINK_C65E1 | |
| 302747 | 32813_1 | AF062275 L03830 |
| 302753 | 33029_1 | M74299 M74302 M74303 |
| 302777 | 33803_1 | AJ230640 AJ230648 |
| 304094 | | H11295 |
| 302824 | 35372_1 | U21260 U21258 |
| 302996 | 41196_1 | AF054663 AF124197 R70292 |
| 325870 | c16_hs | |
| 304240 | | AA009802 |
| 304410 | | AA284508 |
| 304443 | | AA399444 |
| 304475 | | AA428879 |
| 304522 | | AA465405 |
| 304678 | | AA548556 |
| 304705 | | AA564064 |
| 306004 | | AA889992 |
| 306008 | | AA894390 |
| 306013 | | AA896990 |
| 306082 | | AA908508 |
| 336174 | CH22_3567FG_710_1_LINK_DA | |
| 306094 | | AA908877 |
| 304823 | | AA584837 |
| 304872 | | AA595289 |
| 304918 | | AA602697 |
| 304955 | | AA613504 |
| 306249 | | AA933840 |
| 306286 | | AA936892 |
| 306295 | | AA937331 |
| 306317 | | AA947909 |
| 306347 | | AA961144 |
| 306365 | | AA962086 |
| 306398 | | AA970548 |
| 330401 | entrez_D28383 | D28383 |
| 330463 | 460_2 | NM_001055 AA332948 U26309 U09031 L19955 L10819 AI366043 X84654 U71086 AV654451 AJ007418 AA053625 BE168856 AA376730 H12694 AA810348 AA621972 AI818950 AV645367 AI819966 AA910602 AW512449 H67893 AI310497 AI304330 AI339217 AW193588 AW438688 AI818970 AW316799 AA906527 AA777570 N47673 AI336428 AW945133 AI038606 R29692 AW194197 AI304748 H12639 AA053178 AA493213 AA676958 AA113154 AI313469 AI368239 R93183 W24532 U52852 U54701 AL046864 AA365795 |
| 330535 | 1374_-8 | U11872 |
| 332634 | 10404_2 | U24488 NM_007116 |

**TABLE 13B shows the genomic positioning for those primekeys lacking unigene ID's and accession numbers in Table 13. For each predicted exon, we have listed the genomic sequence source used for prediction. Nucleotide locations of each predicted exon are also listed.**

| | | | |
|---|---|---|---|
| Pkey: | | Unique number corresponding to an Eos probeset | |
| Ref: | | Sequence source. The 7 digit numbers in this column are Genbank Identifier (GI) numbers | |
| Strand: | | Indicates DNA strand from which exons were predicted. | |
| Nt_position: | | Indicates nucleotide positions of predicted exons. | |
| | | | |

| **Pkey** | **Ref** | **Strand** | **Nt_position** |
|---|---|---|---|
| | | | |
| 332791 | Dunham, I. et.al. | Plus | 72720-73315 |
| 332792 | Dunham, I. et.al. | Plus | 73381-73768 |
| 332810 | Dunham, I. et.al. | Plus | 304296-304384 |
| 332944 | Dunham, I. et.al. | Plus | 2414825-2414932 |
| 332972 | Dunham, I. et.al. | Plus | 2572152-2572236 |
| 333133 | Dunham, I. et.al. | Plus | 3360058-3360195 |
| 333154 | Dunham, I. et.al. | Plus | 3615887-3616019 |
| 333155 | Dunham, I. et.al. | Plus | 3616832-3617003 |
| 333227 | Dunham, I. et.al. | Plus | 3992866-3992968 |
| 333230 | Dunham, I. et.al. | Plus | 3995507-3996507 |
| 333298 | Dunham, I. et.al. | Plus | 4581537-4581947 |
| 333304 | Dunham, I. et.al. | Plus | 4629943-4630242 |
| 333305 | Dunham, I. et.al. | Plus | 4630388-4630645 |
| 333365 | Dunham, I. et.al. | Plus | 4786883-4787283 |
| 333383 | Dunham, I. et.al. | Plus | 4907179-4907277 |
| 333391 | Dunham, I. et.al. | Plus | 4916697-4916780 |
| 333392 | Dunham, I. et.al. | Plus | 4918294-4918433 |
| 333397 | Dunham, I. et.al. | Plus | 4922466-4922635 |
| 333403 | Dunham, I. et.al. | Plus | 4925140-4925256 |
| 333413 | Dunham, I. et.al. | Plus | 4943824-4943974 |
| 333445 | Dunham, I. et.al. | Plus | 5097827-5097885 |
| 333479 | Dunham, I. et.al. | Plus | 5272855-5272939 |
| 333481 | Dunham, I. et.al. | Plus | 5286358-5286505 |
| 333483 | Dunham, I. et.al. | Plus | 5297945-5298105 |
| 333516 | Dunham, I. et.al. | Plus | 5570204-5570390 |
| 333517 | Dunham, I. et.al. | Plus | 5570729-5570925 |
| 333518 | Dunham, I. et.al. | Plus | 5571761-5572025 |
| 333531 | Dunham, I. et.al. | Plus | 5622622-5622684 |
| 333566 | Dunham, I. et.al. | Plus | 5954226-5954473 |
| 333572 | Dunham, I. et.al. | Plus | 6026896-6027189 |
| 333586 | Dunham, I. at.al. | Plus | 6246834-6247314 |
| 333588 | Dunham, I. et.al. | Plus | 6255445-6255779 |
| 333594 | Dunham, I. et.al. | Plus | 6308990-6309450 |
| 333595 | Dunham, I. et.al. | Plus | 6323103-6323348 |
| 333600 | Dunham, I. et.al. | Plus | 6355629-6355925 |
| 333601 | Dunham, I. et.al. | Plus | 6360075-6360442 |
| 333607 | Dunham, I. et.al. | Plus | 6504431-6504690 |
| 333612 | Dunham, I. et.al. | Plus | 6549563-6549697 |
| 333613 | Dunham, I. et.al. | Plus | 6550643-6550748 |
| 333614 | Dunham, I. et.al. | Plus | 6551227-6551389 |
| 333624 | Dunham, I. et.al. | Plus | 6595146-6595244 |
| 333626 | Dunham, I. et.al. | Plus | 6614174-6614467 |
| 333635 | Dunham, I. et.al. | Plus | 6663683-6663973 |
| 333637 | Dunham, I. et.al. | Plus | 6674968-6675134 |
| 333642 | Dunham, I. et.al. | Plus | 6708760-6709139 |
| 333647 | Dunham, I. et.al. | Plus | 6772502-6772779 |
| 333653 | Dunham, I. et.al. | Plus | 6811130-6811392 |
| 333654 | Dunham, I. et.al. | Plus | 6816731-6816993 |
| 333656 | Dunham, I. et.al. | Plus | 6822087-6822406 |
| 333657 | Dunham, I. et.al. | Plus | 6831369-6831445 |
| 333658 | Dunham, I. et.al. | Plus | 6835282-6835474 |
| 333659 | Dunham, I. et.al. | Plus | 6836179-6836248 |
| 333684 | Dunham, I. et.al. | Plus | 7169561-7169742 |
| 333686 | Dunham, I. et.al. | Plus | 7177117-7177302 |
| 333697 | Dunham, I. et.al. | Plus | 7203859-7203934 |
| 333698 | Dunham, I. et.al. | Plus | 7205279-7205383 |
| 333699 | Dunham, I. et.al. | Plus | 7206101-7206175 |
| 333703 | Dunham, I. et.al. | Plus | 7215559-7215663 |
| 333709 | Dunham, I. et.al. | Plus | 7229730-7229835 |
| 333747 | Dunham, I. et.al. | Plus | 7605884-7606206 |
| 333774 | Dunham, I. et.al. | Plus | 7716509-7716636 |
| 333775 | Dunham, I. et.al. | Plus | 7729983-7730149 |
| 333806 | Dunham, I. et.al. | Plus | 7877475-7877666 |
| 333843 | Dunham, I. et.al. | Plus | 7978762-7978887 |
| 333854 | Dunham, I. et.al. | Plus | 8029446-8029524 |
| 333873 | Dunham, I. et.al. | Plus | 8133266-8133429 |
| 333880 | Dunham, I. et.al. | Plus | 8151923-8152133 |
| 333885 | Dunham, I. et.al. | Plus | 8154352-8154437 |
| 333918 | Dunham, I. et.al. | Plus | 8307124-8307215 |
| 333947 | Dunham, I. et.al. | Plus | 8579888-8579966 |
| 333961 | Dunham, I. et.al. | Plus | 8617999-8618104 |
| 333981 | Dunham, I. et.al. | Plus | 8782374-8782643 |
| 333991 | Dunham, I. et.al. | Plus | 8837419-8837551 |
| 333994 | Dunham, I. et.al. | Plus | 8852749-8852894 |
| 334030 | Dunham, I. et.al. | Plus | 9288463-9288782 |
| 334083 | Dunham, I. et.al. | Plus | 9837016-9837081 |
| 334111 | Dunham, I. et.al. | Plus | 10279365-10279531 |
| 334135 | Dunham, I. et.al. | Plus | 10457085-10457183 |
| 334218 | Dunham, I. et.al. | Plus | 12680289-12680378 |
| 334249 | Dunham, I. et.al. | Plus | 13190430-13190574 |
| 334262 | Dunham, I. et.al. | Plus | 13231452-13231581 |
| 334264 | Dunham, I. et.al. | Plus | 13234447-13234544 |
| 334327 | Dunham, I. et.al. | Plus | 13577413-13577496 |
| 334328 | Dunham, I. et.al. | Plus | 13589868-13589936 |
| 334340 | Dunham, I. et.al. | Plus | 13642407-13642522 |
| 334454 | Dunham, I. et.al. | Plus | 14326506-14326738 |
| 334504 | Dunham, I. et.al. | Plus | 14510206-14510398 |
| 334508 | Dunham, I. et.al. | Plus | 14514936-14515122 |
| 334512 | Dunham, I. et.al. | Plus | 14545933-14546366 |
| 334582 | Dunham, I. et.al. | Plus | 15026255-15026371 |
| 334659 | Dunham, I. et.al. | Plus | 15460624-15460726 |
| 334721 | Dunham, I. et.al. | Plus | 15796816-15796987 |
| 334723 | Dunham, I. et.al. | Plus | 15805317-15805399 |
| 334730 | Dunham, I. et.al. | Plus | 15967830-15967934 |
| 334774 | Dunham, I. et.al. | Plus | 16251857-16252178 |
| 334778 | Dunham, I. et.al. | Plus | 16276180-16276395 |
| 334851 | Dunham, I. et.al. | Plus | 17820110-17820810 |
| 334885 | Dunham, I. et.al. | Plus | 19233667-19233787 |
| 334902 | Dunham, I. et.al. | Plus | 19317083-19317195 |
| 334905 | Dunham, I. et.al. | Plus | 19322553-19322680 |
| 334906 | Dunham, I. et.al. | Plus | 19323493-19323590 |
| 334910 | Dunham, I. et.al. | Plus | 19398155-19398684 |
| 335018 | Dunham, I. et.al. | Plus | 20688288-20688415 |
| 335025 | Dunham, I. et.al. | Plus | 20743941-20744050 |
| 335033 | Dunham, I. et.al. | Plus | 20753188-20753314 |
| 335044 | Dunham, I. et.al. | Plus | 20842088-20842682 |
| 335142 | Dunham, I. et.al. | Plus | 21465105-21465186 |
| 335157 | Dunham, I. et.al. | Plus | 21543302-21544341 |
| 335160 | Dunham, I. et.al. | Plus | 21573388-21573497 |
| 335174 | Dunham, I. et.al. | Plus | 21631301-21631447 |
| 335188 | Dunham, I. et.al. | Plus | 21669118-21669328 |
| 335190 | Dunham, I. et.al. | Plus | 21680807-21680876 |
| 335191 | Dunham, I. et.al. | Plus | 21681110-21681183 |
| 335193 | Dunham, I. et.al. | Plus | 21692208-21692362 |
| 335204 | Dunham, I. et.al. | Plus | 21750636-21750726 |
| 335222 | Dunham, I. et.al. | Plus | 21885542-21885608 |
| 335226 | Dunham, I. et.al. | Plus | 21890838-21890930 |
| 335227 | Dunham, I. et.al. | Plus | 21892145-21892289 |
| 335309 | Dunham, I. et.al. | Plus | 22500158-22500276 |
| 335310 | Dunham, I. et.al. | Plus | 22500714-22500831 |
| 335311 | Dunham, I. et.al. | Plus | 22501602-22501676 |
| 335355 | Dunham, I. et.al. | Plus | 22779222-22779516 |
| 335362 | Dunham, I. et.al. | Plus | 22809167-22809461 |
| 335368 | Dunham, I. et.al. | Plus | 22843D40-22843184 |
| 335384 | Dunham, I. et.al. | Plus | 22918150-22918263 |
| 335385 | Dunham, I. et.al. | Plus | 22919072-22919339 |
| 335436 | Dunham, I. et.al. | Plus | 23427793-23427923 |
| 335440 | Dunham, I. et.al. | Plus | 23458702-23459017 |
| 335441 | Dunham, I. et.al. | Plus | 23460632-23460724 |
| 335450 | Dunham, I. et.al. | Plus | 23480190-23480270 |
| 335453 | Dunham, I. et.al. | Plus | 23483333-23483459 |
| 335458 | Dunham, I. et.al. | Plus | 23490034-23490143 |
| 335464 | Dunham, I. et.al. | Plus | 23500331-23500496 |
| 335496 | Dunham, I. et.al. | Plus | 24164386-24164545 |
| 335497 | Dunham, I. et.al. | Plus | 24167666-24167869 |
| 335498 | Dunham, I. et.al. | Plus | 24172082-24172161 |
| 335499 | Dunham, I. et.al. | Plus | 24176698-24176869 |
| 335500 | Dunham, I. et.al. | Plus | 24178236-24178326 |
| 335507 | Dunham, I. et.al. | Plus | 24219973-24220039 |
| 335510 | Dunham, I. et.al. | Plus | 24222975-24223118 |
| 335513 | Dunham, I. et.al. | Plus | 24224272-24224496 |
| 335627 | Dunham, I. et.al. | Plus | 25150005-25150061 |
| 335651 | Dunham, I. et.al. | Plus | 25317560-25317696 |
| 335655 | Dunham, I. et.al. | Plus | 25333211-25333369 |
| 335656 | Dunham, I. et.al. | Plus | 25333601-25333751 |
| 335658 | Dunham, I. et.al. | Plus | 25336315-25336406 |
| 335663 | Dunham, I. et.al. | Plus | 25342680-25342802 |
| 335665 | Dunham, I. et.al. | Plus | 25344096-25344287 |
| 335667 | Dunham, I. et.al. | Plus | 25345735-25345856 |
| 335668 | Dunham, I. et.al. | Plus | 25346313-25346447 |
| 335689 | Dunham, I. et.al. | Plus | 25454350-25454604 |
| 335690 | Dunham, I. et.al. | Plus | 25455442-25455625 |
| 335715 | Dunham, I. et.al. | Plus | 25565941-25566052 |
| 335719 | Dunham, I. et.al. | Plus | 25593936-25594101 |
| 335734 | Dunham, I. et.al. | Plus | 25688723-25688869 |
| 335744 | Dunham, I. et.al. | Plus | 25716483-25716615 |
| 335809 | Dunham. I. et.al. | Plus | 26310772-26310909 |
| 335819 | Dunham, I. et.al. | Plus | 26356341-26356470 |
| 335822 | Dunham, I. et.al. | Plus | 26364087-26364196 |
| 335872 | Dunham, I. et.al. | Plus | 26820760-26820943 |
| 335885 | Dunham, I. et.al. | Plus | 26933436-26933534 |
| 335968 | Dunham, I. et.al. | Plus | 27743843-27744029 |
| 335971 | Dunham, I. et.al. | Plus | 27752808-27753017 |
| 335975 | Dunham, I. et.al. | Plus | 27801321-27801391 |
| 335976 | Dunham, I. et.al. | Plus | 27809041-27809187 |
| 335989 | Dunham, I. et.al. | Plus | 27983788-27983860 |
| 335990 | Dunham, I. et.al. | Plus | 27988532-27988608 |
| 336010 | Dunham, I. et.al. | Plus | 28570239-28570330 |
| 336093 | Dunham, I. et.al. | Plus | 29556922-29557002 |
| 336126 | Dunham, I. et.al. | Plus | 30057891-30058105 |
| 336129 | Dunham, I. et.al. | Plus | 30062259-30062348 |
| 336187 | Dunham, I. et.al. | Plus | 30433494-30433585 |
| 336188 | Dunham, I. et.al. | Plus | 30434870-30435004 |
| 336225 | Dunham, I. et.al. | Plus | 30833614-30833788 |
| 336371 | Dunham, I. et.al. | Plus | 33968108-33968204 |
| 336373 | Dunham, I. et.al. | Plus | 33976308-33976504 |
| 336377 | Dunham, I. et.al. | Plus | 33994489-33994599 |
| 336380 | Dunham, I. et.al. | Plus | 33995323-33995434 |
| 336383 | Dunham, I. et.al. | Plus | 34005784-34005964 |
| 336384 | Dunham, I. et.al. | Plus | 34007429-34007559 |
| 336385 | Dunham, I. et.al. | Plus | 34007879-34008159 |
| 336386 | Dunham. I. et.al. | Plus | 34012965-34013115 |
| 336441 | Dunham, I. et.al. | Plus | 34187606-34187663 |
| 336444 | Dunham, I. et.al. | Plus | 34190585-34190718 |
| 336484 | Dunham, I. et.al. | Plus | 34237425-34237505 |
| 336497 | Dunham, I. et.al. | Plus | 34267190-34267245 |
| 336499 | Dunham, I. et.al. | Plus | 34267504-34267572 |
| 336503 | Dunham, I. et.al. | Plus | 34271306-34271372 |
| 336548 | Dunham, I. et.al. | Plus | 34353881-34354826 |
| 336552 | Dunham, I. et.al. | Plus | 34356420-34356527 |
| 336553 | Dunham, I. et.al. | Plus | 34356683-34356753 |
| 336567 | Dunham, I. et.al. | Plus | 34428228-34428395 |
| 336568 | Dunham, I. et.al. | Plus | 34428521-34428637 |
| 336659 | Dunham, I. et.al. | Plus | 1896402-1896478 |
| 336715 | Dunham, I. et.al. | Plus | 3110198-3110314 |
| 336803 | Dunham, I. et.al. | Plus | 6106904-6106990 |
| 336805 | Dunham, I. et.al. | Plus | 6126661-6126786 |
| 336850 | Dunham, I. et.al. | Plus | 7745284-7745355 |
| 336857 | Dunham, I. et.al. | Plus | 8130457-8130612 |
| 336911 | Dunham, I. et.al. | Plus | 11035818-11035984 |
| 336949 | Dunham, I. et.al. | Plus | 12818687-12818891 |
| 336950 | Dunham, I. et.al. | Plus | 12875843-12875912 |
| 336958 | Dunham, I. et.al. | Plus | 13203550-13203973 |
| 336993 | Dunham, I. et.al. | Plus | 15096270-15096324 |
| 337076 | Dunham, I. et.al. | Plus | 19338177-19338679 |
| 337109 | Dunham, I. et.al. | Plus | 21166580-21166650 |
| 337123 | Dunham, I. et.al. | Plus | 22052874-22052942 |
| 337151 | Dunham, I. et.al. | Plus | 23106433-23106510 |
| 337189 | Dunham, I. et.al. | Plus | 24225887-24225954 |
| 337241 | Dunham, I. et.al. | Plus | 27280182-27280313 |
| 337337 | Dunham, I. et.al. | Plus | 30395182-30395285 |
| 337353 | Dunham, I. et.al. | Plus | 30804624-30804780 |
| 337384 | Dunham, I. et.al. | Plus | 31333399-31333580 |
| 337396 | Dunham, I. et.al. | Plus | 31585902-31586067 |
| 337414 | Dunham, I. et.al. | Plus | 31953012-31953205 |
| 337418 | Dunham, I. et.al. | Plus | 32014049-32014131 |
| 337461 | Dunham, I. et.al. | Plus | 32803968-32804028 |
| 337480 | Dunham, I. et.al. | Plus | 33219714-33219779 |
| 337482 | Dunham, I. et.al. | Plus | 33227865-33227946 |
| 337483 | Dunham, I. et.al. | Plus | 33237292-33237427 |
| 337490 | Dunham, I. et.al. | Plus | 33318571-33318644 |
| 337522 | Dunham, I. et.al. | Plus | 33963188-33963979 |
| 337532 | Dunham, I. et.al. | Plus | 34187269-34187366 |
| 337552 | Dunham, I. et.al. | Plus | 19497-19600 |
| 337584 | Dunham, I. et.al. | Plus | 945236-945452 |
| 337611 | Dunham, I. et.al. | Plus | 1482883-1483016 |
| 337672 | Dunham, I. et.al. | Plus | 3331236-3331313 |
| 337693 | Dunham, I. et.al. | Plus | 3575975-3576153 |
| 337738 | Dunham, I. et.al. | Plus | 3865738-3865814 |
| 337926 | Dunham, I. et.al. | Plus | 6286377-6286470 |
| 337927 | Dunham, I. et.al. | Plus | 6343033-6343172 |
| 337935 | Dunham, I. et.al. | Plus | 6534661-6534782 |
| 337944 | Dunham, I. et.al. | Plus | 6589383-6589450 |
| 337954 | Dunham, I. et.al. | Plus | 6831483-6831620 |
| 337996 | Dunham, I. et.al. | Plus | 7445532-7445633 |
| 338004 | Dunham, I. et.al. | Plus | 7601363-7601520 |
| 338016 | Dunham, I. et.al. | Plus | 7863131-7863310 |
| 338174 | Dunham, I. et.al. | Plus | 12771102-12771268 |
| 338176 | Dunham, I. et.al. | Plus | 12774072-12774223 |
| 338238 | Dunham, I. et.al. | Plus | 14661936-14662015 |
| 338277 | Dunham, I. et.al. | Plus | 16167622-16167962 |
| 338294 | Dunham, I. et.al. | Plus | 16463958-16464539 |
| 338316 | Dunham, I. et.al. | Plus | 17089711-17089988 |
| 338323 | Dunham, I. et.al. | Plus | 17154655-17154792 |
| 338324 | Dunham, I. et.al. | Plus | 17155309-17155574 |
| 338386 | Dunham, I. et.al. | Plus | 18611213-18611407 |
| 338398 | Dunham, I. et.al. | Plus | 18953492-18953581 |
| 338410 | Dunham, I. et.al. | Plus | 19292807-19292916 |
| 338414 | Dunham, I. et.al. | Plus | 19345573-19345660 |
| 338460 | Dunham, I. et.al. | Plus | 20233372-20233488 |
| 338481 | Dunham, I. et.al. | Plus | 20942659-20942873 |
| 338489 | Dunham, I. et.al. | Plus | 21142605-21143049 |
| 338500 | Dunham, I. et.al. | Plus | 21253847-21253974 |
| 338514 | Dunham, I. et.al. | Plus | 21379420-21379655 |
| 338530 | Dunham, I. et.al. | Plus | 21636361-21636509 |
| 338620 | Dunham, I. et.al. | Plus | 23540239-23540334 |
| 338631 | Dunham, I. et.al. | Plus | 23711167-23711241 |
| 338653 | Dunham, I. et.al. | Plus | 24219427-24219509 |
| 338660 | Dunham, I. et.al. | Plus | 24387122-24387266 |
| 338704 | Dunham, I. et.al. | Plus | 25230432-25230548 |
| 338847 | Dunham, I. et.al. | Plus | 27995337-27995420 |
| 338887 | Dunham, I. et.al. | Plus | 28465244-28465384 |
| 338895 | Dunham, I. et.al. | Plus | 28598893-28599135 |
| 338915 | Dunham, I. et.al. | Plus | 28824881-28824977 |
| 338925 | Dunham, I. et.al. | Plus | 28883892-28884036 |
| 338936 | Dunham, I. et.al. | Plus | 29148022-29148160 |
| 338952 | Dunham, I. et.al. | Plus | 29418831-29418968 |
| 338980 | Dunham, I. et.al. | Plus | 29896789-29896874 |
| 338981 | Dunham, I. et.al. | Plus | 29897917-29898008 |
| 338986 | Dunham, I. et.al. | Plus | 30007287-30007415 |
| 339009 | Dunham, I. et.al. | Plus | 30348477-30348598 |
| 339017 | Dunham, I. et.al. | Plus | 30420896-30421090 |
| 339045 | Dunham, I. et.al. | Plus | 30744286-30744356 |
| 339046 | Dunham, I. et.al. | Plus | 30746269-30746420 |
| 339059 | Dunham, I. et.al. | Plus | 30814655-30814801 |
| 339067 | Dunham, I. et.al. | Plus | 30869347-30869412 |
| 339069 | Dunham, I. et.al. | Plus | 30880975-30881070 |
| 339078 | Dunham, I. et.al. | Plus | 30914310-30914423 |
| 339084 | Dunham, I. et.al. | Plus | 30944556-30944803 |
| 339101 | Dunham, I. et.al. | Plus | 31158047-31158123 |
| 339102 | Dunham, I. et.al. | Plus | 31169321-31169563 |
| 339103 | Dunham, I. et.al. | Plus | 31170343-31170454 |
| 339115 | Dunham, I. et.al. | Plus | 31459869-31459927 |
| 339157 | Dunham, I. et.al. | Plus | 32131701-32131833 |
| 339166 | Dunham, I. et.al. | Plus | 32210902-32211006 |
| 339167 | Dunham, I. et.al. | Plus | 32213567-32213730 |
| 339288 | Dunham, I. et.al. | Plus | 33169611-33169691 |
| 339289 | Dunham, I. et.al. | Plus | 33186756-33186903 |
| 339291 | Dunham, I. et.al. | Plus | 33205057-33205247 |
| 339407 | Dunham, I. et.al. | Plus | 34189461-34189620 |
| 332865 | Dunham, I. et.al. | Minus | 1391482-1391218 |
| 332881 | Dunham, I. et.al. | Minus | 1563520-1563184 |
| 332930 | Dunham, I. et.al. | Minus | 2022565-2022497 |
| 332931 | Dunham, I. et.al. | Minus | 2023651-2023562 |
| 332984 | Dunham, I. et.al. | Minus | 2632606-2632457 |
| 332986 | Dunham, I. et.al. | Minus | 2635398-2635206 |
| 332997 | Dunham, I. et.al. | Minus | 2710509-2710375 |
| 333051 | Dunham, I. et.al. | Minus | 2991973-2991840 |
| 333061 | Dunham, I. et.al. | Minus | 3029631-3029527 |
| 333064 | Dunham, I. et.al. | Minus | 3030722-3030623 |
| 333096 | Dunham, I. et.al. | Minus | 3184234-3184118 |
| 333099 | Dunham, I. et.al. | Minus | 3206796-3206674 |
| 333106 | Dunham, I. et.al. | Minus | 3230744-3230547 |
| 333160 | Dunham, I. et.al. | Minus | 3654893-3654678 |
| 333163 | Dunham, I. et.al. | Minus | 3665124-3664962 |
| 333165 | Dunham, I. et.al. | Minus | 3674052-3673905 |
| 333166 | Dunham, I. et.al. | Minus | 3694664-3694567 |
| 333170 | Dunham, I. et.al. | Minus | 3733394-3733299 |
| 333174 | Dunham, I. et.al. | Minus | 3764284-3764210 |
| 333188 | Dunham, I. et.al. | Minus | 3826990-3826863 |
| 333214 | Dunham, I. et.al. | Minus | 3966559-3966437 |
| 333232 | Dunham, I. et.al. | Minus | 4001551-4001365 |
| 333237 | Dunham, I. et.al. | Minus | 4003326-4003219 |
| 333239 | Dunham, I. et.al. | Minus | 4095861-4094462 |
| 333255 | Dunham, I. et.al. | Minus | 4297883-4297716 |
| 333259 | Dunham, I. et.al. | Minus | 4306769-4306639 |
| 333274 | Dunham, I. et.al. | Minus | 4389146-4388954 |
| 333290 | Dunham, I. et.al. | Minus | 4530734-4530554 |
| 333295 | Dunham, I. et.al. | Minus | 4549290-4549198 |
| 333296 | Dunham, I. et.al. | Minus | 4550766-4550644 |
| 333310 | Dunham, I, et.al. | Minus | 4637315-4637232 |
| 333311 | Dunham, I. et.al. | Minus | 4637933-4637844 |
| 333312 | Dunham, I. et.al. | Minus | 4638794-4638635 |
| 333313 | Dunham, I. et.al. | Minus | 4639397-4639277 |
| 333315 | Dunham, I. et.al. | Minus | 5405980-5405876 |
| 333318 | Dunham, I. et.al. | Minus | 4642636-4642564 |
| 333321 | Dunham, I. et.al. | Minus | 4649080-4648934 |
| 333327 | Dunham, I. et.al. | Minus | 4657947-4657828 |
| 333335 | Dunham, I. et.al. | Minus | 4672656-4672564 |
| 333337 | Dunham, I. et.al. | Minus | 4677930-4677841 |
| 333454 | Dunham, I. et.al. | Minus | 5137007-5136880 |
| 333458 | Dunham, I. et.al. | Minus | 5143942-5143806 |
| 333459 | Dunham, I. et.al. | Minus | 5144548-5144344 |
| 333470 | Dunham, I. et.al. | Minus | 5223319-5223088 |
| 333493 | Dunham, I. et.al. | Minus | 4637315-4637232 |
| 333496 | Dunham, I. et.al. | Minus | 5404643-5404523 |
| 333498 | Dunham, I. et.al. | Minus | 5405980-5405876 |
| 333510 | Dunham, I. et.al. | Minus | 5557628-5557469 |
| 333546 | Dunham, I. et.al. | Minus | 5886643-5886442 |
| 333561 | Dunham, I. et.al. | Minus | 5903659-5903590 |
| 333738 | Dunham, I. et.al. | Minus | 7552160-7552084 |
| 333780 | Dunham, I. et.al. | Minus | 7750367-7750277 |
| 333783 | Dunham, I. et.al. | Minus | 7751850-7751777 |
| 333818 | Dunham, I. et.al. | Minus | 7911959-7911762 |
| 333894 | Dunham, I. et.al. | Minus | 8188855-8188709 |
| 333897 | Dunham, I. et.al. | Minus | 8194390-8194284 |
| 333900 | Dunham, I. et.al. | Minus | 8200268-8200122 |
| 333909 | Dunham, I. et.al. | Minus | 8229639-8229477 |
| 333936 | Dunham, I. et.al. | Minus | 8512805-8512564 |
| 333944 | Dunham, I. et.al. | Minus | 8557051-8556936 |
| 334040 | Dunham, I. et.al. | Minus | 9342995-9342934 |
| 334154 | Dunham, I. et.al. | Minus | 10570714-10570572 |
| 334178 | Dunham, I. et.al. | Minus | 11755052-11754971 |
| 334188 | Dunham, I. et.al. | Minus | 11925963-11925834 |
| 334273 | Dunham, I. et.al. | Minus | 13265608-13265522 |
| 334282 | Dunham, I. et.al. | Minus | 13285293-13285178 |
| 334285 | Dunham, I. et.al. | Minus | 13289990-13289793 |
| 334286 | Dunham, I. et.al. | Minus | 13291759-13291569 |
| 334303 | Dunham, I. et.al. | Minus | 13454331-13454217 |
| 334305 | Dunham, I. et.al. | Minus | 13456310-13456209 |
| 334306 | Dunham, I. et.al. | Minus | 13461157-13461049 |
| 334320 | Dunham, I. et.al. | Minus | 13496857-13496717 |
| 334352 | Dunham, I. et.al. | Minus | 13675908-13675828 |
| 334353 | Dunham, I. et.al. | Minus | 13683722-13683596 |
| 334359 | Dunham, I. et.al. | Minus | 13728664-13728534 |
| 334363 | Dunham, I. et.al. | Minus | 13740004-13739812 |
| 334365 | Dunham, I. et.al. | Minus | 13742078-13741971 |
| 334399 | Dunham, I. et.al. | Minus | 14186289-14186163 |
| 334409 | Dunham, I. et.al. | Minus | 14195181-14195075 |
| 334414 | Dunham, I. et.al. | Minus | 14234033-14233932 |
| 334470 | Dunham, I. et.al. | Minus | 14389581-14389442 |
| 334483 | Dunham, I. et.al. | Minus | 14428355-14428281 |
| 334489 | Dunham, I. et.al. | Minus | 14455428-14454288 |
| 334498 | Dunham, I. et.al. | Minus | 14483789-14483700 |
| 334501 | Dunham, I. et.al. | Minus | 14487509-14487356 |
| 334502 | Dunham, I. et.al. | Minus | 14488605-14488526 |
| 334543 | Dunham, I. et.al. | Minus | 14834496-14834116 |
| 334622 | Dunham, I. et.al. | Minus | 15191678-15191609 |
| 334650 | Dunham, I. et.al. | Minus | 15371251-15371178 |
| 334680 | Dunham, I. et.al. | Minus | 15520047-15519887 |
| 334745 | Dunham, I. et.al. | Minus | 16049960-16049653 |
| 334756 | Dunham, I. et.al. | Minus | 16128678-16128528 |
| 334758 | Dunham, I. et.al. | Minus | 16132368-16132233 |
| 334761 | Dunham, I. et.al. | Minus | 16138424-16138319 |
| 334763 | Dunham, I. et.al. | Minus | 16148136-16148077 |
| 334784 | Dunham, I. et.al. | Minus | 16294548-16294360 |
| 334790 | Dunham, I. et.al. | Minus | 16307576-16307509 |
| 334793 | Dunham, I. et.al. | Minus | 16330748-16330681 |
| 334802 | Dunham, I. et.al. | Minus | 16413158-16413026 |
| 334820 | Dunham, I. et.al. | Minus | 16764338-16764249 |
| 334824 | Dunham, I. et.al. | Minus | 16857777-16857674 |
| 334832 | Dunham, I. et.al. | Minus | 17173957-17173760 |
| 334842 | Dunham, I. et.al. | Minus | 17464352-17464181 |
| 334844 | Dunham, I. et.al. | Minus | 17503891-17503768 |
| 334857 | Dunham, I. et.al. | Minus | 18488368-18488242 |
| 334927 | Dunham, I. et.al. | Minus | 19988711-19987853 |
| 334939 | Dunham, I. et.al. | Minus | 20131162-20131054 |
| 334951 | Dunham, I. et.al. | Minus | 20147708-20147502 |
| 334969 | Dunham, I. et.al. | Minus | 20188176-20188020 |
| 334972 | Dunham, I. et.al. | Minus | 20294734-20294611 |
| 335050 | Dunham, I. et.al. | Minus | 20884109-20883951 |
| 335078 | Dunham, I. et.al. | Minus | 21059529-21059458 |
| 335102 | Dunham, I. et.al. | Minus | 21313841-21313598 |
| 335105 | Dunham, I. et.al. | Minus | 21320563-21320440 |
| 335110 | Dunham, I. et.al. | Minus | 21334136-21333811 |
| 335111 | Dunham, I. et.al. | Minus | 21335946-21335809 |
| 335115 | Dunham, I. et.al. | Minus | 21388250-21388146 |
| 335116 | Dunham, I. et.al. | Minus | 21388573-21388414 |
| 335185 | Dunham, I. et.al. | Minus | 21651593-21651522 |
| 335186 | Dunham, I. et.al. | Minus | 21656436-21656338 |
| 335230 | Dunham, I. et.al. | Minus | 21899517-21898678 |
| 335236 | Dunham, I. et.al. | Minus | 21915016-21914870 |
| 335243 | Dunham, I. et.al. | Minus | 21933519-21933365 |
| 335249 | Dunham, I. et.al. | Minus | 21950851-21950669 |
| 335258 | Dunham, I. et.al. | Minus | 22043431-22043262 |
| 335261 | Dunham, I. et.al. | Minus | 22063937-22063772 |
| 335276 | Dunham, I. et.al. | Minus | 22154036-22153937 |
| 335279 | Dunham, I. et.al. | Minus | 22168834-22168638 |
| 335330 | Dunham, I. et.al. | Minus | 22556589-22556422 |
| 335331 | Dunham, I. et.al. | Minus | 22556823-22556708 |
| 335334 | Dunham, I. et.al. | Minus | 22560390-22560136 |
| 335346 | Dunham, I. et.al. | Minus | 22641097-22640918 |
| 335349 | Dunham, I. et.al. | Minus | 22661861-22661271 |
| 335611 | Dunham, I. et.al. | Minus | 25070825-25070706 |
| 335612 | Dunham, I. et.al. | Minus | 25072328-25072142 |
| 335671 | Dunham, I. et.al. | Minus | 25358629-25358533 |
| 335676 | Dunham, I. et.al. | Minus | 25395274-25395152 |
| 335680 | Dunham, I. et.al. | Minus | 25402437-25402361 |
| 335750 | Dunham, I. et.al. | Minus | 25732501-25731972 |
| 335752 | Dunham, I. et.al. | Minus | 25757026-25756890 |
| 335755 | Dunham, I. et.al. | Minus | 25763806-25763747 |
| 335767 | Dunham, I. et.al. | Minus | 25819547-25819218 |
| 335774 | Dunham, I. et.al. | Minus | 25883733-25883572 |
| 335777 | Dunham, I. et.al. | Minus | 25885770-25885599 |
| 335778 | Dunham, I. et.al. | Minus | 25886469-25886334 |
| 335797 | Dunham, I. et.al. | Minus | 25958182-25958030 |
| 335800 | Dunham, I. et.al. | Minus | 25985373-25985280 |
| 335818 | Dunham, I. et.al. | Minus | 26323886-26323744 |
| 335834 | Dunham, I. et.al. | Minus | 26391707-26391530 |
| 335840 | Dunham, I. et.al. | Minus | 26420596-26420538 |
| 335844 | Dunham, I. et.al. | Minus | 26433427-26433344 |
| 335848 | Dunham, I. et.al. | Minus | 26436727-26436621 |
| 335856 | Dunham, I. et.al. | Minus | 26662452-26662346 |
| 335887 | Dunham, I. et.al. | Minus | 26939225-26938782 |
| 335888 | Dunham, I. et.al. | Minus | 26943037-26942820 |
| 335889 | Dunham, I. et.al. | Minus | 26946988-26946901 |
| 335890 | Dunham, I. et.al. | Minus | 26949087-26948665 |
| 335893 | Dunham, I. et.al. | Minus | 26973898-26973747 |
| 335895 | Dunham, I. et.al. | Minus | 26975307-26975239 |
| 335896 | Dunham, I. et.al. | Minus | 26977639-26977558 |
| 335900 | Dunham, I. et.al. | Minus | 26980354-26980238 |
| 335907 | Dunham, I. et.al. | Minus | 27013352-27013273 |
| 335943 | Dunham, I. et.al. | Minus | 27446610-27446378 |
| 335956 | Dunham, I. et.al. | Minus | 27653729-27653635 |
| 335959 | Dunham, I. et.al. | Minus | 27682313-27682145 |
| 335962 | Dunham, I. et.al. | Minus | 27704276-27704144 |
| 336040 | Dunham, I. et.al. | Minus | 29036458-29036300 |
| 336044 | Dunham, I. et.al. | Minus | 29043828-29043727 |
| 336047 | Dunham, I. et.al. | Minus | 29050617-29050466 |
| 336068 | Dunham, I. et.al. | Minus | 29252077-29251969 |
| 336143 | Dunham, I. et.al. | Minus | 30135948-30135854 |
| 336158 | Dunham, I. et.al. | Minus | 30163730-30163610 |
| 336174 | Dunham, I. et.al. | Minus | 30241988-30241839 |
| 336223 | Dunham, I. et.al. | Minus | 30816306-30816195 |
| 336245 | Dunham, I. et.al. | Minus | 31420569-31420509 |
| 336274 | Dunham, I. et.al. | Minus | 32085468-32085303 |
| 336318 | Dunham, I. et.al. | Minus | 33364452-33364338 |
| 336326 | Dunham, I. et.al. | Minus | 33567328-33567201 |
| 336339 | Dunham, I. et.al. | Minus | 33798479-33798330 |
| 336340 | Dunham, I. et.al. | Minus | 33812069-33811915 |
| 336355 | Dunham, I. et.al. | Minus | 33874750-33874649 |
| 336392 | Dunham, I. et.al. | Minus | 34015868-34015736 |
| 336393 | Dunham, I. et.al. | Minus | 34016145-34015951 |
| 336394 | Dunham, I. et.al. | Minus | 34016457-34016298 |
| 336400 | Dunham, I. et.al. | Minus | 34023437-34023298 |
| 336402 | Dunham, I. et.al. | Minus | 34024090-34023981 |
| 336413 | Dunham, I. et.al. | Minus | 34046702-34046576 |
| 336424 | Dunham, I. et.al. | Minus | 34055549-34055491 |
| 336425 | Dunham, I. et.al. | Minus | 34058544-34058446 |
| 336437 | Dunham, I. et.al. | Minus | 34074154-34074090 |
| 336447 | Dunham, I. et.al. | Minus | 34198207-34197996 |
| 336449 | Dunham, I. et.al. | Minus | 34204707-34204577 |
| 336466 | Dunham, I. et.al. | Minus | 34213195-34213046 |
| 336492 | Dunham, I. et.al. | Minus | 34255578-34255437 |
| 336511 | Dunham, I. et.al. | Minus | 34277480-34277351 |
| 336512 | Dunham, I. et.al. | Minus | 34278373-34278275 |
| 336520 | Dunham, I. et.al. | Minus | 34319184-34319101 |
| 336522 | Dunham, I. et.al. | Minus | 34320169-34320056 |
| 336524 | Dunham, I. et.al. | Minus | 34321055-34320921 |
| 336527 | Dunham, I. et.al. | Minus | 34322071-34321966 |
| 336534 | Dunham, I. et.al. | Minus | 34326797-34326620 |
| 336536 | Dunham, I. et.al. | Minus | 34327678-34327538 |
| 336542 | Dunham, I. et.al. | Minus | 34331316-34331183 |
| 336556 | Dunham, I. et.al. | Minus | 34375244-34374907 |
| 336557 | Dunham, I. et.al. | Minus | 34375443-34375341 |
| 336558 | Dunham, I. et.al. | Minus | 34375825-34375698 |
| 336559 | Dunham, I. et.al. | Minus | 34376430-34376261 |
| 336560 | Dunham, I. et.al. | Minus | 34376814-34376596 |
| 336561 | Dunham, I. et.al. | Minus | 34377168-34376928 |
| 336597 | Dunham, I. et.al. | Minus | 7627912-7627757 |
| 336601 | Dunham, I. et.al. | Minus | 13265853-13265654 |
| 336642 | Dunham, I. et.al. | Minus | 1304281-1304212 |
| 336645 | Dunham, I. et.al. | Minus | 1351268-1351168 |
| 336662 | Dunham, I. et.al. | Minus | 2158060-2157993 |
| 336664 | Dunham, I. et.al. | Minus | 1993558-1993481 |
| 336676 | Dunham, I. et.al. | Minus | 2022565-2022497 |
| 336684 | Dunham, I. et.al. | Minus | 2158060-2157993 |
| 336686 | Dunham, I. et.al. | Minus | 2160698-2160486 |
| 336714 | Dunham, I. et.al. | Minus | 3094026-3093871 |
| 336719 | Dunham, I. et.al. | Minus | 3331631-3331503 |
| 336736 | Dunham, I. et.al. | Minus | 4093128-4093041 |
| 336744 | Dunham, I. et.al. | Minus | 4333001-4332848 |
| 336786 | Dunham, I. et.al. | Minus | 5419973-5419873 |
| 336793 | Dunham, I. et.al. | Minus | 5631345-5631237 |
| 336859 | Dunham, I. et.al. | Minus | 8201756-8201561 |
| 336863 | Dunham, I. et.al. | Minus | 8396673-8396425 |
| 336933 | Dunham, I. et.al. | Minus | 11760045-11759981 |
| 336942 | Dunham, I. et.al. | Minus | 12027537-12027455 |
| 336960 | Dunham, I. et.al. | Minus | 13267243-13267172 |
| 336969 | Dunham, I. et.al. | Minus | 13725722-13725643 |
| 336971 | Dunham, I. et.al. | Minus | 13732308-13732221 |
| 337003 | Dunham, I. et.al. | Minus | 15523541-15523422 |
| 337011 | Dunham, I. et.al. | Minus | 16106423-16106080 |
| 337070 | Dunham, I. et.al. | Minus | 19034423-19034321 |
| 337072 | Dunham, I. et.al. | Minus | 19077452-19077323 |
| 337086 | Dunham, I. et.al. | Minus | 19657011-19656881 |
| 337140 | Dunham, I. et.al. | Minus | 22649450-22649388 |
| 337193 | Dunham, I. et.al. | Minus | 24594969-24594874 |
| 337256 | Dunham, I. et.al. | Minus | 27659956-27659876 |
| 337278 | Dunham, I. et.al. | Minus | 28429017-28428848 |
| 337284 | Dunham, I. et.al. | Minus | 28491414-28491094 |
| 337293 | Dunham, I. et.al. | Minus | 28846334-28845873 |
| 337316 | Dunham, I. et.al. | Minus | 29657129-29656997 |
| 337326 | Dunham, I. et.al. | Minus | 30017199-30017069 |
| 337382 | Dunham, I. et.al. | Minus | 31233666-31233579 |
| 337392 | Dunham, I. et.al. | Minus | 31442311-31442229 |
| 337406 | Dunham, I. et.al. | Minus | 31864840-31864588 |
| 337412 | Dunham, I. et.al. | Minus | 31916487-31916312 |
| 337419 | Dunham, I. et.al. | Minus | 32021496-32021170 |
| 337436 | Dunham, I. et.al. | Minus | 32257869-32257739 |
| 337455 | Dunham, I. et.al. | Minus | 32434517-32434425 |
| 337509 | Dunham, I. et.al. | Minus | 33414613-33414498 |
| 337518 | Dunham, I. et.al. | Minus | 33796750-33796647 |
| 337529 | Dunham, I. et.al. | Minus | 34043668-34043546 |
| 337533 | Dunham, I. et.al. | Minus | 34193388-34193261 |
| 337539 | Dunham, I. et.al. | Minus | 34254490-34254322 |
| 337551 | Dunham, I. et.al. | Minus | 34524446-34524362 |
| 337553 | Dunham, I. et.al. | Minus | 24230-24160 |
| 337591 | Dunham, I. et.al. | Minus | 1006414-1006184 |
| 337592 | Dunham, I. et.al. | Minus | 1007791-1007634 |
| 337593 | Dunham, I. et.al. | Minus | 1009460-1009291 |
| 337607 | Dunham, I. et.al. | Minus | 1355719-1355637 |
| 337612 | Dunham, I. et.al. | Minus | 1570235-1570142 |
| 337635 | Dunham, I. et.al. | Minus | 2169690-2169569 |
| 337824 | Dunham, I. et.al. | Minus | 4559540-4559266 |
| 337825 | Dunham, I. et.al. | Minus | 4567155-4567005 |
| 337850 | Dunham, I. et.al. | Minus | 5077143-5076943 |
| 337854 | Dunham, I. et.al. | Minus | 5153435-5153272 |
| 337913 | Dunham, I. et.al. | Minus | 6149843-6149786 |
| 337915 | Dunham, I. et.al. | Minus | 5922748-5922690 |
| 337968 | Dunham, I. et.al. | Minus | 7095797-7095680 |
| 338010 | Dunham, I. et.al. | Minus | 7754282-7754184 |
| 338012 | Dunham, I. et.al. | Minus | 7761421-7761351 |
| 338017 | Dunham, I. et.al. | Minus | 7864521-7864401 |
| 338065 | Dunham, I. et.al. | Minus | 7235048-7234950 |
| 338094 | Dunham, I. et.al. | Minus | 9595602-9595440 |
| 338129 | Dunham, I. et.al. | Minus | 10915338-10915237 |
| 338132 | Dunham, I. et.al. | Minus | 10989617-10989530 |
| 338150 | Dunham, I. et.al. | Minus | 11478551-11478355 |
| 338157 | Dunham, I. et.al. | Minus | 11731444-11731375 |
| 338195 | Dunham, I. et.al. | Minus | 13484103-13483972 |
| 338255 | Dunham, I. et.al. | Minus | 15242294-15242231 |
| 338276 | Dunham, I. et.al. | Minus | 16109555-16109398 |
| 338431 | Dunham, I. et.al. | Minus | 19747608-19747496 |
| 338448 | Dunham, I. et.al. | Minus | 20151152-20151054 |
| 338451 | Dunham, I. et.al. | Minus | 20174286-20174193 |
| 338477 | Dunham, I. et.al. | Minus | 20821897-20821838 |
| 338534 | Dunham, I. et.al. | Minus | 21771238-21771170 |
| 338682 | Dunham, I. et.al. | Minus | 24800712-24800461 |
| 338684 | Dunham, I. et.al. | Minus | 24827522-24827428 |
| 338689 | Dunham. I. et.al. | Minus | 24893073-24892972 |
| 338695 | Dunham, I. et.al. | Minus | 25104153-25104016 |
| 338825 | Dunham, I. et.al. | Minus | 27664798-27664712 |
| 338842 | Dunham, I. et.al. | Minus | 27824238-27824079 |
| 338893 | Dunham, I. et.al. | Minus | 28491807-28491631 |
| 338904 | Dunham, I. et.al. | Minus | 28766345-28766253 |
| 338935 | Dunham, I. et.al. | Minus | 29071537-29071461 |
| 339022 | Dunham, I. et.al. | Minus | 30523414-30523289 |
| 339034 | Dunham, I. et.al. | Minus | 30621603-30621422 |
| 339190 | Dunham, I. et.al. | Minus | 32403103-32402985 |
| 339212 | Dunham, I. et.al. | Minus | 32494335-32494210 |
| 339213 | Dunham, I. et.al. | Minus | 32496590-32496440 |
| 339216 | Dunham, I. et.al. | Minus | 32504250-32504109 |
| 339233 | Dunham, I. et.al. | Minus | 32751331-32751238 |
| 339258 | Dunham, I. et.al. | Minus | 32934756-32934615 |
| 339262 | Dunham, I. et.al. | Minus | 32971258-32971090 |
| 339263 | Dunham, I. et.al. | Minus | 32974634-32974452 |
| 339265 | Dunham, I. et.al. | Minus | 32975943-32975806 |
| 339338 | Dunham, I. et.al. | Minus | 33468728-33468606 |
| 339396 | Dunham, I. et.al. | Minus | 34017306-34017205 |
| 339400 | Dunham, I. et.al. | Minus | 34045024-34044940 |
| 339425 | Dunham, I. et.al. | Minus | 34407911-34407798 |
| 325207 | 6552430 | Plus | 140049-140170 |
| 329568 | 3962490 | Plus | 36331-36750 |
| 329517 | 3983513 | Minus | 53197-53269 |
| 325313 | 5866865 | Minus | 27385-28192 |
| 325327 | 5866875 | Plus | 75189-75264 |
| 325317 | 5866878 | Minus | 156551-156649 |
| 325257 | 5866895 | Plus | 10867-10955 |
| 329632 | 6729060 | Plus | 192813-193017 |
| 325371 | 5866920 | Minus | 1035422-1035536 |
| 325375 | 5866920 | Minus | 1165503-1165810 |
| 325378 | 5866920 | Minus | 1187981-1188167 |
| 325469 | 6017034 | Plus | 286823-286991 |
| 325470 | 6017034 | Plus | 287578-287663 |
| 325576 | 6552443 | Minus | 137769-137894 |
| 325505 | 6682451 | Minus | 240852-240946 |
| 325543 | 6682452 | Plus | 151873-152057 |
| 329635 | 5302817 | Minus | 62522-62622 |
| 329636 | 5302817 | Minus | 64969-65078 |
| 325593 | 5866992 | Minus | 469726-469860 |
| 325675 | 5867014 | Plus | 955517-955711 |
| 325704 | 5867028 | Plus | 156198-156387 |
| 325682 | 6138923 | Plus | 370618-370763 |
| 325785 | 6381957 | Plus | 61849-62003 |
| 325666 | 6469822 | Plus | 16769-16857 |
| 325818 | 6682490 | Minus | 120278-120559 |
| 329777 | 6002090 | Minus | 191389-191479 |
| 329768 | 6015501 | Plus | 118315-118422 |
| 329759 | 6048280 | Minus | 37647-37730 |
| 329731 | 6065783 | Plus | 158772-158900 |
| 329687 | 6117856 | Minus | 22165-22288 |
| 329676 | 6272128 | Minus | 142207-142359 |
| 329667 | 6272129 | Plus | 101355-101745 |
| 329669 | 6272129 | Plus | 131223-131291 |
| 329670 | 6272129 | Plus | 131351-131495 |
| 329641 | 6468233 | Minus | 105995-106107 |
| 329791 | 6469354 | Minus | 131982-132089 |
| 325826 | 5867048 | Minus | 46361-46458 |
| 325829 | 5867052 | Plus | 232674-233060 |
| 329888 | 6067149 | Minus | 37227-37473 |
| 329893 | 6525313 | Minus | 166123-166791 |
| 329899 | 6563505 | Minus | 111058-111783 |
| 325988 | 5867064 | Plus | 17349-17606 |
| 325855 | 5867067 | Plus | 276141-276251 |
| 325999 | 5867073 | Plus | 149115-149192 |
| 326001 | 5867073 | Plus | 155223-155348 |
| 325886 | 5867087 | Pius | 194694-194915 |
| 325882 | 5867087 | Minus | 8178-8347 |
| 325905 | 5867104 | Plus | 78779-78876 |
| 325922 | 5867122 | Minus | 329063-329134 |
| 325937 | 5867132 | Minus | 152633-152902 |
| 325960 | 5867147 | Minus | 162506-162635 |
| 325961 | 5867147 | Minus | 165106-165209 |
| 325838 | 6552452 | Plus | 171451-171532 |
| 325839 | 6552452 | Plus | 181964-182037 |
| 325840 | 6552452 | Plus | 184380-184547 |
| 325844 | 6552453 | Minus | 14188-14332 |
| 325870 | 6682492 | Plus | 228209-228297 |
| 329984 | 4646193 | Minus | 139780-139890 |
| 329976 | 4878063 | Minus | 62584-62691 |
| 329935 | 6165200 | Minus | 69059-69127 |
| 329916 | 6223624 | Plus | 36396-37195 |
| 330021 | 6671889 | Plus | 120938-121032 |
| 330024 | 6671908 | Minus | 1005-1270 |
| 330028 | 6671908 | Minus | 30015-30144 |
| 326033 | 5867178 | Plus | 37261-37333 |
| 326036 | 5867178 | Minus | 120215-120273 |
| 326056 | 5867184 | Minus | 181553-181690 |
| 326116 | 5867193 | Plus | 45548-45604 |
| 326122 | 5867194 | Plus | 144397-144683 |
| 326138 | 5867203 | Minus | 179374-179436 |
| 326145 | 5867204 | Minus | 52599-52814 |
| 326180 | 5867211 | Minus | 182758-183222 |
| 326201 | 5867216 | Minus | 166168-166959 |
| 326207 | 5867222 | Plus | 48139-48219 |
| 326226 | 5867230 | Plus | 52644-52705 |
| 326233 | 5867232 | Plus | 124788-124863 |
| 326238 | 5867260 | Plus | 64282-64338 |
| 326241 | 5867260 | Minus | 181648-181916 |
| 326243 | 5867261 | Plus | 123838-123978 |
| 326251 | 5867263 | Minus | 82716-82822 |
| 326268 | 5867267 | Plus | 122114-122765 |
| 326124 | 5916395 | Plus | 407102-407560 |
| 326339 | 6056311 | Minus | 164637-165251 |
| 330049 | 4567182 | Minus | 314662-315210 |
| 326358 | 5867293 | Plus | 9122-9195 |
| 326365 | 5867297 | Minus | 96630-96764 |
| 326379 | 5867327 | Plus | 32299-32402 |
| 326382 | 5867327 | Minus | 50420-50503 |
| 326390 | 5867340 | Minus | 108814-110592 |
| 326424 | 5867369 | Minus | 168329-168409 |
| 326453 | 5867399 | Plus | 86222-86423 |
| 326472 | 5867404 | Plus | 293739-293940 |
| 326492 | 5867422 | Plus | 120768-120991 |
| 326533 | 5867441 | Minus | 532153-532280 |
| 330117 | 6015201 | Minus | 7340-7680 |
| 330115 | 6015202 | Plus | 11403-11677 |
| 330116 | 6015202 | Plus | 12109-12418 |
| 330095 | 6015278 | Plus | 15343-15814 |
| 330096 | 6015278 | Plus | 49370-49458 |
| 326644 | 5867559 | Plus | 42684-42819 |
| 326713 | 5867595 | Plus | 121511-121798 |
| 326745 | 5867611 | Plus | 127130-127318 |
| 326752 | 5867615 | Minus | 1214-1562 |
| 326753 | 5867616 | Plus | 12454-12511 |
| 326598 | 5867634 | Plus | 68955-69014 |
| 326667 | 6552455 | Plus | 142311-142441 |
| 326855 | 6552460 | Minus | 111390-111463 |
| 326812 | 6682504 | Plus | 189811-189941 |
| 327005 | 5867664 | Plus | 610847-610907 |
| 327008 | 5867664 | Plus | 928737-928811 |
| 326896 | 5867680 | Minus | 12032-12122 |
| 326904 | 5867684 | Minus | 9280-9606 |
| 326951 | 6004446 | Plus | 193812-193998 |
| 326941 | 6004446 | Plus | 62018-62896 |
| 326943 | 6004446 | Minus | 89242-89427 |
| 326928 | 6456782 | Minus | 291007-291219 |
| 326958 | 6469836 | Minus | 42952-43082 |
| 326959 | 6469836 | Minus | 43159-43301 |
| 327039 | 6531965 | Plus | 694486-694998 |
| 327127 | 6682520 | Plus | 41925-42083 |
| 330158 | 6580367 | Plus | 81966-82456 |
| 327204 | 5867447 | Plus | 165135-165239 |
| 327208 | 5867447 | Plus | 180805-180864 |
| 327266 | 5867462 | Minus | 82400-82615 |
| 327277 | 5867473 | Minus | 165616-165715 |
| 327289 | 5867481 | Plus | 49296-49536 |
| 327296 | 5867492 | Plus | 7627-8166 |
| 327237 | 5867544 | Minus | 59702-59813 |
| 327145 | 5867548 | Minus | 40482-40551 |
| 327333 | 5902477 | Minus | 141448-141609 |
| 327335 | 5902477 | Minus | 142979-143124 |
| 327343 | 6017017 | Minus | 12288-12395 |
| 327350 | 6249563 | Minus | 41890-41985 |
| 327358 | 6552411 | Minus | 3802-3950 |
| 327360 | 6552411 | Minus | 6255-6422 |
| 327409 | 5867750 | Minus | 52949-53011 |
| 327424 | 5867751 | Plus | 160442-160598 |
| 327430 | 5867754 | Plus | 1320-1403 |
| 327470 | 5867772 | Plus | 150910-150973 |
| 327460 | 6004455 | Plus | 175245-175343 |
| 327498 | 6017023 | Minus | 42178-42283 |
| 327509 | 6117815 | Minus | 54882-55053 |
| 327510 | 6117815 | Minus | 56824-56944 |
| 327512 | 6117815 | Plus | 176256-176325 |
| 327535 | 6525279 | Plus | 19105-19175 |
| 330163 | 6042042 | Minus | 20321-20385 |
| 330171 | 6648220 | Plus | 110889-111575 |
| 327579 | 5867824 | Minus | 37229-38335 |
| 327672 | 5867843 | Minus | 69649-69740 |
| 327629 | 5867872 | Plus | 49692-49811 |
| 327640 | 5867890 | Plus | 9448-9566 |
| 327649 | 5867899 | Plus | 205871-205927 |
| 327612 | 6525283 | Plus | 2747-2924 |
| 327718 | 6525284 | Plus | 86123-86186 |
| 327801 | 5867924 | Plus | 23239-23348 |
| 327762 | 5867961 | Minus | 50303-50439 |
| 327763 | 5867961 | Plus | 229347-229476 |
| 327776 | 5867964 | Minus | 164308-164486 |
| 327822 | 5867968 | Minus | 168886-169633 |
| 327823 | 5867968 | Minus | 170359-170433 |
| 327807 | 5867968 | Plus | 33745-33811 |
| 327845 | 6531962 | Plus | 193402-193549 |
| 330228 | 6013527 | Minus | 3719-3787 |
| 330190 | 6165182 | Plus | 36103-36243 |
| 328122 | 5868031 | Plus | 158474-158656 |
| 328132 | 5868038 | Minus | 126737-126839 |
| 328159 | 5868065 | Minus | 52957-53162 |
| 328168 | 5868071 | Plus | 60321-60479 |
| 328175 | 5868073 | Plus | 208-271 |
| 328217 | 5868096 | Minus | 3742-4362 |
| 327865 | 5868130 | Plus | 61503-62205 |
| 327866 | 5868131 | Minus | 2893-3046 |
| 327870 | 5868131 | Plus | 53558-53757 |
| 327879 | 5868142 | Minus | 77722-77793 |
| 327902 | 5868158 | Minus | 133339-133467 |
| 327918 | 5868165 | Plus | 547530-547591 |
| 327934 | 5868184 | Plus | 41830-42036 |
| 327959 | 5868210 | Minus | 46497-46682 |
| 327976 | 5868212 | Minus | 349301-349409 |
| 328020 | 5902482 | Minus | 556386-556652 |
| 328042 | 5902482 | Minus | 1985085-1986626 |
| 328008 | 5902482 | Plus | 296663-297151 |
| 330301 | 2905862 | Minus | 4420-5781 |
| 330299 | 2905881 | Minus | 1020-1382 |
| 328274 | 5868219 | Minus | 31244-31439 |
| 328595 | 5868224 | Plus | 148738-148967 |
| 328591 | 5868227 | Minus | 237647-237726 |
| 328668 | 5868254 | Minus | 10888-10984 |
| 328677 | 5868256 | Minus | 58708-58950 |
| 328687 | 5868262 | Plus | 624479-624585 |
| 328706 | 5868270 | Plus | 165501-165614 |
| 328711 | 5868271 | Minus | 97797-97990 |
| 328730 | 5868289 | Plus | 8068-8214 |
| 328732 | 5868289 | Plus | 37437-37550 |
| 328734 | 5868289 | Plus | 50559-50747 |
| 328752 | 5868298 | Minus | 114911-115087 |
| 328755 | 5868301 | Minus | 145959-146446 |
| 328761 | 5868302 | Minus | 239308-239412 |
| 328775 | 5868309 | Plus | 12845-12920 |
| 328784 | 5868309 | Minus | 74523-74604 |
| 328787 | 5868309 | Plus | 135772-135963 |
| 328809 | 5868327 | Plus | 91792-91849 |
| 328829 | 5868337 | Plus | 36309-36630 |
| 328280 | 5868352 | Plus | 160563-160631 |
| 328311 | 5868371 | Minus | 170560-170826 |
| 328318 | 5868373 | Plus | 414945-415620 |
| 328323 | 5868373 | Minus | 1080089-1080235 |
| 328348 | 5868383 | Minus | 260272-260379 |
| 328377 | 5868390 | Plus | 16947-17023 |
| 328436 | 5868417 | Plus | 203760-203904 |
| 328504 | 5868471 | Plus | 47064-47217 |
| 328506 | 5868471 | Plus | 60716-60830 |
| 328522 | 5868477 | Plus | 1972307-1972452 |
| 328525 | 5868482 | Plus | 12387-14313 |
| 328541 | 5868486 | Plus | 130956-131050 |
| 328662 | 6004473 | Plus | 1184773-1184855 |
| 328663 | 6004473 | Plus | 1185279-1186634 |
| 328803 | 6004475 | Minus | 291716-291948 |
| 328304 | 6004478 | Minus | 3884-3952 |
| 328927 | 5868500 | Minus | 428829-428893 |
| 328936 | 5868500 | Minus | 1352202-1352259 |
| 328939 | 6004481 | Minus | 131139-131320 |
| 328941 | 6456765 | Minus | 9817-9885 |
| 328948 | 6456765 | Plus | 28227-28413 |
| 328968 | 6456775 | Plus | 117442-118283 |
| 330316 | 6007576 | Minus | 119761-119931 |
| 330350 | 3056622 | Minus | 26413-26820 |
| 330351 | 3056622 | Minus | 27522-27614 |
| 330348 | 4544475 | Minus | 19855-19962 |
| 329034 | 5868561 | Minus | 32819-32939 |
| 329046 | 5868569 | Plus | 18971-19030 |
| 329053 | 5868574 | Plus | 426453-426541 |
| 329186 | 5868711 | Minus | 13108-13225 |
| 329237 | 5868729 | Plus | 133238-133339 |
| 329276 | 5868762 | Minus | 222629-222709 |
| 329333 | 5868806 | Plus | 392666-392746 |
| 329376 | 5868859 | Plus | 52356-52694 |
| 329384 | 5868869 | Minus | 116524-116662 |
| 329140 | 6017060 | Plus | 290842-290905 |
| 329317 | 6381976 | Plus | 614823-615209 |
| 329319 | 6381976 | Plus | 721390-721470 |
| 329129 | 6588026 | Plus | 144569-144712 |
| 329373 | 6682537 | Minus | 38950-39301 |
| 329412 | 6682553 | Minus | 68948-69041 |
| 329424 | 5868879 | Plus | 362196-362344 |
| 329446 | 5868886 | Plus | 84776-84899 |
| 329449 | 5868886 | Plus | 97697-97771 |

**TABLE 14: shows genes, including expression sequence tags, down-regulated in prostate tumor tissue compared to normal prostate tissue as analyzed using Affymetrix/Eos Hu02 GeneChip array. Shown are the ratios of "average" normal prostate to "average" prostate cancer tissues.**

| | | | | |
|---|---|---|---|---|
| Pkey: | | Unique Eos probeset identifier number | | |
| ExAccn: | | Exemplar Accession number, Genbank accession numbe | | |
| UnigeneID: | | Unigene number | | |
| Unigene Title: | | Unigene gene title | | |
| R1: | | Background subtracted normal prostate : prostate tumor tissue | | |
| | | | | |

| **Pkey** | **ExAccn** | **UnigeneID** | **Unigene Title** | **R1** |
|---|---|---|---|---|
| | | | | |
| 331328 | AA281133 | Hs.88808 | ESTs | 18.53 |
| 320875 | D60641 | Hs.131921 | ESTs | 14.55 |
| 300994 | AI251936 | Hs.146298 | ESTs | 12.17 |
| 323461 | AA418762 | Hs.190044 | ESTs | 10.55 |
| 301015 | AA947682 | Hs.217173 | ESTs; Weakly similar to Chain A; Cdc42hs-Gdp Complex [H.sapiens] | 10.17 |
| 319419 | AA543096 | Hs.13648 | ESTs; Highly similar to mitogen-induced [M.musculus] | 9.2 |
| 323486 | C05278 | Hs.166800 | ESTs; Moderately similar to [PYRUVATE DEHYDROGENASE (LIPOAMIDE)] KINASE ISOZYME 4 PRECURSOR [H.sapiens] | 8.87 |
| 324882 | AW419080 | Hs.250645 | ESTs | 8 |
| 330569 | U57796 | Hs.57679 | zinc finger protein 192 | 7.88 |
| 330126 | | | CH.21_p2 gi\|6093735 | 7.8 |
| 316265 | AA737400 | Hs.142230 | ESTs | 7.7 |
| 323045 | AA148950 | Hs.188836 | ESTs | 7.64 |
| 320668 | R58399 | Hs.146217 | ESTs | 7.4 |
| 330769 | AA465192 | Hs.16514 | ESTs | 7.15 |
| 312614 | AI766732 | Hs.201194 | ESTs | 7 |
| 314790 | AW341754 | Hs.189305 | ESTs | 6.83 |
| 309979 | AW452118 | Hs.257533 | EST | 6.74 |
| 314236 | AA743396 | Hs.189023 | ESTs | 6.49 |
| 329192 | | | CH.X_hs gi\|5868716 | 6.1 |
| 324307 | AA627642 | Hs.4994 | transducer of ERBB2; 2 (TOB2) | 5.99 |
| 303685 | AW500106 | | EST cluster (not in UniGene) with exon hit | 5.82 |
| 314921 | AW452382 | Hs.257564 | ESTs | 5.8 |
| 315840 | AA679001 | Hs.192221 | ESTs | 5.68 |
| 332776 | AA034364 | Hs.256551 | ESTs; Weakly similar to !!!! ALU CLASS B WARNING ENTRY !!!! [H.sapiens] | 5.43 |
| 313533 | AW298141 | Hs.157975 | ESTs | 5.4 |
| 303494 | F30712 | | EST cluster (not in UniGene) with exon hit | 5.35 |
| 317490 | AI627358 | Hs.148367 | ESTs | 5.31 |
| 332546 | D84454 | Hs.21899 | solute carrier family 35 (UDP-galactose transporter); member2 | 5.25 |
| 334719 | | | CH22_FGENES.421_30 | 5.25 |
| 300679 | AA813958 | Hs.207727 | ESTs; Moderately similar to KIAA0071 [H.sapiens] | 5.22 |
| 311811 | AI625304 | Hs.190312 | ESTs | 5.22 |
| 315310 | AW511298 | Hs.256067 | ESTs | 5.19 |
| 312871 | H86747 | Hs.227602 | KIAA1116 protein | 5.11 |
| 324715 | AI739168 | | EST cluster (not in UniGene) | 4.97 |
| 313870 | AW206435 | Hs.146057 | ESTs | 4.97 |
| 321453 | N50080 | Hs.117827 | ESTs | 4.78 |
| 316160 | AW197887 | Hs.253353 | ESTs | 4.63 |
| 313833 | AA766825 | | EST cluster (not in UniGene) | 4.58 |
| 315850 | AW270550 | Hs.116957 | ESTs | 4.53 |
| 303124 | AF161350 | | EST cluster (not in UniGene) with exon hit | 4.46 |
| 323346 | AL134932 | Hs.143607 | ESTs | 4.4 |
| 301383 | AA913591 | Hs.126480 | ESTs | 4.35 |
| 324513 | AW501678 | Hs.164577 | ESTs | 4.28 |
| 303480 | AA331906 | | EST cluster (not in UniGene) with exon hit | 4.25 |
| 323591 | AA301270 | | EST cluster (not in UniGene) | 4.22 |
| 313603 | AW468119 | | EST cluster (not in UniGene) | 4.2 |
| 317863 | AI733395 | Hs.129124 | ESTs | 4.1 |
| 312381 | R42049 | Hs.195473 | ESTs | 4.08 |
| 317514 | AW451570 | Hs.126850 | ESTs | 4.03 |
| 319750 | AA621606 | Hs.117956 | ESTs | 4.03 |
| 322520 | T55958 | | EST cluster (not in UniGene) | 4 |
| 314754 | AW026761 | Hs.134374 | ESTs | 4 |
| 316088 | AI990652 | Hs.208973 | ESTs | 4 |
| 318473 | AI939339 | Hs.146883 | ESTs | 3.96 |
| 307848 | AI364186 | | EST singleton (not in UniGene) with exon hit | 3.95 |
| 300730 | AW449204 | Hs.257125 | ESTs | 3.94 |
| 303034 | W60843 | Hs.31570 | ESTs | 3.93 |
| 324668 | AI679131 | Hs.201424 | ESTs | 3.9 |
| 324674 | AA541323 | Hs.115831 | ESTs | 3.88 |
| 300547 | N53442 | Hs.143443 | ESTs | 3.83 |
| 316100 | AW203986 | Hs.213003 | ESTs | 3.79 |
| 314801 | AA481027 | Hs.127336 | ESTs; Weakly similar to ORF YGR245c [S.cerevisiae] | 3.75 |
| 320856 | D59945 | | EST cluster (not in UniGene) | 3.74 |
| 313188 | AI039702 | Hs.179573 | collagen; type I; alpha 2 | 3.73 |
| 314187 | AA804409 | Hs.118920 | ESTs | 3.73 |
| 311826 | AA765470 | Hs.122826 | ESTs | 3.7 |
| 302358 | D81150 | | EST cluster (not in UniGene) with exon hit | 3.68 |
| 311441 | Z38720 | Hs.151014 | ESTs | 3.66 |
| 321914 | AA011603 | | EST cluster (not in UniGene) | 3.59 |
| 332216 | H95082 | Hs.102332 | EST | 3.52 |
| 324771 | AA631739 | | EST cluster (not in UniGene) | 3.5 |
| 323691 | AA317561 | | EST cluster (not in UniGene) | 3.49 |
| 303525 | AW516519 | Hs.115130 | ESTs | 3.47 |
| 309709 | AW242630 | | EST singleton (not in UniGene) with exon hit | 3.46 |
| 300038 | | | AFFX control: MurlL4 | 3.38 |
| 316526 | AI088192 | Hs.135474 | ESTs; Weakly similar to ATP-DEPENDENT RNA HELICASE A [H.sapiens] | 3.36 |
| 313029 | AA731520 | Hs.170504 | ESTs | 3.35 |
| 304356 | AA196027 | Hs.195188 | glyceraldehyde-3-phosphate dehydrogenase | 3.34 |
| 314610 | AI948688 | Hs.191805 | ESTs | 3.33 |
| 329815 | | | CH.14_p2 gi\|6624888 | 3.32 |
| 314949 | AI745387 | Hs.239124 | ESTs | 3.31 |
| 300598 | N53574 | Hs.158932 | ESTs | 3.3 |
| 329218 | | | CH.X_hs gi\|5868726 | 3.28 |
| 315706 | AW440742 | Hs.155556 | ESTs | 3.28 |
| 303751 | AW503637 | | EST cluster (not in UniGene) with exon hit | 3.25 |
| 307783 | AI347274 | | EST singleton (not in UniGene) with exon hit | 3.25 |
| 321414 | AA324975 | Hs.128993 | ESTs; Weakly similar to KIAA0465 protein [H.sapiens] | 3025 |
| 312187 | AA700439 | Hs.188490 | ESTs | 3.25 |
| 334061 | | | CH22_FGENES.327_14 | 3.23 |
| 336036 | | | CH22_FGENES.678_7 | 3.23 |
| 321477 | H67818 | Hs.222059 | ESTs | 3.21 |
| 315760 | AW139383 | Hs.245437 | ESTs | 3.2 |
| 316733 | AA811713 | Hs.163222 | ESTs | 3.2 |
| 300855 | AW235248 | Hs.79828 | ESTs | 3.2 |
| 323611 | AA304986 | Hs.145704 | ESTs | 3.19 |
| 314138 | AA740616 | | EST cluster (not in UniGene) | 3.17 |
| 316774 | AA814859 | | EST cluster (not in UniGene) | 3.16 |
| 308884 | AI833131 | Hs.179100 | ESTs | 3.11 |
| 331317 | AA258222 | Hs.87757 | ESTs | 3.1 |
| 317221 | AI989538 | Hs.191074 | ESTs | 3.08 |
| 316386 | AA749062 | Hs.180285 | ESTs | 3.08 |
| 321040 | H26953 | | EST cluster (not in UniGene) | 3.08 |
| 308828 | AI824829 | | EST singleton (not in UniGene) with exon hit | 3.08 |
| 300778 | AA236233 | Hs.188716 | ESTs | 3.07 |
| 316667 | AW015940 | Hs.232234 | ESTs | 3.07 |
| 324614 | AW503101 | | EST cluster (not in UniGene) | 3.07 |
| 316468 | AW293046 | Hs.255158 | ESTs | 3.07 |
| 300671 | AI239706 | Hs.189886 | ESTs | 3.06 |
| 314301 | AW297967 | Hs.188181 | ESTs | 3.05 |
| 312335 | AW043620 | Hs236993 | ESTs | 3.03 |
| 322957 | AA247755 | | EST cluster (not in UniGene) | 3.01 |
| 316848 | AA830053 | Hs.126798 | ESTs | 3.01 |
| 313473 | AA009660 | Hs.251948 | ESTs; Moderately similar to T07D3.7 [C.elegans] | 2.99 |
| 318518 | T27119 | | EST cluster (not in UniGene) | 2.98 |
| 313383 | AI076370 | Hs.134037 | ESTs | 2.97 |
| 331389 | AA458637 | Hs.152207 | ESTs | 2.96 |
| 304257 | AA053294 | | EST singleton (not in UniGene) with exon hit | 2.95 |
| 309917 | AW340014 | | EST singleton (not in UniGene) with exon hit | 2.95 |
| 319661 | H08035 | Hs.21398 | ESTs; Moderately similar to PUTATIVE GLUCOSAMINE-6-PHOSPHATE | |
| | | | ISOMERASE [H.sapiens] | 2.95 |
| 321253 | AI699484 | | EST cluster (not in UniGene) | 2.93 |
| 321193 | AA149508 | Hs.103288 | ESTs | 2.93 |
| 332864 | | | CH22_FGENES.28_4 | 2.92 |
| 300027 | | | | |
| | M11507 | | AFFX control: transferrin receptor | 2.91 |
| 324330 | AA884766 | | EST cluster (not in UniGene) | 2.88 |
| 320014 | AA137114 | Hs.170291 | ESTs | 2.88 |
| 333916 | | | CH22_FGENES.296_5 | 2.88 |
| 318885 | Z43272 | | EST cluster (not in UniGene) | 2.87 |
| 318146 | AI040125 | Hs.150521 | ESTs | 2.87 |
| 323348 | AA233056 | Hs.191518 | ESTs | 2.85 |
| 305703 | AA825148 | Hs.21229 | F-box protein Fbw1b | 2.84 |
| 335862 | | | CH22_FGENES.629_7 | 2.83 |
| 317672 | AW205409 | Hs.127748 | ESTs | 2.82 |
| 323416 | AI610397 | Hs.159560 | ESTs | 2.81 |
| 312652 | AI419909 | Hs.160994 | ESTs | 2.81 |
| 324094 | AA382603 | | EST cluster (not in UniGene) | 2.81 |
| 319761 | R84237 | | EST cluster (not in UniGene) | 2.8 |
| 317013 | AA864468 | Hs.135646 | ESTs | 2.8 |
| 317383 | AA913887 | Hs.126511 | ESTs | 2.78 |
| 314659 | AW277121 | Hs.254881 | ESTs | 2.78 |
| 312479 | AI950844 | Hs.128738 | ESTs; Weakly similar to non-lens beta gamma-crystallin like protein [H.sapiens] | 2.77 |
| 332808 | | | CH22_FGENES.7_10 | 2.75 |
| 311824 | AW293826 | Hs.250610 | ESTs | 2.75 |
| 321992 | C06003 | Hs.116456 | ESTs | 2.73 |
| 316074 | AW517542 | Hs.208382 | ESTs | 2.73 |
| 309839 | AW296076 | | EST singleton (not in UniGene) with exon hit | 2.73 |
| 312071 | AA683529 | Hs.143119 | ESTs | 2.73 |
| 312684 | AW294020 | Hs.117721 | ESTs | 2.72 |
| 332668 | AA062971 | Hs.181161 | ESTs; Weakly similar to INHIBITOR OF APOPTOSIS PROTEIN 1 [M.musculus] | 2.72 |
| 322139 | H53744 | | EST cluster (not in UniGene) | 2.72 |
| 304168 | H77679 | | EST singleton (not in UniGene) with exon hit | 2.72 |
| 325602 | | | CH.13_hs gi\|5866994 | 2.71 |
| 319885 | R59096 | Hs.136698 | ESTs | 2.71 |
| 300611 | N75450 | | EST cluster (not in UniGene) with exon hit | 2.71 |
| 316854 | AA831215 | Hs.159066 | ESTs; Weakly similar to predicted using Genefinder [C.elegans] | 2.69 |
| 318208 | AI091458 | Hs.134559 | ESTs | 2.68 |
| 331623 | R38715 | Hs.153529 | Homo sapiens clone 24540 mRNA sequence | 2.68 |
| 324616 | AI823999 | Hs.162000 | ESTs | 2.68 |
| 304968 | AA614308 | | EST singleton (not in UniGene) with exon hit | 2.67 |
| 314912 | AI431345 | Hs.161784 | ESTs | 2.67 |
| 300767 | AW193466 | Hs.136525 | ESTs | 2.67 |
| 313463 | AI057369 | Hs.122536 | ESTs | 2.65 |
| 320600 | AA135565 | Hs.250739 | ESTs | 2.65 |
| 301180 | AI308989 | Hs.156939 | ESTs | 2.65 |
| 324825 | AA704457 | Hs.255738 | ESTs; Moderately similar to gag [H.sapiens] | 2.65 |
| 300336 | AW292417 | Hs.255074 | ESTs; Moderately similar to high-dsk human papilloma viruses E6 | |
| | | | oncoproteins targeted protein E6TP1 alpha [H.sapiens] | 2.64 |
| 317850 | N29974 | | EST cluster (not in UniGene) | 2.64 |
| 339047 | | | CH22_DA59H18.GENSCAN.28-7 | 2.64 |
| 324580 | AA492588 | | EST cluster (not in UniGene) | 2.63 |
| 321142 | AI817933 | Hs.209584 | ESTs | 2.62 |
| 319478 | R06841 | | EST cluster (not in UniGene) | 2.62 |
| 300793 | AI248571 | Hs.186837 | ESTs | 2.61 |
| 313733 | AA836116 | | EST cluster (not in UniGene) | 2.6 |
| 326505 | | | CH.19_hs gi\|5867435 | 2.6 |
| 314987 | AW015506 | Hs.130730 | ESTs | 2.6 |
| 303114 | AF090948 | | EST cluster (not in UniGene) with exon hit | 2.59 |
| 318709 | H24244 | Hs.240763 | ESTs; Weakly similar to /prediction | 2.58 |
| 312878 | AI209108 | Hs.143946 | ESTs | 2.57 |
| 329224 | | | CH.X_hs gi\|5868728 | 2.56 |
| 328018 | | | CH.06_hs gi\|5902482 | 2.56 |
| 323231 | AA324437 | Hs.177230 | ESTs | 2.55 |
| 312887 | AW157377 | Hs.132910 | ESTs | 2.55 |
| 315183 | AW136134 | Hs.220277 | ESTs | 2.55 |
| 300259 | AI479011 | Hs.170783 | ESTs | 2.54 |
| 313240 | AI743261 | Hs.131860 | ESTs | 2.54 |
| 316697 | AW293174 | Hs.252627 | ESTs | 2.53 |
| 313966 | AI807551 | Hs.189061 | ESTs | 2.53 |
| 331263 | AA015718 | | ze31a12.s1 Soares retina N2b4HR Homo sapiens cDNA clone | |
| | | | IMAGE:38574 3', mRNA sequence | 2.51 |
| 310683 | AW055233 | Hs.160870 | ESTs | 2.5 |
| 302566 | AA085996 | Hs.248572 | Human PAC clone DJ404F18 from Xq23 | 2.5 |
| 302697 | AJ001408 | | EST cluster (not in UniGene) with exon hit | 2.5 |
| 308362 | AI613519 | | EST singleton (not in UniGene) with exon hit | 2.49 |
| 322347 | AF086538 | | EST cluster (not in UniGene) | 2.49 |
| 316240 | AA974253 | Hs.120319 | ESTs | 2.49 |
| 323208 | AA203415 | Hs.136200 | ESTs | 2.48 |
| 321643 | W76005 | Hs.32094 | ESTs | 2.48 |
| 330723 | AA243617 | Hs.31082 | ESTs; Highly similar to db83 [R.norvegicus] | 2.48 |
| 323455 | AA256675 | Hs.200438 | ESTs; Weakly similar to atypical PKC specific binding protein [R.norvegicus] | 2.47 |
| 308383 | AI624497 | | EST singleton (not in UniGene) with exon hit | 2.47 |
| 328744 | | | CH.07_hs gi\|5868290 | 2.47 |
| 332344 | W45574 | Hs.252497 | ESTs | 2.47 |
| 328121 | | | CH.06_hs gi\|5868031 | 2.47 |
| 321915 | AI670955 | Hs.200151 | ESTs | 2.46 |
| 314954 | AA521381 | Hs.187726 | ESTs | 2.45 |
| 302821 | AA188868 | Hs.173933 | ESTs; Weakly similar to NUCLEAR FACTOR 1/X [H.sapiens] | 2.45 |
| 329454 | | | CH.Y_hs gi\|5868887 | 2.45 |
| 336605 | | | CH22_FGENES.420_4 | 2.45 |
| 300664 | AI444628 | Hs.256809 | ESTs | 2.44 |
| 323362 | AL135067 | Hs.117182 | ESTs | 2.44 |
| 300024 | M10098 | | AFFX control:18S ribosomal RNA | 2.44 |
| 325026 | AI671168 | Hs.12285 | ESTs | 2.43 |
| 324510 | AI148353 | Hs.120849 | ESTs | 2.43 |
| 313389 | AI765182 | Hs.119903 | ESTs | 2.43 |
| 301309 | M78276 | Hs.255917 | ESTs | 2.43 |
| 313570 | AA041455 | Hs.209312 | ESTs | 2.43 |
| 316504 | AW135854 | Hs.132458 | ESTs | 2.42 |
| 319401 | R01342 | | EST cluster (not in UniGene) | 2.42 |
| 312827 | AI744361 | Hs.205591 | ESTs; Weakly similar to zinc finger protein Png-1 [M.musculus] | 2.42 |
| 327871 | | | CH.06_hs gi\|5868131 | 2.41 |
| 337173 | | | CH22_FGENES.565-3 | 2.41 |
| 302948 | AA465635 | | EST cluster (not in UniGene) with exon hit | 2.41 |
| 324303 | AL118754 | | EST cluster (not in UniGene) | 2.4 |
| 315527 | AI791138 | Hs.116768 | ESTs | 2.4 |
| 315979 | AA830515 | Hs.222917 | ESTs | 2.4 |
| 331310 | AA253351 | Hs.44439 | STAT induced STAT inhibitor-4 | 2.4 |
| 321095 | AA017595 | Hs.32844 | ESTs | 2.4 |
| 308561 | Al701559 | | EST singleton (not in UniGene) with exon hit | 2.39 |
| 313035 | N36417 | Hs.144928 | ESTs | 2.37 |
| 322114 | AA643791 | Hs.191740 | ESTs | 2.37 |
| 313671 | W49823 | Hs.145553 | ESTs | 2.37 |
| 303211 | AA099548 | Hs.191436 | ESTs; Highly similar to dJ1118D24.4 [H.sapiens] | 2.37 |
| 301256 | AA932948 | | EST cluster (not in UniGene) with exon hit | 2.36 |
| 338165 | | | CH22_EM:AC005500.GENSCAN.212-3 | 2.36 |
| 324692 | AA557952 | | EST cluster (not in UniGene) | 2.35 |
| 318587 | AA779704 | Hs.168830 | ESTs | 2.35 |
| 312378 | R41582 | Hs.109219 | retinal degeneration B beta | 2.35 |
| 318625 | T48446 | Hs.193162 | ESTs | 2.35 |
| 305181 | AA663726 | Hs.116922 | EST | 2.35 |
| 300815 | AA286678 | | EST cluster (not UniGene) with exon hit | 2.34 |
| 324063 | AW292740 | Hs.254815 | ESTs | 2.34 |
| 315859 | AA682305 | Hs.133268 | ESTs | 2.33 |
| 305092 | AA642912 | | EST singleton (not in UniGene) with exon hit | 2.33 |
| 306598 | AI000320 | | EST singleton (not in UniGene) with exon hit | 2.33 |
| 300307 | AI651016 | Hs.246311 | ESTs | 2.33 |
| 321348 | Z49979 | | EST cluster (not in UniGene) | 2.33 |
| 325112 | AI903770 | Hs.124344 | ESTs | 2.32 |
| 336679 | | | CH22_FGENES.43-7 | 2.32 |
| 321383 | AJ002574 | | EST cluster (not in UniGene) | 2.32 |
| 337357 | | | CH22_FGENES.730-6 | 2.31 |
| 300680 | AW468066 | Hs.257712 | ESTs; Weakly similar to KIAA0986 protein [H.sapiens] | 2.31 |
| 327120 | | | CH.21_hs gi\|6531970 | 2.31 |
| 302761 | AW250553 | | EST cluster (not in UniGene) with exon hit | 2.3 |
| 312132 | AI475490 | Hs.170577 | ESTs | 2.3 |
| 315639 | AA827652 | | EST cluster (not in UniGene) | 2.3 |
| 312189 | T95594 | Hs.187435 | ESTs | 2.3 |
| 306537 | AA991705 | | EST singleton (not in UniGene) with exon hit | 2.3 |
| 327061 | | | CH.21_hs gi\|6531965 | 2.3 |
| 315391 | AA759098 | Hs.192007 | ESTs | 2.3 |
| 322384 | AI968646 | Hs.33862 | ESTs | 2.29 |
| 323206 | AA203339 | Hs.220750 | ESTs | 2.29 |
| 318110 | AI680915 | Hs.201379 | ESTs | 2.28 |
| 335250 | | | CH22_FGENES.516_11 | 2.28 |
| 331696 | Z38907 | Hs.91662 | KIAA0888 protein | 2.28 |
| 318327 | AW294013 | Hs.200942 | ESTs | 2.28 |
| 324980 | AA969121 | Hs.254296 | ESTs | 2.28 |
| 319429 | AI608881 | Hs.11482 | ESTs; Highly similar to junctional adhesion molecule [H.sapiens] | 2.28 |
| 310601 | AI970543 | Hs.192605 | ESTs | 2.28 |
| 318905 | Z43395 | | EST cluster (not in UniGene) | 2.28 |
| 323442 | AA252753 | Hs.164039 | ESTs | 2.27 |
| 304428 | AA342250 | Hs.99819 | ubiquitin specific protease 16 | 2.27 |
| 313352 | AW292127 | Hs.144758 | ESTs | 2.27 |
| 316491 | AA766025 | Hs.238794 | EST | 2.27 |
| 317751 | AI697668 | Hs.202241 | ESTs | 2.26 |
| 314136 | AA229781 | Hs.221962 | ESTs | 2.26 |
| 306665 | AI004614 | Hs.130577 | EST | 2.26 |
| 303946 | AW474196 | Hs.221604 | ESTs | 2.25 |
| 313435 | AA769123 | | EST cluster (not in UniGene) | 2.25 |
| 317679 | AA968799 | Hs.150289 | ESTs | 2.25 |
| 322370 | AA330095 | | EST cluster (not in UniGene) | 2.25 |
| 306620 | AI000929 | | EST singleton (not in UniGene) with exon hit | 2.24 |
| 329109 | | | CH.X_hs gi\|5868626 | 2.24 |
| 311043 | AI871209 | Hs.177128 | ESTs | 2.24 |
| 300228 | AI458372 | Hs.158748 | ESTs; Weakly similar to synapsin Ib [M.musculus] | 2.24 |
| 307223 | AI193698 | Hs.184776 | ribosomal protein L23a | 2.24 |
| 309023 | AI888045 | | EST singleton (not in UniGene) with exon hit | 2.23 |
| 310749 | AI493675 | Hs.170332 | ESTs | 2.23 |
| 316769 | AI914939 | Hs.212184 | ESTs | 2.22 |
| 320409 | AA356195 | | EST cluster (not in UniGene) | 2.21 |
| 333149 | | | CH22_FGENES.87_8 | 2.21 |
| 324951 | M86125 | Hs.137487 | ESTs | 221 |
| 321939 | A1791617 | Hs.145068 | ESTs | 2.2 |
| 320594 | AI863952 | Hs.169436 | arginyltransferase 1 | 2.2 |
| 320722 | R67430 | Hs.172787 | ESTs | 2.2 |
| 321781 | D78667 | | EST cluster (not in UniGene) | 2.2 |
| 328903 | | | CH.08_hs gi\|5868514 | 2.2 |
| 303889 | T19204 | | EST cluster (not in UniGene) with exon hit | 2.2 |
| 325045 | T08845 | | EST cluster (not in UniGene) | 2.2 |
| 312828 | AI865455 | Hs.211818 | ESTs; Moderately similar to !!!! ALU SUBFAMILY J WARNING ENTRY !!!! [H.sapiens] | 2.19 |
| 335109 | | | CH22_FGENES.494_15 | 2.18 |
| 330878 | AA131471 | Hs.71440 | ESTs | 2.18 |
| 311289 | AI971362 | Hs.231945 | ESTs | 2.18 |
| 304608 | AA513456 | | EST singleton (not in UniGene) with exon hit | 2.18 |
| 337393 | | | CH22_FGENES.747-4 | 2.18 |
| 332812 | | | CH22_FGENES.7_14 | 2.18 |
| 327665 | | | CH.04_hs gi\|5867839 | 2.18 |
| 314581 | AW504859 | Hs.237849 | ESTs | 2.17 |
| 326508 | | | CH.19_hs gi\|6682496 | 2.17 |
| 301242 | AW161535 | Hs.258803 | ESTs | 2.17 |
| 312780 | AI765651 | Hs.172900 | ESTs | 2.17 |
| 315954 | AW276810 | Hs.254859 | ESTs | 2.16 |
| 311179 | AI880843 | Hs.223333 | ESTs | 2.16 |
| 315320 | AI084182 | Hs.186895 | ESTs | 2.16 |
| 313017 | AI015203 | Hs.118015 | ESTs | 2.16 |
| 312430 | AW139117 | Hs.117494 | ESTs | 2.15 |
| 300864 | AA406539 | Hs.190958 | ESTs | 2.15 |
| 314753 | AA463262 | | EST cluster (not in UniGene) | 2.15 |
| 322574 | AF156548 | | EST cluster (not in UniGene) | 2.15 |
| 321409 | C03864 | | EST cluster (not in UniGene) | 2.15 |
| 321205 | AA002047 | | EST cluster (not in UniGene) | 2.14 |
| 320406 | AA353895 | Hs.152983 | HUS1 (S.pombe) checkpoint homolog | 2.14 |
| 337646 | | | CH22_EM:AC000097.GENSCAN.11-2 | 2.13 |
| 303084 | AF174008 | | EST cluster (not in UniGene) with exon hit | 2.13 |
| 312185 | AA654772 | Hs.186564 | ESTs | 2.13 |
| 306813 | AI066544 | | EST singleton (not in UniGene) with exon hit | 2.13 |
| 314465 | AA602917 | Hs.156974 | ESTs | 2.12 |
| 318168 | AI821782 | Hs.220587 | ESTs; Moderately similar to !!!! ALU SUBFAMILY SC WARNING ENTRY !!!! [H.sapiens] | 2.12 |
| 315990 | AI800041 | Hs.190555 | ESTs | 2.11 |
| 320712 | R66867 | | EST cluster (not in UniGene) | 2.11 |
| 318487 | AI167877 | Hs.143716 | ESTs | 2.11 |
| 317462 | AW015206 | Hs.178784 | ESTs | 2.11 |
| 304384 | AA235482 | Hs.62954 | ferritin; heavy polypeptide 1 | 2.11 |
| 314544 | AA399018 | Hs.250835 | ESTs | 2.1 |
| 319881 | T72744 | | EST cluster (not in UniGene) | 2.1 |
| 328078 | | | CH.06_hs gi\|5868008 | 2.1 |
| 317354 | AW090770 | Hs.192271 | ESTs | 2.1 |
| 308617 | AI738720 | | EST singleton (not in UniGene) with exon hit | 2.09 |
| 311568 | AW439969 | Hs.218177 | ESTs | 2.09 |
| 313605 | AI761786 | Hs.204674 | ESTs | 2.09 |
| 314289 | AA848118 | Hs.221216 | ESTs | 2.08 |
| 332933 | | | CH22_FGENES.38_7 | 2.08 |
| 325498 | | | CH.12_hs gi\|5866967 | 2.08 |
| 313659 | AW296067 | Hs.124106 | ESTs | 2.08 |
| 324596 | AW149321 | Hs.105411 | ESTs | 2.08 |
| 324783 | AA640770 | | EST cluster (not in UniGene) | 2.07 |
| 302696 | AA347452 | | EST cluster (not in UniGene) with exon hit | 2.07 |
| 313418 | AW450674 | Hs.114696 | ESTs | 2.06 |
| 326920 | | | CH.21_hs gi\|6456782 | 2.06 |
| 327574 | | | CH.03_hs gi\|5867818 | 2.06 |
| 323207 | AI052795 | Hs.192201 | ESTs | 2.06 |
| 303753 | AW503733 | Hs.170315 | ESTs | 2.05 |
| 305235 | AA670480 | | EST singleton (not in UniGene) with exon hit | 2.05 |
| 316055 | AA693880 | | EST cluster (not in UniGene) | 2.05 |
| 317194 | AW445167 | Hs.126036 | ESTs | 2.05 |
| 319565 | AW408683 | Hs.32922 | ESTs | 2.05 |
| 335146 | | | CH22_FGENES.499_2 | 2.05 |
| 301475 | AI678183 | Hs.170917 | prostaglandin E receptor 3 (subtype EP3) | 2.04 |
| 312442 | AA120970 | Hs.143199 | ESTs | 2.04 |
| 322502 | R62925 | Hs.243665 | ESTs | 2.04 |
| 303693 | AA290875 | Hs.30120 | ESTs | 2.04 |
| 310179 | AI215643 | Hs.171381 | ESTs | 2.03 |
| 321121 | W23285 | | EST cluster (not in UniGene) | 2.03 |
| 331330 | AA282197 | Hs.89002 | ESTs; Highly similar to CGI-07 protein [H.sapiens] | 2.03 |
| 306557 | AA994530 | | EST singleton (not in UniGene) with exon hit | 2.03 |
| 317865 | AI298794 | Hs.129130 | ESTs | 2.03 |
| 318667 | AI493742 | Hs.165210 | ESTs | 2.02 |
| 318042 | AW294522 | Hs.149991 | ESTs | 2.02 |
| 323818 | AW245528 | Hs.134754 | ESTs | 2.02 |
| 331286 | AA137062 | Hs.103853 | ESTs | 2.01 |
| 311262 | AI989942 | Hs.232150 | ESTs | 2.01 |
| 335601 | | | CH22_FGENES.581_41 | 2.01 |
| 311351 | AI682303 | Hs.201274 | ESTs | 2.01 |
| 312996 | AA249018 | | EST cluster (not in UniGene) | 2.01 |
| 328190 | | | CH.06_hs gi\|5868077 | 2 |
| 338030 | | | CH22_EM:AC005500.GENSCAN.148-16 | 2 |
| 333940 | | | CH22_FGENES.301_6 | 2 |
| 328227 | | | CH.06_hs gi\|5868105 | 2 |
| 331481 | N27448 | Hs.43944 | EST | 2 |
| 335288 | | | CH22_FGENES.527_1 | 2 |
| 307513 | AI274307 | | EST singleton (not in UniGene) with exon hit | 2 |
| 323316 | AL134620 | | EST cluster (not in UniGene) | 2 |
| 319479 | R21945 | Hs.256153 | ESTs | 2 |
| 303482 | AA502583 | Hs.197271 | ESTs | 2 |
| 327489 | | | CH.02_hs gi\|6004459 | 1.99 |
| 323935 | AW175841 | Hs.192183 | ESTs | 1.99 |
| 309575 | AW168096 | Hs.195188 | glyceraldehyde-3-phosphate dehydrogenase | 1.99 |
| 337043 | | | CH22_FGENES.439-19 | 1.98 |
| 312897 | AI828174 | Hs.227049 | ESTs | 1.98 |
| 307881 | AI370434 | | EST singleton (not in UniGene) with exon hit | 1.98 |
| 328656 | | | CH.07_hs gi\|6004473 | 1.98 |
| 314569 | AA813784 | Hs.123001 | ESTs | 1.98 |
| 332783 | W45302 | Hs.87889 | hellcase-mol | 1.98 |
| 315259 | AA701499 | Hs.148115 | ESTs | 1.98 |
| 313171 | N67879 | Hs.157695 | ESTs | 1.97 |
| 318060 | AI241421 | Hs.132236 | ESTs | 1.97 |
| 332256 | N66393 | Hs.102754 | ESTs | 1.97 |
| 312110 | AI962180 | Hs.226803 | ESTs | 1.97 |
| 335864 | | | CH22_FGENES.629_9 | 1.97 |
| 320389 | W00545 | Hs.171785 | ESTs | 1.97 |
| 314065 | AA868267 | Hs.85524 | ESTs | 1.96 |
| 323086 | H15474 | HS.12214 | Homo sapiens clone 23716 mRNA sequence | 1.96 |
| 323919 | AA862973 | Hs.220704 | ESTs | 1.96 |
| 310750 | AI373163 | Hs.170333 | ESTs | 1.96 |
| 309435 | AW090537 | | EST singleton (not in UniGene) with exon hit | 1.96 |
| 300129 | AW028820 | | EST cluster (not in UniGene) with exon hit | 1.96 |
| 320130 | AI820675 | Hs.203804 | ESTs | 1.95 |
| 323787 | AW373446 | Hs.169885 | ESTs; Weakly similar to cDNA EST EMBL:T02216 comes from this gene [C.elegans] | 1.95 |
| 338112 | | | CH22_EM:AC005500.GENSCAN.185-24 | 1.95 |
| 313625 | AW468402 | Hs.254020 | ESTs | 1.95 |
| 325240 | | | CH.10_hs gi\|5866848 | 1.95 |
| 331833 | AA412102 | Hs.250911 | interleukin 13 receptor, alpha 1 | 1.95 |
| 332252 | N63882 | | za21f9.s1 Soares fetal liver spleen 1NFLS Homo sapiens cDNA clone | |
| | | | IMAGE:293225 3', mRNA sequence | 1.95 |
| 300279 | AW237425 | Hs.253817 | ESTs | 1.95 |
| 326023 | | | CH.17_hs gi\|5867245 | 1.95 |
| 321609 | H86021 | Hs.198800 | ESTs; Weakly similar to hMmTRA1b [H.sapiens] | 1.94 |
| 324183 | AA402453 | Hs.113011 | ESTs | 1.94 |
| 336276 | | | CH22_FGENES.762_5 | 1.94 |
| 334913 | | | CH22_FGENES.456_3 | 1.94 |
| 325417 | | | CH.12_hs gi\|5866925 | 1.94 |
| 318489 | AW043590 | Hs.225023 | ESTs | 1.94 |
| 318455 | AI148763 | | EST cluster (not in UniGene) | 1.94 |
| 306890 | AI092235 | | EST singleton (not in UniGene) with exon hit | 1.94 |
| 315073 | AW452948 | Hs.257631 | ESTs | 1.94 |
| 321289 | R84687 | Hs.226306 | ESTs | 1.94 |
| 308521 | AI689808 | | EST singleton (not in UniGene) with exon hit | 1.93 |
| 306382 | AA968967 | | EST singleton (not in UniGene) with exon hit | 1.93 |
| 331320 | AA262999 | Hs.42788 | ESTs | 1.93 |
| 324279 | AA501412 | Hs.191688 | ESTs; Weakly similar to Pro-Pol-dUTPase polyprotein [M.musculus] | 1.93 |
| 309577 | AW168753 | | EST singleton (not in UniGene) with exon hit | 1.93 |
| 327014 | | | CH.21_hs gi\|5867664 | 1.93 |
| 303488 | AW025860 | | EST cluster (not in UniGene) with exon hit | 1.93 |
| 306561 | AA995223 | Hs.129559 | EST | 1.92 |
| 330694 | AA019806 | Hs.108447 | spinocerebellar ataxia 7 (olivopontocerebellar atrophy with retinal degeneration) | 1.92 |
| 313083 | N50545 | Hs.159200 | ESTs | 1.92 |
| 327752 | | | CH.05_hs gi\|5867949 | 1.92 |
| 318674 | AA295490 | | EST cluster (not in UniGene) | 1.92 |
| 301267 | AW297762 | Hs.255690 | ESTs | 1.91 |
| 332092 | AA608787 | Hs.112590 | ESTs | 1.91 |
| 323509 | AL036947 | | EST cluster (not in UniGene) | 1.91 |
| 321452 | AA317554 | | EST cluster (not in UniGene) | 1.91 |
| 311483 | AI765013 | Hs.209128 | ESTs | 1.91 |
| 300976 | AI246374 | Hs.185861 | ESTs | 1.91 |
| 323715 | AA322155 | | EST cluster (not in UniGene) | 1.91 |
| 313800 | AW296132 | Hs.166674 | ESTs | 1.91 |
| 332029 | AA489697 | Hs.145053 | ESTs | 1.91 |
| 304013 | AW518573 | Hs.156110 | Immunoglobulin kappa variable 1D-8 | 1.91 |
| 322019 | AA354549 | Hs.41181 | Homo sapiens mRNA; cDNA DKFZp727C191 (from clone DKFZp127C191) | 1.91 |
| 334150 | | | CH22_FGENES.339_1 | 1.9 |
| 310094 | AW450967 | Hs.235240 | ESTs | 1.9 |
| 316218 | AW207642 | Hs.174021 | ESTs | 1.9 |
| 324774 | AI031771 | Hs.132586 | ESTs | 1.9 |
| 326507 | | | CH.19_hs gi\|5867435 | 1.9 |
| 314570 | AA405696 | | EST cluster (not in UniGene) | 1.9 |
| 336268 | | | CH22_FGENES.758_2 | 1.9 |
| 315278 | AI985544 | Hs.116429 | ESTs | 1.9 |
| 325824 | | | CH.15-hs gi\|5867048 | 1.9 |
| 316277 | AA737780 | Hs.213392 | ESTs | 1.9 |
| 323181 | AA418583 | Hs.143621 | ESTs | 1.9 |
| 301438 | AA961643 | Hs.127716 | ESTs | 1.89 |
| 307050 | AI147341 | Hs.146734 | EST | 1.89 |
| 306830 | AI075803 | | EST singleton (not in UniGene) with exon hit | 1.89 |
| 302426 | AL049925 | Hs.225984 | DKFZP547G0910 protein | 1.89 |
| 320127 | H72615 | Hs.17268 | ESTs | 1.89 |
| 337736 | | | CH22_EM:AC000097.GENSCAN.100-2 | 1.89 |
| 331319 | AA262755 | Hs.194264 | ESTs | 1.88 |
| 310767 | AI377505 | Hs.158835 | ESTs | 1.88 |
| 314880 | AI732169 | Hs.105429 | ESTs | 1.88 |
| 312539 | AI004377 | Hs.200360 | ESTs | 1.88 |
| 309674 | AW205604 | Hs.168034 | ESTs; Weakly similar to !!!! ALU SUBFAMILY SP WARNING ENTRY !!!! [H.sapiens] | 1.88 |
| 314621 | AI627478 | Hs.187670 | ESTs | 1.88 |
| 319495 | AI972146 | Hs.192756 | ESTs | 1.88 |
| 313472 | AA007374 | | EST cluster (not in UniGene) | 1.88 |
| 302705 | U09060 | | EST cluster (not in UniGene) with exon hit | 1.88 |
| 329511 | | | CH.10_p2 gi\|3983514 | 1.88 |
| 317140 | AI699412 | Hs.201925 | ESTs | 1.87 |
| 302598 | AI815985 | Hs.129683 | ubiquitin-conjugating enzyme E2D 1 (homologous to yeast UBC4/5) | 1.87 |
| 301153 | AA725670 | Hs.120485 | ESTs; Weakly similar to serine/threonine kinase with SH3 domain; leucine | |
| | | | zipper domain and proline rich domain [H.sapiens] | 1.87 |
| 332222 | N28271 | Hs.176618 | ESTs | 1.87 |
| 330703 | AA055475 | Hs.104143 | light polypeptide (Lca) clathrin; | 1.87 |
| 318470 | AI159863 | Hs.143713 | ESTs | 1.87 |
| 314014 | AW291847 | Hs.121715 | ESTs; Weakly similar to HP protein [H.sapiens] | 1.87 |
| 300370 | AI827817 | | EST cluster (not in UniGene) with exon hit | 1.86 |
| 312329 | R84768 | Hs.13399 | Homo sapiens clone 25032 mRNA sequence | 1.86 |
| 325587 | | | CH.12_hs gi\|6682462 | 1.86 |
| 310237 | AI884313 | Hs.158906 | ESTs | 1.86 |
| 318872 | R13085 | | EST cluster (not in UniGene) | 1.86 |
| 303431 | AA317915 | | EST cluster (not in UniGene) with exon hit | 1.86 |
| 338427 | | | CH22_EM:AC005500.GENSCAN.349-1 | 1.86 |
| 300452 | AI352293 | Hs.191098 | ESTs | 1.85 |
| 321279 | H85330 | Hs.146060 | ESTs | 1.85 |
| 301690 | F05865 | Hs.249180 | ubiquitin-conjugating enzyme E2E 2 (homologous to yeast UBC4/5) | 1.85 |
| 307932 | AJ230822 | | EST singleton (not in UniGene) with exon hit | 1.85 |
| 318292 | AI679966 | Hs.150603 | ESTs | 1.85 |
| 310254 | AI239811 | Hs.157491 | ESTs | 1.85 |
| 311790 | AW016437 | Hs.233462 | ESTs | 1.84 |
| 314248 | AA278347 | Hs.126078 | ESTs | 1.84 |
| 335586 | | | CH22_FGENES.581_25 | 1.84 |
| 339209 | | | CH22_FF113D11.GENSCAN.6-4 | 1.84 |
| 307954 | AI419692 | | EST singleton (not in UniGene) with exon hit | 1.84 |
| 302549 | AF055136 | Hs.248162 | tectorin alpha | 1.84 |
| 321629 | H87213 | Hs.158092 | ESTs | 1.84 |
| 301239 | AA807558 | | EST cluster (not in UniGene) with exon hit | 1.84 |
| 332434 | N75542 | Hs.75356 | transcription factor 4 | 1.84 |
| 327192 | | | CH.01_hs gi\|5867445 | 1.83 |
| 310214 | AI220072 | Hs.165893 | ESTs | 1.83 |
| 320516 | R33857 | Hs.181479 | ESTs; Weakly similar to E-SELECTIN PRECURSOR [H.sapiens] | 1.83 |
| 324231 | W60827 | | EST cluster (not in UniGene) | 1.83 |
| 336616 | | | CH22_FGENES.613_5 | 1.83 |
| 328799 | | | CH.07_hs gi\|5868316 | 1.83 |
| 324661 | AW504161 | | EST cluster (not in UniGene) | 1.83 |
| 313190 | AA766707 | Hs.153039 | ESTs | 1.83 |
| 301979 | L28168 | Hs.121495 | potassium voltage-gated channel; Isk-related family; member 1 | 1.82 |
| 302099 | AL021397 | Hs.137576 | ribosomal protein L34 pseudogene 1 | 1.82 |
| 320187 | T99949 | | EST cluster (not in UniGene) | 1.82 |
| 320791 | R78808 | Hs.93961 | ESTs; Weakly similar to !!!! ALU CLASS A WARNING ENTRY !!!! [H.sapiens] | 1.82 |
| 305733 | AA829535 | Hs.84298 | CD74 antigen (invariant polypept of MHC; class II antigen-associated) | 1.82 |
| 308280 | AI569349 | Hs.180920 | ribosomal protein S9 | 1.81 |
| 321533 | W78877 | Hs.40111 | ESTs | 1.81 |
| 312946 | AI915122 | Hs.204087 | ESTs; Weakly similar to F33D11.9b [C.elegans] | 1.81 |
| 319474 | H90265 | Hs.100636 | ESTs | 1.81 |
| 329519 | | | CH.10_p2 gi\|3983510 | 1.81 |
| 324685 | AA220982 | | EST cluster (not in UniGene) | 1.81 |
| 320697 | N62937 | Hs.139181 | ESTs | 1.81 |
| 329246 | | | CH.X_hs gi\|5868732 | 1.81 |
| 332000 | AA481271 | Hs.193945 | ESTs | 1.81 |
| 310811 | AI420990 | Hs.161303 | ESTs | 1.81 |
| 325866 | | | CH.16_hs gi\|5867076 | 1.81 |
| 322064 | Z78343 | | EST cluster (not in UniGene) | 1.8 |
| 333712 | | | CH22_FGENES.251_1 | 1.8 |
| 313457 | AA576052 | Hs.193223 | ESTs | 1.8 |
| 321591 | H85687 | Hs.117927 | ESTs | 1.8 |
| 330260 | | | CH.05_p2 gi\|6671884 | 1.8 |
| 311080 | AI656320 | Hs.197711 | ESTs | 1.8 |
| 329522 | | | CH.10_p2 gi\|3983507 | 1.8 |
| 322889 | AA081924 | Hs.211417 | ESTs | 1.8 |
| 300175 | AI275011 | Hs.204877 | ESTs | 1.8 |
| 330976 | H20560 | Hs.244624 | ESTs | 1.8 |
| 300208 | AI341180 | Hs.196115 | ESTs; Weakly similar to FIBRILLIN 1 PRECURSOR [H.sapiens] | 1.79 |
| 319635 | R17531 | | EST cluster (not in UniGene) | 1.79 |
| 313454 | AA730673 | Hs.188634 | ESTs | 1.79 |
| 303093 | AI400310 | Hs.148958 | ESTs | 1.79 |
| 309815 | AW292760 | | EST singleton (not in UniGene) with exon hit | 1.79 |
| 326506 | | | CH.19_hs gi\|5867435 | 1.79 |
| 319845 | AA649011 | Hs.187902 | ESTs | 1.79 |
| 300290 | AI623739 | Hs.186387 | ESTs | 1.79 |
| 312180 | AI248285 | Hs.118348 | ESTs | 1.79 |
| 313058 | D81015 | Hs.125382 | ESTs | 1.79 |
| 330120 | | | CH.19_p2 gi\|6671864 | 1.78 |
| 328412 | | | CH.07_hs gi\|5868405 | 1.78 |
| 302345 | NM_000565 | | EST cluster (not in UniGene) with exon hit | 1.78 |
| 308100 | AI475949 | | EST singleton (not in UniGene) with exon hit | 1.78 |
| 311386 | AW205705 | Hs.207514 | ESTs | 1.78 |
| 330282 | | | CH.05_p2 gi\|6671910 | 1.78 |
| 318856 | Z43011 | Hs.21169 | ESTs | 1.78 |
| 312486 | AA845630 | Hs.117904 | ESTs | 1.78 |
| 325450 | | | CH.12_hs gi\|5866941 | 1.78 |
| 321206 | H54178 | Hs.226469 | ESTs | 1.78 |
| 330977 | H20826 | Hs.31783 | ESTs | 1.78 |
| 303487 | AA333666 | | EST cluster (not in UniGene) with exon hit | 1.77 |
| 310398 | AI264671 | Hs.164166 | ESTs | 1.77 |
| 313230 | AI540166 | Hs.129563 | ESTs | 1.77 |
| 317747 | AI683782 | Hs.128245 | ESTs | 1.77 |
| 303381 | AL038841 | Hs.163313 | ESTs; Weakly similar to !!!! ALU SUBFAMILY SB WARNING ENTRY !!!! [H.sapiens] | 1.77 |
| 336123 | | | CH22_FGENES.701_8 | 1.77 |
| 300185 | AI286182 | Hs.208484 | ESTs | 1.77 |
| 316002 | AW451733 | Hs.119824 | ESTs | 1.77 |
| 319850 | AA001811 | Hs.83722 | ESTs | 1.77 |
| 329941 | | | CH.16_p2 gi\|6165199 | 1.77 |
| 328329 | | | CH.07_hs gi\|5868375 | 1.77 |
| 322934 | AI493054 | Hs.158968 | ESTs | 1.77 |
| 325902 | | | CH.16_hs gi\|5867101 | 1.76 |
| 322239 | W01813 | Hs.12109 | WD40 protein Ciao1 | 1.76 |
| 303530 | AI274851 | Hs.258744 | ESTs | 1.76 |
| 300980 | AI025527 | Hs.222097 | ESTs | 1.76 |
| 331909 | AA437300 | Hs.178210 | ESTs | 1.76 |
| 321553 | H92449 | Hs.116406 | ESTs | 1.76 |
| 301618 | T52760 | | EST cluster(not in UniGene) with exon hit | 1.76 |
| 319592 | AA627356 | Hs.163315 | ESTs | 1.76 |
| 318511 | T26528 | Hs.227175 | ESTs; Weakly similar to !!!! ALU SUBFAMILY SQ WARNING ENTRY !!!! [H.sapiens] | 1.76 |
| 327183 | | | CH.01_hs gi\|5867442 | 1.76 |
| 313516 | AA029058 | Hs.135145 | ESTs | 1.76 |
| 318644 | AI752482 | | EST cluster (not in UniGene) | 1.76 |
| 321632 | AA419617 | | EST cluster (not in UniGene) | 1.76 |
| 324657 | AW451142 | Hs.255628 | ESTs | 1.76 |
| 300437 | AW449374 | Hs.257149 | ESTs | 1.75 |
| 319775 | AA504429 | Hs.6211 | methyl-CpG binding domain protein 1 | 1.75 |
| 314775 | AI149880 | Hs.188809 | ESTs | 1.75 |
| 337460 | | | CH22_FGENES.780-5 | 1.75 |
| 309849 | AW297444 | | EST singleton (not in UniGene) with exon hit | 1.75 |
| 301471 | AA995014 | Hs.129544 | ESTs; Weakly similar to ORF YLL027w [S.cerevisiae] | 1.75 |
| 312739 | AI318426 | Hs.155925 | ESTs | 1.75 |
| 319995 | H15355 | Hs.60887 | ESTs | 1.75 |
| 326495 | | | CH.19_hs gi\|5867423 | 1.75 |
| 337497 | | | CH22_FGENES.801-4 | 1.75 |
| 322633 | AA004534 | Hs.153981 | ESTs | 1.75 |
| 332177 | F10812 | Hs.101433 | ESTs | 1.75 |
| 326930 | | | CH.21_hs gi\|6456782 | 1.75 |
| 316893 | AA837332 | | EST cluster (not in UniGene) | 1.75 |
| 324826 | AA704806 | Hs.143842 | ESTs | 1.75 |
| 311269 | AI656924 | Hs.174257 | ESTs | 1.75 |
| 309375 | AW075342 | | EST singleton (not in UniGene) with exon hit | 1.75 |
| 314171 | AI821895 | Hs.193481 | ESTs | 1.75 |
| 311684 | AI990741 | Hs.252809 | ESTs | 1.75 |
| 334387 | | | CH22_FGENES.380_1 | 1.75 |
| 312195 | AI300101 | Hs.252222 | ESTs | 1.75 |
| 315707 | AI418055 | Hs.161160 | ESTs | 1.74 |
| 324349 | AW501470 | | EST cluster (not in UniGene) | 1.74 |
| 300724 | AI762929 | Hs.206134 | ESTs; Weakly similar to similar to reverse transcriptase [C.elegans] | 1.74 |
| 309906 | AW339340 | | EST singleton (not in UniGene) with exon hit | 1.74 |
| 303714 | AW501336 | | EST cluster (not in UniGene) with exon hit | 1.74 |
| 318704 | Z24981 | | EST cluster (not in UniGene) | 1.74 |
| 303027 | AF111178 | | EST cluster (not in UniGene) with exon hit | 1.74 |
| 322601 | W92924 | | EST cluster (not in UniGene) | 1.74 |
| 319382 | H93199 | Hs.33665 | ESTs | 1.74 |
| 315858 | AA737345 | | EST cluster (not in UniGene) | 1.74 |
| 332243 | N55484 | Hs.220540 | ESTs; Highly similar to ARYL HYDROCARBON RECEPTOR NUCLEAR | |
| | | | TRANSLOCATOR [H.sapiens] | 1.74 |
| 330951 | H02566 | Hs.191268 | Homo sapiens mRNA; cDNA DKFZp434N174 (from clone DKFZp434N174) | 1.74 |
| 324044 | AL045752 | Hs.211519 | ESTs | 1.73 |
| 320630 | AA199847 | | EST cluster (not in UniGene) | 1.73 |
| 327288 | | | CH.01_hs gi\|5867481 | 1.73 |
| 314986 | AI201367 | Hs.142860 | ESTs | 1.73 |
| 319078 | H17255 | Hs.144515 | ESTs | 1.73 |
| 326278 | | | CH.17_hs gi\|5867269 | 1.73 |
| 302552 | H49792 | | EST cluster (not in UniGene) with exon hit | 1.73 |
| 322322 | AF086431 | | EST cluster (not in UniGene) | 1.73 |
| 327075 | | | CH.21_hs gi\|6531965 | 1.73 |
| 317392 | AI797588 | Hs.145459 | ESTs | 1.73 |
| 300810 | AI076890 | Hs.186949 | ESTs | 1.73 |
| 315978 | AA830893 | Hs.119769 | ESTs | 1.73 |
| 323903 | AA773580 | Hs.193598 | ESTs | 1.73 |
| 330803 | AA004699 | Hs.150580 | putative translation initiation factor | 1.73 |
| 309845 | AW296802 | Hs.255580 | EST | 1.73 |
| 314963 | AI689617 | Hs.200934 | ESTs | 1.73 |
| 311710 | F09774 | Hs.175971 | ESTs | 1.73 |
| 315315 | AI984592 | Hs.15088 | ESTs | 1.73 |
| 300378 | AA663560 | Hs.235873 | ESTs; Weakly similar to K11C4.2 [C.elegans] | 1.73 |
| 316141 | AW303457 | | EST cluster (not in UniGene) | 1.72 |
| 319826 | T71739 | Hs.75442 | albumin | 1.72 |
| 312961 | AI033922 | Hs.122517 | ESTs | 1.72 |
| 334379 | | | CH22_FGENES.379_11 | 1.72 |
| 305854 | AA862733 | | EST singleton (not in UniGene) with exon hit | 1.72 |
| 313031 | N34927 | Hs.186566 | ESTs | 1.72 |
| 329728 | | | CH.14_p2 gi\|6065785 | 1.72 |
| 312090 | N57692 | Hs.118064 | ESTs | 1.72 |
| 323341 | AL134875 | Hs.192386 | ESTs | 1.72 |
| 302077 | AA310580 | Hs.132898 | Homo sapiens chromosome 11;BAC CIT-HSP-311e8 (BC269730) | |
| | | | containing the hFEN1 gene | 1.71 |
| 310766 | AI971438 | Hs.158824 | ESTs | 1.71 |
| 311450 | AI809985 | Hs.203340 | ESTs | 1.71 |
| 311792 | AW238064 | Hs.253909 | ESTs | 1.71 |
| 321500 | H71999 | | EST cluster (not in UniGene) | 1.71 |
| 311948 | T78791 | Hs.241569 | ESTs; Moderately smlr to !!!! ALU SUBFAMILY SQ WARNING ENTRY !!!! [H.sapiens] | 1.71 |
| 302270 | R56151 | | EST cluster (not in UniGene) with exon hit | 1.71 |
| 329089 | | | CH.X_hs gi\|5868614 | 1.71 |
| 322331 | AF086467 | | EST cluster (not in UniGene) | 1.71 |
| 318235 | AI080361 | Hs.134217 | ESTs | 1.71 |
| 304561 | AA489792 | | EST singleton (not in UniGene) with exon hit | 1.71 |
| 312681 | AI028149 | Hs.193124 | pyruvate dehydrogenase kinase; isoenzyme 3 | 1.71 |
| 310250 | AI478629 | Hs.158465 | ESTs | 1.71 |
| 338178 | | | CH22_EM:AC005500.GENSCAN.219-6 | 1.71 |
| 338910 | | | CH22_DJ32l10.GENSCAN.11-2 | 1.71 |
| 321225 | AL080073 | Hs.251414 | Homo sapiens mRNA; cDNA DKFZp564B1462 (from clone DKFZp564B1462) | 1.7 |
| 322289 | AA534550 | Hs.539 | ribosomal protein S29 | 1.7 |
| 319802 | Al701489 | Hs.202501 | ESTs | 1.7 |
| 314022 | AW452420 | Hs.248678 | ESTs | 1.7 |
| 314937 | AA515602 | Hs.152330 | ESTs | 1.7 |
| 300580 | AA761322 | Hs.220538 | ESTs | 1.7 |
| 304398 | AA262785 | | EST singleton (not in UnlGene) with exon hit | 1.7 |
| 313421 | AW339515 | Hs.163700 | ESTs | 1.7 |
| 309763 | AW270182 | | EST singleton (not in UniGene) with exon hit | 1.7 |
| 322092 | AF085833 | | EST cluster (not in UniGene) | 1.7 |
| 315603 | AA764768 | Hs.121158 | ESTs | 1.7 |
| 325031 | T08597 | | EST cluster (not in UniGene) | 1.7 |
| 327157 | | | CH.01_hs gi\|5866841 | 1.7 |
| 314809 | AI741461 | Hs.161904 | ESTs | 1.7 |
| 320361 | H67220 | Hs.146406 | nitrilase 1 | 1.69 |
| 324721 | AW402302 | Hs.43616 | ESTs | 1.69 |
| 328624 | | | CH.07_hs gi\|5868246 | 1.69 |
| 303344 | AA255977 | Hs.250646 | ESTs; Highly similar to ubiquitin-conjugating enzyme [M.musculus] | 1.69 |
| 328960 | | | CH.08_hs gi\|6456775 | 1.69 |
| 315702 | AA657501 | Hs.146315 | ESTs | 1.69 |
| 302385 | AJ224172 | Hs.204096 | lipophilin B (uteroglobin family member); prostatein-like | 1.68 |
| 319699 | R14537 | | EST cluster (not in UniGene) | 1.68 |
| 309506 | AW137700 | | EST singleton (not in UniGene) with exon hit | 1.68 |
| 330417 | D84424 | Hs.57697 | hyaluronan synthase 1 | 1.68 |
| 315296 | AA876905 | Hs.125286 | ESTs | 1.68 |
| 328538 | | | CH.07_hs gi\|5868485 | 1.68 |
| 323923 | AA354146 | | EST cluster (not in UniGene) | 1.68 |
| 320303 | AL079289 | Hs.137154 | Homo sapiens mRNA full length insert cDNA clone EUROIMAGE 35971 | 1.68 |
| 302967 | AI927068 | Hs.110853 | ESTs; Weakly similar to R10D12.12 [C.elegans] | 1.68 |
| 310695 | AI472124 | Hs.157757 | ESTs | 1.68 |
| 307512 | AI273815 | Hs.242463 | keratin 8 | 1.68 |
| 338506 | | | CH22_EM:AC005500.GENSCAN.390-10 | 1.68 |
| 331722 | AA195405 | Hs.110347 | Homo sapiens mRNA for alpha integrin binding protein 80; partial | 1.68 |
| 301431 | R05385 | | EST cluster (not in UniGene) with exon hit | 1.68 |
| 318853 | Z42977 | Hs.21062 | ESTs | 1.68 |
| 323032 | AW244073 | Hs.145946 | ESTs | 1.68 |
| 317538 | AW137772 | Hs.185980 | ESTs | 1.68 |
| 325780 | | | CH.14_hs gi\|6381953 | 1.67 |
| 321739 | AL080280 | | EST cluster (not in UniGene) | 1.67 |
| 319808 | T58960 | | EST cluster (not in UniGene) | 1.67 |
| 313443 | AA249037 | | EST cluster (not in UniGene) | 1.67 |
| 331366 | AA424754 | Hs.43149 | ESTs | 1.67 |
| 316443 | AI797592 | Hs.207407 | ESTs | 1.67 |
| 322878 | AA081820 | | EST cluster (not in UniGene) | 1.67 |
| 330320 | | | CH.08_p2 gi\|5932415 | 1.67 |
| 329081 | | | CH.X_hs gi\|5868602 | 1.67 |
| 334026 | | | CH22_FGENES.318_3 | 1.67 |
| 317791 | AI801500 | Hs.128457 | ESTs | 1.67 |
| 322235 | AF086106 | | EST cluster (not in UniGene) | 1.66 |
| 331148 | R73816 | Hs.17385 | ESTs | 1.66 |
| 325452 | | | CH.12_hs gi\|5866941 | 1.66 |
| 315106 | AW452184 | Hs.232100 | ESTs | 1.66 |
| 326014 | | | CH.16_hs gi\|5867160 | 1.66 |
| 307130 | AI185234 | | EST singleton (not in UniGene) with exon hit | 1.66 |
| 300943 | AA524545 | Hs.224630 | ESTs | 1.66 |
| 319402 | W21298 | | EST cluster (not in UniGene) | 1.66 |
| 310889 | AI457946 | Hs.170437 | ESTs; Weakly similar to hyperpolarization-activated; cyclic | |
| | | | nucleotide-gated channel 2 [H.sapiens] | 1.66 |
| 323371 | AL135118 | | EST cluster (not in UniGene) | 1.66 |
| 335568 | | | CH22_FGENES.581_4 | 1.66 |
| 320654 | AW263086 | Hs.118112 | ESTs | 1.66 |
| 338983 | | | CH22_DA59H18.GENSCAN.3-1 | 1.65 |
| 330002 | | | CH.16_p2 gi\|6623963 | 1.65 |
| 315343 | AW205477 | Hs.179891 | ESTs | 1.65 |
| 334487 | | | CH22_FGENES.395_9 | 1.65 |
| 312169 | AI064824 | Hs.193385 | ESTs | 1.65 |
| 309668 | AW204480 | Hs.253414 | EST | 1.65 |
| 309518 | AW148928 | Hs.248895 | EST | 1.65 |
| 307965 | AI421641 | | EST singleton (not in UniGene) with exon hit | 1.65 |
| 316787 | AW369770 | Hs.130351 | ESTs | 1.65 |
| 300835 | AA401858 | Hs.224843 | ESTs | 1.65 |
| 338763 | | | CH22_EM:AC005500.GENSCAN.517-16 | 1.65 |
| 303327 | AA232729 | Hs.154302 | ESTs | 1.65 |
| 313231 | AW139993 | Hs.163682 | ESTs | 1.65 |
| 334073 | | | CH22_FGENES.327_28 | 1.65 |
| 319901 | T77136 | Hs.8765 | RNA helicase-related protein | 1.65 |
| 326530 | | | CH.19_hs gi\|5867441 | 1.65 |
| 301126 | AI802877 | Hs.210843 | ESTs; Weakly similar to dJ1039K5.2 [H.sapiens] | 1.65 |
| 314043 | AA827082 | | EST cluster (not in UniGene) | 1.65 |
| 304387 | AA236027 | | EST singleton (not in UniGene) with exon hit | 1.65 |
| 322932 | AA099732 | | EST cluster (not in UniGene) | 1.65 |
| 337272 | | | CH22_FGENES.660-1 | 1.64 |
| 332694 | AA262768 | Hs.243901 | KIAA1067 protein | 1.64 |
| 318996 | Z44266 | | EST cluster (not in UniGene) | 1.64 |
| 315336 | AW342028 | Hs.256112 | ESTs | 1.64 |
| 313329 | AW293704 | Hs.122658 | ESTs | 1.64 |
| 318088 | AW295409 | Hs.137945 | ESTs | 1.64 |
| 313835 | AI538438 | Hs.159087 | ESTs | 1.64 |
| 320035 | AA378974 | Hs.130720 | ESTs; Weakly similar to CELLULAR NUCLEIC ACID BINDING PROTEIN [H.sapiens] | 1.64 |
| 309372 | AW074330 | | EST singleton (not in UniGene) with exon hit | 1.63 |
| 324157 | AW402236 | | EST cluster (not in UniGene) | 1.63 |
| 323929 | AA354940 | Hs.145958 | ESTs | 1.63 |
| 302490 | AA885502 | Hs.187032 | ESTs | 1.63 |
| 333942 | | | CH22_FGENES.301_8 | 1.63 |
| 327469 | | | CH.02_hs gi\|5867772 | 1.63 |
| 301918 | AA476777 | | EST cluster (not in UniGene) with exon hit | 1.63 |
| 315664 | AI744068 | Hs.160712 | ESTs | 1.63 |
| 304405 | AA282572 | | EST singleton (not in UniGene) with exon hit | 1.63 |
| 310624 | AI341594 | Hs.157522 | ESTs; Moderately similar to env protein [H.sapiens] | 1.63 |
| 319250 | F11623 | | EST cluster (not in UniGene) | 1.63 |
| 310608 | AI962234 | Hs.196102 | ESTs | 1.63 |
| 317348 | AI348076 | Hs.831 | 3-hydroxymethyl-3-methylglutaryl-Coenzyme A lyase (hydroxymethylglutaricaciduria) | 1.63 |
| 306513 | AA989230 | | EST singleton (not in UniGene) with exon hit | 1.63 |
| 320807 | AA086110 | Hs.188536 | Homo sapiens clone 24838 mRNA sequence | 1.63 |
| 303710 | AI269069 | Hs.250852 | ESTs; Highly similar to ubiquitin hydrolyzing enzyme I [H.sapiens] | 1.63 |
| 328291 | | | CH.07_hs gi\|5868363 | 1.63 |
| 304236 | W93278 | | EST singleton (not in UniGene) with exon hit | 1.63 |
| 317683 | AI791700 | Hs.127893 | ESTs | 1.63 |
| 311960 | AW440133 | Hs.189690 | ESTs | 1.62 |
| 312834 | AI028309 | Hs.114246 | ESTs | 1.62 |
| 325326 | | | CH.11_hs gi\|5866875 | 1.62 |
| 313663 | AI953261 | Hs.169813 | ESTs | 1.62 |
| 327526 | | | CH.02_hs gi\|6381882 | 1.62 |
| 300429 | AW449679 | Hs.156739 | ESTs; Highly similar to XG GLYCOPROTEIN PRECURSOR [H.sapiens] | 1.62 |
| 305169 | AA663131 | | EST singleton (not in UniGene) with exon hit | 1.62 |
| 316621 | AI021996 | Hs.122138 | ESTs | 1.62 |
| 329666 | | | CH.14_p2 gi\|6272129 | 1.62 |
| 318035 | AI744130 | Hs.131201 | ESTs | 1.62 |
| 300492 | AL031709 | | multiple UniGene matches | 1.62 |
| 316532 | AI307229 | Hs.184304 | ESTs | 1.62 |
| 332048 | AA496019 | Hs.201591 | ESTs | 1.62 |
| 307113 | AI183686 | | EST singleton (not in UniGene) with exon hit | 1.62 |
| 319127 | N49476 | | EST cluster (not in UniGene) | 1.62 |
| 331155 | R87650 | Hs.33439 | ESTs; Weakly similar to !!!! ALU SUBFAMILY J WARNING ENTRY !!!! [H.sapiens] 1.61 | |
| 338220 | | | CH22_EM:AC005500.GENSCAN.246-9 | 1.61 |
| 315763 | AW515270 | Hs.118342 | ESTs | 1.61 |
| 323571 | AA984133 | Hs.153260 | c-Cbl-interacting protein | 1.61 |
| 312240 | R28628 | Hs.203669 | ESTs | 1.61 |
| 304569 | AA490934 | | EST singleton (not in UniGene) with exon hit | 1.61 |
| 313179 | AI076101 | Hs.131704 | ESTs | 1.61 |
| 326858 | | | CH.20_hs gi\|6552462 | 1.61 |
| 317276 | AI823847 | Hs.129986 | ESTs | 1.61 |
| 312572 | AA350125 | Hs.187499 | ESTs | 1.61 |
| 311932 | AW451654 | Hs.257482 | ESTs | 1.61 |
| 302103 | AA452310 | Hs.26090 | ESTs; Weakly similar to T20B12.1 [C.elegans] | 1.61 |
| 308413 | AI636253 | Hs.196511 | EST | 1.61 |
| 310077 | AI620617 | Hs.148565 | ESTs | 1.61 |
| 337780 | | | CH22_EM:AC000097.GENSCAN.121-2 | 1.61 |
| 327796 | | | CH.05_hs gi\|5867982 | 1.61 |
| 308352 | AI610791 | | EST singleton (not in UniGene) with exon hit | 1.61 |
| 324539 | AI378032 | Hs.125892 | ESTs | 1.61 |
| 303232 | AA437414 | | EST cluster (not in UniGene) with exon hit | 1.61 |
| 337884 | | | CH22_EM:AC005500.GENSCAN.54-2 | 1.61 |
| 303620 | AA397546 | Hs.119151 | ESTs | 1.61 |
| 303481 | AA336839 | | EST cluster (not in UniGene) with exon hit | 1.61 |
| 314481 | AA548589 | Hs.105846 | ESTs | 1.61 |
| 300327 | AI908894 | Hs.245893 | ESTs | 1.6 |
| 323473 | AA262442 | | EST cluster (not in UniGene) | 1.6 |
| 326154 | | | CH.17_hs gi\|5867170 | 1.6 |
| 331920 | AA446885 | Hs.99087 | ESTs; Moderately similar to ZINC FINGER PROTEIN 141 [H.sapiens] | 1.6 |
| 323827 | AW406878 | | EST cluster (not in UniGene) | 1.6 |
| 322452 | W56710 | | EST cluster (not in UniGene) | 1.6 |
| 310597 | AI739071 | Hs.158515 | ESTs | 1.6 |
| 307871 | AI368665 | | EST singleton (not in UniGene) with exon hit | 1.6 |
| 322215 | AF088005 | | EST cluster (not in UniGene) | 1.6 |
| 318420 | AI139857 | Hs.143837 | ESTs | 1.6 |
| 332217 | H98987 | Hs.102383 | EST | 1.6 |
| 324937 | M79230 | Hs.192398 | ESTs | 1.6 |
| 320543 | AF052176 | Hs.158529 | Homo sapiens clone 24457 mRNA sequence | 1.6 |
| 300674 | AW467388 | | EST cluster (not in UniGene) with exon hit | 1.6 |
| 315193 | AI241331 | Hs.131765 | ESTs | 1.6 |
| 319713 | R24204 | | EST cluster (not in UniGene) | 1.6 |
| 301210 | AI379982 | Hs.158944 | ESTs | 1.6 |
| 309365 | AW072861 | | EST singleton (not in UniGene) with exon hit | 1.6 |
| 321403 | AW451454 | Hs.247568 | adenylate kinase 3 | 1.6 |
| 321908 | AA376936 | Hs.20998 | ESTs | 1.6 |
| 303349 | AA382661 | | EST cluster (not in UniGene) with exon hit | 1.6 |
| 324338 | AL138357 | Hs.247514 | ESTs | 1.6 |
| 310599 | AW300144 | | EST cluster (not in UniGene) | 1.6 |
| 333193 | | | CH22_FGENES.98_15 | 1.6 |
| 336433 | | | CH22_FGENES.825_12 | 1.6 |
| 312097 | AI352096 | Hs.157169 | ESTs | 1.6 |
| 311445 | AW204237 | Hs.192703 | ESTs; Weakly similar to !!!! ALU SUBFAMILY J WARNING ENTRY !!!! [H.sapiens] | 1.59 |
| 317736 | AI361722 | Hs.192410 | ESTs | 1.59 |
| 308147 | AI498991 | | ESTsingleton (not in UniGene) with exon hit | 1.59 |
| 313489 | AA017492 | Hs.135655 | ESTs | 1.59 |
| 316289 | AA902488 | Hs.122952 | ESTs | 1.59 |
| 326983 | | | CH.21_hs gi\|5867657 | 1.59 |
| 314781 | AW205298 | Hs.202372 | ESTs | 1.59 |
| 328397 | | | CH.07_hs gi\|5868397 | 1.59 |
| 331970 | AA461084 | Hs.187677 | ESTs | 1.59 |
| 321744 | N91419 | Hs.12028 | ESTs | 1.59 |
| 310509 | AI292181 | Hs.150036 | ESTs | 1.59 |
| 315921 | AI147545 | Hs.114172 | ESTs | 1.59 |
| 322049 | AI928242 | Hs.144383 | ESTs | 1.59 |
| 301161 | AA731518 | | EST cluster (not in UniGene) with exon hit | 1.59 |
| 300548 | AI026836 | Hs.114689 | ESTs | 1.59 |
| 319142 | F07366 | | EST cluster (not in UniGene) | 1.59 |
| 313526 | AW152263 | Hs.249243 | ESTs | 1.59 |
| 305937 | AA883238 | | EST singleton (not in UniGene) with exon hit | 1.58 |
| 330123 | | | CH.19_p2 gi\|6671869 | 1.58 |
| 327819 | | | CH.05_hs gi\|5867968 | 1.58 |
| 318250 | AI478814 | Hs.134603 | ESTs | 1.58 |
| 306760 | AI034094 | Hs.169476 | ubiquitous tubulin; alpha; | 1.58 |
| 322358 | AA220235 | Hs.246836 | ESTs | 1.58 |
| 317866 | AI690269 | Hs.201345 | ESTs | 1.58 |
| 320725 | AA703319 | Hs.120967 | ESTs | 1.58 |
| 311332 | AW292247 | Hs.255052 | ESTs | 1.58 |
| 334893 | | | CH22_FGENES.452_7 | 1.58 |
| 318730 | AA398215 | | EST cluster (not in UniGene) | 1.58 |
| 315889 | AW271639 | Hs.221744 | ESTs | 1.58 |
| 303702 | AW500748 | Hs.224961 | ESTs; Weakly similar to 73 kDA subunit of cleavage and polyadenylation | |
| | | | specificity factor [H.sapiens] | 1.57 |
| 315086 | AI492660 | Hs.170935 | ESTs | 1.57 |
| 332514 | AA156499 | Hs.8454 | protein kinase; cAMP-dependent; regulatory; type II; alpha | 1.57 |
| 335549 | | | CH.22_FGENES.576_10 | 1.57 |
| 329532 | | | CH.10_p2 gi\|3983505 | 1.57 |
| 323140 | AA180467 | | EST cluster (not in UniGene) | 1.57 |
| 313166 | AI801098 | Hs.151500 | ESTs | 1.57 |
| 337896 | | | CH22_EM:AC005500.GENSCAN.56-3 | 1.57 |
| 330658 | AA319514 | Hs.211093 | ESTs | 1.57 |
| 324585 | AI823969 | Hs.132678 | ESTs | 1.57 |
| 317151 | AW298195 | Hs.255735 | ESTs | 1.57 |
| 308818 | AI819700 | Hs.208231 | EST | 1.57 |
| 326547 | | | CH.19_hs gi\|5867307 | 1.57 |
| 318833 | H06234 | Hs.24888 | ESTs | 1.57 |
| 320488 | R31386 | | EST cluster (not in UniGene) | 1.57 |
| 306929 | AI124514 | | EST singleton (not in UniGene) with exon hit | 1.57 |
| 338083 | | | CH22_EM:AC005500.GENSCAN.174-1 | 1.57 |
| 316868 | AI660898 | Hs.195602 | ESTs | 1.57 |
| 310937 | AI472880 | Hs.170480 | ESTs | 1.57 |
| 328638 | | | CH.07_hs gi\|6004473 | 1.57 |
| 310074 | AI651039 | Hs.148559 | ESTs | 1.56 |
| 327058 | | | CH.21_hs gi\|6531965 | 1.56 |
| 320076 | AI653733 | Hs.204079 | ESTs | 1.56 |
| 322345 | AF086529 | | EST cluster (not in UniGene) | 1.56 |
| 314731 | AI745498 | Hs.204579 | ESTs | 1.56 |
| 318687 | H49619 | Hs.127301 | ESTs | 1.56 |
| 303841 | AI934464 | | EST cluster (not in UniGene) with exon hit | 1.56 |
| 302370 | AJ009849 | Hs.199297 | Homo sapiens GNAS1 gene encoding NESP55 | 1.56 |
| 322571 | AF156271 | | EST cluster (not in UniGene) | 1.56 |
| 318050 | AI052093 | Hs.133132 | ESTs | 1.56 |
| 303388 | AL039604 | | EST cluster (not in UniGene) with exon hit | 1.56 |
| 323758 | AA833858 | | EST cluster (not in UniGene) | 1.56 |
| 328369 | | | CH.07_hs gi\|5868388 | 1.56 |
| 329415 | | | CH.Y_hs gi\|5868874 | 1.56 |
| 303915 | AW468839 | Hs.257767 | EST | 1.56 |
| 338794 | | | CH22_EM:AC005500.GENSCAN.528.1 | 1.56 |
| 303074 | AA243481 | Hs.127320 | ESTs; Weakly similar to KIAA0346 [H.sapiens] | 1.56 |
| 318807 | F08434 | | EST cluster (not in UniGene) | 1.56 |
| 334287 | | | CH22_FGENES.369_17 | 1.56 |
| 311928 | AW024798 | Hs.233374 | ESTs | 1.55 |
| 304592 | AA505833 | Hs.162017 | EST | 1.55 |
| 300785 | AA682913 | Hs.247179 | ESTs; Weakly similar to KIAA0319 [H.sapiens] 1.55 | |
| 304921 | AA603092 | | EST singleton (not in UniGene) with exon hit | 1.55 |
| 324605 | AW502851 | Hs.249978 | ESTs | 1.55 |
| 324473 | AW501163 | | EST cluster (not in UniGene) | 1.55 |
| 300566 | H86709 | Hs.21371 | son of sevenless (Drosophila) homolog 1 | 1.55 |
| 314165 | AA761265 | Hs.221281 | ESTs | 1.55 |
| 302868 | AA157392 | | EST cluster (not in UniGene) with exon hit | 1.55 |
| 314034 | AI299137 | Hs.154214 | ESTs | 1.55 |
| 325389 | | | CH.12_hs gi\|5866921 | 1.55 |
| 331849 | AA417078 | Hs.193767 | ESTs | 1.55 |
| 320536 | AA331732 | Hs.137224 | ESTs | 1.55 |
| 303347 | AA258033 | | EST cluster (not in UniGene) with exon hit | 1.55 |
| 315769 | AA744875 | Hs.189413 | ESTs | 1.55 |
| 317031 | AA973297 | Hs.126101 | ESTs | 1.55 |
| 300203 | AI827065 | Hs.224877 | ESTs | 1.55 |
| 304037 | T26438 | | EST singleton (not in UniGene) with exon hit | 1.55 |
| 322613 | AW160507 | | EST cluster (not in UniGene) | 1.54 |
| 317987 | AW138174 | Hs.130651 | ESTs | 1.54 |
| 322313 | AF086386 | | EST cluster (not in UniGene) | 1.54 |
| 323992 | AW411383 | Hs.169688 | ESTs | 1.54 |
| 325303 | | | CH.11_hs gi\|5866908 | 1.54 |
| 312701 | AI457663 | Hs.128127 | ESTs | 1.54 |
| 304787 | AA582678 | | EST singleton (not in UniGene) with exon hit | 1.54 |
| 305849 | AA861571 | | EST singleton (not in UniGene) with exon hit | 1.54 |
| 314557 | AA401367 | Hs.128647 | ESTs | 1.54 |
| 316507 | AI381515 | Hs.158381 | ESTs | 1.54 |
| 315023 | AA533505 | Hs.185844 | ESTs | 1.54 |
| 314920 | AA513406 | Hs.152307 | ESTs | 1.54 |
| 323097 | Z44354 | Hs.180950 | guanine nucleotide binding protein (G protein); q polypeptide | 1.54 |
| 325043 | W27919 | Hs.32944 | inositol polyphosphate-4-phosphatase; type I; 107kD | 1.54 |
| 307892 | AI376086 | Hs.158759 | EST | 1.54 |
| 324573 | AA491600 | Hs.161942 | ESTs | 1.54 |
| 313092 | AI923673 | Hs.212827 | ESTs | 1.54 |
| 324696 | AA641092 | Hs.257339 | ESTs | 1.54 |
| 303019 | AF098363 | | EST cluster (not in UniGene) with exon hit | 1.54 |
| 317158 | AI459140 | Hs.129109 | ESTs | 1.54 |
| 309536 | AW151933 | | EST singleton (not in UniGene) with exon hit | 1.54 |
| 301568 | AI146423 | Hs.146709 | ESTs | 1.53 |
| 315674 | AA651923 | Hs.191850 | ESTs | 1.53 |
| 321861 | N79341 | | EST cluster (not in UniGene) | 1.53 |
| 310890 | AI184510 | Hs.143728 | ESTs | 1.53 |
| 330036 | | | CH.17_p2 gi\|6042048 | 1.53 |
| 316907 | AA843868 | Hs.190567 | ESTs | 1.53 |
| 312299 | AA972712 | Hs.174818 | ESTs | 1.53 |
| 331128 | R51361 | Hs.23423 | ESTs | 1.53 |
| 305177 | AA663591 | | EST singleton (not in UniGene) with exon hit | 1.53 |
| 337685 | | | CH22_EM:AC000097.GENSCAN.77-1 | 1.53 |
| 335290 | | | CH22_FGENES.527_3 | 1.53 |
| 308896 | AI858667 | | EST singleton (not in UniGene) with exon hit | 1.53 |
| 307944 | AI418246 | | EST singleton (not in UniGene) with exon hit | 1.53 |
| 300867 | AW340374 | Hs.121033 | neural precursor cell expressed; developmentally down-regulated 1 | 1.53 |
| 335320 | | | CH22_FGENES.534_7 | 1.53 |
| 329841 | | | CH.14_p2 gi\|6672062 | 1.53 |
| 317916 | AI565071 | Hs.159983 | ESTs | 1.53 |
| 332901 | | | CH22_FGENES.36_2 | 1.53 |
| 305413 | AA724659 | | EST singleton (not in UniGene) with exon hit | 1.53 |
| 316707 | AI016387 | Hs.184406 | ESTs | 1.53 |
| 313693 | AW469180 | Hs.170651 | ESTs | 1.53 |
| 316101 | AA922236 | Hs.221037 | ESTs | 1.53 |
| 320796 | AF038966 | Hs.184543 | secretory carrier membrane protein 1 | 1.53 |
| 307451 | AI248615 | | EST singleton (not in UniGene) with exon hit | 1.53 |
| 323648 | AI679968 | Hs.152060 | ESTs | 1.53 |
| 331482 | N27515 | Hs.40296 | ESTs | 1.53 |
| 318059 | AI023175 | Hs.167022 | ESTs | 1.53 |
| 325958 | | | CH.16_hs gi\|5867142 | 1.53 |
| 315736 | AA664265 | Hs.230213 | ESTs | 1.53 |
| 314740 | AW015667 | Hs.119427 | ESTs | 1.52 |
| 314117 | AA224368 | Hs.185164 | ESTs | 1.52 |
| 301646 | AA313954 | | EST cluster (not in UniGene) with exon hit | 1.52 |
| 338752 | | | CH22_EM:AC005500.GENSCAN.513-10 | 1.52 |
| 309314 | AW009312 | | EST singleton (not in UniGene) with exon hit | 1.52 |
| 301445 | AI208364 | Hs.128233 | ESTs; Weakly similar to REGULATOR OF CHROMOSOME | |
| | | | CONDENSATION [H.sapiens] | 1.52 |
| 308501 | AI685263 | Hs.201150 | EST | 1.52 |
| 312330 | AA635305 | Hs.121574 | ESTs | 1.52 |
| 318040 | AI018150 | Hs.148781 | ESTs | 1.52 |
| 336205 | | | CH22_FGENES.719_10 | 1.52 |
| 325701 | | | CH.14_hs gi\|5867028 | 1.52 |
| 315009 | AW189460 | Hs.208358 | ESTs | 1.52 |
| 303121 | AW407585 | Hs.27769 | ESTs; Weakly similar to mCAC [M.musculus] | 1.52 |
| 309271 | AI986221 | | EST singleton (not in UniGene) with exon hit | 1.52 |
| 328385 | | | CH.07_hs gi\|5868395 | 1.52 |
| 307700 | AI318545 | | EST singleton (not in UniGene) with exon hit | 1.52 |
| 314591 | AW103292 | Hs.245328 | ESTs | 1.52 |
| 304484 | AA432067 | Hs.258373 | ESTs | 1.52 |
| 304382 | AA232873 | | EST singleton (not in UniGene) with exon hit | 1.52 |
| 304232 | W52674 | | EST singleton (not in UniGene) with exon hit | 1.52 |
| 309853 | AW298169 | Hs.57553 | tousled-like kinase 2 | 1.52 |
| 312504 | AW207346 | Hs.143202 | ESTs | 1.52 |
| 313134 | N63406 | Hs.258697 | ESTs | 1.52 |
| 330391 | AF015950 | Hs.115256 | telomerase reverse transcriptase | 1.52 |
| 314342 | AI873046 | Hs.258775 | ESTs | 1.51 |
| 305977 | AA887293 | | EST singleton (not in UniGene) with exon hit | 1.51 |
| 301165 | N85789 | Hs.224155 | ESTs; Weakly similar to PTERIN-4-ALPHA-CARBINOLAMINE | |
| | | | DEHYDRATASE [H.sapiens] | 1.51 |
| 300613 | AI932294 | Hs.249604 | ESTs; Weakly similar to B-CELL LYMPHOMA 6 PROTEIN [H.sapiens] | 1.51 |
| 324124 | AI554212 | Hs.185664 | ESTs; Weakly similar to SERINE/THREONINE-PROTEIN KINASE NRK2 [H.sapiens] | 1.51 |
| 308037 | AI458207 | Hs.174181 | ESTs | 1.51 |
| 323909 | AL043148 | Hs.186257 | ESTs | 1.51 |
| 315464 | AW139500 | Hs.116135 | ESTs | 1.51 |
| 306700 | AI022056 | | EST singleton (not in UniGene) with exon hit | 1.51 |
| 337976 | | | CH22_EM:AC005500.GENSCAN.107-1 | 1.51 |
| 306855 | AI083982 | | EST singleton (not in UniGene) with exon hit | 1.51 |
| 311045 | AI569399 | Hs.174746 | ESTs | 1.51 |
| 315010 | AA531082 | Hs.240049 | ESTs | 1.51 |
| 310205 | AW025248 | Hs.202445 | ESTs | 1.51 |
| 310759 | AW135924 | Hs.224883 | ESTs | 1.51 |
| 310954 | AW449044 | Hs.171298 | ESTs | 1.51 |
| 312019 | T77046 | Hs.188750 | ESTs | 1.51 |
| 334773 | | | CH22_FGENES.430_5 | 1.51 |
| 332043 | AA490831 | Hs.125056 | ESTs | 1.51 |
| 322950 | AA296219 | | EST cluster (not in UniGene) | 1.51 |
| 337920 | | | CH22_EM:AC005500.GENSCAN.67-3 | 1.51 |
| 328993 | | | CH.09_hs gi\|5868536 | 1.51 |
| 309245 | AI972447 | | EST singleton (not in UniGene) with exon hit | 1.51 |
| 312172 | AI222168 | Hs.191168 | ESTs | 1.51 |
| 304039 | T47349 | | EST singleton (not in UniGene) with exon hit | 1.5 |
| 301329 | AI149653 | Hs.190496 | ESTs | 1.5 |
| 313376 | AI949246 | Hs.200381 | ESTs | 1.5 |
| 324248 | AW504918 | | EST cluster (not in UniGene) | 1.5 |
| 308771 | AI809301 | | EST singleton (not in UniGene) with exon hit | 1.5 |
| 334935 | | | CH22_FGENES.464_3 | 1.5 |
| 319764 | AA019827 | | EST cluster (not in UniGene) | 1.5 |
| 318519 | T27135 | | EST cluster (not in UniGene) | 1.5 |
| 332807 | | | CH22_ FGENES.7_9 | 1.5 |
| 322310 | AF086376 | | EST cluster (not in UniGene) | 1.5 |
| 324557 | AA489166 | Hs.156933 | ESTs | 1.5 |
| 332118 | AA609585 | Hs.162689 | EST | 1.5 |
| 319539 | R09027 | | EST cluster (not in UniGene) | 1.5 |
| 313149 | AW291092 | Hs.201058 | ESTs | 1.5 |
| 329722 | | | CH.14_p2 gi\|6065785 | 1.5 |
| 323514 | AA861209 | | EST cluster (not in UniGene) | 1.5 |
| 308078 | AI472621 | | EST singleton (not in UniGene) with exon hit | 1.5 |
| 337965 | | | CH22_EM:AC005500.GENSCAN.100-10 | 1.5 |
| 335905 | | | CH22_FGENES.635_13 | 1.5 |

**TABLE 14A shows the accession numbers for those primekeys lacking unigeneID's for Table 14. For each probeset we have listed the gene cluster number from which the oligonucleotides were designed. Gene clusters were compiled using sequences derived from Genbank ESTs and mRNAs. These sequences were clustered based on sequence similarity using Clustering and Alignment Tools (DoubleTwist, Oakland California). The Genbank accession numbers for sequences comprising each cluster are listed in the "Accession" column.**

| | | |
|---|---|---|
| Pkey: | | Unique Eos probeset identifier number |
| CAT number: | | Gene cluster number |
| Accession: | | Genbank accession numbers |
| | | |

| **Pkey** | **CAT number** | **Accession** |
|---|---|---|
| | | |
| 322064 | 234514_1 | BE261397 Z78343 BE176419 AA383657 N90640 AA334052 AW955761 BE536232 AA374087 AA584776 |
| 321409 | 197898_1 | N71838 AA282003 T54072 AA761419 H92966 AI831371 AI095435 AI690247 R99331 AW964110 AA975590 AA346128 H94196 C03864 |
| 322092 | 46678_1 | AF085833 R69689 AW341677 AA923375 BE327566 AW630415 R69601 AW615339 |
| 321452 | 212379_2 | AW962489 H64300 AA329527 |
| 313603 | 199797_1 | AA284333 AW468119 AA284334 AA810992 |
| 320856 | 36098_1 | AB040928 T94673 AI289313 AI536039 Z44366 BE141499 D60116 D61488 D59945 AA419503 R28090 R72986 H03255 AI189112 AI912312 AW511018 AI401349 AW470144 C14624 AI335797 Z40300 AI014456 D60269 D60115 T16722 AI370673 D60270 |
| 322139 | 46806_1 | H53744 AF075088 H53797 |
| 321500 | 552826_1 | BE004271 AI248023 AI022157 H71999 |
| 313733 | 441212_1 | AA766346 AA809877 AA836116 AW469598 AW977404 |
| 322215 | 47002_1 | AF088005 N51816 N51731 |
| 322235 | 47070_1 | AF086106 AI193589 AW665594 N71795 AA722627 AW665373 AI300251 |
| 321632 | 286374_1 | AW812795 AA419617 H87827 AW299775 AW382168 AW382133 BE171659 AW392392 BE171641 AA541393 |
| 313833 | 120893_1 | AA766825 AA811180 AA085906 AI762946 AW977820 |
| 322310 | 47376_1 | AF086376 W77804 W72689 AA837735 |
| 322313 | 47386_1 | AF086386 W77947 W72708 |
| 322322 | 47434_1 | AF086431 AA886756 AI557237 |
| 322331 | 47467_1 | AF086467 W81444 W81445 |
| 322345 | 47537_1 | W95298 AF086529 AI912190 AW294159 AI458747 W94782 |
| 322347 | 47545_1 | AF086538 W95969 AI631911 W95835 |
| 322370 | 187612_1 | AA330095 W25112 AA249401 |
| 321739 | 43998_1 | AL080280 T73124 H02689 AL080281 |
| 321781 | 1511778_1 | D78667 D78871 C18258 |
| 314570 | 280469_1 | AA904776 AA405696 AA405962 |
| 300129 | 635249_1 | AW028820 AI219068 |
| 322452 | 497108_2 | AI147202 W56755 W56710 |
| 321861 | 1651920_1 | N79341 N99082 N47551 |
| 323140 | 159551_1 | AA180467 AA449184 AA464831 AA505048 |
| 322520 | 38916_1 | T55958 T57205 AF147346 |
| 321914 | 85114_1 | AA011603 N58604 N58611 |
| 322571 | 22297_1 | NM_016102 AF156271 AA781868 AW152318 AW770403 AA909463 AA482996 AA758672 |
| 322574 | 39412_1 | AF156548 AA639797 AI675267 AI825497 AI823355 |
| 314753 | 311451_1 | AA463262 AA463615 AW160405 AW407583 |
| 300370 | 3910_2 | AW136181 AA581939 AK001221 AA694538 AA424043 AI016272 AA098960 AA884473 AI356180 BE391633 AA437086 AI277866 AA098827 AA992680 BE172624 AA424101 AA320776 AW962967 N77431 AW858960 AW858897 T85649 AA357743 AI827817 AI905672 |
| 322601 | 577912_1 | AI082395 W92924 BE048524 AW005302 AI084474 AI369330 AI827710 AW135506 AW298694 |
| 322613 | 34330_1 | AW160507 NM_013367 AF191338 AA384939 AI445790 AA730309 BE397003 BE267753 AI979163 N50386 AW583671 AW583608 BE074466 BE074479 BE074471 AW976283 AA604393 AW162122 W73648 AI823475 N75898 W73713 AW470099 AW513236 AW025055 AW613115 AI923379 W58081 AW664525 AW196795 AI143619 AI565152 AA025406 AA505846 AI685494 AA829964 N59156 N59163 R15442 AA826919 AI610221 AI200120 AA603279 AW150822 AI189513 AI807122 AI016368 AI335868 AW583389 AI193892 AI956157 AI628879 AW591589 AW583446 AI955406 AW148396 AI340255 AI867942 AA748525 AA876991 Z38516 AI874002 AI869474 N63100 AA429094 AA082443 |
| 316055 | 409389_1 | AW105663 AA693880 AW517398 AI768507 BE220851 AW978538 AA831489 |
| 323316 | 981458_1 | BE219300 BE327455 AL134620 R36741 R17996 |
| 300492 | 25768_1 | AL031709 AI249061 AA907658 AI420444 |
| 316141 | 423880_2 | AW303457 AA972713 AA724265 |
| 323371 | 117336_2 | N45114 N51465 BE087338 AI083551 AL135118 BE395609 |
| 307700 | 30923_11 | BE280998 BE254670 BE294951 BE564979 AW405364 AA069256 AA129837 AI559667 BE281405 AW410850 BE041153 AI254811 AW301340 AI613335 AW301411 AI609469 AI611607 AI611616 AI377623 AI335509 AI613544 BE043165 AI371663 AI340452 AI612066 AW072890 AI254558 AI349884 AI370095 AI613383 AI611946 AI613353 AI307414 |
| | | AI318229 AI612685 AW305327 AW268924 AI370063 AI349292 BE049068 AI369098 AW274098 AI344845 AW075187 AI053401 AI345220 BE138515 AI613386 AI583302 AW301955 AI349661 AI307432 AI054168 AI223913 AI612081 AI348942 AI334539 AI309366 AI370098 AI252360 AW086316 AW268911 AW073482 AI379802 AI224284 AI053661 AI334538 AI309369 AI309688 AI310023 AI492709 AI335418 AI053999 AI366989 AW073478 |
| | | AI247058 AI249584 AI305875 AI308585 AW071272 AI271487 AI340719 AI366995 AI223673 AW271066 AI611936 AW071296 AI270796 AI254385 AI251393 AI252562 AW268236 AI254858 AW071317 AI309102 AI609897 AW268971 AI583267 AI792484 AW075168 BE138443 AI254126 |
| | | AI309822 AI310872 AI611953 AI251054 AW276658 AI335405 AW075639 AI311768 AI612028 AW271895 AI612005 AI312240 AW271082 AI371642 AI334879 AI310194 AI310772 AI345419 AI334675 AI223914 AI284707 AI284813 AI349140 AI254853 AI313094 AI310170 AI309499 AI312476 AI376484 AI335467 AI340802 AI309815 AI310168 AI611446 AI345824 BE327775 AI318545 F17185 AW614950 |
| 308362 | 792518_1 | AW998989 AI613519 |
| 307783 | 697809_1 | AI347274 AW844024 |
| 301161 | 427238_1 | AA731518 AA765714 |
| 324094 | 270098_1 | BE395109 AW663898 AW237041 AI492154 BE046906 AI651285 AI983290 AW002590 AI201040 F32424 AA992272 AW271836 |
| 309023 | 4737_1 | AF180681 NM_015313 AA229509 AA225792 AA216413 AI888045 BE005205 AB002380 T55518 BE276097 AW380669 BE142836 AW370976 AA479384 R96425 AI680999 AA595138 H54582 AI022709 T55440 AI041769 AA861144 AW392028 AA479287 AA824634 AI638446 H54691 |
| | | R96382 AA770352 AI640467 AW293491 AA779138 R28298 AA970562 C15590 R84455 AA020769 AL036394 H80566 BE548861 AA301207 AW959414 AI284253 AA043173 W52429 BE544571 R24852 Z42603 F13120 R24340 R24326 T75305 H70110 N56255 AA334210 F11453 AW947285 H80345 AA298992 AW380931 AI267175 |
| | | Z45421 AW380981 W86113 AA663590 AA167577 BE566760 BE169166 AA449904 AA459205 N31126 W03564 N31208 AW993277 N44765 AW605275 D61449 W68572 AA258190 D60496 AW992964 U46277 H04097 AA370360 AW957211 AA159775 AI631243 H83367 H21671 D61077 AW392712 N21112 H98522 N45298 N83629 AI393509 AW022043 |
| | | AA744886 AI580482 AA723286 AI422244 AI423984 D62804 AI088349 AA587890 AI144172 N33275 BE074397 H03399 D62578 AI056639 AI829918 AA579584 AI089460 AI350124 W68573 AI580828 H98897 AI570468 H83715 W86114 AA923123 D57446 AA043174 |
| | | AW337721 AI266551 AI140017 AW022356 D79855 D79650 D79393 D60495 AA788666 AA693443 AW516977 W60139 AI628156 AW473223 AI608892 AA159670 AW440366 AI421529 T50751 AI174374 AA912234 AA724248 AW780400 AA907218 H80514 D57452 AA863419 AA552618 D29614 R44556 T16452 R44935 Z41132 D29188 |
| | | H69692 AI250176 AI078860 AA370359 AW183108 H74200 AA258183 F10723 C00323 R86148 AA860570 AW130073 AL079946 AA410327 AA532614 AA234500 AI151507 AA410288 AW969839 AA483232 AI383200 AA236540 AI807672 H73441 |
| 323473 | 193878_1 | AA262442 AA768862 AA262443 |
| 315639 | 392767 1 | AA827650 AA827652 AW629526 BE044585 AW974451 AA761439 AA648505 AA765803 |
| 322878 | 117013_1 | AA081820 AA082191 AA079811 |
| 301239 | 457668_1 | AA807558 AA827117 AW629567 |
| 301256 | 16720_1 | NM_016603 AF251038 AI124624 AA776579 AW298470 AI304868 AW082724 AI348442 BE218336 N20641 AI018013 AW858832 AW978157 AA815187 AA932948 AF157316 AI444958 W00848 W02935 AI434933 N26335 AA428681 AW371059 AI651612 AW134937 AW968911 AA488815 AL157523 W48766 AW936954 AW936941 AW579205 AW936886 AW936889 N74541 AW936953 AW578421 AW604352 AW367088 AW849258 AW849453 AW371606 AI554921 W49785 H99814 AA805957 AA904606 AW206696 BE169229 AA333951 AA190704 AW936944 AA463219 AA430306 AW805704 N48503 BE222307 AI638612 BE550045 AI805304 AI690987 AA776841 H12690 AW183731 AI380760 AI636261 AA812641 AW592656 AI686132 AA843424 H99220 AW084996 AW128879 AI800871 AA610135 AA191524 AI150076 AI474530 AA748461 N29013 AA746372 N59606 |
| 300611 | 337193_1 | N75450 AA877636 AW137945 W05248 AA514763 AW972399 AI758397 AW195051 |
| 324157 | 247225_2 | AW402931 BE393099 |
| 323509 | 967739_1 | AL036947 T93676 T85475 |
| 323514 | 197787_1 | AA641735 AA281881 AA861209 AA934756 AA835887 AA641795 AA748822 AW295703 |
| 300674 | 466093_1 | AW467388 AA826954 |
| 322932 | 39838_1 | AF168711 AA099732 BE019157 AI380212 BE298159 AA249097 AA305112 AW962349 AW962353 AW401801 BE292961 AI439469 AA442919 AI630537 AA724473 AI814288 AW966815 AI376871 AI860202 AI683132 AA099733 AW627633 AI754022 BE206347 AW183349 AI378222 BE178926 AI473282 W52944 AW752469 AW966817 |
| 323591 | 209807_1 | AA301270 AA301379 AA301366 |
| 322950 | 10774_1 | R85652 AA114024 AA296219 AA375304 AW963796 AW885952 AW020969 AA114025 AI804930 BE350971 AI765355 AW317067 AW974763 H85930 AW172600 AI310231 AW612019 D62908 D62864 AA652738 AI674617 AI494064 AW138666 AI147620 AI147629 AW611793 AI668922 AI971005 AI864742 AA174171 |
| 322957 | 29014_1 | AK001701 AA134337 AA356202 BE163251 AW875175 AW875181 AW875177 BE163389 AK000741 AA247755 AA120819 AW868040 AA309118 AW962348 AA471267 AW996843 AK001452 BE005344 BE617899 AA186588 AA120820 AW363311 AA648105 N71529 BE168417 AW673900 AI858160 AA134338 AA659697 N22162 AI335437 AI311237 AI343171 AI336661 AW268074 AW274348 AA935005 AW576295 AW262626 AW593153 AA730055 AA662650 AA782687 AW894855 AI933533 AW193002 AW899448 AW890142 AW812670 AA085664 AA334191 BE178085 BE180553 AA389680 AA984772 AA442527 W26560 BE384359 AA847210 AW304931 AI669606 AA085613 AW197240 AI632828 AA581646 AW129348 AI017643 AW089030 D20893 AI382955 AI557148 AW499979 |
| 324231 | 975669_1 | W60827 AL079968 AL047234 |
| 324248 | 977901_1 | AW504918 N55410 AL118584 AW839266 |
| 323691 | 221757_1 | AA317561 AI793000 AW235111 AI793178 AA767397 AI263113 AA719462 |
| 315858 | 406384_1 | AA737345 AA682286 AI799378 |
| 301431 | 569736_1 | R05385 AI061251 |
| 324303 233842_1 | | AL118754 AA333202 H38001 |
| 324330 | 300543_1 | AA884766 AW974271 AA592975 AA447312 |
| 300815 | 41537_2 | BE152396 BE152395 AA287515 BE001834 AA286678 AW406477 |
| 324349 | 1154015_1 | AW501470 AW502931 AW499500 |
| 323715 | 225129_1 | AA322155 AA326396 AA326538 |
| 309314 | 23273_-3 | AW009312 |
| 323758 | 229624_1 | AA833858 AW978090 AA327679 AA810436 |
| 309375 | 127_1 | AF286598 AW075342 AB028994 AL043713 AW378914 AA340650 N57166 AW956914 R17961 AA336481 BE393734 AW977867 AW294638 AA927857 AA961627 AW303969 AW894416 AA812119 AA912758 AA424355 AA490582 W30941 AA476693 AA131029 AA127777 AL043714 AA496984 T51117 AA127722 AA594012 AI492876 N76483 AW119061 BE464926 AW303419 AI972370 AI768172 AI826550 AI435432 AI379516 AA778421 AI276089 AA424521 N59361 AA723153 AA723176 AI867487 AA090677 AI827221 AI351027 W02732 AI810729 AA142848 AI082110 N59379 N29744 AI283747 AI148665 AW779845 AI382967 F34319 AI369934 AI282438 AW183449 AA863467 AA813469 AI092645 AI870701 AA863119 |
| 325031 | 266373_2 | T65475 R07576 T17017 F08143 Z43546 |
| 325045 | 1534945_1 | T08845 Z43538 F06691 |
| 324473 | 38795_1 | BE560824 BE513941 AW238907 AA580852 AW501176 BE241846 AW501163 AW751433 AW501340 BE241715 AI910774 |
| 323827 | 235506_1 | AW406878 AW966560 AW966151 AW966496 AA336174 AA335376 AA335537 |
| 302270 | 1734192_1 | R56151 W91936 |
| 301618 | 10967_5 | T52761 T52760 |
| 301646 | 42154_1 | AJ277841 AI630669 AI804370 Z41939 AW751251 AA299456 Z44739 AW860471 Z30158 AW105391 H56997 W84688 AA491201 W84636 AA706815 AI131055 AA483636 AI005075 AW340034 AI332372 AW118195 AI338932 AI191968 AA693932 AI189982 AI193225 AA884163 AA594562 W37747 AA249754 AA746131 AI916540 AI832188 AW946555 AA833838 Z40564 AA861563 F01447 AA887937 AI933659 AW973250 AA566018 AA313954 |
| 323923 | 249295_1 | AA354146 AI184230 AA643525 |
| 324580 | 328264_1 | AA492588 AA492498 AA492571 |
| 316774 | 463723_1 | AA814859 AA814857 AI582623 |
| 309577 | 6483_6 | AW902251 AW168753 |
| 302345 | 29533_1 | X12830 NM_000565 AW503691 X58298 S72848 AA193347 AW503481 AW177946 AW178192 AW178188 AA285233 AA410577 AA193465 AW177939 AW365459 BE221693 |
| 302358 | 1064753_1 | AW207734 D60164 D81150 D81078 D61356 AW996804 |
| 324614 | 215437_1 | AW503101 AA309184 N56323 R70998 |
| 324661 | 385257_1 | AW504161 AW503601 AW505509 |
| 324685 | 41003_1 | AF226667 AA207032 AA100804 AA121287 AA488316 AI808218 AW419048 AI911097 AW132123 AA502311 AW089948 AA100952 AI075431 AW083432 AI990554 BE466029 F28643 AF086422 W79581 AW439007 F37179 W79780 AW439035 AA731381 AW750380 AA251012 AW589846 AA730238 AA329792 AW087255 AA220982 AA082469 AA877260 AA232380 BE298910 |
| 324692 | 351987_1 | AA557952 AA677593 AA618150 |
| 316893 | 473541_1 | AW978189 AA837332 AA856946 AA876935 |
| 303027 | 21796_1 | AF111178 NM_005708 AF105267 AW590040 AI979280 AA001322 BE146329 AA702430 AA702429 AA694221 AI206348 AI206285 AW770197 AA923032 AI379586 AA701165 AW594643 AA001909 AW002368 |
| 324715 | 290035_2 | AI739168 AA426249 AI199636 AW505198 AW977291 AA824583 AA883419 AA724079 AI015524 AI377728 AW293682 AI928140 AA731438 AI092404 AI085630 AA731340 |
| 324771 | 385085_1 | AA631739 AA768584 AW134477 |
| 324783 | 389615_1 | AA640770 AI683112 AA913009 |
| 303114 | 37417_1 | AF090948 AI064898 AI111182 |
| 303124 | 21112_1 | AB018257 BE148640 AA081832 AK001915 AF150217 AF161350 AI219174 AW074664 D60040 AA346065 H28750 AW151783 BE613360 BE612628 BE502031 AW183790 AA992580 AA505815 AI310432 AI678015 AW592679 AA879181 AA806708 AI744110 H24681 C16064 D62900 AI285033 AA346064 AI865123 AW467798 BE221231 AL120676 N89877 AI928370 AI358387 AA748486 AV647478 AV647460 AA312313 AI279340 AW505099 |
| 302552 | 82290_1 | AA005122 H49792 |
| 301918 | 316229_1 | AA476777 T86049 |
| 303232 | 20474_1 | AA437414 AA131479 AA086182 AB037775 AW161063 AW514393 AA332331 AW136197 BE150789 AA425533 AA249605 N88308 AI016201 BE004662 AA291027 R57587 AA424277 AA476391 W07532 T97036 AA218898 AW162629 R57770 W01278 W90204 W90156 AL119197 R84513 AA280103 AA334994 AW965504 AA460868 AA447470 AW138594 W38898 W90028 AI078353 W90078 AA699696 N35523 AA704225 AA035059 AW134892 AA115140 AI142854 H90084 AA826342 AA460694 N46339 AA425344 N56953 AA035569 AI761083 AI658696 AI524818 AI338965 AW069249 AW299871 BE464061 AI189720 AW340682 AI423380 |
| | | AI275122 H17532 N80735 AA826343 AI039694 BE328398 AI192947 AW271286 AI623122 AI922902 AW293087 N22141 AA730657 AW316610 N26473 F06663 Z43610 H14783 R59761 H11540 AI265915 AI681773 AI091748 BE220636 AW841861 AI702181 AI468447 AA907544 AI273941 AW244034 R37769 AA446663 T96929 BE045884 AA476341 H89994 H29043 AW051211 N49522 AA306977 |
| 302696 | 33570_1 | AK000738 AA347452 AW961713 H70832 AI750643 AA362887 AW955588 W44974 AA279599 AW298762 AA452666 AA443355 AI337273 AA446931 AI752977 AA661554 W42674 AI292172 R41163 AA621381 AI244157 |
| 302697 | 43219_1 | AJ001409 AJ001410 |
| 309917 | 57485_2 | AW340014 AW866993 AV651649 |
| 303347 | 192210_1 | AA258033 AA459485 |
| 303349 | 193138_1 | AA382661 AW958642 AA259086 |
| 310599 | 690880_1 | AW300144 AI338491 AI798381 BE220076 |
| 303388 | 969232_1 | AL039604 AL039497 |
| 302761 | 45074_1 | AW250553 L07876 Z36843 R30693 AI190097 AW965317 |
| 318455 | 606341_1 | AI148763 AI903763 AI903753 AI903762 AI903800 AI903801 |
| 317850 | 363835_1 | AI681545 AI951714 AI570397 AW873588 AA836396 AI359986 AI499790 AA773477 AI951615 T07547 AW304709 AF114041 BE176629 Z44580 T30422 T32690 AW953065 H10602 |
| 303431 | 32082_1 | NM_000539 AA019013 AA019367 AA056154 H38735 AA057003 AA021051 H38102 AA015774 AA059291 AA019439 H84843 H83375 AA019914 AA017288 R84449 W26519 H38258 AA018736 H84147 AA018577 AA059353 U49742 H38767 AA318341 AA317553 H86646 H91989 AA317398 AA317378 W29024 W23034 T27877 AW950059 AA017195 R84262 AA057177 H89941 AA019904 H84662 AA015775 AA019368 AA020976 H37900 C20733 H38682 H85197 AA018578 AA017252 AA019440 AA059059 H38651 H84148 AA018560 W25754 C20752 AA317915 AW952115 AA317369 AA019845 R85402 AA019492 AA017196 AA056093 AA056094 AA058836 AA056155 W25957 W23027 AA056159 W23043 W21890 W28951 AA317978 W26459 AA317265 |
| 319127 | 1653640_1 | N49476 Z45911 R21061 |
| 303480 | 232749_1 | AA331906 AA332484 |
| 303481 | 31534_1 | AK001952 AA336839 AW249271 BE247287 AF182002 BE613472 AW962673 AA332235 AW849937 AW849814 H49893 AA477148 AW968944 AF182003 AW007897 BE246145 W76100 AI480141 AW410205 AA609339 AI209111 AW000979 AA330280 AW961554 W72865 H49894 AA514317 AA620407 AA504522 AW472833 AA716609 AW129282 AA347351 AA628378 AW589860 AI636696 AA464632 AA464533 AW874189 AA757076 AA479654 AW517910 AW292357 AW872638 AW262288 AI910666 AW513749 AW238771 AA215797 BE387073 |
| 303487 | 20890_1 | BE143533 AW850432 AK000042 AA333666 AA385314 AW966616 AW793068 AW793414 AA361103 AW390841 AA040095 AW385058 AW799162 AI383115 AI990745 AI653703 BE503693 AW150758 AI949919 AW190450 AW512348 AI625970 AW501057 N52954 AI281378 AI401710 AI648409 AW002659 AI687639 AI093943 R33960 AA040062 AI926267 AI240425 AI520911 AI093428 R52943 |
| 303488 | 36085_1 | AI040372 AB040915 W40569 BE158910 BE158914 D63226 AW025860 AW583088 AA334307 AA210942 AW753212 AW805322 AA362635 BE158911 AW891225 AW994862 AA805451 R28541 AA229347 N48266 AI377788 R28682 R36122 AA811941 AI240742 AI632001 T99965 W01976 AW891205 AW891177 T97433 C15571 AA346850 AA504293 W07500 AI694503 AA489216 AA327725 AW959917 AA694146 N68514 AI076285 AW016246 T07783 AA642400 AA716133 AA805332 R00312 AA705021 AW498605 AW891723 AW891906 AA808025 N29039 N74897 W60393 AA810184 AI627460 AW057516 AA807436 AA760966 AI359295 N78642 N20662 AA830300 W81705 AA832258 AW891718 AI811796 AW515523 Z41735 AA449978 AW891714 AI684539 AW891896 AW071701 AI890916 AI924994 AI039743 AA888524 AA244214 AI015736 AI270105 AI865077 |
| 303494 | 236389_1 | F30712 F35665 AW263888 AI904014 AI904018 AA336927 AA336502 |
| 319142 | 164820_1 | H08370 Z46168 F07366 AA193168 AA193138 |
| 302868 | 12593_1 | AK000290 AI476034 AA465309 BE148761 AW303607 AW958665 AW469635 AI819365 AI243857 AW469326 AA157110 AA278626 AA496257 AA306656 F29732 AA831859 AA312210 AA564476 AA579065 AA769522 AA740386 AI205635 AA491643 AA810400 AA417708 AI567332 AA157392 N53817 AA374229 |
| 318518 | 1205335_1 | R68545 T27119 R25687 AW750672 |
| 318519 | 434741_1 | H13364 T27135 R61679 AA746905 |
| 304168 | 72494_-10 | H77679 |
| 302948 | 21445_1 | AB038995 NM_016530 AK001111 AA465635 AW968716 U66624 AA885459 AA703019 AI040266 AI018689 AI692886 AI125372 AI376796 AI192040 N58161 AL133607 AW503673 AW505479 AA362265 AJ404671 |
| 319250 | 244351_1 | F11623 H17552 AA347728 |
| 318644 | 17700_1 | BE311816 AK000916 AW868037 AW868039 AF228527 AI752482 AW868041 AA077049 AI201537 W55873 AA206019 AA077918 AW968729 AI978828 AW139620 AI093053 AW204025 AI418805 AA598926 AA586345 AA045669 BE314455 AA045668 |
| 318674 | 204968_1 | W01166 AW996900 BE184300 Z44887 T34535 R51495 AW886575 AA295490 AA295162 AA295163 AW937125 T56951 |
| 304232 | 20640_2 | BE386106 W52674 |
| 303685 | 8088_1 | AW500106 BE241915 AW503971 NM_016542 AB040057 AA313812 AK000556 W16504 AI822088 AA259107 AA191319 BE085957 AA309584 BE122687 AW952435 T84469 BE088194 BE088132 AA328562 BE092674 AA263102 T39634 AW992380 R79391 R24392 H03060 AW675066 AI299952 AW020325 D25953 N75199 AA361425 AW612302 AW236333 AW673897 AW953686 N22323 AA649166 AI377099 H03061 AI660072 AW276405 AA809779 AI803430 AW297484 AW510384 AA814816 AA371522 D63035 AA953567 R79392 R24282 AA876831 AW297542 AI699023 AA992652 AI041436 |
| 318704 | 799152_1 | AI631602 AW589676 Z28684 Z24981 |
| 318730 | 275116_1 | Z32887 BE349923 AA398215 AA399231 |
| 303714 | 1155758_1 | AW501336 AW501337 |
| 304387 | 183612_1 | AA236027 BE003275 |
| 304398 | 10169_1 | AA195509 BE394661 AV660757 AA489161 BE165972 AW503705 AA262785 AF123320 Z78357 NM_014171 AF161488 AA248971 BE568575 AA461410 AA165108 AI637731 H75454 AA372934 AW339334 BE568754 BE564697 BE567299 AI681606 BE537269 AW197204 AA290890 AI189393 AW292463 AW470227 F27399 AW611942 BE566888 AW301701 AI675761 AI628429 AA164711 AI797753 AI656879 AI912690 AI675277 AI695099 AI094095 AW014158 BE091059 AI201748 AW236961 AI038003 AI083606 AA401606 AI079405 AI073516 AI655537 AA401475 AI814532 AI079862 AI093789 AI422084 AI216476 AI392760 AA926998 AA781782 Z25198 AI086377 AI185511 AI185539 Z28843 AI223792 AI379563 AA706253 AI433798 AI921885 H75455 AW025269 AI224100 AI083611 AI225057 AW196334 AI572254 AA761628 AI472801 AA283784 |
| 303751 | 468554_1 | AA830149 AW978407 M85983 AW503637 |
| 319401 | 1323199_1 | W00973 N56457 AW992226 T84921 R01342 |
| 319402 | 1003489_1 | R86913 R86901 H25352 R01370 H43764 AW044451 W21298 |
| 318807 | 1536467_1 | F08434 Z42573 H28810 |
| 319478 | 765461_1 | AI524124 R06841 R06842 |
| 318872 | 1534581_1 | Z43108 F06295 R13085 |
| 318885 | 94880_2 | AA742999 Z43272 AA345258 AW956677 AA031942 |
| 303841 | 79133_1 | W19657 BE616760 BE259848 BE382680 BE615587 AI934464 AA322745 T07155 AW961174 AA307302 Z41888 AA621992 AA188400 AW770608 AI147458 AI148408 AI696291 AA972591 |
| 303889 | 1777183_1 | T19204 T36109 T36107 |
| 319539 | 63198_1 | R09027 AA344892 AA329574 AW955648 AW978708 AI567804 AI378935 AW014657 AI804134 R08922 N92947 BE546788 |
| 318905 | 1536408_1 | F08365 Z43395 R54298 |
| 320187 | 396254_1 | T99949 AA654769 AA664550 AW975264 |
| 318996 | 65715_1 | Z44266 H06384 AV655948 |
| 319635 | 163534_1 | R17531 AW960899 AA338366 AW673294 BE047729 BE047722 AA330746 AW841797 H05030 AI142105 R12654 |
| 319699 | 747196_1 | AI458682 H24240 R14537 R18426 AW867082 |
| 319713 | 1699356_1 | R24204 R15712 T84695 |
| 319761 | 75324_2 | AW630974 BE005208 R84237 AA724997 AA334867 AW955777 R18816 |
| 319764 | 88596_1 | AA019827 R18947 H46852 |
| 319808 | 7069_3 | T58960 AA609180 AA621130 AI927236 AA431075 |
| 321040 | 193331_1 | AA261830 AW967855 H26953 AA262478 |
| 320409 | 43709_1 | AA226869 AA296516 AW959753 AA186390 AL359619 AA356195 AA148427 R22748 AI033624 BE548853 H95327 AW579751 BE561649 AA397533 BE617136 AA236444 T89946 AA247450 N55777 W38725 AI743846 AI808406 AA922229 AI051464 W04713 R11251 W19656 AI042319 AA489276 AI224533 H95274 AW269958 T89311 AI890088 AI862754 AI830968 |
| | | AI669336 AI589780 AA534557 AW273839 AI338155 AI126632 N83542 BE046048 AA807028 AA848107 AW167978 AA976930 AA148428 AI289304 AI524262 AI625961 AA773469 AI222288 AI280054 AI242371 AA227222 AA973329 AA296517 AA829436 AA234526 AI149769 AI567865 AA936939 AI590681 AW469308 AI689531 AA486419 AI422051 AI057252 AA626941 AI475352 AW247913 AI222370 AA670122 AW198034 AA486418 AI363794 AA380739 |
| 319881 | 1585983_1 | H51299 H44619 H46391 R86024 H51892 T72744 |
| 320488 | 368456_1 | AI817336 R32883 AA595590 AI743065 R31386 |
| 321121 | 1545647_1 | W23285 H42714 F25381 F37215 |
| 321205 | 81249_1 | AA002047 N72537 H54142 H81580 |
| 321253 | 375160_1 | AA610649 AI699484 H59558 |
| 314043 | 155125_1 | AA827082 AA732246 AA167611 AA830741 |
| 320630 | 17685_2 | AA199847 AA410224 R53323 AW936567 AW936569 AW936568 AW936571 |
| 313435 | 443527_1 | AA769123 AA831715 AW977666 W92553 |
| 313443 | 82292_1 | AA005125 W95019 W93335 AA249037 |
| 313472 | 82811_1 | AA007374 AA007466 AI816886 |
| 321348 | 41762_1 | Z49979 D61703 U30168 |
| 314138 | 179960_1 | AA740616 AA654854 AA229923 |
| 320712 | 57156_2 | R66867 R65678 R82673 W73128 R83101 |
| 321363 | 41924_1 | AW968556 AJ238555 AW968731 AJ002574 AA459446 H70260 AW977557 AA767351 AW268572 AA810719 AI698677 AI300460 AA907450 AA649224 T07415 AI536896 BE018515 AI279865 BE047421 |
| 312996 | 187327_1 | AW368634 AI702169 AI245179 AW368646 BE545574 AA249018 AW368633 N27553 |
| 306513 | | AA989230 |
| 306537 | | AA991705 |
| 306557 | | AA994530 |
| 306598 | | AI000320 |
| 306620 | | AI000929 |
| 306700 | | AI022056 |
| 308078 | | AI472621 |
| 306813 | | AI066544 |
| 306830 | | AI075803 |
| 306855 | | AI083982 |
| 329722 | c14_p2 | |
| 329728 | c14_p2 | |
| 306890 | | AI092235 |
| 308100 | | AI475949 |
| 308147 | | AI498991 |
| 306929 | | AI124514 |
| 308352 | | AI610791 |
| 308383 | | AI624497 |
| 308521 | | AI689808 |
| 308561 | | AI701559 |
| 308617 | | AI738720 |
| 308771 | | AI809301 |
| 308828 | | AI824829 |
| 308896 | | AI858667 |
| 303019 | 41850_1 | AF098363 AF098365 |
| 303084 | 44211_1 | AF174008 AF174027 AF174106 |
| 305092 | AA642912 | |
| 305169 | | AA663131 |
| 305177 | | AA663591 |
| 305235 | | AA670480 |
| 305413 | | AA724659 |
| 305849 | | AA861571 |
| 305854 | | AA862733 |
| 307113 | | AI183686 |
| 307130 | | AI185234 |
| 305937 | | AA883238 |
| 305977 | | AA887293 |
| 307451 | | AI248615 |
| 307513 | | AI274307 |
| 307848 | | AI364186 |
| 307871 | | AI368665 |
| 307881 | | AI370434 |
| 307932 | | AJ230822 |
| 307944 | | AI418246 |
| 307954 | | AI419692 |
| 307965 | | AI421641 |
| 309245 | | AI972447 |
| 309271 | | AI986221 |
| 309365 | | AW072861 |
| 309372 | | AW074330 |
| 309435 | | AW090537 |
| 309506 | | AW137700 |
| 309536 | | AW151933 |
| 309709 | | AW242630 |
| 325417 | c12_hs | |
| 325450 | c12_hs | |
| 325452 | c12_hs | |
| 309815 | | AW292760 |
| 309839 | | AW296076 |
| 309849 | | AW297444 |
| 309906 | | AW339340 |
| 302705 | 31765_1 | U09060 U09061 |
| 304037 | | T26438 |
| 304039 | | T47349 |
| 304236 | | W93278 |
| 304257 | | AA053294 |
| 304382 | | AA232873 |
| 304405 | | AA282572 |
| 304561 | | AA489792 |
| 304569 | | AA490934 |
| 304787 | | AA582678 |
| 304921 | | AA603092 |
| 327819 | c_5_hs | |
| 304968 | | AA614308 |
| 306382 | | AA968967 |
| 331263 | 47479_1 | AW780192 AA015718 W02571 |
| 332252 | 1663967_1 | N63882 T91174 |

**TABLE 14B shows the genomic positioning for those primekeys lacking unigene ID's and accession numbers in Table 14. For each predicted exon, we have listed the genomic sequence source used for prediction. Nucleotide locations of each predicted exon are also listed.**

| | | | |
|---|---|---|---|
| Pkey: | | Unique number corresponding to an Eos probeset | |
| Ref: | | Sequence source. The 7 digit numbers in this column are Genbank Identifier (GI) numbers | |
| Strand: | | Indicates DNA strand from which exons were predicted. | |
| Nt_position: | | Indicates nucleotide positions of predicted exons. | |
| | | | |

| **Pkey** | **Ref** | **Strand** | **Nt_position** |
|---|---|---|---|
| | | | |
| 332807 | Dunham, I. et.al. | Plus | 297686-297808 |
| 332808 | Dunham, I. et.al. | Plus | 298277-298360 |
| 332812 | Dunham, I. et.al. | Plus | 309688-310561 |
| 332901 | Dunham, I. et.al. | Plus | 1841954-1842090 |
| 333149 | Dunham, I. et.al. | Plus | 3574317-3574413 |
| 333916 | Dunham, I. et.al. | Plus | 8298994-8299169 |
| 334026 | Dunham, I. et.al. | Plus | 9196549-9196681 |
| 334061 | Dunham, I. et.al. | Plus | 9686941-9687077 |
| 334073 | Dunham, I. et.al. | Plus | 9792201-9792374 |
| 334150 | Dunham, I. et.al. | Plus | 10529221-10529854 |
| 334379 | Dunham, I. et.al. | Plus | 13908356-13908467 |
| 334719 | Dunham, I. et.al. | Plus | 15778859-15779026 |
| 334773 | Dunham, I. et.al. | Plus | 16235169-16235328 |
| 334893 | Dunham, I. et.al. | Plus | 19302753-19302881 |
| 334935 | Dunham, I. et.al. | Plus | 20108247-20108373 |
| 335146 | Dunham, I. et.al. | Plus | 21491292-21491457 |
| 335320 | Dunham, I. et.al. | Plus | 22542132-22542246 |
| 335568 | Dunham, I. et.al. | Plus | 24935021-24935655 |
| 335586 | Dunham, I. et.al. | Plus | 24990333-24990497 |
| 335601 | Dunham, I. et.al. | Plus | 25044923-25045157 |
| 336036 | Dunham, I. et.al. | Plus | 29019796-29019877 |
| 336123 | Dunham, I. et.al. | Plus | 30051089-30051186 |
| 336268 | Dunham, I. et.al. | Plus | 31997555-31998040 |
| 337173 | Dunham, I. et.al. | Plus | 23624127-23624224 |
| 337460 | Dunham, I. et.al. | Plus | 32536159-32536395 |
| 337685 | Dunham, I. et.al. | Plus | 3547161-3547245 |
| 337736 | Dunham, I. et.al. | Plus | 3850500-3850643 |
| 337780 | Dunham, I. et.al. | Plus | 4113793-4113990 |
| 337965 | Dunham, I. et.al. | Plus | 7034267-7034392 |
| 337976 | Dunham, I. et.al. | Plus | 7166011-7166119 |
| 338030 | Dunham, I. et.al. | Plus | 8072708-8072827 |
| 338112 | Dunham, I. et.al. | Plus | 10391398-10391600 |
| 338165 | Dunham, I. et.al. | Plus | 12205719-12205875 |
| 338178 | Dunham, I. et.al. | Plus | 12800037-12800181 |
| 338427 | Dunham, I. et.al. | Plus | 19685043-19685354 |
| 338506 | Dunham, I. et.al. | Plus | 21221871-21221953 |
| 338794 | Dunham, I. et.al. | Plus | 27114697-27114763 |
| 338910 | Dunham, I. et.al. | Plus | 28795375-28795551 |
| 339047 | Dunham, I. et.al. | Plus | 30760793-30760968 |
| 332864 | Dunham, I. et.al. | Minus | 1390386-1390296 |
| 332933 | Dunham, I. et.al. | Minus | 2035790-2035681 |
| 333193 | Dunham, I. et.al. | Minus | 3832993-3832494 |
| 333712 | Dunham, I. et.al. | Minus | 7286177-7286073 |
| 333940 | Dunham, I. et.al. | Minus | 8523830-8523671 |
| 333942 | Dunham, I. et.al. | Minus | 8552629-8552330 |
| 334287 | Dunham, I. et.al. | Minus | 13294116-13293871 |
| 334387 | Dunham, I. et.al. | Minus | 13946021-13945781 |
| 334487 | Dunham, I. et.al. | Minus | 14432191-14432132 |
| 334913 | Dunham, I. et.al. | Minus | 19463909-19463815 |
| 335109 | Dunham, I. et.al. | Minus | 21325792-21325667 |
| 335250 | Dunham, I. et.al. | Minus | 21952922-21952826 |
| 335288 | Dunham, I. et.al. | Minus | 22304275-22303770 |
| 335290 | Dunham, I. et.al. | Minus | 22309950-22309891 |
| 335549 | Dunham, I. et.al. | Minus | 24666203-24666128 |
| 335862 | Dunham, I. et.al. | Minus | 26690300-26690125 |
| 335864 | Dunham, I. et.al. | Minus | 26694537-26694382 |
| 335905 | Dunham, I. et.al. | Minus | 26988888-26988719 |
| 336205 | Dunham, I. et.al. | Minus | 30477456-30477311 |
| 336276 | Dunham, I. et.al. | Minus | 32093320-32093181 |
| 336433 | Dunham, I. et.al. | Minus | 34067540-34067425 |
| 336605 | Dunham, I. et.al. | Minus | 15616509-15616358 |
| 336616 | Dunham, I. et.al. | Minus | 26021027-26020848 |
| 336679 | Dunham, I. et.al. | Minus | 2035790-2035681 |
| 337043 | Dunham, I. et.al. | Minus | 17407330-17407251 |
| 337272 | Dunham, I. et.al. | Minus | 28241476-28241307 |
| 337357 | Dunham, I. et.al. | Minus | 30906179-30906109 |
| 337393 | Dunham, I. et.al. | Minus | 31471747-31471569 |
| 337497 | Dunham, I. et.al. | Minus | 33371317-33371258 |
| 337646 | Dunham, I. et.al. | Minus | 2648689-2648632 |
| 337920 | Dunham, I. et.al. | Minus | 6051648-6051510 |
| 338083 | Dunham, I. et.al. | Minus | 9318438-9318301 |
| 338220 | Dunham. I. et.al | Minus | 14166440-14166104 |
| 338752 | Dunham, I. et.al. | Minus | 26421374-26421135 |
| 338763 | Dunham, I. et.al. | Minus | 26628148-26628009 |
| 338983 | Dunham, I. et.al. | Minus | 29908865-29908702 |
| 339209 | Dunham, I. et.al. | Minus | 32492953-32492593 |
| 325240 | 5866848 | Minus | 32301-32650 |
| 329532 | 3983505 | Plus | 42937-43014 |
| 329522 | 3983507 | Minus | 35265-35458 |
| 329519 | 3983510 | Plus | 18407-18597 |
| 329511 | 3983514 | Plus | 20965-21325 |
| 325326 | 5866875 | Plus | 47726-48024 |
| 325303 | 5866908 | Minus | 73556-73630 |
| 325389 | 5866921 | Plus | 239672-239759 |
| 325417 | 5866925 | Minus | 110635-110745 |
| 325450 | 5866941 | Minus | 435379-435552 |
| 325452 | 5866941 | Minus | 704103-704202 |
| 325498 | 5866967 | Plus | 173372-173930 |
| 325587 | 6682462 | Plus | 126724-126967 |
| 325602 | 5866994 | Plus | 79122-79251 |
| 325701 | 5867028 | Minus | 72936-73046 |
| 325780 | 6381953 | Plus | 63634-63873 |
| 329722 | 6065785 | Minus | 112713-112992 |
| 329728 | 6065785 | Minus | 207544-207741 |
| 329666 | 6272129 | Plus | 98307-98446 |
| 329815 | 6624888 | Minus | 68431-68720 |
| 329841 | 6672062 | Minus | 40181-40331 |
| 325824 | 5867048 | Minus | 42450-42833 |
| 325866 | 5867076 | Minus | 94358-94628 |
| 325902 | 5867101 | Minus | 127729-127842 |
| 325958 | 5867142 | Plus | 53437-53550 |
| 326014 | 5867160 | Minus | 10358-10447 |
| 329941 | 6165199 | Minus | 34319-34411 |
| 330002 | 6623963 | Plus | 46097-46158 |
| 326154 | 5867170 | Minus | 7103-7179 |
| 326023 | 5867245 | Plus | 171799-171896 |
| 326278 | 5867269 | Plus | 75250-75903 |
| 330036 | 6042048 | Plus | 117120-117216 |
| 326547 | 5867307 | Minus | 623677-623870 |
| 326495 | 5867423 | Plus | 11843-11930 |
| 326507 | 5867435 | Minus | 13038-13111 |
| 326505 | 5867435 | Minus | 8818-8949 |
| 326506 | 5867435 | Minus | 9368-9509 |
| 326530 | 5867441 | Minus | 303000-303122 |
| 326508 | 6682496 | Plus | 78904-79112 |
| 330120 | 6671864 | Minus | 127553-127656 |
| 330123 | 6671869 | Minus | 35311-35406 |
| 326858 | 6552462 | Minus | 69337-69670 |
| 326983 | 5867657 | Minus | 16023-16581 |
| 327014 | 5867664 | Plus | 1017630-1017788 |
| 326930 | 6456782 | Plus | 606950-607705 |
| 326920 | 6456782 | Minus | 42425-42519 |
| 327058 | 6531965 | Plus | 2384268-2384835 |
| 327061 | 6531965 | Minus | 3486389-3486673 |
| 327075 | 6531965 | Plus | 4041318-4041431 |
| 327120 | 6531970 | Minus | 6-1088 |
| 330126 | 6093735 | Plus | 82458-82623 |
| 327157 | 5866841 | Minus | 4408-4746 |
| 327183 | 5867442 | Plus | 84317-84531 |
| 327192 | 5867445 | Minus | 194652-194764 |
| 327288 | 5867481 | Plus | 48583-48773 |
| 327469 | 5867772 | Plus | 145549-145708 |
| 327489 | 6004459 | Minus | 57796-58015 |
| 327526 | 6381882 | Minus | 97010-97123 |
| 327574 | 5867818 | Plus | 68767-69126 |
| 327665 | 5867839 | Plus | 141736-141900 |
| 327752 | 5867949 | Plus | 93721-94421 |
| 327819 | 5867968 | Minus | 92202-92717 |
| 327796 | 5867982 | Plus | 85267-85405 |
| 330260 | 6671884 | Plus | 45203-45269 |
| 330282 | 6671910 | Plus | 3982-4114 |
| 328078 | 5868008 | Plus | 72807-72865 |
| 328121 | 5868031 | Plus | 153782-153850 |
| 328190 | 5868077 | Plus | 21082-21165 |
| 328227 | 5868105 | Minus | 21082-21242 |
| 327871 | 5868131 | Minus | 88889-89221 |
| 328018 | 5902482 | Minus | 542547-543133 |
| 328624 | 5868246 | Minus | 120666-120836 |
| 328744 | 5868290 | Plus | 138639-138722 |
| 328799 | 5868316 | Minus | 80771-80923 |
| 328291 | 5868363 | Minus | 144244-144434 |
| 328329 | 5868375 | Plus | 191709-192239 |
| 328369 | 5868388 | Plus | 75371-75583 |
| 328385 | 5868395 | Plus | 369952-370155 |
| 328397 | 5868397 | Plus | 344967-345063 |
| 328412 | 5868405 | Plus | 86427-86519 |
| 328538 | 5868485 | Plus | 3814-4243 |
| 328656 | 6004473 | Plus | 792616-792729 |
| 328638 | 6004473 | Plus | 294618-294903 |
| 328903 | 5868514 | Plus | 23625-24468 |
| 328960 | 6456775 | Plus | 38547-38837 |
| 330320 | 5932415 | Minus | 54458-54697 |
| 328993 | 5868536 | Plus | 49160-50084 |
| 329081 | 5868602 | Plus | 93368-93510 |
| 329089 | 5868614 | Plus | 25805-26923 |
| 329109 | 5868626 | Plus | 102168-102273 |
| 329192 | 5868716 | Plus | 166936-167020 |
| 329218 | 5868726 | Minus | 71408-71707 |
| 329224 | 5868728 | Plus | 27422-27664 |
| 329246 | 5868732 | Minus | 250541-250792 |
| 329415 | 5868874 | Plus | 1011438-1011818 |
| 329454 | 5868887 | Plus | 51342-51593 |

**TABLE 15: 169 GENES WITH SEQUENCE INFORMATION DEPICTED IN TABLE 16 Table 15 depicts UnigeneID, UnigeneTitle, Primekey, Predicted Cellular Localization, and Exemplar Accession for all of the sequences in Table 16. The information in Table 15 is linked by EosCode to Table 16.**

| | | | | | |
|---|---|---|---|---|---|
| Pkey: | | Unique Eos probeset identifier number | | | |
| ExAccn: | | Exemplar Accession number, Genbank accession number | | | |
| UnigeneID: | | Unigene number | | | |
| Unigene Title: | | Unigene gene title | | | |
| EosCode: | | Internal Eos name | | | |
| Localization: | | Predicted cellular localization of gene product | | | |
| | | | | | |

| **Pkey** | **ExAccn** | **UnigeneID** | **Unigene Title** | **EosCode** | **Localization** |
|---|---|---|---|---|---|
| | | | | | |
| 100394 | D84276 | Hs.66052 | CD38 antigen (p45) | PBC1 | plasma membrane |
| 100452 | D87742 | Hs.241552 | KIAA0268 protein | PAB7 | not determined |
| 101249 | L33881 | Hs.1904 | protein kinase C, iota | OAA1 | cytoplasmic |
| 101485 | M24736 | | selectin E (endothelial adhesion molecul | ACC5 | plasma membrane |
| 101514 | M28214 | Hs.123072 | RAB3B, member RAS oncogene family | PFJ2 | cytoplasmic |
| 101851 | M94250 | Hs.82045 | midkine (neurite growth-promoting factor | LBH9 | secreted |
| 102398 | U42359 | | gb:Human N33 protein form 1 (N33) gene, | PDG3 | |
| 102522 | U53347 | Hs.183556 | solute carrier family 1 (neutral amino a | PFJ4 | plasma membrane |
| 102669 | U71207 | Hs.29279 | eyes absent (Drosophila) homolog 2 | LEM9 | cytoplasmic |
| 103119 | X63629 | Hs.2877 | cadherin 3, type 1, P-cadherin (placenta | LBG2 | plasma membrane |
| 103709 | AA037316 | Hs.13804 | hypothetical protein dJ462023.2 | PDO6 | |
| 104080 | AA402971 | Hs.57771 | kallikrein 11 | PBA6 | secreted |
| 104144 | AA447439 | Hs.183390 | hypothetical protein FLJ13590 | PDM3 | |
| 104691 | AA011176 | Hs.37744 | Homo sapiens beta-1 adrenergic receptor | PAV1 | plasma membrane |
| 105370 | AA236476 | Hs.22791 | transmembrane protein with EGF-like and | PDM9 | plasma membrane |
| 106149 | AA424881 | Hs.256301 | hypothetical protein MGC13170 | PDO8 | |
| 106579 | AA456135 | Hs.23023 | ESTs | PAA4 | plasma membrane |
| 107102 | AA609723 | Hs.30652 | KIAA1344 protein | PAA3 | not determined |
| 107217 | D51095 | | DKFZP586E1621 protein | PDG8 | |
| 108153 | AA054237 | Hs.40808 | ESTs | PBF1 | plasma membrane |
| 109014 | AA156790 | Hs.262036 | ESTs, Weakly similar to Z223_HUMAN ZINC | | PDG7 |
| 109112 | AA169379 | Hs.257924 | hypothetical protein FLJ13782 | BCU4 | not determined |
| 109890 | H04649 | Hs.20843 | Homo sapiens cDNA FLJ11245 fis, clone PL | | PDG4 |
| 110151 | H18836 | Hs.31608 | hypothetical protein FLJ20041 | PAV9 | plasma membrane |
| 112971 | T17185 | Hs.83883 | transmembrane, prostate androgen induced | | CHA1 not determined |
| 113021 | T23855 | Hs.129836 | KIAA1028 protein | PDO3 | |
| 114908 | AA236545 | Hs.54973 | cadherin-like protein VR20 | PFJ6 | plasma membrane |
| 114965 | AA250737 | Hs.72472 | ESTs | BCY2 | mitochondrial |
| 116393 | AA599463 | | hypothetical protein MGC2648 | PDV3 | secreted |
| 116416 | AA609219 | Hs.39982 | ESTs | OAB6 | |
| 117698 | N41002 | Hs.45107 | ESTs | PDT9 | ER |
| 117984 | N51919 | Hs.106778 | ATPase, Ca++ transporting, type 2C, memb | PAJ5 | not determined |
| 118985 | N94303 | Hs.55028 | ESTs, Weakly similar to I54374 gene NF2 | PDM8 | |
| 119018 | N95796 | Hs.278695 | Homo sapiens prostein mRNA, complete cds | PAB2 | plasma membrane |
| 119126 | R45175 | Hs.117183 | ESTs | PBF8 | |
| 120992 | AA398246 | Hs.97594 | KIAA1210 protein | PDG5 | |
| 121710 | AA419011 | | prostate androgen-regulated transcript 1 | PDV5 | |
| 121913 | AA428062 | | ESTs; protease inhibitor 15 (PI15) | BCU7 | vesicular |
| 122041 | AA431407 | Hs.98732 | Homo sapiens Chromosome 16 BAC clone | CIT | PAZ1 not determined |
| 122593 | AA453310 | Hs.128749 | alpha-methylacyl-CoA racemase | PDO1 | |
| 123209 | AA489711 | Hs.203270 | ESTs, Weakly similar to ALU1_HUMAN ALU | SPAA2 | plasma membrane |
| 124526 | N62096 | Hs.293185 | ESTs, Weakly similar to JC7328 amino aci | PAV4 | plasma membrane |
| 126399 | AA128075 | | transmembrane, prostate androgen induced | | PDY4 |
| 126645 | AI167942 | Hs.61635 | six transmembrane epithelial antigen of | PAA5 | plasma membrane |
| 126966 | R38438 | Hs.182575 | solute carrier family 15 (H+/peptide tra | PDO5 | plasma membrane |
| 127537 | AA569531 | Hs.162859 | ESTs | PAA6 | not determined |
| 128790 | AA291725 | Hs.105700 | secreted frizzled-related protein 4 | BCX2 | secreted |
| 129109 | AA491295 | Hs.108708 | calcium/calmodulin-dependent protein kin | PFJ7 | |
| 129184 | W26769 | Hs.109201 | CGI-86 protein | PAV6 | vesicular |
| 129389 | AA621604 | | spondin 2, extracellular matrix protein | CJA5 | not determined |
| 129404 | AA172056 | | ESTs | PAB4 | |
| 129534 | R73640 | Hs.11260 | hypothetical protein FLJ11264 | PAJ3 | secreted |
| 130760 | AA128997 | Hs.18953 | phosphodiesterase 9A | PEE6 | nuclear |
| 131425 | AA219134 | Hs.26691 | ESTs | PBA7 | |
| 132964 | AA031360 | | ESTs | PAA7 | plasma membrane |
| 132967 | AA032221 | Hs.61635 | six transmembrane epithelial antigen of | PM17 | plasma membrane |
| 133179 | U81599 | Hs.66731 | homeo box B13 | PFJ5 | nuclear |
| 133330 | U42360 | Hs.71119 | Putative prostate cancer tumor suppresso | PDM1 | plasma membrane |
| 133520 | X74331 | Hs.74519 | primase, polypeptide 2A (58kD) | PDM2 | |
| 133724 | U07919 | Hs.75746 | aldehyde dehydrogenase 1 family, member | PDT1 | mitochondrial |
| 133724 | U07919 | Hs.75746 | aldehyde dehydrogenase 1 family, member | PDT1 | mitochondrial |
| 133944 | AA045870 | Hs.7780 | Homo sapiens mRNA; cDNA DKFZp564A072 (fr | PAB9 | cytoplasmic |
| 134110 | U41060 | Hs.79136 | LIV-1 protein, estrogen regulated | BCR4 | plasma membrane |
| 301805 | AI800004 | Hs.142846 | hypothetical protein | PEU4 | nuclear |
| 302005 | AI869666 | Hs.123119 | MAD (mothers against decapentaplegic, Dr | PBJ6 | cytoplasmic |
| 302881 | AA508353 | Hs.105314 | relaxin 1 (H1) | PBH3 | secreted |
| 303506 | AA340605 | Hs.105887 | ESTs, Weakly similar to Homolog of rat Z | PEG4 | |
| 303699 | D30891 | Hs.19525 | hypothetical protein FLJ22794 | PBM4 | not determined |
| 303753 | AW503733 | Hs.9414 | KIAA1488 protein | PBY3 | not determined |
| 308050 | AI460004 | Hs.31608 | hypothetical protein FLJ20041 | PEU5 | plasma membrane |
| 310382 | AI734009 | Hs.127699 | KIAA1603 protein | PCQ8 | |
| 310431 | AI420227 | Hs.149358 | ESTs, Weakly similar to A46010 X-linked | PBH1 | plasma membrane. |
| 310573 | AW292180 | Hs.156142 | ESTs | PEN3 | plasma membrane |
| 310598 | AI338013 | Hs.140546 | ESTs | PCW3 | |
| 310816 | AI973051 | Hs.224965 | ESTs | PET5 | |
| 311596 | AI682088 | Hs.79375 | holocarboxylase synthetase (biotin-[prop | PBH8 | |
| 313676 | AA861697 | Hs.120591 | ESTs | PBY2 | |
| 314121 | AI732100 | Hs.187619 | ESTs | PBY1 | |
| 314691 | AW207206 | Hs.136319 | ESTs | BFF8 | not determined |
| 314785 | AI538226 | Hs.32976 | guanine nucleotide binding protein 4 | CBO7 | cytoplasmic |
| 314907 | AI672225 | Hs.222886 | ESTs, Weakly similar to TRHY_HUMAN TRICH | PBM2 | not determined |
| 315051 | AW292425 | | ESTs | PBM9 | |
| 315052 | AA876910 | Hs.134427 | ESTs | PBJ7 | plasma membrane |
| 316442 | AA760894 | Hs.153023 | ESTs | PBJ9 | |
| 317548 | AI654187 | Hs.195704 | ESTs | PBQ6 | |
| 317869 | AW295184 | Hs.129142 | deoxyribonuclease II beta | PBQ7 | |
| 318524 | AW291511 | Hs.159066 | hypothetical protein FW10188 | PBJ1 | cytoplasmic |
| 319191 | AF071538 | | prostate epithelium-specific Ets transcr | PEN1 | |
| 319763 | AA460775 | Hs.6295 | ESTs, Weakly similar to T17248 hypotheti | PEO7 | |
| 320324 | AF071202 | Hs.139336 | ATP-binding cassette, sub-family C (CFTR | PBH5 | plasma membrane |
| 320561 | NM_006953 | Hs.159330 | uroplakin 3 | PEL9 | plasma membrane |
| 320796 | AF038966 | Hs.31218 | secretory carrier membrane protein 1 | PBY4 | not determined |
| 321441 | AW297633 | Hs.118498 | Homo sapiens LUCA-15 protein mRNA, splic | PBY8 | not determined |
| 322303 | W07459 | Hs.157601 | ESTs | CBF9 | secreted |
| 322782 | AA056060 | Hs.202577 | Homo sapiens cDNA FLJ12166 fis, clone MA | PBQ1 | not determined |
| 322818 | AW043782 | Hs.293616 | ESTs | PCQ7 | plasma membrane |
| 323226 | AF055019 | Hs.21906 | Homo sapiens clone 24670 mRNA sequence | PCl2 | not determined |
| 323287 | AA639902 | Hs.104215 | ESTs, Moderately similar to SPCN_HUMAN | SPBJ5 | |
| 324295 | AI146686 | Hs.143691 | ESTs | PBQ9 | not determined |
| 324430 | AA464018 | Hs.184598 | Homo sapiens cDNA: FLJ23241 fis, clone C | PBY6 | not determined |
| 324603 | AW016378 | Hs.292934 | ESTs | PBM3 | |
| 324617 | AA508552 | Hs.195839 | ESTs, Weakly similar to I38022 hypotheti | PBH4 | cytoplasmic |
| 324626 | AI685464 | | gb:tt88f04.x1 NCl_CGAP_Pr28 Homo sapiens | PCW6 | |
| 324658 | AI694767 | Hs.129179 | Homo sapiens cDNA FLJ13581 fis, clone PL | PBJ4 | plasma membrane |
| 324718 | AI557019 | Hs.116467 | small nuclear protein PRAC | CBK1 | nuclear |
| 330211 | | | | PBJ2 | not determined |
| 330546 | U31382 | Hs.299867 | guanine nucleotide binding protein 4 | PEW1 | cytoplasmic |
| 330762 | AA449677 | Hs.15251 | hypothetical protein | PBM1 | not determined |
| 330790 | T48536 | Hs.122764 | TMPRSS2, transmembrane protease, serine | PEL3 | plasma membrane |
| 330892 | AA149579 | Hs.91202 | ESTs | PBQ4 | plasma membrane |
| 331099 | R36671 | Hs.14846 | Homo sapiens mRNA; cDNA DKFZp564D016 (fr | PCQ1 | cytoplasmic |
| 331490 | N32912 | Hs.291039 | ESTs | PCI4 | nuclear |
| 331889 | AA431407 | Hs.98802 | ESTs, Moderately similar to T14342 NSD1 | PBH7 | not determined |
| 332247 | N58172 | | gb:za21f09.s1 Soares fetal liver spleen | PBQ5 | nuclear |
| 332396 | AA340504 | | gb:hw31a09.x1 NCl_CGAP_Kid11 Homo sapien | PBJ8 | not determined |
| 332697 | T94885 | | transgalin 2 | PBQ8 | secreted |
| 332798 | | | | PBH2 | nuclear |
| 334447 | | | | PBY9 | not determined |
| 338255 | | | | PBY7 | not determined |
| 401424 | | | | PFG2 | mitochondrial |
| 407122 | H20276 | Hs.31742 | ESTs | PEW7 | |
| 408430 | S79876 | Hs.44926 | dipeptidylpeptidase IV (CD26, adenosine | PEZ3 | plasma membrane |
| 408826 | AF216077 | Hs.48376 | Homo sapiens clone HB-2 mRNA sequence | | PEY1 |
| 409262 | AK000631 | Hs.52256 | hypothetical protein FLJ20624 | PFG1 | nuclear |
| 409361 | NM_005982 | Hs.54416 | sine oculis homeobox (Drosophila) homolo PEW3 | | nuclear |
| 411096 | U80034 | Hs.68583 | mitochondrial intermediate peptidase | PEZ9 | mitochondrial |
| 413125 | BE244589 | Hs.75207 | glyoxalase I | PFJ3 | cytoplasmic |
| 413623 | AA825721 | Hs.246973 | ESTs | OBH6 | |
| 414422 | AA147224 | Hs.337232 | Homeo box A13 | PFC6 | |
| 415263 | AA948033 | Hs.130853 | ESTs | PEZ5 | |
| 417153 | X57010 | Hs.81343 | "collagen, type II, alpha 1 (primary ost | PFJ1 | secreted |
| 418601 | AA279490 | Hs.86368 | calmegin | PFA1 | ER |
| 418848 | AI820961 | Hs.193465 | ESTs | PEY4 | |
| 418882 | NM_004996 Hs.89433 | | ATP-binding cassette, sub-family C (CFTR OBH2 | | |
| 419839 | U24577 | Hs.93304 | "phospholipase A2, group VII (platelet-a | PFH9 | secreted |
| 421887 | AW161450 | Hs.109201 | CGI-86 protein | PFH2 | plasma membrane |
| 422083 | NM_001141 | Hs.111256 | "arachidonate 15-lipoxygenase, second ty | PFH5 | cytoplasmic |
| 424565 | AW102723 | Hs.75295 | guanylate cyclase 1, soluble, alpha 3 | PFA3 | |
| 425071 | NM_013989 | Hs.154424 | "deiodinase, iodothyronine, type II" | PFH6 | secreted |
| 425710 | AF030880 | | solute carrier family, member 4 | PFD4 | plasma membrane |
| 427958 | AA418000 | Hs.98280 | potassium intermediate/small conductance | PFH1 | plasma membrane |
| 428819 | AL135623 | Hs.193914 | KIAA0575 gene product | PFD6 | nuclear |
| 429900 | AA460421 | Hs.30875 | ESTs | PEZ7 | |
| 429918 | AW873986 | Hs.119383 | ESTs | PEY5 | |
| 430226 | BE245562 | Hs.2551 | adrenergic, beta-2-, receptor, surface | PEZ4 | plasma membrane |
| 431217 | NM_013427 | Hs.250830 | Rho GTPase activating protein 6 | PFG6 | nuclear |
| 431716 | D89053 | Hs.268012 | fatty-acid-Coenzyme A ligase, long-chain | PEZ1 | |
| 431992 | NM_002742 | Hs.2891 | protein kinase C, mu | PFH4 | cytoplasmic |
| 432189 | AA527941 | | gb:nh30c04.s1 NCI_CGAP_Pr3 Homo sapiens | | PFA2 |
| 432244 | AI669973 | Hs.200574 | ESTs | PEW8 | |
| 432437 | W07088 | Hs.293685 | ESTs | PFG3 | |
| 432966 | AA650114 | Hs.325198 | ESTs | PEY3 | |
| 439176 | AI446444 | Hs.190394 | ESTs, Weakly similar to B28096 line-1 pr | PEW5 | |
| 440260 | AI972867 | Hs.7130 | copine IV | PEW6 | |
| 440901 | AA909358 | Hs.128612 | ESTs | PFC8 | |
| 445424 | AB028945 | | cortactin SH3 domain-binding protein | PEZ6 | |
| 446320 | AF126245 | Hs.14791 | "acyl-Coenzyme A dehydrogenase family, m | | PFH7 |
| 447210 | AF035269 | | phosphatidylserine-specific phospholipas | PFH8 | |
| 449156 | AF103907 | Hs.171353 | prostate cancer antigen 3, non-coding DD | PEZ8 | |
| 449625 | NM_014253 | | odz (odd Oz/ten-m, Drosophila) homolog 1 | PEZ2 | plasma membrane |
| 449650 | AF055575 | Hs.23838 | calcium channel, voltage-dependent, L ty | PFD2 | plasma membrane |
| 451939 | U80456 | Hs.27311 | single-minded (Drosophila) homolog 2 | PFJ8 | |
| 451982 | F13036 | Hs.27373 | Homo sapiens mRNA; cDNA DKFZp564O1763 (f | PFG9 | plasma membrane |
| 452039 | AI922988 | | ESTs | PFD8 | |
| 452340 | NM_002202 | Hs.505 | ISL1 transcription factor, LIM/homeodoma PFG4 | | nuclear |
| 452784 | BE463857 | Hs.151258 | hypothetical protein FLJ21062 | PFC5 | cytoplasmic |
| 452946 | X95425 | Hs.31092 | EphA5 | PFH3 | plasma membrane |

**TABLE 15A shows the accession numbers for those primekeys lacking a unigeneID in Table 15. For each probeset we have listed the gene cluster number from which the oligonucleotides were designed. Gene clusters were compiled using sequences derived from Genbank ESTs and mRNAs. These sequences were clustered based on sequence similarity using Clustering and Alignment Tools (DoubleTwist, Oakland California). The Genbank accession numbers for sequences comprising each cluster are listed in the "Accession" column.**

| | | |
|---|---|---|
| Pkey: | | Unique Eos probeset identifier number |
| CAT number: | | Gene cluster number |
| Accession: | | Genbank accession numbers |
| | | |

| **Pkey** | **CAT number** | **Accession** |
|---|---|---|
| | | |
| 116393 | 131543_1 | AI972402 AI634409 AI523716 AI799749 W44518 AI424438 AI688513 AI971048 AI686324 AW013854 AA588483 AA528111 AI627428 AI582200 AI669296 AI826926 AI620526 AI669958 AI972458 AI924500 AA512903 W44517 AA335363 AW238997 BE300165 BE250665 AA284195 AA523420 W52834 AI471970 AI952824 AW003820 AW009463 AA669796 AA114966 AI653342 AA115038 AI342150 AI092100 AI968211 W51994 AI804005 AI201420 AI123210 AI738405 AI674964 AI970341 AW027500 AI493316 AI333193 AI139353 AA599463 AI656163 AI804200 AI365321 AI990213 AI657011 AA650025 AI968810 AI341978 AA599839 AW592602 AA644289 AI468578 AI565265 AI565228 BE221535 AW973052 |
| 101485 | 18113_1 | AA296520 AL021940 M30640 NM_000450 M24736 M61894 AL047443 H39560 AI694691 AA916787 AI214796 AA939085 AI150616 AA412553 AA412545 AI051015 T27654 AA694430 |
| 126399 | 17331_1 | AA088767 AF224278 AA128075 AL035541 AA027926 AI761441 AI972096 AW071693 AI742327 AI377498 AI804815 AI640802 AI885001 AI921394 AA595115 N71820 AI921217 AW007283 AI467828 AI369306 AA917446 AI493698 AA088701 AA126899 AI936228 AW204238 AI039567 AI925027 BE138909 AW452945 AW135998 AA310984 AA027860 AW073519 AI537597 AA953976 AI521341 AW273569 AW050740 AA536113 AA559064 AI474392 AW135709 AA535181 AW572959 AA570597 AI905464 AI677810 AI587642 AW975102 AA424310 AA482527 N64192 AA658276 AW889117 AA486591 AW889172 AI381990 AI381991 AI673419 |
| | | AI990950 AA487031 AI272934 AI150565 AA229168 AW316722 AI142707 BE222396 AA614168 AA122026 AW338227 AA632457 AI968726 AW369662 AA512956 AA541675 AA451748 AI250993 BE146418 AA122025 |
| 132964 | 94346_1 | AI362575 AI805082 AW263421 AI32462 AA135870 AA031360 AA031604 AA298475 AA298464 |
| 129389 | 21074_1 | NM_012445 AB027466 BE407510 BE047605 AA047125 AW084003 AA149494 AA149490 AA292528 AA570505 AA526186 AW006250 AW007762 AI341557 AI799666 AI972710 AI377966 AI962810 AI084783 AI458032 AI190971 AW148913 AA372354 AW970032 AW007426 AA650188 AI123203 AI122890 AI280975 W73595 W73495 AI863238 AA374109 |
| | | AA603986 AW149089 AW957523 AI307748 AI921067 AI336463 F24537 AI380460 AI367500 AI189309 AI814701 AI766921 AW572106 AA037024 AW072576 AA578293 AI288103 AA235464 AW450642 AA574230 AW294024 AI589229 AI580733 AW5122Z7 AA877009 AI660255 AW188597 AA558228 AI572782 AA658397 AI274628 AI866359 AA864573 AI264439 AA621604 AW515493 AW243333 Z39737 AI567038 AA573997 AA573559 AW236431 AI652870 AI684973 AA034505 AA047126 |
| 129404 | 156454_1 | AI267700 AI720344 AA191424 AI023543 AI469633 AA172056 AW958465 AA172236 AW953397 AA355086 |
| 107217 | 9836_1 | AL080235 AA031750 D81382 AI480231 AI095947 AI560953 BE010721 AI870290 AA374945 AA125792 D51527 D51556 AI685541 D51559 AW117286 AA195741 AI675138 AW593439 AI201885 T30590 AW952100 D51095 AA523864 W70043 AA987586 AI421515 AI205532 AA127069 AI337367 D51595 AI453785 AW075677 AW088359 C14287 C14284 |
| 121710 | 19266_1 | AF163474 NM_016590 AF163475 AI761105 AI770098 AA410580 AA411616 AI590343 AI739050 AL050198 AI862645 AA419104 AA513809 AA333032 AI816915 AW139625 AA640889 AI311391 AI627693 AW135514 AA419011 AI269149 AI245259 AI970008 AI970017 AW139445 AA569503 AI761072 AI766179 AI759995 AI300776 AI870129 AW150770 AA226501 AA226220 |
| 121913 | 291015_1 | AI249368 AI742316 AA428062 AA442089 AI864189 BE349476 AI803475 AI584049 BE552085 AI088609 AI264197 AI886144 AI129474 AI307145 BE181300 AW058403 AI696838 AW748598 AA442196 AI216428 |
| 102398 | entrez_U42359U42359 | |
| 315051 | 347217_1 | AW292425 BE467167 AI702953 BE550961 BE222309 AI299348 AI693336 AA541708 |
| 324626 | 336411_1 | AI685454 AW971336 AA513587 AA525142 |
| 319191 | 16065_1 | NM_012391 AF071538 AB031549 AI685592 AI745526 AA662204 AW130657 AA662164 AW971121 AI668916 AA513274 AI991223 AI979170 AW298436 AA639821 AI859010 AW513942 AI687669 AA662521 AA548598 AI345056 AI305374 BE043418 AI432856 AI334840 AI379796 AI492693 |
| | | AI307915 BE042082 AI307834 AI307858 AI309488 BE042210 AI35670 AI371605 AI862491 AI284563 AI306872 AI255044 AI254601 AI251236 AI473073 AI473042 AI432760 AI435664 AI336826 AI289365 AI369096 AI862274 AI334871 AI349863 AI250405 AI377617 AI309895 AI313017 AI862291 AI311936 |
| | | AI378718 AI305722 AI306769 AI308888 AI334565 AI862296 AI344230 AI435685 AI344087 AI378696 AI311209 AI435775 AI310611 AI311154 AI432289 AI31561 AI492681 AI432867 AI335288 AI492796 AI432769 AI310299 AI432273 AI379820 AI275319 AI435753 AI609441 AI432767 AI369100 AI311420 AI349974 AI247157 AI334677 |
| | | AI270910 AI224320 AI305608 AI334489 AI377152 AI350012 AI370086 AI335053 AI306781 AI306750 AI334849 AI334874 AI340380 AI307876 AI305974 AI305972 AI311521 AI334872 AI862509 AI311498 AI335051 AI289684 AI310859 AI311862 AI862483 AI492775 AI307906 AI492708 AI289693 AI340373 |
| | | AI307910 AI311359 AI435653 AI334865 AI311492 AI492809 AI492690 AI431576 AI862268 AI311879 AI308435 AI492792 AI862512 AI275321 AI431568 AI431564 AI307885 AI307926 AI435692 AI435778 AI310182 AI308894 AI492707 AI492713 AI308560 AI307829 AI343234 AI580598 AW472796 AI340918 AI310243 AI309368 AI307920 AI289665 |
| | | AI306777 AW086318 AW086292 AW086378 AI310027 AI275293 AI369082 AI340900 AI306749 AI371558 AW086287 BE043803 AI306793 AI306272 AI287948 AI270917 AI284816 AI336813 AI284546 AI308044 AI275290 AI270872 AI306795 AI289687 AI223570 AI305303 AI289677 AI287742 AI275284 AI306812 |
| | | AI336701 AI371554 AI378719 AI344988 AI223631 AI335141 AI343222 AI284568 AI305357 AI275270 AI345932 AI436549 AI307925 AI311502 AI344238 AI343182 AI308508 AI305988 AI270790 AI379792 AI305647 AI305410 AI432251 AI436517 AI343227 AI305534 AI340387 AI271043 AI305499 AI271046 AI305962 AI289465 AI305378 |
| | | AI289725 AI310848 AI305848 AI289362 AI252964 AI307049 AI310831 AI306993 AI306796 AI224659 AI305969 AI349855 AI306164 AI306948 AI284676 AI309155 AI343202 AI432785 AI306815 AI369081 AI270885 AI289699 AI435704 AI309647 AI305716 AI311281 AI287927 |
| | | AI472995 AI340423 AI270958 AI307069 AI305364 AI270807 AI275306 AI311890 AI275283 AI432750 AI289371 AI432861 AI255113 AI305709 AI473008 AI311168 AI309711 AI377164 AI271201 AI289560 AI309710 AI306195 AI311201 AI287741 AI271066 AI432876 AI275281 AI379795 AI472972 AI311967 AI306826 AI305465 AI270792 AI473019 AI305340 AI270922 AI305995 AI305462 AI254144 AI270969 AI473012 AI305390 |
| | | AI275278 AI223644 AI289692 AI250318 AI305372 AI289691 AI250521 AI306283 AI306814 AI307933 AI473160 AI432903 AI223720 AI254979 AI334862 AI306926 AI289541 AI432248 AI435722 AI435698 AI432859 AI310683 AI473175 AI335144 AI289467 AI436489 AI306928 AI473033 AI305763 AI307868 AI307882 AI348959 AI435736 AI432857 AI432896 AI435735 AI432283 AI473086 AI432863 AI473081 AI432825 AI307840 AI473164 AI432885 AI473166 AI472982 AI435734 AI473060 |
| | | AI473171 AI432279 AI432882 AI334670 AI436512 AI432827 AI432852 AI473051 AI473077 AI435697 AI271509 AI492781 AI472983 AI473018 AI432897 AI473043 AI432871 AI436536 AI473157 AI349715 AI432777 AI473016 AI473158 AI340369 AI307941 AI432773 AI377146 AI492791 AI270950 AI305342 |
| | | AI284604 AI306269 AI284811 AI270811 AI289347 AI334869 AI334852 AI311759 AI250382 AI309520 AI289550 AI305721 AI340870 AI270901 AI308575 AI307904 AI340715 AI270941 AI309808 AI246867 AI473014 AI307039 AI289360 AI473069 AI492786 AI344013 AI305876 AI436510 AI340742 AI473028 AI307891 BE041871 BE041268 BE042340 BE041946 BE041763 AI306173 AI201948 AI926972 AI275769 |
| 338255 | CH22_6856FG_LINK_EM:AC00 | |
| 330211 | c_5_p2 | |
| 332798 | CH22_14FG_6_5_LINK_C4G1.G | |
| 334447 | CH22_1746FG_387_7_LINK_EM | |
| 332247 | 372969_1 | AA669097 AA513815 AA026798 AA676526 AA704429 AA704269 AW118292 AA579216 N58172 |
| 332396 | 20265_1 | AW579842 BE156562 BE156690 BE156489 BE081033 AK001559 BE149402 M85387 AW367811 AW367798 R17370 AI908947 AA382932 R58449 H18732 AA371231 AW962899 AA713530 AW892946 R53463 H11063 AW068542 Z40761 BE176212 BE176155 W23952 W92188 AW374883 AA303497 AW954769 AA036808 BE168063 AW382073 AW382085 AL041475 H80748 AI078161 BE463983 AI805213 AI761264 W94885 N94502 AI623772 AI419532 AI810302 AI634190 AW002516 AW150777 AI352312 AI367474 AW204807 AI675502 AI337026 AW134715 BE328451 AI123157 AI560020 AI300745 AI608631 AI248873 AA742484 AW051635 H18646 AI245045 AA507111 AI640510 AI925594 AA115747 AA143035 AA151106 |
| 332697 | 13699_1 | X51405 NM_001873 T11322 AL118886 BE328175 AW136009 BE467445 AW470313 AA774852 BE504139 AW501046 AA082792 AW389231 AA370044 R36841 AA371457 C04813 R25791 R25556 AW89584 AW903819 AW895871 AW895677 BE159723 AW895664 AW895597 AW895595 AW895665 AW888518 AI903724 F06081 F08503 AL119462 |
| | | AW895730 AW888516 R26511 R26489 AA334126 AA327626 N85713 AW895998 AA223622 F05468 AA370749 W05590 M78202 AA371073 AW498607 R15017 T16991 AA001282 AA001138 AA551566 AA330159 AI922855 AA383512 AA029603 D82246 D82171 T94933 H56545 AA348060 AA176888 R96764 |
| | | AW451817 AA385766 AA452618 AI690057 AA988822 BE549928 AA150901 W57992 AW899925 C05281 AA932042 AA370980 AW962877 W04741 AA369982 AW385948 AA922466 N75882 AI422070 AI361256 AI680224 D57122 T94885 R53266 R46713 T19071 AW796277 AA325333 F04719 F02334 AA358146 AA626597 |
| | | AA358304 AW028099 AL119570 D57290 D58273 D57796 N48555 AI361969 AA329457 D57225 AW024046 AA992606 AW022118 AW021538 AA935845 H89870 H56546 AW961219 AA453239 AW837541 N45521 BE218029 AA318877 AA327740 AW961809 T92139 D53216 D52365 D53363 D53312 D53116 AI547267 |
| | | AA679935 AW026552 AW026418 AW190507 AI927710 AW244108 D50948 AW054991 AW021063 AW022511 AA493436 AI365636 BE464751 AW149384 AA102442 AW771388 AI818251 AI126368 D51049 AI421542 AI559467 AW079779 AW021048 AW023969 AW044214 AI458264 AA027274 AI620254 AW028917 BE219511 AA326242 N67561 AI971273 |
| | | AA878328 D57131 AA770662 AI309299 AI796767 AA613338 W58076 AI566287 AI45573 AI880260 AA001919 AW339259 AI492610 AI492611 R97692 AI301425 AA722603 D58361 AI350323 AA973926 AI431263 AA516126 AA865467 AI925177 N39443 AA001943 AI299371 AI082412 AA665090 AA583433 |
| | | H89871 AA977231 AI362219 AI056096 AI270446 N67524 N22103 AW614224 AA744054 AW243622 AI613188 AI929173 AI350243 AI362138 AA744004 AA176661 D56787 AI955625 AI393109 AI094769 AI479728 AI423107 AI955617 AI034036 AI582196 AW264534 AI418961 AA570761 AI343538 AA650341 |
| | | AA992503 AA770004 AL039666 AI862675 AW190335 AA610274 AW418627 BE467472 D56786 T28749 AI217610 AI359556 T23523 AL040189 AA846222 AA651636 D51280 AI888986 AI521167 AI340177 AW612815 AI625285 AA621607 AA177059 AA229768 AA829788 AI749682 AW190631 N75299 AA230089 |
| | | AI915632 BE069542 AA890020 AA528397 AA995390 BE503860 AA570812 AW339396 AI197986 AI203725 AI282379 AA670375 AA461513 F01728 AW243599 C00856 N75567 R95995 AA150932 R95961 AA648060 AA933800 AA927073 AA101126 AA864190 T93566 BE167472 |
| 425710 | 25529_1 | AF030880 NM_000441 AC002467 AA385554 H23053 AW891838 AI139968 AA653057 AI695233 |
| 432189 | 342819_1 | AA527941 AI810608 AI620190 AA635266 |
| 445424 | 6391_1 | AB028945 T77648 F13328 AL157605 Z46212 AA304736 F11855 T66098 T30174 AW954164 AW176301 AW748243 AA456428 AI369958 AA938565 AW959613 Z42008 AA994779 AI683909 F11019 F10926 AI769597 AI752550 T65015 AI884314 AA643954 Z41838 AW020147 AI038822 AW571822 AA299781 AA894928 AF131790 BE005411 AI902476 AW082695 AA464384 R42750 AW902301 AA464273 R05837 Z38294 H41098 AL134507 M86079 |
| 447210 | 7119_1 | AF035269 AF035268 NM_015900 T96213 U37591 AA156832 AA299371 AI084325 H95977 AI765967 BE221465 AA156726 AI969563 AW024539 AI436791 AI949451 AA843093 AI452756 AA824232 AI306667 T96131 AW207447 AW243556 AW957032 AI084332 H95978 U30998 |
| 449625 | 8113_1 | NM_014253 AF100772 BE088769 AL022718 BE161779 AW863569 BE161640 AL039060 BE168542 AW296554 AA323193 AA235370 AW779760 N48674 AI375997 R45432 D59344 AI203107 F07491 R35360 R25094 AI913631 AI498402 T61382 AI016320 N45526 T61415 AA331486 |
| 452039 | 89513_1 | AI922988 H05475 AA021608 AW169947 AA913750 Z41614 AW800012 |

**TABLE 15B shows the genomic positioning for those primekeys lacking unigene ID's and accession numbers in Table 15. For each predicted exon, we have listed the genomic sequence source used for prediction. Nucleotide locations of each predicted exon are also listed.**

| | | | |
|---|---|---|---|
| Pkey: | | Unique number corresponding to an Eos probeset | |
| Ref: | | Sequence source. The 7 digit numbers in this column are Genbank Identifier (GI) numbers. "Dunham I. et al." refers to the publication entitled "The DNA sequence of human chromosome 22. "Dunham I. et al, Nature (1999) 402:489-495. | |
| Strand: | | Indicates DNA strand from which exons were predicted. | |
| Nt_position: | | Indicates nucleotide positions of predicted exons. | |
| | | | |

| **Pkey** | **Ref** | **Strand** | **Nt_position** |
|---|---|---|---|
| | | | |
| 334447 | Dunham, I. et.al. | Plus | 14308764-14308824 |
| 332798 | Dunham, I. et.al. | Minus | 232147-231974 |
| 338255 | Dunham, I. et.al. | Minus | 15242294-15242231 |
| 330211 | 6013592 | Plus | 59158-59215 |
| 401424 | 8176894 | Plus | 24223-24428 |

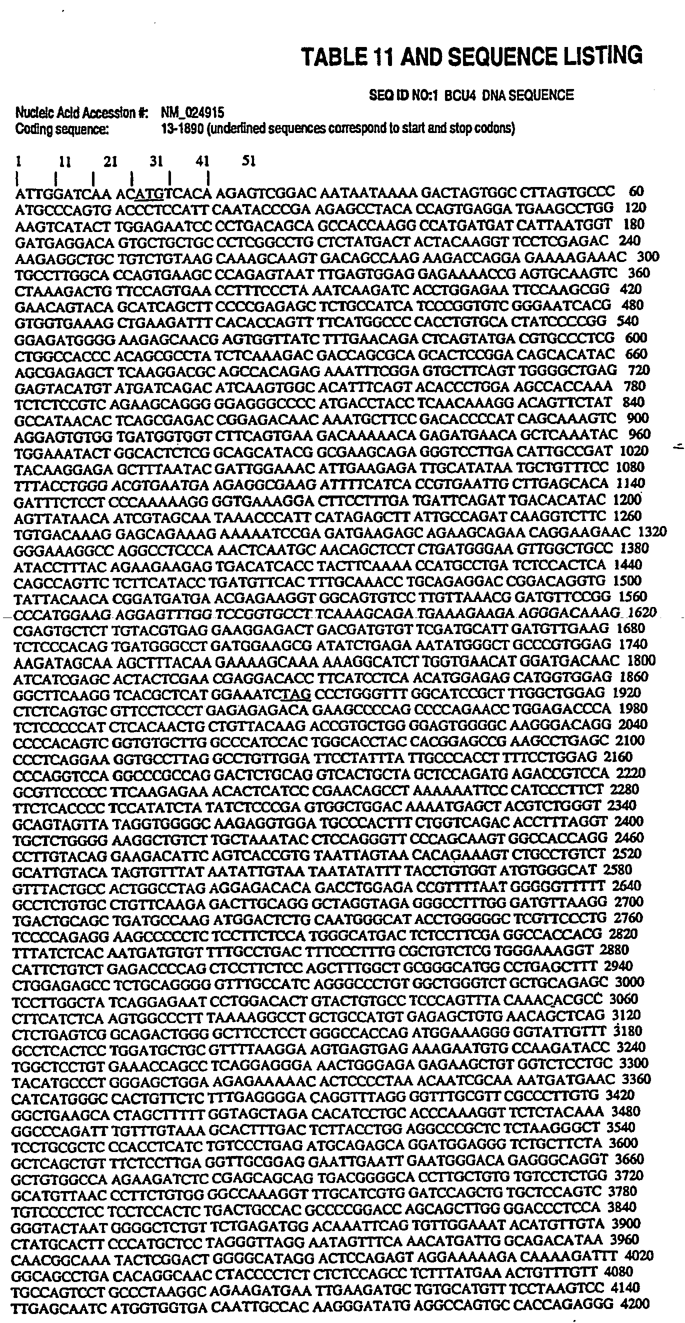

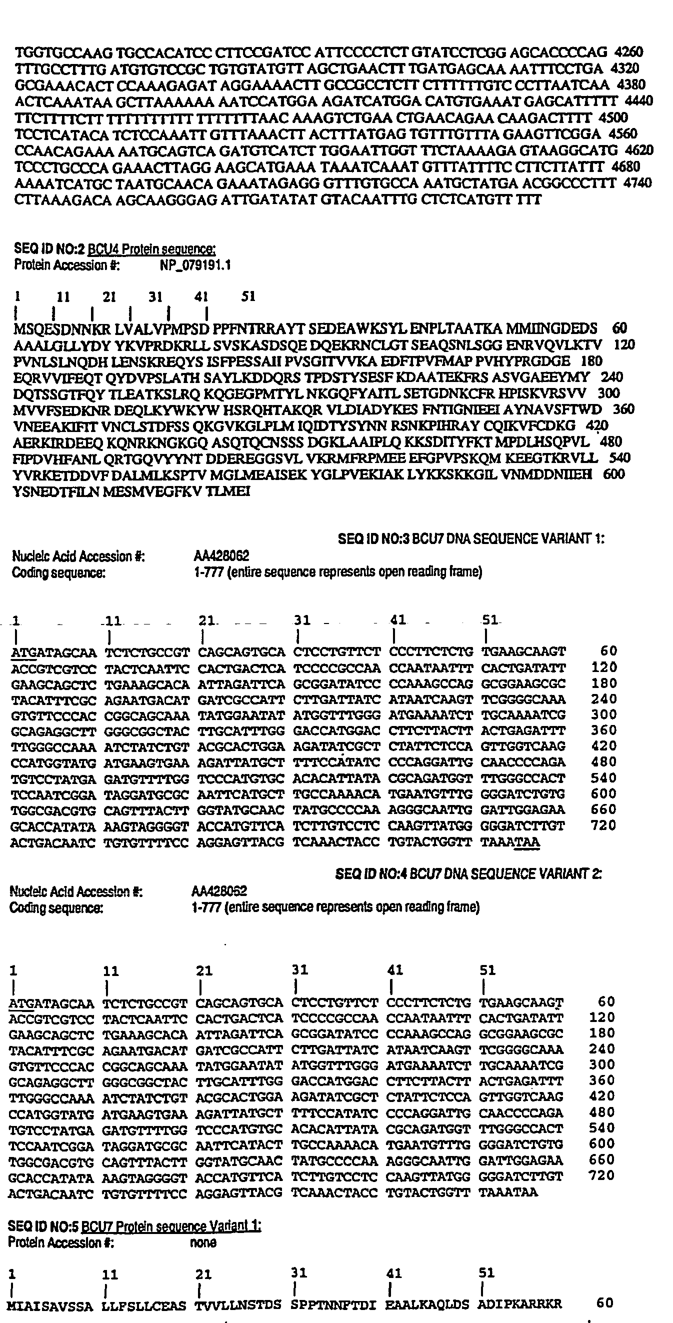

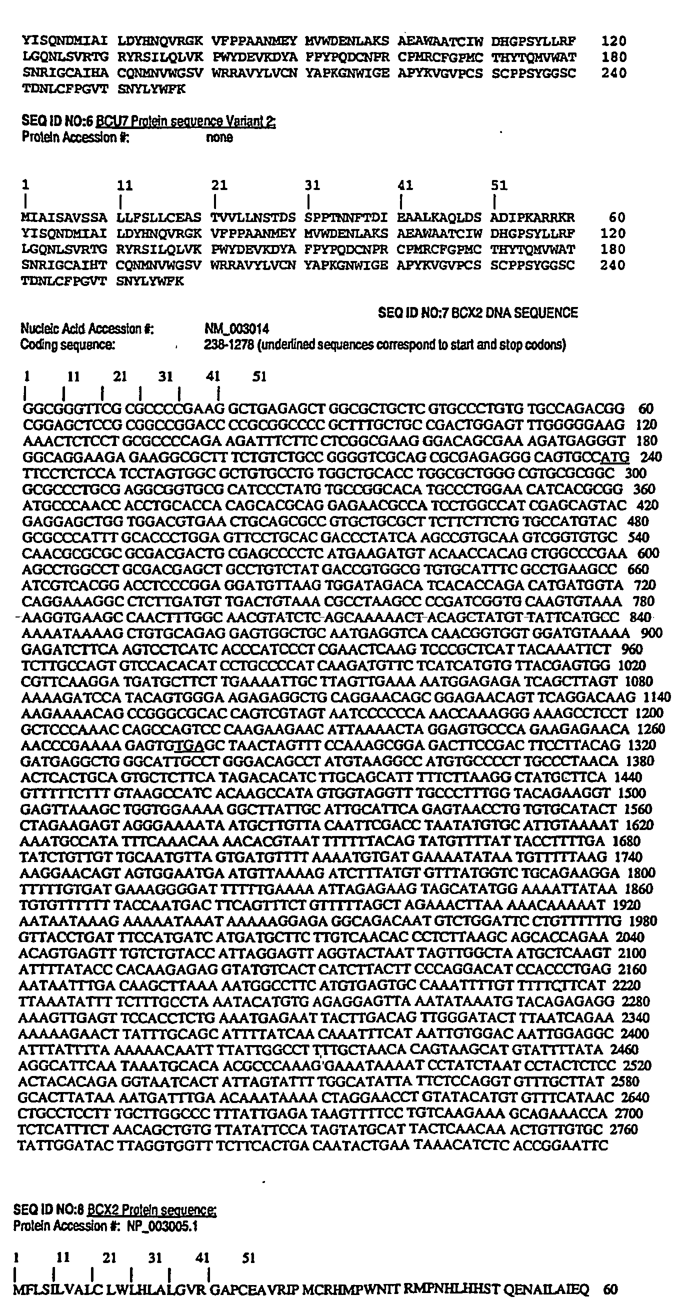

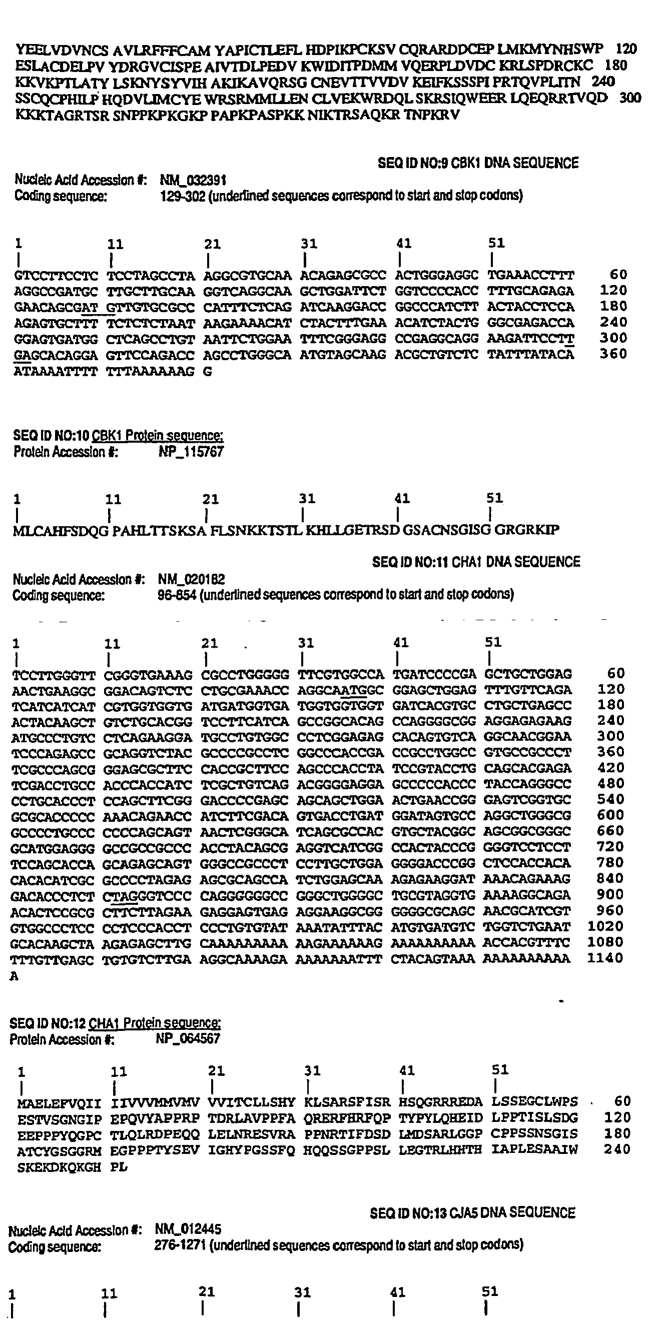

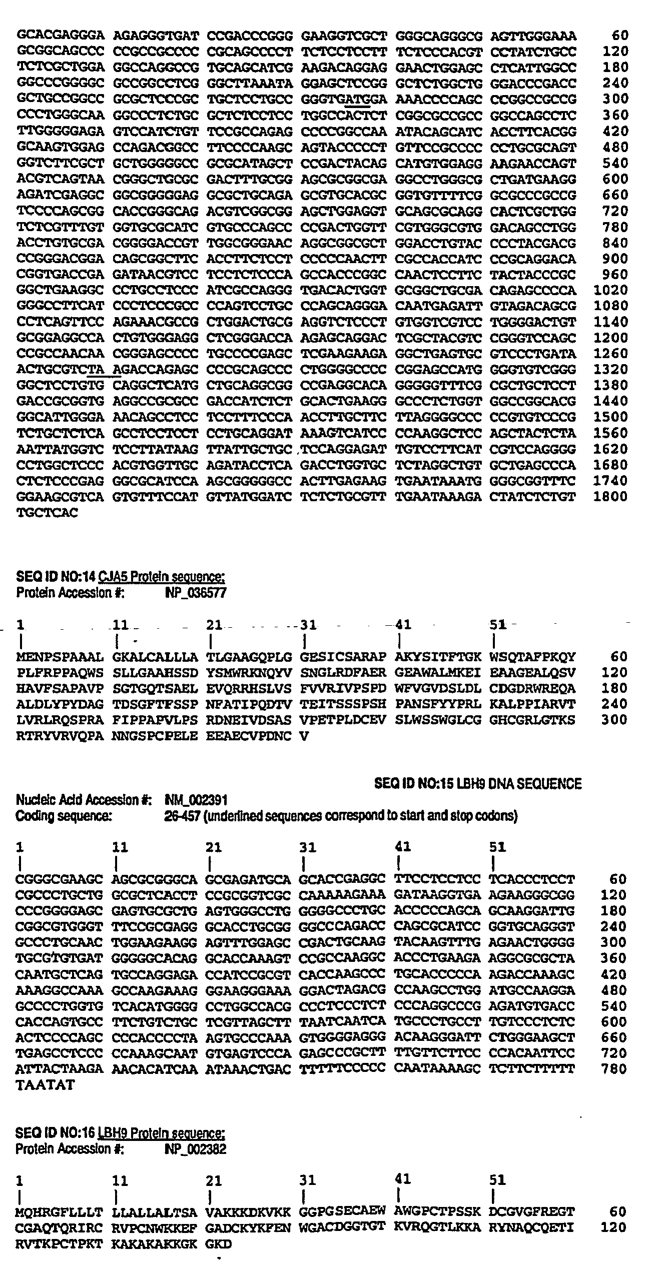

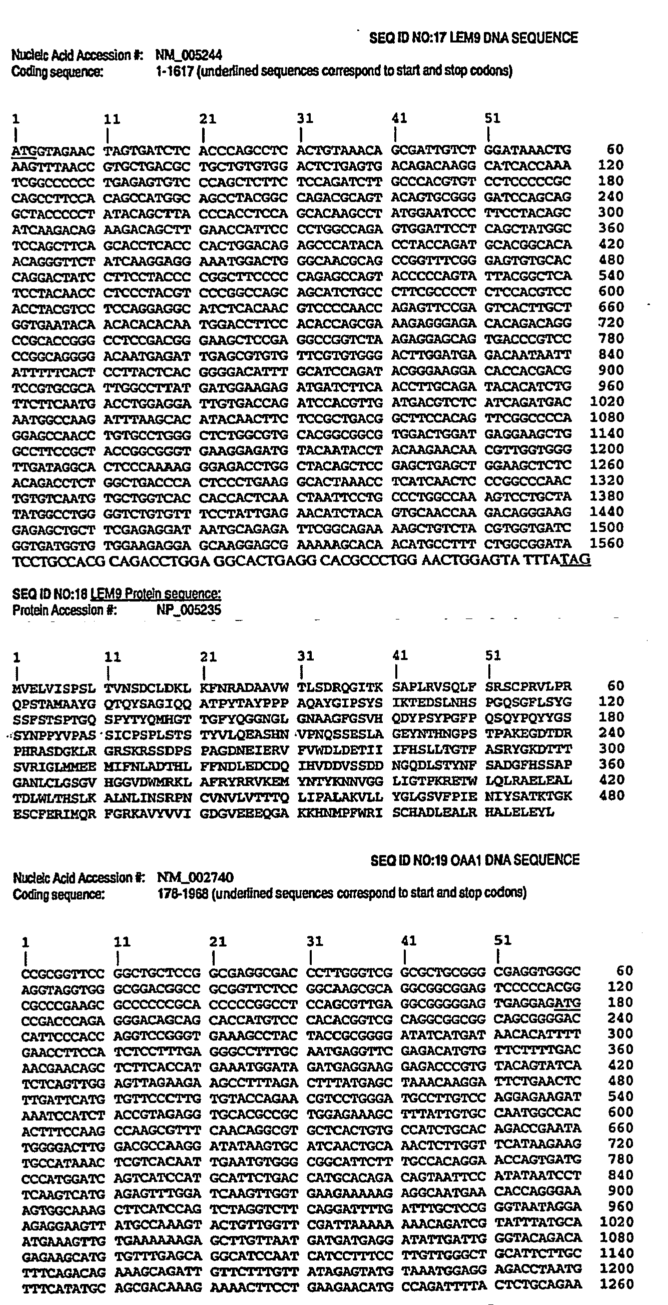

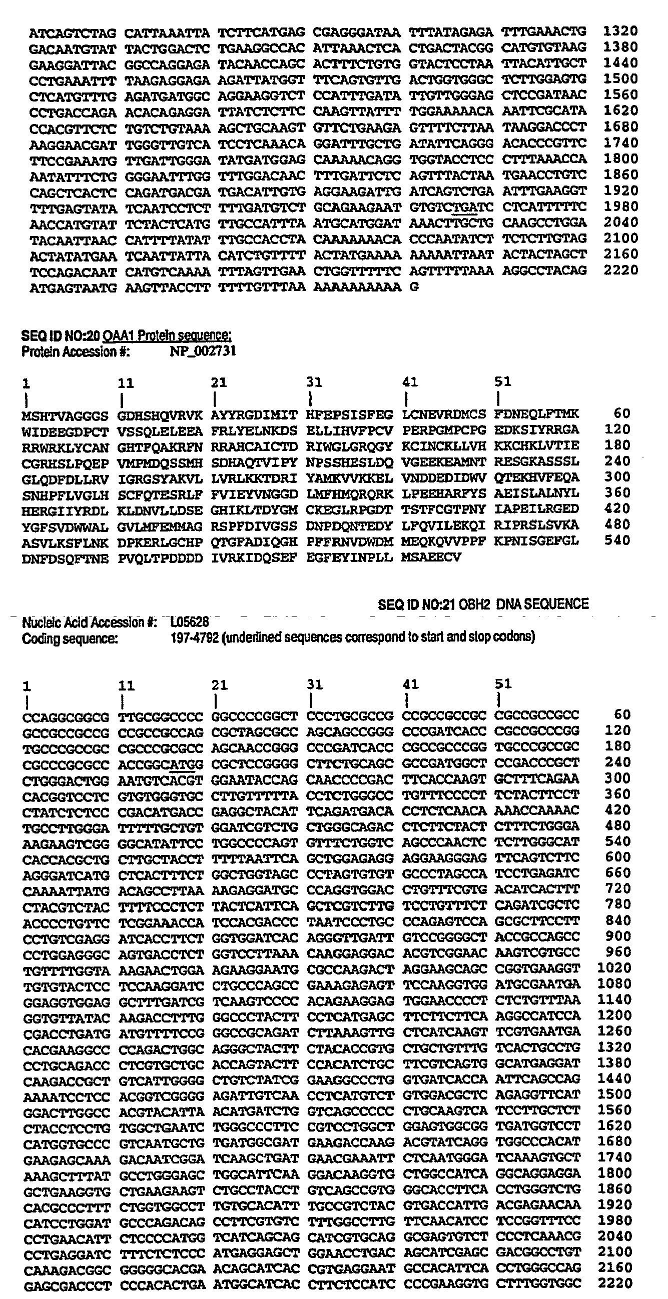

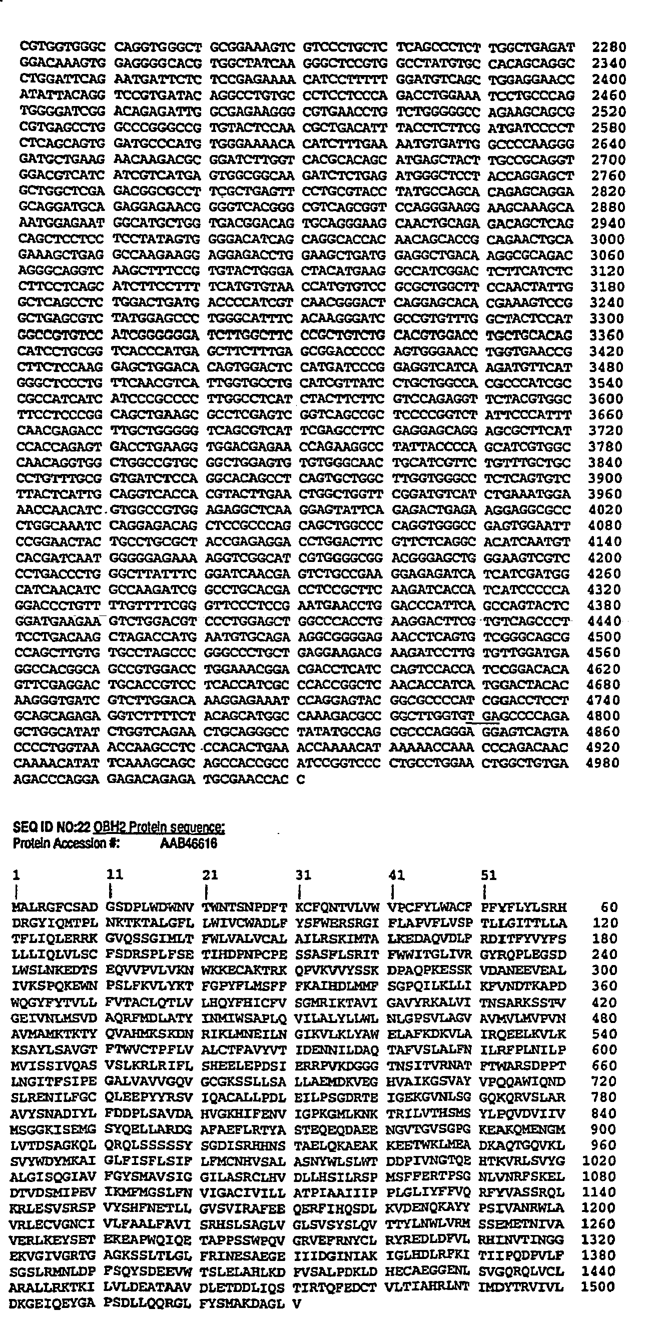

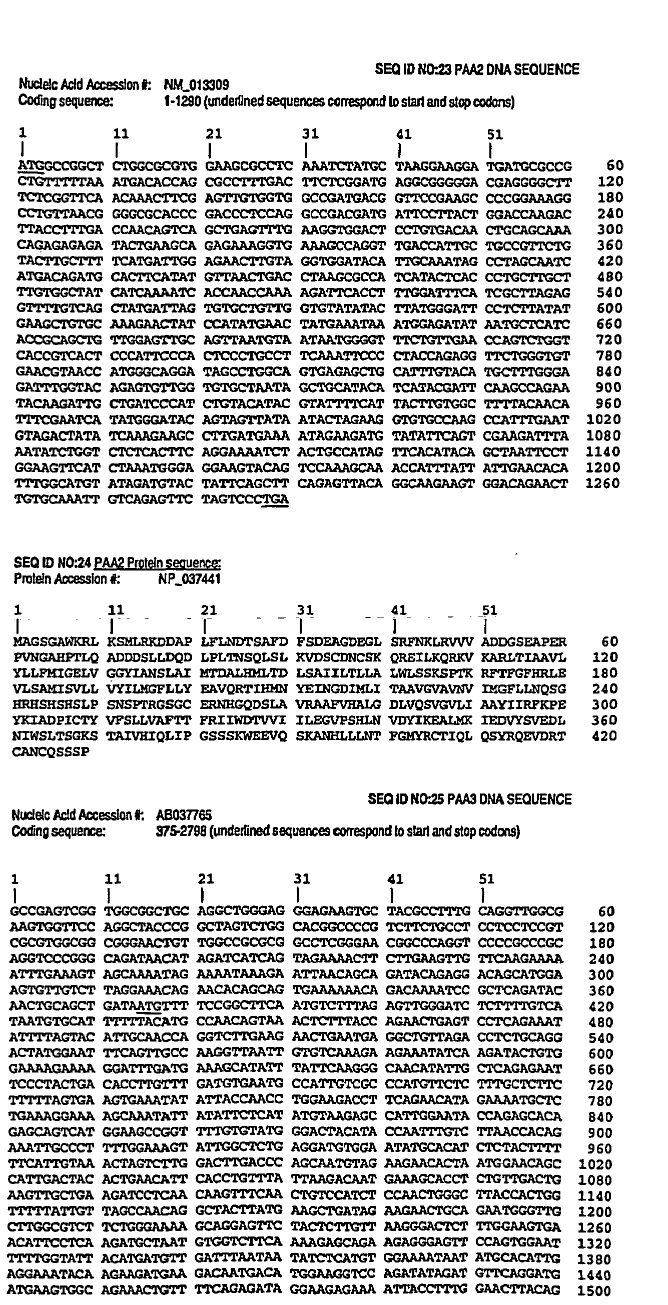

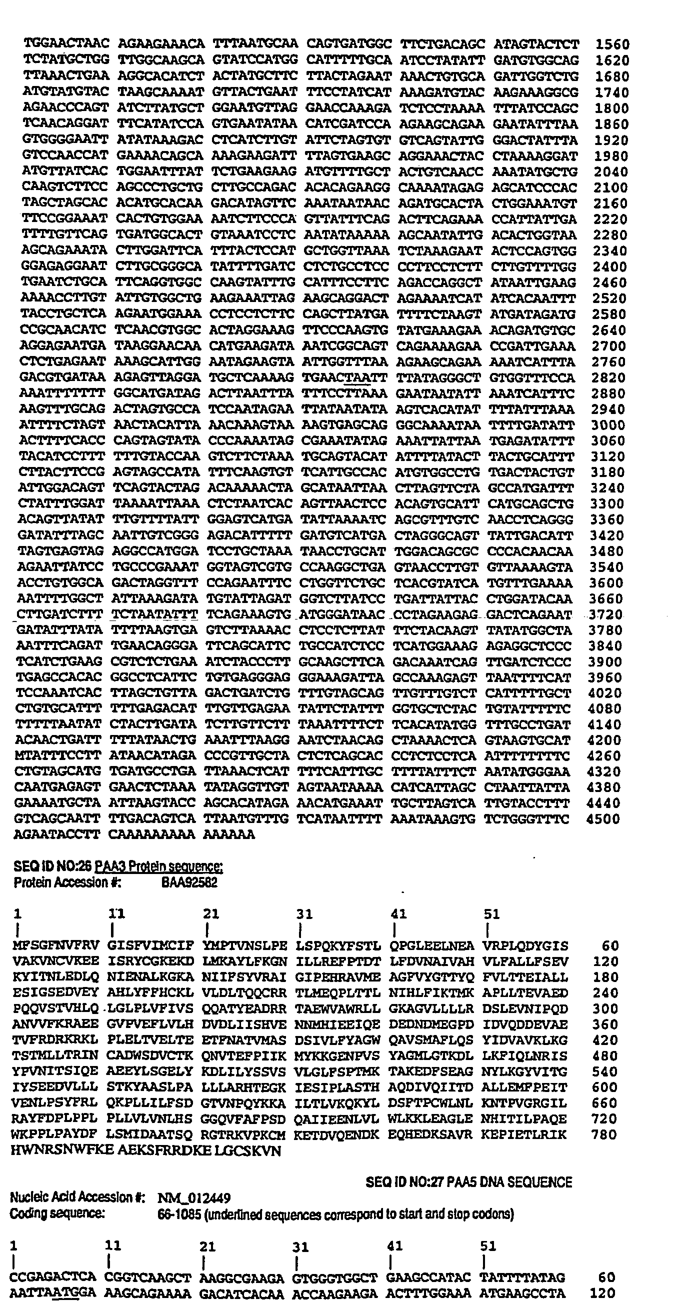

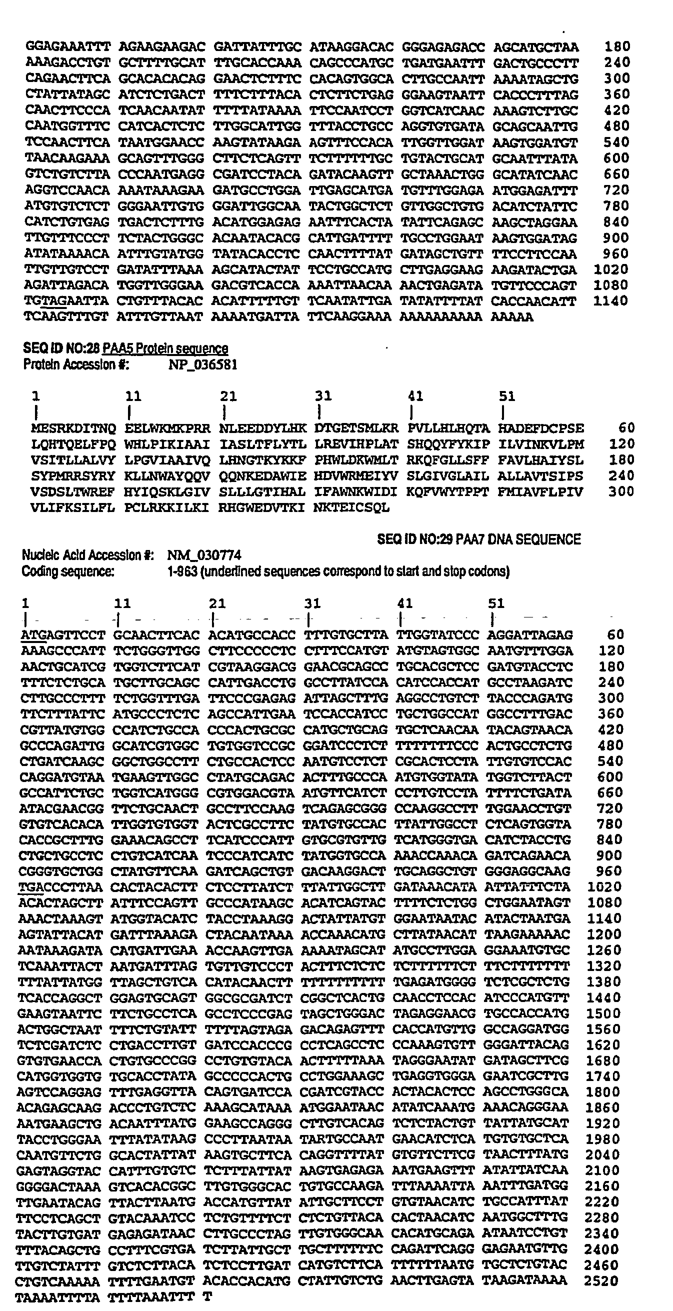

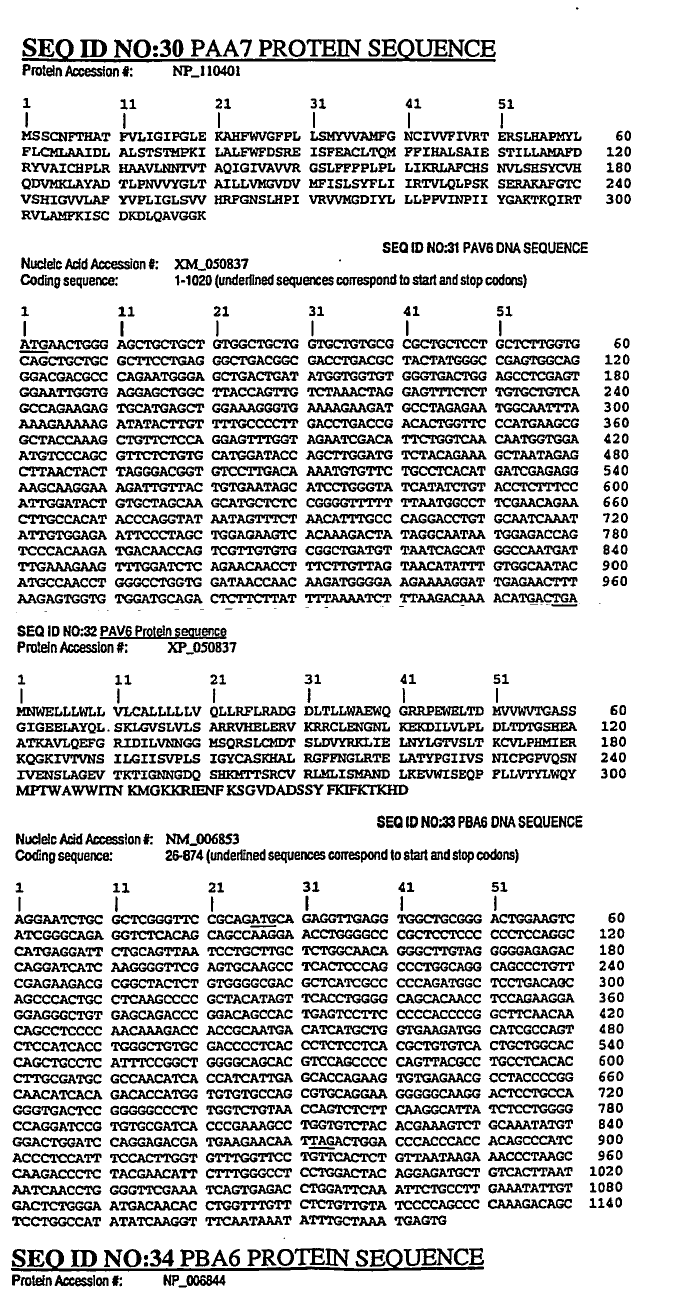

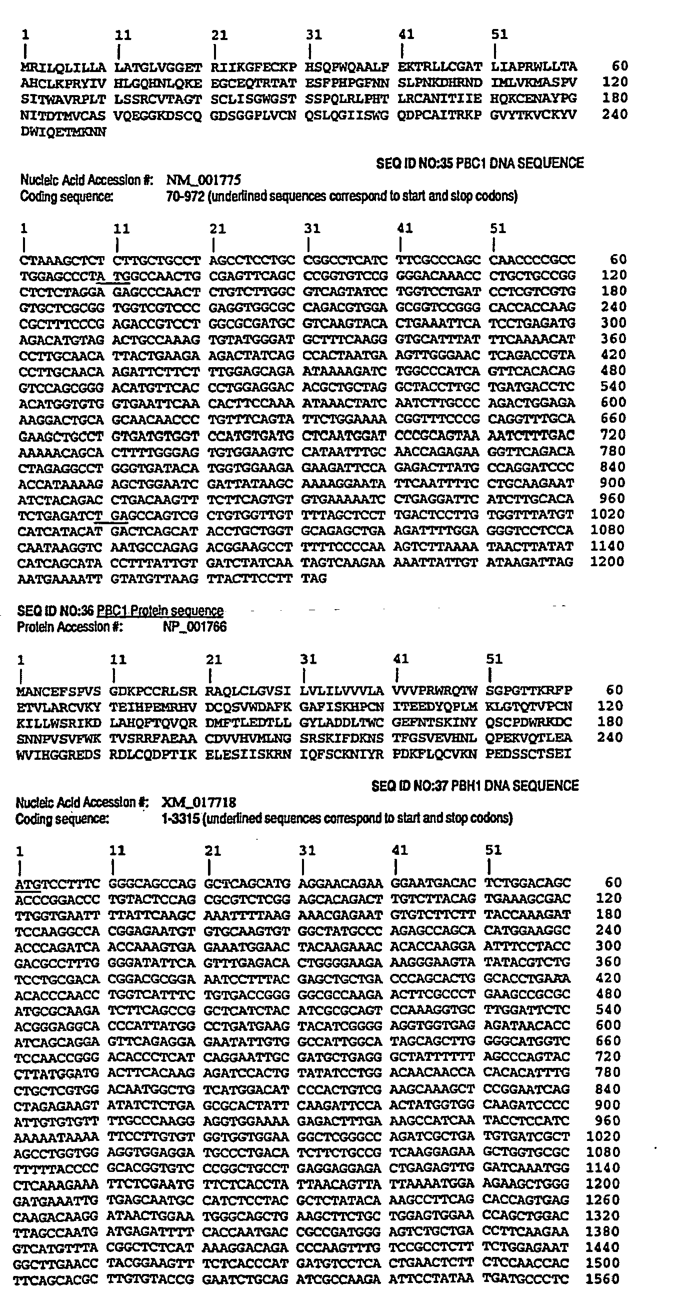

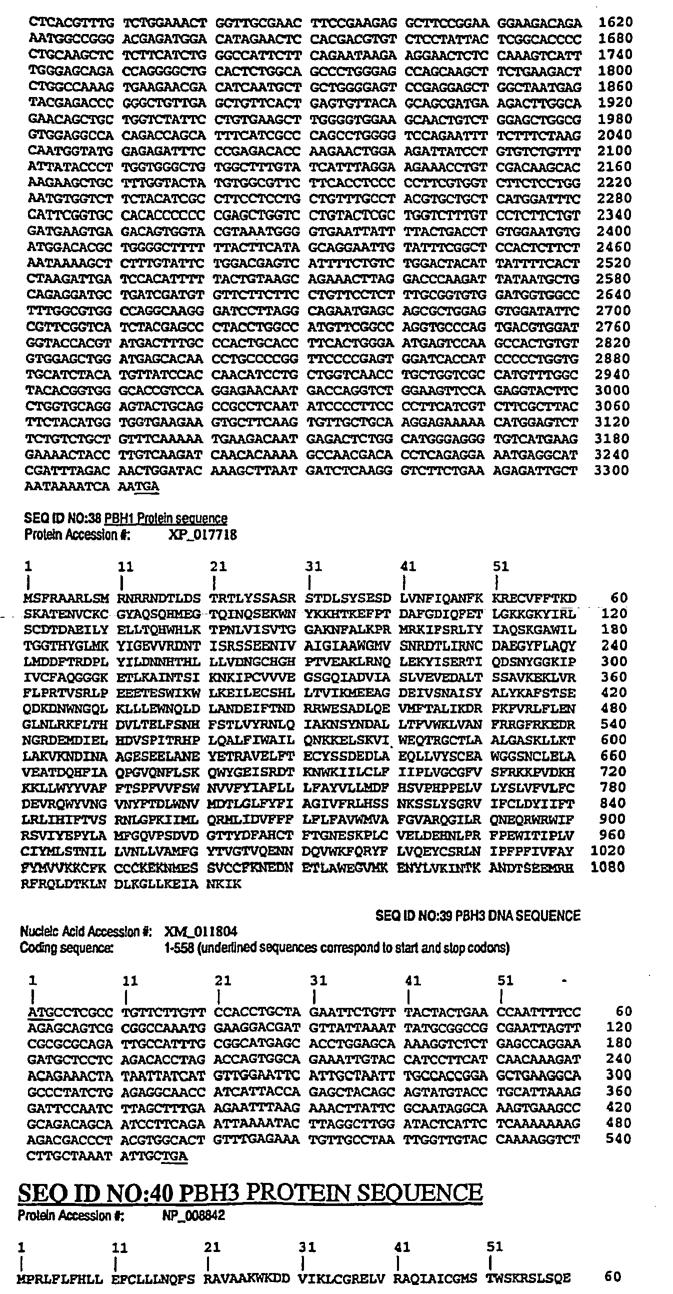

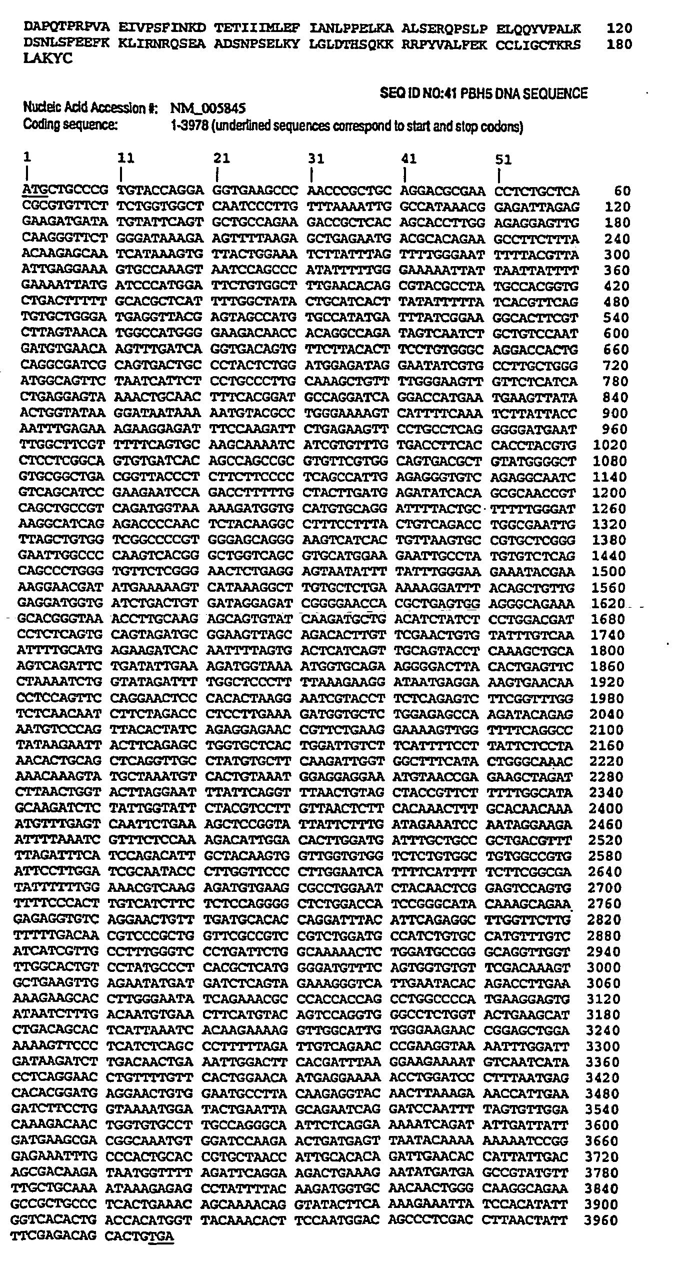

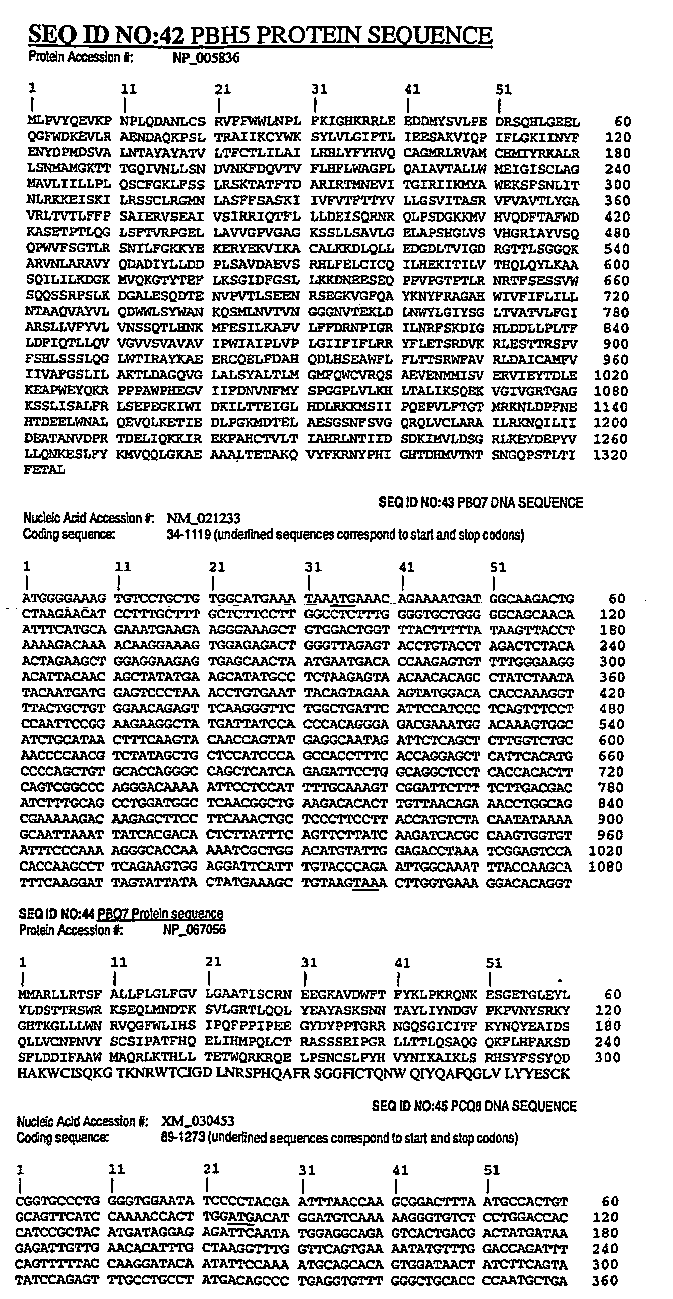

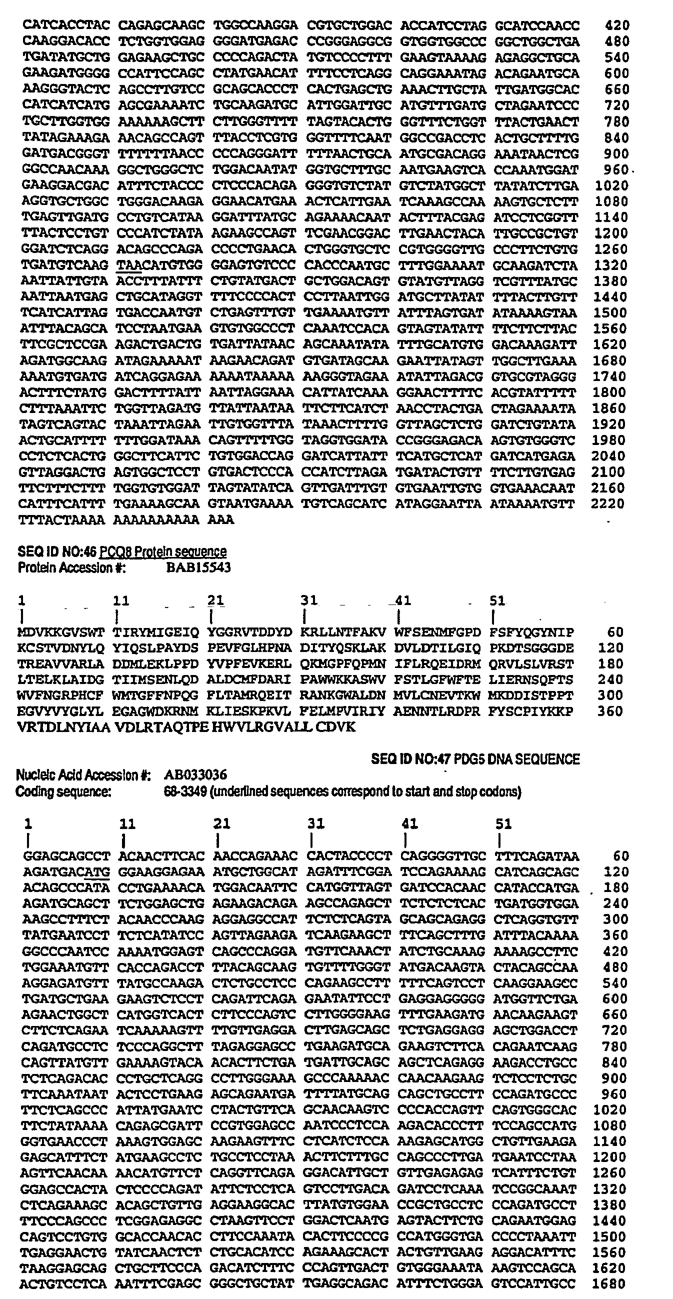

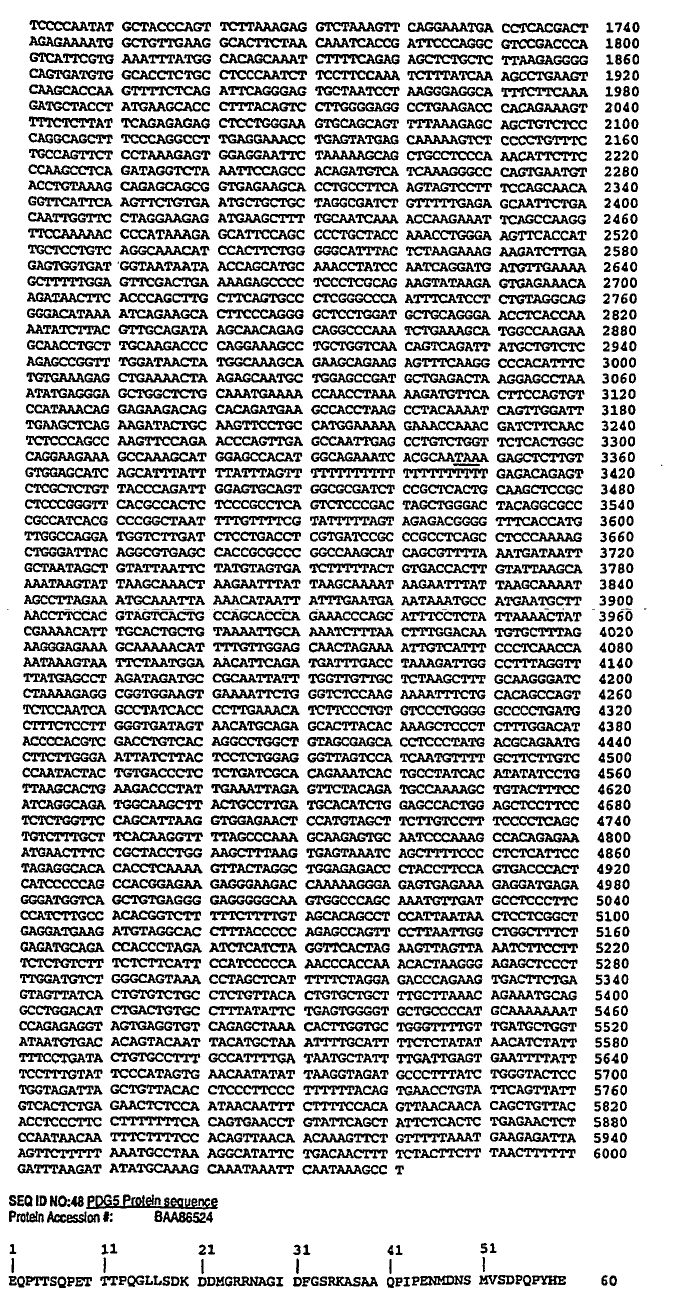

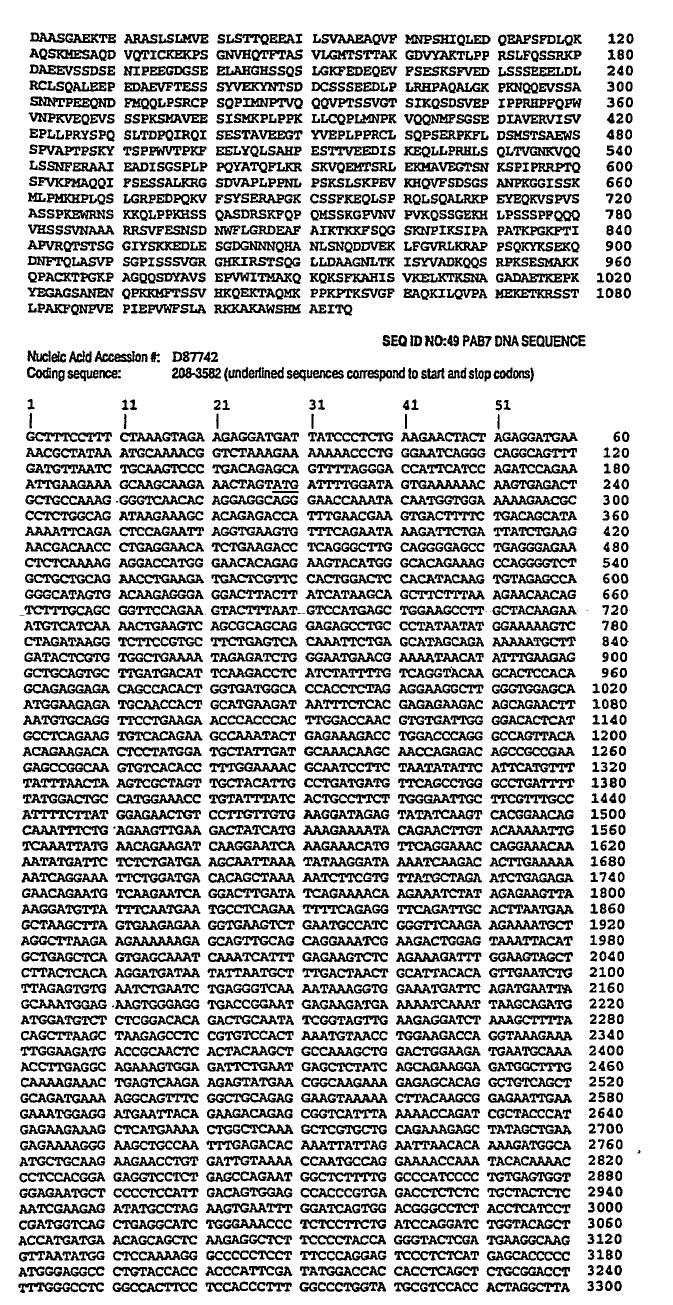

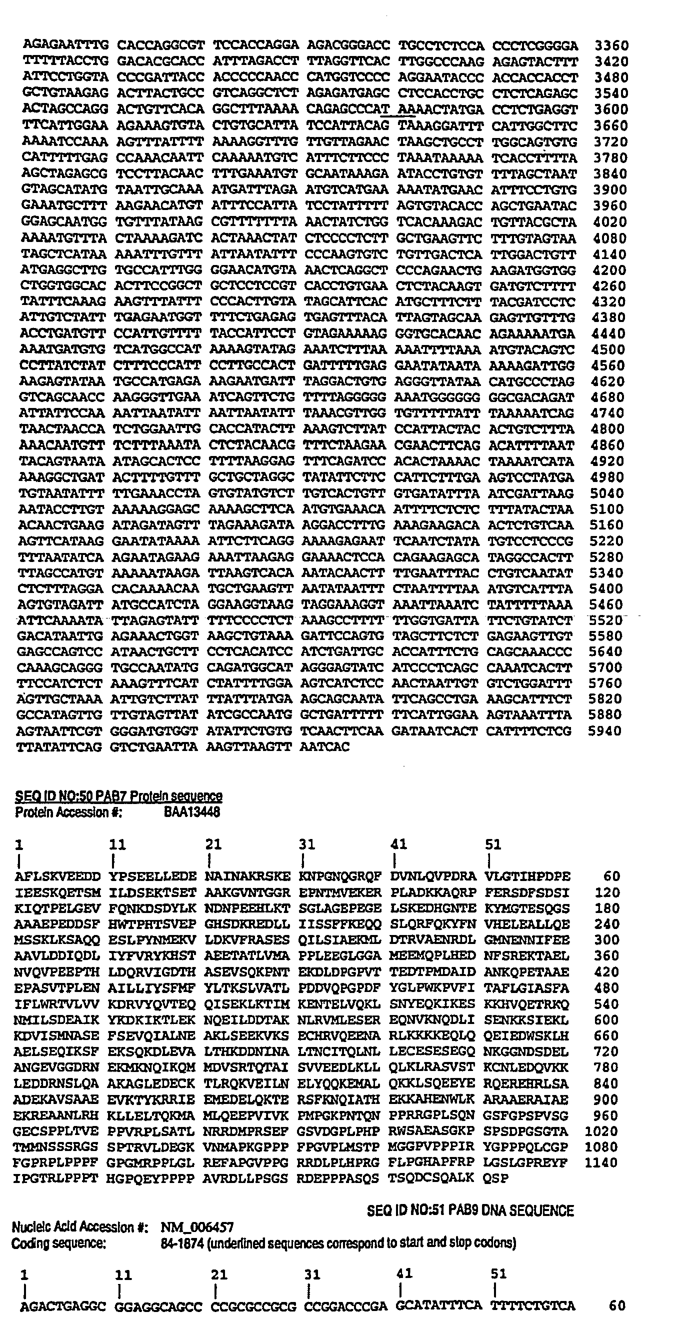

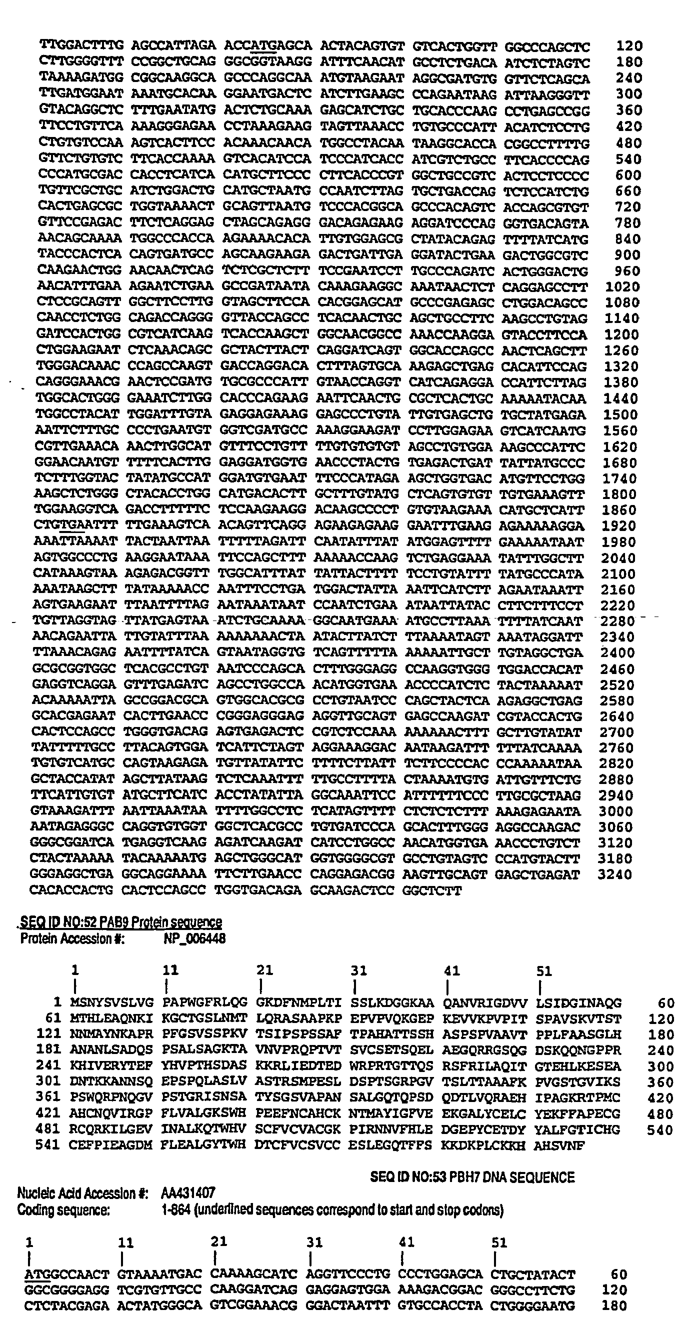

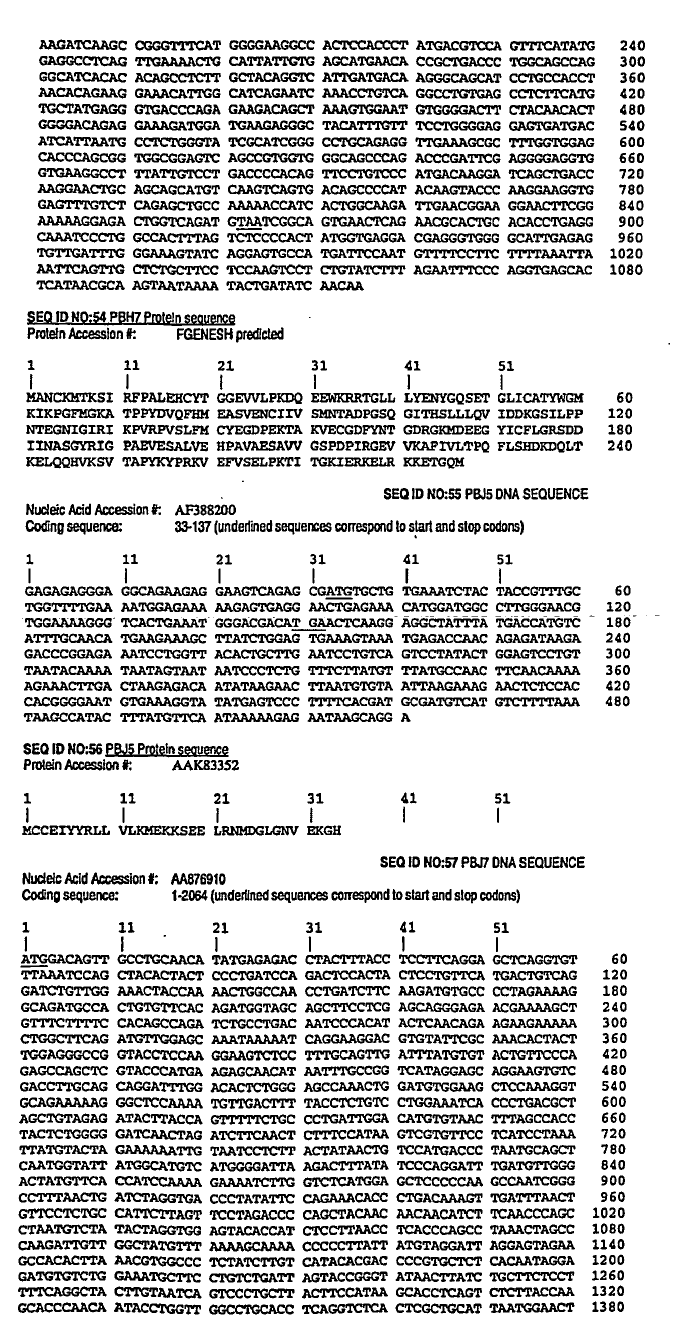

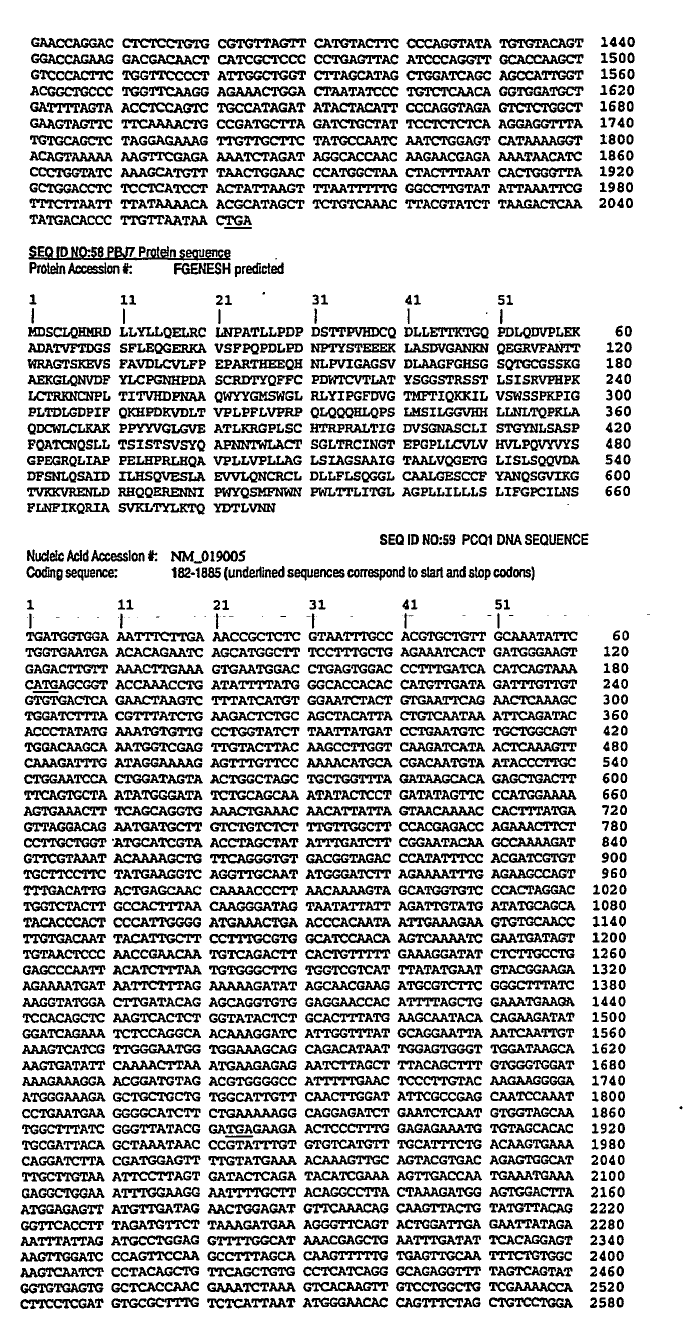

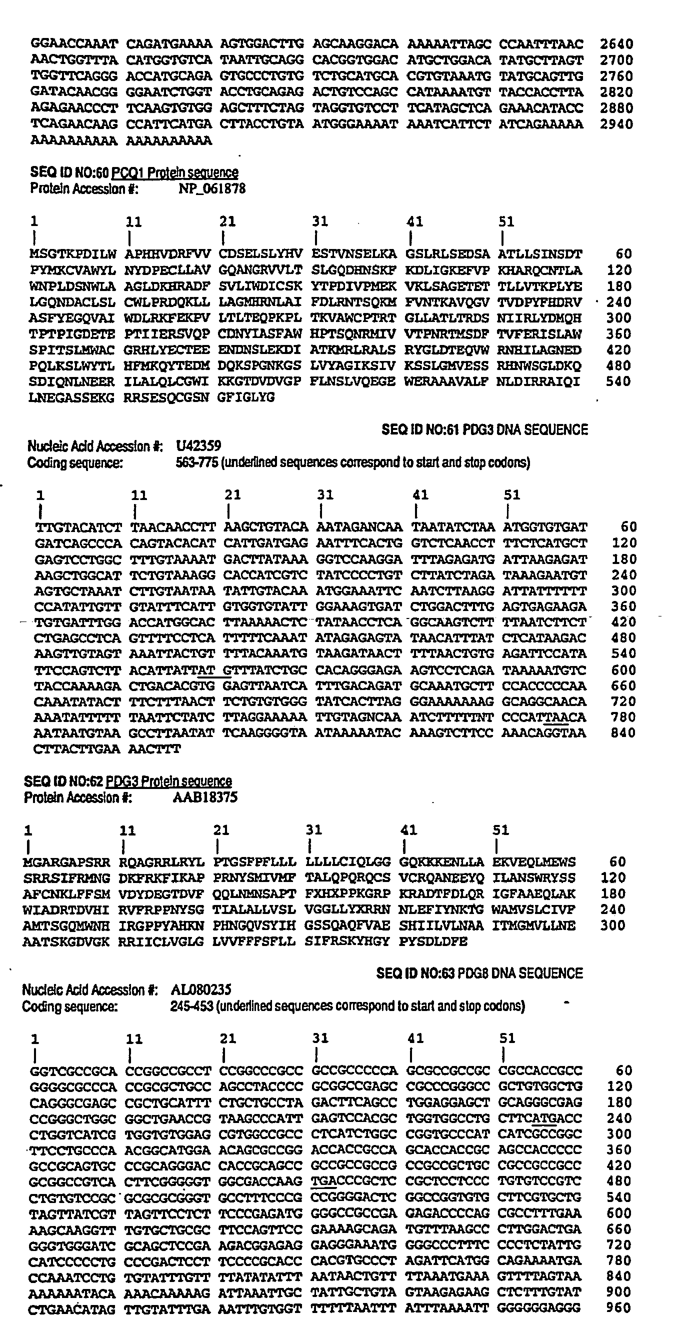

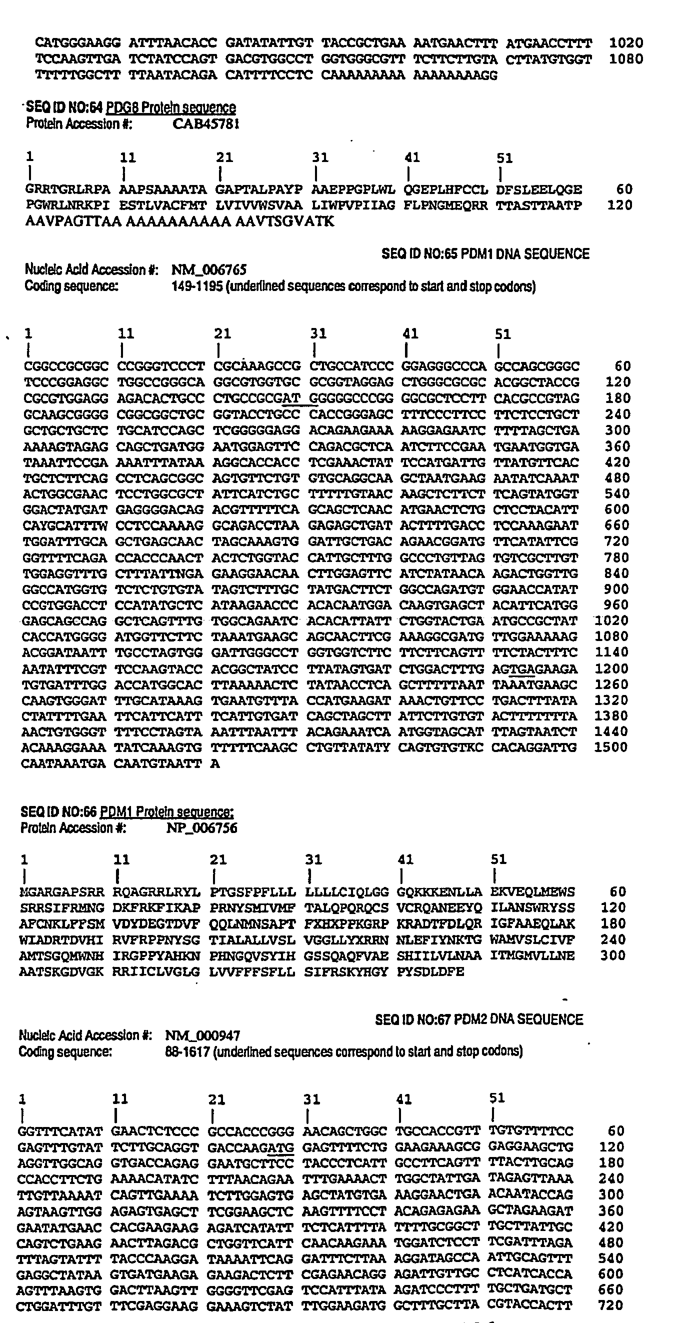

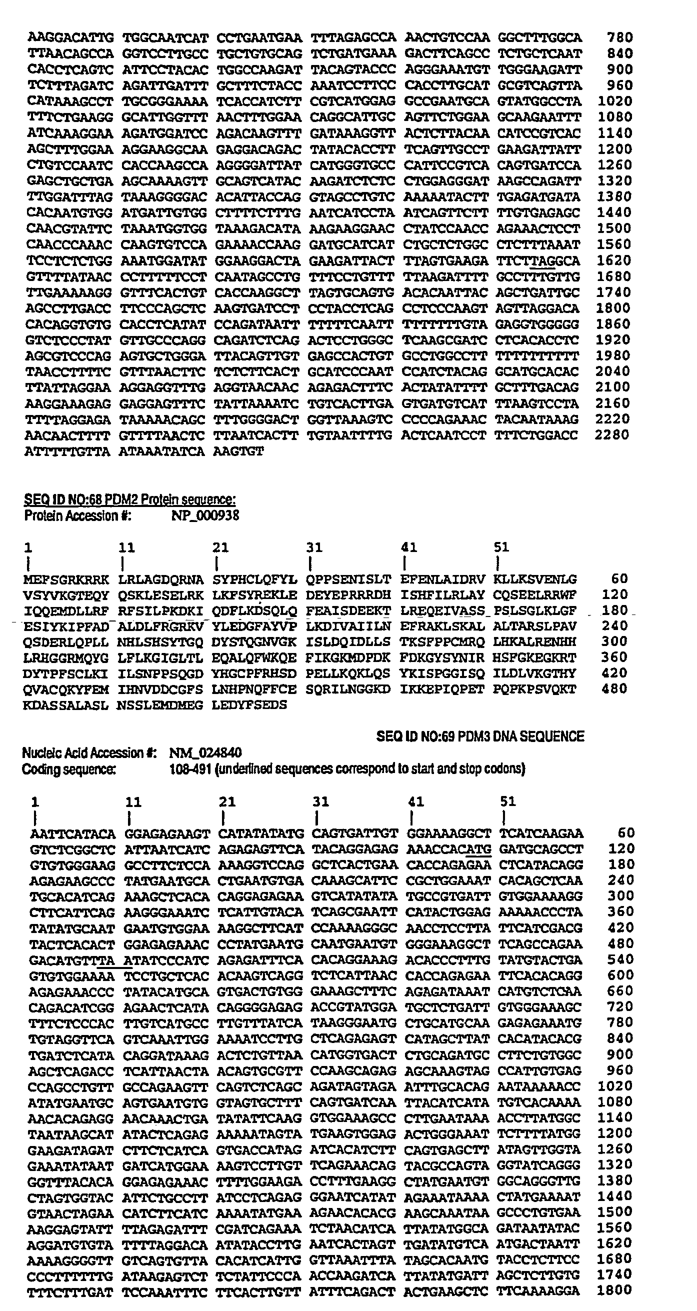

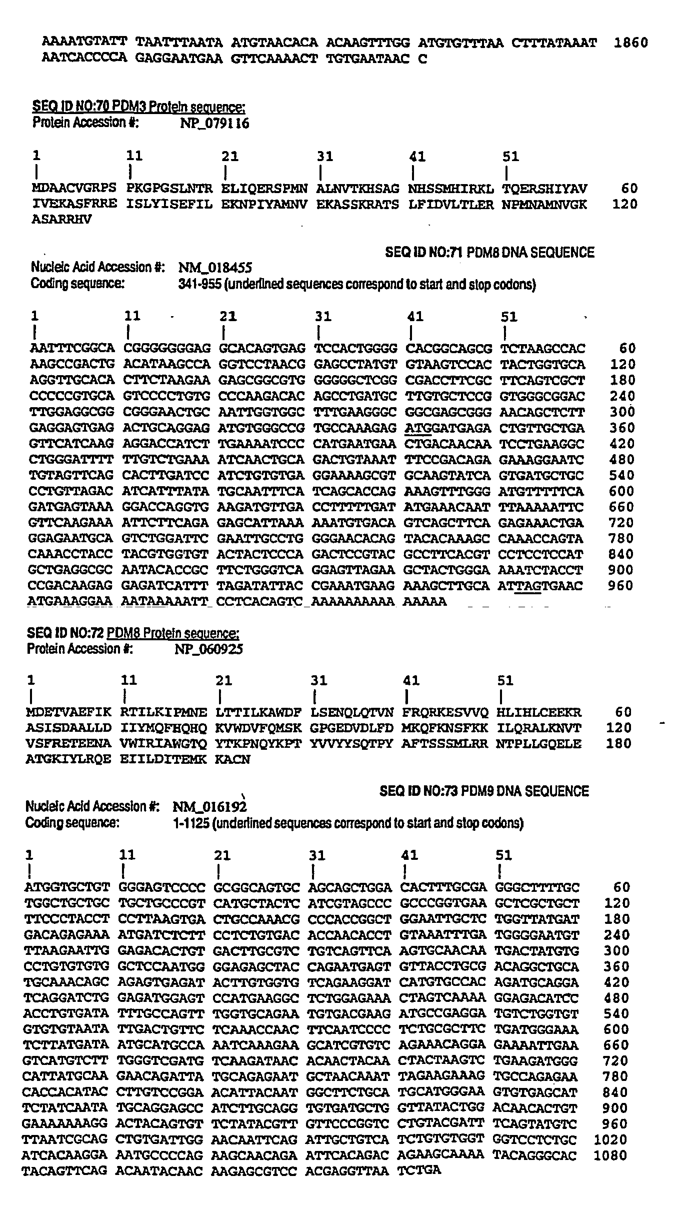

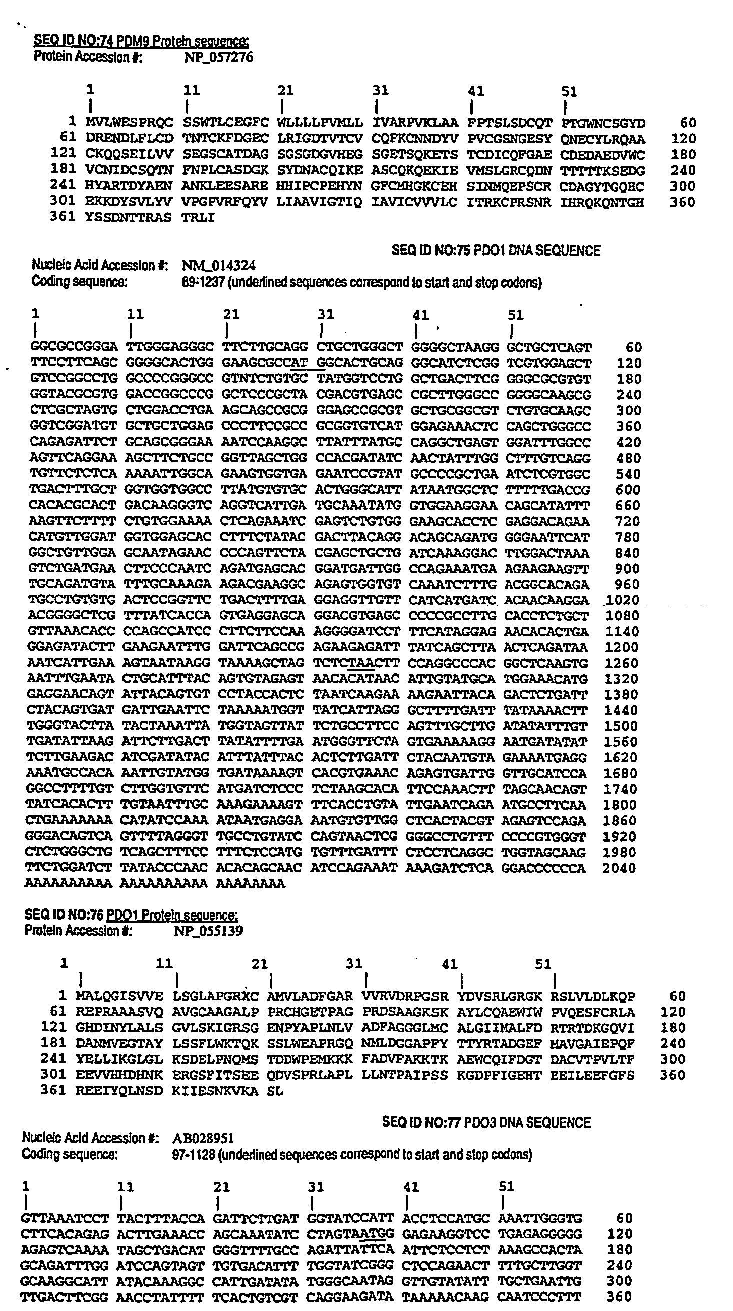

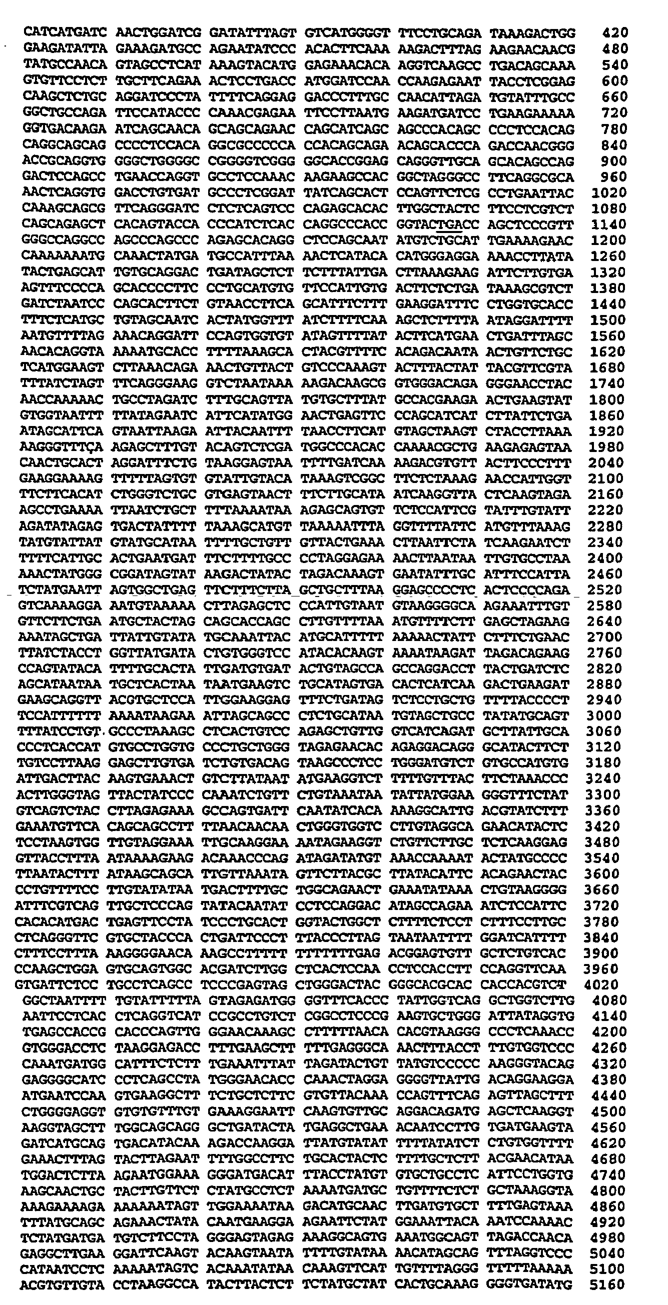

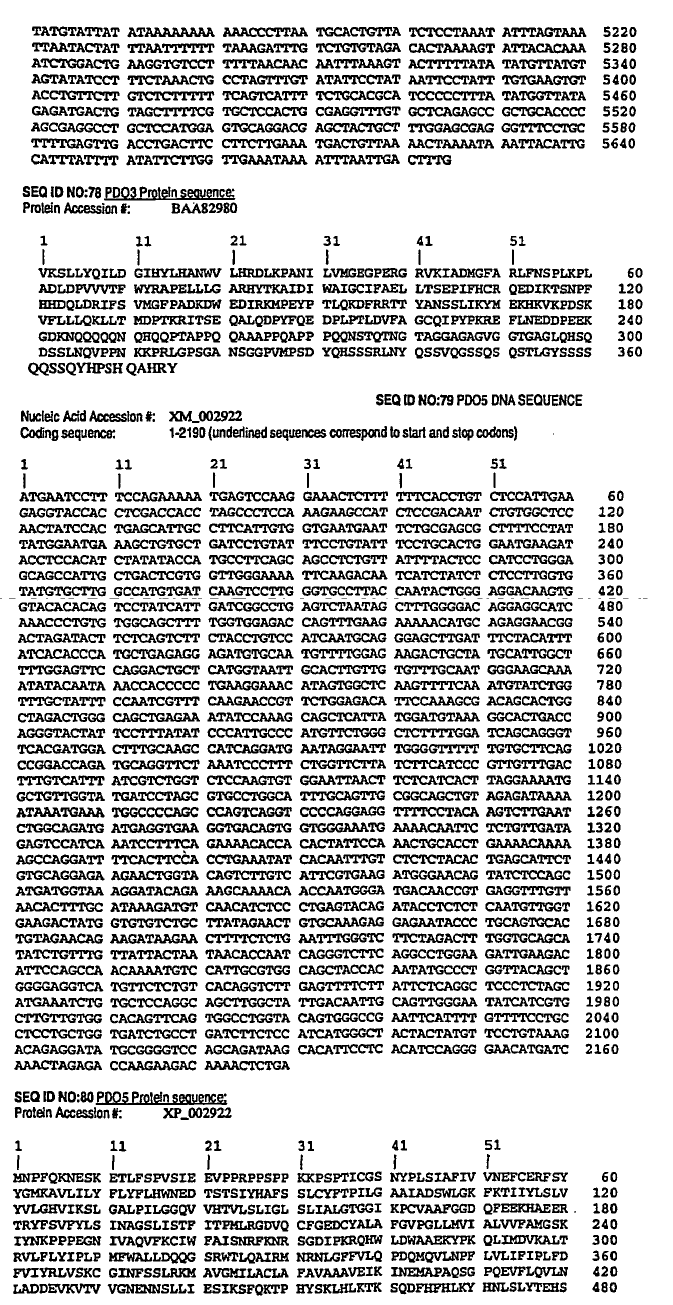

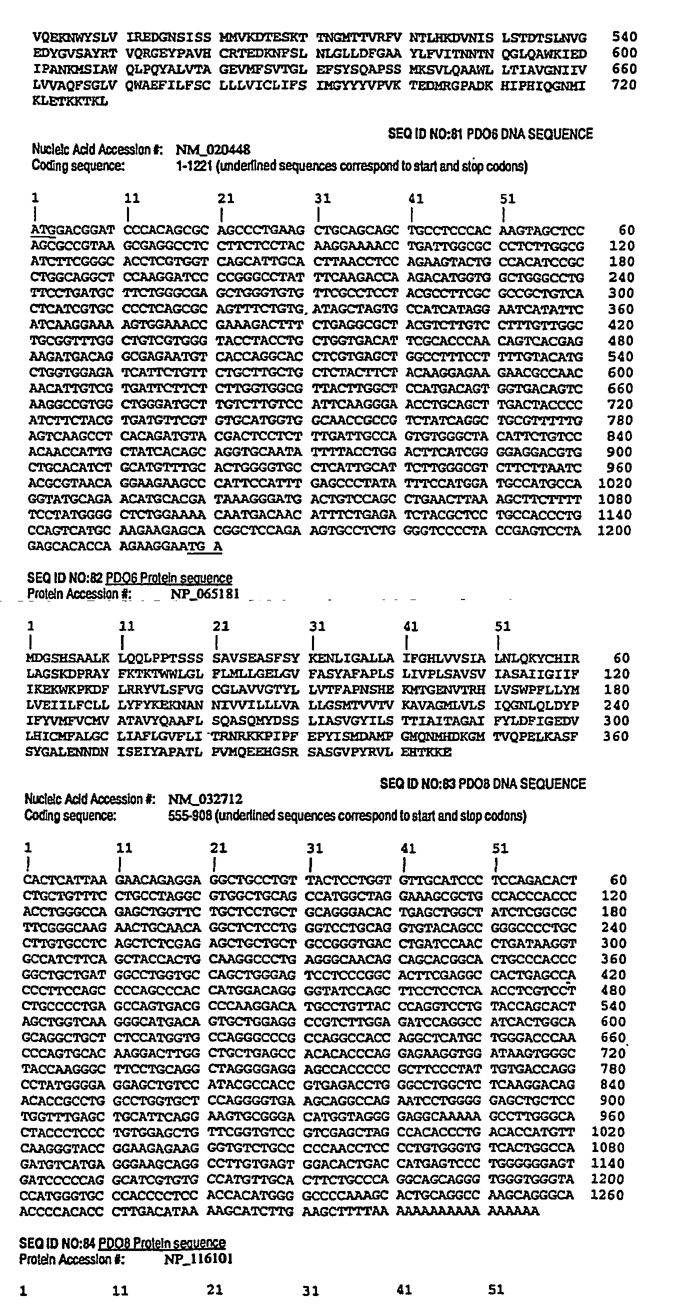

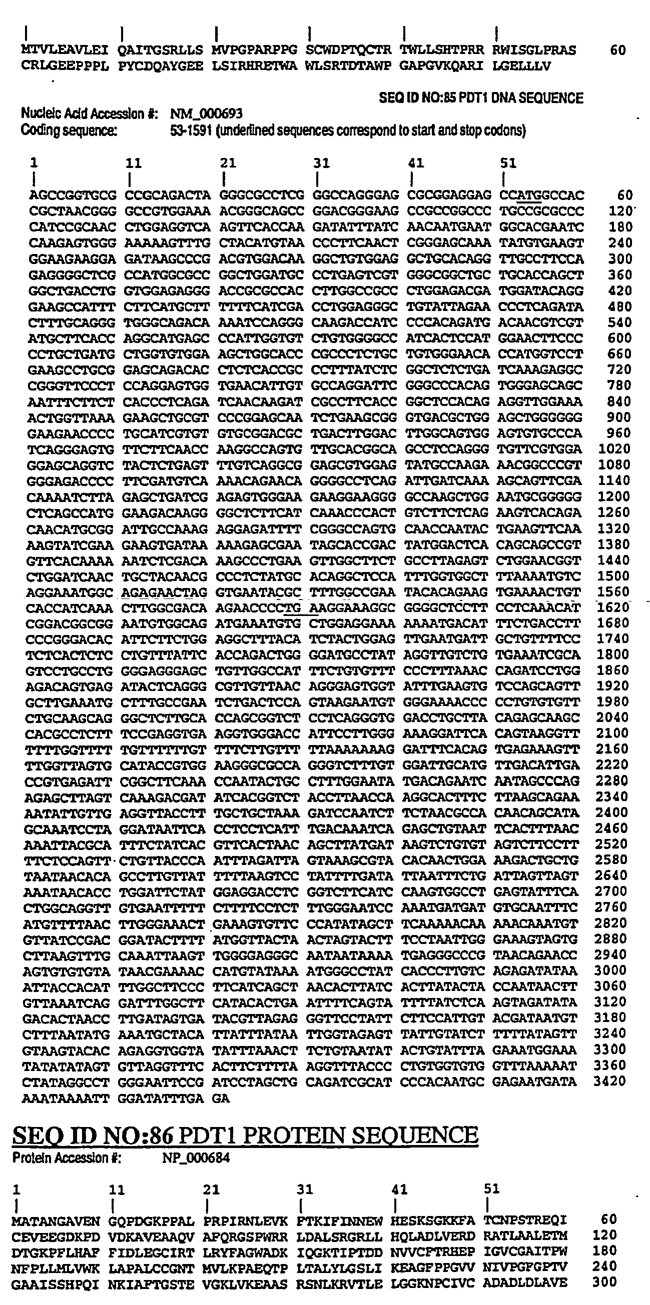

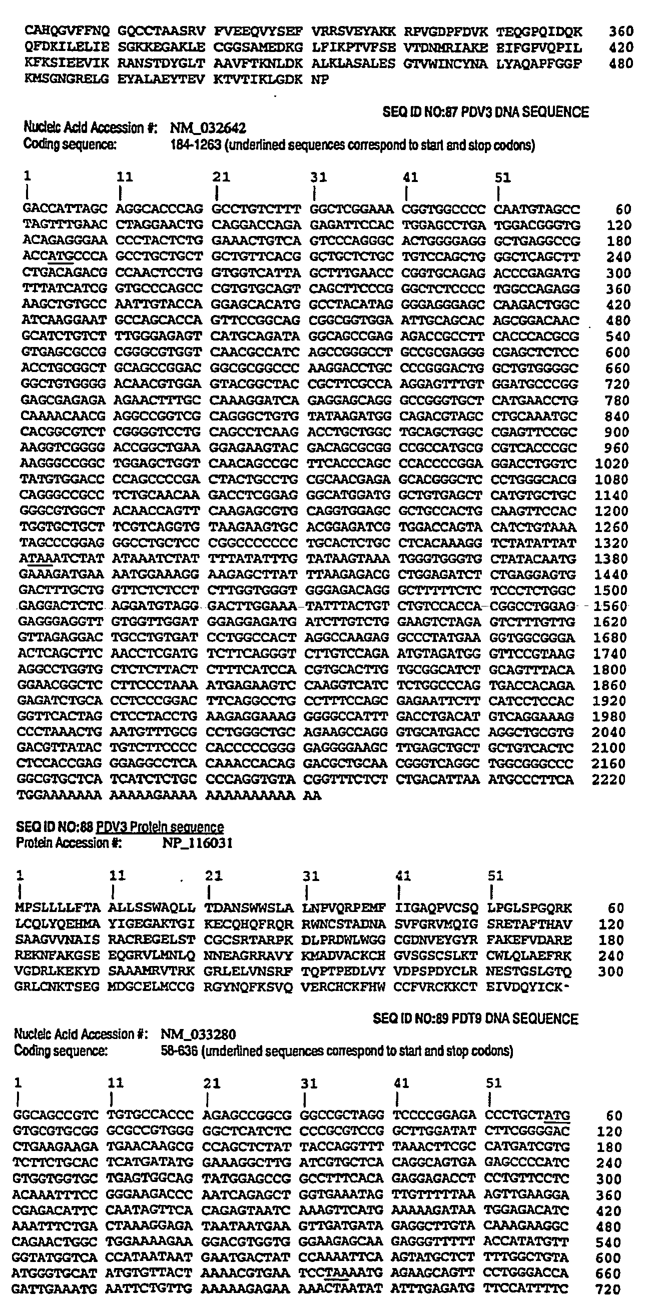

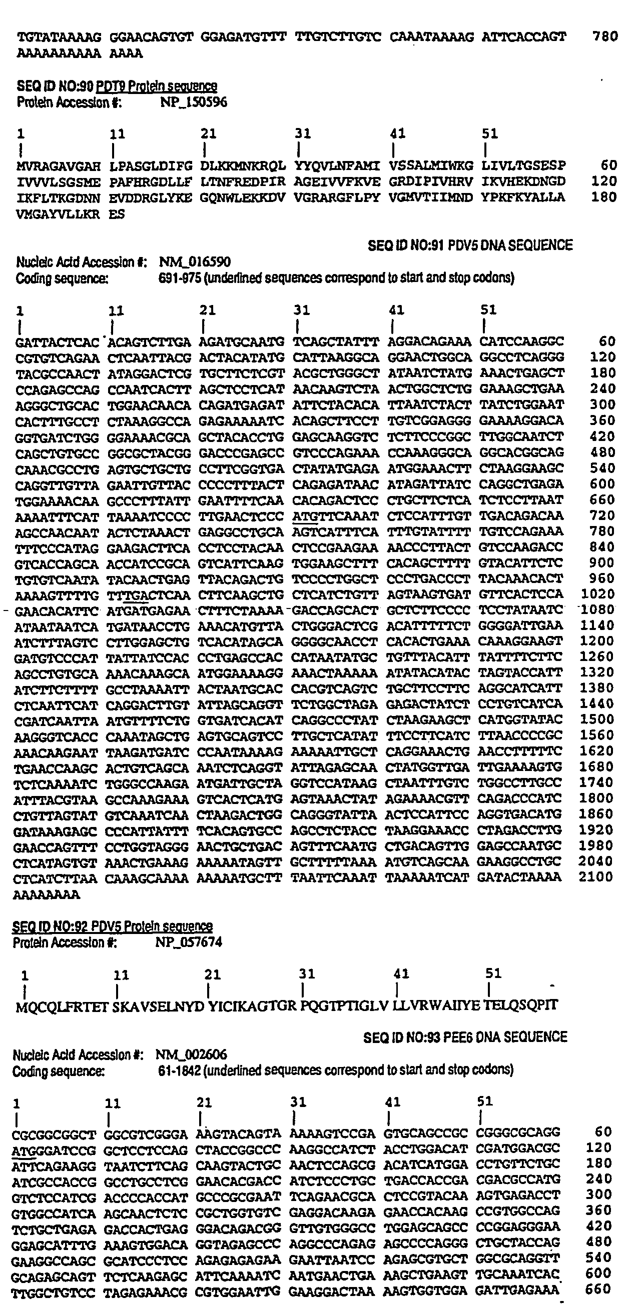

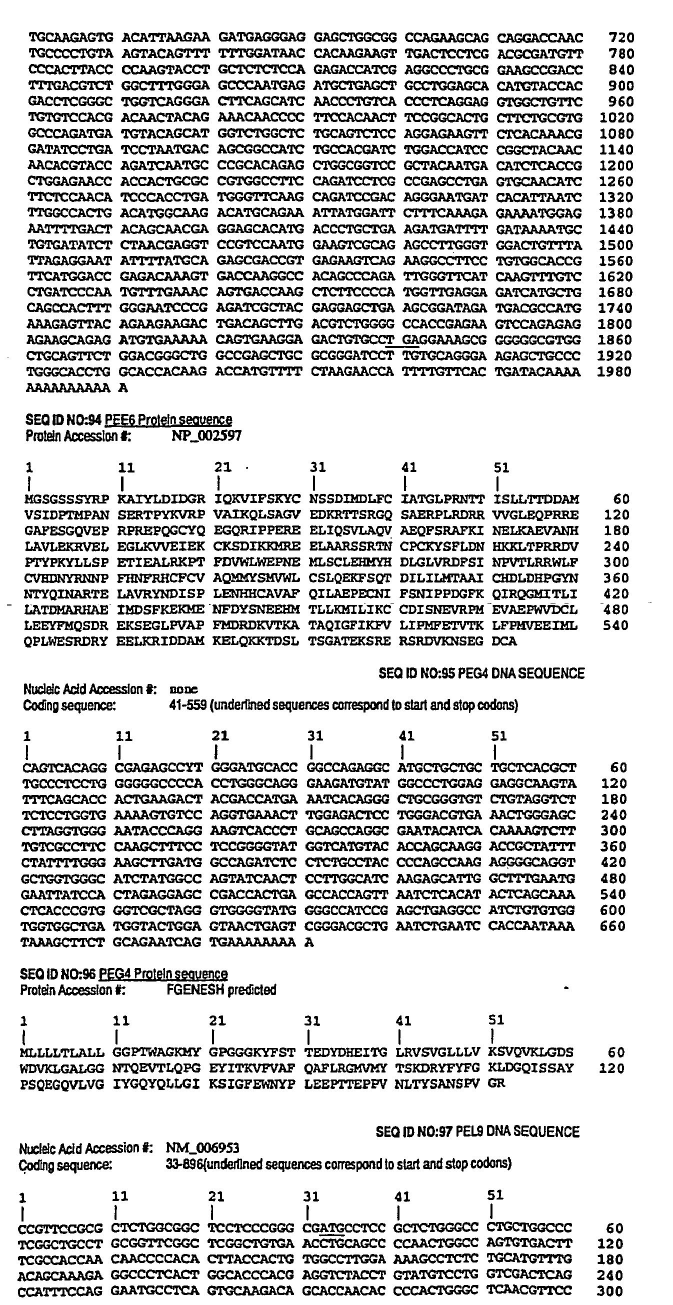

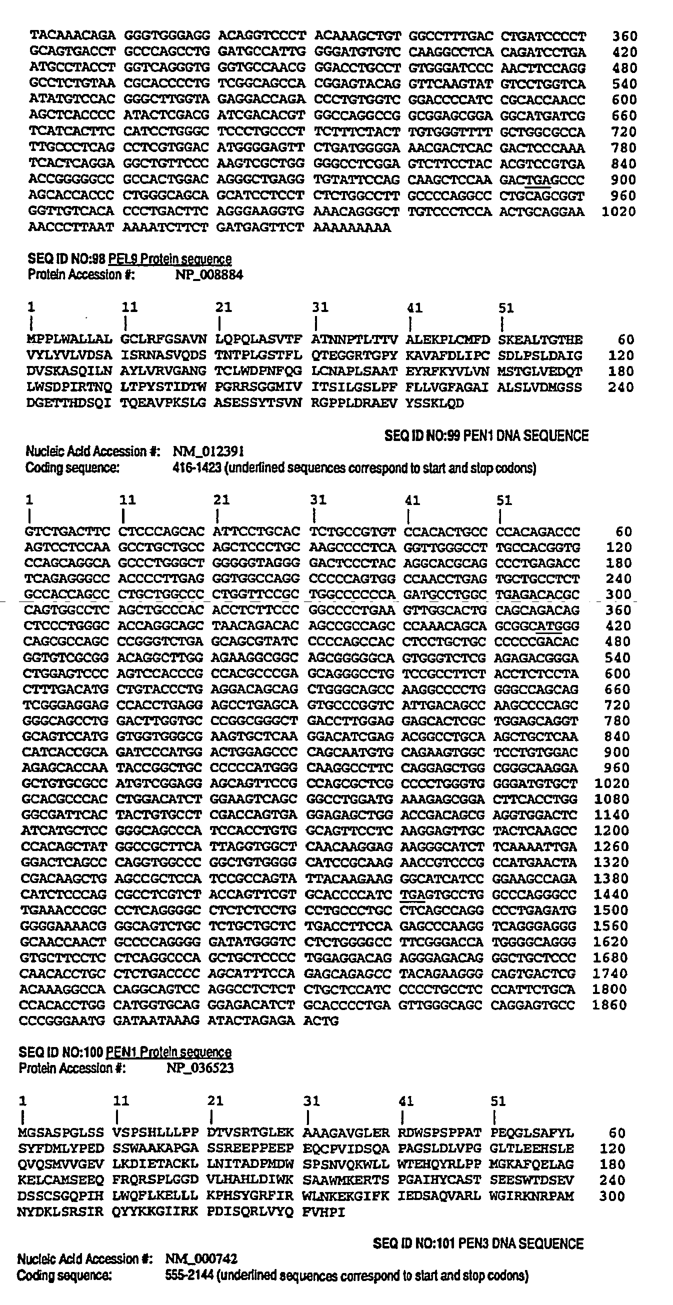

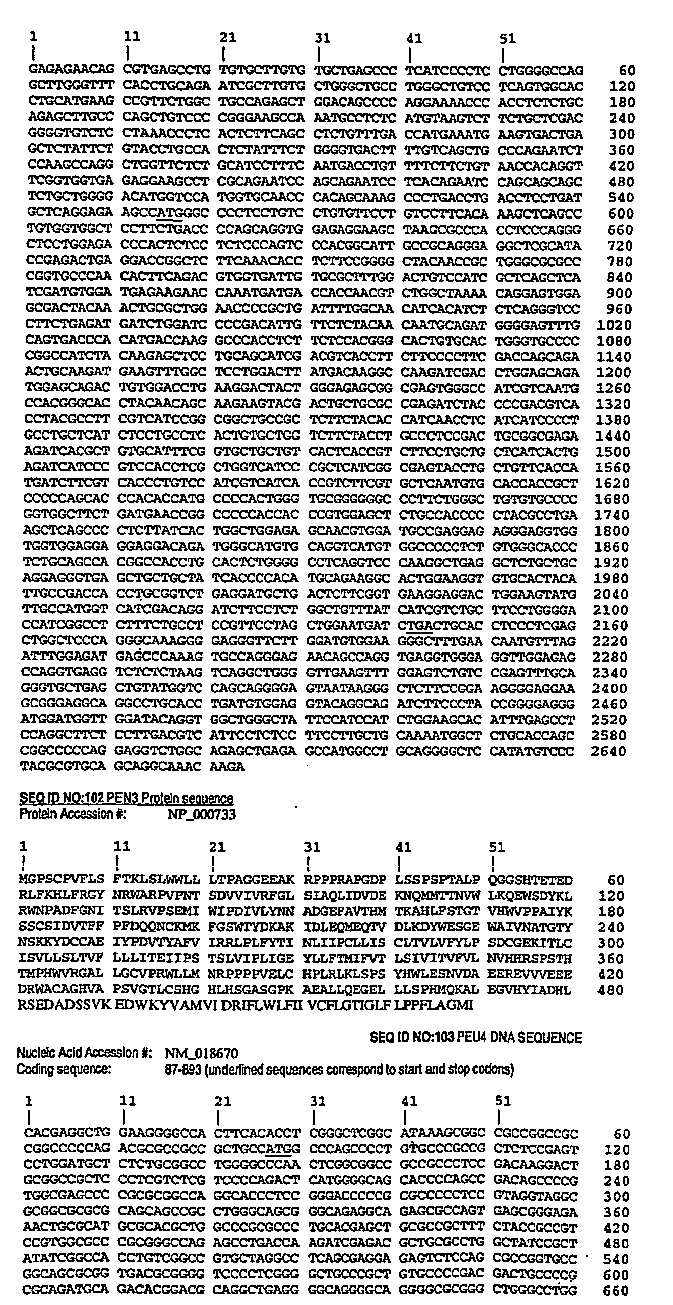

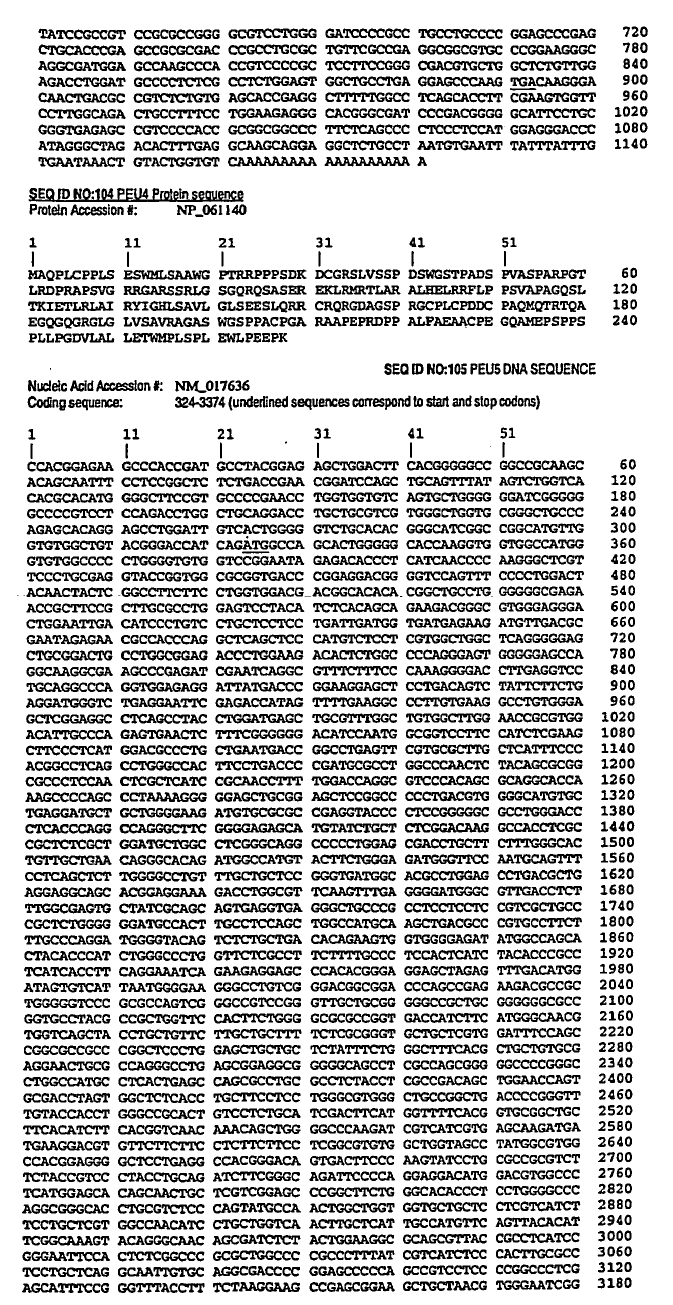

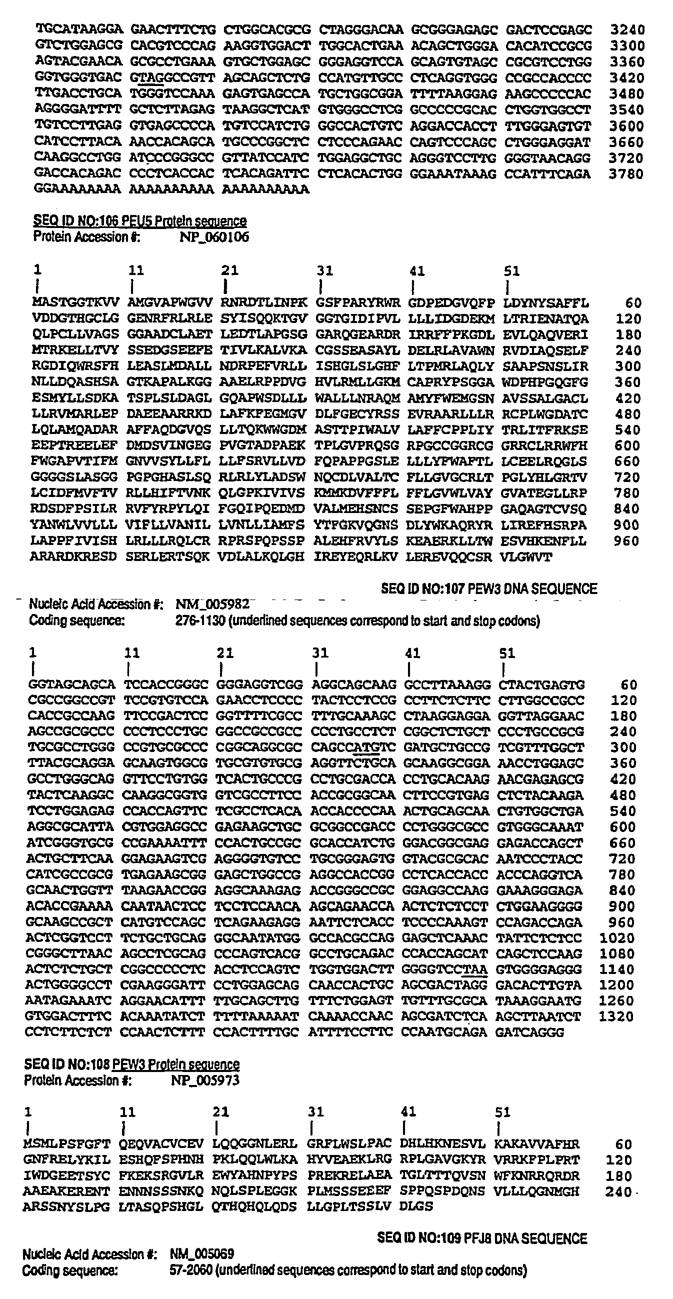

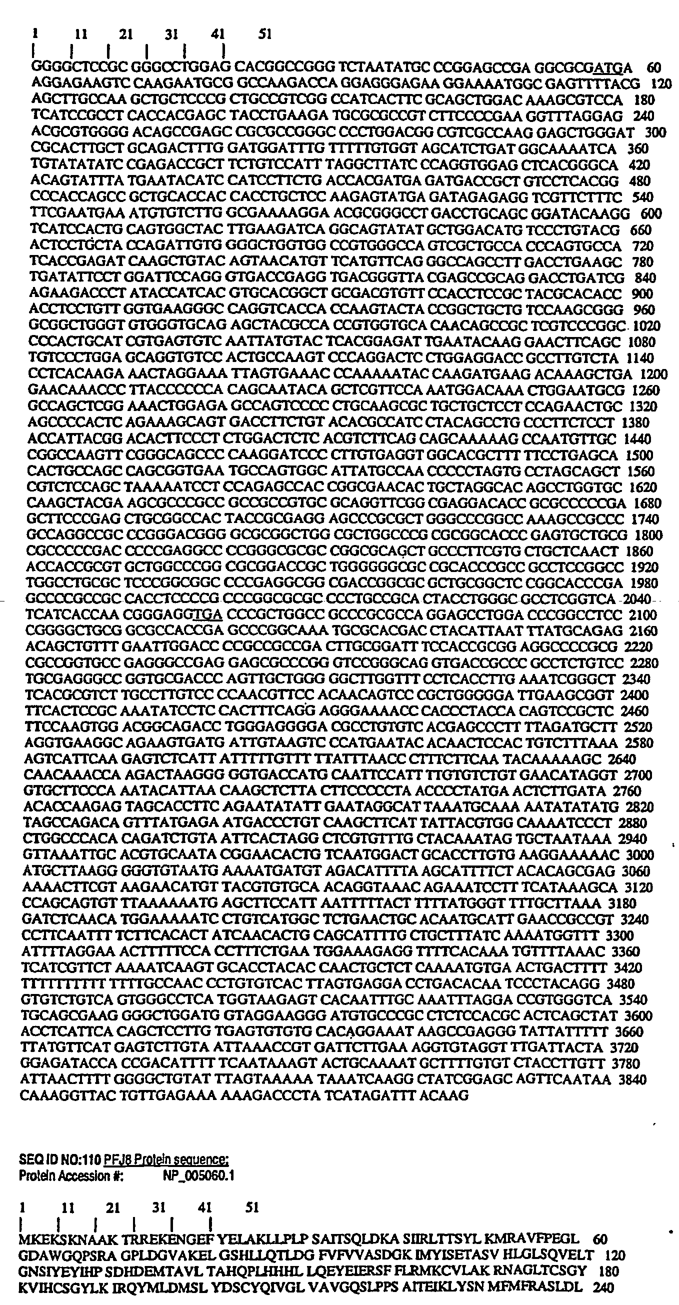

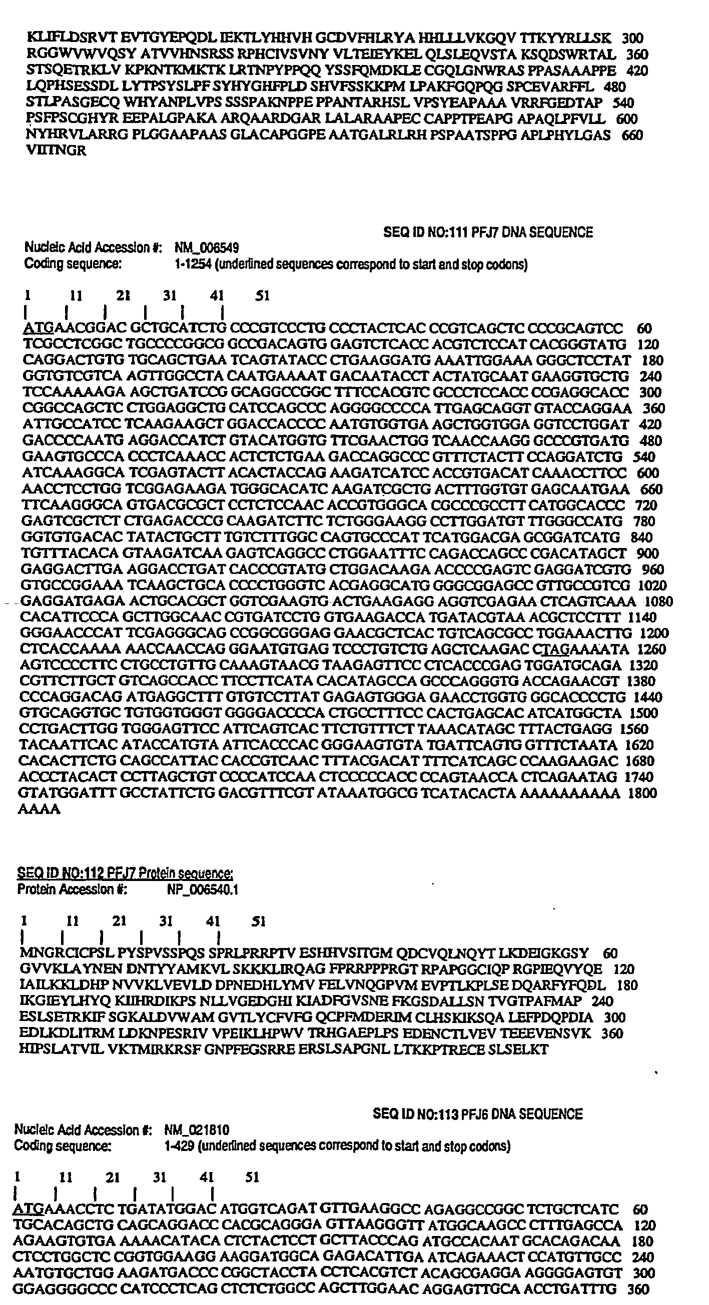

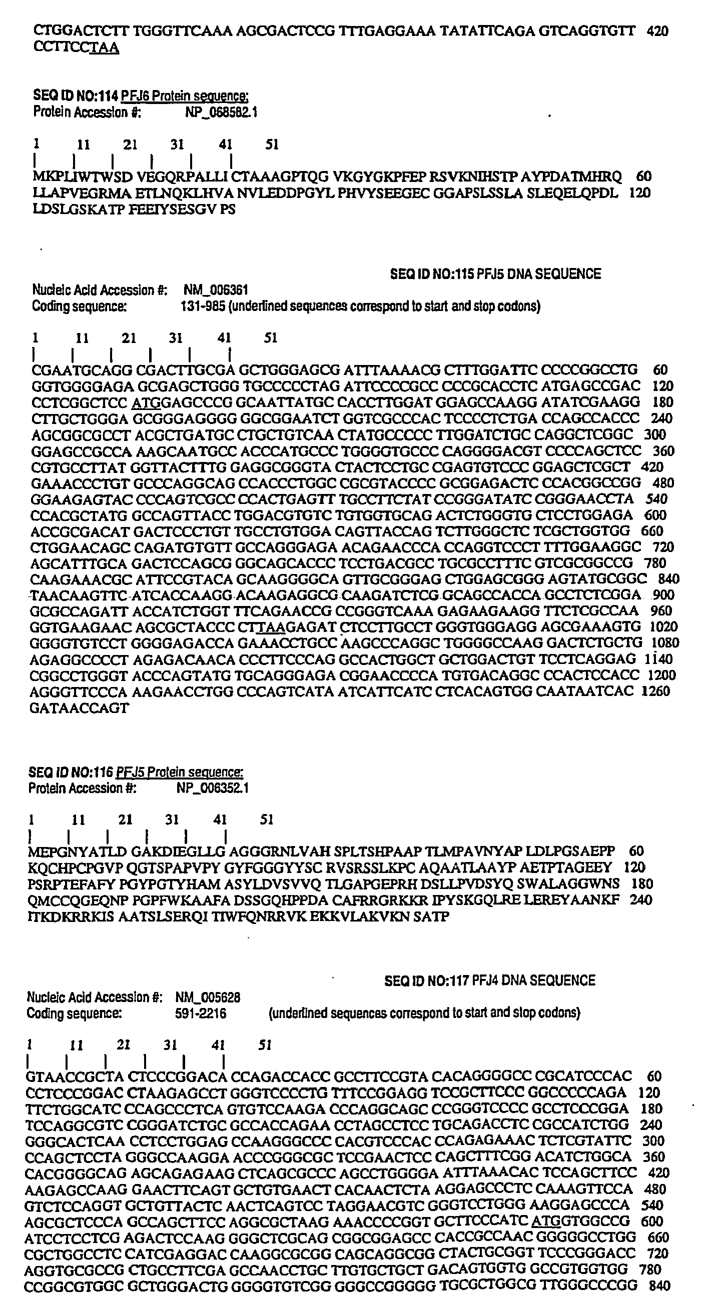

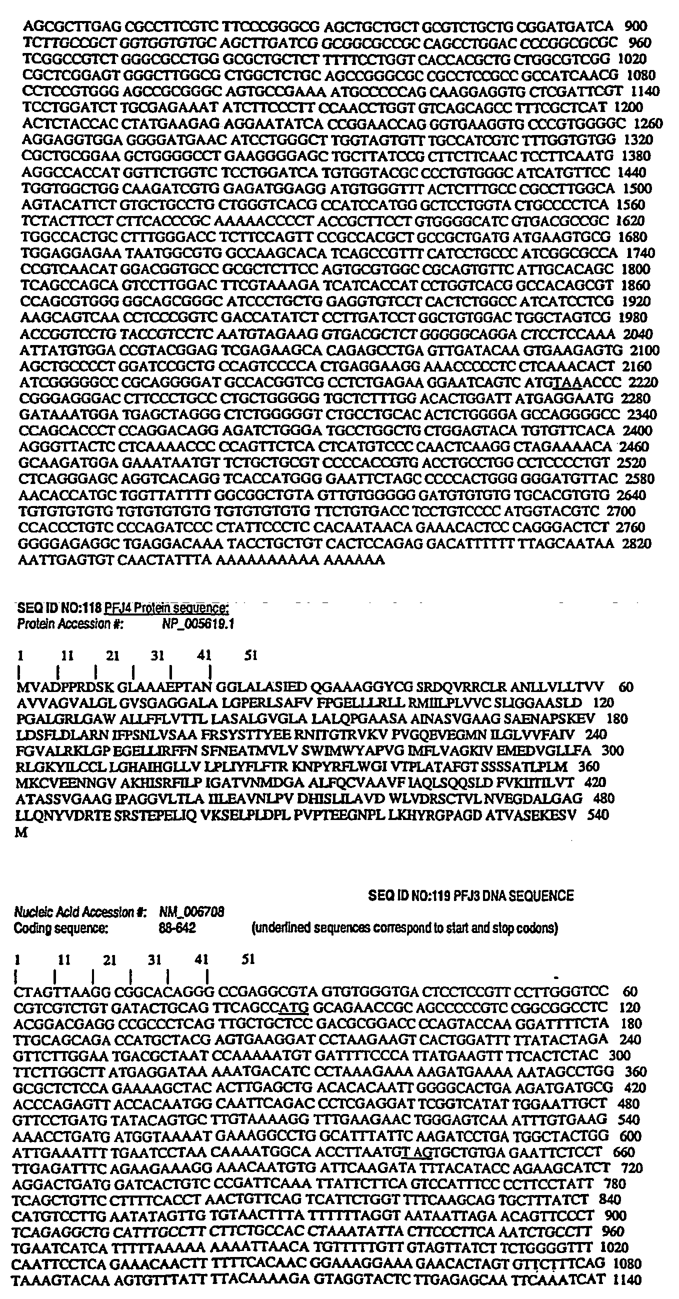

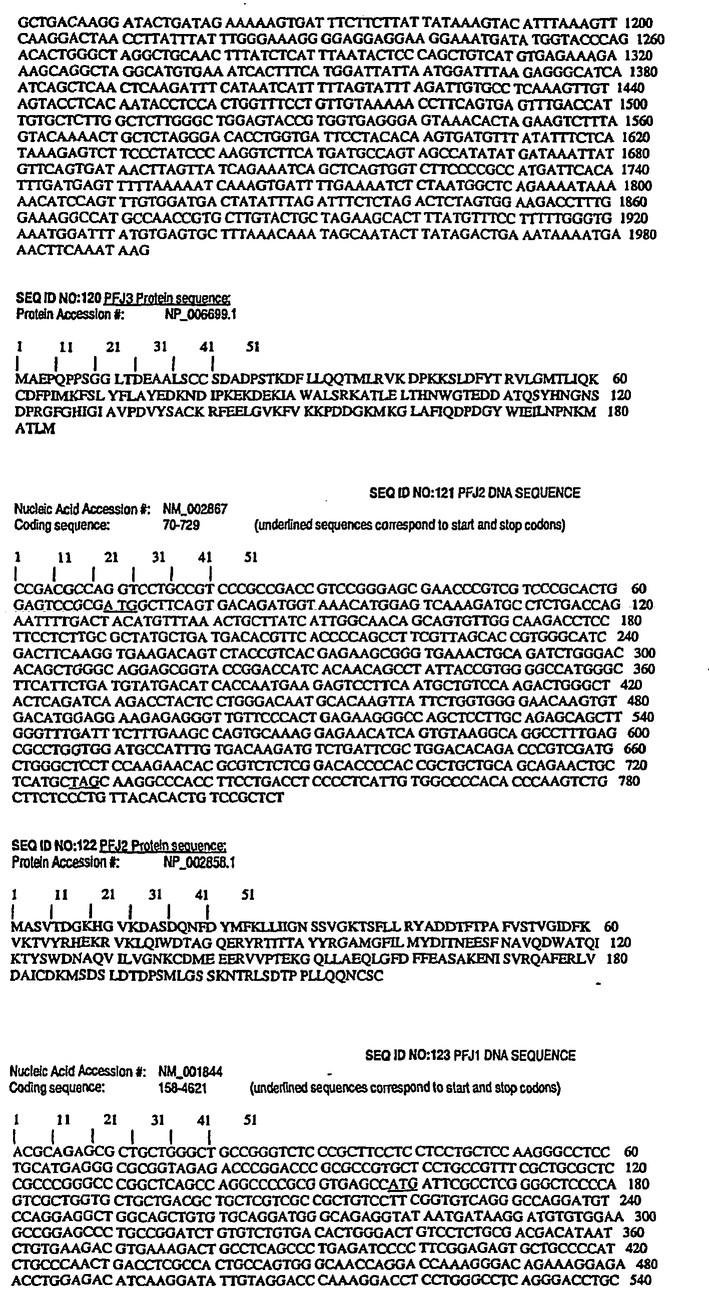

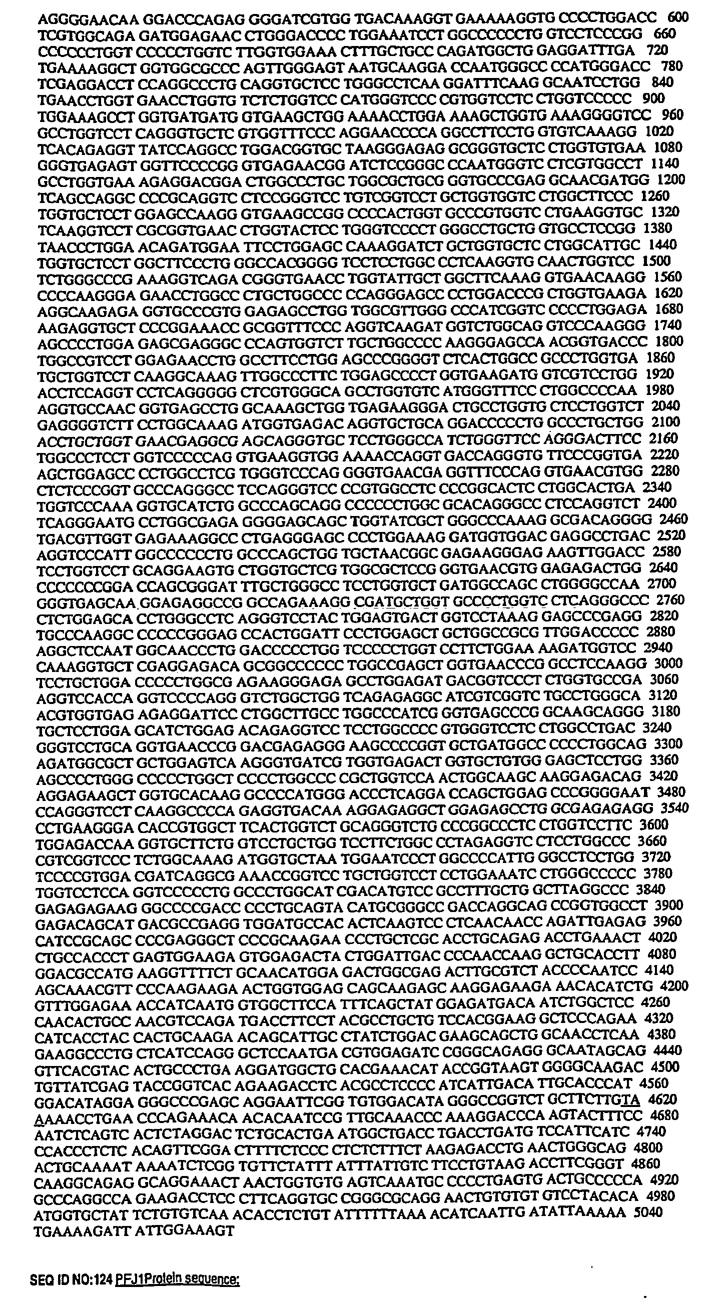

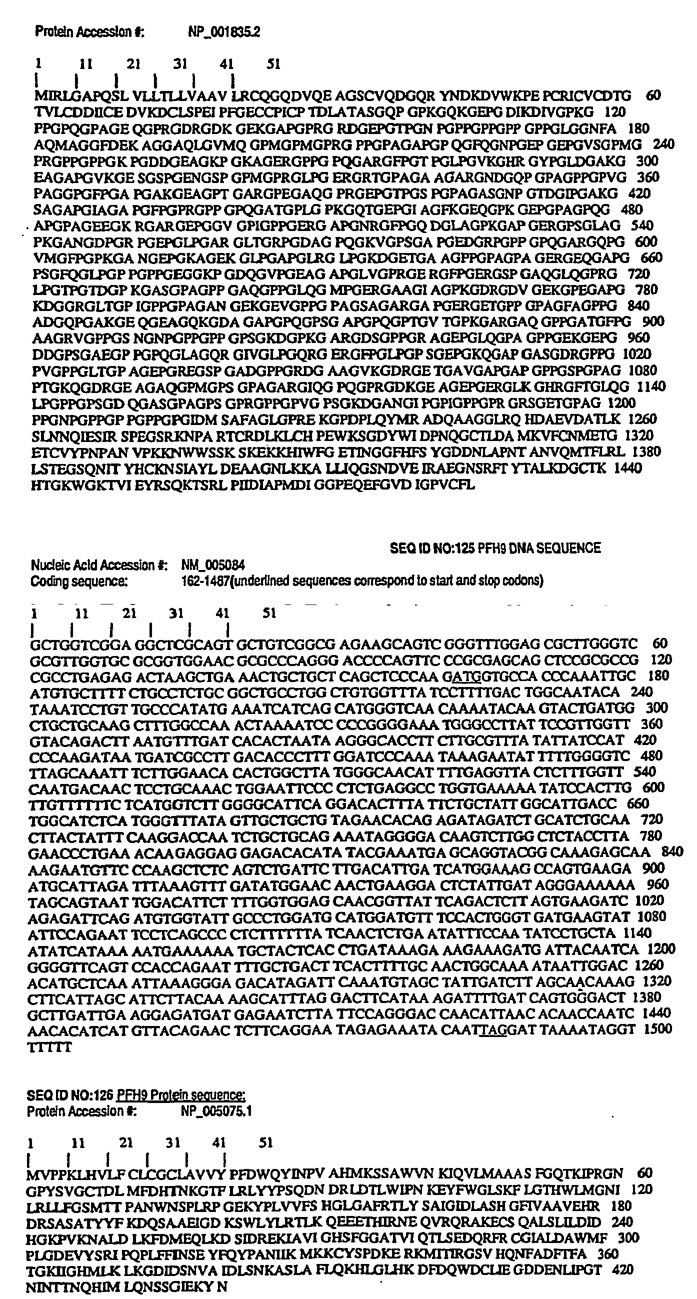

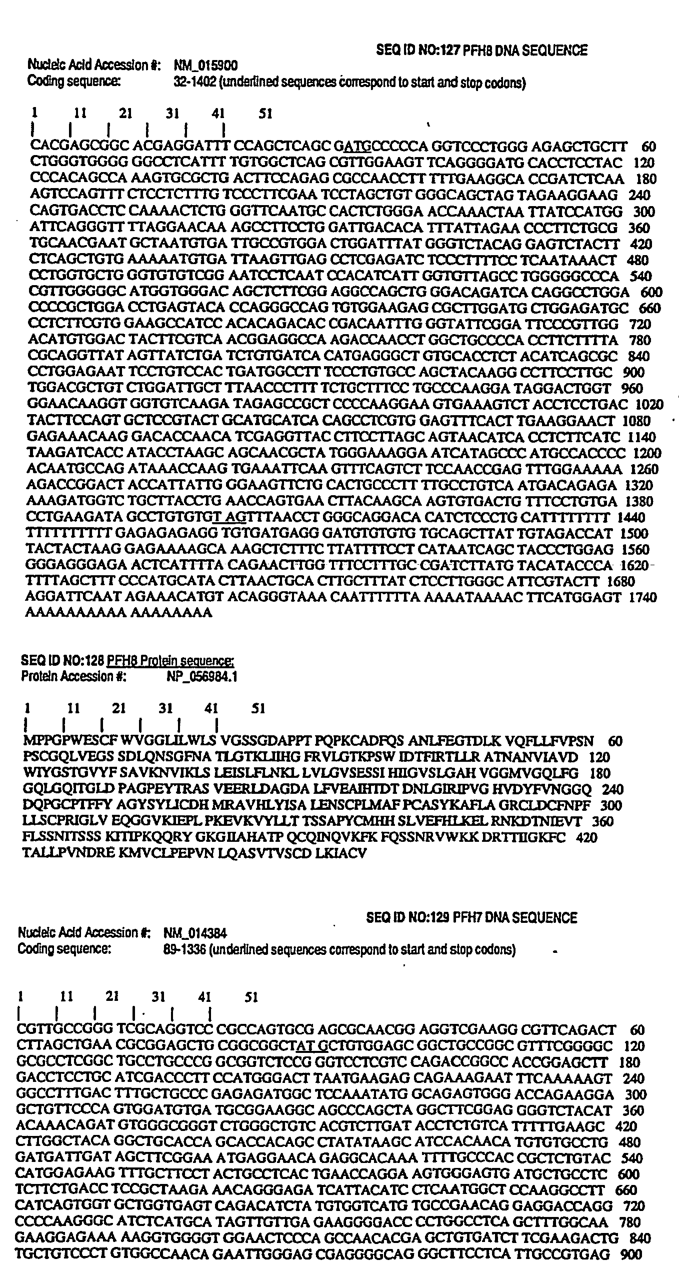

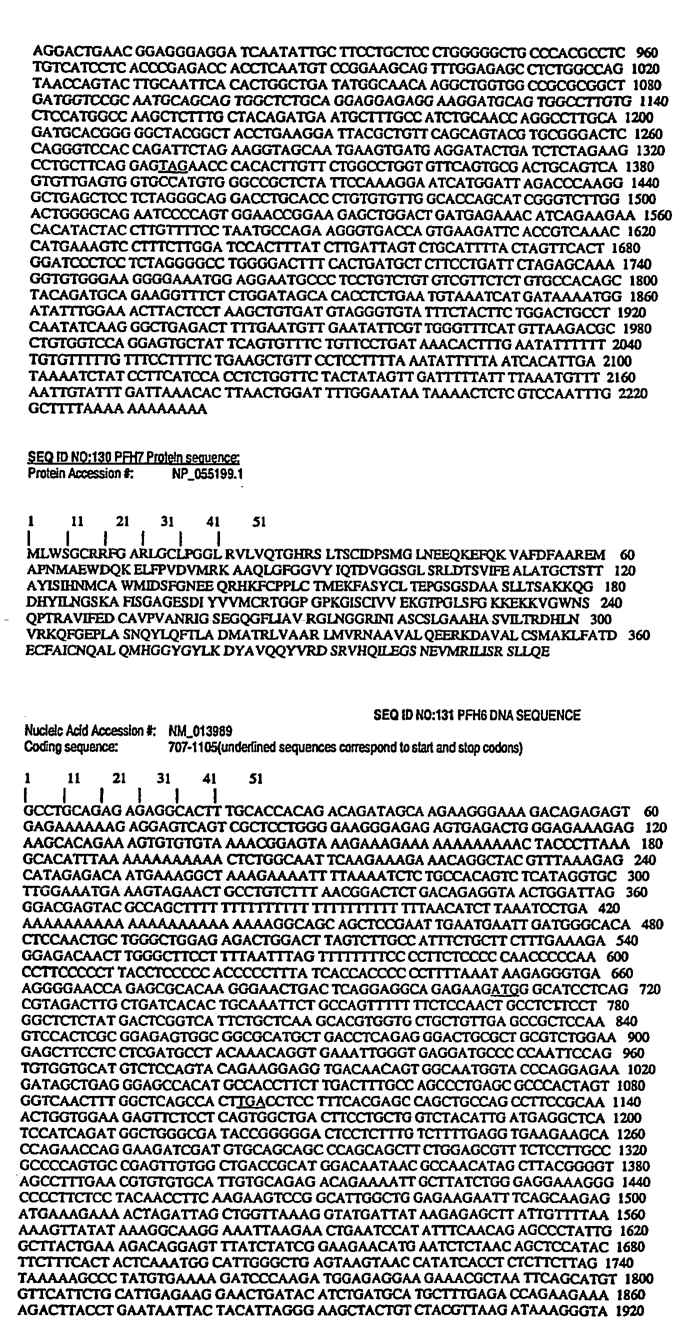

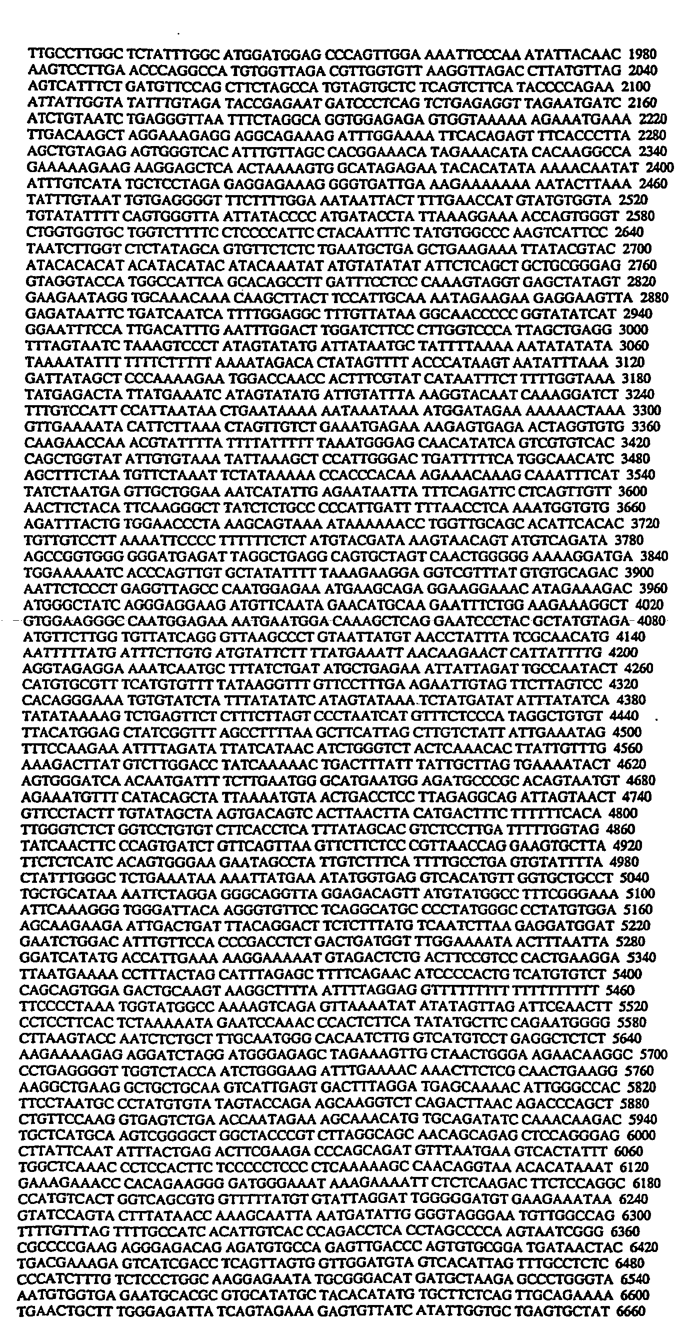

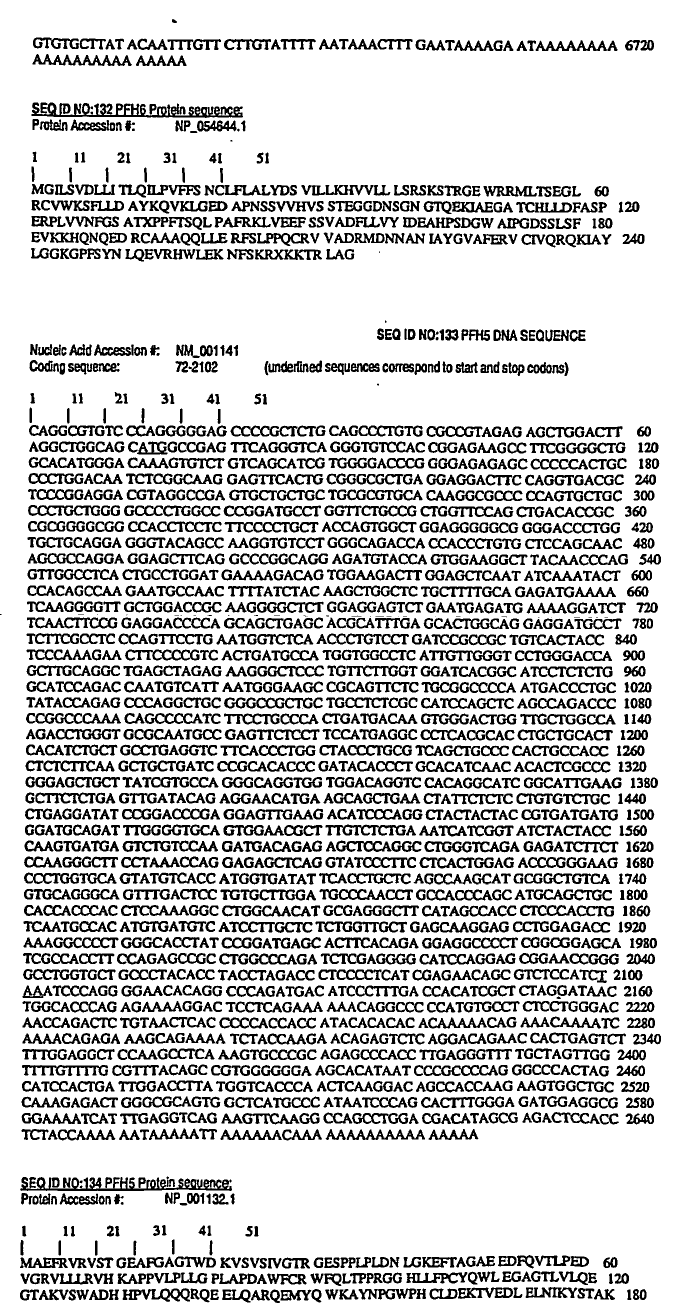

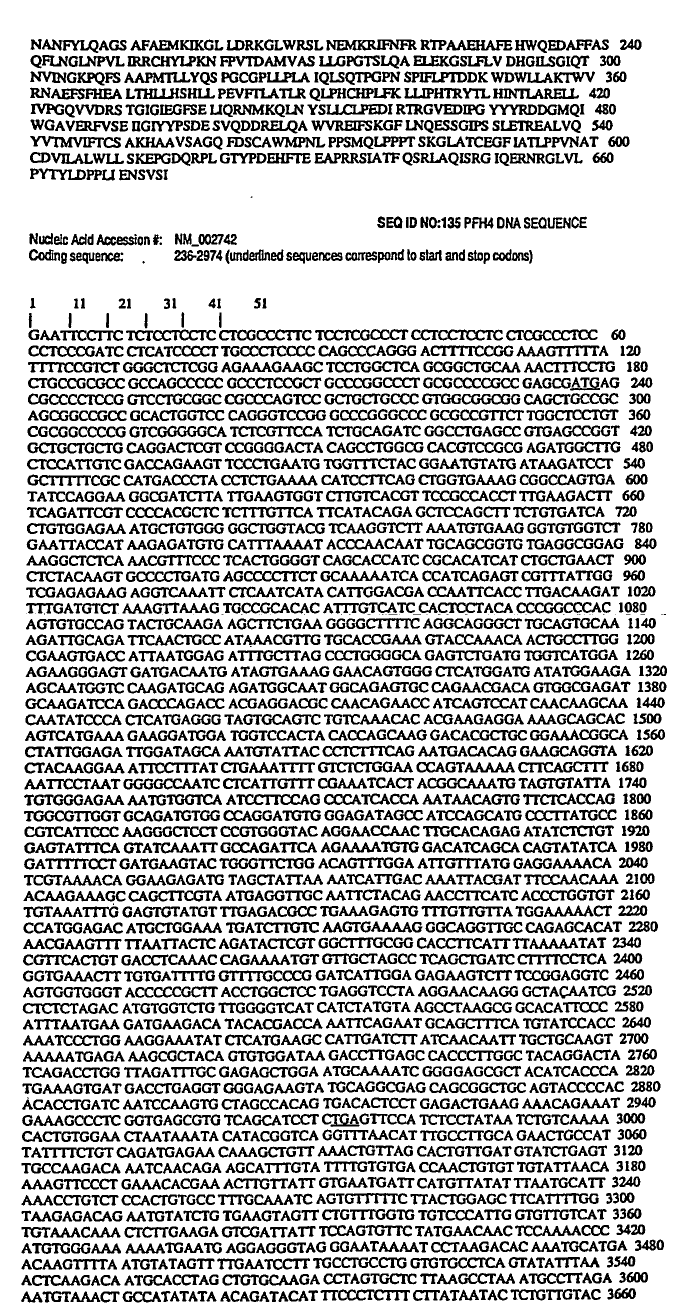

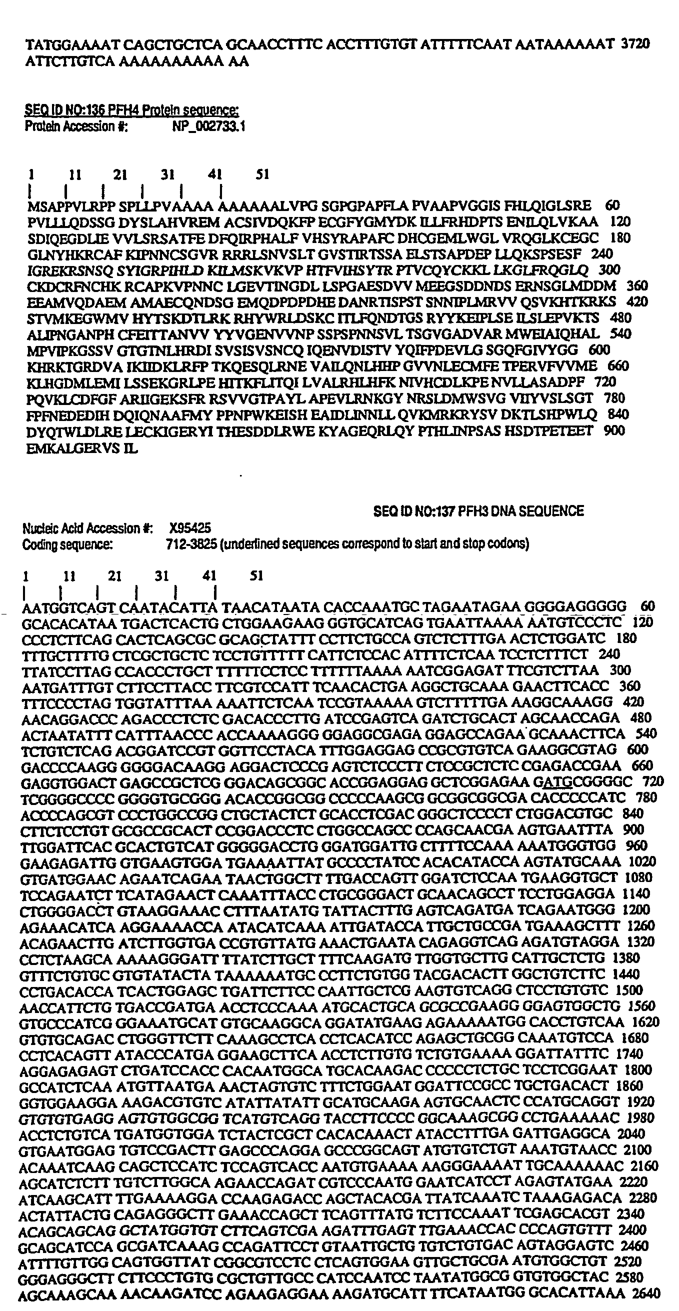

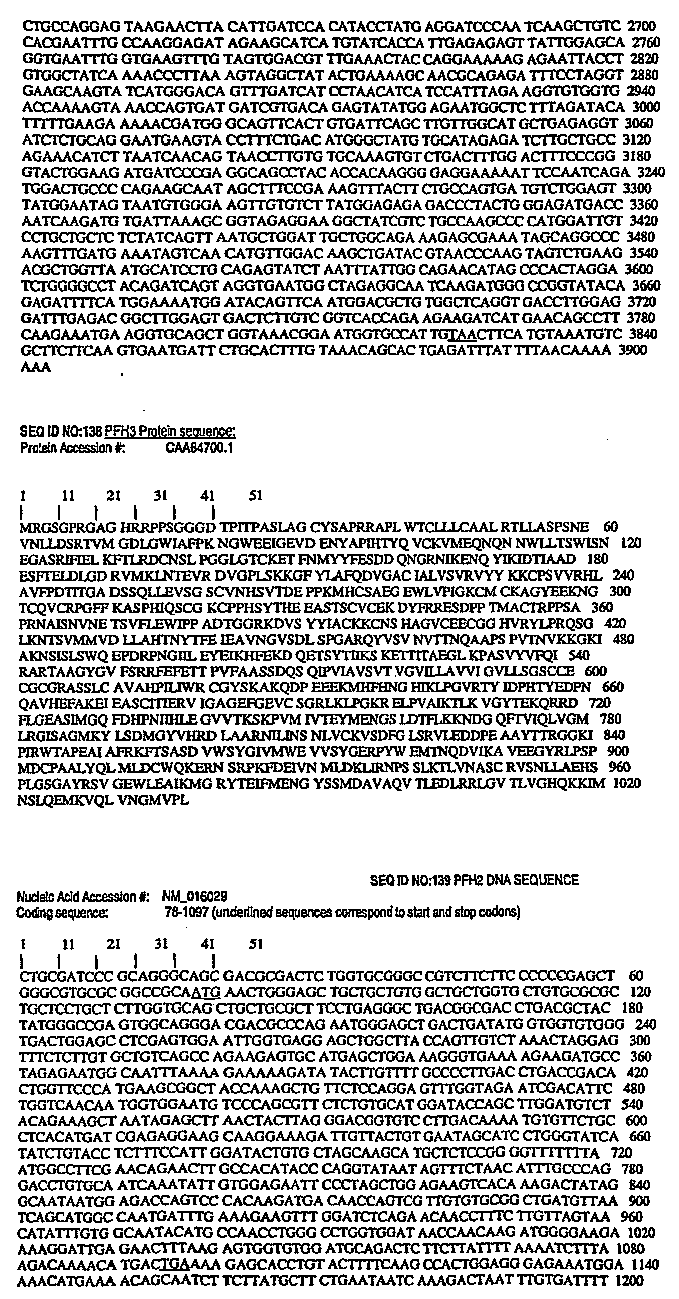

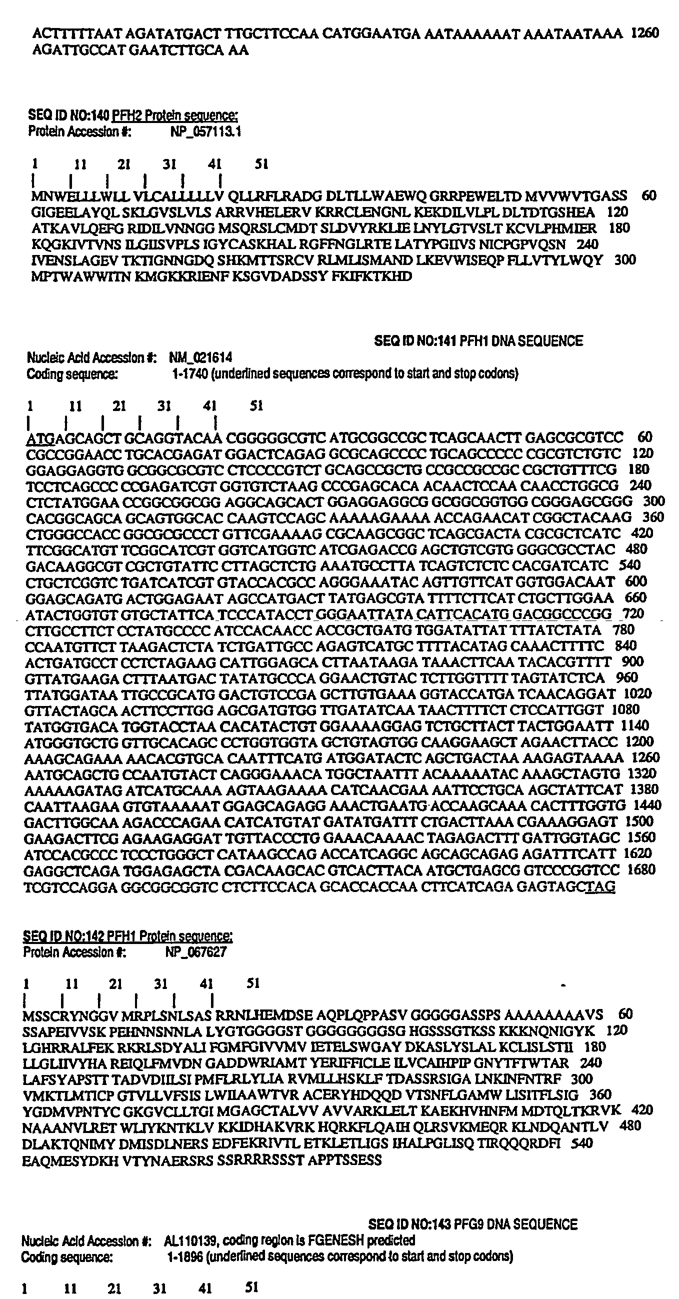

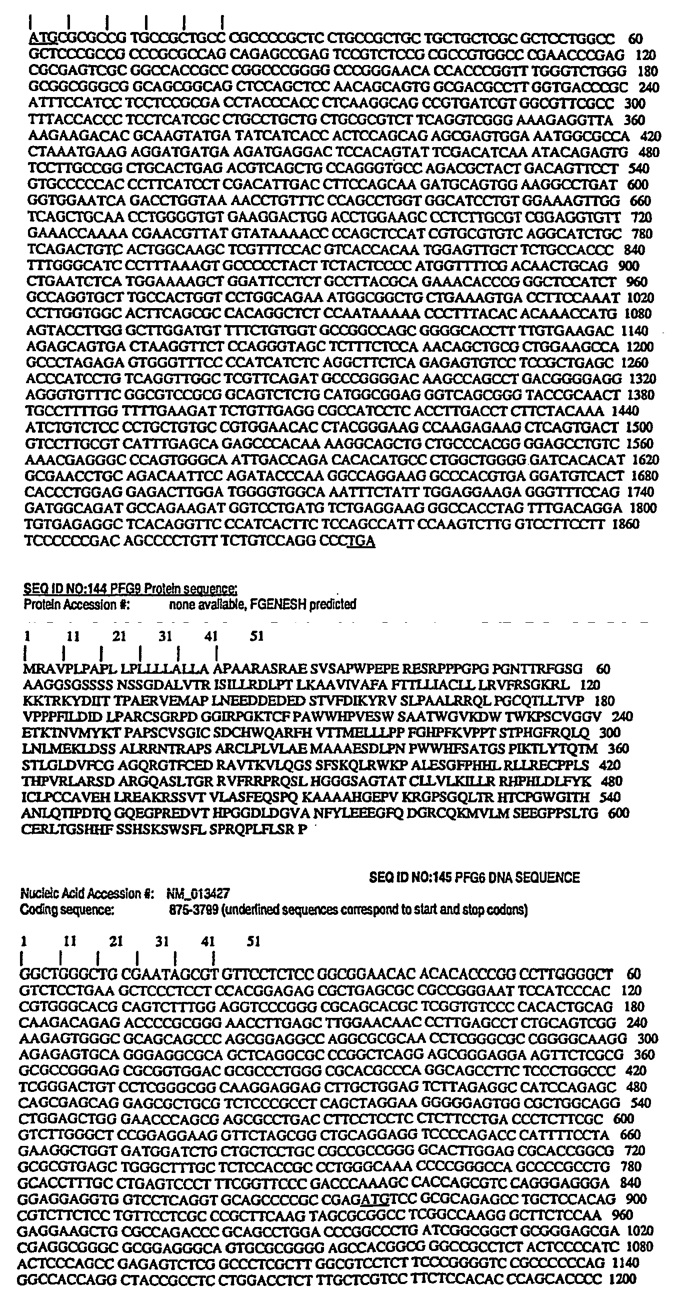

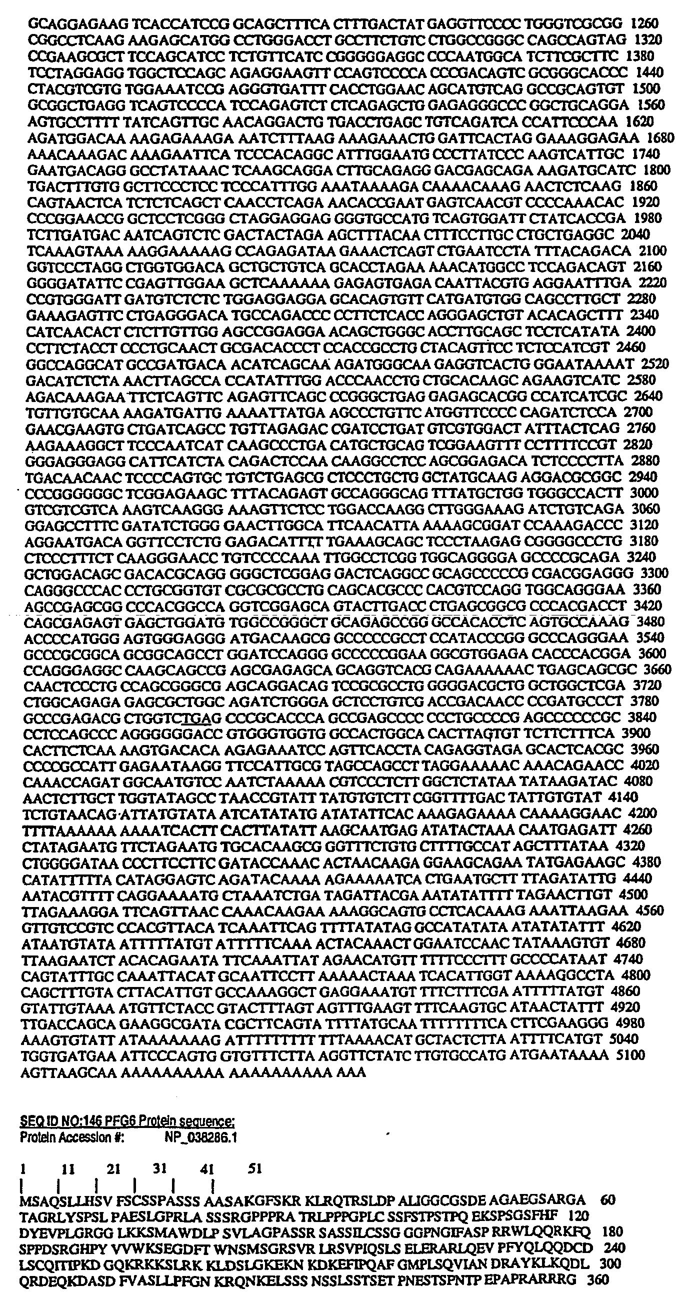

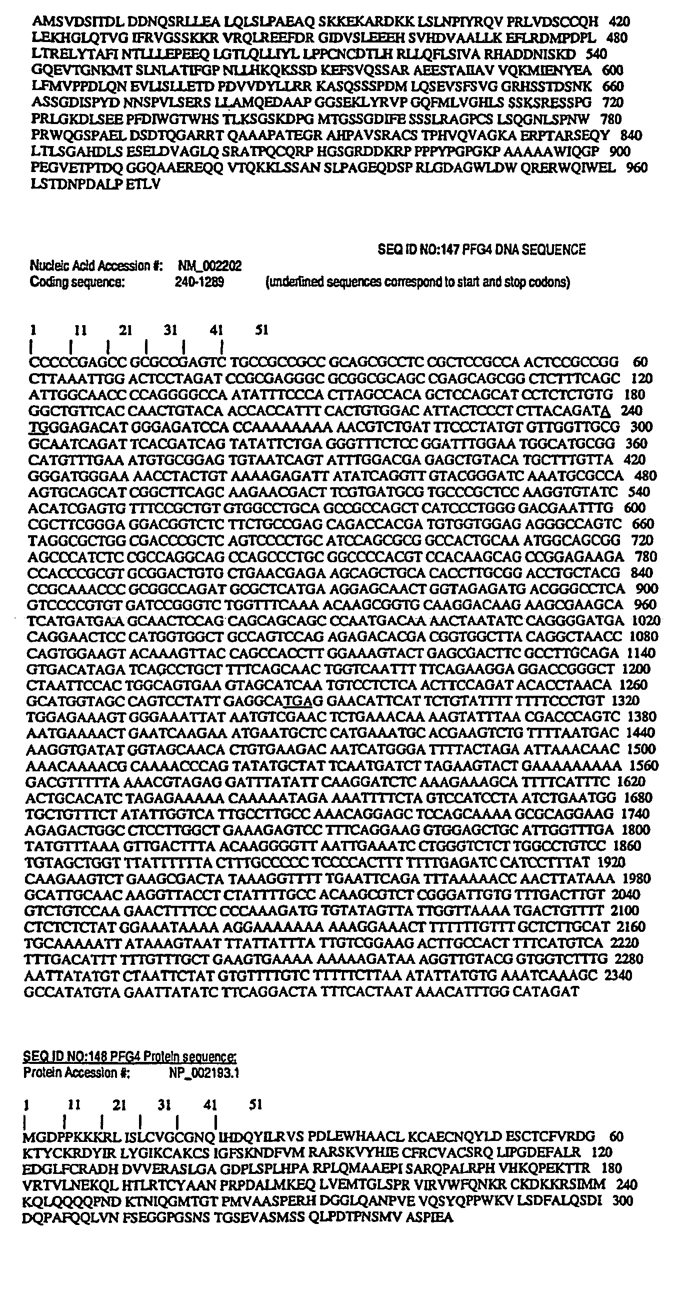

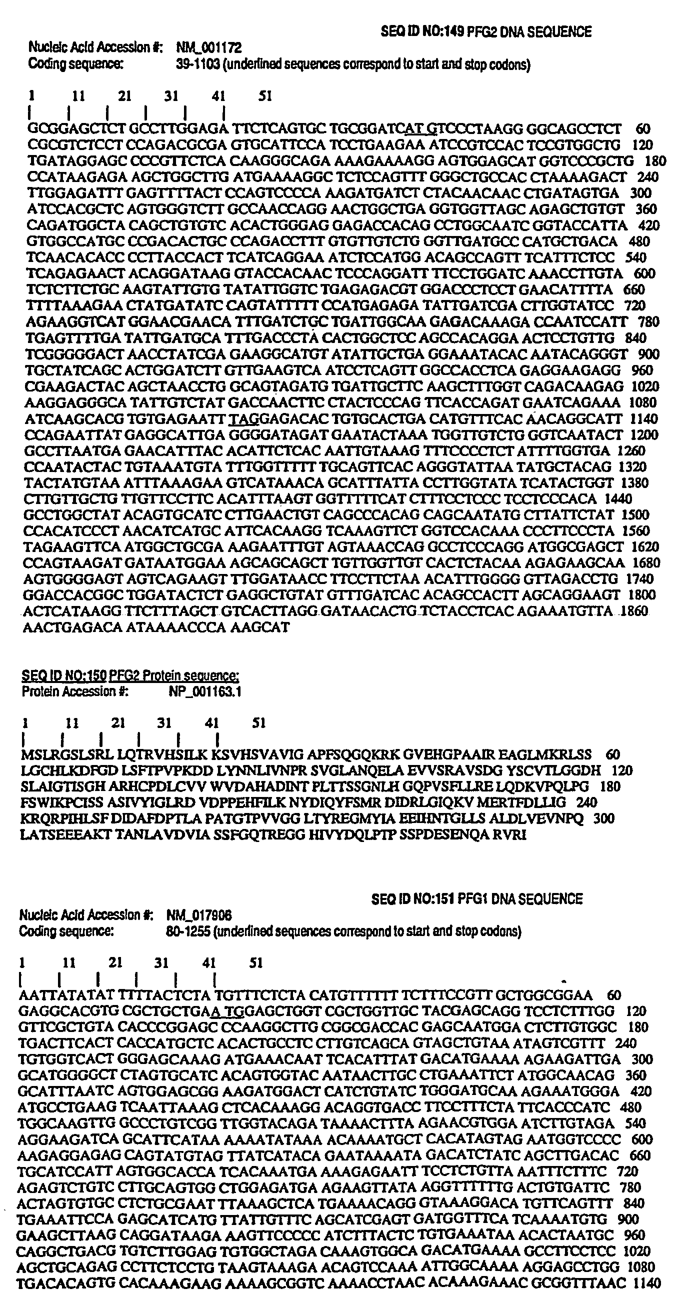

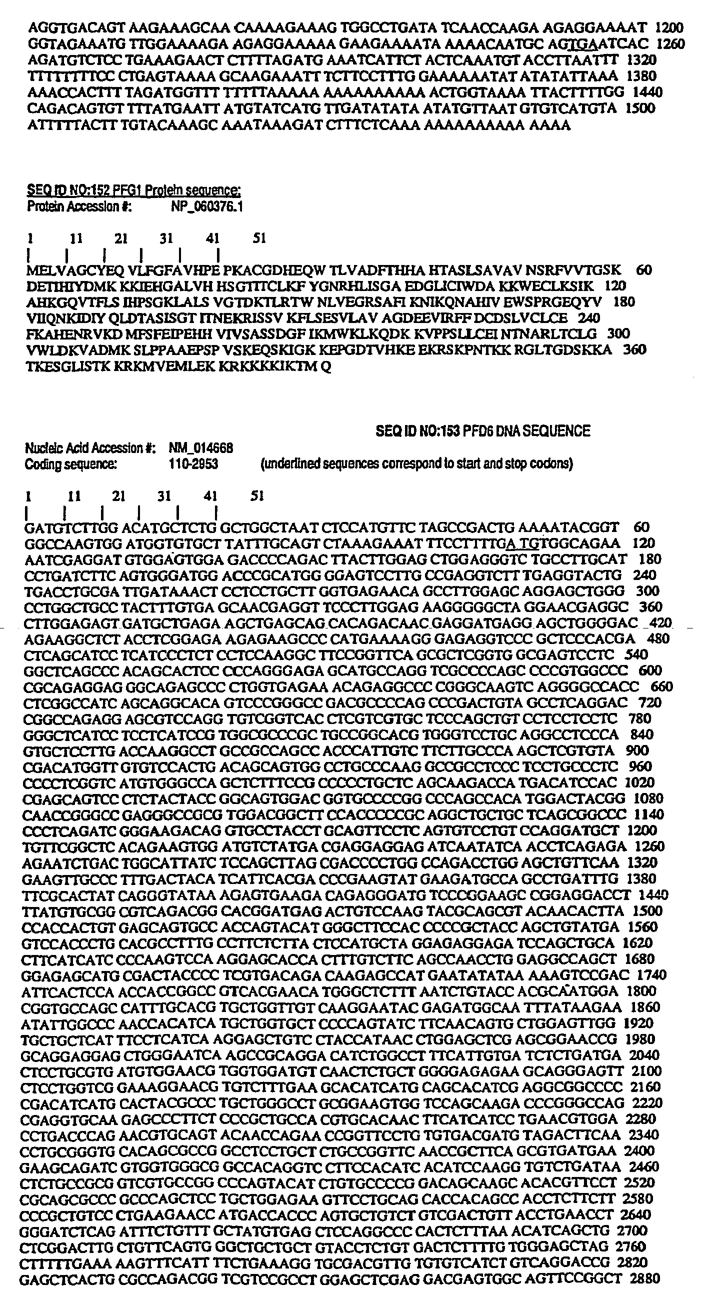

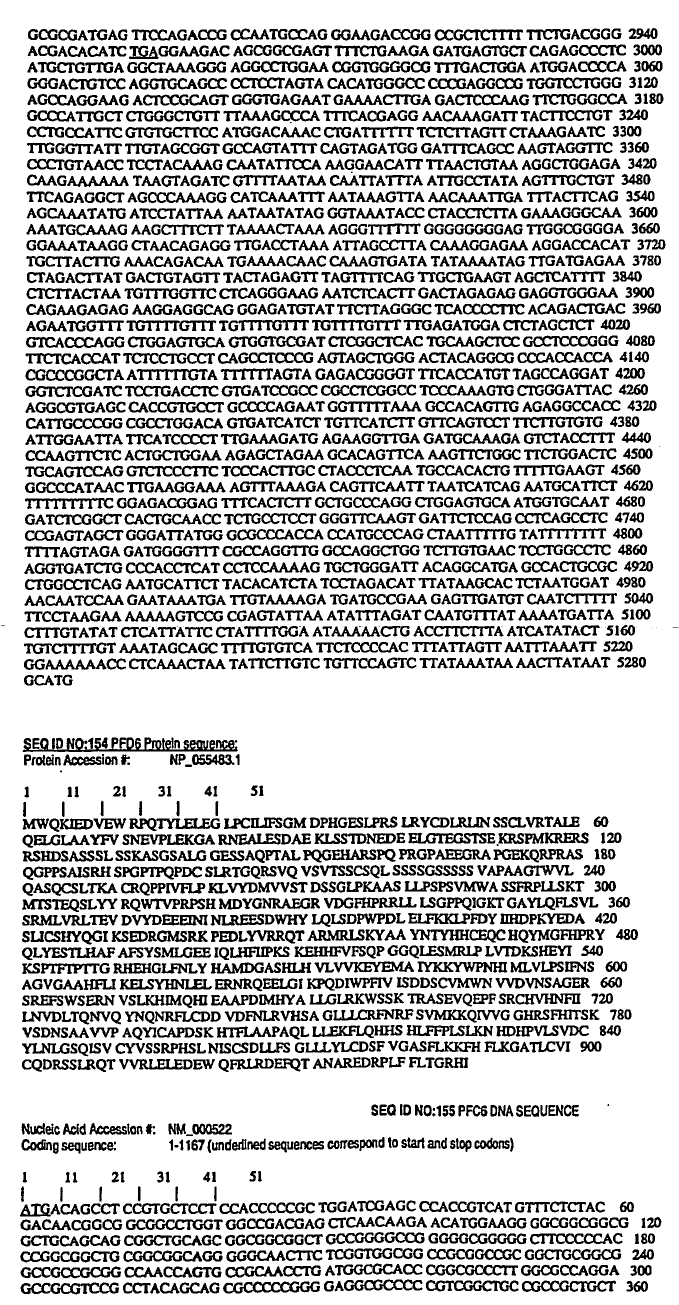

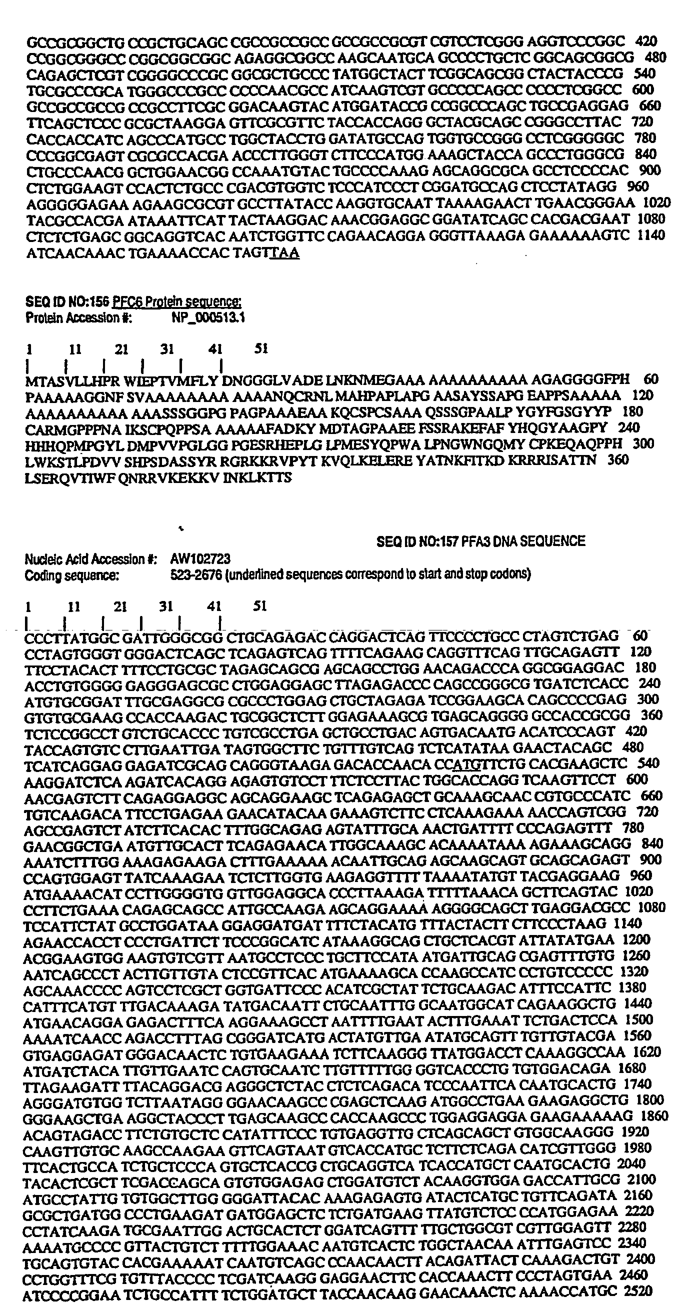

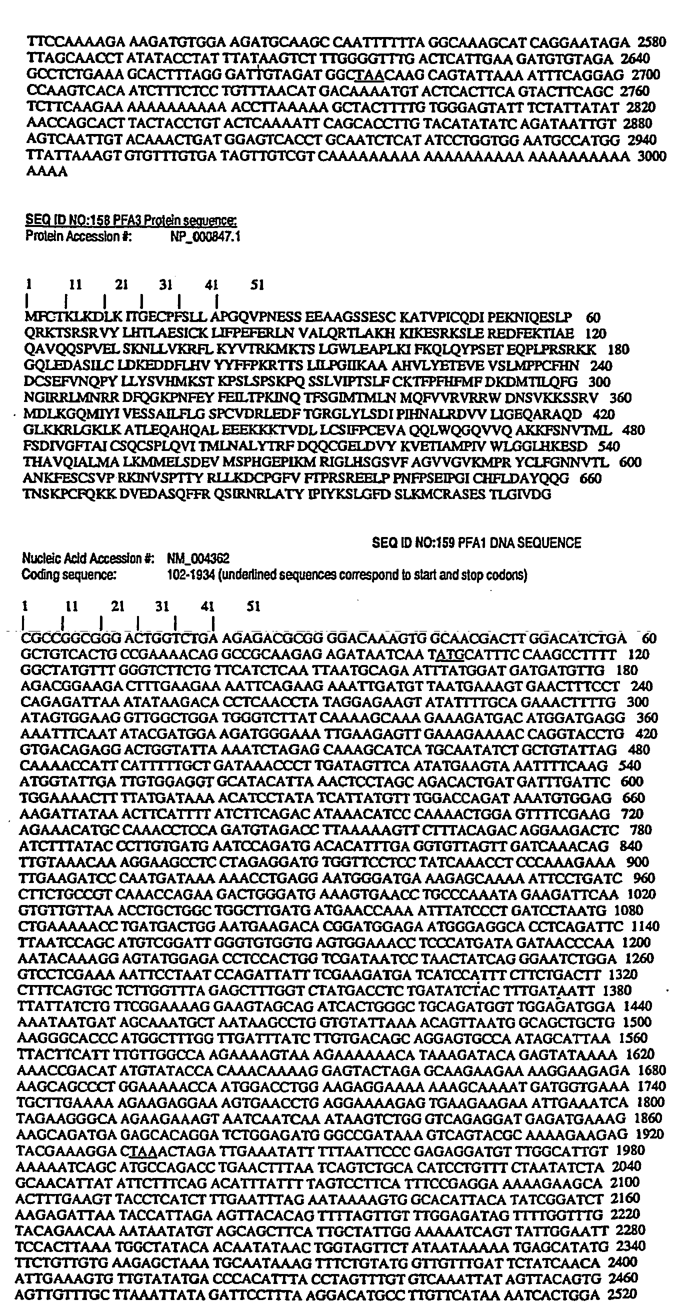

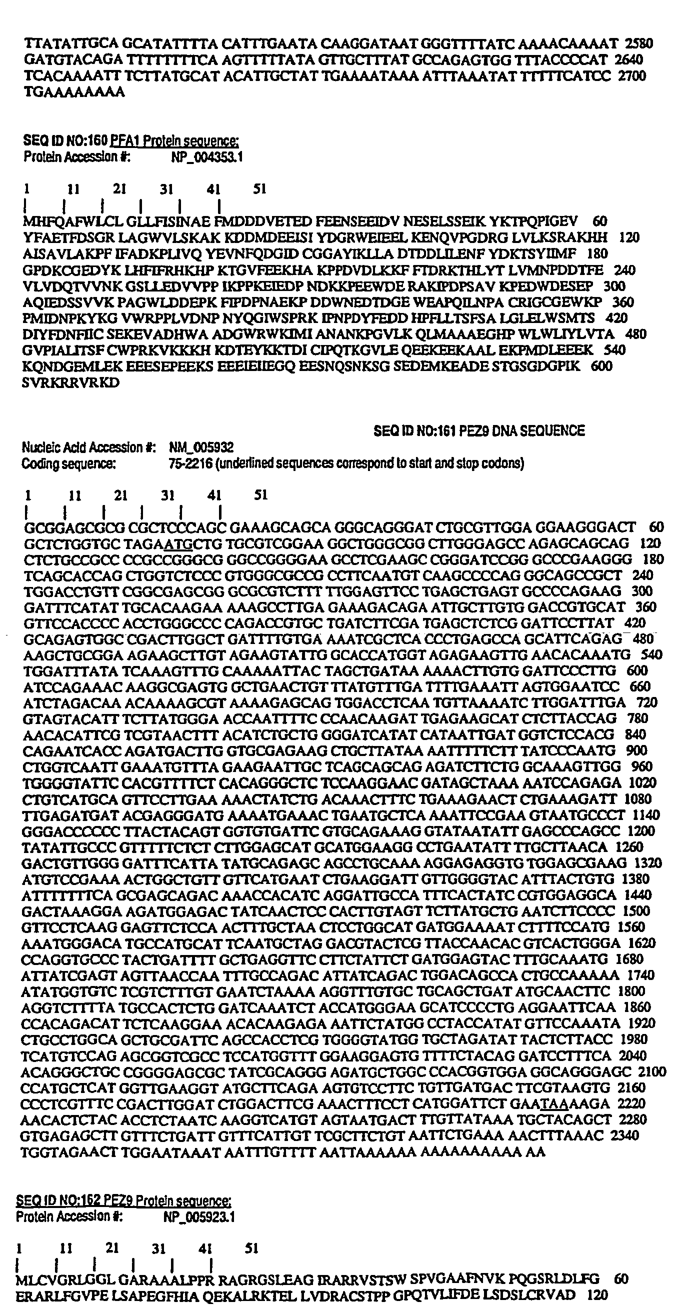

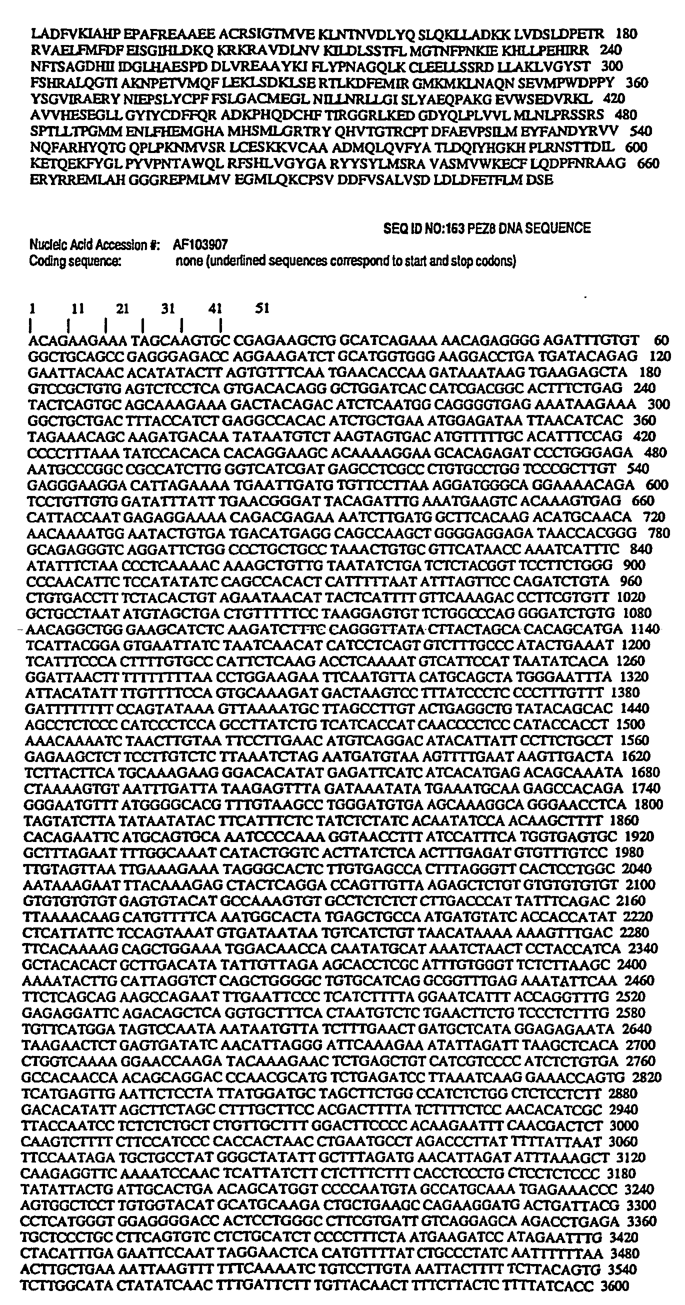

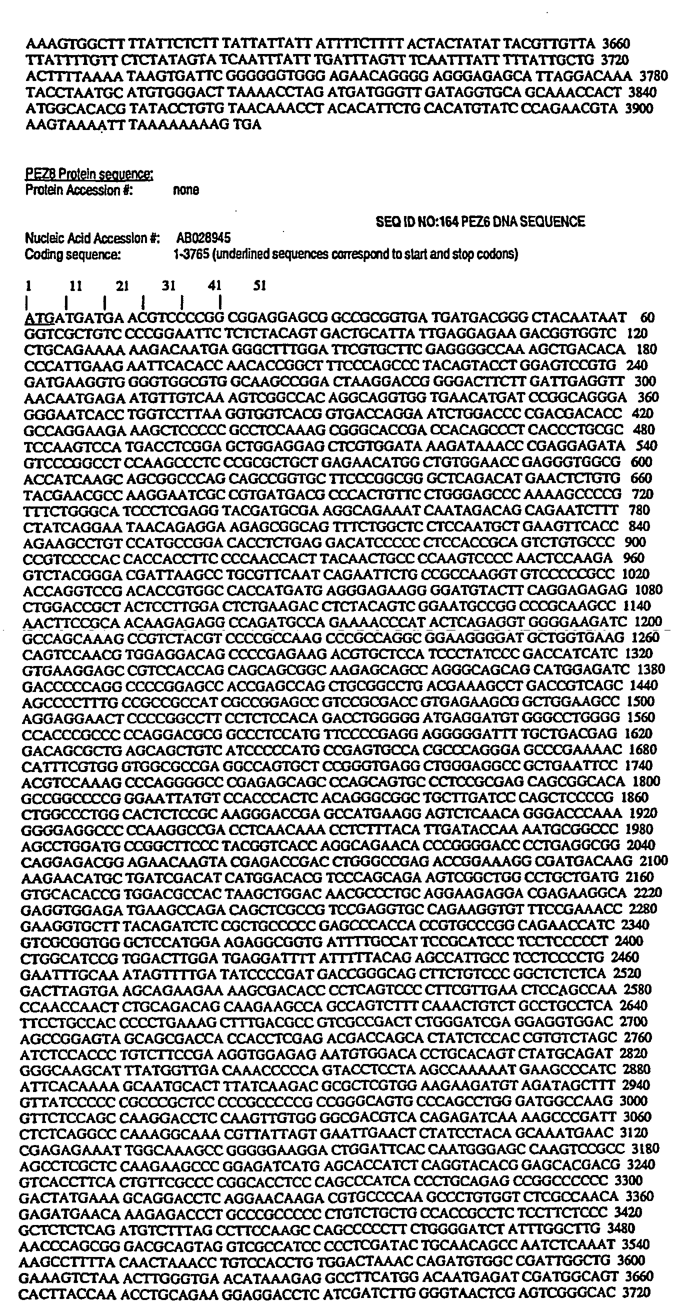

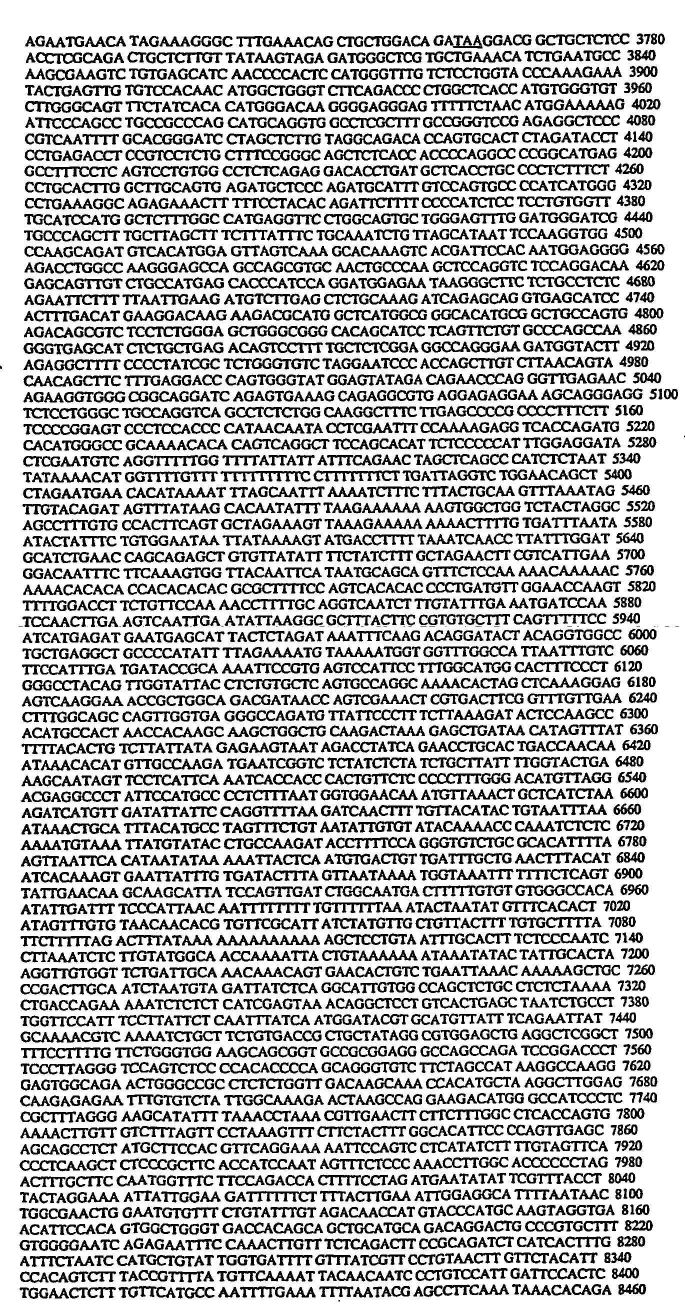

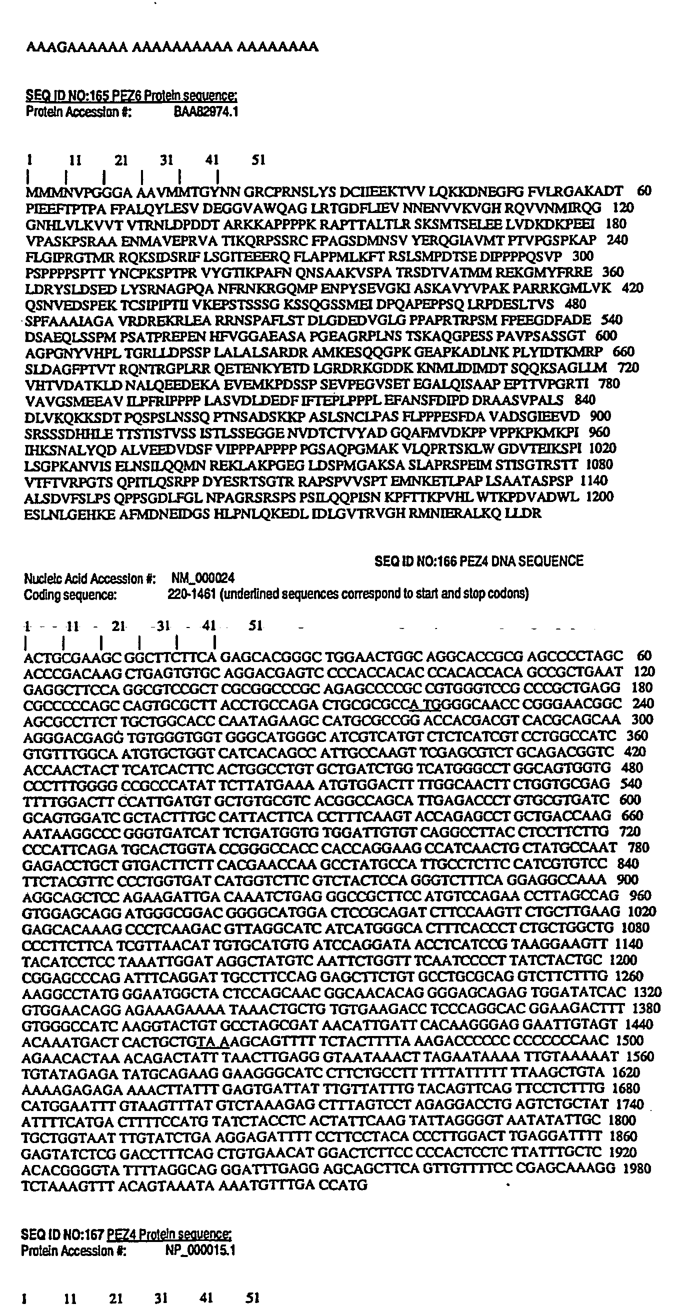

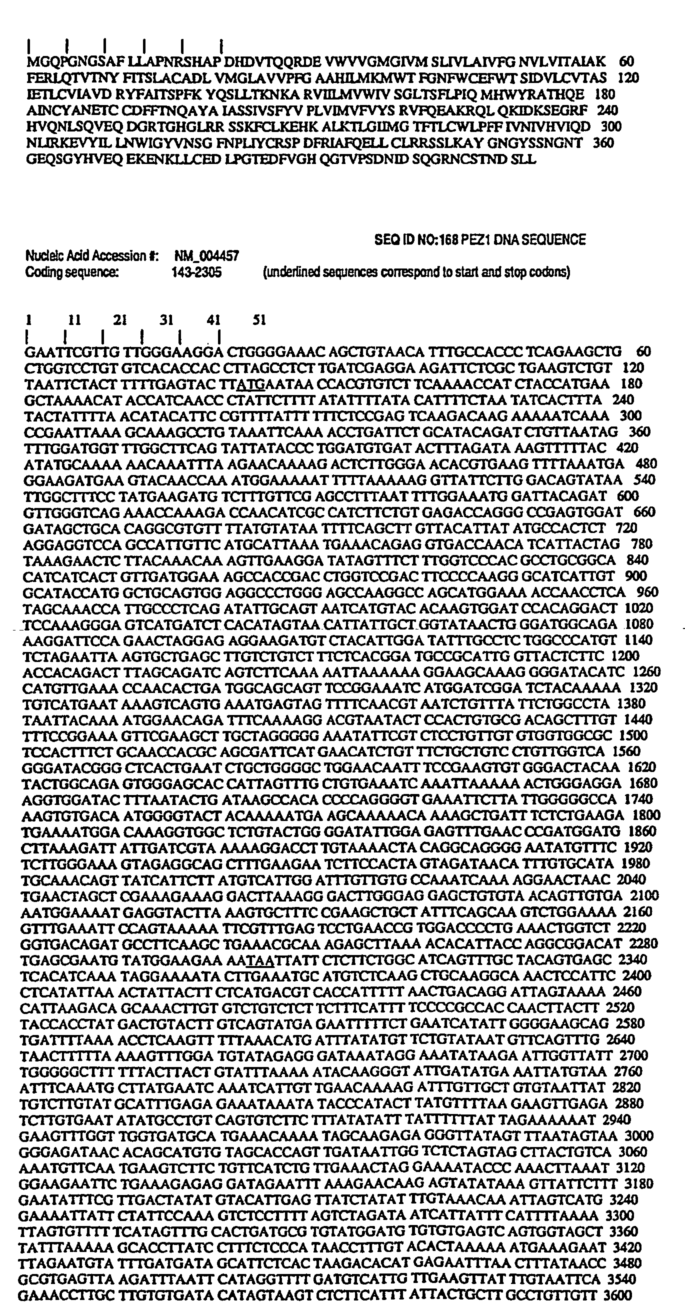

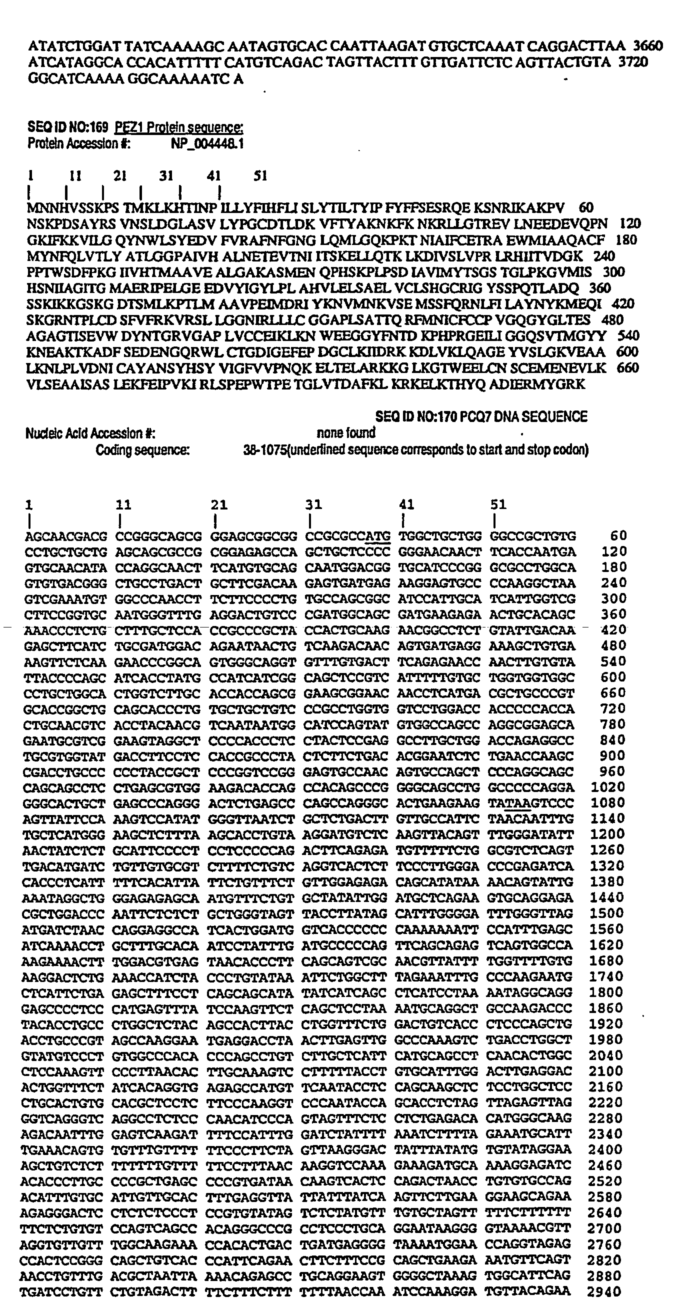

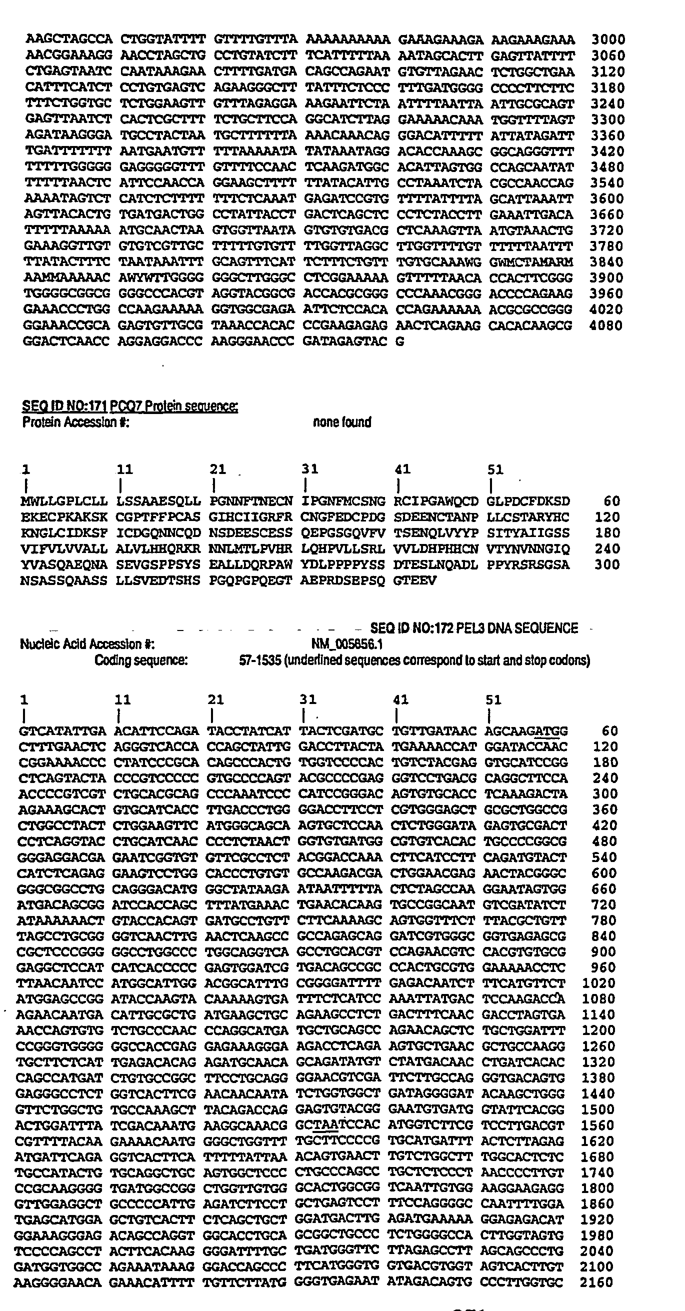

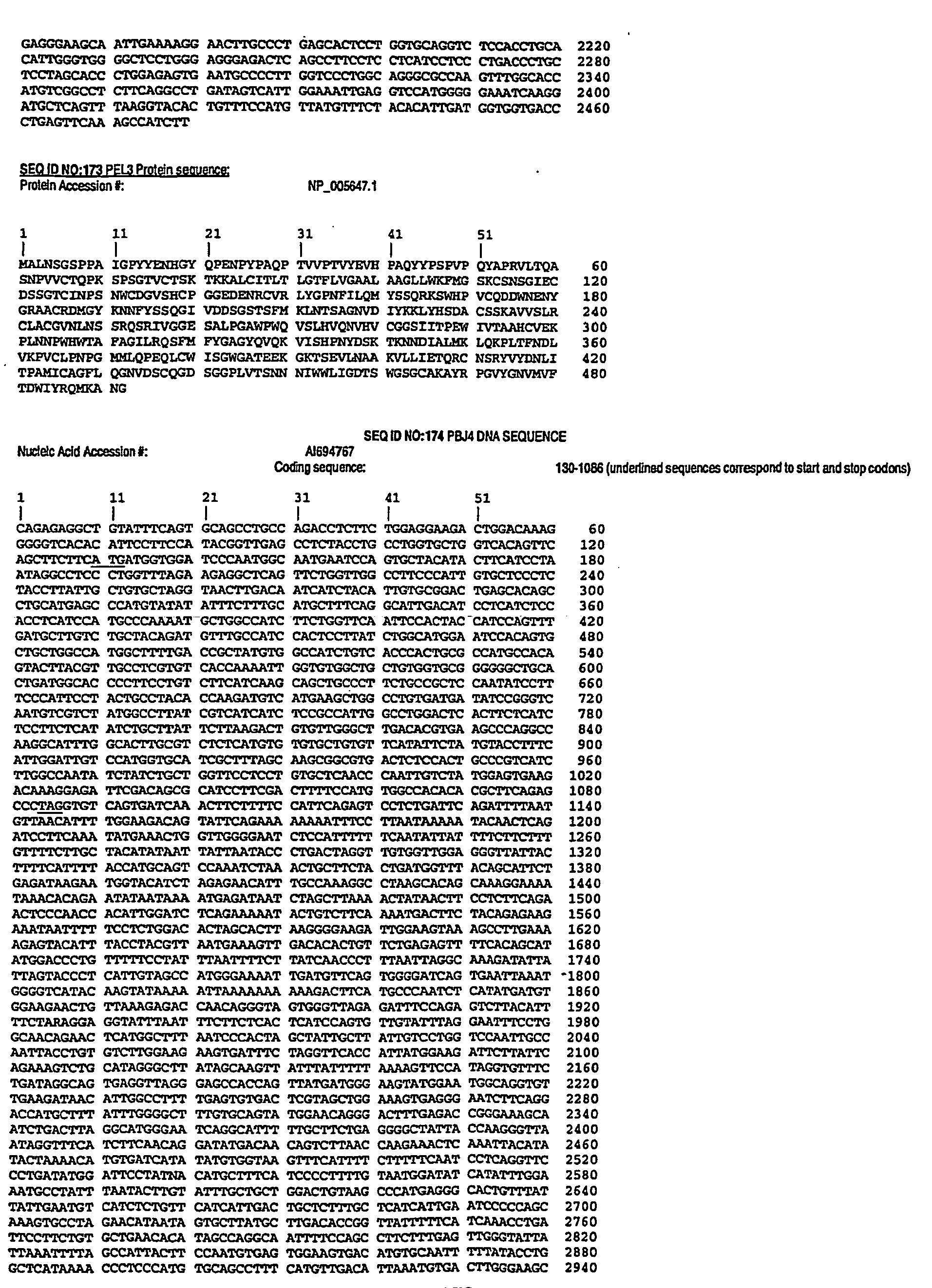

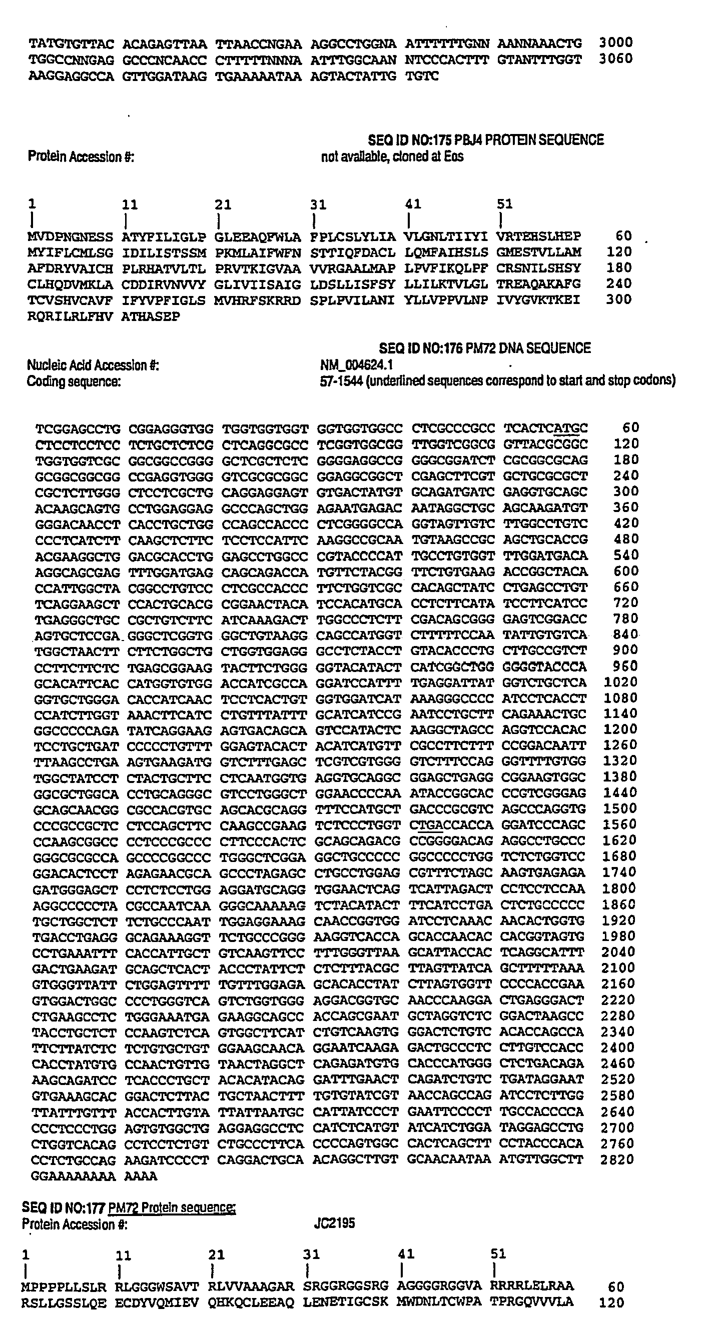

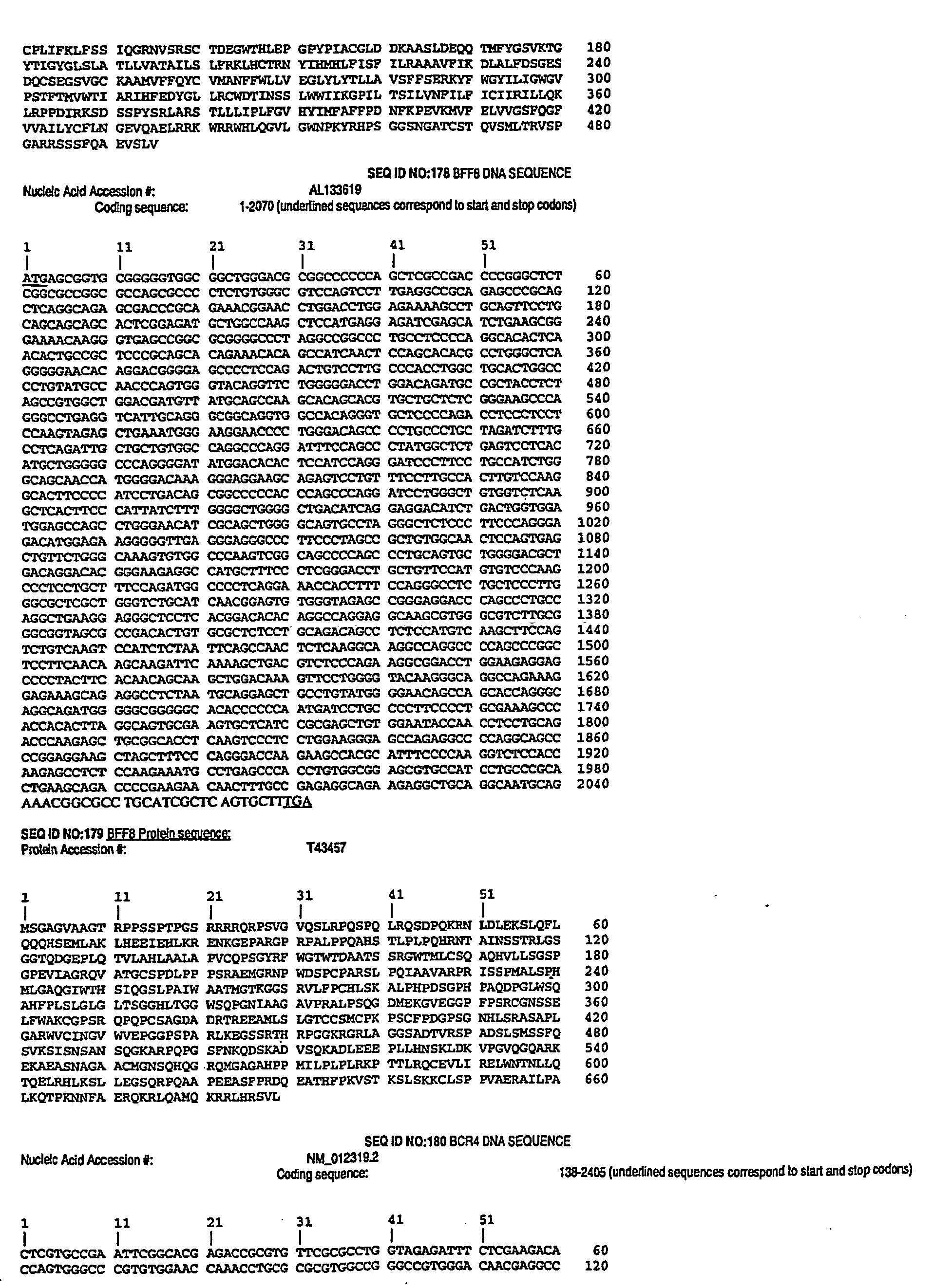

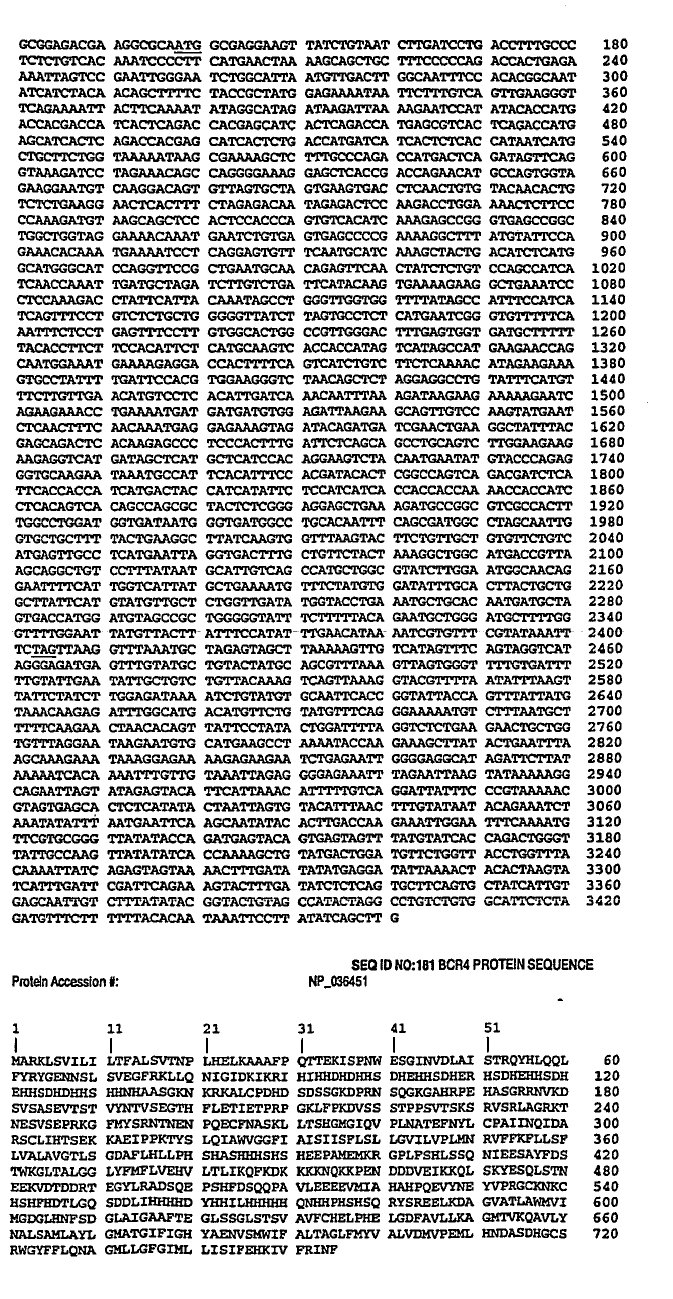

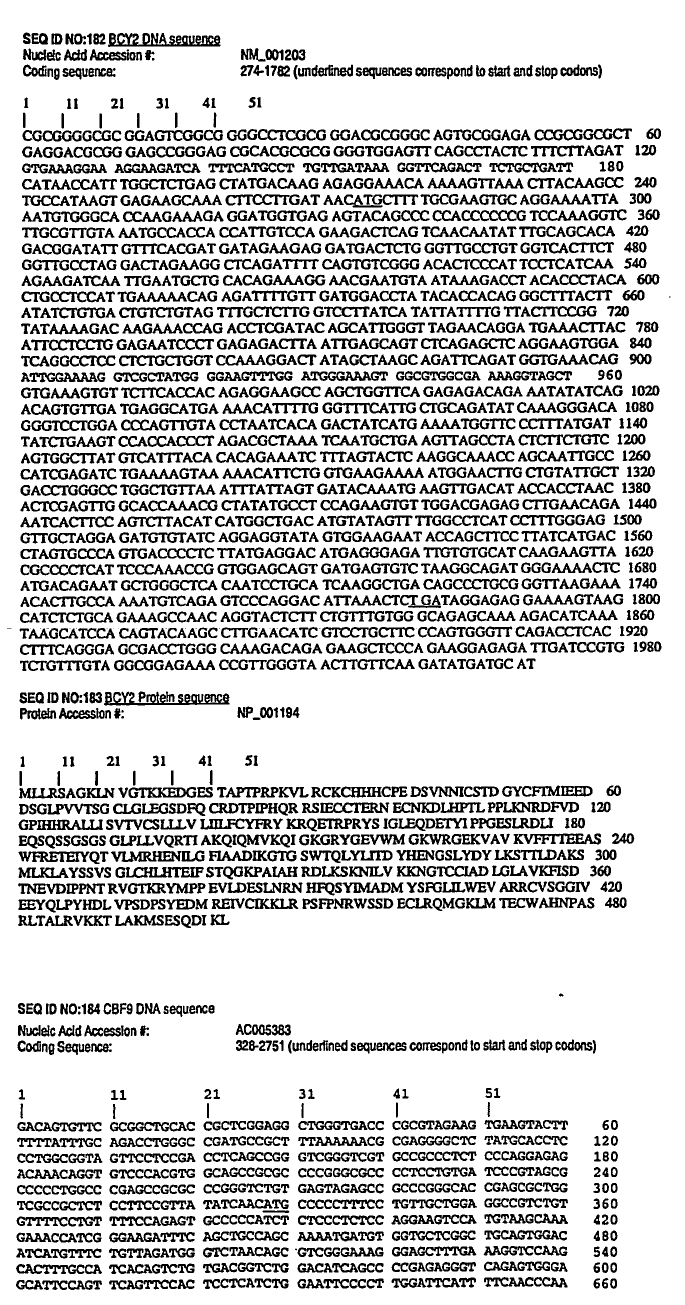

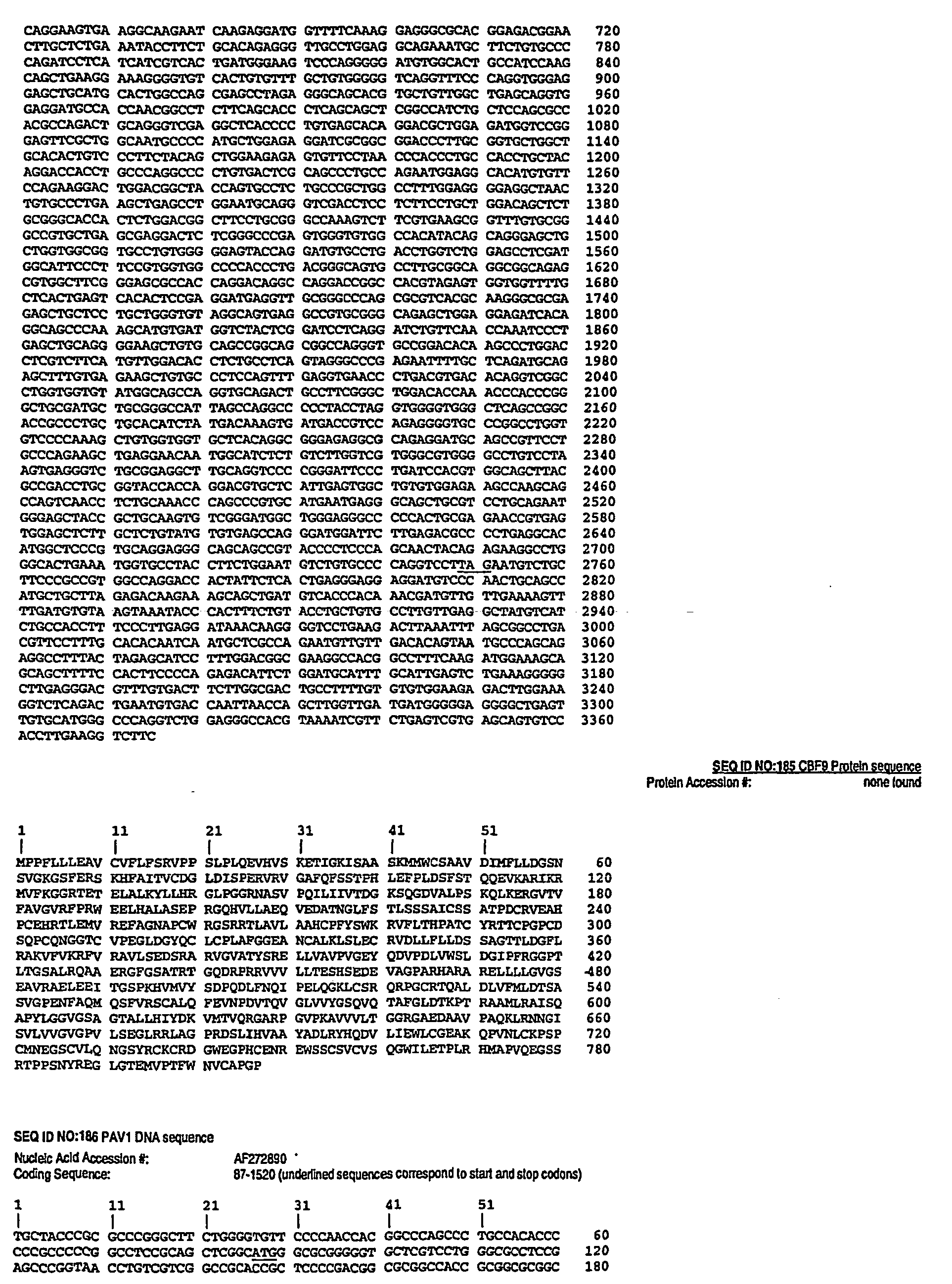

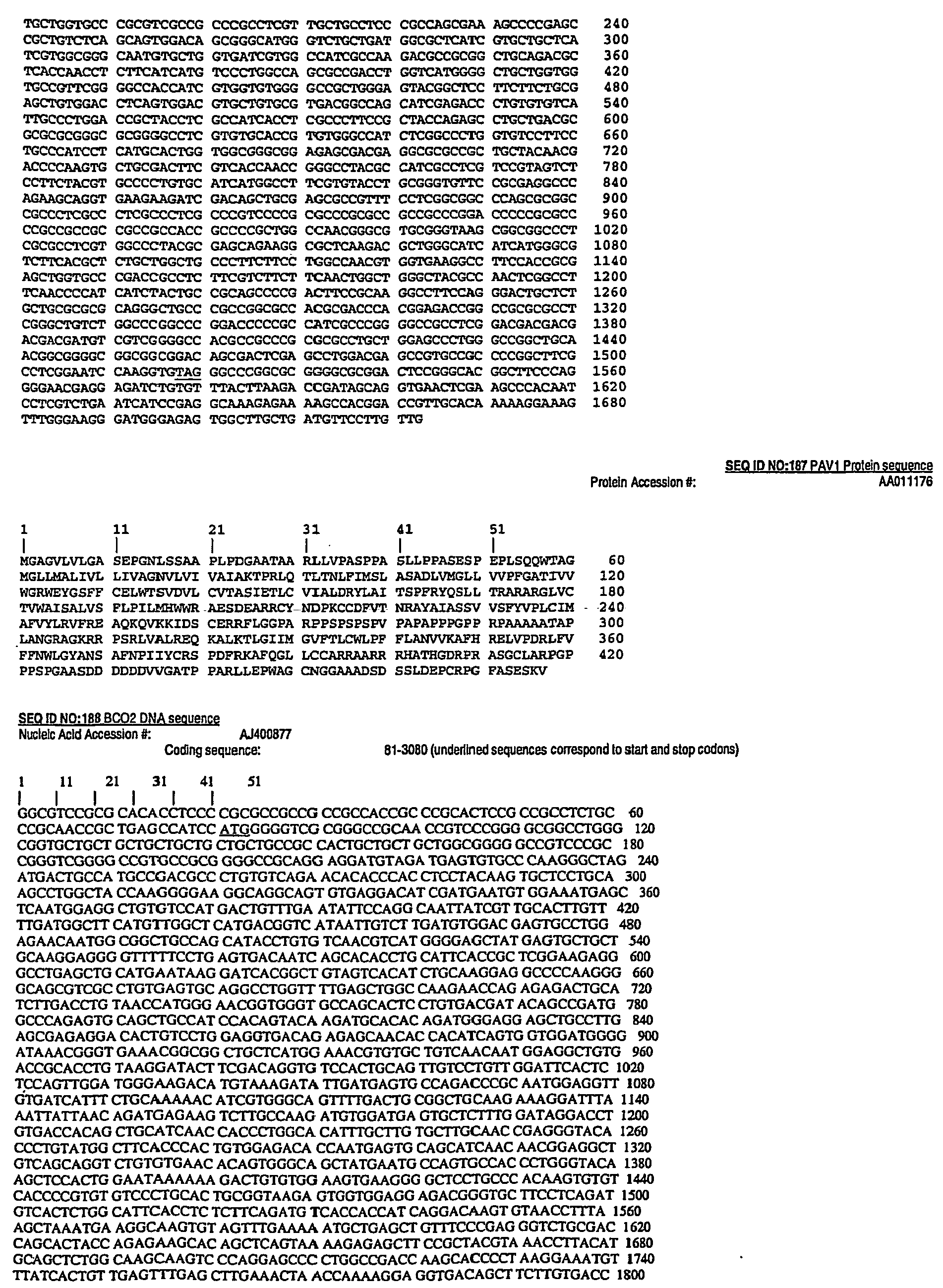

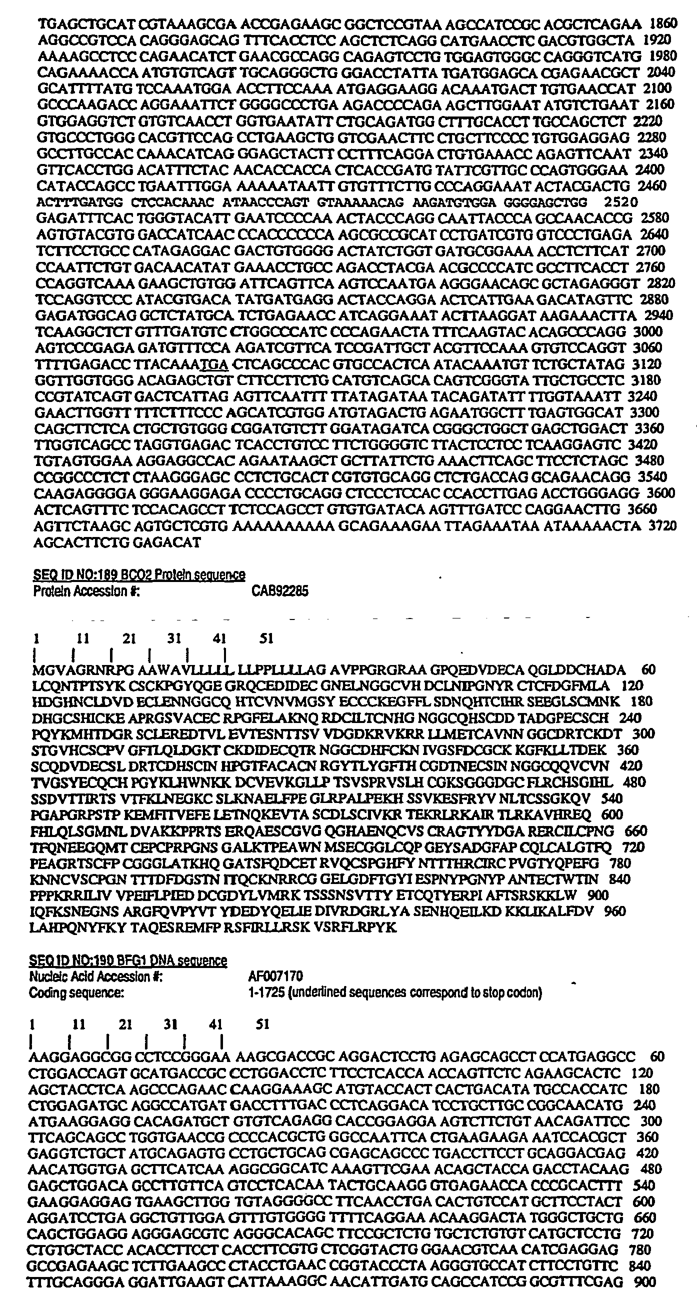

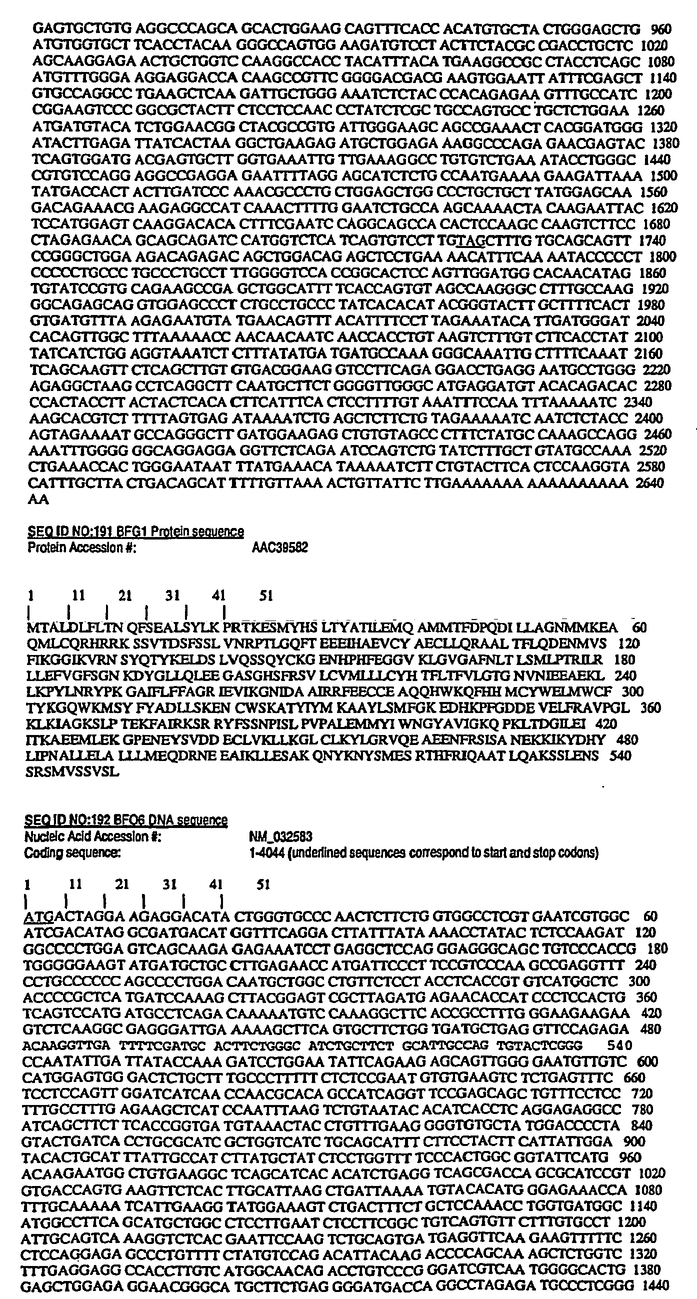

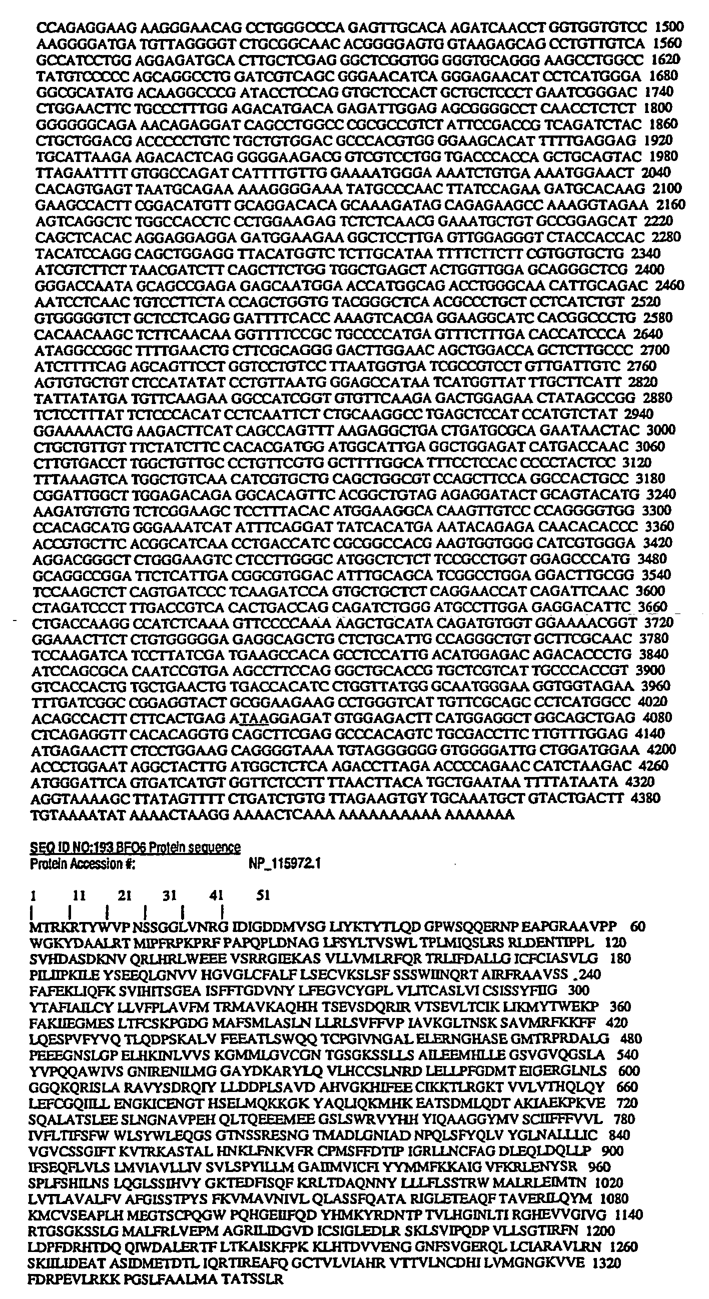

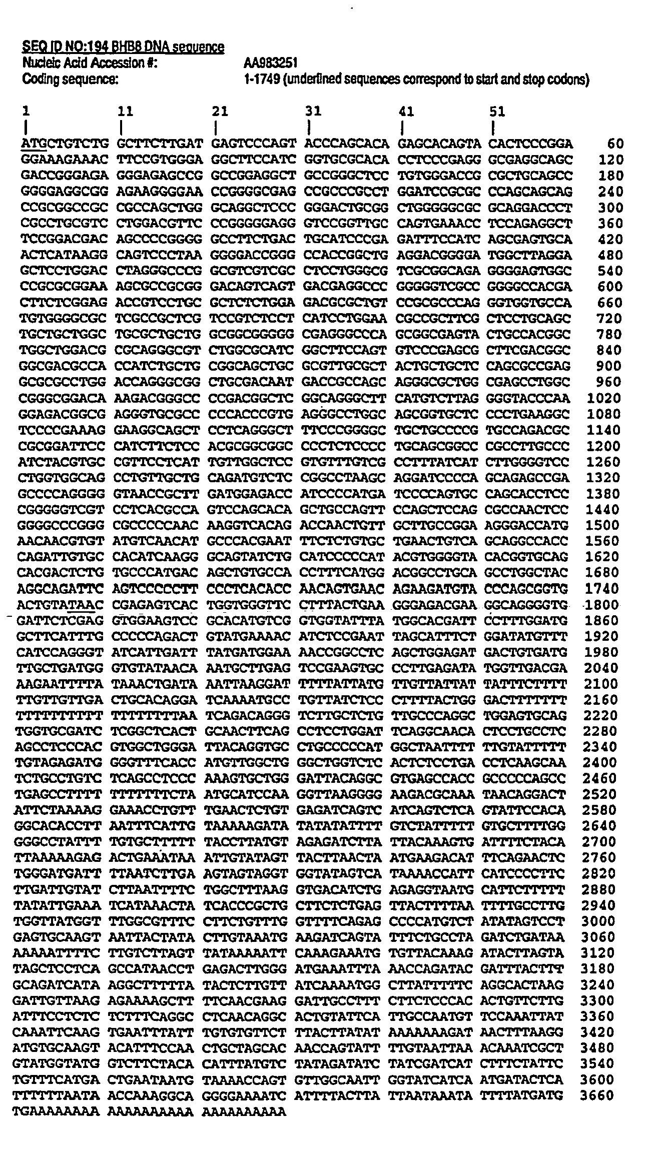

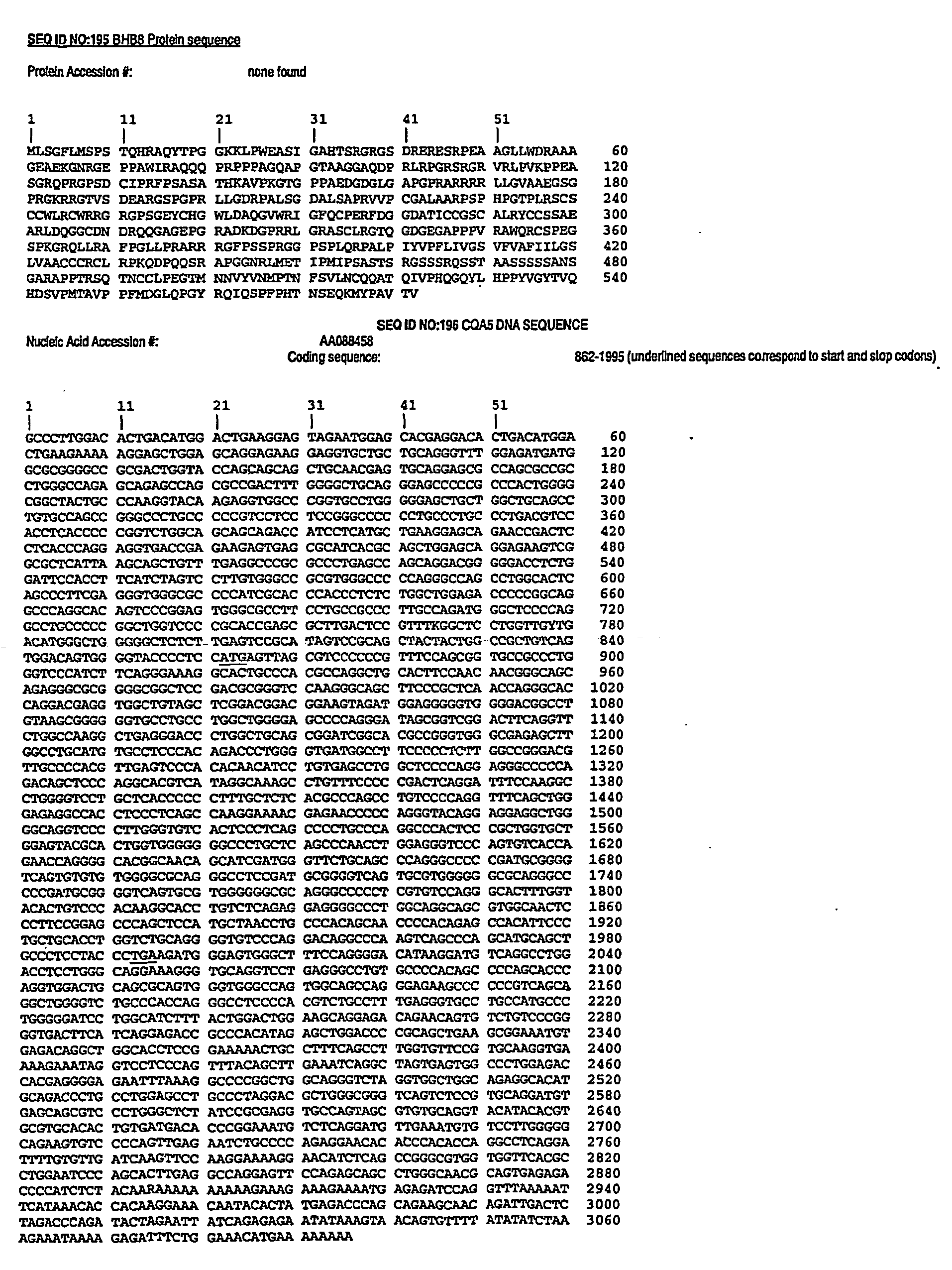

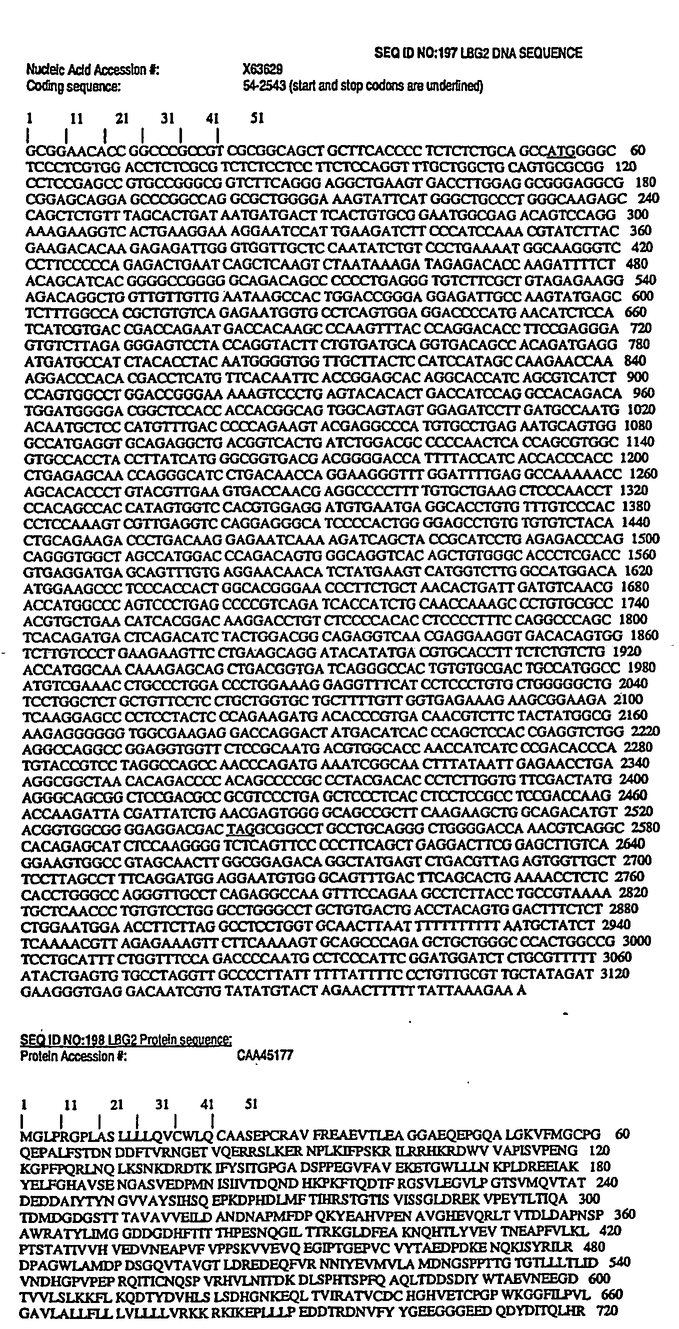

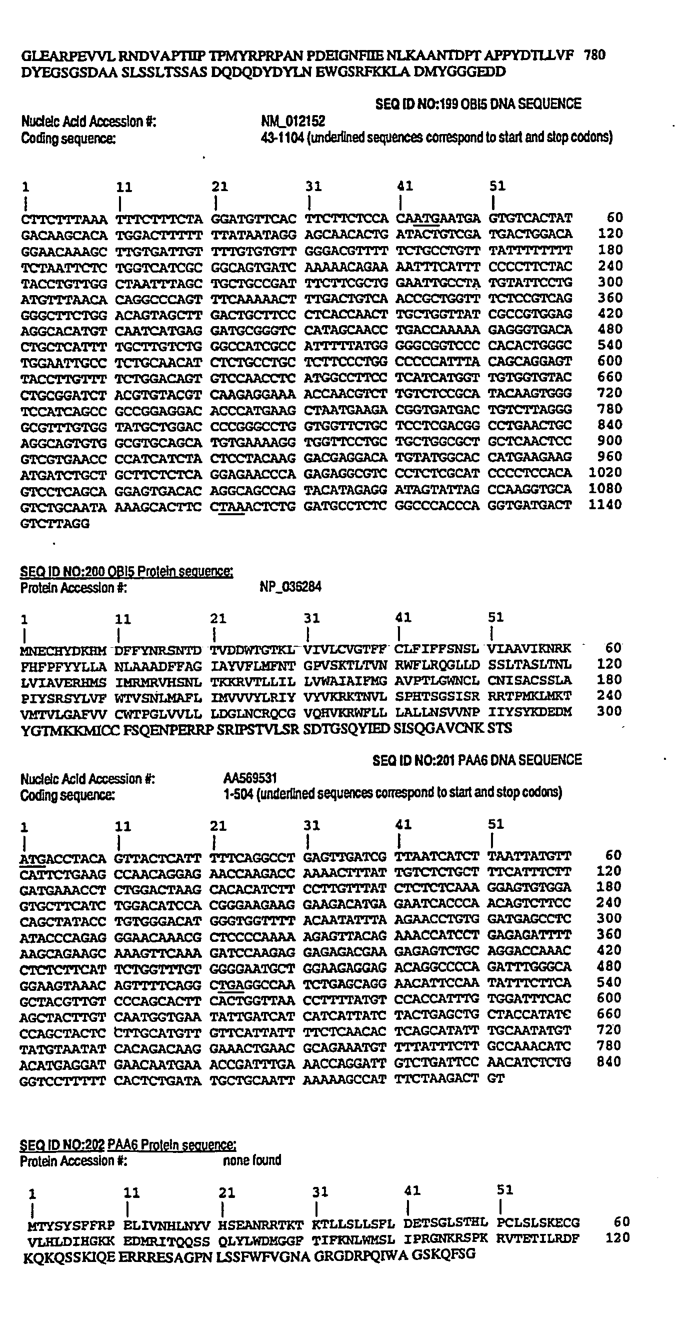

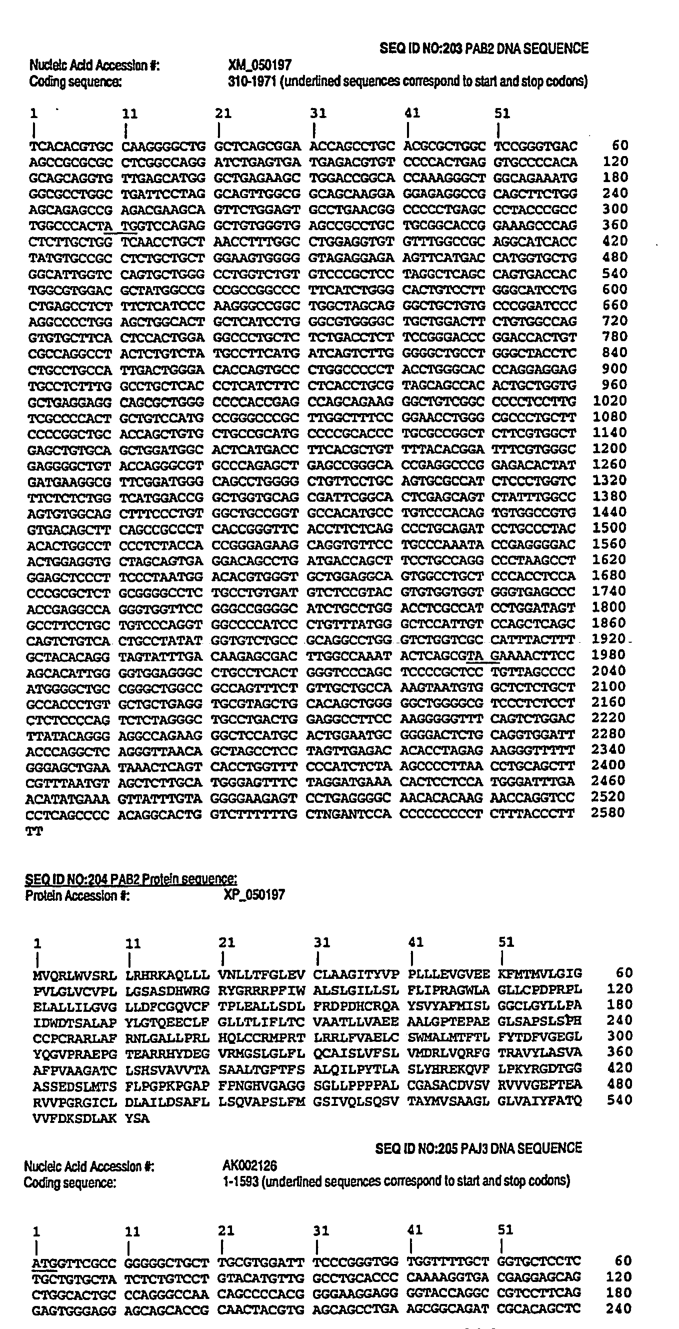

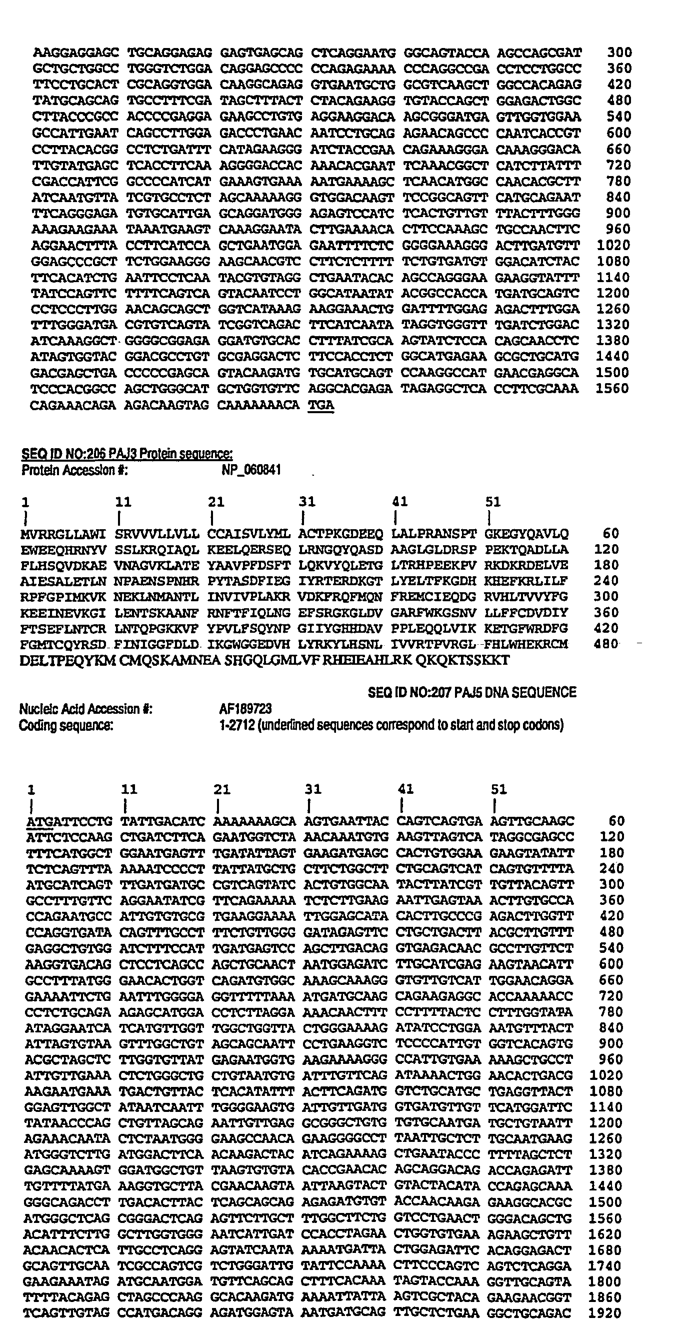

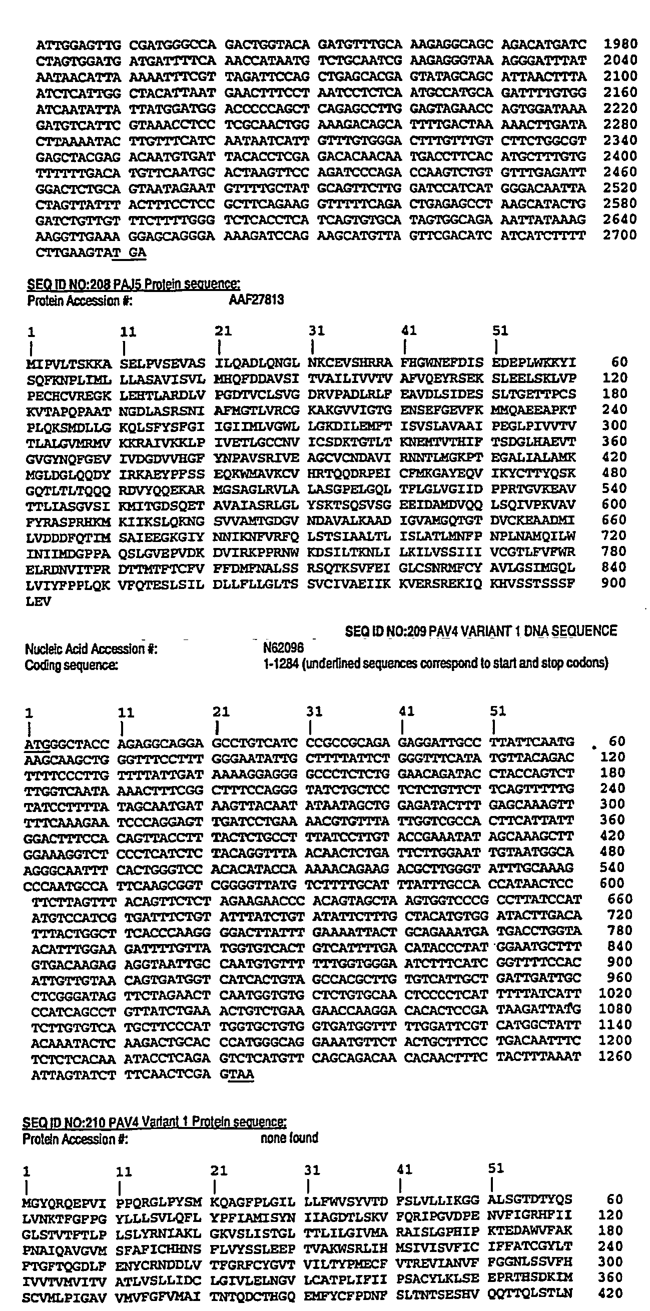

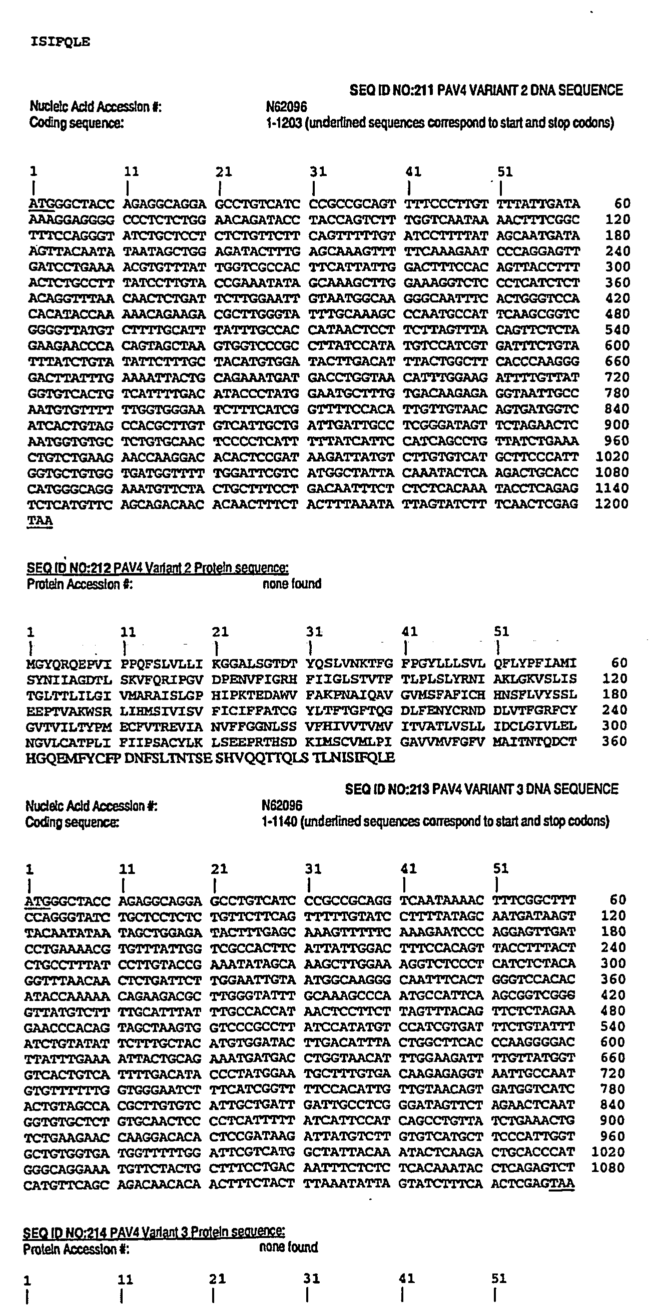

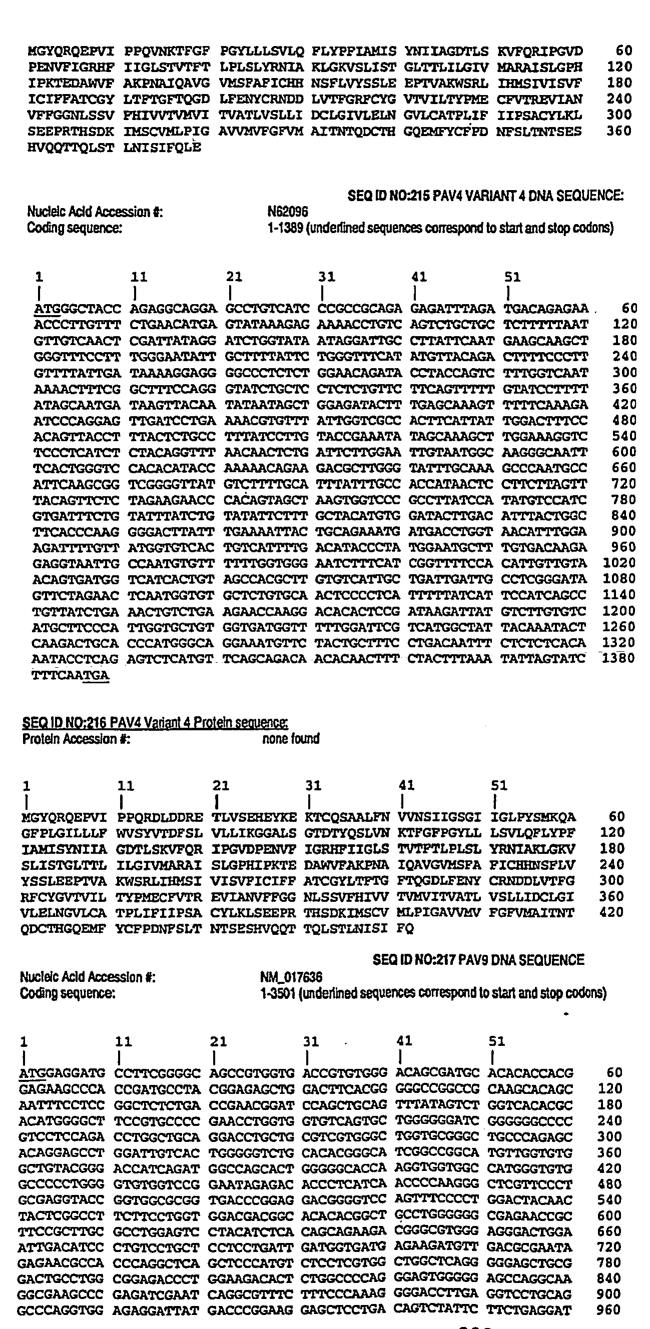

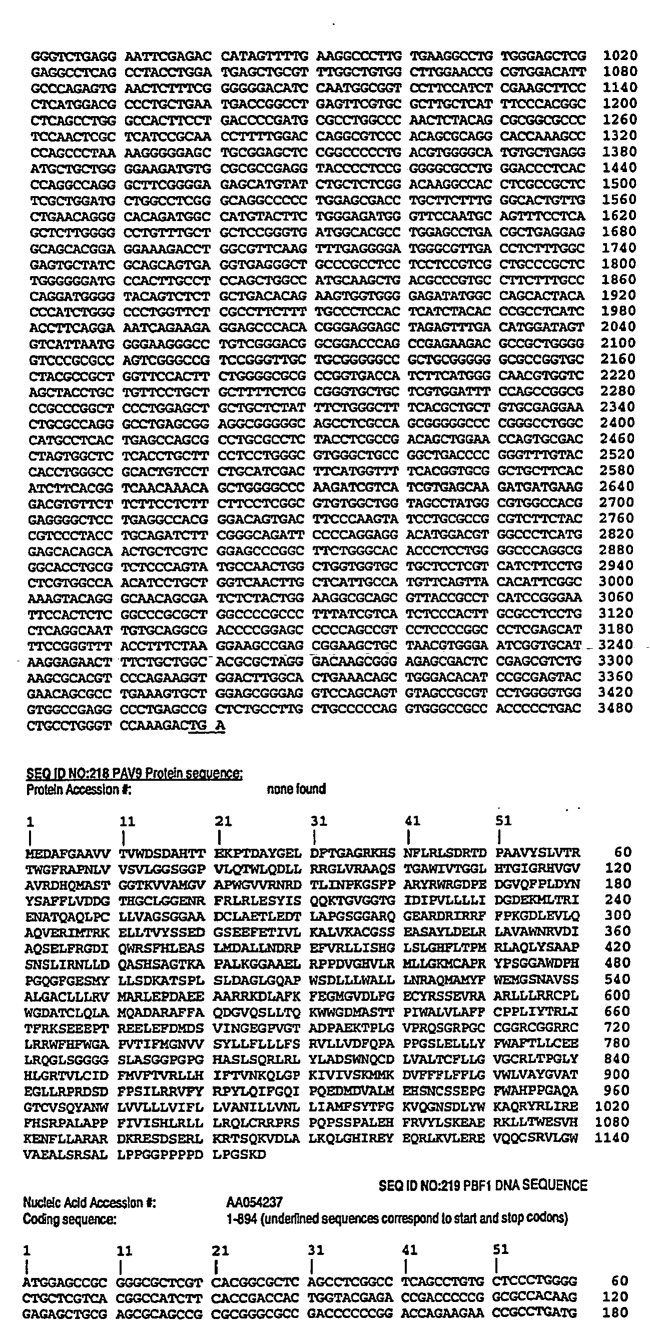

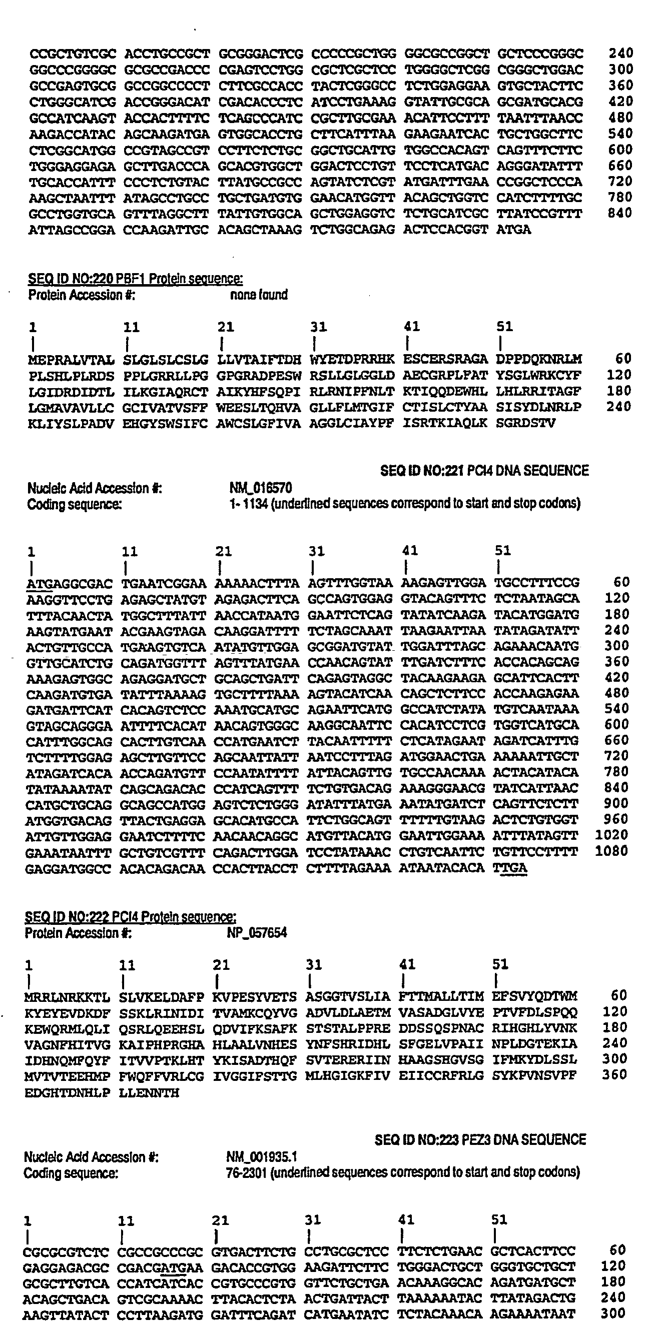

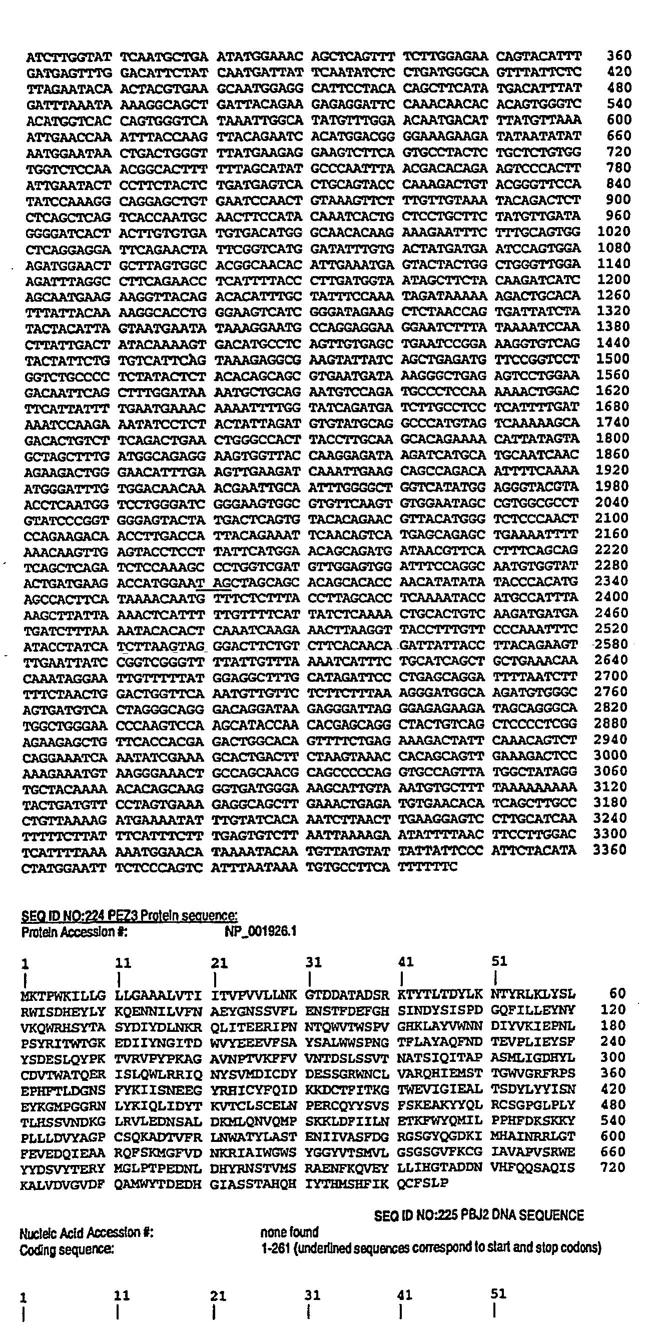

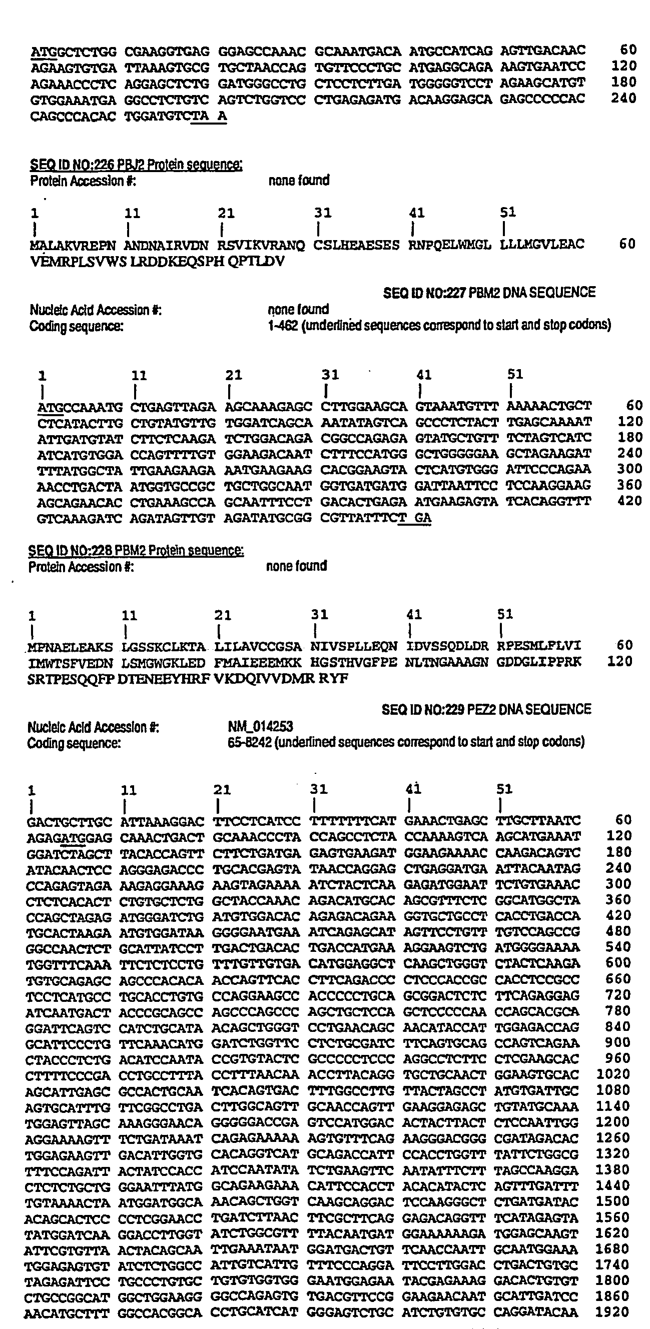

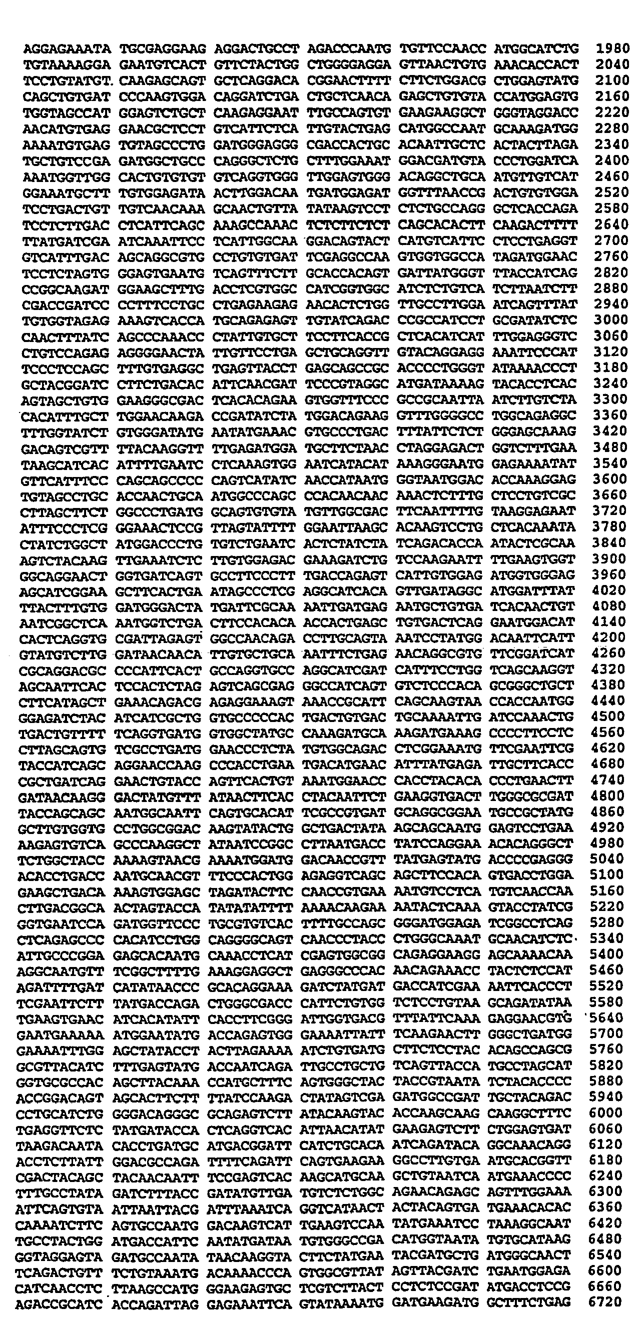

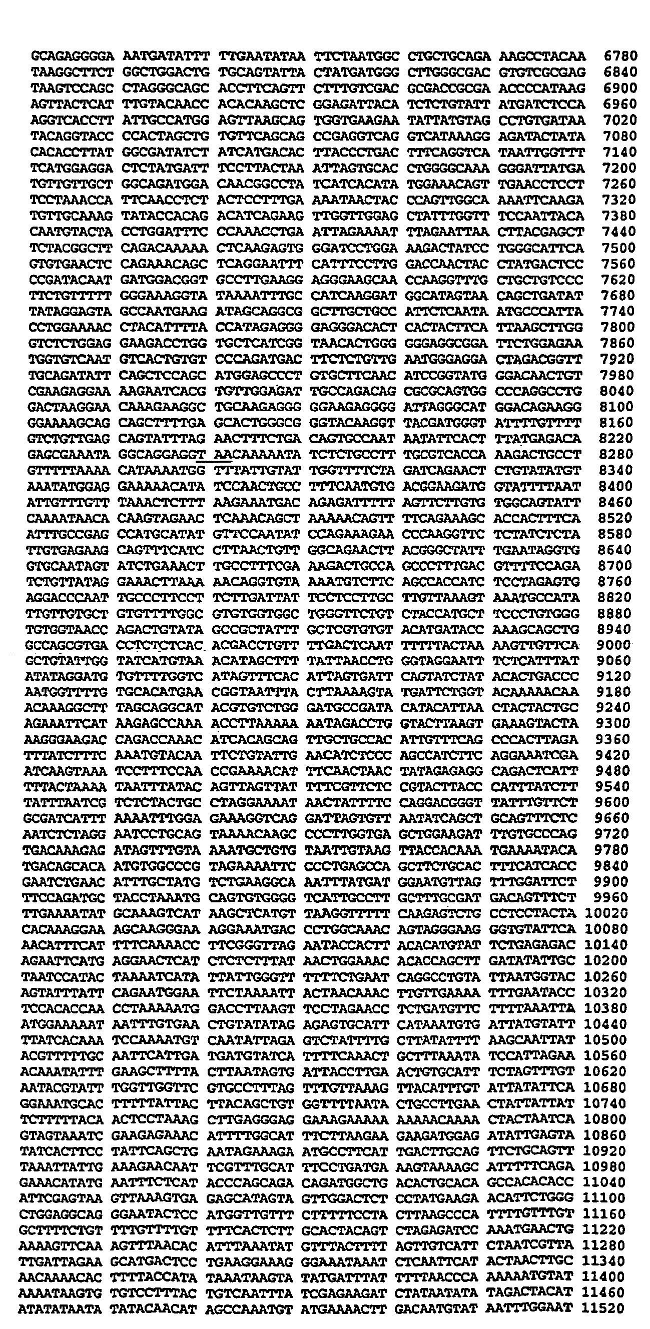

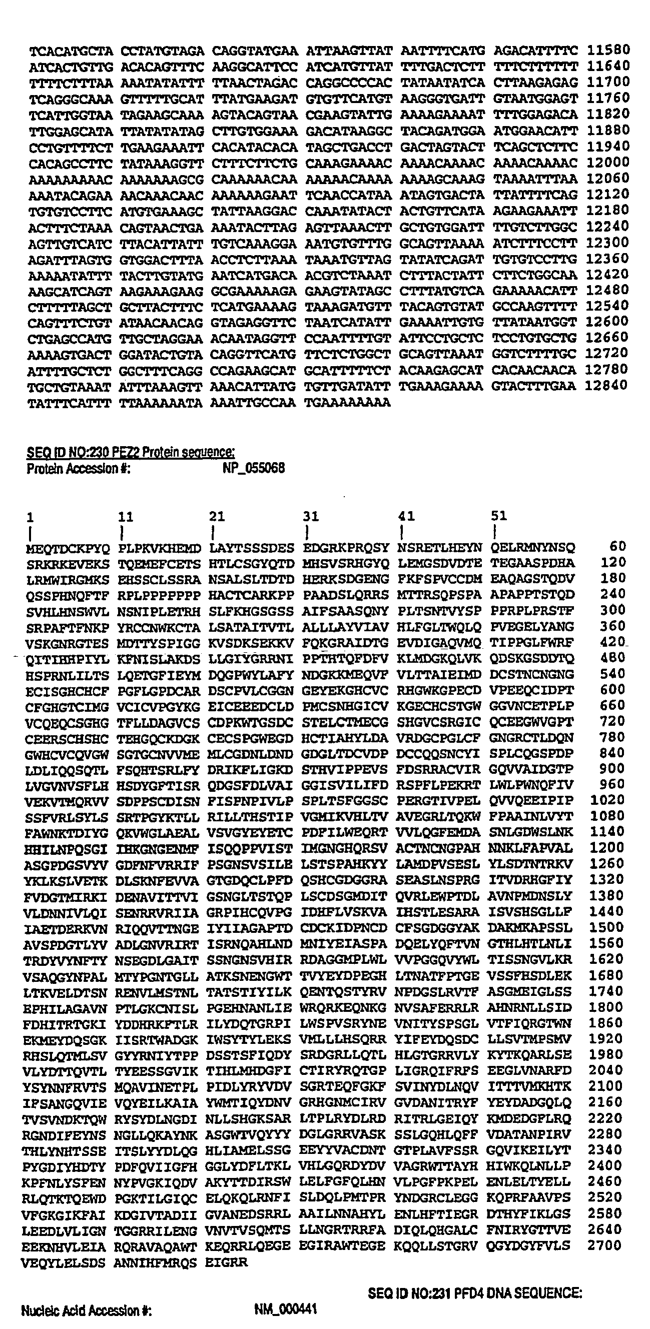

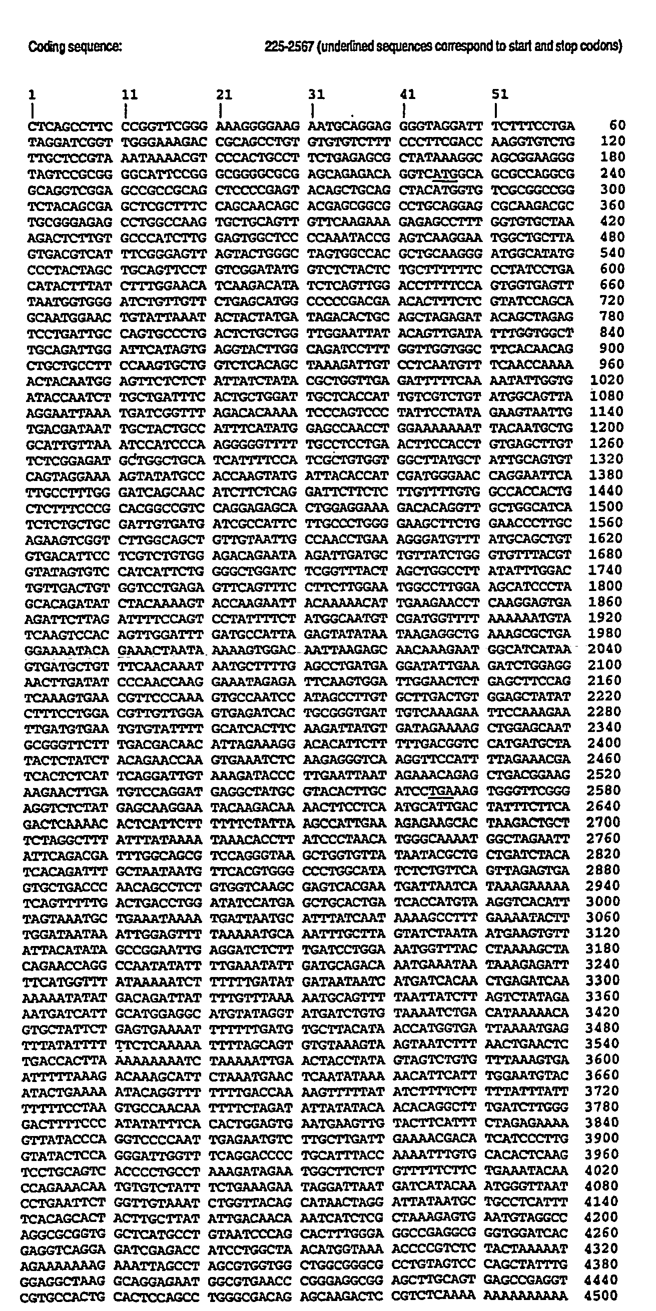

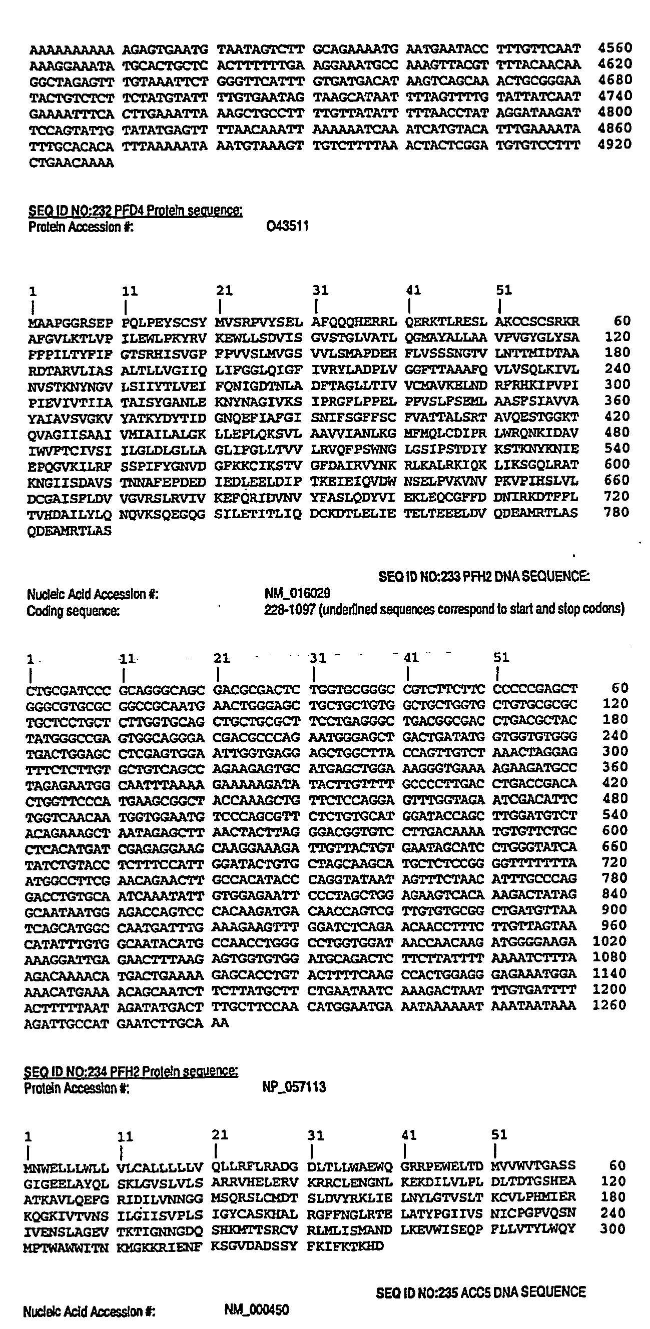

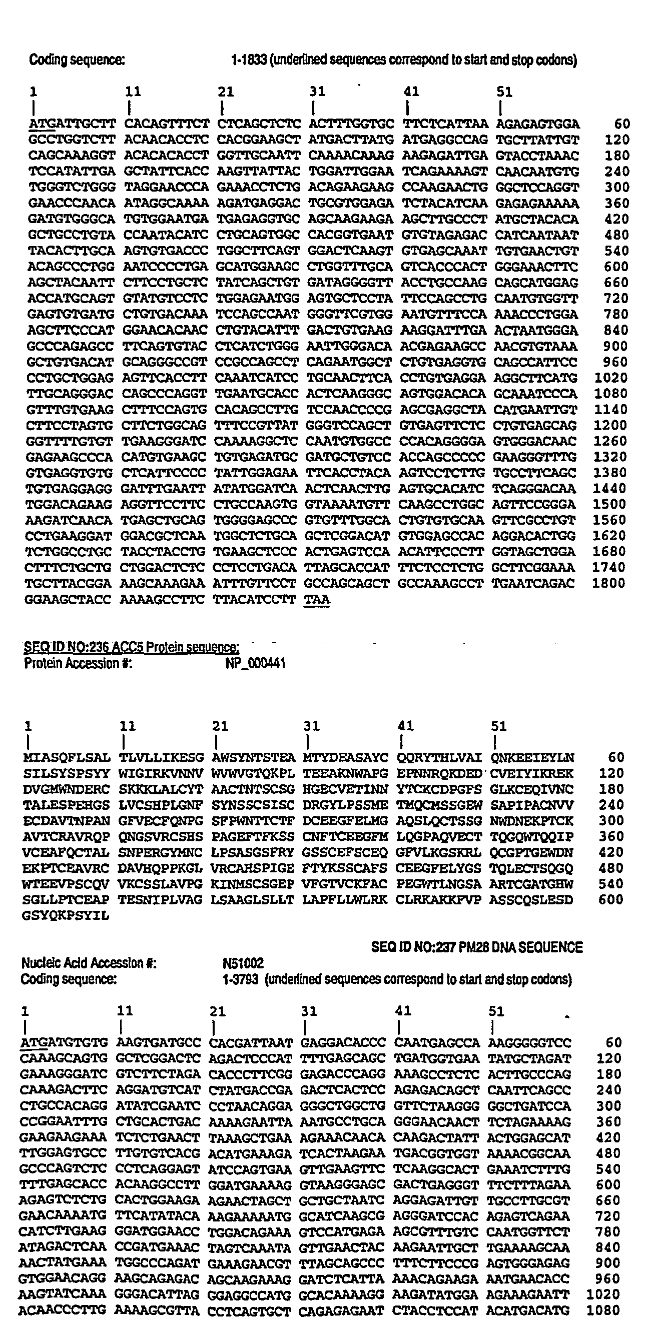

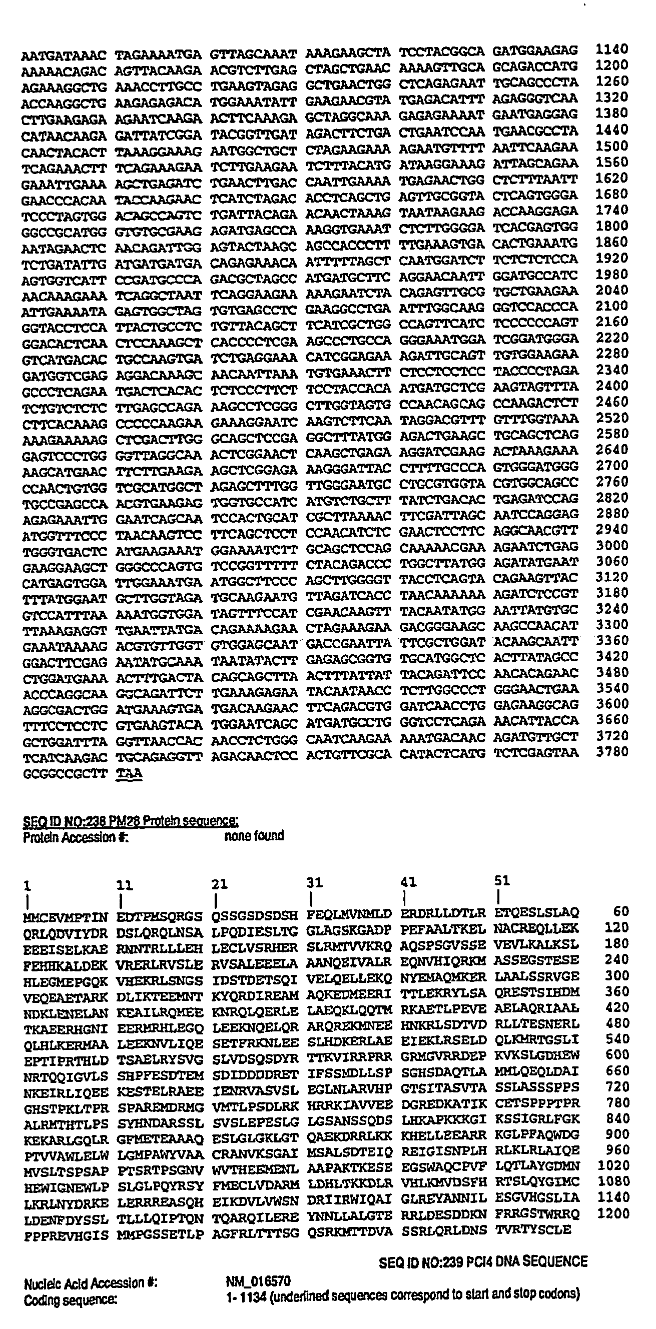

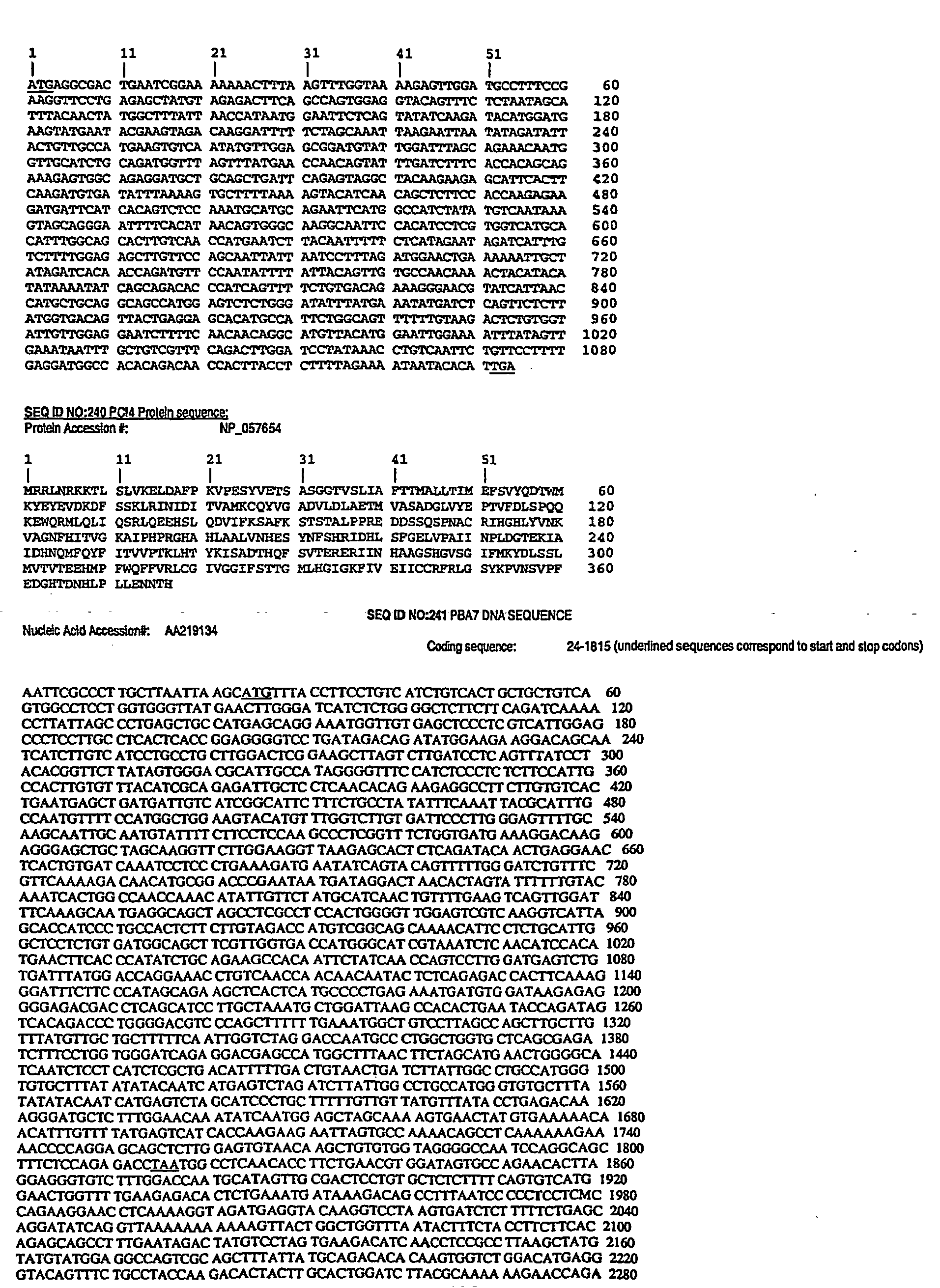

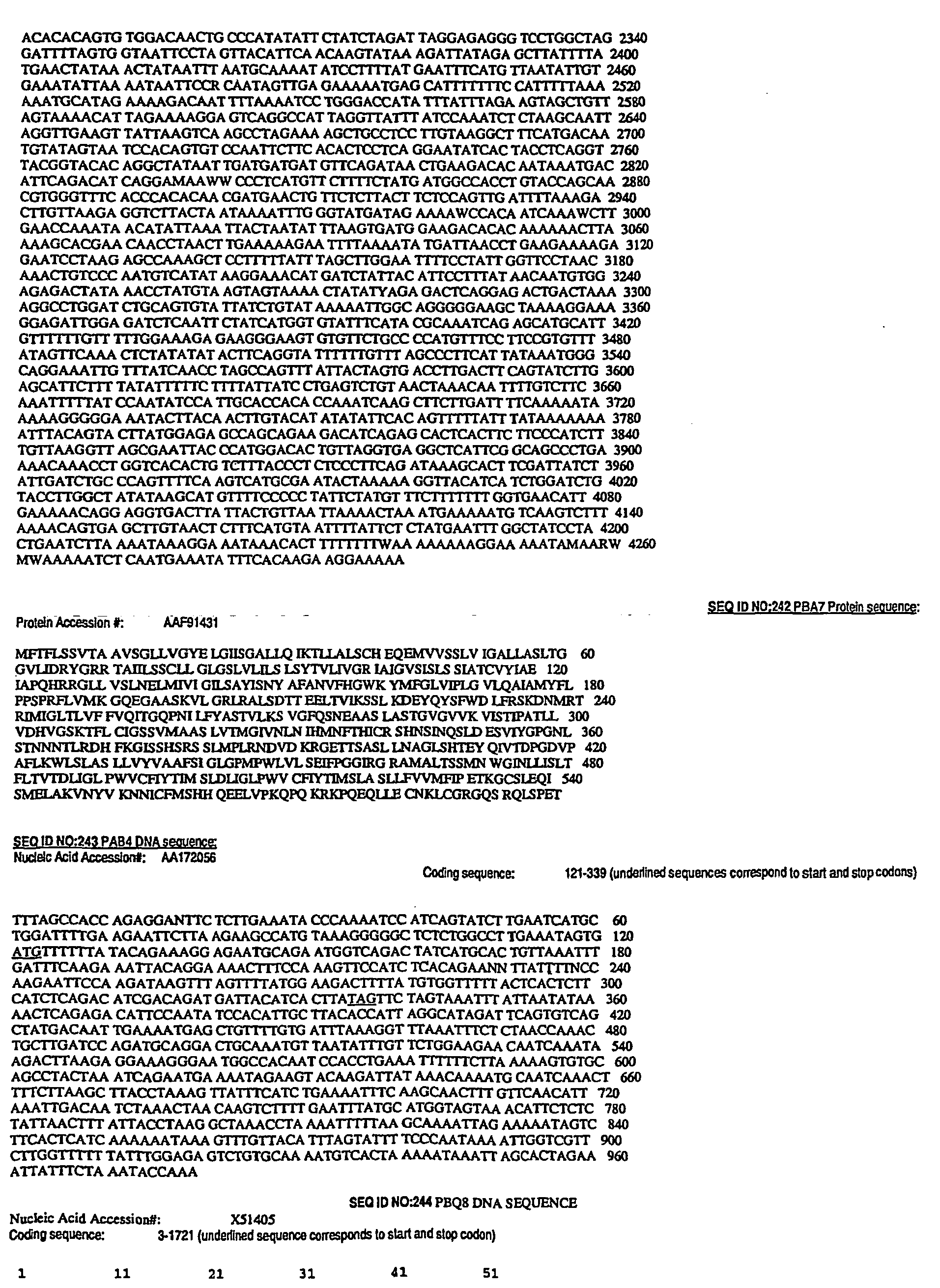

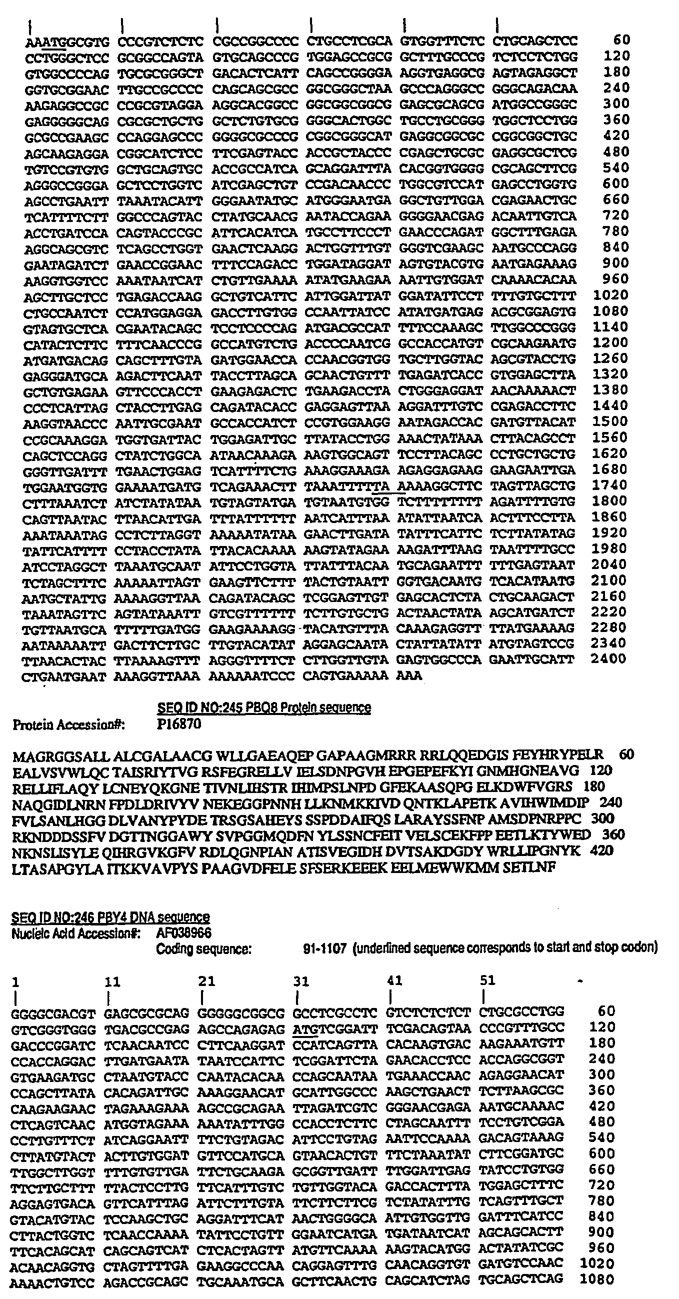

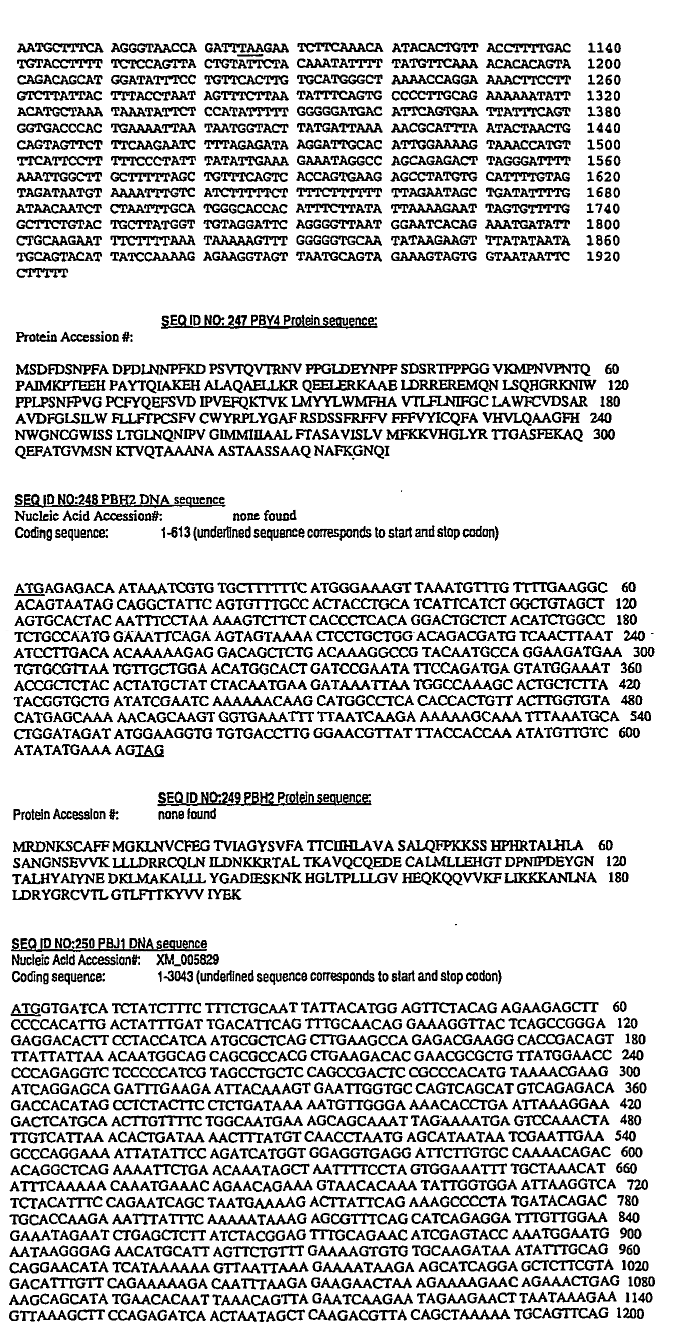

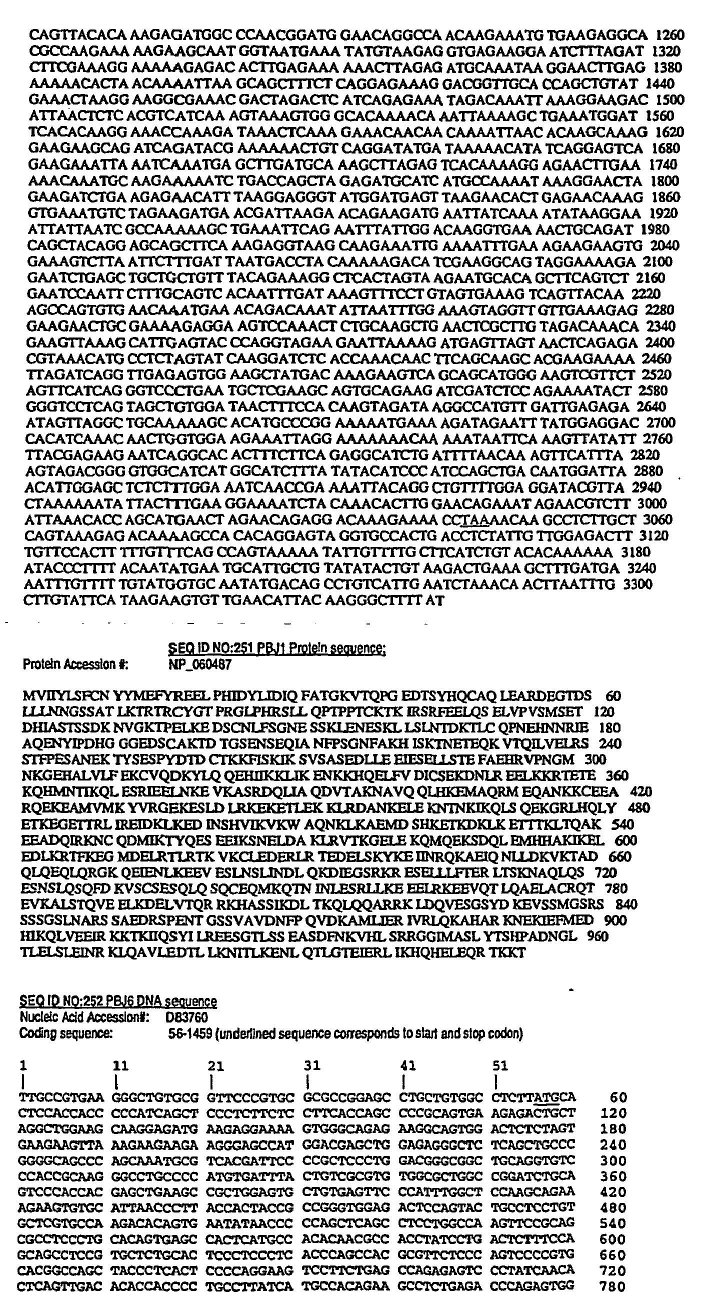

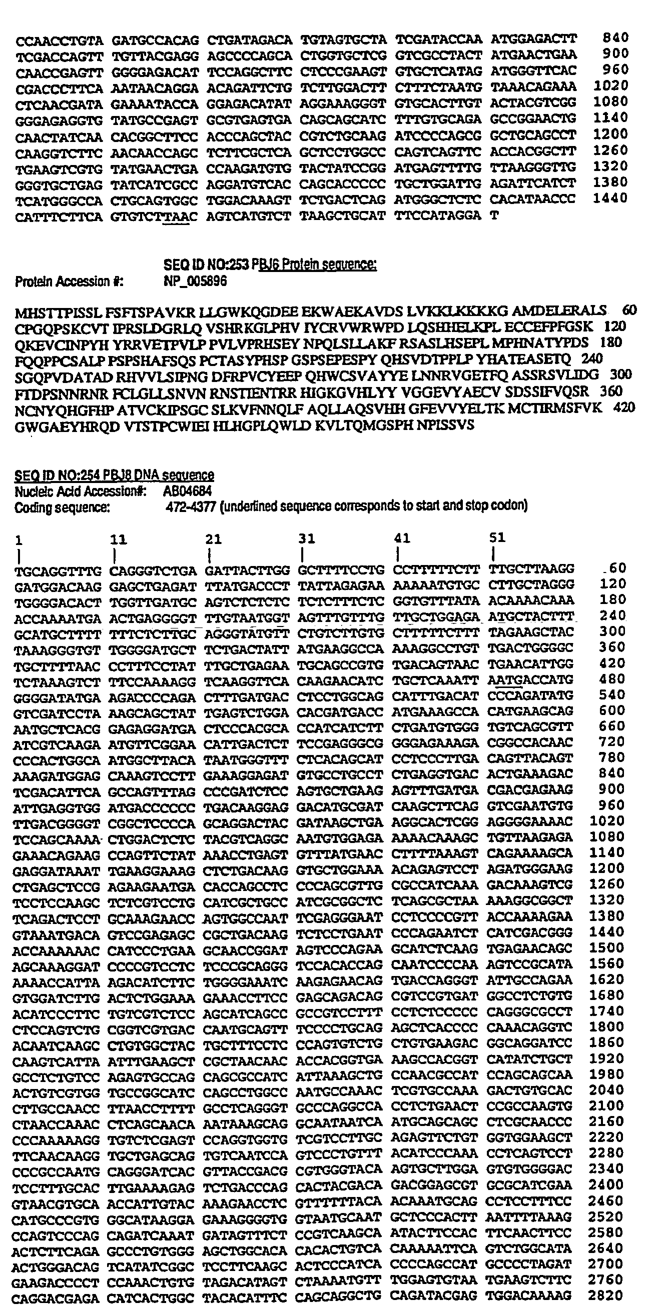

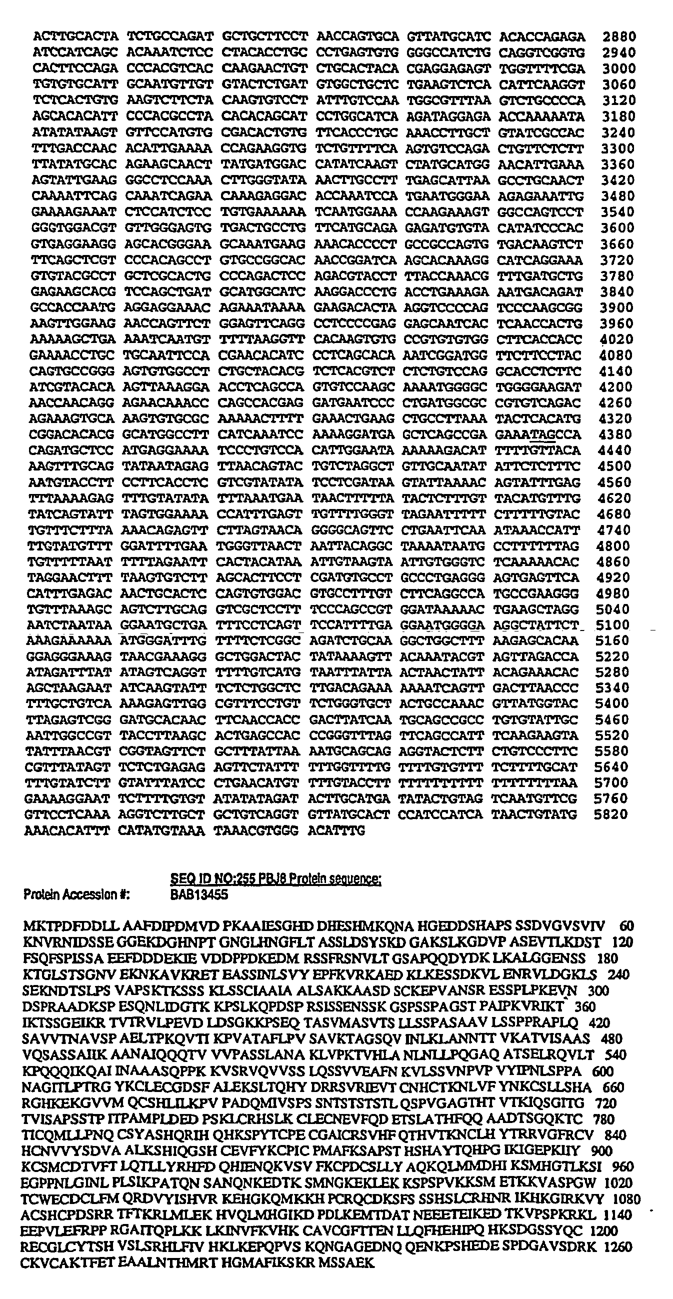

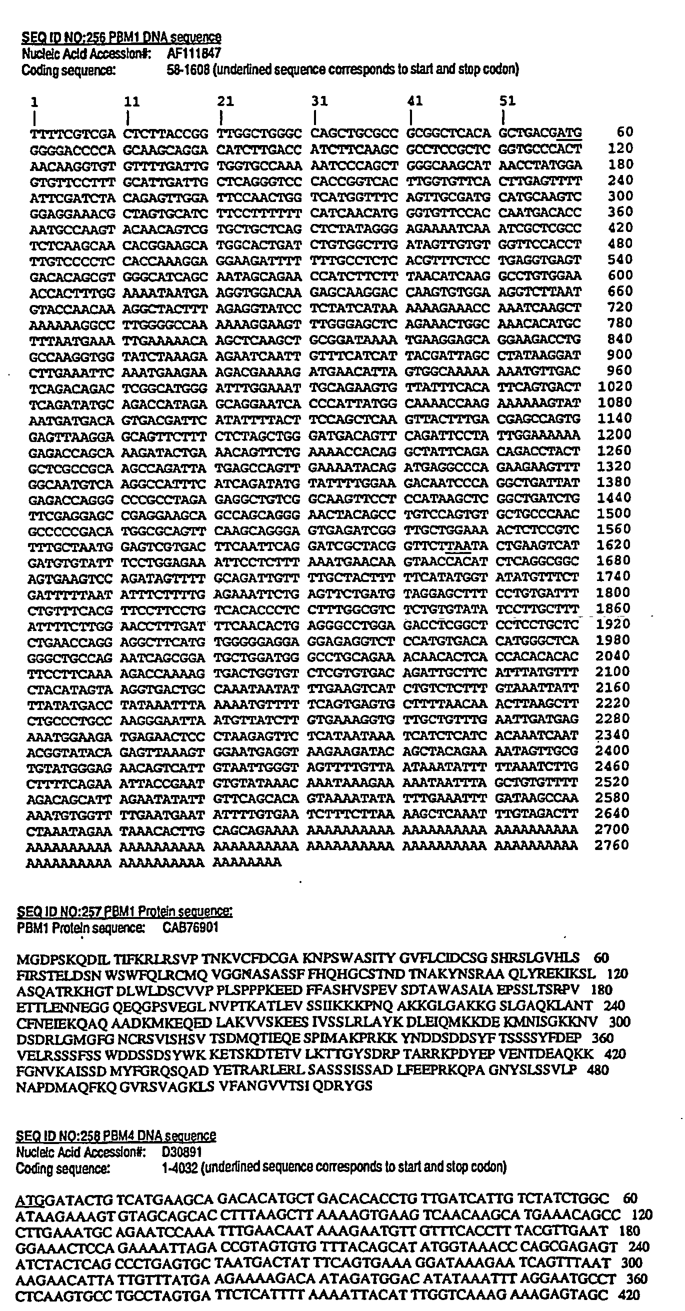

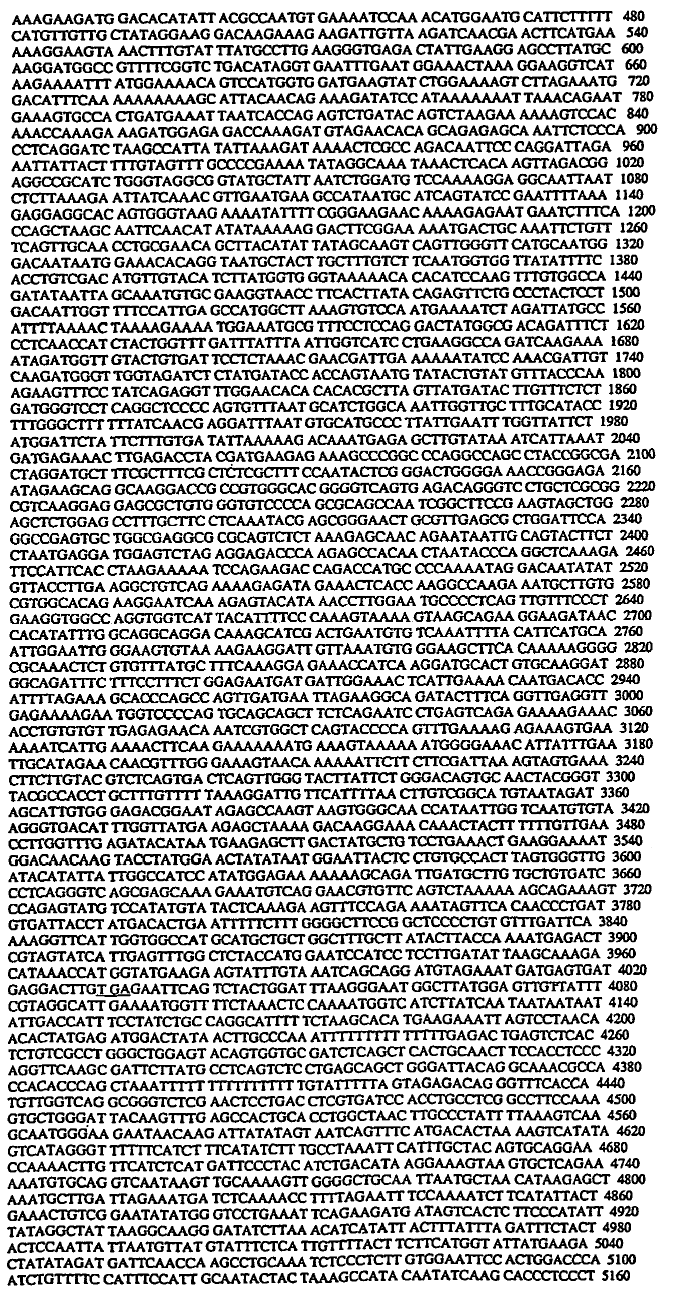

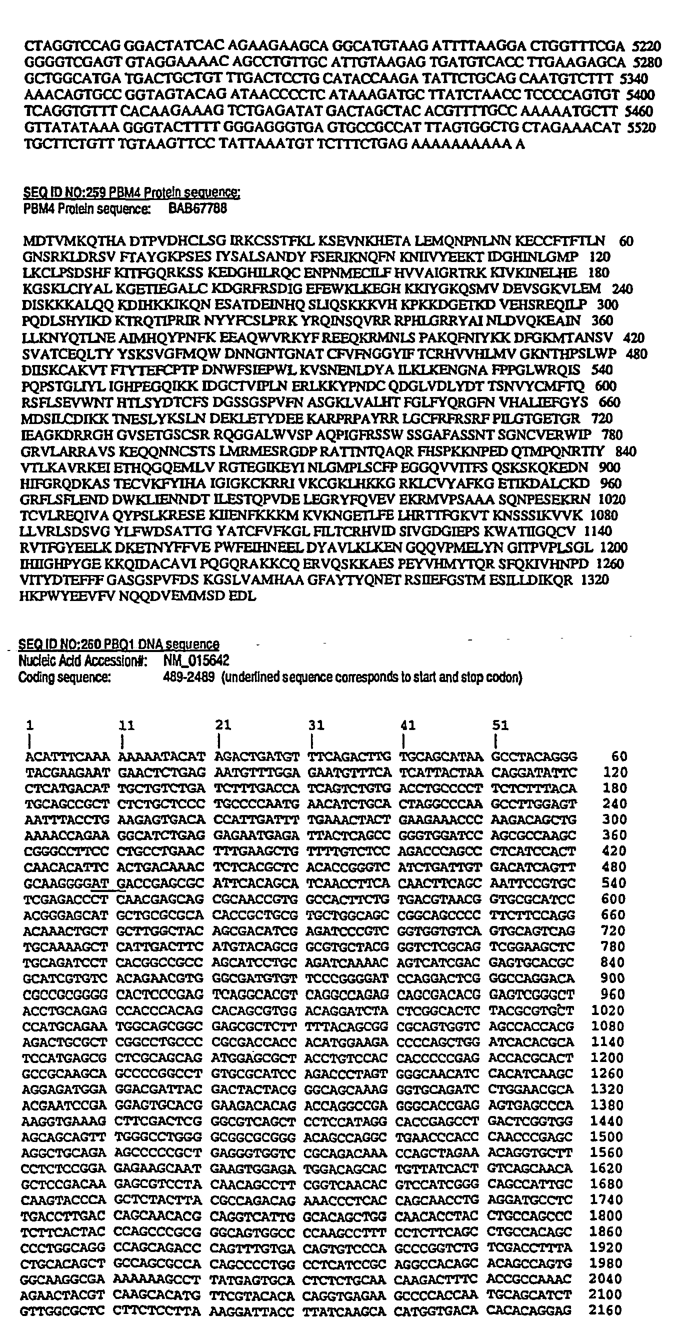

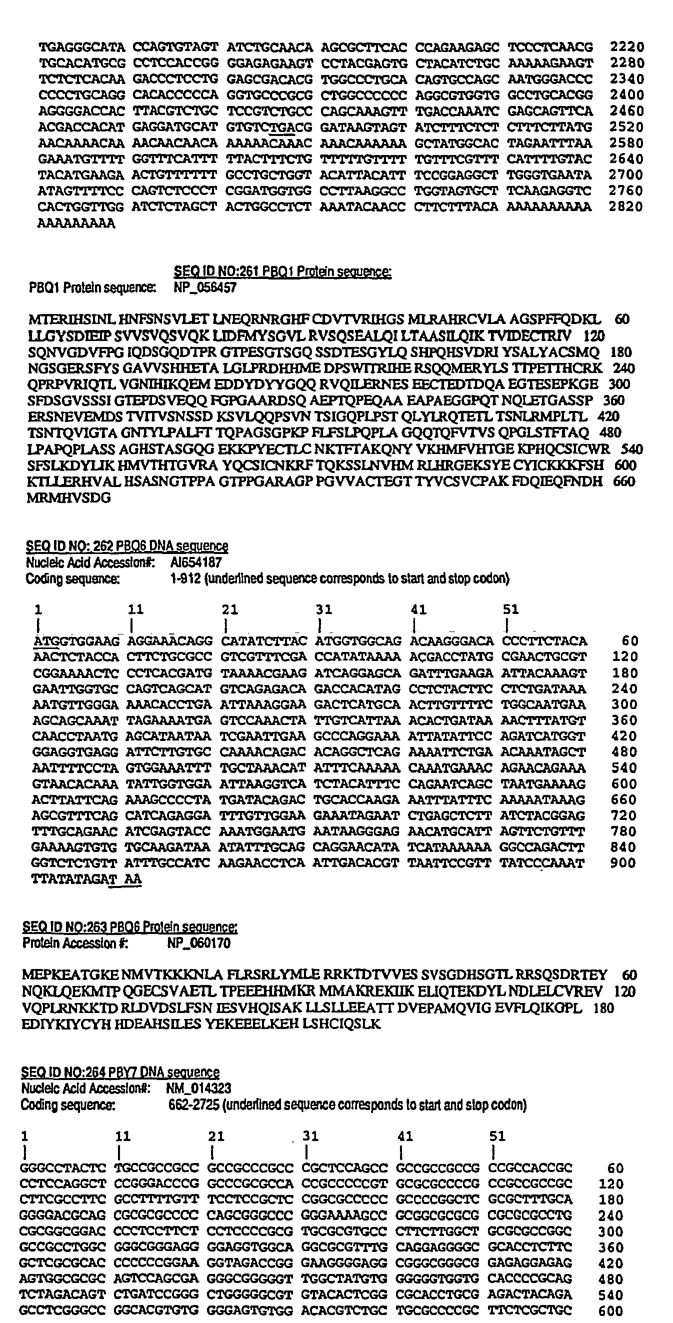

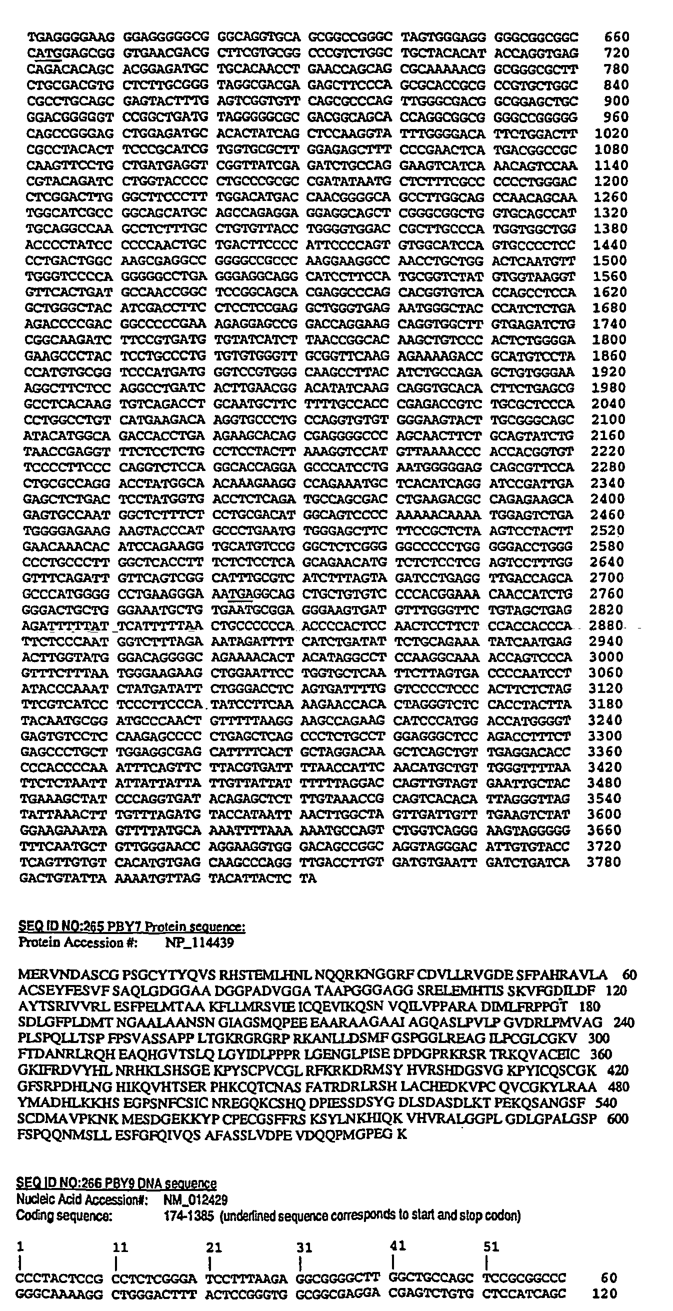

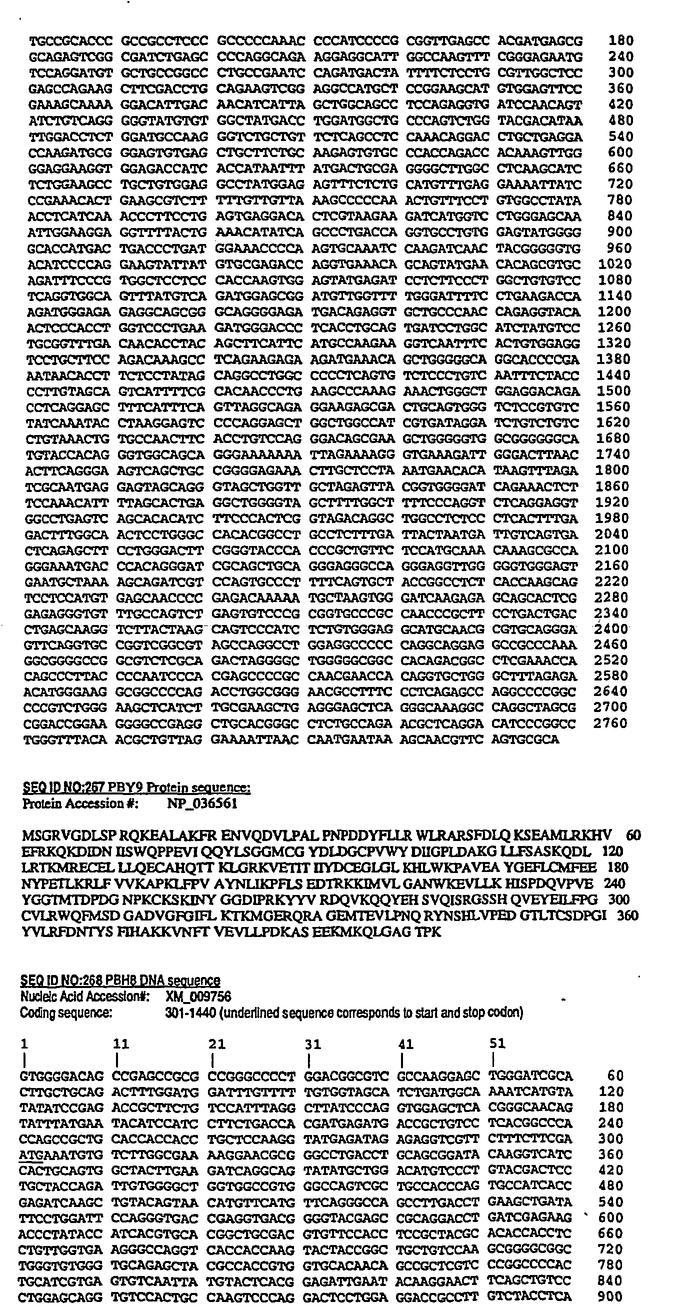

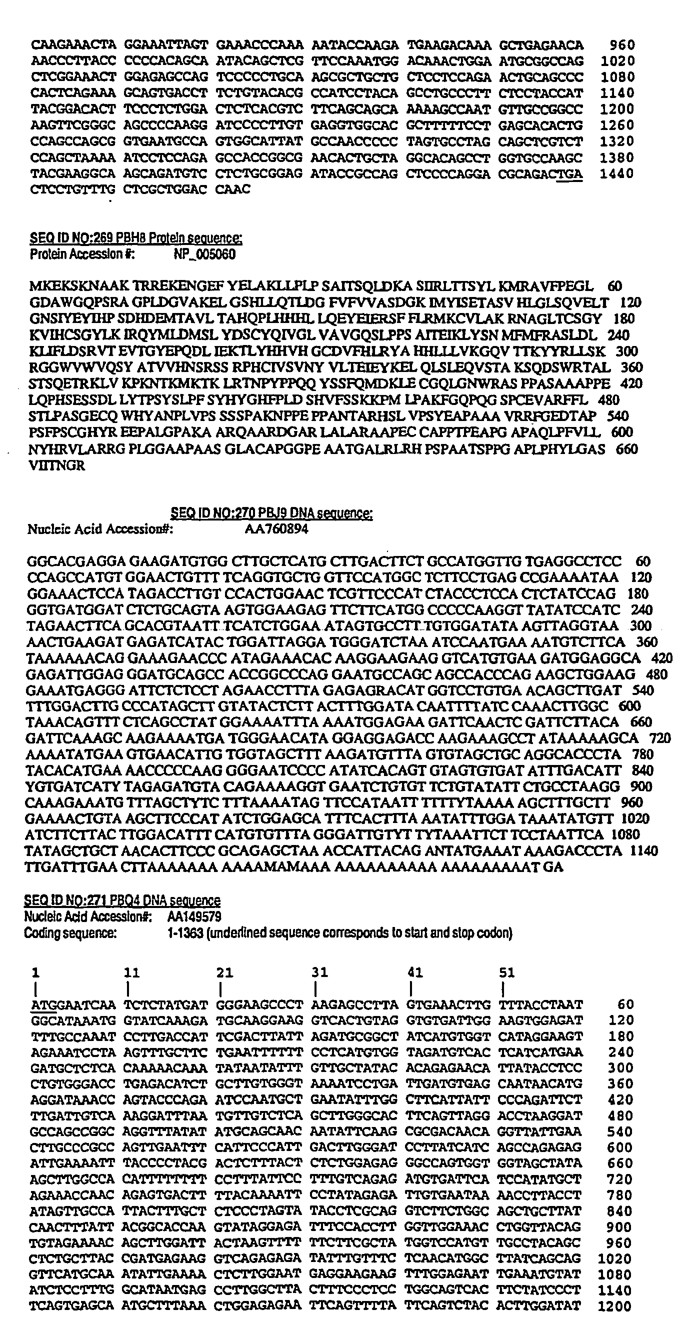

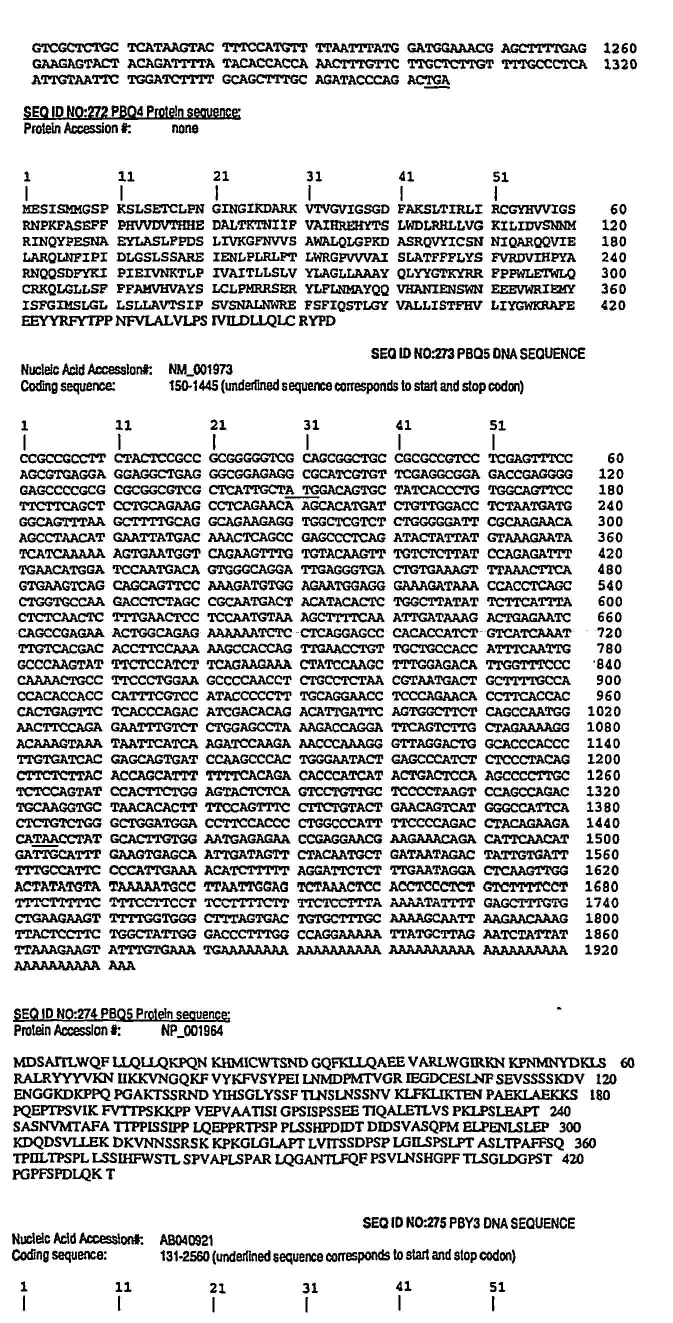

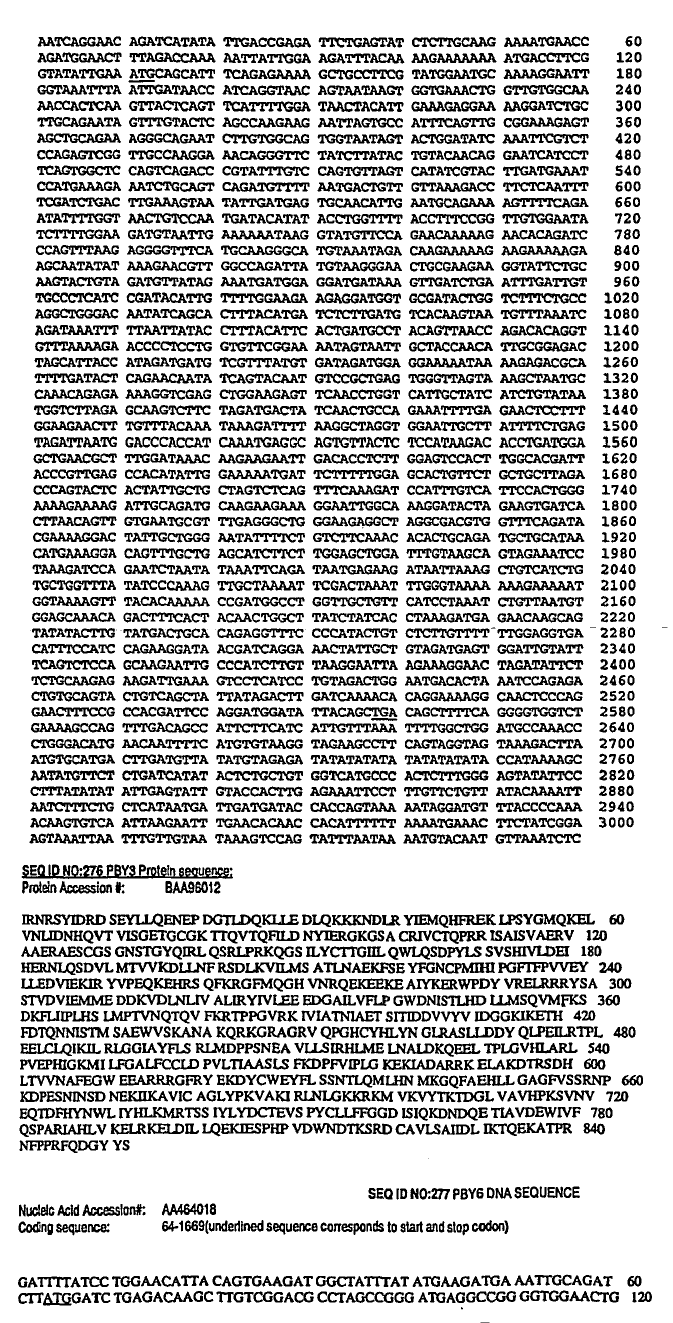

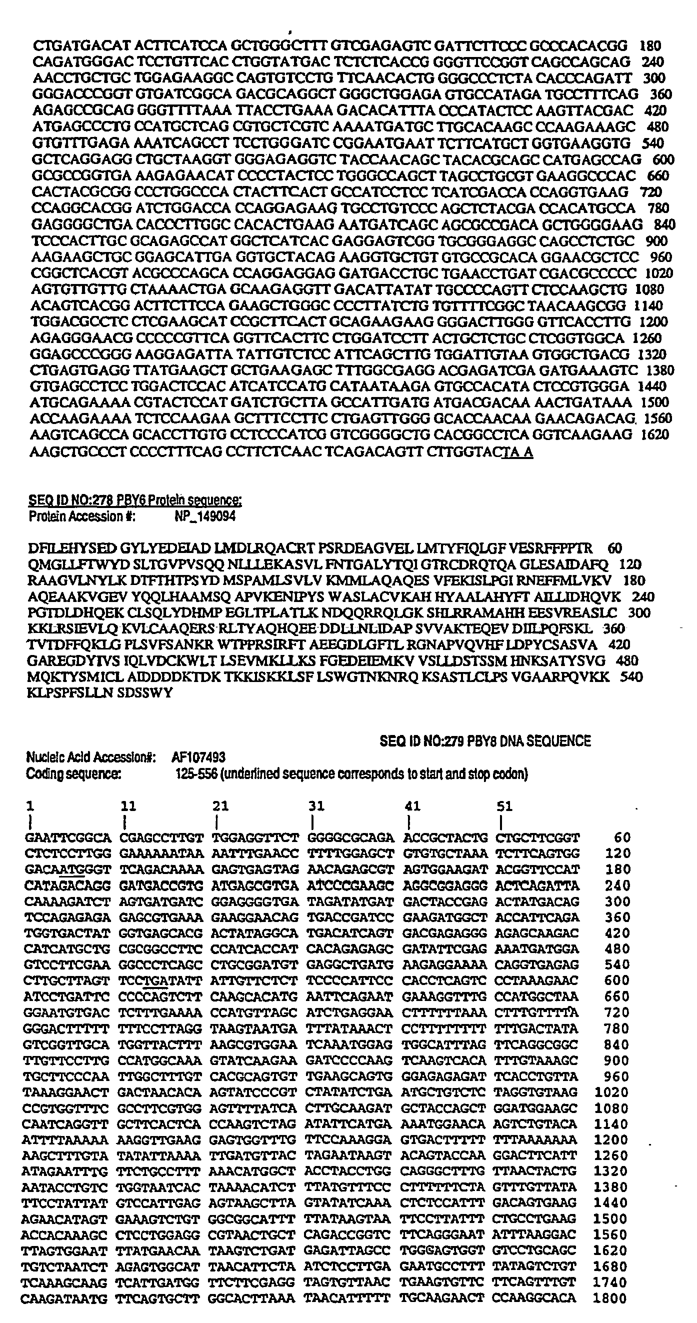

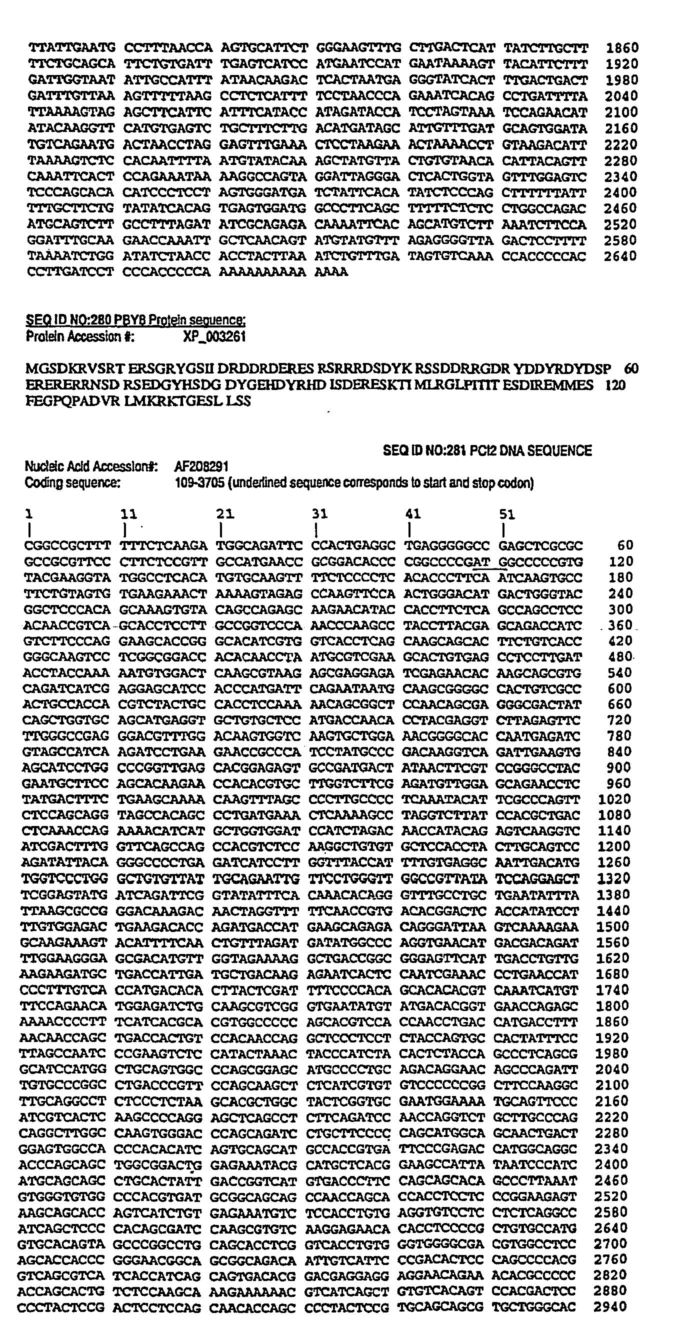

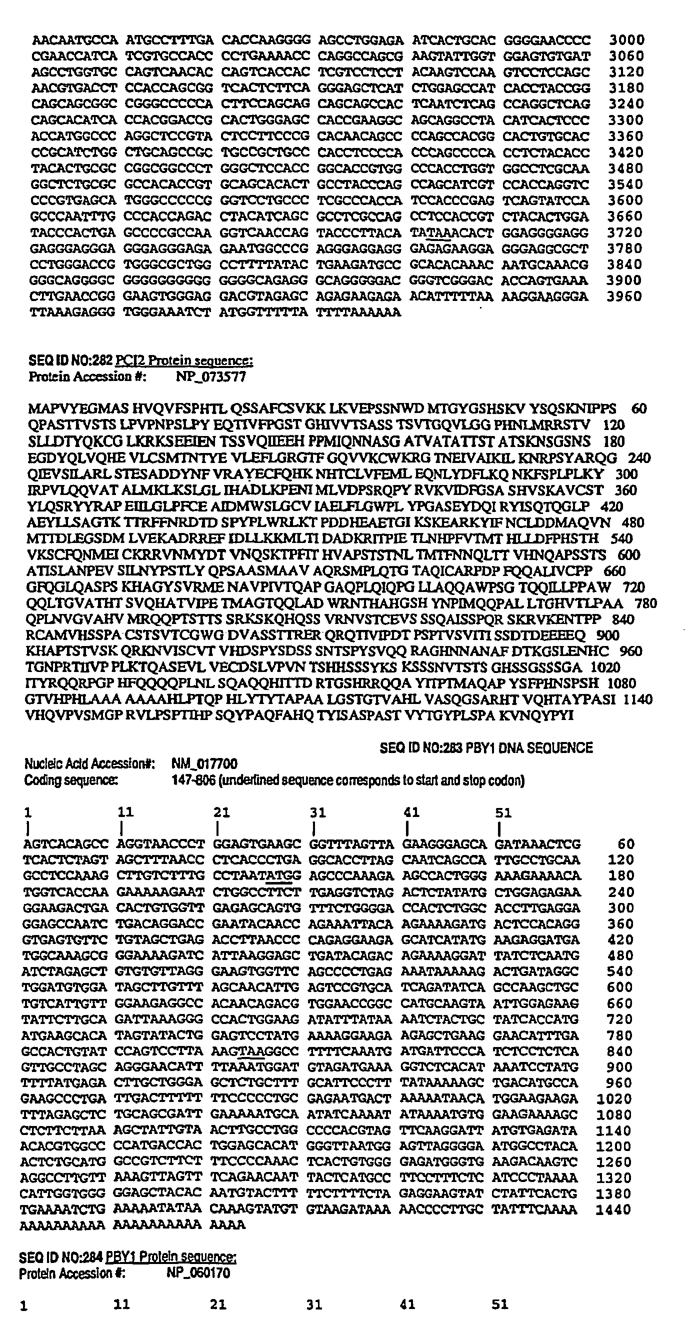

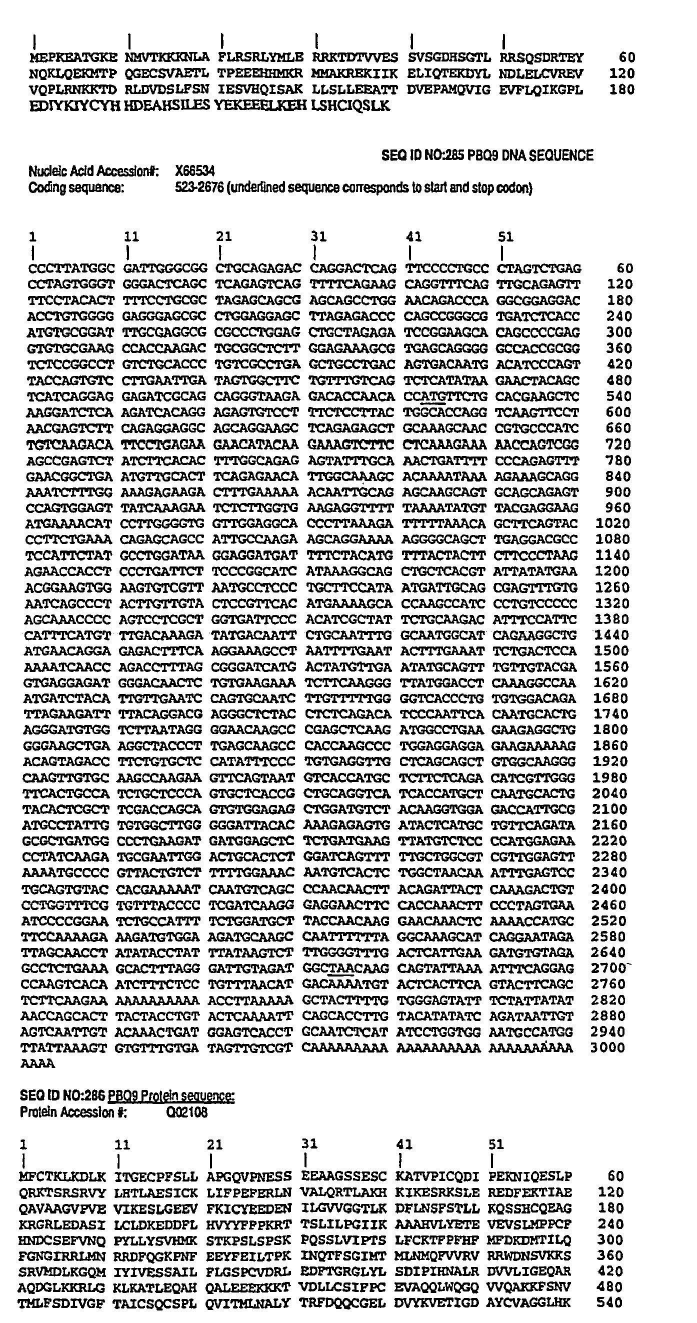

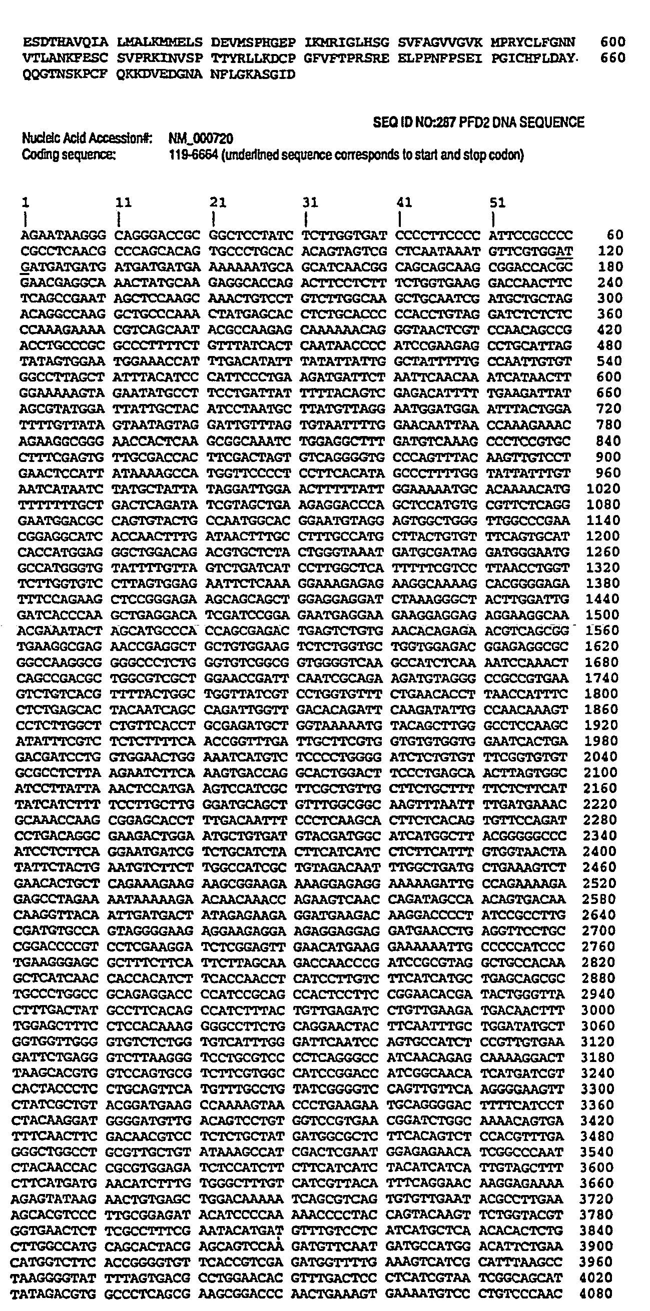

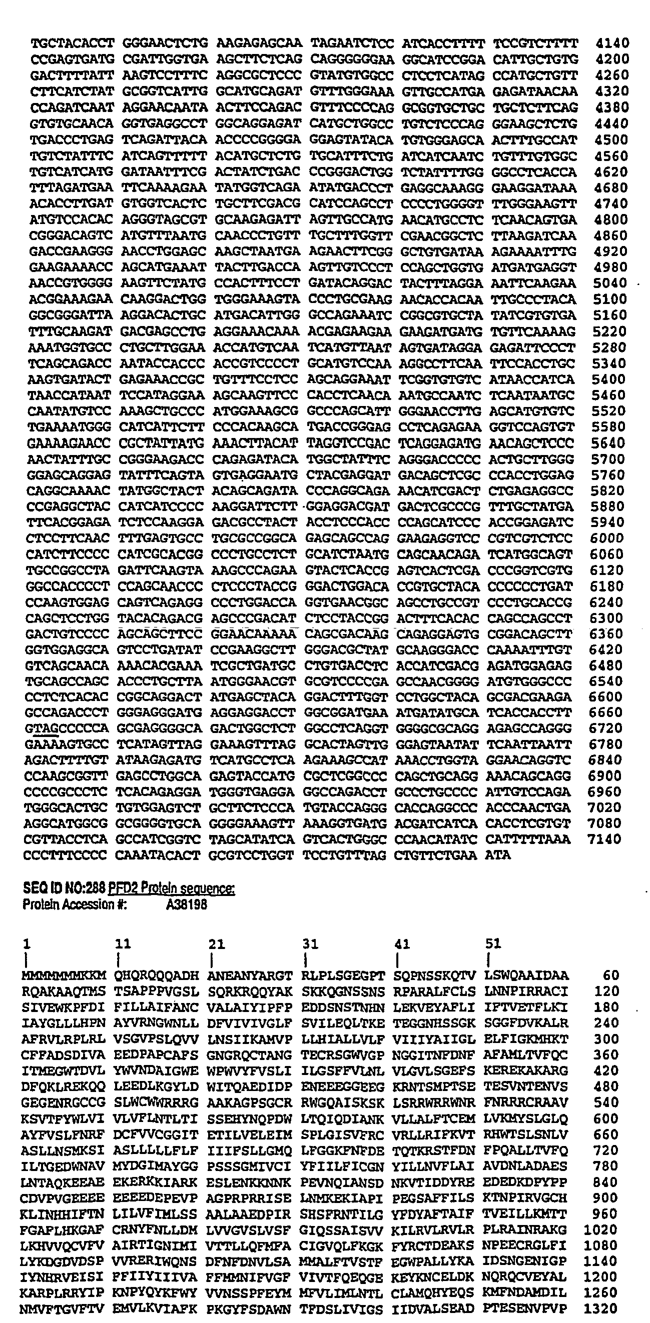

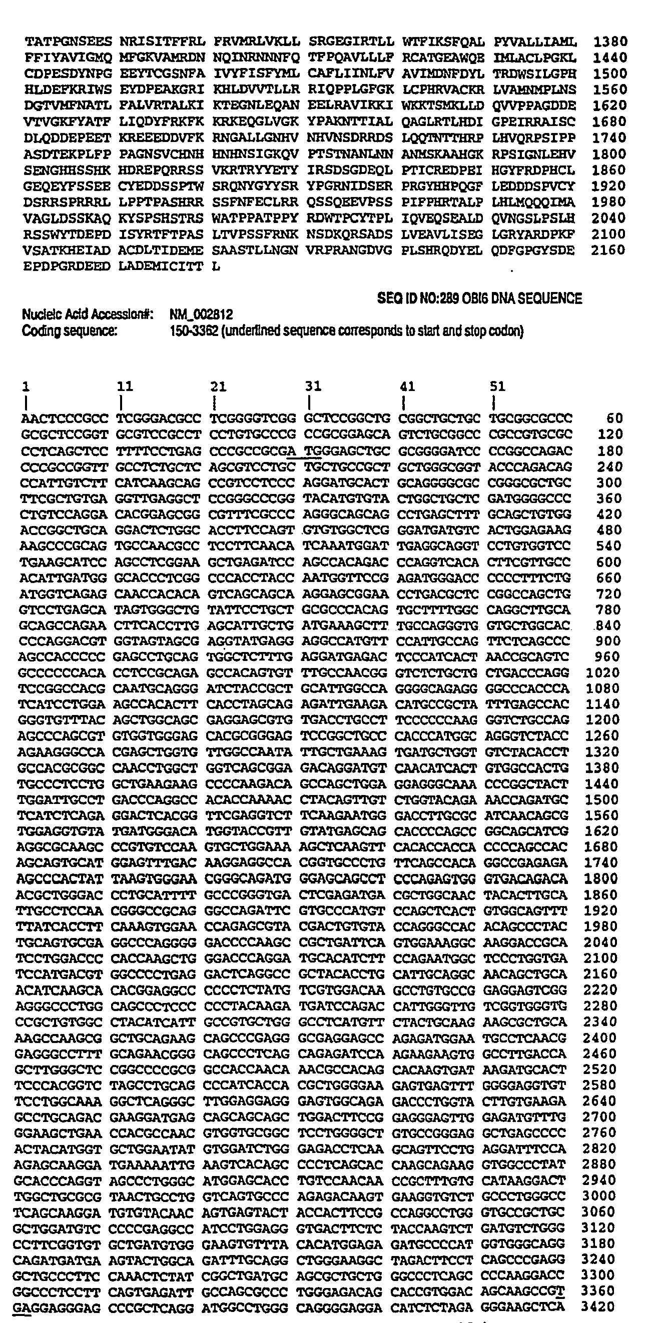

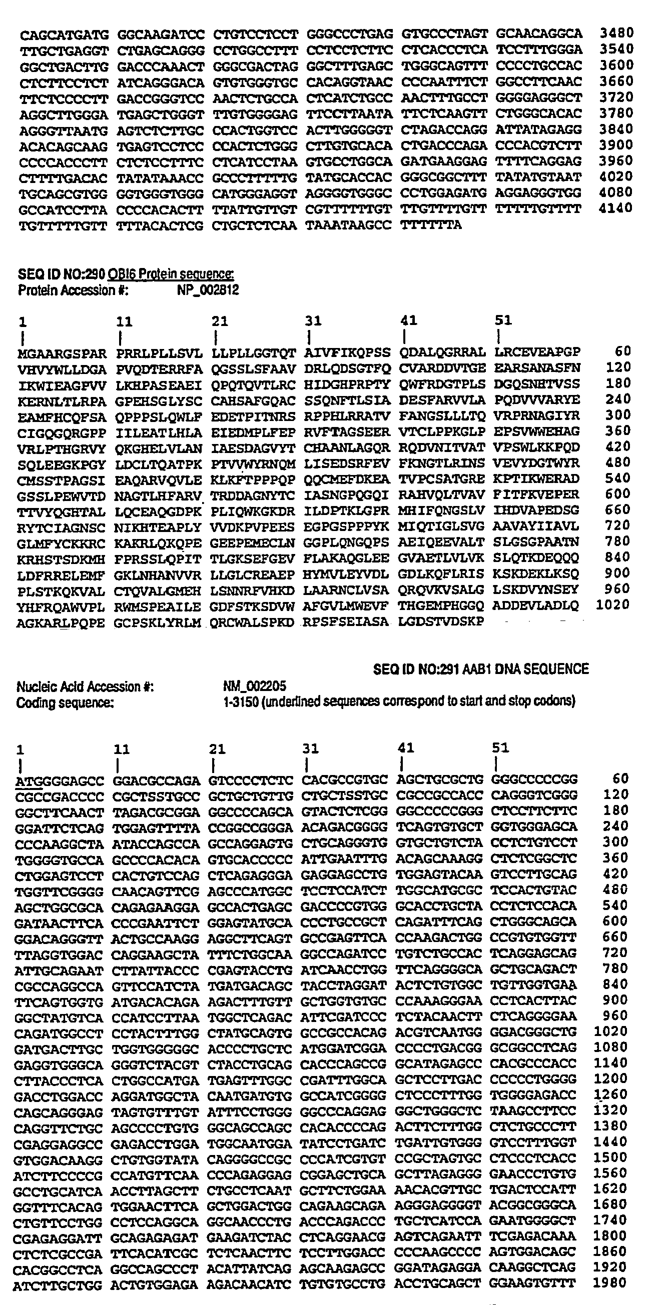

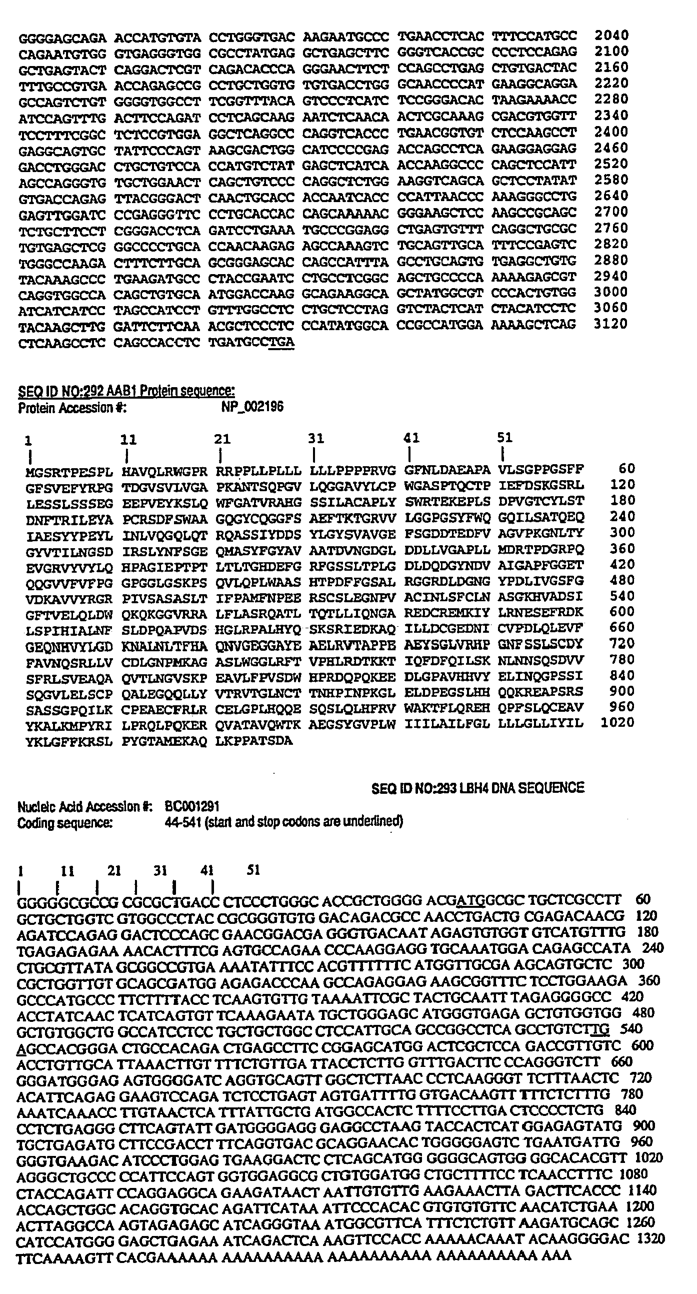

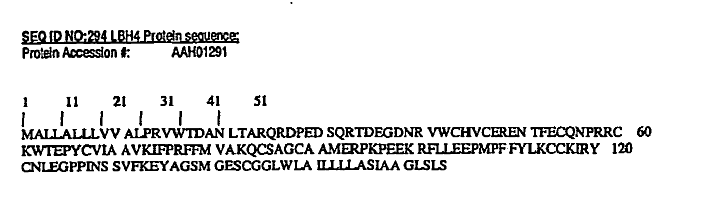

It is understood that the examples described above in no way serve to limit the true scope of this invention, but rather are presented for illustrative purposes. All publications, sequences of accession numbers, and patent applications cited in this specification are herein incorporated by reference as if each individual publication or patent application were specifically and individually indicated to be incorporated by reference.

## Claims

1. A method of detecting a prostate cancer-associated transcript in a cell from a patient, the method comprising contacting a biological sample from the patient with a polynucleotide that selectively hybridizes to a sequence at least 80% identical to a sequence as shown in Table 10.

2. The method of claim 1, wherein the polynucleotide selectively hybridizes to a sequence at least 95% identical to a sequence as shown in Table 10.

3. The method of claim 1, wherein the biological sample is a tissue sample.

4. The method of claim 1, wherein the biological sample comprises isolated nucleic acids.

5. The method of claim 4, wherein the nucleic acids are mRNA.

6. The method of claim 4, further comprising the step of amplifying nucleic acids before the step of contacting the biological sample with the polynucleotide.

7. The method of claim 1, wherein the polynucleotide comprises a sequence as shown in Table 10.

8. The method of claim 1, wherein the polynucleotide is labeled.

9. The method of claim 8, wherein the label is a fluorescent label.

10. The method of claim 1, wherein the polynucleotide is immobilized on a solid surface.

11. The method of claim 1, wherein the patient is undergoing a therapeutic regimen to treat prostate cancer.

12. The method of claim 1, wherein the patient is suspected of having prostate cancer.

13. A method of monitoring the efficacy of a therapeutic treatment of prostate cancer, the method comprising the steps of:
(i) providing a biological sample from a patient undergoing the therapeutic treatment; and
(ii) determining the level of a prostate cancer-associated transcript in the biological sample by contacting the biological sample with a polynucleotide that selectively hybridizes to a sequence at least 80% identical to a sequence as shown in Table 10, thereby monitoring the efficacy of the therapy.

14. The method of claim 13, further comprising the step of: (iii) comparing the level of the prostate cancer-associated transcript to a level of the prostate cancer-associated transcript in a biological sample from the patient prior to, or earlier in, the therapeutic treatment.

15. The method of claim 13, wherein the patient is a human.

16. A method of monitoring the efficacy of a therapeutic treatment of prostate cancer, the method comprising the steps of:
(i) providing a biological sample from a patient undergoing the therapeutic treatment; and
(ii) determining the level of a prostate cancer-associated antibody in the biological sample by contacting the biological sample with a polypeptide encoded by a polynucleotide that selectively hybridizes to a sequence at least 80% identical to a sequence as shown in Table 10, wherein the polypeptide specifically binds to the prostate cancer-associated antibody, thereby monitoring the efficacy of the therapy.

17. The method of claim 16, further comprising the step of (iii) comparing the level of the prostate cancer-associated antibody to a level of the prostate cancer-associated antibody in a biological sample from the patient prior to, or earlier in, the therapeutic treatment.

18. The method of claim 16, wherein the patient is a human.

19. A method of monitoring the efficacy of a therapeutic treatment of prostate cancer, the method comprising the steps of:
(i) providing a biological sample from a patient undergoing the therapeutic treatment; and
(ii) determining, the level of a prostate cancer-associated polypeptide in the biological sample by contacting the biological sample with an antibody, wherein the antibody specifically binds to a polypeptide encoded by a polynucleotide that selectively hybridizes to a sequence at least 80% identical to a sequence as shown in Table 10, thereby monitoring the efficacy of the therapy.

20. The method of claim 19, further comprising the step of: (iii) comparing the level of the prostate cancer-associated polypeptide to a level of the prostate cancer-associated polypeptide in a biological sample from the patient prior to, or earlier in, the therapeutic treatment.

21. The method of claim 19, wherein the patient is a human.

22. An isolated nucleic acid molecule consisting of a polynucleotide sequence as shown in Table 10.

23. The nucleic acid molecule of claim 22, which is labeled.

24. The nucleic acid of claim 23, wherein the label is a fluorescent label

25. An expression vector comprising the nucleic acid of claim 22.

26. A host cell comprising the expression vector of claim 25.

27. An isolated polypeptide which is encoded by a nucleic acid molecule having polynucleotide sequence as shown in Table 10.

28. An antibody that specifically binds a polypeptide of claim 27.

29. The antibody of claim 28, further conjugated to an effector component.

30. The antibody of claim 29, wherein the effector component is a fluorescent label.

31. The antibody of claim 29, wherein the effector component is a radioisotope or a cytotoxic chemical.

32. The antibody of claim 29, which is an antibody fragment.

33. The antibody of claim 29, which is a humanized antibody.

34. A method of detecting a prostate cancer cell in a biological sample from a patient, the method comprising contacting the biological sample with an antibody of claim 28.

35. The method of claim 34, wherein the antibody is further conjugated to an effector component.

36. The method of claim 35, wherein the effector component is a fluorescent label.

37. A method of detecting antibodies specific to prostate cancer in a patient, the method comprising contacting a biological sample from the patient with a polypeptide encoded by a nucleic acid comprises a sequence from Table 10.

38. A method for identifying a compound that modulates a prostate cancer-associated polypeptide, the method comprising the steps of:
(i) contacting the compound with a prostate cancer-associated polypeptide, the polypeptide encoded by a polynucleotide that selectively hybridizes to a sequence at least 80% identical to a sequence as shown in Table 10; and
(ii) determining the functional effect of the compound upon the polypeptide.

39. The method of claim 38, wherein the functional effect is a physical effect.

40. The method of claim 38, wherein the functional effect is a chemical effect.

41. The method of claim 38, wherein the polypeptide is expressed in a eukaryotic host cell or cell membrane.

42. The method of claim 38, wherein the functional effect is determined by measuring ligand binding to the polypeptide.

43. The method of claim 38, wherein the polypeptide is recombinant.

44. A method of inhibiting proliferation of a prostate cancer-associated cell to treat prostate cancer in a patient, the method comprising the step of administering to the subject a therapeutically effective amount of a compound identified using the method of claim 38.

45. The method of claim 44, wherein the compound is an antibody.

46. The method of claim 45, wherein the patient is a human.

47. A drug screening assay comprising the steps of
(i) administering a test compound to a mammal having prostate cancer or a cell isolated therefrom;
(ii) comparing the level of gene expression of a polynucleotide that selectively hybridizes to a sequence at least 80% identical to a sequence as shown in Table 10 in a treated cell or mammal with the level of gene expression of the polynucleotide in a control cell or mammal, wherein a test compound that modulates the level of expression of the polynucleotide is a candidate for the treatment of prostate cancer.

48. The assay of claim 47, wherein the control is a mammal with prostate cancer or a cell therefrom that has not been treated with the test compound.

49. The assay of claim 47, wherein the control is a normal cell or mammal.

50. A method for treating a mammal having prostate cancer comprising administering a compound identified by the assay of claim 47.

51. A pharmaceutical composition for treating a mammal having prostate cancer, the composition comprising a compound identified by the assay of claim 47 and a physiologically acceptable excipient.

52. The method according to claim 1, wherein said biological sample is contacted with a plurality of polynucleotides comprising a first polynucleotide that selectively hybridizes to a sequence at least 80% identical to a first sequence as shown in Table 10; and a second polynucleotide that selectively hybridizes to a second sequence at least 80% identical to a second sequence as shown in Table 10.

53. A method according to claim 52, wherein the plurality of polynucleotides comprises a third polynucleotide that selectively hybridizes to a sequence at least 80% identical to a third sequence as shown in Table 10.

54. A method of detecting a prostate cancer associated transcript, the method comprising contacting a biological sample from the patient with a plurality of polynucleotides wherein at least two of said polynucleotides selectively hybridize to a difference sequence at least 80% identical to a sequence as shown in Table 10.

55. A method of detecting a prostate cancer, the method comprising the steps of:
(i) providing a biological sample from a patient;
(ii) contacting the biological sample with a first polynucleotide that selectively hybridizes to a sequence at least 80% identical to a first sequence as shown in Table 10 to determine the level of a prostate cancer-associated transcript in the biological sample; and with a second polynucleotide that selectively hybridizes to a second sequence at least 80% identical to a sequence not shown in Table 10; wherein the expression of said second sequence is not substantially changed in prostate cancer, to determine the level of expression of a control transcript in the biological sample;
(iii) comparing the level of the prostate cancer-associated transcript to a level of the normal tissue associated transcript in the biological sample.

56. A method of quantitating a prostate cancer-associated transcript in a cell from a patient, the method comprising contacting a biological sample from the patient with a polynucleotide that selectively hybridizes to a sequence at least 80% identical to a sequence as shown in Table 10.

57. The method of claim 56, wherein the polynucleotide selectively hybridizes to a sequence at least 95% identical to a sequence as shown in Table 10.

58. The method of claim 56, wherein the biological sample is a tissue sample.

59. The method of claim 56, wherein the biological sample comprises isolated nucleic acids.

60. The method of claim 56, wherein the nucleic acids are mRNA.

61. The method of claim 59, further comprising the step of amplifying nucleic acids before the step of contacting the biological sample with the polynucleotide.

62. The method of claim 56, wherein the polynucleotide comprises a sequence as shown in Table 10.

63. The method of claim 56, wherein the polynucleotide is labeled.

64. The method of claim 63, wherein the label is a fluorescent label.

65. The method of claim 56, wherein the polynucleotide is immobilized on a solid surface.

66. The method of claim 56, wherein the patient is undergoing a therapeutic regimen to treat metastatic prostate cancer.

67. The method of claim 56, wherein the patient is suspected of having metastatic prostate cancer.

68. A biochip comprising a plurality of polynucleotides that selectively hybridize to a sequence at least 80% identical to a sequence as shown in Table 10.

69. A method of screening drug candidates comprising:
i) providing a cell that expresses an expression profile gene selected from the group consisting of an expression profile gene set forth in Table 10 or fragment thereof;
ii) adding a drug candidate to said cell; and
ii) determining the effect of said drug candidate on the expression of said expression profile gene.

70. A method according to claim 59 wherein said determining comprises comparing the level of expression in the absence of said drug candidate to the level of expression in the presence of said drug candidate.

71. A method of detecting a prostate cancer-associated transcript in a cell from a patient, the method comprising contacting a biological sample from the patient with a polynucleotide that selectively hybridizes to a sequence at least 80% identical to a sequence as shown in Tables 1-16.

72. The method of claim 71 as defined by any one of claims 2-12, 52 or 53.

73. A method of monitoring the efficacy of a therapeutic treatment of prostate cancer, the method comprising the steps of:
(i) providing a biological sample from a patient undergoing the therapeutic treatment; and
(ii) determining the level of a prostate cancer-associated transcript in the biological sample by contacting the biological sample with a polynucleotide that selectively hybridizes to a sequence at least 80% identical to a sequence as shown in Tables 1-16, thereby monitoring the efficacy of the therapy.

74. The method of claim 73 as defined by any one of claims 14 or 15.

75. A method of monitoring the efficacy of a therapeutic treatment of prostate cancer, the method comprising the steps of:
(i) providing a biological sample from a patient undergoing the therapeutic treatment; and
(ii) determining the level of a prostate cancer-associated antibody in the biological sample by contacting the biological sample with a polypeptide encoded by a polynucleotide that selectively hybridizes to a sequence at least 80% identical to a sequence as shown in Tables 1-16, wherein the polypeptide specifically binds to the prostate cancer-associated antibody, thereby monitoring the efficacy of the therapy.

76. The method of claim 75 as defined by any one of claims 17 or 18.

77. A method of monitoring the efficacy of a therapeutic treatment of prostate cancer, the method comprising the steps of:
(i) providing a biological sample from a patient undergoing the therapeutic treatment; and
(ii) determining, the level of a prostate cancer-associated polypeptide in the biological sample by contacting the biological sample with an antibody, wherein the antibody specifically binds to a polypeptide encoded by a polynucleotide that selectively hybridizes to a sequence at least 80% identical to a sequence as shown in Tables 1-16, thereby monitoring the efficacy of the therapy.

78. The method of claim 77 as defined by any one of claims 20 or 21.

79. An isolated nucleic acid molecule consisting of a polynucleotide sequence as shown in Tables 1-16.

80. The nucleic acid of claim 79 as defined by any one of claims 23 or 24.

81. An expression vector comprising the nucleic acid of claim 79.

82. A host cell comprising the expression vector of claim 79.

83. An isolated polypeptide which is encoded by a nucleic acid molecule having polynucleotide sequence as shown in Tables 1-16.

84. An antibody that specifically binds a polypeptide of claim 83.

85. The antibody of claim 84 as defined by any one of claims 29-33.

86. A method of detecting a prostate cancer cell in a biological sample from a patient, the method comprising contacting the biological sample with an antibody of claim 84.

87. The method of claim 86 as defined by any one of claims 35 and 36.

88. A method of detecting antibodies specific to prostate cancer in a patient, the method comprising contacting a biological sample from the patient with a polypeptide encoded by a nucleic acid comprises a sequence from Tables 1-16.

89. A method for identifying a compound that modulates a prostate cancer associated polypeptide, the method comprising the steps of:
(i) contacting the compound with a prostate cancer-associated polypeptide, the polypeptide encoded by a polynucleotide that selectively hybridizes to a sequence at least 80% identical to a sequence as shown in Tables 1-16; and
(ii) determining the functional effect of the compound upon the polypeptide.

90. The method of claim 89 as defined by claims 39-43.

91. A method of inhibiting proliferation of a prostate cancer-associated cell to treat prostate cancer in a patient, the method comprising the step of administering to the subject a therapeutically effective amount of a compound identified using the method of claim 89.

92. The method of claim 91 as defined by any one of claims 45 or 46.

93. A drug screening assay comprising the steps of
(i) administering a test compound to a mammal having prostate cancer or a cell isolated therefrom;
(ii) comparing the level of gene expression of a polynucleotide that selectively hybridizes to a sequence at least 80% identical to a sequence as shown in Tables 1-16 in a treated cell or mammal with the level of gene expression of the polynucleotide in a control cell or mammal, wherein a test compound that modulates the level of expression of the polynucleotide is a candidate for the treatment of prostate cancer.

94. The method of claim 93 as defined by any one of claims 48 or 49.

95. A method for treating a mammal having prostate cancer comprising administering a compound identified by the assay of claim 93.

96. A pharmaceutical composition for treating a mammal having prostate cancer, the composition comprising a compound identified by the assay of claim 93 and a physiologically acceptable excipient.

97. A method of detecting a prostate cancer-associated transcript, the method comprising contacting a biological sample from the patient with a plurality of polynucleotides wherein at least two of said polynucleotides selectively hybridize to a difference sequence at least 80% identical to a sequence as shown in Tables 1-16.

98. A method of detecting a prostate cancer, the method comprising the steps of:
(i) providing a biological sample from a patient;
(ii) contacting the biological sample with a first polynucleotide that selectively hybridizes to a sequence at least 80% identical to a first sequence as shown in Tables 1-16 to determine the level of a prostate cancer-associated transcript in the biological sample; and with a second polynucleotide that selectively hybridizes to a second sequence at least 80% identical to a sequence not shown in Tables 1-16; wherein the expression of said second sequence is not substantially changed in prostate cancer, to determine the level of expression of a control transcript in the biological sample;
(iii) comparing the level of the prostate cancer-associated transcript to a level of the normal tissue associated transcript in the biological sample.

99. A method of quantitating a prostate cancer-associated transcript in a cell from a patient, the method comprising contacting a biological sample from the patient with a polynucleotide that selectively hybridizes to a sequence at least 80% identical to a sequence as shown in Tables 1-16.

100. The method of claim 99 as defined by claims 57-67 or 70.

101. A biochip comprising a plurality of polynucleotides that selectively hybridize to a sequence at least 80% identical to a sequence as shown in Tables 1-16.

102. A method of screening drug candidates comprising:
i) providing a cell that expresses an expression profile gene selected from the group consisting of an expression profile gene set forth in Tables 1-16 or fragment thereof;
ii) adding a drug candidate to said cell; and
ii) determining the effect of said drug candidate on the expression of said expression profile gene.


### Annex to the application documents - subsequently filed sequences listing

SEQUENCE LISTING

<110> Cedars Sinai Medical Center & Garvan Institute of Medical Research

<120> Methods of Diagnosis of Prostate Cancer, Compositions and Methods
of Screening for Modulators of Prostate Cancer

<130> PCB/P05817PEP

<140> EP 07075380.1
<141> 2007-05-21

<150> PCT/US01/32045
<151> 2001-10-12

<150> US 09/687,576
<151> 2000-10-13

<150> US 09/733,288
<151> 2000-12-08

<150> US 09/733,742
<151> 2000-12-08

<150> US 60/263,957
<151> 2001-01-24

<150> US 60/276,791
<151> 2001-03-16

<150> US 60/276,888
<151> 2001-03-16

<150> US 60/281,922
<151> 2001-04-06

<150> US 60/286,214
<151> 2001-04-24

<150> US 09/847,046
<151> 2001-04-30

<150> US 60/288,589
<151> 2001-05-04

<160> 296

<170> PatentIn version 3.3

<210> 1
<211> 4793
<212> DNA
<213> Homo sapiens

<400> 1
attggatcaa acatgtcaca agagtcggac aataataaaa gactagtggc cttagtgccc 60

atgcccagtg accctccatt caatacccga agagcctaca ccagtgagga tgaagcctgg 120

aagtcatact tggagaatcc cctgacagca gccaccaagg ccatgatgat cattaatggt 180

gatgaggaca gtgctgctgc cctcggcctg ctctatgact actacaaggt tcctcgagac 240

aagaggctgc tgtctgtaag caaagcaagt gacagccaag aagaccagga gaaaagaaac 300

tgccttggca ccagtgaagc ccagagtaat ttgagtggag gagaaaaccg agtgcaagtc 360

ctaaagactg ttccagtgaa cctttcccta aatcaagatc acctggagaa ttccaagcgg 420

gaacagtaca gcatcagctt ccccgagagc tctgccatca tcccggtgtc gggaatcacg 480
gtggtgaaag ctgaagattt cacaccagtt ttcatggccc cacctgtgca ctatccccgg 540
ggagatgggg aagagcaacg agtggttatc tttgaacaga ctcagtatga cgtgccctcg 600
ctggccaccc acagcgccta tctcaaagac gaccagcgca gcactccgga cagcacatac 660
agcgagagct tcaaggacgc agccacagag aaatttcgga gtgcttcagt tggggctgag 720
gagtacatgt atgatcagac atcaagtggc acatttcagt acaccctgga agccaccaaa 780
tctctccgtc agaagcaggg ggagggcccc atgacctacc tcaacaaagg acagttctat 840
gccataacac tcagcgagac cggagacaac aaatgcttcc gacaccccat cagcaaagtc 900
aggagtgtgg tgatggtggt cttcagtgaa gacaaaaaca gagatgaaca gctcaaatac 960
tggaaatact ggcactctcg gcagcatacg gcgaagcaga gggtccttga cattgccgat 1020
tacaaggaga gctttaatac gattggaaac attgaagaga ttgcatataa tgctgtttcc 1080
tttacctggg acgtgaatga agaggcgaag attttcatca ccgtgaattg cttgagcaca 1140
gatttctcct cccaaaaagg ggtgaaagga cttcctttga tgattcagat tgacacatac 1200
agttataaca atcgtagcaa taaacccatt catagagctt attgccagat caaggtcttc 1260
tgtgacaaag gagcagaaag aaaaatccga gatgaagagc agaagcagaa caggaagaac 1320
gggaaaggcc aggcctccca aactcaatgc aacagctcct ctgatgggaa gttggctgcc 1380
atacctttac agaagaagag tgacatcacc tacttcaaaa ccatgcctga tctccactca 1440
cagccagttc tcttcatacc tgatgttcac tttgcaaacc tgcagaggac cggacaggtg 1500
tattacaaca cggatgatga acgagaaggt ggcagtgtcc ttgttaaacg gatgttccgg 1560
cccatggaag aggagtttgg tccggtgcct tcaaagcaga tgaaagaaga agggacaaag 1620
cgagtgctct tgtacgtgag gaaggagact gacgatgtgt tcgatgcatt gatgttgaag 1680
tctcccacag tgatgggcct gatggaagcg atatctgaga aatatgggct gcccgtggag 1740
aagatagcaa agctttacaa gaaaagcaaa aaaggcatct tggtgaacat ggatgacaac 1800
atcatcgagc actactcgaa cgaggacacc ttcatcctca acatggagag catggtggag 1860
ggcttcaagg tcacgctcat ggaaatctag ccctgggttt ggcatccgct ttggctggag 1920
ctctcagtgc gttcctccct gagagagaca gaagccccag ccccagaacc tggagaccca 1980
tctcccccat ctcacaactg ctgttacaag accgtgctgg ggagtggggc aagggacagg 2040
ccccacagtc ggtgtgcttg gcccatccac tggcacctac cacggagccg aagcctgagc 2100
ccctcaggaa ggtgccttag gcctgttgga ttcctattta ttgcccacct tttcctggag 2160
cccaggtcca ggcccgccag gactctgcag gtcactgcta gctccagatg agaccgtcca 2220
gcgttccccc ttcaagagaa acactcatcc cgaacagcct aaaaaattcc catcccttct 2280
ttctcacccc tccatatcta tatctcccga gtggctggac aaaatgagct acgtctgggt 2340
gcagtagtta taggtggggc aagaggtgga tgcccacttt ctggtcagac acctttaggt 2400
tgctctgggg aaggctgtct tgctaaatac ctccagggtt cccagcaagt ggccaccagg 2460

ccttgtacag gaagacattc agtcaccgtg taattagtaa cacagaaagt ctgcctgtct 2520
gcattgtaca tagtgtttat aatattgtaa taatatattt tacctgtggt atgtgggcat 2580
gtttactgcc actggcctag aggagacaca gacctggaga ccgttttaat gggggttttt 2640
gcctctgtgc ctgttcaaga gacttgcagg gctaggtaga gggcctttgg gatgttaagg 2700
tgactgcagc tgatgccaag atggactctg caatgggcat acctgggggc tcgttccctg 2760
tccccagagg aagccccctc tccttctcca tgggcatgac tctccttcga ggccaccacg 2820
tttatctcac aatgatgtgt tttgcctgac tttccctttg cgctgtctcg tgggaaaggt 2880
cattctgtct gagaccccag ctccttctcc agctttggct gcgggcatgg cctgagcttt 2940
ctggagagcc tctgcagggg gtttgccatc agggccctgt ggctgggtct gctgcagagc 3000
tccttggcta tcaggagaat cctggacact gtactgtgcc tcccagttta caaacacgcc 3060
cttcatctca agtggccctt taaaaggcct gctgccatgt gagagctgtg aacagctcag 3120
ctctgagtcg gcagactggg gcttcctcct gggccaccag atggaaaggg ggtattgttt 3180
gcctcactcc tggatgctgc gttttaagga agtgagtgag aaagaatgtg ccaagatacc 3240
tggctcctgt gaaaccagcc tcaggaggga aactgggaga gagaagctgt ggtctcctgc 3300
tacatgccct gggagctgga agagaaaaac actcccctaa acaatcgcaa aatgatgaac 3360
catcatgggc cactgttctc tttgagggga caggtttagg ggtttgcgtt cgcccttgtg 3420
ggctgaagca ctagcttttt ggtagctaga cacatcctgc acccaaaggt tctctacaaa 3480
ggcccagatt tgtttgtaaa gcactttgac tcttacctgg aggcccgctc tctaagggct 3540
tcctgcgctc ccacctcatc tgtccctgag atgcagagca ggatggaggg tctgcttcta 3600
gctcagctgt ttctccttga ggttgcggag gaattgaatt gaatgggaca gagggcaggt 3660
gctgtggcca agaagatctc cgagcagcag tgacggggca ccttgctgtg tgtcctctgg 3720
gcatgttaac ccttctgtgg ggccaaaggt ttgcatcgtg gatccagctg tgctccagtc 3780
tgtcccctcc tcctccactc tgactgccac gccccggacc agcagcttgg ggaccctcca 3840
gggtactaat ggggctctgt tctgagatgg acaaattcag tgttggaaat acatgttgta 3900
ctatgcactt cccatgctcc tagggttagg aatagtttca aacatgattg gcagacataa 3960
caacggcaaa tactcggact ggggcatagg actccagagt aggaaaaaga caaaagattt 4020
ggcagcctga cacaggcaac ctacccctct ctctccagcc tctttatgaa actgtttgtt 4080
tgccagtcct gccctaaggc agaagatgaa ttgaagatgc tgtgcatgtt tcctaagtcc 4140
ttgagcaatc atggtggtga caattgccac aagggatatg aggccagtgc caccagaggg 4200
tggtgccaag tgccacatcc cttccgatcc attcccctct gtatcctcgg agcaccccag 4260
tttgcctttg atgtgtccgc tgtgtatgtt agctgaactt tgatgagcaa aatttcctga 4320
gcgaaacact ccaaagagat aggaaaactt gccgcctctt cttttttgtc ccttaatcaa 4380
actcaaataa gcttaaaaaa aatccatgga agatcatgga catgtgaaat gagcattttt 4440
ttcttttctt tttttttttt tttttttaac aaagtctgaa ctgaacagaa caagactttt 4500

tcctcataca tctccaaatt gtttaaactt actttatgag tgtttgttta gaagttcgga 4560

ccaacagaaa aatgcagtca gatgtcatct tggaattggt ttctaaaaga gtaaggcatg 4620

tccctgccca gaaacttagg aagcatgaaa taaatcaaat gtttattttc cttcttattt 4680

aaaatcatgc taatgcaaca gaaatagagg gtttgtgcca aatgctatga acggcccttt 4740

cttaaagaca agcaagggag attgatatat gtacaatttg ctctcatgtt ttt 4793

<210> 2
<211> 625
<212> PRT
<213> Homo sapiens

<400> 2

Met Ser Gln Glu Ser Asp Asn Asn Lys Arg Leu Val Ala Leu Val Pro
1               5                   10                  15

Met Pro Ser Asp Pro Pro Phe Asn Thr Arg Arg Ala Tyr Thr Ser Glu
            20                  25                  30

Asp Glu Ala Trp Lys Ser Tyr Leu Glu Asn Pro Leu Thr Ala Ala Thr
        35                  40                  45

Lys Ala Met Met Ile Ile Asn Gly Asp Glu Asp Ser Ala Ala Ala Leu
    50                  55                  60

Gly Leu Leu Tyr Asp Tyr Tyr Lys Val Pro Arg Asp Lys Arg Leu Leu
65                  70                  75                  80

Ser Val Ser Lys Ala Ser Asp Ser Gln Glu Asp Gln Glu Lys Arg Asn
                85                  90                  95

Cys Leu Gly Thr Ser Glu Ala Gln Ser Asn Leu Ser Gly Gly Glu Asn
            100                 105                 110

Arg Val Gln Val Leu Lys Thr Val Pro Val Asn Leu Ser Leu Asn Gln
        115                 120                 125

Asp His Leu Glu Asn Ser Lys Arg Glu Gln Tyr Ser Ile Ser Phe Pro
    130                 135                 140

Glu Ser Ser Ala Ile Ile Pro Val Ser Gly Ile Thr Val Val Lys Ala
145                 150                 155                 160

Glu Asp Phe Thr Pro Val Phe Met Ala Pro Pro Val His Tyr Pro Arg
                165                 170                 175

Gly Asp Gly Glu Glu Gln Arg Val Val Ile Phe Glu Gln Thr Gln Tyr
            180                 185                 190

Asp Val Pro Ser Leu Ala Thr His Ser Ala Tyr Leu Lys Asp Asp Gln
        195                 200                 205

Arg Ser Thr Pro Asp Ser Thr Tyr Ser Glu Ser Phe Lys Asp Ala Ala
    210                 215                 220

Thr Glu Lys Phe Arg Ser Ala Ser Val Gly Ala Glu Glu Tyr Met Tyr
225                 230                 235                 240

Asp Gln Thr Ser Ser Gly Thr Phe Gln Tyr Thr Leu Glu Ala Thr Lys
                245                 250                 255

Ser Leu Arg Gln Lys Gln Gly Glu Gly Pro Met Thr Tyr Leu Asn Lys
            260                 265                 270

Gly Gln Phe Tyr Ala Ile Thr Leu Ser Glu Thr Gly Asp Asn Lys Cys
        275                 280                 285

Phe Arg His Pro Ile Ser Lys Val Arg Ser Val Val Met Val Val Phe
    290                 295                 300

Ser Glu Asp Lys Asn Arg Asp Glu Gln Leu Lys Tyr Trp Lys Tyr Trp
305                 310                 315                 320

His Ser Arg Gln His Thr Ala Lys Gln Arg Val Leu Asp Ile Ala Asp
                325                 330                 335

Tyr Lys Glu Ser Phe Asn Thr Ile Gly Asn Ile Glu Glu Ile Ala Tyr
            340                 345                 350

Asn Ala Val Ser Phe Thr Trp Asp Val Asn Glu Glu Ala Lys Ile Phe
        355                 360                 365

Ile Thr Val Asn Cys Leu Ser Thr Asp Phe Ser Ser Gln Lys Gly Val
    370                 375                 380

Lys Gly Leu Pro Leu Met Ile Gln Ile Asp Thr Tyr Ser Tyr Asn Asn
385                 390                 395                 400

Arg Ser Asn Lys Pro Ile His Arg Ala Tyr Cys Gln Ile Lys Val Phe
                405                 410                 415

Cys Asp Lys Gly Ala Glu Arg Lys Ile Arg Asp Glu Glu Gln Lys Gln
            420                 425                 430

Asn Arg Lys Asn Gly Lys Gly Gln Ala Ser Gln Thr Gln Cys Asn Ser
        435                 440                 445

Ser Ser Asp Gly Lys Leu Ala Ala Ile Pro Leu Gln Lys Lys Ser Asp
    450                 455                 460

Ile Thr Tyr Phe Lys Thr Met Pro Asp Leu His Ser Gln Pro Val Leu
465                 470                 475                 480

Phe Ile Pro Asp Val His Phe Ala Asn Leu Gln Arg Thr Gly Gln Val
                485                 490                 495

Tyr Tyr Asn Thr Asp Asp Glu Arg Glu Gly Gly Ser Val Leu Val Lys
            500                 505                 510

Arg Met Phe Arg Pro Met Glu Glu Glu Phe Gly Pro Val Pro Ser Lys
        515                 520                 525

Gln Met Lys Glu Glu Gly Thr Lys Arg Val Leu Leu Tyr Val Arg Lys
    530                 535                 540

Glu Thr Asp Asp Val Phe Asp Ala Leu Met Leu Lys Ser Pro Thr Val
545                 550                 555                 560

Met Gly Leu Met Glu Ala Ile Ser Glu Lys Tyr Gly Leu Pro Val Glu
                565                 570                 575

Lys Ile Ala Lys Leu Tyr Lys Lys Ser Lys Lys Gly Ile Leu Val Asn
            580                 585                 590

Met Asp Asp Asn Ile Ile Glu His Tyr Ser Asn Glu Asp Thr Phe Ile
        595                 600                 605

Leu Asn Met Glu Ser Met Val Glu Gly Phe Lys Val Thr Leu Met Glu
    610                 615                 620

Ile

625

<210> 3
<211> 777
<212> DNA
<213> Homo sapiens

<400> 3
atgatagcaa tctctgccgt cagcagtgca ctcctgttct cccttctctg tgaagcaagt 60

accgtcgtcc tactcaattc cactgactca tccccgccaa ccaataattt cactgatatt 120

gaagcagctc tgaaagcaca attagattca gcggatatcc ccaaagccag gcggaagcgc 180

tacatttcgc agaatgacat gatcgccatt cttgattatc ataatcaagt tcggggcaaa 240

gtgttcccac cggcagcaaa tatggaatat atggtttggg atgaaaatct tgcaaaatcg 300

gcagaggctt gggcggctac ttgcatttgg gaccatggac cttcttactt actgagattt 360

ttgggccaaa atctatctgt acgcactgga agatatcgct ctattctcca gttggtcaag 420

ccatggtatg atgaagtgaa agattatgct tttccatatc cccaggattg caaccccaga 480

tgtcctatga gatgttttgg tcccatgtgc acacattata cgcagatggt ttgggccact 540

tccaatcgga taggatgcgc aattcatgct tgccaaaaca tgaatgtttg gggatctgtg 600

tggcgacgtg cagtttactt ggtatgcaac tatgccccaa agggcaattg gattggagaa 660

gcaccatata aagtaggggt accatgttca tcttgtcctc caagttatgg gggatcttgt 720

actgacaatc tgtgttttcc aggagttacg tcaaactacc tgtactggtt taaataa 777

<210> 4
<211> 777
<212> DNA
<213> Homo sapiens

<400> 4
atgatagcaa tctctgccgt cagcagtgca ctcctgttct cccttctctg tgaagcaagt 60

accgtcgtcc tactcaattc cactgactca tccccgccaa ccaataattt cactgatatt 120

gaagcagctc tgaaagcaca attagattca gcggatatcc ccaaagccag gcggaagcgc 180

tacatttcgc agaatgacat gatcgccatt cttgattatc ataatcaagt tcggggcaaa 240

gtgttcccac cggcagcaaa tatggaatat atggtttggg atgaaaatct tgcaaaatcg 300

gcagaggctt gggcggctac ttgcatttgg gaccatggac cttcttactt actgagattt 360

ttgggccaaa atctatctgt acgcactgga agatatcgct ctattctcca gttggtcaag 420

ccatggtatg atgaagtgaa agattatgct tttccatatc cccaggattg caaccccaga 480

tgtcctatga gatgttttgg tcccatgtgc acacattata cgcagatggt ttgggccact 540

tccaatcgga taggatgcgc aattcatact tgccaaaaca tgaatgtttg gggatctgtg 600

tggcgacgtg cagtttactt ggtatgcaac tatgccccaa agggcaattg gattggagaa 660

gcaccatata aagtaggggt accatgttca tcttgtcctc caagttatgg gggatcttgt 720

actgacaatc tgtgttttcc aggagttacg tcaaactacc tgtactggtt taaataa 777

<210> 5
<211> 258

<212>   PRT
<213>   Homo sapiens

<400>   5

Met Ile Ala Ile Ser Ala Val Ser Ser Ala Leu Leu Phe Ser Leu Leu
1               5                   10                  15

Cys Glu Ala Ser Thr Val Val Leu Leu Asn Ser Thr Asp Ser Ser Pro
            20                  25                  30

Pro Thr Asn Asn Phe Thr Asp Ile Glu Ala Ala Leu Lys Ala Gln Leu
        35                  40                  45

Asp Ser Ala Asp Ile Pro Lys Ala Arg Arg Lys Arg Tyr Ile Ser Gln
    50                  55                  60

Asn Asp Met Ile Ala Ile Leu Asp Tyr His Asn Gln Val Arg Gly Lys
65                  70                  75                  80

Val Phe Pro Pro Ala Ala Asn Met Glu Tyr Met Val Trp Asp Glu Asn
                85                  90                  95

Leu Ala Lys Ser Ala Glu Ala Trp Ala Ala Thr Cys Ile Trp Asp His
            100                 105                 110

Gly Pro Ser Tyr Leu Leu Arg Phe Leu Gly Gln Asn Leu Ser Val Arg
        115                 120                 125

Thr Gly Arg Tyr Arg Ser Ile Leu Gln Leu Val Lys Pro Trp Tyr Asp
    130                 135                 140

Glu Val Lys Asp Tyr Ala Phe Pro Tyr Pro Gln Asp Cys Asn Pro Arg
145                 150                 155                 160

Cys Pro Met Arg Cys Phe Gly Pro Met Cys Thr His Tyr Thr Gln Met
                165                 170                 175

Val Trp Ala Thr Ser Asn Arg Ile Gly Cys Ala Ile His Ala Cys Gln
            180                 185                 190

Asn Met Asn Val Trp Gly Ser Val Trp Arg Arg Ala Val Tyr Leu Val
        195                 200                 205

Cys Asn Tyr Ala Pro Lys Gly Asn Trp Ile Gly Glu Ala Pro Tyr Lys
    210                 215                 220

Val Gly Val Pro Cys Ser Ser Cys Pro Pro Ser Tyr Gly Gly Ser Cys
225                 230                 235                 240

Thr Asp Asn Leu Cys Phe Pro Gly Val Thr Ser Asn Tyr Leu Tyr Trp
                245                 250                 255

Phe Lys


<210>   6
<211>   258
<212>   PRT
<213>   Homo sapiens

<400>   6

Met Ile Ala Ile Ser Ala Val Ser Ser Ala Leu Leu Phe Ser Leu Leu
1               5                   10                  15

Cys Glu Ala Ser Thr Val Val Leu Leu Asn Ser Thr Asp Ser Ser Pro

            20                  25                  30

Pro Thr Asn Asn Phe Thr Asp Ile Glu Ala Ala Leu Lys Ala Gln Leu
        35                  40                  45

Asp Ser Ala Asp Ile Pro Lys Ala Arg Arg Lys Arg Tyr Ile Ser Gln
    50                  55                  60

Asn Asp Met Ile Ala Ile Leu Asp Tyr His Asn Gln Val Arg Gly Lys
65                  70                  75                  80

Val Phe Pro Pro Ala Ala Asn Met Glu Tyr Met Val Trp Asp Glu Asn
                85                  90                  95

Leu Ala Lys Ser Ala Glu Ala Trp Ala Ala Thr Cys Ile Trp Asp His
            100                 105                 110

Gly Pro Ser Tyr Leu Leu Arg Phe Leu Gly Gln Asn Leu Ser Val Arg
        115                 120                 125

Thr Gly Arg Tyr Arg Ser Ile Leu Gln Leu Val Lys Pro Trp Tyr Asp
    130                 135                 140

Glu Val Lys Asp Tyr Ala Phe Pro Tyr Pro Gln Asp Cys Asn Pro Arg
145                 150                 155                 160

Cys Pro Met Arg Cys Phe Gly Pro Met Cys Thr His Tyr Thr Gln Met
                165                 170                 175

Val Trp Ala Thr Ser Asn Arg Ile Gly Cys Ala Ile His Thr Cys Gln
            180                 185                 190

Asn Met Asn Val Trp Gly Ser Val Trp Arg Arg Ala Val Tyr Leu Val
        195                 200                 205

Cys Asn Tyr Ala Pro Lys Gly Asn Trp Ile Gly Glu Ala Pro Tyr Lys
    210                 215                 220

Val Gly Val Pro Cys Ser Ser Cys Pro Pro Ser Tyr Gly Gly Ser Cys
225                 230                 235                 240

Thr Asp Asn Leu Cys Phe Pro Gly Val Thr Ser Asn Tyr Leu Tyr Trp
                245                 250                 255

Phe Lys

<210> 7
<211> 2820
<212> DNA
<213> Homo sapiens

<400> 7
ggcgggttcg cgccccgaag gctgagagct ggcgctgctc gtgccctgtg tgccagacgg 60

cggagctccg cggccggacc ccgcggcccc gctttgctgc cgactggagt ttgggggaag 120

aaactctcct gcgccccaga agatttcttc ctcggcgaag ggacagcgaa agatgagggt 180

ggcaggaaga gaaggcgctt tctgtctgcc ggggtcgcag cgcgagaggg cagtgccatg 240

ttcctctcca tcctagtggc gctgtgcctg tggctgcacc tggcgctggg cgtgcgcggc 300

gcgccctgcg aggcggtgcg catccctatg tgccggcaca tgccctggaa catcacgcgg 360

atgcccaacc acctgcacca cagcacgcag gagaacgcca tcctggccat cgagcagtac 420

gaggagctgg tggacgtgaa ctgcagcgcc gtgctgcgct tcttcttctg tgccatgtac 480

gcgcccattt gcaccctgga gttcctgcac gaccctatca agccgtgcaa gtcggtgtgc 540
caacgcgcgc gcgacgactg cgagcccctc atgaagatgt acaaccacag ctggcccgaa 600
agcctggcct gcgacgagct gcctgtctat gaccgtggcg tgtgcatttc gcctgaagcc 660
atcgtcacgg acctcccgga ggatgttaag tggatagaca tcacaccaga catgatggta 720
caggaaaggc ctcttgatgt tgactgtaaa cgcctaagcc ccgatcggtg caagtgtaaa 780
aaggtgaagc caactttggc aacgtatctc agcaaaaact acagctatgt tattcatgcc 840
aaaataaaag ctgtgcagag gagtggctgc aatgaggtca caacggtggt ggatgtaaaa 900
gagatcttca agtcctcatc acccatccct cgaactcaag tcccgctcat tacaaattct 960
tcttgccagt gtccacacat cctgccccat caagatgttc tcatcatgtg ttacgagtgg 1020
cgttcaagga tgatgcttct tgaaaattgc ttagttgaaa aatggagaga tcagcttagt 1080
aaaagatcca tacagtggga agagaggctg caggaacagc ggagaacagt tcaggacaag 1140
aagaaaacag ccgggcgcac cagtcgtagt aatcccccca aaccaaaggg aaagcctcct 1200
gctcccaaac cagccagtcc caagaagaac attaaaacta ggagtgccca gaagagaaca 1260
aacccgaaaa gagtgtgagc taactagttt ccaaagcgga gacttccgac ttccttacag 1320
gatgaggctg ggcattgcct gggacagcct atgtaaggcc atgtgcccct tgccctaaca 1380
actcactgca gtgctcttca tagacacatc ttgcagcatt tttcttaagg ctatgcttca 1440
gtttttcttt gtaagccatc acaagccata gtggtaggtt tgccctttgg tacagaaggt 1500
gagttaaagc tggtggaaaa ggcttattgc attgcattca gagtaacctg tgtgcatact 1560
ctagaagagt agggaaaata atgcttgtta caattcgacc taatatgtgc attgtaaaat 1620
aaatgccata tttcaaacaa aacacgtaat ttttttacag tatgttttat taccttttga 1680
tatctgttgt tgcaatgtta gtgatgtttt aaaatgtgat gaaaatataa tgtttttaag 1740
aaggaacagt agtggaatga atgttaaaag atctttatgt gtttatggtc tgcagaagga 1800
tttttgtgat gaaaggggat tttttgaaaa attagagaag tagcatatgg aaaattataa 1860
tgtgtttttt taccaatgac ttcagtttct gtttttagct agaaacttaa aaacaaaaat 1920
aataataaag aaaaataaat aaaaaggaga ggcagacaat gtctggattc ctgttttttg 1980
gttacctgat ttccatgatc atgatgcttc ttgtcaacac cctcttaagc agcaccagaa 2040
acagtgagtt tgtctgtacc attaggagtt aggtactaat tagttggcta atgctcaagt 2100
attttatacc cacaagagag gtatgtcact catcttactt cccaggacat ccaccctgag 2160
aataatttga caagcttaaa aatggccttc atgtgagtgc caaattttgt ttttcttcat 2220
ttaaatattt tctttgccta aatacatgtg agaggagtta aatataaatg tacagagagg 2280
aaagttgagt tccacctctg aaatgagaat tacttgacag ttgggatact ttaatcagaa 2340
aaaaagaact tatttgcagc attttatcaa caaatttcat aattgtggac aattggaggc 2400
atttatttta aaaaacaatt ttattggcct tttgctaaca cagtaagcat gtattttata 2460
aggcattcaa taaatgcaca acgcccaaag gaaataaaat cctatctaat cctactctcc 2520

actacacaga ggtaatcact attagtattt tggcatatta ttctccaggt gtttgcttat 2580

gcacttataa aatgatttga acaaataaaa ctaggaacct gtatacatgt gtttcataac 2640

ctgcctcctt tgcttggccc tttattgaga taagttttcc tgtcaagaaa gcagaaacca 2700

tctcatttct aacagctgtg ttatattcca tagtatgcat tactcaacaa actgttgtgc 2760

tattggatac ttaggtggtt tcttcactga caatactgaa taaacatctc accggaattc 2820

<210> 8
<211> 346
<212> PRT
<213> Homo sapiens

<400> 8

Met Phe Leu Ser Ile Leu Val Ala Leu Cys Leu Trp Leu His Leu Ala
1               5                   10                  15

Leu Gly Val Arg Gly Ala Pro Cys Glu Ala Val Arg Ile Pro Met Cys
            20                  25                  30

Arg His Met Pro Trp Asn Ile Thr Arg Met Pro Asn His Leu His His
        35                  40                  45

Ser Thr Gln Glu Asn Ala Ile Leu Ala Ile Glu Gln Tyr Glu Glu Leu
    50                  55                  60

Val Asp Val Asn Cys Ser Ala Val Leu Arg Phe Phe Phe Cys Ala Met
65                  70                  75                  80

Tyr Ala Pro Ile Cys Thr Leu Glu Phe Leu His Asp Pro Ile Lys Pro
                85                  90                  95

Cys Lys Ser Val Cys Gln Arg Ala Arg Asp Asp Cys Glu Pro Leu Met
            100                 105                 110

Lys Met Tyr Asn His Ser Trp Pro Glu Ser Leu Ala Cys Asp Glu Leu
        115                 120                 125

Pro Val Tyr Asp Arg Gly Val Cys Ile Ser Pro Glu Ala Ile Val Thr
    130                 135                 140

Asp Leu Pro Glu Asp Val Lys Trp Ile Asp Ile Thr Pro Asp Met Met
145                 150                 155                 160

Val Gln Glu Arg Pro Leu Asp Val Asp Cys Lys Arg Leu Ser Pro Asp
                165                 170                 175

Arg Cys Lys Cys Lys Lys Val Lys Pro Thr Leu Ala Thr Tyr Leu Ser
            180                 185                 190

Lys Asn Tyr Ser Tyr Val Ile His Ala Lys Ile Lys Ala Val Gln Arg
        195                 200                 205

Ser Gly Cys Asn Glu Val Thr Thr Val Val Asp Val Lys Glu Ile Phe
    210                 215                 220

Lys Ser Ser Ser Pro Ile Pro Arg Thr Gln Val Pro Leu Ile Thr Asn
225                 230                 235                 240

Ser Ser Cys Gln Cys Pro His Ile Leu Pro His Gln Asp Val Leu Ile
                245                 250                 255

Met Cys Tyr Glu Trp Arg Ser Arg Met Met Leu Leu Glu Asn Cys Leu
            260                 265                 270

Val Glu Lys Trp Arg Asp Gln Leu Ser Lys Arg Ser Ile Gln Trp Glu
        275                 280                 285

Glu Arg Leu Gln Glu Gln Arg Arg Thr Val Gln Asp Lys Lys Lys Thr
    290                 295                 300

Ala Gly Arg Thr Ser Arg Ser Asn Pro Pro Lys Pro Lys Gly Lys Pro
305                 310                 315                 320

Pro Ala Pro Lys Pro Ala Ser Pro Lys Lys Asn Ile Lys Thr Arg Ser
                325                 330                 335

Ala Gln Lys Arg Thr Asn Pro Lys Arg Val
            340                 345

<210> 9
<211> 381
<212> DNA
<213> Homo sapiens

<400> 9
gtccttcctc tcctagccta aggcgtgcaa acagagcgcc actgggaggc tgaaaccttt      60

aggccgatgc ttgcttgcaa ggtcaggcaa gctggattct ggtccccacc tttgcagaga     120

gaacagcgat gttgtgcgcc catttctcag atcaaggacc ggcccatctt actacctcca     180

agagtgcttt tctctctaat aagaaaacat ctactttgaa acatctactg ggcgagacca     240

ggagtgatgg ctcagcctgt aattctggaa tttcgggagg ccgaggcagg aagattcctt     300

gagcacagga gttccagacc agcctgggca atgtagcaag acgctgtctc tatttataca     360

ataaaatttt tttaaaaaag g                                               381

<210> 10
<211> 57
<212> PRT
<213> Homo sapiens

<400> 10

Met Leu Cys Ala His Phe Ser Asp Gln Gly Pro Ala His Leu Thr Thr
1               5                   10                  15

Ser Lys Ser Ala Phe Leu Ser Asn Lys Lys Thr Ser Thr Leu Lys His
            20                  25                  30

Leu Leu Gly Glu Thr Arg Ser Asp Gly Ser Ala Cys Asn Ser Gly Ile
        35                  40                  45

Ser Gly Gly Arg Gly Arg Lys Ile Pro
    50                  55

<210> 11
<211> 1141
<212> DNA
<213> Homo sapiens

<400> 11
tccttgggtt cgggtgaaag cgcctggggg ttcgtggcca tgatccccga gctgctggag      60

aactgaaggc ggacagtctc ctgcgaaacc aggcaatggc ggagctggag tttgttcaga     120

tcatcatcat cgtggtggtg atgatggtga tggtggtggt gatcacgtgc ctgctgagcc     180

actacaagct gtctgcacgg tccttcatca gccggcacag ccaggggcgg aggagagaag     240

atgccctgtc ctcagaagga tgcctgtggc cctcggagag cacagtgtca ggcaacggaa 300

tcccagagcc gcaggtctac gccccgcctc ggcccaccga ccgcctggcc gtgccgccct 360

tcgcccagcg ggagcgcttc caccgcttcc agcccaccta tccgtacctg cagcacgaga 420

tcgacctgcc acccaccatc tcgctgtcag acggggagga gcccccaccc taccagggcc 480

cctgcaccct ccagcttcgg gaccccgagc agcagctgga actgaaccgg gagtcggtgc 540

gcgcaccccc aaacagaacc atcttcgaca gtgacctgat ggatagtgcc aggctgggcg 600

gcccctgccc ccccagcagt aactcgggca tcagcgccac gtgctacggc agcggcgggc 660

gcatggaggg gccgccgccc acctacagcg aggtcatcgg ccactacccg gggtcctcct 720

tccagcacca gcagagcagt gggccgccct ccttgctgga ggggacccgg ctccaccaca 780

cacacatcgc gcccctagag agcgcagcca tctggagcaa agagaaggat aaacagaaag 840

gacaccctct ctagggtccc caggggggcc gggctggggc tgcgtaggtg aaaaggcaga 900

acactccgcg cttcttagaa gaggagtgag aggaaggcgg ggggcgcagc aacgcatcgt 960

gtggccctcc cctcccacct ccctgtgtat aaatatttac atgtgatgtc tggtctgaat 1020

gcacaagcta agagagcttg caaaaaaaaa aagaaaaaag aaaaaaaaaa accacgtttc 1080

tttgttgagc tgtgtcttga aggcaaaaga aaaaaaattt ctacagtaaa aaaaaaaaaa 1140

a 1141

<210> 12
<211> 252
<212> PRT
<213> Homo sapiens

<400> 12

Met Ala Glu Leu Glu Phe Val Gln Ile Ile Ile Ile Val Val Val Met
1               5                   10                  15

Met Val Met Val Val Val Ile Thr Cys Leu Leu Ser His Tyr Lys Leu
            20                  25                  30

Ser Ala Arg Ser Phe Ile Ser Arg His Ser Gln Gly Arg Arg Arg Glu
        35                  40                  45

Asp Ala Leu Ser Ser Glu Gly Cys Leu Trp Pro Ser Glu Ser Thr Val
    50                  55                  60

Ser Gly Asn Gly Ile Pro Glu Pro Gln Val Tyr Ala Pro Pro Arg Pro
65                  70                  75                  80

Thr Asp Arg Leu Ala Val Pro Pro Phe Ala Gln Arg Glu Arg Phe His
                85                  90                  95

Arg Phe Gln Pro Thr Tyr Pro Tyr Leu Gln His Glu Ile Asp Leu Pro
            100                 105                 110

Pro Thr Ile Ser Leu Ser Asp Gly Glu Glu Pro Pro Pro Tyr Gln Gly
        115                 120                 125

Pro Cys Thr Leu Gln Leu Arg Asp Pro Glu Gln Gln Leu Glu Leu Asn
    130                 135                 140

Arg Glu Ser Val Arg Ala Pro Pro Asn Arg Thr Ile Phe Asp Ser Asp
145                 150                 155                 160

Leu Met Asp Ser Ala Arg Leu Gly Gly Pro Cys Pro Pro Ser Ser Asn
                165                 170                 175

Ser Gly Ile Ser Ala Thr Cys Tyr Gly Ser Gly Gly Arg Met Glu Gly
            180                 185                 190

Pro Pro Pro Thr Tyr Ser Glu Val Ile Gly His Tyr Pro Gly Ser Ser
        195                 200                 205

Phe Gln His Gln Gln Ser Ser Gly Pro Pro Ser Leu Leu Glu Gly Thr
    210                 215                 220

Arg Leu His His Thr His Ile Ala Pro Leu Glu Ser Ala Ala Ile Trp
225                 230                 235                 240

Ser Lys Glu Lys Asp Lys Gln Lys Gly His Pro Leu
                245                 250

<210>  13
<211>  1807
<212>  DNA
<213>  Homo sapiens

<400>  13
gcacgaggga agagggtgat ccgacccggg gaaggtcgct gggcagggcg agttgggaaa      60

gcggcagccc ccgccgcccc cgcagcccct tctcctcctt tctcccacgt cctatctgcc     120

tctcgctgga ggccaggccg tgcagcatcg aagacaggag gaactggagc ctcattggcc     180

ggcccggggc gccggcctcg ggcttaaata ggagctccgg gctctggctg ggacccgacc     240

gctgccggcc gcgctcccgc tgctcctgcc gggtgatgga aaaccccagc ccggccgccg     300

ccctgggcaa ggccctctgc gctctcctcc tggccactct cggcgccgcc ggccagcctc     360

ttgggggaga gtccatctgt tccgccagag ccccggccaa atacagcatc accttcacgg     420

gcaagtggag ccagacggcc ttccccaagc agtaccccct gttccgcccc cctgcgcagt     480

ggtcttcgct gctgggggcc gcgcatagct ccgactacag catgtggagg aagaaccagt     540

acgtcagtaa cgggctgcgc gactttgcgg agcgcggcga ggcctgggcg ctgatgaagg     600

agatcgaggc ggcgggggag gcgctgcaga gcgtgcacgc ggtgttttcg gcgcccgccg     660

tccccagcgg caccgggcag acgtcggcgg agctggaggt gcagcgcagg cactcgctgg     720

tctcgtttgt ggtgcgcatc gtgcccagcc ccgactggtt cgtgggcgtg gacagcctgg     780

acctgtgcga cggggaccgt tggcgggaac aggcggcgct ggacctgtac ccctacgacg     840

ccgggacgga cagcggcttc accttctcct cccccaactt cgccaccatc ccgcaggaca     900

cggtgaccga gataacgtcc tcctctccca gccacccggc caactccttc tactacccgc     960

ggctgaaggc cctgcctccc atcgccaggg tgacactggt gcggctgcga cagagcccca    1020

gggccttcat ccctcccgcc ccagtcctgc ccagcaggga caatgagatt gtagacagcg    1080

cctcagttcc agaaacgccg ctggactgcg aggtctccct gtggtcgtcc tggggactgt    1140

gcggaggcca ctgtgggagg ctcgggacca agagcaggac tcgctacgtc cgggtccagc    1200

ccgccaacaa cgggagcccc tgccccgagc tcgaagaaga ggctgagtgc gtccctgata    1260

actgcgtcta agaccagagc cccgcagccc ctggggcccc cggagccatg gggtgtcggg 1320

ggctcctgtg caggctcatg ctgcaggcgg ccgaggcaca gggggtttcg cgctgctcct 1380

gaccgcggtg aggccgcgcc gaccatctct gcactgaagg gccctctggt ggccggcacg 1440

ggcattggga aacagcctcc tcctttccca accttgcttc ttaggggccc ccgtgtcccg 1500

tctgctctca gcctcctcct cctgcaggat aaagtcatcc ccaaggctcc agctactcta 1560

aattatggtc tccttataag ttattgctgc tccaggagat tgtccttcat cgtccagggg 1620

cctggctccc acgtggttgc agatacctca gacctggtgc tctaggctgt gctgagccca 1680

ctctcccgag ggcgcatcca agcgggggcc acttgagaag tgaataaatg gggcggtttc 1740

ggaagcgtca gtgtttccat gttatggatc tctctgcgtt tgaataaaga ctatctctgt 1800

tgctcac 1807

<210> 14
<211> 331
<212> PRT
<213> Homo sapiens

<400> 14

Met Glu Asn Pro Ser Pro Ala Ala Ala Leu Gly Lys Ala Leu Cys Ala
1               5                   10                  15

Leu Leu Leu Ala Thr Leu Gly Ala Ala Gly Gln Pro Leu Gly Gly Glu
            20                  25                  30

Ser Ile Cys Ser Ala Arg Ala Pro Ala Lys Tyr Ser Ile Thr Phe Thr
        35                  40                  45

Gly Lys Trp Ser Gln Thr Ala Phe Pro Lys Gln Tyr Pro Leu Phe Arg
    50                  55                  60

Pro Pro Ala Gln Trp Ser Ser Leu Leu Gly Ala Ala His Ser Ser Asp
65                  70                  75                  80

Tyr Ser Met Trp Arg Lys Asn Gln Tyr Val Ser Asn Gly Leu Arg Asp
                85                  90                  95

Phe Ala Glu Arg Gly Glu Ala Trp Ala Leu Met Lys Glu Ile Glu Ala
            100                 105                 110

Ala Gly Glu Ala Leu Gln Ser Val His Ala Val Phe Ser Ala Pro Ala
        115                 120                 125

Val Pro Ser Gly Thr Gly Gln Thr Ser Ala Glu Leu Glu Val Gln Arg
    130                 135                 140

Arg His Ser Leu Val Ser Phe Val Val Arg Ile Val Pro Ser Pro Asp
145                 150                 155                 160

Trp Phe Val Gly Val Asp Ser Leu Asp Leu Cys Asp Gly Asp Arg Trp
                165                 170                 175

Arg Glu Gln Ala Ala Leu Asp Leu Tyr Pro Tyr Asp Ala Gly Thr Asp
            180                 185                 190

Ser Gly Phe Thr Phe Ser Ser Pro Asn Phe Ala Thr Ile Pro Gln Asp
        195                 200                 205

Thr Val Thr Glu Ile Thr Ser Ser Ser Pro Ser His Pro Ala Asn Ser

210                 215                 220

Phe Tyr Tyr Pro Arg Leu Lys Ala Leu Pro Pro Ile Ala Arg Val Thr
225                 230                 235                 240

Leu Val Arg Leu Arg Gln Ser Pro Arg Ala Phe Ile Pro Pro Ala Pro
                245                 250                 255

Val Leu Pro Ser Arg Asp Asn Glu Ile Val Asp Ser Ala Ser Val Pro
            260                 265                 270

Glu Thr Pro Leu Asp Cys Glu Val Ser Leu Trp Ser Ser Trp Gly Leu
        275                 280                 285

Cys Gly Gly His Cys Gly Arg Leu Gly Thr Lys Ser Arg Thr Arg Tyr
    290                 295                 300

Val Arg Val Gln Pro Ala Asn Asn Gly Ser Pro Cys Pro Glu Leu Glu
305                 310                 315                 320

Glu Glu Ala Glu Cys Val Pro Asp Asn Cys Val
                325                 330

<210>  15
<211>  786
<212>  DNA
<213>  Homo sapiens

<400>  15
cgggcgaagc agcgcgggca gcgagatgca gcaccgaggc ttcctcctcc tcaccctcct      60

cgccctgctg gcgctcacct ccgcggtcgc caaaaagaaa gataaggtga agaagggcgg     120

cccggggagc gagtgcgctg agtgggcctg ggggccctgc acccccagca gcaaggattg     180

cggcgtgggt ttccgcgagg gcacctgcgg ggcccagacc cagcgcatcc ggtgcagggt     240

gccctgcaac tggaagaagg agtttggagc cgactgcaag tacaagtttg agaactgggg     300

tgcgtgtgat gggggcacag gcaccaaagt ccgccaaggc accctgaaga aggcgcgcta     360

caatgctcag tgccaggaga ccatccgcgt caccaagccc tgcaccccca agaccaaagc     420

aaaggccaaa gccaagaaag ggaagggaaa ggactagacg ccaagcctgg atgccaagga     480

gcccctggtg tcacatgggg cctggccacg ccctccctct cccaggcccg agatgtgacc     540

caccagtgcc ttctgtctgc tcgttagctt taatcaatca tgccctgcct tgtccctctc     600

actccccagc cccaccccta agtgcccaaa gtggggaggg acaagggatt ctgggaagct     660

tgagcctccc ccaaagcaat gtgagtccca gagcccgctt ttgttcttcc ccacaattcc     720

attactaaga aacacatcaa ataaactgac tttttccccc caataaaagc tcttcttttt     780

taatat                                                                786

<210>  16
<211>  143
<212>  PRT
<213>  Homo sapiens

<400>  16

Met Gln His Arg Gly Phe Leu Leu Leu Thr Leu Leu Ala Leu Leu Ala
1               5                   10                  15

Leu Thr Ser Ala Val Ala Lys Lys Lys Asp Lys Val Lys Lys Gly Gly

20                  25                  30

Pro Gly Ser Glu Cys Ala Glu Trp Ala Trp Gly Pro Cys Thr Pro Ser
        35                  40                  45

Ser Lys Asp Cys Gly Val Gly Phe Arg Glu Gly Thr Cys Gly Ala Gln
    50                  55                  60

Thr Gln Arg Ile Arg Cys Arg Val Pro Cys Asn Trp Lys Lys Glu Phe
65                  70                  75                  80

Gly Ala Asp Cys Lys Tyr Lys Phe Glu Asn Trp Gly Ala Cys Asp Gly
                85                  90                  95

Gly Thr Gly Thr Lys Val Arg Gln Gly Thr Leu Lys Lys Ala Arg Tyr
            100                 105                 110

Asn Ala Gln Cys Gln Glu Thr Ile Arg Val Thr Lys Pro Cys Thr Pro
        115                 120                 125

Lys Thr Lys Ala Lys Ala Lys Ala Lys Lys Gly Lys Gly Lys Asp
    130                 135                 140

<210>  17
<211>  1617
<212>  DNA
<213>  Homo sapiens

<400>  17
atggtagaac tagtgatctc acccagcctc actgtaaaca gcgattgtct ggataaactg      60

aagtttaacc gtgctgacgc tgctgtgtgg actctgagtg acagacaagg catcaccaaa     120

tcggcccccc tgagagtgtc ccagctcttc tccagatctt gcccacgtgt cctcccccgc     180

cagccttcca cagccatggc agcctacggc cagacgcagt acagtgcggg gatccagcag     240

gctaccccct atacagctta cccacctcca gcacaagcct atggaatccc ttcctacagc     300

atcaagacag aagacagctt gaaccattcc cctggccaga gtggattcct cagctatggc     360

tccagcttca gcacctcacc cactggacag agcccataca cctaccagat gcacggcaca     420

acagggttct atcaaggagg aaatggactg ggcaacgcag ccggtttcgg gagtgtgcac     480

caggactatc cttcctaccc cggcttcccc cagagccagt acccccagta ttacggctca     540

tcctacaacc ctccctacgt cccggccagc agcatctgcc cttcgcccct ctccacgtcc     600

acctacgtcc tccaggaggc atctcacaac gtccccaacc agagttccga gtcacttgct     660

ggtgaataca acacacacaa tggaccttcc acaccagcga aagagggaga cacagacagg     720

ccgcaccggg cctccgacgg gaagctccga ggccggtcta agaggagcag tgacccgtcc     780

ccggcagggg acaatgagat tgagcgtgtg ttcgtgtggg acttggatga gacaataatt     840

atttttcact ccttactcac ggggacattt gcatccagat acgggaagga caccacgacg     900

tccgtgcgca ttggccttat gatggaagag atgatcttca accttgcaga tacacatctg     960

ttcttcaatg acctggagga ttgtgaccag atccacgttg atgacgtctc atcagatgac    1020

aatggccaag atttaagcac atacaacttc tccgctgacg gcttccacag ttcggcccca    1080

ggagccaacc tgtgcctggg ctctggcgtg cacggcggcg tggactggat gaggaagctg    1140

gccttccgct accggcgggt gaaggagatg tacaatacct acaagaacaa cgttggtggg    1200

ttgataggca ctcccaaaag ggagacctgg ctacagctcc gagctgagct ggaagctctc 1260

acagacctct ggctgaccca ctccctgaag gcactaaacc tcatcaactc ccggcccaac 1320

tgtgtcaatg tgctggtcac caccactcaa ctaattcctg ccctggccaa agtcctgcta 1380

tatggcctgg ggtctgtgtt tcctattgag aacatctaca gtgcaaccaa gacagggaag 1440

gagagctgct tcgagaggat aatgcagaga ttcggcagaa aagctgtcta cgtggtgatc 1500

ggtgatggtg tggaagagga gcaaggagcg aaaaagcaca acatgccttt ctggcggata 1560

tcctgccacg cagacctgga ggcactgagg cacgccctgg aactggagta tttatag 1617

<210> 18
<211> 538
<212> PRT
<213> Homo sapiens

<400> 18

Met Val Glu Leu Val Ile Ser Pro Ser Leu Thr Val Asn Ser Asp Cys
1               5                   10                  15

Leu Asp Lys Leu Lys Phe Asn Arg Ala Asp Ala Ala Val Trp Thr Leu
            20                  25                  30

Ser Asp Arg Gln Gly Ile Thr Lys Ser Ala Pro Leu Arg Val Ser Gln
        35                  40                  45

Leu Phe Ser Arg Ser Cys Pro Arg Val Leu Pro Arg Gln Pro Ser Thr
    50                  55                  60

Ala Met Ala Ala Tyr Gly Gln Thr Gln Tyr Ser Ala Gly Ile Gln Gln
65                  70                  75                  80

Ala Thr Pro Tyr Thr Ala Tyr Pro Pro Pro Ala Gln Ala Tyr Gly Ile
                85                  90                  95

Pro Ser Tyr Ser Ile Lys Thr Glu Asp Ser Leu Asn His Ser Pro Gly
            100                 105                 110

Gln Ser Gly Phe Leu Ser Tyr Gly Ser Ser Phe Ser Thr Ser Pro Thr
        115                 120                 125

Gly Gln Ser Pro Tyr Thr Tyr Gln Met His Gly Thr Thr Gly Phe Tyr
    130                 135                 140

Gln Gly Gly Asn Gly Leu Gly Asn Ala Ala Gly Phe Gly Ser Val His
145                 150                 155                 160

Gln Asp Tyr Pro Ser Tyr Pro Gly Phe Pro Gln Ser Gln Tyr Pro Gln
                165                 170                 175

Tyr Tyr Gly Ser Ser Tyr Asn Pro Pro Tyr Val Pro Ala Ser Ser Ile
            180                 185                 190

Cys Pro Ser Pro Leu Ser Thr Ser Thr Tyr Val Leu Gln Glu Ala Ser
        195                 200                 205

His Asn Val Pro Asn Gln Ser Ser Glu Ser Leu Ala Gly Glu Tyr Asn
    210                 215                 220

Thr His Asn Gly Pro Ser Thr Pro Ala Lys Glu Gly Asp Thr Asp Arg
225                 230                 235                 240

Pro His Arg Ala Ser Asp Gly Lys Leu Arg Gly Arg Ser Lys Arg Ser

                245                 250                 255

Ser Asp Pro Ser Pro Ala Gly Asp Asn Glu Ile Glu Arg Val Phe Val
            260                 265                 270

Trp Asp Leu Asp Glu Thr Ile Ile Ile Phe His Ser Leu Leu Thr Gly
        275                 280                 285

Thr Phe Ala Ser Arg Tyr Gly Lys Asp Thr Thr Thr Ser Val Arg Ile
    290                 295                 300

Gly Leu Met Met Glu Glu Met Ile Phe Asn Leu Ala Asp Thr His Leu
305                 310                 315                 320

Phe Phe Asn Asp Leu Glu Asp Cys Asp Gln Ile His Val Asp Asp Val
                325                 330                 335

Ser Ser Asp Asp Asn Gly Gln Asp Leu Ser Thr Tyr Asn Phe Ser Ala
            340                 345                 350

Asp Gly Phe His Ser Ser Ala Pro Gly Ala Asn Leu Cys Leu Gly Ser
        355                 360                 365

Gly Val His Gly Gly Val Asp Trp Met Arg Lys Leu Ala Phe Arg Tyr
    370                 375                 380

Arg Arg Val Lys Glu Met Tyr Asn Thr Tyr Lys Asn Asn Val Gly Gly
385                 390                 395                 400

Leu Ile Gly Thr Pro Lys Arg Glu Thr Trp Leu Gln Leu Arg Ala Glu
                405                 410                 415

Leu Glu Ala Leu Thr Asp Leu Trp Leu Thr His Ser Leu Lys Ala Leu
            420                 425                 430

Asn Leu Ile Asn Ser Arg Pro Asn Cys Val Asn Val Leu Val Thr Thr
        435                 440                 445

Thr Gln Leu Ile Pro Ala Leu Ala Lys Val Leu Leu Tyr Gly Leu Gly
    450                 455                 460

Ser Val Phe Pro Ile Glu Asn Ile Tyr Ser Ala Thr Lys Thr Gly Lys
465                 470                 475                 480

Glu Ser Cys Phe Glu Arg Ile Met Gln Arg Phe Gly Arg Lys Ala Val
                485                 490                 495

Tyr Val Val Ile Gly Asp Gly Val Glu Glu Glu Gln Gly Ala Lys Lys
            500                 505                 510

His Asn Met Pro Phe Trp Arg Ile Ser Cys His Ala Asp Leu Glu Ala
        515                 520                 525

Leu Arg His Ala Leu Glu Leu Glu Tyr Leu
    530                 535

<210> 19
<211> 2261
<212> DNA
<213> Homo sapiens

<400> 19
ccgcggttcc ggctgctccg gcgaggcgac ccttgggtcg gcgctgcggg cgaggtgggc 60

aggtaggtgg gcggacggcc gcggttctcc ggcaagcgca ggcggcggag tcccccacgg 120

cgcccgaagc gccccccgca cccccggcct ccagcgttga ggcgggggag tgaggagatg 180

ccgacccaga gggacagcag caccatgtcc cacacggtcg caggcggcgg cagcggggac 240
cattcccacc aggtccgggt gaaagcctac taccgcgggg atatcatgat aacacatttt 300
gaaccttcca tctcctttga gggcctttgc aatgaggttc gagacatgtg ttcttttgac 360
aacgaacagc tcttcaccat gaaatggata gatgaggaag gagacccgtg tacagtatca 420
tctcagttgg agttagaaga agcctttaga ctttatgagc taaacaagga ttctgaactc 480
ttgattcatg tgttcccttg tgtaccagaa cgtcctggga tgccttgtcc aggagaagat 540
aaatccatct accgtagagg tgcacgccgc tggagaaagc tttattgtgc caatggccac 600
actttccaag ccaagcgttt caacaggcgt gctcactgtg ccatctgcac agaccgaata 660
tggggacttg gacgccaagg atataagtgc atcaactgca aactcttggt tcataagaag 720
tgccataaac tcgtcacaat tgaatgtggg cggcattctt tgccacagga accagtgatg 780
cccatggatc agtcatccat gcattctgac catgcacaga cagtaattcc atataatcct 840
tcaagtcatg agagtttgga tcaagttggt gaagaaaaag aggcaatgaa caccagggaa 900
agtggcaaag cttcatccag tctaggtctt caggattttg atttgctccg ggtaatagga 960
agaggaagtt atgccaaagt actgttggtt cgattaaaaa aaacagatcg tatttatgca 1020
atgaaagttg tgaaaaaaga gcttgttaat gatgatgagg atattgattg ggtacagaca 1080
gagaagcatg tgtttgagca ggcatccaat catcctttcc ttgttgggct gcattcttgc 1140
tttcagacag aaagcagatt gttctttgtt atagagtatg taaatggagg agacctaatg 1200
tttcatatgc agcgacaaag aaaacttcct gaagaacatg ccagatttta ctctgcagaa 1260
atcagtctag cattaaatta tcttcatgag cgagggataa tttatagaga tttgaaactg 1320
gacaatgtat tactggactc tgaaggccac attaaactca ctgactacgg catgtgtaag 1380
gaaggattac ggccaggaga tacaaccagc actttctgtg gtactcctaa ttacattgct 1440
cctgaaattt taagaggaga agattatggt ttcagtgttg actggtgggc tcttggagtg 1500
ctcatgtttg agatgatggc aggaaggtct ccatttgata ttgttgggag ctccgataac 1560
cctgaccaga acacagagga ttatctcttc caagttattt tggaaaaaca aattcgcata 1620
ccacgttctc tgtctgtaaa agctgcaagt gttctgaaga gttttcttaa taaggaccct 1680
aaggaacgat tgggttgtca tcctcaaaca ggatttgctg atattcaggg acacccgttc 1740
ttccgaaatg ttgattggga tatgatggag caaaaacagg tggtacctcc ctttaaacca 1800
aatatttctg gggaatttgg tttggacaac tttgattctc agtttactaa tgaacctgtc 1860
cagctcactc cagatgacga tgacattgtg aggaagattg atcagtctga atttgaaggt 1920
tttgagtata tcaatcctct tttgatgtct gcagaagaat gtgtctgatc ctcatttttc 1980
aaccatgtat tctactcatg ttgccattta atgcatggat aaacttgctg caagcctgga 2040
tacaattaac cattttatat ttgccaccta caaaaaaaca cccaatatct tctcttgtag 2100
actatatgaa tcaattatta catctgtttt actatgaaaa aaaaattaat actactagct 2160
tccagacaat catgtcaaaa tttagttgaa ctggtttttc agtttttaaa aggcctacag 2220

atgagtaatg aagttacctt ttttgtttaa aaaaaaaaaa g 2261

<210> 20
<211> 587
<212> PRT
<213> Homo sapiens

<400> 20

Met Ser His Thr Val Ala Gly Gly Gly Ser Gly Asp His Ser His Gln
1               5                   10                  15

Val Arg Val Lys Ala Tyr Tyr Arg Gly Asp Ile Met Ile Thr His Phe
            20                  25                  30

Glu Pro Ser Ile Ser Phe Glu Gly Leu Cys Asn Glu Val Arg Asp Met
        35                  40                  45

Cys Ser Phe Asp Asn Glu Gln Leu Phe Thr Met Lys Trp Ile Asp Glu
    50                  55                  60

Glu Gly Asp Pro Cys Thr Val Ser Ser Gln Leu Glu Leu Glu Glu Ala
65                  70                  75                  80

Phe Arg Leu Tyr Glu Leu Asn Lys Asp Ser Glu Leu Leu Ile His Val
                85                  90                  95

Phe Pro Cys Val Pro Glu Arg Pro Gly Met Pro Cys Pro Gly Glu Asp
            100                 105                 110

Lys Ser Ile Tyr Arg Arg Gly Ala Arg Arg Trp Arg Lys Leu Tyr Cys
        115                 120                 125

Ala Asn Gly His Thr Phe Gln Ala Lys Arg Phe Asn Arg Arg Ala His
    130                 135                 140

Cys Ala Ile Cys Thr Asp Arg Ile Trp Gly Leu Gly Arg Gln Gly Tyr
145                 150                 155                 160

Lys Cys Ile Asn Cys Lys Leu Leu Val His Lys Lys Cys His Lys Leu
                165                 170                 175

Val Thr Ile Glu Cys Gly Arg His Ser Leu Pro Gln Glu Pro Val Met
            180                 185                 190

Pro Met Asp Gln Ser Ser Met His Ser Asp His Ala Gln Thr Val Ile
        195                 200                 205

Pro Tyr Asn Pro Ser Ser His Glu Ser Leu Asp Gln Val Gly Glu Glu
    210                 215                 220

Lys Glu Ala Met Asn Thr Arg Glu Ser Gly Lys Ala Ser Ser Ser Leu
225                 230                 235                 240

Gly Leu Gln Asp Phe Asp Leu Leu Arg Val Ile Gly Arg Gly Ser Tyr
                245                 250                 255

Ala Lys Val Leu Leu Val Arg Leu Lys Lys Thr Asp Arg Ile Tyr Ala
            260                 265                 270

Met Lys Val Val Lys Lys Glu Leu Val Asn Asp Asp Glu Asp Ile Asp
        275                 280                 285

Trp Val Gln Thr Glu Lys His Val Phe Glu Gln Ala Ser Asn His Pro
    290                 295                 300

Phe Leu Val Gly Leu His Ser Cys Phe Gln Thr Glu Ser Arg Leu Phe
305                 310                 315                 320

Phe Val Ile Glu Tyr Val Asn Gly Gly Asp Leu Met Phe His Met Gln
                325                 330                 335

Arg Gln Arg Lys Leu Pro Glu Glu His Ala Arg Phe Tyr Ser Ala Glu
            340                 345                 350

Ile Ser Leu Ala Leu Asn Tyr Leu His Glu Arg Gly Ile Ile Tyr Arg
        355                 360                 365

Asp Leu Lys Leu Asp Asn Val Leu Leu Asp Ser Glu Gly His Ile Lys
    370                 375                 380

Leu Thr Asp Tyr Gly Met Cys Lys Glu Gly Leu Arg Pro Gly Asp Thr
385                 390                 395                 400

Thr Ser Thr Phe Cys Gly Thr Pro Asn Tyr Ile Ala Pro Glu Ile Leu
                405                 410                 415

Arg Gly Glu Asp Tyr Gly Phe Ser Val Asp Trp Trp Ala Leu Gly Val
            420                 425                 430

Leu Met Phe Glu Met Met Ala Gly Arg Ser Pro Phe Asp Ile Val Gly
        435                 440                 445

Ser Ser Asp Asn Pro Asp Gln Asn Thr Glu Asp Tyr Leu Phe Gln Val
    450                 455                 460

Ile Leu Glu Lys Gln Ile Arg Ile Pro Arg Ser Leu Ser Val Lys Ala
465                 470                 475                 480

Ala Ser Val Leu Lys Ser Phe Leu Asn Lys Asp Pro Lys Glu Arg Leu
                485                 490                 495

Gly Cys His Pro Gln Thr Gly Phe Ala Asp Ile Gln Gly His Pro Phe
            500                 505                 510

Phe Arg Asn Val Asp Trp Asp Met Met Glu Gln Lys Gln Val Val Pro
        515                 520                 525

Pro Phe Lys Pro Asn Ile Ser Gly Glu Phe Gly Leu Asp Asn Phe Asp
    530                 535                 540

Ser Gln Phe Thr Asn Glu Pro Val Gln Leu Thr Pro Asp Asp Asp Asp
545                 550                 555                 560

Ile Val Arg Lys Ile Asp Gln Ser Glu Phe Glu Gly Phe Glu Tyr Ile
                565                 570                 575

Asn Pro Leu Leu Met Ser Ala Glu Glu Cys Val
            580                 585

<210> 21
<211> 5011
<212> DNA
<213> homo sapiens

<400> 21
ccaggcggcg ttgcggcccc ggccccggct ccctgcgccg ccgccgccgc cgccgccgcc 60

gccgccgccg ccgccgccag cgctagcgcc agcagccggg cccgatcacc cgccgcccgg 120

tgcccgccgc cgcccgcgcc agcaaccggg cccgatcacc cgccgcccgg tgcccgccgc 180

cgcccgcgcc accggcatgg cgctccgggg cttctgcagc gccgatggct ccgacccgct 240

ctgggactgg aatgtcacgt ggaataccag caaccccgac ttcaccaagt gctttcagaa 300

cacggtcctc gtgtgggtgc cttgttttta cctctgggcc tgtttcccct tctacttcct 360
ctatctctcc cgacatgacc gaggctacat tcagatgaca cctctcaaca aaaccaaaac 420
tgccttggga tttttgctgt ggatcgtctg ctgggcagac ctcttctact ctttctggga 480
aagaagtcgg ggcatattcc tggccccagt gtttctggtc agcccaactc tcttgggcat 540
caccacgctg cttgctacct ttttaattca gctggagagg aggaagggag ttcagtcttc 600
agggatcatg ctcactttct ggctggtagc cctagtgtgt gccctagcca tcctgagatc 660
caaaattatg acagccttaa aagaggatgc ccaggtggac ctgtttcgtg acatcacttt 720
ctacgtctac ttttccctct tactcattca gctcgtcttg tcctgtttct cagatcgctc 780
acccctgttc tcggaaacca tccacgaccc taatccctgc ccagagtcca gcgcttcctt 840
cctgtcgagg atcaccttct ggtggatcac agggttgatt gtccggggct accgccagcc 900
cctggagggc agtgacctct ggtccttaaa caaggaggac acgtcggaac aagtcgtgcc 960
tgttttggta aagaactgga agaaggaatg cgccaagact aggaagcagc cggtgaaggt 1020
tgtgtactcc tccaaggatc ctgcccagcc gaaagagagt tccaaggtgg atgcgaatga 1080
ggaggtggag gctttgatcg tcaagtcccc acagaaggag tggaacccct ctctgtttaa 1140
ggtgttatac aagacctttg ggccctactt cctcatgagc ttcttcttca aggccatcca 1200
cgacctgatg atgttttccg ggccgcagat cttaaagttg ctcatcaagt tcgtgaatga 1260
cacgaaggcc ccagactggc agggctactt ctacaccgtg ctgctgtttg tcactgcctg 1320
cctgcagacc ctcgtgctgc accagtactt ccacatctgc ttcgtcagtg gcatgaggat 1380
caagaccgct gtcattgggg ctgtctatcg gaaggccctg gtgatcacca attcagccag 1440
aaaatcctcc acggtcgggg agattgtcaa cctcatgtct gtggacgctc agaggttcat 1500
ggacttggcc acgtacatta acatgatctg gtcagccccc ctgcaagtca tccttgctct 1560
ctacctcctg tggctgaatc tgggcccttc cgtcctggct ggagtggcgg tgatggtcct 1620
catggtgccc gtcaatgctg tgatggcgat gaagaccaag acgtatcagg tggcccacat 1680
gaagagcaaa gacaatcgga tcaagctgat gaacgaaatt ctcaatggga tcaaagtgct 1740
aaagctttat gcctgggagc tggcattcaa ggacaaggtg ctggccatca ggcaggagga 1800
gctgaaggtg ctgaagaagt ctgcctacct gtcagccgtg ggcaccttca cctgggtctg 1860
cacgcccttt ctggtggcct tgtgcacatt tgccgtctac gtgaccattg acgagaacaa 1920
catcctggat gcccagacag ccttcgtgtc tttggccttg ttcaacatcc tccggtttcc 1980
cctgaacatt ctccccatgg tcatcagcag catcgtgcag gcgagtgtct ccctcaaacg 2040
cctgaggatc tttctctccc atgaggagct ggaacctgac agcatcgagc gacggcctgt 2100
caaagacggc gggggcacga acagcatcac cgtgaggaat gccacattca cctgggccag 2160
gagcgaccct cccacactga atggcatcac cttctccatc cccgaaggtg ctttggtggc 2220
cgtggtgggc caggtgggct gcggaaagtc gtccctgctc tcagccctct tggctgagat 2280
ggacaaagtg gaggggcacg tggctatcaa gggctccgtg gcctatgtgc cacagcaggc 2340

ctggattcag aatgattctc tccgagaaaa catccttttt ggatgtcagc tggaggaacc 2400
atattacagg tccgtgatac aggcctgtgc cctcctccca gacctggaaa tcctgcccag 2460
tggggatcgg acagagattg gcgagaaggg cgtgaacctg tctgggggcc agaagcagcg 2520
cgtgagcctg gcccgggccg tgtactccaa cgctgacatt tacctcttcg atgatcccct 2580
ctcagcagtg gatgcccatg tgggaaaaca catctttgaa aatgtgattg gccccaaggg 2640
gatgctgaag aacaagacgc ggatcttggt cacgcacagc atgagctact tgccgcaggt 2700
ggacgtcatc atcgtcatga gtggcggcaa gatctctgag atgggctcct accaggagct 2760
gctggctcga gacggcgcct tcgctgagtt cctgcgtacc tatgccagca cagagcagga 2820
gcaggatgca gaggagaacg gggtcacggg cgtcagcggt ccagggaagg aagcaaagca 2880
aatggagaat ggcatgctgg tgacggacag tgcagggaag caactgcaga gacagctcag 2940
cagctcctcc tcctatagtg gggacatcag caggcaccac aacagcaccg cagaactgca 3000
gaaagctgag gccaagaagg aggagacctg gaagctgatg gaggctgaca aggcgcagac 3060
agggcaggtc aagctttccg tgtactggga ctacatgaag gccatcggac tcttcatctc 3120
cttcctcagc atcttccttt tcatgtgtaa ccatgtgtcc gcgctggctt ccaactattg 3180
gctcagcctc tggactgatg accccatcgt caacgggact caggagcaca cgaaagtccg 3240
gctgagcgtc tatggagccc tgggcatttc acaagggatc gccgtgtttg gctactccat 3300
ggccgtgtcc atcgggggga tcttggcttc ccgctgtctg cacgtggacc tgctgcacag 3360
catcctgcgg tcacccatga gcttctttga gcggaccccc agtgggaacc tggtgaaccg 3420
cttctccaag gagctggaca cagtggactc catgatcccg gaggtcatca agatgttcat 3480
gggctccctg ttcaacgtca ttggtgcctg catcgttatc ctgctggcca cgcccatcgc 3540
cgccatcatc atcccgcccc ttggcctcat ctacttcttc gtccagaggt tctacgtggc 3600
ttcctcccgg cagctgaagc gcctcgagtc ggtcagccgc tccccggtct attcccattt 3660
caacgagacc ttgctggggg tcagcgtcat tcgagccttc gaggagcagg agcgcttcat 3720
ccaccagagt gacctgaagg tggacgagaa ccagaaggcc tattacccca gcatcgtggc 3780
caacaggtgg ctggccgtgc ggctggagtg tgtgggcaac tgcatcgttc tgtttgctgc 3840
cctgtttgcg gtgatctcca ggcacagcct cagtgctggc ttggtgggcc tctcagtgtc 3900
ttactcattg caggtcacca cgtacttgaa ctggctggtt cggatgtcat ctgaaatgga 3960
aaccaacatc gtggccgtgg agaggctcaa ggagtattca gagactgaga aggaggcgcc 4020
ctggcaaatc caggagacag ctccgcccag cagctggccc caggtgggcc gagtggaatt 4080
ccggaactac tgcctgcgct accgagagga cctggacttc gttctcaggc acatcaatgt 4140
cacgatcaat gggggagaaa aggtcggcat cgtggggcgg acgggagctg ggaagtcgtc 4200
cctgaccctg ggcttatttc ggatcaacga gtctgccgaa ggagagatca tcatcgatgg 4260
catcaacatc gccaagatcg gcctgcacga cctccgcttc aagatcacca tcatccccca 4320
ggaccctgtt ttgttttcgg gttccctccg aatgaacctg gacccattca gccagtactc 4380

ggatgaagaa gtctggacgt ccctggagct ggcccacctg aaggacttcg tgtcagccct 4440

tcctgacaag ctagaccatg aatgtgcaga aggcggggag aacctcagtg tcgggcagcg 4500

ccagcttgtg tgcctagccc gggccctgct gaggaagacg aagatccttg tgttggatga 4560

ggccacggca gccgtggacc tggaaacgga cgacctcatc cagtccacca tccggacaca 4620

gttcgaggac tgcaccgtcc tcaccatcgc ccaccggctc aacaccatca tggactacac 4680

aagggtgatc gtcttggaca aaggagaaat ccaggagtac ggcgccccat cggacctcct 4740

gcagcagaga ggtcttttct acagcatggc caaagacgcc ggcttggtgt gagccccaga 4800

gctggcatat ctggtcagaa ctgcagggcc tatatgccag cgcccaggga ggagtcagta 4860

cccctggtaa accaagcctc ccacactgaa accaaaacat aaaaaccaaa cccagacaac 4920

caaaacatat tcaaagcagc agccaccgcc atccggtccc ctgcctggaa ctggctgtga 4980

agacccagga gagacagaga tgcgaaccac c 5011

<210> 22
<211> 1531
<212> PRT
<213> homo sapiens

<400> 22

Met Ala Leu Arg Gly Phe Cys Ser Ala Asp Gly Ser Asp Pro Leu Trp
1               5                   10                  15

Asp Trp Asn Val Thr Trp Asn Thr Ser Asn Pro Asp Phe Thr Lys Cys
            20                  25                  30

Phe Gln Asn Thr Val Leu Val Trp Val Pro Cys Phe Tyr Leu Trp Ala
        35                  40                  45

Cys Phe Pro Phe Tyr Phe Leu Tyr Leu Ser Arg His Asp Arg Gly Tyr
    50                  55                  60

Ile Gln Met Thr Pro Leu Asn Lys Thr Lys Thr Ala Leu Gly Phe Leu
65                  70                  75                  80

Leu Trp Ile Val Cys Trp Ala Asp Leu Phe Tyr Ser Phe Trp Glu Arg
                85                  90                  95

Ser Arg Gly Ile Phe Leu Ala Pro Val Phe Leu Val Ser Pro Thr Leu
            100                 105                 110

Leu Gly Ile Thr Thr Leu Leu Ala Thr Phe Leu Ile Gln Leu Glu Arg
        115                 120                 125

Arg Lys Gly Val Gln Ser Ser Gly Ile Met Leu Thr Phe Trp Leu Val
    130                 135                 140

Ala Leu Val Cys Ala Leu Ala Ile Leu Arg Ser Lys Ile Met Thr Ala
145                 150                 155                 160

Leu Lys Glu Asp Ala Gln Val Asp Leu Phe Arg Asp Ile Thr Phe Tyr
                165                 170                 175

Val Tyr Phe Ser Leu Leu Leu Ile Gln Leu Val Leu Ser Cys Phe Ser
            180                 185                 190

Asp Arg Ser Pro Leu Phe Ser Glu Thr Ile His Asp Pro Asn Pro Cys
        195                 200                 205

Pro Glu Ser Ser Ala Ser Phe Leu Ser Arg Ile Thr Phe Trp Trp Ile
    210                 215                 220

Thr Gly Leu Ile Val Arg Gly Tyr Arg Gln Pro Leu Glu Gly Ser Asp
225                 230                 235                 240

Leu Trp Ser Leu Asn Lys Glu Asp Thr Ser Glu Gln Val Val Pro Val
                245                 250                 255

Leu Val Lys Asn Trp Lys Lys Glu Cys Ala Lys Thr Arg Lys Gln Pro
            260                 265                 270

Val Lys Val Val Tyr Ser Ser Lys Asp Pro Ala Gln Pro Lys Glu Ser
        275                 280                 285

Ser Lys Val Asp Ala Asn Glu Glu Val Glu Ala Leu Ile Val Lys Ser
    290                 295                 300

Pro Gln Lys Glu Trp Asn Pro Ser Leu Phe Lys Val Leu Tyr Lys Thr
305                 310                 315                 320

Phe Gly Pro Tyr Phe Leu Met Ser Phe Phe Phe Lys Ala Ile His Asp
                325                 330                 335

Leu Met Met Phe Ser Gly Pro Gln Ile Leu Lys Leu Leu Ile Lys Phe
            340                 345                 350

Val Asn Asp Thr Lys Ala Pro Asp Trp Gln Gly Tyr Phe Tyr Thr Val
        355                 360                 365

Leu Leu Phe Val Thr Ala Cys Leu Gln Thr Leu Val Leu His Gln Tyr
    370                 375                 380

Phe His Ile Cys Phe Val Ser Gly Met Arg Ile Lys Thr Ala Val Ile
385                 390                 395                 400

Gly Ala Val Tyr Arg Lys Ala Leu Val Ile Thr Asn Ser Ala Arg Lys
                405                 410                 415

Ser Ser Thr Val Gly Glu Ile Val Asn Leu Met Ser Val Asp Ala Gln
            420                 425                 430

Arg Phe Met Asp Leu Ala Thr Tyr Ile Asn Met Ile Trp Ser Ala Pro
        435                 440                 445

Leu Gln Val Ile Leu Ala Leu Tyr Leu Leu Trp Leu Asn Leu Gly Pro
    450                 455                 460

Ser Val Leu Ala Gly Val Ala Val Met Val Leu Met Val Pro Val Asn
465                 470                 475                 480

Ala Val Met Ala Met Lys Thr Lys Thr Tyr Gln Val Ala His Met Lys
                485                 490                 495

Ser Lys Asp Asn Arg Ile Lys Leu Met Asn Glu Ile Leu Asn Gly Ile
            500                 505                 510

Lys Val Leu Lys Leu Tyr Ala Trp Glu Leu Ala Phe Lys Asp Lys Val
        515                 520                 525

Leu Ala Ile Arg Gln Glu Glu Leu Lys Val Leu Lys Lys Ser Ala Tyr
    530                 535                 540

Leu Ser Ala Val Gly Thr Phe Thr Trp Val Cys Thr Pro Phe Leu Val
545                 550                 555                 560

Ala Leu Cys Thr Phe Ala Val Tyr Val Thr Ile Asp Glu Asn Asn Ile
                565                 570                 575

Leu Asp Ala Gln Thr Ala Phe Val Ser Leu Ala Leu Phe Asn Ile Leu
            580                 585                 590

Arg Phe Pro Leu Asn Ile Leu Pro Met Val Ile Ser Ser Ile Val Gln
        595                 600                 605

Ala Ser Val Ser Leu Lys Arg Leu Arg Ile Phe Leu Ser His Glu Glu
    610                 615                 620

Leu Glu Pro Asp Ser Ile Glu Arg Arg Pro Val Lys Asp Gly Gly Gly
625                 630                 635                 640

Thr Asn Ser Ile Thr Val Arg Asn Ala Thr Phe Thr Trp Ala Arg Ser
                645                 650                 655

Asp Pro Pro Thr Leu Asn Gly Ile Thr Phe Ser Ile Pro Glu Gly Ala
            660                 665                 670

Leu Val Ala Val Val Gly Gln Val Gly Cys Gly Lys Ser Ser Leu Leu
        675                 680                 685

Ser Ala Leu Leu Ala Glu Met Asp Lys Val Glu Gly His Val Ala Ile
    690                 695                 700

Lys Gly Ser Val Ala Tyr Val Pro Gln Gln Ala Trp Ile Gln Asn Asp
705                 710                 715                 720

Ser Leu Arg Glu Asn Ile Leu Phe Gly Cys Gln Leu Glu Glu Pro Tyr
                725                 730                 735

Tyr Arg Ser Val Ile Gln Ala Cys Ala Leu Leu Pro Asp Leu Glu Ile
            740                 745                 750

Leu Pro Ser Gly Asp Arg Thr Glu Ile Gly Glu Lys Gly Val Asn Leu
        755                 760                 765

Ser Gly Gly Gln Lys Gln Arg Val Ser Leu Ala Arg Ala Val Tyr Ser
    770                 775                 780

Asn Ala Asp Ile Tyr Leu Phe Asp Asp Pro Leu Ser Ala Val Asp Ala
785                 790                 795                 800

His Val Gly Lys His Ile Phe Glu Asn Val Ile Gly Pro Lys Gly Met
                805                 810                 815

Leu Lys Asn Lys Thr Arg Ile Leu Val Thr His Ser Met Ser Tyr Leu
            820                 825                 830

Pro Gln Val Asp Val Ile Ile Val Met Ser Gly Gly Lys Ile Ser Glu
        835                 840                 845

Met Gly Ser Tyr Gln Glu Leu Leu Ala Arg Asp Gly Ala Phe Ala Glu
    850                 855                 860

Phe Leu Arg Thr Tyr Ala Ser Thr Glu Gln Glu Gln Asp Ala Glu Glu
865                 870                 875                 880

Asn Gly Val Thr Gly Val Ser Gly Pro Gly Lys Glu Ala Lys Gln Met
                885                 890                 895

Glu Asn Gly Met Leu Val Thr Asp Ser Ala Gly Lys Gln Leu Gln Arg
            900                 905                 910

Gln Leu Ser Ser Ser Ser Ser Tyr Ser Gly Asp Ile Ser Arg His His
        915                 920                 925

Asn Ser Thr Ala Glu Leu Gln Lys Ala Glu Ala Lys Lys Glu Glu Thr

930                 935                 940

Trp Lys Leu Met Glu Ala Asp Lys Ala Gln Thr Gly Gln Val Lys Leu
945                 950                 955                 960

Ser Val Tyr Trp Asp Tyr Met Lys Ala Ile Gly Leu Phe Ile Ser Phe
                965                 970                 975

Leu Ser Ile Phe Leu Phe Met Cys Asn His Val Ser Ala Leu Ala Ser
            980                 985                 990

Asn Tyr Trp Leu Ser Leu Trp Thr  Asp Asp Pro Ile Val  Asn Gly Thr
        995                 1000                 1005

Gln Glu  His Thr Lys Val Arg  Leu Ser Val Tyr Gly  Ala Leu Gly
    1010                 1015                 1020

Ile Ser  Gln Gly Ile Ala Val  Phe Gly Tyr Ser Met  Ala Val Ser
    1025                 1030                 1035

Ile Gly  Gly Ile Leu Ala Ser  Arg Cys Leu His Val  Asp Leu Leu
    1040                 1045                 1050

His Ser  Ile Leu Arg Ser Pro  Met Ser Phe Phe Glu  Arg Thr Pro
    1055                 1060                 1065

Ser Gly  Asn Leu Val Asn Arg  Phe Ser Lys Glu Leu  Asp Thr Val
    1070                 1075                 1080

Asp Ser  Met Ile Pro Glu Val  Ile Lys Met Phe Met  Gly Ser Leu
    1085                 1090                 1095

Phe Asn  Val Ile Gly Ala Cys  Ile Val Ile Leu Leu  Ala Thr Pro
    1100                 1105                 1110

Ile Ala  Ala Ile Ile Ile Pro  Pro Leu Gly Leu Ile  Tyr Phe Phe
    1115                 1120                 1125

Val Gln  Arg Phe Tyr Val Ala  Ser Ser Arg Gln Leu  Lys Arg Leu
    1130                 1135                 1140

Glu Ser  Val Ser Arg Ser Pro  Val Tyr Ser His Phe  Asn Glu Thr
    1145                 1150                 1155

Leu Leu  Gly Val Ser Val Ile  Arg Ala Phe Glu Glu  Gln Glu Arg
    1160                 1165                 1170

Phe Ile  His Gln Ser Asp Leu  Lys Val Asp Glu Asn  Gln Lys Ala
    1175                 1180                 1185

Tyr Tyr  Pro Ser Ile Val Ala  Asn Arg Trp Leu Ala  Val Arg Leu
    1190                 1195                 1200

Glu Cys  Val Gly Asn Cys Ile  Val Leu Phe Ala Ala  Leu Phe Ala
    1205                 1210                 1215

Val Ile  Ser Arg His Ser Leu  Ser Ala Gly Leu Val  Gly Leu Ser
    1220                 1225                 1230

Val Ser  Tyr Ser Leu Gln Val  Thr Thr Tyr Leu Asn  Trp Leu Val
    1235                 1240                 1245

Arg Met  Ser Ser Glu Met Glu  Thr Asn Ile Val Ala  Val Glu Arg
    1250                 1255                 1260

Leu Lys  Glu Tyr Ser Glu Thr  Glu Lys Glu Ala Pro  Trp Gln Ile
    1265                 1270                 1275

Gln Glu Thr Ala Pro Pro Ser Ser Trp Pro Gln Val Gly Arg Val
    1280                1285                1290

Glu Phe Arg Asn Tyr Cys Leu Arg Tyr Arg Glu Asp Leu Asp Phe
    1295                1300                1305

Val Leu Arg His Ile Asn Val Thr Ile Asn Gly Gly Glu Lys Val
    1310                1315                1320

Gly Ile Val Gly Arg Thr Gly Ala Gly Lys Ser Ser Leu Thr Leu
    1325                1330                1335

Gly Leu Phe Arg Ile Asn Glu Ser Ala Glu Gly Glu Ile Ile Ile
    1340                1345                1350

Asp Gly Ile Asn Ile Ala Lys Ile Gly Leu His Asp Leu Arg Phe
    1355                1360                1365

Lys Ile Thr Ile Ile Pro Gln Asp Pro Val Leu Phe Ser Gly Ser
    1370                1375                1380

Leu Arg Met Asn Leu Asp Pro Phe Ser Gln Tyr Ser Asp Glu Glu
    1385                1390                1395

Val Trp Thr Ser Leu Glu Leu Ala His Leu Lys Asp Phe Val Ser
    1400                1405                1410

Ala Leu Pro Asp Lys Leu Asp His Glu Cys Ala Glu Gly Gly Glu
    1415                1420                1425

Asn Leu Ser Val Gly Gln Arg Gln Leu Val Cys Leu Ala Arg Ala
    1430                1435                1440

Leu Leu Arg Lys Thr Lys Ile Leu Val Leu Asp Glu Ala Thr Ala
    1445                1450                1455

Ala Val Asp Leu Glu Thr Asp Asp Leu Ile Gln Ser Thr Ile Arg
    1460                1465                1470

Thr Gln Phe Glu Asp Cys Thr Val Leu Thr Ile Ala His Arg Leu
    1475                1480                1485

Asn Thr Ile Met Asp Tyr Thr Arg Val Ile Val Leu Asp Lys Gly
    1490                1495                1500

Glu Ile Gln Glu Tyr Gly Ala Pro Ser Asp Leu Leu Gln Gln Arg
    1505                1510                1515

Gly Leu Phe Tyr Ser Met Ala Lys Asp Ala Gly Leu Val
    1520                1525                1530

<210> 23
<211> 1290
<212> DNA
<213> homo sapiens

<400> 23
atggccggct ctggcgcgtg gaagcgcctc aaatctatgc taaggaagga tgatgcgccg 60

ctgtttttaa atgacaccag cgcctttgac ttctcggatg aggcggggga cgaggggctt 120

tctcggttca acaaacttcg agttgtggtg gccgatgacg gttccgaagc cccggaaagg 180

cctgttaacg gggcgcaccc gaccctccag gccgacgatg attccttact ggaccaagac 240

ttacctttga ccaacagtca gctgagtttg aaggtggact cctgtgacaa ctgcagcaaa 300

cagagagaga tactgaagca gagaaaggtg aaagccaggt tgaccattgc tgccgttctg 360

tacttgcttt tcatgattgg agaacttgta ggtggataca ttgcaaatag cctagcaatc 420

atgacagatg cacttcatat gttaactgac ctaagcgcca tcatactcac cctgcttgct 480

ttgtggctat catcaaaatc accaaccaaa agattcacct ttggatttca tcgcttagag 540

gttttgtcag ctatgattag tgtgctgttg gtgtatatac ttatgggatt cctcttatat 600

gaagctgtgc aaagaactat ccatatgaac tatgaaataa atggagatat aatgctcatc 660

accgcagctg ttggagttgc agttaatgta ataatggggt ttctgttgaa ccagtctggt 720

caccgtcact cccattccca ctccctgcct tcaaattccc ctaccagagg ttctgggtgt 780

gaacgtaacc atgggcagga tagcctggca gtgagagctg catttgtaca tgctttggga 840

gatttggtac agagtgttgg tgtgctaata gctgcataca tcatacgatt caagccagaa 900

tacaagattg ctgatcccat ctgtacatac gtattttcat tacttgtggc ttttacaaca 960

tttcgaatca tatgggatac agtagttata atactagaag gtgtgccaag ccatttgaat 1020

gtagactata tcaaagaagc cttgatgaaa atagaagatg tatattcagt cgaagattta 1080

aatatctggt ctctcacttc aggaaaatct actgccatag ttcacataca gctaattcct 1140

ggaagttcat ctaaatggga ggaagtacag tccaaagcaa accatttatt attgaacaca 1200

tttggcatgt atagatgtac tattcagctt cagagttaca ggcaagaagt ggacagaact 1260

tgtgcaaatt gtcagagttc tagtccctga 1290

<210> 24
<211> 429
<212> PRT
<213> homo sapiens

<400> 24

Met Ala Gly Ser Gly Ala Trp Lys Arg Leu Lys Ser Met Leu Arg Lys
1               5                   10                  15

Asp Asp Ala Pro Leu Phe Leu Asn Asp Thr Ser Ala Phe Asp Phe Ser
            20                  25                  30

Asp Glu Ala Gly Asp Glu Gly Leu Ser Arg Phe Asn Lys Leu Arg Val
        35                  40                  45

Val Val Ala Asp Asp Gly Ser Glu Ala Pro Glu Arg Pro Val Asn Gly
    50                  55                  60

Ala His Pro Thr Leu Gln Ala Asp Asp Asp Ser Leu Leu Asp Gln Asp
65                  70                  75                  80

Leu Pro Leu Thr Asn Ser Gln Leu Ser Leu Lys Val Asp Ser Cys Asp
                85                  90                  95

Asn Cys Ser Lys Gln Arg Glu Ile Leu Lys Gln Arg Lys Val Lys Ala
            100                 105                 110

Arg Leu Thr Ile Ala Ala Val Leu Tyr Leu Leu Phe Met Ile Gly Glu
        115                 120                 125

Leu Val Gly Gly Tyr Ile Ala Asn Ser Leu Ala Ile Met Thr Asp Ala
    130                 135                 140

Leu His Met Leu Thr Asp Leu Ser Ala Ile Ile Leu Thr Leu Leu Ala

145                 150                 155                 160

Leu Trp Leu Ser Ser Lys Ser Pro Thr Lys Arg Phe Thr Phe Gly Phe
                165                 170                 175

His Arg Leu Glu Val Leu Ser Ala Met Ile Ser Val Leu Leu Val Tyr
            180                 185                 190

Ile Leu Met Gly Phe Leu Leu Tyr Glu Ala Val Gln Arg Thr Ile His
        195                 200                 205

Met Asn Tyr Glu Ile Asn Gly Asp Ile Met Leu Ile Thr Ala Ala Val
    210                 215                 220

Gly Val Ala Val Asn Val Ile Met Gly Phe Leu Leu Asn Gln Ser Gly
225                 230                 235                 240

His Arg His Ser His Ser His Ser Leu Pro Ser Asn Ser Pro Thr Arg
                245                 250                 255

Gly Ser Gly Cys Glu Arg Asn His Gly Gln Asp Ser Leu Ala Val Arg
            260                 265                 270

Ala Ala Phe Val His Ala Leu Gly Asp Leu Val Gln Ser Val Gly Val
        275                 280                 285

Leu Ile Ala Ala Tyr Ile Ile Arg Phe Lys Pro Glu Tyr Lys Ile Ala
    290                 295                 300

Asp Pro Ile Cys Thr Tyr Val Phe Ser Leu Leu Val Ala Phe Thr Thr
305                 310                 315                 320

Phe Arg Ile Ile Trp Asp Thr Val Val Ile Ile Leu Glu Gly Val Pro
                325                 330                 335

Ser His Leu Asn Val Asp Tyr Ile Lys Glu Ala Leu Met Lys Ile Glu
            340                 345                 350

Asp Val Tyr Ser Val Glu Asp Leu Asn Ile Trp Ser Leu Thr Ser Gly
        355                 360                 365

Lys Ser Thr Ala Ile Val His Ile Gln Leu Ile Pro Gly Ser Ser Ser
    370                 375                 380

Lys Trp Glu Glu Val Gln Ser Lys Ala Asn His Leu Leu Leu Asn Thr
385                 390                 395                 400

Phe Gly Met Tyr Arg Cys Thr Ile Gln Leu Gln Ser Tyr Arg Gln Glu
                405                 410                 415

Val Asp Arg Thr Cys Ala Asn Cys Gln Ser Ser Ser Pro
            420                 425

<210>  25
<211>  4526
<212>  DNA
<213>  homo sapiens

<400>  25
gccgagtcgg tggcggctgc aggctgggag ggagaagtgc tacgcctttg caggttggcg      60

aagtggttcc aggctacccg gctagtctgg cacggccccg tcttctgcct cctcctccgt     120

cgcgtggcgg cgggaactgt tggccgcgcg gcctcgggaa cggcccaggt ccccgcccgc     180

aggtcccggg cagataacat agatcatcag tagaaaactt cttgaagttg ttcaagaaaa     240

atttgaaagt agcaaaatag aaaataaaga attaacagca gatacagagg acagcatgga     300

agtgttgtct taggaaacag aacacagcag tgaaaaaaca gacaaaatcc gctcagatac 360
aactgcagct gataatgttt tccggcttca atgtctttag agttgggatc tcttttgtca 420
taatgtgcat tttttacatg ccaacagtaa actctttacc agaactgagt cctcagaaat 480
attttagtac attgcaacca ggtcttgaag aactgaatga ggctgttaga cctctgcagg 540
actatggaat ttcagttgcc aaggttaatt gtgtcaaaga agaaatatca agatactgtg 600
gaaaagaaaa ggatttgatg aaagcatatt tattcaaggg caacatattg ctcagagaat 660
tccctactga caccttgttt gatgtgaatg ccattgtcgc ccatgttctc tttgctcttc 720
tttttagtga agtgaaatat attaccaacc tggaagacct tcagaacata gaaaatgctc 780
tgaaaggaaa agcaaatatt atattctcat atgtaagagc cattggaata ccagagcaca 840
gagcagtcat ggaagccggt tttgtgtatg ggactacata ccaatttgtc ttaaccacag 900
aaattgccct tttggaaagt attggctctg aggatgtgga atatgcacat ctctactttt 960
ttcattgtaa actagtcttg gacttgaccc agcaatgtag aagaacacta atggaacagc 1020
cattgactac actgaacatt cacctgttta ttaagacaat gaaagcacct ctgttgactg 1080
aagttgctga agatcctcaa caagtttcaa ctgtccatct ccaactgggc ttaccactgg 1140
tttttattgt tagccaacag gctacttatg aagctgatag aagaactgca gaatgggttg 1200
cttggcgtct tctgggaaaa gcaggagttc tactcttgtt aagggactct ttggaagtga 1260
acattcctca agatgctaat gtggtcttca aaagagcaga agagggagtt ccagtggaat 1320
ttttggtatt acatgatgtt gatttaataa tatctcatgt ggaaaataat atgcacattg 1380
aggaaataca agaagatgaa gacaatgaca tggaaggtcc agatatagat gttcaggatg 1440
atgaagtggc agaaactgtt ttcagagata ggaagagaaa attacctttg gaacttacag 1500
tggaactaac agaagaaaca tttaatgcaa cagtgatggc ttctgacagc atagtactct 1560
tctatgctgg ttggcaagca gtatccatgg catttttgca atcctatatt gatgtggcag 1620
ttaaactgaa aggcacatct actatgcttc ttactagaat aaactgtgca gattggtctg 1680
atgtatgtac taagcaaaat gttactgaat ttcctatcat aaagatgtac aagaaaggcg 1740
agaacccagt atcttatgct ggaatgttag gaaccaaaga tctcctaaaa tttatccagc 1800
tcaacaggat ttcatatcca gtgaatataa catcgatcca agaagcagaa gaatatttaa 1860
gtggggaatt atataaagac ctcatcttgt attctagtgt gtcagtattg ggactattta 1920
gtccaaccat gaaaacagca aaagaagatt ttagtgaagc aggaaactac ctaaaaggat 1980
atgttatcac tggaatttat tctgaagaag atgttttgct actgtcaacc aaatatgctg 2040
caagtcttcc agccctgctg cttgccagac acacagaagg caaaatagag agcatcccac 2100
tagctagcac acatgcacaa gacatagttc aaataataac agatgcacta ctggaaatgt 2160
ttccggaaat cactgtggaa aatcttccca gttatttcag acttcagaaa ccattattga 2220
ttttgttcag tgatggcact gtaaatcctc aatataaaaa agcaatattg acactggtaa 2280
agcagaaata cttggattca tttactccat gctggttaaa tctaaagaat actccagtgg 2340

ggagaggaat cttgcgggca tattttgatc ctctgcctcc ccttcctctt cttgttttgg 2400
tgaatctgca ttcaggtggc caagtatttg catttccttc agaccaggct ataattgaag 2460
aaaaccttgt attgtggctg aagaaattag aagcaggact agaaaatcat atcacaattt 2520
tacctgctca agaatggaaa cctcctcttc cagcttatga ttttctaagt atgatagatg 2580
ccgcaacatc tcaacgtggc actaggaaag ttcccaagtg tatgaaagaa acagatgtgc 2640
aggagaatga taaggaacaa catgaagata aatcggcagt cagaaaagaa ccgattgaaa 2700
ctctgagaat aaagcattgg aatagaagta attggtttaa agaagcagaa aaatcattta 2760
gacgtgataa agagttagga tgctcaaaag tgaactaatt ttatagggct gtggtttcca 2820
aaattttttt ggcatgatag acttaattta tttccttaaa gaataatatt aaatcatttc 2880
aagtttgcag actagtgcca tccaatagaa ttataatata agtcacatat tttatttaaa 2940
attttctagt aactacatta aacaaagtaa aagtgagcag ggcaaaataa ttttgatatt 3000
acttttcacc cagtagtata cccaaaatag cgaaatatag aaattattaa tgagatattt 3060
tacatccttt tttgtaccaa gtcttctaaa tgcagtacat attttatact tactgcattt 3120
cttacttccg agtagccata tttcaagtgt tcattgccac atgtggcctg tgactactgt 3180
attggacagt tcagtactag acaaaaacta gcataattaa cttagttcta gccatgattt 3240
ctatttggat taaaattaaa ctctaatcac agttaactcc acagtgcatt catgcagctg 3300
acagttatat ttgttttatt ggagtcatga tattaaaatc agcgtttgtc aacctcaggg 3360
gatatttagc aattgtcggg agacattttt gatgtcatga ctagggcagt tattgacatt 3420
tagtgagtag aggccatgga tcctgctaaa taacctgcat tggacagcgc cccacaacaa 3480
agaattatcc tgcccgaaat ggtagtcgtg ccaaggctga gtaaccttgt gttaaaagta 3540
acctgtggca gactaggttt ccagaatttc ctggttctgc tcacgtatca tgtttgaaaa 3600
aattttggct attaaagata tgtattagat ggtcttatcc tgattattac ctggatacaa 3660
cttgatcttt tctaatattt tcagaaagtg atgggataac cctagaagag gactcagaat 3720
gatatttata ttttaagtga gtcttaaaac ctcctcttat ttctacaagt tatatggcta 3780
aatttcagat tgaacaggga ttcagcattc tgccatctcc tcatggaaag agaggctccc 3840
tcatctgaag cgtctctgaa atctaccctt gcaagcttca gacaaatcag ttgatctccc 3900
tgagccacac ggcctcattc tgtgagggag ggaaagatta gccaaagagt taattttcat 3960
tccaaatcac ttagctgtta gactgatctg tttgtagcag ttgtttgtct catttttgct 4020
ctgtgcattt tttgagacat ttgttgagaa tattctattt ggtgctctac tgtatttttc 4080
tttttaatat ctacttgata tcttgttctt taaattttct tcacatatgg tttgcctgat 4140
acaactgatt tttataactg aaatttaagg aatctaacag ctaaaactca gtaagtgcat 4200
mtatttcctt ataacataga cccgttgcta ctctcagcac cctctcctca attttttttc 4260
ctgtagcatg tgatgcctga ttaaactcat tttcatttgc ttttatttct aatatgggaa 4320
caatgagagt gaactctaaa tataggttgt agtaataaaa catcattagc ctaattatta 4380

gaaaatgcta attaagtacc agcacataga aacatgaaat tgcttagtca ttgtaccttt 4440

gtcagcaatt ttgacagtca ttaatgtttg tcataatttt aaataaagtg tctgggtttc 4500

agaatacctt caaaaaaaaa aaaaaa 4526

<210> 26
<211> 807
<212> PRT
<213> homo sapiens

<400> 26

Met Phe Ser Gly Phe Asn Val Phe Arg Val Gly Ile Ser Phe Val Ile
1               5                   10                  15

Met Cys Ile Phe Tyr Met Pro Thr Val Asn Ser Leu Pro Glu Leu Ser
            20                  25                  30

Pro Gln Lys Tyr Phe Ser Thr Leu Gln Pro Gly Leu Glu Glu Leu Asn
        35                  40                  45

Glu Ala Val Arg Pro Leu Gln Asp Tyr Gly Ile Ser Val Ala Lys Val
    50                  55                  60

Asn Cys Val Lys Glu Glu Ile Ser Arg Tyr Cys Gly Lys Glu Lys Asp
65                  70                  75                  80

Leu Met Lys Ala Tyr Leu Phe Lys Gly Asn Ile Leu Leu Arg Glu Phe
                85                  90                  95

Pro Thr Asp Thr Leu Phe Asp Val Asn Ala Ile Val Ala His Val Leu
            100                 105                 110

Phe Ala Leu Leu Phe Ser Glu Val Lys Tyr Ile Thr Asn Leu Glu Asp
        115                 120                 125

Leu Gln Asn Ile Glu Asn Ala Leu Lys Gly Lys Ala Asn Ile Ile Phe
    130                 135                 140

Ser Tyr Val Arg Ala Ile Gly Ile Pro Glu His Arg Ala Val Met Glu
145                 150                 155                 160

Ala Gly Phe Val Tyr Gly Thr Thr Tyr Gln Phe Val Leu Thr Thr Glu
                165                 170                 175

Ile Ala Leu Leu Glu Ser Ile Gly Ser Glu Asp Val Glu Tyr Ala His
            180                 185                 190

Leu Tyr Phe Phe His Cys Lys Leu Val Leu Asp Leu Thr Gln Gln Cys
        195                 200                 205

Arg Arg Thr Leu Met Glu Gln Pro Leu Thr Thr Leu Asn Ile His Leu
    210                 215                 220

Phe Ile Lys Thr Met Lys Ala Pro Leu Leu Thr Glu Val Ala Glu Asp
225                 230                 235                 240

Pro Gln Gln Val Ser Thr Val His Leu Gln Leu Gly Leu Pro Leu Val
                245                 250                 255

Phe Ile Val Ser Gln Gln Ala Thr Tyr Glu Ala Asp Arg Arg Thr Ala
            260                 265                 270

Glu Trp Val Ala Trp Arg Leu Leu Gly Lys Ala Gly Val Leu Leu Leu
        275                 280                 285

Leu Arg Asp Ser Leu Glu Val Asn Ile Pro Gln Asp Ala Asn Val Val
    290                 295                 300

Phe Lys Arg Ala Glu Glu Gly Val Pro Val Glu Phe Leu Val Leu His
305                 310                 315                 320

Asp Val Asp Leu Ile Ile Ser His Val Glu Asn Asn Met His Ile Glu
                325                 330                 335

Glu Ile Gln Glu Asp Glu Asp Asn Asp Met Glu Gly Pro Asp Ile Asp
            340                 345                 350

Val Gln Asp Asp Glu Val Ala Glu Thr Val Phe Arg Asp Arg Lys Arg
        355                 360                 365

Lys Leu Pro Leu Glu Leu Thr Val Glu Leu Thr Glu Glu Thr Phe Asn
    370                 375                 380

Ala Thr Val Met Ala Ser Asp Ser Ile Val Leu Phe Tyr Ala Gly Trp
385                 390                 395                 400

Gln Ala Val Ser Met Ala Phe Leu Gln Ser Tyr Ile Asp Val Ala Val
                405                 410                 415

Lys Leu Lys Gly Thr Ser Thr Met Leu Leu Thr Arg Ile Asn Cys Ala
            420                 425                 430

Asp Trp Ser Asp Val Cys Thr Lys Gln Asn Val Thr Glu Phe Pro Ile
        435                 440                 445

Ile Lys Met Tyr Lys Lys Gly Glu Asn Pro Val Ser Tyr Ala Gly Met
    450                 455                 460

Leu Gly Thr Lys Asp Leu Leu Lys Phe Ile Gln Leu Asn Arg Ile Ser
465                 470                 475                 480

Tyr Pro Val Asn Ile Thr Ser Ile Gln Glu Ala Glu Glu Tyr Leu Ser
                485                 490                 495

Gly Glu Leu Tyr Lys Asp Leu Ile Leu Tyr Ser Ser Val Ser Val Leu
            500                 505                 510

Gly Leu Phe Ser Pro Thr Met Lys Thr Ala Lys Glu Asp Phe Ser Glu
        515                 520                 525

Ala Gly Asn Tyr Leu Lys Gly Tyr Val Ile Thr Gly Ile Tyr Ser Glu
    530                 535                 540

Glu Asp Val Leu Leu Leu Ser Thr Lys Tyr Ala Ala Ser Leu Pro Ala
545                 550                 555                 560

Leu Leu Leu Ala Arg His Thr Glu Gly Lys Ile Glu Ser Ile Pro Leu
                565                 570                 575

Ala Ser Thr His Ala Gln Asp Ile Val Gln Ile Ile Thr Asp Ala Leu
            580                 585                 590

Leu Glu Met Phe Pro Glu Ile Thr Val Glu Asn Leu Pro Ser Tyr Phe
        595                 600                 605

Arg Leu Gln Lys Pro Leu Leu Ile Leu Phe Ser Asp Gly Thr Val Asn
    610                 615                 620

Pro Gln Tyr Lys Lys Ala Ile Leu Thr Leu Val Lys Gln Lys Tyr Leu
625                 630                 635                 640

Asp Ser Phe Thr Pro Cys Trp Leu Asn Leu Lys Asn Thr Pro Val Gly
                645                 650                 655

Arg Gly Ile Leu Arg Ala Tyr Phe Asp Pro Leu Pro Pro Leu Pro Leu
            660                 665                 670

Leu Val Leu Val Asn Leu His Ser Gly Gly Gln Val Phe Ala Phe Pro
        675                 680                 685

Ser Asp Gln Ala Ile Ile Glu Glu Asn Leu Val Leu Trp Leu Lys Lys
    690                 695                 700

Leu Glu Ala Gly Leu Glu Asn His Ile Thr Ile Leu Pro Ala Gln Glu
705                 710                 715                 720

Trp Lys Pro Pro Leu Pro Ala Tyr Asp Phe Leu Ser Met Ile Asp Ala
                725                 730                 735

Ala Thr Ser Gln Arg Gly Thr Arg Lys Val Pro Lys Cys Met Lys Glu
            740                 745                 750

Thr Asp Val Gln Glu Asn Asp Lys Glu Gln His Glu Asp Lys Ser Ala
        755                 760                 765

Val Arg Lys Glu Pro Ile Glu Thr Leu Arg Ile Lys His Trp Asn Arg
    770                 775                 780

Ser Asn Trp Phe Lys Glu Ala Glu Lys Ser Phe Arg Arg Asp Lys Glu
785                 790                 795                 800

Leu Gly Cys Ser Lys Val Asn
                805

<210>  27
<211>  1195
<212>  DNA
<213>  Homo sapiens

<400>  27
ccgagactca cggtcaagct aaggcgaaga gtgggtggct gaagccatac tattttatag      60

aattaatgga aagcagaaaa gacatcacaa accaagaaga actttggaaa atgaagccta     120

ggagaaattt agaagaagac gattatttgc ataaggacac gggagagacc agcatgctaa     180

aaagacctgt gcttttgcat ttgcaccaaa cagcccatgc tgatgaattt gactgccctt     240

cagaacttca gcacacacag gaactctttc cacagtggca cttgccaatt aaaatagctg     300

ctattatagc atctctgact tttctttaca ctcttctgag ggaagtaatt caccctttag     360

caacttccca tcaacaatat ttttataaaa ttccaatcct ggtcatcaac aaagtcttgc     420

caatggtttc catcactctc ttggcattgg tttacctgcc aggtgtgata gcagcaattg     480

tccaacttca taatggaacc aagtataaga agtttccaca ttggttggat aagtggatgt     540

taacaagaaa gcagtttggg cttctcagtt tcttttttgc tgtactgcat gcaatttata     600

gtctgtctta cccaatgagg cgatcctaca gatacaagtt gctaaactgg gcatatcaac     660

aggtccaaca aaataaagaa gatgcctgga ttgagcatga tgtttggaga atggagattt     720

atgtgtctct gggaattgtg ggattggcaa tactggctct gttggctgtg acatctattc     780

catctgtgag tgactctttg acatggagag aatttcacta tattcagagc aagctaggaa     840

ttgtttccct tctactgggc acaatacacg cattgatttt tgcctggaat aagtggatag     900

atataaaaca atttgtatgg tatacacctc caacttttat gatagctgtt ttccttccaa     960

ttgttgtcct gatatttaaa agcatactat tcctgccatg cttgaggaag aagatactga 1020

agattagaca tggttgggaa gacgtcacca aaattaacaa aactgagata tgttcccagt 1080

tgtagaatta ctgtttacac acatttttgt tcaatattga tatattttat caccaacatt 1140

tcaagtttgt atttgttaat aaaatgatta ttcaaggaaa aaaaaaaaaa aaaaa 1195

<210> 28
<211> 339
<212> PRT
<213> Homo sapiens

<400> 28

Met Glu Ser Arg Lys Asp Ile Thr Asn Gln Glu Glu Leu Trp Lys Met
1               5                   10                  15

Lys Pro Arg Arg Asn Leu Glu Glu Asp Asp Tyr Leu His Lys Asp Thr
            20                  25                  30

Gly Glu Thr Ser Met Leu Lys Arg Pro Val Leu Leu His Leu His Gln
        35                  40                  45

Thr Ala His Ala Asp Glu Phe Asp Cys Pro Ser Glu Leu Gln His Thr
    50                  55                  60

Gln Glu Leu Phe Pro Gln Trp His Leu Pro Ile Lys Ile Ala Ala Ile
65                  70                  75                  80

Ile Ala Ser Leu Thr Phe Leu Tyr Thr Leu Leu Arg Glu Val Ile His
                85                  90                  95

Pro Leu Ala Thr Ser His Gln Gln Tyr Phe Tyr Lys Ile Pro Ile Leu
            100                 105                 110

Val Ile Asn Lys Val Leu Pro Met Val Ser Ile Thr Leu Leu Ala Leu
        115                 120                 125

Val Tyr Leu Pro Gly Val Ile Ala Ala Ile Val Gln Leu His Asn Gly
    130                 135                 140

Thr Lys Tyr Lys Lys Phe Pro His Trp Leu Asp Lys Trp Met Leu Thr
145                 150                 155                 160

Arg Lys Gln Phe Gly Leu Leu Ser Phe Phe Phe Ala Val Leu His Ala
                165                 170                 175

Ile Tyr Ser Leu Ser Tyr Pro Met Arg Arg Ser Tyr Arg Tyr Lys Leu
            180                 185                 190

Leu Asn Trp Ala Tyr Gln Gln Val Gln Gln Asn Lys Glu Asp Ala Trp
        195                 200                 205

Ile Glu His Asp Val Trp Arg Met Glu Ile Tyr Val Ser Leu Gly Ile
    210                 215                 220

Val Gly Leu Ala Ile Leu Ala Leu Leu Ala Val Thr Ser Ile Pro Ser
225                 230                 235                 240

Val Ser Asp Ser Leu Thr Trp Arg Glu Phe His Tyr Ile Gln Ser Lys
                245                 250                 255

Leu Gly Ile Val Ser Leu Leu Leu Gly Thr Ile His Ala Leu Ile Phe
            260                 265                 270

Ala Trp Asn Lys Trp Ile Asp Ile Lys Gln Phe Val Trp Tyr Thr Pro
        275                 280                 285

Pro Thr Phe Met Ile Ala Val Phe Leu Pro Ile Val Val Leu Ile Phe
    290                 295                 300

Lys Ser Ile Leu Phe Leu Pro Cys Leu Arg Lys Lys Ile Leu Lys Ile
305                 310                 315                 320

Arg His Gly Trp Glu Asp Val Thr Lys Ile Asn Lys Thr Glu Ile Cys
                325                 330                 335

Ser Gln Leu

<210>  29
<211>  2541
<212>  DNA
<213>  Homo sapiens

<400>  29
atgagttcct gcaacttcac acatgccacc tttgtgctta ttggtatccc aggattagag      60

aaagcccatt tctgggttgg cttccccctc ctttccatgt atgtagtggc aatgtttgga      120

aactgcatcg tggtcttcat cgtaaggacg gaacgcagcc tgcacgctcc gatgtacctc      180

tttctctgca tgcttgcagc cattgacctg gccttatcca catccaccat gcctaagatc      240

cttgcccttt tctggtttga ttcccgagag attagctttg aggcctgtct tacccagatg      300

ttctttattc atgccctctc agccattgaa tccaccatcc tgctggccat ggcctttgac      360

cgttatgtgg ccatctgcca cccactgcgc catgctgcag tgctcaacaa tacagtaaca      420

gcccagattg gcatcgtggc tgtggtccgc ggatccctct tttttttccc actgcctctg      480

ctgatcaagc ggctggcctt ctgccactcc aatgtcctct cgcactccta ttgtgtccac      540

caggatgtaa tgaagttggc ctatgcagac actttgccca atgtggtata tggtcttact      600

gccattctgc tggtcatggg cgtggacgta atgttcatct ccttgtccta ttttctgata      660

atacgaacgg ttctgcaact gccttccaag tcagagcggg ccaaggcctt tggaacctgt      720

gtgtcacaca ttggtgtggt actcgccttc tatgtgccac ttattggcct ctcagtggta      780

caccgctttg gaaacagcct tcatcccatt gtgcgtgttg tcatgggtga catctacctg      840

ctgctgcctc ctgtcatcaa tcccatcatc tatggtgcca aaaccaaaca gatcagaaca      900

cgggtgctgg ctatgttcaa gatcagctgt gacaaggact tgcaggctgt gggaggcaag      960

tgacccttaa cactacactt ctccttatct ttattggctt gataaacata attatttcta     1020

acactagctt atttccagtt gcccataagc acatcagtac ttttctctgg ctggaatagt     1080

aaactaaagt atggtacatc tacctaaagg actattatgt ggaataatac atactaatga     1140

agtattacat gatttaaaga ctacaataaa accaaacatg cttataacat taagaaaaac     1200

aataaagata catgattgaa accaagttga aaaatagcat atgccttgga ggaaatgtgc     1260

tcaaattact aatgatttag tgttgtccct actttctctc tcttttttct ttcttttttt     1320

tttattatgg ttagctgtca catacaactt tttttttttt tgagatgggg tctcgctctg     1380

tcaccaggct ggagtgcagt ggcgcgatct cggctcactg caacctccac atcccatgtt     1440

gaagtaattc ttctgcctca gcctcccgag tagctgggac tagaggaacg tgccaccatg 1500
actggctaat tttctgtatt ttttagtaga gacagagttt caccatgttg gccaggatgg 1560
tctcgatctc ctgaccttgt gatccacccg cctcagcctc ccaaagtgtt gggattacag 1620
gtgtgaacca ctgtgcccgg cctgtgtaca actttttaaa tagggaatat gatagcttcg 1680
catggtggtg tgcacctata gcccccactg cctggaaagc tgaggtggga gaatcgcttg 1740
agtccaggag tttgaggtta cagtgatcca cgatcgtacc actacactcc agcctgggca 1800
acagagcaag accctgtctc aaagcataaa atggaataac atatcaaatg aaacagggaa 1860
aatgaagctg acaatttatg gaagccaggg cttgtcacag tctctactgt tattatgcat 1920
tacctgggaa tttatataag cccttaataa tartgccaat gaacatctca tgtgtgctca 1980
caatgttctg gcactattat aagtgcttca caggttttat gtgttcttcg taactttatg 2040
gagtaggtac catttgtgtc tctttattat aagtgagaga aatgaagttt atattatcaa 2100
ggggactaaa gtcacacggc ttgtgggcac tgtgccaaga tttaaaatta aatttgatgg 2160
ttgaatacag ttacttaatg accatgttat attgcttcct gtgtaacatc tgccatttat 2220
ttcctcagct gtacaaatcc tctgttttct ctctgttaca cactaacatc aatggctttg 2280
tacttgtgat gagagataac cttgccctag ttgtgggcaa cacatgcaga ataatcctgt 2340
tttacagctg cctttcgtga tcttattgct tgcttttttc cagattcagg gagaatgttg 2400
ttgtctattt gtctcttaca tctccttgat catgtcttca ttttttaatg tgctctgtac 2460
ctgtcaaaaa ttttgaatgt acaccacatg ctattgtctg aacttgagta taagataaaa 2520
taaaatttta ttttaaattt t 2541

<210> 30
<211> 320
<212> PRT
<213> Homo sapiens

<400> 30

Met Ser Ser Cys Asn Phe Thr His Ala Thr Phe Val Leu Ile Gly Ile
1               5                   10                  15

Pro Gly Leu Glu Lys Ala His Phe Trp Val Gly Phe Pro Leu Leu Ser
            20                  25                  30

Met Tyr Val Val Ala Met Phe Gly Asn Cys Ile Val Val Phe Ile Val
        35                  40                  45

Arg Thr Glu Arg Ser Leu His Ala Pro Met Tyr Leu Phe Leu Cys Met
    50                  55                  60

Leu Ala Ala Ile Asp Leu Ala Leu Ser Thr Ser Thr Met Pro Lys Ile
65                  70                  75                  80

Leu Ala Leu Phe Trp Phe Asp Ser Arg Glu Ile Ser Phe Glu Ala Cys
                85                  90                  95

Leu Thr Gln Met Phe Phe Ile His Ala Leu Ser Ala Ile Glu Ser Thr
            100                 105                 110

Ile Leu Leu Ala Met Ala Phe Asp Arg Tyr Val Ala Ile Cys His Pro

115                 120                 125

Leu Arg His Ala Ala Val Leu Asn Asn Thr Val Thr Ala Gln Ile Gly
    130                 135                 140

Ile Val Ala Val Val Arg Gly Ser Leu Phe Phe Phe Pro Leu Pro Leu
145                 150                 155                 160

Leu Ile Lys Arg Leu Ala Phe Cys His Ser Asn Val Leu Ser His Ser
                165                 170                 175

Tyr Cys Val His Gln Asp Val Met Lys Leu Ala Tyr Ala Asp Thr Leu
            180                 185                 190

Pro Asn Val Val Tyr Gly Leu Thr Ala Ile Leu Leu Val Met Gly Val
        195                 200                 205

Asp Val Met Phe Ile Ser Leu Ser Tyr Phe Leu Ile Ile Arg Thr Val
    210                 215                 220

Leu Gln Leu Pro Ser Lys Ser Glu Arg Ala Lys Ala Phe Gly Thr Cys
225                 230                 235                 240

Val Ser His Ile Gly Val Val Leu Ala Phe Tyr Val Pro Leu Ile Gly
                245                 250                 255

Leu Ser Val Val His Arg Phe Gly Asn Ser Leu His Pro Ile Val Arg
            260                 265                 270

Val Val Met Gly Asp Ile Tyr Leu Leu Leu Pro Pro Val Ile Asn Pro
        275                 280                 285

Ile Ile Tyr Gly Ala Lys Thr Lys Gln Ile Arg Thr Arg Val Leu Ala
    290                 295                 300

Met Phe Lys Ile Ser Cys Asp Lys Asp Leu Gln Ala Val Gly Gly Lys
305                 310                 315                 320

<210>  31
<211>  1020
<212>  DNA
<213>  Homo sapiens

<400>  31
atgaactggg agctgctgct gtggctgctg gtgctgtgcg cgctgctcct gctcttggtg      60

cagctgctgc gcttcctgag ggctgacggc gacctgacgc tactatgggc cgagtggcag     120

ggacgacgcc cagaatggga gctgactgat atggtggtgt gggtgactgg agcctcgagt     180

ggaattggtg aggagctggc ttaccagttg tctaaactag gagtttctct tgtgctgtca     240

gccagaagag tgcatgagct ggaaagggtg aaaagaagat gcctagagaa tggcaattta     300

aaagaaaaag atatacttgt tttgcccctt gacctgaccg acactggttc ccatgaagcg     360

gctaccaaag ctgttctcca ggagtttggt agaatcgaca ttctggtcaa caatggtgga     420

atgtcccagc gttctctgtg catggatacc agcttggatg tctacagaaa gctaatagag     480

cttaactact tagggacggt gtccttgaca aaatgtgttc tgcctcacat gatcgagagg     540

aagcaaggaa agattgttac tgtgaatagc atcctgggta tcatatctgt acctctttcc     600

attggatact gtgctagcaa gcatgctctc cggggttttt ttaatggcct tcgaacagaa     660

cttgccacat acccaggtat aatagtttct aacatttgcc caggacctgt gcaatcaaat     720

attgtggaga attccctagc tggagaagtc acaaagacta taggcaataa tggagaccag 780

tcccacaaga tgacaaccag tcgttgtgtg cggctgatgt taatcagcat ggccaatgat 840

ttgaaagaag tttggatctc agaacaacct ttcttgttag taacatattt gtggcaatac 900

atgccaacct gggcctggtg gataaccaac aagatgggga agaaaaggat tgagaacttt 960

aagagtggtg tggatgcaga ctcttcttat tttaaaatct ttaagacaaa acatgactga 1020

<210> 32
<211> 339
<212> PRT
<213> Homo sapiens

<400> 32

Met Asn Trp Glu Leu Leu Leu Trp Leu Leu Val Leu Cys Ala Leu Leu
1               5                   10                  15

Leu Leu Leu Val Gln Leu Leu Arg Phe Leu Arg Ala Asp Gly Asp Leu
            20                  25                  30

Thr Leu Leu Trp Ala Glu Trp Gln Gly Arg Arg Pro Glu Trp Glu Leu
        35                  40                  45

Thr Asp Met Val Val Trp Val Thr Gly Ala Ser Ser Gly Ile Gly Glu
    50                  55                  60

Glu Leu Ala Tyr Gln Leu Ser Lys Leu Gly Val Ser Leu Val Leu Ser
65                  70                  75                  80

Ala Arg Arg Val His Glu Leu Glu Arg Val Lys Arg Arg Cys Leu Glu
                85                  90                  95

Asn Gly Asn Leu Lys Glu Lys Asp Ile Leu Val Leu Pro Leu Asp Leu
            100                 105                 110

Thr Asp Thr Gly Ser His Glu Ala Ala Thr Lys Ala Val Leu Gln Glu
        115                 120                 125

Phe Gly Arg Ile Asp Ile Leu Val Asn Asn Gly Gly Met Ser Gln Arg
    130                 135                 140

Ser Leu Cys Met Asp Thr Ser Leu Asp Val Tyr Arg Lys Leu Ile Glu
145                 150                 155                 160

Leu Asn Tyr Leu Gly Thr Val Ser Leu Thr Lys Cys Val Leu Pro His
                165                 170                 175

Met Ile Glu Arg Lys Gln Gly Lys Ile Val Thr Val Asn Ser Ile Leu
            180                 185                 190

Gly Ile Ile Ser Val Pro Leu Ser Ile Gly Tyr Cys Ala Ser Lys His
        195                 200                 205

Ala Leu Arg Gly Phe Phe Asn Gly Leu Arg Thr Glu Leu Ala Thr Tyr
    210                 215                 220

Pro Gly Ile Ile Val Ser Asn Ile Cys Pro Gly Pro Val Gln Ser Asn
225                 230                 235                 240

Ile Val Glu Asn Ser Leu Ala Gly Glu Val Thr Lys Thr Ile Gly Asn
                245                 250                 255

Asn Gly Asp Gln Ser His Lys Met Thr Thr Ser Arg Cys Val Arg Leu
            260                 265                 270

Met Leu Ile Ser Met Ala Asn Asp Leu Lys Glu Val Trp Ile Ser Glu
        275                 280                 285

Gln Pro Phe Leu Leu Val Thr Tyr Leu Trp Gln Tyr Met Pro Thr Trp
    290                 295                 300

Ala Trp Trp Ile Thr Asn Lys Met Gly Lys Lys Arg Ile Glu Asn Phe
305                 310                 315                 320

Lys Ser Gly Val Asp Ala Asp Ser Ser Tyr Phe Lys Ile Phe Lys Thr
                325                 330                 335

Lys His Asp

<210>  33
<211>  1186
<212>  DNA
<213>  Homo sapiens

<400>  33
aggaatctgc gctcgggttc cgcagatgca gaggttgagg tggctgcggg actggaagtc      60

atcgggcaga ggtctcacag cagccaagga acctggggcc cgctcctccc ccctccaggc     120

catgaggatt ctgcagttaa tcctgcttgc tctggcaaca gggcttgtag ggggagagac     180

caggatcatc aaggggttcg agtgcaagcc tcactcccag ccctggcagg cagccctgtt     240

cgagaagacg cggctactct gtggggcgac gctcatcgcc cccagatggc tcctgacagc     300

agcccactgc ctcaagcccc gctacatagt tcacctgggg cagcacaacc tccagaagga     360

ggagggctgt gagcagaccc ggacagccac tgagtccttc ccccaccccg gcttcaacaa     420

cagcctcccc aacaaagacc accgcaatga catcatgctg gtgaagatgg catcgccagt     480

ctccatcacc tgggctgtgc gacccctcac cctctcctca cgctgtgtca ctgctggcac     540

cagctgcctc atttccggct ggggcagcac gtccagcccc cagttacgcc tgcctcacac     600

cttgcgatgc gccaacatca ccatcattga gcaccagaag tgtgagaacg cctaccccgg     660

caacatcaca gacaccatgg tgtgtgccag cgtgcaggaa gggggcaagg actcctgcca     720

gggtgactcc gggggccctc tggtctgtaa ccagtctctt caaggcatta tctcctgggg     780

ccaggatccg tgtgcgatca cccgaaagcc tggtgtctac acgaaagtct gcaaatatgt     840

ggactggatc caggagacga tgaagaacaa ttagactgga cccacccacc acagcccatc     900

accctccatt tccacttggt gtttggttcc tgttcactct gttaataaga aaccctaagc     960

caagaccctc tacgaacatt ctttgggcct cctggactac aggagatgct gtcacttaat    1020

aatcaacctg gggttcgaaa tcagtgagac ctggattcaa attctgcctt gaaatattgt    1080

gactctggga atgacaacac ctggtttgtt ctctgttgta tccccagccc caaagacagc    1140

tcctggccat atatcaaggt ttcaataaat atttgctaaa tgagtg                   1186

<210>  34
<211>  250
<212>  PRT
<213>  Homo sapiens

<400>  34

Met Arg Ile Leu Gln Leu Ile Leu Leu Ala Leu Ala Thr Gly Leu Val
1               5                   10                  15

Gly Gly Glu Thr Arg Ile Ile Lys Gly Phe Glu Cys Lys Pro His Ser
            20                  25                  30

Gln Pro Trp Gln Ala Ala Leu Phe Glu Lys Thr Arg Leu Leu Cys Gly
        35                  40                  45

Ala Thr Leu Ile Ala Pro Arg Trp Leu Leu Thr Ala Ala His Cys Leu
    50                  55                  60

Lys Pro Arg Tyr Ile Val His Leu Gly Gln His Asn Leu Gln Lys Glu
65                  70                  75                  80

Glu Gly Cys Glu Gln Thr Arg Thr Ala Thr Glu Ser Phe Pro His Pro
                85                  90                  95

Gly Phe Asn Asn Ser Leu Pro Asn Lys Asp His Arg Asn Asp Ile Met
            100                 105                 110

Leu Val Lys Met Ala Ser Pro Val Ser Ile Thr Trp Ala Val Arg Pro
        115                 120                 125

Leu Thr Leu Ser Ser Arg Cys Val Thr Ala Gly Thr Ser Cys Leu Ile
    130                 135                 140

Ser Gly Trp Gly Ser Thr Ser Ser Pro Gln Leu Arg Leu Pro His Thr
145                 150                 155                 160

Leu Arg Cys Ala Asn Ile Thr Ile Ile Glu His Gln Lys Cys Glu Asn
                165                 170                 175

Ala Tyr Pro Gly Asn Ile Thr Asp Thr Met Val Cys Ala Ser Val Gln
            180                 185                 190

Glu Gly Gly Lys Asp Ser Cys Gln Gly Asp Ser Gly Gly Pro Leu Val
        195                 200                 205

Cys Asn Gln Ser Leu Gln Gly Ile Ile Ser Trp Gly Gln Asp Pro Cys
    210                 215                 220

Ala Ile Thr Arg Lys Pro Gly Val Tyr Thr Lys Val Cys Lys Tyr Val
225                 230                 235                 240

Asp Trp Ile Gln Glu Thr Met Lys Asn Asn
                245                 250

<210>  35
<211>  1233
<212>  DNA
<213>  Homo sapiens

<400>  35
ctaaagctct cttgctgcct agcctcctgc cggcctcatc ttcgcccagc caaccccgcc      60

tggagcccta tggccaactg cgagttcagc ccggtgtccg gggacaaacc ctgctgccgg     120

ctctctagga gagcccaact ctgtcttggc gtcagtatcc tggtcctgat cctcgtcgtg     180

gtgctcgcgg tggtcgtccc gaggtggcgc cagacgtgga gcggtccggg caccaccaag     240

cgctttcccg agaccgtcct ggcgcgatgc gtcaagtaca ctgaaattca tcctgagatg     300

agacatgtag actgccaaag tgtatgggat gctttcaagg gtgcatttat ttcaaaacat     360

ccttgcaaca ttactgaaga agactatcag ccactaatga agttgggaac tcagaccgta     420

ccttgcaaca agattcttct ttggagcaga ataaaagatc tggcccatca gttcacacag 480

gtccagcggg acatgttcac cctggaggac acgctgctag gctaccttgc tgatgacctc 540

acatggtgtg gtgaattcaa cacttccaaa ataaactatc aatcttgccc agactggaga 600

aaggactgca gcaacaaccc tgtttcagta ttctggaaaa cggtttcccg caggtttgca 660

gaagctgcct gtgatgtggt ccatgtgatg ctcaatggat cccgcagtaa aatctttgac 720

aaaaacagca cttttgggag tgtggaagtc cataatttgc aaccagagaa ggttcagaca 780

ctagaggcct gggtgataca tggtggaaga gaagattcca gagacttatg ccaggatccc 840

accataaaag agctggaatc gattataagc aaaaggaata ttcaattttc ctgcaagaat 900

atctacagac ctgacaagtt tcttcagtgt gtgaaaaatc ctgaggattc atcttgcaca 960

tctgagatct gagccagtcg ctgtggttgt tttagctcct tgactccttg tggtttatgt 1020

catcatacat gactcagcat acctgctggt gcagagctga agattttgga gggtcctcca 1080

caataaggtc aatgccagag acggaagcct ttttccccaa agtcttaaaa taacttatat 1140

catcagcata cctttattgt gatctatcaa tagtcaagaa aaattattgt ataagattag 1200

aatgaaaatt gtatgttaag ttacttcctt tag 1233

<210> 36
<211> 300
<212> PRT
<213> Homo sapiens

<400> 36

Met Ala Asn Cys Glu Phe Ser Pro Val Ser Gly Asp Lys Pro Cys Cys
1               5                   10                  15

Arg Leu Ser Arg Arg Ala Gln Leu Cys Leu Gly Val Ser Ile Leu Val
            20                  25                  30

Leu Ile Leu Val Val Val Leu Ala Val Val Val Pro Arg Trp Arg Gln
        35                  40                  45

Thr Trp Ser Gly Pro Gly Thr Thr Lys Arg Phe Pro Glu Thr Val Leu
    50                  55                  60

Ala Arg Cys Val Lys Tyr Thr Glu Ile His Pro Glu Met Arg His Val
65                  70                  75                  80

Asp Cys Gln Ser Val Trp Asp Ala Phe Lys Gly Ala Phe Ile Ser Lys
                85                  90                  95

His Pro Cys Asn Ile Thr Glu Glu Asp Tyr Gln Pro Leu Met Lys Leu
            100                 105                 110

Gly Thr Gln Thr Val Pro Cys Asn Lys Ile Leu Leu Trp Ser Arg Ile
        115                 120                 125

Lys Asp Leu Ala His Gln Phe Thr Gln Val Gln Arg Asp Met Phe Thr
    130                 135                 140

Leu Glu Asp Thr Leu Leu Gly Tyr Leu Ala Asp Asp Leu Thr Trp Cys
145                 150                 155                 160

Gly Glu Phe Asn Thr Ser Lys Ile Asn Tyr Gln Ser Cys Pro Asp Trp
                165                 170                 175

Arg Lys Asp Cys Ser Asn Asn Pro Val Ser Val Phe Trp Lys Thr Val
            180                 185                 190

Ser Arg Arg Phe Ala Glu Ala Ala Cys Asp Val Val His Val Met Leu
        195                 200                 205

Asn Gly Ser Arg Ser Lys Ile Phe Asp Lys Asn Ser Thr Phe Gly Ser
    210                 215                 220

Val Glu Val His Asn Leu Gln Pro Glu Lys Val Gln Thr Leu Glu Ala
225                 230                 235                 240

Trp Val Ile His Gly Gly Arg Glu Asp Ser Arg Asp Leu Cys Gln Asp
                245                 250                 255

Pro Thr Ile Lys Glu Leu Glu Ser Ile Ile Ser Lys Arg Asn Ile Gln
            260                 265                 270

Phe Ser Cys Lys Asn Ile Tyr Arg Pro Asp Lys Phe Leu Gln Cys Val
        275                 280                 285

Lys Asn Pro Glu Asp Ser Ser Cys Thr Ser Glu Ile
    290                 295                 300

<210>  37
<211>  3315
<212>  DNA
<213>  Homo sapiens

<400>  37
atgtcctttc gggcagccag gctcagcatg aggaacagaa ggaatgacac tctggacagc      60

acccggaccc tgtactccag cgcgtctcgg agcacagact tgtcttacag tgaaagcgac     120

ttggtgaatt ttattcaagc aaattttaag aaacgagaat gtgtcttctt taccaaagat     180

tccaaggcca cggagaatgt gtgcaagtgt ggctatgccc agagccagca catggaaggc     240

acccagatca accaaagtga gaaatggaac tacaagaaac acaccaagga atttcctacc     300

gacgcctttg gggatattca gtttgagaca ctggggaaga aagggaagta tatacgtctg     360

tcctgcgaca cggacgcgga aatcctttac gagctgctga cccagcactg gcacctgaaa     420

acacccaacc tggtcatttc tgtgaccggg ggcgccaaga acttcgccct gaagccgcgc     480

atgcgcaaga tcttcagccg gctcatctac atcgcgcagt ccaaaggtgc ttggattctc     540

acgggaggca cccattatgg cctgatgaag tacatcgggg aggtggtgag agataacacc     600

atcagcagga gttcagagga gaatattgtg gccattggca tagcagcttg gggcatggtc     660

tccaaccggg acaccctcat caggaattgc gatgctgagg gctatttttt agcccagtac     720

cttatggatg acttcacaag agatccactg tatatcctgg acaacaacca cacacatttg     780

ctgctcgtgg acaatggctg tcatggacat cccactgtcg aagcaaagct ccggaatcag     840

ctagagaagt atatctctga gcgcactatt caagattcca actatggtgg caagatcccc     900

attgtgtgtt ttgcccaagg aggtggaaaa gagactttga aagccatcaa tacctccatc     960

aaaaataaaa ttccttgtgt ggtggtggaa ggctcgggcc agatcgctga tgtgatcgct    1020

agcctggtgg aggtggagga tgccctgaca tcttctgccg tcaaggagaa gctggtgcgc    1080

tttttacccc gcacggtgtc ccggctgcct gaggaggaga ctgagagttg gatcaaatgg    1140

ctcaaagaaa ttctcgaatg ttctcaccta ttaacagtta ttaaaatgga agaagctggg 1200
gatgaaattg tgagcaatgc catctcctac gctctataca aagccttcag caccagtgag 1260
caagacaagg ataactggaa tgggcagctg aagcttctgc tggagtggaa ccagctggac 1320
ttagccaatg atgagatttt caccaatgac cgccgatggg agtctgctga ccttcaagaa 1380
gtcatgttta cggctctcat aaaggacaga cccaagtttg tccgcctctt tctggagaat 1440
ggcttgaacc tacggaagtt tctcacccat gatgtcctca ctgaactctt ctccaaccac 1500
ttcagcacgc ttgtgtaccg gaatctgcag atcgccaaga attcctataa tgatgccctc 1560
ctcacgtttg tctggaaact ggttgcgaac ttccgaagag gcttccggaa ggaagacaga 1620
aatggccggg acgagatgga catagaactc cacgacgtgt ctcctattac tcggcacccc 1680
ctgcaagctc tcttcatctg ggccattctt cagaataaga aggaactctc caaagtcatt 1740
tgggagcaga ccaggggctg cactctggca gccctgggag ccagcaagct tctgaagact 1800
ctggccaaag tgaagaacga catcaatgct gctggggagt ccgaggagct ggctaatgag 1860
tacgagaccc gggctgttga gctgttcact gagtgttaca gcagcgatga agacttggca 1920
gaacagctgc tggtctattc ctgtgaagct tggggtggaa gcaactgtct ggagctggcg 1980
gtggaggcca cagaccagca tttcatcgcc cagcctgggg tccagaattt tctttctaag 2040
caatggtatg gagagatttc ccgagacacc aagaactgga agattatcct gtgtctgttt 2100
attataccct tggtgggctg tggctttgta tcatttagga agaaacctgt cgacaagcac 2160
aagaagctgc tttggtacta tgtggcgttc ttcacctccc ccttcgtggt cttctcctgg 2220
aatgtggtct tctacatcgc cttcctcctg ctgtttgcct acgtgctgct catggatttc 2280
cattcggtgc cacacccccc cgagctggtc ctgtactcgc tggtctttgt cctcttctgt 2340
gatgaagtga gacagtggta cgtaaatggg gtgaattatt ttactgacct gtggaatgtg 2400
atggacacgc tggggctttt ttacttcata gcaggaattg tatttcggct ccactcttct 2460
aataaaagct ctttgtattc tggacgagtc attttctgtc tggactacat tattttcact 2520
ctaagattga tccacatttt tactgtaagc agaaacttag gacccaagat tataatgctg 2580
cagaggatgc tgatcgatgt gttcttcttc ctgttcctct ttgcggtgtg gatggtggcc 2640
tttggcgtgg ccaggcaagg gatccttagg cagaatgagc agcgctggag gtggatattc 2700
cgttcggtca tctacgagcc ctacctggcc atgttcggcc aggtgcccag tgacgtggat 2760
ggtaccacgt atgactttgc ccactgcacc ttcactggga atgagtccaa gccactgtgt 2820
gtggagctgg atgagcacaa cctgccccgg ttccccgagt ggatcaccat ccccctggtg 2880
tgcatctaca tgttatccac caacatcctg ctggtcaacc tgctggtcgc catgtttggc 2940
tacacggtgg gcaccgtcca ggagaacaat gaccaggtct ggaagttcca gaggtacttc 3000
ctggtgcagg agtactgcag ccgcctcaat atccccttcc ccttcatcgt cttcgcttac 3060
ttctacatgg tggtgaagaa gtgcttcaag tgttgctgca aggagaaaaa catggagtct 3120
tctgtctgct gtttcaaaaa tgaagacaat gagactctgg catgggaggg tgtcatgaag 3180

gaaaactacc ttgtcaagat caacacaaaa gccaacgaca cctcagagga aatgaggcat 3240
cgatttagac aactggatac aaagcttaat gatctcaagg gtcttctgaa agagattgct 3300
aataaaatca aatga 3315

<210> 38
<211> 1104
<212> PRT
<213> Homo sapiens

<400> 38

Met Ser Phe Arg Ala Ala Arg Leu Ser Met Arg Asn Arg Arg Asn Asp
1               5                   10                  15

Thr Leu Asp Ser Thr Arg Thr Leu Tyr Ser Ser Ala Ser Arg Ser Thr
            20                  25                  30

Asp Leu Ser Tyr Ser Glu Ser Asp Leu Val Asn Phe Ile Gln Ala Asn
        35                  40                  45

Phe Lys Lys Arg Glu Cys Val Phe Phe Thr Lys Asp Ser Lys Ala Thr
    50                  55                  60

Glu Asn Val Cys Lys Cys Gly Tyr Ala Gln Ser Gln His Met Glu Gly
65                  70                  75                  80

Thr Gln Ile Asn Gln Ser Glu Lys Trp Asn Tyr Lys Lys His Thr Lys
                85                  90                  95

Glu Phe Pro Thr Asp Ala Phe Gly Asp Ile Gln Phe Glu Thr Leu Gly
            100                 105                 110

Lys Lys Gly Lys Tyr Ile Arg Leu Ser Cys Asp Thr Asp Ala Glu Ile
        115                 120                 125

Leu Tyr Glu Leu Leu Thr Gln His Trp His Leu Lys Thr Pro Asn Leu
    130                 135                 140

Val Ile Ser Val Thr Gly Gly Ala Lys Asn Phe Ala Leu Lys Pro Arg
145                 150                 155                 160

Met Arg Lys Ile Phe Ser Arg Leu Ile Tyr Ile Ala Gln Ser Lys Gly
                165                 170                 175

Ala Trp Ile Leu Thr Gly Gly Thr His Tyr Gly Leu Met Lys Tyr Ile
            180                 185                 190

Gly Glu Val Val Arg Asp Asn Thr Ile Ser Arg Ser Ser Glu Glu Asn
        195                 200                 205

Ile Val Ala Ile Gly Ile Ala Ala Trp Gly Met Val Ser Asn Arg Asp
    210                 215                 220

Thr Leu Ile Arg Asn Cys Asp Ala Glu Gly Tyr Phe Leu Ala Gln Tyr
225                 230                 235                 240

Leu Met Asp Asp Phe Thr Arg Asp Pro Leu Tyr Ile Leu Asp Asn Asn
                245                 250                 255

His Thr His Leu Leu Leu Val Asp Asn Gly Cys His Gly His Pro Thr
            260                 265                 270

Val Glu Ala Lys Leu Arg Asn Gln Leu Glu Lys Tyr Ile Ser Glu Arg
        275                 280                 285

Thr Ile Gln Asp Ser Asn Tyr Gly Gly Lys Ile Pro Ile Val Cys Phe
    290                 295                 300

Ala Gln Gly Gly Gly Lys Glu Thr Leu Lys Ala Ile Asn Thr Ser Ile
305                 310                 315                 320

Lys Asn Lys Ile Pro Cys Val Val Val Glu Gly Ser Gly Gln Ile Ala
                325                 330                 335

Asp Val Ile Ala Ser Leu Val Glu Val Glu Asp Ala Leu Thr Ser Ser
            340                 345                 350

Ala Val Lys Glu Lys Leu Val Arg Phe Leu Pro Arg Thr Val Ser Arg
        355                 360                 365

Leu Pro Glu Glu Glu Thr Glu Ser Trp Ile Lys Trp Leu Lys Glu Ile
    370                 375                 380

Leu Glu Cys Ser His Leu Leu Thr Val Ile Lys Met Glu Glu Ala Gly
385                 390                 395                 400

Asp Glu Ile Val Ser Asn Ala Ile Ser Tyr Ala Leu Tyr Lys Ala Phe
                405                 410                 415

Ser Thr Ser Glu Gln Asp Lys Asp Asn Trp Asn Gly Gln Leu Lys Leu
            420                 425                 430

Leu Leu Glu Trp Asn Gln Leu Asp Leu Ala Asn Asp Glu Ile Phe Thr
        435                 440                 445

Asn Asp Arg Arg Trp Glu Ser Ala Asp Leu Gln Glu Val Met Phe Thr
    450                 455                 460

Ala Leu Ile Lys Asp Arg Pro Lys Phe Val Arg Leu Phe Leu Glu Asn
465                 470                 475                 480

Gly Leu Asn Leu Arg Lys Phe Leu Thr His Asp Val Leu Thr Glu Leu
                485                 490                 495

Phe Ser Asn His Phe Ser Thr Leu Val Tyr Arg Asn Leu Gln Ile Ala
            500                 505                 510

Lys Asn Ser Tyr Asn Asp Ala Leu Leu Thr Phe Val Trp Lys Leu Val
        515                 520                 525

Ala Asn Phe Arg Arg Gly Phe Arg Lys Glu Asp Arg Asn Gly Arg Asp
    530                 535                 540

Glu Met Asp Ile Glu Leu His Asp Val Ser Pro Ile Thr Arg His Pro
545                 550                 555                 560

Leu Gln Ala Leu Phe Ile Trp Ala Ile Leu Gln Asn Lys Lys Glu Leu
                565                 570                 575

Ser Lys Val Ile Trp Glu Gln Thr Arg Gly Cys Thr Leu Ala Ala Leu
            580                 585                 590

Gly Ala Ser Lys Leu Leu Lys Thr Leu Ala Lys Val Lys Asn Asp Ile
        595                 600                 605

Asn Ala Ala Gly Glu Ser Glu Glu Leu Ala Asn Glu Tyr Glu Thr Arg
    610                 615                 620

Ala Val Glu Leu Phe Thr Glu Cys Tyr Ser Ser Asp Glu Asp Leu Ala
625                 630                 635                 640

Glu Gln Leu Leu Val Tyr Ser Cys Glu Ala Trp Gly Gly Ser Asn Cys

                645                 650                 655
Leu Glu Leu Ala Val Glu Ala Thr Asp Gln His Phe Ile Ala Gln Pro
            660                 665                 670
Gly Val Gln Asn Phe Leu Ser Lys Gln Trp Tyr Gly Glu Ile Ser Arg
        675                 680                 685
Asp Thr Lys Asn Trp Lys Ile Ile Leu Cys Leu Phe Ile Ile Pro Leu
    690                 695                 700
Val Gly Cys Gly Phe Val Ser Phe Arg Lys Lys Pro Val Asp Lys His
705                 710                 715                 720
Lys Lys Leu Leu Trp Tyr Tyr Val Ala Phe Phe Thr Ser Pro Phe Val
                725                 730                 735
Val Phe Ser Trp Asn Val Val Phe Tyr Ile Ala Phe Leu Leu Leu Phe
            740                 745                 750
Ala Tyr Val Leu Leu Met Asp Phe His Ser Val Pro His Pro Pro Glu
        755                 760                 765
Leu Val Leu Tyr Ser Leu Val Phe Val Leu Phe Cys Asp Glu Val Arg
    770                 775                 780
Gln Trp Tyr Val Asn Gly Val Asn Tyr Phe Thr Asp Leu Trp Asn Val
785                 790                 795                 800
Met Asp Thr Leu Gly Leu Phe Tyr Phe Ile Ala Gly Ile Val Phe Arg
                805                 810                 815
Leu His Ser Ser Asn Lys Ser Ser Leu Tyr Ser Gly Arg Val Ile Phe
            820                 825                 830
Cys Leu Asp Tyr Ile Ile Phe Thr Leu Arg Leu Ile His Ile Phe Thr
        835                 840                 845
Val Ser Arg Asn Leu Gly Pro Lys Ile Ile Met Leu Gln Arg Met Leu
    850                 855                 860
Ile Asp Val Phe Phe Phe Leu Phe Leu Phe Ala Val Trp Met Val Ala
865                 870                 875                 880
Phe Gly Val Ala Arg Gln Gly Ile Leu Arg Gln Asn Glu Gln Arg Trp
                885                 890                 895
Arg Trp Ile Phe Arg Ser Val Ile Tyr Glu Pro Tyr Leu Ala Met Phe
            900                 905                 910
Gly Gln Val Pro Ser Asp Val Asp Gly Thr Thr Tyr Asp Phe Ala His
        915                 920                 925
Cys Thr Phe Thr Gly Asn Glu Ser Lys Pro Leu Cys Val Glu Leu Asp
    930                 935                 940
Glu His Asn Leu Pro Arg Phe Pro Glu Trp Ile Thr Ile Pro Leu Val
945                 950                 955                 960
Cys Ile Tyr Met Leu Ser Thr Asn Ile Leu Leu Val Asn Leu Leu Val
                965                 970                 975
Ala Met Phe Gly Tyr Thr Val Gly Thr Val Gln Glu Asn Asn Asp Gln
            980                 985                 990
Val Trp Lys Phe Gln Arg Tyr Phe Leu Val Gln Glu Tyr Cys Ser Arg
        995                 1000                1005

Leu Asn Ile Pro Phe Pro Phe Ile Val Phe Ala Tyr Phe Tyr Met
    1010                1015                1020

Val Val Lys Lys Cys Phe Lys Cys Cys Cys Lys Glu Lys Asn Met
    1025                1030                1035

Glu Ser Ser Val Cys Cys Phe Lys Asn Glu Asp Asn Glu Thr Leu
    1040                1045                1050

Ala Trp Glu Gly Val Met Lys Glu Asn Tyr Leu Val Lys Ile Asn
    1055                1060                1065

Thr Lys Ala Asn Asp Thr Ser Glu Glu Met Arg His Arg Phe Arg
    1070                1075                1080

Gln Leu Asp Thr Lys Leu Asn Asp Leu Lys Gly Leu Leu Lys Glu
    1085                1090                1095

Ile Ala Asn Lys Ile Lys
    1100

<210>  39
<211>  558
<212>  DNA
<213>  Homo sapiens

<400>  39
atgcctcgcc tgttcttgtt ccacctgcta gaattctgtt tactactgaa ccaattttcc      60

agagcagtcg cggccaaatg gaaggacgat gttattaaat tatgcggccg cgaattagtt     120

cgcgcgcaga ttgccatttg cggcatgagc acctggagca aaaggtctct gagccaggaa     180

gatgctcctc agacacctag accagtggca gaaattgtac catccttcat caacaaagat     240

acagaaacta taattatcat gttggaattc attgctaatt tgccaccgga gctgaaggca     300

gccctatctg agaggcaacc atcattacca gagctacagc agtatgtacc tgcattaaag     360

gattccaatc ttagctttga agaatttaag aaacttattc gcaataggca aagtgaagcc     420

gcagacagca atccttcaga attaaaatac ttaggcttgg atactcattc tcaaaaaaag     480

agacgaccct acgtggcact gtttgagaaa tgttgcctaa ttggttgtac caaaaggtct     540

cttgctaaat attgctga                                                   558

<210>  40
<211>  185
<212>  PRT
<213>  Homo sapiens

<400>  40

Met Pro Arg Leu Phe Leu Phe His Leu Leu Glu Phe Cys Leu Leu Leu
1               5                   10                  15

Asn Gln Phe Ser Arg Ala Val Ala Ala Lys Trp Lys Asp Asp Val Ile
            20                  25                  30

Lys Leu Cys Gly Arg Glu Leu Val Arg Ala Gln Ile Ala Ile Cys Gly
        35                  40                  45

Met Ser Thr Trp Ser Lys Arg Ser Leu Ser Gln Glu Asp Ala Pro Gln
    50                  55                  60

Thr Pro Arg Pro Val Ala Glu Ile Val Pro Ser Phe Ile Asn Lys Asp
65                  70                  75                  80

Thr Glu Thr Ile Ile Ile Met Leu Glu Phe Ile Ala Asn Leu Pro Pro
                85                  90                  95

Glu Leu Lys Ala Ala Leu Ser Glu Arg Gln Pro Ser Leu Pro Glu Leu
            100                 105                 110

Gln Gln Tyr Val Pro Ala Leu Lys Asp Ser Asn Leu Ser Phe Glu Glu
        115                 120                 125

Phe Lys Lys Leu Ile Arg Asn Arg Gln Ser Glu Ala Ala Asp Ser Asn
    130                 135                 140

Pro Ser Glu Leu Lys Tyr Leu Gly Leu Asp Thr His Ser Gln Lys Lys
145                 150                 155                 160

Arg Arg Pro Tyr Val Ala Leu Phe Glu Lys Cys Cys Leu Ile Gly Cys
                165                 170                 175

Thr Lys Arg Ser Leu Ala Lys Tyr Cys
            180                 185

<210>  41
<211>  3978
<212>  DNA
<213>  Homo sapiens

<400>  41
atgctgcccg tgtaccagga ggtgaagccc aacccgctgc aggacgcgaa cctctgctca      60

cgcgtgttct tctggtggct caatcccttg tttaaaattg gccataaacg gagattagag     120

gaagatgata tgtattcagt gctgccagaa gaccgctcac agcaccttgg agaggagttg     180

caagggttct gggataaaga agttttaaga gctgagaatg acgcacagaa gccttcttta     240

acaagagcaa tcataaagtg ttactggaaa tcttatttag ttttgggaat ttttacgtta     300

attgaggaaa gtgccaaagt aatccagccc atatttttgg gaaaaattat taattatttt     360

gaaaattatg atcccatgga ttctgtggct ttgaacacag cgtacgccta tgccacggtg     420

ctgacttttt gcacgctcat tttggctata ctgcatcact tatattttta tcacgttcag     480

tgtgctggga tgaggttacg agtagccatg tgccatatga tttatcggaa ggcacttcgt     540

cttagtaaca tggccatggg gaagacaacc acaggccaga tagtcaatct gctgtccaat     600

gatgtgaaca agtttgatca ggtgacagtg ttcttacact tcctgtgggc aggaccactg     660

caggcgatcg cagtgactgc cctactctgg atggagatag gaatatcgtg ccttgctggg     720

atggcagttc taatcattct cctgcccttg caaagctgtt ttgggaagtt gttctcatca     780

ctgaggagta aaactgcaac tttcacggat gccaggatca ggaccatgaa tgaagttata     840

actggtataa ggataataaa aatgtacgcc tgggaaaagt cattttcaaa tcttattacc     900

aatttgagaa agaaggagat ttccaagatt ctgagaagtt cctgcctcag ggggatgaat     960

ttggcttcgt ttttcagtgc aagcaaaatc atcgtgtttg tgaccttcac cacctacgtg    1020

ctcctcggca gtgtgatcac agccagccgc gtgttcgtgg cagtgacgct gtatggggct    1080

gtgcggctga cggttaccct cttcttcccc tcagccattg agagggtgtc agaggcaatc    1140

gtcagcatcc gaagaatcca gacctttttg ctacttgatg agatatcaca gcgcaaccgt 1200
cagctgccgt cagatggtaa aaagatggtg catgtgcagg attttactgc tttttgggat 1260
aaggcatcag agaccccaac tctacaaggc ctttccttta ctgtcagacc tggcgaattg 1320
ttagctgtgg tcggccccgt gggagcaggg aagtcatcac tgttaagtgc cgtgctcggg 1380
gaattggccc caagtcacgg gctggtcagc gtgcatggaa gaattgccta tgtgtctcag 1440
cagccctggg tgttctcggg aactctgagg agtaatattt tatttgggaa gaaatacgaa 1500
aaggaacgat atgaaaaagt cataaaggct tgtgctctga aaaaggattt acagctgttg 1560
gaggatggtg atctgactgt gataggagat cggggaacca cgctgagtgg agggcagaaa 1620
gcacgggtaa accttgcaag agcagtgtat caagatgctg acatctatct cctggacgat 1680
cctctcagtg cagtagatgc ggaagttagc agacacttgt tcgaactgtg tatttgtcaa 1740
attttgcatg agaagatcac aattttagtg actcatcagt tgcagtacct caaagctgca 1800
agtcagattc tgatattgaa agatggtaaa atggtgcaga aggggactta cactgagttc 1860
ctaaaatctg gtatagattt tggctccctt ttaaagaagg ataatgagga aagtgaacaa 1920
cctccagttc caggaactcc cacactaagg aatcgtacct tctcagagtc ttcggtttgg 1980
tctcaacaat cttctagacc ctccttgaaa gatggtgctc tggagagcca agatacagag 2040
aatgtcccag ttacactatc agaggagaac cgttctgaag gaaaagttgg ttttcaggcc 2100
tataagaatt acttcagagc tggtgctcac tggattgtct tcattttcct tattctccta 2160
aacactgcag ctcaggttgc ctatgtgctt caagattggt ggctttcata ctgggcaaac 2220
aaacaaagta tgctaaatgt cactgtaaat ggaggaggaa atgtaaccga gaagctagat 2280
cttaactggt acttaggaat ttattcaggt ttaactgtag ctaccgttct ttttggcata 2340
gcaagatctc tattggtatt ctacgtcctt gttaactctt cacaaacttt gcacaacaaa 2400
atgtttgagt caattctgaa agctccggta ttattctttg atagaaatcc aataggaaga 2460
attttaaatc gtttctccaa agacattgga cacttggatg atttgctgcc gctgacgttt 2520
ttagatttca tccagacatt gctacaagtg gttggtgtgg tctctgtggc tgtggccgtg 2580
attccttgga tcgcaatacc cttggttccc cttggaatca ttttcatttt tcttcggcga 2640
tattttttgg aaacgtcaag agatgtgaag cgcctggaat ctacaactcg gagtccagtg 2700
ttttcccact tgtcatcttc tctccagggg ctctggacca tccgggcata caaagcagaa 2760
gagaggtgtc aggaactgtt tgatgcacac caggatttac attcagaggc ttggttcttg 2820
tttttgacaa cgtcccgctg gttcgccgtc cgtctggatg ccatctgtgc catgtttgtc 2880
atcatcgttg cctttgggtc cctgattctg gcaaaaactc tggatgccgg gcaggttggt 2940
ttggcactgt cctatgccct cacgctcatg gggatgtttc agtggtgtgt tcgacaaagt 3000
gctgaagttg agaatatgat gatctcagta gaaagggtca ttgaatacac agaccttgaa 3060
aaagaagcac cttgggaata tcagaaacgc ccaccaccag cctggcccca tgaaggagtg 3120
ataatctttg acaatgtgaa cttcatgtac agtccaggtg ggcctctggt actgaagcat 3180

ctgacagcac tcattaaatc acaagaaaag gttggcattg tgggaagaac cggagctgga 3240

aaaagttccc tcatctcagc cctttttaga ttgtcagaac ccgaaggtaa aatttggatt 3300

gataagatct tgacaactga aattggactt cacgatttaa ggaagaaaat gtcaatcata 3360

cctcaggaac ctgttttgtt cactggaaca atgaggaaaa acctggatcc ctttaatgag 3420

cacacggatg aggaactgtg gaatgcctta caagaggtac aacttaaaga aaccattgaa 3480

gatcttcctg gtaaaatgga tactgaatta gcagaatcag gatccaattt tagtgttgga 3540

caaagacaac tggtgtgcct tgccagggca attctcagga aaaatcagat attgattatt 3600

gatgaagcga cggcaaatgt ggatccaaga actgatgagt taatacaaaa aaaaatccgg 3660

gagaaatttg cccactgcac cgtgctaacc attgcacaca gattgaacac cattattgac 3720

agcgacaaga taatggtttt agattcagga agactgaaag aatatgatga gccgtatgtt 3780

ttgctgcaaa ataaagagag cctattttac aagatggtgc aacaactggg caaggcagaa 3840

gccgctgccc tcactgaaac agcaaaacag gtatacttca aaagaaatta tccacatatt 3900

ggtcacactg accacatggt tacaaacact tccaatggac agccctcgac cttaactatt 3960

ttcgagacag cactgtga 3978

<210> 42
<211> 1325
<212> PRT
<213> Homo sapiens

<400> 42

Met Leu Pro Val Tyr Gln Glu Val Lys Pro Asn Pro Leu Gln Asp Ala
1               5                   10                  15

Asn Leu Cys Ser Arg Val Phe Phe Trp Trp Leu Asn Pro Leu Phe Lys
            20                  25                  30

Ile Gly His Lys Arg Arg Leu Glu Glu Asp Asp Met Tyr Ser Val Leu
        35                  40                  45

Pro Glu Asp Arg Ser Gln His Leu Gly Glu Glu Leu Gln Gly Phe Trp
    50                  55                  60

Asp Lys Glu Val Leu Arg Ala Glu Asn Asp Ala Gln Lys Pro Ser Leu
65                  70                  75                  80

Thr Arg Ala Ile Ile Lys Cys Tyr Trp Lys Ser Tyr Leu Val Leu Gly
                85                  90                  95

Ile Phe Thr Leu Ile Glu Glu Ser Ala Lys Val Ile Gln Pro Ile Phe
            100                 105                 110

Leu Gly Lys Ile Ile Asn Tyr Phe Glu Asn Tyr Asp Pro Met Asp Ser
        115                 120                 125

Val Ala Leu Asn Thr Ala Tyr Ala Tyr Ala Thr Val Leu Thr Phe Cys
    130                 135                 140

Thr Leu Ile Leu Ala Ile Leu His His Leu Tyr Phe Tyr His Val Gln
145                 150                 155                 160

Cys Ala Gly Met Arg Leu Arg Val Ala Met Cys His Met Ile Tyr Arg
                165                 170                 175

Lys Ala Leu Arg Leu Ser Asn Met Ala Met Gly Lys Thr Thr Thr Gly
            180                 185                 190

Gln Ile Val Asn Leu Leu Ser Asn Asp Val Asn Lys Phe Asp Gln Val
        195                 200                 205

Thr Val Phe Leu His Phe Leu Trp Ala Gly Pro Leu Gln Ala Ile Ala
    210                 215                 220

Val Thr Ala Leu Leu Trp Met Glu Ile Gly Ile Ser Cys Leu Ala Gly
225                 230                 235                 240

Met Ala Val Leu Ile Ile Leu Leu Pro Leu Gln Ser Cys Phe Gly Lys
                245                 250                 255

Leu Phe Ser Ser Leu Arg Ser Lys Thr Ala Thr Phe Thr Asp Ala Arg
            260                 265                 270

Ile Arg Thr Met Asn Glu Val Ile Thr Gly Ile Arg Ile Ile Lys Met
        275                 280                 285

Tyr Ala Trp Glu Lys Ser Phe Ser Asn Leu Ile Thr Asn Leu Arg Lys
    290                 295                 300

Lys Glu Ile Ser Lys Ile Leu Arg Ser Ser Cys Leu Arg Gly Met Asn
305                 310                 315                 320

Leu Ala Ser Phe Phe Ser Ala Ser Lys Ile Ile Val Phe Val Thr Phe
                325                 330                 335

Thr Thr Tyr Val Leu Leu Gly Ser Val Ile Thr Ala Ser Arg Val Phe
            340                 345                 350

Val Ala Val Thr Leu Tyr Gly Ala Val Arg Leu Thr Val Thr Leu Phe
        355                 360                 365

Phe Pro Ser Ala Ile Glu Arg Val Ser Glu Ala Ile Val Ser Ile Arg
    370                 375                 380

Arg Ile Gln Thr Phe Leu Leu Leu Asp Glu Ile Ser Gln Arg Asn Arg
385                 390                 395                 400

Gln Leu Pro Ser Asp Gly Lys Lys Met Val His Val Gln Asp Phe Thr
                405                 410                 415

Ala Phe Trp Asp Lys Ala Ser Glu Thr Pro Thr Leu Gln Gly Leu Ser
            420                 425                 430

Phe Thr Val Arg Pro Gly Glu Leu Leu Ala Val Val Gly Pro Val Gly
        435                 440                 445

Ala Gly Lys Ser Ser Leu Leu Ser Ala Val Leu Gly Glu Leu Ala Pro
    450                 455                 460

Ser His Gly Leu Val Ser Val His Gly Arg Ile Ala Tyr Val Ser Gln
465                 470                 475                 480

Gln Pro Trp Val Phe Ser Gly Thr Leu Arg Ser Asn Ile Leu Phe Gly
                485                 490                 495

Lys Lys Tyr Glu Lys Glu Arg Tyr Glu Lys Val Ile Lys Ala Cys Ala
            500                 505                 510

Leu Lys Lys Asp Leu Gln Leu Leu Glu Asp Gly Asp Leu Thr Val Ile
        515                 520                 525

Gly Asp Arg Gly Thr Thr Leu Ser Gly Gly Gln Lys Ala Arg Val Asn
    530                 535                 540

Leu Ala Arg Ala Val Tyr Gln Asp Ala Asp Ile Tyr Leu Leu Asp Asp
545                 550                 555                 560

Pro Leu Ser Ala Val Asp Ala Glu Val Ser Arg His Leu Phe Glu Leu
                565                 570                 575

Cys Ile Cys Gln Ile Leu His Glu Lys Ile Thr Ile Leu Val Thr His
            580                 585                 590

Gln Leu Gln Tyr Leu Lys Ala Ala Ser Gln Ile Leu Ile Leu Lys Asp
        595                 600                 605

Gly Lys Met Val Gln Lys Gly Thr Tyr Thr Glu Phe Leu Lys Ser Gly
    610                 615                 620

Ile Asp Phe Gly Ser Leu Leu Lys Lys Asp Asn Glu Glu Ser Glu Gln
625                 630                 635                 640

Pro Pro Val Pro Gly Thr Pro Thr Leu Arg Asn Arg Thr Phe Ser Glu
                645                 650                 655

Ser Ser Val Trp Ser Gln Gln Ser Ser Arg Pro Ser Leu Lys Asp Gly
            660                 665                 670

Ala Leu Glu Ser Gln Asp Thr Glu Asn Val Pro Val Thr Leu Ser Glu
        675                 680                 685

Glu Asn Arg Ser Glu Gly Lys Val Gly Phe Gln Ala Tyr Lys Asn Tyr
    690                 695                 700

Phe Arg Ala Gly Ala His Trp Ile Val Phe Ile Phe Leu Ile Leu Leu
705                 710                 715                 720

Asn Thr Ala Ala Gln Val Ala Tyr Val Leu Gln Asp Trp Trp Leu Ser
                725                 730                 735

Tyr Trp Ala Asn Lys Gln Ser Met Leu Asn Val Thr Val Asn Gly Gly
            740                 745                 750

Gly Asn Val Thr Glu Lys Leu Asp Leu Asn Trp Tyr Leu Gly Ile Tyr
        755                 760                 765

Ser Gly Leu Thr Val Ala Thr Val Leu Phe Gly Ile Ala Arg Ser Leu
    770                 775                 780

Leu Val Phe Tyr Val Leu Val Asn Ser Ser Gln Thr Leu His Asn Lys
785                 790                 795                 800

Met Phe Glu Ser Ile Leu Lys Ala Pro Val Leu Phe Phe Asp Arg Asn
                805                 810                 815

Pro Ile Gly Arg Ile Leu Asn Arg Phe Ser Lys Asp Ile Gly His Leu
            820                 825                 830

Asp Asp Leu Leu Pro Leu Thr Phe Leu Asp Phe Ile Gln Thr Leu Leu
        835                 840                 845

Gln Val Val Gly Val Val Ser Val Ala Val Ala Val Ile Pro Trp Ile
    850                 855                 860

Ala Ile Pro Leu Val Pro Leu Gly Ile Ile Phe Ile Phe Leu Arg Arg
865                 870                 875                 880

Tyr Phe Leu Glu Thr Ser Arg Asp Val Lys Arg Leu Glu Ser Thr Thr
                885                 890                 895

Arg Ser Pro Val Phe Ser His Leu Ser Ser Ser Leu Gln Gly Leu Trp

900                 905                 910

Thr Ile Arg Ala Tyr Lys Ala Glu Glu Arg Cys Gln Glu Leu Phe Asp
        915                 920                 925

Ala His Gln Asp Leu His Ser Glu Ala Trp Phe Leu Phe Leu Thr Thr
    930                 935                 940

Ser Arg Trp Phe Ala Val Arg Leu Asp Ala Ile Cys Ala Met Phe Val
945                 950                 955                 960

Ile Ile Val Ala Phe Gly Ser Leu Ile Leu Ala Lys Thr Leu Asp Ala
                965                 970                 975

Gly Gln Val Gly Leu Ala Leu Ser Tyr Ala Leu Thr Leu Met Gly Met
            980                 985                 990

Phe Gln Trp Cys Val Arg Gln Ser  Ala Glu Val Glu Asn  Met Met Ile
        995                 1000                 1005

Ser Val  Glu Arg Val Ile Glu  Tyr Thr Asp Leu Glu  Lys Glu Ala
    1010                 1015                 1020

Pro Trp  Glu Tyr Gln Lys Arg  Pro Pro Pro Ala Trp  Pro His Glu
    1025                 1030                 1035

Gly Val  Ile Ile Phe Asp Asn  Val Asn Phe Met Tyr  Ser Pro Gly
    1040                 1045                 1050

Gly Pro  Leu Val Leu Lys His  Leu Thr Ala Leu Ile  Lys Ser Gln
    1055                 1060                 1065

Glu Lys  Val Gly Ile Val Gly  Arg Thr Gly Ala Gly  Lys Ser Ser
    1070                 1075                 1080

Leu Ile  Ser Ala Leu Phe Arg  Leu Ser Glu Pro Glu  Gly Lys Ile
    1085                 1090                 1095

Trp Ile  Asp Lys Ile Leu Thr  Thr Glu Ile Gly Leu  His Asp Leu
    1100                 1105                 1110

Arg Lys  Lys Met Ser Ile Ile  Pro Gln Glu Pro Val  Leu Phe Thr
    1115                 1120                 1125

Gly Thr  Met Arg Lys Asn Leu  Asp Pro Phe Asn Glu  His Thr Asp
    1130                 1135                 1140

Glu Glu  Leu Trp Asn Ala Leu  Gln Glu Val Gln Leu  Lys Glu Thr
    1145                 1150                 1155

Ile Glu  Asp Leu Pro Gly Lys  Met Asp Thr Glu Leu  Ala Glu Ser
    1160                 1165                 1170

Gly Ser  Asn Phe Ser Val Gly  Gln Arg Gln Leu Val  Cys Leu Ala
    1175                 1180                 1185

Arg Ala  Ile Leu Arg Lys Asn  Gln Ile Leu Ile Ile  Asp Glu Ala
    1190                 1195                 1200

Thr Ala  Asn Val Asp Pro Arg  Thr Asp Glu Leu Ile  Gln Lys Lys
    1205                 1210                 1215

Ile Arg  Glu Lys Phe Ala His  Cys Thr Val Leu Thr  Ile Ala His
    1220                 1225                 1230

Arg Leu  Asn Thr Ile Ile Asp  Ser Asp Lys Ile Met  Val Leu Asp
    1235                 1240                 1245

Ser Gly Arg Leu Lys Glu Tyr Asp Glu Pro Tyr Val Leu Leu Gln
    1250                1255                1260

Asn Lys Glu Ser Leu Phe Tyr Lys Met Val Gln Gln Leu Gly Lys
    1265                1270                1275

Ala Glu Ala Ala Ala Leu Thr Glu Thr Ala Lys Gln Val Tyr Phe
    1280                1285                1290

Lys Arg Asn Tyr Pro His Ile Gly His Thr Asp His Met Val Thr
    1295                1300                1305

Asn Thr Ser Asn Gly Gln Pro Ser Thr Leu Thr Ile Phe Glu Thr
    1310                1315                1320

Ala Leu
    1325

<210>  43
<211>  1140
<212>  DNA
<213>  Homo sapiens

<400>  43
atggggaaag tgtcctgctg tggcatgaaa taaatgaaac agaaaatgat ggcaagactg      60

ctaagaacat cctttgcttt gctcttcctt ggcctctttg gggtgctggg ggcagcaaca     120

atttcatgca gaaatgaaga agggaaagct gtggactggt ttacttttta taagttacct     180

aaaagacaaa acaaggaaag tggagagact gggttagagt acctgtacct agactctaca     240

actagaagct ggaggaagag tgagcaacta atgaatgaca ccaagagtgt tttgggaagg     300

acattacaac agctatatga agcatatgcc tctaagagta acaacacagc ctatctaata     360

tacaatgatg gagtccctaa acctgtgaat tacagtagaa agtatggaca caccaaaggt     420

ttactgctgt ggaacagagt tcaagggttc tggctgattc attccatccc tcagtttcct     480

ccaattccgg aagaaggcta tgattatcca cccacaggga gacgaaatgg acaaagtggc     540

atctgcataa ctttcaagta caaccagtat gaggcaatag attctcagct cttggtctgc     600

aaccccaacg tctatagctg ctccatccca gccacctttc accaggagct cattcacatg     660

ccccagctgt gcaccagggc cagctcatca gagattcctg gcaggctcct caccacactt     720

cagtcggccc agggacaaaa attcctccat tttgcaaagt cggattcttt tcttgacgac     780

atctttgcag cctggatggc tcaacggctg aagacacact tgttaacaga aacctggcag     840

cgaaaaagac aagagcttcc ttcaaactgc tcccttcctt accatgtcta caatataaaa     900

gcaattaaat tatcacgaca ctcttatttc agttcttatc aagatcacgc caagtggtgt     960

atttcccaaa agggcaccaa aaatcgctgg acatgtattg gagacctaaa tcggagtcca    1020

caccaagcct tcagaagtgg aggattcatt tgtacccaga attggcaaat ttaccaagca    1080

tttcaaggat tagtattata ctatgaaagc tgtaagtaaa cttggtgaaa ggacacaggt    1140

<210>  44
<211>  357
<212>  PRT
<213>  Homo sapiens

<400>  44

Met Met Ala Arg Leu Leu Arg Thr Ser Phe Ala Leu Leu Phe Leu Gly
1               5                   10                  15

Leu Phe Gly Val Leu Gly Ala Ala Thr Ile Ser Cys Arg Asn Glu Glu
            20                  25                  30

Gly Lys Ala Val Asp Trp Phe Thr Phe Tyr Lys Leu Pro Lys Arg Gln
        35                  40                  45

Asn Lys Glu Ser Gly Glu Thr Gly Leu Glu Tyr Leu Tyr Leu Asp Ser
    50                  55                  60

Thr Thr Arg Ser Trp Arg Lys Ser Glu Gln Leu Met Asn Asp Thr Lys
65                  70                  75                  80

Ser Val Leu Gly Arg Thr Leu Gln Gln Leu Tyr Glu Ala Tyr Ala Ser
                85                  90                  95

Lys Ser Asn Asn Thr Ala Tyr Leu Ile Tyr Asn Asp Gly Val Pro Lys
            100                 105                 110

Pro Val Asn Tyr Ser Arg Lys Tyr Gly His Thr Lys Gly Leu Leu Leu
        115                 120                 125

Trp Asn Arg Val Gln Gly Phe Trp Leu Ile His Ser Ile Pro Gln Phe
    130                 135                 140

Pro Pro Ile Pro Glu Glu Gly Tyr Asp Tyr Pro Pro Thr Gly Arg Arg
145                 150                 155                 160

Asn Gly Gln Ser Gly Ile Cys Ile Thr Phe Lys Tyr Asn Gln Tyr Glu
                165                 170                 175

Ala Ile Asp Ser Gln Leu Leu Val Cys Asn Pro Asn Val Tyr Ser Cys
            180                 185                 190

Ser Ile Pro Ala Thr Phe His Gln Glu Leu Ile His Met Pro Gln Leu
        195                 200                 205

Cys Thr Arg Ala Ser Ser Ser Glu Ile Pro Gly Arg Leu Leu Thr Thr
    210                 215                 220

Leu Gln Ser Ala Gln Gly Gln Lys Phe Leu His Phe Ala Lys Ser Asp
225                 230                 235                 240

Ser Phe Leu Asp Asp Ile Phe Ala Ala Trp Met Ala Gln Arg Leu Lys
                245                 250                 255

Thr His Leu Leu Thr Glu Thr Trp Gln Arg Lys Arg Gln Glu Leu Pro
            260                 265                 270

Ser Asn Cys Ser Leu Pro Tyr His Val Tyr Asn Ile Lys Ala Ile Lys
        275                 280                 285

Leu Ser Arg His Ser Tyr Phe Ser Ser Tyr Gln Asp His Ala Lys Trp
    290                 295                 300

Cys Ile Ser Gln Lys Gly Thr Lys Asn Arg Trp Thr Cys Ile Gly Asp
305                 310                 315                 320

Leu Asn Arg Ser Pro His Gln Ala Phe Arg Ser Gly Gly Phe Ile Cys
                325                 330                 335

Thr Gln Asn Trp Gln Ile Tyr Gln Ala Phe Gln Gly Leu Val Leu Tyr
            340                 345                 350

Tyr Glu Ser Cys Lys

        355

<210> 45
<211> 2243
<212> DNA
<213> Homo sapiens

<400> 45
cggtgccctg gggtggaata tcccctacga atttaaccaa gcggacttta atgccactgt 60

gcagttcatc caaaaccact tggatgacat ggatgtcaaa aagggtgtct cctggaccac 120

catccgctac atgataggag agattcaata tggaggcaga gtcactgacg actatgataa 180

gagattgttg aacacatttg ctaaggtttg gttcagtgaa aatatgtttg gaccagattt 240

cagtttttac caaggataca atattccaaa atgcagcaca gtggataact atcttcagta 300

tatccagagt ttgcctgcct atgacagccc tgaggtgttt gggctgcacc ccaatgctga 360

catcacctac cagagcaagc tggccaagga cgtgctggac accatcctag gcatccaacc 420

caaggacacc tctggtggag gggatgagac ccgggaggcg gtggtggccc ggctggctga 480

tgatatgctg gagaagctgc ccccagacta tgtccccttt gaagtaaaag agaggctgca 540

gaagatgggg ccattccagc ctatgaacat tttcctcagg caggaaatag acagaatgca 600

aagggtactc agccttgtcc gcagcaccct cactgagctg aaacttgcta ttgatggcac 660

catcatcatg agcgaaaatc tgcaagatgc attggattgc atgtttgatg ctagaatccc 720

tgcttggtgg aaaaaagctt cttgggtttt tagtacactg ggtttctggt ttactgaact 780

tatagaaaga aacagccagt ttacctcgtg ggttttcaat ggccgacctc actgcttttg 840

gatgacgggt ttttttaacc cccagggatt tttaactgca atgcgacagg aaataactcg 900

ggccaacaaa ggctgggctc tggacaatat ggtgctttgc aatgaagtca ccaaatggat 960

gaaggacgac atttctaccc ctcccacaga gggtgtctat gtctatggct tatatcttga 1020

aggtgctggc tgggacaaga ggaacatgaa actcattgaa tcaaagccaa aagtgctctt 1080

tgagttgatg cctgtcataa ggatttatgc agaaaacaat actttacgag atcctcggtt 1140

ttactcctgt cccatctata agaagccagt tcgaacggac ttgaactaca ttgccgctgt 1200

ggatctcagg acagcccaga cccctgaaca ctgggtgctc cgtggggttg cccttctgtg 1260

tgatgtcaag taacatgtgg ggagtgtccc cacccaatgc tttggaaaat gcaagatcta 1320

aattattgta acctttattt ctgtatgact gctggacagt gtatgttagg tcgtttatgc 1380

aattaatgag ctgcataggt tttccccact ccttaattgg atgcttatat tttacttgtt 1440

tcatcattag tgaccaatgt ctgagtttgt tgaaaatgtt atttagtgat ataaaagtaa 1500

atttacagca tcctaatgaa gtgtggccct caaatccaca gtagtatatt ttcttcttac 1560

ttcgctccga agactgactg tgattataac agcaaatata tttgcatgtg gacaaagatt 1620

agatggcaag atagaaaaat aagaacagat gtgatagcaa gaattatagt tggcttgaaa 1680

aaatgtgatg atcaggagaa aaaataaaaa aagggtagaa atattagacg gtgcgtaggg 1740

actttctatg gacttttatt aattaggaaa cattatcaaa ggaacttttc acgtattttt 1800

ctttaaattc tggttagatg ttattaataa ttcttcatct aacctactga ctagaaaata 1860

tagtcagtac taaattagaa ttgtggttta taaacttttg gttagctctg gatctgtata 1920

actgcatttt tttggataaa cagtttttgg taggtggata ccgggagaca agtgtgggtc 1980

cctctcactg ggcttcattc tgtggaccag gatcattatt tcatgctcat gatcatgaga 2040

gttaggactg agtggctcct gtgactccca ccatcttaga tgatactgtt ttcttgtgag 2100

ttctttcttt tggtgtggat tagtatatca gttgatttgt gtgaattgtg gtgaaacaat 2160

catttcattt tgaaaagcaa gtaatgaaaa tgtcagcatc ataggaatta ataaaatgtt 2220

tttactaaaa aaaaaaaaaa aaa 2243

<210> 46
<211> 394
<212> PRT
<213> Homo sapiens

<400> 46

<210>  47
<211>  6041
<212>  DNA
<213>  Homo sapiens

<400>  47
ggagcagcct acaacttcac aaccagaaac cactacccct caggggttgc tttcagataa      60

agatgacatg ggaaggagaa atgctggcat agatttcgga tccagaaaag catcagcagc     120

acagcccata cctgaaaaca tggacaattc catggttagt gatccacaac cataccatga     180

agatgcagct tctggagctg agaagacaga agccagagct tctctctcac tgatggtgga     240

aagcctttct acaacccaag aggaggccat tctctcagta gcagcagagg ctcaggtgtt     300

tatgaatcct tctcatatcc agttagaaga tcaagaagct ttcagctttg atttacaaaa     360

ggcccaatcc aaaatggagt cagcccagga tgttcaaact atctgcaaag aaaagccttc     420

tggaaatgtt caccagacct ttacagcaag tgttttgggt atgacaagta ctacagccaa     480

aggagatgtt tatgccaaga ctctgcctcc cagaagcctt tttcagtcct caaggaagcc     540

tgatgctgaa gaagtctcct cagattcaga gaatattcct gaggaggggg atggttctga     600

agaactggct catggtcact cttcccagtc cttggggaag tttgaagatg aacaagaagt     660

cttctcagaa tcaaaaagtt ttgttgagga cttgagcagc tctgaggagg agctggacct     720

cagatgcctc tcccaggctt tagaggagcc tgaagatgca gaagtcttca cagaatcaag     780

cagttatgtt gaaaagtaca acacttctga tgattgcagc agctcagagg aagacctgcc     840

tctcagacac cctgctcagg ccttgggaaa gcccaaaaac caacaagaag tctcctctgc     900

ttcaaataat actcctgaag agcagaatga ttttatgcag cagctgcctt ccagatgccc     960

ttctcagccc attatgaatc ctactgttca gcaacaagtc cccaccagtt cagtgggcac 1020
ttctataaaa cagagcgatt ccgtggagcc aatccctcca agacaccctt tccagccatg 1080
ggtgaaccct aaagtggagc aagaagtttc ctcatctcca aagagcatgg ctgttgaaga 1140
gagcatttct atgaagcctc tgcctcctaa acttctttgc cagcccttga tgaatcctaa 1200
agttcaacaa aacatgttct caggttcaga ggacattgct gttgagagag tcatttctgt 1260
ggagccacta ctccccagat attctcctca gtccttgaca gatcctcaaa tccggcaaat 1320
ctcagaaagc acagctgttg aggaaggcac ttatgtggaa ccgctgcctc ccagatgcct 1380
ttcccagccc tcggagaggc ctaagttcct ggactcaatg agtacttctg cagaatggag 1440
cagtcctgtg gcaccaacac cttccaaata cacttccccg ccatgggtga cccctaaatt 1500
tgaggaactg tatcaactct ctgcacatcc agaaagcact actgttgaag aggacatttc 1560
taaggagcag ctgcttccca gacatctttc ccagttgact gtgggaaata aagtccagca 1620
actgtcctca aatttcgagc gggctgctat tgaggcagac atttctggga gtccattgcc 1680
tccccaatat gctacccagt tcttaaagag gtctaaagtt caggaaatga cctcacgact 1740
agagaaaatg gctgttgaag gcacttctaa caaatcaccg attcccaggc gtccgaccca 1800
gtcattcgtg aaatttatgg cacagcaaat cttttcagag agctctgctc ttaagagggg 1860
cagtgatgtg gcacctctgc ctcccaatct tccttccaaa tctttatcaa agcctgaagt 1920
caagcaccaa gttttctcag attcagggag tgctaatcct aagggaggca tttcttcaaa 1980
gatgctacct atgaagcacc ctttacagtc cttggggagg cctgaagacc cacagaaagt 2040
tttctcttat tcagagagag ctcctgggaa gtgcagcagt tttaaagagc agctgtctcc 2100
caggcagctt tcccaggcct tgaggaaacc tgagtatgag caaaaagtct cccctgtttc 2160
tgccagttct cctaaagagt ggaggaattc taaaaagcag ctgcctccca aacattcttc 2220
ccaagcctca gataggtcta aattccagcc acagatgtca tcaaagggcc cagtgaatgt 2280
acctgtaaag cagagcagcg gtgagaagca cctgccttca agtagtcctt tccagcaaca 2340
ggttcattca agttctgtga atgctgctgc taggcgatct gtttttgaga gcaattctga 2400
caattggttc ctaggaagag atgaagcttt tgcaatcaaa accaagaaat tcagccaagg 2460
ttccaaaaac cccataaaga gcattccagc ccctgctacc aaacctggga agttcaccat 2520
tgctcctgtc aggcaaacat ccacttctgg gggcatttac tctaagaaag aagatcttga 2580
gagtggtgat ggtaataata accagcatgc aaacctatcc aatcaggatg atgttgaaaa 2640
gctttttgga gttcgactga aaagagcccc tccctcgcag aagtataaga gtgagaaaca 2700
agataacttc acccagcttg cttcagtgcc ctcgggccca atttcatcct ctgtaggcag 2760
gggacataaa atcagaagca cttcccaggg gctcctggat gctgcaggga acctcaccaa 2820
aatatcttac gttgcagata agcaacagag caggcccaaa tctgaaagca tggccaagaa 2880
gcaacctgct tgcaagaccc caggaaagcc tgctggtcaa cagtcagatt atgctgtctc 2940
agagccggtt tggataacta tggcaaagca gaagcagaag agtttcaagg cccacatttc 3000

tgtgaaagag ctgaaaacta agagcaatgc tggagccgat gctgagacta aggagcctaa 3060
atatgaggga gctggctctg caaatgaaaa ccaacctaaa aagatgttca cttccagtgt 3120
ccataaacag gagaagacag cacagatgaa gccacctaag cctacaaaat cagttggatt 3180
tgaagctcag aagatactgc aagttcctgc catggaaaaa gaaaccaaac gatcttcaac 3240
tctcccagcc aagttccaga acccagttga gccaattgag cctgtctggt tctcactggc 3300
caggaagaaa gccaaagcat ggagccacat ggcagaaatc acgcaataaa gagctcttgt 3360
gtggagcatc agcatttatt ttatttagtt tttttttttt tttttttttt gagacagagt 3420
ctcgctctgt tacccagatt ggagtgcagt ggcgcgatct ccgctcactg caagctccgc 3480
ctcccgggtt cacgccactc tcccgcctca gtctcccgac tagctgggac tacaggcgcc 3540
cgccatcacg cccggctaat tttgttttcg tatttttagt agagacgggg tttcaccatg 3600
ttggccagga tggtcttgat ctcctgacct cgtgatccgc ccgcctcagc ctcccaaaag 3660
ctgggattac aggcgtgagc caccgcgccc ggccaagcat cagcgtttta aatgataatt 3720
gctaatagct gtattaattc tatgtagtga tctttttact gtgaccactt gtattaagca 3780
aaataagtat taagcaaact aagaatttat taagcaaaat aagaatttat taagcaaaat 3840
agccttagaa atgcaaatta aaacataatt atttgaatga aataaatgcc atgaatgctt 3900
aaccttccac gtagtcactg ccagcaccca gaaacccagc atttcctcta ttaaaactat 3960
cgaaaacatt tgcactgctg taaaattgca aaatctttaa ctttggacaa tgtgctttag 4020
aagggagaaa gcaaaaacat tttgttggag caactagaaa attgtcattt ccctcaacca 4080
aataaagtaa ttctaatgga aacattcaga tgatttgacc taaagattgg cctttaggtt 4140
ttatgagcct agatagatgc cgcaattatt tggttgttgc tctaagcttt gcaagggatc 4200
ctaaaagagg cggtggaagt gaaaattctg ggtctccaag aaaatttctg cacagccagt 4260
tctccaatca gcctatcacc ccttgaaaca tcttccctgt gtccctgggg gcccctgatg 4320
ctttctcctt gggtgatagt aacatgcaga gcacttacac aaagctccct ctttggacat 4380
accccacgtc gacctgtcac aggcctggct gtagcgagca cctccctatg acgcagaatg 4440
cttcttggga attatcttac tcctctggag ggttagtcca tcaatgtttt gcttcttgtc 4500
ccaatactac tgtgaccctc tctgatcgca cagaaatcac tgcctatcac atatatcctg 4560
ttaagcactg aagaccctat tgaaattaga gttctacaga tgccaaaagc tgtactttcc 4620
atcaggcaga tggcaagctt actgccttga tgcacatctg gagccactgg agctccttcc 4680
tctctggttc cagcattaag gtggagaact ccatgtagct tcttgtcctt tcccctcagc 4740
tgtctttgct tcacaaggtt ttagcccaaa gcaagagtgc aatcccaaag ccacagagaa 4800
atgaactttc cgctacctgg aagctttaag tgagtaaatc agcttttccc ctctcattcc 4860
tagaggcaca cacctcaaaa gttactaggc tggagagacc ctaccttcca gtgacccact 4920
catcccccag ccacggagaa gagggaagac caaaaaggga gagtgagaaa gaggatgaga 4980
gggatggtca gctgtgaggg gagggggcaa gtggcccagc aaatgttgat gcctcccttc 5040

ccatcttgcc acacggtctt tttcttttgt agcacagcct ccattaataa ctcctcggct 5100
gaggatgaag atgtaggcac ctttaccccc agagccagtt ccttaattgg ctggctttct 5160
gagatgcaga ccaccctaga atctcatcta ggttcactag aagttagtta aatcttcctt 5220
tctctgtctt tctcttcatt ccatccccca aacccaccaa acactaaggg agagctccct 5280
ttggatgtct gggcagtaaa cctagctcat ttttctagga gacccagaag tgacttctga 5340
gtagttatca ctgtgtctgc ctctgttaca ctgtgctgct ttgcttaaac agaaatgcag 5400
gcctggacat ctgactgtgc ctttatattc tgagtggggt gctgccccat gcaaaaaaat 5460
ccagagaggt agtgaggtgt cagagctaaa cacttggtgc tgggttttgt tgatgctggt 5520
ataatgtgac acagtacaat tacatgctaa attttgcatt ttctctatat aacatctatt 5580
tttcctgata ctgtgccttt gccattttga taatgctatt ttgattgagt gaattttatt 5640
tcctttgtat tcccatagtg aacaatatat taaggtagat gccctttatc tgggtactcc 5700
tggtagatta gctgttacac ctcccttccc ttttttacag tgaacctgta ttcagttatt 5760
gtcactctga gaactctcca ataacaattt cttttccaca gttaacaaca cagctgttac 5820
acctcccttc ctttttttca cagtgaacct gtattcagct attctcactc tgagaactct 5880
ccaataacaa tttcttttcc acagttaaca acaaagttct gtttttaaat gaagagatta 5940
agttcttttt aaatgcctaa aggcatattc tgacaacttt tctacttctt taactttttt 6000
gatttaagat atatgcaaag caaataaatt caataaagcc t 6041

<210> 48
<211> 1115
<212> PRT
<213> Homo sapiens

<400> 48
   Glu Gln Pro Thr Thr Ser Gln Pro Glu Thr Thr Thr Pro Gln Gly Leu
   1 5 10 15
   Leu Ser Asp Lys Asp Asp Met Gly Arg Arg Asn Ala Gly Ile Asp Phe
   20 25 30
   Gly Ser Arg Lys Ala Ser Ala Ala Gln Pro Ile Pro Glu Asn Met Asp
   35 40 45
   Asn Ser Met Val Ser Asp Pro Gln Pro Tyr His Glu Asp Ala Ala Ser
   50 55 60
   Gly Ala Glu Lys Thr Glu Ala Arg Ala Ser Leu Ser Leu Met Val Glu
   65 70 75 80
   Ser Leu Ser Thr Thr Gln Glu Glu Ala Ile Leu Ser Val Ala Ala Glu
   85 90 95
   Ala Gln Val Phe Met Asn Pro Ser His Ile Gln Leu Glu Asp Gln Glu
   100 105 110
   Ala Phe Ser Phe Asp Leu Gln Lys Ala Gln Ser Lys Met Glu Ser Ala
   115 120 125
   Gln Asp Val Gln Thr Ile Cys Lys Glu Lys Pro Ser Gly Asn Val His
   130 135 140
   Gln Thr Phe Thr Ala Ser Val Leu Gly Met Thr Ser Thr Thr Ala Lys
   145                 150                 155                 160
   Gly Asp Val Tyr Ala Lys Thr Leu Pro Pro Arg Ser Leu Phe Gln Ser
                   165                 170                 175
   Ser Arg Lys Pro Asp Ala Glu Glu Val Ser Ser Asp Ser Glu Asn Ile
               180                 185                 190
   Pro Glu Glu Gly Asp Gly Ser Glu Glu Leu Ala His Gly His Ser Ser
           195                 200                 205
   Gln Ser Leu Gly Lys Phe Glu Asp Glu Gln Glu Val Phe Ser Glu Ser
       210                 215                 220
   Lys Ser Phe Val Glu Asp Leu Ser Ser Ser Glu Glu Glu Leu Asp Leu
   225                 230                 235                 240
   Arg Cys Leu Ser Gln Ala Leu Glu Glu Pro Glu Asp Ala Glu Val Phe
                   245                 250                 255
   Thr Glu Ser Ser Ser Tyr Val Glu Lys Tyr Asn Thr Ser Asp Asp Cys
               260                 265                 270
   Ser Ser Ser Glu Glu Asp Leu Pro Leu Arg His Pro Ala Gln Ala Leu
           275                 280                 285
   Gly Lys Pro Lys Asn Gln Gln Glu Val Ser Ser Ala Ser Asn Asn Thr
       290                 295                 300
   Pro Glu Glu Gln Asn Asp Phe Met Gln Gln Leu Pro Ser Arg Cys Pro
   305                 310                 315                 320
   Ser Gln Pro Ile Met Asn Pro Thr Val Gln Gln Gln Val Pro Thr Ser
                   325                 330                 335
   Ser Val Gly Thr Ser Ile Lys Gln Ser Asp Ser Val Glu Pro Ile Pro
               340                 345                 350
   Pro Arg His Pro Phe Gln Pro Trp Val Asn Pro Lys Val Glu Gln Glu
           355                 360                 365
   Val Ser Ser Ser Pro Lys Ser Met Ala Val Glu Glu Ser Ile Ser Met
       370                 375                 380
   Lys Pro Leu Pro Pro Lys Leu Leu Cys Gln Pro Leu Met Asn Pro Lys
   385                 390                 395                 400
   Val Gln Gln Asn Met Phe Ser Gly Ser Glu Asp Ile Ala Val Glu Arg
                   405                 410                 415
   Val Ile Ser Val Glu Pro Leu Leu Pro Arg Tyr Ser Pro Gln Ser Leu
               420                 425                 430
   Thr Asp Pro Gln Ile Arg Gln Ile Ser Glu Ser Thr Ala Val Glu Glu
           435                 440                 445
   Gly Thr Tyr Val Glu Pro Leu Pro Pro Arg Cys Leu Ser Gln Pro Ser
       450                 455                 460
   Glu Arg Pro Lys Phe Leu Asp Ser Met Ser Thr Ser Ala Glu Trp Ser
   465                 470                 475                 480
   Ser Pro Val Ala Pro Thr Pro Ser Lys Tyr Thr Ser Pro Pro Trp Val
                   485                 490                 495
   Thr Pro Lys Phe Glu Glu Leu Tyr Gln Leu Ser Ala His Pro Glu Ser
   500 505 510
   Thr Thr Val Glu Glu Asp Ile Ser Lys Glu Gln Leu Leu Pro Arg His
   515 520 525
   Leu Ser Gln Leu Thr Val Gly Asn Lys Val Gln Gln Leu Ser Ser Asn
   530 535 540
   Phe Glu Arg Ala Ala Ile Glu Ala Asp Ile Ser Gly Ser Pro Leu Pro
   545 550 555 560
   Pro Gln Tyr Ala Thr Gln Phe Leu Lys Arg Ser Lys Val Gln Glu Met
   565 570 575
   Thr Ser Arg Leu Glu Lys Met Ala Val Glu Gly Thr Ser Asn Lys Ser
   580 585 590
   Pro Ile Pro Arg Arg Pro Thr Gln Ser Phe Val Lys Phe Met Ala Gln
   595 600 605
   Gln Ile Phe Ser Glu Ser Ser Ala Leu Lys Arg Gly Ser Asp Val Ala
   610 615 620
   Pro Leu Pro Pro Asn Leu Pro Ser Lys Ser Leu Ser Lys Pro Glu Val
   625 630 635 640
   Lys His Gln Val Phe Ser Asp Ser Gly Ser Ala Asn Pro Lys Gly Gly
   645 650 655
   Ile Ser Ser Lys Met Leu Pro Met Lys His Pro Leu Gln Ser Leu Gly
   660 665 670
   Arg Pro Glu Asp Pro Gln Lys Val Phe Ser Tyr Ser Glu Arg Ala Pro
   675 680 685
   Gly Lys Cys Ser Ser Phe Lys Glu Gln Leu Ser Pro Arg Gln Leu Ser
   690 695 700
   Gln Ala Leu Arg Lys Pro Glu Tyr Glu Gln Lys Val Ser Pro Val Ser
   705 710 715 720
   Ala Ser Ser Pro Lys Glu Trp Arg Asn Ser Lys Lys Gln Leu Pro Pro
   725 730 735
   Lys His Ser Ser Gln Ala Ser Asp Arg Ser Lys Phe Gln Pro Gln Met
   740 745 750
   Ser Ser Lys Gly Pro Val Asn Val Pro Val Lys Gln Ser Ser Gly Glu
   755 760 765
   Lys His Leu Pro Ser Ser Ser Pro Phe Gln Gln Gln Val His Ser Ser
   770 775 780
   Ser Val Asn Ala Ala Ala Arg Arg Ser Val Phe Glu Ser Asn Ser Asp
   785 790 795 800
   Asn Trp Phe Leu Gly Arg Asp Glu Ala Phe Ala Ile Lys Thr Lys Lys
   805 810 815
   Phe Ser Gln Gly Ser Lys Asn Pro Ile Lys Ser Ile Pro Ala Pro Ala
   820 825 830
   Thr Lys Pro Gly Lys Phe Thr Ile Ala Pro Val Arg Gln Thr Ser Thr
   835 840 845
   Ser Gly Gly Ile Tyr Ser Lys Lys Glu Asp Leu Glu Ser Gly Asp Gly
   850 855 860
   Asn Asn Asn Gln His Ala Asn Leu Ser Asn Gln Asp Asp Val Glu Lys
   865                 870                 875                 880
   Leu Phe Gly Val Arg Leu Lys Arg Ala Pro Pro Ser Gln Lys Tyr Lys
                   885                 890                 895
   Ser Glu Lys Gln Asp Asn Phe Thr Gln Leu Ala Ser Val Pro Ser Gly
               900                 905                 910
   Pro Ile Ser Ser Ser Val Gly Arg Gly His Lys Ile Arg Ser Thr Ser
           915                 920                 925
   Gln Gly Leu Leu Asp Ala Ala Gly Asn Leu Thr Lys Ile Ser Tyr Val
       930                 935                 940
   Ala Asp Lys Gln Gln Ser Arg Pro Lys Ser Glu Ser Met Ala Lys Lys
   945                 950                 955                 960
   Gln Pro Ala Cys Lys Thr Pro Gly Lys Pro Ala Gly Gln Gln Ser Asp
                   965                 970                 975
   Tyr Ala Val Ser Glu Pro Val Trp Ile Thr Met Ala Lys Gln Lys Gln
               980                 985                 990
   Lys Ser Phe Lys Ala His Ile Ser  Val Lys Glu Leu Lys  Thr Lys Ser
           995                  1000                 1005
   Asn Ala  Gly Ala Asp Ala Glu  Thr Lys Glu Pro Lys  Tyr Glu Gly
       1010                 1015                 1020
   Ala Gly  Ser Ala Asn Glu Asn  Gln Pro Lys Lys Met  Phe Thr Ser
       1025                 1030                 1035
   Ser Val  His Lys Gln Glu Lys  Thr Ala Gln Met Lys  Pro Pro Lys
       1040                 1045                 1050
   Pro Thr  Lys Ser Val Gly Phe  Glu Ala Gln Lys Ile  Leu Gln Val
       1055                 1060                 1065
   Pro Ala  Met Glu Lys Glu Thr  Lys Arg Ser Ser Thr  Leu Pro Ala
       1070                 1075                 1080
   Lys Phe  Gln Asn Pro Val Glu  Pro Ile Glu Pro Val  Trp Phe Ser
       1085                 1090                 1095
   Leu Ala  Arg Lys Lys Ala Lys  Ala Trp Ser His Met  Ala Glu Ile
       1100                 1105                 1110
   Thr Gln
       1115

<210>  49
<211>  5976
<212>  DNA
<213>  Homo sapiens

<400>  49
gctttccttt ctaaagtaga agaggatgat tatccctctg aagaactact agaggatgaa      60

aacgctataa atgcaaaacg gtctaaagaa aaaaaccctg ggaatcaggg caggcagttt     120

gatgttaatc tgcaagtccc tgacagagca gttttaggga ccattcatcc agatccagaa     180

attgaagaaa gcaagcaaga aactagtatg attttggata gtgaaaaaac aagtgagact     240

gctgccaaag gggtcaacac aggaggcagg gaaccaaata caatggtgga aaaagaacgc     300

cctctggcag ataagaaagc acagagacca tttgaacgaa gtgacttttc tgacagcata     360

aaaattcaga ctccagaatt aggtgaagtg tttcagaata aagattctga ttatctgaag 420
aacgacaacc ctgaggaaca tctgaagacc tcagggcttg caggggagcc tgagggagaa 480
ctctcaaaag aggaccatgg gaacacagag aagtacatgg gcacagaaag ccaggggtct 540
gctgctgcag aacctgaaga tgactcgttc cactggactc cacatacaag tgtagagcca 600
gggcatagtg acaagaggga ggacttactt atcataagca gcttctttaa agaacaacag 660
tctttgcagc ggttccagaa gtactttaat gtccatgagc tggaagcctt gctacaagaa 720
atgtcatcaa aactgaagtc agcgcagcag gagagcctgc cctataatat ggaaaaagtc 780
ctagataagg tcttccgtgc ttctgagtca caaattctga gcatagcaga aaaaatgctt 840
gatactcgtg tggctgaaaa tagagatctg ggaatgaacg aaaataacat atttgaagag 900
gctgcagtgc ttgatgacat tcaagacctc atctattttg tcaggtacaa gcactccaca 960
gcagaggaga cagccacact ggtgatggca ccacctctag aggaaggctt gggtggagca 1020
atggaagaga tgcaaccact gcatgaagat aatttctcac gagagaagac agcagaactt 1080
aatgtgcagg ttcctgaaga acccacccac ttggaccaac gtgtgattgg ggacactcat 1140
gcctcagaag tgtcacagaa gccaaatact gagaaagacc tggacccagg gccagttaca 1200
acagaagaca ctcctatgga tgctattgat gcaaacaagc aaccagagac agccgccgaa 1260
gagccggcaa gtgtcacacc tttggaaaac gcaatccttc taatatattc attcatgttt 1320
tatttaacta agtcgctagt tgctacattg cctgatgatg ttcagcctgg gcctgatttt 1380
tatggactgc catggaaacc tgtatttatc actgccttct tgggaattgc ttcgtttgcc 1440
attttcttat ggagaactgt ccttgttgtg aaggatagag tatatcaagt cacggaacag 1500
caaatttctg agaagttgaa gactatcatg aaagaaaata cagaacttgt acaaaaattg 1560
tcaaattatg aacagaagat caaggaatca aagaaacatg ttcaggaaac caggaaacaa 1620
aatatgattc tctctgatga agcaattaaa tataaggata aaatcaagac acttgaaaaa 1680
aatcaggaaa ttctggatga cacagctaaa aatcttcgtg ttatgctaga atctgagaga 1740
gaacagaatg tcaagaatca ggacttgata tcagaaaaca agaaatctat agagaagtta 1800
aaggatgtta tttcaatgaa tgcctcagaa ttttcagagg ttcagattgc acttaatgaa 1860
gctaagctta gtgaagagaa ggtgaagtct gaatgccatc gggttcaaga agaaaatgct 1920
aggcttaaga agaaaaaaga gcagttgcag caggaaatcg aagactggag taaattacat 1980
gctgagctca gtgagcaaat caaatcattt gagaagtctc agaaagattt ggaagtagct 2040
cttactcaca aggatgataa tattaatgct ttgactaact gcattacaca gttgaatctg 2100
ttagagtgtg aatctgaatc tgagggtcaa aataaaggtg gaaatgattc agatgaatta 2160
gcaaatggag aagtgggagg tgaccggaat gagaagatga aaaatcaaat taagcagatg 2220
atggatgtct ctcggacaca gactgcaata tcggtagttg aagaggatct aaagctttta 2280
cagcttaagc taagagcctc cgtgtccact aaatgtaacc tggaagacca ggtaaagaaa 2340
ttggaagatg accgcaactc actacaagct gccaaagctg gactggaaga tgaatgcaaa 2400

accttgaggc agaaagtgga gattctgaat gagctctatc agcagaagga gatggctttg 2460
caaaagaaac tgagtcaaga agagtatgaa cggcaagaaa gagagcacag gctgtcagct 2520
gcagatgaaa aggcagtttc ggctgcagag gaagtaaaaa cttacaagcg gagaattgaa 2580
gaaatggagg atgaattaca gaagacagag cggtcattta aaaaccagat cgctacccat 2640
gagaagaaag ctcatgaaaa ctggctcaaa gctcgtgctg cagaaagagc tatagctgaa 2700
gagaaaaggg aagctgccaa tttgagacac aaattattag aattaacaca aaagatggca 2760
atgctgcaag aagaacctgt gattgtaaaa ccaatgccag gaaaaccaaa tacacaaaac 2820
cctccacgga gaggtcctct gagccagaat ggctcttttg gcccatcccc tgtgagtggt 2880
ggagaatgct cccctccatt gacagtggag ccacccgtga gacctctctc tgctactctc 2940
aatcgaagag atatgcctag aagtgaattt ggatcagtgg acgggcctct acctcatcct 3000
cgatggtcag ctgaggcatc tgggaaaccc tctccttctg atccaggatc tggtacagct 3060
accatgatga acagcagctc aagaggctct tcccctacca gggtactcga tgaaggcaag 3120
gttaatatgg ctccaaaagg gccccctcct ttcccaggag tccctctcat gagcaccccc 3180
atgggaggcc ctgtaccacc acccattcga tatggaccac cacctcagct ctgcggacct 3240
tttgggcctc ggccacttcc tccacccttt ggccctggta tgcgtccacc actaggctta 3300
agagaatttg caccaggcgt tccaccagga agacgggacc tgcctctcca ccctcgggga 3360
tttttacctg gacacgcacc atttagacct ttaggttcac ttggcccaag agagtacttt 3420
attcctggta cccgattacc acccccaacc catggtcccc aggaataccc accaccacct 3480
gctgtaagag acttactgcc gtcaggctct agagatgagc ctccacctgc ctctcagagc 3540
actagccagg actgttcaca ggctttaaaa cagagcccat aaaactatga cctctgaggt 3600
ttcattggaa agaaagtgta ctgtgcatta tccattacag taaaggattt cattggcttc 3660
aaaatccaaa agtttatttt aaaaggtttg ttgttagaac taagctgcct tggcagtgtg 3720
catttttgag ccaaacaatt caaaaatgtc atttcttccc taaataaaaa tcacctttta 3780
agctagagcg tccttacaac tttgaaatgt gcaataaaga atacctgtgt tttagctaat 3840
gtagcatatg taattgcaaa atgatttaga atgtcatgaa aaatatgaac atttcctgtg 3900
gaaatgcttt aagaacatgt atttccatta tcctattttt agtgtacacc agctgaatac 3960
ggagcaatgg tgtttataag cgttttttta aactatctgg tcacaaagac tgttacgcta 4020
aaaatgttta ctaaaagatc actaaactat ctcccctctt gctgaagttc tttgtagtaa 4080
tagctcataa aaatttgttt attaatattt cccaagtgtc tgttgactca ttggactgtt 4140
atgaggcttg tgccatttgg ggaacatgta aactcaggct cccagaactg aagatggtgg 4200
ctggtggcac acttccggct gctcctccgt cacctgtgaa ctctacaagt gatgtctttt 4260
tatttcaaag aagtttattt cccacttgta tagcattcac atgctttctt tacgatcctc 4320
attgtctatt tgagaatggt tttctgagag tgagtttaca ttagtagcaa gagttgtttg 4380
acctgatgtt ccattgtttt taccattcct gtagaaaaag ggtgcacaac agaaaaatga 4440

aaatgatgtg tcatggccat aaaagtatag aaatctttaa aaattttaaa atgtacagtc 4500
ccttatctat ctttcccatt ccttgccact gatttttgag gaatataata aaaagattgg 4560
aagagtataa tgccatgaga aagaatgatt taggactgtg agggttataa catgccctag 4620
gtcagcaacc aagggttgaa atcagttctg ttttaggggg aaatgggggg ggcgacagat 4680
attattccaa aattaatatt aattaatatt taaacgttgg tgtttttatt taaaaatcag 4740
taactaacca tctggaattg caccatactt aaagtcttat ccattactac actgtcttta 4800
aaacaatgtt tctttaaata ctctacaacg tttctaagaa cgaacttcag acattttaat 4860
tacagtaata atagcactcc ttttaaggag tttcagatcc acactaaaac taaaatcata 4920
aaaggctgat acttttgttt gctgctaggc tatattcttc cattctttga agtcctatga 4980
tgtaatattt ttgaaaccta gtgtatgtct tgtcactgtt gtgatattta atcgattaag 5040
aataccttgt aaaaaggagc aaaagcttca atgtgaaaca attttctctc tttatactaa 5100
acaactgaag atagatagtt tagaaagata aggacctttg aaagaagaca actctgtcaa 5160
agttcataag gaatataaaa attcttcagg aaaagagaat tcaatctata tgtcctcccg 5220
tttaatatca agaatagaag aaattaagag gaaaactcca cagaagagca taggccactt 5280
ttagccatgt aaaaataaga ttaagtcaca aatacaactt ttgaatttac ctgtcaatat 5340
ctctttagga cacaaaacaa tgctgaagtt aatataattt ctaattttaa atgtcattta 5400
agtgtagatt atgccatcta ggaaggtaag taggaaaggt aaattaaatc tatttttaaa 5460
attcaaaata ttagagtatt tttcccctct aaagcctttt ttggtgatta ttctgtatct 5520
gacataattg agaaactggt aagctgtaaa gattccagtg tagcttctct gagaagttgt 5580
gagccagtcc ataactgctt cctcacatcc atctgattgc accatttctg cagcaaaccc 5640
caaagcaggg tgccaatatg cagatggcat agggagtatc atccctcagc caaatcactt 5700
ttccatctct aaagtttcat ctattttgga agtcatctcc aactaattgt gtctggattt 5760
agttgctaaa attgtcttat ttatttatga agcagcaata ttcagcctga aagcatttct 5820
gccatagttg ttgtagttat atcgccaatg gctgattttt ttcattggaa agtaaattta 5880
agtaattcgt gggatgtggt atattctgtg tcaacttcaa gataatcact cattttctcg 5940
ttatattcag gtctgaatta aagttaagtt aatcac 5976

<210> 50
<211> 1193
<212> PRT
<213> Homo sapiens

<400> 50

Ala Phe Leu Ser Lys Val Glu Glu Asp Asp Tyr Pro Ser Glu Glu Leu
1               5                   10                  15

Leu Glu Asp Glu Asn Ala Ile Asn Ala Lys Arg Ser Lys Glu Lys Asn
            20                  25                  30

Pro Gly Asn Gln Gly Arg Gln Phe Asp Val Asn Leu Gln Val Pro Asp
        35                  40                  45

Arg Ala Val Leu Gly Thr Ile His Pro Asp Pro Glu Ile Glu Glu Ser
    50                  55                  60

Lys Gln Glu Thr Ser Met Ile Leu Asp Ser Glu Lys Thr Ser Glu Thr
65                  70                  75                  80

Ala Ala Lys Gly Val Asn Thr Gly Gly Arg Glu Pro Asn Thr Met Val
                85                  90                  95

Glu Lys Glu Arg Pro Leu Ala Asp Lys Lys Ala Gln Arg Pro Phe Glu
            100                 105                 110

Arg Ser Asp Phe Ser Asp Ser Ile Lys Ile Gln Thr Pro Glu Leu Gly
        115                 120                 125

Glu Val Phe Gln Asn Lys Asp Ser Asp Tyr Leu Lys Asn Asp Asn Pro
    130                 135                 140

Glu Glu His Leu Lys Thr Ser Gly Leu Ala Gly Glu Pro Glu Gly Glu
145                 150                 155                 160

Leu Ser Lys Glu Asp His Gly Asn Thr Glu Lys Tyr Met Gly Thr Glu
                165                 170                 175

Ser Gln Gly Ser Ala Ala Ala Glu Pro Glu Asp Asp Ser Phe His Trp
            180                 185                 190

Thr Pro His Thr Ser Val Glu Pro Gly His Ser Asp Lys Arg Glu Asp
        195                 200                 205

Leu Leu Ile Ile Ser Ser Phe Phe Lys Glu Gln Gln Ser Leu Gln Arg
    210                 215                 220

Phe Gln Lys Tyr Phe Asn Val His Glu Leu Glu Ala Leu Leu Gln Glu
225                 230                 235                 240

Met Ser Ser Lys Leu Lys Ser Ala Gln Gln Glu Ser Leu Pro Tyr Asn
                245                 250                 255

Met Glu Lys Val Leu Asp Lys Val Phe Arg Ala Ser Glu Ser Gln Ile
            260                 265                 270

Leu Ser Ile Ala Glu Lys Met Leu Asp Thr Arg Val Ala Glu Asn Arg
        275                 280                 285

Asp Leu Gly Met Asn Glu Asn Asn Ile Phe Glu Glu Ala Ala Val Leu
    290                 295                 300

Asp Asp Ile Gln Asp Leu Ile Tyr Phe Val Arg Tyr Lys His Ser Thr
305                 310                 315                 320

Ala Glu Glu Thr Ala Thr Leu Val Met Ala Pro Pro Leu Glu Glu Gly
                325                 330                 335

Leu Gly Gly Ala Met Glu Glu Met Gln Pro Leu His Glu Asp Asn Phe
            340                 345                 350

Ser Arg Glu Lys Thr Ala Glu Leu Asn Val Gln Val Pro Glu Glu Pro
        355                 360                 365

Thr His Leu Asp Gln Arg Val Ile Gly Asp Thr His Ala Ser Glu Val
    370                 375                 380

Ser Gln Lys Pro Asn Thr Glu Lys Asp Leu Asp Pro Gly Pro Val Thr
385                 390                 395                 400

Thr Glu Asp Thr Pro Met Asp Ala Ile Asp Ala Asn Lys Gln Pro Glu

                405                 410                 415

Thr Ala Ala Glu Glu Pro Ala Ser Val Thr Pro Leu Glu Asn Ala Ile
            420                 425                 430

Leu Leu Ile Tyr Ser Phe Met Phe Tyr Leu Thr Lys Ser Leu Val Ala
        435                 440                 445

Thr Leu Pro Asp Asp Val Gln Pro Gly Pro Asp Phe Tyr Gly Leu Pro
    450                 455                 460

Trp Lys Pro Val Phe Ile Thr Ala Phe Leu Gly Ile Ala Ser Phe Ala
465                 470                 475                 480

Ile Phe Leu Trp Arg Thr Val Leu Val Val Lys Asp Arg Val Tyr Gln
                485                 490                 495

Val Thr Glu Gln Gln Ile Ser Glu Lys Leu Lys Thr Ile Met Lys Glu
            500                 505                 510

Asn Thr Glu Leu Val Gln Lys Leu Ser Asn Tyr Glu Gln Lys Ile Lys
        515                 520                 525

Glu Ser Lys Lys His Val Gln Glu Thr Arg Lys Gln Asn Met Ile Leu
    530                 535                 540

Ser Asp Glu Ala Ile Lys Tyr Lys Asp Lys Ile Lys Thr Leu Glu Lys
545                 550                 555                 560

Asn Gln Glu Ile Leu Asp Asp Thr Ala Lys Asn Leu Arg Val Met Leu
                565                 570                 575

Glu Ser Glu Arg Glu Gln Asn Val Lys Asn Gln Asp Leu Ile Ser Glu
            580                 585                 590

Asn Lys Lys Ser Ile Glu Lys Leu Lys Asp Val Ile Ser Met Asn Ala
        595                 600                 605

Ser Glu Phe Ser Glu Val Gln Ile Ala Leu Asn Glu Ala Lys Leu Ser
    610                 615                 620

Glu Glu Lys Val Lys Ser Glu Cys His Arg Val Gln Glu Glu Asn Ala
625                 630                 635                 640

Arg Leu Lys Lys Lys Lys Glu Gln Leu Gln Gln Glu Ile Glu Asp Trp
                645                 650                 655

Ser Lys Leu His Ala Glu Leu Ser Glu Gln Ile Lys Ser Phe Glu Lys
            660                 665                 670

Ser Gln Lys Asp Leu Glu Val Ala Leu Thr His Lys Asp Asp Asn Ile
        675                 680                 685

Asn Ala Leu Thr Asn Cys Ile Thr Gln Leu Asn Leu Leu Glu Cys Glu
    690                 695                 700

Ser Glu Ser Glu Gly Gln Asn Lys Gly Gly Asn Asp Ser Asp Glu Leu
705                 710                 715                 720

Ala Asn Gly Glu Val Gly Gly Asp Arg Asn Glu Lys Met Lys Asn Gln
                725                 730                 735

Ile Lys Gln Met Met Asp Val Ser Arg Thr Gln Thr Ala Ile Ser Val
            740                 745                 750

Val Glu Glu Asp Leu Lys Leu Leu Gln Leu Lys Leu Arg Ala Ser Val
        755                 760                 765

Ser Thr Lys Cys Asn Leu Glu Asp Gln Val Lys Lys Leu Glu Asp Asp
    770                 775                 780

Arg Asn Ser Leu Gln Ala Ala Lys Ala Gly Leu Glu Asp Glu Cys Lys
785                 790                 795                 800

Thr Leu Arg Gln Lys Val Glu Ile Leu Asn Glu Leu Tyr Gln Gln Lys
                805                 810                 815

Glu Met Ala Leu Gln Lys Lys Leu Ser Gln Glu Glu Tyr Glu Arg Gln
            820                 825                 830

Glu Arg Glu His Arg Leu Ser Ala Ala Asp Glu Lys Ala Val Ser Ala
        835                 840                 845

Ala Glu Glu Val Lys Thr Tyr Lys Arg Arg Ile Glu Glu Met Glu Asp
    850                 855                 860

Glu Leu Gln Lys Thr Glu Arg Ser Phe Lys Asn Gln Ile Ala Thr His
865                 870                 875                 880

Glu Lys Lys Ala His Glu Asn Trp Leu Lys Ala Arg Ala Ala Glu Arg
                885                 890                 895

Ala Ile Ala Glu Glu Lys Arg Glu Ala Ala Asn Leu Arg His Lys Leu
            900                 905                 910

Leu Glu Leu Thr Gln Lys Met Ala Met Leu Gln Glu Glu Pro Val Ile
        915                 920                 925

Val Lys Pro Met Pro Gly Lys Pro Asn Thr Gln Asn Pro Pro Arg Arg
    930                 935                 940

Gly Pro Leu Ser Gln Asn Gly Ser Phe Gly Pro Ser Pro Val Ser Gly
945                 950                 955                 960

Gly Glu Cys Ser Pro Pro Leu Thr Val Glu Pro Pro Val Arg Pro Leu
                965                 970                 975

Ser Ala Thr Leu Asn Arg Arg Asp Met Pro Arg Ser Glu Phe Gly Ser
            980                 985                 990

Val Asp Gly Pro Leu Pro His Pro  Arg Trp Ser Ala Glu  Ala Ser Gly
        995                 1000                 1005

Lys Pro  Ser Pro Ser Asp Pro  Gly Ser Gly Thr Ala  Thr Met Met
    1010                 1015                 1020

Asn Ser  Ser Ser Arg Gly Ser  Ser Pro Thr Arg Val  Leu Asp Glu
    1025                 1030                 1035

Gly Lys  Val Asn Met Ala Pro  Lys Gly Pro Pro Pro  Phe Pro Gly
    1040                 1045                 1050

Val Pro  Leu Met Ser Thr Pro  Met Gly Gly Pro Val  Pro Pro Pro
    1055                 1060                 1065

Ile Arg  Tyr Gly Pro Pro Pro  Gln Leu Cys Gly Pro  Phe Gly Pro
    1070                 1075                 1080

Arg Pro  Leu Pro Pro Pro Phe  Gly Pro Gly Met Arg  Pro Pro Leu
    1085                 1090                 1095

Gly Leu  Arg Glu Phe Ala Pro  Gly Val Pro Pro Gly  Arg Arg Asp
    1100                 1105                 1110

Leu Pro  Leu His Pro Arg Gly  Phe Leu Pro Gly His  Ala Pro Phe
    1115                 1120                 1125

Arg Pro Leu Gly Ser Leu Gly Pro Arg Glu Tyr Phe Ile Pro Gly
    1130                1135                1140

Thr Arg Leu Pro Pro Pro Thr His Gly Pro Gln Glu Tyr Pro Pro
    1145                1150                1155

Pro Pro Ala Val Arg Asp Leu Leu Pro Ser Gly Ser Arg Asp Glu
    1160                1165                1170

Pro Pro Pro Ala Ser Gln Ser Thr Ser Gln Asp Cys Ser Gln Ala
    1175                1180                1185

Leu Lys Gln Ser Pro
    1190

<210> 51
<211> 3287
<212> DNA
<213> Homo sapiens

<400> 51
agactgaggc ggaggcagcc ccgcgccgcg ccggacccga gcatatttca ttttctgtca 60

ttggactttg agccattaga accatgagca actacagtgt gtcactggtt ggcccagctc 120

cttggggttt ccggctgcag ggcggtaagg atttcaacat gcctctgaca atctctagtc 180

taaaagatgg cggcaaggca gcccaggcaa atgtaagaat aggcgatgtg gttctcagca 240

ttgatggaat aaatgcacaa ggaatgactc atcttgaagc ccagaataag attaagggtt 300

gtacaggctc tttgaatatg actctgcaaa gagcatctgc tgcacccaag cctgagccgg 360

ttcctgttca aaagggagaa cctaaagaag tagttaaacc tgtgcccatt acatctcctg 420

ctgtgtccaa agtcacttcc acaaacaaca tggcctacaa taaggcacca cggccttttg 480

gttctgtgtc ttcaccaaaa gtcacatcca tcccatcacc atcgtctgcc ttcaccccag 540

cccatgcgac cacctcatca catgcttccc cttcacccgt ggctgccgtc actcctcccc 600

tgttcgctgc atctggactg catgctaatg ccaatcttag tgctgaccag tctccatctg 660

cactgagcgc tggtaaaact gcagttaatg tcccacggca gcccacagtc accagcgtgt 720

gttccgagac ttctcaggag ctagcagagg gacagagaag aggatcccag ggtgacagta 780

aacagcaaaa tggcccacca agaaaacaca ttgtggagcg ctatacagag ttttatcatg 840

tacccactca cagtgatgcc agcaagaaga gactgattga ggatactgaa gactggcgtc 900

caagaactgg aacaactcag tctcgctctt tccgaatcct tgcccagatc actgggactg 960

aacatttgaa agaatctgaa gccgataata caaagaaggc aaataactct caggagcctt 1020

ctccgcagtt ggcttccttg gtagcttcca cacggagcat gcccgagagc ctggacagcc 1080

caacctctgg cagaccaggg gttaccagcc tcacaactgc agctgccttc aagcctgtag 1140

gatccactgg cgtcatcaag tcaccaagct ggcaacggcc aaaccaagga gtaccttcca 1200

ctggaagaat ctcaaacagc gctacttact caggatcagt ggcaccagcc aactcagctt 1260

tgggacaaac ccagccaagt gaccaggaca ctttagtgca aagagctgag cacattccag 1320

cagggaaacg aactccgatg tgcgcccatt gtaaccaggt catcagagga ccattcttag 1380

tggcactggg gaaatcttgg cacccagaag aattcaactg cgctcactgc aaaaatacaa 1440
tggcctacat tggatttgta gaggagaaag gagccctgta ttgtgagctg tgctatgaga 1500
aattctttgc ccctgaatgt ggtcgatgcc aaaggaagat ccttggagaa gtcatcaatg 1560
cgttgaaaca aacttggcat gtttcctgtt ttgtgtgtgt agcctgtgga aagcccattc 1620
ggaacaatgt ttttcacttg gaggatggtg aaccctactg tgagactgat tattatgccc 1680
tctttggtac tatatgccat ggatgtgaat ttcccataga agctggtgac atgttcctgg 1740
aagctctggg ctacacctgg catgacactt gctttgtatg ctcagtgtgt tgtgaaagtt 1800
tggaaggtca gacctttttc tccaagaagg acaagcccct gtgtaagaaa catgctcatt 1860
ctgtgaattt ttgaaagtca acagttcagg agaagagaag gaatttgaag agaaaaagga 1920
aaattaaaat tactaattaa tttttagatt caatatttat atggagtttt gaaaaataat 1980
agtggccctg aaggaataaa ttccagcttt aaaaaccaag tctgaggaaa tatttggctt 2040
cataaagtaa agagacggtt tggcatttat tattactttt tcctgtattt tatgcccata 2100
aaataagctt tataaaaacc aatttcctga tggactatta aattcatctt agaataaatt 2160
agtgaagaat ttaattttag aataaataat ccaatctgaa ataattatac cttctttcct 2220
tgttaggtag ttatgagtaa atctgcaaaa ggcaatgaaa atgccttaaa ttttatcaat 2280
aacagaatta ttgtatttaa aaaaaaacta atacttatct ttaaaatagt aaataggatt 2340
ttaaacagag aattttatca gtaataggtg tcagttttta aaaaattgct tgtaggctga 2400
gcgcggtggc tcacgcctgt aatcccagca ctttgggagg ccaaggtggg tggaccacat 2460
gaggtcagga gtttgagatc agcctggcca acatggtgaa accccatctc tactaaaaat 2520
acaaaaatta gccggacgca gtggcacgcg cctgtaatcc cagctactca agaggctgag 2580
gcacgagaat cacttgaacc cgggagggag aggttgcagt gagccaagat cgtaccactg 2640
cactccagcc tgggtgacag agtgagactc cgtctccaaa aaaaaacttt gcttgtatat 2700
tatttttgcc ttacagtgga tcattctagt aggaaaggac aataagattt tttatcaaaa 2760
tgtgtcatgc cagtaagaga tgttatattc ttttcttatt tcttccccac ccaaaaataa 2820
gctaccatat agcttataag tctcaaattt ttgcctttta ctaaaatgtg attgtttctg 2880
ttcattgtgt atgcttcatc acctatatta ggcaaattcc attttttccc ttgcgctaag 2940
gtaaagattt aattaaataa ttttggcctc tcatagtttt ctctctcttt aaagagaata 3000
aatagagggc caggtgtggt ggctcacgcc tgtgatccca gcactttggg aggccaagac 3060
gggcggatca tgaggtcaag agatcaagat catcctggcc aacatggtga aaccctgtct 3120
ctactaaaaa tacaaaaatg agctgggcat ggtggggcgt gcctgtagtc ccatgtactt 3180
gggaggctga ggcaggaaaa ttcttgaacc caggagacgg aagttgcagt gagctgagat 3240
cacaccactg cactccagcc tggtgacaga gcaagactcc ggctctt 3287

<210> 52
<211> 596
<212> PRT
<213> Homo sapiens

<400> 52

Met Ser Asn Tyr Ser Val Ser Leu Val Gly Pro Ala Pro Trp Gly Phe
1               5                   10                  15

Arg Leu Gln Gly Gly Lys Asp Phe Asn Met Pro Leu Thr Ile Ser Ser
            20                  25                  30

Leu Lys Asp Gly Gly Lys Ala Ala Gln Ala Asn Val Arg Ile Gly Asp
        35                  40                  45

Val Val Leu Ser Ile Asp Gly Ile Asn Ala Gln Gly Met Thr His Leu
    50                  55                  60

Glu Ala Gln Asn Lys Ile Lys Gly Cys Thr Gly Ser Leu Asn Met Thr
65                  70                  75                  80

Leu Gln Arg Ala Ser Ala Ala Pro Lys Pro Glu Pro Val Pro Val Gln
                85                  90                  95

Lys Gly Glu Pro Lys Glu Val Val Lys Pro Val Pro Ile Thr Ser Pro
            100                 105                 110

Ala Val Ser Lys Val Thr Ser Thr Asn Asn Met Ala Tyr Asn Lys Ala
        115                 120                 125

Pro Arg Pro Phe Gly Ser Val Ser Ser Pro Lys Val Thr Ser Ile Pro
    130                 135                 140

Ser Pro Ser Ser Ala Phe Thr Pro Ala His Ala Thr Thr Ser Ser His
145                 150                 155                 160

Ala Ser Pro Ser Pro Val Ala Ala Val Thr Pro Pro Leu Phe Ala Ala
                165                 170                 175

Ser Gly Leu His Ala Asn Ala Asn Leu Ser Ala Asp Gln Ser Pro Ser
            180                 185                 190

Ala Leu Ser Ala Gly Lys Thr Ala Val Asn Val Pro Arg Gln Pro Thr
        195                 200                 205

Val Thr Ser Val Cys Ser Glu Thr Ser Gln Glu Leu Ala Glu Gly Gln
    210                 215                 220

Arg Arg Gly Ser Gln Gly Asp Ser Lys Gln Gln Asn Gly Pro Pro Arg
225                 230                 235                 240

Lys His Ile Val Glu Arg Tyr Thr Glu Phe Tyr His Val Pro Thr His
                245                 250                 255

Ser Asp Ala Ser Lys Lys Arg Leu Ile Glu Asp Thr Glu Asp Trp Arg
            260                 265                 270

Pro Arg Thr Gly Thr Thr Gln Ser Arg Ser Phe Arg Ile Leu Ala Gln
        275                 280                 285

Ile Thr Gly Thr Glu His Leu Lys Glu Ser Glu Ala Asp Asn Thr Lys
    290                 295                 300

Lys Ala Asn Asn Ser Gln Glu Pro Ser Pro Gln Leu Ala Ser Leu Val
305                 310                 315                 320

Ala Ser Thr Arg Ser Met Pro Glu Ser Leu Asp Ser Pro Thr Ser Gly
                325                 330                 335

Arg Pro Gly Val Thr Ser Leu Thr Thr Ala Ala Ala Phe Lys Pro Val
            340                 345                 350

Gly Ser Thr Gly Val Ile Lys Ser Pro Ser Trp Gln Arg Pro Asn Gln
        355                 360                 365

Gly Val Pro Ser Thr Gly Arg Ile Ser Asn Ser Ala Thr Tyr Ser Gly
    370                 375                 380

Ser Val Ala Pro Ala Asn Ser Ala Leu Gly Gln Thr Gln Pro Ser Asp
385                 390                 395                 400

Gln Asp Thr Leu Val Gln Arg Ala Glu His Ile Pro Ala Gly Lys Arg
                405                 410                 415

Thr Pro Met Cys Ala His Cys Asn Gln Val Ile Arg Gly Pro Phe Leu
            420                 425                 430

Val Ala Leu Gly Lys Ser Trp His Pro Glu Glu Phe Asn Cys Ala His
        435                 440                 445

Cys Lys Asn Thr Met Ala Tyr Ile Gly Phe Val Glu Glu Lys Gly Ala
    450                 455                 460

Leu Tyr Cys Glu Leu Cys Tyr Glu Lys Phe Phe Ala Pro Glu Cys Gly
465                 470                 475                 480

Arg Cys Gln Arg Lys Ile Leu Gly Glu Val Ile Asn Ala Leu Lys Gln
                485                 490                 495

Thr Trp His Val Ser Cys Phe Val Cys Val Ala Cys Gly Lys Pro Ile
            500                 505                 510

Arg Asn Asn Val Phe His Leu Glu Asp Gly Glu Pro Tyr Cys Glu Thr
        515                 520                 525

Asp Tyr Tyr Ala Leu Phe Gly Thr Ile Cys His Gly Cys Glu Phe Pro
    530                 535                 540

Ile Glu Ala Gly Asp Met Phe Leu Glu Ala Leu Gly Tyr Thr Trp His
545                 550                 555                 560

Asp Thr Cys Phe Val Cys Ser Val Cys Cys Glu Ser Leu Glu Gly Gln
                565                 570                 575

Thr Phe Phe Ser Lys Lys Asp Lys Pro Leu Cys Lys Lys His Ala His
            580                 585                 590

Ser Val Asn Phe
        595

<210>  53
<211>  1115
<212>  DNA
<213>  Homo sapiens

<400>  53
atggccaact gtaaaatgac caaaagcatc aggttccctg ccctggagca ctgctatact      60

ggcggggagg tcgtgttgcc caaggatcag gaggagtgga aaagacggac gggccttctg     120

ctctacgaga actatgggca gtcggaaacg ggactaattt gtgccaccta ctggggaatg     180

aagatcaagc cgggtttcat ggggaaggcc actccaccct atgacgtcca gtttcatatg     240

gaggcctcag ttgaaaactg cattattgtg agcatgaaca ccgctgaccc tggcagccag     300

ggcatcacac acagcctctt gctacaggtc attgatgaca agggcagcat cctgccacct     360

aacacagaag gaaacattgg catcagaatc aaacctgtca ggcctgtgag cctcttcatg     420

tgctatgagg gtgacccaga gaagacagct aaagtggaat gtggggactt ctacaacact 480
ggggacagag gaaagatgga tgaagagggc tacatttgtt tcctggggag gagtgatgac 540
atcattaatg cctctgggta tcgcatcggg cctgcagagg ttgaaagcgc tttggtggag 600
cacccagcgg tggcggagtc agccgtggtg ggcagcccag acccgattcg aggggaggtg 660
gtgaaggcct ttattgtcct gaccccacag ttcctgtccc atgacaagga tcagctgacc 720
aaggaactgc agcagcatgt caagtcagtg acagccccat acaagtaccc aaggaaggtg 780
gagtttgtct cagagctgcc aaaaaccatc actggcaaga ttgaacggaa ggaacttcgg 840
aaaaaggaga ctggtcagat gtaatcggca gtgaactcag aacgcactgc acacctgagg 900
caaatccctg gccactttag tctccccact atggtgagga cgagggtggg gcattgagag 960
tgttgatttg ggaaagtatc aggagtgcca tgattccaat gttttccttc ttttaaatta 1020
aattcagttg ctctgcttcc tccaagtcct ctgtatcttt agaatttccc aggtgagcac 1080
tcataacgca agtaataaaa tactgatatc aacaa 1115

<210> 54
<211> 287
<212> PRT
<213> Homo sapiens

<400> 54

Met Ala Asn Cys Lys Met Thr Lys Ser Ile Arg Phe Pro Ala Leu Glu
1               5                   10                  15

His Cys Tyr Thr Gly Gly Glu Val Val Leu Pro Lys Asp Gln Glu Glu
            20                  25                  30

Trp Lys Arg Arg Thr Gly Leu Leu Leu Tyr Glu Asn Tyr Gly Gln Ser
        35                  40                  45

Glu Thr Gly Leu Ile Cys Ala Thr Tyr Trp Gly Met Lys Ile Lys Pro
    50                  55                  60

Gly Phe Met Gly Lys Ala Thr Pro Pro Tyr Asp Val Gln Phe His Met
65                  70                  75                  80

Glu Ala Ser Val Glu Asn Cys Ile Ile Val Ser Met Asn Thr Ala Asp
                85                  90                  95

Pro Gly Ser Gln Gly Ile Thr His Ser Leu Leu Leu Gln Val Ile Asp
            100                 105                 110

Asp Lys Gly Ser Ile Leu Pro Pro Asn Thr Glu Gly Asn Ile Gly Ile
        115                 120                 125

Arg Ile Lys Pro Val Arg Pro Val Ser Leu Phe Met Cys Tyr Glu Gly
    130                 135                 140

Asp Pro Glu Lys Thr Ala Lys Val Glu Cys Gly Asp Phe Tyr Asn Thr
145                 150                 155                 160

Gly Asp Arg Gly Lys Met Asp Glu Glu Gly Tyr Ile Cys Phe Leu Gly
                165                 170                 175

Arg Ser Asp Asp Ile Ile Asn Ala Ser Gly Tyr Arg Ile Gly Pro Ala
            180                 185                 190

Glu Val Glu Ser Ala Leu Val Glu His Pro Ala Val Ala Glu Ser Ala
        195                 200                 205

Val Val Gly Ser Pro Asp Pro Ile Arg Gly Glu Val Val Lys Ala Phe
    210                 215                 220

Ile Val Leu Thr Pro Gln Phe Leu Ser His Asp Lys Asp Gln Leu Thr
225                 230                 235                 240

Lys Glu Leu Gln Gln His Val Lys Ser Val Thr Ala Pro Tyr Lys Tyr
                245                 250                 255

Pro Arg Lys Val Glu Phe Val Ser Glu Leu Pro Lys Thr Ile Thr Gly
            260                 265                 270

Lys Ile Glu Arg Lys Glu Leu Arg Lys Lys Glu Thr Gly Gln Met
        275                 280                 285

<210> 55
<211> 521
<212> DNA
<213> Homo sapiens

<400> 55
gagagaggga ggcagaagag gaagtcagag cgatgtgctg tgaaatctac taccgtttgc 60

tggttttgaa aatggagaaa aagagtgagg aactgagaaa catggatggc cttgggaacg 120

tggaaaaggg tcactgaaat gggacgacat gaactcaagg aggctattta tgaccatgtc 180

atttgcaaca tgaagaaagc ttatctggag tgaaagtaaa tgagaccaac agagataaga 240

gacccggaga aatcctggtt acactgcttg aatcctgtca gtcctatact ggagtcctgt 300

taatacaaaa taatagtaat aatccctctg tttcttatgt ttatgccaac ttcaacaaaa 360

agaaacttga ctaagagaca atataagaac ttaatgtgta attaagaaag aactctccac 420

cacggggaat gtgaaaggta tatgagtccc ttttcacgat gcgatgtcat gtcttttaaa 480

taagccatac tttatgttca ataaaaagag aataagcagg a 521

<210> 56
<211> 34
<212> PRT
<213> Homo sapiens

<400> 56

Met Cys Cys Glu Ile Tyr Tyr Arg Leu Leu Val Leu Lys Met Glu Lys
1               5                   10                  15

Lys Ser Glu Glu Leu Arg Asn Met Asp Gly Leu Gly Asn Val Glu Lys
            20                  25                  30

Gly His

<210> 57
<211> 2064
<212> DNA
<213> Homo sapiens

<400> 57
atggacagtt gcctgcaaca tatgagagac ctactttacc tccttcagga gctcaggtgt 60

ttaaatccag ctacactact ccctgatcca gactccacta ctcctgttca tgactgtcag 120

gatctgttgg aaactaccaa aactggccaa cctgatcttc aagatgtgcc cctagaaaag 180
gcagatgcca ctgtgttcac agatggtagc agcttcctcg agcagggaga acgaaaagct 240
gtttcttttc cacagccaga tctgcctgac aatcccacat actcaacaga agaagaaaaa 300
ctggcttcag atgttggagc aaataaaaat caggaaggac gtgtattcgc aaacactact 360
tggagggccg gtacctccaa ggaagtctcc tttgcagttg atttatgtgt actgttccca 420
gagccagctc gtacccatga agagcaacat aatttgccgg tcataggagc aggaagtgtc 480
gaccttgcag caggatttgg acactctggg agccaaactg gatgtggaag ctccaaaggt 540
gcagaaaaag ggctccaaaa tgttgacttt tacctctgtc ctggaaatca ccctgacgct 600
agctgtagag atacttacca gtttttctgc cctgattgga catgtgtaac tttagccacc 660
tactctgggg gatcaactag atcttcaact ctttccataa gtcgtgttcc tcatcctaaa 720
ttatgtacta gaaaaaattg taatcctctt actataactg tccatgaccc taatgcagct 780
caatggtatt atggcatgtc atggggatta agactttata tcccaggatt tgatgttggg 840
actatgttca ccatccaaaa gaaaatcttg gtctcatgga gctcccccaa gccaatcggg 900
cctttaactg atctaggtga ccctatattc cagaaacacc ctgacaaagt tgatttaact 960
gttcctctgc cattcttagt tcctagaccc cagctacaac aacaacatct tcaacccagc 1020
ctaatgtcta tactaggtgg agtacaccat ctccttaacc tcacccagcc taaactagcc 1080
caagattgtt ggctatgttt aaaagcaaaa cccccttatt atgtaggatt aggagtagaa 1140
gccacactta aacgtggccc tctatcttgt catacacgac cccgtgctct cacaatagga 1200
gatgtgtctg gaaatgcttc ctgtctgatt agtaccgggt ataacttatc tgcttctcct 1260
tttcaggcta cttgtaatca gtccctgctt acttccataa gcacctcagt ctcttaccaa 1320
gcacccaaca atacctggtt ggcctgcacc tcaggtctca ctcgctgcat taatggaact 1380
gaaccaggac ctctcctgtg cgtgttagtt catgtacttc cccaggtata tgtgtacagt 1440
ggaccagaag gacgacaact catcgctccc cctgagttac atcccaggtt gcaccaagct 1500
gtcccacttc tggttcccct attggctggt cttagcatag ctggatcagc agccattggt 1560
acggctgccc tggttcaagg agaaactgga ctaatatccc tgtctcaaca ggtggatgct 1620
gattttagta acctccagtc tgccatagat atactacatt cccaggtaga gtctctggct 1680
gaagtagttc ttcaaaactg ccgatgctta gatctgctat tcctctctca aggaggttta 1740
tgtgcagctc taggagaaag ttgttgcttc tatgccaatc aatctggagt cataaaaggt 1800
acagtaaaaa aagttcgaga aaatctagat aggcaccaac aagaacgaga aaataacatc 1860
ccctggtatc aaagcatgtt taactggaac ccatggctaa ctactttaat cactgggtta 1920
gctggacctc tcctcatcct actattaagt ttaatttttg ggccttgtat attaaattcg 1980
tttcttaatt ttataaaaca acgcatagct tctgtcaaac ttacgtatct taagactcaa 2040
tatgacaccc ttgttaataa ctga 2064
<210> 58

<211>  687
<212>  PRT
<213>  Homo sapiens

<400>  58

Met Asp Ser Cys Leu Gln His Met Arg Asp Leu Leu Tyr Leu Leu Gln
1               5                   10                  15

Glu Leu Arg Cys Leu Asn Pro Ala Thr Leu Leu Pro Asp Pro Asp Ser
            20                  25                  30

Thr Thr Pro Val His Asp Cys Gln Asp Leu Leu Glu Thr Thr Lys Thr
        35                  40                  45

Gly Gln Pro Asp Leu Gln Asp Val Pro Leu Glu Lys Ala Asp Ala Thr
    50                  55                  60

Val Phe Thr Asp Gly Ser Ser Phe Leu Glu Gln Gly Glu Arg Lys Ala
65                  70                  75                  80

Val Ser Phe Pro Gln Pro Asp Leu Pro Asp Asn Pro Thr Tyr Ser Thr
                85                  90                  95

Glu Glu Glu Lys Leu Ala Ser Asp Val Gly Ala Asn Lys Asn Gln Glu
            100                 105                 110

Gly Arg Val Phe Ala Asn Thr Thr Trp Arg Ala Gly Thr Ser Lys Glu
        115                 120                 125

Val Ser Phe Ala Val Asp Leu Cys Val Leu Phe Pro Glu Pro Ala Arg
    130                 135                 140

Thr His Glu Glu Gln His Asn Leu Pro Val Ile Gly Ala Gly Ser Val
145                 150                 155                 160

Asp Leu Ala Ala Gly Phe Gly His Ser Gly Ser Gln Thr Gly Cys Gly
                165                 170                 175

Ser Ser Lys Gly Ala Glu Lys Gly Leu Gln Asn Val Asp Phe Tyr Leu
            180                 185                 190

Cys Pro Gly Asn His Pro Asp Ala Ser Cys Arg Asp Thr Tyr Gln Phe
        195                 200                 205

Phe Cys Pro Asp Trp Thr Cys Val Thr Leu Ala Thr Tyr Ser Gly Gly
    210                 215                 220

Ser Thr Arg Ser Ser Thr Leu Ser Ile Ser Arg Val Pro His Pro Lys
225                 230                 235                 240

Leu Cys Thr Arg Lys Asn Cys Asn Pro Leu Thr Ile Thr Val His Asp
                245                 250                 255

Pro Asn Ala Ala Gln Trp Tyr Tyr Gly Met Ser Trp Gly Leu Arg Leu
            260                 265                 270

Tyr Ile Pro Gly Phe Asp Val Gly Thr Met Phe Thr Ile Gln Lys Lys
        275                 280                 285

Ile Leu Val Ser Trp Ser Ser Pro Lys Pro Ile Gly Pro Leu Thr Asp
    290                 295                 300

Leu Gly Asp Pro Ile Phe Gln Lys His Pro Asp Lys Val Asp Leu Thr
305                 310                 315                 320

Val Pro Leu Pro Phe Leu Val Pro Arg Pro Gln Leu Gln Gln Gln His
                325                 330                 335

Leu Gln Pro Ser Leu Met Ser Ile Leu Gly Gly Val His His Leu Leu
            340                 345                 350

Asn Leu Thr Gln Pro Lys Leu Ala Gln Asp Cys Trp Leu Cys Leu Lys
        355                 360                 365

Ala Lys Pro Pro Tyr Tyr Val Gly Leu Gly Val Glu Ala Thr Leu Lys
    370                 375                 380

Arg Gly Pro Leu Ser Cys His Thr Arg Pro Arg Ala Leu Thr Ile Gly
385                 390                 395                 400

Asp Val Ser Gly Asn Ala Ser Cys Leu Ile Ser Thr Gly Tyr Asn Leu
                405                 410                 415

Ser Ala Ser Pro Phe Gln Ala Thr Cys Asn Gln Ser Leu Leu Thr Ser
            420                 425                 430

Ile Ser Thr Ser Val Ser Tyr Gln Ala Pro Asn Asn Thr Trp Leu Ala
        435                 440                 445

Cys Thr Ser Gly Leu Thr Arg Cys Ile Asn Gly Thr Glu Pro Gly Pro
    450                 455                 460

Leu Leu Cys Val Leu Val His Val Leu Pro Gln Val Tyr Val Tyr Ser
465                 470                 475                 480

Gly Pro Glu Gly Arg Gln Leu Ile Ala Pro Pro Glu Leu His Pro Arg
                485                 490                 495

Leu His Gln Ala Val Pro Leu Leu Val Pro Leu Leu Ala Gly Leu Ser
            500                 505                 510

Ile Ala Gly Ser Ala Ala Ile Gly Thr Ala Ala Leu Val Gln Gly Glu
        515                 520                 525

Thr Gly Leu Ile Ser Leu Ser Gln Gln Val Asp Ala Asp Phe Ser Asn
    530                 535                 540

Leu Gln Ser Ala Ile Asp Ile Leu His Ser Gln Val Glu Ser Leu Ala
545                 550                 555                 560

Glu Val Val Leu Gln Asn Cys Arg Cys Leu Asp Leu Leu Phe Leu Ser
                565                 570                 575

Gln Gly Gly Leu Cys Ala Ala Leu Gly Glu Ser Cys Cys Phe Tyr Ala
            580                 585                 590

Asn Gln Ser Gly Val Ile Lys Gly Thr Val Lys Lys Val Arg Glu Asn
        595                 600                 605

Leu Asp Arg His Gln Gln Glu Arg Glu Asn Asn Ile Pro Trp Tyr Gln
    610                 615                 620

Ser Met Phe Asn Trp Asn Pro Trp Leu Thr Thr Leu Ile Thr Gly Leu
625                 630                 635                 640

Ala Gly Pro Leu Leu Ile Leu Leu Leu Ser Leu Ile Phe Gly Pro Cys
                645                 650                 655

Ile Leu Asn Ser Phe Leu Asn Phe Ile Lys Gln Arg Ile Ala Ser Val
            660                 665                 670

Lys Leu Thr Tyr Leu Lys Thr Gln Tyr Asp Thr Leu Val Asn Asn
        675                 680                 685

<210>  59

<211>  2960
<212>  DNA
<213>  Homo sapiens

<400>  59
tgatggtgga aatttcttga aaccgctctc gtaatttgcc acgtgctgtt gcaaatattc      60

tggtgaatga acacagaatc agcatggctt tcctttgctg agaaatcact gatgggaagt     120

gagacttgtt aaacttgaaa gtgaatggac ctgagtggac cctttgatca catcagtaaa     180

catgagcggt accaaacctg atattttatg ggcaccacac catgttgata gatttgttgt     240

gtgtgactca gaactaagtc tttatcatgt ggaatctact gtgaattcag aactcaaagc     300

tggatcttta cgtttatctg aagactctgc agctacatta ctgtcaataa attcagatac     360

accctatatg aaatgtgttg cctggtatct taattatgat cctgaatgtc tgctggcagt     420

tggacaagca aatggtcgag ttgtacttac aagccttggt caagatcata actcaaagtt     480

caaagatttg ataggaaaag agtttgttcc aaaacatgca cgacaatgta atacccttgc      540

ctggaatcca ctggatagta actggctagc tgctggttta gataagcaca gagctgactt     600

ttcagtgcta atatgggata tctgcagcaa atatactcct gatatagttc ccatggaaaa     660

agtgaaactt tcagcaggtg aaactgaaac aacattatta gtaacaaaac cactttatga     720

gttaggacag aatgatgctt gtctgtctct ttgttggctt ccacgagacc agaaacttct     780

ccttgctggt atgcatcgta acctagctat atttgatctt cggaatacaa gccaaaagat     840

gttcgtaaat acaaaagctg ttcagggtgt gacggtagac ccatatttcc acgatcgtgt     900

tgcttccttc tatgaaggtc aggttgcaat atgggatctt agaaaatttg agaagccagt     960

tttgacattg actgagcaac caaaaccctt aacaaaagta gcatggtgtc ccactaggac    1020

tggtctactt gccactttaa caagggatag taatattatt agattgtatg atatgcagca    1080

tacacccact cccattgggg atgaaactga acccacaata attgaaagaa gtgtgcaacc    1140

ttgtgacaat tacattgctt cctttgcgtg gcatccaaca agtcaaaatc gaatgatagt    1200

tgtaactccc aaccgaacaa tgtcagactt cactgttttt gaaaggatat ctcttgcctg    1260

gagcccaatt acatctttaa tgtgggcttg tggtcgtcat ttatatgaat gtacggaaga    1320

agaaaatgat aattctttag aaaaagatat agcaacgaag atgcgtcttc gggctttatc    1380

aaggtatgga cttgatacag agcaggtgtg gaggaaccac attttagctg gaaatgaaga     1440

tccacagctc aagtcactct ggtatactct gcactttatg aagcaataca cagaagatat    1500

ggatcagaaa tctccaggca acaaaggatc attggtttat gcaggaatta aatcaattgt    1560

aaagtcatcg ttgggaatgg tggaaagcag cagacataat tggagtgggt tggataagca    1620

aagtgatatt caaaacttaa atgaagagag aatcttagct ttacagcttt gtgggtggat    1680

aaagaaagga acggatgtag acgtggggcc atttttgaac tcccttgtac aagaagggga     1740

atgggaaaga gctgctgctg tggcattgtt caacttggat attcgccgag caatccaaat    1800

cctgaatgaa ggggcatctt ctgaaaaagg caggagatct gaatctcaat gtggtagcaa    1860

tggctttatc gggttatacg gatgagaaga actccctttg gagagaaatg tgtagcacac    1920

tgcgattaca gctaaataac ccgtatttgt gtgtcatgtt tgcatttctg acaagtgaaa 1980

caggatctta cgatggagtt ttgtatgaaa acaaagttgc agtacgtgac agagtggcat 2040

ttgcttgtaa attccttagt gatactcaga tacatcgaaa agttgaccaa tgaaatgaaa 2100

gaggctggaa atttggaagg aattttgctt acaggcctta ctaaagatgg agtggactta 2160

atggagagtt atgttgatag aactggagat gttcaaacag caagttactg tatgttacag 2220

ggttcacctt tagatgttct taaagatgaa agggttcagt actggattga gaattataga 2280

aatttattag atgcctggag gttttggcat aaacgagctg aatttgatat tcacaggagt 2340

aagttggatc ccagttccaa gcctttagca caagtttttg tgagttgcaa tttctgtggc 2400

aagtcaatct cctacagctg ttcagctgtg cctcatcagg gcagaggttt tagtcagtat 2460

ggtgtgagtg gctcaccaac gaaatctaaa gtcacaagtt gtcctggctg tcgaaaacca 2520

cttcctcgat gtgcgctttg tctcattaat atgggaacac cagtttctag ctgtcctgga 2580

ggaaccaaat cagatgaaaa agtggacttg agcaaggaca aaaaattagc ccaatttaac 2640

aactggttta catggtgtca taattgcagg cacggtggac atgctggaca tatgcttagt 2700

tggttcaggg accatgcaga gtgccctgtg tctgcatgca cgtgtaaatg tatgcagttg 2760

gatacaacgg ggaatctggt acctgcagag actgtccagc cataaaatgt taccacctta 2820

agagaaccct tcaagtgtgg agctttctag taggtgtcct tcatagctca gaaacatacc 2880

tcagaacaag ccattcatga cttacctgta atgggaaaat aaatcattct atcagaaaaa 2940

aaaaaaaaaa aaaaaaaaaa 2960

<210> 60
<211> 567
<212> PRT
<213> Homo sapiens

<400> 60

Met Ser Gly Thr Lys Pro Asp Ile Leu Trp Ala Pro His His Val Asp
1               5                   10                  15

Arg Phe Val Val Cys Asp Ser Glu Leu Ser Leu Tyr His Val Glu Ser
            20                  25                  30

Thr Val Asn Ser Glu Leu Lys Ala Gly Ser Leu Arg Leu Ser Glu Asp
        35                  40                  45

Ser Ala Ala Thr Leu Leu Ser Ile Asn Ser Asp Thr Pro Tyr Met Lys
    50                  55                  60

Cys Val Ala Trp Tyr Leu Asn Tyr Asp Pro Glu Cys Leu Leu Ala Val
65                  70                  75                  80

Gly Gln Ala Asn Gly Arg Val Val Leu Thr Ser Leu Gly Gln Asp His
                85                  90                  95

Asn Ser Lys Phe Lys Asp Leu Ile Gly Lys Glu Phe Val Pro Lys His
            100                 105                 110

Ala Arg Gln Cys Asn Thr Leu Ala Trp Asn Pro Leu Asp Ser Asn Trp
        115                 120                 125

Leu Ala Ala Gly Leu Asp Lys His Arg Ala Asp Phe Ser Val Leu Ile
    130                 135                 140

Trp Asp Ile Cys Ser Lys Tyr Thr Pro Asp Ile Val Pro Met Glu Lys
145                 150                 155                 160

Val Lys Leu Ser Ala Gly Glu Thr Glu Thr Thr Leu Leu Val Thr Lys
                165                 170                 175

Pro Leu Tyr Glu Leu Gly Gln Asn Asp Ala Cys Leu Ser Leu Cys Trp
            180                 185                 190

Leu Pro Arg Asp Gln Lys Leu Leu Leu Ala Gly Met His Arg Asn Leu
        195                 200                 205

Ala Ile Phe Asp Leu Arg Asn Thr Ser Gln Lys Met Phe Val Asn Thr
    210                 215                 220

Lys Ala Val Gln Gly Val Thr Val Asp Pro Tyr Phe His Asp Arg Val
225                 230                 235                 240

Ala Ser Phe Tyr Glu Gly Gln Val Ala Ile Trp Asp Leu Arg Lys Phe
                245                 250                 255

Glu Lys Pro Val Leu Thr Leu Thr Glu Gln Pro Lys Pro Leu Thr Lys
            260                 265                 270

Val Ala Trp Cys Pro Thr Arg Thr Gly Leu Leu Ala Thr Leu Thr Arg
        275                 280                 285

Asp Ser Asn Ile Ile Arg Leu Tyr Asp Met Gln His Thr Pro Thr Pro
    290                 295                 300

Ile Gly Asp Glu Thr Glu Pro Thr Ile Ile Glu Arg Ser Val Gln Pro
305                 310                 315                 320

Cys Asp Asn Tyr Ile Ala Ser Phe Ala Trp His Pro Thr Ser Gln Asn
                325                 330                 335

Arg Met Ile Val Val Thr Pro Asn Arg Thr Met Ser Asp Phe Thr Val
            340                 345                 350

Phe Glu Arg Ile Ser Leu Ala Trp Ser Pro Ile Thr Ser Leu Met Trp
        355                 360                 365

Ala Cys Gly Arg His Leu Tyr Glu Cys Thr Glu Glu Glu Asn Asp Asn
    370                 375                 380

Ser Leu Glu Lys Asp Ile Ala Thr Lys Met Arg Leu Arg Ala Leu Ser
385                 390                 395                 400

Arg Tyr Gly Leu Asp Thr Glu Gln Val Trp Arg Asn His Ile Leu Ala
                405                 410                 415

Gly Asn Glu Asp Pro Gln Leu Lys Ser Leu Trp Tyr Thr Leu His Phe
            420                 425                 430

Met Lys Gln Tyr Thr Glu Asp Met Asp Gln Lys Ser Pro Gly Asn Lys
        435                 440                 445

Gly Ser Leu Val Tyr Ala Gly Ile Lys Ser Ile Val Lys Ser Ser Leu
    450                 455                 460

Gly Met Val Glu Ser Ser Arg His Asn Trp Ser Gly Leu Asp Lys Gln
465                 470                 475                 480

Ser Asp Ile Gln Asn Leu Asn Glu Glu Arg Ile Leu Ala Leu Gln Leu
                485                 490                 495

Cys Gly Trp Ile Lys Lys Gly Thr Asp Val Asp Val Gly Pro Phe Leu
            500                 505                 510

Asn Ser Leu Val Gln Glu Gly Glu Trp Glu Arg Ala Ala Ala Val Ala
        515                 520                 525

Leu Phe Asn Leu Asp Ile Arg Arg Ala Ile Gln Ile Leu Asn Glu Gly
    530                 535                 540

Ala Ser Ser Glu Lys Gly Arg Arg Ser Glu Ser Gln Cys Gly Ser Asn
545                 550                 555                 560

Gly Phe Ile Gly Leu Tyr Gly
                565

<210>  61
<211>  856
<212>  DNA
<213>  Homo sapiens

<220>
<221>  misc_feature
<222>  (37)..(37)
<223>  n is a, c, g, or t

<220>
<221>  misc_feature
<222>  (757)..(757)
<223>  n is a, c, g, or t

<220>
<221>  misc_feature
<222>  (769)..(769)
<223>  n is a, c, g, or t

<400>  61
ttgtacatct taacaacctt aagctgtaca aatagancaa taatatctaa atggtgtgat      60

gatcagccca cagtacacat cattgatgag aatttcactg gtctcaacct ttctcatgct     120

gagtcctggc tttgtaaaat gacttataaa ggtccaagga tttagagatg attaagagat     180

aagctggcat tctgtaaagg caccatcgtc tatcccctgt cttatctaga taaagaatgt     240

agtgctaaat cttgtaataa tattgtacaa atggaaattc aatcttaagg attatttttt     300

ccatattgtt gtatttcatt gtggtgtatt ggaaagtgat ctggactttg agtgagaaga     360

tgtgatttgg accatggcac ttaaaaactc tataacctca ggcaagtctt ttaatcttct     420

ctgagcctca gttttcctca tttttcaaat atagagagta taacatttat ctcataagac     480

aagttgtagt aaattactgt tttacaaatg taagataact tttaactgtg agattccata     540

ttccagtctt acattattat gtttatctgc cacagggaga agtcctcaga taaaaatgtc     600

taccaaaaga ctgacacgtg gagttaatca tttgacagat gcaaatgctt ccacccccaa     660

caaatatact ttctttaact tctgtgtggg tatcacttag ggaaaaaaag gcaggcaaca     720

aaatattttt taattctatc ttaggaaaaa ttgtagncaa atctttttnt cccattaaca     780

aataatgtaa gccttaatat tcaaggggta ataaaaatac aaagtcttcc aaacaggtaa     840

cttacttgaa aacttt                                                     856

<210>  62

<211>  348
<212>  PRT
<213>  Homo sapiens

<220>
<221>  misc_feature
<222>  (152)..(152)
<223>  Xaa can be any naturally occurring amino acid

<220>
<221>  misc_feature
<222>  (154)..(154)
<223>  Xaa can be any naturally occurring amino acid

<220>
<221>  misc_feature
<222>  (217)..(217)
<223>  Xaa can be any naturally occurring amino acid

<400>  62

Met Gly Ala Arg Gly Ala Pro Ser Arg Arg Arg Gln Ala Gly Arg Arg
1               5                   10                  15

Leu Arg Tyr Leu Pro Thr Gly Ser Phe Pro Phe Leu Leu Leu Leu Leu
            20                  25                  30

Leu Leu Cys Ile Gln Leu Gly Gly Gly Gln Lys Lys Lys Glu Asn Leu
        35                  40                  45

Leu Ala Glu Lys Val Glu Gln Leu Met Glu Trp Ser Ser Arg Arg Ser
    50                  55                  60

Ile Phe Arg Met Asn Gly Asp Lys Phe Arg Lys Phe Ile Lys Ala Pro
65                  70                  75                  80

Pro Arg Asn Tyr Ser Met Ile Val Met Phe Thr Ala Leu Gln Pro Gln
                85                  90                  95

Arg Gln Cys Ser Val Cys Arg Gln Ala Asn Glu Glu Tyr Gln Ile Leu
            100                 105                 110

Ala Asn Ser Trp Arg Tyr Ser Ser Ala Phe Cys Asn Lys Leu Phe Phe
        115                 120                 125

Ser Met Val Asp Tyr Asp Glu Gly Thr Asp Val Phe Gln Gln Leu Asn
    130                 135                 140

Met Asn Ser Ala Pro Thr Phe Xaa His Xaa Pro Pro Lys Gly Arg Pro
145                 150                 155                 160

Lys Arg Ala Asp Thr Phe Asp Leu Gln Arg Ile Gly Phe Ala Ala Glu
                165                 170                 175

Gln Leu Ala Lys Trp Ile Ala Asp Arg Thr Asp Val His Ile Arg Val
            180                 185                 190

Phe Arg Pro Pro Asn Tyr Ser Gly Thr Ile Ala Leu Ala Leu Leu Val
        195                 200                 205

Ser Leu Val Gly Gly Leu Leu Tyr Xaa Arg Arg Asn Asn Leu Glu Phe
    210                 215                 220

Ile Tyr Asn Lys Thr Gly Trp Ala Met Val Ser Leu Cys Ile Val Phe
225                 230                 235                 240

Ala Met Thr Ser Gly Gln Met Trp Asn His Ile Arg Gly Pro Pro Tyr
                245                 250                 255

Ala His Lys Asn Pro His Asn Gly Gln Val Ser Tyr Ile His Gly Ser
            260                 265                 270

Ser Gln Ala Gln Phe Val Ala Glu Ser His Ile Ile Leu Val Leu Asn
        275                 280                 285

Ala Ala Ile Thr Met Gly Met Val Leu Leu Asn Glu Ala Ala Thr Ser
    290                 295                 300

Lys Gly Asp Val Gly Lys Arg Arg Ile Ile Cys Leu Val Gly Leu Gly
305                 310                 315                 320

Leu Val Val Phe Phe Phe Ser Phe Leu Leu Ser Ile Phe Arg Ser Lys
                325                 330                 335

Tyr His Gly Tyr Pro Tyr Ser Asp Leu Asp Phe Glu
            340                 345

<210>  63
<211>  1130
<212>  DNA
<213>  Homo sapiens

<400>  63
ggtcgccgca ccggccgcct ccggcccgcc gccgccccca gcgccgccgc cgccaccgcc      60

ggggcgccca ccgcgctgcc agcctacccc gcggccgagc cgcccgggcc gctgtggctg     120

cagggcgagc cgctgcattt ctgctgccta gacttcagcc tggaggagct gcagggcgag     180

ccgggctggc ggctgaaccg taagcccatt gagtccacgc tggtggcctg cttcatgacc     240

ctggtcatcg tggtgtggag cgtggccgcc ctcatctggc cggtgcccat catcgccggc     300

ttcctgccca acggcatgga acagcgccgg accaccgcca gcaccaccgc agccaccccc     360

gccgcagtgc ccgcagggac caccgcagcc gccgccgccg ccgccgctgc cgccgccgcc     420

gcggccgtca cttcgggggt ggcgaccaag tgacccgctc cgctcctccc tgtgtccgtc     480

ctgtgtccgc gcgcgcgggt gcctttcccg ccggggactc ggccggtgtg cttcgtgctg     540

tagttatcgt tagttcctct tcccgagatg gggccgccga gagaccccag cgcctttgaa     600

aagcaaggtt tgtgctgcgc ttccagttcc gaaaagcaga tgtttaagcc cttggactga     660

gggtgggatc gcagctccga agacggagag gagggaaatg ggccctttcc ccctctattg     720

catccccctg cccgactcct tccccgcacc cacgtgccct agattcatgg cagaaaatga     780

ccaaatcctg tgtatttgtt ttatatattt aataactgtt ttaaatgaaa gttttagtaa     840

aaaaaataca aaacaaaaag attaaattgc tattgctgta gtaagagaag ctctttgtat     900

ctgaacatag ttgtatttga aatttgtggt tttttaattt atttaaaatt ggggggaggg     960

catgggaagg atttaacacc gatatattgt taccgctgaa aatgaacttt atgaaccttt    1020

tccaagttga tctatccagt gacgtggcct ggtgggcgtt tcttcttgta cttatgtggt    1080

tttttggctt ttaatacaga cattttcctc caaaaaaaaa aaaaaaaagg               1130

<210>  64
<211>  150
<212>  PRT
<213>  Homo sapiens

<400>  64

Gly Arg Arg Thr Gly Arg Leu Arg Pro Ala Ala Ala Pro Ser Ala Ala
1               5                   10                  15

Ala Ala Thr Ala Gly Ala Pro Thr Ala Leu Pro Ala Tyr Pro Ala Ala
            20                  25                  30

Glu Pro Pro Gly Pro Leu Trp Leu Gln Gly Glu Pro Leu His Phe Cys
        35                  40                  45

Cys Leu Asp Phe Ser Leu Glu Glu Leu Gln Gly Glu Pro Gly Trp Arg
    50                  55                  60

Leu Asn Arg Lys Pro Ile Glu Ser Thr Leu Val Ala Cys Phe Met Thr
65                  70                  75                  80

Leu Val Ile Val Val Trp Ser Val Ala Ala Leu Ile Trp Pro Val Pro
                85                  90                  95

Ile Ile Ala Gly Phe Leu Pro Asn Gly Met Glu Gln Arg Arg Thr Thr
            100                 105                 110

Ala Ser Thr Thr Ala Ala Thr Pro Ala Ala Val Pro Ala Gly Thr Thr
        115                 120                 125

Ala Ala Ala Ala Ala Ala Ala Ala Ala Ala Ala Ala Ala Ala Val Thr
    130                 135                 140

Ser Gly Val Ala Thr Lys
145                 150

<210>  65
<211>  1521
<212>  DNA
<213>  Homo sapiens

<220>
<221>  misc_feature
<222>  (798)..(798)
<223>  n is a, c, g, or t

<400>  65
cggccgcggc ccgggtccct cgcaaagccg ctgccatccc ggagggccca gccagcgggc      60

tcccggaggc tggccgggca ggcgtggtgc gcggtaggag ctgggcgcgc acggctaccg     120

cgcgtggagg agacactgcc ctgccgcgat gggggcccgg ggcgctcctt cacgccgtag     180

gcaagcgggg cggcggctgc ggtacctgcc caccgggagc tttcccttcc ttctcctgct     240

gctgctgctc tgcatccagc tcgggggagg acagaagaaa aaggagaatc ttttagctga     300

aaaagtagag cagctgatgg aatggagttc cagacgctca atcttccgaa tgaatggtga     360

taaattccga aaatttataa aggcaccacc tcgaaactat tccatgattg ttatgttcac     420

tgctcttcag cctcagcggc agtgttctgt gtgcaggcaa gctaatgaag aatatcaaat     480

actggcgaac tcctggcgct attcatctgc tttttgtaac aagctcttct tcagtatggt     540

ggactatgat gaggggacag acgtttttca gcagctcaac atgaactctg ctcctacatt     600

caygcatttw cctccaaaag gcagacctaa gagagctgat acttttgacc tccaaagaat     660

tggatttgca gctgagcaac tagcaaagtg gattgctgac agaacggatg ttcatattcg     720

ggttttcaga ccacccaact actctggtac cattgctttg gccctgttag tgtcgcttgt     780

tggaggtttg ctttattnga gaaggaacaa cttggagttc atctataaca agactggttg 840

ggccatggtg tctctgtgta tagtctttgc tatgacttct ggccagatgt ggaaccatat 900

ccgtggacct ccatatgctc ataagaaccc acacaatgga caagtgagct acattcatgg 960

gagcagccag gctcagtttg tggcagaatc acacattatt ctggtactga atgccgctat 1020

caccatgggg atggttcttc taaatgaagc agcaacttcg aaaggcgatg ttggaaaaag 1080

acggataatt tgcctagtgg gattgggcct ggtggtcttc ttcttcagtt ttctactttc 1140

aatatttcgt tccaagtacc acggctatcc ttatagtgat ctggactttg agtgagaaga 1200

tgtgatttgg accatggcac ttaaaaactc tataacctca gctttttaat taaatgaagc 1260

caagtgggat ttgcataaag tgaatgttta ccatgaagat aaactgttcc tgactttata 1320

ctattttgaa ttcattcatt tcattgtgat cagctagctt attcttgtgt acttttttta 1380

aactgtgggt tttcctagta aatttaattt acagaaatca atggtagcat ttagtaatct 1440

acaaaggaaa tatcaaagtg tttttcaagc ctgttataty cagtgtgtkc cacaggattg 1500

caataaatga caatgtaatt a 1521

<210> 66
<211> 348
<212> PRT
<213> Homo sapiens

<220>
<221> misc_feature
<222> (152)..(152)
<223> Xaa can be any naturally occurring amino acid

<220>
<221> misc_feature
<222> (154)..(154)
<223> Xaa can be any naturally occurring amino acid

<220>
<221> misc_feature
<222> (217)..(217)
<223> Xaa can be any naturally occurring amino acid

<400> 66

Met Gly Ala Arg Gly Ala Pro Ser Arg Arg Arg Gln Ala Gly Arg Arg
1               5                   10                  15

Leu Arg Tyr Leu Pro Thr Gly Ser Phe Pro Phe Leu Leu Leu Leu Leu
            20                  25                  30

Leu Leu Cys Ile Gln Leu Gly Gly Gly Gln Lys Lys Lys Glu Asn Leu
        35                  40                  45

Leu Ala Glu Lys Val Glu Gln Leu Met Glu Trp Ser Ser Arg Arg Ser
    50                  55                  60

Ile Phe Arg Met Asn Gly Asp Lys Phe Arg Lys Phe Ile Lys Ala Pro
65                  70                  75                  80

Pro Arg Asn Tyr Ser Met Ile Val Met Phe Thr Ala Leu Gln Pro Gln
                85                  90                  95

Arg Gln Cys Ser Val Cys Arg Gln Ala Asn Glu Glu Tyr Gln Ile Leu

            100                 105                 110

Ala Asn Ser Trp Arg Tyr Ser Ser Ala Phe Cys Asn Lys Leu Phe Phe
        115                 120                 125

Ser Met Val Asp Tyr Asp Glu Gly Thr Asp Val Phe Gln Gln Leu Asn
    130                 135                 140

Met Asn Ser Ala Pro Thr Phe Xaa His Xaa Pro Pro Lys Gly Arg Pro
145                 150                 155                 160

Lys Arg Ala Asp Thr Phe Asp Leu Gln Arg Ile Gly Phe Ala Ala Glu
                165                 170                 175

Gln Leu Ala Lys Trp Ile Ala Asp Arg Thr Asp Val His Ile Arg Val
            180                 185                 190

Phe Arg Pro Pro Asn Tyr Ser Gly Thr Ile Ala Leu Ala Leu Leu Val
        195                 200                 205

Ser Leu Val Gly Gly Leu Leu Tyr Xaa Arg Arg Asn Asn Leu Glu Phe
    210                 215                 220

Ile Tyr Asn Lys Thr Gly Trp Ala Met Val Ser Leu Cys Ile Val Phe
225                 230                 235                 240

Ala Met Thr Ser Gly Gln Met Trp Asn His Ile Arg Gly Pro Pro Tyr
                245                 250                 255

Ala His Lys Asn Pro His Asn Gly Gln Val Ser Tyr Ile His Gly Ser
            260                 265                 270

Ser Gln Ala Gln Phe Val Ala Glu Ser His Ile Ile Leu Val Leu Asn
        275                 280                 285

Ala Ala Ile Thr Met Gly Met Val Leu Leu Asn Glu Ala Ala Thr Ser
    290                 295                 300

Lys Gly Asp Val Gly Lys Arg Arg Ile Ile Cys Leu Val Gly Leu Gly
305                 310                 315                 320

Leu Val Val Phe Phe Phe Ser Phe Leu Leu Ser Ile Phe Arg Ser Lys
                325                 330                 335

Tyr His Gly Tyr Pro Tyr Ser Asp Leu Asp Phe Glu
            340                 345

<210> 67
<211> 2306
<212> DNA
<213> Homo sapiens

<400> 67
ggtttcatat gaactctccc gccacccggg aacagctggc tgccaccgtt tgtgttttcc 60

gagtttgtat tcttgcaggt gaccaagatg gagttttctg gaagaaagcg gaggaagctg 120

aggttggcag gtgaccagag gaatgcttcc taccctcatt gccttcagtt ttacttgcag 180

ccaccttctg aaaacatatc tttaacagaa tttgaaaact tggctattga tagagttaaa 240

ttgttaaaat cagttgaaaa tcttggagtg agctatgtga aaggaactga acaataccag 300

agtaagttgg agagtgagct tcggaagctc aagttttcct acagagagaa gctagaagat 360

gaatatgaac cacgaagaag agatcatatt tctcatttta ttttgcggct tgcttattgc 420

cagtctgaag aacttagacg ctggttcatt caacaagaaa tggatctcct tcgatttaga 480

tttagtattt tacccaagga taaaattcag gatttcttaa aggatagcca attgcagttt 540
gaggctataa gtgatgaaga gaagactctt cgagaacagg agattgttgc ctcatcacca 600
agtttaagtg gacttaagtt ggggttcgag tccatttata agatcccttt tgctgatgct 660
ctggatttgt ttcgaggaag gaaagtctat ttggaagatg gctttgctta cgtaccactt 720
aaggacattg tggcaatcat cctgaatgaa tttagagcca aactgtccaa ggctttggca 780
ttaacagcca ggtccttgcc tgctgtgcag tctgatgaaa gacttcagcc tctgctcaat 840
cacctcagtc attcctacac tggccaagat tacagtaccc agggaaatgt tgggaagatt 900
tctttagatc agattgattt gctttctacc aaatccttcc caccttgcat gcgtcagtta 960
cataaagcct tgcgggaaaa tcaccatctt cgtcatggag gccgaatgca gtatggccta 1020
tttctgaagg gcattggttt aactttggaa caggcattgc agttctggaa gcaagaattt 1080
atcaaaggaa agatggatcc agacaagttt gataaaggtt actcttacaa catccgtcac 1140
agctttggaa aggaaggcaa gaggacagac tatacacctt tcagttgcct gaagattatt 1200
ctgtccaatc caccaagcca aggggattat catgggtgcc cattccgtca cagtgatcca 1260
gagctgctga agcaaaagtt gcagtcatac aagatctctc ctggagggat aagccagatt 1320
ttggatttag taaaggggac acattaccag gtagcctgtc aaaaatactt tgagatgata 1380
cacaatgtgg atgattgtgg cttttctttg aatcatccta atcagttctt ttgtgagagc 1440
caacgtattc taaatggtgg taaagacata aagaaggaac ctatccaacc agaaactcct 1500
caacccaaac caagtgtcca gaaaaccaag gatgcatcat ctgctctggc ctctttaaat 1560
tcctctctgg aaatggatat ggaaggacta gaagattact ttagtgaaga ttcttaggca 1620
gttttataac cctttttcct caatagcctg tttcctgttt ttaagatttt gcctttgttg 1680
ttgaaaaagg gtttcactgt caccaaggct tagtgcagtg acacaattac agctgattgc 1740
agccttgacc ttcccagctc aagtgatcct cctacctcag cctcccaagt agttaggaca 1800
cacaggtgtg cacctcatat ccagataatt tttttcaatt tttttttgta gaggtggggg 1860
gtctccctat gttgcccagg cagatctcag actcctgggc tcaagcgatc ctcacacctc 1920
agcgtcccag agtgctggga ttacagttgt gagccactgt gcctggcctt tttttttttt 1980
taaccttttc gtttaacttc tctcttcact gcatcccaat ccatctacag gcatgcacac 2040
ttattaggaa aggaggtttg aggtaacaac agagactttc actatatttt gctttgacag 2100
aaggaaagag gaggagtttc tattaaaatc tgtcacttga gtgatgtcat ttaagtccta 2160
ttttaggaga taaaaacagc tttggggact ggttaaagtc ccccagaaac tacaataaag 2220
aacaactttt gttttaactc ttaatcactt tgtaattttg actcaatcct tttctggacc 2280
atttttgtta ataaatatca aagtgt 2306

<210> 68
<211> 509
<212> PRT
<213> Homo sapiens

<400> 68

<210> 69
<211> 1901
<212> DNA
<213> Homo sapiens

<400> 69
aattcataca ggagagaagt catatatatg cagtgattgt ggaaaaggct tcatcaagaa 60

gtctcggctc attaatcatc agagagttca tacaggagag aaaccacatg gatgcagcct 120

gtgtgggaag gccttctcca aaaggtccag gctcactgaa caccagagaa ctcatacagg 180

agagaagccc tatgaatgca ctgaatgtga caaagcattc cgctggaaat cacagctcaa 240

tgcacatcag aaagctcaca caggagagaa gtcatatata tgccgtgatt gtggaaaagg 300

cttcattcag aagggaaatc tcattgtaca tcagcgaatt catactggag aaaaacccta 360

tatatgcaat gaatgtggaa aaggcttcat ccaaaagggc aacctcctta ttcatcgacg 420

tactcacact ggagagaaac cctatgaatg caatgaatgt gggaaaggct tcagccagaa 480

gacatgttta atatcccatc agagatttca cacaggaaag acaccctttg tatgtactga 540

gtgtggaaaa tcctgctcac acaagtcagg tctcattaac caccagagaa ttcacacagg 600

agagaaaccc tatacatgca gtgactgtgg gaaagctttc agagataaat catgtctcaa 660

cagacatcgg agaactcata caggggagag accgtatgga tgctctgatt gtgggaaagc 720

tttctcccac ttgtcatgcc ttgtttatca taagggaatg ctgcatgcaa gagagaaatg 780

tgtaggttca gtcaaattgg aaaatccttg ctcagagagt catagcttat cacatacacg 840

tgatctcata caggataaag actctgttaa catggtgact ctgcagatgc cttctgtggc 900

agctcagacc tcattaacta acagtgcgtt ccaagcagag agcaaagtag ccattgtgag 960

ccagcctgtt gccagaagtt cagtctcagc agatagtaga atttgcacag aataaaaacc 1020

atatgaatgc agtgaatgtg gtagtgcttt cagtgatcaa ttacatcata tgtcacaaaa 1080

aacacagagg aacaaactga tatattcaag gtggaaagcc cttgaataaa accttatggc 1140

taataagcat atactcagag aaaaatagta tgaagtggag actgggaaat tcttttatgg 1200

gaagatagat cttctcatca gtgaccatag atcacatctt cagtgagctt atagttggta 1260

gaaatataat gatcatggaa aagtccttgt tcagaaacag tacgccagta ggtatcaggg 1320

ggtttacaca ggagagaaac ttttggaaga cctttgaagg ctatgaatgt ggcagggttg 1380

ctagtggtac attctgcctt atcctcagag ggaatcatat agaaataaaa ctatgaaaat 1440

gtaactagaa catcttcatc aaaatatgaa agaacacacg aagcaaataa gccctgtgaa 1500

aaggagtatt ttagagattt cgatcagaaa tctaacatca ttatatggca gataatatac 1560

aggatgtgta ttttaggaca atataccttg aatcactagt tgatatgtca atgactaatt 1620

aaaaggggtt gtcagtgtta cacatcattg gttaaattta tagcacaatg tacctcttcc 1680

cccttttttg ataagagtct tctattccca accaagatca ttatatgatt agctcttgtg 1740

tttctttgat tccaaatttc ttcacttgtt atttcagact actgaagctc ttcaaaagga 1800

aaaatgtatt taatttaata atgtaacaca acaagtttgg atgtgtttaa ctttataaat 1860

aatcacccca gaggaatgaa gttcaaaact tgtgaataac c 1901

<210> 70
<211> 127
<212> PRT
<213> Homo sapiens

<400> 70

Met Asp Ala Ala Cys Val Gly Arg Pro Ser Pro Lys Gly Pro Gly Ser
1               5                   10                  15

Leu Asn Thr Arg Glu Leu Ile Gln Glu Arg Ser Pro Met Asn Ala Leu
            20                  25                  30

Asn Val Thr Lys His Ser Ala Gly Asn His Ser Ser Met His Ile Arg
        35                  40                  45

Lys Leu Thr Gln Glu Arg Ser His Ile Tyr Ala Val Ile Val Glu Lys
    50                  55                  60

Ala Ser Phe Arg Arg Glu Ile Ser Leu Tyr Ile Ser Glu Phe Ile Leu
65                  70                  75                  80

Glu Lys Asn Pro Ile Tyr Ala Met Asn Val Glu Lys Ala Ser Ser Lys
                85                  90                  95

Arg Ala Thr Ser Leu Phe Ile Asp Val Leu Thr Leu Glu Arg Asn Pro
            100                 105                 110

Met Asn Ala Met Asn Val Gly Lys Ala Ser Ala Arg Arg His Val
        115                 120                 125

<210> 71
<211> 1005
<212> DNA
<213> Homo sapiens

<400> 71
aatttcggca cgggggggag gcacagtgag tccactgggg cacggcagcg tctaagccac 60

aagccgactg acataagcca ggtcctaacg gagcctatgt gtaagtccac tactggtgca 120

aggttgcaca cttctaagaa gagcggcgtg gggggctcgg cgaccttcgc ttcagtcgct 180

cccccgtgca gtcccctgtg cccaagacac agcctgatgc ttgtgctccg gtgggcggac 240

ttggaggcgg cgggaactgc aattggtggc tttgaagggc ggcgagcggg aacagctctt 300

gaggagtgag actgcaggag atgtgggccg tgccaaagag atggatgaga ctgttgctga 360

gttcatcaag aggaccatct tgaaaatccc catgaatgaa ctgacaacaa tcctgaaggc 420

ctgggatttt ttgtctgaaa atcaactgca gactgtaaat ttccgacaga gaaaggaatc 480

tgtagttcag cacttgatcc atctgtgtga ggaaaagcgt gcaagtatca gtgatgctgc 540

cctgttagac atcatttata tgcaatttca tcagcaccag aaagtttggg atgtttttca 600

gatgagtaaa ggaccaggtg aagatgttga cctttttgat atgaaacaat ttaaaaattc 660

gttcaagaaa attcttcaga gagcattaaa aaatgtgaca gtcagcttca gagaaactga 720

ggagaatgca gtctggattc gaattgcctg gggaacacag tacacaaagc caaaccagta 780

caaacctacc tacgtggtgt actactccca gactccgtac gccttcacgt cctcctccat 840

gctgaggcgc aatacaccgc ttctgggtca ggagttagaa gctactggga aaatctacct 900

ccgacaagag gagatcattt tagatattac cgaaatgaag aaagcttgca attagtgaac 960

atgaaaggaa aataaaaatt cctcacagtc aaaaaaaaaa aaaaa 1005

<210> 72
<211> 204
<212> PRT
<213> Homo sapiens

<400> 72

Met Asp Glu Thr Val Ala Glu Phe Ile Lys Arg Thr Ile Leu Lys Ile
1               5                   10                  15

Pro Met Asn Glu Leu Thr Thr Ile Leu Lys Ala Trp Asp Phe Leu Ser
            20                  25                  30

Glu Asn Gln Leu Gln Thr Val Asn Phe Arg Gln Arg Lys Glu Ser Val
        35                  40                  45

Val Gln His Leu Ile His Leu Cys Glu Glu Lys Arg Ala Ser Ile Ser
    50                  55                  60

Asp Ala Ala Leu Leu Asp Ile Ile Tyr Met Gln Phe His Gln His Gln
65                  70                  75                  80

Lys Val Trp Asp Val Phe Gln Met Ser Lys Gly Pro Gly Glu Asp Val
                85                  90                  95

Asp Leu Phe Asp Met Lys Gln Phe Lys Asn Ser Phe Lys Lys Ile Leu
            100                 105                 110

Gln Arg Ala Leu Lys Asn Val Thr Val Ser Phe Arg Glu Thr Glu Glu
        115                 120                 125

Asn Ala Val Trp Ile Arg Ile Ala Trp Gly Thr Gln Tyr Thr Lys Pro

130                 135                 140

Asn Gln Tyr Lys Pro Thr Tyr Val Val Tyr Tyr Ser Gln Thr Pro Tyr
145                 150                 155                 160

Ala Phe Thr Ser Ser Ser Met Leu Arg Arg Asn Thr Pro Leu Leu Gly
                165                 170                 175

Gln Glu Leu Glu Ala Thr Gly Lys Ile Tyr Leu Arg Gln Glu Glu Ile
            180                 185                 190

Ile Leu Asp Ile Thr Glu Met Lys Lys Ala Cys Asn
        195                 200

<210>  73
<211>  1125
<212>  DNA
<213>  Homo sapiens

<400>  73
atggtgctgt gggagtcccc gcggcagtgc agcagctgga cactttgcga gggcttttgc      60

tggctgctgc tgctgcccgt catgctactc atcgtagccc gcccggtgaa gctcgctgct     120

ttccctacct ccttaagtga ctgccaaacg cccaccggct ggaattgctc tggttatgat     180

gacagagaaa atgatctctt cctctgtgac accaacacct gtaaatttga tggggaatgt     240

ttaagaattg gagacactgt gacttgcgtc tgtcagttca agtgcaacaa tgactatgtg     300

cctgtgtgtg gctccaatgg ggagagctac cagaatgagt gttacctgcg acaggctgca     360

tgcaaacagc agagtgagat acttgtggtg tcagaaggat catgtgccac agatgcagga     420

tcaggatctg gagatggagt ccatgaaggc tctggagaaa ctagtcaaaa ggagacatcc     480

acctgtgata tttgccagtt tggtgcagaa tgtgacgaag atgccgagga tgtctggtgt     540

gtgtgtaata ttgactgttc tcaaaccaac ttcaatcccc tctgcgcttc tgatgggaaa     600

tcttatgata atgcatgcca aatcaaagaa gcatcgtgtc agaaacagga gaaaattgaa     660

gtcatgtctt tgggtcgatg tcaagataac acaactacaa ctactaagtc tgaagatggg     720

cattatgcaa gaacagatta tgcagagaat gctaacaaat tagaagaaag tgccagagaa     780

caccacatac cttgtccgga acattacaat ggcttctgca tgcatgggaa gtgtgagcat     840

tctatcaata tgcaggagcc atcttgcagg tgtgatgctg gttatactgg acaacactgt     900

gaaaaaaagg actacagtgt tctatacgtt gttcccggtc ctgtacgatt tcagtatgtc     960

ttaatcgcag ctgtgattgg aacaattcag attgctgtca tctgtgtggt ggtcctctgc    1020

atcacaagga aatgccccag aagcaacaga attcacagac agaagcaaaa tacagggcac    1080

tacagttcag acaatacaac aagagcgtcc acgaggttaa tctga                    1125

<210>  74
<211>  374
<212>  PRT
<213>  Homo sapiens

<400>  74

Met Val Leu Trp Glu Ser Pro Arg Gln Cys Ser Ser Trp Thr Leu Cys
1               5                   10                  15

Glu Gly Phe Cys Trp Leu Leu Leu Leu Pro Val Met Leu Leu Ile Val
            20                  25                  30

Ala Arg Pro Val Lys Leu Ala Ala Phe Pro Thr Ser Leu Ser Asp Cys
        35                  40                  45

Gln Thr Pro Thr Gly Trp Asn Cys Ser Gly Tyr Asp Asp Arg Glu Asn
    50                  55                  60

Asp Leu Phe Leu Cys Asp Thr Asn Thr Cys Lys Phe Asp Gly Glu Cys
65                  70                  75                  80

Leu Arg Ile Gly Asp Thr Val Thr Cys Val Cys Gln Phe Lys Cys Asn
                85                  90                  95

Asn Asp Tyr Val Pro Val Cys Gly Ser Asn Gly Glu Ser Tyr Gln Asn
            100                 105                 110

Glu Cys Tyr Leu Arg Gln Ala Ala Cys Lys Gln Gln Ser Glu Ile Leu
        115                 120                 125

Val Val Ser Glu Gly Ser Cys Ala Thr Asp Ala Gly Ser Gly Ser Gly
    130                 135                 140

Asp Gly Val His Glu Gly Ser Gly Glu Thr Ser Gln Lys Glu Thr Ser
145                 150                 155                 160

Thr Cys Asp Ile Cys Gln Phe Gly Ala Glu Cys Asp Glu Asp Ala Glu
                165                 170                 175

Asp Val Trp Cys Val Cys Asn Ile Asp Cys Ser Gln Thr Asn Phe Asn
            180                 185                 190

Pro Leu Cys Ala Ser Asp Gly Lys Ser Tyr Asp Asn Ala Cys Gln Ile
        195                 200                 205

Lys Glu Ala Ser Cys Gln Lys Gln Glu Lys Ile Glu Val Met Ser Leu
    210                 215                 220

Gly Arg Cys Gln Asp Asn Thr Thr Thr Thr Thr Lys Ser Glu Asp Gly
225                 230                 235                 240

His Tyr Ala Arg Thr Asp Tyr Ala Glu Asn Ala Asn Lys Leu Glu Glu
                245                 250                 255

Ser Ala Arg Glu His His Ile Pro Cys Pro Glu His Tyr Asn Gly Phe
            260                 265                 270

Cys Met His Gly Lys Cys Glu His Ser Ile Asn Met Gln Glu Pro Ser
        275                 280                 285

Cys Arg Cys Asp Ala Gly Tyr Thr Gly Gln His Cys Glu Lys Lys Asp
    290                 295                 300

Tyr Ser Val Leu Tyr Val Val Pro Gly Pro Val Arg Phe Gln Tyr Val
305                 310                 315                 320

Leu Ile Ala Ala Val Ile Gly Thr Ile Gln Ile Ala Val Ile Cys Val
                325                 330                 335

Val Val Leu Cys Ile Thr Arg Lys Cys Pro Arg Ser Asn Arg Ile His
            340                 345                 350

Arg Gln Lys Gln Asn Thr Gly His Tyr Ser Ser Asp Asn Thr Thr Arg
        355                 360                 365

Ala Ser Thr Arg Leu Ile
    370

<210> 75
<211> 2068
<212> DNA
<213> Homo sapiens

<220>
<221> misc_feature
<222> (143)..(143)
<223> n is a, c, g, or t

<400> 75
ggcgccggga ttgggagggc ttcttgcagg ctgctgggct ggggctaagg gctgctcagt 60

ttccttcagc ggggcactgg gaagcgccat ggcactgcag ggcatctcgg tcgtggagct 120

gtccggcctg gccccgggcc gtntctgtgc tatggtcctg gctgacttcg gggcgcgtgt 180

ggtacgcgtg gaccggcccg gctcccgcta cgacgtgagc cgcttgggcc ggggcaagcg 240

ctcgctagtg ctggacctga agcagccgcg ggagccgcgt gctgcggcgt ctgtgcaagc 300

ggtcggatgt gctgctggag cccttccgcc gcggtgtcat ggagaaactc cagctgggcc 360

cagagattct gcagcgggaa aatccaaggc ttatttatgc caggctgagt ggatttggcc 420

agttcaggaa agcttctgcc ggttagctgg ccacgatatc aactatttgg ctttgtcagg 480

tgttctctca aaaattggca gaagtggtga gaatccgtat gccccgctga atctcgtggc 540

tgactttgct ggtggtggcc ttatgtgtgc actgggcatt ataatggctc tttttgaccg 600

cacacgcact gacaagggtc aggtcattga tgcaaatatg gtggaaggaa cagcatattt 660

aagttctttt ctgtggaaaa ctcagaaatc gagtctgtgg gaagcacctc gaggacagaa 720

catgttggat ggtggagcac ctttctatac gacttacagg acagcagatg gggaattcat 780

ggctgttgga gcaatagaac cccagttcta cgagctgctg atcaaaggac ttggactaaa 840

gtctgatgaa cttcccaatc agatgagcac ggatgattgg ccagaaatga agaagaagtt 900

tgcagatgta tttgcaaaga agacgaaggc agagtggtgt caaatctttg acggcacaga 960

tgcctgtgtg actccggttc tgacttttga ggaggttgtt catcatgatc acaacaagga 1020

acggggctcg tttatcacca gtgaggagca ggacgtgagc ccccgccttg cacctctgct 1080

gttaaacacc ccagccatcc cttcttccaa aggggatcct ttcataggag aacacactga 1140

ggagatactt gaagaatttg gattcagccg agaagagatt tatcagctta actcagataa 1200

aatcattgaa agtaataagg taaaagctag tctctaactt ccaggcccac ggctcaagtg 1260

aatttgaata ctgcatttac agtgtagagt aacacataac attgtatgca tggaaacatg 1320

gaggaacagt attacagtgt cctaccactc taatcaagaa aagaattaca gactctgatt 1380

ctacagtgat gattgaattc taaaaatggt tatcattagg gcttttgatt tataaaactt 1440

tgggtactta tactaaatta tggtagttat tctgccttcc agtttgcttg atatatttgt 1500

tgatattaag attcttgact tatattttga atgggttcta gtgaaaaagg aatgatatat 1560

tcttgaagac atcgatatac atttatttac actcttgatt ctacaatgta gaaaatgagg 1620

aaatgccaca aattgtatgg tgataaaagt cacgtgaaac agagtgattg gttgcatcca 1680

ggccttttgt cttggtgttc atgatctccc tctaagcaca ttccaaactt tagcaacagt 1740
tatcacactt tgtaatttgc aaagaaaagt ttcacctgta ttgaatcaga atgccttcaa 1800
ctgaaaaaaa catatccaaa ataatgagga aatgtgttgg ctcactacgt agagtccaga 1860
gggacagtca gttttagggt tgcctgtatc cagtaactcg gggcctgttt ccccgtgggt 1920
ctctgggctg tcagctttcc tttctccatg tgtttgattt ctcctcaggc tggtagcaag 1980
ttctggatct tatacccaac acacagcaac atccagaaat aaagatctca ggacccccca 2040
aaaaaaaaaa aaaaaaaaaa aaaaaaaa 2068

<210> 76
<211> 382
<212> PRT
<213> Homo sapiens

<220>
<221> misc_feature
<222> (19)..(19)
<223> Xaa can be any naturally occurring amino acid

<400> 76

Met Ala Leu Gln Gly Ile Ser Val Val Glu Leu Ser Gly Leu Ala Pro
1               5                   10                  15

Gly Arg Xaa Cys Ala Met Val Leu Ala Asp Phe Gly Ala Arg Val Val
            20                  25                  30

Arg Val Asp Arg Pro Gly Ser Arg Tyr Asp Val Ser Arg Leu Gly Arg
        35                  40                  45

Gly Lys Arg Ser Leu Val Leu Asp Leu Lys Gln Pro Arg Glu Pro Arg
    50                  55                  60

Ala Ala Ala Ser Val Gln Ala Val Gly Cys Ala Ala Gly Ala Leu Pro
65                  70                  75                  80

Pro Arg Cys His Gly Glu Thr Pro Ala Gly Pro Arg Asp Ser Ala Ala
                85                  90                  95

Gly Lys Ser Lys Ala Tyr Leu Cys Gln Ala Glu Trp Ile Trp Pro Val
            100                 105                 110

Gln Glu Ser Phe Cys Arg Leu Ala Gly His Asp Ile Asn Tyr Leu Ala
        115                 120                 125

Leu Ser Gly Val Leu Ser Lys Ile Gly Arg Ser Gly Glu Asn Pro Tyr
    130                 135                 140

Ala Pro Leu Asn Leu Val Ala Asp Phe Ala Gly Gly Gly Leu Met Cys
145                 150                 155                 160

Ala Leu Gly Ile Ile Met Ala Leu Phe Asp Arg Thr Arg Thr Asp Lys
                165                 170                 175

Gly Gln Val Ile Asp Ala Asn Met Val Glu Gly Thr Ala Tyr Leu Ser
            180                 185                 190

Ser Phe Leu Trp Lys Thr Gln Lys Ser Ser Leu Trp Glu Ala Pro Arg
        195                 200                 205

Gly Gln Asn Met Leu Asp Gly Gly Ala Pro Phe Tyr Thr Thr Tyr Arg
    210                 215                 220

Thr Ala Asp Gly Glu Phe Met Ala Val Gly Ala Ile Glu Pro Gln Phe
225                 230                 235                 240

Tyr Glu Leu Leu Ile Lys Gly Leu Gly Leu Lys Ser Asp Glu Leu Pro
                245                 250                 255

Asn Gln Met Ser Thr Asp Asp Trp Pro Glu Met Lys Lys Lys Phe Ala
            260                 265                 270

Asp Val Phe Ala Lys Lys Thr Lys Ala Glu Trp Cys Gln Ile Phe Asp
        275                 280                 285

Gly Thr Asp Ala Cys Val Thr Pro Val Leu Thr Phe Glu Glu Val Val
    290                 295                 300

His His Asp His Asn Lys Glu Arg Gly Ser Phe Ile Thr Ser Glu Glu
305                 310                 315                 320

Gln Asp Val Ser Pro Arg Leu Ala Pro Leu Leu Leu Asn Thr Pro Ala
                325                 330                 335

Ile Pro Ser Ser Lys Gly Asp Pro Phe Ile Gly Glu His Thr Glu Glu
            340                 345                 350

Ile Leu Glu Glu Phe Gly Phe Ser Arg Glu Glu Ile Tyr Gln Leu Asn
        355                 360                 365

Ser Asp Lys Ile Ile Glu Ser Asn Lys Val Lys Ala Ser Leu
    370                 375                 380

<210>  77
<211>  5685
<212>  DNA
<213>  Homo sapiens

<400>  77
gttaaatcct tactttacca gattcttgat ggtatccatt acctccatgc aaattgggtg      60

cttcacagag acttgaaacc agcaaatatc ctagtaatgg gagaaggtcc tgagaggggg     120

agagtcaaaa tagctgacat gggttttgcc agattattca attctcctct aaagccacta     180

gcagatttgg atccagtagt tgtgacattt tggtatcggg ctccagaact tttgcttggt     240

gcaaggcatt atacaaaggc cattgatata tgggcaatag gttgtatatt tgctgaattg     300

ttgacttcgg aacctatttt tcactgtcgt caggaagata taaaaacaag caatcccttt     360

catcatgatc aactggatcg gatatttagt gtcatggggt ttcctgcaga taaagactgg     420

gaagatatta gaaagatgcc agaatatccc acacttcaaa aagactttag aagaacaacg     480

tatgccaaca gtagcctcat aaagtacatg gagaaacaca aggtcaagcc tgacagcaaa     540

gtgttcctct tgcttcagaa actcctgacc atggatccaa ccaagagaat tacctcggag     600

caagctctgc aggatcccta ttttcaggag gaccctttgc caacattaga tgtatttgcc     660

ggctgccaga ttccataccc caaacgagaa ttccttaatg aagatgatcc tgaagaaaaa     720

ggtgacaaga atcagcaaca gcagcagaac cagcatcagc agcccacagc ccctccacag     780

caggcagcag cccctccaca ggcgccccca ccacagcaga acagcaccca gaccaacggg     840

accgcaggtg gggctggggc cggggtcggg ggcaccggag cagggttgca gcacagccag     900

gactccagcc tgaaccaggt gcctccaaac aagaagccac ggctagggcc ttcaggcgca     960

aactcaggtg gacctgtgat gccctcggat tatcagcact ccagttctcg cctgaattac 1020
caaagcagcg ttcagggatc ctctcagtcc cagagcacac ttggctactc ttcctcgtct 1080
cagcagagct cacagtacca cccatctcac caggcccacc ggtactgacc agctcccgtt 1140
gggccaggcc agcccagccc agagcacagg ctccagcaat atgtctgcat tgaaaagaac 1200
caaaaaaatg caaactatga tgccatttaa aactcataca catgggagga aaaccttata 1260
tactgagcat tgtgcaggac tgatagctct tctttattga cttaaagaag attcttgtga 1320
agtttcccca gcaccccttc cctgcatgtg ttccattgtg acttctctga taaagcgtct 1380
gatctaatcc cagcacttct gtaaccttca gcatttcttt gaaggatttc ctggtgcacc 1440
tttctcatgc tgtagcaatc actatggttt atcttttcaa agctctttta ataggatttt 1500
aatgttttag aaacaggatt ccagtggtgt atagttttat acttcatgaa ctgatttagc 1560
aacacaggta aaaatgcacc ttttaaagca ctacgttttc acagacaata actgttctgc 1620
tcatggaagt cttaaacaga aactgttact gtcccaaagt actttactat tacgttcgta 1680
tttatctagt ttcagggaag gtctaataaa aagacaagcg gtgggacaga gggaacctac 1740
aaccaaaaac tgcctagatc tttgcagtta tgtgctttat gccacgaaga actgaagtat 1800
gtggtaattt ttatagaatc attcatatgg aactgagttc ccagcatcat cttattctga 1860
atagcattca gtaattaaga attacaattt taaccttcat gtagctaagt ctaccttaaa 1920
aagggtttca agagctttgt acagtctcga tggcccacac caaaacgctg aagagagtaa 1980
caactgcact aggatttctg taaggagtaa ttttgatcaa aagacgtgtt acttcccttt 2040
gaaggaaaag tttttagtgt gtattgtaca taaagtcggc ttctctaaag aaccattggt 2100
ttcttcacat ctgggtctgc gtgagtaact ttcttgcata atcaaggtta ctcaagtaga 2160
agcctgaaaa ttaatctgct tttaaaataa agagcagtgt tctccattcg tatttgtatt 2220
agatatagag tgactatttt taaagcatgt taaaaattta ggttttattc atgtttaaag 2280
tatgtattat gtatgcataa ttttgctgtt gttactgaaa cttaattcta tcaagaatct 2340
ttttcattgc actgaatgat ttcttttgcc cctaggagaa aacttaataa ttgtgcctaa 2400
aaactatggg cggatagtat aagactatac tagacaaagt gaatatttgc atttccatta 2460
tctatgaatt agtggctgag ttctttctta gctgctttaa ggagcccctc actccccaga 2520
gtcaaaagga aatgtaaaaa cttagagctc ccattgtaat gtaaggggca agaaatttgt 2580
gttcttctga atgctactag cagcaccagc cttgttttaa atgttttctt gagctagaag 2640
aaatagctga ttattgtata tgcaaattac atgcattttt aaaaactatt ctttctgaac 2700
ttatctacct ggttatgata ctgtgggtcc atacacaagt aaaataagat tagacagaag 2760
ccagtataca ttttgcacta ttgatgtgat actgtagcca gccaggacct tactgatctc 2820
agcataataa tgctcactaa taatgaagtc tgcatagtga cactcatcaa gactgaagat 2880
gaagcaggtt acgtgctcca ttggaaggag tttctgatag tctcctgctg ttttacccct 2940
tccatttttt aaaataagaa attagcagcc ctctgcataa tgtagctgcc tatatgcagt 3000

tttatcctgt gccctaaagc ctcactgtcc agagctgttg gtcatcagat gcttattgca 3060
ccctcaccat gtgcctggtg ccctgctggg tagagaacac agaggacagg gcatacttct 3120
tgtccttaag gagcttgtga tctgtgacag taagccctcc tgggatgtct gtgccatgtg 3180
attgacttac aagtgaaact gtcttataat atgaaggtct ttttgtttac ttctaaaccc 3240
acttgggtag ttactatccc caaatctgtt ctgtaaataa tattatggaa gggtttctat 3300
gtcagtctac cttagagaaa gccagtgatt caatatcaca aaaggcattg acgtatcttt 3360
gaaatgttca cagcagcctt ttaacaacaa ctgggtggtc cttgtaggca gaacatactc 3420
tcctaagtgg ttgtaggaaa ttgcaaggaa aatagaaggt ctgttcttgc tctcaaggag 3480
gttaccttta ataaaagaag acaaacccag atagatatgt aaaccaaaat actatgcccc 3540
ttaatacttt ataagcagca ttgttaaata gttcttacgc ttatacattc acagaactac 3600
cctgttttcc ttgtatataa tgacttttgc tggcagaact gaaatataaa ctgtaagggg 3660
atttcgtcag ttgctcccag tatacaatat cctccaggac atagccagaa atctccattc 3720
cacacatgac tgagttccta tccctgcact ggtactggct cttttctcct ctttccttgc 3780
ctcagggttc gtgctaccca ctgattccct ttacccttag taataatttt ggatcatttt 3840
ctttccttta aaggggaaca aagccttttt tttttttgag acggagtgtt gctctgtcac 3900
ccaagctgga gtgcagtggc acgatcttgg ctcactccaa cctccacctt ccaggttcaa 3960
gtgattctcc tgcctcagcc tcccgagtag ctgggactac gggcacgcac caccacgtct 4020
ggctaatttt tgtattttta gtagagatgg ggtttcaccc tattggtcag gctggtcttg 4080
aattcctcac ctcaggtcat ccgcctgtct cggcctcccg aagtgctggg attataggtg 4140
tgagccaccg cacccagttg ggaacaaagc ctttttaaca cacgtaaggg ccctcaaacc 4200
gtgggacctc taaggagacc tttgaagctt tttgagggca aactttacct ttgtggtccc 4260
caaatgatgg catttctctt tgaaatttat tagatactgt tatgtccccc aagggtacag 4320
gaggggcatc cctcagccta tgggaacacc caaactagga ggggttattg acaggaagga 4380
atgaatccaa gtgaaggctt tctgctcttc gtgttacaaa ccagtttcag agttagcttt 4440
ctggggaggt gtgtgtttgt gaaaggaatt caagtgttgc aggacagatg agctcaaggt 4500
aaggtagctt tggcagcagg gctgatacta tgaggctgaa acaatccttg tgatgaagta 4560
gatcatgcag tgacatacaa agaccaagga ttatgtatat ttttatatct ctgtggtttt 4620
gaaactttag tacttagaat tttggccttc tgcactactc ttttgctctt acgaacataa 4680
tggactctta agaatggaaa gggatgacat ttacctatgt gtgctgcctc attcctggtg 4740
aagcaactgc tacttgttct ctatgcctct aaaatgatgc tgttttctct gctaaaggta 4800
aaagaaaaga aaaaaatagt tggaaaataa gacatgcaac ttgatgtgct tttgagtaaa 4860
tttatgcagc agaaactata caatgaagga agaattctat ggaaattaca aatccaaaac 4920
tctatgatga tgtcttccta gggagtagag aaaggcagtg aaatggcagt tagaccaaca 4980
gaggcttgaa ggattcaagt acaagtaata ttttgtataa aacatagcag tttaggtccc 5040

cataatcctc aaaaatagtc acaaatataa caaagttcat tgttttaggg tttttaaaaa 5100

acgtgttgta cctaaggcca tacttactct tctatgctat cactgcaaag gggtgatatg 5160

tatgtattat ataaaaaaaa aaacccttaa tgcactgtta tctcctaaat atttagtaaa 5220

ttaatactat ttaatttttt taaagatttg tctgtgtaga cactaaaagt attacacaaa 5280

atctggactg aaggtgtcct ttttaacaac aatttaaagt actttttata tatgttatgt 5340

agtatatcct ttctaaactg cctagtttgt atattcctat aattcctatt tgtgaagtgt 5400

acctgttctt gtctcttttt tcagtcattt tctgcacgca tcccccttta tatggttata 5460

gagatgactg tagcttttcg tgctccactg cgaggtttgt gctcagagcc gctgcacccc 5520

agcgaggcct gctccatgga gtgcaggacg agctactgct ttggagcgag ggtttcctgc 5580

ttttgagttg acctgacttc cttcttgaaa tgactgttaa aactaaaata aattacattg 5640

catttatttt atattcttgg ttgaaataaa atttaattga ctttg 5685

<210> 78
<211> 375
<212> PRT
<213> Homo sapiens

<400> 78

Val Lys Ser Leu Leu Tyr Gln Ile Leu Asp Gly Ile His Tyr Leu His
1               5                   10                  15

Ala Asn Trp Val Leu His Arg Asp Leu Lys Pro Ala Asn Ile Leu Val
            20                  25                  30

Met Gly Glu Gly Pro Glu Arg Gly Arg Val Lys Ile Ala Asp Met Gly
        35                  40                  45

Phe Ala Arg Leu Phe Asn Ser Pro Leu Lys Pro Leu Ala Asp Leu Asp
    50                  55                  60

Pro Val Val Val Thr Phe Trp Tyr Arg Ala Pro Glu Leu Leu Leu Gly
65                  70                  75                  80

Ala Arg His Tyr Thr Lys Ala Ile Asp Ile Trp Ala Ile Gly Cys Ile
                85                  90                  95

Phe Ala Glu Leu Leu Thr Ser Glu Pro Ile Phe His Cys Arg Gln Glu
            100                 105                 110

Asp Ile Lys Thr Ser Asn Pro Phe His His Asp Gln Leu Asp Arg Ile
        115                 120                 125

Phe Ser Val Met Gly Phe Pro Ala Asp Lys Asp Trp Glu Asp Ile Arg
    130                 135                 140

Lys Met Pro Glu Tyr Pro Thr Leu Gln Lys Asp Phe Arg Arg Thr Thr
145                 150                 155                 160

Tyr Ala Asn Ser Ser Leu Ile Lys Tyr Met Glu Lys His Lys Val Lys
                165                 170                 175

Pro Asp Ser Lys Val Phe Leu Leu Leu Gln Lys Leu Leu Thr Met Asp
            180                 185                 190

Pro Thr Lys Arg Ile Thr Ser Glu Gln Ala Leu Gln Asp Pro Tyr Phe
        195                 200                 205

Gln Glu Asp Pro Leu Pro Thr Leu Asp Val Phe Ala Gly Cys Gln Ile
    210                 215                 220

Pro Tyr Pro Lys Arg Glu Phe Leu Asn Glu Asp Asp Pro Glu Glu Lys
225                 230                 235                 240

Gly Asp Lys Asn Gln Gln Gln Gln Gln Asn Gln His Gln Gln Pro Thr
                245                 250                 255

Ala Pro Pro Gln Gln Ala Ala Ala Pro Pro Gln Ala Pro Pro Pro Gln
            260                 265                 270

Gln Asn Ser Thr Gln Thr Asn Gly Thr Ala Gly Gly Ala Gly Ala Gly
        275                 280                 285

Val Gly Gly Thr Gly Ala Gly Leu Gln His Ser Gln Asp Ser Ser Leu
    290                 295                 300

Asn Gln Val Pro Pro Asn Lys Lys Pro Arg Leu Gly Pro Ser Gly Ala
305                 310                 315                 320

Asn Ser Gly Gly Pro Val Met Pro Ser Asp Tyr Gln His Ser Ser Ser
                325                 330                 335

Arg Leu Asn Tyr Gln Ser Ser Val Gln Gly Ser Ser Gln Ser Gln Ser
            340                 345                 350

Thr Leu Gly Tyr Ser Ser Ser Ser Gln Gln Ser Ser Gln Tyr His Pro
        355                 360                 365

Ser His Gln Ala His Arg Tyr
    370                 375

<210>  79
<211>  2190
<212>  DNA
<213>  Homo sapiens

<400>  79
atgaatcctt tccagaaaaa tgagtccaag gaaactcttt tttcacctgt ctccattgaa      60

gaggtaccac ctcgaccacc tagccctcca aagaagccat ctccgacaat ctgtggctcc     120

aactatccac tgagcattgc cttcattgtg gtgaatgaat tctgcgagcg cttttcctat     180

tatggaatga aagctgtgct gatcctgtat ttcctgtatt tcctgcactg gaatgaagat     240

acctccacat ctatatacca tgccttcagc agcctctgtt attttactcc catcctggga     300

gcagccattg ctgactcgtg gttgggaaaa ttcaagacaa tcatctatct ctccttggtg     360

tatgtgcttg gccatgtgat caagtccttg ggtgccttac caatactggg aggacaagtg     420

gtacacacag tcctatcatt gatcggcctg agtctaatag ctttggggac aggaggcatc     480

aaaccctgtg tggcagcttt tggtggagac cagtttgaag aaaaacatgc agaggaacgg     540

actagatact tctcagtctt ctacctgtcc atcaatgcag ggagcttgat ttctacattt     600

atcacaccca tgctgagagg agatgtgcaa tgttttggag aagactgcta tgcattggct     660

tttggagttc caggactgct catggtaatt gcacttgttg tgtttgcaat gggaagcaaa     720

atatacaata aaccaccccc tgaaggaaac atagtggctc aagttttcaa atgtatctgg     780

tttgctattt ccaatcgttt caagaaccgt tctggagaca ttccaaagcg acagcactgg     840

ctagactggg cagctgagaa atatccaaag cagctcatta tggatgtaaa ggcactgacc     900

agggtactat tcctttatat cccattgccc atgttctggg ctcttttgga tcagcagggt 960
tcacgatgga ctttgcaagc catcaggatg aataggaatt tggggttttt tgtgcttcag 1020
ccggaccaga tgcaggttct aaatcccttt ctggttctta tcttcatccc gttgtttgac 1080
tttgtcattt atcgtctggt ctccaagtgt ggaattaact tctcatcact taggaaaatg 1140
gctgttggta tgatcctagc gtgcctggca tttgcagttg cggcagctgt agagataaaa 1200
ataaatgaaa tggccccagc ccagtcaggt ccccaggagg ttttcctaca agtcttgaat 1260
ctggcagatg atgaggtgaa ggtgacagtg gtgggaaatg aaaacaattc tctgttgata 1320
gagtccatca aatcctttca gaaaacacca cactattcca aactgcacct gaaaacaaaa 1380
agccaggatt ttcacttcca cctgaaatat cacaatttgt ctctctacac tgagcattct 1440
gtgcaggaga agaactggta cagtcttgtc attcgtgaag atgggaacag tatctccagc 1500
atgatggtaa aggatacaga aagcaaaaca accaatggga tgacaaccgt gaggtttgtt 1560
aacactttgc ataaagatgt caacatctcc ctgagtacag atacctctct caatgttggt 1620
gaagactatg gtgtgtctgc ttatagaact gtgcaaagag gagaataccc tgcagtgcac 1680
tgtagaacag aagataagaa cttttctctg aatttgggtc ttctagactt tggtgcagca 1740
tatctgtttg ttattactaa taacaccaat cagggtcttc aggcctggaa gattgaagac 1800
attccagcca acaaaatgtc cattgcgtgg cagctaccac aatatgccct ggttacagct 1860
ggggaggtca tgttctctgt cacaggtctt gagttttctt attctcaggc tccctctagc 1920
atgaaatctg tgctccaggc agcttggcta ttgacaattg cagttgggaa tatcatcgtg 1980
cttgttgtgg cacagttcag tggcctggta cagtgggccg aattcatttt gttttcctgc 2040
ctcctgctgg tgatctgcct gatcttctcc atcatgggct actactatgt tcctgtaaag 2100
acagaggata tgcggggtcc agcagataag cacattcctc acatccaggg gaacatgatc 2160
aaactagaga ccaagaagac aaaactctga 2190

<210> 80
<211> 729
<212> PRT
<213> Homo sapiens

<400> 80

<210>  81
<211>  1221
<212>  DNA
<213>  Homo sapiens

<400>  81
atggacggat cccacagcgc agccctgaag ctgcagcagc tgcctcccac aagtagctcc      60

agcgccgtaa gcgaggcctc cttctcctac aaggaaaacc tgattggcgc cctcttggcg     120

atcttcgggc acctcgtggt cagcattgca cttaacctcc agaagtactg ccacatccgc     180

ctggcaggct ccaaggatcc ccgggcctat ttcaagacca agacatggtg gctgggcctg     240

ttcctgatgc ttctgggcga gctgggtgtg ttcgcctcct acgccttcgc gccgctgtca 300

ctcatcgtgc ccctcagcgc agtttctgtg atagctagtg ccatcatagg aatcatattc 360

atcaaggaaa agtggaaacc gaaagacttt ctgaggcgct acgtcttgtc ctttgttggc 420

tgcggtttgg ctgtcgtggg tacctacctg ctggtgacat tcgcacccaa cagtcacgag 480

aagatgacag gcgagaatgt caccaggcac ctcgtgagct ggcctttcct tttgtacatg 540

ctggtggaga tcattctgtt ctgcttgctg ctctacttct acaaggagaa gaacgccaac 600

aacattgtcg tgattcttct cttggtggcg ttacttggct ccatgacagt ggtgacagtc 660

aaggccgtgg ctgggatgct tgtcttgtcc attcaaggga acctgcagct tgactacccc 720

atcttctacg tgatgttcgt gtgcatggtg gcaaccgccg tctatcaggc tgcgtttttg 780

agtcaagcct cacagatgta cgactcctct ttgattgcca gtgtgggcta cattctgtcc 840

acaaccattg ctatcacagc aggtgcaata ttttacctgg acttcatcgg ggaggacgtg 900

ctgcacatct gcatgtttgc actggggtgc ctcattgcat tcttgggcgt cttcttaatc 960

acgcgtaaca ggaagaagcc attccatttt gagccctata tttccatgga tgccatgcca 1020

ggtatgcaga acatgcacga taaagggatg actgtccagc ctgaacttaa agcttctttt 1080

tcctatgggg ctctggaaaa caatgacaac atttctgaga tctacgctcc tgccaccctg 1140

ccagtcatgc aagaagagca cggctccaga agtgcctctg ggtcccccta ccgagtccta 1200

gagcacacca agaaggaatg a 1221

<210> 82
<211> 406
<212> PRT
<213> Homo sapiens

<400> 82

Met Asp Gly Ser His Ser Ala Ala Leu Lys Leu Gln Gln Leu Pro Pro
1               5                   10                  15

Thr Ser Ser Ser Ser Ala Val Ser Glu Ala Ser Phe Ser Tyr Lys Glu
            20                  25                  30

Asn Leu Ile Gly Ala Leu Leu Ala Ile Phe Gly His Leu Val Val Ser
        35                  40                  45

Ile Ala Leu Asn Leu Gln Lys Tyr Cys His Ile Arg Leu Ala Gly Ser
    50                  55                  60

Lys Asp Pro Arg Ala Tyr Phe Lys Thr Lys Thr Trp Trp Leu Gly Leu
65                  70                  75                  80

Phe Leu Met Leu Leu Gly Glu Leu Gly Val Phe Ala Ser Tyr Ala Phe
                85                  90                  95

Ala Pro Leu Ser Leu Ile Val Pro Leu Ser Ala Val Ser Val Ile Ala
            100                 105                 110

Ser Ala Ile Ile Gly Ile Ile Phe Ile Lys Glu Lys Trp Lys Pro Lys
        115                 120                 125

Asp Phe Leu Arg Arg Tyr Val Leu Ser Phe Val Gly Cys Gly Leu Ala

130                 135                 140

Val Val Gly Thr Tyr Leu Leu Val Thr Phe Ala Pro Asn Ser His Glu
145                 150                 155                 160

Lys Met Thr Gly Glu Asn Val Thr Arg His Leu Val Ser Trp Pro Phe
                165                 170                 175

Leu Leu Tyr Met Leu Val Glu Ile Ile Leu Phe Cys Leu Leu Leu Tyr
            180                 185                 190

Phe Tyr Lys Glu Lys Asn Ala Asn Asn Ile Val Val Ile Leu Leu Leu
        195                 200                 205

Val Ala Leu Leu Gly Ser Met Thr Val Val Thr Val Lys Ala Val Ala
    210                 215                 220

Gly Met Leu Val Leu Ser Ile Gln Gly Asn Leu Gln Leu Asp Tyr Pro
225                 230                 235                 240

Ile Phe Tyr Val Met Phe Val Cys Met Val Ala Thr Ala Val Tyr Gln
                245                 250                 255

Ala Ala Phe Leu Ser Gln Ala Ser Gln Met Tyr Asp Ser Ser Leu Ile
            260                 265                 270

Ala Ser Val Gly Tyr Ile Leu Ser Thr Thr Ile Ala Ile Thr Ala Gly
        275                 280                 285

Ala Ile Phe Tyr Leu Asp Phe Ile Gly Glu Asp Val Leu His Ile Cys
    290                 295                 300

Met Phe Ala Leu Gly Cys Leu Ile Ala Phe Leu Gly Val Phe Leu Ile
305                 310                 315                 320

Thr Arg Asn Arg Lys Lys Pro Ile Pro Phe Glu Pro Tyr Ile Ser Met
                325                 330                 335

Asp Ala Met Pro Gly Met Gln Asn Met His Asp Lys Gly Met Thr Val
            340                 345                 350

Gln Pro Glu Leu Lys Ala Ser Phe Ser Tyr Gly Ala Leu Glu Asn Asn
        355                 360                 365

Asp Asn Ile Ser Glu Ile Tyr Ala Pro Ala Thr Leu Pro Val Met Gln
    370                 375                 380

Glu Glu His Gly Ser Arg Ser Ala Ser Gly Val Pro Tyr Arg Val Leu
385                 390                 395                 400

Glu His Thr Lys Lys Glu
                405

<210>  83
<211>  1316
<212>  DNA
<213>  Homo sapiens

<400>  83
cactcattaa gaacagagga ggctgcctgt tactcctggt gttgcatccc tccagacact      60

ctgctgtttc ctgcctaggc gtggctgcag ccatggctag gaaagcgctg ccacccaccc     120

acctgggcca gagctggttc tgctcctgct gcagggacac tgagctggct atctcggcgc     180

ttcgggcaag aactgcaaca ggctctcctg ggtcctgcag gtgtacagcc gggcccctgc     240

cttgtgcctc agctctcgag agctgctgct gccgggtgac ctgatccaac ctgataaggt     300

gccatcttca gctaccactg caaggccctg agggcaacag cagcacggca ctgcccaccc 360
ggctgctgat ggcctggtgc cagctgggag tcctcccggc acttcgaggc cactgagcca 420
cccttccagc cccagcccac catggacagg ggtatccagc ttcctcctca acctcgtcct 480
ctgcccctga gccagtgacg cccaaggaca tgcctgttac ccaggtcctg taccagcact 540
agctggtcaa gggcatgaca gtgctggagg ccgtcttgga gatccaggcc atcactggca 600
gcaggctgct ctccatggtg ccagggcccg ccaggccacc aggctcatgc tgggacccaa 660
cccagtgcac aaggacttgg ctgctgagcc acacacccag gagaaggtgg ataagtgggc 720
taccaagggc ttcctgcagg ctaggggagg agccaccccc gcttccctat tgtgaccagg 780
cctatgggga ggagctgtcc atacgccacc gtgagacctg ggcctggctc tcaaggacag 840
acaccgcctg gcctggtgct ccaggggtga agcaggccag aatcctgggg gagctgctcc 900
tggtttgagc tgcattcagg aagtgcggga catggtaggg gaggcaaaaa gccttgggca 960
ctaccctccc tgtggagctg ttcggtgtcc gtcgagctag ccacaccctg acaccatgtt 1020
caagggtacc ggaagagaag ggtgtctgcc cccaacctcc cctgtgggtg tcactggcca 1080
gatgtcatga gggaagcagg ccttgtgagt ggacactgac catgagtccc tggggggagt 1140
gatcccccag gcatcgtgtg ccatgttgca cttctgccca ggcagcaggg tgggtgggta 1200
ccatgggtgc ccacccctcc accacatggg gccccaaagc actgcaggcc aagcagggca 1260
accccacacc cttgacataa aagcatcttg aagcttttaa aaaaaaaaaa aaaaaa 1316

<210> 84
<211> 117
<212> PRT
<213> Homo sapiens

<400> 84

Met Thr Val Leu Glu Ala Val Leu Glu Ile Gln Ala Ile Thr Gly Ser
1               5                   10                  15

Arg Leu Leu Ser Met Val Pro Gly Pro Ala Arg Pro Pro Gly Ser Cys
            20                  25                  30

Trp Asp Pro Thr Gln Cys Thr Arg Thr Trp Leu Leu Ser His Thr Pro
        35                  40                  45

Arg Arg Arg Trp Ile Ser Gly Leu Pro Arg Ala Ser Cys Arg Leu Gly
    50                  55                  60

Glu Glu Pro Pro Pro Leu Pro Tyr Cys Asp Gln Ala Tyr Gly Glu Glu
65                  70                  75                  80

Leu Ser Ile Arg His Arg Glu Thr Trp Ala Trp Leu Ser Arg Thr Asp
                85                  90                  95

Thr Ala Trp Pro Gly Ala Pro Gly Val Lys Gln Ala Arg Ile Leu Gly
            100                 105                 110

Glu Leu Leu Leu Val
        115

<210> 85
<211> 3442

<212>  DNA
<213>  Homo sapiens

<400>  85
agccggtgcg ccgcagacta gggcgcctcg ggccagggag cgcggaggag ccatggccac      60

cgctaacggg gccgtggaaa acgggcagcc ggacgggaag ccgccggccc tgccgcgccc     120

catccgcaac ctggaggtca agttcaccaa gatatttatc aacaatgaat ggcacgaatc     180

caagagtggg aaaaagtttg ctacatgtaa cccttcaact cgggagcaaa tatgtgaagt     240

ggaagaagga gataagcccg acgtggacaa ggctgtggag gctgcacagg ttgccttcca     300

gaggggctcg ccatggcgcc ggctggatgc cctgagtcgt gggcggctgc tgcaccagct     360

ggctgacctg gtggagaggg accgcgccac cttggccgcc ctggagacga tggatacagg     420

gaagccattt cttcatgctt ttttcatcga cctggagggc tgtattagaa ccctcagata     480

ctttgcaggg tgggcagaca aaatccaggg caagaccatc cccacagatg acaacgtcgt     540

atgcttcacc aggcatgagc ccattggtgt ctgtggggcc atcactccat ggaacttccc     600

cctgctgatg ctggtgtgga agctggcacc cgccctctgc tgtgggaaca ccatggtcct     660

gaagcctgcg gagcagacac ctctcaccgc cctttatctc ggctctctga tcaaagaggc     720

cgggttccct ccaggagtgg tgaacattgt gccaggattc gggcccacag tgggagcagc     780

aatttcttct caccctcaga tcaacaagat cgccttcacc ggctccacag aggttggaaa     840

actggttaaa gaagctgcgt cccggagcaa tctgaagcgg gtgacgctgg agctgggggg     900

gaagaacccc tgcatcgtgt gtgcggacgc tgacttggac ttggcagtgg agtgtgccca     960

tcagggagtg ttcttcaacc aaggccagtg ttgcacggca gcctccaggg tgttcgtgga    1020

ggagcaggtc tactctgagt ttgtcaggcg gagcgtggag tatgccaaga aacggcccgt    1080

gggagacccc ttcgatgtca aaacagaaca ggggcctcag attgatcaaa agcagttcga    1140

caaaatctta gagctgatcg agagtgggaa gaaggaaggg gccaagctgg aatgcggggg    1200

ctcagccatg gaagacaagg ggctcttcat caaacccact gtcttctcag aagtcacaga    1260

caacatgcgg attgccaaag aggagatttt cgggccagtg caaccaatac tgaagttcaa    1320

aagtatcgaa gaagtgataa aaagagcgaa tagcaccgac tatggactca cagcagccgt    1380

gttcacaaaa aatctcgaca aagccctgaa gttggcttct gccttagagt ctggaacggt    1440

ctggatcaac tgctacaacg ccctctatgc acaggctcca tttggtggct ttaaaatgtc    1500

aggaaatggc agagaactag gtgaatacgc tttggccgaa tacacagaag tgaaaactgt    1560

caccatcaaa cttggcgaca agaacccctg aaggaaaggc ggggctcctt cctcaaacat    1620

cggacggcgg aatgtggcag atgaaatgtg ctggaggaaa aaaatgacat ttctgacctt    1680

cccgggacac attcttctgg aggctttaca tctactggag ttgaatgatt gctgttttcc    1740

tctcactctc ctgtttattc accagactgg ggatgcctat aggttgtctg tgaaatcgca    1800

gtcctgcctg gggagggagc tgttggccat ttctgtgttt ccctttaaac cagatcctgg    1860

agacagtgag atactcaggg cgttgttaac agggagtggt atttgaagtg tccagcagtt    1920

gcttgaaatg ctttgccgaa tctgactcca gtaagaatgt gggaaaaccc cctgtgtgtt 1980
ctgcaagcag ggctcttgca ccagcggtct cctcagggtg gacctgctta cagagcaagc 2040
cacgcctctt tccgaggtga aggtgggacc attccttggg aaaggattca cagtaaggtt 2100
ttttggtttt tgttttttgt tttcttgttt ttaaaaaaag gatttcacag tgagaaagtt 2160
ttggttagtg cataccgtgg aagggcgcca gggtctttgt ggattgcatg ttgacattga 2220
ccgtgagatt cggcttcaaa ccaatactgc ctttggaata tgacagaatc aatagcccag 2280
agagcttagt caaagacgat atcacggtct accttaacca aggcactttc ttaagcagaa 2340
aatattgttg aggttacctt tgctgctaaa gatccaatct tctaacgcca caacagcata 2400
gcaaatccta ggataattca cctcctcatt tgacaaatca gagctgtaat tcactttaac 2460
aaattacgca tttctatcac gttcactaac agcttatgat aagtctgtgt agtcttcctt 2520
ttctccagtt ctgttaccca atttagatta gtaaagcgta cacaactgga aagactgctg 2580
taataacaca gccttgttat ttttaagtcc tattttgata ttaatttctg attagttagt 2640
aaataacacc tggattctat ggaggacctc ggtcttcatc caagtggcct gagtatttca 2700
ctggcaggtt gtgaattttt cttttcctct ttgggaatcc aaatgatgat gtgcaatttc 2760
atgttttaac ttgggaaact gaaagtgttc ccatatagct tcaaaaacaa aaacaaatgt 2820
gttatccgac ggatactttt atggttacta actagtactt tcctaattgg gaaagtagtg 2880
cttaagtttg caaattaagt tggggagggc aataataaaa tgagggcccg taacagaacc 2940
agtgtgtgta taacgaaaac catgtataaa atgggcctat cacccttgtc agagatataa 3000
attaccacat ttggcttccc ttcatcagct aacacttatc acttatacta ccaataactt 3060
gttaaatcag gatttggctt catacactga attttcagta ttttatctca agtagatata 3120
gacactaacc ttgatagtga tacgttagag ggttcctatt cttccattgt acgataatgt 3180
ctttaatatg aaatgctaca ttatttataa ttggtagagt tattgtatct ttttatagtt 3240
gtaagtacac agaggtggta tatttaaact tctgtaatat actgtattta gaaatggaaa 3300
tatatatagt gttaggtttc acttctttta aggtttaccc ctgtggtgtg gtttaaaaat 3360
ctataggcct gggaattccg atcctagctg cagatcgcat cccacaatgc gagaatgata 3420
aaataaaatt ggatatttga ga 3442

<210> 86
<211> 512
<212> PRT
<213> Homo sapiens

<400> 86

Met Ala Thr Ala Asn Gly Ala Val Glu Asn Gly Gln Pro Asp Gly Lys
1               5                   10                  15

Pro Pro Ala Leu Pro Arg Pro Ile Arg Asn Leu Glu Val Lys Phe Thr
            20                  25                  30

Lys Ile Phe Ile Asn Asn Glu Trp His Glu Ser Lys Ser Gly Lys Lys
        35                  40                  45

Phe Ala Thr Cys Asn Pro Ser Thr Arg Glu Gln Ile Cys Glu Val Glu
    50                  55                  60

Glu Gly Asp Lys Pro Asp Val Asp Lys Ala Val Glu Ala Ala Gln Val
65                  70                  75                  80

Ala Phe Gln Arg Gly Ser Pro Trp Arg Arg Leu Asp Ala Leu Ser Arg
                85                  90                  95

Gly Arg Leu Leu His Gln Leu Ala Asp Leu Val Glu Arg Asp Arg Ala
            100                 105                 110

Thr Leu Ala Ala Leu Glu Thr Met Asp Thr Gly Lys Pro Phe Leu His
        115                 120                 125

Ala Phe Phe Ile Asp Leu Glu Gly Cys Ile Arg Thr Leu Arg Tyr Phe
    130                 135                 140

Ala Gly Trp Ala Asp Lys Ile Gln Gly Lys Thr Ile Pro Thr Asp Asp
145                 150                 155                 160

Asn Val Val Cys Phe Thr Arg His Glu Pro Ile Gly Val Cys Gly Ala
                165                 170                 175

Ile Thr Pro Trp Asn Phe Pro Leu Leu Met Leu Val Trp Lys Leu Ala
            180                 185                 190

Pro Ala Leu Cys Cys Gly Asn Thr Met Val Leu Lys Pro Ala Glu Gln
        195                 200                 205

Thr Pro Leu Thr Ala Leu Tyr Leu Gly Ser Leu Ile Lys Glu Ala Gly
    210                 215                 220

Phe Pro Pro Gly Val Val Asn Ile Val Pro Gly Phe Gly Pro Thr Val
225                 230                 235                 240

Gly Ala Ala Ile Ser Ser His Pro Gln Ile Asn Lys Ile Ala Phe Thr
                245                 250                 255

Gly Ser Thr Glu Val Gly Lys Leu Val Lys Glu Ala Ala Ser Arg Ser
            260                 265                 270

Asn Leu Lys Arg Val Thr Leu Glu Leu Gly Gly Lys Asn Pro Cys Ile
        275                 280                 285

Val Cys Ala Asp Ala Asp Leu Asp Leu Ala Val Glu Cys Ala His Gln
    290                 295                 300

Gly Val Phe Phe Asn Gln Gly Gln Cys Cys Thr Ala Ala Ser Arg Val
305                 310                 315                 320

Phe Val Glu Glu Gln Val Tyr Ser Glu Phe Val Arg Arg Ser Val Glu
                325                 330                 335

Tyr Ala Lys Lys Arg Pro Val Gly Asp Pro Phe Asp Val Lys Thr Glu
            340                 345                 350

Gln Gly Pro Gln Ile Asp Gln Lys Gln Phe Asp Lys Ile Leu Glu Leu
        355                 360                 365

Ile Glu Ser Gly Lys Lys Glu Gly Ala Lys Leu Glu Cys Gly Gly Ser
    370                 375                 380

Ala Met Glu Asp Lys Gly Leu Phe Ile Lys Pro Thr Val Phe Ser Glu
385                 390                 395                 400

Val Thr Asp Asn Met Arg Ile Ala Lys Glu Glu Ile Phe Gly Pro Val

405                 410                 415

Gln Pro Ile Leu Lys Phe Lys Ser Ile Glu Glu Val Ile Lys Arg Ala
        420                 425                 430

Asn Ser Thr Asp Tyr Gly Leu Thr Ala Ala Val Phe Thr Lys Asn Leu
        435                 440                 445

Asp Lys Ala Leu Lys Leu Ala Ser Ala Leu Glu Ser Gly Thr Val Trp
    450                 455                 460

Ile Asn Cys Tyr Asn Ala Leu Tyr Ala Gln Ala Pro Phe Gly Gly Phe
465                 470                 475                 480

Lys Met Ser Gly Asn Gly Arg Glu Leu Gly Glu Tyr Ala Leu Ala Glu
                485                 490                 495

Tyr Thr Glu Val Lys Thr Val Thr Ile Lys Leu Gly Asp Lys Asn Pro
            500                 505                 510

<210>  87
<211>  2252
<212>  DNA
<213>  Homo sapiens

<400>  87
gaccattagc aggcacccag gcctgtcttt ggctcggaaa cggtggcccc caatgtagcc      60

tagtttgaac ctaggaactg caggaccaga gagattccac tggagcctga tggacgggtg     120

acagagggaa ccctactctg gaaactgtca gtcccagggc actggggagg gctgaggccg     180

accatgccca gcctgctgct gctgttcacg gctgctctgc tgtccagctg ggctcagctt     240

ctgacagacg ccaactcctg gtggtcatta gctttgaacc cggtgcagag acccgagatg     300

tttatcatcg gtgcccagcc cgtgtgcagt cagcttcccg ggctctcccc tggccagagg     360

aagctgtgcc aattgtacca ggagcacatg gcctacatag gggagggagc caagactggc     420

atcaaggaat gccagcacca gttccggcag cggcggtgga attgcagcac agcggacaac     480

gcatctgtct ttgggagagt catgcagata ggcagccgag agaccgcctt cacccacgcg     540

gtgagcgccg cgggcgtggt caacgccatc agccgggcct gccgcgaggg cgagctctcc     600

acctgcggct gcagccggac ggcgcggccc aaggacctgc cccgggactg gctgtggggc     660

ggctgtgggg acaacgtgga gtacggctac cgcttcgcca aggagtttgt ggatgcccgg     720

gagcgagaga agaactttgc caaaggatca gaggagcagg gccgggtgct catgaacctg     780

caaaacaacg aggccggtcg cagggctgtg tataagatgg cagacgtagc ctgcaaatgc     840

cacggcgtct cggggtcctg cagcctcaag acctgctggc tgcagctggc cgagttccgc     900

aaggtcgggg accggctgaa ggagaagtac gacagcgcgg ccgccatgcg cgtcacccgc     960

aagggccggc tggagctggt caacagccgc ttcacccagc ccaccccgga ggacctggtc    1020

tatgtggacc ccagccccga ctactgcctg cgcaacgaga gcacgggctc cctgggcacg    1080

cagggccgcc tctgcaacaa gacctcggag ggcatggatg gctgtgagct catgtgctgc    1140

gggcgtggct acaaccagtt caagagcgtg caggtggagc gctgccactg caagttccac    1200

tggtgctgct tcgtcaggtg taagaagtgc acggagatcg tggaccagta catctgtaaa    1260

tagcccggag ggcctgctcc cggccccccc tgcactctgc ctcacaaagg tctatattat 1320

ataaatctat ataaatctat tttatatttg tataagtaaa tgggtgggtg ctatacaatg 1380

gaaagatgaa aatggaaagg aagagcttat ttaagagacg ctggagatct ctgaggagtg 1440

gactttgctg gttctctcct cttggtgggt gggagacagg gctttttctc tccctctggc 1500

gaggactctc aggatgtagg gacttggaaa tatttactgt ctgtccacca cggcctggag 1560

gagggaggtt gtggttggat ggaggagatg atcttgtctg gaagtctaga gtctttgttg 1620

gttagaggac tgcctgtgat cctggccact aggccaagag gccctatgaa ggtggcggga 1680

actcagcttc aacctcgatg tcttcagggt cttgtccaga atgtagatgg gttccgtaag 1740

aggcctggtg ctctcttact ctttcatcca cgtgcacttg tgcggcatct gcagtttaca 1800

ggaacggctc cttccctaaa atgagaagtc caaggtcatc tctggcccag tgaccacaga 1860

gagatctgca cctcccggac ttcaggcctg cctttccagc gagaattctt catcctccac 1920

ggttcactag ctcctacctg aagaggaaag ggggccattt gacctgacat gtcaggaaag 1980

ccctaaactg aatgtttgcg cctgggctgc agaagccagg gtgcatgacc aggctgcgtg 2040

gacgttatac tgtcttcccc cacccccggg gaggggaagc ttgagctgct gctgtcactc 2100

ctccaccgag ggaggcctca caaaccacag gacgctgcaa cgggtcaggc tggcgggccc 2160

ggcgtgctca tcatctctgc cccaggtgta cggtttctct ctgacattaa atgcccttca 2220

tggaaaaaaa aaaaagaaaa aaaaaaaaaa aa 2252

<210> 88
<211> 359
<212> PRT
<213> Homo sapiens

<400> 88

Met Pro Ser Leu Leu Leu Leu Phe Thr Ala Ala Leu Leu Ser Ser Trp
1               5                   10                  15

Ala Gln Leu Leu Thr Asp Ala Asn Ser Trp Trp Ser Leu Ala Leu Asn
            20                  25                  30

Pro Val Gln Arg Pro Glu Met Phe Ile Ile Gly Ala Gln Pro Val Cys
        35                  40                  45

Ser Gln Leu Pro Gly Leu Ser Pro Gly Gln Arg Lys Leu Cys Gln Leu
    50                  55                  60

Tyr Gln Glu His Met Ala Tyr Ile Gly Glu Gly Ala Lys Thr Gly Ile
65                  70                  75                  80

Lys Glu Cys Gln His Gln Phe Arg Gln Arg Arg Trp Asn Cys Ser Thr
                85                  90                  95

Ala Asp Asn Ala Ser Val Phe Gly Arg Val Met Gln Ile Gly Ser Arg
            100                 105                 110

Glu Thr Ala Phe Thr His Ala Val Ser Ala Ala Gly Val Val Asn Ala
        115                 120                 125

Ile Ser Arg Ala Cys Arg Glu Gly Glu Leu Ser Thr Cys Gly Cys Ser
    130                 135                 140

Arg Thr Ala Arg Pro Lys Asp Leu Pro Arg Asp Trp Leu Trp Gly Gly
145                 150                 155                 160

Cys Gly Asp Asn Val Glu Tyr Gly Tyr Arg Phe Ala Lys Glu Phe Val
                165                 170                 175

Asp Ala Arg Glu Arg Glu Lys Asn Phe Ala Lys Gly Ser Glu Glu Gln
            180                 185                 190

Gly Arg Val Leu Met Asn Leu Gln Asn Asn Glu Ala Gly Arg Arg Ala
        195                 200                 205

Val Tyr Lys Met Ala Asp Val Ala Cys Lys Cys His Gly Val Ser Gly
    210                 215                 220

Ser Cys Ser Leu Lys Thr Cys Trp Leu Gln Leu Ala Glu Phe Arg Lys
225                 230                 235                 240

Val Gly Asp Arg Leu Lys Glu Lys Tyr Asp Ser Ala Ala Ala Met Arg
                245                 250                 255

Val Thr Arg Lys Gly Arg Leu Glu Leu Val Asn Ser Arg Phe Thr Gln
            260                 265                 270

Pro Thr Pro Glu Asp Leu Val Tyr Val Asp Pro Ser Pro Asp Tyr Cys
        275                 280                 285

Leu Arg Asn Glu Ser Thr Gly Ser Leu Gly Thr Gln Gly Arg Leu Cys
    290                 295                 300

Asn Lys Thr Ser Glu Gly Met Asp Gly Cys Glu Leu Met Cys Cys Gly
305                 310                 315                 320

Arg Gly Tyr Asn Gln Phe Lys Ser Val Gln Val Glu Arg Cys His Cys
                325                 330                 335

Lys Phe His Trp Cys Cys Phe Val Arg Cys Lys Lys Cys Thr Glu Ile
            340                 345                 350

Val Asp Gln Tyr Ile Cys Lys
        355

<210>  89
<211>  794
<212>  DNA
<213>  Homo sapiens

<400>  89
ggcagccgtc tgtgccaccc agagccggcg ggccgctagg tccccggaga ccctgctatg      60

gtgcgtgcgg gcgccgtggg ggctcatctc cccgcgtccg gcttggatat cttcggggac     120

ctgaagaaga tgaacaagcg ccagctctat taccaggttt taaacttcgc catgatcgtg     180

tcttctgcac tcatgatatg gaaaggcttg atcgtgctca caggcagtga gagccccatc     240

gtggtggtgc tgagtggcag tatggagccg gcctttcaca gaggagacct cctgttcctc     300

acaaatttcc gggaagaccc aatcagagct ggtgaaatag ttgtttttaa agttgaagga     360

cgagacattc caatagttca cagagtaatc aaagttcatg aaaaagataa tggagacatc     420

aaatttctga ctaaaggaga taataatgaa gttgatgata gaggcttgta caaagaaggc     480

cagaactggc tggaaaagaa ggacgtggtg ggaagagcaa gagggttttt accatatgtt     540

ggtatggtca ccataataat gaatgactat ccaaaattca agtatgctct tttggctgta     600

atgggtgcat atgtgttact aaaacgtgaa tcctaaaatg agaagcagtt cctgggacca 660

gattgaaatg aattctgttg aaaaagagaa aaactaatat atttgagatg ttccattttc 720

tgtataaaag ggaacagtgt ggagatgttt ttgtcttgtc caaataaaag attcaccagt 780

aaaaaaaaaa aaaa 794

<210> 90
<211> 192
<212> PRT
<213> Homo sapiens

<400> 90

Met Val Arg Ala Gly Ala Val Gly Ala His Leu Pro Ala Ser Gly Leu
1               5                   10                  15

Asp Ile Phe Gly Asp Leu Lys Lys Met Asn Lys Arg Gln Leu Tyr Tyr
            20                  25                  30

Gln Val Leu Asn Phe Ala Met Ile Val Ser Ser Ala Leu Met Ile Trp
        35                  40                  45

Lys Gly Leu Ile Val Leu Thr Gly Ser Glu Ser Pro Ile Val Val Val
    50                  55                  60

Leu Ser Gly Ser Met Glu Pro Ala Phe His Arg Gly Asp Leu Leu Phe
65                  70                  75                  80

Leu Thr Asn Phe Arg Glu Asp Pro Ile Arg Ala Gly Glu Ile Val Val
                85                  90                  95

Phe Lys Val Glu Gly Arg Asp Ile Pro Ile Val His Arg Val Ile Lys
            100                 105                 110

Val His Glu Lys Asp Asn Gly Asp Ile Lys Phe Leu Thr Lys Gly Asp
        115                 120                 125

Asn Asn Glu Val Asp Asp Arg Gly Leu Tyr Lys Glu Gly Gln Asn Trp
    130                 135                 140

Leu Glu Lys Lys Asp Val Val Gly Arg Ala Arg Gly Phe Leu Pro Tyr
145                 150                 155                 160

Val Gly Met Val Thr Ile Ile Met Asn Asp Tyr Pro Lys Phe Lys Tyr
                165                 170                 175

Ala Leu Leu Ala Val Met Gly Ala Tyr Val Leu Leu Lys Arg Glu Ser
            180                 185                 190

<210> 91
<211> 2108
<212> DNA
<213> Homo sapiens

<400> 91
gattactcac acagtcttga agatgcaatg tcagctattt aggacagaaa catccaaggc 60

cgtgtcagaa ctcaattacg actacatatg cattaaggca ggaactggca ggcctcaggg 120

tacgccaact ataggactcg tgcttctcgt acgctgggct ataatctatg aaactgagct 180

ccagagccag ccaatcactt agctcctcat aacaagtcta actggctctg gaaagctgaa 240

agggctgcac tggaacaaca cagatgagat attctacaca ttaatctact tatctggaat 300

cactttgcct ctaaaggcca gagaaaaatc acagcttcct tgtcggaggg gaaaaggaca 360

ggtgatctgg ggaaaacgca gctacacctg gagcaaggtc tcttcccggc ttggcaatct 420
cagctgtgcc ggcgctacgg gacccgagcc gtcccagaaa ccaaagggca ggcacggcag 480
caaacgcctg agtgctgctg ccttcggtga ctatatgaga atggaaactt ctaaggaagc 540
caggttgtta gaattgttac cccctttact cagagataac atagattatc caggctgaga 600
tggaaaacaa gccctttatt gaattttcaa cacagactcc ctgcttctca tctccttaat 660
aaaatttcat taaaatcccc ttgaactccc atgttcaaat ctccatttgt tgacagacaa 720
agccaacaat actctaaact gaggcctgca agtcatttca tttgtatttt tgtccagaaa 780
tttcccatag gaagacttca cctcctacaa ctccgaagaa aacccttact gtccaagacc 840
gtcaccagca accatccgca gtcattcaag tggaagcttt cacagctttt gtacattctc 900
tgtgtcaata tacaactgag ttacagactg tcccctggct ccctgaccct tacaaacact 960
aaaagttttg tttgactcaa cttcaagctg ctcatctgtt agtaagtgat gttcactcca 1020
gaacacattc atgatgagaa ctttctaaaa gaccagcact gctcttcccc tcctataatc 1080
ataataatca tgataacctg aaacatgtta ctgggactcg acatttttct ggggattgaa 1140
atctttagtc cttggagctg tcacatagca ggggcaacct cacactgaaa caaaggaagt 1200
gatgtcccat tattatccac cctgagccac cataatatgc tgtttacatt tattttcttc 1260
agcctgtgca aaacaaagca atggaaaagg aaactaaaaa atatacatac tagtaccatt 1320
atcttctttt gcctaaaatt actaatgcac cacgtcagtc tgcttccttc aggcatcatt 1380
ctcaattcat caggacttgt attagcaggt tctggctaga gagactatct cctgtcatca 1440
cgatcaatta atgttttctg gtgatcacat caggccctat ctaagaagct catggtatac 1500
aagggtcacc caaatagctg agtgcagtcc ttgctcatat ttccttcatc ttaaccccgc 1560
aaacaagaat taagatgatc ccaataaaag aaaaattgct caggaaactg aacctttttc 1620
tgaaccaagc actgtcagca aatctcaggt attagagcaa ctatggttga ttgaaaagtg 1680
tctcaaaatc tgggccaaga atgattgcta ggtccataag ctaatttgtc tggccttgcc 1740
atttacgtaa gccaaagaaa gtcactcatg agtaaactat agaaaacgtt cagacccatc 1800
ctgttagtat gtcaaatcaa ctaagactgg cagggtatta actccattcc aggtgacatg 1860
gataaagagc cccattattt tcacagtgcc agcctctacc taaggaaacc ctagaccttg 1920
gaaccagttt cctggtaggg aactgctgac agtttcaatg ctgacagttg gagccaatgc 1980
ctcatagtgt aaactgaaag aaaaatagtt gctttttaaa atgtcagcaa gaaggcctgc 2040
ctcatcttaa caaagcaaaa aaaaatgctt taattcaaat taaaaatcat gatactaaaa 2100
aaaaaaaa 2108

<210> 92
<211> 59
<212> PRT
<213> Homo sapiens

<400> 92

Met Gln Cys Gln Leu Phe Arg Thr Glu Thr Ser Lys Ala Val Ser Glu
1               5                   10                  15

Leu Asn Tyr Asp Tyr Ile Cys Ile Lys Ala Gly Thr Gly Arg Pro Gln
            20                  25                  30

Gly Thr Pro Thr Ile Gly Leu Val Leu Leu Val Arg Trp Ala Ile Ile
        35                  40                  45

Tyr Glu Thr Glu Leu Gln Ser Gln Pro Ile Thr
    50                  55

<210>  93
<211>  1991
<212>  DNA
<213>  Homo sapiens

<400>  93
cgcggcggct ggcgtcggga aagtacagta aaaagtccga gtgcagccgc cgggcgcagg      60

atgggatccg gctcctccag ctaccggccc aaggccatct acctggacat cgatggacgc     120

attcagaagg taatcttcag caagtactgc aactccagcg acatcatgga cctgttctgc     180

atcgccaccg gcctgcctcg gaacacgacc atctccctgc tgaccaccga cgacgccatg     240

gtctccatcg accccaccat gcccgcgaat tcagaacgca ctccgtacaa agtgagacct     300

gtggccatca agcaactctc cgctggtgtc gaggacaaga gaaccacaag ccgtggccag     360

tctgctgaga gaccactgag ggacagacgg gttgtgggcc tggagcagcc ccggagggaa     420

ggagcatttg aaagtggaca ggtagagccc aggcccagag agccccaggg ctgctaccag     480

gaaggccagc gcatccctcc agagagagaa gaattaatcc agagcgtgct ggcgcaggtt     540

gcagagcagt tctcaagagc attcaaaatc aatgaactga aagctgaagt tgcaaatcac     600

ttggctgtcc tagagaaacg cgtggaattg gaaggactaa aagtggtgga gattgagaaa     660

tgcaagagtg acattaagaa gatgagggag gagctggcgg ccagaagcag caggaccaac     720

tgcccctgta agtacagttt tttggataac cacaagaagt tgactcctcg acgcgatgtt     780

cccacttacc ccaagtacct gctctctcca gagaccatcg aggccctgcg gaagccgacc     840

tttgacgtct ggctttggga gcccaatgag atgctgagct gcctggagca catgtaccac     900

gacctcgggc tggtcaggga cttcagcatc aaccctgtca ccctcaggag gtggctgttc     960

tgtgtccacg acaactacag aaacaacccc ttccacaact tccggcactg cttctgcgtg    1020

gcccagatga tgtacagcat ggtctggctc tgcagtctcc aggagaagtt ctcacaaacg    1080

gatatcctga tcctaatgac agcggccatc tgccacgatc tggaccatcc cggctacaac    1140

aacacgtacc agatcaatgc ccgcacagag ctggcggtcc gctacaatga catctcaccg    1200

ctggagaacc accactgcgc cgtggccttc cagatcctcg ccgagcctga gtgcaacatc    1260

ttctccaaca tcccacctga tgggttcaag cagatccgac agggaatgat cacattaatc    1320

ttggccactg acatggcaag acatgcagaa attatggatt ctttcaaaga gaaaatggag    1380

aattttgact acagcaacga ggagcacatg accctgctga agatgatttt gataaaatgc    1440

tgtgatatct ctaacgaggt ccgtccaatg gaagtcgcag agccttgggt ggactgttta    1500

ttagaggaat attttatgca gagcgaccgt gagaagtcag aaggccttcc tgtggcaccg 1560

ttcatggacc gagacaaagt gaccaaggcc acagcccaga ttgggttcat caagtttgtc 1620

ctgatcccaa tgtttgaaac agtgaccaag ctcttcccca tggttgagga gatcatgctg 1680

cagccacttt gggaatcccg agatcgctac gaggagctga agcggataga tgacgccatg 1740

aaagagttac agaagaagac tgacagcttg acgtctgggg ccaccgagaa gtccagagag 1800

agaagcagag atgtgaaaaa cagtgaagga gactgtgcct gaggaaagcg gggggcgtgg 1860

ctgcagttct ggacgggctg gccgagctgc gcgggatcct tgtgcaggga agagctgccc 1920

tgggcacctg gcaccacaag accatgtttt ctaagaacca ttttgttcac tgatacaaaa 1980

aaaaaaaaaa a 1991

<210> 94
<211> 593
<212> PRT
<213> Homo sapiens

<400> 94

Met Gly Ser Gly Ser Ser Ser Tyr Arg Pro Lys Ala Ile Tyr Leu Asp
1               5                   10                  15

Ile Asp Gly Arg Ile Gln Lys Val Ile Phe Ser Lys Tyr Cys Asn Ser
            20                  25                  30

Ser Asp Ile Met Asp Leu Phe Cys Ile Ala Thr Gly Leu Pro Arg Asn
        35                  40                  45

Thr Thr Ile Ser Leu Leu Thr Thr Asp Asp Ala Met Val Ser Ile Asp
    50                  55                  60

Pro Thr Met Pro Ala Asn Ser Glu Arg Thr Pro Tyr Lys Val Arg Pro
65                  70                  75                  80

Val Ala Ile Lys Gln Leu Ser Ala Gly Val Glu Asp Lys Arg Thr Thr
                85                  90                  95

Ser Arg Gly Gln Ser Ala Glu Arg Pro Leu Arg Asp Arg Arg Val Val
            100                 105                 110

Gly Leu Glu Gln Pro Arg Arg Glu Gly Ala Phe Glu Ser Gly Gln Val
        115                 120                 125

Glu Pro Arg Pro Arg Glu Pro Gln Gly Cys Tyr Gln Glu Gly Gln Arg
    130                 135                 140

Ile Pro Pro Glu Arg Glu Glu Leu Ile Gln Ser Val Leu Ala Gln Val
145                 150                 155                 160

Ala Glu Gln Phe Ser Arg Ala Phe Lys Ile Asn Glu Leu Lys Ala Glu
                165                 170                 175

Val Ala Asn His Leu Ala Val Leu Glu Lys Arg Val Glu Leu Glu Gly
            180                 185                 190

Leu Lys Val Val Glu Ile Glu Lys Cys Lys Ser Asp Ile Lys Lys Met
        195                 200                 205

Arg Glu Glu Leu Ala Ala Arg Ser Ser Arg Thr Asn Cys Pro Cys Lys
    210                 215                 220

Tyr Ser Phe Leu Asp Asn His Lys Lys Leu Thr Pro Arg Arg Asp Val
225                 230                 235                 240

Pro Thr Tyr Pro Lys Tyr Leu Leu Ser Pro Glu Thr Ile Glu Ala Leu
                245                 250                 255

Arg Lys Pro Thr Phe Asp Val Trp Leu Trp Glu Pro Asn Glu Met Leu
            260                 265                 270

Ser Cys Leu Glu His Met Tyr His Asp Leu Gly Leu Val Arg Asp Phe
        275                 280                 285

Ser Ile Asn Pro Val Thr Leu Arg Arg Trp Leu Phe Cys Val His Asp
    290                 295                 300

Asn Tyr Arg Asn Asn Pro Phe His Asn Phe Arg His Cys Phe Cys Val
305                 310                 315                 320

Ala Gln Met Met Tyr Ser Met Val Trp Leu Cys Ser Leu Gln Glu Lys
                325                 330                 335

Phe Ser Gln Thr Asp Ile Leu Ile Leu Met Thr Ala Ala Ile Cys His
            340                 345                 350

Asp Leu Asp His Pro Gly Tyr Asn Asn Thr Tyr Gln Ile Asn Ala Arg
        355                 360                 365

Thr Glu Leu Ala Val Arg Tyr Asn Asp Ile Ser Pro Leu Glu Asn His
    370                 375                 380

His Cys Ala Val Ala Phe Gln Ile Leu Ala Glu Pro Glu Cys Asn Ile
385                 390                 395                 400

Phe Ser Asn Ile Pro Pro Asp Gly Phe Lys Gln Ile Arg Gln Gly Met
                405                 410                 415

Ile Thr Leu Ile Leu Ala Thr Asp Met Ala Arg His Ala Glu Ile Met
            420                 425                 430

Asp Ser Phe Lys Glu Lys Met Glu Asn Phe Asp Tyr Ser Asn Glu Glu
        435                 440                 445

His Met Thr Leu Leu Lys Met Ile Leu Ile Lys Cys Cys Asp Ile Ser
    450                 455                 460

Asn Glu Val Arg Pro Met Glu Val Ala Glu Pro Trp Val Asp Cys Leu
465                 470                 475                 480

Leu Glu Glu Tyr Phe Met Gln Ser Asp Arg Glu Lys Ser Glu Gly Leu
                485                 490                 495

Pro Val Ala Pro Phe Met Asp Arg Asp Lys Val Thr Lys Ala Thr Ala
            500                 505                 510

Gln Ile Gly Phe Ile Lys Phe Val Leu Ile Pro Met Phe Glu Thr Val
        515                 520                 525

Thr Lys Leu Phe Pro Met Val Glu Glu Ile Met Leu Gln Pro Leu Trp
    530                 535                 540

Glu Ser Arg Asp Arg Tyr Glu Glu Leu Lys Arg Ile Asp Asp Ala Met
545                 550                 555                 560

Lys Glu Leu Gln Lys Lys Thr Asp Ser Leu Thr Ser Gly Ala Thr Glu
                565                 570                 575

Lys Ser Arg Glu Arg Ser Arg Asp Val Lys Asn Ser Glu Gly Asp Cys
            580                 585                 590

Ala

<210>  95
<211>  691
<212>  DNA
<213>  Homo sapiens

<400>  95
cagtcacagg cgagagccyt gggatgcacc ggccagaggc atgctgctgc tgctcacgct      60

tgccctcctg gggggcccca cctgggcagg gaagatgtat ggccctggag gaggcaagta     120

tttcagcacc actgaagact acgaccatga aatcacaggg ctgcgggtgt ctgtaggtct     180

tctcctggtg aaaagtgtcc aggtgaaact tggagactcc tgggacgtga aactgggagc     240

cttaggtggg aatacccagg aagtcaccct gcagccaggc gaatacatca caaaagtctt     300

tgtcgccttc caagctttcc tccggggtat ggtcatgtac accagcaagg accgctattt     360

ctattttggg aagcttgatg gccagatctc ctctgcctac cccagccaag aggggcaggt     420

gctggtgggc atctatggcc agtatcaact ccttggcatc aagagcattg gctttgaatg     480

gaattatcca ctagaggagc cgaccactga gccaccagtt aatctcacat actcagcaaa     540

ctcacccgtg ggtcgctagg gtggggtatg gggccatccg agctgaggcc atctgtgtgg     600

tggtggctga tggtactgga gtaactgagt cgggacgctg aatctgaatc caccaataaa     660

taaagcttct gcagaatcag tgaaaaaaaa a                                    691

<210>  96
<211>  172
<212>  PRT
<213>  Homo sapiens

<400>  96

Met Leu Leu Leu Leu Thr Leu Ala Leu Leu Gly Gly Pro Thr Trp Ala
1               5                   10                  15

Gly Lys Met Tyr Gly Pro Gly Gly Gly Lys Tyr Phe Ser Thr Thr Glu
            20                  25                  30

Asp Tyr Asp His Glu Ile Thr Gly Leu Arg Val Ser Val Gly Leu Leu
        35                  40                  45

Leu Val Lys Ser Val Gln Val Lys Leu Gly Asp Ser Trp Asp Val Lys
    50                  55                  60

Leu Gly Ala Leu Gly Gly Asn Thr Gln Glu Val Thr Leu Gln Pro Gly
65                  70                  75                  80

Glu Tyr Ile Thr Lys Val Phe Val Ala Phe Gln Ala Phe Leu Arg Gly
                85                  90                  95

Met Val Met Tyr Thr Ser Lys Asp Arg Tyr Phe Tyr Phe Gly Lys Leu
            100                 105                 110

Asp Gly Gln Ile Ser Ser Ala Tyr Pro Ser Gln Glu Gly Gln Val Leu
        115                 120                 125

Val Gly Ile Tyr Gly Gln Tyr Gln Leu Leu Gly Ile Lys Ser Ile Gly
    130                 135                 140

Phe Glu Trp Asn Tyr Pro Leu Glu Glu Pro Thr Thr Glu Pro Pro Val
145                 150                 155                 160

Asn Leu Thr Tyr Ser Ala Asn Ser Pro Val Gly Arg
                165                 170

<210>  97
<211>  1059
<212>  DNA
<213>  Homo sapiens

<400>  97
ccgttccgcg ctctggcggc tcctcccggg cgatgcctcc gctctgggcc ctgctggccc      60

tcggctgcct gcggttcggc tcggctgtga acctgcagcc ccaactggcc agtgtgactt     120

tcgccaccaa caaccccaca cttaccactg tggccttgga aaagcctctc tgcatgtttg     180

acagcaaaga ggccctcact ggcacccacg aggtctacct gtatgtcctg gtcgactcag     240

ccatttccag gaatgcctca gtgcaagaca gcaccaacac cccactgggc tcaacgttcc     300

tacaaacaga gggtgggagg acaggtccct acaaagctgt ggcctttgac ctgatcccct     360

gcagtgacct gcccagcctg gatgccattg gggatgtgtc caaggcctca cagatcctga     420

atgcctacct ggtcagggtg ggtgccaacg ggacctgcct gtgggatccc aacttccagg     480

gcctctgtaa cgcacccctg tcggcagcca cggagtacag gttcaagtat gtcctggtca     540

atatgtccac gggcttggta gaggaccaga ccctgtggtc ggaccccatc cgcaccaacc     600

agctcacccc atactcgacg atcgacacgt ggccaggccg gcggagcgga ggcatgatcg     660

tcatcacttc catcctgggc tccctgccct tctttctact tgtgggtttt gctggcgcca     720

ttgccctcag cctcgtggac atggggagtt ctgatgggga aacgactcac gactcccaaa     780

tcactcagga ggctgttccc aagtcgctgg gggcctcgga gtcttcctac acgtccgtga     840

accgggggcc gccactggac agggctgagg tgtattccag caagctccaa gactgagccc     900

agcaccaccc ctgggcagca gcatcctcct ctctggcctt gccccaggcc ctgcagcggt     960

ggttgtcaca ccctgacttc agggaaggtg aaacagggct tgtccctcca actgcaggaa    1020

aacccttaat aaaatcttct gatgagttct aaaaaaaaa                           1059

<210>  98
<211>  287
<212>  PRT
<213>  Homo sapiens

<400>  98

Met Pro Pro Leu Trp Ala Leu Leu Ala Leu Gly Cys Leu Arg Phe Gly
1               5                   10                  15

Ser Ala Val Asn Leu Gln Pro Gln Leu Ala Ser Val Thr Phe Ala Thr
            20                  25                  30

Asn Asn Pro Thr Leu Thr Thr Val Ala Leu Glu Lys Pro Leu Cys Met
        35                  40                  45

Phe Asp Ser Lys Glu Ala Leu Thr Gly Thr His Glu Val Tyr Leu Tyr
    50                  55                  60

Val Leu Val Asp Ser Ala Ile Ser Arg Asn Ala Ser Val Gln Asp Ser
65                  70                  75                  80

Thr Asn Thr Pro Leu Gly Ser Thr Phe Leu Gln Thr Glu Gly Gly Arg
                85                  90                  95

Thr Gly Pro Tyr Lys Ala Val Ala Phe Asp Leu Ile Pro Cys Ser Asp
            100                 105                 110

Leu Pro Ser Leu Asp Ala Ile Gly Asp Val Ser Lys Ala Ser Gln Ile
        115                 120                 125

Leu Asn Ala Tyr Leu Val Arg Val Gly Ala Asn Gly Thr Cys Leu Trp
    130                 135                 140

Asp Pro Asn Phe Gln Gly Leu Cys Asn Ala Pro Leu Ser Ala Ala Thr
145                 150                 155                 160

Glu Tyr Arg Phe Lys Tyr Val Leu Val Asn Met Ser Thr Gly Leu Val
                165                 170                 175

Glu Asp Gln Thr Leu Trp Ser Asp Pro Ile Arg Thr Asn Gln Leu Thr
            180                 185                 190

Pro Tyr Ser Thr Ile Asp Thr Trp Pro Gly Arg Arg Ser Gly Gly Met
        195                 200                 205

Ile Val Ile Thr Ser Ile Leu Gly Ser Leu Pro Phe Phe Leu Leu Val
    210                 215                 220

Gly Phe Ala Gly Ala Ile Ala Leu Ser Leu Val Asp Met Gly Ser Ser
225                 230                 235                 240

Asp Gly Glu Thr Thr His Asp Ser Gln Ile Thr Gln Glu Ala Val Pro
                245                 250                 255

Lys Ser Leu Gly Ala Ser Glu Ser Ser Tyr Thr Ser Val Asn Arg Gly
            260                 265                 270

Pro Pro Leu Asp Arg Ala Glu Val Tyr Ser Ser Lys Leu Gln Asp
        275                 280                 285

<210>  99
<211>  1894
<212>  DNA
<213>  Homo sapiens

<400>  99
gtctgacttc ctcccagcac attcctgcac tctgccgtgt ccacactgcc ccacagaccc      60

agtcctccaa gcctgctgcc agctccctgc aagcccctca ggttgggcct tgccacggtg     120

ccagcaggca gccctgggct gggggtaggg gactccctac aggcacgcag ccctgagacc     180

tcagagggcc accccttgag ggtggccagg cccccagtgg ccaacctgag tgctgcctct     240

gccaccagcc ctgctggccc ctggttccgc tggcccccca gatgcctggc tgagacacgc     300

cagtggcctc agctgcccac acctcttccc ggcccctgaa gttggcactg cagcagacag     360

ctccctgggc accaggcagc taacagacac agccgccagc ccaaacagca gcggcatggg     420

cagcgccagc ccgggtctga gcagcgtatc ccccagccac ctcctgctgc cccccgacac     480

ggtgtcgcgg acaggcttgg agaaggcggc agcgggggca gtgggtctcg agagacggga     540

ctggagtccc agtccacccg ccacgcccga gcagggcctg tccgccttct acctctccta     600

ctttgacatg ctgtaccctg aggacagcag ctgggcagcc aaggcccctg gggccagcag 660
tcgggaggag ccacctgagg agcctgagca gtgcccggtc attgacagcc aagccccagc 720
gggcagcctg gacttggtgc ccggcgggct gaccttggag gagcactcgc tggagcaggt 780
gcagtccatg gtggtgggcg aagtgctcaa ggacatcgag acggcctgca agctgctcaa 840
catcaccgca gatcccatgg actggagccc cagcaatgtg cagaagtggc tcctgtggac 900
agagcaccaa taccggctgc cccccatggg caaggccttc caggagctgg cgggcaagga 960
gctgtgcgcc atgtcggagg agcagttccg ccagcgctcg cccctgggtg gggatgtgct 1020
gcacgcccac ctggacatct ggaagtcagc ggcctggatg aaagagcgga cttcacctgg 1080
ggcgattcac tactgtgcct cgaccagtga ggagagctgg accgacagcg aggtggactc 1140
atcatgctcc gggcagccca tccacctgtg gcagttcctc aaggagttgc tactcaagcc 1200
ccacagctat ggccgcttca ttaggtggct caacaaggag aagggcatct tcaaaattga 1260
ggactcagcc caggtggccc ggctgtgggg catccgcaag aaccgtcccg ccatgaacta 1320
cgacaagctg agccgctcca tccgccagta ttacaagaag ggcatcatcc ggaagccaga 1380
catctcccag cgcctcgtct accagttcgt gcaccccatc tgagtgcctg gcccagggcc 1440
tgaaacccgc cctcaggggc ctctctcctg cctgccctgc ctcagccagg ccctgagatg 1500
ggggaaaacg ggcagtctgc tctgctgctc tgaccttcca gagcccaagg tcagggaggg 1560
gcaaccaact gccccagggg gatatgggtc ctctggggcc ttcgggacca tggggcaggg 1620
gtgcttcctc ctcaggccca gctgctcccc tggaggacag agggagacag ggctgctccc 1680
caacacctgc ctctgacccc agcatttcca gagcagagcc tacagaaggg cagtgactcg 1740
acaaaggcca caggcagtcc aggcctctct ctgctccatc cccctgcctc ccattctgca 1800
ccacacctgg catggtgcag ggagacatct gcacccctga gttgggcagc caggagtgcc 1860
cccgggaatg gataataaag atactagaga actg 1894

<210> 100
<211> 335
<212> PRT
<213> Homo sapiens

<400> 100

Met Gly Ser Ala Ser Pro Gly Leu Ser Ser Val Ser Pro Ser His Leu
1               5                   10                  15

Leu Leu Pro Pro Asp Thr Val Ser Arg Thr Gly Leu Glu Lys Ala Ala
            20                  25                  30

Ala Gly Ala Val Gly Leu Glu Arg Arg Asp Trp Ser Pro Ser Pro Pro
        35                  40                  45

Ala Thr Pro Glu Gln Gly Leu Ser Ala Phe Tyr Leu Ser Tyr Phe Asp
    50                  55                  60

Met Leu Tyr Pro Glu Asp Ser Ser Trp Ala Ala Lys Ala Pro Gly Ala
65                  70                  75                  80

Ser Ser Arg Glu Glu Pro Pro Glu Glu Pro Glu Gln Cys Pro Val Ile

85                  90                  95

Asp Ser Gln Ala Pro Ala Gly Ser Leu Asp Leu Val Pro Gly Gly Leu
            100                 105                 110

Thr Leu Glu Glu His Ser Leu Glu Gln Val Gln Ser Met Val Val Gly
        115                 120                 125

Glu Val Leu Lys Asp Ile Glu Thr Ala Cys Lys Leu Leu Asn Ile Thr
    130                 135                 140

Ala Asp Pro Met Asp Trp Ser Pro Ser Asn Val Gln Lys Trp Leu Leu
145                 150                 155                 160

Trp Thr Glu His Gln Tyr Arg Leu Pro Pro Met Gly Lys Ala Phe Gln
                165                 170                 175

Glu Leu Ala Gly Lys Glu Leu Cys Ala Met Ser Glu Glu Gln Phe Arg
            180                 185                 190

Gln Arg Ser Pro Leu Gly Gly Asp Val Leu His Ala His Leu Asp Ile
        195                 200                 205

Trp Lys Ser Ala Ala Trp Met Lys Glu Arg Thr Ser Pro Gly Ala Ile
    210                 215                 220

His Tyr Cys Ala Ser Thr Ser Glu Glu Ser Trp Thr Asp Ser Glu Val
225                 230                 235                 240

Asp Ser Ser Cys Ser Gly Gln Pro Ile His Leu Trp Gln Phe Leu Lys
                245                 250                 255

Glu Leu Leu Leu Lys Pro His Ser Tyr Gly Arg Phe Ile Arg Trp Leu
            260                 265                 270

Asn Lys Glu Lys Gly Ile Phe Lys Ile Glu Asp Ser Ala Gln Val Ala
        275                 280                 285

Arg Leu Trp Gly Ile Arg Lys Asn Arg Pro Ala Met Asn Tyr Asp Lys
    290                 295                 300

Leu Ser Arg Ser Ile Arg Gln Tyr Tyr Lys Lys Gly Ile Ile Arg Lys
305                 310                 315                 320

Pro Asp Ile Ser Gln Arg Leu Val Tyr Gln Phe Val His Pro Ile
                325                 330                 335

<210>  101
<211>  2664
<212>  DNA
<213>  Homo sapiens

<400>  101
gagagaacag cgtgagcctg tgtgcttgtg tgctgagccc tcatcccctc ctggggccag      60

gcttgggttt cacctgcaga atcgcttgtg ctgggctgcc tgggctgtcc tcagtggcac     120

ctgcatgaag ccgttctggc tgccagagct ggacagcccc aggaaaaccc acctctctgc     180

agagcttgcc cagctgtccc cgggaagcca aatgcctctc atgtaagtct tctgctcgac     240

ggggtgtctc ctaaaccctc actcttcagc ctctgtttga ccatgaaatg aagtgactga     300

gctctattct gtacctgcca ctctatttct ggggtgactt ttgtcagctg cccagaatct     360

ccaagccagg ctggttctct gcatcctttc aatgacctgt tttcttctgt aaccacaggt     420

tcggtggtga gaggaagcct cgcagaatcc agcagaatcc tcacagaatc cagcagcagc     480

tctgctgggg acatggtcca tggtgcaacc cacagcaaag ccctgacctg acctcctgat 540
gctcaggaga agccatgggc ccctcctgtc ctgtgttcct gtccttcaca aagctcagcc 600
tgtggtggct ccttctgacc ccagcaggtg gagaggaagc taagcgccca cctcccaggg 660
ctcctggaga cccactctcc tctcccagtc ccacggcatt gccgcaggga ggctcgcata 720
ccgagactga ggaccggctc ttcaaacacc tcttccgggg ctacaaccgc tgggcgcgcc 780
cggtgcccaa cacttcagac gtggtgattg tgcgctttgg actgtccatc gctcagctca 840
tcgatgtgga tgagaagaac caaatgatga ccaccaacgt ctggctaaaa caggagtgga 900
gcgactacaa actgcgctgg aaccccgctg attttggcaa catcacatct ctcagggtcc 960
cttctgagat gatctggatc cccgacattg ttctctacaa caatgcagat ggggagtttg 1020
cagtgaccca catgaccaag gcccacctct tctccacggg cactgtgcac tgggtgcccc 1080
cggccatcta caagagctcc tgcagcatcg acgtcacctt cttccccttc gaccagcaga 1140
actgcaagat gaagtttggc tcctggactt atgacaaggc caagatcgac ctggagcaga 1200
tggagcagac tgtggacctg aaggactact gggagagcgg cgagtgggcc atcgtcaatg 1260
ccacgggcac ctacaacagc aagaagtacg actgctgcgc cgagatctac cccgacgtca 1320
cctacgcctt cgtcatccgg cggctgccgc tcttctacac catcaacctc atcatcccct 1380
gcctgctcat ctcctgcctc actgtgctgg tcttctacct gccctccgac tgcggcgaga 1440
agatcacgct gtgcatttcg gtgctgctgt cactcaccgt cttcctgctg ctcatcactg 1500
agatcatccc gtccacctcg ctggtcatcc cgctcatcgg cgagtacctg ctgttcacca 1560
tgatcttcgt caccctgtcc atcgtcatca ccgtcttcgt gctcaatgtg caccaccgct 1620
cccccagcac ccacaccatg ccccactggg tgcggggggc ccttctgggc tgtgtgcccc 1680
ggtggcttct gatgaaccgg cccccaccac ccgtggagct ctgccacccc ctacgcctga 1740
agctcagccc ctcttatcac tggctggaga gcaacgtgga tgccgaggag agggaggtgg 1800
tggtggagga ggaggacaga tgggcatgtg caggtcatgt ggccccctct gtgggcaccc 1860
tctgcagcca cggccacctg cactctgggg cctcaggtcc caaggctgag gctctgctgc 1920
aggagggtga gctgctgcta tcaccccaca tgcagaaggc actggaaggt gtgcactaca 1980
ttgccgacca cctgcggtct gaggatgctg actcttcggt gaaggaggac tggaagtatg 2040
ttgccatggt catcgacagg atcttcctct ggctgtttat catcgtctgc ttcctgggga 2100
ccatcggcct ctttctgcct ccgttcctag ctggaatgat ctgactgcac ctccctcgag 2160
ctggctccca gggcaaaggg gagggttctt ggatgtggaa gggctttgaa caatgtttag 2220
atttggagat gagcccaaag tgccagggag aacagccagg tgaggtggga ggttggagag 2280
ccaggtgagg tctctctaag tcaggctggg gttgaagttt ggagtctgtc cgagtttgca 2340
gggtgctgag ctgtatggtc cagcagggga gtaataaggg ctcttccgga aggggaggaa 2400
gcgggaggca ggcctgcacc tgatgtggag gtacaggcag atcttcccta ccggggaggg 2460
atggatggtt ggatacaggt ggctgggcta ttccatccat ctggaagcac atttgagcct 2520

ccaggcttct ccttgacgtc attcctctcc ttccttgctg caaaatggct ctgcaccagc 2580

cggcccccag gaggtctggc agagctgaga gccatggcct gcaggggctc catatgtccc 2640

tacgcgtgca gcaggcaaac aaga 2664

<210> 102
<211> 529
<212> PRT
<213> Homo sapiens

<400> 102

Met Gly Pro Ser Cys Pro Val Phe Leu Ser Phe Thr Lys Leu Ser Leu
1               5                   10                  15

Trp Trp Leu Leu Leu Thr Pro Ala Gly Gly Glu Glu Ala Lys Arg Pro
            20                  25                  30

Pro Pro Arg Ala Pro Gly Asp Pro Leu Ser Ser Pro Ser Pro Thr Ala
        35                  40                  45

Leu Pro Gln Gly Gly Ser His Thr Glu Thr Glu Asp Arg Leu Phe Lys
    50                  55                  60

His Leu Phe Arg Gly Tyr Asn Arg Trp Ala Arg Pro Val Pro Asn Thr
65                  70                  75                  80

Ser Asp Val Val Ile Val Arg Phe Gly Leu Ser Ile Ala Gln Leu Ile
                85                  90                  95

Asp Val Asp Glu Lys Asn Gln Met Met Thr Thr Asn Val Trp Leu Lys
            100                 105                 110

Gln Glu Trp Ser Asp Tyr Lys Leu Arg Trp Asn Pro Ala Asp Phe Gly
        115                 120                 125

Asn Ile Thr Ser Leu Arg Val Pro Ser Glu Met Ile Trp Ile Pro Asp
    130                 135                 140

Ile Val Leu Tyr Asn Asn Ala Asp Gly Glu Phe Ala Val Thr His Met
145                 150                 155                 160

Thr Lys Ala His Leu Phe Ser Thr Gly Thr Val His Trp Val Pro Pro
                165                 170                 175

Ala Ile Tyr Lys Ser Ser Cys Ser Ile Asp Val Thr Phe Phe Pro Phe
            180                 185                 190

Asp Gln Gln Asn Cys Lys Met Lys Phe Gly Ser Trp Thr Tyr Asp Lys
        195                 200                 205

Ala Lys Ile Asp Leu Glu Gln Met Glu Gln Thr Val Asp Leu Lys Asp
    210                 215                 220

Tyr Trp Glu Ser Gly Glu Trp Ala Ile Val Asn Ala Thr Gly Thr Tyr
225                 230                 235                 240

Asn Ser Lys Lys Tyr Asp Cys Cys Ala Glu Ile Tyr Pro Asp Val Thr
                245                 250                 255

Tyr Ala Phe Val Ile Arg Arg Leu Pro Leu Phe Tyr Thr Ile Asn Leu
            260                 265                 270

Ile Ile Pro Cys Leu Leu Ile Ser Cys Leu Thr Val Leu Val Phe Tyr
        275                 280                 285

Leu Pro Ser Asp Cys Gly Glu Lys Ile Thr Leu Cys Ile Ser Val Leu
    290                 295                 300

Leu Ser Leu Thr Val Phe Leu Leu Leu Ile Thr Glu Ile Ile Pro Ser
305                 310                 315                 320

Thr Ser Leu Val Ile Pro Leu Ile Gly Glu Tyr Leu Leu Phe Thr Met
                325                 330                 335

Ile Phe Val Thr Leu Ser Ile Val Ile Thr Val Phe Val Leu Asn Val
            340                 345                 350

His His Arg Ser Pro Ser Thr His Thr Met Pro His Trp Val Arg Gly
        355                 360                 365

Ala Leu Leu Gly Cys Val Pro Arg Trp Leu Leu Met Asn Arg Pro Pro
    370                 375                 380

Pro Pro Val Glu Leu Cys His Pro Leu Arg Leu Lys Leu Ser Pro Ser
385                 390                 395                 400

Tyr His Trp Leu Glu Ser Asn Val Asp Ala Glu Glu Arg Glu Val Val
                405                 410                 415

Val Glu Glu Glu Asp Arg Trp Ala Cys Ala Gly His Val Ala Pro Ser
            420                 425                 430

Val Gly Thr Leu Cys Ser His Gly His Leu His Ser Gly Ala Ser Gly
        435                 440                 445

Pro Lys Ala Glu Ala Leu Leu Gln Glu Gly Glu Leu Leu Leu Ser Pro
    450                 455                 460

His Met Gln Lys Ala Leu Glu Gly Val His Tyr Ile Ala Asp His Leu
465                 470                 475                 480

Arg Ser Glu Asp Ala Asp Ser Ser Val Lys Glu Asp Trp Lys Tyr Val
                485                 490                 495

Ala Met Val Ile Asp Arg Ile Phe Leu Trp Leu Phe Ile Ile Val Cys
            500                 505                 510

Phe Leu Gly Thr Ile Gly Leu Phe Leu Pro Pro Phe Leu Ala Gly Met
        515                 520                 525

Ile

<210>  103
<211>  1181
<212>  DNA
<213>  Homo sapiens

<400>  103
cacgaggctg gaaggggcca cttcacacct cgggctcggc ataaagcggc cgccggccgc      60

cggcccccag acgcgccgcc gctgccatgg cccagcccct gtgcccgccg ctctccgagt     120

cctggatgct ctctgcggcc tggggcccaa ctcggcggcc gccgccctcc gacaaggact     180

gcggccgctc cctcgtctcg tccccagact catggggcag caccccagcc gacagccccg     240

tggcgagccc cgcgcggcca ggcaccctcc gggacccccg cgccccctcc gtaggtaggc     300

gcggcgcgcg cagcagccgc ctgggcagcg ggcagaggca gagcgccagt gagcgggaga     360

aactgcgcat gcgcacgctg gcccgcgccc tgcacgagct gcgccgcttt ctaccgccgt     420

ccgtggcgcc cgcgggccag agcctgacca agatcgagac gctgcgcctg gctatccgct 480

atatcggcca cctgtcggcc gtgctaggcc tcagcgagga gagtctccag cgccggtgcc 540

ggcagcgcgg tgacgcgggg tcccctcggg gctgcccgct gtgccccgac gactgccccg 600

cgcagatgca gacacggacg caggctgagg ggcaggggca ggggcgcggg ctgggcctgg 660

tatccgccgt ccgcgccggg gcgtcctggg gatccccgcc tgcctgcccc ggagcccgag 720

ctgcacccga gccgcgcgac ccgcctgcgc tgttcgccga ggcggcgtgc ccggaagggc 780

aggcgatgga gccaagccca ccgtccccgc tccttccggg cgacgtgctg gctctgttgg 840

agacctggat gcccctctcg cctctggagt ggctgcctga ggagcccaag tgacaaggga 900

caactgacgc cgtctctgtg agcaccgagg ctttttggcc tcagcacctt cgaagtggtt 960

ccttggcaga ctgcctttcc tggaagaggg cacgggcgat cccgacgggg gcattcctgc 1020

gggtgagagc cgtccccacc gcggcggccc ttctcagccc ctccctccat ggagggaccc 1080

atagggctag acactttgag gcaagcagga ggctctgcct aatgtgaatt tatttatttg 1140

tgaataaact gtactggtgt caaaaaaaaa aaaaaaaaaa a 1181

<210> 104
<211> 268
<212> PRT
<213> Homo sapiens

<400> 104

Met Ala Gln Pro Leu Cys Pro Pro Leu Ser Glu Ser Trp Met Leu Ser
1               5                   10                  15

Ala Ala Trp Gly Pro Thr Arg Arg Pro Pro Pro Ser Asp Lys Asp Cys
            20                  25                  30

Gly Arg Ser Leu Val Ser Ser Pro Asp Ser Trp Gly Ser Thr Pro Ala
        35                  40                  45

Asp Ser Pro Val Ala Ser Pro Ala Arg Pro Gly Thr Leu Arg Asp Pro
    50                  55                  60

Arg Ala Pro Ser Val Gly Arg Arg Gly Ala Arg Ser Ser Arg Leu Gly
65                  70                  75                  80

Ser Gly Gln Arg Gln Ser Ala Ser Glu Arg Glu Lys Leu Arg Met Arg
                85                  90                  95

Thr Leu Ala Arg Ala Leu His Glu Leu Arg Arg Phe Leu Pro Pro Ser
            100                 105                 110

Val Ala Pro Ala Gly Gln Ser Leu Thr Lys Ile Glu Thr Leu Arg Leu
        115                 120                 125

Ala Ile Arg Tyr Ile Gly His Leu Ser Ala Val Leu Gly Leu Ser Glu
    130                 135                 140

Glu Ser Leu Gln Arg Arg Cys Arg Gln Arg Gly Asp Ala Gly Ser Pro
145                 150                 155                 160

Arg Gly Cys Pro Leu Cys Pro Asp Asp Cys Pro Ala Gln Met Gln Thr
                165                 170                 175

Arg Thr Gln Ala Glu Gly Gln Gly Gln Gly Arg Gly Leu Gly Leu Val
            180                 185                 190

Ser Ala Val Arg Ala Gly Ala Ser Trp Gly Ser Pro Pro Ala Cys Pro
        195                 200                 205

Gly Ala Arg Ala Ala Pro Glu Pro Arg Asp Pro Pro Ala Leu Phe Ala
    210                 215                 220

Glu Ala Ala Cys Pro Glu Gly Gln Ala Met Glu Pro Ser Pro Pro Ser
225                 230                 235                 240

Pro Leu Leu Pro Gly Asp Val Leu Ala Leu Leu Glu Thr Trp Met Pro
                245                 250                 255

Leu Ser Pro Leu Glu Trp Leu Pro Glu Glu Pro Lys
            260                 265

<210>  105
<211>  3810
<212>  DNA
<213>  Homo sapiens

<400>  105
ccacggagaa gcccaccgat gcctacggag agctggactt cacgggggcc ggccgcaagc      60
acagcaattt cctccggctc tctgaccgaa cggatccagc tgcagtttat agtctggtca     120
cacgcacatg gggcttccgt gccccgaacc tggtggtgtc agtgctgggg ggatcggggg     180
gccccgtcct ccagacctgg ctgcaggacc tgctgcgtcg tgggctggtg cgggctgccc     240
agagcacagg agcctggatt gtcactgggg gtctgcacac gggcatcggc cggcatgttg     300
gtgtggctgt acgggaccat cagatggcca gcactggggg caccaaggtg gtggccatgg     360
gtgtggcccc ctggggtgtg gtccggaata gagacaccct catcaacccc aagggctcgt     420
tccctgcgag gtaccggtgg cgcggtgacc cggaggacgg ggtccagttt cccctggact     480
acaactactc ggccttcttc ctggtggacg acggcacaca cggctgcctg gggggcgaga     540
accgcttccg cttgcgcctg gagtcctaca tctcacagca gaagacgggc gtgggaggga     600
ctggaattga catccctgtc ctgctcctcc tgattgatgg tgatgagaag atgttgacgc     660
gaatagagaa cgccacccag gctcagctcc catgtctcct cgtggctggc tcagggggag     720
ctgcggactg cctggcggag accctggaag acactctggc cccagggagt gggggagcca     780
ggcaaggcga agcccgagat cgaatcaggc gtttctttcc caaaggggac cttgaggtcc     840
tgcaggccca ggtggagagg attatgaccc ggaaggagct cctgacagtc tattcttctg     900
aggatgggtc tgaggaattc gagaccatag ttttgaaggc ccttgtgaag gcctgtggga     960
gctcggaggc ctcagcctac ctggatgagc tgcgtttggc tgtggcttgg aaccgcgtgg    1020
acattgccca gagtgaactc tttcgggggg acatccaatg gcggtccttc catctcgaag    1080
cttccctcat ggacgccctg ctgaatgacc ggcctgagtt cgtgcgcttg ctcatttccc    1140
acggcctcag cctgggccac ttcctgaccc cgatgcgcct ggcccaactc tacagcgcgg    1200
cgccctccaa ctcgctcatc cgcaaccttt tggaccaggc gtcccacagc gcaggcacca    1260
aagccccagc cctaaaaggg ggagctgcgg agctccggcc ccctgacgtg gggcatgtgc    1320
tgaggatgct gctggggaag atgtgcgcgc cgaggtaccc ctccgggggc gcctgggacc    1380

ctcacccagg ccagggcttc ggggagagca tgtatctgct ctcggacaag gccacctcgc 1440
cgctctcgct ggatgctggc ctcgggcagg ccccctggag cgacctgctt ctttgggcac 1500
tgttgctgaa cagggcacag atggccatgt acttctggga gatgggttcc aatgcagttt 1560
cctcagctct tggggcctgt ttgctgctcc gggtgatggc acgcctggag cctgacgctg 1620
aggaggcagc acggaggaaa gacctggcgt tcaagtttga ggggatgggc gttgacctct 1680
ttggcgagtg ctatcgcagc agtgaggtga gggctgcccg cctcctcctc cgtcgctgcc 1740
cgctctgggg ggatgccact tgcctccagc tggccatgca agctgacgcc cgtgccttct 1800
ttgcccagga tggggtacag tctctgctga cacagaagtg gtggggagat atggccagca 1860
ctacacccat ctgggccctg gttctcgcct tcttttgccc tccactcatc tacacccgcc 1920
tcatcacctt caggaaatca gaagaggagc ccacacggga ggagctagag tttgacatgg 1980
atagtgtcat taatggggaa gggcctgtcg ggacggcgga cccagccgag aagacgccgc 2040
tgggggtccc gcgccagtcg ggccgtccgg gttgctgcgg gggccgctgc ggggggcgcc 2100
ggtgcctacg ccgctggttc cacttctggg gcgcgccggt gaccatcttc atgggcaacg 2160
tggtcagcta cctgctgttc ttgctgcttt tctcgcgggt gctgctcgtg gatttccagc 2220
cggcgccgcc cggctccctg gagctgctgc tctatttctg ggctttcacg ctgctgtgcg 2280
aggaactgcg ccagggcctg agcggaggcg ggggcagcct cgccagcggg ggccccgggc 2340
ctggccatgc ctcactgagc cagcgcctgc gcctctacct cgccgacagc tggaaccagt 2400
gcgacctagt ggctctcacc tgcttcctcc tgggcgtggg ctgccggctg accccgggtt 2460
tgtaccacct gggccgcact gtcctctgca tcgacttcat ggttttcacg gtgcggctgc 2520
ttcacatctt cacggtcaac aaacagctgg ggcccaagat cgtcatcgtg agcaagatga 2580
tgaaggacgt gttcttcttc ctcttcttcc tcggcgtgtg gctggtagcc tatggcgtgg 2640
ccacggaggg gctcctgagg ccacgggaca gtgacttccc aagtatcctg cgccgcgtct 2700
tctaccgtcc ctacctgcag atcttcgggc agattcccca ggaggacatg gacgtggccc 2760
tcatggagca cagcaactgc tcgtcggagc ccggcttctg ggcacaccct cctggggccc 2820
aggcgggcac ctgcgtctcc cagtatgcca actggctggt ggtgctgctc ctcgtcatct 2880
tcctgctcgt ggccaacatc ctgctggtca acttgctcat tgccatgttc agttacacat 2940
tcggcaaagt acagggcaac agcgatctct actggaaggc gcagcgttac cgcctcatcc 3000
gggaattcca ctctcggccc gcgctggccc cgccctttat cgtcatctcc cacttgcgcc 3060
tcctgctcag gcaattgtgc aggcgacccc ggagccccca gccgtcctcc ccggccctcg 3120
agcatttccg ggtttacctt tctaaggaag ccgagcggaa gctgctaacg tgggaatcgg 3180
tgcataagga gaactttctg ctggcacgcg ctagggacaa gcgggagagc gactccgagc 3240
gtctggagcg cacgtcccag aaggtggact tggcactgaa acagctggga cacatccgcg 3300
agtacgaaca gcgcctgaaa gtgctggagc gggaggtcca gcagtgtagc cgcgtcctgg 3360
ggtgggtgac gtaggccgtt agcagctctg ccatgttgcc ctcaggtggg ccgccacccc 3420

ttgacctgca tgggtccaaa gagtgagcca tgctggcgga ttttaaggag aagcccccac 3480

aggggatttt gctcttagag taaggctcat gtgggcctcg gcccccgcac ctggtggcct 3540

tgtccttgag gtgagcccca tgtccatctg ggccactgtc aggaccacct ttgggagtgt 3600

catccttaca aaccacagca tgcccggctc ctcccagaac cagtcccagc ctgggaggat 3660

caaggcctgg atcccgggcc gttatccatc tggaggctgc agggtccttg gggtaacagg 3720

gaccacagac ccctcaccac tcacagattc ctcacactgg ggaaataaag ccatttcaga 3780

ggaaaaaaaa aaaaaaaaaa aaaaaaaaaa 3810

<210> 106
<211> 1016
<212> PRT
<213> Homo sapiens

<400> 106

Met Ala Ser Thr Gly Gly Thr Lys Val Val Ala Met Gly Val Ala Pro
1               5                   10                  15

Trp Gly Val Val Arg Asn Arg Asp Thr Leu Ile Asn Pro Lys Gly Ser
            20                  25                  30

Phe Pro Ala Arg Tyr Arg Trp Arg Gly Asp Pro Glu Asp Gly Val Gln
        35                  40                  45

Phe Pro Leu Asp Tyr Asn Tyr Ser Ala Phe Phe Leu Val Asp Asp Gly
    50                  55                  60

Thr His Gly Cys Leu Gly Gly Glu Asn Arg Phe Arg Leu Arg Leu Glu
65                  70                  75                  80

Ser Tyr Ile Ser Gln Gln Lys Thr Gly Val Gly Gly Thr Gly Ile Asp
                85                  90                  95

Ile Pro Val Leu Leu Leu Leu Ile Asp Gly Asp Glu Lys Met Leu Thr
            100                 105                 110

Arg Ile Glu Asn Ala Thr Gln Ala Gln Leu Pro Cys Leu Leu Val Ala
        115                 120                 125

Gly Ser Gly Gly Ala Ala Asp Cys Leu Ala Glu Thr Leu Glu Asp Thr
    130                 135                 140

Leu Ala Pro Gly Ser Gly Gly Ala Arg Gln Gly Glu Ala Arg Asp Arg
145                 150                 155                 160

Ile Arg Arg Phe Phe Pro Lys Gly Asp Leu Glu Val Leu Gln Ala Gln
                165                 170                 175

Val Glu Arg Ile Met Thr Arg Lys Glu Leu Leu Thr Val Tyr Ser Ser
            180                 185                 190

Glu Asp Gly Ser Glu Glu Phe Glu Thr Ile Val Leu Lys Ala Leu Val
        195                 200                 205

Lys Ala Cys Gly Ser Ser Glu Ala Ser Ala Tyr Leu Asp Glu Leu Arg
    210                 215                 220

Leu Ala Val Ala Trp Asn Arg Val Asp Ile Ala Gln Ser Glu Leu Phe
225                 230                 235                 240

Arg Gly Asp Ile Gln Trp Arg Ser Phe His Leu Glu Ala Ser Leu Met

                245                 250                 255

Asp Ala Leu Leu Asn Asp Arg Pro Glu Phe Val Arg Leu Leu Ile Ser
            260                 265                 270

His Gly Leu Ser Leu Gly His Phe Leu Thr Pro Met Arg Leu Ala Gln
        275                 280                 285

Leu Tyr Ser Ala Ala Pro Ser Asn Ser Leu Ile Arg Asn Leu Leu Asp
    290                 295                 300

Gln Ala Ser His Ser Ala Gly Thr Lys Ala Pro Ala Leu Lys Gly Gly
305                 310                 315                 320

Ala Ala Glu Leu Arg Pro Pro Asp Val Gly His Val Leu Arg Met Leu
                325                 330                 335

Leu Gly Lys Met Cys Ala Pro Arg Tyr Pro Ser Gly Gly Ala Trp Asp
            340                 345                 350

Pro His Pro Gly Gln Gly Phe Gly Glu Ser Met Tyr Leu Leu Ser Asp
        355                 360                 365

Lys Ala Thr Ser Pro Leu Ser Leu Asp Ala Gly Leu Gly Gln Ala Pro
    370                 375                 380

Trp Ser Asp Leu Leu Leu Trp Ala Leu Leu Leu Asn Arg Ala Gln Met
385                 390                 395                 400

Ala Met Tyr Phe Trp Glu Met Gly Ser Asn Ala Val Ser Ser Ala Leu
                405                 410                 415

Gly Ala Cys Leu Leu Leu Arg Val Met Ala Arg Leu Glu Pro Asp Ala
            420                 425                 430

Glu Glu Ala Ala Arg Arg Lys Asp Leu Ala Phe Lys Phe Glu Gly Met
        435                 440                 445

Gly Val Asp Leu Phe Gly Glu Cys Tyr Arg Ser Ser Glu Val Arg Ala
    450                 455                 460

Ala Arg Leu Leu Leu Arg Arg Cys Pro Leu Trp Gly Asp Ala Thr Cys
465                 470                 475                 480

Leu Gln Leu Ala Met Gln Ala Asp Ala Arg Ala Phe Phe Ala Gln Asp
                485                 490                 495

Gly Val Gln Ser Leu Leu Thr Gln Lys Trp Trp Gly Asp Met Ala Ser
            500                 505                 510

Thr Thr Pro Ile Trp Ala Leu Val Leu Ala Phe Phe Cys Pro Pro Leu
        515                 520                 525

Ile Tyr Thr Arg Leu Ile Thr Phe Arg Lys Ser Glu Glu Glu Pro Thr
    530                 535                 540

Arg Glu Glu Leu Glu Phe Asp Met Asp Ser Val Ile Asn Gly Glu Gly
545                 550                 555                 560

Pro Val Gly Thr Ala Asp Pro Ala Glu Lys Thr Pro Leu Gly Val Pro
                565                 570                 575

Arg Gln Ser Gly Arg Pro Gly Cys Cys Gly Gly Arg Cys Gly Gly Arg
            580                 585                 590

Arg Cys Leu Arg Arg Trp Phe His Phe Trp Gly Ala Pro Val Thr Ile
        595                 600                 605

Phe Met Gly Asn Val Val Ser Tyr Leu Leu Phe Leu Leu Leu Phe Ser
    610                 615                 620

Arg Val Leu Leu Val Asp Phe Gln Pro Ala Pro Pro Gly Ser Leu Glu
625                 630                 635                 640

Leu Leu Leu Tyr Phe Trp Ala Phe Thr Leu Leu Cys Glu Glu Leu Arg
                645                 650                 655

Gln Gly Leu Ser Gly Gly Gly Gly Ser Leu Ala Ser Gly Gly Pro Gly
            660                 665                 670

Pro Gly His Ala Ser Leu Ser Gln Arg Leu Arg Leu Tyr Leu Ala Asp
        675                 680                 685

Ser Trp Asn Gln Cys Asp Leu Val Ala Leu Thr Cys Phe Leu Leu Gly
    690                 695                 700

Val Gly Cys Arg Leu Thr Pro Gly Leu Tyr His Leu Gly Arg Thr Val
705                 710                 715                 720

Leu Cys Ile Asp Phe Met Val Phe Thr Val Arg Leu Leu His Ile Phe
                725                 730                 735

Thr Val Asn Lys Gln Leu Gly Pro Lys Ile Val Ile Val Ser Lys Met
            740                 745                 750

Met Lys Asp Val Phe Phe Phe Leu Phe Phe Leu Gly Val Trp Leu Val
        755                 760                 765

Ala Tyr Gly Val Ala Thr Glu Gly Leu Leu Arg Pro Arg Asp Ser Asp
    770                 775                 780

Phe Pro Ser Ile Leu Arg Arg Val Phe Tyr Arg Pro Tyr Leu Gln Ile
785                 790                 795                 800

Phe Gly Gln Ile Pro Gln Glu Asp Met Asp Val Ala Leu Met Glu His
                805                 810                 815

Ser Asn Cys Ser Ser Glu Pro Gly Phe Trp Ala His Pro Pro Gly Ala
            820                 825                 830

Gln Ala Gly Thr Cys Val Ser Gln Tyr Ala Asn Trp Leu Val Val Leu
        835                 840                 845

Leu Leu Val Ile Phe Leu Leu Val Ala Asn Ile Leu Leu Val Asn Leu
    850                 855                 860

Leu Ile Ala Met Phe Ser Tyr Thr Phe Gly Lys Val Gln Gly Asn Ser
865                 870                 875                 880

Asp Leu Tyr Trp Lys Ala Gln Arg Tyr Arg Leu Ile Arg Glu Phe His
                885                 890                 895

Ser Arg Pro Ala Leu Ala Pro Pro Phe Ile Val Ile Ser His Leu Arg
            900                 905                 910

Leu Leu Leu Arg Gln Leu Cys Arg Arg Pro Arg Ser Pro Gln Pro Ser
        915                 920                 925

Ser Pro Ala Leu Glu His Phe Arg Val Tyr Leu Ser Lys Glu Ala Glu
    930                 935                 940

Arg Lys Leu Leu Thr Trp Glu Ser Val His Lys Glu Asn Phe Leu Leu
945                 950                 955                 960

Ala Arg Ala Arg Asp Lys Arg Glu Ser Asp Ser Glu Arg Leu Glu Arg
                965                 970                 975

Thr Ser Gln Lys Val Asp Leu Ala Leu Lys Gln Leu Gly His Ile Arg
            980                 985                 990

Glu Tyr Glu Gln Arg Leu Lys Val Leu Glu Arg Glu Val Gln Gln Cys
        995                 1000                1005

Ser Arg Val Leu Gly Trp Val Thr
    1010                1015

<210> 107
<211> 1378
<212> DNA
<213> Homo sapiens

<400> 107
ggtagcagca tccaccgggc gggaggtcgg aggcagcaag gccttaaagg ctactgagtg      60

cgccggccgt tccgtgtcca gaacctcccc tactcctccg ccttctcttc cttggccgcc     120

caccgccaag ttccgactcc ggttttcgcc tttgcaaagc ctaaggagga ggttaggaac     180

agccgcgccc ccctccctgc ggccgccgcc ccctgcctct cggctctgct ccctgccgcg     240

tgcgcctggg ccgtgcgccc cggcaggcgc cagccatgtc gatgctgccg tcgtttggct     300

ttacgcagga gcaagtggcg tgcgtgtgcg aggttctgca gcaaggcgga aacctggagc     360

gcctgggcag gttcctgtgg tcactgcccg cctgcgacca cctgcacaag aacgagagcg     420

tactcaaggc caaggcggtg gtcgccttcc accgcggcaa cttccgtgag ctctacaaga     480

tcctggagag ccaccagttc tcgcctcaca accaccccaa actgcagcaa ctgtggctga     540

aggcgcatta cgtggaggcc gagaagctgc gcggccgacc cctgggcgcc gtgggcaaat     600

atcgggtgcg ccgaaaattt ccactgccgc gcaccatctg ggacggcgag gagaccagct     660

actgcttcaa ggagaagtcg aggggtgtcc tgcgggagtg gtacgcgcac aatccctacc     720

catcgccgcg tgagaagcgg gagctggccg aggccaccgg cctcaccacc acccaggtca     780

gcaactggtt taagaaccgg aggcaaagag accgggccgc ggaggccaag gaaagggaga     840

acaccgaaaa caataactcc tcctccaaca agcagaacca actctctcct ctggaagggg     900

gcaagccgct catgtccagc tcagaagagg aattctcacc tccccaaagt ccagaccaga     960

actcggtcct tctgctgcag ggcaatatgg gccacgccag gagctcaaac tattctctcc    1020

cgggcttaac agcctcgcag cccagtcacg gcctgcagac ccaccagcat cagctccaag    1080

actctctgct cggccccctc acctccagtc tggtggactt ggggtcctaa gtggggaggg    1140

actggggcct cgaagggatt cctggagcag caaccactgc agcgactagg gacacttgta    1200

aatagaaatc aggaacattt ttgcagcttg tttctggagt tgtttgcgca taaaggaatg    1260

gtggactttc acaaatatct ttttaaaaat caaaaccaac agcgatctca agcttaatct    1320

cctcttctct ccaactcttt ccacttttgc attttccttc ccaatgcaga gatcaggg       1378

<210> 108
<211> 284
<212> PRT
<213> Homo sapiens

<400> 108

<210>  109
<211>  3885
<212>  DNA
<213>  Homo sapiens

<400>  109
ggggctccgc gggcctggag cacggccggg tctaatatgc ccggagccga ggcgcgatga      60

aggagaagtc caagaatgcg gccaagacca ggagggagaa ggaaaatggc gagttttacg     120

agcttgccaa gctgctcccg ctgccgtcgg ccatcacttc gcagctggac aaagcgtcca     180

tcatccgcct caccacgagc tacctgaaga tgcgcgccgt cttccccgaa ggtttaggag     240

acgcgtgggg acagccgagc cgcgccgggc ccctggacgg cgtcgccaag gagctgggat 300
cgcacttgct gcagactttg gatggatttg tttttgtggt agcatctgat ggcaaaatca 360
tgtatatatc cgagaccgct tctgtccatt taggcttatc ccaggtggag ctcacgggca 420
acagtattta tgaatacatc catccttctg accacgatga gatgaccgct gtcctcacgg 480
cccaccagcc gctgcaccac cacctgctcc aagagtatga gatagagagg tcgttctttc 540
ttcgaatgaa atgtgtcttg gcgaaaagga acgcgggcct gacctgcagc ggatacaagg 600
tcatccactg cagtggctac ttgaagatca ggcagtatat gctggacatg tccctgtacg 660
actcctgcta ccagattgtg gggctggtgg ccgtgggcca gtcgctgcca cccagtgcca 720
tcaccgagat caagctgtac agtaacatgt tcatgttcag ggccagcctt gacctgaagc 780
tgatattcct ggattccagg gtgaccgagg tgacgggtta cgagccgcag gacctgatcg 840
agaagaccct ataccatcac gtgcacggct gcgacgtgtt ccacctccgc tacgcacacc 900
acctcctgtt ggtgaagggc caggtcacca ccaagtacta ccggctgctg tccaagcggg 960
gcggctgggt gtgggtgcag agctacgcca ccgtggtgca caacagccgc tcgtcccggc 1020
cccactgcat cgtgagtgtc aattatgtac tcacggagat tgaatacaag gaacttcagc 1080
tgtccctgga gcaggtgtcc actgccaagt cccaggactc ctggaggacc gccttgtcta 1140
cctcacaaga aactaggaaa ttagtgaaac ccaaaaatac caagatgaag acaaagctga 1200
gaacaaaccc ttacccccca cagcaataca gctcgttcca aatggacaaa ctggaatgcg 1260
gccagctcgg aaactggaga gccagtcccc ctgcaagcgc tgctgctcct ccagaactgc 1320
agccccactc agaaagcagt gaccttctgt acacgccatc ctacagcctg cccttctcct 1380
accattacgg acacttccct ctggactctc acgtcttcag cagcaaaaag ccaatgttgc 1440
cggccaagtt cgggcagccc caaggatccc cttgtgaggt ggcacgcttt ttcctgagca 1500
cactgccagc cagcggtgaa tgccagtggc attatgccaa ccccctagtg cctagcagct 1560
cgtctccagc taaaaatcct ccagagccac cggcgaacac tgctaggcac agcctggtgc 1620
caagctacga agcgcccgcc gccgccgtgc gcaggttcgg cgaggacacc gcgcccccga 1680
gcttcccgag ctgcggccac taccgcgagg agcccgcgct gggcccggcc aaagccgccc 1740
gccaggccgc ccgggacggg gcgcggctgg cgctggcccg cgcggcaccc gagtgctgcg 1800
cgcccccgac ccccgaggcc ccgggcgcgc cggcgcagct gcccttcgtg ctgctcaact 1860
accaccgcgt gctggcccgg cgcggaccgc tggggggcgc cgcacccgcc gcctccggcc 1920
tggcctgcgc tcccggcggc cccgaggcgg cgaccggcgc gctgcggctc cggcacccga 1980
gccccgccgc cacctccccg cccggcgcgc ccctgccgca ctacctgggc gcctcggtca 2040
tcatcaccaa cgggaggtga cccgctggcc gcccgcgcca ggagcctgga cccggcctcc 2100
cggggctgcg gcgccaccga gcccggcaaa tgcgcacgac ctacattaat ttatgcagag 2160
acagctgttt gaattggacc ccgccgccga cttgcggatt tccaccgcgg aggccccgcg 2220
cgccggtgcc gagggccgag gagcgcccgg gtccgggcag gtgaccgccc gcctctgtcc 2280

tgcgagggcc ggtgcgaccc agttgctggg ggcttggttt cctcaccttg aaatcgggct 2340
tcacgcgtct tgccttgtcc ccaacgttcc acaacagtcc cgctggggga ttgaagcggt 2400
ttcactccgc aaatatcctc cactttcagg agggaaaacc caccctacca cagtccgctc 2460
ttccaagtgg acggcagacc tgggagggga cgcctgtgtc acgagccctt ttagatgctt 2520
aggtgaaggc agaagtgatg attgtaagtc ccatgaatac acaactccac tgtctttaaa 2580
agtcattcaa gagtctcatt atttttgttt ttatttaacc ctttcttcaa tacaaaaagc 2640
caacaaacca agactaaggg ggtgaccatg caattccatt ttgtgtctgt gaacataggt 2700
gtgcttccca aatacattaa caagctctta cttcccccta acccctatga actcttgata 2760
acaccaagag tagcaccttc agaatatatt gaataggcat taaatgcaaa aatatatatg 2820
tagccagaca gtttatgaga atgaccctgt caagcttcat tattacgtgg caaaatccct 2880
ctggcccaca cagatctgta attcactagg ctcgtgtttg ctacaaatag tgctaataaa 2940
gttaaattgc acgtgcaata cggaacactg tcaatggact gcaccttgtg aaggaaaaac 3000
atgcttaagg gggtgtaatg aaaatgatgt agacatttta agcattttct acacagcgag 3060
aaaacttcgt aagaacatgt tacgtgtgca acaggtaaac agaaatcctt tcataaagca 3120
ccagcagtgt ttaaaaaatg agcttccatt aatttttact ttttatgggt tttgcttaaa 3180
gatctcaaca tggaaaaatc ctgtcatggc tctgaactgc acaatgcatt gaaccgccgt 3240
ccttcaattt tcttcacact atcaacactg cagcattttg ctgctttatc aaaatggttt 3300
attttaggaa actttttcca cctttctgaa tggaaagagg ttttcacaaa tgttttaaac 3360
tcatcgttct aaaatcaagt gcacctacac caactgctct caaaatgtga actgactttt 3420
tttttttttt ttttgccaac cctgtgtcac ttagtgagga cctgacacaa tccctacagg 3480
gtgtctgtca gtgggcctca tggtaagagt cacaatttgc aaatttagga ccgtgggtca 3540
tgcagcgaag gggctggatg gtaggaaggg atgtgcccgc ctctccacgc actcagctat 3600
acctcattca cagctccttg tgagtgtgtg cacaggaaat aagccgaggg tattattttt 3660
ttatgttcat gagtcttgta attaaaccgt gattcttgaa aggtgtaggt ttgattacta 3720
ggagatacca ccgacatttt tcaataaagt actgcaaaat gcttttgtgt ctaccttgtt 3780
attaactttt ggggctgtat ttagtaaaaa taaatcaagg ctatcggagc agttcaataa 3840
caaaggttac tgttgagaaa aaagacccta tcatagattt acaag 3885

<210> 110
<211> 667
<212> PRT
<213> Homo sapiens

<400> 110

Met Lys Glu Lys Ser Lys Asn Ala Ala Lys Thr Arg Arg Glu Lys Glu
1               5                   10                  15

Asn Gly Glu Phe Tyr Glu Leu Ala Lys Leu Leu Pro Leu Pro Ser Ala
            20                  25                  30

Ile Thr Ser Gln Leu Asp Lys Ala Ser Ile Ile Arg Leu Thr Thr Ser
        35                  40                  45

Tyr Leu Lys Met Arg Ala Val Phe Pro Glu Gly Leu Gly Asp Ala Trp
    50                  55                  60

Gly Gln Pro Ser Arg Ala Gly Pro Leu Asp Gly Val Ala Lys Glu Leu
65                  70                  75                  80

Gly Ser His Leu Leu Gln Thr Leu Asp Gly Phe Val Phe Val Val Ala
                85                  90                  95

Ser Asp Gly Lys Ile Met Tyr Ile Ser Glu Thr Ala Ser Val His Leu
            100                 105                 110

Gly Leu Ser Gln Val Glu Leu Thr Gly Asn Ser Ile Tyr Glu Tyr Ile
        115                 120                 125

His Pro Ser Asp His Asp Glu Met Thr Ala Val Leu Thr Ala His Gln
    130                 135                 140

Pro Leu His His His Leu Leu Gln Glu Tyr Glu Ile Glu Arg Ser Phe
145                 150                 155                 160

Phe Leu Arg Met Lys Cys Val Leu Ala Lys Arg Asn Ala Gly Leu Thr
                165                 170                 175

Cys Ser Gly Tyr Lys Val Ile His Cys Ser Gly Tyr Leu Lys Ile Arg
            180                 185                 190

Gln Tyr Met Leu Asp Met Ser Leu Tyr Asp Ser Cys Tyr Gln Ile Val
        195                 200                 205

Gly Leu Val Ala Val Gly Gln Ser Leu Pro Pro Ser Ala Ile Thr Glu
    210                 215                 220

Ile Lys Leu Tyr Ser Asn Met Phe Met Phe Arg Ala Ser Leu Asp Leu
225                 230                 235                 240

Lys Leu Ile Phe Leu Asp Ser Arg Val Thr Glu Val Thr Gly Tyr Glu
                245                 250                 255

Pro Gln Asp Leu Ile Glu Lys Thr Leu Tyr His His Val His Gly Cys
            260                 265                 270

Asp Val Phe His Leu Arg Tyr Ala His His Leu Leu Leu Val Lys Gly
        275                 280                 285

Gln Val Thr Thr Lys Tyr Tyr Arg Leu Leu Ser Lys Arg Gly Gly Trp
    290                 295                 300

Val Trp Val Gln Ser Tyr Ala Thr Val Val His Asn Ser Arg Ser Ser
305                 310                 315                 320

Arg Pro His Cys Ile Val Ser Val Asn Tyr Val Leu Thr Glu Ile Glu
                325                 330                 335

Tyr Lys Glu Leu Gln Leu Ser Leu Glu Gln Val Ser Thr Ala Lys Ser
            340                 345                 350

Gln Asp Ser Trp Arg Thr Ala Leu Ser Thr Ser Gln Glu Thr Arg Lys
        355                 360                 365

Leu Val Lys Pro Lys Asn Thr Lys Met Lys Thr Lys Leu Arg Thr Asn
    370                 375                 380

Pro Tyr Pro Pro Gln Gln Tyr Ser Ser Phe Gln Met Asp Lys Leu Glu
385                 390                 395                 400

Cys Gly Gln Leu Gly Asn Trp Arg Ala Ser Pro Pro Ala Ser Ala Ala
                405                 410                 415

Ala Pro Pro Glu Leu Gln Pro His Ser Glu Ser Ser Asp Leu Leu Tyr
            420                 425                 430

Thr Pro Ser Tyr Ser Leu Pro Phe Ser Tyr His Tyr Gly His Phe Pro
        435                 440                 445

Leu Asp Ser His Val Phe Ser Ser Lys Lys Pro Met Leu Pro Ala Lys
    450                 455                 460

Phe Gly Gln Pro Gln Gly Ser Pro Cys Glu Val Ala Arg Phe Phe Leu
465                 470                 475                 480

Ser Thr Leu Pro Ala Ser Gly Glu Cys Gln Trp His Tyr Ala Asn Pro
                485                 490                 495

Leu Val Pro Ser Ser Ser Ser Pro Ala Lys Asn Pro Pro Glu Pro Pro
            500                 505                 510

Ala Asn Thr Ala Arg His Ser Leu Val Pro Ser Tyr Glu Ala Pro Ala
        515                 520                 525

Ala Ala Val Arg Arg Phe Gly Glu Asp Thr Ala Pro Pro Ser Phe Pro
    530                 535                 540

Ser Cys Gly His Tyr Arg Glu Glu Pro Ala Leu Gly Pro Ala Lys Ala
545                 550                 555                 560

Ala Arg Gln Ala Ala Arg Asp Gly Ala Arg Leu Ala Leu Ala Arg Ala
                565                 570                 575

Ala Pro Glu Cys Cys Ala Pro Pro Thr Pro Glu Ala Pro Gly Ala Pro
            580                 585                 590

Ala Gln Leu Pro Phe Val Leu Leu Asn Tyr His Arg Val Leu Ala Arg
        595                 600                 605

Arg Gly Pro Leu Gly Gly Ala Ala Pro Ala Ala Ser Gly Leu Ala Cys
    610                 615                 620

Ala Pro Gly Gly Pro Glu Ala Ala Thr Gly Ala Leu Arg Leu Arg His
625                 630                 635                 640

Pro Ser Pro Ala Ala Thr Ser Pro Pro Gly Ala Pro Leu Pro His Tyr
                645                 650                 655

Leu Gly Ala Ser Val Ile Ile Thr Asn Gly Arg
            660                 665

<210>  111
<211>  1804
<212>  DNA
<213>  Homo sapiens

<400>  111
atgaacggac gctgcatctg cccgtccctg ccctactcac ccgtcagctc cccgcagtcc      60

tcgcctcggc tgccccggcg gccgacagtg gagtctcacc acgtctccat cacgggtatg     120

caggactgtg tgcagctgaa tcagtatacc ctgaaggatg aaattggaaa gggctcctat     180

ggtgtcgtca agttggccta caatgaaaat gacaatacct actatgcaat gaaggtgctg     240

tccaaaaaga agctgatccg gcaggccggc tttccacgtc gccctccacc ccgaggcacc     300

cggccagctc ctggaggctg catccagccc aggggcccca ttgagcaggt gtaccaggaa 360
attgccatcc tcaagaagct ggaccacccc aatgtggtga agctggtgga ggtcctggat 420
gaccccaatg aggaccatct gtacatggtg ttcgaactgg tcaaccaagg gcccgtgatg 480
gaagtgccca ccctcaaacc actctctgaa gaccaggccc gtttctactt ccaggatctg 540
atcaaaggca tcgagtactt acactaccag aagatcatcc accgtgacat caaaccttcc 600
aacctcctgg tcggagaaga tgggcacatc aagatcgctg actttggtgt gagcaatgaa 660
ttcaagggca gtgacgcgct cctctccaac accgtgggca cgcccgcctt catggcaccc 720
gagtcgctct ctgagacccg caagatcttc tctgggaagg ccttggatgt ttgggccatg 780
ggtgtgacac tatactgctt tgtctttggc cagtgcccat tcatggacga gcggatcatg 840
tgtttacaca gtaagatcaa gagtcaggcc ctggaatttc cagaccagcc cgacatagct 900
gaggacttga aggacctgat cacccgtatg ctggacaaga accccgagtc gaggatcgtg 960
gtgccggaaa tcaagctgca cccctgggtc acgaggcatg gggcggagcc gttgccgtcg 1020
gaggatgaga actgcacgct ggtcgaagtg actgaagagg aggtcgagaa ctcagtcaaa 1080
cacattccca gcttggcaac cgtgatcctg gtgaagacca tgatacgtaa acgctccttt 1140
gggaacccat tcgagggcag ccggcgggag gaacgctcac tgtcagcgcc tggaaacttg 1200
ctcaccaaaa aaccaaccag ggaatgtgag tccctgtctg agctcaagac ctagaaaata 1260
agtccccttc ctgcctgttg caaagtaacg taagagttcc ctcacccgag tggatgcaga 1320
cgttcttgct gtcagccacc ttccttcata cacatagcca gcccagggtg accagaacgt 1380
cccaggacag atgaggcttt gtgtccttat gagagtggga gaacctggtg ggcacccctg 1440
gtgcaggtgc tgtggtgggt ggggacccca ctgcctttcc cactgagcac atcatggcta 1500
cctgacttgg tgggagttcc attcagtcac ttctgtttct taaacatagc tttactgagg 1560
tacaattcac ataccatgta attcacccac gggaagtgta tgattcagtg gtttctaata 1620
cacacttctg cagccattac caccgtcaac tttacgacat tttcatcagc ccaagaagac 1680
accctacact ccttagctgt ccccatccaa ctcccccacc ccagtaacca ctcagaatag 1740
gtatggattt gcctattctg gacgtttcgt ataaatggcg tcatacacta aaaaaaaaaa 1800
aaaa 1804

<210> 112
<211> 417
<212> PRT
<213> Homo sapiens

<400> 112

Met Asn Gly Arg Cys Ile Cys Pro Ser Leu Pro Tyr Ser Pro Val Ser
1               5                   10                  15

Ser Pro Gln Ser Ser Pro Arg Leu Pro Arg Arg Pro Thr Val Glu Ser
            20                  25                  30

His His Val Ser Ile Thr Gly Met Gln Asp Cys Val Gln Leu Asn Gln
        35                  40                  45

Tyr Thr Leu Lys Asp Glu Ile Gly Lys Gly Ser Tyr Gly Val Val Lys
    50                  55                  60

Leu Ala Tyr Asn Glu Asn Asp Asn Thr Tyr Tyr Ala Met Lys Val Leu
65                  70                  75                  80

Ser Lys Lys Lys Leu Ile Arg Gln Ala Gly Phe Pro Arg Arg Pro Pro
                85                  90                  95

Pro Arg Gly Thr Arg Pro Ala Pro Gly Gly Cys Ile Gln Pro Arg Gly
            100                 105                 110

Pro Ile Glu Gln Val Tyr Gln Glu Ile Ala Ile Leu Lys Lys Leu Asp
        115                 120                 125

His Pro Asn Val Val Lys Leu Val Glu Val Leu Asp Asp Pro Asn Glu
    130                 135                 140

Asp His Leu Tyr Met Val Phe Glu Leu Val Asn Gln Gly Pro Val Met
145                 150                 155                 160

Glu Val Pro Thr Leu Lys Pro Leu Ser Glu Asp Gln Ala Arg Phe Tyr
                165                 170                 175

Phe Gln Asp Leu Ile Lys Gly Ile Glu Tyr Leu His Tyr Gln Lys Ile
            180                 185                 190

Ile His Arg Asp Ile Lys Pro Ser Asn Leu Leu Val Gly Glu Asp Gly
        195                 200                 205

His Ile Lys Ile Ala Asp Phe Gly Val Ser Asn Glu Phe Lys Gly Ser
    210                 215                 220

Asp Ala Leu Leu Ser Asn Thr Val Gly Thr Pro Ala Phe Met Ala Pro
225                 230                 235                 240

Glu Ser Leu Ser Glu Thr Arg Lys Ile Phe Ser Gly Lys Ala Leu Asp
                245                 250                 255

Val Trp Ala Met Gly Val Thr Leu Tyr Cys Phe Val Phe Gly Gln Cys
            260                 265                 270

Pro Phe Met Asp Glu Arg Ile Met Cys Leu His Ser Lys Ile Lys Ser
        275                 280                 285

Gln Ala Leu Glu Phe Pro Asp Gln Pro Asp Ile Ala Glu Asp Leu Lys
    290                 295                 300

Asp Leu Ile Thr Arg Met Leu Asp Lys Asn Pro Glu Ser Arg Ile Val
305                 310                 315                 320

Val Pro Glu Ile Lys Leu His Pro Trp Val Thr Arg His Gly Ala Glu
                325                 330                 335

Pro Leu Pro Ser Glu Asp Glu Asn Cys Thr Leu Val Glu Val Thr Glu
            340                 345                 350

Glu Glu Val Glu Asn Ser Val Lys His Ile Pro Ser Leu Ala Thr Val
        355                 360                 365

Ile Leu Val Lys Thr Met Ile Arg Lys Arg Ser Phe Gly Asn Pro Phe
    370                 375                 380

Glu Gly Ser Arg Arg Glu Glu Arg Ser Leu Ser Ala Pro Gly Asn Leu
385                 390                 395                 400

Leu Thr Lys Lys Pro Thr Arg Glu Cys Glu Ser Leu Ser Glu Leu Lys
                405                 410                 415

Thr

<210> 113
<211> 429
<212> DNA
<213> Homo sapiens

<400> 113
atgaaacctc tgatatggac atggtcagat gttgaaggcc agaggccggc tctgctcatc 60

tgcacagctg cagcaggacc cacgcaggga gttaagggtt atggcaagcc ctttgagcca 120

agaagtgtga aaaacataca ctctactcct gcttacccag atgccacaat gcacagacaa 180

ctcctggctc cggtggaagg aaggatggca gagacattga atcagaaact ccatgttgcc 240

aatgtgctgg aagatgaccc cggctaccta cctcacgtct acagcgagga aggggagtgt 300

ggaggggccc catccctcag ctctctggcc agcttggaac aggagttgca acctgatttg 360

ctggactctt tgggttcaaa agcgactccg tttgaggaaa tatattcaga gtcaggtgtt 420

ccttcctaa 429

<210> 114
<211> 142
<212> PRT
<213> Homo sapiens

<400> 114

Met Lys Pro Leu Ile Trp Thr Trp Ser Asp Val Glu Gly Gln Arg Pro
1               5                   10                  15

Ala Leu Leu Ile Cys Thr Ala Ala Ala Gly Pro Thr Gln Gly Val Lys
            20                  25                  30

Gly Tyr Gly Lys Pro Phe Glu Pro Arg Ser Val Lys Asn Ile His Ser
        35                  40                  45

Thr Pro Ala Tyr Pro Asp Ala Thr Met His Arg Gln Leu Leu Ala Pro
    50                  55                  60

Val Glu Gly Arg Met Ala Glu Thr Leu Asn Gln Lys Leu His Val Ala
65                  70                  75                  80

Asn Val Leu Glu Asp Asp Pro Gly Tyr Leu Pro His Val Tyr Ser Glu
                85                  90                  95

Glu Gly Glu Cys Gly Gly Ala Pro Ser Leu Ser Ser Leu Ala Ser Leu
            100                 105                 110

Glu Gln Glu Leu Gln Pro Asp Leu Leu Asp Ser Leu Gly Ser Lys Ala
        115                 120                 125

Thr Pro Phe Glu Glu Ile Tyr Ser Glu Ser Gly Val Pro Ser
    130                 135                 140

<210> 115
<211> 1270
<212> DNA
<213> Homo sapiens

<400> 115
   cgaatgcagg cgacttgcga gctgggagcg atttaaaacg ctttggattc ccccggcctg 60
   ggtggggaga gcgagctggg tgccccctag attccccgcc cccgcacctc atgagccgac 120
   cctcggctcc atggagcccg gcaattatgc caccttggat ggagccaagg atatcgaagg 180
   cttgctggga gcgggagggg ggcggaatct ggtcgcccac tcccctctga ccagccaccc 240
   agcggcgcct acgctgatgc ctgctgtcaa ctatgccccc ttggatctgc caggctcggc 300
   ggagccgcca aagcaatgcc acccatgccc tggggtgccc caggggacgt ccccagctcc 360
   cgtgccttat ggttactttg gaggcgggta ctactcctgc cgagtgtccc ggagctcgct 420
   gaaaccctgt gcccaggcag ccaccctggc cgcgtacccc gcggagactc ccacggccgg 480
   ggaagagtac cccagtcgcc ccactgagtt tgccttctat ccgggatatc cgggaaccta 540
   ccacgctatg gccagttacc tggacgtgtc tgtggtgcag actctgggtg ctcctggaga 600
   accgcgacat gactccctgt tgcctgtgga cagttaccag tcttgggctc tcgctggtgg 660
   ctggaacagc cagatgtgtt gccagggaga acagaaccca ccaggtccct tttggaaggc 720
   agcatttgca gactccagcg ggcagcaccc tcctgacgcc tgcgcctttc gtcgcggccg 780
   caagaaacgc attccgtaca gcaaggggca gttgcgggag ctggagcggg agtatgcggc 840
   taacaagttc atcaccaagg acaagaggcg caagatctcg gcagccacca gcctctcgga 900
   gcgccagatt accatctggt ttcagaaccg ccgggtcaaa gagaagaagg ttctcgccaa 960
   ggtgaagaac agcgctaccc cttaagagat ctccttgcct gggtgggagg agcgaaagtg 1020
   ggggtgtcct ggggagacca gaaacctgcc aagcccaggc tggggccaag gactctgctg 1080
   agaggcccct agagacaaca cccttcccag gccactggct gctggactgt tcctcaggag 1140
   cggcctgggt acccagtatg tgcagggaga cggaacccca tgtgacaggc ccactccacc 1200
   agggttccca aagaacctgg cccagtcata atcattcatc ctcacagtgg caataatcac 1260
   gataaccagt 1270

<210> 116
<211> 284
<212> PRT
<213> Homo sapiens

<400> 116

Met Glu Pro Gly Asn Tyr Ala Thr Leu Asp Gly Ala Lys Asp Ile Glu
1               5                   10                  15

Gly Leu Leu Gly Ala Gly Gly Gly Arg Asn Leu Val Ala His Ser Pro
            20                  25                  30

Leu Thr Ser His Pro Ala Ala Pro Thr Leu Met Pro Ala Val Asn Tyr
        35                  40                  45

Ala Pro Leu Asp Leu Pro Gly Ser Ala Glu Pro Pro Lys Gln Cys His
    50                  55                  60

Pro Cys Pro Gly Val Pro Gln Gly Thr Ser Pro Ala Pro Val Pro Tyr
65                  70                  75                  80

Gly Tyr Phe Gly Gly Gly Tyr Tyr Ser Cys Arg Val Ser Arg Ser Ser
                85                  90                  95

Leu Lys Pro Cys Ala Gln Ala Ala Thr Leu Ala Ala Tyr Pro Ala Glu

100                 105                 110

Thr Pro Thr Ala Gly Glu Glu Tyr Pro Ser Arg Pro Thr Glu Phe Ala
        115                 120                 125

Phe Tyr Pro Gly Tyr Pro Gly Thr Tyr His Ala Met Ala Ser Tyr Leu
    130                 135                 140

Asp Val Ser Val Val Gln Thr Leu Gly Ala Pro Gly Glu Pro Arg His
145                 150                 155                 160

Asp Ser Leu Leu Pro Val Asp Ser Tyr Gln Ser Trp Ala Leu Ala Gly
                165                 170                 175

Gly Trp Asn Ser Gln Met Cys Cys Gln Gly Glu Gln Asn Pro Pro Gly
            180                 185                 190

Pro Phe Trp Lys Ala Ala Phe Ala Asp Ser Ser Gly Gln His Pro Pro
        195                 200                 205

Asp Ala Cys Ala Phe Arg Arg Gly Arg Lys Lys Arg Ile Pro Tyr Ser
    210                 215                 220

Lys Gly Gln Leu Arg Glu Leu Glu Arg Glu Tyr Ala Ala Asn Lys Phe
225                 230                 235                 240

Ile Thr Lys Asp Lys Arg Arg Lys Ile Ser Ala Ala Thr Ser Leu Ser
                245                 250                 255

Glu Arg Gln Ile Thr Ile Trp Phe Gln Asn Arg Arg Val Lys Glu Lys
            260                 265                 270

Lys Val Leu Ala Lys Val Lys Asn Ser Ala Thr Pro
        275                 280

<210>  117
<211>  2856
<212>  DNA
<213>  Homo sapiens

<400>  117
gtaaccgcta ctcccggaca ccagaccacc gccttccgta cacaggggcc cgcatcccac      60

cctcccggac ctaagagcct gggtcccctg tttccggagg tccgcttccc ggcccccaga     120

ttctggcatc ccagccctca gtgtccaaga cccaggcagc ccgggtcccc gcctcccgga     180

tccaggcgtc cgggatctgc gccaccagaa cctagcctcc tgcagacctc cgccatctgg     240

gggcactcaa cctcctggag ccaagggccc cacgtcccac ccagagaaac tctcgtattc     300

ccagctccta gggccaagga acccgggcgc tccgaactcc cagctttcgg acatctggca     360

cacggggcag agcagagaag ctcagcgccc agcctgggga atttaaacac tccagcttcc     420

aagagccaag gaacttcagt gctgtgaact cacaactcta aggagccctc caaagttcca     480

gtctccaggt gctgttactc aactcagtcc taggaacgtc gggtcctggg aaggagccca     540

agcgctccca gccagcttcc aggcgctaag aaaccccggt gcttcccatc atggtggccg     600

atcctcctcg agactccaag gggctcgcag cggcggagcc caccgccaac gggggcctgg     660

cgctggcctc catcgaggac caaggcgcgg cagcaggcgg ctactgcggt tcccgggacc     720

aggtgcgccg ctgccttcga gccaacctgc ttgtgctgct gacagtggtg gccgtggtgg     780

ccggcgtggc gctgggactg ggggtgtcgg gggccggggg tgcgctggcg ttgggcccgg     840

agcgcttgag cgccttcgtc ttcccgggcg agctgctgct gcgtctgctg cggatgatca 900
tcttgccgct ggtggtgtgc agcttgatcg gcggcgccgc cagcctggac cccggcgcgc 960
tcggccgtct gggcgcctgg gcgctgctct ttttcctggt caccacgctg ctggcgtcgg 1020
cgctcggagt gggcttggcg ctggctctgc agccgggcgc cgcctccgcc gccatcaacg 1080
cctccgtggg agccgcgggc agtgccgaaa atgcccccag caaggaggtg ctcgattcgt 1140
tcctggatct tgcgagaaat atcttccctt ccaacctggt gtcagcagcc tttcgctcat 1200
actctaccac ctatgaagag aggaatatca ccggaaccag ggtgaaggtg cccgtggggc 1260
aggaggtgga ggggatgaac atcctgggct tggtagtgtt tgccatcgtc tttggtgtgg 1320
cgctgcggaa gctggggcct gaaggggagc tgcttatccg cttcttcaac tccttcaatg 1380
aggccaccat ggttctggtc tcctggatca tgtggtacgc ccctgtgggc atcatgttcc 1440
tggtggctgg caagatcgtg gagatggagg atgtgggttt actctttgcc cgccttggca 1500
agtacattct gtgctgcctg ctgggtcacg ccatccatgg gctcctggta ctgcccctca 1560
tctacttcct cttcacccgc aaaaacccct accgcttcct gtggggcatc gtgacgccgc 1620
tggccactgc ctttgggacc tcttccagtt ccgccacgct gccgctgatg atgaagtgcg 1680
tggaggagaa taatggcgtg gccaagcaca tcagccgttt catcctgccc atcggcgcca 1740
ccgtcaacat ggacggtgcc gcgctcttcc agtgcgtggc cgcagtgttc attgcacagc 1800
tcagccagca gtccttggac ttcgtaaaga tcatcaccat cctggtcacg gccacagcgt 1860
ccagcgtggg ggcagcgggc atccctgctg gaggtgtcct cactctggcc atcatcctcg 1920
aagcagtcaa cctcccggtc gaccatatct ccttgatcct ggctgtggac tggctagtcg 1980
accggtcctg taccgtcctc aatgtagaag gtgacgctct gggggcagga ctcctccaaa 2040
attatgtgga ccgtacggag tcgagaagca cagagcctga gttgatacaa gtgaagagtg 2100
agctgcccct ggatccgctg ccagtcccca ctgaggaagg aaaccccctc ctcaaacact 2160
atcgggggcc cgcaggggat gccacggtcg cctctgagaa ggaatcagtc atgtaaaccc 2220
cgggagggac cttccctgcc ctgctggggg tgctctttgg acactggatt atgaggaatg 2280
gataaatgga tgagctaggg ctctgggggt ctgcctgcac actctgggga gccaggggcc 2340
ccagcaccct ccaggacagg agatctggga tgcctggctg ctggagtaca tgtgttcaca 2400
agggttactc ctcaaaaccc ccagttctca ctcatgtccc caactcaagg ctagaaaaca 2460
gcaagatgga gaaataatgt tctgctgcgt ccccaccgtg acctgcctgg cctcccctgt 2520
ctcagggagc aggtcacagg tcaccatggg gaattctagc ccccactggg gggatgttac 2580
aacaccatgc tggttatttt ggcggctgta gttgtggggg gatgtgtgtg tgcacgtgtg 2640
tgtgtgtgtg tgtgtgtgtg tgtgtgtgtg ttctgtgacc tcctgtcccc atggtacgtc 2700
ccaccctgtc cccagatccc ctattccctc cacaataaca gaaacactcc cagggactct 2760
ggggagaggc tgaggacaaa tacctgctgt cactccagag gacatttttt ttagcaataa 2820
aattgagtgt caactattta aaaaaaaaaa aaaaaa 2856

<210>  118
<211>  541
<212>  PRT
<213>  Homo sapiens

<400>  118

Met Val Ala Asp Pro Pro Arg Asp Ser Lys Gly Leu Ala Ala Ala Glu
1               5                   10                  15

Pro Thr Ala Asn Gly Gly Leu Ala Leu Ala Ser Ile Glu Asp Gln Gly
            20                  25                  30

Ala Ala Ala Gly Gly Tyr Cys Gly Ser Arg Asp Gln Val Arg Arg Cys
        35                  40                  45

Leu Arg Ala Asn Leu Leu Val Leu Leu Thr Val Val Ala Val Val Ala
    50                  55                  60

Gly Val Ala Leu Gly Leu Gly Val Ser Gly Ala Gly Gly Ala Leu Ala
65                  70                  75                  80

Leu Gly Pro Glu Arg Leu Ser Ala Phe Val Phe Pro Gly Glu Leu Leu
                85                  90                  95

Leu Arg Leu Leu Arg Met Ile Ile Leu Pro Leu Val Val Cys Ser Leu
            100                 105                 110

Ile Gly Gly Ala Ala Ser Leu Asp Pro Gly Ala Leu Gly Arg Leu Gly
        115                 120                 125

Ala Trp Ala Leu Leu Phe Phe Leu Val Thr Thr Leu Leu Ala Ser Ala
    130                 135                 140

Leu Gly Val Gly Leu Ala Leu Ala Leu Gln Pro Gly Ala Ala Ser Ala
145                 150                 155                 160

Ala Ile Asn Ala Ser Val Gly Ala Ala Gly Ser Ala Glu Asn Ala Pro
                165                 170                 175

Ser Lys Glu Val Leu Asp Ser Phe Leu Asp Leu Ala Arg Asn Ile Phe
            180                 185                 190

Pro Ser Asn Leu Val Ser Ala Ala Phe Arg Ser Tyr Ser Thr Thr Tyr
        195                 200                 205

Glu Glu Arg Asn Ile Thr Gly Thr Arg Val Lys Val Pro Val Gly Gln
    210                 215                 220

Glu Val Glu Gly Met Asn Ile Leu Gly Leu Val Val Phe Ala Ile Val
225                 230                 235                 240

Phe Gly Val Ala Leu Arg Lys Leu Gly Pro Glu Gly Glu Leu Leu Ile
                245                 250                 255

Arg Phe Phe Asn Ser Phe Asn Glu Ala Thr Met Val Leu Val Ser Trp
            260                 265                 270

Ile Met Trp Tyr Ala Pro Val Gly Ile Met Phe Leu Val Ala Gly Lys
        275                 280                 285

Ile Val Glu Met Glu Asp Val Gly Leu Leu Phe Ala Arg Leu Gly Lys
    290                 295                 300

Tyr Ile Leu Cys Cys Leu Leu Gly His Ala Ile His Gly Leu Leu Val
305                 310                 315                 320

Leu Pro Leu Ile Tyr Phe Leu Phe Thr Arg Lys Asn Pro Tyr Arg Phe
                325                 330                 335

Leu Trp Gly Ile Val Thr Pro Leu Ala Thr Ala Phe Gly Thr Ser Ser
            340                 345                 350

Ser Ser Ala Thr Leu Pro Leu Met Met Lys Cys Val Glu Glu Asn Asn
        355                 360                 365

Gly Val Ala Lys His Ile Ser Arg Phe Ile Leu Pro Ile Gly Ala Thr
    370                 375                 380

Val Asn Met Asp Gly Ala Ala Leu Phe Gln Cys Val Ala Ala Val Phe
385                 390                 395                 400

Ile Ala Gln Leu Ser Gln Gln Ser Leu Asp Phe Val Lys Ile Ile Thr
                405                 410                 415

Ile Leu Val Thr Ala Thr Ala Ser Ser Val Gly Ala Ala Gly Ile Pro
            420                 425                 430

Ala Gly Gly Val Leu Thr Leu Ala Ile Ile Leu Glu Ala Val Asn Leu
        435                 440                 445

Pro Val Asp His Ile Ser Leu Ile Leu Ala Val Asp Trp Leu Val Asp
    450                 455                 460

Arg Ser Cys Thr Val Leu Asn Val Glu Gly Asp Ala Leu Gly Ala Gly
465                 470                 475                 480

Leu Leu Gln Asn Tyr Val Asp Arg Thr Glu Ser Arg Ser Thr Glu Pro
                485                 490                 495

Glu Leu Ile Gln Val Lys Ser Glu Leu Pro Leu Asp Pro Leu Pro Val
            500                 505                 510

Pro Thr Glu Glu Gly Asn Pro Leu Leu Lys His Tyr Arg Gly Pro Ala
        515                 520                 525

Gly Asp Ala Thr Val Ala Ser Glu Lys Glu Ser Val Met
    530                 535                 540

<210>  119
<211>  1993
<212>  DNA
<213>  Homo sapiens

<400>  119
ctagttaagg cggcacaggg ccgaggcgta gtgtgggtga ctcctccgtt ccttgggtcc      60

cgtcgtctgt gatactgcag ttcagccatg gcagaaccgc agcccccgtc cggcggcctc     120

acggacgagg ccgccctcag ttgctgctcc gacgcggacc ccagtaccaa ggattttcta     180

ttgcagcaga ccatgctacg agtgaaggat cctaagaagt cactggattt ttatactaga     240

gttcttggaa tgacgctaat ccaaaaatgt gattttccca ttatgaagtt ttcactctac     300

ttcttggctt atgaggataa aaatgacatc cctaaagaaa aagatgaaaa aatagcctgg     360

gcgctctcca gaaaagctac acttgagctg acacacaatt ggggcactga agatgatgcg     420

acccagagtt accacaatgg caattcagac cctcgaggat tcggtcatat tggaattgct     480

gttcctgatg tatacagtgc ttgtaaaagg tttgaagaac tgggagtcaa atttgtgaag     540

aaacctgatg atggtaaaat gaaaggcctg gcatttattc aagatcctga tggctactgg     600

attgaaattt tgaatcctaa caaaatggca accttaatgt agtgctgtga gaattctcct 660
ttgagatttc agaagaaagg aaacaatgtg attcaagata tttacatacc agaagcatct 720
aggactgatg gatcactgtc ccgattcaaa ttattcttca gtccatttcc ccttcctatt 780
tcagctgttc cttttcacct aactgttcag tcattctggt tttcaagcag tgctttatct 840
catgtccttg aatatagttg tgtaacttta ttttttaggt aataattaga acagttccct 900
tcagaggctg catttgcctt cttctgccac ctaaatatta cttcccttca aatctgcctt 960
tgaatcatca tttttaaaaa aaaattaaca tgtttttgtt gtagttatct tctggggttt 1020
caattcctca gaaacaactt ttttcacaac ggaaaggaaa gaacactagt gttctttcag 1080
taaagtacaa agtgtttatt ttacaaaaga gtaggtactc ttgagagcaa ttcaaatcat 1140
gctgacaagg atactgatag aaaaagtgat ttcttcttat tataaagtac atttaaagtt 1200
caaggactaa ccttatttat ttgggaaagg ggaggaggaa ggaaatgata tggtacccag 1260
acactgggct aggctgcaac tttatctcat ttaatactcc cagctgtcat gtgagaaaga 1320
aagcaggcta ggcatgtgaa atcactttca tggattatta atggatttaa gagggcatca 1380
atcagctcaa ctcaagattt cataatcatt tttagtattt agattgtgcc tcaaagttgt 1440
agtacctcac aatacctcca ctggtttcct gttgtaaaaa ccttcagtga gtttgaccat 1500
tgtgctcttg gctcttgggc tggagtaccg tggtgaggga gtaaacacta gaagtcttta 1560
gtacaaaact gctctaggga cacctggtga ttcctacaca agtgatgttt atatttctca 1620
taaagagtct tccctatccc aaggtcttca tgatgccagt agccatatat gataaattat 1680
gttcagtgat aacttagtta tcagaaatca gctcagtggt cttccccgcc atgattcaca 1740
tttgatgagt ttttaaaaat caaagtgatt ttgaaaatct ctaatggctc agaaaataaa 1800
aacatccagt ttgtggatga ctatatttag atttctctag actctagtgg aagacctttg 1860
gaaaggccat gccaaccgtg cttgtactgc tagaagcact ttatgtttcc tttttgggtg 1920
aaatggattt atgtgagtgc tttaaacaaa tagcaatact tatagactga aataaaatga 1980
aacttcaaat aag 1993

<210> 120
<211> 184
<212> PRT
<213> Homo sapiens

<400> 120

Met Ala Glu Pro Gln Pro Pro Ser Gly Gly Leu Thr Asp Glu Ala Ala
1               5                   10                  15

Leu Ser Cys Cys Ser Asp Ala Asp Pro Ser Thr Lys Asp Phe Leu Leu
            20                  25                  30

Gln Gln Thr Met Leu Arg Val Lys Asp Pro Lys Lys Ser Leu Asp Phe
        35                  40                  45

Tyr Thr Arg Val Leu Gly Met Thr Leu Ile Gln Lys Cys Asp Phe Pro
    50                  55                  60

Ile Met Lys Phe Ser Leu Tyr Phe Leu Ala Tyr Glu Asp Lys Asn Asp

65                  70                  75                  80

Ile Pro Lys Glu Lys Asp Glu Lys Ile Ala Trp Ala Leu Ser Arg Lys
                85                  90                  95

Ala Thr Leu Glu Leu Thr His Asn Trp Gly Thr Glu Asp Asp Ala Thr
            100                 105                 110

Gln Ser Tyr His Asn Gly Asn Ser Asp Pro Arg Gly Phe Gly His Ile
        115                 120                 125

Gly Ile Ala Val Pro Asp Val Tyr Ser Ala Cys Lys Arg Phe Glu Glu
    130                 135                 140

Leu Gly Val Lys Phe Val Lys Lys Pro Asp Asp Gly Lys Met Lys Gly
145                 150                 155                 160

Leu Ala Phe Ile Gln Asp Pro Asp Gly Tyr Trp Ile Glu Ile Leu Asn
                165                 170                 175

Pro Asn Lys Met Ala Thr Leu Met
            180

<210>  121
<211>  808
<212>  DNA
<213>  Homo sapiens

<400>  121
ccgacgccag gtcctgccgt cccgccgacc gtccgggagc gaacccgtcg tcccgcactg      60

gagtccgcga tggcttcagt gacagatggt aaacatggag tcaaagatgc ctctgaccag     120

aattttgact acatgtttaa actgcttatc attggcaaca gcagtgttgg caagacctcc     180

ttcctcttgc gctatgctga tgacacgttc accccagcct tcgttagcac cgtgggcatc     240

gacttcaagg tgaagacagt ctaccgtcac gagaagcggg tgaaactgca gatctgggac     300

acagctgggc aggagcggta ccggaccatc acaacagcct attaccgtgg ggccatgggc     360

ttcattctga tgtatgacat caccaatgaa gagtccttca atgctgtcca agactgggct     420

actcagatca agacctactc ctgggacaat gcacaagtta ttctggtggg gaacaagtgt     480

gacatggagg aagagagggt tgttcccact gagaagggcc agctccttgc agagcagctt     540

gggtttgatt tctttgaagc cagtgcaaag gagaacatca gtgtaaggca ggcctttgag     600

cgcctggtgg atgccatttg tgacaagatg tctgattcgc tggacacaga cccgtcgatg     660

ctgggctcct ccaagaacac gcgtctctcg gacaccccac cgctgctgca gcagaactgc     720

tcatgctagc aaggcccacc ttcctgacct cccctcattg tggccccaca cccaagtctg     780

cttctccctg ttacacactg tccgctct                                        808

<210>  122
<211>  219
<212>  PRT
<213>  Homo sapiens

<400>  122

Met Ala Ser Val Thr Asp Gly Lys His Gly Val Lys Asp Ala Ser Asp
1               5                   10                  15

Gln Asn Phe Asp Tyr Met Phe Lys Leu Leu Ile Ile Gly Asn Ser Ser

            20                  25                  30

Val Gly Lys Thr Ser Phe Leu Leu Arg Tyr Ala Asp Asp Thr Phe Thr
        35                  40                  45

Pro Ala Phe Val Ser Thr Val Gly Ile Asp Phe Lys Val Lys Thr Val
    50                  55                  60

Tyr Arg His Glu Lys Arg Val Lys Leu Gln Ile Trp Asp Thr Ala Gly
65                  70                  75                  80

Gln Glu Arg Tyr Arg Thr Ile Thr Thr Ala Tyr Tyr Arg Gly Ala Met
                85                  90                  95

Gly Phe Ile Leu Met Tyr Asp Ile Thr Asn Glu Glu Ser Phe Asn Ala
            100                 105                 110

Val Gln Asp Trp Ala Thr Gln Ile Lys Thr Tyr Ser Trp Asp Asn Ala
        115                 120                 125

Gln Val Ile Leu Val Gly Asn Lys Cys Asp Met Glu Glu Glu Arg Val
    130                 135                 140

Val Pro Thr Glu Lys Gly Gln Leu Leu Ala Glu Gln Leu Gly Phe Asp
145                 150                 155                 160

Phe Phe Glu Ala Ser Ala Lys Glu Asn Ile Ser Val Arg Gln Ala Phe
                165                 170                 175

Glu Arg Leu Val Asp Ala Ile Cys Asp Lys Met Ser Asp Ser Leu Asp
            180                 185                 190

Thr Asp Pro Ser Met Leu Gly Ser Ser Lys Asn Thr Arg Leu Ser Asp
        195                 200                 205

Thr Pro Pro Leu Leu Gln Gln Asn Cys Ser Cys
    210                 215

<210> 123
<211> 5060
<212> DNA
<213> Homo sapiens

<400> 123
acgcagagcg ctgctgggct gccgggtctc ccgcttcctc ctcctgctcc aagggcctcc 60

tgcatgaggg cgcggtagag acccggaccc gcgccgtgct cctgccgttt cgctgcgctc 120

cgcccgggcc cggctcagcc aggccccgcg gtgagccatg attcgcctcg gggctcccca 180

gtcgctggtg ctgctgacgc tgctcgtcgc cgctgtcctt cggtgtcagg gccaggatgt 240

ccaggaggct ggcagctgtg tgcaggatgg gcagaggtat aatgataagg atgtgtggaa 300

gccggagccc tgccggatct gtgtctgtga cactgggact gtcctctgcg acgacataat 360

ctgtgaagac gtgaaagact gcctcagccc tgagatcccc ttcggagagt gctgccccat 420

ctgcccaact gacctcgcca ctgccagtgg gcaaccagga ccaaagggac agaaaggaga 480

acctggagac atcaaggata ttgtaggacc caaaggacct cctgggcctc agggacctgc 540

aggggaacaa ggacccagag gggatcgtgg tgacaaaggt gaaaaaggtg cccctggacc 600

tcgtggcaga gatggagaac ctgggacccc tggaaatcct ggcccccctg gtcctcccgg 660

cccccctggt ccccctggtc ttggtggaaa ctttgctgcc cagatggctg gaggatttga 720

tgaaaaggct ggtggcgccc agttgggagt aatgcaagga ccaatgggcc ccatgggacc 780
tcgaggacct ccaggccctg caggtgctcc tgggcctcaa ggatttcaag gcaatcctgg 840
tgaacctggt gaacctggtg tctctggtcc catgggtccc cgtggtcctc ctggtccccc 900
tggaaagcct ggtgatgatg gtgaagctgg aaaacctgga aaagctggtg aaaggggtcc 960
gcctggtcct cagggtgctc gtggtttccc aggaacccca ggccttcctg gtgtcaaagg 1020
tcacagaggt tatccaggcc tggacggtgc taagggagag gcgggtgctc ctggtgtgaa 1080
gggtgagagt ggttccccgg gtgagaacgg atctccgggc ccaatgggtc ctcgtggcct 1140
gcctggtgaa agaggacgga ctggccctgc tggcgctgcg ggtgcccgag gcaacgatgg 1200
tcagccaggc cccgcaggtc ctccgggtcc tgtcggtcct gctggtggtc ctggcttccc 1260
tggtgctcct ggagccaagg gtgaagccgg ccccactggt gcccgtggtc ctgaaggtgc 1320
tcaaggtcct cgcggtgaac ctggtactcc tgggtcccct gggcctgctg gtgcctccgg 1380
taaccctgga acagatggaa ttcctggagc caaaggatct gctggtgctc ctggcattgc 1440
tggtgctcct ggcttccctg ggccacgggg tcctcctggc cctcaaggtg caactggtcc 1500
tctgggcccg aaaggtcaga cgggtgaacc tggtattgct ggcttcaaag gtgaacaagg 1560
ccccaaggga gaacctggcc ctgctggccc ccagggagcc cctggacccg ctggtgaaga 1620
aggcaagaga ggtgcccgtg gagagcctgg tggcgttggg cccatcggtc cccctggaga 1680
aagaggtgct cccggaaacc gcggtttccc aggtcaagat ggtctggcag gtcccaaggg 1740
agcccctgga gagcgagggc ccagtggtct tgctggcccc aagggagcca acggtgaccc 1800
tggccgtcct ggagaacctg gccttcctgg agcccggggt ctcactggcc gccctggtga 1860
tgctggtcct caaggcaaag ttggcccttc tggagcccct ggtgaagatg gtcgtcctgg 1920
acctccaggt cctcaggggg ctcgtgggca gcctggtgtc atgggtttcc ctggccccaa 1980
aggtgccaac ggtgagcctg gcaaagctgg tgagaaggga ctgcctggtg ctcctggtct 2040
gaggggtctt cctggcaaag atggtgagac aggtgctgca ggaccccctg gccctgctgg 2100
acctgctggt gaacgaggcg agcagggtgc tcctgggcca tctgggttcc agggacttcc 2160
tggccctcct ggtcccccag gtgaaggtgg aaaaccaggt gaccagggtg ttcccggtga 2220
agctggagcc cctggcctcg tgggtcccag gggtgaacga ggtttcccag gtgaacgtgg 2280
ctctcccggt gcccagggcc tccagggtcc ccgtggcctc cccggcactc ctggcactga 2340
tggtcccaaa ggtgcatctg gcccagcagg cccccctggc gcacagggcc ctccaggtct 2400
tcagggaatg cctggcgaga ggggagcagc tggtatcgct gggcccaaag gcgacagggg 2460
tgacgttggt gagaaaggcc ctgagggagc ccctggaaag gatggtggac gaggcctgac 2520
aggtcccatt ggcccccctg gcccagctgg tgctaacggc gagaagggag aagttggacc 2580
tcctggtcct gcaggaagtg ctggtgctcg tggcgctccg ggtgaacgtg gagagactgg 2640
cccccccgga ccagcgggat ttgctgggcc tcctggtgct gatggccagc ctggggccaa 2700
gggtgagcaa ggagaggccg gccagaaagg cgatgctggt gcccctggtc ctcagggccc 2760

ctctggagca cctgggcctc agggtcctac tggagtgact ggtcctaaag gagcccgagg 2820
tgcccaaggc cccccgggag ccactggatt ccctggagct gctggccgcg ttggaccccc 2880
aggctccaat ggcaaccctg gaccccctgg tccccctggt ccttctggaa aagatggtcc 2940
caaaggtgct cgaggagaca gcggcccccc tggccgagct ggtgaacccg gcctccaagg 3000
tcctgctgga ccccctggcg agaagggaga gcctggagat gacggtccct ctggtgccga 3060
aggtccacca ggtccccagg gtctggctgg tcagagaggc atcgtcggtc tgcctgggca 3120
acgtggtgag agaggattcc ctggcttgcc tggcccatcg ggtgagcccg gcaagcaggg 3180
tgctcctgga gcatctggag acagaggtcc tcctggcccc gtgggtcctc ctggcctgac 3240
gggtcctgca ggtgaacccg gacgagaggg aagccccggt gctgatggcc cccctggcag 3300
agatggcgct gctggagtca agggtgatcg tggtgagact ggtgctgtgg gagctcctgg 3360
agcccctggg ccccctggct cccctggccc cgctggtcca actggcaagc aaggagacag 3420
aggagaagct ggtgcacaag gccccatggg accctcagga ccagctggag cccggggaat 3480
ccagggtcct caaggcccca gaggtgacaa aggagaggct ggagagcctg gcgagagagg 3540
cctgaaggga caccgtggct tcactggtct gcagggtctg cccggccctc ctggtccttc 3600
tggagaccaa ggtgcttctg gtcctgctgg tccttctggc cctagaggtc ctcctggccc 3660
cgtcggtccc tctggcaaag atggtgctaa tggaatccct ggccccattg ggcctcctgg 3720
tccccgtgga cgatcaggcg aaaccggtcc tgctggtcct cctggaaatc ctgggccccc 3780
tggtcctcca ggtccccctg gccctggcat cgacatgtcc gcctttgctg gcttaggccc 3840
gagagagaag ggccccgacc ccctgcagta catgcgggcc gaccaggcag ccggtggcct 3900
gagacagcat gacgccgagg tggatgccac actcaagtcc ctcaacaacc agattgagag 3960
catccgcagc cccgagggct cccgcaagaa ccctgctcgc acctgcagag acctgaaact 4020
ctgccaccct gagtggaaga gtggagacta ctggattgac cccaaccaag gctgcacctt 4080
ggacgccatg aaggttttct gcaacatgga gactggcgag acttgcgtct accccaatcc 4140
agcaaacgtt cccaagaaga actggtggag cagcaagagc aaggagaaga aacacatctg 4200
gtttggagaa accatcaatg gtggcttcca tttcagctat ggagatgaca atctggctcc 4260
caacactgcc aacgtccaga tgaccttcct acgcctgctg tccacggaag gctcccagaa 4320
catcacctac cactgcaaga acagcattgc ctatctggac gaagcagctg gcaacctcaa 4380
gaaggccctg ctcatccagg gctccaatga cgtggagatc cgggcagagg gcaatagcag 4440
gttcacgtac actgccctga aggatggctg cacgaaacat accggtaagt ggggcaagac 4500
tgttatcgag taccggtcac agaagacctc acgcctcccc atcattgaca ttgcacccat 4560
ggacatagga gggcccgagc aggaattcgg tgtggacata gggccggtct gcttcttgta 4620
aaaacctgaa cccagaaaca acacaatccg ttgcaaaccc aaaggaccca agtactttcc 4680
aatctcagtc actctaggac tctgcactga atggctgacc tgacctgatg tccattcatc 4740
ccaccctctc acagttcgga cttttctccc ctctctttct aagagacctg aactgggcag 4800

actgcaaaat aaaatctcgg tgttctattt atttattgtc ttcctgtaag accttcgggt 4860

caaggcagag gcaggaaact aactggtgtg agtcaaatgc cccctgagtg actgccccca 4920

gcccaggcca gaagacctcc cttcaggtgc cgggcgcagg aactgtgtgt gtcctacaca 4980

atggtgctat tctgtgtcaa acacctctgt attttttaaa acatcaattg atattaaaaa 5040

tgaaaagatt attggaaagt 5060

<210> 124
<211> 1487
<212> PRT
<213> Homo sapiens

<400> 124

Met Ile Arg Leu Gly Ala Pro Gln Ser Leu Val Leu Leu Thr Leu Leu
1               5                   10                  15

Val Ala Ala Val Leu Arg Cys Gln Gly Gln Asp Val Gln Glu Ala Gly
            20                  25                  30

Ser Cys Val Gln Asp Gly Gln Arg Tyr Asn Asp Lys Asp Val Trp Lys
        35                  40                  45

Pro Glu Pro Cys Arg Ile Cys Val Cys Asp Thr Gly Thr Val Leu Cys
    50                  55                  60

Asp Asp Ile Ile Cys Glu Asp Val Lys Asp Cys Leu Ser Pro Glu Ile
65                  70                  75                  80

Pro Phe Gly Glu Cys Cys Pro Ile Cys Pro Thr Asp Leu Ala Thr Ala
                85                  90                  95

Ser Gly Gln Pro Gly Pro Lys Gly Gln Lys Gly Glu Pro Gly Asp Ile
            100                 105                 110

Lys Asp Ile Val Gly Pro Lys Gly Pro Pro Gly Pro Gln Gly Pro Ala
        115                 120                 125

Gly Glu Gln Gly Pro Arg Gly Asp Arg Gly Asp Lys Gly Glu Lys Gly
    130                 135                 140

Ala Pro Gly Pro Arg Gly Arg Asp Gly Glu Pro Gly Thr Pro Gly Asn
145                 150                 155                 160

Pro Gly Pro Pro Gly Pro Pro Gly Pro Pro Gly Pro Pro Gly Leu Gly
                165                 170                 175

Gly Asn Phe Ala Ala Gln Met Ala Gly Gly Phe Asp Glu Lys Ala Gly
            180                 185                 190

Gly Ala Gln Leu Gly Val Met Gln Gly Pro Met Gly Pro Met Gly Pro
        195                 200                 205

Arg Gly Pro Pro Gly Pro Ala Gly Ala Pro Gly Pro Gln Gly Phe Gln
    210                 215                 220

Gly Asn Pro Gly Glu Pro Gly Glu Pro Gly Val Ser Gly Pro Met Gly
225                 230                 235                 240

Pro Arg Gly Pro Pro Gly Pro Pro Gly Lys Pro Gly Asp Asp Gly Glu
                245                 250                 255

Ala Gly Lys Pro Gly Lys Ala Gly Glu Arg Gly Pro Pro Gly Pro Gln
            260                 265                 270

Gly Ala Arg Gly Phe Pro Gly Thr Pro Gly Leu Pro Gly Val Lys Gly
        275                 280                 285

His Arg Gly Tyr Pro Gly Leu Asp Gly Ala Lys Gly Glu Ala Gly Ala
    290                 295                 300

Pro Gly Val Lys Gly Glu Ser Gly Ser Pro Gly Glu Asn Gly Ser Pro
305                 310                 315                 320

Gly Pro Met Gly Pro Arg Gly Leu Pro Gly Glu Arg Gly Arg Thr Gly
                325                 330                 335

Pro Ala Gly Ala Ala Gly Ala Arg Gly Asn Asp Gly Gln Pro Gly Pro
            340                 345                 350

Ala Gly Pro Pro Gly Pro Val Gly Pro Ala Gly Gly Pro Gly Phe Pro
        355                 360                 365

Gly Ala Pro Gly Ala Lys Gly Glu Ala Gly Pro Thr Gly Ala Arg Gly
    370                 375                 380

Pro Glu Gly Ala Gln Gly Pro Arg Gly Glu Pro Gly Thr Pro Gly Ser
385                 390                 395                 400

Pro Gly Pro Ala Gly Ala Ser Gly Asn Pro Gly Thr Asp Gly Ile Pro
                405                 410                 415

Gly Ala Lys Gly Ser Ala Gly Ala Pro Gly Ile Ala Gly Ala Pro Gly
            420                 425                 430

Phe Pro Gly Pro Arg Gly Pro Pro Gly Pro Gln Gly Ala Thr Gly Pro
        435                 440                 445

Leu Gly Pro Lys Gly Gln Thr Gly Glu Pro Gly Ile Ala Gly Phe Lys
    450                 455                 460

Gly Glu Gln Gly Pro Lys Gly Glu Pro Gly Pro Ala Gly Pro Gln Gly
465                 470                 475                 480

Ala Pro Gly Pro Ala Gly Glu Glu Gly Lys Arg Gly Ala Arg Gly Glu
                485                 490                 495

Pro Gly Gly Val Gly Pro Ile Gly Pro Pro Gly Glu Arg Gly Ala Pro
            500                 505                 510

Gly Asn Arg Gly Phe Pro Gly Gln Asp Gly Leu Ala Gly Pro Lys Gly
        515                 520                 525

Ala Pro Gly Glu Arg Gly Pro Ser Gly Leu Ala Gly Pro Lys Gly Ala
    530                 535                 540

Asn Gly Asp Pro Gly Arg Pro Gly Glu Pro Gly Leu Pro Gly Ala Arg
545                 550                 555                 560

Gly Leu Thr Gly Arg Pro Gly Asp Ala Gly Pro Gln Gly Lys Val Gly
                565                 570                 575

Pro Ser Gly Ala Pro Gly Glu Asp Gly Arg Pro Gly Pro Pro Gly Pro
            580                 585                 590

Gln Gly Ala Arg Gly Gln Pro Gly Val Met Gly Phe Pro Gly Pro Lys
        595                 600                 605

Gly Ala Asn Gly Glu Pro Gly Lys Ala Gly Glu Lys Gly Leu Pro Gly
    610                 615                 620

Ala Pro Gly Leu Arg Gly Leu Pro Gly Lys Asp Gly Glu Thr Gly Ala
625                 630                 635                 640

Ala Gly Pro Pro Gly Pro Ala Gly Pro Ala Gly Glu Arg Gly Glu Gln
                645                 650                 655

Gly Ala Pro Gly Pro Ser Gly Phe Gln Gly Leu Pro Gly Pro Pro Gly
            660                 665                 670

Pro Pro Gly Glu Gly Gly Lys Pro Gly Asp Gln Gly Val Pro Gly Glu
        675                 680                 685

Ala Gly Ala Pro Gly Leu Val Gly Pro Arg Gly Glu Arg Gly Phe Pro
    690                 695                 700

Gly Glu Arg Gly Ser Pro Gly Ala Gln Gly Leu Gln Gly Pro Arg Gly
705                 710                 715                 720

Leu Pro Gly Thr Pro Gly Thr Asp Gly Pro Lys Gly Ala Ser Gly Pro
                725                 730                 735

Ala Gly Pro Pro Gly Ala Gln Gly Pro Pro Gly Leu Gln Gly Met Pro
            740                 745                 750

Gly Glu Arg Gly Ala Ala Gly Ile Ala Gly Pro Lys Gly Asp Arg Gly
        755                 760                 765

Asp Val Gly Glu Lys Gly Pro Glu Gly Ala Pro Gly Lys Asp Gly Gly
    770                 775                 780

Arg Gly Leu Thr Gly Pro Ile Gly Pro Pro Gly Pro Ala Gly Ala Asn
785                 790                 795                 800

Gly Glu Lys Gly Glu Val Gly Pro Pro Gly Pro Ala Gly Ser Ala Gly
                805                 810                 815

Ala Arg Gly Ala Pro Gly Glu Arg Gly Glu Thr Gly Pro Pro Gly Pro
            820                 825                 830

Ala Gly Phe Ala Gly Pro Pro Gly Ala Asp Gly Gln Pro Gly Ala Lys
        835                 840                 845

Gly Glu Gln Gly Glu Ala Gly Gln Lys Gly Asp Ala Gly Ala Pro Gly
    850                 855                 860

Pro Gln Gly Pro Ser Gly Ala Pro Gly Pro Gln Gly Pro Thr Gly Val
865                 870                 875                 880

Thr Gly Pro Lys Gly Ala Arg Gly Ala Gln Gly Pro Pro Gly Ala Thr
                885                 890                 895

Gly Phe Pro Gly Ala Ala Gly Arg Val Gly Pro Pro Gly Ser Asn Gly
            900                 905                 910

Asn Pro Gly Pro Pro Gly Pro Pro Gly Pro Ser Gly Lys Asp Gly Pro
        915                 920                 925

Lys Gly Ala Arg Gly Asp Ser Gly Pro Pro Gly Arg Ala Gly Glu Pro
    930                 935                 940

Gly Leu Gln Gly Pro Ala Gly Pro Pro Gly Glu Lys Gly Glu Pro Gly
945                 950                 955                 960

Asp Asp Gly Pro Ser Gly Ala Glu Gly Pro Pro Gly Pro Gln Gly Leu
                965                 970                 975

Ala Gly Gln Arg Gly Ile Val Gly Leu Pro Gly Gln Arg Gly Glu Arg

            980                 985                 990

Gly Phe Pro Gly Leu Pro Gly Pro Ser Gly Glu Pro Gly Lys Gln Gly
        995                 1000                1005

Ala Pro Gly Ala Ser Gly Asp Arg Gly Pro Pro Gly Pro Val Gly
    1010                1015                1020

Pro Pro Gly Leu Thr Gly Pro Ala Gly Glu Pro Gly Arg Glu Gly
    1025                1030                1035

Ser Pro Gly Ala Asp Gly Pro Pro Gly Arg Asp Gly Ala Ala Gly
    1040                1045                1050

Val Lys Gly Asp Arg Gly Glu Thr Gly Ala Val Gly Ala Pro Gly
    1055                1060                1065

Ala Pro Gly Pro Pro Gly Ser Pro Gly Pro Ala Gly Pro Thr Gly
    1070                1075                1080

Lys Gln Gly Asp Arg Gly Glu Ala Gly Ala Gln Gly Pro Met Gly
    1085                1090                1095

Pro Ser Gly Pro Ala Gly Ala Arg Gly Ile Gln Gly Pro Gln Gly
    1100                1105                1110

Pro Arg Gly Asp Lys Gly Glu Ala Gly Glu Pro Gly Glu Arg Gly
    1115                1120                1125

Leu Lys Gly His Arg Gly Phe Thr Gly Leu Gln Gly Leu Pro Gly
    1130                1135                1140

Pro Pro Gly Pro Ser Gly Asp Gln Gly Ala Ser Gly Pro Ala Gly
    1145                1150                1155

Pro Ser Gly Pro Arg Gly Pro Pro Gly Pro Val Gly Pro Ser Gly
    1160                1165                1170

Lys Asp Gly Ala Asn Gly Ile Pro Gly Pro Ile Gly Pro Pro Gly
    1175                1180                1185

Pro Arg Gly Arg Ser Gly Glu Thr Gly Pro Ala Gly Pro Pro Gly
    1190                1195                1200

Asn Pro Gly Pro Pro Gly Pro Pro Gly Pro Pro Gly Pro Gly Ile
    1205                1210                1215

Asp Met Ser Ala Phe Ala Gly Leu Gly Pro Arg Glu Lys Gly Pro
    1220                1225                1230

Asp Pro Leu Gln Tyr Met Arg Ala Asp Gln Ala Ala Gly Gly Leu
    1235                1240                1245

Arg Gln His Asp Ala Glu Val Asp Ala Thr Leu Lys Ser Leu Asn
    1250                1255                1260

Asn Gln Ile Glu Ser Ile Arg Ser Pro Glu Gly Ser Arg Lys Asn
    1265                1270                1275

Pro Ala Arg Thr Cys Arg Asp Leu Lys Leu Cys His Pro Glu Trp
    1280                1285                1290

Lys Ser Gly Asp Tyr Trp Ile Asp Pro Asn Gln Gly Cys Thr Leu
    1295                1300                1305

Asp Ala Met Lys Val Phe Cys Asn Met Glu Thr Gly Glu Thr Cys
    1310                1315                1320

Val Tyr Pro Asn Pro Ala Asn Val Pro Lys Lys Asn Trp Trp Ser
    1325                1330                1335

Ser Lys Ser Lys Glu Lys Lys His Ile Trp Phe Gly Glu Thr Ile
    1340                1345                1350

Asn Gly Gly Phe His Phe Ser Tyr Gly Asp Asp Asn Leu Ala Pro
    1355                1360                1365

Asn Thr Ala Asn Val Gln Met Thr Phe Leu Arg Leu Leu Ser Thr
    1370                1375                1380

Glu Gly Ser Gln Asn Ile Thr Tyr His Cys Lys Asn Ser Ile Ala
    1385                1390                1395

Tyr Leu Asp Glu Ala Ala Gly Asn Leu Lys Lys Ala Leu Leu Ile
    1400                1405                1410

Gln Gly Ser Asn Asp Val Glu Ile Arg Ala Glu Gly Asn Ser Arg
    1415                1420                1425

Phe Thr Tyr Thr Ala Leu Lys Asp Gly Cys Thr Lys His Thr Gly
    1430                1435                1440

Lys Trp Gly Lys Thr Val Ile Glu Tyr Arg Ser Gln Lys Thr Ser
    1445                1450                1455

Arg Leu Pro Ile Ile Asp Ile Ala Pro Met Asp Ile Gly Gly Pro
    1460                1465                1470

Glu Gln Glu Phe Gly Val Asp Ile Gly Pro Val Cys Phe Leu
    1475                1480                1485

<210> 125
<211> 1505
<212> DNA
<213> Homo sapiens

<400> 125
gctggtcgga ggctcgcagt gctgtcggcg agaagcagtc gggtttggag cgcttgggtc 60

gcgttggtgc gcggtggaac gcgcccaggg accccagttc ccgcgagcag ctccgcgccg 120

cgcctgagag actaagctga aactgctgct cagctcccaa gatggtgcca cccaaattgc 180

atgtgctttt ctgcctctgc ggctgcctgg ctgtggttta tccttttgac tggcaataca 240

taaatcctgt tgcccatatg aaatcatcag catgggtcaa caaaatacaa gtactgatgg 300

ctgctgcaag ctttggccaa actaaaatcc cccggggaaa tgggccttat tccgttggtt 360

gtacagactt aatgtttgat cacactaata agggcacctt cttgcgttta tattatccat 420

cccaagataa tgatcgcctt gacacccttt ggatcccaaa taaagaatat ttttggggtc 480

ttagcaaatt tcttggaaca cactggctta tgggcaacat tttgaggtta ctctttggtt 540

caatgacaac tcctgcaaac tggaattccc ctctgaggcc tggtgaaaaa tatccacttg 600

ttgttttttc tcatggtctt ggggcattca ggacacttta ttctgctatt ggcattgacc 660

tggcatctca tgggtttata gttgctgctg tagaacacag agatagatct gcatctgcaa 720

cttactattt caaggaccaa tctgctgcag aaatagggga caagtcttgg ctctacctta 780

gaaccctgaa acaagaggag gagacacata tacgaaatga gcaggtacgg caaagagcaa 840

aagaatgttc ccaagctctc agtctgattc ttgacattga tcatggaaag ccagtgaaga 900
atgcattaga tttaaagttt gatatggaac aactgaagga ctctattgat agggaaaaaa 960
tagcagtaat tggacattct tttggtggag caacggttat tcagactctt agtgaagatc 1020
agagattcag atgtggtatt gccctggatg catggatgtt tccactgggt gatgaagtat 1080
attccagaat tcctcagccc ctctttttta tcaactctga atatttccaa tatcctgcta 1140
atatcataaa aatgaaaaaa tgctactcac ctgataaaga aagaaagatg attacaatca 1200
ggggttcagt ccaccagaat tttgctgact tcacttttgc aactggcaaa ataattggac 1260
acatgctcaa attaaaggga gacatagatt caaatgtagc tattgatctt agcaacaaag 1320
cttcattagc attcttacaa aagcatttag gacttcataa agattttgat cagtgggact 1380
gcttgattga aggagatgat gagaatctta ttccagggac caacattaac acaaccaatc 1440
aacacatcat gttacagaac tcttcaggaa tagagaaata caattaggat taaaataggt 1500
ttttt 1505

<210> 126
<211> 441
<212> PRT
<213> Homo sapiens

<400> 126

Met Val Pro Pro Lys Leu His Val Leu Phe Cys Leu Cys Gly Cys Leu
1               5                   10                  15

Ala Val Val Tyr Pro Phe Asp Trp Gln Tyr Ile Asn Pro Val Ala His
            20                  25                  30

Met Lys Ser Ser Ala Trp Val Asn Lys Ile Gln Val Leu Met Ala Ala
        35                  40                  45

Ala Ser Phe Gly Gln Thr Lys Ile Pro Arg Gly Asn Gly Pro Tyr Ser
    50                  55                  60

Val Gly Cys Thr Asp Leu Met Phe Asp His Thr Asn Lys Gly Thr Phe
65                  70                  75                  80

Leu Arg Leu Tyr Tyr Pro Ser Gln Asp Asn Asp Arg Leu Asp Thr Leu
                85                  90                  95

Trp Ile Pro Asn Lys Glu Tyr Phe Trp Gly Leu Ser Lys Phe Leu Gly
            100                 105                 110

Thr His Trp Leu Met Gly Asn Ile Leu Arg Leu Leu Phe Gly Ser Met
        115                 120                 125

Thr Thr Pro Ala Asn Trp Asn Ser Pro Leu Arg Pro Gly Glu Lys Tyr
    130                 135                 140

Pro Leu Val Val Phe Ser His Gly Leu Gly Ala Phe Arg Thr Leu Tyr
145                 150                 155                 160

Ser Ala Ile Gly Ile Asp Leu Ala Ser His Gly Phe Ile Val Ala Ala
                165                 170                 175

Val Glu His Arg Asp Arg Ser Ala Ser Ala Thr Tyr Tyr Phe Lys Asp
            180                 185                 190

Gln Ser Ala Ala Glu Ile Gly Asp Lys Ser Trp Leu Tyr Leu Arg Thr
        195                 200                 205

Leu Lys Gln Glu Glu Glu Thr His Ile Arg Asn Glu Gln Val Arg Gln
    210                 215                 220

Arg Ala Lys Glu Cys Ser Gln Ala Leu Ser Leu Ile Leu Asp Ile Asp
225                 230                 235                 240

His Gly Lys Pro Val Lys Asn Ala Leu Asp Leu Lys Phe Asp Met Glu
                245                 250                 255

Gln Leu Lys Asp Ser Ile Asp Arg Glu Lys Ile Ala Val Ile Gly His
            260                 265                 270

Ser Phe Gly Gly Ala Thr Val Ile Gln Thr Leu Ser Glu Asp Gln Arg
        275                 280                 285

Phe Arg Cys Gly Ile Ala Leu Asp Ala Trp Met Phe Pro Leu Gly Asp
    290                 295                 300

Glu Val Tyr Ser Arg Ile Pro Gln Pro Leu Phe Phe Ile Asn Ser Glu
305                 310                 315                 320

Tyr Phe Gln Tyr Pro Ala Asn Ile Ile Lys Met Lys Lys Cys Tyr Ser
                325                 330                 335

Pro Asp Lys Glu Arg Lys Met Ile Thr Ile Arg Gly Ser Val His Gln
            340                 345                 350

Asn Phe Ala Asp Phe Thr Phe Ala Thr Gly Lys Ile Ile Gly His Met
        355                 360                 365

Leu Lys Leu Lys Gly Asp Ile Asp Ser Asn Val Ala Ile Asp Leu Ser
    370                 375                 380

Asn Lys Ala Ser Leu Ala Phe Leu Gln Lys His Leu Gly Leu His Lys
385                 390                 395                 400

Asp Phe Asp Gln Trp Asp Cys Leu Ile Glu Gly Asp Asp Glu Asn Leu
                405                 410                 415

Ile Pro Gly Thr Asn Ile Asn Thr Thr Asn Gln His Ile Met Leu Gln
            420                 425                 430

Asn Ser Ser Gly Ile Glu Lys Tyr Asn
        435                 440

<210>  127
<211>  1758
<212>  DNA
<213>  Homo sapiens

<400>  127
cacgagcggc acgaggattt ccagctcagc gatgccccca ggtccctggg agagctgctt      60

ctgggtgggg ggcctcattt tgtggctcag cgttggaagt tcaggggatg cacctcctac     120

cccacagcca aagtgcgctg acttccagag cgccaacctt tttgaaggca ccgatctcaa     180

agtccagttt ctcctctttg tcccttcgaa tcctagctgt gggcagctag tagaaggaag     240

cagtgacctc caaaactctg ggttcaatgc cactctggga accaaactaa ttatccatgg     300

attcagggtt ttaggaacaa agccttcctg gattgacaca tttattagaa cccttctgcg     360

tgcaacgaat gctaatgtga ttgccgtgga ctggatttat gggtctacag gagtctactt     420

ctcagctgtg aaaaatgtga ttaagttgag cctcgagatc tccctttcc tcaataaact 480

cctggtgctg ggtgtgtcgg aatcctcaat ccacatcatt ggtgttagcc tgggggccca 540

cgttgggggc atggtgggac agctcttcgg aggccagctg ggacagatca caggcctgga 600

ccccgctgga cctgagtaca ccagggccag tgtggaagag cgcttggatg ctggagatgc 660

cctcttcgtg gaagccatcc acacagacac cgacaatttg ggtattcgga ttcccgttgg 720

acatgtggac tacttcgtca acggaggcca agaccaacct ggctgcccca ccttctttta 780

cgcaggttat agttatctga tctgtgatca catgagggct gtgcacctct acatcagcgc 840

cctggagaat tcctgtccac tgatggcctt tccctgtgcc agctacaagg ccttccttgc 900

tggacgctgt ctggattgct ttaacccttt tctgctttcc tgcccaagga taggactggt 960

ggaacaaggt ggtgtcaaga tagagccgct ccccaaggaa gtgaaagtct acctcctgac 1020

tacttccagt gctccgtact gcatgcatca cagcctcgtg gagtttcact tgaaggaact 1080

gagaaacaag gacaccaaca tcgaggttac cttccttagc agtaacatca cctcttcatc 1140

taagatcacc atacctaagc agcaacgcta tgggaaagga atcatagccc atgccacccc 1200

acaatgccag ataaaccaag tgaaattcaa gtttcagtct tccaaccgag tttggaaaaa 1260

agaccggact accattattg ggaagttctg cactgccctt ttgcctgtca atgacagaga 1320

aaagatggtc tgcttacctg aaccagtgaa cttacaagca agtgtgactg tttcctgtga 1380

cctgaagata gcctgtgtgt agtttaacct gggcaggaca catctccctg catttttttt 1440

tttttttttt gagagagagg tgtgatgagg gatgtgtgtg tgcagcttat tgtagaccat 1500

tactactaag gagaaaagca aagctctttc ttattttcct cataatcagc taccctggag 1560

gggagggaga actcatttta cagaacttgg tttcctttgc cgatcttatg tacataccca 1620

ttttagcttt cccatgcata cttaactgca cttgctttat ctccttgggc attcgtactt 1680

aggattcaat agaaacatgt acagggtaaa caatttttta aaaataaaac ttcatggagt 1740

aaaaaaaaaa aaaaaaaa 1758

<210> 128
<211> 456
<212> PRT
<213> Homo sapiens

<400> 128

Met Pro Pro Gly Pro Trp Glu Ser Cys Phe Trp Val Gly Gly Leu Ile
1               5                   10                  15

Leu Trp Leu Ser Val Gly Ser Ser Gly Asp Ala Pro Pro Thr Pro Gln
            20                  25                  30

Pro Lys Cys Ala Asp Phe Gln Ser Ala Asn Leu Phe Glu Gly Thr Asp
        35                  40                  45

Leu Lys Val Gln Phe Leu Leu Phe Val Pro Ser Asn Pro Ser Cys Gly
    50                  55                  60

Gln Leu Val Glu Gly Ser Ser Asp Leu Gln Asn Ser Gly Phe Asn Ala
65                  70                  75                  80

Thr Leu Gly Thr Lys Leu Ile Ile His Gly Phe Arg Val Leu Gly Thr
                85                  90                  95

Lys Pro Ser Trp Ile Asp Thr Phe Ile Arg Thr Leu Leu Arg Ala Thr
            100                 105                 110

Asn Ala Asn Val Ile Ala Val Asp Trp Ile Tyr Gly Ser Thr Gly Val
        115                 120                 125

Tyr Phe Ser Ala Val Lys Asn Val Ile Lys Leu Ser Leu Glu Ile Ser
    130                 135                 140

Leu Phe Leu Asn Lys Leu Leu Val Leu Gly Val Ser Glu Ser Ser Ile
145                 150                 155                 160

His Ile Ile Gly Val Ser Leu Gly Ala His Val Gly Gly Met Val Gly
                165                 170                 175

Gln Leu Phe Gly Gly Gln Leu Gly Gln Ile Thr Gly Leu Asp Pro Ala
            180                 185                 190

Gly Pro Glu Tyr Thr Arg Ala Ser Val Glu Glu Arg Leu Asp Ala Gly
        195                 200                 205

Asp Ala Leu Phe Val Glu Ala Ile His Thr Asp Thr Asp Asn Leu Gly
    210                 215                 220

Ile Arg Ile Pro Val Gly His Val Asp Tyr Phe Val Asn Gly Gly Gln
225                 230                 235                 240

Asp Gln Pro Gly Cys Pro Thr Phe Phe Tyr Ala Gly Tyr Ser Tyr Leu
                245                 250                 255

Ile Cys Asp His Met Arg Ala Val His Leu Tyr Ile Ser Ala Leu Glu
            260                 265                 270

Asn Ser Cys Pro Leu Met Ala Phe Pro Cys Ala Ser Tyr Lys Ala Phe
        275                 280                 285

Leu Ala Gly Arg Cys Leu Asp Cys Phe Asn Pro Phe Leu Leu Ser Cys
    290                 295                 300

Pro Arg Ile Gly Leu Val Glu Gln Gly Gly Val Lys Ile Glu Pro Leu
305                 310                 315                 320

Pro Lys Glu Val Lys Val Tyr Leu Leu Thr Thr Ser Ser Ala Pro Tyr
                325                 330                 335

Cys Met His His Ser Leu Val Glu Phe His Leu Lys Glu Leu Arg Asn
            340                 345                 350

Lys Asp Thr Asn Ile Glu Val Thr Phe Leu Ser Ser Asn Ile Thr Ser
        355                 360                 365

Ser Ser Lys Ile Thr Ile Pro Lys Gln Gln Arg Tyr Gly Lys Gly Ile
    370                 375                 380

Ile Ala His Ala Thr Pro Gln Cys Gln Ile Asn Gln Val Lys Phe Lys
385                 390                 395                 400

Phe Gln Ser Ser Asn Arg Val Trp Lys Lys Asp Arg Thr Thr Ile Ile
                405                 410                 415

Gly Lys Phe Cys Thr Ala Leu Leu Pro Val Asn Asp Arg Glu Lys Met
            420                 425                 430

Val Cys Leu Pro Glu Pro Val Asn Leu Gln Ala Ser Val Thr Val Ser
        435                 440                 445

Cys Asp Leu Lys Ile Ala Cys Val
    450                 455

<210>  129
<211>  2238
<212>  DNA
<213>  Homo sapiens

<400>  129
   cgttgccggg tcgcaggtcc cgccagtgcg agcgcaacgg aggtcgaagg cgttcagact      60
   cttagctgaa cgcggagctg cggcggctat gctgtggagc ggctgccggc gtttcggggc     120
   gcgcctcggc tgcctgcccg gcggtctccg ggtcctcgtc cagaccggcc accggagctt     180
   gacctcctgc atcgaccctt ccatgggact taatgaagag cagaaagaat ttcaaaaagt     240
   ggcctttgac tttgctgccc gagagatggc tccaaatatg gcagagtggg accagaagga     300
   gctgttccca gtggatgtga tgcggaaggc agcccagcta ggcttcggag gggtctacat     360
   acaaacagat gtgggcgggt ctgggctgtc acgtcttgat acctctgtca tttttgaagc     420
   cttggctaca ggctgcacca gcaccacagc ctatataagc atccacaaca tgtgtgcctg     480
   gatgattgat agcttcggaa atgaggaaca gaggcacaaa ttttgcccac cgctctgtac     540
   catggagaag tttgcttcct actgcctcac tgaaccagga agtgggagtg atgctgcctc     600
   tcttctgacc tccgctaaga aacagggaga tcattacatc ctcaatggct ccaaggcctt     660
   catcagtggt gctggtgagt cagacatcta tgtggtcatg tgccgaacag gaggaccagg     720
   ccccaagggc atctcatgca tagttgttga gaaggggacc cctggcctca gctttggcaa     780
   gaaggagaaa aaggtggggt ggaactccca gccaacacga gctgtgatct tcgaagactg     840
   tgctgtccct gtggccaaca gaattgggag cgaggggcag ggcttcctca ttgccgtgag     900
   aggactgaac ggagggagga tcaatattgc ttcctgctcc ctgggggctg cccacgcctc     960
   tgtcatcctc acccgagacc acctcaatgt ccggaagcag tttggagagc ctctggccag    1020
   taaccagtac ttgcaattca cactggctga tatggcaaca aggctggtgg ccgcgcggct    1080
   gatggtccgc aatgcagcag tggctctgca ggaggagagg aaggatgcag tggccttgtg    1140
   ctccatggcc aagctctttg ctacagatga atgctttgcc atctgcaacc aggccttgca    1200
   gatgcacggg ggctacggct acctgaagga ttacgctgtt cagcagtacg tgcgggactc    1260
   cagggtccac cagattctag aaggtagcaa tgaagtgatg aggatactga tctctagaag    1320
   cctgcttcag gagtagaacc cacacttgtt ctggcctggt gttcagtgcg actgcagtca    1380
   gtgttgagtg gtgccatgtg ggccgctcta ttccaaagga atcatggatt agacccaagg    1440
   gctgagctcc tctagggcag gacctgcacc ctgtgtgttg gcaccagcat cgggtcttgg    1500
   actggggcag aatccccagt ggaaccggaa gagctggact gatgagaaac atcagaagaa    1560
   cacatactac cttgttttcc taatgccaga agggtgacca gtgaagattc accgtcaaac    1620
   catgaaagtc ctttcttgga tccactttat cttgattagt ctgcatttta ctagttcact    1680
   ggatccctcc tctaggggcc tggggacttt cactgatgct cttcctgatt ctagagcaaa    1740
   ggtgtgggaa ggggaaatgg aggaatgccc tcctgtctgt gtcgttctct gtgccacagc 1800
   tacagatgca gaaggtttct ctggatagca cacctctgaa tgtaaatcat gataaaatgg 1860
   atatttggaa acttactcct aagctgtgat gtagggtgta tttctacttc tggactgcct 1920
   caatatcaag ggctgagact tttgaatgtt gaatattcgt tgggtttcat gttaagacgc 1980
   ctgtggtcca ggagtgctat tcagtgtttc tgttcctgat aaacactttg aatatttttt 2040
   tgtgtttttg tttccttttc tgaagctgtt cctcctttta aatattttta atcacattga 2100
   taaaatctat ccttcatcca cctctggttc tactatagtt gatttttatt ttaaatgttt 2160
   aattgtattt gattaaacac ttaactggat tttggaataa taaaactctc gtccaatttg 2220
   gcttttaaaa aaaaaaaa 2238

<210> 130
<211> 415
<212> PRT
<213> Homo sapiens

<400> 130

<210>  131
<211>  6735
<212>  DNA
<213>  Homo sapiens

<400>  131
gcctgcagag agaggcactt tgcaccacag acagatagca agaagggaaa gacagagagt      60

gagaaaaaag aggagtcagt cgctcctggg gaagggagag agtgagactg ggagaaagag     120

aagcacagaa agtgtgtgta aaacggagta aagaaagaaa aaaaaaaaac tacccttaaa     180

gcacatttaa aaaaaaaaaa ctctggcaat tcaagaaaga aacaggctac gtttaaagag     240

catagagaca atgaaaggct aaagaaaatt ttaaaatctc tgccacagtc tcataggtgc     300

ttggaaatga aagtagaact gcctgtcttt aacggactct gacagaggta actggattag     360

ggacgagtac gccagctttt tttttttttt tttttttttt tttaacatct taaatcctga     420

aaaaaaaaaa aaaaaaaaaa aaaaggcagc agctccgaat tgaatgaatt gatgggcaca     480

ctccaactgc tgggctggag agactggact tagtcttgcc atttctgctt ctttgaaaga     540

ggagacaact tgggcttcct tttaatttag ttttttttcc ccttctcccc caacccccaa     600

ccttccccct tacctccccc acccccttta tcaccacccc ccttttaaat aagagggtga     660

aggggaacca gagcgcacaa gggaactgac tcaggaggca gagaagatgg gcatcctcag     720

cgtagacttg ctgatcacac tgcaaattct gccagttttt ttctccaact gcctcttcct     780

ggctctctat gactcggtca ttctgctcaa gcacgtggtg ctgctgttga gccgctccaa     840

gtccactcgc ggagagtggc ggcgcatgct gacctcagag ggactgcgct gcgtctggaa 900
gagcttcctc ctcgatgcct acaaacaggt gaaattgggt gaggatgccc ccaattccag 960
tgtggtgcat gtctccagta cagaaggagg tgacaacagt ggcaatggta cccaggagaa 1020
gatagctgag ggagccacat gccaccttct tgactttgcc agccctgagc gcccactagt 1080
ggtcaacttt ggctcagcca cttgacctcc tttcacgagc cagctgccag ccttccgcaa 1140
actggtggaa gagttctcct cagtggctga cttcctgctg gtctacattg atgaggctca 1200
tccatcagat ggctgggcga taccggggga ctcctctttg tcttttgagg tgaagaagca 1260
ccagaaccag gaagatcgat gtgcagcagc ccagcagctt ctggagcgtt tctccttgcc 1320
gccccagtgc cgagttgtgg ctgaccgcat ggacaataac gccaacatag cttacggggt 1380
agcctttgaa cgtgtgtgca ttgtgcagag acagaaaatt gcttatctgg gaggaaaggg 1440
ccccttctcc tacaaccttc aagaagtccg gcattggctg gagaagaatt tcagcaagag 1500
atgaaagaaa actagattag ctggttaaag gtatgattat aagagagctt attgttttaa 1560
aaagttatat aaaggcaagg aaattaagaa ctgaatccat atttcaacag agccctattg 1620
gcttactgaa agacaggagt ttatctatcg gaagaacatg aatctctaac agctccatac 1680
ttctttcact actcaaatgg cattgggctg agtaagtaac catatcacct ctcttcttag 1740
taaaaagccc tatgtgaaaa gatcccaaga tggagaggaa gaaacgctaa ttcagcatgt 1800
gttcattctg cattgagaag gaactgatac atctgatgca tgctttgaga ccagaagaaa 1860
agacttacct gaataattac tacattaggg aagctactgt ctacgttaag ataaagggta 1920
ttgccttggc tctatttggc atggatggag cccagttgga aaattcccaa atattacaac 1980
aagtccttga acccaggcca tgtggttaga cgttggtgtt aaggttagac cttatgttag 2040
agtcatttct gatgttccag cttctagcca tgtagtgctc tcagtcttca taccccagaa 2100
attattggta tatttgtaga taccgagaat gatccctcag tctgagaggt tagaatgatc 2160
atctgtaatc tgagggttaa tttctaggca ggtggagaga gtggtaaaaa agaaatgaaa 2220
ttgacaagct aggaaagagg aggcagaaag atttggaaaa ttcacagagt ttcaccctta 2280
agctgtagag agtgggtcac atttgttagc cacggaaaca tagaaacata cacaaggcca 2340
gaaaaagaag aaggagctca actaaaagtg gcatagagaa tacacatata aaaacaatat 2400
atttgtcata tgctcctaga gaggagaaag gggtgattga aagaaaaaaa aatacttaaa 2460
tatttgtaat tgtgaggggt ttcttttgga aataattact tttgaaccat gtatgtggta 2520
tgtatatttt cagtgggtta attatacccc atgataccta ttaaaggaaa accagtgggt 2580
ctggtggtgc tggtcttttc ctccccattc ctacaatttc tatgtggccc aagtcattcc 2640
taatcttggt ctctatagca gtgttctctc tgaatgctga gctgaagaaa ttatacgtac 2700
atacacacat acatacatac atacaaatat atgtatatat attctcagct gctgcgggag 2760
gtaggtacca tggccattca gcacagcctt gatttcctcc caaagtaggt gagctatagt 2820
gaagaatagg tgcaaacaaa caagcttact tccattgcaa aatagaagaa gaggaagtta 2880

gagataattc tgatcaatca ttttggaggc tttgttataa ggcaaccccc ggtatatcat 2940
ggaatttcca ttgacatttg aatttggact tggatcttcc cttggtccca ttagctgagg 3000
tttagtaatc taaagtccct atagtatatg attataatgc tattttaaaa aatatatata 3060
taaaatattt ttttcttttt aaaatagaca ctatagtttt acccataagt aatatttaaa 3120
gattatagct cccaaaagaa tggaccaacc actttcgtat cataatttct ttttggtaaa 3180
tatgagacta ttatgaaatc atagtatatg attgtattta aaggtacaat caaaggatct 3240
tttgtccatt ccattaataa ctgaataaaa aataaataaa atggatagaa aaaaactaaa 3300
gttgaaaata cattcttaaa ctagttgtct gaaatgagaa aagagtgaga actaggtgtg 3360
caagaaccaa acgtatttta ttttattttt taaatgggag caacatatca gtcgtgtcac 3420
cagctggtat attgtgtaaa tattaaagct ccattgggac tgatttttca tggcaacatc 3480
agctttctaa tgttctaaat tctataaaaa ccacccacaa agaaacaaag caaatttcat 3540
tatctaatga gttgctggaa aatcatattg agaataatta tttcagattc ctcagttgtt 3600
aacttctaca ttcaagggct tatctctgcc cccattgatt tttaacctca aaatggtgtg 3660
agatttactg tggaacccta aagcagtaaa ataaaaaacc tggttgcagc acattcacac 3720
tgttgtcctt aaaattcccc ttttttctct atgtacgata aagtaacagt atgtcagata 3780
agccggtggg gggatgagat taggctgagg cagtgctagt caactggggg aaaaggatga 3840
tggaaaaatc acccagttgt gctatatttt taaagaagga ggtcgtttat gtgtgcagac 3900
aattctccct gaggttagcc caatggagaa atgaagcaga ggaaggaaac atagaaagac 3960
atgggctatc agggaggaag atgttcaata gaacatgcaa gaatttctgg aagaaaggct 4020
gtggaagggc caatggagaa aatgaatgga caaagctcag gaatccctac gctatgtaga 4080
atgttcttgg tgttatcagg gttaagccct gtaattatgt aacctattta tcgcaacatg 4140
aatttttatg atttcttgtg atgtattctt ttatgaaatt aacaagaact cattattttg 4200
aggtagagga aaatcaatgc tttatctgat atgctgagaa attattagat tgccaatact 4260
catgtgcgtt tcatgtgttt tataaggttt gttcctttga agaattgtag ttcttagtcc 4320
cacagggaaa tgtgtatcta tttatatatc atagtataaa tctatgatat atttatatca 4380
tatataaaag tctgagttct ctttcttagt ccctaatcat gtttctccca taggctgtgt 4440
ttacatggag ctatcggttt agccttttaa gcttcattag cttgtctatt attgaaatag 4500
tttccaagaa attttagata ttatcataac atctgggtct actcaaacac ttattgtttg 4560
aaagacttat gtcttggacc tatcaaaaac tgactttatt tattgcttag tgaaaatact 4620
agtgggatca acaatgattt tcttgaatgg gcatgaatgg agatgcccgc acagtaatgt 4680
agaaatgttt catacagcta ttaaaatgta actgacctcc ttagaggcag attagtaact 4740
gttcctactt tgtatagcta agtgacagtc acttaactta catgactttc ttttttcaca 4800
ttgggtctct ggtcctgtgt cttcacctca tttatagcac gtctccttga tttttggtag 4860
tatcaacttc ccagtgatct gttcagttaa gttcttctcc cgttaaccag gaagtgctta 4920

ttctctcatc acagtgggaa gaatagccta ttgtctttca ttttgcctga gtgtatttta 4980
ctatttgggc tctgaaataa aaattatgaa atatggtgag gtcacatgtt ggtgctgcct 5040
tgctgcataa aattctagga gggcaggtta ggagacagtt atgtatggcc tttcgggaaa 5100
attcaaaggg tgggattaca agggtgttcc tcaggcatgc ccctatgggc cctatgtgga 5160
agcaagaaga attgactgat ttacaggact tctctttatg tcaatcttaa gaggatggat 5220
gaatctggac atttgttcca cccgacctct gactgatggt ttggaaaata actttaatta 5280
ggatcatatg accattgaaa aaggaaaaat gtagactctg acttccgtcc cactgaagga 5340
ttaatgaaaa cctttactag catttagagc ttttcagaac atccccactg tcatgtgtct 5400
cagcagtgga gactgcaagt aaggctttta attttaggag gttttttttt tttttttttt 5460
ttcccctaaa tggtatggcc aaaagtcaga gttaaaatat atatagttag attccaactt 5520
cctccttcac tctaaaaata gaatccaaac ccactcttca tatatgcttc cagaatgggg 5580
cttaagtacc aatctctgct ttgcaatggg cacaatcttg gtcatgtcct gaggctctct 5640
aagaaaagag aggatctagg atgggagagc tagaaagttg ctaactggga agaacaaggc 5700
cctgaggggt tggtctacca atctgggaag atttgaaaac aaacttctcg caactgaagg 5760
aaggctgaag gctgctgcaa gtcattgagt gactttagga tgagcaaaac attgggccac 5820
ttcctaatgc cctatgtgta tagtaccaga agcaaggtct cagacttaac agacccagct 5880
ctgttccaag gtgagtctga accaatagaa agcaaacatg tgcagatatc caaacaagac 5940
tgctcatgca agtcggggct ggctacccgt cttaggcagc aacagcagag ctccagggag 6000
cttattcaat atttactgag acttcgaaga cccagcagat gtttaatgaa gtcactattt 6060
tggctcaaac cctccacttc tccccctccc ctcaaaaagc caacaggtaa acacataaat 6120
gaaagaaacc cacagaaggg gatgggaaat aaagaaaatt ctctcaagac ttctccaggc 6180
ccatgtcact ggtcagcgtg gtttttatgt gtattaggat tgggggatgt gaagaaataa 6240
gtatccagta ctttataacc aaagcaatta aatgatattg gggtagggaa tgttggccag 6300
ttttgtttag ttttgccatc acattgtcac ccagacctca cctagcccca agtaatcggg 6360
cgccccgaag agggagacag agatgtgcca gagttgaccc agtgtgcgga tgataactac 6420
tgacgaaaga gtcatcgacc tcagttagtg gttggatgta gtcacattag tttgcctctc 6480
cccatctttg tctccctggc aaggagaata tgcgggacat gatgctaaga gccctgggta 6540
aatgtggtga gaatgcacgc gtgcatatgc tacacatatg tgcttctcag ttgcagaaaa 6600
tgaactgctt tgggagatta tcagtagaaa gagtgttatc atattggtgc tgagtgctat 6660
gtgtgcttat acaatttgtt cttgtatttt aataaacttt gaataaaaga ataaaaaaaa 6720
aaaaaaaaaa aaaaa 6735

<210> 132
<211> 273
<212> PRT
<213> Homo sapiens

<220>
<221>  misc_feature
<222>  (133)..(133)
<223>  Xaa can be any naturally occurring amino acid

<220>
<221>  misc_feature
<222>  (266)..(266)
<223>  Xaa can be any naturally occurring amino acid

<400>  132

Met Gly Ile Leu Ser Val Asp Leu Leu Ile Thr Leu Gln Ile Leu Pro
1               5                   10                  15

Val Phe Phe Ser Asn Cys Leu Phe Leu Ala Leu Tyr Asp Ser Val Ile
            20                  25                  30

Leu Leu Lys His Val Val Leu Leu Leu Ser Arg Ser Lys Ser Thr Arg
        35                  40                  45

Gly Glu Trp Arg Arg Met Leu Thr Ser Glu Gly Leu Arg Cys Val Trp
    50                  55                  60

Lys Ser Phe Leu Leu Asp Ala Tyr Lys Gln Val Lys Leu Gly Glu Asp
65                  70                  75                  80

Ala Pro Asn Ser Ser Val Val His Val Ser Ser Thr Glu Gly Gly Asp
                85                  90                  95

Asn Ser Gly Asn Gly Thr Gln Glu Lys Ile Ala Glu Gly Ala Thr Cys
            100                 105                 110

His Leu Leu Asp Phe Ala Ser Pro Glu Arg Pro Leu Val Val Asn Phe
        115                 120                 125

Gly Ser Ala Thr Xaa Pro Pro Phe Thr Ser Gln Leu Pro Ala Phe Arg
    130                 135                 140

Lys Leu Val Glu Glu Phe Ser Ser Val Ala Asp Phe Leu Leu Val Tyr
145                 150                 155                 160

Ile Asp Glu Ala His Pro Ser Asp Gly Trp Ala Ile Pro Gly Asp Ser
                165                 170                 175

Ser Leu Ser Phe Glu Val Lys Lys His Gln Asn Gln Glu Asp Arg Cys
            180                 185                 190

Ala Ala Ala Gln Gln Leu Leu Glu Arg Phe Ser Leu Pro Pro Gln Cys
        195                 200                 205

Arg Val Val Ala Asp Arg Met Asp Asn Asn Ala Asn Ile Ala Tyr Gly
    210                 215                 220

Val Ala Phe Glu Arg Val Cys Ile Val Gln Arg Gln Lys Ile Ala Tyr
225                 230                 235                 240

Leu Gly Gly Lys Gly Pro Phe Ser Tyr Asn Leu Gln Glu Val Arg His
                245                 250                 255

Trp Leu Glu Lys Asn Phe Ser Lys Arg Xaa Lys Lys Thr Arg Leu Ala
            260                 265                 270

Gly

<210>  133

<211>  2685
<212>  DNA
<213>  Homo sapiens

<400>  133
caggcgtgtc ccagggggag ccccgctctg cagccctgtg cgccgtagag agctggactt      60

aggctggcag catggccgag ttcagggtca gggtgtccac cggagaagcc ttcggggctg     120

gcacatggga caaagtgtct gtcagcatcg tggggacccg gggagagagc cccccactgc     180

ccctggacaa tctcggcaag gagttcactg cgggcgctga ggaggacttc caggtgacgc     240

tcccggagga cgtaggccga gtgctgctgc tgcgcgtgca caaggcgccc ccagtgctgc     300

ccctgctggg gcccctggcc ccggatgcct ggttctgccg ctggttccag ctgacaccgc     360

cgcggggcgg ccacctcctc ttcccctgct accagtggct ggagggggcg gggaccctgg     420

tgctgcagga gggtacagcc aaggtgtcct gggcagacca ccaccctgtg ctccagcaac     480

agcgccagga ggagcttcag gcccggcagg agatgtacca gtggaaggct tacaacccag     540

gttggcctca ctgcctggat gaaaagacag tggaagactt ggagctcaat atcaaatact     600

ccacagccaa gaatgccaac ttttatctac aagctggctc tgcttttgca gagatgaaaa     660

tcaaggggtt gctggaccgc aaggggctct ggaggagtct gaatgagatg aaaaggatct     720

tcaacttccg gaggacccca gcagctgagc acgcatttga gcactggcag gaggatgcct     780

tcttcgcctc ccagttcctg aatggtctca accctgtcct gatccgccgc tgtcactacc     840

tcccaaagaa cttccccgtc actgatgcca tggtggcctc attgttgggt cctgggacca     900

gcttgcaggc tgagctagag aagggctccc tgttcttggt ggatcacggc atcctctctg     960

gcatccagac caatgtcatt aatgggaagc cgcagttctc tgcggcccca atgaccctgc    1020

tataccagag cccaggctgc gggccgctgc tgcctctcgc catccagctc agccagaccc    1080

ccggcccaaa cagccccatc ttcctgccca ctgatgacaa gtgggactgg ttgctggcca    1140

agacctgggt gcgcaatgcc gagttctcct tccatgaggc cctcacgcac ctgctgcact    1200

cacatctgct gcctgaggtc ttcaccctgg ctaccctgcg tcagctgccc cactgccacc    1260

ctctcttcaa gctgctgatc ccgcacaccc gatacaccct gcacatcaac acactcgccc    1320

gggagctgct tatcgtgcca gggcaggtgg tggacaggtc cacaggcatc ggcattgaag    1380

gcttctctga gttgatacag aggaacatga agcagctgaa ctattctctc ctgtgtctgc    1440

ctgaggatat ccggacccga ggagttgaag acatcccagg ctactactac cgtgatgatg    1500

ggatgcagat ttggggtgca gtggaacgct ttgtctctga aatcatcggt atctactacc    1560

caagtgatga gtctgtccaa gatgacagag agctccaggc ctgggtcaga gagatcttct    1620

ccaagggctt cctaaaccag gagagctcag gtatcccttc ctcactggag acccgggaag    1680

ccctggtgca gtatgtcacc atggtgatat tcacctgctc agccaagcat gcggctgtca    1740

gtgcagggca gtttgactcc tgtgcttgga tgcccaacct gccacccagc atgcagctgc    1800

caccacccac ctccaaaggc ctggcaacat gcgagggctt catagccacc ctcccacctg    1860

tcaatgccac atgtgatgtc atccttgctc tctggttgct gagcaaggag cctggagacc    1920

aaaggcccct gggcacctat ccggatgagc acttcacaga ggaggcccct cggcggagca 1980
tcgccacctt ccagagccgc ctggcccaga tctcgagggg catccaggag cggaaccggg 2040
gcctggtgct gccctacacc tacctagacc ctcccctcat cgagaacagc gtctccatct 2100
aaatcccagg ggaacacagg cccagatgac atccctttga ccacatcgct ctaggataac 2160
tggcacccag agaaaaggac tcctcagaaa aaacaggccc ccatgtgcct ctcctgggac 2220
aaccagactc tgtaactcac ccccaccacc atacacacac acaaaaacag aaacaaaatc 2280
aaaacagaga aagcagaaaa tctaccaaga acagagtctc aggacagaac cactgagtct 2340
tttggaggct ccaagcctca aagtgcccgc agagcccacc ttgagggttt tgctagttgg 2400
ttttgttttg cgtttacagc cgtgggggga agcacataat cccgccccag ggcccactag 2460
catccactga ttggacctta tggtcaccca actcaaggac agccaccaag aagtggctgc 2520
caaagagact gggcgcagtg gctcatgccc ataatcccag cactttggga gatggaggcg 2580
ggaaaatcat ttgaggtcag aagttcaagg ccagcctgga cgacatagcg agactccacc 2640
tctaccaaaa aataaaaatt aaaaaacaaa aaaaaaaaaa aaaaa 2685

<210> 134
<211> 676
<212> PRT
<213> Homo sapiens

<400> 134

Met Ala Glu Phe Arg Val Arg Val Ser Thr Gly Glu Ala Phe Gly Ala
1               5                   10                  15

Gly Thr Trp Asp Lys Val Ser Val Ser Ile Val Gly Thr Arg Gly Glu
            20                  25                  30

Ser Pro Pro Leu Pro Leu Asp Asn Leu Gly Lys Glu Phe Thr Ala Gly
        35                  40                  45

Ala Glu Glu Asp Phe Gln Val Thr Leu Pro Glu Asp Val Gly Arg Val
    50                  55                  60

Leu Leu Leu Arg Val His Lys Ala Pro Pro Val Leu Pro Leu Leu Gly
65                  70                  75                  80

Pro Leu Ala Pro Asp Ala Trp Phe Cys Arg Trp Phe Gln Leu Thr Pro
                85                  90                  95

Pro Arg Gly Gly His Leu Leu Phe Pro Cys Tyr Gln Trp Leu Glu Gly
            100                 105                 110

Ala Gly Thr Leu Val Leu Gln Glu Gly Thr Ala Lys Val Ser Trp Ala
        115                 120                 125

Asp His His Pro Val Leu Gln Gln Gln Arg Gln Glu Glu Leu Gln Ala
    130                 135                 140

Arg Gln Glu Met Tyr Gln Trp Lys Ala Tyr Asn Pro Gly Trp Pro His
145                 150                 155                 160

Cys Leu Asp Glu Lys Thr Val Glu Asp Leu Glu Leu Asn Ile Lys Tyr
                165                 170                 175

Ser Thr Ala Lys Asn Ala Asn Phe Tyr Leu Gln Ala Gly Ser Ala Phe

            180                 185                 190

Ala Glu Met Lys Ile Lys Gly Leu Leu Asp Arg Lys Gly Leu Trp Arg
        195                 200                 205

Ser Leu Asn Glu Met Lys Arg Ile Phe Asn Phe Arg Arg Thr Pro Ala
    210                 215                 220

Ala Glu His Ala Phe Glu His Trp Gln Glu Asp Ala Phe Phe Ala Ser
225                 230                 235                 240

Gln Phe Leu Asn Gly Leu Asn Pro Val Leu Ile Arg Arg Cys His Tyr
                245                 250                 255

Leu Pro Lys Asn Phe Pro Val Thr Asp Ala Met Val Ala Ser Leu Leu
            260                 265                 270

Gly Pro Gly Thr Ser Leu Gln Ala Glu Leu Glu Lys Gly Ser Leu Phe
        275                 280                 285

Leu Val Asp His Gly Ile Leu Ser Gly Ile Gln Thr Asn Val Ile Asn
    290                 295                 300

Gly Lys Pro Gln Phe Ser Ala Ala Pro Met Thr Leu Leu Tyr Gln Ser
305                 310                 315                 320

Pro Gly Cys Gly Pro Leu Leu Pro Leu Ala Ile Gln Leu Ser Gln Thr
                325                 330                 335

Pro Gly Pro Asn Ser Pro Ile Phe Leu Pro Thr Asp Asp Lys Trp Asp
            340                 345                 350

Trp Leu Leu Ala Lys Thr Trp Val Arg Asn Ala Glu Phe Ser Phe His
        355                 360                 365

Glu Ala Leu Thr His Leu Leu His Ser His Leu Leu Pro Glu Val Phe
    370                 375                 380

Thr Leu Ala Thr Leu Arg Gln Leu Pro His Cys His Pro Leu Phe Lys
385                 390                 395                 400

Leu Leu Ile Pro His Thr Arg Tyr Thr Leu His Ile Asn Thr Leu Ala
                405                 410                 415

Arg Glu Leu Leu Ile Val Pro Gly Gln Val Val Asp Arg Ser Thr Gly
            420                 425                 430

Ile Gly Ile Glu Gly Phe Ser Glu Leu Ile Gln Arg Asn Met Lys Gln
        435                 440                 445

Leu Asn Tyr Ser Leu Leu Cys Leu Pro Glu Asp Ile Arg Thr Arg Gly
    450                 455                 460

Val Glu Asp Ile Pro Gly Tyr Tyr Tyr Arg Asp Asp Gly Met Gln Ile
465                 470                 475                 480

Trp Gly Ala Val Glu Arg Phe Val Ser Glu Ile Ile Gly Ile Tyr Tyr
                485                 490                 495

Pro Ser Asp Glu Ser Val Gln Asp Asp Arg Glu Leu Gln Ala Trp Val
            500                 505                 510

Arg Glu Ile Phe Ser Lys Gly Phe Leu Asn Gln Glu Ser Ser Gly Ile
        515                 520                 525

Pro Ser Ser Leu Glu Thr Arg Glu Ala Leu Val Gln Tyr Val Thr Met
    530                 535                 540

Val Ile Phe Thr Cys Ser Ala Lys His Ala Ala Val Ser Ala Gly Gln
545                 550                 555                 560

Phe Asp Ser Cys Ala Trp Met Pro Asn Leu Pro Pro Ser Met Gln Leu
                565                 570                 575

Pro Pro Pro Thr Ser Lys Gly Leu Ala Thr Cys Glu Gly Phe Ile Ala
            580                 585                 590

Thr Leu Pro Pro Val Asn Ala Thr Cys Asp Val Ile Leu Ala Leu Trp
        595                 600                 605

Leu Leu Ser Lys Glu Pro Gly Asp Gln Arg Pro Leu Gly Thr Tyr Pro
    610                 615                 620

Asp Glu His Phe Thr Glu Glu Ala Pro Arg Arg Ser Ile Ala Thr Phe
625                 630                 635                 640

Gln Ser Arg Leu Ala Gln Ile Ser Arg Gly Ile Gln Glu Arg Asn Arg
                645                 650                 655

Gly Leu Val Leu Pro Tyr Thr Tyr Leu Asp Pro Pro Leu Ile Glu Asn
            660                 665                 670

Ser Val Ser Ile
        675

<210>  135
<211>  3742
<212>  DNA
<213>  Homo sapiens

<400>  135
gaattccttc tctcctcctc ctcgcccttc tcctcgccct cctcctcctc ctcgccctcc      60

cctcccgatc ctcatcccct tgccctcccc cagcccaggg acttttccgg aaagttttta     120

ttttccgtct gggctctcgg agaaagaagc tcctggctca gcggctgcaa aactttcctg     180

ctgccgcgcc gccagccccc gccctccgct gcccggccct gcgccccgcc gagcgatgag     240

cgcccctccg gtcctgcggc cgcccagtcc gctgctgccc gtggcggcgg cagctgccgc     300

agcggccgcc gcactggtcc cagggtccgg gcccgggccc gcgccgttct tggctcctgt     360

cgcggccccg gtcgggggca tctcgttcca tctgcagatc ggcctgagcc gtgagccggt     420

gctgctgctg caggactcgt ccggggacta cagcctggcg cacgtccgcg agatggcttg     480

ctccattgtc gaccagaagt tccctgaatg tggtttctac ggaatgtatg ataagatcct     540

gctttttcgc catgacccta cctctgaaaa catccttcag ctggtgaaag cggccagtga     600

tatccaggaa ggcgatctta ttgaagtggt cttgtcacgt tccgccacct ttgaagactt     660

tcagattcgt ccccacgctc tctttgttca ttcatacaga gctccagctt tctgtgatca     720

ctgtggagaa atgctgtggg ggctggtacg tcaaggtctt aaatgtgaag ggtgtggtct     780

gaattaccat aagagatgtg catttaaaat acccaacaat tgcagcggtg tgaggcggag     840

aaggctctca aacgtttccc tcactggggt cagcaccatc cgcacatcat ctgctgaact     900

ctctacaagt gcccctgatg agccccttct gcaaaaatca ccatcagagt cgtttattgg     960

tcgagagaag aggtcaaatt ctcaatcata cattggacga ccaattcacc ttgacaagat    1020

tttgatgtct aaagttaaag tgccgcacac atttgtcatc cactcctaca cccggcccac    1080

agtgtgccag tactgcaaga agcttctgaa ggggcttttc aggcagggct tgcagtgcaa 1140
agattgcaga ttcaactgcc ataaacgttg tgcaccgaaa gtaccaaaca actgccttgg 1200
cgaagtgacc attaatggag atttgcttag ccctggggca gagtctgatg tggtcatgga 1260
agaagggagt gatgacaatg atagtgaaag gaacagtggg ctcatggatg atatggaaga 1320
agcaatggtc caagatgcag agatggcaat ggcagagtgc cagaacgaca gtggcgagat 1380
gcaagatcca gacccagacc acgaggacgc caacagaacc atcagtccat caacaagcaa 1440
caatatccca ctcatgaggg tagtgcagtc tgtcaaacac acgaagagga aaagcagcac 1500
agtcatgaaa gaaggatgga tggtccacta caccagcaag gacacgctgc ggaaacggca 1560
ctattggaga ttggatagca aatgtattac cctctttcag aatgacacag gaagcaggta 1620
ctacaaggaa attcctttat ctgaaatttt gtctctggaa ccagtaaaaa cttcagcttt 1680
aattcctaat ggggccaatc ctcattgttt cgaaatcact acggcaaatg tagtgtatta 1740
tgtgggagaa aatgtggtca atccttccag cccatcacca aataacagtg ttctcaccag 1800
tggcgttggt gcagatgtgg ccaggatgtg ggagatagcc atccagcatg cccttatgcc 1860
cgtcattccc aagggctcct ccgtgggtac aggaaccaac ttgcacagag atatctctgt 1920
gagtatttca gtatcaaatt gccagattca agaaaatgtg gacatcagca cagtatatca 1980
gatttttcct gatgaagtac tgggttctgg acagtttgga attgtttatg gaggaaaaca 2040
tcgtaaaaca ggaagagatg tagctattaa aatcattgac aaattacgat ttccaacaaa 2100
acaagaaagc cagcttcgta atgaggttgc aattctacag aaccttcatc accctggtgt 2160
tgtaaatttg gagtgtatgt ttgagacgcc tgaaagagtg tttgttgtta tggaaaaact 2220
ccatggagac atgctggaaa tgatcttgtc aagtgaaaag ggcaggttgc cagagcacat 2280
aacgaagttt ttaattactc agatactcgt ggctttgcgg caccttcatt ttaaaaatat 2340
cgttcactgt gacctcaaac cagaaaatgt gttgctagcc tcagctgatc cttttcctca 2400
ggtgaaactt tgtgattttg gttttgcccg gatcattgga gagaagtctt tccggaggtc 2460
agtggtgggt acccccgctt acctggctcc tgaggtccta aggaacaagg gctacaatcg 2520
ctctctagac atgtggtctg ttggggtcat catctatgta agcctaagcg gcacattccc 2580
atttaatgaa gatgaagaca tacacgacca aattcagaat gcagctttca tgtatccacc 2640
aaatccctgg aaggaaatat ctcatgaagc cattgatctt atcaacaatt tgctgcaagt 2700
aaaaatgaga aagcgctaca gtgtggataa gaccttgagc cacccttggc tacaggacta 2760
tcagacctgg ttagatttgc gagagctgga atgcaaaatc ggggagcgct acatcaccca 2820
tgaaagtgat gacctgaggt gggagaagta tgcaggcgag cagcggctgc agtaccccac 2880
acacctgatc aatccaagtg ctagccacag tgacactcct gagactgaag aaacagaaat 2940
gaaagccctc ggtgagcgtg tcagcatcct ctgagttcca tctcctataa tctgtcaaaa 3000
cactgtggaa ctaataaata catacggtca ggtttaacat ttgccttgca gaactgccat 3060
tattttctgt cagatgagaa caaagctgtt aaactgttag cactgttgat gtatctgagt 3120

tgccaagaca aatcaacaga agcatttgta ttttgtgtga ccaactgtgt tgtattaaca 3180

aaagttccct gaaacacgaa acttgttatt gtgaatgatt catgttatat ttaatgcatt 3240

aaacctgtct ccactgtgcc tttgcaaatc agtgtttttc ttactggagc ttcattttgg 3300

taagagacag aatgtatctg tgaagtagtt ctgtttggtg tgtcccattg gtgttgtcat 3360

tgtaaacaaa ctcttgaaga gtcgattatt tccagtgttc tatgaacaac tccaaaaccc 3420

atgtgggaaa aaaatgaatg aggagggtag ggaataaaat cctaagacac aaatgcatga 3480

acaagtttta atgtatagtt ttgaatcctt tgcctgcctg gtgtgcctca gtatatttaa 3540

actcaagaca atgcacctag ctgtgcaaga cctagtgctc ttaagcctaa atgccttaga 3600

aatgtaaact gccatatata acagatacat ttccctcttt cttataatac tctgttgtac 3660

tatggaaaat cagctgctca gcaacctttc acctttgtgt atttttcaat aataaaaaat 3720

attcttgtca aaaaaaaaaa aa 3742

<210> 136
<211> 912
<212> PRT
<213> Homo sapiens

<400> 136

Met Ser Ala Pro Pro Val Leu Arg Pro Pro Ser Pro Leu Leu Pro Val
1               5                   10                  15

Ala Ala Ala Ala Ala Ala Ala Ala Ala Ala Leu Val Pro Gly Ser Gly
            20                  25                  30

Pro Gly Pro Ala Pro Phe Leu Ala Pro Val Ala Ala Pro Val Gly Gly
        35                  40                  45

Ile Ser Phe His Leu Gln Ile Gly Leu Ser Arg Glu Pro Val Leu Leu
    50                  55                  60

Leu Gln Asp Ser Ser Gly Asp Tyr Ser Leu Ala His Val Arg Glu Met
65                  70                  75                  80

Ala Cys Ser Ile Val Asp Gln Lys Phe Pro Glu Cys Gly Phe Tyr Gly
                85                  90                  95

Met Tyr Asp Lys Ile Leu Leu Phe Arg His Asp Pro Thr Ser Glu Asn
            100                 105                 110

Ile Leu Gln Leu Val Lys Ala Ala Ser Asp Ile Gln Glu Gly Asp Leu
        115                 120                 125

Ile Glu Val Val Leu Ser Arg Ser Ala Thr Phe Glu Asp Phe Gln Ile
    130                 135                 140

Arg Pro His Ala Leu Phe Val His Ser Tyr Arg Ala Pro Ala Phe Cys
145                 150                 155                 160

Asp His Cys Gly Glu Met Leu Trp Gly Leu Val Arg Gln Gly Leu Lys
                165                 170                 175

Cys Glu Gly Cys Gly Leu Asn Tyr His Lys Arg Cys Ala Phe Lys Ile
            180                 185                 190

Pro Asn Asn Cys Ser Gly Val Arg Arg Arg Arg Leu Ser Asn Val Ser
        195                 200                 205

Leu Thr Gly Val Ser Thr Ile Arg Thr Ser Ser Ala Glu Leu Ser Thr
    210                 215                 220

Ser Ala Pro Asp Glu Pro Leu Leu Gln Lys Ser Pro Ser Glu Ser Phe
225                 230                 235                 240

Ile Gly Arg Glu Lys Arg Ser Asn Ser Gln Ser Tyr Ile Gly Arg Pro
                245                 250                 255

Ile His Leu Asp Lys Ile Leu Met Ser Lys Val Lys Val Pro His Thr
            260                 265                 270

Phe Val Ile His Ser Tyr Thr Arg Pro Thr Val Cys Gln Tyr Cys Lys
        275                 280                 285

Lys Leu Leu Lys Gly Leu Phe Arg Gln Gly Leu Gln Cys Lys Asp Cys
    290                 295                 300

Arg Phe Asn Cys His Lys Arg Cys Ala Pro Lys Val Pro Asn Asn Cys
305                 310                 315                 320

Leu Gly Glu Val Thr Ile Asn Gly Asp Leu Leu Ser Pro Gly Ala Glu
                325                 330                 335

Ser Asp Val Val Met Glu Glu Gly Ser Asp Asp Asn Asp Ser Glu Arg
            340                 345                 350

Asn Ser Gly Leu Met Asp Asp Met Glu Glu Ala Met Val Gln Asp Ala
        355                 360                 365

Glu Met Ala Met Ala Glu Cys Gln Asn Asp Ser Gly Glu Met Gln Asp
    370                 375                 380

Pro Asp Pro Asp His Glu Asp Ala Asn Arg Thr Ile Ser Pro Ser Thr
385                 390                 395                 400

Ser Asn Asn Ile Pro Leu Met Arg Val Val Gln Ser Val Lys His Thr
                405                 410                 415

Lys Arg Lys Ser Ser Thr Val Met Lys Glu Gly Trp Met Val His Tyr
            420                 425                 430

Thr Ser Lys Asp Thr Leu Arg Lys Arg His Tyr Trp Arg Leu Asp Ser
        435                 440                 445

Lys Cys Ile Thr Leu Phe Gln Asn Asp Thr Gly Ser Arg Tyr Tyr Lys
    450                 455                 460

Glu Ile Pro Leu Ser Glu Ile Leu Ser Leu Glu Pro Val Lys Thr Ser
465                 470                 475                 480

Ala Leu Ile Pro Asn Gly Ala Asn Pro His Cys Phe Glu Ile Thr Thr
                485                 490                 495

Ala Asn Val Val Tyr Tyr Val Gly Glu Asn Val Val Asn Pro Ser Ser
            500                 505                 510

Pro Ser Pro Asn Asn Ser Val Leu Thr Ser Gly Val Gly Ala Asp Val
        515                 520                 525

Ala Arg Met Trp Glu Ile Ala Ile Gln His Ala Leu Met Pro Val Ile
    530                 535                 540

Pro Lys Gly Ser Ser Val Gly Thr Gly Thr Asn Leu His Arg Asp Ile
545                 550                 555                 560

Ser Val Ser Ile Ser Val Ser Asn Cys Gln Ile Gln Glu Asn Val Asp

                565                 570                 575
Ile Ser Thr Val Tyr Gln Ile Phe Pro Asp Glu Val Leu Gly Ser Gly
            580                 585                 590
Gln Phe Gly Ile Val Tyr Gly Gly Lys His Arg Lys Thr Gly Arg Asp
        595                 600                 605
Val Ala Ile Lys Ile Ile Asp Lys Leu Arg Phe Pro Thr Lys Gln Glu
    610                 615                 620
Ser Gln Leu Arg Asn Glu Val Ala Ile Leu Gln Asn Leu His His Pro
625                 630                 635                 640
Gly Val Val Asn Leu Glu Cys Met Phe Glu Thr Pro Glu Arg Val Phe
                645                 650                 655
Val Val Met Glu Lys Leu His Gly Asp Met Leu Glu Met Ile Leu Ser
            660                 665                 670
Ser Glu Lys Gly Arg Leu Pro Glu His Ile Thr Lys Phe Leu Ile Thr
        675                 680                 685
Gln Ile Leu Val Ala Leu Arg His Leu His Phe Lys Asn Ile Val His
    690                 695                 700
Cys Asp Leu Lys Pro Glu Asn Val Leu Leu Ala Ser Ala Asp Pro Phe
705                 710                 715                 720
Pro Gln Val Lys Leu Cys Asp Phe Gly Phe Ala Arg Ile Ile Gly Glu
                725                 730                 735
Lys Ser Phe Arg Arg Ser Val Val Gly Thr Pro Ala Tyr Leu Ala Pro
            740                 745                 750
Glu Val Leu Arg Asn Lys Gly Tyr Asn Arg Ser Leu Asp Met Trp Ser
        755                 760                 765
Val Gly Val Ile Ile Tyr Val Ser Leu Ser Gly Thr Phe Pro Phe Asn
    770                 775                 780
Glu Asp Glu Asp Ile His Asp Gln Ile Gln Asn Ala Ala Phe Met Tyr
785                 790                 795                 800
Pro Pro Asn Pro Trp Lys Glu Ile Ser His Glu Ala Ile Asp Leu Ile
                805                 810                 815
Asn Asn Leu Leu Gln Val Lys Met Arg Lys Arg Tyr Ser Val Asp Lys
            820                 825                 830
Thr Leu Ser His Pro Trp Leu Gln Asp Tyr Gln Thr Trp Leu Asp Leu
        835                 840                 845
Arg Glu Leu Glu Cys Lys Ile Gly Glu Arg Tyr Ile Thr His Glu Ser
    850                 855                 860
Asp Asp Leu Arg Trp Glu Lys Tyr Ala Gly Glu Gln Arg Leu Gln Tyr
865                 870                 875                 880
Pro Thr His Leu Ile Asn Pro Ser Ala Ser His Ser Asp Thr Pro Glu
                885                 890                 895
Thr Glu Glu Thr Glu Met Lys Ala Leu Gly Glu Arg Val Ser Ile Leu
            900                 905                 910

<210> 137
<211> 3903
<212> DNA

<213>  Homo sapiens

<400>  137
aatggtcagt caatacatta taacataata caccaaatgc tagaatagaa ggggaggggg      60

gcacacataa tgactcactg ctggaagaag ggtgcatcag tgaattaaaa aatgtccctc     120

ccctcttcag cactcagcgc gcagctattt ccttctgcca gtctctttga actctggatc     180

tttgcttttg ctcgctgctc tcctgttttt cattctccac attttctcaa tcctctttct     240

ttatccttag ccaccctgct tttttcctcc ttttttaaaa aatcggagat ttcgtcttaa     300

aatgatttgt cttccttacc ttcgtccatt tcaacactga aggctgcaaa gaacttcacc     360

tttcccctag tggtatttaa aaattctcaa tccgtaaaaa gtctttttga aaggcaaagg     420

aacaggaccc agaccctctc gacacccttg atccgagtca gatctgcact agcaaccaga     480

actaatattt catttaaccc accaaaaggg ggaggcgaga ggagccagaa gcaaacttca     540

tctgtctcag acggatccgt ggttcctaca tttggaggag ccgcgtgtca gaaggcgtag     600

gaccccaagg ggggacaagg aggactcccg agtctccctt ctccgctctc cgagaccgaa     660

gaggtggact gagccgctcg ggacagcggc accggaggag gctcggagaa gatgcggggc     720

tcggggcccc ggggtgcggg acaccggcgg cccccaagcg gcggcggcga cacccccatc     780

accccagcgt ccctggccgg ctgctactct gcacctcgac gggctcccct ctggacgtgc     840

cttctcctgt gcgccgcact ccggaccctc ctggccagcc ccagcaacga agtgaattta     900

ttggattcac gcactgtcat gggggacctg ggatggattg cttttccaaa aaatgggtgg     960

gaagagattg gtgaagtgga tgaaaattat gcccctatcc acacatacca agtatgcaaa    1020

gtgatggaac agaatcagaa taactggctt ttgaccagtt ggatctccaa tgaaggtgct    1080

tccagaatct tcatagaact caaatttacc ctgcgggact gcaacagcct tcctggagga    1140

ctggggacct gtaaggaaac ctttaatatg tattactttg agtcagatga tcagaatggg    1200

agaaacatca aggaaaacca atacatcaaa attgatacca ttgctgccga tgaaagcttt    1260

acagaacttg atcttggtga ccgtgttatg aaactgaata cagaggtcag agatgtagga    1320

cctctaagca aaaagggatt ttatcttgct tttcaagatg ttggtgcttg cattgctctg    1380

gtttctgtgc gtgtatacta taaaaaatgc ccttctgtgg tacgacactt ggctgtcttc    1440

cctgacacca tcactggagc tgattcttcc caattgctcg aagtgtcagg ctcctgtgtc    1500

aaccattctg tgaccgatga acctcccaaa atgcactgca gcgccgaagg ggagtggctg    1560

gtgcccatcg ggaaatgcat gtgcaaggca ggatatgaag agaaaaatgg cacctgtcaa    1620

gtgtgcagac ctgggttctt caaagcctca cctcacatcc agagctgcgg caaatgtcca    1680

cctcacagtt atacccatga ggaagcttca acctcttgtg tctgtgaaaa ggattatttc    1740

aggagagagt ctgatccacc cacaatggca tgcacaagac ccccctctgc tcctcggaat    1800

gccatctcaa atgttaatga aactagtgtc tttctggaat ggattccgcc tgctgacact    1860

ggtggaagga aagacgtgtc atattatatt gcatgcaaga agtgcaactc ccatgcaggt    1920

gtgtgtgagg agtgtggcgg tcatgtcagg taccttcccc ggcaaagcgg cctgaaaaac    1980

acctctgtca tgatggtgga tctactcgct cacacaaact atacctttga gattgaggca 2040
gtgaatggag tgtccgactt gagcccagga gcccggcagt atgtgtctgt aaatgtaacc 2100
acaaatcaag cagctccatc tccagtcacc aatgtgaaaa aagggaaaat tgcaaaaaac 2160
agcatctctt tgtcttggca agaaccagat cgtcccaatg gaatcatcct agagtatgaa 2220
atcaagcatt ttgaaaagga ccaagagacc agctacacga ttatcaaatc taaagagaca 2280
actattactg cagagggctt gaaaccagct tcagtttatg tcttccaaat tcgagcacgt 2340
acagcagcag gctatggtgt cttcagtcga agatttgagt ttgaaaccac cccagtgttt 2400
gcagcatcca gcgatcaaag ccagattcct gtaattgctg tgtctgtgac agtaggagtc 2460
attttgttgg cagtggttat cggcgtcctc ctcagtggaa gttgctgcga atgtggctgt 2520
gggagggctt cttccctgtg cgctgttgcc catccaatcc taatatggcg gtgtggctac 2580
agcaaagcaa aacaagatcc agaagaggaa aagatgcatt ttcataatgg gcacattaaa 2640
ctgccaggag taagaactta cattgatcca catacctatg aggatcccaa tcaagctgtc 2700
cacgaatttg ccaaggagat agaagcatca tgtatcacca ttgagagagt tattggagca 2760
ggtgaatttg gtgaagtttg tagtggacgt ttgaaactac caggaaaaag agaattacct 2820
gtggctatca aaacccttaa agtaggctat actgaaaagc aacgcagaga tttcctaggt 2880
gaagcaagta tcatgggaca gtttgatcat cctaacatca tccatttaga aggtgtggtg 2940
accaaaagta aaccagtgat gatcgtgaca gagtatatgg agaatggctc tttagataca 3000
tttttgaaga aaaacgatgg gcagttcact gtgattcagc ttgttggcat gctgagaggt 3060
atctctgcag gaatgaagta cctttctgac atgggctatg tgcatagaga tcttgctgcc 3120
agaaacatct taatcaacag taaccttgtg tgcaaagtgt ctgactttgg actttcccgg 3180
gtactggaag atgatcccga ggcagcctac accacaaggg gaggaaaaat tccaatcaga 3240
tggactgccc cagaagcaat agctttccga aagtttactt ctgccagtga tgtctggagt 3300
tatggaatag taatgtggga agttgtgtct tatggagaga gaccctactg ggagatgacc 3360
aatcaagatg tgattaaagc ggtagaggaa ggctatcgtc tgccaagccc catggattgt 3420
cctgctgctc tctatcagtt aatgctggat tgctggcaga aagagcgaaa tagcaggccc 3480
aagtttgatg aaatagtcaa catgttggac aagctgatac gtaacccaag tagtctgaag 3540
acgctggtta atgcatcctg cagagtatct aatttattgg cagaacatag cccactagga 3600
tctggggcct acagatcagt aggtgaatgg ctagaggcaa tcaagatggg ccggtataca 3660
gagattttca tggaaaatgg atacagttca atggacgctg tggctcaggt gaccttggag 3720
gatttgagac ggcttggagt gactcttgtc ggtcaccaga agaagatcat gaacagcctt 3780
caagaaatga aggtgcagct ggtaaacgga atggtgccat tgtaacttca tgtaaatgtc 3840
gcttcttcaa gtgaatgatt ctgcactttg taaacagcac tgagatttat tttaacaaaa 3900
aaa 3903
<210> 138

<211>  1037
<212>  PRT
<213>  Homo sapiens

<400>  138

Met Arg Gly Ser Gly Pro Arg Gly Ala Gly His Arg Arg Pro Pro Ser
1               5                   10                  15

Gly Gly Gly Asp Thr Pro Ile Thr Pro Ala Ser Leu Ala Gly Cys Tyr
            20                  25                  30

Ser Ala Pro Arg Arg Ala Pro Leu Trp Thr Cys Leu Leu Leu Cys Ala
        35                  40                  45

Ala Leu Arg Thr Leu Leu Ala Ser Pro Ser Asn Glu Val Asn Leu Leu
    50                  55                  60

Asp Ser Arg Thr Val Met Gly Asp Leu Gly Trp Ile Ala Phe Pro Lys
65                  70                  75                  80

Asn Gly Trp Glu Glu Ile Gly Glu Val Asp Glu Asn Tyr Ala Pro Ile
                85                  90                  95

His Thr Tyr Gln Val Cys Lys Val Met Glu Gln Asn Gln Asn Asn Trp
            100                 105                 110

Leu Leu Thr Ser Trp Ile Ser Asn Glu Gly Ala Ser Arg Ile Phe Ile
        115                 120                 125

Glu Leu Lys Phe Thr Leu Arg Asp Cys Asn Ser Leu Pro Gly Gly Leu
    130                 135                 140

Gly Thr Cys Lys Glu Thr Phe Asn Met Tyr Tyr Phe Glu Ser Asp Asp
145                 150                 155                 160

Gln Asn Gly Arg Asn Ile Lys Glu Asn Gln Tyr Ile Lys Ile Asp Thr
                165                 170                 175

Ile Ala Ala Asp Glu Ser Phe Thr Glu Leu Asp Leu Gly Asp Arg Val
            180                 185                 190

Met Lys Leu Asn Thr Glu Val Arg Asp Val Gly Pro Leu Ser Lys Lys
        195                 200                 205

Gly Phe Tyr Leu Ala Phe Gln Asp Val Gly Ala Cys Ile Ala Leu Val
    210                 215                 220

Ser Val Arg Val Tyr Tyr Lys Lys Cys Pro Ser Val Val Arg His Leu
225                 230                 235                 240

Ala Val Phe Pro Asp Thr Ile Thr Gly Ala Asp Ser Ser Gln Leu Leu
                245                 250                 255

Glu Val Ser Gly Ser Cys Val Asn His Ser Val Thr Asp Glu Pro Pro
            260                 265                 270

Lys Met His Cys Ser Ala Glu Gly Glu Trp Leu Val Pro Ile Gly Lys
        275                 280                 285

Cys Met Cys Lys Ala Gly Tyr Glu Glu Lys Asn Gly Thr Cys Gln Val
    290                 295                 300

Cys Arg Pro Gly Phe Phe Lys Ala Ser Pro His Ile Gln Ser Cys Gly
305                 310                 315                 320

Lys Cys Pro Pro His Ser Tyr Thr His Glu Glu Ala Ser Thr Ser Cys

                325                 330                 335

Val Cys Glu Lys Asp Tyr Phe Arg Arg Glu Ser Asp Pro Pro Thr Met
            340                 345                 350

Ala Cys Thr Arg Pro Pro Ser Ala Pro Arg Asn Ala Ile Ser Asn Val
        355                 360                 365

Asn Glu Thr Ser Val Phe Leu Glu Trp Ile Pro Pro Ala Asp Thr Gly
    370                 375                 380

Gly Arg Lys Asp Val Ser Tyr Tyr Ile Ala Cys Lys Lys Cys Asn Ser
385                 390                 395                 400

His Ala Gly Val Cys Glu Glu Cys Gly Gly His Val Arg Tyr Leu Pro
                405                 410                 415

Arg Gln Ser Gly Leu Lys Asn Thr Ser Val Met Met Val Asp Leu Leu
            420                 425                 430

Ala His Thr Asn Tyr Thr Phe Glu Ile Glu Ala Val Asn Gly Val Ser
        435                 440                 445

Asp Leu Ser Pro Gly Ala Arg Gln Tyr Val Ser Val Asn Val Thr Thr
    450                 455                 460

Asn Gln Ala Ala Pro Ser Pro Val Thr Asn Val Lys Lys Gly Lys Ile
465                 470                 475                 480

Ala Lys Asn Ser Ile Ser Leu Ser Trp Gln Glu Pro Asp Arg Pro Asn
                485                 490                 495

Gly Ile Ile Leu Glu Tyr Glu Ile Lys His Phe Glu Lys Asp Gln Glu
            500                 505                 510

Thr Ser Tyr Thr Ile Ile Lys Ser Lys Glu Thr Thr Ile Thr Ala Glu
        515                 520                 525

Gly Leu Lys Pro Ala Ser Val Tyr Val Phe Gln Ile Arg Ala Arg Thr
    530                 535                 540

Ala Ala Gly Tyr Gly Val Phe Ser Arg Arg Phe Glu Phe Glu Thr Thr
545                 550                 555                 560

Pro Val Phe Ala Ala Ser Ser Asp Gln Ser Gln Ile Pro Val Ile Ala
                565                 570                 575

Val Ser Val Thr Val Gly Val Ile Leu Leu Ala Val Val Ile Gly Val
            580                 585                 590

Leu Leu Ser Gly Ser Cys Cys Glu Cys Gly Cys Gly Arg Ala Ser Ser
        595                 600                 605

Leu Cys Ala Val Ala His Pro Ile Leu Ile Trp Arg Cys Gly Tyr Ser
    610                 615                 620

Lys Ala Lys Gln Asp Pro Glu Glu Glu Lys Met His Phe His Asn Gly
625                 630                 635                 640

His Ile Lys Leu Pro Gly Val Arg Thr Tyr Ile Asp Pro His Thr Tyr
                645                 650                 655

Glu Asp Pro Asn Gln Ala Val His Glu Phe Ala Lys Glu Ile Glu Ala
            660                 665                 670

Ser Cys Ile Thr Ile Glu Arg Val Ile Gly Ala Gly Glu Phe Gly Glu
        675                 680                 685

Val Cys Ser Gly Arg Leu Lys Leu Pro Gly Lys Arg Glu Leu Pro Val
    690                 695                 700

Ala Ile Lys Thr Leu Lys Val Gly Tyr Thr Glu Lys Gln Arg Arg Asp
705                 710                 715                 720

Phe Leu Gly Glu Ala Ser Ile Met Gly Gln Phe Asp His Pro Asn Ile
                725                 730                 735

Ile His Leu Glu Gly Val Val Thr Lys Ser Lys Pro Val Met Ile Val
            740                 745                 750

Thr Glu Tyr Met Glu Asn Gly Ser Leu Asp Thr Phe Leu Lys Lys Asn
        755                 760                 765

Asp Gly Gln Phe Thr Val Ile Gln Leu Val Gly Met Leu Arg Gly Ile
    770                 775                 780

Ser Ala Gly Met Lys Tyr Leu Ser Asp Met Gly Tyr Val His Arg Asp
785                 790                 795                 800

Leu Ala Ala Arg Asn Ile Leu Ile Asn Ser Asn Leu Val Cys Lys Val
                805                 810                 815

Ser Asp Phe Gly Leu Ser Arg Val Leu Glu Asp Asp Pro Glu Ala Ala
            820                 825                 830

Tyr Thr Thr Arg Gly Gly Lys Ile Pro Ile Arg Trp Thr Ala Pro Glu
        835                 840                 845

Ala Ile Ala Phe Arg Lys Phe Thr Ser Ala Ser Asp Val Trp Ser Tyr
    850                 855                 860

Gly Ile Val Met Trp Glu Val Val Ser Tyr Gly Glu Arg Pro Tyr Trp
865                 870                 875                 880

Glu Met Thr Asn Gln Asp Val Ile Lys Ala Val Glu Glu Gly Tyr Arg
                885                 890                 895

Leu Pro Ser Pro Met Asp Cys Pro Ala Ala Leu Tyr Gln Leu Met Leu
            900                 905                 910

Asp Cys Trp Gln Lys Glu Arg Asn Ser Arg Pro Lys Phe Asp Glu Ile
        915                 920                 925

Val Asn Met Leu Asp Lys Leu Ile Arg Asn Pro Ser Ser Leu Lys Thr
    930                 935                 940

Leu Val Asn Ala Ser Cys Arg Val Ser Asn Leu Leu Ala Glu His Ser
945                 950                 955                 960

Pro Leu Gly Ser Gly Ala Tyr Arg Ser Val Gly Glu Trp Leu Glu Ala
                965                 970                 975

Ile Lys Met Gly Arg Tyr Thr Glu Ile Phe Met Glu Asn Gly Tyr Ser
            980                 985                 990

Ser Met Asp Ala Val Ala Gln Val  Thr Leu Glu Asp Leu  Arg Arg Leu
        995                 1000                 1005

Gly Val  Thr Leu Val Gly His  Gln Lys Lys Ile Met  Asn Ser Leu
    1010                 1015                 1020

Gln Glu  Met Lys Val Gln Leu  Val Asn Gly Met Val  Pro Leu
    1025                 1030                 1035

<210>  139
<211>  1282
<212>  DNA
<213>  Homo sapiens

<400>  139
ctgcgatccc gcagggcagc gacgcgactc tggtgcgggc cgtcttcttc cccccgagct      60

gggcgtgcgc ggccgcaatg aactgggagc tgctgctgtg gctgctggtg ctgtgcgcgc     120

tgctcctgct cttggtgcag ctgctgcgct tcctgagggc tgacggcgac ctgacgctac     180

tatgggccga gtggcaggga cgacgcccag aatgggagct gactgatatg gtggtgtggg     240

tgactggagc ctcgagtgga attggtgagg agctggctta ccagttgtct aaactaggag     300

tttctcttgt gctgtcagcc agaagagtgc atgagctgga aagggtgaaa agaagatgcc     360

tagagaatgg caatttaaaa gaaaaagata tacttgtttt gccccttgac ctgaccgaca     420

ctggttccca tgaagcggct accaaagctg ttctccagga gtttggtaga atcgacattc     480

tggtcaacaa tggtggaatg tcccagcgtt ctctgtgcat ggataccagc ttggatgtct     540

acagaaagct aatagagctt aactacttag ggacggtgtc cttgacaaaa tgtgttctgc     600

ctcacatgat cgagaggaag caaggaaaga ttgttactgt gaatagcatc ctgggtatca     660

tatctgtacc tctttccatt ggatactgtg ctagcaagca tgctctccgg ggttttttta      720

atggccttcg aacagaactt gccacatacc caggtataat agtttctaac atttgcccag     780

gacctgtgca atcaaatatt gtggagaatt ccctagctgg agaagtcaca aagactatag     840

gcaataatgg agaccagtcc cacaagatga caaccagtcg ttgtgtgcgg ctgatgttaa     900

tcagcatggc caatgatttg aaagaagttt ggatctcaga acaacctttc ttgttagtaa     960

catatttgtg gcaatacatg ccaacctggg cctggtggat aaccaacaag atggggaaga    1020

aaaggattga gaactttaag agtggtgtgg atgcagactc ttcttatttt aaaatcttta    1080

agacaaaaca tgactgaaaa gagcacctgt acttttcaag ccactggagg gagaaatgga    1140

aaacatgaaa acagcaatct tcttatgctt ctgaataatc aaagactaat ttgtgatttt    1200

actttttaat agatatgact ttgcttccaa catggaatga aataaaaaat aaataataaa    1260

agattgccat gaatcttgca aa                                              1282

<210>  140
<211>  339
<212>  PRT
<213>  Homo sapiens

<400>  140

Met Asn Trp Glu Leu Leu Leu Trp Leu Leu Val Leu Cys Ala Leu Leu
1               5                   10                  15

Leu Leu Leu Val Gln Leu Leu Arg Phe Leu Arg Ala Asp Gly Asp Leu
            20                  25                  30

Thr Leu Leu Trp Ala Glu Trp Gln Gly Arg Arg Pro Glu Trp Glu Leu
        35                  40                  45

Thr Asp Met Val Val Trp Val Thr Gly Ala Ser Ser Gly Ile Gly Glu
    50                  55                  60

Glu Leu Ala Tyr Gln Leu Ser Lys Leu Gly Val Ser Leu Val Leu Ser
65                  70                  75                  80

Ala Arg Arg Val His Glu Leu Glu Arg Val Lys Arg Arg Cys Leu Glu
                85                  90                  95

Asn Gly Asn Leu Lys Glu Lys Asp Ile Leu Val Leu Pro Leu Asp Leu
            100                 105                 110

Thr Asp Thr Gly Ser His Glu Ala Ala Thr Lys Ala Val Leu Gln Glu
        115                 120                 125

Phe Gly Arg Ile Asp Ile Leu Val Asn Asn Gly Gly Met Ser Gln Arg
    130                 135                 140

Ser Leu Cys Met Asp Thr Ser Leu Asp Val Tyr Arg Lys Leu Ile Glu
145                 150                 155                 160

Leu Asn Tyr Leu Gly Thr Val Ser Leu Thr Lys Cys Val Leu Pro His
                165                 170                 175

Met Ile Glu Arg Lys Gln Gly Lys Ile Val Thr Val Asn Ser Ile Leu
            180                 185                 190

Gly Ile Ile Ser Val Pro Leu Ser Ile Gly Tyr Cys Ala Ser Lys His
        195                 200                 205

Ala Leu Arg Gly Phe Phe Asn Gly Leu Arg Thr Glu Leu Ala Thr Tyr
    210                 215                 220

Pro Gly Ile Ile Val Ser Asn Ile Cys Pro Gly Pro Val Gln Ser Asn
225                 230                 235                 240

Ile Val Glu Asn Ser Leu Ala Gly Glu Val Thr Lys Thr Ile Gly Asn
                245                 250                 255

Asn Gly Asp Gln Ser His Lys Met Thr Thr Ser Arg Cys Val Arg Leu
            260                 265                 270

Met Leu Ile Ser Met Ala Asn Asp Leu Lys Glu Val Trp Ile Ser Glu
        275                 280                 285

Gln Pro Phe Leu Leu Val Thr Tyr Leu Trp Gln Tyr Met Pro Thr Trp
    290                 295                 300

Ala Trp Trp Ile Thr Asn Lys Met Gly Lys Lys Arg Ile Glu Asn Phe
305                 310                 315                 320

Lys Ser Gly Val Asp Ala Asp Ser Ser Tyr Phe Lys Ile Phe Lys Thr
                325                 330                 335

Lys His Asp

<210>  141
<211>  1740
<212>  DNA
<213>  Homo sapiens

<400>  141
atgagcagct gcaggtacaa cgggggcgtc atgcggccgc tcagcaactt gagcgcgtcc      60

cgccggaacc tgcacgagat ggactcagag gcgcagcccc tgcagccccc cgcgtctgtc     120

ggaggaggtg gcggcgcgtc ctccccgtct gcagccgctg ccgccgccgc cgctgtttcg     180

tcctcagccc ccgagatcgt ggtgtctaag cccgagcaca acaactccaa caacctggcg     240

ctctatggaa ccggcggcgg aggcagcact ggaggaggcg gcggcggtgg cgggagcggg 300
cacggcagca gcagtggcac caagtccagc aaaaagaaaa accagaacat cggctacaag 360
ctgggccacc ggcgcgccct gttcgaaaag cgcaagcggc tcagcgacta cgcgctcatc 420
ttcggcatgt tcggcatcgt ggtcatggtc atcgagaccg agctgtcgtg gggcgcctac 480
gacaaggcgt cgctgtattc cttagctctg aaatgcctta tcagtctctc cacgatcatc 540
ctgctcggtc tgatcatcgt gtaccacgcc agggaaatac agttgttcat ggtggacaat 600
ggagcagatg actggagaat agccatgact tatgagcgta ttttcttcat ctgcttggaa 660
atactggtgt gtgctattca tcccatacct gggaattata cattcacatg gacggcccgg 720
cttgccttct cctatgcccc atccacaacc accgctgatg tggatattat tttatctata 780
ccaatgttct taagactcta tctgattgcc agagtcatgc ttttacatag caaacttttc 840
actgatgcct cctctagaag cattggagca cttaataaga taaacttcaa tacacgtttt 900
gttatgaaga ctttaatgac tatatgccca ggaactgtac tcttggtttt tagtatctca 960
ttatggataa ttgccgcatg gactgtccga gcttgtgaaa ggtaccatga tcaacaggat 1020
gttactagca acttccttgg agcgatgtgg ttgatatcaa taacttttct ctccattggt 1080
tatggtgaca tggtacctaa cacatactgt ggaaaaggag tctgcttact tactggaatt 1140
atgggtgctg gttgcacagc cctggtggta gctgtagtgg caaggaagct agaacttacc 1200
aaagcagaaa aacacgtgca caatttcatg atggatactc agctgactaa aagagtaaaa 1260
aatgcagctg ccaatgtact cagggaaaca tggctaattt acaaaaatac aaagctagtg 1320
aaaaagatag atcatgcaaa agtaagaaaa catcaacgaa aattcctgca agctattcat 1380
caattaagaa gtgtaaaaat ggagcagagg aaactgaatg accaagcaaa cactttggtg 1440
gacttggcaa agacccagaa catcatgtat gatatgattt ctgacttaaa cgaaaggagt 1500
gaagacttcg agaagaggat tgttaccctg gaaacaaaac tagagacttt gattggtagc 1560
atccacgccc tccctgggct cataagccag accatcaggc agcagcagag agatttcatt 1620
gaggctcaga tggagagcta cgacaagcac gtcacttaca atgctgagcg gtcccggtcc 1680
tcgtccagga ggcggcggtc ctcttccaca gcaccaccaa cttcatcaga gagtagctag 1740
<210> 142
<211> 579
<212> PRT
<213> Homo sapiens

<400> 142

Met Ser Ser Cys Arg Tyr Asn Gly Gly Val Met Arg Pro Leu Ser Asn
1               5                   10                  15

Leu Ser Ala Ser Arg Arg Asn Leu His Glu Met Asp Ser Glu Ala Gln
            20                  25                  30

Pro Leu Gln Pro Pro Ala Ser Val Gly Gly Gly Gly Ala Ser Ser
        35                  40                  45

Pro Ser Ala Ala Ala Ala Ala Ala Ala Ala Val Ser Ser Ser Ala Pro

50                  55                  60

Glu Ile Val Val Ser Lys Pro Glu His Asn Asn Ser Asn Asn Leu Ala
65                  70                  75                  80

Leu Tyr Gly Thr Gly Gly Gly Gly Ser Thr Gly Gly Gly Gly Gly Gly
                85                  90                  95

Gly Gly Ser Gly His Gly Ser Ser Ser Gly Thr Lys Ser Ser Lys Lys
            100                 105                 110

Lys Asn Gln Asn Ile Gly Tyr Lys Leu Gly His Arg Arg Ala Leu Phe
        115                 120                 125

Glu Lys Arg Lys Arg Leu Ser Asp Tyr Ala Leu Ile Phe Gly Met Phe
    130                 135                 140

Gly Ile Val Val Met Val Ile Glu Thr Glu Leu Ser Trp Gly Ala Tyr
145                 150                 155                 160

Asp Lys Ala Ser Leu Tyr Ser Leu Ala Leu Lys Cys Leu Ile Ser Leu
                165                 170                 175

Ser Thr Ile Ile Leu Leu Gly Leu Ile Ile Val Tyr His Ala Arg Glu
            180                 185                 190

Ile Gln Leu Phe Met Val Asp Asn Gly Ala Asp Asp Trp Arg Ile Ala
        195                 200                 205

Met Thr Tyr Glu Arg Ile Phe Phe Ile Cys Leu Glu Ile Leu Val Cys
    210                 215                 220

Ala Ile His Pro Ile Pro Gly Asn Tyr Thr Phe Thr Trp Thr Ala Arg
225                 230                 235                 240

Leu Ala Phe Ser Tyr Ala Pro Ser Thr Thr Thr Ala Asp Val Asp Ile
                245                 250                 255

Ile Leu Ser Ile Pro Met Phe Leu Arg Leu Tyr Leu Ile Ala Arg Val
            260                 265                 270

Met Leu Leu His Ser Lys Leu Phe Thr Asp Ala Ser Ser Arg Ser Ile
        275                 280                 285

Gly Ala Leu Asn Lys Ile Asn Phe Asn Thr Arg Phe Val Met Lys Thr
    290                 295                 300

Leu Met Thr Ile Cys Pro Gly Thr Val Leu Leu Val Phe Ser Ile Ser
305                 310                 315                 320

Leu Trp Ile Ile Ala Ala Trp Thr Val Arg Ala Cys Glu Arg Tyr His
                325                 330                 335

Asp Gln Gln Asp Val Thr Ser Asn Phe Leu Gly Ala Met Trp Leu Ile
            340                 345                 350

Ser Ile Thr Phe Leu Ser Ile Gly Tyr Gly Asp Met Val Pro Asn Thr
        355                 360                 365

Tyr Cys Gly Lys Gly Val Cys Leu Leu Thr Gly Ile Met Gly Ala Gly
    370                 375                 380

Cys Thr Ala Leu Val Val Ala Val Val Ala Arg Lys Leu Glu Leu Thr
385                 390                 395                 400

Lys Ala Glu Lys His Val His Asn Phe Met Met Asp Thr Gln Leu Thr
                405                 410                 415

Lys Arg Val Lys Asn Ala Ala Ala Asn Val Leu Arg Glu Thr Trp Leu
            420                 425                 430

Ile Tyr Lys Asn Thr Lys Leu Val Lys Lys Ile Asp His Ala Lys Val
        435                 440                 445

Arg Lys His Gln Arg Lys Phe Leu Gln Ala Ile His Gln Leu Arg Ser
    450                 455                 460

Val Lys Met Glu Gln Arg Lys Leu Asn Asp Gln Ala Asn Thr Leu Val
465                 470                 475                 480

Asp Leu Ala Lys Thr Gln Asn Ile Met Tyr Asp Met Ile Ser Asp Leu
                485                 490                 495

Asn Glu Arg Ser Glu Asp Phe Glu Lys Arg Ile Val Thr Leu Glu Thr
            500                 505                 510

Lys Leu Glu Thr Leu Ile Gly Ser Ile His Ala Leu Pro Gly Leu Ile
        515                 520                 525

Ser Gln Thr Ile Arg Gln Gln Gln Arg Asp Phe Ile Glu Ala Gln Met
    530                 535                 540

Glu Ser Tyr Asp Lys His Val Thr Tyr Asn Ala Glu Arg Ser Arg Ser
545                 550                 555                 560

Ser Ser Arg Arg Arg Arg Ser Ser Ser Thr Ala Pro Pro Thr Ser Ser
                565                 570                 575

Glu Ser Ser

<210>  143
<211>  1896
<212>  DNA
<213>  Homo sapiens

<400>  143
atgcgcgccg tgccgctgcc cgccccgctc ctgccgctgc tgctgctcgc gctcctggcc      60

gctcccgccg cccgcgccag cagagccgag tccgtctccg cgccgtggcc cgaacccgag     120

cgcgagtcgc ggccaccgcc cggcccgggg cccgggaaca ccacccggtt tgggtctggg     180

gcggcgggcg gcagcggcag ctccagctcc aacagcagtg gcgacgcctt ggtgacccgc     240

atttccatcc tcctccgcga cctacccacc ctcaaggcag ccgtgatcgt ggcgttcgcc     300

tttaccaccc tcctcatcgc ctgcctgctg ctgcgcgtct tcaggtcggg aaagaggtta     360

aagaagacac gcaagtatga tatcatcacc actccagcag agcgagtgga aatggcgcca     420

ctaaatgaag aggatgatga agatgaggac tccacagtat tcgacatcaa atacagagtg     480

tccttgccgg ctgcactgag acgtcagctg ccagggtgcc agacgctact gacagttcct     540

gtgcccccac ccttcatcct cgacattgac cttccagcaa gatgcagtgg aaggcctgat     600

ggtggaatca gacctggtaa aacctgtttc ccagcctggt ggcatcctgt ggaaagttgg     660

tcagctgcaa cctggggtgt gaaggactgg acctggaagc cctcttgcgt cggaggtgtt     720

gaaaccaaaa cgaacgttat gtataaaacc ccagctccat cgtgcgtgtc aggcatctgc     780

tcagactgtc actggcaagc tcgtttccac gtcaccacaa tggagttgct tctgccaccc     840

tttgggcatc cctttaaagt gccccctact tctactcccc atggttttcg acaactgcag     900

ctgaatctca tggaaaagct ggattcctct gccttacgca gaaacacccg ggctccatct 960
gccaggtgct tgccactggt cctggcagaa atggcggctg ctgaaagtga ccttccaaat 1020
ccttggtggc acttcagcgc cacaggctct ccaataaaaa ccctttacac acaaaccatg 1080
agtaccttgg gcttggatgt tttctgtggt gccggccagc ggggcacctt ttgtgaagac 1140
agagcagtga ctaaggttct ccagggtagc tctttctcca aacagctgcg ctggaagcca 1200
gccctagaga gtgggtttcc ccatcatctc aggcttctca gagagtgtcc tccgctgagc 1260
acccatcctg tcaggttggc tcgttcagat gcccggggac aagccagcct gacggggagg 1320
agggtgtttc ggcgtccgcg gcagtctctg catggcggag ggtcagcggg taccgcaact 1380
tgccttttgg ttttgaagat tctgttgagg cgccatcctc accttgacct cttctacaaa 1440
atctgtctcc cctgctgtgc cgtggaacac ctacgggaag ccaagagaag ctcagtgact 1500
gtccttgcgt catttgagca gagcccacaa aaggcagctg ctgcccacgg ggagcctgtc 1560
aaacgagggc ccagtgggca attgaccaga cacacatgcc ctggctgggg gatcacacat 1620
gcgaacctgc agacaattcc agatacccaa ggccaggaag gcccacgtga ggatgtcact 1680
caccctggag gagacttgga tggggtggca aatttctatt tggaggaaga gggtttccag 1740
gatggcagat gccagaagat ggtcctgatg tctgaggaag ggccacctag tttgacagga 1800
tgtgagaggc tcacaggttc ccatcacttc tccagccatt ccaagtcttg gtccttcctt 1860
tccccccgac agcccctgtt tctgtccagg ccctga 1896

<210> 144
<211> 631
<212> PRT
<213> Homo sapiens

<400> 144

<210>  145
<211>  5133
<212>  DNA
<213>  Homo sapiens

<400>  145
ggctgggctg cgaatagcgt gttcctctcc ggcggaacac acacacccgg ccttggggct      60

gtctcctgaa gctccctcct ccacggagag cgctgagcgc cgccgggaat tccatcccac     120

cgtgggcacg cagtctttgg aggtcccggg cgcagcacgc tcggtgtccc cacactgcag     180

caagacagag accccgcggg aaccttgagc ttggaacaac ccttgagcct ctgcagtcgg     240

aagagtgggc gcagcagccc agcggaggcc aggcgcgcaa cctcgggcgc cggggcaagg     300

agagagtgca gggaggcgca gctcaggcgc ccggctcagg agcgggagga agttctcgcg     360

gcgccgggag cgcggtggac gcgccctggg cgcacgccca ggcagccttc tccctggccc     420

tcgggactgt cctcgggcgg caaggaggag cttgctggag tcttagaggc catccagagc     480

cagcgagcag gagcgctgcg tctcccgcct cagctaggaa gggggagtgg cgctggcagg     540

ctggagctgg gaacccagcg agcgcctgac cttcctcctc ctcttcctga ccctcttcgc     600

gtcttgggct ccggaggaag gttctagcgg ctgcaggagg tccccagacc cattttccta     660

gaaggctggt gatggatctg ctgctcctgc cgccgccggg gcacttggag cgcaccggcg     720

gcgcgtgagc tgggctttgc tctccaccgc cctgggcaaa ccccgggcca gccccgcctg     780

gcacctttgc ctgagtccct ttcggttccc gacccaaagc caccagcgtc cagggaggga     840

ggaggaggtg gtcctcaggt gcagccccgc cgagatgtcc gcgcagagcc tgctccacag     900

cgtcttctcc tgttcctcgc ccgcttcaag tagcgcggcc tcggccaagg gcttctccaa     960

gaggaagctg cgccagaccc gcagcctgga cccggccctg atcggcggct gcgggagcga    1020

cgaggcgggc gcggagggca gtgcgcgggg agccacggcg ggccgcctct actccccatc    1080

actcccagcc gagagtctcg gccctcgctt ggcgtcctct tcccggggtc cgccccccag 1140
ggccaccagg ctaccgcctc ctggacctct ttgctcgtcc ttctccacac ccagcacccc 1200
gcaggagaag tcaccatccg gcagctttca ctttgactat gaggttcccc tgggtcgcgg 1260
cggcctcaag aagagcatgg cctgggacct gccttctgtc ctggccgggc cagccagtag 1320
ccgaagcgct tccagcatcc tctgttcatc cgggggaggc cccaatggca tcttcgcttc 1380
tcctaggagg tggctccagc agaggaagtt ccagtcccca cccgacagtc gcgggcaccc 1440
ctacgtcgtg tggaaatccg agggtgattt cacctggaac agcatgtcag gccgcagtgt 1500
gcggctgagg tcagtcccca tccagagtct ctcagagctg gagagggccc ggctgcagga 1560
agtgcctttt tatcagttgc aacaggactg tgacctgagc tgtcagatca ccattcccaa 1620
agatggacaa aagagaaaga aatctttaag aaagaaactg gattcactag gaaaggagaa 1680
aaacaaagac aaagaattca tcccacaggc atttggaatg cccttatccc aagtcattgc 1740
gaatgacagg gcctataaac tcaagcagga cttgcagagg gacgagcaga aagatgcatc 1800
tgactttgtg gcttccctcc tcccatttgg aaataaaaga caaaacaaag aactctcaag 1860
cagtaactca tctctcagct caacctcaga aacaccgaat gagtcaacgt ccccaaacac 1920
cccggaaccg gctcctcggg ctaggaggag gggtgccatg tcagtggatt ctatcaccga 1980
tcttgatgac aatcagtctc gactactaga agctttacaa ctttccttgc ctgctgaggc 2040
tcaaagtaaa aaggaaaaag ccagagataa gaaactcagt ctgaatccta tttacagaca 2100
ggtccctagg ctggtggaca gctgctgtca gcacctagaa aaacatggcc tccagacagt 2160
ggggatattc cgagttggaa gctcaaaaaa gagagtgaga caattacgtg aggaatttga 2220
ccgtgggatt gatgtctctc tggaggagga gcacagtgtt catgatgtgg cagccttgct 2280
gaaagagttc ctgagggaca tgccagaccc ccttctcacc agggagctgt acacagcttt 2340
catcaacact ctcttgttgg agccggagga acagctgggc accttgcagc tcctcatata 2400
ccttctacct ccctgcaact gcgacaccct ccaccgcctg ctacagttcc tctccatcgt 2460
ggccaggcat gccgatgaca acatcagcaa agatgggcaa gaggtcactg ggaataaaat 2520
gacatctcta aacttagcca ccatatttgg acccaacctg ctgcacaagc agaagtcatc 2580
agacaaagaa ttctcagttc agagttcagc ccgggctgag gagagcacgg ccatcatcgc 2640
tgttgtgcaa aagatgattg aaaattatga agccctgttc atggttcccc cagatctcca 2700
gaacgaagtg ctgatcagcc tgttagagac cgatcctgat gtcgtggact atttactcag 2760
aagaaaggct tcccaatcat caagccctga catgctgcag tcggaagttt ccttttccgt 2820
gggagggagg cattcatcta cagactccaa caaggcctcc agcggagaca tctcccctta 2880
tgacaacaac tccccagtgc tgtctgagcg ctccctgctg gctatgcaag aggacgcggc 2940
cccggggggc tcggagaagc tttacagagt gccagggcag tttatgctgg tgggccactt 3000
gtcgtcgtca aagtcaaggg aaagttctcc tggaccaagg cttgggaaag atctgtcaga 3060
ggagcctttc gatatctggg gaacttggca ttcaacatta aaaagcggat ccaaagaccc 3120

aggaatgaca ggttcctctg gagacatttt tgaaagcagc tccctaagag cggggccctg 3180
ctccctttct caagggaacc tgtccccaaa ttggcctcgg tggcagggga gccccgcaga 3240
gctggacagc gacacgcagg gggctcggag gactcaggcc gcagcccccg cgacggaggg 3300
cagggcccac cctgcggtgt cgcgcgcctg cagcacgccc cacgtccagg tggcagggaa 3360
agccgagcgg cccacggcca ggtcggagca gtacttgacc ctgagcggcg cccacgacct 3420
cagcgagagt gagctggatg tggccgggct gcagagccgg gccacacctc agtgccaaag 3480
accccatggg agtgggaggg atgacaagcg gcccccgcct ccatacccgg gcccagggaa 3540
gcccgcggca gcggcagcct ggatccaggg gcccccggaa ggcgtggaga cacccacgga 3600
ccagggaggc caagcagccg agcgagagca gcaggtcacg cagaaaaaac tgagcagcgc 3660
caactccctg ccagcgggcg agcaggacag tccgcgcctg ggggacgctg gctggctcga 3720
ctggcagaga gagcgctggc agatctggga gctcctgtcg accgacaacc ccgatgccct 3780
gcccgagacg ctggtctgag cccgcaccca gccgagcccc ccctgccccg agccccccgc 3840
cctccagccc aggggggacc gtgggtggtg gccactggca cacttagtgt tcttctttca 3900
cacttctcaa aagtgacaca agagaaatcc agttcaccta cagaggtaga gcactcacgc 3960
ccccgccatt gagaataagg ttccattgcg tagccagcct taggaaaaac aaacagaacc 4020
caaaccagat ggcaatgtcc aatctaaaaa cgtccctctt ggctctataa tataagatac 4080
aactcttgct tggtatagcc taaccgtatt tatgtgtctt cggttttgac tattgtgtat 4140
tctgtaacag attatgtata atcatatatg atatattcac aaagagaaaa caaaaggaac 4200
ttttaaaaaa aaaatcactt cacttatatt aagcaatgag atatactaaa caatgagatt 4260
ctatagaatg ttctagaatg tgcacaagcg ggtttctgtg cttttgccat agctttataa 4320
ctggggataa cccttccttc gataccaaac actaacaaga ggaagcagaa tatgagaagc 4380
catattttta cataggagtc agatacaaaa agaaaaatca ctgaatgctt ttagatattg 4440
aatacgtttt caggaaaatg ctaaatctga tagattacga aatatatttt tagaacttgt 4500
ttagaaagga ttcagttaac caaacaagaa aaaggcagtg cctcacaaag aaattaagaa 4560
gttgtccgtc ccacgttaca tcaaattcag ttttatatag gccatatata atatatattt 4620
ataatgtata atttttatgt atttttcaaa actacaaact ggaatccaac tataaagtgt 4680
ttaagaatct acacagaata ttcaaattat agaacatgtt ttttcccttt gccccataat 4740
cagtatttgc caaattacat gcaattcctt aaaaactaaa tcacattggt aaaaggccta 4800
cagctttgta cttacattgt gccaaaggct gaggaaatgt tttctttcga atttttatgt 4860
gtattgtaaa atgttctacc gtactttagt agtttgaagt tttcaagtgc ataactattt 4920
ttgaccagca gaaggcgata cgcttcagta ttttatgcaa ttttttttca cttcgaaggg 4980
aaagtgtatt ataaaaaaag attttttttt tttaaaacat gctactctta attttcatgt 5040
tggtgatgaa attcccagtg gtgtttctta aggttctatc ttgtgccatg atgaataaaa 5100
agttaagcaa aaaaaaaaaa aaaaaaaaaa aaa 5133

<210> 146
<211> 974
<212> PRT
<213> Homo sapiens

<400> 146

Met Ser Ala Gln Ser Leu Leu His Ser Val Phe Ser Cys Ser Ser Pro
1               5                   10                  15

Ala Ser Ser Ser Ala Ala Ser Ala Lys Gly Phe Ser Lys Arg Lys Leu
            20                  25                  30

Arg Gln Thr Arg Ser Leu Asp Pro Ala Leu Ile Gly Gly Cys Gly Ser
        35                  40                  45

Asp Glu Ala Gly Ala Glu Gly Ser Ala Arg Gly Ala Thr Ala Gly Arg
    50                  55                  60

Leu Tyr Ser Pro Ser Leu Pro Ala Glu Ser Leu Gly Pro Arg Leu Ala
65                  70                  75                  80

Ser Ser Ser Arg Gly Pro Pro Pro Arg Ala Thr Arg Leu Pro Pro Pro
                85                  90                  95

Gly Pro Leu Cys Ser Ser Phe Ser Thr Pro Ser Thr Pro Gln Glu Lys
            100                 105                 110

Ser Pro Ser Gly Ser Phe His Phe Asp Tyr Glu Val Pro Leu Gly Arg
        115                 120                 125

Gly Gly Leu Lys Lys Ser Met Ala Trp Asp Leu Pro Ser Val Leu Ala
    130                 135                 140

Gly Pro Ala Ser Ser Arg Ser Ala Ser Ser Ile Leu Cys Ser Ser Gly
145                 150                 155                 160

Gly Gly Pro Asn Gly Ile Phe Ala Ser Pro Arg Arg Trp Leu Gln Gln
                165                 170                 175

Arg Lys Phe Gln Ser Pro Pro Asp Ser Arg Gly His Pro Tyr Val Val
            180                 185                 190

Trp Lys Ser Glu Gly Asp Phe Thr Trp Asn Ser Met Ser Gly Arg Ser
        195                 200                 205

Val Arg Leu Arg Ser Val Pro Ile Gln Ser Leu Ser Glu Leu Glu Arg
    210                 215                 220

Ala Arg Leu Gln Glu Val Pro Phe Tyr Gln Leu Gln Gln Asp Cys Asp
225                 230                 235                 240

Leu Ser Cys Gln Ile Thr Ile Pro Lys Asp Gly Gln Lys Arg Lys Lys
                245                 250                 255

Ser Leu Arg Lys Lys Leu Asp Ser Leu Gly Lys Glu Lys Asn Lys Asp
            260                 265                 270

Lys Glu Phe Ile Pro Gln Ala Phe Gly Met Pro Leu Ser Gln Val Ile
        275                 280                 285

Ala Asn Asp Arg Ala Tyr Lys Leu Lys Gln Asp Leu Gln Arg Asp Glu
    290                 295                 300

Gln Lys Asp Ala Ser Asp Phe Val Ala Ser Leu Leu Pro Phe Gly Asn
305                 310                 315                 320

Lys Arg Gln Asn Lys Glu Leu Ser Ser Ser Asn Ser Ser Leu Ser Ser
                325                 330                 335

Thr Ser Glu Thr Pro Asn Glu Ser Thr Ser Pro Asn Thr Pro Glu Pro
            340                 345                 350

Ala Pro Arg Ala Arg Arg Arg Gly Ala Met Ser Val Asp Ser Ile Thr
        355                 360                 365

Asp Leu Asp Asp Asn Gln Ser Arg Leu Leu Glu Ala Leu Gln Leu Ser
    370                 375                 380

Leu Pro Ala Glu Ala Gln Ser Lys Lys Glu Lys Ala Arg Asp Lys Lys
385                 390                 395                 400

Leu Ser Leu Asn Pro Ile Tyr Arg Gln Val Pro Arg Leu Val Asp Ser
                405                 410                 415

Cys Cys Gln His Leu Glu Lys His Gly Leu Gln Thr Val Gly Ile Phe
            420                 425                 430

Arg Val Gly Ser Ser Lys Lys Arg Val Arg Gln Leu Arg Glu Glu Phe
        435                 440                 445

Asp Arg Gly Ile Asp Val Ser Leu Glu Glu Glu His Ser Val His Asp
    450                 455                 460

Val Ala Ala Leu Leu Lys Glu Phe Leu Arg Asp Met Pro Asp Pro Leu
465                 470                 475                 480

Leu Thr Arg Glu Leu Tyr Thr Ala Phe Ile Asn Thr Leu Leu Leu Glu
                485                 490                 495

Pro Glu Glu Gln Leu Gly Thr Leu Gln Leu Leu Ile Tyr Leu Leu Pro
            500                 505                 510

Pro Cys Asn Cys Asp Thr Leu His Arg Leu Leu Gln Phe Leu Ser Ile
        515                 520                 525

Val Ala Arg His Ala Asp Asp Asn Ile Ser Lys Asp Gly Gln Glu Val
    530                 535                 540

Thr Gly Asn Lys Met Thr Ser Leu Asn Leu Ala Thr Ile Phe Gly Pro
545                 550                 555                 560

Asn Leu Leu His Lys Gln Lys Ser Ser Asp Lys Glu Phe Ser Val Gln
                565                 570                 575

Ser Ser Ala Arg Ala Glu Glu Ser Thr Ala Ile Ile Ala Val Val Gln
            580                 585                 590

Lys Met Ile Glu Asn Tyr Glu Ala Leu Phe Met Val Pro Pro Asp Leu
        595                 600                 605

Gln Asn Glu Val Leu Ile Ser Leu Leu Glu Thr Asp Pro Asp Val Val
    610                 615                 620

Asp Tyr Leu Leu Arg Arg Lys Ala Ser Gln Ser Ser Ser Pro Asp Met
625                 630                 635                 640

Leu Gln Ser Glu Val Ser Phe Ser Val Gly Gly Arg His Ser Ser Thr
                645                 650                 655

Asp Ser Asn Lys Ala Ser Ser Gly Asp Ile Ser Pro Tyr Asp Asn Asn
            660                 665                 670

Ser Pro Val Leu Ser Glu Arg Ser Leu Leu Ala Met Gln Glu Asp Ala
        675                 680                 685

Ala Pro Gly Gly Ser Glu Lys Leu Tyr Arg Val Pro Gly Gln Phe Met
    690                 695                 700

Leu Val Gly His Leu Ser Ser Ser Lys Ser Arg Glu Ser Ser Pro Gly
705                 710                 715                 720

Pro Arg Leu Gly Lys Asp Leu Ser Glu Glu Pro Phe Asp Ile Trp Gly
                725                 730                 735

Thr Trp His Ser Thr Leu Lys Ser Gly Ser Lys Asp Pro Gly Met Thr
            740                 745                 750

Gly Ser Ser Gly Asp Ile Phe Glu Ser Ser Ser Leu Arg Ala Gly Pro
        755                 760                 765

Cys Ser Leu Ser Gln Gly Asn Leu Ser Pro Asn Trp Pro Arg Trp Gln
    770                 775                 780

Gly Ser Pro Ala Glu Leu Asp Ser Asp Thr Gln Gly Ala Arg Arg Thr
785                 790                 795                 800

Gln Ala Ala Ala Pro Ala Thr Glu Gly Arg Ala His Pro Ala Val Ser
                805                 810                 815

Arg Ala Cys Ser Thr Pro His Val Gln Val Ala Gly Lys Ala Glu Arg
            820                 825                 830

Pro Thr Ala Arg Ser Glu Gln Tyr Leu Thr Leu Ser Gly Ala His Asp
        835                 840                 845

Leu Ser Glu Ser Glu Leu Asp Val Ala Gly Leu Gln Ser Arg Ala Thr
    850                 855                 860

Pro Gln Cys Gln Arg Pro His Gly Ser Gly Arg Asp Asp Lys Arg Pro
865                 870                 875                 880

Pro Pro Pro Tyr Pro Gly Pro Gly Lys Pro Ala Ala Ala Ala Ala Trp
                885                 890                 895

Ile Gln Gly Pro Pro Glu Gly Val Glu Thr Pro Thr Asp Gln Gly Gly
            900                 905                 910

Gln Ala Ala Glu Arg Glu Gln Gln Val Thr Gln Lys Lys Leu Ser Ser
        915                 920                 925

Ala Asn Ser Leu Pro Ala Gly Glu Gln Asp Ser Pro Arg Leu Gly Asp
    930                 935                 940

Ala Gly Trp Leu Asp Trp Gln Arg Glu Arg Trp Gln Ile Trp Glu Leu
945                 950                 955                 960

Leu Ser Thr Asp Asn Pro Asp Ala Leu Pro Glu Thr Leu Val
                965                 970

<210>  147
<211>  2397
<212>  DNA
<213>  Homo sapiens

<400>  147
cccccgagcc gcgccgagtc tgccgccgcc gcagcgcctc cgctccgcca actccgccgg      60

cttaaattgg actcctagat ccgcgagggc gcggcgcagc cgagcagcgg ctctttcagc     120

attggcaacc ccaggggcca atatttccca cttagccaca gctccagcat cctctctgtg     180

ggctgttcac caactgtaca accaccattt cactgtggac attactccct cttacagata 240
tgggagacat gggagatcca ccaaaaaaaa aacgtctgat ttccctatgt gttggttgcg 300
gcaatcagat tcacgatcag tatattctga gggtttctcc ggatttggaa tggcatgcgg 360
catgtttgaa atgtgcggag tgtaatcagt atttggacga gagctgtaca tgctttgtta 420
gggatgggaa aacctactgt aaaagagatt atatcaggtt gtacgggatc aaatgcgcca 480
agtgcagcat cggcttcagc aagaacgact tcgtgatgcg tgcccgctcc aaggtgtatc 540
acatcgagtg tttccgctgt gtggcctgca gccgccagct catccctggg gacgaatttg 600
cgcttcggga ggacggtctc ttctgccgag cagaccacga tgtggtggag agggccagtc 660
taggcgctgg cgacccgctc agtcccctgc atccagcgcg gccactgcaa atggcagcgg 720
agcccatctc cgccaggcag ccagccctgc ggccccacgt ccacaagcag ccggagaaga 780
ccacccgcgt gcggactgtg ctgaacgaga agcagctgca caccttgcgg acctgctacg 840
ccgcaaaccc gcggccagat gcgctcatga aggagcaact ggtagagatg acgggcctca 900
gtccccgtgt gatccgggtc tggtttcaaa acaagcggtg caaggacaag aagcgaagca 960
tcatgatgaa gcaactccag cagcagcagc ccaatgacaa aactaatatc caggggatga 1020
caggaactcc catggtggct gccagtccag agagacacga cggtggctta caggctaacc 1080
cagtggaagt acaaagttac cagccacctt ggaaagtact gagcgacttc gccttgcaga 1140
gtgacataga tcagcctgct tttcagcaac tggtcaattt ttcagaagga ggaccgggct 1200
ctaattccac tggcagtgaa gtagcatcaa tgtcctctca acttccagat acacctaaca 1260
gcatggtagc cagtcctatt gaggcatgag gaacattcat tctgtatttt ttttccctgt 1320
tggagaaagt gggaaattat aatgtcgaac tctgaaacaa aagtatttaa cgacccagtc 1380
aatgaaaact gaatcaagaa atgaatgctc catgaaatgc acgaagtctg ttttaatgac 1440
aaggtgatat ggtagcaaca ctgtgaagac aatcatggga ttttactaga attaaacaac 1500
aaacaaaacg caaaacccag tatatgctat tcaatgatct tagaagtact gaaaaaaaaa 1560
gacgttttta aaacgtagag gatttatatt caaggatctc aaagaaagca ttttcatttc 1620
actgcacatc tagagaaaaa caaaaataga aaattttcta gtccatccta atctgaatgg 1680
tgctgtttct atattggtca ttgccttgcc aaacaggagc tccagcaaaa gcgcaggaag 1740
agagactggc ctccttggct gaaagagtcc tttcaggaag gtggagctgc attggtttga 1800
tatgtttaaa gttgacttta acaaggggtt aattgaaatc ctgggtctct tggcctgtcc 1860
tgtagctggt ttatttttta ctttgccccc tccccacttt ttttgagatc catcctttat 1920
caagaagtct gaagcgacta taaaggtttt tgaattcaga tttaaaaacc aacttataaa 1980
gcattgcaac aaggttacct ctattttgcc acaagcgtct cgggattgtg tttgacttgt 2040
gtctgtccaa gaacttttcc cccaaagatg tgtatagtta ttggttaaaa tgactgtttt 2100
ctctctctat ggaaataaaa aggaaaaaaa aaaggaaact ttttttgttt gctcttgcat 2160
tgcaaaaatt ataaagtaat ttattattta ttgtcggaag acttgccact tttcatgtca 2220

tttgacattt tttgtttgct gaagtgaaaa aaaaagataa aggttgtacg gtggtctttg 2280

aattatatgt ctaattctat gtgttttgtc tttttcttaa atattatgtg aaatcaaagc 2340

gccatatgta gaattatatc ttcaggacta tttcactaat aaacatttgg catagat 2397

<210> 148
<211> 346
<212> PRT
<213> Homo sapiens

<400> 148

Met Gly Asp Pro Pro Lys Lys Lys Arg Leu Ile Ser Leu Cys Val Gly
1               5                   10                  15

Cys Gly Asn Gln Ile His Asp Gln Tyr Ile Leu Arg Val Ser Pro Asp
            20                  25                  30

Leu Glu Trp His Ala Ala Cys Leu Lys Cys Ala Glu Cys Asn Gln Tyr
        35                  40                  45

Leu Asp Glu Ser Cys Thr Cys Phe Val Arg Asp Gly Lys Thr Tyr Cys
    50                  55                  60

Lys Arg Asp Tyr Ile Arg Leu Tyr Gly Ile Lys Cys Ala Lys Cys Ser
65                  70                  75                  80

Ile Gly Phe Ser Lys Asn Asp Phe Val Met Arg Ala Arg Ser Lys Val
                85                  90                  95

Tyr His Ile Glu Cys Phe Arg Cys Val Ala Cys Ser Arg Gln Leu Ile
            100                 105                 110

Pro Gly Asp Glu Phe Ala Leu Arg Glu Asp Gly Leu Phe Cys Arg Ala
        115                 120                 125

Asp His Asp Val Val Glu Arg Ala Ser Leu Gly Ala Gly Asp Pro Leu
    130                 135                 140

Ser Pro Leu His Pro Ala Arg Pro Leu Gln Met Ala Ala Glu Pro Ile
145                 150                 155                 160

Ser Ala Arg Gln Pro Ala Leu Arg Pro His Val His Lys Gln Pro Glu
                165                 170                 175

Lys Thr Thr Arg Val Arg Thr Val Leu Asn Glu Lys Gln Leu His Thr
            180                 185                 190

Leu Arg Thr Cys Tyr Ala Ala Asn Pro Arg Pro Asp Ala Leu Met Lys
        195                 200                 205

Glu Gln Leu Val Glu Met Thr Gly Leu Ser Pro Arg Val Ile Arg Val
    210                 215                 220

Trp Phe Gln Asn Lys Arg Cys Lys Asp Lys Lys Arg Ser Ile Met Met
225                 230                 235                 240

Lys Gln Leu Gln Gln Gln Pro Asn Asp Lys Thr Asn Ile Gln Gly
                245                 250                 255

Met Thr Gly Thr Pro Met Val Ala Ala Ser Pro Glu Arg His Asp Gly
                260                 265                 270

Gly Leu Gln Ala Asn Pro Val Glu Val Gln Ser Tyr Gln Pro Pro Trp
            275                 280                 285

Lys Val Leu Ser Asp Phe Ala Leu Gln Ser Asp Ile Asp Gln Pro Ala

290                 295                 300

Phe Gln Gln Leu Val Asn Phe Ser Glu Gly Gly Pro Gly Ser Asn Ser
305                 310                 315                 320

Thr Gly Ser Glu Val Ala Ser Met Ser Ser Gln Leu Pro Asp Thr Pro
                325                 330                 335

Asn Ser Met Val Ala Ser Pro Ile Glu Ala
            340                 345

<210>  149
<211>  1886
<212>  DNA
<213>  Homo sapiens

<400>  149
gcggagctct gccttggaga ttctcagtgc tgcggatcat gtccctaagg ggcagcctct      60

cgcgtctcct ccagacgcga gtgcattcca tcctgaagaa atccgtccac tccgtggctg     120

tgataggagc cccgttctca caagggcaga aaagaaaagg agtggagcat ggtcccgctg     180

ccataagaga agctggcttg atgaaaaggc tctccagttt gggctgccac ctaaaagact     240

ttggagattt gagttttact ccagtcccca aagatgatct ctacaacaac ctgatagtga     300

atccacgctc agtgggtctt gccaaccagg aactggctga ggtggttagc agagctgtgt     360

cagatggcta cagctgtgtc acactgggag gagaccacag cctggcaatc ggtaccatta     420

gtggccatgc ccgacactgc ccagaccttt gtgttgtctg ggttgatgcc catgctgaca     480

tcaacacacc ccttaccact tcatcaggaa atctccatgg acagccagtt tcatttctcc     540

tcagagaact acaggataag gtaccacaac tcccaggatt ttcctggatc aaaccttgta     600

tctcttctgc aagtattgtg tatattggtc tgagagacgt ggaccctcct gaacatttta     660

ttttaaagaa ctatgatatc cagtattttt ccatgagaga tattgatcga cttggtatcc     720

agaaggtcat ggaacgaaca tttgatctgc tgattggcaa gagacaaaga ccaatccatt     780

tgagttttga tattgatgca tttgacccta cactggctcc agccacagga actcctgttg     840

tcgggggact aacctatcga gaaggcatgt atattgctga ggaaatacac aatacagggt     900

tgctatcagc actggatctt gttgaagtca atcctcagtt ggccacctca gaggaagagg     960

cgaagactac agctaacctg gcagtagatg tgattgcttc aagctttggt cagacaagag    1020

aaggagggca tattgtctat gaccaacttc ctactcccag ttcaccagat gaatcagaaa    1080

atcaagcacg tgtgagaatt taggagacac tgtgcactga catgtttcac aacaggcatt    1140

ccagaattat gaggcattga ggggatagat gaatactaaa tggttgtctg ggtcaatact    1200

gccttaatga gaacatttac acattctcac aattgtaaag tttcccctct attttggtga    1260

ccaatactac tgtaaatgta tttggttttt tgcagttcac agggtattaa tatgctacag    1320

tactatgtaa atttaaagaa gtcataaaca gcatttatta ccttggtata tcatactggt    1380

cttgttgctg ttgttccttc acatttaagt ggtttttcat ctttcctccc tcctcccaca    1440

gcctggctat acagtgcatc cttgaactgt cagcccacag cagcaatatg cttattctat    1500

ccacatccct aacatcatgc attcacaagg tcaaagttct ggtccacaaa cccttcccta    1560

tagaagttca atggctgcga aagaatttgt agtaaaccag gcctcccagg atggcgagct 1620

ccagtaagat gataatggaa agcagcagct tgttggttgt cactctacaa agagaagcaa 1680

agtggggagt agtcagaagt ttggataacc ttccttctaa acatttgggg gttagacctg 1740

ggaccacggc tggatactct gaggctgtat gtttgatcac acagccactt agcaggaagt 1800

actcataagg ttctttagct gtcacttagg gataacactg tctacctcac agaaatgtta 1860

aactgagaca ataaaaccca aagcat 1886

<210> 150
<211> 354
<212> PRT
<213> Homo sapiens

<400> 150

Met Ser Leu Arg Gly Ser Leu Ser Arg Leu Leu Gln Thr Arg Val His
1               5                   10                  15

Ser Ile Leu Lys Lys Ser Val His Ser Val Ala Val Ile Gly Ala Pro
            20                  25                  30

Phe Ser Gln Gly Gln Lys Arg Lys Gly Val Glu His Gly Pro Ala Ala
        35                  40                  45

Ile Arg Glu Ala Gly Leu Met Lys Arg Leu Ser Ser Leu Gly Cys His
    50                  55                  60

Leu Lys Asp Phe Gly Asp Leu Ser Phe Thr Pro Val Pro Lys Asp Asp
65                  70                  75                  80

Leu Tyr Asn Asn Leu Ile Val Asn Pro Arg Ser Val Gly Leu Ala Asn
                85                  90                  95

Gln Glu Leu Ala Glu Val Val Ser Arg Ala Val Ser Asp Gly Tyr Ser
            100                 105                 110

Cys Val Thr Leu Gly Gly Asp His Ser Leu Ala Ile Gly Thr Ile Ser
        115                 120                 125

Gly His Ala Arg His Cys Pro Asp Leu Cys Val Val Trp Val Asp Ala
    130                 135                 140

His Ala Asp Ile Asn Thr Pro Leu Thr Thr Ser Ser Gly Asn Leu His
145                 150                 155                 160

Gly Gln Pro Val Ser Phe Leu Leu Arg Glu Leu Gln Asp Lys Val Pro
                165                 170                 175

Gln Leu Pro Gly Phe Ser Trp Ile Lys Pro Cys Ile Ser Ser Ala Ser
            180                 185                 190

Ile Val Tyr Ile Gly Leu Arg Asp Val Asp Pro Pro Glu His Phe Ile
        195                 200                 205

Leu Lys Asn Tyr Asp Ile Gln Tyr Phe Ser Met Arg Asp Ile Asp Arg
    210                 215                 220

Leu Gly Ile Gln Lys Val Met Glu Arg Thr Phe Asp Leu Leu Ile Gly
225                 230                 235                 240

Lys Arg Gln Arg Pro Ile His Leu Ser Phe Asp Ile Asp Ala Phe Asp
                245                 250                 255

Pro Thr Leu Ala Pro Ala Thr Gly Thr Pro Val Val Gly Gly Leu Thr
            260                 265                 270

Tyr Arg Glu Gly Met Tyr Ile Ala Glu Glu Ile His Asn Thr Gly Leu
        275                 280                 285

Leu Ser Ala Leu Asp Leu Val Glu Val Asn Pro Gln Leu Ala Thr Ser
    290                 295                 300

Glu Glu Glu Ala Lys Thr Thr Ala Asn Leu Ala Val Asp Val Ile Ala
305                 310                 315                 320

Ser Ser Phe Gly Gln Thr Arg Glu Gly Gly His Ile Val Tyr Asp Gln
                325                 330                 335

Leu Pro Thr Pro Ser Ser Pro Asp Glu Ser Glu Asn Gln Ala Arg Val
            340                 345                 350

Arg Ile

<210>  151
<211>  1554
<212>  DNA
<213>  Homo sapiens

<400>  151
aattatatat ttttactcta tgtttctcta catgtttttt tctttccgtt gctggcggaa      60

gaggcacgtg cgctgctgaa tggagctggt cgctggttgc tacgagcagg tcctctttgg     120

gttcgctgta cacccggagc ccaaggcttg cggcgaccac gagcaatgga ctcttgtggc     180

tgacttcact caccatgctc acactgcctc cttgtcagca gtagctgtaa atagtcgttt     240

tgtggtcact gggagcaaag atgaaacaat tcacatttat gacatgaaaa agaagattga     300

gcatggggct ctagtgcatc acagtggtac aataacttgc ctgaaattct atggcaacag     360

gcatttaatc agtggagcgg aagatggact catctgtatc tgggatgcaa agaaatggga     420

atgcctgaag tcaattaaag ctcacaaagg acaggtgacc ttcctttcta ttcacccatc     480

tggcaagttg gccctgtcgg ttggtacaga taaaacttta agaacgtgga atcttgtaga     540

aggaagatca gcattcataa aaaatataaa acaaaatgct cacatagtag aatggtcccc     600

aagaggagag cagtatgtag ttatcataca gaataaaata gacatctatc agcttgacac     660

tgcatccatt agtggcacca tcacaaatga aaagagaatt tcctctgtta aatttctttc     720

agagtctgtc cttgcagtgg ctggagatga agaagttata aggttttttg actgtgattc     780

actagtgtgc ctctgcgaat ttaaagctca tgaaaacagg gtaaaggaca tgttcagttt     840

tgaaattcca gagcatcatg ttattgtttc agcatcgagt gatggtttca tcaaaatgtg     900

gaagcttaag caggataaga aagttccccc atctttactc tgtgaaataa acactaatgc     960

caggctgacg tgtcttggag tgtggctaga caaagtggca gacatgaaaa gccttcctcc    1020

agctgcagag ccttctcctg taagtaaaga acagtccaaa attggcaaaa aggagcctgg    1080

tgacacagtg cacaaagaag aaaagcggtc aaaacctaac acaaagaaac gcggtttaac    1140

aggtgacagt aagaaagcaa caaaagaaag tggcctgata tcaaccaaga agaggaaaat    1200

ggtagaaatg ttggaaaaga agaggaaaaa gaagaaaata aaaacaatgc agtgaatcac 1260

agatgtctcc tgaaagaact cttttagatg aaatcattct actcaaatgt accttaattt 1320

ttttttttcc ctgagtaaaa gcaagaaatt tcttcctttg gaaaaaatat atatattaaa 1380

aaaccacttt tagatggttt tttttaaaaa aaaaaaaaaa actggtaaaa ttacttttgg 1440

cagacagtgt tttatgaatt atgtatcatg ttgatatata atatgttaat gtgtcatgta 1500

atttttactt tgtacaaagc aaataaagat ctttctcaaa aaaaaaaaaa aaaa 1554

<210> 152
<211> 391
<212> PRT
<213> Homo sapiens

<400> 152

Met Glu Leu Val Ala Gly Cys Tyr Glu Gln Val Leu Phe Gly Phe Ala
1               5                   10                  15

Val His Pro Glu Pro Lys Ala Cys Gly Asp His Glu Gln Trp Thr Leu
            20                  25                  30

Val Ala Asp Phe Thr His His Ala His Thr Ala Ser Leu Ser Ala Val
        35                  40                  45

Ala Val Asn Ser Arg Phe Val Val Thr Gly Ser Lys Asp Glu Thr Ile
    50                  55                  60

His Ile Tyr Asp Met Lys Lys Lys Ile Glu His Gly Ala Leu Val His
65                  70                  75                  80

His Ser Gly Thr Ile Thr Cys Leu Lys Phe Tyr Gly Asn Arg His Leu
                85                  90                  95

Ile Ser Gly Ala Glu Asp Gly Leu Ile Cys Ile Trp Asp Ala Lys Lys
            100                 105                 110

Trp Glu Cys Leu Lys Ser Ile Lys Ala His Lys Gly Gln Val Thr Phe
        115                 120                 125

Leu Ser Ile His Pro Ser Gly Lys Leu Ala Leu Ser Val Gly Thr Asp
    130                 135                 140

Lys Thr Leu Arg Thr Trp Asn Leu Val Glu Gly Arg Ser Ala Phe Ile
145                 150                 155                 160

Lys Asn Ile Lys Gln Asn Ala His Ile Val Glu Trp Ser Pro Arg Gly
                165                 170                 175

Glu Gln Tyr Val Val Ile Ile Gln Asn Lys Ile Asp Ile Tyr Gln Leu
            180                 185                 190

Asp Thr Ala Ser Ile Ser Gly Thr Ile Thr Asn Glu Lys Arg Ile Ser
        195                 200                 205

Ser Val Lys Phe Leu Ser Glu Ser Val Leu Ala Val Ala Gly Asp Glu
    210                 215                 220

Glu Val Ile Arg Phe Phe Asp Cys Asp Ser Leu Val Cys Leu Cys Glu
225                 230                 235                 240

Phe Lys Ala His Glu Asn Arg Val Lys Asp Met Phe Ser Phe Glu Ile
                245                 250                 255

Pro Glu His His Val Ile Val Ser Ala Ser Ser Asp Gly Phe Ile Lys

260                 265                 270

Met Trp Lys Leu Lys Gln Asp Lys Lys Val Pro Pro Ser Leu Leu Cys
        275                 280                 285

Glu Ile Asn Thr Asn Ala Arg Leu Thr Cys Leu Gly Val Trp Leu Asp
    290                 295                 300

Lys Val Ala Asp Met Lys Ser Leu Pro Pro Ala Ala Glu Pro Ser Pro
305                 310                 315                 320

Val Ser Lys Glu Gln Ser Lys Ile Gly Lys Lys Glu Pro Gly Asp Thr
                325                 330                 335

Val His Lys Glu Glu Lys Arg Ser Lys Pro Asn Thr Lys Lys Arg Gly
            340                 345                 350

Leu Thr Gly Asp Ser Lys Lys Ala Thr Lys Glu Ser Gly Leu Ile Ser
        355                 360                 365

Thr Lys Lys Arg Lys Met Val Glu Met Leu Glu Lys Lys Arg Lys Lys
    370                 375                 380

Lys Lys Ile Lys Thr Met Gln
385                 390

<210>  153
<211>  5285
<212>  DNA
<213>  Homo sapiens

<400>  153
gatgtcttgg acatgctctg gctggctaat ctccatgttc tagccgactg aaaatacggt      60

ggccaagtgg atggtgtgct tatttgcagt ctaaagaaat ttccttttga tgtggcagaa     120

aatcgaggat gtggagtgga gaccccagac ttacttggag ctggagggtc tgccttgcat     180

cctgatcttc agtgggatgg acccgcatgg ggagtccttg ccgaggtctt tgaggtactg     240

tgacctgcga ttgataaact cctcctgctt ggtgagaaca gccttggagc aggagctggg     300

cctggctgcc tactttgtga gcaacgaggt tcccttggag aagggggcta ggaacgaggc     360

cttggagagt gatgctgaga agctgagcag cacagacaac gaggatgagg agctggggac     420

agaaggctct acctcggaga agagaagccc catgaaaagg gagaggtccc gctcccacga     480

ctcagcatcc tcatccctct cctccaaggc ttccggttca gcgctcggtg gcgagtcctc     540

ggctcagccc acagcactcc cccagggaga gcatgccagg tcgccccagc cccgtggccc     600

cgcagaggag ggcagagccc ctggtgagaa acagaggccc cgggcaagtc aggggccacc     660

ctcggccatc agcaggcaca gtcccgggcc gacgccccag cccgactgta gcctcaggac     720

cggccagagg agcgtccagg tgtcggtcac ctcgtcgtgc tcccagctgt cctcctcctc     780

gggctcatcc tcctcatccg tggcgcccgc tgccggcacg tgggtcctgc aggcctccca     840

gtgctccttg accaaggcct gccgccagcc acccattgtc ttcttgccca agctcgtgta     900

cgacatggtt gtgtccactg acagcagtgg cctgcccaag gccgcctccc tcctgccctc     960

cccctcggtc atgtgggcca gctctttccg ccccctgctc agcaagacca tgacatccac    1020

cgagcagtcc ctctactacc ggcagtggac ggtgccccgg cccagccaca tggactacgg    1080

caaccgggcc gagggccgcg tggacggctt ccacccccgc aggctgctgc tcagcggccc 1140
ccctcagatc gggaagacag gtgcctacct gcagttcctc agtgtcctgt ccaggatgct 1200
tgttcggctc acagaagtgg atgtctatga cgaggaggag atcaatatca acctcagaga 1260
agaatctgac tggcattatc tccagcttag cgacccctgg ccagacctgg agctgttcaa 1320
gaagttgccc tttgactaca tcattcacga cccgaagtat gaagatgcca gcctgatttg 1380
ttcgcactat cagggtataa agagtgaaga cagagggatg tcccggaagc cggaggacct 1440
ttatgtgcgg cgtcagacgg cacggatgag actgtccaag tacgcagcgt acaacactta 1500
ccaccactgt gagcagtgcc accagtacat gggcttccac ccccgctacc agctgtatga 1560
gtccaccctg cacgcctttg ccttctctta ctccatgcta ggagaggaga tccagctgca 1620
cttcatcatc cccaagtcca aggagcacca ctttgtcttc agccaacctg gaggccagct 1680
ggagagcatg cgactacccc tcgtgacaga caagagccat gaatatataa aaagtccgac 1740
attcactcca accaccggcc gtcacgaaca tgggctcttt aatctgtacc acgcaatgga 1800
cggtgccagc catttgcacg tgctggttgt caaggaatac gagatggcaa tttataagaa 1860
atattggccc aaccacatca tgctggtgct ccccagtatc ttcaacagtg ctggagttgg 1920
tgctgctcat ttcctcatca aggagctgtc ctaccataac ctggagctcg agcggaaccg 1980
gcaggaggag ctgggaatca agccgcagga catctggcct ttcattgtga tctctgatga 2040
ctcctgcgtg atgtggaacg tggtggatgt caactctgct ggggagagaa gcagggagtt 2100
ctcctggtcg gaaaggaacg tgtctttgaa gcacatcatg cagcacatcg aggcggcccc 2160
cgacatcatg cactacgccc tgctgggcct gcggaagtgg tccagcaaga cccgggccag 2220
cgaggtgcaa gagcccttct cccgctgcca cgtgcacaac ttcatcatcc tgaacgtgga 2280
cctgacccag aacgtgcagt acaaccagaa ccggttcctg tgtgacgatg tagacttcaa 2340
cctgcgggtg cacagcgccg gcctcctgct ctgccggttc aaccgcttca gcgtgatgaa 2400
gaagcagatc gtggtgggcg gccacaggtc cttccacatc acatccaagg tgtctgataa 2460
ctctgccgcg gtcgtgccgg cccagtacat ctgtgccccg gacagcaagc acacgttcct 2520
cgcagcgccc gcccagctcc tgctggagaa gttcctgcag caccacagcc acctcttctt 2580
cccgctgtcc ctgaagaacc atgaccaccc agtgctgtct gtcgactgtt acctgaacct 2640
gggatctcag atttctgttt gctatgtgag ctccaggccc cactctttaa acatcagctg 2700
ctcggacttg ctgttcagtg ggctgctgct gtacctctgt gactcttttg tgggagctag 2760
ctttttgaaa aagtttcatt ttctgaaagg tgcgacgttg tgtgtcatct gtcaggaccg 2820
gagctcactg cgccagacgg tcgtccgcct ggagctcgag gacgagtggc agttccggct 2880
gcgcgatgag ttccagaccg ccaatgccag ggaagaccgg ccgctctttt ttctgacggg 2940
acgacacatc tgaggaagac agcggcgagt tttctgaaga gatgagtgct cagagccctc 3000
atgctgttga ggctaaaggg aggcctggaa cggtggggcg tttgactgga atggacccca 3060
gggactgtcc aggtgcagcc cctcctagta cacatgggcc cccgaggccg tggtcctggg 3120

agccaggaag actccgcagt gggtgagaat gaaaacttga gactcccaag ttctgggcca 3180
gcccattgct ctgggctgtt ttaaagccca tttcacgagg aacaaagatt tacttcctgt 3240
cctgccattc gtgtgcttcc atggacaaac ctgatttttt tctcttagtt ctaaagaatc 3300
ttgggttatt ttgtagcggt gccagtattt cagtagatgg gatttcagcc aagtaggttc 3360
ccctgtaacc tcctacaaag caatattcca aaggaacatt ttaactgtaa aggctggaga 3420
caagaaaaaa taagtagatc gttttaataa caattattta attgcctata agtttgctgt 3480
ttcagaggct agcccaaagg catcaaattt aataaagtta aacaaattga tttacttcag 3540
agcaaatatg atcctattaa aataatatag ggtaaatacc ctacctctta gaaagggcaa 3600
aaatgcaaag aagctttctt taaaactaaa agggtttttt ggggggggag ttggcgggga 3660
ggaaataagg ctaacagagg ttgacctaaa attagcctta caaaggagaa aggaccacat 3720
tgcttacttg aaacagacaa tgaaaacaac caaagtgata tataaaatag ttgatgagaa 3780
ctagacttat gactgtagtt tactagagtt tagttttcag ttgctgaagt agctcatttt 3840
ctcttactaa tgtttggttc ctcagggaag aatctcactt gactagagag gaggtgggaa 3900
cagaagagag aaggaggcag ggagatgtat ttcttagggc tcaccccttc acagactgac 3960
agaatggttt tgttttgttt tgttttgttt tgttttgttt ttgagatgga ctctagctct 4020
gtcacccagg ctggagtgca gtggtgcgat ctcggctcac tgcaagctcc gcctcccggg 4080
ttctcaccat tctcctgcct cagcctcccg agtagctggg actacaggcg cccaccacca 4140
cgcccggcta attttttgta ttttttagta gagacggggt ttcaccatgt tagccaggat 4200
ggtctcgatc tcctgacctc gtgatccgcc cgcctcggcc tcccaaagtg ctgggattac 4260
aggcgtgagc caccgtgcct gccccagaat ggtttttaaa gccacagttg agaggccacc 4320
cattgcccgg cgcctggaca gtgatcatct tgttcatctt gttcagtcct ttcttgtgtg 4380
attggaatta ttcatcccct ttgaaagatg agaaggttga gatgcaaaga gtctaccttt 4440
ccaagttctc actgctggaa agagctagaa gcacagttca aagttctggc ttctggactc 4500
tgcagtccag gtctcccttc tcccacttgc ctaccctcaa tgccacactg tttttgaagt 4560
ggcccataac ttgaaggaaa agtttaaaga cagttcaatt taatcatcag aatgcattct 4620
tttttttttc ggagacggag tttcactctt gctgcccagg ctggagtgca atggtgcaat 4680
gatctcggct cactgcaacc tctgcctcct gggttcaagt gattctccag cctcagcctc 4740
ccgagtagct gggattatgg gcgcccacca ccatgcccag ctaatttttg tatttttttt 4800
ttttagtaga gatggggttt cgccaggttg gccaggctgg tcttgtgaac tcctggcctc 4860
aggtgatctg cccacctcat cctccaaaag tgctgggatt acaggcatga gccactgcgc 4920
ctggcctcag aatgcattct tacacatcta tcctagacat ttataagcac tctaatggat 4980
aacaatccaa gaataaatga ttgtaaaaga tgatgccgaa gagttgatgt caatcttttt 5040
ttcctaagaa aaaaagtccg cgagtattaa atatttagat caatgtttat aaaatgatta 5100
ctttgtatat ctcattattc ctattttgga ataaaaactg accttcttta atcatatact 5160

tgtcttttgt aaatagcagc ttttgtgtca ttctccccac tttattagtt aatttaaatt 5220

ggaaaaaacc ctcaaactaa tattcttgtc tgttccagtc ttataaataa aacttataat 5280

gcatg 5285

<210> 154
<211> 947
<212> PRT
<213> Homo sapiens

<400> 154
   Met Trp Gln Lys Ile Glu Asp Val Glu Trp Arg Pro Gln Thr Tyr Leu
   1 5 10 15
   Glu Leu Glu Gly Leu Pro Cys Ile Leu Ile Phe Ser Gly Met Asp Pro
   20 25 30
   His Gly Glu Ser Leu Pro Arg Ser Leu Arg Tyr Cys Asp Leu Arg Leu
   35 40 45
   Ile Asn Ser Ser Cys Leu Val Arg Thr Ala Leu Glu Gln Glu Leu Gly
   50 55 60
   Leu Ala Ala Tyr Phe Val Ser Asn Glu Val Pro Leu Glu Lys Gly Ala
   65 70 75 80
   Arg Asn Glu Ala Leu Glu Ser Asp Ala Glu Lys Leu Ser Ser Thr Asp
   85 90 95
   Asn Glu Asp Glu Glu Leu Gly Thr Glu Gly Ser Thr Ser Glu Lys Arg
   100 105 110
   Ser Pro Met Lys Arg Glu Arg Ser Arg Ser His Asp Ser Ala Ser Ser
   115 120 125
   Ser Leu Ser Ser Lys Ala Ser Gly Ser Ala Leu Gly Gly Glu Ser Ser
   130 135 140
   Ala Gln Pro Thr Ala Leu Pro Gln Gly Glu His Ala Arg Ser Pro Gln
   145 150 155 160
   Pro Arg Gly Pro Ala Glu Glu Gly Arg Ala Pro Gly Glu Lys Gln Arg
   165 170 175
   Pro Arg Ala Ser Gln Gly Pro Pro Ser Ala Ile Ser Arg His Ser Pro
   180 185 190
   Gly Pro Thr Pro Gln Pro Asp Cys Ser Leu Arg Thr Gly Gln Arg Ser
   195 200 205
   Val Gln Val Ser Val Thr Ser Ser Cys Ser Gln Leu Ser Ser Ser Ser
   210 215 220
   Gly Ser Ser Ser Ser Ser Val Ala Pro Ala Ala Gly Thr Trp Val Leu
   225 230 235 240
   Gln Ala Ser Gln Cys Ser Leu Thr Lys Ala Cys Arg Gln Pro Pro Ile
   245 250 255
   Val Phe Leu Pro Lys Leu Val Tyr Asp Met Val Val Ser Thr Asp Ser
   260 265 270
   Ser Gly Leu Pro Lys Ala Ala Ser Leu Leu Pro Ser Pro Ser Val Met
   275 280 285
   Trp Ala Ser Ser Phe Arg Pro Leu Leu Ser Lys Thr Met Thr Ser Thr
       290                 295                 300
   Glu Gln Ser Leu Tyr Tyr Arg Gln Trp Thr Val Pro Arg Pro Ser His
   305                 310                 315                 320
   Met Asp Tyr Gly Asn Arg Ala Glu Gly Arg Val Asp Gly Phe His Pro
                   325                 330                 335
   Arg Arg Leu Leu Leu Ser Gly Pro Pro Gln Ile Gly Lys Thr Gly Ala
               340                 345                 350
   Tyr Leu Gln Phe Leu Ser Val Leu Ser Arg Met Leu Val Arg Leu Thr
           355                 360                 365
   Glu Val Asp Val Tyr Asp Glu Glu Glu Ile Asn Ile Asn Leu Arg Glu
       370                 375                 380
   Glu Ser Asp Trp His Tyr Leu Gln Leu Ser Asp Pro Trp Pro Asp Leu
   385                 390                 395                 400
   Glu Leu Phe Lys Lys Leu Pro Phe Asp Tyr Ile Ile His Asp Pro Lys
                   405                 410                 415
   Tyr Glu Asp Ala Ser Leu Ile Cys Ser His Tyr Gln Gly Ile Lys Ser
               420                 425                 430
   Glu Asp Arg Gly Met Ser Arg Lys Pro Glu Asp Leu Tyr Val Arg Arg
           435                 440                 445
   Gln Thr Ala Arg Met Arg Leu Ser Lys Tyr Ala Ala Tyr Asn Thr Tyr
       450                 455                 460
   His His Cys Glu Gln Cys His Gln Tyr Met Gly Phe His Pro Arg Tyr
   465                 470                 475                 480
   Gln Leu Tyr Glu Ser Thr Leu His Ala Phe Ala Phe Ser Tyr Ser Met
                   485                 490                 495
   Leu Gly Glu Glu Ile Gln Leu His Phe Ile Ile Pro Lys Ser Lys Glu
               500                 505                 510
   His His Phe Val Phe Ser Gln Pro Gly Gly Gln Leu Glu Ser Met Arg
           515                 520                 525
   Leu Pro Leu Val Thr Asp Lys Ser His Glu Tyr Ile Lys Ser Pro Thr
       530                 535                 540
   Phe Thr Pro Thr Thr Gly Arg His Glu His Gly Leu Phe Asn Leu Tyr
   545                 550                 555                 560
   His Ala Met Asp Gly Ala Ser His Leu His Val Leu Val Val Lys Glu
                   565                 570                 575
   Tyr Glu Met Ala Ile Tyr Lys Lys Tyr Trp Pro Asn His Ile Met Leu
               580                 585                 590
   Val Leu Pro Ser Ile Phe Asn Ser Ala Gly Val Gly Ala Ala His Phe
           595                 600                 605
   Leu Ile Lys Glu Leu Ser Tyr His Asn Leu Glu Leu Glu Arg Asn Arg
       610                 615                 620
   Gln Glu Glu Leu Gly Ile Lys Pro Gln Asp Ile Trp Pro Phe Ile Val
   625                 630                 635                 640
   Ile Ser Asp Asp Ser Cys Val Met Trp Asn Val Val Asp Val Asn Ser
   645 650 655
   Ala Gly Glu Arg Ser Arg Glu Phe Ser Trp Ser Glu Arg Asn Val Ser
   660 665 670
   Leu Lys His Ile Met Gln His Ile Glu Ala Ala Pro Asp Ile Met His
   675 680 685
   Tyr Ala Leu Leu Gly Leu Arg Lys Trp Ser Ser Lys Thr Arg Ala Ser
   690 695 700
   Glu Val Gln Glu Pro Phe Ser Arg Cys His Val His Asn Phe Ile Ile
   705 710 715 720
   Leu Asn Val Asp Leu Thr Gln Asn Val Gln Tyr Asn Gln Asn Arg Phe
   725 730 735
   Leu Cys Asp Asp Val Asp Phe Asn Leu Arg Val His Ser Ala Gly Leu
   740 745 750
   Leu Leu Cys Arg Phe Asn Arg Phe Ser Val Met Lys Lys Gln Ile Val
   755 760 765
   Val Gly Gly His Arg Ser Phe His Ile Thr Ser Lys Val Ser Asp Asn
   770 775 780
   Ser Ala Ala Val Val Pro Ala Gln Tyr Ile Cys Ala Pro Asp Ser Lys
   785 790 795 800
   His Thr Phe Leu Ala Ala Pro Ala Gln Leu Leu Leu Glu Lys Phe Leu
   805 810 815
   Gln His His Ser His Leu Phe Phe Pro Leu Ser Leu Lys Asn His Asp
   820 825 830
   His Pro Val Leu Ser Val Asp Cys Tyr Leu Asn Leu Gly Ser Gln Ile
   835 840 845
   Ser Val Cys Tyr Val Ser Ser Arg Pro His Ser Leu Asn Ile Ser Cys
   850 855 860
   Ser Asp Leu Leu Phe Ser Gly Leu Leu Leu Tyr Leu Cys Asp Ser Phe
   865 870 875 880
   Val Gly Ala Ser Phe Leu Lys Lys Phe His Phe Leu Lys Gly Ala Thr
   885 890 895
   Leu Cys Val Ile Cys Gln Asp Arg Ser Ser Leu Arg Gln Thr Val Val
   900 905 910
   Arg Leu Glu Leu Glu Asp Glu Trp Gln Phe Arg Leu Arg Asp Glu Phe
   915 920 925
   Gln Thr Ala Asn Ala Arg Glu Asp Arg Pro Leu Phe Phe Leu Thr Gly
   930 935 940
   Arg His Ile
   945

<210> 155
<211> 1167
<212> DNA
<213> Homo sapiens

<400> 155
atgacagcct ccgtgctcct ccacccccgc tggatcgagc ccaccgtcat gtttctctac 60

gacaacggcg gcggcctggt ggccgacgag ctcaacaaga acatggaagg ggcggcggcg 120

gctgcagcag cggctgcagc ggcggcggct gccggggccg ggggcggggg cttcccccac 180
ccggcggctg cggcggcagg gggcaacttc tcggtggcgg ccgcggccgc ggctgcggcg 240
gccgccgcgg ccaaccagtg ccgcaacctg atggcgcacc cggcgccctt ggcgccagga 300
gccgcgtccg cctacagcag cgcccccggg gaggcgcccc cgtcggctgc cgccgctgct 360
gccgcggctg ccgctgcagc cgccgccgcc gccgccgcgt cgtcctcggg aggtcccggc 420
ccggcgggcc cggcggcggc agaggcggcc aagcaatgca gcccctgctc ggcagcggcg 480
cagagctcgt cggggcccgc ggcgctgccc tatggctact tcggcagcgg ctactacccg 540
tgcgcccgca tgggcccgcc ccccaacgcc atcaagtcgt gcccccagcc cccctcggcc 600
gccgccgccg ccgccttcgc ggacaagtac atggataccg ccggcccagc tgccgaggag 660
ttcagctccc gcgctaagga gttcgcgttc taccaccagg gctacgcagc cgggccttac 720
caccaccatc agcccatgcc tggctacctg gatatgccag tggtgccggg cctcgggggc 780
cccggcgagt cgcgccacga acccttgggt cttcccatgg aaagctacca gccctgggcg 840
ctgcccaacg gctggaacgg ccaaatgtac tgccccaaag agcaggcgca gcctccccac 900
ctctggaagt ccactctgcc cgacgtggtc tcccatccct cggatgccag ctcctatagg 960
agggggagaa agaagcgcgt gccttatacc aaggtgcaat taaaagaact tgaacgggaa 1020
tacgccacga ataaattcat tactaaggac aaacggaggc ggatatcagc cacgacgaat 1080
ctctctgagc ggcaggtcac aatctggttc cagaacagga gggttaaaga gaaaaaagtc 1140
atcaacaaac tgaaaaccac tagttaa 1167

<210> 156
<211> 388
<212> PRT
<213> Homo sapiens

<400> 156

Met Thr Ala Ser Val Leu Leu His Pro Arg Trp Ile Glu Pro Thr Val
1               5                   10                  15

Met Phe Leu Tyr Asp Asn Gly Gly Gly Leu Val Ala Asp Glu Leu Asn
            20                  25                  30

Lys Asn Met Glu Gly Ala Ala Ala Ala Ala Ala Ala Ala Ala Ala Ala
        35                  40                  45

Ala Ala Ala Gly Ala Gly Gly Gly Gly Phe Pro His Pro Ala Ala Ala
    50                  55                  60

Ala Ala Gly Gly Asn Phe Ser Val Ala Ala Ala Ala Ala Ala Ala Ala
65                  70                  75                  80

Ala Ala Ala Ala Asn Gln Cys Arg Asn Leu Met Ala His Pro Ala Pro
                85                  90                  95

Leu Ala Pro Gly Ala Ala Ser Ala Tyr Ser Ser Ala Pro Gly Glu Ala
            100                 105                 110

Pro Pro Ser Ala Ala Ala Ala Ala Ala Ala Ala Ala Ala Ala Ala Ala
        115                 120                 125

Ala Ala Ala Ala Ala Ser Ser Ser Gly Gly Pro Gly Pro Ala Gly Pro
    130                 135                 140

Ala Ala Ala Glu Ala Ala Lys Gln Cys Ser Pro Cys Ser Ala Ala Ala
145                 150                 155                 160

Gln Ser Ser Ser Gly Pro Ala Ala Leu Pro Tyr Gly Tyr Phe Gly Ser
                165                 170                 175

Gly Tyr Tyr Pro Cys Ala Arg Met Gly Pro Pro Pro Asn Ala Ile Lys
            180                 185                 190

Ser Cys Pro Gln Pro Pro Ser Ala Ala Ala Ala Ala Ala Phe Ala Asp
        195                 200                 205

Lys Tyr Met Asp Thr Ala Gly Pro Ala Ala Glu Glu Phe Ser Ser Arg
    210                 215                 220

Ala Lys Glu Phe Ala Phe Tyr His Gln Gly Tyr Ala Ala Gly Pro Tyr
225                 230                 235                 240

His His His Gln Pro Met Pro Gly Tyr Leu Asp Met Pro Val Val Pro
                245                 250                 255

Gly Leu Gly Gly Pro Gly Glu Ser Arg His Glu Pro Leu Gly Leu Pro
            260                 265                 270

Met Glu Ser Tyr Gln Pro Trp Ala Leu Pro Asn Gly Trp Asn Gly Gln
        275                 280                 285

Met Tyr Cys Pro Lys Glu Gln Ala Gln Pro Pro His Leu Trp Lys Ser
    290                 295                 300

Thr Leu Pro Asp Val Val Ser His Pro Ser Asp Ala Ser Ser Tyr Arg
305                 310                 315                 320

Arg Gly Arg Lys Lys Arg Val Pro Tyr Thr Lys Val Gln Leu Lys Glu
                325                 330                 335

Leu Glu Arg Glu Tyr Ala Thr Asn Lys Phe Ile Thr Lys Asp Lys Arg
            340                 345                 350

Arg Arg Ile Ser Ala Thr Thr Asn Leu Ser Glu Arg Gln Val Thr Ile
        355                 360                 365

Trp Phe Gln Asn Arg Arg Val Lys Glu Lys Lys Val Ile Asn Lys Leu
    370                 375                 380

Lys Thr Thr Ser
385

<210>  157
<211>  3004
<212>  DNA
<213>  Homo sapiens

<400>  157
cccttatggc gattgggcgg ctgcagagac caggactcag ttcccctgcc ctagtctgag      60

cctagtgggt gggactcagc tcagagtcag ttttcagaag caggtttcag ttgcagagtt     120

ttcctacact tttcctgcgc tagagcagcg agcagcctgg aacagaccca ggcggaggac     180

acctgtgggg gagggagcgc ctggaggagc ttagagaccc cagccgggcg tgatctcacc     240

atgtgcggat ttgcgaggcg cgccctggag ctgctagaga tccggaagca cagccccgag     300

gtgtgcgaag ccaccaagac tgcggctctt ggagaaagcg tgagcagggg gccaccgcgg     360

tctccggcct gtctgcaccc tgtcgcctga gctgcctgac agtgacaatg acatcccagt 420
taccagtgtc cttgaattga tagtggcttc tgtttgtcag tctcatataa gaactacagc 480
tcatcaggag gagatcgcag cagggtaaga gacaccaaca ccatgttctg cacgaagctc 540
aaggatctca agatcacagg agagtgtcct ttctccttac tggcaccagg tcaagttcct 600
aacgagtctt cagaggaggc agcaggaagc tcagagagct gcaaagcaac cgtgcccatc 660
tgtcaagaca ttcctgagaa gaacatacaa gaaagtcttc ctcaaagaaa aaccagtcgg 720
agccgagtct atcttcacac tttggcagag agtatttgca aactgatttt cccagagttt 780
gaacggctga atgttgcact tcagagaaca ttggcaaagc acaaaataaa agaaagcagg 840
aaatctttgg aaagagaaga ctttgaaaaa acaattgcag agcaagcagt gcagcagagt 900
ccagtggagt tatcaaagaa tctcttggtg aagaggtttt taaaatatgt tacgaggaag 960
atgaaaacat ccttggggtg gttggaggca cccttaaaga tttttaaaca gcttcagtac 1020
ccttctgaaa cagagcagcc attgccaaga agcaggaaaa aggggcagct tgaggacgcc 1080
tccattctat gcctggataa ggaggatgat tttctacatg tttactactt cttccctaag 1140
agaaccacct ccctgattct tcccggcatc ataaaggcag ctgctcacgt attatatgaa 1200
acggaagtgg aagtgtcgtt aatgcctccc tgcttccata atgattgcag cgagtttgtg 1260
aatcagccct acttgttgta ctccgttcac atgaaaagca ccaagccatc cctgtccccc 1320
agcaaacccc agtcctcgct ggtgattccc acatcgctat tctgcaagac atttccattc 1380
catttcatgt ttgacaaaga tatgacaatt ctgcaatttg gcaatggcat cagaaggctg 1440
atgaacagga gagactttca aggaaagcct aattttgaat actttgaaat tctgactcca 1500
aaaatcaacc agacctttag cgggatcatg actatgttga atatgcagtt tgttgtacga 1560
gtgaggagat gggacaactc tgtgaagaaa tcttcaaggg ttatggacct caaaggccaa 1620
atgatctaca ttgttgaatc cagtgcaatc ttgtttttgg ggtcaccctg tgtggacaga 1680
ttagaagatt ttacaggacg agggctctac ctctcagaca tcccaattca caatgcactg 1740
agggatgtgg tcttaatagg ggaacaagcc cgagctcaag atggcctgaa gaagaggctg 1800
gggaagctga aggctaccct tgagcaagcc caccaagccc tggaggagga gaagaaaaag 1860
acagtagacc ttctgtgctc catatttccc tgtgaggttg ctcagcagct gtggcaaggg 1920
caagttgtgc aagccaagaa gttcagtaat gtcaccatgc tcttctcaga catcgttggg 1980
ttcactgcca tctgctccca gtgctcaccg ctgcaggtca tcaccatgct caatgcactg 2040
tacactcgct tcgaccagca gtgtggagag ctggatgtct acaaggtgga gaccattgcg 2100
atgcctattg tgtggcttgg gggattacac aaagagagtg atactcatgc tgttcagata 2160
gcgctgatgg ccctgaagat gatggagctc tctgatgaag ttatgtctcc ccatggagaa 2220
cctatcaaga tgcgaattgg actgcactct ggatcagttt ttgctggcgt cgttggagtt 2280
aaaatgcccc gttactgtct ttttggaaac aatgtcactc tggctaacaa atttgagtcc 2340
tgcagtgtac cacgaaaaat caatgtcagc ccaacaactt acagattact caaagactgt 2400

cctggtttcg tgtttacccc tcgatcaagg gaggaacttc caccaaactt ccctagtgaa 2460

atccccggaa tctgccattt tctggatgct taccaacaag gaacaaactc aaaaccatgc 2520

ttccaaaaga aagatgtgga agatgcaagc caatttttta ggcaaagcat caggaataga 2580

ttagcaacct atatacctat ttataagtct ttggggtttg actcattgaa gatgtgtaga 2640

gcctctgaaa gcactttagg gattgtagat ggctaacaag cagtattaaa atttcaggag 2700

ccaagtcaca atctttctcc tgtttaacat gacaaaatgt actcacttca gtacttcagc 2760

tcttcaagaa aaaaaaaaaa accttaaaaa gctacttttg tgggagtatt tctattatat 2820

aaccagcact tactacctgt actcaaaatt cagcaccttg tacatatatc agataattgt 2880

agtcaattgt acaaactgat ggagtcacct gcaatctcat atcctggtgg aatgccatgg 2940

ttattaaagt gtgtttgtga tagttgtcgt caaaaaaaaa aaaaaaaaaa aaaaaaaaaa 3000

aaaa 3004

<210> 158
<211> 717
<212> PRT
<213> Homo sapiens

<400> 158

Met Phe Cys Thr Lys Leu Lys Asp Leu Lys Ile Thr Gly Glu Cys Pro
1               5                   10                  15

Phe Ser Leu Leu Ala Pro Gly Gln Val Pro Asn Glu Ser Ser Glu Glu
            20                  25                  30

Ala Ala Gly Ser Ser Glu Ser Cys Lys Ala Thr Val Pro Ile Cys Gln
        35                  40                  45

Asp Ile Pro Glu Lys Asn Ile Gln Glu Ser Leu Pro Gln Arg Lys Thr
    50                  55                  60

Ser Arg Ser Arg Val Tyr Leu His Thr Leu Ala Glu Ser Ile Cys Lys
65                  70                  75                  80

Leu Ile Phe Pro Glu Phe Glu Arg Leu Asn Val Ala Leu Gln Arg Thr
                85                  90                  95

Leu Ala Lys His Lys Ile Lys Glu Ser Arg Lys Ser Leu Glu Arg Glu
            100                 105                 110

Asp Phe Glu Lys Thr Ile Ala Glu Gln Ala Val Gln Gln Ser Pro Val
        115                 120                 125

Glu Leu Ser Lys Asn Leu Leu Val Lys Arg Phe Leu Lys Tyr Val Thr
    130                 135                 140

Arg Lys Met Lys Thr Ser Leu Gly Trp Leu Glu Ala Pro Leu Lys Ile
145                 150                 155                 160

Phe Lys Gln Leu Gln Tyr Pro Ser Glu Thr Glu Gln Pro Leu Pro Arg
                165                 170                 175

Ser Arg Lys Lys Gly Gln Leu Glu Asp Ala Ser Ile Leu Cys Leu Asp
            180                 185                 190

Lys Glu Asp Asp Phe Leu His Val Tyr Tyr Phe Phe Pro Lys Arg Thr
        195                 200                 205

Thr Ser Leu Ile Leu Pro Gly Ile Ile Lys Ala Ala Ala His Val Leu
    210                 215                 220

Tyr Glu Thr Glu Val Glu Val Ser Leu Met Pro Pro Cys Phe His Asn
225                 230                 235                 240

Asp Cys Ser Glu Phe Val Asn Gln Pro Tyr Leu Leu Tyr Ser Val His
                245                 250                 255

Met Lys Ser Thr Lys Pro Ser Leu Ser Pro Ser Lys Pro Gln Ser Ser
            260                 265                 270

Leu Val Ile Pro Thr Ser Leu Phe Cys Lys Thr Phe Pro Phe His Phe
        275                 280                 285

Met Phe Asp Lys Asp Met Thr Ile Leu Gln Phe Gly Asn Gly Ile Arg
    290                 295                 300

Arg Leu Met Asn Arg Arg Asp Phe Gln Gly Lys Pro Asn Phe Glu Tyr
305                 310                 315                 320

Phe Glu Ile Leu Thr Pro Lys Ile Asn Gln Thr Phe Ser Gly Ile Met
                325                 330                 335

Thr Met Leu Asn Met Gln Phe Val Val Arg Val Arg Arg Trp Asp Asn
            340                 345                 350

Ser Val Lys Lys Ser Ser Arg Val Met Asp Leu Lys Gly Gln Met Ile
        355                 360                 365

Tyr Ile Val Glu Ser Ser Ala Ile Leu Phe Leu Gly Ser Pro Cys Val
    370                 375                 380

Asp Arg Leu Glu Asp Phe Thr Gly Arg Gly Leu Tyr Leu Ser Asp Ile
385                 390                 395                 400

Pro Ile His Asn Ala Leu Arg Asp Val Val Leu Ile Gly Glu Gln Ala
                405                 410                 415

Arg Ala Gln Asp Gly Leu Lys Lys Arg Leu Gly Lys Leu Lys Ala Thr
            420                 425                 430

Leu Glu Gln Ala His Gln Ala Leu Glu Glu Glu Lys Lys Lys Thr Val
        435                 440                 445

Asp Leu Leu Cys Ser Ile Phe Pro Cys Glu Val Ala Gln Gln Leu Trp
    450                 455                 460

Gln Gly Gln Val Val Gln Ala Lys Lys Phe Ser Asn Val Thr Met Leu
465                 470                 475                 480

Phe Ser Asp Ile Val Gly Phe Thr Ala Ile Cys Ser Gln Cys Ser Pro
                485                 490                 495

Leu Gln Val Ile Thr Met Leu Asn Ala Leu Tyr Thr Arg Phe Asp Gln
            500                 505                 510

Gln Cys Gly Glu Leu Asp Val Tyr Lys Val Glu Thr Ile Ala Met Pro
        515                 520                 525

Ile Val Trp Leu Gly Gly Leu His Lys Glu Ser Asp Thr His Ala Val
    530                 535                 540

Gln Ile Ala Leu Met Ala Leu Lys Met Met Glu Leu Ser Asp Glu Val
545                 550                 555                 560

Met Ser Pro His Gly Glu Pro Ile Lys Met Arg Ile Gly Leu His Ser

565                 570                 575

Gly Ser Val Phe Ala Gly Val Val Gly Val Lys Met Pro Arg Tyr Cys
            580                 585                 590

Leu Phe Gly Asn Asn Val Thr Leu Ala Asn Lys Phe Glu Ser Cys Ser
        595                 600                 605

Val Pro Arg Lys Ile Asn Val Ser Pro Thr Thr Tyr Arg Leu Leu Lys
    610                 615                 620

Asp Cys Pro Gly Phe Val Phe Thr Pro Arg Ser Arg Glu Glu Leu Pro
625                 630                 635                 640

Pro Asn Phe Pro Ser Glu Ile Pro Gly Ile Cys His Phe Leu Asp Ala
                645                 650                 655

Tyr Gln Gln Gly Thr Asn Ser Lys Pro Cys Phe Gln Lys Lys Asp Val
            660                 665                 670

Glu Asp Ala Ser Gln Phe Phe Arg Gln Ser Ile Arg Asn Arg Leu Ala
        675                 680                 685

Thr Tyr Ile Pro Ile Tyr Lys Ser Leu Gly Phe Asp Ser Leu Lys Met
    690                 695                 700

Cys Arg Ala Ser Glu Ser Thr Leu Gly Ile Val Asp Gly
705                 710                 715

<210>  159
<211>  2710
<212>  DNA
<213>  Homo sapiens

<400>  159
cgccggcggg actggtctga agagacgcgg ggacaaagtg gcaacgactt ggacatctga      60

gctgtcactg ccgaaaacag gccgcaagag agataatcaa tatgcatttc caagcctttt     120

ggctatgttt gggtcttctg ttcatctcaa ttaatgcaga atttatggat gatgatgttg     180

agacggaaga ctttgaagaa aattcagaag aaattgatgt taatgaaagt gaactttcct     240

cagagattaa atataagaca cctcaaccta taggagaagt atattttgca gaaacttttg     300

atagtggaag gttggctgga tgggtcttat caaaagcaaa gaaagatgac atggatgagg     360

aaatttcaat atacgatgga agatgggaaa ttgaagagtt gaaagaaaac caggtacctg     420

gtgacagagg actggtatta aaatctagag caaagcatca tgcaatatct gctgtattag     480

caaaaccatt catttttgct gataaaccct tgatagttca atatgaagta aattttcaag     540

atggtattga ttgtggaggt gcatacatta aactcctagc agacactgat gatttgattc     600

tggaaaactt ttatgataaa acatcctata tcattatgtt tggaccagat aaatgtggag     660

aagattataa acttcatttt atcttcagac ataaacatcc caaaactgga gttttcgaag     720

agaaacatgc caaacctcca gatgtagacc ttaaaaagtt ctttacagac aggaagactc     780

atctttatac ccttgtgatg aatccagatg acacatttga ggtgttagtt gatcaaacag     840

ttgtaaacaa aggaagcctc ctagaggatg tggttcctcc tatcaaacct cccaaagaaa     900

ttgaagatcc caatgataaa aaacctgagg aatgggatga aagagcaaaa attcctgatc     960

cttctgccgt caaaccagaa gactgggatg aaagtgaacc tgcccaaata gaagattcaa    1020

gtgttgttaa acctgctggc tggcttgatg atgaaccaaa atttatccct gatcctaatg 1080
ctgaaaaacc tgatgactgg aatgaagaca cggatggaga atgggaggca cctcagattc 1140
ttaatccagc atgtcggatt gggtgtggtg agtggaaacc tcccatgata gataacccaa 1200
aatacaaagg agtatggaga cctccactgg tcgataatcc taactatcag ggaatctgga 1260
gtcctcgaaa aattcctaat ccagattatt tcgaagatga tcatccattt cttctgactt 1320
ctttcagtgc tcttggttta gagctttggt ctatgacctc tgatatctac tttgataatt 1380
ttattatctg ttcggaaaag gaagtagcag atcactgggc tgcagatggt tggagatgga 1440
aaataatgat agcaaatgct aataagcctg gtgtattaaa acagttaatg gcagctgctg 1500
aagggcaccc atggctttgg ttgatttatc ttgtgacagc aggagtgcca atagcattaa 1560
ttacttcatt ttgttggcca agaaaagtaa agaaaaaaca taaagataca gagtataaaa 1620
aaaccgacat atgtatacca caaacaaaag gagtactaga gcaagaagaa aaggaagaga 1680
aagcagccct ggaaaaacca atggacctgg aagaggaaaa aaagcaaaat gatggtgaaa 1740
tgcttgaaaa agaagaggaa agtgaacctg aggaaaagag tgaagaagaa attgaaatca 1800
tagaagggca agaagaaagt aatcaatcaa ataagtctgg gtcagaggat gagatgaaag 1860
aagcagatga gagcacagga tctggagatg ggccgataaa gtcagtacgc aaaagaagag 1920
tacgaaagga ctaaactaga ttgaaatatt tttaattccc gagaggatgt ttggcattgt 1980
aaaaatcagc atgccagacc tgaactttaa tcagtctgca catcctgttt ctaatatcta 2040
gcaacattat attctttcag acatttattt tagtccttca tttccgagga aaaagaagca 2100
actttgaagt tacctcatct ttgaatttag aataaaagtg gcacattaca tatcggatct 2160
aagagattaa taccattaga agttacacag ttttagttgt ttggagatag ttttggtttg 2220
tacagaacaa aataatatgt agcagcttca ttgctattgg aaaaatcagt tattggaatt 2280
tccacttaaa tggctataca acaatataac tggtagttct ataataaaaa tgagcatatg 2340
ttctgttgtg aagagctaaa tgcaataaag tttctgtatg gttgtttgat tctatcaaca 2400
attgaaagtg ttgtatatga cccacattta cctagtttgt gtcaaattat agttacagtg 2460
agttgtttgc ttaaattata gattccttta aggacatgcc ttgttcataa aatcactgga 2520
ttatattgca gcatatttta catttgaata caaggataat gggttttatc aaaacaaaat 2580
gatgtacaga ttttttttca agtttttata gttgctttat gccagagtgg tttaccccat 2640
tcacaaaatt tcttatgcat acattgctat tgaaaataaa atttaaatat tttttcatcc 2700
tgaaaaaaaa 2710

<210> 160
<211> 610
<212> PRT
<213> Homo sapiens

<400> 160

Met His Phe Gln Ala Phe Trp Leu Cys Leu Gly Leu Leu Phe Ile Ser
1               5                   10                  15

Ile Asn Ala Glu Phe Met Asp Asp Asp Val Glu Thr Glu Asp Phe Glu
            20                  25                  30

Glu Asn Ser Glu Glu Ile Asp Val Asn Glu Ser Glu Leu Ser Ser Glu
        35                  40                  45

Ile Lys Tyr Lys Thr Pro Gln Pro Ile Gly Glu Val Tyr Phe Ala Glu
    50                  55                  60

Thr Phe Asp Ser Gly Arg Leu Ala Gly Trp Val Leu Ser Lys Ala Lys
65                  70                  75                  80

Lys Asp Asp Met Asp Glu Glu Ile Ser Ile Tyr Asp Gly Arg Trp Glu
                85                  90                  95

Ile Glu Glu Leu Lys Glu Asn Gln Val Pro Gly Asp Arg Gly Leu Val
            100                 105                 110

Leu Lys Ser Arg Ala Lys His His Ala Ile Ser Ala Val Leu Ala Lys
        115                 120                 125

Pro Phe Ile Phe Ala Asp Lys Pro Leu Ile Val Gln Tyr Glu Val Asn
    130                 135                 140

Phe Gln Asp Gly Ile Asp Cys Gly Gly Ala Tyr Ile Lys Leu Leu Ala
145                 150                 155                 160

Asp Thr Asp Asp Leu Ile Leu Glu Asn Phe Tyr Asp Lys Thr Ser Tyr
                165                 170                 175

Ile Ile Met Phe Gly Pro Asp Lys Cys Gly Glu Asp Tyr Lys Leu His
            180                 185                 190

Phe Ile Phe Arg His Lys His Pro Lys Thr Gly Val Phe Glu Glu Lys
        195                 200                 205

His Ala Lys Pro Pro Asp Val Asp Leu Lys Lys Phe Phe Thr Asp Arg
    210                 215                 220

Lys Thr His Leu Tyr Thr Leu Val Met Asn Pro Asp Asp Thr Phe Glu
225                 230                 235                 240

Val Leu Val Asp Gln Thr Val Val Asn Lys Gly Ser Leu Leu Glu Asp
                245                 250                 255

Val Val Pro Pro Ile Lys Pro Pro Lys Glu Ile Glu Asp Pro Asn Asp
            260                 265                 270

Lys Lys Pro Glu Glu Trp Asp Glu Arg Ala Lys Ile Pro Asp Pro Ser
        275                 280                 285

Ala Val Lys Pro Glu Asp Trp Asp Glu Ser Glu Pro Ala Gln Ile Glu
    290                 295                 300

Asp Ser Ser Val Val Lys Pro Ala Gly Trp Leu Asp Asp Glu Pro Lys
305                 310                 315                 320

Phe Ile Pro Asp Pro Asn Ala Glu Lys Pro Asp Asp Trp Asn Glu Asp
                325                 330                 335

Thr Asp Gly Glu Trp Glu Ala Pro Gln Ile Leu Asn Pro Ala Cys Arg
            340                 345                 350

Ile Gly Cys Gly Glu Trp Lys Pro Pro Met Ile Asp Asn Pro Lys Tyr
        355                 360                 365

Lys Gly Val Trp Arg Pro Pro Leu Val Asp Asn Pro Asn Tyr Gln Gly

370                 375                 380

Ile Trp Ser Pro Arg Lys Ile Pro Asn Pro Asp Tyr Phe Glu Asp Asp
385                 390                 395                 400

His Pro Phe Leu Leu Thr Ser Phe Ser Ala Leu Gly Leu Glu Leu Trp
                405                 410                 415

Ser Met Thr Ser Asp Ile Tyr Phe Asp Asn Phe Ile Ile Cys Ser Glu
            420                 425                 430

Lys Glu Val Ala Asp His Trp Ala Ala Asp Gly Trp Arg Trp Lys Ile
        435                 440                 445

Met Ile Ala Asn Ala Asn Lys Pro Gly Val Leu Lys Gln Leu Met Ala
    450                 455                 460

Ala Ala Glu Gly His Pro Trp Leu Trp Leu Ile Tyr Leu Val Thr Ala
465                 470                 475                 480

Gly Val Pro Ile Ala Leu Ile Thr Ser Phe Cys Trp Pro Arg Lys Val
                485                 490                 495

Lys Lys Lys His Lys Asp Thr Glu Tyr Lys Lys Thr Asp Ile Cys Ile
            500                 505                 510

Pro Gln Thr Lys Gly Val Leu Glu Gln Glu Glu Lys Glu Glu Lys Ala
        515                 520                 525

Ala Leu Glu Lys Pro Met Asp Leu Glu Glu Glu Lys Lys Gln Asn Asp
    530                 535                 540

Gly Glu Met Leu Glu Lys Glu Glu Glu Ser Glu Pro Glu Glu Lys Ser
545                 550                 555                 560

Glu Glu Glu Ile Glu Ile Ile Glu Gly Gln Glu Glu Ser Asn Gln Ser
                565                 570                 575

Asn Lys Ser Gly Ser Glu Asp Glu Met Lys Glu Ala Asp Glu Ser Thr
            580                 585                 590

Gly Ser Gly Asp Gly Pro Ile Lys Ser Val Arg Lys Arg Arg Val Arg
        595                 600                 605

Lys Asp
    610

<210>  161
<211>  2392
<212>  DNA
<213>  Homo sapiens

<400>  161
gcggagcgcg cgctcccagc gaaagcagca gggcagggat ctgcgttgga ggaagggact      60

gctctggtgc tagaatgctg tgcgtcggaa ggctgggcgg cttgggagcc agagcagcag     120

ctctgccgcc ccgccgggcg ggccggggaa gcctcgaagc cgggatccgg gcccgaaggg     180

tcagcaccag ctggtctccc gtgggcgccg ccttcaatgt caagccccag ggcagccgct     240

tggacctgtt cggcgagcgg gcgcgtcttt ttggagttcc tgagctgagt gccccagaag     300

gatttcatat tgcacaagaa aaagccttga gaaagacaga attgcttgtg gaccgtgcat     360

gttccacccc acctgggccc cagaccgtgc tgatcttcga tgagctctcg gattccttat     420

gcagagtggc cgacttggct gattttgtga aaatcgctca ccctgagcca gcattcagag     480

aagctgcgga agaagcttgt agaagtattg gcaccatggt agagaagttg aacacaaatg 540
tggatttata tcaaagtttg caaaaattac tagctgataa aaaacttgtg gattcccttg 600
atccagaaac aaggcgagtg gctgaactgt ttatgtttga ttttgaaatt agtggaatcc 660
atctagacaa acaaaagcgt aaaagagcag tggacctcaa tgttaaaatc ttggatttga 720
gtagtacatt tcttatggga accaattttc ccaacaagat tgagaagcat ctcttaccag 780
aacacattcg tcgtaacttt acatctgctg gggatcatat cataattgat ggtctccacg 840
cagaatcacc agatgacttg gtgcgagaag ctgcttataa aatttttctt tatcccaatg 900
ctggtcaatt gaaatgttta gaagaattgc tcagcagcag agatcttctg gcaaagttgg 960
tggggtattc cacgttttct cacagggctc tccaaggaac gatagctaaa aatccagaga 1020
ctgtcatgca gttccttgaa aaactatctg acaaactttc tgaaagaact ctgaaagatt 1080
ttgagatgat acgagggatg aaaatgaaac tgaatgctca aaattccgaa gtaatgccct 1140
gggacccccc ttactacagt ggtgtgattc gtgcagaaag gtataatatt gagcccagcc 1200
tatattgccc gtttttctct cttggagcat gcatggaagg cctgaatatt ttgcttaaca 1260
gactgttggg gatttcatta tatgcagagc agcctgcaaa aggagaggtg tggagcgaag 1320
atgtccgaaa actggctgtt gttcatgaat ctgaaggatt gttggggtac atttactgtg 1380
atttttttca gcgagcagac aaaccacatc aggattgcca tttcactatc cgtggaggca 1440
gactaaagga agatggagac tatcaactcc cacttgtagt tcttatgctg aatcttcccc 1500
gttcctcaag gagttctcca actttgctaa ctcctggcat gatggaaaat cttttccatg 1560
aaatgggaca tgccatgcat tcaatgctag gacgtactcg ttaccaacac gtcactggga 1620
ccaggtgccc tactgatttt gctgaggttc cttctattct gatggagtac tttgcaaatg 1680
attatcgagt agttaaccaa tttgccagac attatcagac tggacagcca ctgccaaaaa 1740
atatggtgtc tcgtctttgt gaatctaaaa aggtttgtgc tgcagctgat atgcaacttc 1800
aggtctttta tgccactctg gatcaaatct accatgggaa gcatcccctg aggaattcaa 1860
ccacagacat tctcaaggaa acacaagaga aattctatgg cctaccatat gttccaaata 1920
ctgcctggca gctgcgattc agccacctcg tggggtatgg tgctagatat tactcttacc 1980
tcatgtccag agcggtcgcc tccatggttt ggaaggagtg ttttctacag gatcctttca 2040
acagggctgc cggggagcgc tatcgcaggg agatgctggc ccacggtgga ggcagggagc 2100
ccatgctcat ggttgaaggt atgcttcaga agtgtccttc tgttgatgac ttcgtaagtg 2160
ccctcgtttc cgacttggat ctggacttcg aaactttcct catggattct gaataaaaga 2220
aacactctac acctctaatc aaggtcatgt agtaatgact ttgttataaa tgctacagct 2280
gtgagagctt gtttctgatt gtttcattgt tcgcttctgt aattctgaaa aactttaaac 2340
tggtagaact tggaataaat aatttgtttt aattaaaaaa aaaaaaaaaa aa 2392
<210> 162
<211> 713
<212> PRT

<213>  Homo sapiens

<400>  162

Met Leu Cys Val Gly Arg Leu Gly Gly Leu Gly Ala Arg Ala Ala Ala
1               5                   10                  15

Leu Pro Pro Arg Arg Ala Gly Arg Gly Ser Leu Glu Ala Gly Ile Arg
            20                  25                  30

Ala Arg Arg Val Ser Thr Ser Trp Ser Pro Val Gly Ala Ala Phe Asn
        35                  40                  45

Val Lys Pro Gln Gly Ser Arg Leu Asp Leu Phe Gly Glu Arg Ala Arg
    50                  55                  60

Leu Phe Gly Val Pro Glu Leu Ser Ala Pro Glu Gly Phe His Ile Ala
65                  70                  75                  80

Gln Glu Lys Ala Leu Arg Lys Thr Glu Leu Leu Val Asp Arg Ala Cys
                85                  90                  95

Ser Thr Pro Pro Gly Pro Gln Thr Val Leu Ile Phe Asp Glu Leu Ser
            100                 105                 110

Asp Ser Leu Cys Arg Val Ala Asp Leu Ala Asp Phe Val Lys Ile Ala
        115                 120                 125

His Pro Glu Pro Ala Phe Arg Glu Ala Ala Glu Glu Ala Cys Arg Ser
    130                 135                 140

Ile Gly Thr Met Val Glu Lys Leu Asn Thr Asn Val Asp Leu Tyr Gln
145                 150                 155                 160

Ser Leu Gln Lys Leu Leu Ala Asp Lys Lys Leu Val Asp Ser Leu Asp
                165                 170                 175

Pro Glu Thr Arg Arg Val Ala Glu Leu Phe Met Phe Asp Phe Glu Ile
            180                 185                 190

Ser Gly Ile His Leu Asp Lys Gln Lys Arg Lys Arg Ala Val Asp Leu
        195                 200                 205

Asn Val Lys Ile Leu Asp Leu Ser Ser Thr Phe Leu Met Gly Thr Asn
    210                 215                 220

Phe Pro Asn Lys Ile Glu Lys His Leu Leu Pro Glu His Ile Arg Arg
225                 230                 235                 240

Asn Phe Thr Ser Ala Gly Asp His Ile Ile Ile Asp Gly Leu His Ala
                245                 250                 255

Glu Ser Pro Asp Asp Leu Val Arg Glu Ala Ala Tyr Lys Ile Phe Leu
            260                 265                 270

Tyr Pro Asn Ala Gly Gln Leu Lys Cys Leu Glu Glu Leu Leu Ser Ser
        275                 280                 285

Arg Asp Leu Leu Ala Lys Leu Val Gly Tyr Ser Thr Phe Ser His Arg
    290                 295                 300

Ala Leu Gln Gly Thr Ile Ala Lys Asn Pro Glu Thr Val Met Gln Phe
305                 310                 315                 320

Leu Glu Lys Leu Ser Asp Lys Leu Ser Glu Arg Thr Leu Lys Asp Phe
                325                 330                 335

Glu Met Ile Arg Gly Met Lys Met Lys Leu Asn Ala Gln Asn Ser Glu

340                 345                 350

Val Met Pro Trp Asp Pro Pro Tyr Tyr Ser Gly Val Ile Arg Ala Glu
        355                 360                 365

Arg Tyr Asn Ile Glu Pro Ser Leu Tyr Cys Pro Phe Phe Ser Leu Gly
    370                 375                 380

Ala Cys Met Glu Gly Leu Asn Ile Leu Leu Asn Arg Leu Leu Gly Ile
385                 390                 395                 400

Ser Leu Tyr Ala Glu Gln Pro Ala Lys Gly Glu Val Trp Ser Glu Asp
                405                 410                 415

Val Arg Lys Leu Ala Val Val His Glu Ser Glu Gly Leu Leu Gly Tyr
            420                 425                 430

Ile Tyr Cys Asp Phe Phe Gln Arg Ala Asp Lys Pro His Gln Asp Cys
        435                 440                 445

His Phe Thr Ile Arg Gly Gly Arg Leu Lys Glu Asp Gly Asp Tyr Gln
    450                 455                 460

Leu Pro Leu Val Val Leu Met Leu Asn Leu Pro Arg Ser Ser Arg Ser
465                 470                 475                 480

Ser Pro Thr Leu Leu Thr Pro Gly Met Met Glu Asn Leu Phe His Glu
                485                 490                 495

Met Gly His Ala Met His Ser Met Leu Gly Arg Thr Arg Tyr Gln His
            500                 505                 510

Val Thr Gly Thr Arg Cys Pro Thr Asp Phe Ala Glu Val Pro Ser Ile
        515                 520                 525

Leu Met Glu Tyr Phe Ala Asn Asp Tyr Arg Val Val Asn Gln Phe Ala
    530                 535                 540

Arg His Tyr Gln Thr Gly Gln Pro Leu Pro Lys Asn Met Val Ser Arg
545                 550                 555                 560

Leu Cys Glu Ser Lys Lys Val Cys Ala Ala Ala Asp Met Gln Leu Gln
                565                 570                 575

Val Phe Tyr Ala Thr Leu Asp Gln Ile Tyr His Gly Lys His Pro Leu
            580                 585                 590

Arg Asn Ser Thr Thr Asp Ile Leu Lys Glu Thr Gln Glu Lys Phe Tyr
        595                 600                 605

Gly Leu Pro Tyr Val Pro Asn Thr Ala Trp Gln Leu Arg Phe Ser His
    610                 615                 620

Leu Val Gly Tyr Gly Ala Arg Tyr Tyr Ser Tyr Leu Met Ser Arg Ala
625                 630                 635                 640

Val Ala Ser Met Val Trp Lys Glu Cys Phe Leu Gln Asp Pro Phe Asn
                645                 650                 655

Arg Ala Ala Gly Glu Arg Tyr Arg Arg Glu Met Leu Ala His Gly Gly
            660                 665                 670

Gly Arg Glu Pro Met Leu Met Val Glu Gly Met Leu Gln Lys Cys Pro
        675                 680                 685

Ser Val Asp Asp Phe Val Ser Ala Leu Val Ser Asp Leu Asp Leu Asp
    690                 695                 700

Phe Glu Thr Phe Leu Met Asp Ser Glu
705                 710

<210>  163
<211>  3923
<212>  DNA
<213>  Homo sapiens

<400>  163
acagaagaaa tagcaagtgc cgagaagctg gcatcagaaa aacagagggg agatttgtgt      60

ggctgcagcc gagggagacc aggaagatct gcatggtggg aaggacctga tgatacagag     120

gaattacaac acatatactt agtgtttcaa tgaacaccaa gataaataag tgaagagcta     180

gtccgctgtg agtctcctca gtgacacagg gctggatcac catcgacggc actttctgag     240

tactcagtgc agcaaagaaa gactacagac atctcaatgg caggggtgag aaataagaaa     300

ggctgctgac tttaccatct gaggccacac atctgctgaa atggagataa ttaacatcac     360

tagaaacagc aagatgacaa tataatgtct aagtagtgac atgtttttgc acatttccag     420

cccctttaaa tatccacaca cacaggaagc acaaaaggaa gcacagagat ccctgggaga     480

aatgcccggc cgccatcttg ggtcatcgat gagcctcgcc ctgtgcctgg tcccgcttgt     540

gagggaagga cattagaaaa tgaattgatg tgttccttaa aggatgggca ggaaaacaga     600

tcctgttgtg gatatttatt tgaacgggat tacagatttg aaatgaagtc acaaagtgag     660

cattaccaat gagaggaaaa cagacgagaa aatcttgatg gcttcacaag acatgcaaca     720

aacaaaatgg aatactgtga tgacatgagg cagccaagct ggggaggaga taaccacggg     780

gcagagggtc aggattctgg ccctgctgcc taaactgtgc gttcataacc aaatcatttc     840

atatttctaa ccctcaaaac aaagctgttg taatatctga tctctacggt tccttctggg     900

cccaacattc tccatatatc cagccacact catttttaat atttagttcc cagatctgta     960

ctgtgacctt tctacactgt agaataacat tactcatttt gttcaaagac ccttcgtgtt    1020

gctgcctaat atgtagctga ctgtttttcc taaggagtgt tctggcccag gggatctgtg    1080

aacaggctgg gaagcatctc aagatctttc cagggttata cttactagca cacagcatga    1140

tcattacgga gtgaattatc taatcaacat catcctcagt gtctttgccc atactgaaat    1200

tcatttccca cttttgtgcc cattctcaag acctcaaaat gtcattccat taatatcaca    1260

ggattaactt ttttttttaa cctggaagaa ttcaatgtta catgcagcta tgggaattta    1320

attacatatt ttgttttcca gtgcaaagat gactaagtcc tttatccctc ccctttgttt    1380

gatttttttt ccagtataaa gttaaaatgc ttagccttgt actgaggctg tatacagcac    1440

agcctctccc catccctcca gccttatctg tcatcaccat caacccctcc cataccacct    1500

aaacaaaatc taacttgtaa ttccttgaac atgtcaggac atacattatt ccttctgcct    1560

gagaagctct tccttgtctc ttaaatctag aatgatgtaa agttttgaat aagttgacta    1620

tcttacttca tgcaaagaag ggacacatat gagattcatc atcacatgag acagcaaata    1680

ctaaaagtgt aatttgatta taagagttta gataaatata tgaaatgcaa gagccacaga    1740

gggaatgttt atggggcacg tttgtaagcc tgggatgtga agcaaaggca gggaacctca 1800
tagtatctta tataatatac ttcatttctc tatctctatc acaatatcca acaagctttt 1860
cacagaattc atgcagtgca aatccccaaa ggtaaccttt atccatttca tggtgagtgc 1920
gctttagaat tttggcaaat catactggtc acttatctca actttgagat gtgtttgtcc 1980
ttgtagttaa ttgaaagaaa tagggcactc ttgtgagcca ctttagggtt cactcctggc 2040
aataaagaat ttacaaagag ctactcagga ccagttgtta agagctctgt gtgtgtgtgt 2100
gtgtgtgtgt gagtgtacat gccaaagtgt gcctctctct cttgacccat tatttcagac 2160
ttaaaacaag catgttttca aatggcacta tgagctgcca atgatgtatc accaccatat 2220
ctcattattc tccagtaaat gtgataataa tgtcatctgt taacataaaa aaagtttgac 2280
ttcacaaaag cagctggaaa tggacaacca caatatgcat aaatctaact cctaccatca 2340
gctacacact gcttgacata tattgttaga agcacctcgc atttgtgggt tctcttaagc 2400
aaaatacttg cattaggtct cagctggggc tgtgcatcag gcggtttgag aaatattcaa 2460
ttctcagcag aagccagaat ttgaattccc tcatctttta ggaatcattt accaggtttg 2520
gagaggattc agacagctca ggtgctttca ctaatgtctc tgaacttctg tccctctttg 2580
tgttcatgga tagtccaata aataatgtta tctttgaact gatgctcata ggagagaata 2640
taagaactct gagtgatatc aacattaggg attcaaagaa atattagatt taagctcaca 2700
ctggtcaaaa ggaaccaaga tacaaagaac tctgagctgt catcgtcccc atctctgtga 2760
gccacaacca acagcaggac ccaacgcatg tctgagatcc ttaaatcaag gaaaccagtg 2820
tcatgagttg aattctccta ttatggatgc tagcttctgg ccatctctgg ctctcctctt 2880
gacacatatt agcttctagc ctttgcttcc acgactttta tcttttctcc aacacatcgc 2940
ttaccaatcc tctctctgct ctgttgcttt ggacttcccc acaagaattt caacgactct 3000
caagtctttt cttccatccc caccactaac ctgaatgcct agacccttat ttttattaat 3060
ttccaataga tgctgcctat gggctatatt gctttagatg aacattagat atttaaagct 3120
caagaggttc aaaatccaac tcattatctt ctctttcttt cacctccctg ctcctctccc 3180
tatattactg attgcactga acagcatggt ccccaatgta gccatgcaaa tgagaaaccc 3240
agtggctcct tgtggtacat gcatgcaaga ctgctgaagc cagaaggatg actgattacg 3300
cctcatgggt ggaggggacc actcctgggc cttcgtgatt gtcaggagca agacctgaga 3360
tgctccctgc cttcagtgtc ctctgcatct cccctttcta atgaagatcc atagaatttg 3420
ctacatttga gaattccaat taggaactca catgttttat ctgccctatc aattttttaa 3480
acttgctgaa aattaagttt tttcaaaatc tgtccttgta aattactttt tcttacagtg 3540
tcttggcata ctatatcaac tttgattctt tgttacaact tttcttactc ttttatcacc 3600
aaagtggctt ttattctctt tattattatt attttctttt actactatat tacgttgtta 3660
ttattttgtt ctctatagta tcaatttatt tgatttagtt tcaatttatt tttattgctg 3720
acttttaaaa taagtgattc ggggggtggg agaacagggg agggagagca ttaggacaaa 3780

tacctaatgc atgtgggact taaaacctag atgatgggtt gataggtgca gcaaaccact 3840

atggcacacg tatacctgtg taacaaacct acacattctg cacatgtatc ccagaacgta 3900

aagtaaaatt taaaaaaaag tga 3923

<210> 164
<211> 8488
<212> DNA
<213> Homo sapiens

<400> 164
   atgatgatga acgtccccgg cggaggagcg gccgcggtga tgatgacggg ctacaataat 60
   ggtcgctgtc cccggaattc tctctacagt gactgcatta ttgaggagaa gacggtggtc 120
   ctgcagaaaa aagacaatga gggctttgga ttcgtgcttc gaggggccaa agctgacaca 180
   cccattgaag aattcacacc aacaccggct ttcccagccc tacagtacct ggagtccgtg 240
   gatgaaggtg gggtggcgtg gcaagccgga ctaaggaccg gggacttctt gattgaggtt 300
   aacaatgaga atgttgtcaa agtcggccac aggcaggtgg tgaacatgat ccggcaggga 360
   gggaatcacc tggtccttaa ggtggtcacg gtgaccagga atctggaccc cgacgacacc 420
   gccaggaaga aagctccccc gcctccaaag cgggcaccga ccacagccct caccctgcgc 480
   tccaagtcca tgacctcgga gctggaggag ctcgtggata aagataaacc cgaggagata 540
   gtcccggcct ccaagccctc ccgcgctgct gagaacatgg ctgtggaacc gagggtggcg 600
   accatcaagc agcggcccag cagccggtgc ttcccggcgg gctcagacat gaactctgtg 660
   tacgaacgcc aaggaatcgc cgtgatgacg cccactgttc ctgggagccc aaaagccccg 720
   tttctgggca tccctcgagg tacgatgcga aggcagaaat caatagacag cagaatcttt 780
   ctatcaggaa taacagagga agagcggcag tttctggctc ctccaatgct gaagttcacc 840
   agaagcctgt ccatgccgga cacctctgag gacatccccc ctccaccgca gtctgtgccc 900
   ccgtccccac caccaccttc cccaaccact tacaactgcc ccaagtcccc aactccaaga 960
   gtctacggga cgattaagcc tgcgttcaat cagaattctg ccgccaaggt gtcccccgcc 1020
   accaggtccg acaccgtggc caccatgatg agggagaagg ggatgtactt caggagagag 1080
   ctggaccgct actccttgga ctctgaagac ctctacagtc ggaatgccgg cccgcaagcc 1140
   aacttccgca acaagagagg ccagatgcca gaaaacccat actcagaggt ggggaagatc 1200
   gccagcaaag ccgtctacgt ccccgccaag cccgccaggc ggaaggggat gctggtgaag 1260
   cagtccaacg tggaggacag ccccgagaag acgtgctcca tccctatccc gaccatcatc 1320
   gtgaaggagc cgtccaccag cagcagcggc aagagcagcc agggcagcag catggagatc 1380
   gacccccagg ccccggagcc accgagccag ctgcggcctg acgaaagcct gaccgtcagc 1440
   agcccctttg ccgccgccat cgccggagcc gtccgcgacc gtgagaagcg gctggaagcc 1500
   aggaggaact ccccggcctt cctctccaca gacctggggg atgaggatgt gggcctgggg 1560
   ccacccgccc ccaggacgcg gccctccatg ttccccgagg agggggattt tgctgacgag 1620
   gacagcgctg agcagctgtc atcccccatg ccgagtgcca cgcccaggga gcccgaaaac 1680
   catttcgtgg gtggcgccga ggccagtgct ccgggtgagg ctgggaggcc gctgaattcc 1740
   acgtccaaag cccaggggcc cgagagcagc ccagcagtgc cctccgcgag cagcggcaca 1800
   gccggccccg ggaattatgt ccacccactc acagggcggc tgcttgatcc cagctccccg 1860
   ctggccctgg cactctccgc aagggaccga gccatgaagg agtctcaaca gggacccaaa 1920
   ggggaggccc ccaaggccga cctcaacaaa cctctttaca ttgataccaa aatgcggccc 1980
   agcctggatg ccggcttccc tacggtcacc aggcagaaca cccggggacc cctgaggcgg 2040
   caggagacgg agaacaagta cgagaccgac ctgggccgag accggaaagg cgatgacaag 2100
   aagaacatgc tgatcgacat catggacacg tcccagcaga agtcggctgg cctgctgatg 2160
   gtgcacaccg tggacgccac taagctggac aacgccctgc aggaagagga cgagaaggca 2220
   gaggtggaga tgaagccaga cagctcgccg tccgaggtgc cagaaggtgt ttccgaaacc 2280
   gaaggtgctt tacagatctc cgctgccccc gagcccacca ccgtgcccgg cagaaccatc 2340
   gtcgcggtgg gctccatgga agaggcggtg attttgccat tccgcatccc tcctccccct 2400
   ctggcatccg tggacttgga tgaggatttt atttttacag agccattgcc tcctcccctg 2460
   gaatttgcaa atagttttga tatccccgat gaccgggcag cttctgtccc ggctctctca 2520
   gacttagtga agcagaagaa aagcgacacc cctcagtccc cttcgttgaa ctccagccaa 2580
   ccaaccaact ctgcagacag caagaagcca gccagtcttt caaactgtct gcctgcctca 2640
   ttcctgccac cccctgaaag ctttgacgcc gtcgccgact ctgggatcga ggaggtggac 2700
   agccggagta gcagcgacca ccacctcgag acgaccagca ctatctccac cgtgtctagc 2760
   atctccaccc tgtcttccga aggtggagag aatgtggaca cctgcacagt ctatgcagat 2820
   gggcaagcat ttatggttga caaaccccca gtacctccta agccaaaaat gaagcccatc 2880
   attcacaaaa gcaatgcact ttatcaagac gcgctcgtgg aagaagatgt agatagcttt 2940
   gttatccccc cgcccgctcc cccgcccccg ccgggcagtg cccagcctgg gatggccaag 3000
   gttctccagc caaggacctc caagttgtgg ggcgacgtca cagagatcaa aagcccgatt 3060
   ctctcaggcc caaaggcaaa cgttattagt gaattgaact ctatcctaca gcaaatgaac 3120
   cgagagaaat tggcaaagcc gggggaagga ctggattcac caatgggagc caagtccgcc 3180
   agcctcgctc caagaagccc ggagatcatg agcaccatct caggtacacg gagcacgacg 3240
   gtcaccttca ctgttcgccc cggcacctcc cagcccatca ccctgcagag ccggcccccc 3300
   gactatgaaa gcaggacctc aggaacaaga cgtgccccaa gccctgtggt ctcgccaaca 3360
   gagatgaaca aagagaccct gcccgccccc ctgtctgctg ccaccgcctc tccttctccc 3420
   gctctctcag atgtctttag ccttccaagc cagccccctt ctggggatct atttggcttg 3480
   aacccagcgg gacgcagtag gtcgccatcc ccctcgatac tgcaacagcc aatctcaaat 3540
   aagcctttta caactaaacc tgtccacctg tggactaaac cagatgtggc cgattggctg 3600
   gaaagtctaa acttgggtga acataaagag gccttcatgg acaatgagat cgatggcagt 3660
   cacttaccaa acctgcagaa ggaggacctc atcgatcttg gggtaactcg agtcgggcac 3720
   agaatgaaca tagaaagggc tttgaaacag ctgctggaca gataaggacg gctgctctcc 3780
   acctcgcaga ctgctcttgt tataagtaga gatgggctcg tgctgaaaca tctgaatgcc 3840
   aagcgaagtc tgtgagcatc aaccccactc catgggtttg tctcctggta cccaaagaaa 3900
   tactgagttg tgtccacaac atggctgggt cttcagaccc ctggctcacc atgtgggtgt 3960
   cttgggcagt ttctatcaca catgggacaa ggggagggag tttttctaac atggaaaaag 4020
   attcccagcc tgccgcccag catgcaggtg gcctcgcttt gccgggtccg agaggctccc 4080
   cgtcaatttt gcacgggatc ctagctcttg taggcagaca ccagtgcact ctagatacct 4140
   cctgagacct ccgtcctctg ctttccgggc agctctcacc accccaggcc ccggcatgag 4200
   gcctttcctc agtcctgtgg cctctcagag gacacctgat gctcacctgc ccctctttct 4260
   cctgcacttg gcttgcagtg agatgctccc agatgcattt gtccagtgcc ccatcatggg 4320
   cctgaaaggc agagaaactt tttcctacac agattctttt ccccatctcc tcctgtggtt 4380
   tgcatccatg gctctttggc catgaggttc ctggcagtgc tgggagtttg gatgggatcg 4440
   tgcccagctt tgcttagctt tctttatttc tgcaaatctg ttagcataat tccaaggtgg 4500
   ccaagcagat gtcacatgga gttagtcaaa gcacaaagtc acgattccac aatggagggg 4560
   agacctggcc aagggagcca gccagcgtgc aactgcccaa gctccaggtc tccaggacaa 4620
   gagcagttgt ctgccatgag cacccatcca ggatggagaa taagggcttc tctgcctctc 4680
   agaattcttt ttaattgaag atgtcttgag ctctgcaaag atcagagcag gtgagcatcc 4740
   actttgacat gaaggacaag aagacgcatg gctcatggcg ggcacatgcg gctgccagtg 4800
   agacagcgtc tcctctggga gctgggcggg cacagcatcc tcagttctgt gcccagccaa 4860
   gggtgagcat ctctgctgag acagtccttt tgctctcgga ggccagggaa gatggtactt 4920
   agaggctttt cccctatcgc tctgggtgtc taggaatccc accagcttgt cttaacagta 4980
   caacagcttc tttgaggacc cagtgggtat ggagtataga cagaacccag ggttgagaac 5040
   agaaggtggg cggcaggatc agagtgaaag cagaggcgtg aggagaggaa agcagggagg 5100
   tctcctgggc tgccaggtca gcctctctgg caaggctttc ttgagccccg cccctttctt 5160
   tccccggagt ccctccaccc cataacaata cctcgaattt ccaaaagagg tcaccagatg 5220
   cacatgggcc gcaaaacaca cagtcaggct tccagcacat tctcccccat ttggaggata 5280
   ctcgaatgtc aggtttttgg ttttattatt atttcagaac tagctcagcc catctctaat 5340
   tataaaacat ggttttgttt tttttttttc ctttttttct tgattaggtc tggaacagct 5400
   ctagaatgaa cacataaaat ttagcaattt aaaatctttc tttactgcaa gtttaaatag 5460
   ttgtacagat agtttataag cacaatattt taagaaaaaa aagtggctgg tctactaggc 5520
   agcctttgtg ccacttcagt gctagaaagt taaagaaaaa aaaacttttg tgatttaata 5580
   atactatttc tgtggaataa ttataaaagt atgacctttt taaatcaacc ttatttggat 5640
   gcatctgaac cagcagagct gtgttatatt ttctatcttt gctagaactt cgtcattgaa 5700
   ggacaatttc ttcaaagtgg ttacaattca taatgcagca gtttctccaa aaacaaaaac 5760
   aaaacacaca ccacacacac gcgcttttcc agtcacacac ccctgatgtt ggaaccaagt 5820
   ttttggacct tctgttccaa aaccttttgc aggtcaatct ttgtatttga aatgatccaa 5880
   tccaacttga agtcaattga atattaaggc gctttacttc cgtgtgcttt cagtttttcc 5940
   atcatgagat gaatgagcat tactctagat aaatttcaag acaggatact acaggtggcc 6000
   tgctgaggct gccccatatt ttagaaaatg taaaaatggt ggtttggcca ttaatttgtc 6060
   ttccatttga tgataccgca aaattccgtg agtccattcc tttggcatgg cactttccct 6120
   gggcctacag ttggtattac ctctgtgctc agtgccaggc aaaacactag ctcaaaggag 6180
   agtcaaggaa accgctggca gacgataacc agtcgaaact cgtgacttcg gtttgttgaa 6240
   ctttggcagc cagttggtga gggccagatg ttattccctt tcttaaagat actccaagcc 6300
   acatgccact aaccacaagc aagctggctg caagactaaa gagctgataa catagtttat 6360
   ttttacactg tcttattata gagaagtaat agacctatca gaacctgcac tgaccaacaa 6420
   ataaacacat gttgccaaga tgaatcggtc tctatctcta tctgcttatt ttggtactga 6480
   aagcaatagt tcctcattca aatcaccacc cactgttctc cccctttggg acatgttagg 6540
   acgaggccct attccatgcc cctctttaat ggtggaacaa atgttaaact gctcatctaa 6600
   agatcatgtt gatattattc caggttttaa gatcaacttt tgttacatac tgtaatttaa 6660
   ataaactgca tttacatgcc tagtttctgt aatattgtgt atacaaaacc caaatctctc 6720
   aaaatgtaaa ttatgtatac ctgccaagat accttttcca gggtgtctgc gcacatttta 6780
   agttaattca cataatataa aaattactca atgtgactgt tgatttgctg aactttacat 6840
   atcacaaagt gaattatttg tgatacttta gttaataaaa tggtaaattt ttttctcagt 6900
   tattgaacaa gcaagcatta tccagttgat ctggcaatga ctttttgtgt gtgggccaca 6960
   atattgattt tcccattaac aatttttttt tgttttttaa atactaatat gtttcacact 7020
   atagtttgtg taacaacacg tgttcgcatt atctatgttg ctgttacttt tgtgctttta 7080
   ttctttttag actttataaa aaaaaaaaaa agctcctgta atttgcactt tctcccaatc 7140
   cttaaatctc ttgtatggca accaaaatta ctgtaaaaaa ataaatatac tattgcacta 7200
   aggttgtggt tctgattgca aacaaacagt gaacactgtc tgaattaaac aaaaagctgc 7260
   ccgacttgca atctaatgta gattatctca ggcattgtgg ccagctctgc ctctctaaaa 7320
   ctgaccagaa aaatctctct catcgagtaa acaggctcct gtcactgagc taatctgcct 7380
   tggttccatt tccttattct caatttatca atggatacgt gcatgttatt tcagaattat 7440
   gcaaaacgtc aaaatctgct tctgtgaccg ctgctatagg cgtggagctg aggctcggct 7500
   tttccttttg ttctgggtgg aagcagcggt gccgcggagg gccagccaga tccggaccct 7560
   tcccttaggg tccagtctcc ccacacccca gcagggtgtc ttctagccat aaggccaagg 7620
   gagtggcaga actgggccgc ctctctggtt gacaagcaaa ccacatgcta aggcttggag 7680
   caagagagaa tttgtgtcta ttggcaaaga actaagccag gaagacatgg gccatccctc 7740
   cgctttaggg aagcatattt taaacctaaa cgttgaactt cttctttggc ctcaccagtg 7800
   aaaacttgtt gtctttagtt cctaaagttt cttctacttt ggcacattcc ccagttgagc 7860
   agcagcctct atgcttccac gttcaggaaa aattccagtc ctcatatctt ttgtagttca 7920
   ccctcaagct ctcccgcttc accatccaat agtttctccc aaaccttggc acccccctag 7980
   actttgcttc caatggtttc ttccagacca cttttcctag atgaatatat tcgtttacct 8040
   tactaggaaa attattggaa gattttttct tttacttgaa attggaggca ttttaataac 8100
   tggcgaactg gaatgtgttt ctgtatttgt agacaaccat gtacccatgc aagtaggtga 8160
   acattccaca gtggctgggt gaccacagca gctgcatgca gacaggactg cccgtgcttt 8220
   gtggggaatc agagaatttc caaacttgtt tctcagactt ccgcagatct catcactttg 8280
   atttctaatc catgctgtat tggtgatttt gtttatcgtt cctgtaactt gttctacatt 8340
   ccacagtctt taccgtttta tgttcaaaat tacaacaatc cctgtccatt gattccactc 8400
   tggaactctt tgttcatgcc aattttgaaa ttttaatacg agccttcaaa taaacacaga 8460
   aaagaaaaaa aaaaaaaaaa aaaaaaaa 8488

<210> 165
<211> 1254
<212> PRT
<213> Homo sapiens

<400> 165

Met Met Met Asn Val Pro Gly Gly Gly Ala Ala Ala Val Met Met Thr
1               5                   10                  15

Gly Tyr Asn Asn Gly Arg Cys Pro Arg Asn Ser Leu Tyr Ser Asp Cys
            20                  25                  30

Ile Ile Glu Glu Lys Thr Val Val Leu Gln Lys Lys Asp Asn Glu Gly
        35                  40                  45

Phe Gly Phe Val Leu Arg Gly Ala Lys Ala Asp Thr Pro Ile Glu Glu
    50                  55                  60

Phe Thr Pro Thr Pro Ala Phe Pro Ala Leu Gln Tyr Leu Glu Ser Val
65                  70                  75                  80

Asp Glu Gly Gly Val Ala Trp Gln Ala Gly Leu Arg Thr Gly Asp Phe
                85                  90                  95

Leu Ile Glu Val Asn Asn Glu Asn Val Val Lys Val Gly His Arg Gln
            100                 105                 110

Val Val Asn Met Ile Arg Gln Gly Gly Asn His Leu Val Leu Lys Val
        115                 120                 125

Val Thr Val Thr Arg Asn Leu Asp Pro Asp Asp Thr Ala Arg Lys Lys
    130                 135                 140

Ala Pro Pro Pro Pro Lys Arg Ala Pro Thr Thr Ala Leu Thr Leu Arg
145                 150                 155                 160

Ser Lys Ser Met Thr Ser Glu Leu Glu Glu Leu Val Asp Lys Asp Lys
                165                 170                 175

Pro Glu Glu Ile Val Pro Ala Ser Lys Pro Ser Arg Ala Ala Glu Asn
            180                 185                 190

Met Ala Val Glu Pro Arg Val Ala Thr Ile Lys Gln Arg Pro Ser Ser

195                 200                 205

Arg Cys Phe Pro Ala Gly Ser Asp Met Asn Ser Val Tyr Glu Arg Gln
    210                 215                 220

Gly Ile Ala Val Met Thr Pro Thr Val Pro Gly Ser Pro Lys Ala Pro
225                 230                 235                 240

Phe Leu Gly Ile Pro Arg Gly Thr Met Arg Arg Gln Lys Ser Ile Asp
                245                 250                 255

Ser Arg Ile Phe Leu Ser Gly Ile Thr Glu Glu Glu Arg Gln Phe Leu
            260                 265                 270

Ala Pro Pro Met Leu Lys Phe Thr Arg Ser Leu Ser Met Pro Asp Thr
        275                 280                 285

Ser Glu Asp Ile Pro Pro Pro Pro Gln Ser Val Pro Pro Ser Pro Pro
    290                 295                 300

Pro Pro Ser Pro Thr Thr Tyr Asn Cys Pro Lys Ser Pro Thr Pro Arg
305                 310                 315                 320

Val Tyr Gly Thr Ile Lys Pro Ala Phe Asn Gln Asn Ser Ala Ala Lys
                325                 330                 335

Val Ser Pro Ala Thr Arg Ser Asp Thr Val Ala Thr Met Met Arg Glu
            340                 345                 350

Lys Gly Met Tyr Phe Arg Arg Glu Leu Asp Arg Tyr Ser Leu Asp Ser
        355                 360                 365

Glu Asp Leu Tyr Ser Arg Asn Ala Gly Pro Gln Ala Asn Phe Arg Asn
    370                 375                 380

Lys Arg Gly Gln Met Pro Glu Asn Pro Tyr Ser Glu Val Gly Lys Ile
385                 390                 395                 400

Ala Ser Lys Ala Val Tyr Val Pro Ala Lys Pro Ala Arg Arg Lys Gly
                405                 410                 415

Met Leu Val Lys Gln Ser Asn Val Glu Asp Ser Pro Glu Lys Thr Cys
            420                 425                 430

Ser Ile Pro Ile Pro Thr Ile Ile Val Lys Glu Pro Ser Thr Ser Ser
        435                 440                 445

Ser Gly Lys Ser Ser Gln Gly Ser Ser Met Glu Ile Asp Pro Gln Ala
    450                 455                 460

Pro Glu Pro Pro Ser Gln Leu Arg Pro Asp Glu Ser Leu Thr Val Ser
465                 470                 475                 480

Ser Pro Phe Ala Ala Ala Ile Ala Gly Ala Val Arg Asp Arg Glu Lys
                485                 490                 495

Arg Leu Glu Ala Arg Arg Asn Ser Pro Ala Phe Leu Ser Thr Asp Leu
            500                 505                 510

Gly Asp Glu Asp Val Gly Leu Gly Pro Pro Ala Pro Arg Thr Arg Pro
        515                 520                 525

Ser Met Phe Pro Glu Glu Gly Asp Phe Ala Asp Glu Asp Ser Ala Glu
    530                 535                 540

Gln Leu Ser Ser Pro Met Pro Ser Ala Thr Pro Arg Glu Pro Glu Asn
545                 550                 555                 560

His Phe Val Gly Gly Ala Glu Ala Ser Ala Pro Gly Glu Ala Gly Arg
                565                 570                 575

Pro Leu Asn Ser Thr Ser Lys Ala Gln Gly Pro Glu Ser Ser Pro Ala
            580                 585                 590

Val Pro Ser Ala Ser Ser Gly Thr Ala Gly Pro Gly Asn Tyr Val His
        595                 600                 605

Pro Leu Thr Gly Arg Leu Leu Asp Pro Ser Ser Pro Leu Ala Leu Ala
    610                 615                 620

Leu Ser Ala Arg Asp Arg Ala Met Lys Glu Ser Gln Gln Gly Pro Lys
625                 630                 635                 640

Gly Glu Ala Pro Lys Ala Asp Leu Asn Lys Pro Leu Tyr Ile Asp Thr
                645                 650                 655

Lys Met Arg Pro Ser Leu Asp Ala Gly Phe Pro Thr Val Thr Arg Gln
            660                 665                 670

Asn Thr Arg Gly Pro Leu Arg Arg Gln Glu Thr Glu Asn Lys Tyr Glu
        675                 680                 685

Thr Asp Leu Gly Arg Asp Arg Lys Gly Asp Asp Lys Lys Asn Met Leu
    690                 695                 700

Ile Asp Ile Met Asp Thr Ser Gln Gln Lys Ser Ala Gly Leu Leu Met
705                 710                 715                 720

Val His Thr Val Asp Ala Thr Lys Leu Asp Asn Ala Leu Gln Glu Glu
                725                 730                 735

Asp Glu Lys Ala Glu Val Glu Met Lys Pro Asp Ser Ser Pro Ser Glu
            740                 745                 750

Val Pro Glu Gly Val Ser Glu Thr Glu Gly Ala Leu Gln Ile Ser Ala
        755                 760                 765

Ala Pro Glu Pro Thr Thr Val Pro Gly Arg Thr Ile Val Ala Val Gly
    770                 775                 780

Ser Met Glu Glu Ala Val Ile Leu Pro Phe Arg Ile Pro Pro Pro Pro
785                 790                 795                 800

Leu Ala Ser Val Asp Leu Asp Glu Asp Phe Ile Phe Thr Glu Pro Leu
                805                 810                 815

Pro Pro Pro Leu Glu Phe Ala Asn Ser Phe Asp Ile Pro Asp Asp Arg
            820                 825                 830

Ala Ala Ser Val Pro Ala Leu Ser Asp Leu Val Lys Gln Lys Lys Ser
        835                 840                 845

Asp Thr Pro Gln Ser Pro Ser Leu Asn Ser Ser Gln Pro Thr Asn Ser
    850                 855                 860

Ala Asp Ser Lys Lys Pro Ala Ser Leu Ser Asn Cys Leu Pro Ala Ser
865                 870                 875                 880

Phe Leu Pro Pro Pro Glu Ser Phe Asp Ala Val Ala Asp Ser Gly Ile
                885                 890                 895

Glu Glu Val Asp Ser Arg Ser Ser Ser Asp His His Leu Glu Thr Thr
            900                 905                 910

Ser Thr Ile Ser Thr Val Ser Ser Ile Ser Thr Leu Ser Ser Glu Gly
        915                 920                 925

Gly Glu Asn Val Asp Thr Cys Thr Val Tyr Ala Asp Gly Gln Ala Phe
    930                 935                 940

Met Val Asp Lys Pro Pro Val Pro Pro Lys Pro Lys Met Lys Pro Ile
945                 950                 955                 960

Ile His Lys Ser Asn Ala Leu Tyr Gln Asp Ala Leu Val Glu Glu Asp
                965                 970                 975

Val Asp Ser Phe Val Ile Pro Pro Pro Ala Pro Pro Pro Pro Pro Gly
            980                 985                 990

Ser Ala Gln Pro Gly Met Ala Lys  Val Leu Gln Pro Arg  Thr Ser Lys
        995                 1000                1005

Leu Trp  Gly Asp Val Thr Glu  Ile Lys Ser Pro Ile  Leu Ser Gly
    1010                 1015                 1020

Pro Lys  Ala Asn Val Ile Ser  Glu Leu Asn Ser Ile  Leu Gln Gln
    1025                 1030                 1035

Met Asn  Arg Glu Lys Leu Ala  Lys Pro Gly Glu Gly  Leu Asp Ser
    1040                 1045                 1050

Pro Met  Gly Ala Lys Ser Ala  Ser Leu Ala Pro Arg  Ser Pro Glu
    1055                 1060                 1065

Ile Met  Ser Thr Ile Ser Gly  Thr Arg Ser Thr Thr  Val Thr Phe
    1070                 1075                 1080

Thr Val  Arg Pro Gly Thr Ser  Gln Pro Ile Thr Leu  Gln Ser Arg
    1085                 1090                 1095

Pro Pro  Asp Tyr Glu Ser Arg  Thr Ser Gly Thr Arg  Arg Ala Pro
    1100                 1105                 1110

Ser Pro  Val Val Ser Pro Thr  Glu Met Asn Lys Glu  Thr Leu Pro
    1115                 1120                 1125

Ala Pro  Leu Ser Ala Ala Thr  Ala Ser Pro Ser Pro  Ala Leu Ser
    1130                 1135                 1140

Asp Val  Phe Ser Leu Pro Ser  Gln Pro Pro Ser Gly  Asp Leu Phe
    1145                 1150                 1155

Gly Leu  Asn Pro Ala Gly Arg  Ser Arg Ser Pro Ser  Pro Ser Ile
    1160                 1165                 1170

Leu Gln  Gln Pro Ile Ser Asn  Lys Pro Phe Thr Thr  Lys Pro Val
    1175                 1180                 1185

His Leu  Trp Thr Lys Pro Asp  Val Ala Asp Trp Leu  Glu Ser Leu
    1190                 1195                 1200

Asn Leu  Gly Glu His Lys Glu  Ala Phe Met Asp Asn  Glu Ile Asp
    1205                 1210                 1215

Gly Ser  His Leu Pro Asn Leu  Gln Lys Glu Asp Leu  Ile Asp Leu
    1220                 1225                 1230

Gly Val  Thr Arg Val Gly His  Arg Met Asn Ile Glu  Arg Ala Leu
    1235                 1240                 1245

Lys Gln  Leu Leu Asp Arg
    1250

<210>  166
<211>  2015
<212>  DNA
<213>  Homo sapiens

<400>  166
actgcgaagc ggcttcttca gagcacgggc tggaactggc aggcaccgcg agcccctagc      60
acccgacaag ctgagtgtgc aggacgagtc cccaccacac ccacaccaca gccgctgaat     120
gaggcttcca ggcgtccgct cgcggcccgc agagccccgc cgtgggtccg cccgctgagg     180
cgcccccagc cagtgcgctt acctgccaga ctgcgcgcca tggggcaacc cgggaacggc     240
agcgccttct tgctggcacc caatagaagc catgcgccgg accacgacgt cacgcagcaa     300
agggacgagg tgtgggtggt gggcatgggc atcgtcatgt ctctcatcgt cctggccatc     360
gtgtttggca atgtgctggt catcacagcc attgccaagt tcgagcgtct gcagacggtc     420
accaactact tcatcacttc actggcctgt gctgatctgg tcatgggcct ggcagtggtg     480
ccctttgggg ccgcccatat tcttatgaaa atgtggactt ttggcaactt ctggtgcgag     540
ttttggactt ccattgatgt gctgtgcgtc acggccagca ttgagaccct gtgcgtgatc     600
gcagtggatc gctactttgc cattacttca cctttcaagt accagagcct gctgaccaag     660
aataaggccc gggtgatcat tctgatggtg tggattgtgt caggccttac ctccttcttg     720
cccattcaga tgcactggta ccgggccacc caccaggaag ccatcaactg ctatgccaat     780
gagacctgct gtgacttctt cacgaaccaa gcctatgcca ttgcctcttc catcgtgtcc     840
ttctacgttc ccctggtgat catggtcttc gtctactcca gggtctttca ggaggccaaa     900
aggcagctcc agaagattga caaatctgag ggccgcttcc atgtccagaa ccttagccag     960
gtggagcagg atgggcggac ggggcatgga ctccgcagat cttccaagtt ctgcttgaag    1020
gagcacaaag ccctcaagac gttaggcatc atcatgggca ctttcaccct ctgctggctg    1080
cccttcttca tcgttaacat tgtgcatgtg atccaggata acctcatccg taaggaagtt    1140
tacatcctcc taaattggat aggctatgtc aattctggtt tcaatcccct tatctactgc    1200
cggagcccag atttcaggat tgccttccag gagcttctgt gcctgcgcag gtcttctttg    1260
aaggcctatg ggaatggcta ctccagcaac ggcaacacag gggagcagag tggatatcac    1320
gtggaacagg agaaagaaaa taaactgctg tgtgaagacc tcccaggcac ggaagacttt    1380
gtgggccatc aaggtactgt gcctagcgat aacattgatt cacaagggag gaattgtagt    1440
acaaatgact cactgctgta aagcagtttt tctactttta aagacccccc cccccccaac    1500
agaacactaa acagactatt taacttgagg gtaataaact tagaataaaa ttgtaaaaat    1560
tgtatagaga tatgcagaag gaagggcatc cttctgcctt ttttattttt ttaagctgta    1620
aaaagagaga aaacttattt gagtgattat ttgttatttg tacagttcag ttcctctttg    1680
catggaattt gtaagtttat gtctaaagag ctttagtcct agaggacctg agtctgctat    1740
attttcatga cttttccatg tatctacctc actattcaag tattaggggt aatatattgc    1800
tgctggtaat ttgtatctga aggagatttt ccttcctaca cccttggact tgaggatttt    1860

gagtatctcg gacctttcag ctgtgaacat ggactcttcc cccactcctc ttatttgctc 1920

acacggggta ttttaggcag ggatttgagg agcagcttca gttgttttcc cgagcaaagg 1980

tctaaagttt acagtaaata aaatgtttga ccatg 2015

<210> 167
<211> 413
<212> PRT
<213> Homo sapiens

<400> 167

Met Gly Gln Pro Gly Asn Gly Ser Ala Phe Leu Leu Ala Pro Asn Arg
1               5                   10                  15

Ser His Ala Pro Asp His Asp Val Thr Gln Gln Arg Asp Glu Val Trp
            20                  25                  30

Val Val Gly Met Gly Ile Val Met Ser Leu Ile Val Leu Ala Ile Val
        35                  40                  45

Phe Gly Asn Val Leu Val Ile Thr Ala Ile Ala Lys Phe Glu Arg Leu
    50                  55                  60

Gln Thr Val Thr Asn Tyr Phe Ile Thr Ser Leu Ala Cys Ala Asp Leu
65                  70                  75                  80

Val Met Gly Leu Ala Val Val Pro Phe Gly Ala Ala His Ile Leu Met
                85                  90                  95

Lys Met Trp Thr Phe Gly Asn Phe Trp Cys Glu Phe Trp Thr Ser Ile
            100                 105                 110

Asp Val Leu Cys Val Thr Ala Ser Ile Glu Thr Leu Cys Val Ile Ala
        115                 120                 125

Val Asp Arg Tyr Phe Ala Ile Thr Ser Pro Phe Lys Tyr Gln Ser Leu
    130                 135                 140

Leu Thr Lys Asn Lys Ala Arg Val Ile Ile Leu Met Val Trp Ile Val
145                 150                 155                 160

Ser Gly Leu Thr Ser Phe Leu Pro Ile Gln Met His Trp Tyr Arg Ala
                165                 170                 175

Thr His Gln Glu Ala Ile Asn Cys Tyr Ala Asn Glu Thr Cys Cys Asp
            180                 185                 190

Phe Phe Thr Asn Gln Ala Tyr Ala Ile Ala Ser Ser Ile Val Ser Phe
        195                 200                 205

Tyr Val Pro Leu Val Ile Met Val Phe Val Tyr Ser Arg Val Phe Gln
    210                 215                 220

Glu Ala Lys Arg Gln Leu Gln Lys Ile Asp Lys Ser Glu Gly Arg Phe
225                 230                 235                 240

His Val Gln Asn Leu Ser Gln Val Glu Gln Asp Gly Arg Thr Gly His
                245                 250                 255

Gly Leu Arg Arg Ser Ser Lys Phe Cys Leu Lys Glu His Lys Ala Leu
            260                 265                 270

Lys Thr Leu Gly Ile Ile Met Gly Thr Phe Thr Leu Cys Trp Leu Pro
        275                 280                 285

Phe Phe Ile Val Asn Ile Val His Val Ile Gln Asp Asn Leu Ile Arg
    290                 295                 300

Lys Glu Val Tyr Ile Leu Leu Asn Trp Ile Gly Tyr Val Asn Ser Gly
305                 310                 315                 320

Phe Asn Pro Leu Ile Tyr Cys Arg Ser Pro Asp Phe Arg Ile Ala Phe
                325                 330                 335

Gln Glu Leu Leu Cys Leu Arg Arg Ser Ser Leu Lys Ala Tyr Gly Asn
            340                 345                 350

Gly Tyr Ser Ser Asn Gly Asn Thr Gly Glu Gln Ser Gly Tyr His Val
        355                 360                 365

Glu Gln Glu Lys Glu Asn Lys Leu Leu Cys Glu Asp Leu Pro Gly Thr
    370                 375                 380

Glu Asp Phe Val Gly His Gln Gly Thr Val Pro Ser Asp Asn Ile Asp
385                 390                 395                 400

Ser Gln Gly Arg Asn Cys Ser Thr Asn Asp Ser Leu Leu
                405                 410

<210>  168
<211>  3741
<212>  DNA
<213>  Homo sapiens

<400>  168
gaattcgttg ttgggaagga ctggggaaac agctgtaaca tttgccaccc tcagaagctg      60

ctggtcctgt gtcacaccac cttagcctct tgatcgagga agattctcgc tgaagtctgt     120

taattctact ttttgagtac ttatgaataa ccacgtgtct tcaaaaccat ctaccatgaa     180

gctaaaacat accatcaacc ctattctttt atattttata cattttctaa tatcacttta     240

tactatttta acatacattc cgttttattt tttctccgag tcaagacaag aaaaatcaaa     300

ccgaattaaa gcaaagcctg taaattcaaa acctgattct gcatacagat ctgttaatag     360

tttggatggt ttggcttcag tattataccc tggatgtgat actttagata aagtttttac     420

atatgcaaaa aacaaattta agaacaaaag actcttggga acacgtgaag ttttaaatga     480

ggaagatgaa gtacaaccaa atggaaaaat ttttaaaaag gttattcttg gacagtataa     540

ttggctttcc tatgaagatg tctttgttcg agcctttaat tttggaaatg gattacagat     600

gttgggtcag aaaccaaaga ccaacatcgc catcttctgt gagaccaggg ccgagtggat     660

gatagctgca caggcgtgtt ttatgtataa ttttcagctt gttacattat atgccactct     720

aggaggtcca gccattgttc atgcattaaa tgaaacagag gtgaccaaca tcattactag     780

taaagaactc ttacaaacaa agttgaagga tatagtttct ttggtcccac gcctgcggca     840

catcatcact gttgatggaa agccaccgac ctggtccgac ttccccaagg gcatcattgt     900

gcataccatg gctgcagtgg aggccctggg agccaaggcc agcatggaaa accaacctca     960

tagcaaacca ttgccctcag atattgcagt aatcatgtac acaagtggat ccacaggact    1020

tccaaaggga gtcatgatct cacatagtaa cattattgct ggtataactg ggatggcaga    1080

aaggattcca gaactaggag aggaagatgt ctacattgga tatttgcctc tggcccatgt    1140

tctagaatta agtgctgagc ttgtctgtct ttctcacgga tgccgcattg gttactcttc 1200
accacagact ttagcagatc agtcttcaaa aattaaaaaa ggaagcaaag gggatacatc 1260
catgttgaaa ccaacactga tggcagcagt tccggaaatc atggatcgga tctacaaaaa 1320
tgtcatgaat aaagtcagtg aaatgagtag ttttcaacgt aatctgttta ttctggccta 1380
taattacaaa atggaacaga tttcaaaagg acgtaatact ccactgtgcg acagctttgt 1440
tttccggaaa gttcgaagct tgctaggggg aaatattcgt ctcctgttgt gtggtggcgc 1500
tccactttct gcaaccacgc agcgattcat gaacatctgt ttctgctgtc ctgttggtca 1560
gggatacggg ctcactgaat ctgctggggc tggaacaatt tccgaagtgt gggactacaa 1620
tactggcaga gtgggagcac cattagtttg ctgtgaaatc aaattaaaaa actgggagga 1680
aggtggatac tttaatactg ataagccaca ccccaggggt gaaattctta ttgggggcca 1740
aagtgtgaca atggggtact acaaaaatga agcaaaaaca aaagctgatt tctctgaaga 1800
tgaaaatgga caaaggtggc tctgtactgg ggatattgga gagtttgaac ccgatggatg 1860
cttaaagatt attgatcgta aaaaggacct tgtaaaacta caggcagggg aatatgtttc 1920
tcttgggaaa gtagaggcag ctttgaagaa tcttccacta gtagataaca tttgtgcata 1980
tgcaaacagt tatcattctt atgtcattgg atttgttgtg ccaaatcaaa aggaactaac 2040
tgaactagct cgaaagaaag gacttaaagg gacttgggag gagctgtgta acagttgtga 2100
aatggaaaat gaggtactta aagtgctttc cgaagctgct atttcagcaa gtctggaaaa 2160
gtttgaaatt ccagtaaaaa ttcgtttgag tcctgaaccg tggacccctg aaactggtct 2220
ggtgacagat gccttcaagc tgaaacgcaa agagcttaaa acacattacc aggcggacat 2280
tgagcgaatg tatggaagaa aataattatt ctcttctggc atcagtttgc tacagtgagc 2340
tcacatcaaa taggaaaata cttgaaatgc atgtctcaag ctgcaaggca aactccattc 2400
ctcatattaa actattactt ctcatgacgt caccattttt aactgacagg attagtaaaa 2460
cattaagaca gcaaacttgt gtctgtctct tctttcattt tccccgccac caacttactt 2520
taccacctat gactgtactt gtcagtatga gaatttttct gaatcatatt ggggaagcag 2580
tgattttaaa acctcaagtt tttaaacatg atttatatgt tctgtataat gttcagtttg 2640
taacttttta aaagtttgga tgtatagagg gataaatagg aaatataaga attggttatt 2700
tgggggcttt tttacttact gtatttaaaa atacaagggt attgatatga aattatgtaa 2760
atttcaaatg cttatgaatc aaatcattgt tgaacaaaag atttgttgct gtgtaattat 2820
tgtcttgtat gcatttgaga gaaataaata tacccatact tatgttttaa gaagttgaga 2880
tcttgtgaat atatgcctgt cagtgtcttc tttatatatt tattttttat tagaaaaaat 2940
gaagtttggt tggtgatgca tgaaacaaaa tagcaagaga gggttatagt ttaatagtaa 3000
gggagataac acagcatgtg tagcaccagt tgataattgg tctctagtag cttactgtca 3060
aaatgttcaa tgaagtcttc tgttcatctg ttgaaactag gaaaataccc aaacttaaat 3120
ggaagaattc tgaaagagag gatagaattt aaagaacaag agtatataaa gttattcttt 3180

gaatatttcg ttgactatat gtacattgag ttatctatat ttgtaaacaa attagtcatg 3240

gaaaattatt ctattccaaa gtctcctttt agtctagata atcattattt cattttaaaa 3300

ttagtgtttt tcatagtttg cactgatgcg tgtatggatg tgtgtgagtc agtggtagct 3360

tatttaaaaa gcaccttatc ctttctccca taacctttgt acactaaaaa atgaaagaat 3420

ttagaatgta tttgatgata gcattctcac taagacacat gagaatttaa ctttataacc 3480

gcgtgagtta agatttaatt cataggtttt gatgtcattg ttgaagttat ttgtaattca 3540

gaaaccttgc ttgtgtgata catagtaagt ctcttcattt attactgctt gcctgttgtt 3600

atatctggat tatcaaaagc aatagtgcac caattaagat gtgctcaaat caggacttaa 3660

atcataggca ccacattttt catgtcagac tagttacttt gttgattctc agttactgta 3720

ggcatcaaaa ggcaaaaatc a 3741

<210> 169
<211> 720
<212> PRT
<213> Homo sapiens

<400> 169

Met Asn Asn His Val Ser Ser Lys Pro Ser Thr Met Lys Leu Lys His
1               5                   10                  15

Thr Ile Asn Pro Ile Leu Leu Tyr Phe Ile His Phe Leu Ile Ser Leu
            20                  25                  30

Tyr Thr Ile Leu Thr Tyr Ile Pro Phe Tyr Phe Phe Ser Glu Ser Arg
        35                  40                  45

Gln Glu Lys Ser Asn Arg Ile Lys Ala Lys Pro Val Asn Ser Lys Pro
    50                  55                  60

Asp Ser Ala Tyr Arg Ser Val Asn Ser Leu Asp Gly Leu Ala Ser Val
65                  70                  75                  80

Leu Tyr Pro Gly Cys Asp Thr Leu Asp Lys Val Phe Thr Tyr Ala Lys
                85                  90                  95

Asn Lys Phe Lys Asn Lys Arg Leu Leu Gly Thr Arg Glu Val Leu Asn
            100                 105                 110

Glu Glu Asp Glu Val Gln Pro Asn Gly Lys Ile Phe Lys Lys Val Ile
        115                 120                 125

Leu Gly Gln Tyr Asn Trp Leu Ser Tyr Glu Asp Val Phe Val Arg Ala
    130                 135                 140

Phe Asn Phe Gly Asn Gly Leu Gln Met Leu Gly Gln Lys Pro Lys Thr
145                 150                 155                 160

Asn Ile Ala Ile Phe Cys Glu Thr Arg Ala Glu Trp Met Ile Ala Ala
                165                 170                 175

Gln Ala Cys Phe Met Tyr Asn Phe Gln Leu Val Thr Leu Tyr Ala Thr
            180                 185                 190

Leu Gly Gly Pro Ala Ile Val His Ala Leu Asn Glu Thr Glu Val Thr
        195                 200                 205

Asn Ile Ile Thr Ser Lys Glu Leu Leu Gln Thr Lys Leu Lys Asp Ile
    210                 215                 220

Val Ser Leu Val Pro Arg Leu Arg His Ile Ile Thr Val Asp Gly Lys
225                 230                 235                 240

Pro Pro Thr Trp Ser Asp Phe Pro Lys Gly Ile Ile Val His Thr Met
                245                 250                 255

Ala Ala Val Glu Ala Leu Gly Ala Lys Ala Ser Met Glu Asn Gln Pro
            260                 265                 270

His Ser Lys Pro Leu Pro Ser Asp Ile Ala Val Ile Met Tyr Thr Ser
        275                 280                 285

Gly Ser Thr Gly Leu Pro Lys Gly Val Met Ile Ser His Ser Asn Ile
    290                 295                 300

Ile Ala Gly Ile Thr Gly Met Ala Glu Arg Ile Pro Glu Leu Gly Glu
305                 310                 315                 320

Glu Asp Val Tyr Ile Gly Tyr Leu Pro Leu Ala His Val Leu Glu Leu
                325                 330                 335

Ser Ala Glu Leu Val Cys Leu Ser His Gly Cys Arg Ile Gly Tyr Ser
            340                 345                 350

Ser Pro Gln Thr Leu Ala Asp Gln Ser Ser Lys Ile Lys Lys Gly Ser
        355                 360                 365

Lys Gly Asp Thr Ser Met Leu Lys Pro Thr Leu Met Ala Ala Val Pro
    370                 375                 380

Glu Ile Met Asp Arg Ile Tyr Lys Asn Val Met Asn Lys Val Ser Glu
385                 390                 395                 400

Met Ser Ser Phe Gln Arg Asn Leu Phe Ile Leu Ala Tyr Asn Tyr Lys
                405                 410                 415

Met Glu Gln Ile Ser Lys Gly Arg Asn Thr Pro Leu Cys Asp Ser Phe
            420                 425                 430

Val Phe Arg Lys Val Arg Ser Leu Leu Gly Gly Asn Ile Arg Leu Leu
        435                 440                 445

Leu Cys Gly Gly Ala Pro Leu Ser Ala Thr Thr Gln Arg Phe Met Asn
    450                 455                 460

Ile Cys Phe Cys Cys Pro Val Gly Gln Gly Tyr Gly Leu Thr Glu Ser
465                 470                 475                 480

Ala Gly Ala Gly Thr Ile Ser Glu Val Trp Asp Tyr Asn Thr Gly Arg
                485                 490                 495

Val Gly Ala Pro Leu Val Cys Cys Glu Ile Lys Leu Lys Asn Trp Glu
            500                 505                 510

Glu Gly Gly Tyr Phe Asn Thr Asp Lys Pro His Pro Arg Gly Glu Ile
        515                 520                 525

Leu Ile Gly Gly Gln Ser Val Thr Met Gly Tyr Tyr Lys Asn Glu Ala
    530                 535                 540

Lys Thr Lys Ala Asp Phe Ser Glu Asp Glu Asn Gly Gln Arg Trp Leu
545                 550                 555                 560

Cys Thr Gly Asp Ile Gly Glu Phe Glu Pro Asp Gly Cys Leu Lys Ile
                565                 570                 575

Ile Asp Arg Lys Lys Asp Leu Val Lys Leu Gln Ala Gly Glu Tyr Val
            580                 585                 590

Ser Leu Gly Lys Val Glu Ala Ala Leu Lys Asn Leu Pro Leu Val Asp
        595                 600                 605

Asn Ile Cys Ala Tyr Ala Asn Ser Tyr His Ser Tyr Val Ile Gly Phe
    610                 615                 620

Val Val Pro Asn Gln Lys Glu Leu Thr Glu Leu Ala Arg Lys Lys Gly
625                 630                 635                 640

Leu Lys Gly Thr Trp Glu Glu Leu Cys Asn Ser Cys Glu Met Glu Asn
                645                 650                 655

Glu Val Leu Lys Val Leu Ser Glu Ala Ala Ile Ser Ala Ser Leu Glu
            660                 665                 670

Lys Phe Glu Ile Pro Val Lys Ile Arg Leu Ser Pro Glu Pro Trp Thr
        675                 680                 685

Pro Glu Thr Gly Leu Val Thr Asp Ala Phe Lys Leu Lys Arg Lys Glu
    690                 695                 700

Leu Lys Thr His Tyr Gln Ala Asp Ile Glu Arg Met Tyr Gly Arg Lys
705                 710                 715                 720

<210>  170
<211>  4121
<212>  DNA
<213>  Homo sapiens

<400>  170
agcaacgacg ccgggcagcg ggagcggcgg ccgcgccatg tggctgctgg ggccgctgtg      60

cctgctgctg agcagcgccg cggagagcca gctgctcccc gggaacaact tcaccaatga     120

gtgcaacata ccaggcaact tcatgtgcag caatggacgg tgcatcccgg gcgcctggca     180

gtgtgacggg ctgcctgact gcttcgacaa gagtgatgag aaggagtgcc ccaaggctaa     240

gtcgaaatgt ggcccaacct tcttcccctg tgccagcggc atccattgca tcattggtcg     300

cttccggtgc aatgggtttg aggactgtcc cgatggcagc gatgaagaga actgcacagc     360

aaaccctctg ctttgctcca ccgcccgcta ccactgcaag aacggcctct gtattgacaa     420

gagcttcatc tgcgatggac agaataactg tcaagacaac agtgatgagg aaagctgtga     480

aagttctcaa gaacccggca gtgggcaggt gtttgtgact tcagagaacc aacttgtgta     540

ttaccccagc atcacctatg ccatcatcgg cagctccgtc atttttgtgc tggtggtggc     600

cctgctggca ctggtcttgc accaccagcg gaagcggaac aacctcatga cgctgcccgt     660

gcaccggctg cagcaccctg tgctgctgtc ccgcctggtg gtcctggacc acccccacca     720

ctgcaacgtc acctacaacg tcaataatgg catccagtat gtggccagcc aggcggagca     780

gaatgcgtcg gaagtaggct ccccaccctc ctactccgag gccttgctgg accagaggcc     840

tgcgtggtat gaccttcctc caccgcccta ctcttctgac acggaatctc tgaaccaagc     900

cgacctgccc ccctaccgct cccggtccgg gagtgccaac agtgccagct cccaggcagc     960

cagcagcctc ctgagcgtgg aagacaccag ccacagcccg gggcagcctg gcccccagga    1020

gggcactgct gagcccaggg actctgagcc cagccagggc actgaagaag tataagtccc    1080

agttattcca aagtccatat gggttaatct gctctgactt gttgccattc taacaatttg 1140
tgctcatggg aagctcttta agcacctgta aggatgtctc aagttacagt ttgggatatt 1200
aactatctct gcattcccct cctcccccag acttcagaga tgtttttctg gcgtctcagt 1260
tgacatgatc tgttgtgcgt cttttctgtc aggtcactct tcccttggga cccgagatca 1320
caccctcatt tttcacatta ttctgtttct gttggagaga cagcatataa aacagtattg 1380
aaataggctg ggagagagca atgtttctgt gctatattgg atgctcagaa gtgcaggaga 1440
cgctggaccc aattctctct gctgggtagt taccttatag catttgggga tttgggttag 1500
atgatctaac caggaggcca tcactggatg gtcacccccc caaaaaaatt ccatttgagc 1560
atcaaaacct gctttgcaca atcctatttg atgcccccag ttcagcagag tcagtggcca 1620
aagaaaactt tggacgtgag taacaccctt cagcagtcgc aacgttattt tggttttgtg 1680
aaggactctg aaaccatcta ccctgtataa attctggctt tagaaatttg cccaagaatg 1740
ctcattctga gagctttcct cagcagcata tatcatcagc ctcatcctaa aataggcagg 1800
gagcccctcc catgagttta tccaagttct cagctcctaa aatgcaggct gccaagaccc 1860
tacacctgcc ctggctctac agccacttac ctggtttctg gactgtcacc ctcccagctg 1920
acctgcccgt agccaaggaa tgaggaccta acttgagttg gcccaaagtc tgacctggct 1980
gtatgtccct gtggcccaca cccagcctgt cttgctcatt catgcagcct caacactggc 2040
ctccaaagtt cccttaacac ttgcaaagtc ctttttacct gtgcatttgg acttgaggac 2100
actggtttct atcacaggtg agagccatgt tcaatacctc cagcaagctc tcctggctcc 2160
ctgcactgtg cacgctcctc ttcccaaggt cccaatacca gcacctctag ttagagttag 2220
ggtcagggtc aggcctctcc caacatccca gtagtttctc ctctgagaca catgggcaag 2280
agacaatttg gagtcaagat tttccatttg gatctatttt aaatctttta gaaatgcatt 2340
tgaaacagtg tgtttgtttt ttcccttcta gttaagggac tatttatatg tgtataggaa 2400
agctgtctct ttttttgttt ttcctttaac aaggtccaaa gaaagatgca aaaggagatc 2460
acacccttgc cccgctgagc cccgtgataa caagtcactc cagactaacc tgtgtgccag 2520
acatttgtgc attgttgcac tttgaggtta ttatttatca agttcttgaa ggaagcagaa 2580
agagggactc ctctctccct ccgtgtatag tctctatgtt tgtgctagtt tttctttttt 2640
ttctctgtgt ccagtcagcc acagggcccg cctccctgca ggaataaggg gtaaaacgtt 2700
aggtgttgtt tggcaagaaa ccacactgac tgatgagggg taaaatggaa ccaggtagag 2760
ccactccggg cagctgtcac ccattcagaa cttctttccg cagctgaaga aatgttcagt 2820
aacctgtttg acgctaatta aaacagagcc tgcaggaagt ggggctaaag tggcattcag 2880
tgatcctgtt ctgtagactt ttctttcttt ttttaaccaa atccaaagga tgttacagaa 2940
aagctagcca ctggtatttt gttttgttta aaaaaaaaaa gaaagaaaga aagaaagaaa 3000
aacggaaagg aacctagctg cctgtatctt tcatttttaa aatagcactt gagttatttt 3060
ctgagtaatc caataaagaa cttttgatga cagccagaat gtgttagaac tctggctgaa 3120

catttcatct cctgtgagtc agaagggctt tatttctccc tttgatgggg ccccttcttc 3180
tttctggtgc tctggaagtt gtttagagga aagaattcta attttaatta attgcgcagt 3240
gagttaatct cactcgcttt tctgcttcca ggcatcttag gaaaaacaaa tggttttagt 3300
agataaggga tgcctactaa tgctttttta aaacaaacag ggacattttt attatagatt 3360
tgattttttt aatgaatgtt tttaaaaata tataaatagg acaccaaagc ggcagggttt 3420
tttttggggg gagggggttt gttttccaac tcaagatggc acattagtgg ccagcaatat 3480
tttttaactc attccaacca ggaagctttt ttatacattg cctaaatcta cgccaaccag 3540
aaaatagtct catctctttt tttctcaaat gagatccgtg ttttatttta gcattaaatt 3600
agttacactg tgatgactgg cctattacct gactcagctc cctctacctt gaaattgaca 3660
tttttaaaaa atgcaactaa gtggttaata gtgtgtgacg ctcaaagtta atgtaaactg 3720
gaaaggttgt gtgtcgttgc tttttgtgtt ttggttaggc ttggttttgt tttttaattt 3780
ttatactttc taataaattt gcagtttcat tctttctgtt tgtgcaaawg gwmctamarm 3840
aammaaaaac awywttgggg gggcttgggc ctcggaaaaa gtttttaaca ccacttcggg 3900
tggggcggcg gggcccacgt aggtacggcg accacgcggg cccaaacggg accccagaag 3960
gaaaccctgg ccaagaaaaa ggtggcgaga attctccaca ccagaaaaaa acgcgccggg 4020
ggaaaccgca gagtgttgcg taaaccacac ccgaagagag aactcagaag cacacaagcg 4080
ggactcaacc aggaggaccc aagggaaccc gatagagtac g 4121

<210> 171
<211> 345
<212> PRT
<213> Homo sapiens

<400> 171

Met Trp Leu Leu Gly Pro Leu Cys Leu Leu Leu Ser Ser Ala Ala Glu
1               5                   10                  15

Ser Gln Leu Leu Pro Gly Asn Asn Phe Thr Asn Glu Cys Asn Ile Pro
            20                  25                  30

Gly Asn Phe Met Cys Ser Asn Gly Arg Cys Ile Pro Gly Ala Trp Gln
        35                  40                  45

Cys Asp Gly Leu Pro Asp Cys Phe Asp Lys Ser Asp Glu Lys Glu Cys
    50                  55                  60

Pro Lys Ala Lys Ser Lys Cys Gly Pro Thr Phe Phe Pro Cys Ala Ser
65                  70                  75                  80

Gly Ile His Cys Ile Ile Gly Arg Phe Arg Cys Asn Gly Phe Glu Asp
                85                  90                  95

Cys Pro Asp Gly Ser Asp Glu Glu Asn Cys Thr Ala Asn Pro Leu Leu
            100                 105                 110

Cys Ser Thr Ala Arg Tyr His Cys Lys Asn Gly Leu Cys Ile Asp Lys
        115                 120                 125

Ser Phe Ile Cys Asp Gly Gln Asn Asn Cys Gln Asp Asn Ser Asp Glu
    130                 135                 140

Glu Ser Cys Glu Ser Ser Gln Glu Pro Gly Ser Gly Gln Val Phe Val
145                 150                 155                 160

Thr Ser Glu Asn Gln Leu Val Tyr Tyr Pro Ser Ile Thr Tyr Ala Ile
                165                 170                 175

Ile Gly Ser Ser Val Ile Phe Val Leu Val Val Ala Leu Leu Ala Leu
            180                 185                 190

Val Leu His His Gln Arg Lys Arg Asn Asn Leu Met Thr Leu Pro Val
        195                 200                 205

His Arg Leu Gln His Pro Val Leu Leu Ser Arg Leu Val Val Leu Asp
    210                 215                 220

His Pro His His Cys Asn Val Thr Tyr Asn Val Asn Asn Gly Ile Gln
225                 230                 235                 240

Tyr Val Ala Ser Gln Ala Glu Gln Asn Ala Ser Glu Val Gly Ser Pro
                245                 250                 255

Pro Ser Tyr Ser Glu Ala Leu Leu Asp Gln Arg Pro Ala Trp Tyr Asp
            260                 265                 270

Leu Pro Pro Pro Pro Tyr Ser Ser Asp Thr Glu Ser Leu Asn Gln Ala
        275                 280                 285

Asp Leu Pro Pro Tyr Arg Ser Arg Ser Gly Ser Ala Asn Ser Ala Ser
    290                 295                 300

Ser Gln Ala Ala Ser Ser Leu Leu Ser Val Glu Asp Thr Ser His Ser
305                 310                 315                 320

Pro Gly Gln Pro Gly Pro Gln Glu Gly Thr Ala Glu Pro Arg Asp Ser
                325                 330                 335

Glu Pro Ser Gln Gly Thr Glu Glu Val
            340                 345

<210>  172
<211>  2479
<212>  DNA
<213>  Homo sapiens

<400>  172
gtcatattga acattccaga tacctatcat tactcgatgc tgttgataac agcaagatgg      60

ctttgaactc agggtcacca ccagctattg gaccttacta tgaaaaccat ggataccaac     120

cggaaaaccc ctatcccgca cagcccactg tggtccccac tgtctacgag gtgcatccgg     180

ctcagtacta cccgtccccc gtgccccagt acgccccgag ggtcctgacg caggcttcca     240

accccgtcgt ctgcacgcag cccaaatccc catccgggac agtgtgcacc tcaaagacta     300

agaaagcact gtgcatcacc ttgaccctgg ggaccttcct cgtgggagct gcgctggccg     360

ctggcctact ctggaagttc atgggcagca agtgctccaa ctctgggata gagtgcgact     420

cctcaggtac ctgcatcaac ccctctaact ggtgtgatgg cgtgtcacac tgccccggcg     480

gggaggacga gaatcggtgt gttcgcctct acggaccaaa cttcatcctt cagatgtact     540

catctcagag gaagtcctgg caccctgtgt gccaagacga ctggaacgag aactacgggc     600

gggcggcctg cagggacatg ggctataaga ataattttta ctctagccaa ggaatagtgg     660

atgacagcgg atccaccagc tttatgaaac tgaacacaag tgccggcaat gtcgatatct 720
ataaaaaact gtaccacagt gatgcctgtt cttcaaaagc agtggtttct ttacgctgtt 780
tagcctgcgg ggtcaacttg aactcaagcc gccagagcag gatcgtgggc ggtgagagcg 840
cgctcccggg ggcctggccc tggcaggtca gcctgcacgt ccagaacgtc cacgtgtgcg 900
gaggctccat catcaccccc gagtggatcg tgacagccgc ccactgcgtg gaaaaacctc 960
ttaacaatcc atggcattgg acggcatttg cggggatttt gagacaatct ttcatgttct 1020
atggagccgg ataccaagta caaaaagtga tttctcatcc aaattatgac tccaagacca 1080
agaacaatga cattgcgctg atgaagctgc agaagcctct gactttcaac gacctagtga 1140
aaccagtgtg tctgcccaac ccaggcatga tgctgcagcc agaacagctc tgctggattt 1200
ccgggtgggg ggccaccgag gagaaaggga agacctcaga agtgctgaac gctgccaagg 1260
tgcttctcat tgagacacag agatgcaaca gcagatatgt ctatgacaac ctgatcacac 1320
cagccatgat ctgtgccggc ttcctgcagg ggaacgtcga ttcttgccag ggtgacagtg 1380
gagggcctct ggtcacttcg aacaacaata tctggtggct gataggggat acaagctggg 1440
gttctggctg tgccaaagct tacagaccag gagtgtacgg gaatgtgatg gtattcacgg 1500
actggattta tcgacaaatg aaggcaaacg gctaatccac atggtcttcg tccttgacgt 1560
cgttttacaa gaaaacaatg gggctggttt tgcttccccg tgcatgattt actcttagag 1620
atgattcaga ggtcacttca tttttattaa acagtgaact tgtctggctt tggcactctc 1680
tgccatactg tgcaggctgc agtggctccc ctgcccagcc tgctctccct aaccccttgt 1740
ccgcaagggg tgatggccgg ctggttgtgg gcactggcgg tcaattgtgg aaggaagagg 1800
gttggaggct gcccccattg agatcttcct gctgagtcct ttccaggggc caattttgga 1860
tgagcatgga gctgtcactt ctcagctgct ggatgacttg agatgaaaaa ggagagacat 1920
ggaaagggag acagccaggt ggcacctgca gcggctgccc tctggggcca cttggtagtg 1980
tccccagcct acttcacaag gggattttgc tgatgggttc ttagagcctt agcagccctg 2040
gatggtggcc agaaataaag ggaccagccc ttcatgggtg gtgacgtggt agtcacttgt 2100
aaggggaaca gaaacatttt tgttcttatg gggtgagaat atagacagtg cccttggtgc 2160
gagggaagca attgaaaagg aacttgccct gagcactcct ggtgcaggtc tccacctgca 2220
cattgggtgg ggctcctggg agggagactc agccttcctc ctcatcctcc ctgaccctgc 2280
tcctagcacc ctggagagtg aatgcccctt ggtccctggc agggcgccaa gtttggcacc 2340
atgtcggcct cttcaggcct gatagtcatt ggaaattgag gtccatgggg gaaatcaagg 2400
atgctcagtt taaggtacac tgtttccatg ttatgtttct acacattgat ggtggtgacc 2460
ctgagttcaa agccatctt 2479

<210> 173
<211> 492
<212> PRT
<213> Homo sapiens

<400> 173

Met Ala Leu Asn Ser Gly Ser Pro Pro Ala Ile Gly Pro Tyr Tyr Glu
1               5                   10                  15

Asn His Gly Tyr Gln Pro Glu Asn Pro Tyr Pro Ala Gln Pro Thr Val
            20                  25                  30

Val Pro Thr Val Tyr Glu Val His Pro Ala Gln Tyr Tyr Pro Ser Pro
        35                  40                  45

Val Pro Gln Tyr Ala Pro Arg Val Leu Thr Gln Ala Ser Asn Pro Val
    50                  55                  60

Val Cys Thr Gln Pro Lys Ser Pro Ser Gly Thr Val Cys Thr Ser Lys
65                  70                  75                  80

Thr Lys Lys Ala Leu Cys Ile Thr Leu Thr Leu Gly Thr Phe Leu Val
                85                  90                  95

Gly Ala Ala Leu Ala Ala Gly Leu Leu Trp Lys Phe Met Gly Ser Lys
            100                 105                 110

Cys Ser Asn Ser Gly Ile Glu Cys Asp Ser Ser Gly Thr Cys Ile Asn
        115                 120                 125

Pro Ser Asn Trp Cys Asp Gly Val Ser His Cys Pro Gly Gly Glu Asp
    130                 135                 140

Glu Asn Arg Cys Val Arg Leu Tyr Gly Pro Asn Phe Ile Leu Gln Met
145                 150                 155                 160

Tyr Ser Ser Gln Arg Lys Ser Trp His Pro Val Cys Gln Asp Asp Trp
                165                 170                 175

Asn Glu Asn Tyr Gly Arg Ala Ala Cys Arg Asp Met Gly Tyr Lys Asn
            180                 185                 190

Asn Phe Tyr Ser Ser Gln Gly Ile Val Asp Asp Ser Gly Ser Thr Ser
        195                 200                 205

Phe Met Lys Leu Asn Thr Ser Ala Gly Asn Val Asp Ile Tyr Lys Lys
    210                 215                 220

Leu Tyr His Ser Asp Ala Cys Ser Ser Lys Ala Val Val Ser Leu Arg
225                 230                 235                 240

Cys Leu Ala Cys Gly Val Asn Leu Asn Ser Ser Arg Gln Ser Arg Ile
                245                 250                 255

Val Gly Gly Glu Ser Ala Leu Pro Gly Ala Trp Pro Trp Gln Val Ser
            260                 265                 270

Leu His Val Gln Asn Val His Val Cys Gly Gly Ser Ile Ile Thr Pro
        275                 280                 285

Glu Trp Ile Val Thr Ala Ala His Cys Val Glu Lys Pro Leu Asn Asn
    290                 295                 300

Pro Trp His Trp Thr Ala Phe Ala Gly Ile Leu Arg Gln Ser Phe Met
305                 310                 315                 320

Phe Tyr Gly Ala Gly Tyr Gln Val Gln Lys Val Ile Ser His Pro Asn
                325                 330                 335

Tyr Asp Ser Lys Thr Lys Asn Asn Asp Ile Ala Leu Met Lys Leu Gln
            340                 345                 350

Lys Pro Leu Thr Phe Asn Asp Leu Val Lys Pro Val Cys Leu Pro Asn
        355                 360                 365

Pro Gly Met Met Leu Gln Pro Glu Gln Leu Cys Trp Ile Ser Gly Trp
    370                 375                 380

Gly Ala Thr Glu Glu Lys Gly Lys Thr Ser Glu Val Leu Asn Ala Ala
385                 390                 395                 400

Lys Val Leu Leu Ile Glu Thr Gln Arg Cys Asn Ser Arg Tyr Val Tyr
                405                 410                 415

Asp Asn Leu Ile Thr Pro Ala Met Ile Cys Ala Gly Phe Leu Gln Gly
            420                 425                 430

Asn Val Asp Ser Cys Gln Gly Asp Ser Gly Gly Pro Leu Val Thr Ser
        435                 440                 445

Asn Asn Asn Ile Trp Trp Leu Ile Gly Asp Thr Ser Trp Gly Ser Gly
    450                 455                 460

Cys Ala Lys Ala Tyr Arg Pro Gly Val Tyr Gly Asn Val Met Val Phe
465                 470                 475                 480

Thr Asp Trp Ile Tyr Arg Gln Met Lys Ala Asn Gly
                485                 490

<210>  174
<211>  3104
<212>  DNA
<213>  Homo sapiens

<220>
<221>  misc_feature
<222>  (2539)..(2539)
<223>  n is a, c, g, or t

<220>
<221>  misc_feature
<222>  (2967)..(2967)
<223>  n is a, c, g, or t

<220>
<221>  misc_feature
<222>  (2979)..(2979)
<223>  n is a, c, g, or t

<220>
<221>  misc_feature
<222>  (2989)..(2990)
<223>  n is a, c, g, or t

<220>
<221>  misc_feature
<222>  (2993)..(2994)
<223>  n is a, c, g, or t

<220>
<221>  misc_feature
<222>  (3006)..(3007)
<223>  n is a, c, g, or t

<220>
<221>  misc_feature
<222>  (3015)..(3015)
<223>  n is a, c, g, or t

<220>

<221>  misc_feature
<222>  (3027)..(3029)
<223>  n is a, c, g, or t

<220>
<221>  misc_feature
<222>  (3040)..(3041)
<223>  n is a, c, g, or t

<220>
<221>  misc_feature
<222>  (3054)..(3054)
<223>  n is a, c, g, or t

<400>  174
cagagaggct gtatttcagt gcagcctgcc agacctcttc tggaggaaga ctggacaaag      60

ggggtcacac attccttcca tacggttgag cctctacctg cctggtgctg gtcacagttc     120

agcttcttca tgatggtgga tcccaatggc aatgaatcca gtgctacata cttcatccta     180

ataggcctcc ctggtttaga agaggctcag ttctggttgg ccttcccatt gtgctccctc     240

taccttattg ctgtgctagg taacttgaca atcatctaca ttgtgcggac tgagcacagc     300

ctgcatgagc ccatgtatat atttctttgc atgctttcag gcattgacat cctcatctcc     360

acctcatcca tgcccaaaat gctggccatc ttctggttca attccactac catccagttt     420

gatgcttgtc tgctacagat gtttgccatc cactccttat ctggcatgga atccacagtg     480

ctgctggcca tggcttttga ccgctatgtg gccatctgtc acccactgcg ccatgccaca     540

gtacttacgt tgcctcgtgt caccaaaatt ggtgtggctg ctgtggtgcg gggggctgca     600

ctgatggcac cccttcctgt cttcatcaag cagctgccct tctgccgctc caatatcctt     660

tcccattcct actgcctaca ccaagatgtc atgaagctgg cctgtgatga tatccgggtc     720

aatgtcgtct atggccttat cgtcatcatc tccgccattg gcctggactc acttctcatc     780

tccttctcat atctgcttat tcttaagact gtgttgggct tgacacgtga agcccaggcc     840

aaggcatttg gcacttgcgt ctctcatgtg tgtgctgtgt tcatattcta tgtacctttc     900

attggattgt ccatggtgca tcgctttagc aagcggcgtg actctccact gcccgtcatc     960

ttggccaata tctatctgct ggttcctcct gtgctcaacc caattgtcta tggagtgaag    1020

acaaaggaga ttcgacagcg catccttcga cttttccatg tggccacaca cgcttcagag    1080

ccctaggtgt cagtgatcaa acttcttttc cattcagagt cctctgattc agattttaat    1140

gttaacattt tggaagacag tattcagaaa aaaaatttcc ttaataaaaa tacaactcag    1200

atccttcaaa tatgaaactg gttggggaat ctccattttt tcaatattat tttcttcttt    1260

gttttcttgc tacatataat tattaatacc ctgactaggt tgtggttgga gggttattac    1320

ttttcatttt accatgcagt ccaaatctaa actgcttcta ctgatggttt acagcattct    1380

gagataagaa tggtacatct agagaacatt tgccaaaggc ctaagcacag caaaggaaaa    1440

taaacacaga atataataaa atgagataat ctagcttaaa actataactt cctcttcaga    1500

actcccaacc acattggatc tcagaaaaat actgtcttca aaatgacttc tacagagaag    1560

aaataatttt tcctctggac actagcactt aaggggaaga ttggaagtaa agccttgaaa    1620

agagtacatt tacctacgtt aatgaaagtt gacacactgt tctgagagtt ttcacagcat 1680
atggaccctg tttttcctat ttaattttct tatcaaccct ttaattaggc aaagatatta 1740
ttagtaccct cattgtagcc atgggaaaat tgatgttcag tggggatcag tgaattaaat 1800
ggggtcatac aagtataaaa attaaaaaaa aaagacttca tgcccaatct catatgatgt 1860
ggaagaactg ttaaagagac caacagggta gtgggttaga gatttccaga gtcttacatt 1920
ttctaragga ggtatttaat ttcttctcac tcatccagtg ttgtatttag gaatttcctg 1980
gcaacagaac tcatggcttt aatcccacta gctattgctt attgtcctgg tccaattgcc 2040
aattacctgt gtcttggaag aagtgatttc taggttcacc attatggaag attcttattc 2100
agaaagtctg catagggctt atagcaagtt atttattttt aaaagttcca taggtgtttc 2160
tgataggcag tgaggttagg gagccaccag ttatgatggg aagtatggaa tggcaggtgt 2220
tgaagataac attggccttt tgagtgtgac tcgtagctgg aaagtgaggg aatcttcagg 2280
accatgcttt atttggggct ttgtgcagta tggaacaggg actttgagac cgggaaagca 2340
atctgactta ggcatgggaa tcaggcattt ttgcttctga ggggctatta ccaagggtta 2400
ataggtttca tcttcaacag gatatgacaa cagtcttaac caagaaactc aaattacata 2460
tactaaaaca tgtgatcata tatgtggtaa gtttcatttt ctttttcaat cctcaggttc 2520
cctgatatgg attcctatna catgctttca tccccttttg taatggatat catatttgga 2580
aatgcctatt taatacttgt atttgctgct ggactgtaag cccatgaggg cactgtttat 2640
tattgaatgt catctctgtt catcattgac tgctctttgc tcatcattga atcccccagc 2700
aaagtgccta gaacataata gtgcttatgc ttgacaccgg ttatttttca tcaaacctga 2760
ttccttctgt gctgaacaca tagccaggca attttccagc cttctttgag ttgggtatta 2820
ttaaatttta gccattactt ccaatgtgag tggaagtgac atgtgcaatt tttatacctg 2880
gctcataaaa ccctcccatg tgcagccttt catgttgaca ttaaatgtga cttgggaagc 2940
tatgtgttac acagagttaa ttaaccngaa aggcctggna attttttgnn aannaaactg 3000
tggccnngag gcccncaacc ctttttnnna atttggcaan ntcccacttt gtantttggt 3060
aaggaggcca gttggataag tgaaaaataa agtactattg tgtc 3104

<210> 175
<211> 317
<212> PRT
<213> Homo sapiens

<400> 175

Met Val Asp Pro Asn Gly Asn Glu Ser Ser Ala Thr Tyr Phe Ile Leu
1               5                   10                  15

Ile Gly Leu Pro Gly Leu Glu Glu Ala Gln Phe Trp Leu Ala Phe Pro
            20                  25                  30

Leu Cys Ser Leu Tyr Leu Ile Ala Val Leu Gly Asn Leu Thr Ile Ile
        35                  40                  45

Tyr Ile Val Arg Thr Glu His Ser Leu His Glu Pro Met Tyr Ile Phe

    50                  55                  60

Leu Cys Met Leu Ser Gly Ile Asp Ile Leu Ile Ser Thr Ser Ser Met
65                  70                  75                  80

Pro Lys Met Leu Ala Ile Phe Trp Phe Asn Ser Thr Thr Ile Gln Phe
                85                  90                  95

Asp Ala Cys Leu Leu Gln Met Phe Ala Ile His Ser Leu Ser Gly Met
            100                 105                 110

Glu Ser Thr Val Leu Leu Ala Met Ala Phe Asp Arg Tyr Val Ala Ile
        115                 120                 125

Cys His Pro Leu Arg His Ala Thr Val Leu Thr Leu Pro Arg Val Thr
    130                 135                 140

Lys Ile Gly Val Ala Ala Val Val Arg Gly Ala Ala Leu Met Ala Pro
145                 150                 155                 160

Leu Pro Val Phe Ile Lys Gln Leu Pro Phe Cys Arg Ser Asn Ile Leu
                165                 170                 175

Ser His Ser Tyr Cys Leu His Gln Asp Val Met Lys Leu Ala Cys Asp
            180                 185                 190

Asp Ile Arg Val Asn Val Val Tyr Gly Leu Ile Val Ile Ile Ser Ala
        195                 200                 205

Ile Gly Leu Asp Ser Leu Leu Ile Ser Phe Ser Tyr Leu Leu Ile Leu
    210                 215                 220

Lys Thr Val Leu Gly Leu Thr Arg Glu Ala Gln Ala Lys Ala Phe Gly
225                 230                 235                 240

Thr Cys Val Ser His Val Cys Ala Val Phe Ile Phe Tyr Val Pro Phe
                245                 250                 255

Ile Gly Leu Ser Met Val His Arg Phe Ser Lys Arg Arg Asp Ser Pro
            260                 265                 270

Leu Pro Val Ile Leu Ala Asn Ile Tyr Leu Leu Val Pro Pro Val Leu
        275                 280                 285

Asn Pro Ile Val Tyr Gly Val Lys Thr Lys Glu Ile Arg Gln Arg Ile
    290                 295                 300

Leu Arg Leu Phe His Val Ala Thr His Ala Ser Glu Pro
305                 310                 315

<210> 176
<211> 2834
<212> DNA
<213> Homo sapiens

<400> 176
tcggagcctg cggagggtgg tggtggtggt ggtggtggcc ctcgcccgcc tcactcatgc 60

ctcctcctcc tctgctctcg ctcaggcgcc tcggtggcgg ttggtcggcg gttacgcggc 120

tggtggtcgc ggcggccggg gctcgctctc ggggaggccg gggcggatct cgcggcgcag 180

gcggcggcgg ccgaggtggg gtcgcgcggc ggaggcggct cgagcttcgt gctgcgcgct 240

cgctcttggg ctcctcgctg caggaggagt gtgactatgt gcagatgatc gaggtgcagc 300

acaagcagtg cctggaggag gcccagctgg agaatgagac aataggctgc agcaagatgt 360

gggacaacct cacctgctgg ccagccaccc ctcggggcca ggtagttgtc ttggcctgtc 420
ccctcatctt caagctcttc tcctccattc aaggccgcaa tgtaagccgc agctgcaccg 480
acgaaggctg gacgcacctg gagcctggcc cgtaccccat tgcctgtggt ttggatgaca 540
aggcagcgag tttggatgag cagcagacca tgttctacgg ttctgtgaag accggctaca 600
ccattggcta cggcctgtcc ctcgccaccc ttctggtcgc cacagctatc ctgagcctgt 660
tcaggaagct ccactgcacg cggaactaca tccacatgca cctcttcata tccttcatcc 720
tgagggctgc cgctgtcttc atcaaagact tggccctctt cgacagcggg gagtcggacc 780
agtgctccga gggctcggtg ggctgtaagg cagccatggt ctttttccaa tattgtgtca 840
tggctaactt cttctggctg ctggtggagg gcctctacct gtacaccctg cttgccgtct 900
ccttcttctc tgagcggaag tacttctggg ggtacatact catcggctgg ggggtaccca 960
gcacattcac catggtgtgg accatcgcca ggatccattt tgaggattat ggtctgctca 1020
ggtgctggga caccatcaac tcctcactgt ggtggatcat aaagggcccc atcctcacct 1080
ccatcttggt aaacttcatc ctgtttattt gcatcatccg aatcctgctt cagaaactgc 1140
ggcccccaga tatcaggaag agtgacagca gtccatactc aaggctagcc aggtccacac 1200
tcctgctgat ccccctgttt ggagtacact acatcatgtt cgccttcttt ccggacaatt 1260
ttaagcctga agtgaagatg gtctttgagc tcgtcgtggg gtctttccag ggttttgtgg 1320
tggctatcct ctactgcttc ctcaatggtg aggtgcaggc ggagctgagg cggaagtggc 1380
ggcgctggca cctgcagggc gtcctgggct ggaaccccaa ataccggcac ccgtcgggag 1440
gcagcaacgg cgccacgtgc agcacgcagg tttccatgct gacccgcgtc agcccaggtg 1500
cccgccgctc ctccagcttc caagccgaag tctccctggt ctgaccacca ggatcccagc 1560
ccaagcggcc cctcccgccc cttcccactc gcagcagacg ccggggacag aggcctgccc 1620
gggcgcgcca gccccggccc tgggctcgga ggctgccccc ggccccctgg tctctggtcc 1680
ggacactcct agagaacgca gccctagagc ctgcctggag cgtttctagc aagtgagaga 1740
gatgggagct cctctcctgg aggatgcagg tggaactcag tcattagact cctcctccaa 1800
aggcccccta cgccaatcaa gggcaaaaag tctacatact ttcatcctga ctctgccccc 1860
tgctggctct tctgcccaat tggaggaaag caaccggtgg atcctcaaac aacactggtg 1920
tgacctgagg gcagaaaggt tctgcccggg aaggtcacca gcaccaacac cacggtagtg 1980
cctgaaattt caccattgct gtcaagttcc tttgggttaa gcattaccac tcaggcattt 2040
gactgaagat gcagctcact accctattct ctctttacgc ttagttatca gctttttaaa 2100
gtgggttatt ctggagtttt tgtttggaga gcacacctat cttagtggtt ccccaccgaa 2160
gtggactggc ccctgggtca gtctggtggg aggacggtgc aacccaagga ctgagggact 2220
ctgaagcctc tgggaaatga gaaggcagcc accagcgaat gctaggtctc ggactaagcc 2280
tacctgctct ccaagtctca gtggcttcat ctgtcaagtg ggactctgtc acaccagcca 2340
ttcttatctc tctgtgctgt ggaagcaaca ggaatcaaga gactgccctc cttgtccacc 2400

cacctatgtg ccaactgttg taactaggct cagagatgtg cacccatggg ctctgacaga 2460

aagcagatcc tcaccctgct acacatacag gatttgaact cagatctgtc tgataggaat 2520

gtgaaagcac ggactcttac tgctaacttt tgtgtatcgt aaccagccag atcctcttgg 2580

ttatttgttt accacttgta ttattaatgc cattatccct gaattcccct tgccacccca 2640

ccctccctgg agtgtggctg aggaggcctc catctcatgt atcatctgga taggagcctg 2700

ctggtcacag cctcctctgt ctgcccttca ccccagtggc cactcagctt cctacccaca 2760

cctctgccag aagatcccct caggactgca acaggcttgt gcaacaataa atgttggctt 2820

ggaaaaaaaa aaaa 2834

<210> 177
<211> 495
<212> PRT
<213> Homo sapiens

<400> 177

Met Pro Pro Pro Pro Leu Leu Ser Leu Arg Arg Leu Gly Gly Gly Trp
1               5                   10                  15

Ser Ala Val Thr Arg Leu Val Val Ala Ala Ala Gly Ala Arg Ser Arg
            20                  25                  30

Gly Gly Arg Gly Gly Ser Arg Gly Ala Gly Gly Gly Gly Arg Gly Gly
        35                  40                  45

Val Ala Arg Arg Arg Arg Leu Glu Leu Arg Ala Ala Arg Ser Leu Leu
    50                  55                  60

Gly Ser Ser Leu Gln Glu Glu Cys Asp Tyr Val Gln Met Ile Glu Val
65                  70                  75                  80

Gln His Lys Gln Cys Leu Glu Glu Ala Gln Leu Glu Asn Glu Thr Ile
                85                  90                  95

Gly Cys Ser Lys Met Trp Asp Asn Leu Thr Cys Trp Pro Ala Thr Pro
            100                 105                 110

Arg Gly Gln Val Val Val Leu Ala Cys Pro Leu Ile Phe Lys Leu Phe
        115                 120                 125

Ser Ser Ile Gln Gly Arg Asn Val Ser Arg Ser Cys Thr Asp Glu Gly
    130                 135                 140

Trp Thr His Leu Glu Pro Gly Pro Tyr Pro Ile Ala Cys Gly Leu Asp
145                 150                 155                 160

Asp Lys Ala Ala Ser Leu Asp Glu Gln Gln Thr Met Phe Tyr Gly Ser
                165                 170                 175

Val Lys Thr Gly Tyr Thr Ile Gly Tyr Gly Leu Ser Leu Ala Thr Leu
            180                 185                 190

Leu Val Ala Thr Ala Ile Leu Ser Leu Phe Arg Lys Leu His Cys Thr
        195                 200                 205

Arg Asn Tyr Ile His Met His Leu Phe Ile Ser Phe Ile Leu Arg Ala
    210                 215                 220

Ala Ala Val Phe Ile Lys Asp Leu Ala Leu Phe Asp Ser Gly Glu Ser
225                 230                 235                 240

Asp Gln Cys Ser Glu Gly Ser Val Gly Cys Lys Ala Ala Met Val Phe
                245                 250                 255

Phe Gln Tyr Cys Val Met Ala Asn Phe Phe Trp Leu Leu Val Glu Gly
            260                 265                 270

Leu Tyr Leu Tyr Thr Leu Leu Ala Val Ser Phe Phe Ser Glu Arg Lys
        275                 280                 285

Tyr Phe Trp Gly Tyr Ile Leu Ile Gly Trp Gly Val Pro Ser Thr Phe
    290                 295                 300

Thr Met Val Trp Thr Ile Ala Arg Ile His Phe Glu Asp Tyr Gly Leu
305                 310                 315                 320

Leu Arg Cys Trp Asp Thr Ile Asn Ser Ser Leu Trp Trp Ile Ile Lys
                325                 330                 335

Gly Pro Ile Leu Thr Ser Ile Leu Val Asn Phe Ile Leu Phe Ile Cys
            340                 345                 350

Ile Ile Arg Ile Leu Leu Gln Lys Leu Arg Pro Pro Asp Ile Arg Lys
        355                 360                 365

Ser Asp Ser Ser Pro Tyr Ser Arg Leu Ala Arg Ser Thr Leu Leu Leu
    370                 375                 380

Ile Pro Leu Phe Gly Val His Tyr Ile Met Phe Ala Phe Phe Pro Asp
385                 390                 395                 400

Asn Phe Lys Pro Glu Val Lys Met Val Phe Glu Leu Val Val Gly Ser
                405                 410                 415

Phe Gln Gly Phe Val Val Ala Ile Leu Tyr Cys Phe Leu Asn Gly Glu
            420                 425                 430

Val Gln Ala Glu Leu Arg Arg Lys Trp Arg Arg Trp His Leu Gln Gly
        435                 440                 445

Val Leu Gly Trp Asn Pro Lys Tyr Arg His Pro Ser Gly Gly Ser Asn
    450                 455                 460

Gly Ala Thr Cys Ser Thr Gln Val Ser Met Leu Thr Arg Val Ser Pro
465                 470                 475                 480

Gly Ala Arg Arg Ser Ser Ser Phe Gln Ala Glu Val Ser Leu Val
                485                 490                 495

<210> 178
<211> 2070
<212> DNA
<213> Homo sapiens

<400> 178
atgagcggtg cgggggtggc ggctgggacg cggcccccca gctcgccgac cccgggctct 60

cggcgccggc gccagcgccc ctctgtgggc gtccagtcct tgaggccgca gagcccgcag 120

ctcaggcaga gcgacccgca gaaacggaac ctggacctgg agaaaagcct gcagttcctg 180

cagcagcagc actcggagat gctggccaag ctccatgagg agatcgagca tctgaagcgg 240

gaaaacaagg gtgagccggc gcggggccct aggccggccc tgcctcccca ggcacactca 300

acactgccgc tcccgcagca cagaaacaca gccatcaact ccagcacacg cctgggctca 360

gggggaacac aggacgggga gcccctccag actgtccttg cccacctggc tgcactggcc 420

cctgtatgcc aacccagtgg gtacaggttc tgggggacct ggacagatgc cgctacctct 480
agccgtggct ggacgatgtt atgcagccaa gcacagcacg tgctgctctc gggaagccca 540
gggcctgagg tcattgcagg gcggcaggtg gccacagggt gctccccaga cctccctcct 600
ccaagtagag ctgaaatggg aaggaacccc tgggacagcc cctgccctgc tagatctttg 660
cctcagattg ctgctgtggc caggcccagg atttccagcc ctatggctct gagtcctcac 720
atgctggggg cccaggggat atggacacac tccatccagg gatcccttcc tgccatctgg 780
gcagcaacca tggggacaaa gggaggaagc agagtcctgt ttccttgcca cttgtccaag 840
gcacttcccc atcctgacag cggcccccac ccagcccagg atcctgggct gtggtctcaa 900
gctcacttcc cattatcttt ggggctgggg ctgacatcag gaggacatct gactggtgga 960
tggagccagc ctgggaacat cgcagctggg gcagtgccta gggctctccc ttcccaggga 1020
gacatggaga agggggttga gggagggccc ttccctagcc gctgtggcaa ctccagtgag 1080
ctgttctggg caaagtgtgg cccaagtcgg cagccccagc cctgcagtgc tggggacgct 1140
gacaggacac gggaagaggc catgctttcc ctcgggacct gctgttccat gtgtcccaag 1200
ccctcctgct ttccagatgg cccctcagga aaccaccttt ccagggcctc tgctcccttg 1260
ggcgctcgct gggtctgcat caacggagtg tgggtagagc cgggaggacc cagccctgcc 1320
aggctgaagg agggctcctc acggacacac aggccaggag gcaagcgtgg gcgtcttgcg 1380
ggcggtagcg ccgacactgt gcgctctcct gcagacagcc tctccatgtc aagcttccag 1440
tctgtcaagt ccatctctaa ttcagccaac tctcaaggca aggccaggcc ccagcccggc 1500
tccttcaaca agcaagattc aaaagctgac gtctcccaga aggcggacct ggaagaggag 1560
cccctacttc acaacagcaa gctggacaaa gttcctgggg tacaagggca ggccagaaag 1620
gagaaagcag aggcctctaa tgcaggagct gcctgtatgg ggaacagcca gcaccagggc 1680
aggcagatgg gggcgggggc acacccccca atgatcctgc cccttcccct gcgaaagccc 1740
accacactta ggcagtgcga agtgctcatc cgcgagctgt ggaataccaa cctcctgcag 1800
acccaagagc tgcggcacct caagtccctc ctggaaggga gccagaggcc ccaggcagcc 1860
ccggaggaag ctagctttcc cagggaccaa gaagccacgc atttccccaa ggtctccacc 1920
aagagcctct ccaagaaatg cctgagccca cctgtggcgg agcgtgccat cctgcccgca 1980
ctgaagcaga ccccgaagaa caactttgcc gagaggcaga agaggctgca ggcaatgcag 2040
aaacggcgcc tgcatcgctc agtgctttga 2070

<210> 179
<211> 689
<212> PRT
<213> Homo sapiens

<400> 179

<210>  180
<211>  3461
<212>  DNA
<213>  Homo sapiens

<400>  180
ctcgtgccga attcggcacg agaccgcgtg ttcgcgcctg gtagagattt ctcgaagaca      60

ccagtgggcc cgtgtggaac caaacctgcg cgcgtggccg ggccgtggga caacgaggcc 120
gcggagacga aggcgcaatg gcgaggaagt tatctgtaat cttgatcctg acctttgccc 180
tctctgtcac aaatcccctt catgaactaa aagcagctgc tttcccccag accactgaga 240
aaattagtcc gaattgggaa tctggcatta atgttgactt ggcaatttcc acacggcaat 300
atcatctaca acagcttttc taccgctatg gagaaaataa ttctttgtca gttgaagggt 360
tcagaaaatt acttcaaaat ataggcatag ataagattaa aagaatccat atacaccatg 420
accacgacca tcactcagac cacgagcatc actcagacca tgagcgtcac tcagaccatg 480
agcatcactc agaccacgag catcactctg accatgatca tcactctcac cataatcatg 540
ctgcttctgg taaaaataag cgaaaagctc tttgcccaga ccatgactca gatagttcag 600
gtaaagatcc tagaaacagc caggggaaag gagctcaccg accagaacat gccagtggta 660
gaaggaatgt caaggacagt gttagtgcta gtgaagtgac ctcaactgtg tacaacactg 720
tctctgaagg aactcacttt ctagagacaa tagagactcc aagacctgga aaactcttcc 780
ccaaagatgt aagcagctcc actccaccca gtgtcacatc aaagagccgg gtgagccggc 840
tggctggtag gaaaacaaat gaatctgtga gtgagccccg aaaaggcttt atgtattcca 900
gaaacacaaa tgaaaatcct caggagtgtt tcaatgcatc aaagctactg acatctcatg 960
gcatgggcat ccaggttccg ctgaatgcaa cagagttcaa ctatctctgt ccagccatca 1020
tcaaccaaat tgatgctaga tcttgtctga ttcatacaag tgaaaagaag gctgaaatcc 1080
ctccaaagac ctattcatta caaatagcct gggttggtgg ttttatagcc atttccatca 1140
tcagtttcct gtctctgctg ggggttatct tagtgcctct catgaatcgg gtgtttttca 1200
aatttctcct gagtttcctt gtggcactgg ccgttgggac tttgagtggt gatgcttttt 1260
tacaccttct tccacattct catgcaagtc accaccatag tcatagccat gaagaaccag 1320
caatggaaat gaaaagagga ccacttttca gtcatctgtc ttctcaaaac atagaagaaa 1380
gtgcctattt tgattccacg tggaagggtc taacagctct aggaggcctg tatttcatgt 1440
ttcttgttga acatgtcctc acattgatca aacaatttaa agataagaag aaaaagaatc 1500
agaagaaacc tgaaaatgat gatgatgtgg agattaagaa gcagttgtcc aagtatgaat 1560
ctcaactttc aacaaatgag gagaaagtag atacagatga tcgaactgaa ggctatttac 1620
gagcagactc acaagagccc tcccactttg attctcagca gcctgcagtc ttggaagaag 1680
aagaggtcat gatagctcat gctcatccac aggaagtcta caatgaatat gtacccagag 1740
ggtgcaagaa taaatgccat tcacatttcc acgatacact cggccagtca gacgatctca 1800
ttcaccacca tcatgactac catcatattc tccatcatca ccaccaccaa aaccaccatc 1860
ctcacagtca cagccagcgc tactctcggg aggagctgaa agatgccggc gtcgccactt 1920
tggcctggat ggtgataatg ggtgatggcc tgcacaattt cagcgatggc ctagcaattg 1980
gtgctgcttt tactgaaggc ttatcaagtg gtttaagtac ttctgttgct gtgttctgtc 2040
atgagttgcc tcatgaatta ggtgactttg ctgttctact aaaggctggc atgaccgtta 2100

agcaggctgt cctttataat gcattgtcag ccatgctggc gtatcttgga atggcaacag 2160
gaattttcat tggtcattat gctgaaaatg tttctatgtg gatatttgca cttactgctg 2220
gcttattcat gtatgttgct ctggttgata tggtacctga aatgctgcac aatgatgcta 2280
gtgaccatgg atgtagccgc tgggggtatt tctttttaca gaatgctggg atgcttttgg 2340
gttttggaat tatgttactt atttccatat ttgaacataa aatcgtgttt cgtataaatt 2400
tctagttaag gtttaaatgc tagagtagct taaaaagttg tcatagtttc agtaggtcat 2460
agggagatga gtttgtatgc tgtactatgc agcgtttaaa gttagtgggt tttgtgattt 2520
ttgtattgaa tattgctgtc tgttacaaag tcagttaaag gtacgtttta atatttaagt 2580
tattctatct tggagataaa atctgtatgt gcaattcacc ggtattacca gtttattatg 2640
taaacaagag atttggcatg acatgttctg tatgtttcag ggaaaaatgt ctttaatgct 2700
ttttcaagaa ctaacacagt tattcctata ctggatttta ggtctctgaa gaactgctgg 2760
tgtttaggaa taagaatgtg catgaagcct aaaataccaa gaaagcttat actgaattta 2820
agcaaagaaa taaaggagaa aagagaagaa tctgagaatt ggggaggcat agattcttat 2880
aaaaatcaca aaatttgttg taaattagag gggagaaatt tagaattaag tataaaaagg 2940
cagaattagt atagagtaca ttcattaaac atttttgtca ggattatttc ccgtaaaaac 3000
gtagtgagca ctctcatata ctaattagtg tacatttaac tttgtataat acagaaatct 3060
aaatatattt aatgaattca agcaatatac acttgaccaa gaaattggaa tttcaaaatg 3120
ttcgtgcggg ttatatacca gatgagtaca gtgagtagtt tatgtatcac cagactgggt 3180
tattgccaag ttatatatca ccaaaagctg tatgactgga tgttctggtt acctggttta 3240
caaaattatc agagtagtaa aactttgata tatatgagga tattaaaact acactaagta 3300
tcatttgatt cgattcagaa agtactttga tatctctcag tgcttcagtg ctatcattgt 3360
gagcaattgt ctttatatac ggtactgtag ccatactagg cctgtctgtg gcattctcta 3420
gatgtttctt ttttacacaa taaattcctt atatcagctt g 3461

<210> 181
<211> 755
<212> PRT
<213> Homo sapiens

<400> 181

Met Ala Arg Lys Leu Ser Val Ile Leu Ile Leu Thr Phe Ala Leu Ser
1               5                   10                  15

Val Thr Asn Pro Leu His Glu Leu Lys Ala Ala Ala Phe Pro Gln Thr
            20                  25                  30

Thr Glu Lys Ile Ser Pro Asn Trp Glu Ser Gly Ile Asn Val Asp Leu
        35                  40                  45

Ala Ile Ser Thr Arg Gln Tyr His Leu Gln Gln Leu Phe Tyr Arg Tyr
    50                  55                  60

Gly Glu Asn Asn Ser Leu Ser Val Glu Gly Phe Arg Lys Leu Leu Gln
65                  70                  75                  80

Asn Ile Gly Ile Asp Lys Ile Lys Arg Ile His Ile His His Asp His
                85                  90                  95

Asp His His Ser Asp His Glu His His Ser Asp His Glu Arg His Ser
            100                 105                 110

Asp His Glu His His Ser Asp His Glu His His Ser Asp His Asp His
        115                 120                 125

His Ser His His Asn His Ala Ala Ser Gly Lys Asn Lys Arg Lys Ala
    130                 135                 140

Leu Cys Pro Asp His Asp Ser Asp Ser Ser Gly Lys Asp Pro Arg Asn
145                 150                 155                 160

Ser Gln Gly Lys Gly Ala His Arg Pro Glu His Ala Ser Gly Arg Arg
                165                 170                 175

Asn Val Lys Asp Ser Val Ser Ala Ser Glu Val Thr Ser Thr Val Tyr
            180                 185                 190

Asn Thr Val Ser Glu Gly Thr His Phe Leu Glu Thr Ile Glu Thr Pro
        195                 200                 205

Arg Pro Gly Lys Leu Phe Pro Lys Asp Val Ser Ser Ser Thr Pro Pro
    210                 215                 220

Ser Val Thr Ser Lys Ser Arg Val Ser Arg Leu Ala Gly Arg Lys Thr
225                 230                 235                 240

Asn Glu Ser Val Ser Glu Pro Arg Lys Gly Phe Met Tyr Ser Arg Asn
                245                 250                 255

Thr Asn Glu Asn Pro Gln Glu Cys Phe Asn Ala Ser Lys Leu Leu Thr
            260                 265                 270

Ser His Gly Met Gly Ile Gln Val Pro Leu Asn Ala Thr Glu Phe Asn
        275                 280                 285

Tyr Leu Cys Pro Ala Ile Ile Asn Gln Ile Asp Ala Arg Ser Cys Leu
    290                 295                 300

Ile His Thr Ser Glu Lys Lys Ala Glu Ile Pro Pro Lys Thr Tyr Ser
305                 310                 315                 320

Leu Gln Ile Ala Trp Val Gly Gly Phe Ile Ala Ile Ser Ile Ile Ser
                325                 330                 335

Phe Leu Ser Leu Leu Gly Val Ile Leu Val Pro Leu Met Asn Arg Val
            340                 345                 350

Phe Phe Lys Phe Leu Leu Ser Phe Leu Val Ala Leu Ala Val Gly Thr
        355                 360                 365

Leu Ser Gly Asp Ala Phe Leu His Leu Leu Pro His Ser His Ala Ser
    370                 375                 380

His His His Ser His Ser His Glu Glu Pro Ala Met Glu Met Lys Arg
385                 390                 395                 400

Gly Pro Leu Phe Ser His Leu Ser Ser Gln Asn Ile Glu Glu Ser Ala
                405                 410                 415

Tyr Phe Asp Ser Thr Trp Lys Gly Leu Thr Ala Leu Gly Gly Leu Tyr
            420                 425                 430

Phe Met Phe Leu Val Glu His Val Leu Thr Leu Ile Lys Gln Phe Lys
        435                 440                 445

Asp Lys Lys Lys Lys Asn Gln Lys Lys Pro Glu Asn Asp Asp Asp Val
    450                 455                 460

Glu Ile Lys Lys Gln Leu Ser Lys Tyr Glu Ser Gln Leu Ser Thr Asn
465                 470                 475                 480

Glu Glu Lys Val Asp Thr Asp Asp Arg Thr Glu Gly Tyr Leu Arg Ala
                485                 490                 495

Asp Ser Gln Glu Pro Ser His Phe Asp Ser Gln Gln Pro Ala Val Leu
            500                 505                 510

Glu Glu Glu Glu Val Met Ile Ala His Ala His Pro Gln Glu Val Tyr
        515                 520                 525

Asn Glu Tyr Val Pro Arg Gly Cys Lys Asn Lys Cys His Ser His Phe
    530                 535                 540

His Asp Thr Leu Gly Gln Ser Asp Asp Leu Ile His His His His Asp
545                 550                 555                 560

Tyr His His Ile Leu His His His His His Gln Asn His His Pro His
                565                 570                 575

Ser His Ser Gln Arg Tyr Ser Arg Glu Glu Leu Lys Asp Ala Gly Val
            580                 585                 590

Ala Thr Leu Ala Trp Met Val Ile Met Gly Asp Gly Leu His Asn Phe
        595                 600                 605

Ser Asp Gly Leu Ala Ile Gly Ala Ala Phe Thr Glu Gly Leu Ser Ser
    610                 615                 620

Gly Leu Ser Thr Ser Val Ala Val Phe Cys His Glu Leu Pro His Glu
625                 630                 635                 640

Leu Gly Asp Phe Ala Val Leu Leu Lys Ala Gly Met Thr Val Lys Gln
                645                 650                 655

Ala Val Leu Tyr Asn Ala Leu Ser Ala Met Leu Ala Tyr Leu Gly Met
            660                 665                 670

Ala Thr Gly Ile Phe Ile Gly His Tyr Ala Glu Asn Val Ser Met Trp
        675                 680                 685

Ile Phe Ala Leu Thr Ala Gly Leu Phe Met Tyr Val Ala Leu Val Asp
    690                 695                 700

Met Val Pro Glu Met Leu His Asn Asp Ala Ser Asp His Gly Cys Ser
705                 710                 715                 720

Arg Trp Gly Tyr Phe Phe Leu Gln Asn Ala Gly Met Leu Leu Gly Phe
                725                 730                 735

Gly Ile Met Leu Leu Ile Ser Ile Phe Glu His Lys Ile Val Phe Arg
            740                 745                 750

Ile Asn Phe
        755

<210>  182
<211>  2032
<212>  DNA
<213>  Homo sapiens

<400>  182
cgcggggcgc ggagtcggcg gggcctcgcg ggacgcgggc agtgcggaga ccgcggcgct      60

gaggacgcgg gagccgggag cgcacgcgcg gggtggagtt cagcctactc tttcttagat 120
gtgaaaggaa aggaagatca tttcatgcct tgttgataaa ggttcagact tctgctgatt 180
cataaccatt tggctctgag ctatgacaag agaggaaaca aaaagttaaa cttacaagcc 240
tgccataagt gagaagcaaa cttccttgat aacatgcttt tgcgaagtgc aggaaaatta 300
aatgtgggca ccaagaaaga ggatggtgag agtacagccc ccaccccccg tccaaaggtc 360
ttgcgttgta aatgccacca ccattgtcca gaagactcag tcaacaatat ttgcagcaca 420
gacggatatt gtttcacgat gatagaagag gatgactctg ggttgcctgt ggtcacttct 480
ggttgcctag gactagaagg ctcagatttt cagtgtcggg acactcccat tcctcatcaa 540
agaagatcaa ttgaatgctg cacagaaagg aacgaatgta ataaagacct acaccctaca 600
ctgcctccat tgaaaaacag agattttgtt gatggaccta tacaccacag ggctttactt 660
atatctgtga ctgtctgtag tttgctcttg gtccttatca tattattttg ttacttccgg 720
tataaaagac aagaaaccag acctcgatac agcattgggt tagaacagga tgaaacttac 780
attcctcctg gagaatccct gagagactta attgagcagt ctcagagctc aggaagtgga 840
tcaggcctcc ctctgctggt ccaaaggact atagctaagc agattcagat ggtgaaacag 900
attggaaaag gtcgctatgg ggaagtttgg atgggaaagt ggcgtggcga aaaggtagct 960
gtgaaagtgt tcttcaccac agaggaagcc agctggttca gagagacaga aatatatcag 1020
acagtgttga tgaggcatga aaacattttg ggtttcattg ctgcagatat caaagggaca 1080
gggtcctgga cccagttgta cctaatcaca gactatcatg aaaatggttc cctttatgat 1140
tatctgaagt ccaccaccct agacgctaaa tcaatgctga agttagccta ctcttctgtc 1200
agtggcttat gtcatttaca cacagaaatc tttagtactc aaggcaaacc agcaattgcc 1260
catcgagatc tgaaaagtaa aaacattctg gtgaagaaaa atggaacttg ctgtattgct 1320
gacctgggcc tggctgttaa atttattagt gatacaaatg aagttgacat accacctaac 1380
actcgagttg gcaccaaacg ctatatgcct ccagaagtgt tggacgagag cttgaacaga 1440
aatcacttcc agtcttacat catggctgac atgtatagtt ttggcctcat cctttgggag 1500
gttgctagga gatgtgtatc aggaggtata gtggaagaat accagcttcc ttatcatgac 1560
ctagtgccca gtgacccctc ttatgaggac atgagggaga ttgtgtgcat caagaagtta 1620
cgcccctcat tcccaaaccg gtggagcagt gatgagtgtc taaggcagat gggaaaactc 1680
atgacagaat gctgggctca caatcctgca tcaaggctga cagccctgcg ggttaagaaa 1740
acacttgcca aaatgtcaga gtcccaggac attaaactct gataggagag gaaaagtaag 1800
catctctgca gaaagccaac aggtactctt ctgtttgtgg gcagagcaaa agacatcaaa 1860
taagcatcca cagtacaagc cttgaacatc gtcctgcttc ccagtgggtt cagacctcac 1920
ctttcaggga gcgacctggg caaagacaga gaagctccca gaaggagaga ttgatccgtg 1980
tctgtttgta ggcggagaaa ccgttgggta acttgttcaa gatatgatgc at 2032
<210> 183

<211>  502
<212>  PRT
<213>  Homo sapiens

<400>  183

<210>  184
<211>  3375
<212>  DNA
<213>  Homo sapiens

<400>  184
gacagtgttc gcggctgcac cgctcggagg ctgggtgacc cgcgtagaag tgaagtactt      60

ttttatttgc agacctgggc cgatgccgct ttaaaaaacg cgaggggctc tatgcacctc     120

cctggcggta gttcctccga cctcagccgg gtcgggtcgt gccgccctct cccaggagag     180

acaaacaggt gtcccacgtg gcagccgcgc cccgggcgcc cctcctgtga tcccgtagcg     240

ccccctggcc cgagccgcgc ccgggtctgt gagtagagcc gcccgggcac cgagcgctgg     300

tcgccgctct ccttccgtta tatcaacatg ccccctttcc tgttgctgga ggccgtctgt     360

gttttcctgt tttccagagt gcccccatct ctccctctcc aggaagtcca tgtaagcaaa     420

gaaaccatcg ggaagatttc agctgccagc aaaatgatgt ggtgctcggc tgcagtggac     480

atcatgtttc tgttagatgg gtctaacagc gtcgggaaag ggagctttga aaggtccaag     540

cactttgcca tcacagtctg tgacggtctg gacatcagcc ccgagagggt cagagtggga     600

gcattccagt tcagttccac tcctcatctg gaattcccct tggattcatt ttcaacccaa     660

caggaagtga aggcaagaat caagaggatg gttttcaaag gagggcgcac ggagacggaa     720

cttgctctga aataccttct gcacagaggg ttgcctggag gcagaaatgc ttctgtgccc     780

cagatcctca tcatcgtcac tgatgggaag tcccaggggg atgtggcact gccatccaag     840

cagctgaagg aaaggggtgt cactgtgttt gctgtggggg tcaggtttcc caggtgggag 900
gagctgcatg cactggccag cgagcctaga gggcagcacg tgctgttggc tgagcaggtg 960
gaggatgcca ccaacggcct cttcagcacc ctcagcagct cggccatctg ctccagcgcc 1020
acgccagact gcagggtcga ggctcacccc tgtgagcaca ggacgctgga gatggtccgg 1080
gagttcgctg gcaatgcccc atgctggaga ggatcgcggc ggacccttgc ggtgctggct 1140
gcacactgtc ccttctacag ctggaagaga gtgttcctaa cccaccctgc cacctgctac 1200
aggaccacct gcccaggccc ctgtgactcg cagccctgcc agaatggagg cacatgtgtt 1260
ccagaaggac tggacggcta ccagtgcctc tgcccgctgg cctttggagg ggaggctaac 1320
tgtgccctga agctgagcct ggaatgcagg gtcgacctcc tcttcctgct ggacagctct 1380
gcgggcacca ctctggacgg cttcctgcgg gccaaagtct tcgtgaagcg gtttgtgcgg 1440
gccgtgctga gcgaggactc tcgggcccga gtgggtgtgg ccacatacag cagggagctg 1500
ctggtggcgg tgcctgtggg ggagtaccag gatgtgcctg acctggtctg gagcctcgat 1560
ggcattccct tccgtggtgg ccccaccctg acgggcagtg ccttgcggca ggcggcagag 1620
cgtggcttcg ggagcgccac caggacaggc caggaccggc cacgtagagt ggtggttttg 1680
ctcactgagt cacactccga ggatgaggtt gcgggcccag cgcgtcacgc aagggcgcga 1740
gagctgctcc tgctgggtgt aggcagtgag gccgtgcggg cagagctgga ggagatcaca 1800
ggcagcccaa agcatgtgat ggtctactcg gatcctcagg atctgttcaa ccaaatccct 1860
gagctgcagg ggaagctgtg cagccggcag cggccagggt gccggacaca agccctggac 1920
ctcgtcttca tgttggacac ctctgcctca gtagggcccg agaattttgc tcagatgcag 1980
agctttgtga gaagctgtgc cctccagttt gaggtgaacc ctgacgtgac acaggtcggc 2040
ctggtggtgt atggcagcca ggtgcagact gccttcgggc tggacaccaa acccacccgg 2100
gctgcgatgc tgcgggccat tagccaggcc ccctacctag gtggggtggg ctcagccggc 2160
accgccctgc tgcacatcta tgacaaagtg atgaccgtcc agaggggtgc ccggcctggt 2220
gtccccaaag ctgtggtggt gctcacaggc gggagaggcg cagaggatgc agccgttcct 2280
gcccagaagc tgaggaacaa tggcatctct gtcttggtcg tgggcgtggg gcctgtccta 2340
agtgagggtc tgcggaggct tgcaggtccc cgggattccc tgatccacgt ggcagcttac 2400
gccgacctgc ggtaccacca ggacgtgctc attgagtggc tgtgtggaga agccaagcag 2460
ccagtcaacc tctgcaaacc cagcccgtgc atgaatgagg gcagctgcgt cctgcagaat 2520
gggagctacc gctgcaagtg tcgggatggc tgggagggcc cccactgcga gaaccgtgag 2580
tggagctctt gctctgtatg tgtgagccag ggatggattc ttgagacgcc cctgaggcac 2640
atggctcccg tgcaggaggg cagcagccgt acccctccca gcaactacag agaaggcctg 2700
ggcactgaaa tggtgcctac cttctggaat gtctgtgccc caggtcctta gaatgtctgc 2760
ttcccgccgt ggccaggacc actattctca ctgagggagg aggatgtccc aactgcagcc 2820
atgctgctta gagacaagaa agcagctgat gtcacccaca aacgatgttg ttgaaaagtt 2880

ttgatgtgta agtaaatacc cactttctgt acctgctgtg ccttgttgag gctatgtcat 2940

ctgccacctt tcccttgagg ataaacaagg ggtcctgaag acttaaattt agcggcctga 3000

cgttcctttg cacacaatca atgctcgcca gaatgttgtt gacacagtaa tgcccagcag 3060

aggcctttac tagagcatcc tttggacggc gaaggccacg gcctttcaag atggaaagca 3120

gcagcttttc cacttcccca gagacattct ggatgcattt gcattgagtc tgaaaggggg 3180

cttgagggac gtttgtgact tcttggcgac tgccttttgt gtgtggaaga gacttggaaa 3240

ggtctcagac tgaatgtgac caattaacca gcttggttga tgatggggga ggggctgagt 3300

tgtgcatggg cccaggtctg gagggccacg taaaatcgtt ctgagtcgtg agcagtgtcc 3360

accttgaagg tcttc 3375

<210> 185
<211> 807
<212> PRT
<213> Homo sapiens

<400> 185

Met Pro Pro Phe Leu Leu Leu Glu Ala Val Cys Val Phe Leu Phe Ser
1               5                   10                  15

Arg Val Pro Pro Ser Leu Pro Leu Gln Glu Val His Val Ser Lys Glu
            20                  25                  30

Thr Ile Gly Lys Ile Ser Ala Ala Ser Lys Met Met Trp Cys Ser Ala
        35                  40                  45

Ala Val Asp Ile Met Phe Leu Leu Asp Gly Ser Asn Ser Val Gly Lys
    50                  55                  60

Gly Ser Phe Glu Arg Ser Lys His Phe Ala Ile Thr Val Cys Asp Gly
65                  70                  75                  80

Leu Asp Ile Ser Pro Glu Arg Val Arg Val Gly Ala Phe Gln Phe Ser
                85                  90                  95

Ser Thr Pro His Leu Glu Phe Pro Leu Asp Ser Phe Ser Thr Gln Gln
            100                 105                 110

Glu Val Lys Ala Arg Ile Lys Arg Met Val Phe Lys Gly Gly Arg Thr
        115                 120                 125

Glu Thr Glu Leu Ala Leu Lys Tyr Leu Leu His Arg Gly Leu Pro Gly
    130                 135                 140

Gly Arg Asn Ala Ser Val Pro Gln Ile Leu Ile Ile Val Thr Asp Gly
145                 150                 155                 160

Lys Ser Gln Gly Asp Val Ala Leu Pro Ser Lys Gln Leu Lys Glu Arg
                165                 170                 175

Gly Val Thr Val Phe Ala Val Gly Val Arg Phe Pro Arg Trp Glu Glu
            180                 185                 190

Leu His Ala Leu Ala Ser Glu Pro Arg Gly Gln His Val Leu Leu Ala
        195                 200                 205

Glu Gln Val Glu Asp Ala Thr Asn Gly Leu Phe Ser Thr Leu Ser Ser
    210                 215                 220

Ser Ala Ile Cys Ser Ser Ala Thr Pro Asp Cys Arg Val Glu Ala His

225                 230                 235                 240

Pro Cys Glu His Arg Thr Leu Glu Met Val Arg Glu Phe Ala Gly Asn
                245                 250                 255

Ala Pro Cys Trp Arg Gly Ser Arg Arg Thr Leu Ala Val Leu Ala Ala
            260                 265                 270

His Cys Pro Phe Tyr Ser Trp Lys Arg Val Phe Leu Thr His Pro Ala
        275                 280                 285

Thr Cys Tyr Arg Thr Thr Cys Pro Gly Pro Cys Asp Ser Gln Pro Cys
    290                 295                 300

Gln Asn Gly Gly Thr Cys Val Pro Glu Gly Leu Asp Gly Tyr Gln Cys
305                 310                 315                 320

Leu Cys Pro Leu Ala Phe Gly Gly Glu Ala Asn Cys Ala Leu Lys Leu
                325                 330                 335

Ser Leu Glu Cys Arg Val Asp Leu Leu Phe Leu Leu Asp Ser Ser Ala
            340                 345                 350

Gly Thr Thr Leu Asp Gly Phe Leu Arg Ala Lys Val Phe Val Lys Arg
        355                 360                 365

Phe Val Arg Ala Val Leu Ser Glu Asp Ser Arg Ala Arg Val Gly Val
    370                 375                 380

Ala Thr Tyr Ser Arg Glu Leu Leu Val Ala Val Pro Val Gly Glu Tyr
385                 390                 395                 400

Gln Asp Val Pro Asp Leu Val Trp Ser Leu Asp Gly Ile Pro Phe Arg
                405                 410                 415

Gly Gly Pro Thr Leu Thr Gly Ser Ala Leu Arg Gln Ala Ala Glu Arg
            420                 425                 430

Gly Phe Gly Ser Ala Thr Arg Thr Gly Gln Asp Arg Pro Arg Arg Val
        435                 440                 445

Val Val Leu Leu Thr Glu Ser His Ser Glu Asp Glu Val Ala Gly Pro
    450                 455                 460

Ala Arg His Ala Arg Ala Arg Glu Leu Leu Leu Leu Gly Val Gly Ser
465                 470                 475                 480

Glu Ala Val Arg Ala Glu Leu Glu Glu Ile Thr Gly Ser Pro Lys His
                485                 490                 495

Val Met Val Tyr Ser Asp Pro Gln Asp Leu Phe Asn Gln Ile Pro Glu
            500                 505                 510

Leu Gln Gly Lys Leu Cys Ser Arg Gln Arg Pro Gly Cys Arg Thr Gln
        515                 520                 525

Ala Leu Asp Leu Val Phe Met Leu Asp Thr Ser Ala Ser Val Gly Pro
    530                 535                 540

Glu Asn Phe Ala Gln Met Gln Ser Phe Val Arg Ser Cys Ala Leu Gln
545                 550                 555                 560

Phe Glu Val Asn Pro Asp Val Thr Gln Val Gly Leu Val Val Tyr Gly
                565                 570                 575

Ser Gln Val Gln Thr Ala Phe Gly Leu Asp Thr Lys Pro Thr Arg Ala
            580                 585                 590

Ala Met Leu Arg Ala Ile Ser Gln Ala Pro Tyr Leu Gly Gly Val Gly
        595                 600                 605

Ser Ala Gly Thr Ala Leu Leu His Ile Tyr Asp Lys Val Met Thr Val
    610                 615                 620

Gln Arg Gly Ala Arg Pro Gly Val Pro Lys Ala Val Val Val Leu Thr
625                 630                 635                 640

Gly Gly Arg Gly Ala Glu Asp Ala Ala Val Pro Ala Gln Lys Leu Arg
                645                 650                 655

Asn Asn Gly Ile Ser Val Leu Val Val Gly Val Gly Pro Val Leu Ser
            660                 665                 670

Glu Gly Leu Arg Arg Leu Ala Gly Pro Arg Asp Ser Leu Ile His Val
        675                 680                 685

Ala Ala Tyr Ala Asp Leu Arg Tyr His Gln Asp Val Leu Ile Glu Trp
    690                 695                 700

Leu Cys Gly Glu Ala Lys Gln Pro Val Asn Leu Cys Lys Pro Ser Pro
705                 710                 715                 720

Cys Met Asn Glu Gly Ser Cys Val Leu Gln Asn Gly Ser Tyr Arg Cys
                725                 730                 735

Lys Cys Arg Asp Gly Trp Glu Gly Pro His Cys Glu Asn Arg Glu Trp
            740                 745                 750

Ser Ser Cys Ser Val Cys Val Ser Gln Gly Trp Ile Leu Glu Thr Pro
        755                 760                 765

Leu Arg His Met Ala Pro Val Gln Glu Gly Ser Ser Arg Thr Pro Pro
    770                 775                 780

Ser Asn Tyr Arg Glu Gly Leu Gly Thr Glu Met Val Pro Thr Phe Trp
785                 790                 795                 800

Asn Val Cys Ala Pro Gly Pro
                805

<210>  186
<211>  1723
<212>  DNA
<213>  Homo sapiens

<400>  186
tgctacccgc gcccgggctt ctggggtgtt ccccaaccac ggcccagccc tgccacaccc      60

cccgcccccg gcctccgcag ctcggcatgg gcgcgggggt gctcgtcctg ggcgcctccg     120

agcccggtaa cctgtcgtcg gccgcaccgc tccccgacgg cgcggccacc gcggcgcggc     180

tgctggtgcc cgcgtcgccg cccgcctcgt tgctgcctcc cgccagcgaa agccccgagc     240

cgctgtctca gcagtggaca gcgggcatgg gtctgctgat ggcgctcatc gtgctgctca     300

tcgtggcggg caatgtgctg gtgatcgtgg ccatcgccaa gacgccgcgg ctgcagacgc     360

tcaccaacct cttcatcatg tccctggcca gcgccgacct ggtcatgggg ctgctggtgg     420

tgccgttcgg ggccaccatc gtggtgtggg gccgctggga gtacggctcc ttcttctgcg     480

agctgtggac ctcagtggac gtgctgtgcg tgacggccag catcgagacc ctgtgtgtca     540

ttgccctgga ccgctacctc gccatcacct cgcccttccg ctaccagagc ctgctgacgc     600

gcgcgcgggc gcggggcctc gtgtgcaccg tgtgggccat ctcggccctg gtgtccttcc 660

tgcccatcct catgcactgg tggcgggcgg agagcgacga ggcgcgccgc tgctacaacg 720

accccaagtg ctgcgacttc gtcaccaacc gggcctacgc catcgcctcg tccgtagtct 780

ccttctacgt gcccctgtgc atcatggcct tcgtgtacct gcgggtgttc cgcgaggccc 840

agaagcaggt gaagaagatc gacagctgcg agcgccgttt cctcggcggc ccagcgcggc 900

cgccctcgcc ctcgccctcg cccgtccccg cgcccgcgcc gccgcccgga cccccgcgcc 960

ccgccgccgc cgccgccacc gccccgctgg ccaacgggcg tgcgggtaag cggcggccct 1020

cgcgcctcgt ggccctacgc gagcagaagg cgctcaagac gctgggcatc atcatgggcg 1080

tcttcacgct ctgctggctg cccttcttcc tggccaacgt ggtgaaggcc ttccaccgcg 1140

agctggtgcc cgaccgcctc ttcgtcttct tcaactggct gggctacgcc aactcggcct 1200

tcaaccccat catctactgc cgcagccccg acttccgcaa ggccttccag ggactgctct 1260

gctgcgcgcg cagggctgcc cgccggcgcc acgcgaccca cggagaccgg ccgcgcgcct 1320

cgggctgtct ggcccggccc ggacccccgc catcgcccgg ggccgcctcg gacgacgacg 1380

acgacgatgt cgtcggggcc acgccgcccg cgcgcctgct ggagccctgg gccggctgca 1440

acggcggggc ggcggcggac agcgactcga gcctggacga gccgtgccgc cccggcttcg 1500

cctcggaatc caaggtgtag ggcccggcgc ggggcgcgga ctccgggcac ggcttcccag 1560

gggaacgagg agatctgtgt ttacttaaga ccgatagcag gtgaactcga agcccacaat 1620

cctcgtctga atcatccgag gcaaagagaa aagccacgga ccgttgcaca aaaaggaaag 1680

tttgggaagg gatgggagag tggcttgctg atgttccttg ttg 1723

<210> 187
<211> 477
<212> PRT
<213> <seq186;dna; Homo sapiens

<400> 187

Met Gly Ala Gly Val Leu Val Leu Gly Ala Ser Glu Pro Gly Asn Leu
1               5                   10                  15

Ser Ser Ala Ala Pro Leu Pro Asp Gly Ala Ala Thr Ala Ala Arg Leu
            20                  25                  30

Leu Val Pro Ala Ser Pro Pro Ala Ser Leu Leu Pro Pro Ala Ser Glu
        35                  40                  45

Ser Pro Glu Pro Leu Ser Gln Gln Trp Thr Ala Gly Met Gly Leu Leu
    50                  55                  60

Met Ala Leu Ile Val Leu Leu Ile Val Ala Gly Asn Val Leu Val Ile
65                  70                  75                  80

Val Ala Ile Ala Lys Thr Pro Arg Leu Gln Thr Leu Thr Asn Leu Phe
                85                  90                  95

Ile Met Ser Leu Ala Ser Ala Asp Leu Val Met Gly Leu Leu Val Val
            100                 105                 110

Pro Phe Gly Ala Thr Ile Val Val Trp Gly Arg Trp Glu Tyr Gly Ser
        115                 120                 125

Phe Phe Cys Glu Leu Trp Thr Ser Val Asp Val Leu Cys Val Thr Ala
    130                 135                 140

Ser Ile Glu Thr Leu Cys Val Ile Ala Leu Asp Arg Tyr Leu Ala Ile
145                 150                 155                 160

Thr Ser Pro Phe Arg Tyr Gln Ser Leu Leu Thr Arg Ala Arg Ala Arg
                165                 170                 175

Gly Leu Val Cys Thr Val Trp Ala Ile Ser Ala Leu Val Ser Phe Leu
            180                 185                 190

Pro Ile Leu Met His Trp Trp Arg Ala Glu Ser Asp Glu Ala Arg Arg
        195                 200                 205

Cys Tyr Asn Asp Pro Lys Cys Cys Asp Phe Val Thr Asn Arg Ala Tyr
    210                 215                 220

Ala Ile Ala Ser Ser Val Val Ser Phe Tyr Val Pro Leu Cys Ile Met
225                 230                 235                 240

Ala Phe Val Tyr Leu Arg Val Phe Arg Glu Ala Gln Lys Gln Val Lys
                245                 250                 255

Lys Ile Asp Ser Cys Glu Arg Arg Phe Leu Gly Gly Pro Ala Arg Pro
            260                 265                 270

Pro Ser Pro Ser Pro Ser Pro Val Pro Ala Pro Ala Pro Pro Pro Gly
        275                 280                 285

Pro Pro Arg Pro Ala Ala Ala Ala Ala Thr Ala Pro Leu Ala Asn Gly
    290                 295                 300

Arg Ala Gly Lys Arg Arg Pro Ser Arg Leu Val Ala Leu Arg Glu Gln
305                 310                 315                 320

Lys Ala Leu Lys Thr Leu Gly Ile Ile Met Gly Val Phe Thr Leu Cys
                325                 330                 335

Trp Leu Pro Phe Phe Leu Ala Asn Val Val Lys Ala Phe His Arg Glu
            340                 345                 350

Leu Val Pro Asp Arg Leu Phe Val Phe Phe Asn Trp Leu Gly Tyr Ala
        355                 360                 365

Asn Ser Ala Phe Asn Pro Ile Ile Tyr Cys Arg Ser Pro Asp Phe Arg
    370                 375                 380

Lys Ala Phe Gln Gly Leu Leu Cys Cys Ala Arg Arg Ala Ala Arg Arg
385                 390                 395                 400

Arg His Ala Thr His Gly Asp Arg Pro Arg Ala Ser Gly Cys Leu Ala
                405                 410                 415

Arg Pro Gly Pro Pro Pro Ser Pro Gly Ala Ala Ser Asp Asp Asp Asp
            420                 425                 430

Asp Asp Val Val Gly Ala Thr Pro Pro Ala Arg Leu Leu Glu Pro Trp
        435                 440                 445

Ala Gly Cys Asn Gly Gly Ala Ala Ala Asp Ser Asp Ser Ser Leu Asp
    450                 455                 460

Glu Pro Cys Arg Pro Gly Phe Ala Ser Glu Ser Lys Val
465                 470                 475

<210> 188
<211> 3737
<212> DNA
<213> Homo sapiens

<400> 188
ggcgtccgcg cacacctccc cgcgccgccg ccgccaccgc ccgcactccg ccgcctctgc 60

ccgcaaccgc tgagccatcc atgggggtcg cgggccgcaa ccgtcccggg gcggcctggg 120

cggtgctgct gctgctgctg ctgctgccgc cactgctgct gctggcgggg gccgtcccgc 180

cgggtcgggg ccgtgccgcg gggccgcagg aggatgtaga tgagtgtgcc caagggctag 240

atgactgcca tgccgacgcc ctgtgtcaga acacacccac ctcctacaag tgctcctgca 300

agcctggcta ccaaggggaa ggcaggcagt gtgaggacat cgatgaatgt ggaaatgagc 360

tcaatggagg ctgtgtccat gactgtttga atattccagg caattatcgt tgcacttgtt 420

ttgatggctt catgttggct catgacggtc ataattgtct tgatgtggac gagtgcctgg 480

agaacaatgg cggctgccag catacctgtg tcaacgtcat ggggagctat gagtgctgct 540

gcaaggaggg gtttttcctg agtgacaatc agcacacctg cattcaccgc tcggaagagg 600

gcctgagctg catgaataag gatcacggct gtagtcacat ctgcaaggag gccccaaggg 660

gcagcgtcgc ctgtgagtgc aggcctggtt ttgagctggc caagaaccag agagactgca 720

tcttgacctg taaccatggg aacggtgggt gccagcactc ctgtgacgat acagccgatg 780

gcccagagtg cagctgccat ccacagtaca agatgcacac agatgggagg agctgccttg 840

agcgagagga cactgtcctg gaggtgacag agagcaacac cacatcagtg gtggatgggg 900

ataaacgggt gaaacggcgg ctgctcatgg aaacgtgtgc tgtcaacaat ggaggctgtg 960

accgcacctg taaggatact tcgacaggtg tccactgcag ttgtcctgtt ggattcactc 1020

tccagttgga tgggaagaca tgtaaagata ttgatgagtg ccagacccgc aatggaggtt 1080

gtgatcattt ctgcaaaaac atcgtgggca gttttgactg cggctgcaag aaaggattta 1140

aattattaac agatgagaag tcttgccaag atgtggatga gtgctctttg gataggacct 1200

gtgaccacag ctgcatcaac caccctggca catttgcttg tgcttgcaac cgagggtaca 1260

ccctgtatgg cttcacccac tgtggagaca ccaatgagtg cagcatcaac aacggaggct 1320

gtcagcaggt ctgtgtgaac acagtgggca gctatgaatg ccagtgccac cctgggtaca 1380

agctccactg gaataaaaaa gactgtgtgg aagtgaaggg gctcctgccc acaagtgtgt 1440

caccccgtgt gtccctgcac tgcggtaaga gtggtggagg agacgggtgc ttcctcagat 1500

gtcactctgg cattcacctc tcttcagatg tcaccaccat caggacaagt gtaaccttta 1560

agctaaatga aggcaagtgt agtttgaaaa atgctgagct gtttcccgag ggtctgcgac 1620

cagcactacc agagaagcac agctcagtaa aagagagctt ccgctacgta aaccttacat 1680

gcagctctgg caagcaagtc ccaggagccc ctggccgacc aagcacccct aaggaaatgt 1740

ttatcactgt tgagtttgag cttgaaacta accaaaagga ggtgacagct tcttgtgacc 1800

tgagctgcat cgtaaagcga accgagaagc ggctccgtaa agccatccgc acgctcagaa 1860

aggccgtcca cagggagcag tttcacctcc agctctcagg catgaacctc gacgtggcta 1920
aaaagcctcc cagaacatct gaacgccagg cagagtcctg tggagtgggc cagggtcatg 1980
cagaaaacca atgtgtcagt tgcagggctg ggacctatta tgatggagca cgagaacgct 2040
gcattttatg tccaaatgga accttccaaa atgaggaagg acaaatgact tgtgaaccat 2100
gcccaagacc aggaaattct ggggccctga agaccccaga agcttggaat atgtctgaat 2160
gtggaggtct gtgtcaacct ggtgaatatt ctgcagatgg ctttgcacct tgccagctct 2220
gtgccctggg cacgttccag cctgaagctg gtcgaacttc ctgcttcccc tgtggaggag 2280
gccttgccac caaacatcag ggagctactt cctttcagga ctgtgaaacc agagttcaat 2340
gttcacctgg acatttctac aacaccacca ctcaccgatg tattcgttgc ccagtgggaa 2400
cataccagcc tgaatttgga aaaaataatt gtgtttcttg cccaggaaat actacgactg 2460
actttgatgg ctccacaaac ataacccagt gtaaaaacag aagatgtgga ggggagctgg 2520
gagatttcac tgggtacatt gaatccccaa actacccagg caattaccca gccaacaccg 2580
agtgtacgtg gaccatcaac ccacccccca agcgccgcat cctgatcgtg gtccctgaga 2640
tcttcctgcc catagaggac gactgtgggg actatctggt gatgcggaaa acctcttcat 2700
ccaattctgt gacaacatat gaaacctgcc agacctacga acgccccatc gccttcacct 2760
ccaggtcaaa gaagctgtgg attcagttca agtccaatga agggaacagc gctagagggt 2820
tccaggtccc atacgtgaca tatgatgagg actaccagga actcattgaa gacatagttc 2880
gagatggcag gctctatgca tctgagaacc atcaggaaat acttaaggat aagaaactta 2940
tcaaggctct gtttgatgtc ctggcccatc cccagaacta tttcaagtac acagcccagg 3000
agtcccgaga gatgtttcca agatcgttca tccgattgct acgttccaaa gtgtccaggt 3060
ttttgagacc ttacaaatga ctcagcccac gtgccactca atacaaatgt tctgctatag 3120
ggttggtggg acagagctgt cttccttctg catgtcagca cagtcgggta ttgctgcctc 3180
ccgtatcagt gactcattag agttcaattt ttatagataa tacagatatt ttggtaaatt 3240
gaacttggtt tttctttccc agcatcgtgg atgtagactg agaatggctt tgagtggcat 3300
cagcttctca ctgctgtggg cggatgtctt ggatagatca cgggctggct gagctggact 3360
ttggtcagcc taggtgagac tcacctgtcc ttctggggtc ttactcctcc tcaaggagtc 3420
tgtagtggaa aggaggccac agaataagct gcttattctg aaacttcagc ttcctctagc 3480
ccggccctct ctaagggagc cctctgcact cgtgtgcagg ctctgaccag gcagaacagg 3540
caagagggga gggaaggaga cccctgcagg ctccctccac ccaccttgag acctgggagg 3600
actcagtttc tccacagcct tctccagcct gtgtgataca agtttgatcc caggaacttg 3660
agttctaagc agtgctcgtg aaaaaaaaaa gcagaaagaa ttagaaataa ataaaaacta 3720
agcacttctg gagacat 3737

<210> 189
<211> 999
<212> PRT
<213> Homo sapiens

<400>  189

Met Gly Val Ala Gly Arg Asn Arg Pro Gly Ala Ala Trp Ala Val Leu
1               5                   10                  15

Leu Leu Leu Leu Leu Leu Pro Pro Leu Leu Leu Leu Ala Gly Ala Val
            20                  25                  30

Pro Pro Gly Arg Gly Arg Ala Ala Gly Pro Gln Glu Asp Val Asp Glu
        35                  40                  45

Cys Ala Gln Gly Leu Asp Asp Cys His Ala Asp Ala Leu Cys Gln Asn
    50                  55                  60

Thr Pro Thr Ser Tyr Lys Cys Ser Cys Lys Pro Gly Tyr Gln Gly Glu
65                  70                  75                  80

Gly Arg Gln Cys Glu Asp Ile Asp Glu Cys Gly Asn Glu Leu Asn Gly
                85                  90                  95

Gly Cys Val His Asp Cys Leu Asn Ile Pro Gly Asn Tyr Arg Cys Thr
            100                 105                 110

Cys Phe Asp Gly Phe Met Leu Ala His Asp Gly His Asn Cys Leu Asp
        115                 120                 125

Val Asp Glu Cys Leu Glu Asn Asn Gly Gly Cys Gln His Thr Cys Val
    130                 135                 140

Asn Val Met Gly Ser Tyr Glu Cys Cys Cys Lys Glu Gly Phe Phe Leu
145                 150                 155                 160

Ser Asp Asn Gln His Thr Cys Ile His Arg Ser Glu Glu Gly Leu Ser
                165                 170                 175

Cys Met Asn Lys Asp His Gly Cys Ser His Ile Cys Lys Glu Ala Pro
            180                 185                 190

Arg Gly Ser Val Ala Cys Glu Cys Arg Pro Gly Phe Glu Leu Ala Lys
        195                 200                 205

Asn Gln Arg Asp Cys Ile Leu Thr Cys Asn His Gly Asn Gly Gly Cys
    210                 215                 220

Gln His Ser Cys Asp Asp Thr Ala Asp Gly Pro Glu Cys Ser Cys His
225                 230                 235                 240

Pro Gln Tyr Lys Met His Thr Asp Gly Arg Ser Cys Leu Glu Arg Glu
                245                 250                 255

Asp Thr Val Leu Glu Val Thr Glu Ser Asn Thr Thr Ser Val Val Asp
            260                 265                 270

Gly Asp Lys Arg Val Lys Arg Arg Leu Leu Met Glu Thr Cys Ala Val
        275                 280                 285

Asn Asn Gly Gly Cys Asp Arg Thr Cys Lys Asp Thr Ser Thr Gly Val
    290                 295                 300

His Cys Ser Cys Pro Val Gly Phe Thr Leu Gln Leu Asp Gly Lys Thr
305                 310                 315                 320

Cys Lys Asp Ile Asp Glu Cys Gln Thr Arg Asn Gly Gly Cys Asp His
                325                 330                 335

Phe Cys Lys Asn Ile Val Gly Ser Phe Asp Cys Gly Cys Lys Lys Gly
            340                 345                 350

Phe Lys Leu Leu Thr Asp Glu Lys Ser Cys Gln Asp Val Asp Glu Cys
        355                 360                 365

Ser Leu Asp Arg Thr Cys Asp His Ser Cys Ile Asn His Pro Gly Thr
    370                 375                 380

Phe Ala Cys Ala Cys Asn Arg Gly Tyr Thr Leu Tyr Gly Phe Thr His
385                 390                 395                 400

Cys Gly Asp Thr Asn Glu Cys Ser Ile Asn Asn Gly Gly Cys Gln Gln
                405                 410                 415

Val Cys Val Asn Thr Val Gly Ser Tyr Glu Cys Gln Cys His Pro Gly
            420                 425                 430

Tyr Lys Leu His Trp Asn Lys Lys Asp Cys Val Glu Val Lys Gly Leu
        435                 440                 445

Leu Pro Thr Ser Val Ser Pro Arg Val Ser Leu His Cys Gly Lys Ser
    450                 455                 460

Gly Gly Gly Asp Gly Cys Phe Leu Arg Cys His Ser Gly Ile His Leu
465                 470                 475                 480

Ser Ser Asp Val Thr Thr Ile Arg Thr Ser Val Thr Phe Lys Leu Asn
                485                 490                 495

Glu Gly Lys Cys Ser Leu Lys Asn Ala Glu Leu Phe Pro Glu Gly Leu
            500                 505                 510

Arg Pro Ala Leu Pro Glu Lys His Ser Ser Val Lys Glu Ser Phe Arg
        515                 520                 525

Tyr Val Asn Leu Thr Cys Ser Ser Gly Lys Gln Val Pro Gly Ala Pro
    530                 535                 540

Gly Arg Pro Ser Thr Pro Lys Glu Met Phe Ile Thr Val Glu Phe Glu
545                 550                 555                 560

Leu Glu Thr Asn Gln Lys Glu Val Thr Ala Ser Cys Asp Leu Ser Cys
                565                 570                 575

Ile Val Lys Arg Thr Glu Lys Arg Leu Arg Lys Ala Ile Arg Thr Leu
            580                 585                 590

Arg Lys Ala Val His Arg Glu Gln Phe His Leu Gln Leu Ser Gly Met
        595                 600                 605

Asn Leu Asp Val Ala Lys Lys Pro Pro Arg Thr Ser Glu Arg Gln Ala
    610                 615                 620

Glu Ser Cys Gly Val Gly Gln Gly His Ala Glu Asn Gln Cys Val Ser
625                 630                 635                 640

Cys Arg Ala Gly Thr Tyr Tyr Asp Gly Ala Arg Glu Arg Cys Ile Leu
                645                 650                 655

Cys Pro Asn Gly Thr Phe Gln Asn Glu Glu Gly Gln Met Thr Cys Glu
            660                 665                 670

Pro Cys Pro Arg Pro Gly Asn Ser Gly Ala Leu Lys Thr Pro Glu Ala
        675                 680                 685

Trp Asn Met Ser Glu Cys Gly Gly Leu Cys Gln Pro Gly Glu Tyr Ser
    690                 695                 700

Ala Asp Gly Phe Ala Pro Cys Gln Leu Cys Ala Leu Gly Thr Phe Gln

705                 710                 715                 720

Pro Glu Ala Gly Arg Thr Ser Cys Phe Pro Cys Gly Gly Gly Leu Ala
                725                 730                 735

Thr Lys His Gln Gly Ala Thr Ser Phe Gln Asp Cys Glu Thr Arg Val
            740                 745                 750

Gln Cys Ser Pro Gly His Phe Tyr Asn Thr Thr Thr His Arg Cys Ile
        755                 760                 765

Arg Cys Pro Val Gly Thr Tyr Gln Pro Glu Phe Gly Lys Asn Asn Cys
    770                 775                 780

Val Ser Cys Pro Gly Asn Thr Thr Thr Asp Phe Asp Gly Ser Thr Asn
785                 790                 795                 800

Ile Thr Gln Cys Lys Asn Arg Arg Cys Gly Gly Glu Leu Gly Asp Phe
                805                 810                 815

Thr Gly Tyr Ile Glu Ser Pro Asn Tyr Pro Gly Asn Tyr Pro Ala Asn
            820                 825                 830

Thr Glu Cys Thr Trp Thr Ile Asn Pro Pro Pro Lys Arg Arg Ile Leu
        835                 840                 845

Ile Val Val Pro Glu Ile Phe Leu Pro Ile Glu Asp Asp Cys Gly Asp
    850                 855                 860

Tyr Leu Val Met Arg Lys Thr Ser Ser Ser Asn Ser Val Thr Thr Tyr
865                 870                 875                 880

Glu Thr Cys Gln Thr Tyr Glu Arg Pro Ile Ala Phe Thr Ser Arg Ser
                885                 890                 895

Lys Lys Leu Trp Ile Gln Phe Lys Ser Asn Glu Gly Asn Ser Ala Arg
            900                 905                 910

Gly Phe Gln Val Pro Tyr Val Thr Tyr Asp Glu Asp Tyr Gln Glu Leu
        915                 920                 925

Ile Glu Asp Ile Val Arg Asp Gly Arg Leu Tyr Ala Ser Glu Asn His
    930                 935                 940

Gln Glu Ile Leu Lys Asp Lys Lys Leu Ile Lys Ala Leu Phe Asp Val
945                 950                 955                 960

Leu Ala His Pro Gln Asn Tyr Phe Lys Tyr Thr Ala Gln Glu Ser Arg
                965                 970                 975

Glu Met Phe Pro Arg Ser Phe Ile Arg Leu Leu Arg Ser Lys Val Ser
            980                 985                 990

Arg Phe Leu Arg Pro Tyr Lys
        995

<210>  190
<211>  2642
<212>  DNA
<213>  Homo sapiens

<400>  190
aaggaggcgg cctccgggaa aagcgaccgc aggactcctg agagcagcct ccatgaggcc      60

ctggaccagt gcatgaccgc cctggacctc ttcctcacca accagttctc agaagcactc     120

agctacctca agcccagaac caaggaaagc atgtaccact cactgacata tgccaccatc     180

ctggagatgc aggccatgat gacctttgac cctcaggaca tcctgcttgc cggcaacatg 240
atgaaggagg cacagatgct gtgtcagagg caccggagga agtcttctgt aacagattcc 300
ttcagcagcc tggtgaaccg ccccacgctg ggccaattca ctgaagaaga aatccacgct 360
gaggtctgct atgcagagtg cctgctgcag cgagcagccc tgaccttcct gcaggacgag 420
aacatggtga gcttcatcaa aggcggcatc aaagttcgaa acagctacca gacctacaag 480
gagctggaca gccttgttca gtcctcacaa tactgcaagg gtgagaacca cccgcacttt 540
gaaggaggag tgaagcttgg tgtaggggcc ttcaacctga cactgtccat gcttcctact 600
aggatcctga ggctgttgga gtttgtgggg ttttcaggaa acaaggacta tgggctgctg 660
cagctggagg agggagcgtc agggcacagc ttccgctctg tgctctgtgt catgctcctg 720
ctgtgctacc acaccttcct caccttcgtg ctcggtactg ggaacgtcaa catcgaggag 780
gccgagaagc tcttgaagcc ctacctgaac cggtacccta agggtgccat cttcctgttc 840
tttgcaggga ggattgaagt cattaaaggc aacattgatg cagccatccg gcgtttcgag 900
gagtgctgtg aggcccagca gcactggaag cagtttcacc acatgtgcta ctgggagctg 960
atgtggtgct tcacctacaa gggccagtgg aagatgtcct acttctacgc cgacctgctc 1020
agcaaggaga actgctggtc caaggccacc tacatttaca tgaaggccgc ctacctcagc 1080
atgtttggga aggaggacca caagccgttc ggggacgacg aagtggaatt atttcgagct 1140
gtgccaggcc tgaagctcaa gattgctggg aaatctctac ccacagagaa gtttgccatc 1200
cggaagtccc ggcgctactt ctcctccaac cctatctcgc tgccagtgcc tgctctggaa 1260
atgatgtaca tctggaacgg ctacgccgtg attgggaagc agccgaaact cacggatggg 1320
atacttgaga ttatcactaa ggctgaagag atgctggaga aaggcccaga gaacgagtac 1380
tcagtggatg acgagtgctt ggtgaaattg ttgaaaggcc tgtgtctgaa atacctgggc 1440
cgtgtccagg aggccgagga gaattttagg agcatctctg ccaatgaaaa gaagattaaa 1500
tatgaccact acttgatccc aaacgccctg ctggagctgg ccctgctgct tatggagcaa 1560
gacagaaacg aagaggccat caaacttttg gaatctgcca agcaaaacta caagaattac 1620
tccatggagt caaggacaca ctttcgaatc caggcagcca cactccaagc caagtcttcc 1680
ctagagaaca gcagcagatc catggtctca tcagtgtcct tgtagctttg tgcagcagtt 1740
ccgggctgga agacagagac agctggacag agctcctgaa aacatttcaa aataccccct 1800
ccccctgccc tgccctgcct ttggggtcca ccggcactcc agttggatgg cacaacatag 1860
tgtatccgtg cagaagccga gctggcattt tcaccagtgt agccaagggc ctttgccaag 1920
ggcagagcag gtggagccct ctgcctgccc tatcacacat acgggtactt gcttttcact 1980
gtgatgttta agagaatgta tgaacagttt acattttcct tagaaataca ttgatgggat 2040
cacagttggc tttaaaaacc aacaacaatc aaccacctgt aagtctttgt cttcacctat 2100
tatcatctgg aggtaaatct ctttatatga tgatgccaaa gggcaaattg cttttcaaat 2160
tcagcaagtt ctcagcttgt gtgacggaag gtccttcaga ggacctgagg aatgcctggg 2220

agaggctaag cctcaggctt caatgcttct ggggttgggc atgaggatgt acacagacac 2280

ccactacctt actactcaca cttcatttca ctccttttgt aaatttccaa tttaaaaatc 2340

aagcacgtct ttttagtgag ataaaatctg agctcttctg tagaaaaatc aatctctacc 2400

agtagaaaat gccagggctt gatggaagag ctgtgtagcc ctttctatgc caaagccagg 2460

aaatttgggg ggcaggagga ggttctcaga atccagtctg tatctttgct gtatgccaaa 2520

ctgaaaccac tgggaataat ttatgaaaca taaaaatctt ctgtacttca ctccaaggta 2580

catttgctta ctgacagcat ttttgttaaa actgttattc ttgaaaaaaa aaaaaaaaaa 2640

aa 2642

<210> 191
<211> 550
<212> PRT
<213> Homo sapiens

<400> 191

Met Thr Ala Leu Asp Leu Phe Leu Thr Asn Gln Phe Ser Glu Ala Leu
1               5                   10                  15

Ser Tyr Leu Lys Pro Arg Thr Lys Glu Ser Met Tyr His Ser Leu Thr
            20                  25                  30

Tyr Ala Thr Ile Leu Glu Met Gln Ala Met Met Thr Phe Asp Pro Gln
        35                  40                  45

Asp Ile Leu Leu Ala Gly Asn Met Met Lys Glu Ala Gln Met Leu Cys
    50                  55                  60

Gln Arg His Arg Arg Lys Ser Ser Val Thr Asp Ser Phe Ser Ser Leu
65                  70                  75                  80

Val Asn Arg Pro Thr Leu Gly Gln Phe Thr Glu Glu Glu Ile His Ala
                85                  90                  95

Glu Val Cys Tyr Ala Glu Cys Leu Leu Gln Arg Ala Ala Leu Thr Phe
            100                 105                 110

Leu Gln Asp Glu Asn Met Val Ser Phe Ile Lys Gly Gly Ile Lys Val
        115                 120                 125

Arg Asn Ser Tyr Gln Thr Tyr Lys Glu Leu Asp Ser Leu Val Gln Ser
    130                 135                 140

Ser Gln Tyr Cys Lys Gly Glu Asn His Pro His Phe Glu Gly Gly Val
145                 150                 155                 160

Lys Leu Gly Val Gly Ala Phe Asn Leu Thr Leu Ser Met Leu Pro Thr
                165                 170                 175

Arg Ile Leu Arg Leu Leu Glu Phe Val Gly Phe Ser Gly Asn Lys Asp
            180                 185                 190

Tyr Gly Leu Leu Gln Leu Glu Glu Gly Ala Ser Gly His Ser Phe Arg
        195                 200                 205

Ser Val Leu Cys Val Met Leu Leu Leu Cys Tyr His Thr Phe Leu Thr
    210                 215                 220

Phe Val Leu Gly Thr Gly Asn Val Asn Ile Glu Glu Ala Glu Lys Leu
225                 230                 235                 240

Leu Lys Pro Tyr Leu Asn Arg Tyr Pro Lys Gly Ala Ile Phe Leu Phe
                245                 250                 255

Phe Ala Gly Arg Ile Glu Val Ile Lys Gly Asn Ile Asp Ala Ala Ile
            260                 265                 270

Arg Arg Phe Glu Glu Cys Cys Glu Ala Gln Gln His Trp Lys Gln Phe
        275                 280                 285

His His Met Cys Tyr Trp Glu Leu Met Trp Cys Phe Thr Tyr Lys Gly
    290                 295                 300

Gln Trp Lys Met Ser Tyr Phe Tyr Ala Asp Leu Leu Ser Lys Glu Asn
305                 310                 315                 320

Cys Trp Ser Lys Ala Thr Tyr Ile Tyr Met Lys Ala Ala Tyr Leu Ser
                325                 330                 335

Met Phe Gly Lys Glu Asp His Lys Pro Phe Gly Asp Asp Glu Val Glu
            340                 345                 350

Leu Phe Arg Ala Val Pro Gly Leu Lys Leu Lys Ile Ala Gly Lys Ser
        355                 360                 365

Leu Pro Thr Glu Lys Phe Ala Ile Arg Lys Ser Arg Arg Tyr Phe Ser
    370                 375                 380

Ser Asn Pro Ile Ser Leu Pro Val Pro Ala Leu Glu Met Met Tyr Ile
385                 390                 395                 400

Trp Asn Gly Tyr Ala Val Ile Gly Lys Gln Pro Lys Leu Thr Asp Gly
                405                 410                 415

Ile Leu Glu Ile Ile Thr Lys Ala Glu Glu Met Leu Glu Lys Gly Pro
            420                 425                 430

Glu Asn Glu Tyr Ser Val Asp Asp Glu Cys Leu Val Lys Leu Leu Lys
        435                 440                 445

Gly Leu Cys Leu Lys Tyr Leu Gly Arg Val Gln Glu Ala Glu Glu Asn
    450                 455                 460

Phe Arg Ser Ile Ser Ala Asn Glu Lys Lys Ile Lys Tyr Asp His Tyr
465                 470                 475                 480

Leu Ile Pro Asn Ala Leu Leu Glu Leu Ala Leu Leu Leu Met Glu Gln
                485                 490                 495

Asp Arg Asn Glu Glu Ala Ile Lys Leu Leu Glu Ser Ala Lys Gln Asn
            500                 505                 510

Tyr Lys Asn Tyr Ser Met Glu Ser Arg Thr His Phe Arg Ile Gln Ala
        515                 520                 525

Ala Thr Leu Gln Ala Lys Ser Ser Leu Glu Asn Ser Ser Arg Ser Met
    530                 535                 540

Val Ser Ser Val Ser Leu
545                 550

<210>  192
<211>  4427
<212>  DNA
<213>  Homo sapiens

<400>  192
atgactagga agaggacata ctgggtgccc aactcttctg gtggcctcgt gaatcgtggc      60

atcgacatag gcgatgacat ggtttcagga cttatttata aaacctatac tctccaagat 120
ggcccctgga gtcagcaaga gagaaatcct gaggctccag ggagggcagc tgtcccaccg 180
tgggggaagt atgatgctgc cttgagaacc atgattccct tccgtcccaa gccgaggttt 240
cctgcccccc agcccctgga caatgctggc ctgttctcct acctcaccgt gtcatggctc 300
accccgctca tgatccaaag cttacggagt cgcttagatg agaacaccat ccctccactg 360
tcagtccatg atgcctcaga caaaaatgtc caaaggcttc accgcctttg ggaagaagaa 420
gtctcaaggc gagggattga aaaagcttca gtgcttctgg tgatgctgag gttccagaga 480
acaaggttga ttttcgatgc acttctgggc atctgcttct gcattgccag tgtactcggg 540
ccaatattga ttataccaaa gatcctggaa tattcagaag agcagttggg gaatgttgtc 600
catggagtgg gactctgctt tgcccttttt ctctccgaat gtgtgaagtc tctgagtttc 660
tcctccagtt ggatcatcaa ccaacgcaca gccatcaggt tccgagcagc tgtttcctcc 720
tttgcctttg agaagctcat ccaatttaag tctgtaatac acatcacctc aggagaggcc 780
atcagcttct tcaccggtga tgtaaactac ctgtttgaag gggtgtgcta tggaccccta 840
gtactgatca cctgcgcatc gctggtcatc tgcagcattt cttcctactt cattattgga 900
tacactgcat ttattgccat cttatgctat ctcctggttt tcccactggc ggtattcatg 960
acaagaatgg ctgtgaaggc tcagcatcac acatctgagg tcagcgacca gcgcatccgt 1020
gtgaccagtg aagttctcac ttgcattaag ctgattaaaa tgtacacatg ggagaaacca 1080
tttgcaaaaa tcattgaagg tatggaaagt ctgactttct gctccaaacc tggtgatggc 1140
atggccttca gcatgctggc ctccttgaat ctccttcggc tgtcagtgtt ctttgtgcct 1200
attgcagtca aaggtctcac gaattccaag tctgcagtga tgaggttcaa gaagtttttc 1260
ctccaggaga gccctgtttt ctatgtccag acattacaag accccagcaa agctctggtc 1320
tttgaggagg ccaccttgtc atggcaacag acctgtcccg ggatcgtcaa tggggcactg 1380
gagctggaga ggaacgggca tgcttctgag gggatgacca ggcctagaga tgccctcggg 1440
ccagaggaag aagggaacag cctgggccca gagttgcaca agatcaacct ggtggtgtcc 1500
aaggggatga tgttaggggt ctgcggcaac acggggagtg gtaagagcag cctgttgtca 1560
gccatcctgg aggagatgca cttgctcgag ggctcggtgg gggtgcaggg aagcctggcc 1620
tatgtccccc agcaggcctg gatcgtcagc gggaacatca gggagaacat cctcatggga 1680
ggcgcatatg acaaggcccg atacctccag gtgctccact gctgctccct gaatcgggac 1740
ctggaacttc tgccctttgg agacatgaca gagattggag agcggggcct caacctctct 1800
ggggggcaga aacagaggat cagcctggcc cgcgccgtct attccgaccg tcagatctac 1860
ctgctggacg accccctgtc tgctgtggac gcccacgtgg ggaagcacat ttttgaggag 1920
tgcattaaga agacactcag ggggaagacg gtcgtcctgg tgacccacca gctgcagtac 1980
ttagaatttt gtggccagat cattttgttg gaaaatggga aaatctgtga aaatggaact 2040
cacagtgagt taatgcagaa aaaggggaaa tatgcccaac ttatccagaa gatgcacaag 2100

gaagccactt cggacatgtt gcaggacaca gcaaagatag cagagaagcc aaaggtagaa 2160
agtcaggctc tggccacctc cctggaagag tctctcaacg gaaatgctgt gccggagcat 2220
cagctcacac aggaggagga gatggaagaa ggctccttga gttggagggt ctaccaccac 2280
tacatccagg cagctggagg ttacatggtc tcttgcataa ttttcttctt cgtggtgctg 2340
atcgtcttct taacgatctt cagcttctgg tggctgagct actggttgga gcagggctcg 2400
gggaccaata gcagccgaga gagcaatgga accatggcag acctgggcaa cattgcagac 2460
aatcctcaac tgtccttcta ccagctggtg tacgggctca acgccctgct cctcatctgt 2520
gtgggggtct gctcctcagg gattttcacc aaagtcacga ggaaggcatc cacggccctg 2580
cacaacaagc tcttcaacaa ggttttccgc tgccccatga gtttctttga caccatccca 2640
ataggccggc ttttgaactg cttcgcaggg gacttggaac agctggacca gctcttgccc 2700
atcttttcag agcagttcct ggtcctgtcc ttaatggtga tcgccgtcct gttgattgtc 2760
agtgtgctgt ctccatatat cctgttaatg ggagccataa tcatggttat ttgcttcatt 2820
tattatatga tgttcaagaa ggccatcggt gtgttcaaga gactggagaa ctatagccgg 2880
tctcctttat tctcccacat cctcaattct ctgcaaggcc tgagctccat ccatgtctat 2940
ggaaaaactg aagacttcat cagccagttt aagaggctga ctgatgcgca gaataactac 3000
ctgctgttgt ttctatcttc cacacgatgg atggcattga ggctggagat catgaccaac 3060
cttgtgacct tggctgttgc cctgttcgtg gcttttggca tttcctccac cccctactcc 3120
tttaaagtca tggctgtcaa catcgtgctg cagctggcgt ccagcttcca ggccactgcc 3180
cggattggct tggagacaga ggcacagttc acggctgtag agaggatact gcagtacatg 3240
aagatgtgtg tctcggaagc tcctttacac atggaaggca caagttgtcc ccaggggtgg 3300
ccacagcatg gggaaatcat atttcaggat tatcacatga aatacagaga caacacaccc 3360
accgtgcttc acggcatcaa cctgaccatc cgcggccacg aagtggtggg catcgtggga 3420
aggacgggct ctgggaagtc ctccttgggc atggctctct tccgcctggt ggagcccatg 3480
gcaggccgga ttctcattga cggcgtggac atttgcagca tcggcctgga ggacttgcgg 3540
tccaagctct cagtgatccc tcaagatcca gtgctgctct caggaaccat cagattcaac 3600
ctagatccct ttgaccgtca cactgaccag cagatctggg atgccttgga gaggacattc 3660
ctgaccaagg ccatctcaaa gttccccaaa aagctgcata cagatgtggt ggaaaacggt 3720
ggaaacttct ctgtggggga gaggcagctg ctctgcattg ccagggctgt gcttcgcaac 3780
tccaagatca tccttatcga tgaagccaca gcctccattg acatggagac agacaccctg 3840
atccagcgca caatccgtga agccttccag ggctgcaccg tgctcgtcat tgcccaccgt 3900
gtcaccactg tgctgaactg tgaccacatc ctggttatgg gcaatgggaa ggtggtagaa 3960
tttgatcggc cggaggtact gcggaagaag cctgggtcat tgttcgcagc cctcatggcc 4020
acagccactt cttcactgag ataaggagat gtggagactt catggaggct ggcagctgag 4080
ctcagaggtt cacacaggtg cagcttcgag gcccacagtc tgcgaccttc ttgtttggag 4140

atgagaactt ctcctggaag caggggtaaa tgtagggggg gtggggattg ctggatggaa 4200

accctggaat aggctacttg atggctctca agaccttaga accccagaac catctaagac 4260

atgggattca gtgatcatgt ggttctcctt ttaacttaca tgctgaataa ttttataata 4320

aggtaaaagc ttatagtttt ctgatctgtg ttagaagtgy tgcaaatgct gtactgactt 4380

tgtaaaatat aaaactaagg aaaactcaaa aaaaaaaaaa aaaaaaa 4427

<210> 193
<211> 1347
<212> PRT
<213> Homo sapiens

<400> 193

Met Thr Arg Lys Arg Thr Tyr Trp Val Pro Asn Ser Ser Gly Gly Leu
1               5                   10                  15

Val Asn Arg Gly Ile Asp Ile Gly Asp Asp Met Val Ser Gly Leu Ile
            20                  25                  30

Tyr Lys Thr Tyr Thr Leu Gln Asp Gly Pro Trp Ser Gln Gln Glu Arg
        35                  40                  45

Asn Pro Glu Ala Pro Gly Arg Ala Ala Val Pro Pro Trp Gly Lys Tyr
    50                  55                  60

Asp Ala Ala Leu Arg Thr Met Ile Pro Phe Arg Pro Lys Pro Arg Phe
65                  70                  75                  80

Pro Ala Pro Gln Pro Leu Asp Asn Ala Gly Leu Phe Ser Tyr Leu Thr
                85                  90                  95

Val Ser Trp Leu Thr Pro Leu Met Ile Gln Ser Leu Arg Ser Arg Leu
            100                 105                 110

Asp Glu Asn Thr Ile Pro Pro Leu Ser Val His Asp Ala Ser Asp Lys
        115                 120                 125

Asn Val Gln Arg Leu His Arg Leu Trp Glu Glu Glu Val Ser Arg Arg
    130                 135                 140

Gly Ile Glu Lys Ala Ser Val Leu Leu Val Met Leu Arg Phe Gln Arg
145                 150                 155                 160

Thr Arg Leu Ile Phe Asp Ala Leu Leu Gly Ile Cys Phe Cys Ile Ala
                165                 170                 175

Ser Val Leu Gly Pro Ile Leu Ile Ile Pro Lys Ile Leu Glu Tyr Ser
            180                 185                 190

Glu Glu Gln Leu Gly Asn Val Val His Gly Val Gly Leu Cys Phe Ala
        195                 200                 205

Leu Phe Leu Ser Glu Cys Val Lys Ser Leu Ser Phe Ser Ser Ser Trp
    210                 215                 220

Ile Ile Asn Gln Arg Thr Ala Ile Arg Phe Arg Ala Ala Val Ser Ser
225                 230                 235                 240

Phe Ala Phe Glu Lys Leu Ile Gln Phe Lys Ser Val Ile His Ile Thr
                245                 250                 255

Ser Gly Glu Ala Ile Ser Phe Phe Thr Gly Asp Val Asn Tyr Leu Phe
            260                 265                 270

Glu Gly Val Cys Tyr Gly Pro Leu Val Leu Ile Thr Cys Ala Ser Leu
        275                 280                 285

Val Ile Cys Ser Ile Ser Ser Tyr Phe Ile Ile Gly Tyr Thr Ala Phe
    290                 295                 300

Ile Ala Ile Leu Cys Tyr Leu Leu Val Phe Pro Leu Ala Val Phe Met
305                 310                 315                 320

Thr Arg Met Ala Val Lys Ala Gln His His Thr Ser Glu Val Ser Asp
                325                 330                 335

Gln Arg Ile Arg Val Thr Ser Glu Val Leu Thr Cys Ile Lys Leu Ile
            340                 345                 350

Lys Met Tyr Thr Trp Glu Lys Pro Phe Ala Lys Ile Ile Glu Gly Met
        355                 360                 365

Glu Ser Leu Thr Phe Cys Ser Lys Pro Gly Asp Gly Met Ala Phe Ser
    370                 375                 380

Met Leu Ala Ser Leu Asn Leu Leu Arg Leu Ser Val Phe Phe Val Pro
385                 390                 395                 400

Ile Ala Val Lys Gly Leu Thr Asn Ser Lys Ser Ala Val Met Arg Phe
                405                 410                 415

Lys Lys Phe Phe Leu Gln Glu Ser Pro Val Phe Tyr Val Gln Thr Leu
            420                 425                 430

Gln Asp Pro Ser Lys Ala Leu Val Phe Glu Glu Ala Thr Leu Ser Trp
        435                 440                 445

Gln Gln Thr Cys Pro Gly Ile Val Asn Gly Ala Leu Glu Leu Glu Arg
    450                 455                 460

Asn Gly His Ala Ser Glu Gly Met Thr Arg Pro Arg Asp Ala Leu Gly
465                 470                 475                 480

Pro Glu Glu Glu Gly Asn Ser Leu Gly Pro Glu Leu His Lys Ile Asn
                485                 490                 495

Leu Val Val Ser Lys Gly Met Met Leu Gly Val Cys Gly Asn Thr Gly
            500                 505                 510

Ser Gly Lys Ser Ser Leu Leu Ser Ala Ile Leu Glu Glu Met His Leu
        515                 520                 525

Leu Glu Gly Ser Val Gly Val Gln Gly Ser Leu Ala Tyr Val Pro Gln
    530                 535                 540

Gln Ala Trp Ile Val Ser Gly Asn Ile Arg Glu Asn Ile Leu Met Gly
545                 550                 555                 560

Gly Ala Tyr Asp Lys Ala Arg Tyr Leu Gln Val Leu His Cys Cys Ser
                565                 570                 575

Leu Asn Arg Asp Leu Glu Leu Leu Pro Phe Gly Asp Met Thr Glu Ile
            580                 585                 590

Gly Glu Arg Gly Leu Asn Leu Ser Gly Gly Gln Lys Gln Arg Ile Ser
        595                 600                 605

Leu Ala Arg Ala Val Tyr Ser Asp Arg Gln Ile Tyr Leu Leu Asp Asp
    610                 615                 620

Pro Leu Ser Ala Val Asp Ala His Val Gly Lys His Ile Phe Glu Glu

625                 630                 635                 640

Cys Ile Lys Lys Thr Leu Arg Gly Lys Thr Val Val Leu Val Thr His
                645                 650                 655

Gln Leu Gln Tyr Leu Glu Phe Cys Gly Gln Ile Ile Leu Leu Glu Asn
            660                 665                 670

Gly Lys Ile Cys Glu Asn Gly Thr His Ser Glu Leu Met Gln Lys Lys
        675                 680                 685

Gly Lys Tyr Ala Gln Leu Ile Gln Lys Met His Lys Glu Ala Thr Ser
    690                 695                 700

Asp Met Leu Gln Asp Thr Ala Lys Ile Ala Glu Lys Pro Lys Val Glu
705                 710                 715                 720

Ser Gln Ala Leu Ala Thr Ser Leu Glu Glu Ser Leu Asn Gly Asn Ala
                725                 730                 735

Val Pro Glu His Gln Leu Thr Gln Glu Glu Glu Met Glu Glu Gly Ser
            740                 745                 750

Leu Ser Trp Arg Val Tyr His His Tyr Ile Gln Ala Ala Gly Gly Tyr
        755                 760                 765

Met Val Ser Cys Ile Ile Phe Phe Phe Val Val Leu Ile Val Phe Leu
    770                 775                 780

Thr Ile Phe Ser Phe Trp Trp Leu Ser Tyr Trp Leu Glu Gln Gly Ser
785                 790                 795                 800

Gly Thr Asn Ser Ser Arg Glu Ser Asn Gly Thr Met Ala Asp Leu Gly
                805                 810                 815

Asn Ile Ala Asp Asn Pro Gln Leu Ser Phe Tyr Gln Leu Val Tyr Gly
            820                 825                 830

Leu Asn Ala Leu Leu Leu Ile Cys Val Gly Val Cys Ser Ser Gly Ile
        835                 840                 845

Phe Thr Lys Val Thr Arg Lys Ala Ser Thr Ala Leu His Asn Lys Leu
    850                 855                 860

Phe Asn Lys Val Phe Arg Cys Pro Met Ser Phe Phe Asp Thr Ile Pro
865                 870                 875                 880

Ile Gly Arg Leu Leu Asn Cys Phe Ala Gly Asp Leu Glu Gln Leu Asp
                885                 890                 895

Gln Leu Leu Pro Ile Phe Ser Glu Gln Phe Leu Val Leu Ser Leu Met
            900                 905                 910

Val Ile Ala Val Leu Leu Ile Val Ser Val Leu Ser Pro Tyr Ile Leu
        915                 920                 925

Leu Met Gly Ala Ile Ile Met Val Ile Cys Phe Ile Tyr Tyr Met Met
    930                 935                 940

Phe Lys Lys Ala Ile Gly Val Phe Lys Arg Leu Glu Asn Tyr Ser Arg
945                 950                 955                 960

Ser Pro Leu Phe Ser His Ile Leu Asn Ser Leu Gln Gly Leu Ser Ser
                965                 970                 975

Ile His Val Tyr Gly Lys Thr Glu Asp Phe Ile Ser Gln Phe Lys Arg
            980                 985                 990

Leu Thr Asp Ala Gln Asn Asn Tyr Leu Leu Leu Phe Leu Ser Ser Thr
        995                 1000                1005

Arg Trp Met Ala Leu Arg Leu Glu Ile Met Thr Asn Leu Val Thr
    1010                1015                1020

Leu Ala Val Ala Leu Phe Val Ala Phe Gly Ile Ser Ser Thr Pro
    1025                1030                1035

Tyr Ser Phe Lys Val Met Ala Val Asn Ile Val Leu Gln Leu Ala
    1040                1045                1050

Ser Ser Phe Gln Ala Thr Ala Arg Ile Gly Leu Glu Thr Glu Ala
    1055                1060                1065

Gln Phe Thr Ala Val Glu Arg Ile Leu Gln Tyr Met Lys Met Cys
    1070                1075                1080

Val Ser Glu Ala Pro Leu His Met Glu Gly Thr Ser Cys Pro Gln
    1085                1090                1095

Gly Trp Pro Gln His Gly Glu Ile Ile Phe Gln Asp Tyr His Met
    1100                1105                1110

Lys Tyr Arg Asp Asn Thr Pro Thr Val Leu His Gly Ile Asn Leu
    1115                1120                1125

Thr Ile Arg Gly His Glu Val Val Gly Ile Val Gly Arg Thr Gly
    1130                1135                1140

Ser Gly Lys Ser Ser Leu Gly Met Ala Leu Phe Arg Leu Val Glu
    1145                1150                1155

Pro Met Ala Gly Arg Ile Leu Ile Asp Gly Val Asp Ile Cys Ser
    1160                1165                1170

Ile Gly Leu Glu Asp Leu Arg Ser Lys Leu Ser Val Ile Pro Gln
    1175                1180                1185

Asp Pro Val Leu Leu Ser Gly Thr Ile Arg Phe Asn Leu Asp Pro
    1190                1195                1200

Phe Asp Arg His Thr Asp Gln Gln Ile Trp Asp Ala Leu Glu Arg
    1205                1210                1215

Thr Phe Leu Thr Lys Ala Ile Ser Lys Phe Pro Lys Lys Leu His
    1220                1225                1230

Thr Asp Val Val Glu Asn Gly Gly Asn Phe Ser Val Gly Glu Arg
    1235                1240                1245

Gln Leu Leu Cys Ile Ala Arg Ala Val Leu Arg Asn Ser Lys Ile
    1250                1255                1260

Ile Leu Ile Asp Glu Ala Thr Ala Ser Ile Asp Met Glu Thr Asp
    1265                1270                1275

Thr Leu Ile Gln Arg Thr Ile Arg Glu Ala Phe Gln Gly Cys Thr
    1280                1285                1290

Val Leu Val Ile Ala His Arg Val Thr Thr Val Leu Asn Cys Asp
    1295                1300                1305

His Ile Leu Val Met Gly Asn Gly Lys Val Val Glu Phe Asp Arg
    1310                1315                1320

Pro Glu Val Leu Arg Lys Lys Pro Gly Ser Leu Phe Ala Ala Leu

1325                1330                1335

Met Ala Thr Ala Thr Ser Ser Leu Arg
    1340                1345

<210>  194
<211>  3690
<212>  DNA
<213>  Homo sapiens

<400>  194
atgctgtctg gcttcttgat gagtcccagt acccagcaca gagcacagta cactcccgga      60

ggaaagaaac ttccgtggga ggcttccatc ggtgcgcaca cctcccgagg gcgaggcagc     120

gaccgggaga gggagagccg gccggaggct gccgggctcc tgtgggaccg cgctgcagcc     180

ggggaggcgg agaaggggaa ccggggcgag ccgcccgcct ggatccgcgc ccagcagcag     240

ccgcggccgc cgccagctgg gcaggctccc gggactgcgg ctgggggcgc gcaggaccct     300

cgcctgcgtc ctggacgttc ccgggggagg gtccggttgc cagtgaaacc tccagaggct     360

tccggacgac agccccgggg gccttctgac tgcatcccga gatttccatc agcgagtgca     420

actcataagg cagtccctaa ggggaccggg ccaccggctg aggacgggga tggcttagga     480

gctcctggac ctagggcccg gcgtcgtcgc ctcctgggcg tcgcggcaga ggggagtggc     540

ccgcgcggaa agcgccgcgg gacagtcagt gacgaggccc gggggtcgcc ggggccacga     600

cttctcggag accgtcctgc gctctctgga gacgcgctgt ccgcgcccag ggtggtgcca     660

tgtggggcgc tcgccgctcg tccgtctcct catcctggaa cgccgcttcg ctcctgcagc     720

tgctgctggc tgcgctgctg gcggcggggg cgagggccca gcggcgagta ctgccacggc     780

tggctggacg cgcagggcgt ctggcgcatc ggcttccagt gtcccgagcg cttcgacggc     840

ggcgacgcca ccatctgctg cggcagctgc gcgttgcgct actgctgctc cagcgccgag     900

gcgcgcctgg accagggcgg ctgcgacaat gaccgccagc agggcgctgg cgagcctggc     960

cgggcggaca aagacgggcc ccgacggctc ggcagggctt catgtcttag gggtacccaa     1020

ggagacggcg agggtgcgcc cccacccgtg agggcctggc agcggtgctc ccctgaaggc    1080

tccccgaaag gaaggcagct cctcagggct ttcccggggc tgctgccccg tgccagacgc    1140

cgcggattcc catcttctcc acgcggcggc ccctctcccc tgcagcggcc cgccttgccc    1200

atctacgtgc cgttcctcat tgttggctcc gtgtttgtcg cctttatcat cttggggtcc    1260

ctggtggcag cctgttgctg cagatgtctc cggcctaagc aggatcccca gcagagccga    1320

gccccagggg gtaaccgctt gatggagacc atccccatga tccccagtgc cagcacctcc    1380

cgggggtcgt cctcacgcca gtccagcaca gctgccagtt ccagctccag cgccaactcc    1440

ggggcccggg cgcccccaac aaggtcacag accaactgtt gcttgccgga agggaccatg    1500

aacaacgtgt atgtcaacat gcccacgaat ttctctgtgc tgaactgtca gcaggccacc    1560

cagattgtgc cacatcaagg gcagtatctg catcccccat acgtggggta cacggtgcag    1620

cacgactctg tgcccatgac agctgtgcca cctttcatgg acggcctgca gcctggctac    1680

aggcagattc agtccccctt ccctcacacc aacagtgaac agaagatgta cccagcggtg    1740

actgtataac cgagagtcac tggtgggttc ctttactgaa gggagacgaa ggcaggggtg 1800
gattctcgag gtggaagtcc gcacatgtcg gtggtattta tggcacgatt cctttggatg 1860
gcttcatttg cccccagact gtatgaaaac atctccgaat tagcatttct ggatatgttt 1920
catccagggt atcattgatt tatgatggaa aaccggcctc agctggagat gactgtgatg 1980
ttgctgatgg gtgtataaca aatgcttgag tccgaagtgc ccttgagata tggttgacga 2040
aagaatttta taaactgata aattaaggat ttttattatg ttgttattat tatttctttt 2100
ttgttgttga ctgcacagga tcaaaatgcc tgttatctcc cttttactgg gacttttttt 2160
tttttttttt ttttttttaa tcagacaggg tcttgctctg ttgcccaggc tggagtgcag 2220
tggtgcgatc tcggctcact gcaacttcag cctcctggat tcaggcaaca ctcctgcctc 2280
agcctcccac gtggctggga ttacaggtgc ctgcccccat ggctaatttt ttgtattttt 2340
tgtagagatg gggtttcacc atgttggctg ggctggtctc actctcctga cctcaagcaa 2400
tctgcctgtc tcagcctccc aaagtgctgg gattacaggc gtgagccacc gcccccagcc 2460
tgagcctttt tttttttcta atgcatccaa ggttaagggg aagacgcaaa taacaggact 2520
attctaaaag gaaacctgtt tgaactctgt gagatcagtc atcagtctca gtattccaca 2580
ggcacacctt aatttcattg taaaaagata tatatatttt gtctattttt gtgcttttgg 2640
gggcctattt tgtgcttttt taccttatgt agagatctta ttacaaagtg attttctaca 2700
ttaaaaagag actgaaataa attgtatagt tacttaacta atgaagacat ttcagaactc 2760
tgggatgatt ttaatcttga agtagtaggt ggtatagtca taaaaccatt catccccttc 2820
ttgattgtat cttaattttc tggctttaag gtgacatctg agaggtaatg cattcttttt 2880
tatattgaaa tcataaacta tcacccgctg cttctctgag ttacttttaa ttttgccttg 2940
tggttatggt ttggcgtttc cttctgtttg gttttcagag ccccatgtct atatagtcct 3000
gagtgcaagt aattactata cttgtaaatg aagatcagta tttctgccta gatctgataa 3060
aaaaattttc ttgtcttagt tataaaaatt caaagaaatg tgttacaaag atacttagta 3120
tagctcctca gccataacct gagacttggg atgaaattta aaccagatac gatttacttt 3180
gcagatcata aggcttttta tactcttgtt atcaaaatgg cttatttttc aggcactaag 3240
gattgttaag agaaaagctt ttcaacgaag gattgccttt cttctcccac actgttcttg 3300
atttcctctc tctttcaggc ctcaacaggc actgtattca ttgccaatgt tccaaattat 3360
caaattcaag tgaatttatt tgtgtgttct ttacttatat aaaaaaagat aactttaagg 3420
atgtgcaagt acatttccaa ctgctagcac aaccagtatt ttgtaattaa acaaatcgct 3480
gtatggtatg gtcttctaca catttatgtc tatagatatc tatcgatcat ctttctattc 3540
tgtttcatga ctgaataatg taaaaccagt gttggcaatt ggtatcatca atgatactca 3600
ttttttaata accaaaggca ggggaaaatc attttactta ttaataaata ttttatgatg 3660
tgaaaaaaaa aaaaaaaaaa aaaaaaaaaa 3690
<210> 195

<211>  582
<212>  PRT
<213>  Homo sapiens

<400>  195

<210> 196
<211> 3096
<212> DNA
<213> Homo sapiens

<400> 196
gcccttggac actgacatgg actgaaggag tagaatggag cacgaggaca ctgacatgga 60

ctgaagaaaa aggagctgga gcaggagaag gaggtgctgc tgcagggttt ggagatgatg 120

gcgcggggcc gcgactggta ccagcagcag ctgcaacgag tgcaggagcg ccagcgccgc 180

ctgggccaga gcagagccag cgccgacttt ggggctgcag ggagcccccg cccactgggg 240

cggctactgc ccaaggtaca agaggtggcc cggtgcctgg gggagctgct ggctgcagcc 300

tgtgccagcc gggccctgcc cccgtcctcc tccgggcccc cctgccctgc cctgacgtcc 360

acctcacccc cggtctggca gcagcagacc atcctcatgc tgaaggagca gaaccgactc 420

ctcacccagg aggtgaccga gaagagtgag cgcatcacgc agctggagca ggagaagtcg 480
gcgctcatta agcagctgtt tgaggcccgc gccctgagcc agcaggacgg gggacctctg 540
gattccacct tcatctagtc cttgtgggcc gcgtgggccc ccagggccag cctggcactc 600
agcccttcga gggtgggcgc cccatcgcac ccaccctctc tggctggaga cccccggcag 660
gcccaggcac agtcccggag tgggcgcctt cctgccgccc ttgccagatg ggctccccag 720
gcctgccccc ggctggtccc cgcaccgagc gcttgactcc gtttkggctc ctggttgytg 780
acatgggctg ggggctctct tgagtccgca tagtccgcag ctactactgg ccgctgtcag 840
tggacagtgg ggtacccctc catgagttag cgtccccccg tttccagcgg tgccgccctg 900
ggtcccatct tcagggaaag gcactgccca cgccaggctg cacttccaac aacgggcagc 960
agagggcgcg gggcggctcc gacgcgggtc caagggcagc ttcccgctca accagggcac 1020
caggacgagg tggctgtagc tcggacggac ggaagtagat ggagggggtg gggacggcct 1080
gtaagcgggg ggtgcctgcc tggctgggga gccccaggga tagcggtcgg acttcaggtt 1140
ctggccaagg ctgagggacc ctggctgcag cggatcggca cgccgggtgg gcgagagctt 1200
ggcctgcatg tgcctcccac agaccctggg gtgatggcct tccccctctt ggccgggacg 1260
ttgccccacg ttgagtccca cacaacatcc tgtgagcctg gctccccagg agggccccca 1320
gacagctccc aggcacgtca taggcaaagc ctgtttcccc cgactcagga tttccaaggc 1380
ctggggtcct gctcaccccc ctttgctctc acgcccagcc tgtccccagg tttcagctgg 1440
gagaggccac ctccctcagc caaggaaaac gagaaccccc agggtacagg aggaggctgg 1500
ggcaggtccc cttgggtgtc actccctcag cccctgccca ggcccactcc cgctggtgct 1560
ggagtacgca ctggtggggg ggccctgctc agcccaacct ggagggtccc agtgtcacca 1620
gaaccagggg cacggcaaca gcatcgatgg gttctgcagc ccagggcccc cgatgcgggg 1680
tcagtgtgtg tggggcgcag ggcctccgat gcggggtcag tgcgtggggg gcgcagggcc 1740
cccgatgcgg ggtcagtgcg tggggggcgc agggccccct cgtgtccagg gcactttggt 1800
acactgtccc acaaggcacc tgtctcagag gaggggccct ggcaggcagc gtggcaactc 1860
ccttccggag cccagctcca tgctaacctg cccacagcaa ccccacagag ccacattccc 1920
tgctgcacct ggtctgcagg ggtgtcccag gacaggccca agtcagccca gcatgcagct 1980
gccctcctac cctgaagatg ggagtgggct ttccagggga cataaggatg tcaggcctgg 2040
acctcctggg caggaaaggg tgcaggtcct gagggcctgt gccccacagc cccagcaccc 2100
aggtggactg cagcgcagtg ggtgggccag tggcagccag ggagaagccc cccgtcagca 2160
ggctggggtc tgcccaccag ggcctcccca cgtctgcctt tgagggtgcc tgccatgccc 2220
tgggggatcc tggcatcttt actggactgg aagcaggaga cagaacagtg tctgtcccgg 2280
ggtgacttca tcaggagacc gcccacatag agctggaccc cgcagctgaa gcggaaatgt 2340
gagacaggct ggcacctccg gaaaaactgc ctttcagcct tggtgttccg tgcaaggtga 2400
aaagaaatag gtcctcccag tttacagctt gaaatcaggc tagtgagtgg ccctggagac 2460

cacgagggga gaatttaaag gccccggctg gcagggtcta ggtggctggc agaggcacat 2520

gcagaccctg cctggagcct gccctaggac gctgggcggg tcagtctccg tgcaggatgt 2580

gagcagcgtc cctgggctct atccgcgagg tgccagtagc gtgtgcaggt acatacacgt 2640

gcgtgcacac tgtgatgaca cccggaaatg tctcaggatg ttgaaatgtg tccttggggg 2700

cagaagtgtc cccagttgag aatctgcccc agaggaacac acccacacca ggcctcagga 2760

ttttgtgttg atcaagttcc aaggaaaagg aacatctcag ccgggcgtgg tggttcacgc 2820

ctggaatccc agcacttgag gccaggagtt ccagagcagc ctgggcaacg cagtgagaga 2880

ccccatctct acaaraaaaa aaaaagaaag aaagaaaatg agagatccag gtttaaaaat 2940

tcataaacac cacaaggaaa caatacacta tgagacccag cagaagcaac agattgactc 3000

tagacccaga tactagaatt atcagagaga atataaagta acagtgtttt atatatctaa 3060

agaaataaaa gagatttctg gaaacatgaa aaaaaa 3096

<210> 197
<211> 3171
<212> DNA
<213> Homo sapiens

<400> 197
gcggaacacc ggcccgccgt cgcggcagct gcttcacccc tctctctgca gccatggggc 60

tccctcgtgg acctctcgcg tctctcctcc ttctccaggt ttgctggctg cagtgcgcgg 120

cctccgagcc gtgccgggcg gtcttcaggg aggctgaagt gaccttggag gcgggaggcg 180

cggagcagga gcccggccag gcgctgggga aagtattcat gggctgccct gggcaagagc 240

cagctctgtt tagcactgat aatgatgact tcactgtgcg gaatggcgag acagtccagg 300

aaagaaggtc actgaaggaa aggaatccat tgaagatctt cccatccaaa cgtatcttac 360

gaagacacaa gagagattgg gtggttgctc caatatctgt ccctgaaaat ggcaagggtc 420

ccttccccca gagactgaat cagctcaagt ctaataaaga tagagacacc aagattttct 480

acagcatcac ggggccgggg gcagacagcc cccctgaggg tgtcttcgct gtagagaagg 540

agacaggctg gttgttgttg aataagccac tggaccggga ggagattgcc aagtatgagc 600

tctttggcca cgctgtgtca gagaatggtg cctcagtgga ggaccccatg aacatctcca 660

tcatcgtgac cgaccagaat gaccacaagc ccaagtttac ccaggacacc ttccgaggga 720

gtgtcttaga gggagtccta ccaggtactt ctgtgatgca ggtgacagcc acagatgagg 780

atgatgccat ctacacctac aatggggtgg ttgcttactc catccatagc caagaaccaa 840

aggacccaca cgacctcatg ttcacaattc accggagcac aggcaccatc agcgtcatct 900

ccagtggcct ggaccgggaa aaagtccctg agtacacact gaccatccag gccacagaca 960

tggatgggga cggctccacc accacggcag tggcagtagt ggagatcctt gatgccaatg 1020

acaatgctcc catgtttgac ccccagaagt acgaggccca tgtgcctgag aatgcagtgg 1080

gccatgaggt gcagaggctg acggtcactg atctggacgc ccccaactca ccagcgtggc 1140

gtgccaccta ccttatcatg ggcggtgacg acggggacca ttttaccatc accacccacc 1200

ctgagagcaa ccagggcatc ctgacaacca ggaagggttt ggattttgag gccaaaaacc 1260
agcacaccct gtacgttgaa gtgaccaacg aggccccttt tgtgctgaag ctcccaacct 1320
ccacagccac catagtggtc cacgtggagg atgtgaatga ggcacctgtg tttgtcccac 1380
cctccaaagt cgttgaggtc caggagggca tccccactgg ggagcctgtg tgtgtctaca 1440
ctgcagaaga ccctgacaag gagaatcaaa agatcagcta ccgcatcctg agagacccag 1500
cagggtggct agccatggac ccagacagtg ggcaggtcac agctgtgggc accctcgacc 1560
gtgaggatga gcagtttgtg aggaacaaca tctatgaagt catggtcttg gccatggaca 1620
atggaagccc tcccaccact ggcacgggaa cccttctgct aacactgatt gatgtcaacg 1680
accatggccc agtccctgag ccccgtcaga tcaccatctg caaccaaagc cctgtgcgcc 1740
acgtgctgaa catcacggac aaggacctgt ctccccacac ctcccctttc caggcccagc 1800
tcacagatga ctcagacatc tactggacgg cagaggtcaa cgaggaaggt gacacagtgg 1860
tcttgtccct gaagaagttc ctgaagcagg atacatatga cgtgcacctt tctctgtctg 1920
accatggcaa caaagagcag ctgacggtga tcagggccac tgtgtgcgac tgccatggcc 1980
atgtcgaaac ctgccctgga ccctggaaag gaggtttcat cctccctgtg ctgggggctg 2040
tcctggctct gctgttcctc ctgctggtgc tgcttttgtt ggtgagaaag aagcggaaga 2100
tcaaggagcc cctcctactc ccagaagatg acacccgtga caacgtcttc tactatggcg 2160
aagagggggg tggcgaagag gaccaggact atgacatcac ccagctccac cgaggtctgg 2220
aggccaggcc ggaggtggtt ctccgcaatg acgtggcacc aaccatcatc ccgacaccca 2280
tgtaccgtcc taggccagcc aacccagatg aaatcggcaa ctttataatt gagaacctga 2340
aggcggctaa cacagacccc acagccccgc cctacgacac cctcttggtg ttcgactatg 2400
agggcagcgg ctccgacgcc gcgtccctga gctccctcac ctcctccgcc tccgaccaag 2460
accaagatta cgattatctg aacgagtggg gcagccgctt caagaagctg gcagacatgt 2520
acggtggcgg ggaggacgac taggcggcct gcctgcaggg ctggggacca aacgtcaggc 2580
cacagagcat ctccaagggg tctcagttcc cccttcagct gaggacttcg gagcttgtca 2640
ggaagtggcc gtagcaactt ggcggagaca ggctatgagt ctgacgttag agtggttgct 2700
tccttagcct ttcaggatgg aggaatgtgg gcagtttgac ttcagcactg aaaacctctc 2760
cacctgggcc agggttgcct cagaggccaa gtttccagaa gcctcttacc tgccgtaaaa 2820
tgctcaaccc tgtgtcctgg gcctgggcct gctgtgactg acctacagtg gactttctct 2880
ctggaatgga accttcttag gcctcctggt gcaacttaat tttttttttt aatgctatct 2940
tcaaaacgtt agagaaagtt cttcaaaagt gcagcccaga gctgctgggc ccactggccg 3000
tcctgcattt ctggtttcca gaccccaatg cctcccattc ggatggatct ctgcgttttt 3060
atactgagtg tgcctaggtt gccccttatt ttttattttc cctgttgcgt tgctatagat 3120
gaagggtgag gacaatcgtg tatatgtact agaacttttt tattaaagaa a 3171
<210> 198

<211>  829
<212>  PRT
<213>  Homo sapiens

<400>  198

<210>  199
<211>  1148
<212>  DNA
<213>  Homo sapiens

<400>  199
cttctttaaa tttctttcta ggatgttcac ttcttctcca caatgaatga gtgtcactat      60

gacaagcaca tggacttttt ttataatagg agcaacactg atactgtcga tgactggaca     120

ggaacaaagc ttgtgattgt tttgtgtgtt gggacgtttt tctgcctgtt tatttttttt     180

tctaattctc tggtcatcgc ggcagtgatc aaaaacagaa aatttcattt ccccttctac     240

tacctgttgg ctaatttagc tgctgccgat ttcttcgctg gaattgccta tgtattcctg     300

atgtttaaca caggcccagt ttcaaaaact ttgactgtca accgctggtt tctccgtcag     360

gggcttctgg acagtagctt gactgcttcc ctcaccaact tgctggttat cgccgtggag     420

aggcacatgt caatcatgag gatgcgggtc catagcaacc tgaccaaaaa gagggtgaca     480

ctgctcattt tgcttgtctg ggccatcgcc atttttatgg gggcggtccc cacactgggc     540

tggaattgcc tctgcaacat ctctgcctgc tcttccctgg cccccattta cagcaggagt     600

taccttgttt tctggacagt gtccaacctc atggccttcc tcatcatggt tgtggtgtac     660

ctgcggatct acgtgtacgt caagaggaaa accaacgtct tgtctccgca tacaagtggg     720

tccatcagcc gccggaggac acccatgaag ctaatgaaga cggtgatgac tgtcttaggg     780

gcgtttgtgg tatgctggac cccgggcctg gtggttctgc tcctcgacgg cctgaactgc     840

aggcagtgtg gcgtgcagca tgtgaaaagg tggttcctgc tgctggcgct gctcaactcc     900

gtcgtgaacc ccatcatcta ctcctacaag gacgaggaca tgtatggcac catgaagaag     960

atgatctgct gcttctctca ggagaaccca gagaggcgtc cctctcgcat cccctccaca    1020

gtcctcagca ggagtgacac aggcagccag tacatagagg atagtattag ccaaggtgca    1080

gtctgcaata aaagcacttc ctaaactctg gatgcctctc ggcccaccca ggtgatgact 1140

gtcttagg 1148

<210> 200
<211> 353
<212> PRT
<213> Homo sapiens

<400> 200

Met Asn Glu Cys His Tyr Asp Lys His Met Asp Phe Phe Tyr Asn Arg
1               5                   10                  15

Ser Asn Thr Asp Thr Val Asp Asp Trp Thr Gly Thr Lys Leu Val Ile
            20                  25                  30

Val Leu Cys Val Gly Thr Phe Phe Cys Leu Phe Ile Phe Phe Ser Asn
        35                  40                  45

Ser Leu Val Ile Ala Ala Val Ile Lys Asn Arg Lys Phe His Phe Pro
    50                  55                  60

Phe Tyr Tyr Leu Leu Ala Asn Leu Ala Ala Ala Asp Phe Phe Ala Gly
65                  70                  75                  80

Ile Ala Tyr Val Phe Leu Met Phe Asn Thr Gly Pro Val Ser Lys Thr
                85                  90                  95

Leu Thr Val Asn Arg Trp Phe Leu Arg Gln Gly Leu Leu Asp Ser Ser
            100                 105                 110

Leu Thr Ala Ser Leu Thr Asn Leu Leu Val Ile Ala Val Glu Arg His
        115                 120                 125

Met Ser Ile Met Arg Met Arg Val His Ser Asn Leu Thr Lys Lys Arg
    130                 135                 140

Val Thr Leu Leu Ile Leu Leu Val Trp Ala Ile Ala Ile Phe Met Gly
145                 150                 155                 160

Ala Val Pro Thr Leu Gly Trp Asn Cys Leu Cys Asn Ile Ser Ala Cys
                165                 170                 175

Ser Ser Leu Ala Pro Ile Tyr Ser Arg Ser Tyr Leu Val Phe Trp Thr
            180                 185                 190

Val Ser Asn Leu Met Ala Phe Leu Ile Met Val Val Val Tyr Leu Arg
        195                 200                 205

Ile Tyr Val Tyr Val Lys Arg Lys Thr Asn Val Leu Ser Pro His Thr
    210                 215                 220

Ser Gly Ser Ile Ser Arg Arg Arg Thr Pro Met Lys Leu Met Lys Thr
225                 230                 235                 240

Val Met Thr Val Leu Gly Ala Phe Val Val Cys Trp Thr Pro Gly Leu
                245                 250                 255

Val Val Leu Leu Leu Asp Gly Leu Asn Cys Arg Gln Cys Gly Val Gln
            260                 265                 270

His Val Lys Arg Trp Phe Leu Leu Leu Ala Leu Leu Asn Ser Val Val
        275                 280                 285

Asn Pro Ile Ile Tyr Ser Tyr Lys Asp Glu Asp Met Tyr Gly Thr Met
    290                 295                 300

Lys Lys Met Ile Cys Cys Phe Ser Gln Glu Asn Pro Glu Arg Arg Pro
305                 310                 315                 320

Ser Arg Ile Pro Ser Thr Val Leu Ser Arg Ser Asp Thr Gly Ser Gln
                325                 330                 335

Tyr Ile Glu Asp Ser Ile Ser Gln Gly Ala Val Cys Asn Lys Ser Thr
            340                 345                 350

Ser

<210>  201
<211>  892
<212>  DNA
<213>  Homo sapiens

<400>  201
atgacctaca gttactcatt tttcaggcct gagttgatcg ttaatcatct taattatgtt      60

cattctgaag ccaacaggag aaccaagacc aaaactttat tgtctctgct ttcatttctt     120

gatgaaacct ctggactaag cacacatctt ccttgtttat ctctctcaaa ggagtgtgga     180

gtgcttcatc tggacatcca cgggaagaag gaagacatga gaatcaccca acagtcttcc     240

cagctatacc tgtgggacat gggtggtttt acaatattta agaacctgtg gatgagcctc     300

atacccagag ggaacaaacg ctccccaaaa agagttacag aaaccatcct gagagatttt     360

aagcagaagc aaagttcaaa gatccaagag gagagacgaa gagagtctgc aggaccaaac     420

ctctcttcat tctggtttgt ggggaatgct ggaagaggag acaggcccca gatttgggca     480

ggaagtaaac agttttcagg ctgaggccaa tctgagcagg aacattccaa tatttcttca     540

gctacgttgt cccagcactt cactggttaa ccttttatgt ccaccatttg tggatttcac     600

agctacttgt caatggtgaa tattgatcat catcattatc tactgagctg ctaccatatc     660

ccagctactc cttgcatgtt gttcattatt ttctcaacac tcagcatatt tgcaatatgt     720

tatgtaatat cacagacaag gaaactgaac gcagaaatgt tttatttctt gccaaacatc     780

acatgaggat gaacaatgaa accgatttga aaccaggatt gtctgattcc aacatctctg     840

ggtccttttt cactctgata tgctgcaatt aaaaagccat ttctaagact gt             892

<210>  202
<211>  167
<212>  PRT
<213>  Homo sapiens

<400>  202

<210>  203
<211>  2582
<212>  DNA
<213>  Homo sapiens

<220>
<221>  misc_feature
<222>  (2553)..(2553)
<223>  n is a, c, g, or t

<220>
<221>  misc_feature
<222>  (2556)..(2556)
<223>  n is a, c, g, or t

<400>  203
tcacacgtgc caaggggctg gctcagcgga accagcctgc acgcgctggc tccgggtgac      60

agccgcgcgc ctcggccagg atctgagtga tgagacgtgt ccccactgag gtgccccaca     120

gcagcaggtg ttgagcatgg gctgagaagc tggaccggca ccaaagggct ggcagaaatg     180

ggcgcctggc tgattcctag gcagttggcg gcagcaagga ggagaggccg cagcttctgg     240

agcagagccg agacgaagca gttctggagt gcctgaacgg ccccctgagc cctacccgcc     300

tggcccacta tggtccagag gctgtgggtg agccgcctgc tgcggcaccg gaaagcccag     360

ctcttgctgg tcaacctgct aacctttggc ctggaggtgt gtttggccgc aggcatcacc     420

tatgtgccgc ctctgctgct ggaagtgggg gtagaggaga agttcatgac catggtgctg     480

ggcattggtc cagtgctggg cctggtctgt gtcccgctcc taggctcagc cagtgaccac     540

tggcgtggac gctatggccg ccgccggccc ttcatctggg cactgtcctt gggcatcctg     600

ctgagcctct ttctcatccc aagggccggc tggctagcag ggctgctgtg cccggatccc     660

aggcccctgg agctggcact gctcatcctg ggcgtggggc tgctggactt ctgtggccag     720

gtgtgcttca ctccactgga ggccctgctc tctgacctct tccgggaccc ggaccactgt     780

cgccaggcct actctgtcta tgccttcatg atcagtcttg ggggctgcct gggctacctc     840

ctgcctgcca ttgactggga caccagtgcc ctggccccct acctgggcac ccaggaggag     900

tgcctctttg gcctgctcac cctcatcttc ctcacctgcg tagcagccac actgctggtg     960

gctgaggagg cagcgctggg ccccaccgag ccagcagaag ggctgtcggc cccctccttg 1020
tcgccccact gctgtccatg ccgggcccgc ttggctttcc ggaacctggg cgccctgctt 1080
ccccggctgc accagctgtg ctgccgcatg ccccgcaccc tgcgccggct cttcgtggct 1140
gagctgtgca gctggatggc actcatgacc ttcacgctgt tttacacgga tttcgtgggc 1200
gaggggctgt accagggcgt gcccagagct gagccgggca ccgaggcccg gagacactat 1260
gatgaaggcg ttcggatggg cagcctgggg ctgttcctgc agtgcgccat ctccctggtc 1320
ttctctctgg tcatggaccg gctggtgcag cgattcggca ctcgagcagt ctatttggcc 1380
agtgtggcag ctttccctgt ggctgccggt gccacatgcc tgtcccacag tgtggccgtg 1440
gtgacagctt cagccgccct caccgggttc accttctcag ccctgcagat cctgccctac 1500
acactggcct ccctctacca ccgggagaag caggtgttcc tgcccaaata ccgaggggac 1560
actggaggtg ctagcagtga ggacagcctg atgaccagct tcctgccagg ccctaagcct 1620
ggagctccct tccctaatgg acacgtgggt gctggaggca gtggcctgct cccacctcca 1680
cccgcgctct gcggggcctc tgcctgtgat gtctccgtac gtgtggtggt gggtgagccc 1740
accgaggcca gggtggttcc gggccggggc atctgcctgg acctcgccat cctggatagt 1800
gccttcctgc tgtcccaggt ggccccatcc ctgtttatgg gctccattgt ccagctcagc 1860
cagtctgtca ctgcctatat ggtgtctgcc gcaggcctgg gtctggtcgc catttacttt 1920
gctacacagg tagtatttga caagagcgac ttggccaaat actcagcgta gaaaacttcc 1980
agcacattgg ggtggagggc ctgcctcact gggtcccagc tccccgctcc tgttagcccc 2040
atggggctgc cgggctggcc gccagtttct gttgctgcca aagtaatgtg gctctctgct 2100
gccaccctgt gctgctgagg tgcgtagctg cacagctggg ggctggggcg tccctctcct 2160
ctctccccag tctctagggc tgcctgactg gaggccttcc aagggggttt cagtctggac 2220
ttatacaggg aggccagaag ggctccatgc actggaatgc ggggactctg caggtggatt 2280
acccaggctc agggttaaca gctagcctcc tagttgagac acacctagag aagggttttt 2340
gggagctgaa taaactcagt cacctggttt cccatctcta agccccttaa cctgcagctt 2400
cgtttaatgt agctcttgca tgggagtttc taggatgaaa cactcctcca tgggatttga 2460
acatatgaaa gttatttgta ggggaagagt cctgaggggc aacacacaag aaccaggtcc 2520
cctcagcccc acaggcactg gtcttttttg ctngantcca ccccccccct ctttaccctt 2580
tt 2582

<210> 204
<211> 553
<212> PRT
<213> Homo sapiens

<400> 204

Met Val Gln Arg Leu Trp Val Ser Arg Leu Leu Arg His Arg Lys Ala
1               5                   10                  15

Gln Leu Leu Leu Val Asn Leu Leu Thr Phe Gly Leu Glu Val Cys Leu
            20                  25                  30

Ala Ala Gly Ile Thr Tyr Val Pro Pro Leu Leu Leu Glu Val Gly Val
        35                  40                  45

Glu Glu Lys Phe Met Thr Met Val Leu Gly Ile Gly Pro Val Leu Gly
    50                  55                  60

Leu Val Cys Val Pro Leu Leu Gly Ser Ala Ser Asp His Trp Arg Gly
65                  70                  75                  80

Arg Tyr Gly Arg Arg Arg Pro Phe Ile Trp Ala Leu Ser Leu Gly Ile
                85                  90                  95

Leu Leu Ser Leu Phe Leu Ile Pro Arg Ala Gly Trp Leu Ala Gly Leu
            100                 105                 110

Leu Cys Pro Asp Pro Arg Pro Leu Glu Leu Ala Leu Leu Ile Leu Gly
        115                 120                 125

Val Gly Leu Leu Asp Phe Cys Gly Gln Val Cys Phe Thr Pro Leu Glu
    130                 135                 140

Ala Leu Leu Ser Asp Leu Phe Arg Asp Pro Asp His Cys Arg Gln Ala
145                 150                 155                 160

Tyr Ser Val Tyr Ala Phe Met Ile Ser Leu Gly Gly Cys Leu Gly Tyr
                165                 170                 175

Leu Leu Pro Ala Ile Asp Trp Asp Thr Ser Ala Leu Ala Pro Tyr Leu
            180                 185                 190

Gly Thr Gln Glu Glu Cys Leu Phe Gly Leu Leu Thr Leu Ile Phe Leu
        195                 200                 205

Thr Cys Val Ala Ala Thr Leu Leu Val Ala Glu Glu Ala Ala Leu Gly
    210                 215                 220

Pro Thr Glu Pro Ala Glu Gly Leu Ser Ala Pro Ser Leu Ser Pro His
225                 230                 235                 240

Cys Cys Pro Cys Arg Ala Arg Leu Ala Phe Arg Asn Leu Gly Ala Leu
                245                 250                 255

Leu Pro Arg Leu His Gln Leu Cys Cys Arg Met Pro Arg Thr Leu Arg
            260                 265                 270

Arg Leu Phe Val Ala Glu Leu Cys Ser Trp Met Ala Leu Met Thr Phe
        275                 280                 285

Thr Leu Phe Tyr Thr Asp Phe Val Gly Glu Gly Leu Tyr Gln Gly Val
    290                 295                 300

Pro Arg Ala Glu Pro Gly Thr Glu Ala Arg Arg His Tyr Asp Glu Gly
305                 310                 315                 320

Val Arg Met Gly Ser Leu Gly Leu Phe Leu Gln Cys Ala Ile Ser Leu
                325                 330                 335

Val Phe Ser Leu Val Met Asp Arg Leu Val Gln Arg Phe Gly Thr Arg
            340                 345                 350

Ala Val Tyr Leu Ala Ser Val Ala Ala Phe Pro Val Ala Ala Gly Ala
        355                 360                 365

Thr Cys Leu Ser His Ser Val Ala Val Val Thr Ala Ser Ala Ala Leu
    370                 375                 380

Thr Gly Phe Thr Phe Ser Ala Leu Gln Ile Leu Pro Tyr Thr Leu Ala

385                 390                 395                 400

Ser Leu Tyr His Arg Glu Lys Gln Val Phe Leu Pro Lys Tyr Arg Gly
                405                 410                 415

Asp Thr Gly Gly Ala Ser Ser Glu Asp Ser Leu Met Thr Ser Phe Leu
            420                 425                 430

Pro Gly Pro Lys Pro Gly Ala Pro Phe Pro Asn Gly His Val Gly Ala
        435                 440                 445

Gly Gly Ser Gly Leu Leu Pro Pro Pro Pro Ala Leu Cys Gly Ala Ser
    450                 455                 460

Ala Cys Asp Val Ser Val Arg Val Val Val Gly Glu Pro Thr Glu Ala
465                 470                 475                 480

Arg Val Val Pro Gly Arg Gly Ile Cys Leu Asp Leu Ala Ile Leu Asp
                485                 490                 495

Ser Ala Phe Leu Leu Ser Gln Val Ala Pro Ser Leu Phe Met Gly Ser
            500                 505                 510

Ile Val Gln Leu Ser Gln Ser Val Thr Ala Tyr Met Val Ser Ala Ala
        515                 520                 525

Gly Leu Gly Leu Val Ala Ile Tyr Phe Ala Thr Gln Val Val Phe Asp
    530                 535                 540

Lys Ser Asp Leu Ala Lys Tyr Ser Ala
545                 550

<210>  205
<211>  1593
<212>  DNA
<213>  Homo sapiens

<400>  205
atggttcgcc gggggctgct tgcgtggatt tcccgggtgg tggttttgct ggtgctcctc      60

tgctgtgcta tctctgtcct gtacatgttg gcctgcaccc caaaaggtga cgaggagcag     120

ctggcactgc ccagggccaa cagccccacg gggaaggagg ggtaccaggc cgtccttcag     180

gagtgggagg agcagcaccg caactacgtg agcagcctga agcggcagat cgcacagctc     240

aaggaggagc tgcaggagag gagtgagcag ctcaggaatg ggcagtacca agccagcgat     300

gctgctggcc tgggtctgga caggagcccc ccagagaaaa cccaggccga cctcctggcc     360

ttcctgcact cgcaggtgga caaggcagag gtgaatgctg gcgtcaagct ggccacagag     420

tatgcagcag tgcctttcga tagctttact ctacagaagg tgtaccagct ggagactggc     480

cttacccgcc accccgagga gaagcctgtg aggaaggaca agcgggatga gttggtggaa     540

gccattgaat cagccttgga gaccctgaac aatcctgcag agaacagccc caatcaccgt     600

ccttacacgg cctctgattt catagaaggg atctaccgaa cagaaaggga caaagggaca     660

ttgtatgagc tcaccttcaa aggggaccac aaacacgaat tcaaacggct catcttattt     720

cgaccattcg gccccatcat gaaagtgaaa aatgaaaagc tcaacatggc caacacgctt     780

atcaatgtta tcgtgcctct agcaaaaagg gtggacaagt tccggcagtt catgcagaat     840

ttcagggaga tgtgcattga gcaggatggg agagtccatc tcactgttgt ttactttggg     900

aaagaagaaa taaatgaagt caaaggaata cttgaaaaca cttccaaagc tgccaacttc 960

aggaacttta ccttcatcca gctgaatgga gaattttctc ggggaaaggg acttgatgtt 1020

ggagcccgct tctggaaggg aagcaacgtc cttctctttt tctgtgatgt ggacatctac 1080

ttcacatctg aattcctcaa tacgtgtagg ctgaatacac agccagggaa gaaggtattt 1140

tatccagttc ttttcagtca gtacaatcct ggcataatat acggccacca tgatgcagtc 1200

cctcccttgg aacagcagct ggtcataaag aaggaaactg gattttggag agactttgga 1260

tttgggatga cgtgtcagta tcggtcagac ttcatcaata taggtgggtt tgatctggac 1320

atcaaaggct ggggcggaga ggatgtgcac ctttatcgca agtatctcca cagcaacctc 1380

atagtggtac ggacgcctgt gcgaggactc ttccacctct ggcatgagaa gcgctgcatg 1440

gacgagctga cccccgagca gtacaagatg tgcatgcagt ccaaggccat gaacgaggca 1500

tcccacggcc agctgggcat gctggtgttc aggcacgaga tagaggctca ccttcgcaaa 1560

cagaaacaga agacaagtag caaaaaaaca tga 1593

<210> 206
<211> 530
<212> PRT
<213> Homo sapiens

<400> 206

<210>  207
<211>  2712

<212>  DNA
<213>  Homo sapiens

<400>  207
atgattcctg tattgacatc aaaaaaagca agtgaattac cagtcagtga agttgcaagc      60

attctccaag ctgatcttca gaatggtcta aacaaatgtg aagttagtca taggcgagcc     120

tttcatggct ggaatgagtt tgatattagt gaagatgagc cactgtggaa gaagtatatt     180

tctcagttta aaaatcccct tattatgctg cttctggctt ctgcagtcat cagtgtttta     240

atgcatcagt ttgatgatgc cgtcagtatc actgtggcaa tacttatcgt tgttacagtt     300

gcctttgttc aggaatatcg ttcagaaaaa tctcttgaag aattgagtaa acttgtgcca     360

ccagaatgcc attgtgtgcg tgaaggaaaa ttggagcata cacttgcccg agacttggtt     420

ccaggtgata cagtttgcct ttctgttggg gatagagttc ctgctgactt acgcttgttt     480

gaggctgtgg atctttccat tgatgagtcc agcttgacag gtgagacaac gccttgttct     540

aaggtgacag ctcctcagcc agctgcaact aatggagatc ttgcatcgag aagtaacatt     600

gcctttatgg gaacactggt cagatgtggc aaagcaaagg gtgttgtcat tggaacagga     660

gaaaattctg aatttgggga ggtttttaaa atgatgcaag cagaagaggc accaaaaacc     720

cctctgcaga agagcatgga cctcttagga aaacaacttt ccttttactc ctttggtata     780

ataggaatca tcatgttggt tggctggtta ctgggaaaag atatcctgga aatgtttact     840

attagtgtaa gtttggctgt agcagcaatt cctgaaggtc tccccattgt ggtcacagtg     900

acgctagctc ttggtgttat gagaatggtg aagaaaaggg ccattgtgaa aaagctgcct     960

attgttgaaa ctctgggctg ctgtaatgtg atttgttcag ataaaactgg aacactgacg    1020

aagaatgaaa tgactgttac tcacatattt acttcagatg gtctgcatgc tgaggttact    1080

ggagttggct ataatcaatt tggggaagtg attgttgatg gtgatgttgt tcatggattc    1140

tataacccag ctgttagcag aattgttgag gcgggctgtg tgtgcaatga tgctgtaatt    1200

agaaacaata ctctaatggg gaagccaaca gaaggggcct taattgctct tgcaatgaag    1260

atgggtcttg atggacttca acaagactac atcagaaaag ctgaataccc ttttagctct    1320

gagcaaaagt ggatggctgt taagtgtgta caccgaacac agcaggacag accagagatt    1380

tgttttatga aaggtgctta cgaacaagta attaagtact gtactacata ccagagcaaa    1440

gggcagacct tgacacttac tcagcagcag agagatgtgt accaacaaga gaaggcacgc    1500

atgggctcag cgggactcag agttcttgct ttggcttctg gtcctgaact gggacagctg    1560

acatttcttg gcttggtggg aatcattgat ccacctagaa ctggtgtgaa agaagctgtt    1620

acaacactca ttgcctcagg agtatcaata aaaatgatta ctggagattc acaggagact    1680

gcagttgcaa tcgccagtcg tctgggattg tattccaaaa cttcccagtc agtctcagga    1740

gaagaaatag atgcaatgga tgttcagcag ctttcacaaa tagtaccaaa ggttgcagta    1800

ttttacagag ctagcccaag gcacaagatg aaaattatta agtcgctaca gaagaacggt    1860

tcagttgtag ccatgacagg agatggagta aatgatgcag ttgctctgaa ggctgcagac    1920

attggagttg cgatgggcca gactggtaca gatgtttgca aagaggcagc agacatgatc 1980

ctagtggatg atgattttca aaccataatg tctgcaatcg aagagggtaa agggatttat 2040

aataacatta aaaatttcgt tagattccag ctgagcacga gtatagcagc attaacttta 2100

atctcattgg ctacattaat gaactttcct aatcctctca atgccatgca gattttgtgg 2160

atcaatatta ttatggatgg acccccagct cagagccttg gagtagaacc agtggataaa 2220

gatgtcattc gtaaacctcc tcgcaactgg aaagacagca ttttgactaa aaacttgata 2280

cttaaaatac ttgtttcatc aataatcatt gtttgtggga ctttgtttgt cttctggcgt 2340

gagctacgag acaatgtgat tacacctcga gacacaacaa tgaccttcac atgctttgtg 2400

ttttttgaca tgttcaatgc actaagttcc agatcccaga ccaagtctgt gtttgagatt 2460

ggactctgca gtaatagaat gttttgctat gcagttcttg gatccatcat gggacaatta 2520

ctagttattt actttcctcc gcttcagaag gtttttcaga ctgagagcct aagcatactg 2580

gatctgttgt ttcttttggg tctcacctca tcagtgtgca tagtggcaga aattataaag 2640

aaggttgaaa ggagcaggga aaagatccag aagcatgtta gttcgacatc atcatctttt 2700

cttgaagtat ga 2712

<210> 208
<211> 903
<212> PRT
<213> Homo sapiens

<400> 208

Met Ile Pro Val Leu Thr Ser Lys Lys Ala Ser Glu Leu Pro Val Ser
1               5                   10                  15

Glu Val Ala Ser Ile Leu Gln Ala Asp Leu Gln Asn Gly Leu Asn Lys
            20                  25                  30

Cys Glu Val Ser His Arg Arg Ala Phe His Gly Trp Asn Glu Phe Asp
        35                  40                  45

Ile Ser Glu Asp Glu Pro Leu Trp Lys Lys Tyr Ile Ser Gln Phe Lys
    50                  55                  60

Asn Pro Leu Ile Met Leu Leu Leu Ala Ser Ala Val Ile Ser Val Leu
65                  70                  75                  80

Met His Gln Phe Asp Asp Ala Val Ser Ile Thr Val Ala Ile Leu Ile
                85                  90                  95

Val Val Thr Val Ala Phe Val Gln Glu Tyr Arg Ser Glu Lys Ser Leu
            100                 105                 110

Glu Glu Leu Ser Lys Leu Val Pro Pro Glu Cys His Cys Val Arg Glu
        115                 120                 125

Gly Lys Leu Glu His Thr Leu Ala Arg Asp Leu Val Pro Gly Asp Thr
    130                 135                 140

Val Cys Leu Ser Val Gly Asp Arg Val Pro Ala Asp Leu Arg Leu Phe
145                 150                 155                 160

Glu Ala Val Asp Leu Ser Ile Asp Glu Ser Ser Leu Thr Gly Glu Thr
                165                 170                 175

Thr Pro Cys Ser Lys Val Thr Ala Pro Gln Pro Ala Ala Thr Asn Gly
            180                 185                 190

Asp Leu Ala Ser Arg Ser Asn Ile Ala Phe Met Gly Thr Leu Val Arg
        195                 200                 205

Cys Gly Lys Ala Lys Gly Val Val Ile Gly Thr Gly Glu Asn Ser Glu
    210                 215                 220

Phe Gly Glu Val Phe Lys Met Met Gln Ala Glu Glu Ala Pro Lys Thr
225                 230                 235                 240

Pro Leu Gln Lys Ser Met Asp Leu Leu Gly Lys Gln Leu Ser Phe Tyr
                245                 250                 255

Ser Phe Gly Ile Ile Gly Ile Ile Met Leu Val Gly Trp Leu Leu Gly
            260                 265                 270

Lys Asp Ile Leu Glu Met Phe Thr Ile Ser Val Ser Leu Ala Val Ala
        275                 280                 285

Ala Ile Pro Glu Gly Leu Pro Ile Val Val Thr Val Thr Leu Ala Leu
    290                 295                 300

Gly Val Met Arg Met Val Lys Lys Arg Ala Ile Val Lys Lys Leu Pro
305                 310                 315                 320

Ile Val Glu Thr Leu Gly Cys Cys Asn Val Ile Cys Ser Asp Lys Thr
                325                 330                 335

Gly Thr Leu Thr Lys Asn Glu Met Thr Val Thr His Ile Phe Thr Ser
            340                 345                 350

Asp Gly Leu His Ala Glu Val Thr Gly Val Gly Tyr Asn Gln Phe Gly
        355                 360                 365

Glu Val Ile Val Asp Gly Asp Val Val His Gly Phe Tyr Asn Pro Ala
    370                 375                 380

Val Ser Arg Ile Val Glu Ala Gly Cys Val Cys Asn Asp Ala Val Ile
385                 390                 395                 400

Arg Asn Asn Thr Leu Met Gly Lys Pro Thr Glu Gly Ala Leu Ile Ala
                405                 410                 415

Leu Ala Met Lys Met Gly Leu Asp Gly Leu Gln Gln Asp Tyr Ile Arg
            420                 425                 430

Lys Ala Glu Tyr Pro Phe Ser Ser Glu Gln Lys Trp Met Ala Val Lys
        435                 440                 445

Cys Val His Arg Thr Gln Gln Asp Arg Pro Glu Ile Cys Phe Met Lys
    450                 455                 460

Gly Ala Tyr Glu Gln Val Ile Lys Tyr Cys Thr Thr Tyr Gln Ser Lys
465                 470                 475                 480

Gly Gln Thr Leu Thr Leu Thr Gln Gln Gln Arg Asp Val Tyr Gln Gln
                485                 490                 495

Glu Lys Ala Arg Met Gly Ser Ala Gly Leu Arg Val Leu Ala Leu Ala
            500                 505                 510

Ser Gly Pro Glu Leu Gly Gln Leu Thr Phe Leu Gly Leu Val Gly Ile
        515                 520                 525

Ile Asp Pro Pro Arg Thr Gly Val Lys Glu Ala Val Thr Thr Leu Ile
    530                 535                 540

Ala Ser Gly Val Ser Ile Lys Met Ile Thr Gly Asp Ser Gln Glu Thr
545                 550                 555                 560

Ala Val Ala Ile Ala Ser Arg Leu Gly Leu Tyr Ser Lys Thr Ser Gln
                565                 570                 575

Ser Val Ser Gly Glu Glu Ile Asp Ala Met Asp Val Gln Gln Leu Ser
            580                 585                 590

Gln Ile Val Pro Lys Val Ala Val Phe Tyr Arg Ala Ser Pro Arg His
        595                 600                 605

Lys Met Lys Ile Ile Lys Ser Leu Gln Lys Asn Gly Ser Val Val Ala
    610                 615                 620

Met Thr Gly Asp Gly Val Asn Asp Ala Val Ala Leu Lys Ala Ala Asp
625                 630                 635                 640

Ile Gly Val Ala Met Gly Gln Thr Gly Thr Asp Val Cys Lys Glu Ala
                645                 650                 655

Ala Asp Met Ile Leu Val Asp Asp Asp Phe Gln Thr Ile Met Ser Ala
            660                 665                 670

Ile Glu Glu Gly Lys Gly Ile Tyr Asn Asn Ile Lys Asn Phe Val Arg
        675                 680                 685

Phe Gln Leu Ser Thr Ser Ile Ala Ala Leu Thr Leu Ile Ser Leu Ala
    690                 695                 700

Thr Leu Met Asn Phe Pro Asn Pro Leu Asn Ala Met Gln Ile Leu Trp
705                 710                 715                 720

Ile Asn Ile Ile Met Asp Gly Pro Pro Ala Gln Ser Leu Gly Val Glu
                725                 730                 735

Pro Val Asp Lys Asp Val Ile Arg Lys Pro Pro Arg Asn Trp Lys Asp
            740                 745                 750

Ser Ile Leu Thr Lys Asn Leu Ile Leu Lys Ile Leu Val Ser Ser Ile
        755                 760                 765

Ile Ile Val Cys Gly Thr Leu Phe Val Phe Trp Arg Glu Leu Arg Asp
    770                 775                 780

Asn Val Ile Thr Pro Arg Asp Thr Thr Met Thr Phe Thr Cys Phe Val
785                 790                 795                 800

Phe Phe Asp Met Phe Asn Ala Leu Ser Ser Arg Ser Gln Thr Lys Ser
                805                 810                 815

Val Phe Glu Ile Gly Leu Cys Ser Asn Arg Met Phe Cys Tyr Ala Val
            820                 825                 830

Leu Gly Ser Ile Met Gly Gln Leu Leu Val Ile Tyr Phe Pro Pro Leu
        835                 840                 845

Gln Lys Val Phe Gln Thr Glu Ser Leu Ser Ile Leu Asp Leu Leu Phe
    850                 855                 860

Leu Leu Gly Leu Thr Ser Ser Val Cys Ile Val Ala Glu Ile Ile Lys
865                 870                 875                 880

Lys Val Glu Arg Ser Arg Glu Lys Ile Gln Lys His Val Ser Ser Thr
                885                 890                 895

Ser Ser Ser Phe Leu Glu Val

            900

<210>  209
<211>  1284
<212>  DNA
<213>  Homo sapiens

<400>  209
atgggctacc agaggcagga gcctgtcatc ccgccgcaga gaggattgcc ttattcaatg      60

aagcaagctg ggtttccttt gggaatattg cttttattct gggtttcata tgttacagac     120

ttttcccttg ttttattgat aaaaggaggg gccctctctg gaacagatac ctaccagtct     180

ttggtcaata aaactttcgg ctttccaggg tatctgctcc tctctgttct tcagtttttg     240

tatcctttta tagcaatgat aagttacaat ataatagctg gagatacttt gagcaaagtt     300

tttcaaagaa tcccaggagt tgatcctgaa aacgtgttta ttggtcgcca cttcattatt     360

ggactttcca cagttacctt tactctgcct ttatccttgt accgaaatat agcaaagctt     420

ggaaaggtct ccctcatctc tacaggttta acaactctga ttcttggaat tgtaatggca     480

agggcaattt cactgggtcc acacatacca aaaacagaag acgcttgggt atttgcaaag     540

cccaatgcca ttcaagcggt cggggttatg tcttttgcat ttatttgcca ccataactcc     600

ttcttagttt acagttctct agaagaaccc acagtagcta agtggtcccg ccttatccat     660

atgtccatcg tgatttctgt atttatctgt atattctttg ctacatgtgg atacttgaca     720

tttactggct tcacccaagg ggacttattt gaaaattact gcagaaatga tgacctggta     780

acatttggaa gattttgtta tggtgtcact gtcattttga cataccctat ggaatgcttt     840

gtgacaagag aggtaattgc caatgtgttt tttggtggga atctttcatc ggttttccac     900

attgttgtaa cagtgatggt catcactgta gccacgcttg tgtcattgct gattgattgc     960

ctcgggatag ttctagaact caatggtgtg ctctgtgcaa ctcccctcat ttttatcatt    1020

ccatcagcct gttatctgaa actgtctgaa gaaccaagga cacactccga taagattatg    1080

tcttgtgtca tgcttcccat tggtgctgtg gtgatggttt ttggattcgt catggctatt    1140

acaaatactc aagactgcac ccatgggcag gaaatgttct actgctttcc tgacaatttc    1200

tctctcacaa atacctcaga gtctcatgtt cagcagacaa cacaactttc tactttaaat    1260

attagtatct ttcaactcga gtaa                                           1284

<210>  210
<211>  427
<212>  PRT
<213>  Homo sapiens

<400>  210

Met Gly Tyr Gln Arg Gln Glu Pro Val Ile Pro Pro Gln Arg Gly Leu
1               5                   10                  15

Pro Tyr Ser Met Lys Gln Ala Gly Phe Pro Leu Gly Ile Leu Leu Leu
            20                  25                  30

Phe Trp Val Ser Tyr Val Thr Asp Phe Ser Leu Val Leu Leu Ile Lys
        35                  40                  45

Gly Gly Ala Leu Ser Gly Thr Asp Thr Tyr Gln Ser Leu Val Asn Lys
    50                  55                  60

Thr Phe Gly Phe Pro Gly Tyr Leu Leu Leu Ser Val Leu Gln Phe Leu
65                  70                  75                  80

Tyr Pro Phe Ile Ala Met Ile Ser Tyr Asn Ile Ile Ala Gly Asp Thr
                85                  90                  95

Leu Ser Lys Val Phe Gln Arg Ile Pro Gly Val Asp Pro Glu Asn Val
            100                 105                 110

Phe Ile Gly Arg His Phe Ile Ile Gly Leu Ser Thr Val Thr Phe Thr
        115                 120                 125

Leu Pro Leu Ser Leu Tyr Arg Asn Ile Ala Lys Leu Gly Lys Val Ser
    130                 135                 140

Leu Ile Ser Thr Gly Leu Thr Thr Leu Ile Leu Gly Ile Val Met Ala
145                 150                 155                 160

Arg Ala Ile Ser Leu Gly Pro His Ile Pro Lys Thr Glu Asp Ala Trp
                165                 170                 175

Val Phe Ala Lys Pro Asn Ala Ile Gln Ala Val Gly Val Met Ser Phe
            180                 185                 190

Ala Phe Ile Cys His His Asn Ser Phe Leu Val Tyr Ser Ser Leu Glu
        195                 200                 205

Glu Pro Thr Val Ala Lys Trp Ser Arg Leu Ile His Met Ser Ile Val
    210                 215                 220

Ile Ser Val Phe Ile Cys Ile Phe Phe Ala Thr Cys Gly Tyr Leu Thr
225                 230                 235                 240

Phe Thr Gly Phe Thr Gln Gly Asp Leu Phe Glu Asn Tyr Cys Arg Asn
                245                 250                 255

Asp Asp Leu Val Thr Phe Gly Arg Phe Cys Tyr Gly Val Thr Val Ile
            260                 265                 270

Leu Thr Tyr Pro Met Glu Cys Phe Val Thr Arg Glu Val Ile Ala Asn
        275                 280                 285

Val Phe Phe Gly Gly Asn Leu Ser Ser Val Phe His Ile Val Val Thr
    290                 295                 300

Val Met Val Ile Thr Val Ala Thr Leu Val Ser Leu Leu Ile Asp Cys
305                 310                 315                 320

Leu Gly Ile Val Leu Glu Leu Asn Gly Val Leu Cys Ala Thr Pro Leu
                325                 330                 335

Ile Phe Ile Ile Pro Ser Ala Cys Tyr Leu Lys Leu Ser Glu Glu Pro
            340                 345                 350

Arg Thr His Ser Asp Lys Ile Met Ser Cys Val Met Leu Pro Ile Gly
        355                 360                 365

Ala Val Val Met Val Phe Gly Phe Val Met Ala Ile Thr Asn Thr Gln
    370                 375                 380

Asp Cys Thr His Gly Gln Glu Met Phe Tyr Cys Phe Pro Asp Asn Phe
385                 390                 395                 400

Ser Leu Thr Asn Thr Ser Glu Ser His Val Gln Gln Thr Thr Gln Leu
                405                 410                 415

Ser Thr Leu Asn Ile Ser Ile Phe Gln Leu Glu
            420                 425

<210>  211
<211>  1203
<212>  DNA
<213>  Homo sapiens

<400>  211
atgggctacc agaggcagga gcctgtcatc ccgccgcagt tttcccttgt tttattgata      60

aaaggagggg ccctctctgg aacagatacc taccagtctt tggtcaataa aactttcggc     120

tttccagggt atctgctcct ctctgttctt cagtttttgt atccttttat agcaatgata     180

agttacaata taatagctgg agatactttg agcaaagttt ttcaaagaat cccaggagtt     240

gatcctgaaa acgtgtttat tggtcgccac ttcattattg gactttccac agttaccttt     300

actctgcctt tatccttgta ccgaaatata gcaaagcttg gaaaggtctc cctcatctct     360

acaggtttaa caactctgat tcttggaatt gtaatggcaa gggcaatttc actgggtcca     420

cacataccaa aaacagaaga cgcttgggta tttgcaaagc ccaatgccat tcaagcggtc     480

ggggttatgt cttttgcatt tatttgccac cataactcct tcttagttta cagttctcta     540

gaagaaccca cagtagctaa gtggtcccgc cttatccata tgtccatcgt gatttctgta     600

tttatctgta tattctttgc tacatgtgga tacttgacat ttactggctt cacccaaggg     660

gacttatttg aaaattactg cagaaatgat gacctggtaa catttggaag attttgttat     720

ggtgtcactg tcattttgac ataccctatg gaatgctttg tgacaagaga ggtaattgcc     780

aatgtgtttt ttggtgggaa tctttcatcg gttttccaca ttgttgtaac agtgatggtc     840

atcactgtag ccacgcttgt gtcattgctg attgattgcc tcgggatagt tctagaactc     900

aatggtgtgc tctgtgcaac tcccctcatt tttatcattc catcagcctg ttatctgaaa     960

ctgtctgaag aaccaaggac acactccgat aagattatgt cttgtgtcat gcttcccatt    1020

ggtgctgtgg tgatggtttt tggattcgtc atggctatta caaatactca agactgcacc    1080

catgggcagg aaatgttcta ctgctttcct gacaatttct ctctcacaaa tacctcagag    1140

tctcatgttc agcagacaac acaactttct actttaaata ttagtatctt tcaactcgag    1200

taa                                                                  1203

<210>  212
<211>  400
<212>  PRT
<213>  Homo sapiens

<400>  212

Met Gly Tyr Gln Arg Gln Glu Pro Val Ile Pro Pro Gln Phe Ser Leu
1               5                   10                  15

Val Leu Leu Ile Lys Gly Gly Ala Leu Ser Gly Thr Asp Thr Tyr Gln
            20                  25                  30

Ser Leu Val Asn Lys Thr Phe Gly Phe Pro Gly Tyr Leu Leu Leu Ser
        35                  40                  45

Val Leu Gln Phe Leu Tyr Pro Phe Ile Ala Met Ile Ser Tyr Asn Ile
    50                  55                  60

Ile Ala Gly Asp Thr Leu Ser Lys Val Phe Gln Arg Ile Pro Gly Val
65                  70                  75                  80

Asp Pro Glu Asn Val Phe Ile Gly Arg His Phe Ile Ile Gly Leu Ser
                85                  90                  95

Thr Val Thr Phe Thr Leu Pro Leu Ser Leu Tyr Arg Asn Ile Ala Lys
            100                 105                 110

Leu Gly Lys Val Ser Leu Ile Ser Thr Gly Leu Thr Thr Leu Ile Leu
        115                 120                 125

Gly Ile Val Met Ala Arg Ala Ile Ser Leu Gly Pro His Ile Pro Lys
    130                 135                 140

Thr Glu Asp Ala Trp Val Phe Ala Lys Pro Asn Ala Ile Gln Ala Val
145                 150                 155                 160

Gly Val Met Ser Phe Ala Phe Ile Cys His His Asn Ser Phe Leu Val
                165                 170                 175

Tyr Ser Ser Leu Glu Glu Pro Thr Val Ala Lys Trp Ser Arg Leu Ile
            180                 185                 190

His Met Ser Ile Val Ile Ser Val Phe Ile Cys Ile Phe Phe Ala Thr
        195                 200                 205

Cys Gly Tyr Leu Thr Phe Thr Gly Phe Thr Gln Gly Asp Leu Phe Glu
    210                 215                 220

Asn Tyr Cys Arg Asn Asp Asp Leu Val Thr Phe Gly Arg Phe Cys Tyr
225                 230                 235                 240

Gly Val Thr Val Ile Leu Thr Tyr Pro Met Glu Cys Phe Val Thr Arg
                245                 250                 255

Glu Val Ile Ala Asn Val Phe Phe Gly Gly Asn Leu Ser Ser Val Phe
            260                 265                 270

His Ile Val Val Thr Val Met Val Ile Thr Val Ala Thr Leu Val Ser
        275                 280                 285

Leu Leu Ile Asp Cys Leu Gly Ile Val Leu Glu Leu Asn Gly Val Leu
    290                 295                 300

Cys Ala Thr Pro Leu Ile Phe Ile Ile Pro Ser Ala Cys Tyr Leu Lys
305                 310                 315                 320

Leu Ser Glu Glu Pro Arg Thr His Ser Asp Lys Ile Met Ser Cys Val
                325                 330                 335

Met Leu Pro Ile Gly Ala Val Val Met Val Phe Gly Phe Val Met Ala
            340                 345                 350

Ile Thr Asn Thr Gln Asp Cys Thr His Gly Gln Glu Met Phe Tyr Cys
        355                 360                 365

Phe Pro Asp Asn Phe Ser Leu Thr Asn Thr Ser Glu Ser His Val Gln
    370                 375                 380

Gln Thr Thr Gln Leu Ser Thr Leu Asn Ile Ser Ile Phe Gln Leu Glu
385                 390                 395                 400

<210>  213
<211>  1140

<212>  DNA
<213>  Homo sapiens

<400>  213
atgggctacc agaggcagga gcctgtcatc ccgccgcagg tcaataaaac tttcggcttt      60

ccagggtatc tgctcctctc tgttcttcag tttttgtatc cttttatagc aatgataagt     120

tacaatataa tagctggaga tactttgagc aaagtttttc aaagaatccc aggagttgat     180

cctgaaaacg tgtttattgg tcgccacttc attattggac tttccacagt tacctttact     240

ctgcctttat ccttgtaccg aaatatagca aagcttggaa aggtctccct catctctaca     300

ggtttaacaa ctctgattct tggaattgta atggcaaggg caatttcact gggtccacac     360

ataccaaaaa cagaagacgc ttgggtattt gcaaagccca atgccattca agcggtcggg     420

gttatgtctt ttgcatttat ttgccaccat aactccttct tagtttacag ttctctagaa     480

gaacccacag tagctaagtg gtcccgcctt atccatatgt ccatcgtgat ttctgtattt     540

atctgtatat tctttgctac atgtggatac ttgacattta ctggcttcac ccaaggggac     600

ttatttgaaa attactgcag aaatgatgac ctggtaacat ttggaagatt ttgttatggt     660

gtcactgtca ttttgacata ccctatggaa tgctttgtga caagagaggt aattgccaat     720

gtgtttttgg gtgggaatct ttcatcggtt ttccacattg ttgtaacagt gatggtcatc     780

actgtagcca cgcttgtgtc attgctgatt gattgcctcg ggatagttct agaactcaat     840

ggtgtgctct gtgcaactcc cctcattttt atcattccat cagcctgtta tctgaaactg     900

tctgaagaac caaggacaca ctccgataag attatgtctt gtgtcatgct tcccattggt     960

gctgtggtga tggtttttgg attcgtcatg gctattacaa atactcaaga ctgcacccat    1020

gggcaggaaa tgttctactg ctttcctgac aatttctctc tcacaaatac ctcagagtct    1080

catgttcagc agacaacaca actttctact ttaaatatta gtatctttca actcgagtaa    1140

<210>  214
<211>  379
<212>  PRT
<213>  Homo sapiens

<400>  214

Met Gly Tyr Gln Arg Gln Glu Pro Val Ile Pro Pro Gln Val Asn Lys
1               5                   10                  15

Thr Phe Gly Phe Pro Gly Tyr Leu Leu Leu Ser Val Leu Gln Phe Leu
            20                  25                  30

Tyr Pro Phe Ile Ala Met Ile Ser Tyr Asn Ile Ile Ala Gly Asp Thr
        35                  40                  45

Leu Ser Lys Val Phe Gln Arg Ile Pro Gly Val Asp Pro Glu Asn Val
    50                  55                  60

Phe Ile Gly Arg His Phe Ile Ile Gly Leu Ser Thr Val Thr Phe Thr
65                  70                  75                  80

Leu Pro Leu Ser Leu Tyr Arg Asn Ile Ala Lys Leu Gly Lys Val Ser
                85                  90                  95

Leu Ile Ser Thr Gly Leu Thr Thr Leu Ile Leu Gly Ile Val Met Ala

            100                 105                 110

Arg Ala Ile Ser Leu Gly Pro His Ile Pro Lys Thr Glu Asp Ala Trp
        115                 120                 125

Val Phe Ala Lys Pro Asn Ala Ile Gln Ala Val Gly Val Met Ser Phe
    130                 135                 140

Ala Phe Ile Cys His His Asn Ser Phe Leu Val Tyr Ser Ser Leu Glu
145                 150                 155                 160

Glu Pro Thr Val Ala Lys Trp Ser Arg Leu Ile His Met Ser Ile Val
                165                 170                 175

Ile Ser Val Phe Ile Cys Ile Phe Phe Ala Thr Cys Gly Tyr Leu Thr
            180                 185                 190

Phe Thr Gly Phe Thr Gln Gly Asp Leu Phe Glu Asn Tyr Cys Arg Asn
        195                 200                 205

Asp Asp Leu Val Thr Phe Gly Arg Phe Cys Tyr Gly Val Thr Val Ile
    210                 215                 220

Leu Thr Tyr Pro Met Glu Cys Phe Val Thr Arg Glu Val Ile Ala Asn
225                 230                 235                 240

Val Phe Phe Gly Gly Asn Leu Ser Ser Val Phe His Ile Val Val Thr
                245                 250                 255

Val Met Val Ile Thr Val Ala Thr Leu Val Ser Leu Leu Ile Asp Cys
            260                 265                 270

Leu Gly Ile Val Leu Glu Leu Asn Gly Val Leu Cys Ala Thr Pro Leu
        275                 280                 285

Ile Phe Ile Ile Pro Ser Ala Cys Tyr Leu Lys Leu Ser Glu Glu Pro
    290                 295                 300

Arg Thr His Ser Asp Lys Ile Met Ser Cys Val Met Leu Pro Ile Gly
305                 310                 315                 320

Ala Val Val Met Val Phe Gly Phe Val Met Ala Ile Thr Asn Thr Gln
                325                 330                 335

Asp Cys Thr His Gly Gln Glu Met Phe Tyr Cys Phe Pro Asp Asn Phe
            340                 345                 350

Ser Leu Thr Asn Thr Ser Glu Ser His Val Gln Gln Thr Thr Gln Leu
        355                 360                 365

Ser Thr Leu Asn Ile Ser Ile Phe Gln Leu Glu
    370                 375

<210> 215
<211> 1389
<212> DNA
<213> Homo sapiens

<400> 215
atgggctacc agaggcagga gcctgtcatc ccgccgcaga gagatttaga tgacagagaa 60

acccttgttt ctgaacatga gtataaagag aaaacctgtc agtctgctgc tctttttaat 120

gttgtcaact cgattatagg atctggtata ataggattgc cttattcaat gaagcaagct 180

gggtttcctt tgggaatatt gcttttattc tgggtttcat atgttacaga cttttccctt 240

gttttattga taaaaggagg ggccctctct ggaacagata cctaccagtc tttggtcaat 300

aaaactttcg gctttccagg gtatctgctc ctctctgttc ttcagttttt gtatcctttt 360
atagcaatga taagttacaa tataatagct ggagatactt tgagcaaagt ttttcaaaga 420
atcccaggag ttgatcctga aaacgtgttt attggtcgcc acttcattat tggactttcc 480
acagttacct ttactctgcc tttatccttg taccgaaata tagcaaagct tggaaaggtc 540
tccctcatct ctacaggttt aacaactctg attcttggaa ttgtaatggc aagggcaatt 600
tcactgggtc cacacatacc aaaaacagaa gacgcttggg tatttgcaaa gcccaatgcc 660
attcaagcgg tcggggttat gtcttttgca tttatttgcc accataactc cttcttagtt 720
tacagttctc tagaagaacc cacagtagct aagtggtccc gccttatcca tatgtccatc 780
gtgatttctg tatttatctg tatattcttt gctacatgtg gatacttgac atttactggc 840
ttcacccaag ggacttattt tgaaaattac tgcagaaatg atgacctggt aacatttgga 900
agattttgtt atggtgtcac tgtcattttg acatacccta tggaatgctt tgtgacaaga 960
gaggtaattg ccaatgtgtt ttttggtggg aatctttcat cggttttcca cattgttgta 1020
acagtgatgg tcatcactgt agccacgctt gtgtcattgc tgattgattg cctcgggata 1080
gttctagaac tcaatggtgt gctctgtgca actcccctca tttttatcat tccatcagcc 1140
tgttatctga aactgtctga agaaccaagg acacactccg ataagattat gtcttgtgtc 1200
atgcttccca ttggtgctgt ggtgatggtt tttggattcg tcatggctat tacaaatact 1260
caagactgca cccatgggca ggaaatgttc tactgctttc ctgacaattt ctctctcaca 1320
aatacctcag agtctcatgt tcagcagaca acacaacttt ctactttaaa tattagtatc 1380
tttcaatga 1389

<210> 216
<211> 462
<212> PRT
<213> Homo sapiens

<400> 216

Met Gly Tyr Gln Arg Gln Glu Pro Val Ile Pro Pro Gln Arg Asp Leu
1               5                   10                  15

Asp Asp Arg Glu Thr Leu Val Ser Glu His Glu Tyr Lys Glu Lys Thr
            20                  25                  30

Cys Gln Ser Ala Ala Leu Phe Asn Val Val Asn Ser Ile Ile Gly Ser
        35                  40                  45

Gly Ile Ile Gly Leu Pro Tyr Ser Met Lys Gln Ala Gly Phe Pro Leu
    50                  55                  60

Gly Ile Leu Leu Leu Phe Trp Val Ser Tyr Val Thr Asp Phe Ser Leu
65                  70                  75                  80

Val Leu Leu Ile Lys Gly Gly Ala Leu Ser Gly Thr Asp Thr Tyr Gln
                85                  90                  95

Ser Leu Val Asn Lys Thr Phe Gly Phe Pro Gly Tyr Leu Leu Leu Ser
            100                 105                 110

Val Leu Gln Phe Leu Tyr Pro Phe Ile Ala Met Ile Ser Tyr Asn Ile

        115                 120                 125

Ile Ala Gly Asp Thr Leu Ser Lys Val Phe Gln Arg Ile Pro Gly Val
    130                 135                 140

Asp Pro Glu Asn Val Phe Ile Gly Arg His Phe Ile Ile Gly Leu Ser
145                 150                 155                 160

Thr Val Thr Phe Thr Leu Pro Leu Ser Leu Tyr Arg Asn Ile Ala Lys
                165                 170                 175

Leu Gly Lys Val Ser Leu Ile Ser Thr Gly Leu Thr Thr Leu Ile Leu
            180                 185                 190

Gly Ile Val Met Ala Arg Ala Ile Ser Leu Gly Pro His Ile Pro Lys
        195                 200                 205

Thr Glu Asp Ala Trp Val Phe Ala Lys Pro Asn Ala Ile Gln Ala Val
    210                 215                 220

Gly Val Met Ser Phe Ala Phe Ile Cys His His Asn Ser Phe Leu Val
225                 230                 235                 240

Tyr Ser Ser Leu Glu Glu Pro Thr Val Ala Lys Trp Ser Arg Leu Ile
                245                 250                 255

His Met Ser Ile Val Ile Ser Val Phe Ile Cys Ile Phe Phe Ala Thr
            260                 265                 270

Cys Gly Tyr Leu Thr Phe Thr Gly Phe Thr Gln Gly Asp Leu Phe Glu
        275                 280                 285

Asn Tyr Cys Arg Asn Asp Asp Leu Val Thr Phe Gly Arg Phe Cys Tyr
    290                 295                 300

Gly Val Thr Val Ile Leu Thr Tyr Pro Met Glu Cys Phe Val Thr Arg
305                 310                 315                 320

Glu Val Ile Ala Asn Val Phe Phe Gly Gly Asn Leu Ser Ser Val Phe
                325                 330                 335

His Ile Val Val Thr Val Met Val Ile Thr Val Ala Thr Leu Val Ser
            340                 345                 350

Leu Leu Ile Asp Cys Leu Gly Ile Val Leu Glu Leu Asn Gly Val Leu
        355                 360                 365

Cys Ala Thr Pro Leu Ile Phe Ile Ile Pro Ser Ala Cys Tyr Leu Lys
    370                 375                 380

Leu Ser Glu Glu Pro Arg Thr His Ser Asp Lys Ile Met Ser Cys Val
385                 390                 395                 400

Met Leu Pro Ile Gly Ala Val Val Met Val Phe Gly Phe Val Met Ala
                405                 410                 415

Ile Thr Asn Thr Gln Asp Cys Thr His Gly Gln Glu Met Phe Tyr Cys
            420                 425                 430

Phe Pro Asp Asn Phe Ser Leu Thr Asn Thr Ser Glu Ser His Val Gln
        435                 440                 445

Gln Thr Thr Gln Leu Ser Thr Leu Asn Ile Ser Ile Phe Gln
    450                 455                 460

<210> 217
<211> 3501

<212>  DNA
<213>  Homo sapiens

<400>  217
atggaggatg ccttcggggc agccgtggtg accgtgtggg acagcgatgc acacaccacg      60

gagaagccca ccgatgccta cggagagctg gacttcacgg gggccggccg caagcacagc     120

aatttcctcc ggctctctga ccgaacggat ccagctgcag tttatagtct ggtcacacgc     180

acatggggct tccgtgcccc gaacctggtg gtgtcagtgc tggggggatc ggggggcccc     240

gtcctccaga cctggctgca ggacctgctg cgtcgtgggc tggtgcgggc tgcccagagc     300

acaggagcct ggattgtcac tgggggtctg cacacgggca tcggccggca tgttggtgtg     360

gctgtacggg accatcagat ggccagcact gggggcacca aggtggtggc catgggtgtg     420

gccccctggg gtgtggtccg gaatagagac accctcatca accccaaggg ctcgttccct     480

gcgaggtacc ggtggcgcgg tgacccggag gacggggtcc agtttcccct ggactacaac     540

tactcggcct tcttcctggt ggacgacggc acacacggct gcctgggggg cgagaaccgc     600

ttccgcttgc gcctggagtc ctacatctca cagcagaaga cgggcgtggg agggactgga     660

attgacatcc ctgtcctgct cctcctgatt gatggtgatg agaagatgtt gacgcgaata     720

gagaacgcca cccaggctca gctcccatgt ctcctcgtgg ctggctcagg gggagctgcg     780

gactgcctgg cggagaccct ggaagacact ctggccccag ggagtggggg agccaggcaa     840

ggcgaagccc gagatcgaat caggcgtttc tttcccaaag gggaccttga ggtcctgcag     900

gcccaggtgg agaggattat gacccggaag gagctcctga cagtctattc ttctgaggat     960

gggtctgagg aattcgagac catagttttg aaggcccttg tgaaggcctg tgggagctcg    1020

gaggcctcag cctacctgga tgagctgcgt ttggctgtgg cttggaaccg cgtggacatt    1080

gcccagagtg aactctttcg gggggacatc caatggcggt ccttccatct cgaagcttcc    1140

ctcatggacg ccctgctgaa tgaccggcct gagttcgtgc gcttgctcat ttcccacggc    1200

ctcagcctgg gccacttcct gaccccgatg cgcctggccc aactctacag cgcggcgccc    1260

tccaactcgc tcatccgcaa ccttttggac caggcgtccc acagcgcagg caccaaagcc    1320

ccagccctaa aggggggagc tgcggagctc cggccccctg acgtggggca tgtgctgagg    1380

atgctgctgg ggaagatgtg cgcgccgagg tacccctccg ggggcgcctg ggaccctcac    1440

ccaggccagg gcttcgggga gagcatgtat ctgctctcgg acaaggccac ctcgccgctc    1500

tcgctggatg ctggcctcgg gcaggccccc tggagcgacc tgcttctttg ggcactgttg    1560

ctgaacaggg cacagatggc catgtacttc tgggagatgg gttccaatgc agtttcctca    1620

gctcttgggg cctgtttgct gctccgggtg atggcacgcc tggagcctga cgctgaggag    1680

gcagcacgga ggaaagacct ggcgttcaag tttgagggga tgggcgttga cctctttggc    1740

gagtgctatc gcagcagtga ggtgagggct gcccgcctcc tcctccgtcg ctgcccgctc    1800

tggggggatg ccacttgcct ccagctggcc atgcaagctg acgcccgtgc cttctttgcc    1860

caggatgggg tacagtctct gctgacacag aagtggtggg gagatatggc cagcactaca    1920

cccatctggg ccctggttct cgccttcttt tgccctccac tcatctacac ccgcctcatc 1980
accttcagga aatcagaaga ggagcccaca cgggaggagc tagagtttga catggatagt 2040
gtcattaatg gggaagggcc tgtcgggacg gcggacccag ccgagaagac gccgctgggg 2100
gtcccgcgcc agtcgggccg tccgggttgc tgcgggggcc gctgcggggg gcgccggtgc 2160
ctacgccgct ggttccactt ctggggcgcg ccggtgacca tcttcatggg caacgtggtc 2220
agctacctgc tgttcctgct gcttttctcg cgggtgctgc tcgtggattt ccagccggcg 2280
ccgcccggct ccctggagct gctgctctat ttctgggctt tcacgctgct gtgcgaggaa 2340
ctgcgccagg gcctgagcgg aggcgggggc agcctcgcca gcgggggccc cgggcctggc 2400
catgcctcac tgagccagcg cctgcgcctc tacctcgccg acagctggaa ccagtgcgac 2460
ctagtggctc tcacctgctt cctcctgggc gtgggctgcc ggctgacccc gggtttgtac 2520
cacctgggcc gcactgtcct ctgcatcgac ttcatggttt tcacggtgcg gctgcttcac 2580
atcttcacgg tcaacaaaca gctggggccc aagatcgtca tcgtgagcaa gatgatgaag 2640
gacgtgttct tcttcctctt cttcctcggc gtgtggctgg tagcctatgg cgtggccacg 2700
gaggggctcc tgaggccacg ggacagtgac ttcccaagta tcctgcgccg cgtcttctac 2760
cgtccctacc tgcagatctt cgggcagatt ccccaggagg acatggacgt ggccctcatg 2820
gagcacagca actgctcgtc ggagcccggc ttctgggcac accctcctgg ggcccaggcg 2880
ggcacctgcg tctcccagta tgccaactgg ctggtggtgc tgctcctcgt catcttcctg 2940
ctcgtggcca acatcctgct ggtcaacttg ctcattgcca tgttcagtta cacattcggc 3000
aaagtacagg gcaacagcga tctctactgg aaggcgcagc gttaccgcct catccgggaa 3060
ttccactctc ggcccgcgct ggccccgccc tttatcgtca tctcccactt gcgcctcctg 3120
ctcaggcaat tgtgcaggcg accccggagc ccccagccgt cctccccggc cctcgagcat 3180
ttccgggttt acctttctaa ggaagccgag cggaagctgc taacgtggga atcggtgcat 3240
aaggagaact ttctgctggc acgcgctagg gacaagcggg agagcgactc cgagcgtctg 3300
aagcgcacgt cccagaaggt ggacttggca ctgaaacagc tgggacacat ccgcgagtac 3360
gaacagcgcc tgaaagtgct ggagcgggag gtccagcagt gtagccgcgt cctggggtgg 3420
gtggccgagg ccctgagccg ctctgccttg ctgcccccag gtgggccgcc accccctgac 3480
ctgcctgggt ccaaagactg a 3501

<210> 218
<211> 1166
<212> PRT
<213> Homo sapiens

<400> 218

<210> 219
<211> 894
<212> DNA
<213> Homo sapiens

<400> 219
atggagccgc gggcgctcgt cacggcgctc agcctcggcc tcagcctgtg ctccctgggg      60

ctgctcgtca cggccatctt caccgaccac tggtacgaga ccgacccccg gcgccacaag     120

gagagctgcg agcgcagccg cgcgggcgcc gaccccccgg accagaagaa ccgcctgatg     180

ccgctgtcgc acctgccgct gcgggactcg cccccgctgg ggcgccggct gctcccgggc     240

ggcccggggc gcgccgaccc cgagtcctgg cgctcgctcc tggggctcgg cgggctggac     300

gccgagtgcg gccggcccct cttcgccacc tactcgggcc tctggaggaa gtgctacttc     360

ctgggcatcg accgggacat cgacaccctc atcctgaaag gtattgcgca gcgatgcacg     420

gccatcaagt accacttttc tcagcccatc cgcttgcgaa acattccttt taatttaacc     480

aagaccatac agcaagatga gtggcacctg cttcatttaa gaagaatcac tgctggcttc     540

ctcggcatgg ccgtagccgt ccttctctgc ggctgcattg tggccacagt cagtttcttc     600

tgggaggaga gcttgaccca gcacgtggct ggactcctgt tcctcatgac agggatattt     660

tgcaccattt ccctctgtac ttatgccgcc agtatctcgt atgatttgaa ccggctccca     720

aagctaattt atagcctgcc tgctgatgtg gaacatggtt acagctggtc catcttttgc     780

gcctggtgca gtttaggctt tattgtggca gctggaggtc tctgcatcgc ttatccgttt     840

attagccgga ccaagattgc acagctaaag tctggcagag actccacggt atga           894

<210> 220
<211> 297
<212> PRT
<213> Homo sapiens

<400> 220

Met Glu Pro Arg Ala Leu Val Thr Ala Leu Ser Leu Gly Leu Ser Leu
1               5                   10                  15

Cys Ser Leu Gly Leu Leu Val Thr Ala Ile Phe Thr Asp His Trp Tyr
            20                  25                  30

Glu Thr Asp Pro Arg Arg His Lys Glu Ser Cys Glu Arg Ser Arg Ala
        35                  40                  45

Gly Ala Asp Pro Pro Asp Gln Lys Asn Arg Leu Met Pro Leu Ser His
    50                  55                  60

Leu Pro Leu Arg Asp Ser Pro Pro Leu Gly Arg Arg Leu Leu Pro Gly
65                  70                  75                  80

Gly Pro Gly Arg Ala Asp Pro Glu Ser Trp Arg Ser Leu Leu Gly Leu
                85                  90                  95

Gly Gly Leu Asp Ala Glu Cys Gly Arg Pro Leu Phe Ala Thr Tyr Ser
            100                 105                 110

Gly Leu Trp Arg Lys Cys Tyr Phe Leu Gly Ile Asp Arg Asp Ile Asp
        115                 120                 125

Thr Leu Ile Leu Lys Gly Ile Ala Gln Arg Cys Thr Ala Ile Lys Tyr
    130                 135                 140

His Phe Ser Gln Pro Ile Arg Leu Arg Asn Ile Pro Phe Asn Leu Thr
145                 150                 155                 160

Lys Thr Ile Gln Gln Asp Glu Trp His Leu Leu His Leu Arg Arg Ile
                165                 170                 175

Thr Ala Gly Phe Leu Gly Met Ala Val Ala Val Leu Leu Cys Gly Cys
            180                 185                 190

Ile Val Ala Thr Val Ser Phe Phe Trp Glu Glu Ser Leu Thr Gln His
        195                 200                 205

Val Ala Gly Leu Leu Phe Leu Met Thr Gly Ile Phe Cys Thr Ile Ser
    210                 215                 220

Leu Cys Thr Tyr Ala Ala Ser Ile Ser Tyr Asp Leu Asn Arg Leu Pro
225                 230                 235                 240

Lys Leu Ile Tyr Ser Leu Pro Ala Asp Val Glu His Gly Tyr Ser Trp
                245                 250                 255

Ser Ile Phe Cys Ala Trp Cys Ser Leu Gly Phe Ile Val Ala Ala Gly
            260                 265                 270

Gly Leu Cys Ile Ala Tyr Pro Phe Ile Ser Arg Thr Lys Ile Ala Gln
        275                 280                 285

Leu Lys Ser Gly Arg Asp Ser Thr Val
    290                 295

<210>  221
<211>  1134
<212>  DNA
<213>  Homo sapiens

<400>  221
atgaggcgac tgaatcggaa aaaaacttta agtttggtaa aagagttgga tgcctttccg      60

aaggttcctg agagctatgt agagacttca gccagtggag gtacagtttc tctaatagca     120

tttacaacta tggctttatt aaccataatg gaattctcag tatatcaaga tacatggatg     180

aagtatgaat acgaagtaga caaggatttt tctagcaaat taagaattaa tatagatatt     240

actgttgcca tgaagtgtca atatgttgga gcggatgtat tggatttagc agaaacaatg     300

gttgcatctg cagatggttt agtttatgaa ccaacagtat ttgatctttc accacagcag     360

aaagagtggc agaggatgct gcagctgatt cagagtaggc tacaagaaga gcattcactt     420

caagatgtga tatttaaaag tgcttttaaa agtacatcaa cagctcttcc accaagagaa     480

gatgattcat cacagtctcc aaatgcatgc agaattcatg gccatctata tgtcaataaa 540

gtagcaggga attttcacat aacagtgggc aaggcaattc cacatcctcg tggtcatgca 600

catttggcag cacttgtcaa ccatgaatct tacaattttt ctcatagaat agatcatttg 660

tcttttggag agcttgttcc agcaattatt aatcctttag atggaactga aaaaattgct 720

atagatcaca accagatgtt ccaatatttt attacagttg tgccaacaaa actacataca 780

tataaaatat cagcagacac ccatcagttt tctgtgacag aaagggaacg tatcattaac 840

catgctgcag gcagccatgg agtctctggg atatttatga aatatgatct cagttctctt 900

atggtgacag ttactgagga gcacatgcca ttctggcagt tttttgtaag actctgtggt 960

attgttggag gaatcttttc aacaacaggc atgttacatg gaattggaaa atttatagtt 1020

gaaataattt gctgtcgttt cagacttgga tcctataaac ctgtcaattc tgttcctttt 1080

gaggatggcc acacagacaa ccacttacct cttttagaaa ataatacaca ttga 1134

<210> 222
<211> 377
<212> PRT
<213> Homo sapiens

<400> 222

Met Arg Arg Leu Asn Arg Lys Lys Thr Leu Ser Leu Val Lys Glu Leu
1               5                   10                  15

Asp Ala Phe Pro Lys Val Pro Glu Ser Tyr Val Glu Thr Ser Ala Ser
            20                  25                  30

Gly Gly Thr Val Ser Leu Ile Ala Phe Thr Thr Met Ala Leu Leu Thr
        35                  40                  45

Ile Met Glu Phe Ser Val Tyr Gln Asp Thr Trp Met Lys Tyr Glu Tyr
    50                  55                  60

Glu Val Asp Lys Asp Phe Ser Ser Lys Leu Arg Ile Asn Ile Asp Ile
65                  70                  75                  80

Thr Val Ala Met Lys Cys Gln Tyr Val Gly Ala Asp Val Leu Asp Leu
                85                  90                  95

Ala Glu Thr Met Val Ala Ser Ala Asp Gly Leu Val Tyr Glu Pro Thr
            100                 105                 110

Val Phe Asp Leu Ser Pro Gln Gln Lys Glu Trp Gln Arg Met Leu Gln
        115                 120                 125

Leu Ile Gln Ser Arg Leu Gln Glu Glu His Ser Leu Gln Asp Val Ile
    130                 135                 140

Phe Lys Ser Ala Phe Lys Ser Thr Ser Thr Ala Leu Pro Pro Arg Glu
145                 150                 155                 160

Asp Asp Ser Ser Gln Ser Pro Asn Ala Cys Arg Ile His Gly His Leu
                165                 170                 175

Tyr Val Asn Lys Val Ala Gly Asn Phe His Ile Thr Val Gly Lys Ala
            180                 185                 190

Ile Pro His Pro Arg Gly His Ala His Leu Ala Ala Leu Val Asn His
        195                 200                 205

Glu Ser Tyr Asn Phe Ser His Arg Ile Asp His Leu Ser Phe Gly Glu
    210                 215                 220

Leu Val Pro Ala Ile Ile Asn Pro Leu Asp Gly Thr Glu Lys Ile Ala
225                 230                 235                 240

Ile Asp His Asn Gln Met Phe Gln Tyr Phe Ile Thr Val Val Pro Thr
                245                 250                 255

Lys Leu His Thr Tyr Lys Ile Ser Ala Asp Thr His Gln Phe Ser Val
            260                 265                 270

Thr Glu Arg Glu Arg Ile Ile Asn His Ala Ala Gly Ser His Gly Val
        275                 280                 285

Ser Gly Ile Phe Met Lys Tyr Asp Leu Ser Ser Leu Met Val Thr Val
    290                 295                 300

Thr Glu Glu His Met Pro Phe Trp Gln Phe Phe Val Arg Leu Cys Gly
305                 310                 315                 320

Ile Val Gly Gly Ile Phe Ser Thr Thr Gly Met Leu His Gly Ile Gly
                325                 330                 335

Lys Phe Ile Val Glu Ile Ile Cys Cys Arg Phe Arg Leu Gly Ser Tyr
            340                 345                 350

Lys Pro Val Asn Ser Val Pro Phe Glu Asp Gly His Thr Asp Asn His
        355                 360                 365

Leu Pro Leu Leu Glu Asn Asn Thr His
    370                 375

<210>  223
<211>  3407
<212>  DNA
<213>  Homo sapiens

<400>  223
cgcgcgtctc cgccgcccgc gtgacttctg cctgcgctcc ttctctgaac gctcacttcc      60

gaggagacgc cgacgatgaa gacaccgtgg aagattcttc tgggactgct gggtgctgct     120

gcgcttgtca ccatcatcac cgtgcccgtg gttctgctga acaaaggcac agatgatgct     180

acagctgaca gtcgcaaaac ttacactcta actgattact taaaaaatac ttatagactg     240

aagttatact ccttaagatg gatttcagat catgaatatc tctacaaaca agaaaataat     300

atcttggtat tcaatgctga atatggaaac agctcagttt tcttggagaa cagtacattt     360

gatgagtttg gacattctat caatgattat tcaatatctc ctgatgggca gtttattctc     420

ttagaataca actacgtgaa gcaatggagg cattcctaca cagcttcata tgacatttat     480

gatttaaata aaaggcagct gattacagaa gagaggattc caaacaacac acagtgggtc     540

acatggtcac cagtgggtca taaattggca tatgtttgga acaatgacat ttatgttaaa     600

attgaaccaa atttaccaag ttacagaatc acatggacgg ggaaagaaga tataatatat     660

aatggaataa ctgactgggt ttatgaagag gaagtcttca gtgcctactc tgctctgtgg     720

tggtctccaa acggcacttt tttagcatat gcccaattta acgacacaga agtcccactt     780

attgaatact ccttctactc tgatgagtca ctgcagtacc caaagactgt acgggttcca     840

tatccaaagg caggagctgt gaatccaact gtaaagttct ttgttgtaaa tacagactct     900

ctcagctcag tcaccaatgc aacttccata caaatcactg ctcctgcttc tatgttgata 960
ggggatcact acttgtgtga tgtgacatgg gcaacacaag aaagaatttc tttgcagtgg 1020
ctcaggagga ttcagaacta ttcggtcatg gatatttgtg actatgatga atccagtgga 1080
agatggaact gcttagtggc acggcaacac attgaaatga gtactactgg ctgggttgga 1140
agatttaggc cttcagaacc tcattttacc cttgatggta atagcttcta caagatcatc 1200
agcaatgaag aaggttacag acacatttgc tatttccaaa tagataaaaa agactgcaca 1260
tttattacaa aaggcacctg ggaagtcatc gggatagaag ctctaaccag tgattatcta 1320
tactacatta gtaatgaata taaaggaatg ccaggaggaa ggaatcttta taaaatccaa 1380
cttattgact atacaaaagt gacatgcctc agttgtgagc tgaatccgga aaggtgtcag 1440
tactattctg tgtcattcag taaagaggcg aagtattatc agctgagatg ttccggtcct 1500
ggtctgcccc tctatactct acacagcagc gtgaatgata aagggctgag agtcctggaa 1560
gacaattcag ctttggataa aatgctgcag aatgtccaga tgccctccaa aaaactggac 1620
ttcattattt tgaatgaaac aaaattttgg tatcagatga tcttgcctcc tcattttgat 1680
aaatccaaga aatatcctct actattagat gtgtatgcag gcccatgtag tcaaaaagca 1740
gacactgtct tcagactgaa ctgggccact taccttgcaa gcacagaaaa cattatagta 1800
gctagctttg atggcagagg aagtggttac caaggagata agatcatgca tgcaatcaac 1860
agaagactgg gaacatttga agttgaagat caaattgaag cagccagaca attttcaaaa 1920
atgggatttg tggacaacaa acgaattgca atttggggct ggtcatatgg agggtacgta 1980
acctcaatgg tcctgggatc gggaagtggc gtgttcaagt gtggaatagc cgtggcgcct 2040
gtatcccggt gggagtacta tgactcagtg tacacagaac gttacatggg tctcccaact 2100
ccagaagaca accttgacca ttacagaaat tcaacagtca tgagcagagc tgaaaatttt 2160
aaacaagttg agtacctcct tattcatgga acagcagatg ataacgttca ctttcagcag 2220
tcagctcaga tctccaaagc cctggtcgat gttggagtgg atttccaggc aatgtggtat 2280
actgatgaag accatggaat agctagcagc acagcacacc aacatatata tacccacatg 2340
agccacttca taaaacaatg tttctcttta ccttagcacc tcaaaatacc atgccattta 2400
aagcttatta aaactcattt ttgttttcat tatctcaaaa ctgcactgtc aagatgatga 2460
tgatctttaa aatacacact caaatcaaga aacttaaggt tacctttgtt cccaaatttc 2520
atacctatca tcttaagtag ggacttctgt cttcacaaca gattattacc ttacagaagt 2580
ttgaattatc cggtcgggtt ttattgttta aaatcatttc tgcatcagct gctgaaacaa 2640
caaataggaa ttgtttttat ggaggctttg catagattcc ctgagcagga ttttaatctt 2700
tttctaactg gactggttca aatgttgttc tcttctttaa agggatggca agatgtgggc 2760
agtgatgtca ctagggcagg gacaggataa gagggattag ggagagaaga tagcagggca 2820
tggctgggaa cccaagtcca agcataccaa cacgagcagg ctactgtcag ctcccctcgg 2880
agaagagctg ttcaccacga gactggcaca gttttctgag aaagactatt caaacagtct 2940

caggaaatca aatatcgaaa gcactgactt ctaagtaaac cacagcagtt gaaagactcc 3000

aaagaaatgt aagggaaact gccagcaacg cagcccccag gtgccagtta tggctatagg 3060

tgctacaaaa acacagcaag ggtgatggga aagcattgta aatgtgcttt taaaaaaaaa 3120

tactgatgtt cctagtgaaa gaggcagctt gaaactgaga tgtgaacaca tcagcttgcc 3180

ctgttaaaag atgaaaatat ttgtatcaca aatcttaact tgaaggagtc cttgcatcaa 3240

tttttcttat ttcatttctt tgagtgtctt aattaaaaga atattttaac ttccttggac 3300

tcattttaaa aaatggaaca taaaatacaa tgttatgtat tattattccc attctacata 3360

ctatggaatt tctcccagtc atttaataaa tgtgccttca tttttc 3407

<210> 224
<211> 766
<212> PRT
<213> Homo sapiens

<400> 224

<210>  225
<211>  261
<212>  DNA
<213>  Homo sapiens

<400>  225
atggctctgg cgaaggtgag ggagccaaac gcaaatgaca atgccatcag agttgacaac      60

agaagtgtga ttaaagtgcg tgctaaccag tgttccctgc atgaggcaga aagtgaatcc     120

agaaaccctc aggagctctg gatgggcctg ctcctcttga tgggggtcct agaagcatgt     180

gtggaaatga ggcctctgtc agtctggtcc ctgagagatg acaaggagca gagcccccac     240

cagcccacac tggatgtcta a                                               261

<210>  226
<211>  86
<212>  PRT
<213>  Homo sapiens

<400>  226

Met Ala Leu Ala Lys Val Arg Glu Pro Asn Ala Asn Asp Asn Ala Ile
1               5                   10                  15

Arg Val Asp Asn Arg Ser Val Ile Lys Val Arg Ala Asn Gln Cys Ser
            20                  25                  30

Leu His Glu Ala Glu Ser Glu Ser Arg Asn Pro Gln Glu Leu Trp Met
        35                  40                  45

Gly Leu Leu Leu Leu Met Gly Val Leu Glu Ala Cys Val Glu Met Arg
    50                  55                  60

Pro Leu Ser Val Trp Ser Leu Arg Asp Asp Lys Glu Gln Ser Pro His
65                  70                  75                  80

Gln Pro Thr Leu Asp Val
                85

<210>  227
<211>  462
<212>  DNA
<213>  Homo sapiens

<400>  227
atgccaaatg ctgagttaga agcaaagagc cttggaagca gtaaatgttt aaaaactgct      60

ctcatacttg ctgtatgttg tggatcagca aatatagtca gccctctact tgagcaaaat     120

attgatgtat cttctcaaga tctggacaga cggccagaga gtatgctgtt tctagtcatc     180

atcatgtgga ccagttttgt ggaagacaat ctttccatgg gctgggggaa gctagaagat     240

tttatggcta ttgaagaaga aatgaagaag cacggaagta ctcatgtggg attcccagaa     300

aacctgacta atggtgccgc tgctggcaat ggtgatgatg gattaattcc tccaaggaag     360

agcagaacac ctgaaagcca gcaatttcct gacactgaga atgaagagta tcacaggttt     420

gtcaaagatc agatagttgt agatatgcgg cgttatttct ga                        462

<210>  228
<211>  153
<212>  PRT
<213>  Homo sapiens

<400>  228

<210>  229
<211>  12880
<212>  DNA

<213>  Homo sapiens

<400>  229
gactgcttgc attaaaggac ttcctcatcc tttttttcat gaaactgagc ttgcttaatc      60

agagatggag caaactgact gcaaacccta ccagcctcta ccaaaagtca agcatgaaat     120

ggatctagct tacaccagtt cttctgatga gagtgaagat ggaagaaaac caagacagtc     180

atacaactcc agggagaccc tgcacgagta taaccaggag ctgaggatga attacaatag     240

ccagagtaga aagaggaaag aagtagaaaa atctactcaa gagatggaat tctgtgaaac     300

ctctcacact ctgtgctctg gctaccaaac agacatgcac agcgtttctc ggcatggcta     360

ccagctagag atgggatctg atgtggacac agagacagaa ggtgctgcct cacctgacca     420

tgcactaaga atgtggataa ggggaatgaa atcagagcat agttcctgtt tgtccagccg     480

ggccaactct gcattatcct tgactgacac tgaccatgaa aggaagtctg atggggaaaa     540

tggtttcaaa ttctctcctg tttgttgtga catggaggct caagctgggt ctactcaaga     600

tgtgcagagc agcccacaca accagttcac cttcagaccc ctcccaccgc cacctccgcc     660

tcctcatgcc tgcacctgtg ccaggaagcc accccctgca gcggactctc ttcagaggag     720

atcaatgact acccgcagcc agcccagccc agctgctcca gctcccccaa ccagcacgca     780

ggattcagtc catctgcata acagctgggt cctgaacagc aacataccat tggagaccag     840

gcattccctg ttcaaacatg gatctggttc ctctgcgatc ttcagtgcag ccagtcagaa     900

ctaccctctg acatccaata ccgtgtactc gccccctccc aggcctcttc ctcgaagcac     960

cttttcccga cctgccttta cctttaacaa accttacagg tgctgcaact ggaagtgcac    1020

agcattgagc gccactgcaa tcacagtgac tttggccttg ttactagcct atgtgattgc    1080

agtgcatttg ttcggcctga cttggcagtt gcaaccagtt gaaggagagc tgtatgcaaa    1140

tggagttagc aaagggaaca gggggaccga gtccatggac actacttact ctccaattgg    1200

aggaaaagtt tctgataaat cagagaaaaa agtgtttcag aagggacggg cgatagacac    1260

tggagaagtt gacattggtg cacaggtcat gcagaccatt ccacctggtt tattctggcg    1320

tttccagatt actatccacc atccaatata tctgaagttc aatatttctt tagccaagga    1380

ctctctgctg ggaatttatg gcagaagaaa cattccacct acacatactc agtttgattt    1440

tgtaaaacta atggatggca aacagctggt caagcaggac tccaagggct ctgatgatac    1500

acagcactcc cctcggaacc tgatcttaac ttcgcttcag gagacaggtt tcatagagta    1560

tatggatcaa ggaccttggt atctggcgtt ttacaatgat ggaaaaaaga tggagcaagt    1620

attcgtgtta actacagcaa ttgaaataat ggatgactgt tcaaccaatt gcaatggaaa    1680

tggagagtgt atctctggcc attgtcattg tttcccagga ttccttggac ctgactgtgc    1740

tagagattcc tgccctgtgc tgtgtggtgg gaatggagaa tacgagaaag gacactgtgt    1800

ctgccggcat ggctggaagg ggccagagtg tgacgttccg gaagaacaat gcattgatcc    1860

aacatgcttt ggccacggca cctgcatcat gggagtctgc atctgtgtgc caggatacaa    1920

aggagaaata tgcgaggaag aggactgcct agacccaatg tgttccaacc atggcatctg    1980

tgtaaaagga gaatgtcact gttctactgg ctggggagga gttaactgtg aaacaccact 2040
tcctgtatgt caagagcagt gctcaggaca cggaactttt cttctggacg ctggagtatg 2100
cagctgtgat cccaagtgga caggatctga ctgctcaaca gagctgtgta ccatggagtg 2160
tggtagccat ggagtctgct caagaggaat ttgccagtgt gaagaaggct gggtaggacc 2220
aacatgtgag gaacgctcct gtcattctca ttgtactgag catggccaat gcaaagatgg 2280
aaaatgtgag tgtagccctg gatgggaggg cgaccactgc acaattgctc actacttaga 2340
tgctgtccga gatggctgcc cagggctctg ctttggaaat ggacgatgta ccctggatca 2400
aaatggttgg cactgtgtgt gtcaggtggg ttggagtggg acaggctgca atgttgtcat 2460
ggaaatgctt tgtggagata acttggacaa tgatggagat ggtttaaccg actgtgtgga 2520
tcctgactgt tgtcaacaaa gcaactgtta tataagtcct ctctgccagg gctcaccaga 2580
tcctcttgac ctcattcagc aaagccaaac tctcttctct cagcacactt caagactttt 2640
ttatgatcga atcaaattcc tcattggcaa ggacagtact catgtcattc ctcctgaggt 2700
gtcatttgac agcaggcgtg cctgtgtgat tcgaggccaa gtggtggcca tagatggaac 2760
tcctctagtg ggagtgaatg tcagtttctt gcaccacagt gattatgggt ttaccatcag 2820
ccggcaagat ggaagctttg acctcgtggc catcggtggc atctctgtca tcttaatctt 2880
cgaccgatcc cctttcctgc ctgagaagag aacactctgg ttgccttgga atcagtttat 2940
tgtggtagag aaagtcacca tgcagagagt tgtatcagac ccgccatcct gcgatatctc 3000
caactttatc agcccaaacc ctattgtgct tccttcaccg ctcacatcat ttggagggtc 3060
ctgtccagag aggggaacta ttgttcctga gctgcaggtt gtacaggagg aaattcccat 3120
tccctccagc tttgtgaggc tgagttacct gagcagccgc acccctgggt ataaaaccct 3180
gctacggatc cttctgacac attcaacgat tcccgtaggc atgataaaag tacacctcac 3240
agtagctgtg gaagggcgac tcacacagaa gtggtttccc gccgcaatta atcttgtcta 3300
cacatttgct tggaacaaga ccgatatcta tggacagaag gtttggggcc tggcagaggc 3360
tttggtatct gtgggatatg aatatgaaac gtgccctgac tttattctct gggagcaaag 3420
gacagtcgtt ttacaaggtt ttgagatgga tgcttctaac ctaggagact ggtctttgaa 3480
taagcatcac attttgaatc ctcaaagtgg aatcatacat aaagggaatg gagaaaatat 3540
gttcatttcc cagcagcccc cagtcatatc aaccataatg ggtaatggac accaaaggag 3600
tgtagcctgc accaactgca atggcccagc ccacaacaac aaactctttg ctcctgtcgc 3660
cttagcttct ggccctgatg gcagtgtgta tgttggcgac ttcaattttg taaggagaat 3720
atttccctcg ggaaactccg ttagtatttt ggaattaagc acaagtcctg ctcacaaata 3780
ctatctggct atggaccctg tgtctgaatc actctatcta tcagacacca atactcgcaa 3840
agtctacaag ttgaaatctc ttgtggagac gaaagatctg tccaagaatt ttgaagtggt 3900
ggcaggaact ggtgatcagt gccttccctt tgaccagagt cattgtggag atggtgggag 3960
agcatcggaa gcttcactga atagccctcg aggcatcaca gttgataggc atggatttat 4020

ttactttgtg gatgggacta tgattcgcaa aattgatgag aatgctgtga tcacaactgt 4080
aatcggctca aatggtctga cttccacaca accactgagc tgtgactcag gaatggacat 4140
cactcaggtg cgattagagt ggccaacaga ccttgcagta aatcctatgg acaattcatt 4200
gtatgtcttg gataacaaca ttgtgctgca aatttctgag aacaggcgtg ttcggatcat 4260
cgcaggacgc cccattcact gccaggtgcc aggcatcgat catttcctgg tcagcaaggt 4320
agcaattcac tccactctag agtcagcgag ggccatcagt gtctcccaca gcgggctgct 4380
cttcatagct gaaacagacg agaggaaagt aaaccgcatt cagcaagtaa ccaccaatgg 4440
ggagatctac atcatcgctg gtgcccccac tgactgtgac tgcaaaattg atccaaactg 4500
tgactgtttt tcaggtgatg gtggctatgc caaagatgca aagatgaaag ccccttcctc 4560
cttagcagtg tcgcctgatg gaaccctcta tgtggcagac ctcggaaatg ttcgaattcg 4620
taccatcagc aggaaccaag cccacctgaa tgacatgaac atttatgaga ttgcttcacc 4680
cgctgatcag gaactgtacc agttcactgt aaatggaacc cacctacaca ccctgaactt 4740
gataacaagg gactatgttt ataacttcac ctacaattct gaaggtgact tgggcgcgat 4800
taccagcagc aatggcaatt cagtgcacat tcgccgtgat gcaggcggaa tgccgctatg 4860
gcttgtggtg cctggcggac aagtatactg gctgactata agcagcaatg gagtcctgaa 4920
aagagtgtca gcccaaggct ataatccggc cttaatgacc tatccaggaa acacagggct 4980
tctggctacc aaaagtaacg aaaatggatg gacaaccgtt tatgagtatg accccgaggg 5040
acacctgacc aatgcaacgt ttcccactgg agaggtcagc agcttccaca gtgacctgga 5100
gaagctgaca aaagtggagc tagatacttc caaccgtgaa aatgtcctca tgtcaaccaa 5160
cttgacggca actagtacca tatatatttt aaaacaagaa aatactcaaa gtacctatcg 5220
ggtgaatcca gatggttccc tgcgtgtcac ttttgccagc gggatggaga tcggcctcag 5280
ctcagagccc cacatcctgg caggggcagt caaccctacc ctgggcaaat gcaacatctc 5340
attgcccgga gagcacaatg caaacctcat cgagtggcgg cagaggaagg agcaaaacaa 5400
aggcaatgtt tcggcttttg aaaggaggct gagggcccac aacagaaacc tactctccat 5460
agattttgat catataaccc gcacaggaaa gatctatgat gaccatcgaa aattcaccct 5520
tcgaattctt tatgaccaga ctgggcgacc cattctgtgg tctcctgtaa gcagatataa 5580
tgaagtgaac atcacatatt caccttcggg attggtgacg tttattcaaa gaggaacgtg 5640
gaatgaaaaa atggaatatg accagagtgg gaaaattatt tcaagaactt gggctgatgg 5700
gaaaatttgg agctatacct acttagaaaa atctgtgatg cttctcctac acagccagcg 5760
gcgttacatc tttgagtatg accaatcaga ttgcctgctg tcagttacca tgcctagcat 5820
ggtgcgccac agcttacaaa ccatgctttc agtgggctac taccgtaata tctacacccc 5880
accggacagt agcacttctt ttatccaaga ctatagtcga gatggccgat tgctacagac 5940
cctgcatctg ggacagggcg gcagagtctt atacaagtac accaagcaag caaggctttc 6000
tgaggttctc tatgatacca ctcaggtcac attaacatat gaagagtctt ctggagtgat 6060

taagacaata cacctgatgc atgacggatt catctgcaca atcagataca ggcaaacagg 6120
acctcttatt ggacgccaga ttttcagatt cagtgaagaa ggccttgtga atgcacggtt 6180
cgactacagc tacaacaatt tccgagtcac aagcatgcaa gctgtaatca atgaaacccc 6240
tttgcctata gatctttacc gatatgttga tgtctctggc agaacagagc agtttggaaa 6300
attcagtgta attaattacg atttaaatca ggtcataact actacagtga tgaaacacac 6360
caaaatcttc agtgccaatg gacaagtcat tgaagtccaa tatgaaatcc taaaggcaat 6420
tgcctactgg atgaccattc aatatgataa tgtgggccga catggtaata tgtgcataag 6480
ggtaggagta gatgccaata taacaaggta cttctatgaa tacgatgctg atgggcaact 6540
tcagactgtt tctgtaaatg acaaaaccca gtggcgttat agttacgatc tgaatggaga 6600
catcaacctc ttaagccatg ggaagagtgc tcgtcttact cctctccgat atgacctccg 6660
agaccgcatc accagattag gagaaattca gtataaaatg gatgaagatg gctttctgag 6720
gcagagggga aatgatattt ttgaatataa ttctaatggc ctgctgcaga aagcctacaa 6780
taaggcttct ggctggactg tgcagtatta ctatgatggg cttgggcgac gtgtcgcgag 6840
taagtccagc ctagggcagc accttcagtt ctttgtcgac gcgaccgcga accccataag 6900
agttactcat ttgtacaacc acacaagctc ggagattaca tctctgtatt atgatctcca 6960
aggtcacctt attgccatgg agttaagcag tggtgaagaa tattatgtag cctgtgataa 7020
tacaggtacc ccactagctg tgttcagcag ccgaggtcag gtcataaagg agatactata 7080
cacaccttat ggcgatatct atcatgacac ttaccctgac tttcaggtca taattggttt 7140
tcatggagga ctctatgatt tccttactaa attagtgcac ctggggcaaa gggattatga 7200
tgttgttgct ggcagatgga caacggccta tcatcacata tggaaacagt tgaacctcct 7260
tcctaaacca ttcaacctct actcctttga aaataactac ccagttggca aaattcaaga 7320
tgttgcaaag tataccacag acatcagaag ttggttggag ctatttggtt tccaattaca 7380
caatgtacta cctggatttc ccaaacctga attagaaaat ttagaattaa cttacgagct 7440
tctacggctt cagacaaaaa ctcaagagtg ggatcctgga aagactatcc tgggcattca 7500
gtgtgaactc cagaaacagc tcaggaattt catttccttg gaccaactac ctatgactcc 7560
ccgatacaat gatggacggt gccttgaagg agggaagcaa ccaaggtttg ctgctgtccc 7620
ttctgttttt gggaaaggta taaaatttgc catcaaggat ggcatagtaa cagctgatat 7680
tataggagta gccaatgaag atagcaggcg gcttgctgcc attctcaata atgcccatta 7740
cctggaaaac ctacatttta ccatagaggg gagggacact cactacttca ttaagcttgg 7800
gtctctggag gaagacctgg tgctcatcgg taacactggg gggaggcgga ttctggagaa 7860
tggtgtcaat gtcactgtgt cccagatgac ttctctgttg aatgggagga ctagacggtt 7920
tgcagatatt cagctccagc atggagccct gtgcttcaac atccggtatg ggacaactgt 7980
cgaagaggaa aagaatcacg tgttggagat tgccagacag cgcgcagtgg cccaggcctg 8040
gactaaggaa caaagaaggc tgcaagaggg ggaagagggg attagggcat ggacagaagg 8100

ggaaaagcag cagcttttga gcactgggcg ggtacaaggt tacgatgggt attttgtttt 8160
gtctgttgag cagtatttag aactttctga cagtgccaat aatattcact ttatgagaca 8220
gagcgaaata ggcaggaggt aacaaaaata tctctgcctt tgcgtcacca aagactgcct 8280
gtttttaaaa cataaaatgg tttattgtat tggttttcta gatcagaact ctgtatatgt 8340
aaatatggag gaaaaacata tccaactgcc tttcaatgtg acggaagatg gtattttaat 8400
attgtttgtt taaactcttt aagaaatgac agagattttt agttcttgtg tggcagtatt 8460
caaaataaca caagtagaac tcaaacagct aaaaacagtt ttcagaaagc accactttca 8520
atttgccgag ccatgcatat gttccaatat ccagaaagaa cccaaggttc tctatctcta 8580
ttgtgagaag cagtttcatc cttaactgtt ggcagaactt acgggctatt tgaataggtg 8640
gtgcaatagt atctgaaact tgcctttcga aagactgcca gccctttgac gttttccaga 8700
tctgttatag gaaacttaaa aacaggtgta aaatgtcttc agccaccatc tcctagagtg 8760
aggacccaat tgcccttcct tcttgattat tcctccttgc ttgttaaagt aaatgccata 8820
ttgttgtgct gtgttttggc gtgtggtggc tgggttctgt ctaccatgct tccctgtggg 8880
tgtggtaacc agactgtata gccgctattt gctcgtgtgt acatgatacc aaagcagctg 8940
gccagcgtga cctctctcac acgacctgtt ttgactcaat tttttactaa aagttgttca 9000
gctgtattgg tatcatgtaa acatagcttt tattaacctg ggtaggaatt tctcatttat 9060
atataggatg tgttttggtc atagtttcac attagtgatt cagtatctat acactgaccc 9120
aatggttttg tgcacatgaa cggtaattta cttaaaagta tgattctggt acaaaaacaa 9180
acaaaggctt tagcaggcat acgtgtctgg gatgccgata catacattaa ctactactgc 9240
agaaattcat aagagccaaa accttaaaaa aatagacctg gtacttaagt gaaagtacta 9300
aagggaagac cagaccaaac atcacagcag ttgctgccac attgtttcag cccacttaga 9360
tttatctttc aaatgtacaa ttctgtattg aacatctccc agccatcttc aggaaatcga 9420
atcaagtaaa tcctttccaa ccgaaaacat ttcaactaac tatagagagg cagactcatt 9480
tttactaaaa taatttatac agttagttat tttcgttctc cgtacttacc catttatctt 9540
tatttaatcg tctctactgc ctaggaaaat aactattttc caggacgggt tatttgttct 9600
gcgatcattt aaaatttgga gaaaggtcag gattagtgtt aatatcagct gcagtttctc 9660
aatctctagg aatcctgcag taaaacaagc cccttggtga gctggaagat ttgtgcccag 9720
tgacaaagag atagtttgta aaatgctgtg taattgtaag ttaccacaaa tgaaaataca 9780
tgacagcaca atgtggcccg tagaaaattc ccctgagcca gcttctgcac tttcatcacc 9840
gaatctgaac atttgctatg tctgaaggca aatttatgat ggaatgttag tttggattct 9900
ttccagatgc tacctaaatg cagtgtgggg tcattgcctt gctttgcgat gacagtttct 9960
ttgaaaatat gcaaagtcat aagctcatgt taaggttttt caagagtctg cctcctacta 10020
cacaaaggaa agcaagggaa aggaaatgac cctggcaaac agtagggaag ggtgtattca 10080
aacatttcat tttcaaaacc ttcgggttag aataccactt acacatgtat tctgagagac 10140

agaattcatg aggaactcat ctctctttat aactggaaac acaccagctt gatatattgc 10200
taatccatac taaaatcata ttattgggtt ttttctgaat caggcctgta ttaatggtac 10260
agtatttatt cagaatggaa ttctaaaatt actaacaaac ttgttgaaaa tttgaatacc 10320
tccacaccaa cctaaaaatg gaccttaagt tcctagaacc tctgatgttc ttttaaatta 10380
atggaaaaat aatttgtgaa ctgtatatag agagtgcatt cataaatgtg attatgtatt 10440
ttatcacaaa tccaaaatgt caatattaga gtctattttg cttatatttt aagcaattat 10500
acgtttttgc aattcattga tgatgtatca ttttcaaact gctttaaata tccattagaa 10560
acaaatattt gaagctttta cttaatagtg attaccttga actgtgcatt tctagtttgt 10620
aatacgtatt tggttggttc gtgcctttag tttgttaaag ttacatttgt attatattca 10680
ggaaatgcac tttttattac ttacagctgt ggttttaata ctgccttgaa ctattattat 10740
tctttttaca actcctaaag cttgagggag gaaagaaaaa aaaaacaaaa ctactaatca 10800
gtagtaaatc gaagagaaac attttggcat ttcttaagaa gaagatggag atattgagta 10860
tatcacttcc tattcagctg aatagaaaga atgccttcat tgacttgcag ttctgcagtt 10920
taaattattg aaagaacaat tcgtttgcat ttcctgatga aagtaaaagc atttttcaga 10980
gaaacatatg aatttctcat acccagcaga cagatggctg acactgcaca gccacacacc 11040
attcgagtaa gttaaagtga gagcatagta gttggactct cctatgaaga acattctggg 11100
ctggaggcag ggaatactcc atggttgttt cttttcccta cttaagccca ttttgtttgt 11160
gcttttctgt tttgttttgt tttcactctt gcactacagt ctagagatcc aaatgaactg 11220
aaaagttcaa agtttaacac atttaaatat gtttactttt agttgtcatt ctaatcgtta 11280
ttgattagaa gcatgactcc tgaaggaaag ggaaataaat ctcaattcat actaacttgc 11340
aacaaaacac ttttaccata taaataagta tatgatttat ttttaaccca aaaaatgtat 11400
aaaataagtg tgtcctttac tgtcaattta tcgagaagat ctataatata tagactacat 11460
atatataata tatacaacat agccaaatgt atgaaaactt gacaatgtat aatttggaat 11520
tcacatgcta cctatgtaga caggtatgaa attaagttat aattttcatg agacattttc 11580
atcactgttg acacagtttc aaggcattcc atcatgttat tttgactctt tttctttttt 11640
ttttctttaa aaatatattt ttaactagac caggccccac tataatatca cttaagagag 11700
tcagggcaaa gtttttgcat ttatgaagat gtgttcatgt aagggtgatt gtaatggagt 11760
tcattggtaa tagaagcaaa agtacagtaa cgaagtattg aaaagaaaat tttggagaca 11820
ttggagcata ttatatatag cttgtggaaa gacataaggc tacagatgga atggaacatt 11880
cctgttttct tgaagaaatt cacatacaca tagctgacct gactagtact tcagctcttc 11940
cacagccttc tataaaggtt ctttcttctg caaagaaaac aaaacaaaac aaaacaaaac 12000
aaaaaaaaac aaaaaaagcg caaaaaacaa aaaaacaaaa aaaagcaaag taaaatttaa 12060
aaatacagaa aacaaacaac aaaaaagaat tcaaccataa atagtgacta ttattttcag 12120
tgtgtccttc atgtgaaagc tattaaggac caaatatact actgttcata agaagaaatt 12180

actttctaaa cagtaactga aaatacttag agttaaactt gctgtggatt ttgtcttggc 12240

agttgtcatc ttacattatt tgtcaaagga aatgtgtttg gcagttaaaa atctttcctt 12300

agatttagtg gtggacttta acctcttaaa taaatgttag tatatcagat tgtgtccttg 12360

aaaaatattt tacttgtatg aatcatgaca acgtctaaat ctttactatt cttctggcaa 12420

aagcatcagt aagaaagaag gcgaaaaaga gaagtatagc ctttatgtca gaaaaacatt 12480

ctttttagct gcttactttc tcatgaaaag taaagatgtt tacagtgtat gccaagtttt 12540

cagtttctgt ataacaacag gtagaggttc taatcatatt gaaaattgtg ttataatggt 12600

ctgagccatg ttgctaggaa acaataggtt ccaattttgt attcctgctc tcctgtgctg 12660

aaaagtgact ggatactgta caggttcatg ttctctggct gcagttaaat ggtcttttgc 12720

attttgctct ggctttcagg ccagaagcat gcatttttct acaagagcat cacaacaaca 12780

tgctgtaaat atttaaagtt aaacattatg tgttgatatt tgaaagaaaa gtactttgaa 12840

tatttcattt ttaaaaaata aaattgccaa tgaaaaaaaa 12880

<210> 230
<211> 2725
<212> PRT
<213> Homo sapiens

<400> 230

Met Glu Gln Thr Asp Cys Lys Pro Tyr Gln Pro Leu Pro Lys Val Lys
1               5                   10                  15

His Glu Met Asp Leu Ala Tyr Thr Ser Ser Ser Asp Glu Ser Glu Asp
            20                  25                  30

Gly Arg Lys Pro Arg Gln Ser Tyr Asn Ser Arg Glu Thr Leu His Glu
        35                  40                  45

Tyr Asn Gln Glu Leu Arg Met Asn Tyr Asn Ser Gln Ser Arg Lys Arg
    50                  55                  60

Lys Glu Val Glu Lys Ser Thr Gln Glu Met Glu Phe Cys Glu Thr Ser
65                  70                  75                  80

His Thr Leu Cys Ser Gly Tyr Gln Thr Asp Met His Ser Val Ser Arg
                85                  90                  95

His Gly Tyr Gln Leu Glu Met Gly Ser Asp Val Asp Thr Glu Thr Glu
            100                 105                 110

Gly Ala Ala Ser Pro Asp His Ala Leu Arg Met Trp Ile Arg Gly Met
        115                 120                 125

Lys Ser Glu His Ser Ser Cys Leu Ser Ser Arg Ala Asn Ser Ala Leu
    130                 135                 140

Ser Leu Thr Asp Thr Asp His Glu Arg Lys Ser Asp Gly Glu Asn Gly
145                 150                 155                 160

Phe Lys Phe Ser Pro Val Cys Cys Asp Met Glu Ala Gln Ala Gly Ser
                165                 170                 175

Thr Gln Asp Val Gln Ser Ser Pro His Asn Gln Phe Thr Phe Arg Pro
            180                 185                 190

Leu Pro Pro Pro Pro Pro Pro Pro His Ala Cys Thr Cys Ala Arg Lys
        195                 200                 205

Pro Pro Pro Ala Ala Asp Ser Leu Gln Arg Arg Ser Met Thr Thr Arg
    210                 215                 220

Ser Gln Pro Ser Pro Ala Ala Pro Ala Pro Pro Thr Ser Thr Gln Asp
225                 230                 235                 240

Ser Val His Leu His Asn Ser Trp Val Leu Asn Ser Asn Ile Pro Leu
                245                 250                 255

Glu Thr Arg His Ser Leu Phe Lys His Gly Ser Gly Ser Ser Ala Ile
            260                 265                 270

Phe Ser Ala Ala Ser Gln Asn Tyr Pro Leu Thr Ser Asn Thr Val Tyr
        275                 280                 285

Ser Pro Pro Pro Arg Pro Leu Pro Arg Ser Thr Phe Ser Arg Pro Ala
    290                 295                 300

Phe Thr Phe Asn Lys Pro Tyr Arg Cys Cys Asn Trp Lys Cys Thr Ala
305                 310                 315                 320

Leu Ser Ala Thr Ala Ile Thr Val Thr Leu Ala Leu Leu Leu Ala Tyr
                325                 330                 335

Val Ile Ala Val His Leu Phe Gly Leu Thr Trp Gln Leu Gln Pro Val
            340                 345                 350

Glu Gly Glu Leu Tyr Ala Asn Gly Val Ser Lys Gly Asn Arg Gly Thr
        355                 360                 365

Glu Ser Met Asp Thr Thr Tyr Ser Pro Ile Gly Gly Lys Val Ser Asp
    370                 375                 380

Lys Ser Glu Lys Lys Val Phe Gln Lys Gly Arg Ala Ile Asp Thr Gly
385                 390                 395                 400

Glu Val Asp Ile Gly Ala Gln Val Met Gln Thr Ile Pro Pro Gly Leu
                405                 410                 415

Phe Trp Arg Phe Gln Ile Thr Ile His His Pro Ile Tyr Leu Lys Phe
            420                 425                 430

Asn Ile Ser Leu Ala Lys Asp Ser Leu Leu Gly Ile Tyr Gly Arg Arg
        435                 440                 445

Asn Ile Pro Pro Thr His Thr Gln Phe Asp Phe Val Lys Leu Met Asp
    450                 455                 460

Gly Lys Gln Leu Val Lys Gln Asp Ser Lys Gly Ser Asp Asp Thr Gln
465                 470                 475                 480

His Ser Pro Arg Asn Leu Ile Leu Thr Ser Leu Gln Glu Thr Gly Phe
                485                 490                 495

Ile Glu Tyr Met Asp Gln Gly Pro Trp Tyr Leu Ala Phe Tyr Asn Asp
            500                 505                 510

Gly Lys Lys Met Glu Gln Val Phe Val Leu Thr Thr Ala Ile Glu Ile
        515                 520                 525

Met Asp Asp Cys Ser Thr Asn Cys Asn Gly Asn Gly Glu Cys Ile Ser
    530                 535                 540

Gly His Cys His Cys Phe Pro Gly Phe Leu Gly Pro Asp Cys Ala Arg

545                 550                 555                 560

Asp Ser Cys Pro Val Leu Cys Gly Gly Asn Gly Glu Tyr Glu Lys Gly
                565                 570                 575

His Cys Val Cys Arg His Gly Trp Lys Gly Pro Glu Cys Asp Val Pro
            580                 585                 590

Glu Glu Gln Cys Ile Asp Pro Thr Cys Phe Gly His Gly Thr Cys Ile
        595                 600                 605

Met Gly Val Cys Ile Cys Val Pro Gly Tyr Lys Gly Glu Ile Cys Glu
    610                 615                 620

Glu Glu Asp Cys Leu Asp Pro Met Cys Ser Asn His Gly Ile Cys Val
625                 630                 635                 640

Lys Gly Glu Cys His Cys Ser Thr Gly Trp Gly Gly Val Asn Cys Glu
                645                 650                 655

Thr Pro Leu Pro Val Cys Gln Glu Gln Cys Ser Gly His Gly Thr Phe
            660                 665                 670

Leu Leu Asp Ala Gly Val Cys Ser Cys Asp Pro Lys Trp Thr Gly Ser
        675                 680                 685

Asp Cys Ser Thr Glu Leu Cys Thr Met Glu Cys Gly Ser His Gly Val
    690                 695                 700

Cys Ser Arg Gly Ile Cys Gln Cys Glu Glu Gly Trp Val Gly Pro Thr
705                 710                 715                 720

Cys Glu Glu Arg Ser Cys His Ser His Cys Thr Glu His Gly Gln Cys
                725                 730                 735

Lys Asp Gly Lys Cys Glu Cys Ser Pro Gly Trp Glu Gly Asp His Cys
            740                 745                 750

Thr Ile Ala His Tyr Leu Asp Ala Val Arg Asp Gly Cys Pro Gly Leu
        755                 760                 765

Cys Phe Gly Asn Gly Arg Cys Thr Leu Asp Gln Asn Gly Trp His Cys
    770                 775                 780

Val Cys Gln Val Gly Trp Ser Gly Thr Gly Cys Asn Val Val Met Glu
785                 790                 795                 800

Met Leu Cys Gly Asp Asn Leu Asp Asn Asp Gly Asp Gly Leu Thr Asp
                805                 810                 815

Cys Val Asp Pro Asp Cys Cys Gln Gln Ser Asn Cys Tyr Ile Ser Pro
            820                 825                 830

Leu Cys Gln Gly Ser Pro Asp Pro Leu Asp Leu Ile Gln Gln Ser Gln
        835                 840                 845

Thr Leu Phe Ser Gln His Thr Ser Arg Leu Phe Tyr Asp Arg Ile Lys
    850                 855                 860

Phe Leu Ile Gly Lys Asp Ser Thr His Val Ile Pro Pro Glu Val Ser
865                 870                 875                 880

Phe Asp Ser Arg Arg Ala Cys Val Ile Arg Gly Gln Val Val Ala Ile
                885                 890                 895

Asp Gly Thr Pro Leu Val Gly Val Asn Val Ser Phe Leu His His Ser
            900                 905                 910

Asp Tyr Gly Phe Thr Ile Ser Arg Gln Asp Gly Ser Phe Asp Leu Val
        915                 920                 925

Ala Ile Gly Gly Ile Ser Val Ile Leu Ile Phe Asp Arg Ser Pro Phe
    930                 935                 940

Leu Pro Glu Lys Arg Thr Leu Trp Leu Pro Trp Asn Gln Phe Ile Val
945                 950                 955                 960

Val Glu Lys Val Thr Met Gln Arg Val Val Ser Asp Pro Pro Ser Cys
                965                 970                 975

Asp Ile Ser Asn Phe Ile Ser Pro Asn Pro Ile Val Leu Pro Ser Pro
            980                 985                 990

Leu Thr Ser Phe Gly Gly Ser Cys  Pro Glu Arg Gly Thr  Ile Val Pro
        995                 1000                1005

Glu Leu  Gln Val Val Gln Glu  Glu Ile Pro Ile Pro  Ser Ser Phe
    1010                 1015                 1020

Val Arg  Leu Ser Tyr Leu Ser  Ser Arg Thr Pro Gly  Tyr Lys Thr
    1025                 1030                 1035

Leu Leu  Arg Ile Leu Leu Thr  His Ser Thr Ile Pro  Val Gly Met
    1040                 1045                 1050

Ile Lys  Val His Leu Thr Val  Ala Val Glu Gly Arg  Leu Thr Gln
    1055                 1060                 1065

Lys Trp  Phe Pro Ala Ala Ile  Asn Leu Val Tyr Thr  Phe Ala Trp
    1070                 1075                 1080

Asn Lys  Thr Asp Ile Tyr Gly  Gln Lys Val Trp Gly  Leu Ala Glu
    1085                 1090                 1095

Ala Leu  Val Ser Val Gly Tyr  Glu Tyr Glu Thr Cys  Pro Asp Phe
    1100                 1105                 1110

Ile Leu  Trp Glu Gln Arg Thr  Val Val Leu Gln Gly  Phe Glu Met
    1115                 1120                 1125

Asp Ala  Ser Asn Leu Gly Asp  Trp Ser Leu Asn Lys  His His Ile
    1130                 1135                 1140

Leu Asn  Pro Gln Ser Gly Ile  Ile His Lys Gly Asn  Gly Glu Asn
    1145                 1150                 1155

Met Phe  Ile Ser Gln Gln Pro  Pro Val Ile Ser Thr  Ile Met Gly
    1160                 1165                 1170

Asn Gly  His Gln Arg Ser Val  Ala Cys Thr Asn Cys  Asn Gly Pro
    1175                 1180                 1185

Ala His  Asn Asn Lys Leu Phe  Ala Pro Val Ala Leu  Ala Ser Gly
    1190                 1195                 1200

Pro Asp  Gly Ser Val Tyr Val  Gly Asp Phe Asn Phe  Val Arg Arg
    1205                 1210                 1215

Ile Phe  Pro Ser Gly Asn Ser  Val Ser Ile Leu Glu  Leu Ser Thr
    1220                 1225                 1230

Ser Pro  Ala His Lys Tyr Tyr  Leu Ala Met Asp Pro  Val Ser Glu
    1235                 1240                 1245

Ser Leu  Tyr Leu Ser Asp Thr  Asn Thr Arg Lys Val  Tyr Lys Leu

1250                    1255                    1260

Lys Ser Leu Val Glu Thr Lys Asp Leu Ser Lys Asn Phe Glu Val
    1265                    1270                    1275

Val Ala Gly Thr Gly Asp Gln Cys Leu Pro Phe Asp Gln Ser His
    1280                    1285                    1290

Cys Gly Asp Gly Gly Arg Ala Ser Glu Ala Ser Leu Asn Ser Pro
    1295                    1300                    1305

Arg Gly Ile Thr Val Asp Arg His Gly Phe Ile Tyr Phe Val Asp
    1310                    1315                    1320

Gly Thr Met Ile Arg Lys Ile Asp Glu Asn Ala Val Ile Thr Thr
    1325                    1330                    1335

Val Ile Gly Ser Asn Gly Leu Thr Ser Thr Gln Pro Leu Ser Cys
    1340                    1345                    1350

Asp Ser Gly Met Asp Ile Thr Gln Val Arg Leu Glu Trp Pro Thr
    1355                    1360                    1365

Asp Leu Ala Val Asn Pro Met Asp Asn Ser Leu Tyr Val Leu Asp
    1370                    1375                    1380

Asn Asn Ile Val Leu Gln Ile Ser Glu Asn Arg Arg Val Arg Ile
    1385                    1390                    1395

Ile Ala Gly Arg Pro Ile His Cys Gln Val Pro Gly Ile Asp His
    1400                    1405                    1410

Phe Leu Val Ser Lys Val Ala Ile His Ser Thr Leu Glu Ser Ala
    1415                    1420                    1425

Arg Ala Ile Ser Val Ser His Ser Gly Leu Leu Phe Ile Ala Glu
    1430                    1435                    1440

Thr Asp Glu Arg Lys Val Asn Arg Ile Gln Gln Val Thr Thr Asn
    1445                    1450                    1455

Gly Glu Ile Tyr Ile Ile Ala Gly Ala Pro Thr Asp Cys Asp Cys
    1460                    1465                    1470

Lys Ile Asp Pro Asn Cys Asp Cys Phe Ser Gly Asp Gly Gly Tyr
    1475                    1480                    1485

Ala Lys Asp Ala Lys Met Lys Ala Pro Ser Ser Leu Ala Val Ser
    1490                    1495                    1500

Pro Asp Gly Thr Leu Tyr Val Ala Asp Leu Gly Asn Val Arg Ile
    1505                    1510                    1515

Arg Thr Ile Ser Arg Asn Gln Ala His Leu Asn Asp Met Asn Ile
    1520                    1525                    1530

Tyr Glu Ile Ala Ser Pro Ala Asp Gln Glu Leu Tyr Gln Phe Thr
    1535                    1540                    1545

Val Asn Gly Thr His Leu His Thr Leu Asn Leu Ile Thr Arg Asp
    1550                    1555                    1560

Tyr Val Tyr Asn Phe Thr Tyr Asn Ser Glu Gly Asp Leu Gly Ala
    1565                    1570                    1575

Ile Thr Ser Ser Asn Gly Asn Ser Val His Ile Arg Arg Asp Ala
    1580                    1585                    1590

Gly Gly Met Pro Leu Trp Leu Val Val Pro Gly Gly Gln Val Tyr
    1595                1600                1605

Trp Leu Thr Ile Ser Ser Asn Gly Val Leu Lys Arg Val Ser Ala
    1610                1615                1620

Gln Gly Tyr Asn Pro Ala Leu Met Thr Tyr Pro Gly Asn Thr Gly
    1625                1630                1635

Leu Leu Ala Thr Lys Ser Asn Glu Asn Gly Trp Thr Thr Val Tyr
    1640                1645                1650

Glu Tyr Asp Pro Glu Gly His Leu Thr Asn Ala Thr Phe Pro Thr
    1655                1660                1665

Gly Glu Val Ser Ser Phe His Ser Asp Leu Glu Lys Leu Thr Lys
    1670                1675                1680

Val Glu Leu Asp Thr Ser Asn Arg Glu Asn Val Leu Met Ser Thr
    1685                1690                1695

Asn Leu Thr Ala Thr Ser Thr Ile Tyr Ile Leu Lys Gln Glu Asn
    1700                1705                1710

Thr Gln Ser Thr Tyr Arg Val Asn Pro Asp Gly Ser Leu Arg Val
    1715                1720                1725

Thr Phe Ala Ser Gly Met Glu Ile Gly Leu Ser Ser Glu Pro His
    1730                1735                1740

Ile Leu Ala Gly Ala Val Asn Pro Thr Leu Gly Lys Cys Asn Ile
    1745                1750                1755

Ser Leu Pro Gly Glu His Asn Ala Asn Leu Ile Glu Trp Arg Gln
    1760                1765                1770

Arg Lys Glu Gln Asn Lys Gly Asn Val Ser Ala Phe Glu Arg Arg
    1775                1780                1785

Leu Arg Ala His Asn Arg Asn Leu Leu Ser Ile Asp Phe Asp His
    1790                1795                1800

Ile Thr Arg Thr Gly Lys Ile Tyr Asp Asp His Arg Lys Phe Thr
    1805                1810                1815

Leu Arg Ile Leu Tyr Asp Gln Thr Gly Arg Pro Ile Leu Trp Ser
    1820                1825                1830

Pro Val Ser Arg Tyr Asn Glu Val Asn Ile Thr Tyr Ser Pro Ser
    1835                1840                1845

Gly Leu Val Thr Phe Ile Gln Arg Gly Thr Trp Asn Glu Lys Met
    1850                1855                1860

Glu Tyr Asp Gln Ser Gly Lys Ile Ile Ser Arg Thr Trp Ala Asp
    1865                1870                1875

Gly Lys Ile Trp Ser Tyr Thr Tyr Leu Glu Lys Ser Val Met Leu
    1880                1885                1890

Leu Leu His Ser Gln Arg Arg Tyr Ile Phe Glu Tyr Asp Gln Ser
    1895                1900                1905

Asp Cys Leu Leu Ser Val Thr Met Pro Ser Met Val Arg His Ser
    1910                1915                1920

Leu Gln Thr Met Leu Ser Val Gly Tyr Tyr Arg Asn Ile Tyr Thr

1925                    1930                    1935

Pro Pro Asp Ser Ser Thr Ser Phe Ile Gln Asp Tyr Ser Arg Asp
    1940                    1945                    1950

Gly Arg Leu Leu Gln Thr Leu His Leu Gly Thr Gly Arg Arg Val
    1955                    1960                    1965

Leu Tyr Lys Tyr Thr Lys Gln Ala Arg Leu Ser Glu Val Leu Tyr
    1970                    1975                    1980

Asp Thr Thr Gln Val Thr Leu Thr Tyr Glu Glu Ser Ser Gly Val
    1985                    1990                    1995

Ile Lys Thr Ile His Leu Met His Asp Gly Phe Ile Cys Thr Ile
    2000                    2005                    2010

Arg Tyr Arg Gln Thr Gly Pro Leu Ile Gly Arg Gln Ile Phe Arg
    2015                    2020                    2025

Phe Ser Glu Glu Gly Leu Val Asn Ala Arg Phe Asp Tyr Ser Tyr
    2030                    2035                    2040

Asn Asn Phe Arg Val Thr Ser Met Gln Ala Val Ile Asn Glu Thr
    2045                    2050                    2055

Pro Leu Pro Ile Asp Leu Tyr Arg Tyr Val Asp Val Ser Gly Arg
    2060                    2065                    2070

Thr Glu Gln Phe Gly Lys Phe Ser Val Ile Asn Tyr Asp Leu Asn
    2075                    2080                    2085

Gln Val Ile Thr Thr Thr Val Met Lys His Thr Lys Ile Phe Ser
    2090                    2095                    2100

Ala Asn Gly Gln Val Ile Glu Val Gln Tyr Glu Ile Leu Lys Ala
    2105                    2110                    2115

Ile Ala Tyr Trp Met Thr Ile Gln Tyr Asp Asn Val Gly Arg His
    2120                    2125                    2130

Gly Asn Met Cys Ile Arg Val Gly Val Asp Ala Asn Ile Thr Arg
    2135                    2140                    2145

Tyr Phe Tyr Glu Tyr Asp Ala Asp Gly Gln Leu Gln Thr Val Ser
    2150                    2155                    2160

Val Asn Asp Lys Thr Gln Trp Arg Tyr Ser Tyr Asp Leu Asn Gly
    2165                    2170                    2175

Asp Ile Asn Leu Leu Ser His Gly Lys Ser Ala Arg Leu Thr Pro
    2180                    2185                    2190

Leu Arg Tyr Asp Leu Arg Asp Arg Ile Thr Arg Leu Gly Glu Ile
    2195                    2200                    2205

Gln Tyr Lys Met Asp Glu Asp Gly Phe Leu Arg Gln Arg Gly Asn
    2210                    2215                    2220

Asp Ile Phe Glu Tyr Asn Ser Asn Gly Leu Leu Gln Lys Ala Tyr
    2225                    2230                    2235

Asn Lys Ala Ser Gly Trp Thr Val Gln Tyr Tyr Tyr Asp Gly Leu
    2240                    2245                    2250

Gly Arg Arg Val Ala Ser Lys Ser Ser Leu Gly Gln His Leu Gln
    2255                    2260                    2265

Phe Phe Val Asp Ala Thr Ala Asn Pro Ile Arg Val Thr His Leu
    2270                2275                2280

Tyr Asn His Thr Ser Ser Glu Ile Thr Ser Leu Tyr Tyr Asp Leu
    2285                2290                2295

Gln Gly His Leu Ile Ala Met Glu Leu Ser Ser Gly Glu Glu Tyr
    2300                2305                2310

Tyr Val Ala Cys Asp Asn Thr Gly Thr Pro Leu Ala Val Phe Ser
    2315                2320                2325

Ser Arg Gly Gln Val Ile Lys Glu Ile Leu Tyr Thr Pro Tyr Gly
    2330                2335                2340

Asp Ile Tyr His Asp Thr Tyr Pro Asp Phe Gln Val Ile Ile Gly
    2345                2350                2355

Phe His Gly Gly Leu Tyr Asp Phe Leu Thr Lys Leu Val His Leu
    2360                2365                2370

Gly Gln Arg Asp Tyr Asp Val Val Ala Gly Arg Trp Thr Thr Ala
    2375                2380                2385

Tyr His His Ile Trp Lys Gln Leu Asn Leu Leu Pro Lys Pro Phe
    2390                2395                2400

Asn Leu Tyr Ser Phe Glu Asn Asn Tyr Pro Val Gly Lys Ile Gln
    2405                2410                2415

Asp Val Ala Lys Tyr Thr Thr Asp Ile Arg Ser Trp Leu Glu Leu
    2420                2425                2430

Phe Gly Phe Gln Leu His Asn Val Leu Pro Gly Phe Pro Lys Pro
    2435                2440                2445

Glu Leu Glu Asn Leu Glu Leu Thr Tyr Glu Leu Leu Arg Leu Gln
    2450                2455                2460

Thr Lys Thr Gln Glu Trp Asp Pro Gly Lys Thr Ile Leu Gly Ile
    2465                2470                2475

Gln Cys Glu Leu Gln Lys Gln Leu Arg Asn Phe Ile Ser Leu Asp
    2480                2485                2490

Gln Leu Pro Met Thr Pro Arg Tyr Asn Asp Gly Arg Cys Leu Glu
    2495                2500                2505

Gly Gly Lys Gln Pro Arg Phe Ala Ala Val Pro Ser Val Phe Gly
    2510                2515                2520

Lys Gly Ile Lys Phe Ala Ile Lys Asp Gly Ile Val Thr Ala Asp
    2525                2530                2535

Ile Ile Gly Val Ala Asn Glu Asp Ser Arg Arg Leu Ala Ala Ile
    2540                2545                2550

Leu Asn Asn Ala His Tyr Leu Glu Asn Leu His Phe Thr Ile Glu
    2555                2560                2565

Gly Arg Asp Thr His Tyr Phe Ile Lys Leu Gly Ser Leu Glu Glu
    2570                2575                2580

Asp Leu Val Leu Ile Gly Asn Thr Gly Gly Arg Arg Ile Leu Glu
    2585                2590                2595

Asn Gly Val Asn Val Thr Val Ser Gln Met Thr Ser Leu Leu Asn

2600                    2605                    2610

Gly Arg Thr Arg Arg Phe Ala Asp Ile Gln Leu Gln His Gly Ala
    2615                2620                2625

Leu Cys Phe Asn Ile Arg Tyr Gly Thr Thr Val Glu Glu Glu Lys
    2630                2635                2640

Asn His Val Leu Glu Ile Ala Arg Gln Arg Ala Val Ala Gln Ala
    2645                2650                2655

Trp Thr Lys Glu Gln Arg Arg Leu Gln Glu Gly Glu Glu Gly Ile
    2660                2665                2670

Arg Ala Trp Thr Glu Gly Glu Lys Gln Gln Leu Leu Ser Thr Gly
    2675                2680                2685

Arg Val Gln Gly Tyr Asp Gly Tyr Phe Val Leu Ser Val Glu Gln
    2690                2695                2700

Tyr Leu Glu Leu Ser Asp Ser Ala Asn Asn Ile His Phe Met Arg
    2705                2710                2715

Gln Ser Glu Ile Gly Arg Arg
    2720                2725

<210>  231
<211>  4930
<212>  DNA
<213>  Homo sapiens

<400>  231
ctcagccttc ccggttcggg aaaggggaag aatgcaggag gggtaggatt tctttcctga      60

taggatcggt tgggaaagac cgcagcctgt gtgtgtcttt cccttcgacc aaggtgtctg     120

ttgctccgta aataaaacgt cccactgcct tctgagagcg ctataaaggc agcggaaggg     180

tagtccgcgg ggcattccgg gcggggcgcg agcagagaca ggtcatggca gcgccaggcg     240

gcaggtcgga gccgccgcag ctccccgagt acagctgcag ctacatggtg tcgcggccgg     300

tctacagcga gctcgctttc cagcaacagc acgagcggcg cctgcaggag cgcaagacgc     360

tgcgggagag cctggccaag tgctgcagtt gttcaagaaa gagagccttt ggtgtgctaa     420

agactcttgt gcccatcttg gagtggctcc ccaaataccg agtcaaggaa tggctgctta     480

gtgacgtcat ttcgggagtt agtactgggc tagtggccac gctgcaaggg atggcatatg     540

ccctactagc tgcagttcct gtcggatatg gtctctactc tgcttttttc cctatcctga     600

catactttat ctttggaaca tcaagacata tctcagttgg accttttcca gtggtgagtt     660

taatggtggg atctgttgtt ctgagcatgg cccccgacga acactttctc gtatccagca     720

gcaatggaac tgtattaaat actactatga tagacactgc agctagagat acagctagag     780

tcctgattgc cagtgccctg actctgctgg ttggaattat acagttgata tttggtggct     840

tgcagattgg attcatagtg aggtacttgg cagatccttt ggttggtggc ttcacaacag     900

ctgctgcctt ccaagtgctg gtctcacagc taaagattgt cctcaatgtt tcaaccaaaa     960

actacaatgg agttctctct attatctata cgctggttga gatttttcaa aatattggtg    1020

ataccaatct tgctgatttc actgctggat tgctcaccat tgtcgtctgt atggcagtta    1080

aggaattaaa tgatcggttt agacacaaaa tcccagtccc tattcctata gaagtaattg 1140
tgacgataat tgctactgcc atttcatatg gagccaacct ggaaaaaaat tacaatgctg 1200
gcattgttaa atccatccca agggggtttt tgcctcctga acttccacct gtgagcttgt 1260
tctcggagat gctggctgca tcattttcca tcgctgtggt ggcttatgct attgcagtgt 1320
cagtaggaaa agtatatgcc accaagtatg attacaccat cgatgggaac caggaattca 1380
ttgcctttgg gatcagcaac atcttctcag gattcttctc ttgttttgtg gccaccactg 1440
ctctttcccg cacggccgtc caggagagca ctggaggaaa gacacaggtt gctggcatca 1500
tctctgctgc gattgtgatg atcgccattc ttgccctggg gaagcttctg gaacccttgc 1560
agaagtcggt cttggcagct gttgtaattg ccaacctgaa agggatgttt atgcagctgt 1620
gtgacattcc tcgtctgtgg agacagaata agattgatgc tgttatctgg gtgtttacgt 1680
gtatagtgtc catcattctg gggctggatc tcggtttact agctggcctt atatttggac 1740
tgttgactgt ggtcctgaga gttcagtttc cttcttggaa tggccttgga agcatcccta 1800
gcacagatat ctacaaaagt accaagaatt acaaaaacat tgaagaacct caaggagtga 1860
agattcttag attttccagt cctattttct atggcaatgt cgatggtttt aaaaaatgta 1920
tcaagtccac agttggattt gatgccatta gagtatataa taagaggctg aaagcgctga 1980
ggaaaataca gaaactaata aaaagtggac aattaagagc aacaaagaat ggcatcataa 2040
gtgatgctgt ttcaacaaat aatgcttttg agcctgatga ggatattgaa gatctggagg 2100
aacttgatat cccaaccaag gaaatagaga ttcaagtgga ttggaactct gagcttccag 2160
tcaaagtgaa cgttcccaaa gtgccaatcc atagccttgt gcttgactgt ggagctatat 2220
ctttcctgga cgttgttgga gtgagatcac tgcgggtgat tgtcaaagaa ttccaaagaa 2280
ttgatgtgaa tgtgtatttt gcatcacttc aagattatgt gatagaaaag ctggagcaat 2340
gcgggttctt tgacgacaac attagaaagg acacattctt tttgacggtc catgatgcta 2400
tactctatct acagaaccaa gtgaaatctc aagagggtca aggttccatt ttagaaacga 2460
tcactctcat tcaggattgt aaagataccc ttgaattaat agaaacagag ctgacggaag 2520
aagaacttga tgtccaggat gaggctatgc gtacacttgc atcctgaaag tgggttcggg 2580
aggtctctat gagcaaggaa tacaagacaa aacttcctca atgcattgac tatttcttca 2640
gactcaaaac actcattctt ttttctatta agccattgaa agagaagcac taagactgct 2700
tctaggcttt atttataaaa taaacacctt atccctaaca tgggcaaaat ggctagaatt 2760
attcagacga tttggcagcg tccagggtaa gctggtgtta taatacgctg ctgatctaca 2820
tcacagattt gctaataatg ttcacgtggg ccctggcata tctctgttca gttagagtga 2880
gtgctgaccc aacagcctct gtggtcaagc gagtcacgaa tgattaatca taaagaaaaa 2940
tcagtttttg actgacctgg atatccatga gctgcactga tcaccatgta aggtcacatt 3000
tagtaaatgc tgaaataaaa tgattaatgc atttatcaat aaaagccttt gaaaatactt 3060
tggataataa attggagttt taaaaatgca aatttgctta gtatctaata atgaagtgtt 3120

attacatata gccggaattg aggatctctt tgatcctgga aatggtttac ctaaaagcta 3180
cagaaccagg ccaatatatt ttgaaatatt gatgcagaca aatgaaataa taaagagatt 3240
ttcatggttt ataaaaatct tttttgatat gataataatc atgatcacaa ctgagatcaa 3300
aaaaatatat gacagattat tttgtttaaa aatgcagttt taattatctt agtctataga 3360
aatgatcatt gcatggaggc atgtataggt atgatctgtg taaaatctga cataaaaaca 3420
gtgctattct gagtgaaaat ttttttgatg tgcttacata accatggtga ttaaaatgag 3480
tttatatttt ttctcaaaaa ttttagcagt gtgtaaagta agtaatcttt aactgaactc 3540
tgaccactta aaaaaaaatc taaaaattga actacctata gtagtctgtg tttaaagtga 3600
atttttaaag acaaagcatt ctaaatgaac tcaatataaa aacattcatt tggaatgtac 3660
atactgaaaa atacaggttt ttttgaccaa aagtttttat atcttttctt tttatttatt 3720
tttttcctaa gtgccaacaa ttttctagat attatataca acacaggctt tgatcttggg 3780
gacttttccc atatatttca cactggagtg aatgaagttg tacttcattt ctagagaaaa 3840
gttataccca ggtccccaat tgagaatgtc ttgcttgatt gaaaacgaca tcatcccttg 3900
gtatactcca gggattggtt tcaggacccc tgcatttacc aaaatttgtg cacactcaag 3960
tcctgcagtc acccctgcct aaagatagaa tggcttctct gtttttcttc tgaaatacaa 4020
ccagaaacaa tgtgtctatt tctgaaagaa taggattaat gatcatacaa atgggttaat 4080
cctgaattct ggttgtaaat ctggttacag cataactagg attataatgc tgcctcattt 4140
tcacagcact acttgcttat attgacaaca aatcatctcg ctaaagagtg aatgtaggcc 4200
aggcgcggtg gctcatgcct gtaatcccag cactttggga ggccgaggcg ggtggatcac 4260
gaggtcagga gatcgagacc atcctggcta acatggtaaa accccgtctc tactaaaaat 4320
agaaaaaaag aaattagcct agcgtggtgg ctggcgggcg cctgtagtcc cagctatttg 4380
ggaggctaag gcaggagaat ggcgtgaacc cgggaggcgg agcttgcagt gagccgaggt 4440
cgtgccactg cactccagcc tgggcgacag agcaagactc cgtctcaaaa aaaaaaaaaa 4500
aaaaaaaaaa agagtgaatg taatagtctt gcagaaaatg aatgaatacc tttgttcaat 4560
aaaggaaata tgcactgctc acttttttga aggaaatgcc aaagttacgt tttacaacaa 4620
ggctagagtt tgtaaattct gggttcattt gtgatgacat aagtcagcaa actgcgggaa 4680
tactgtctct tctatgtatt ttgtgaatag taagcataat tttagttttg tattatcaat 4740
gaaaatttca cttgaaatta aagctgcctt ttgttatatt tttaacctat aggataagat 4800
tccagtattg tatatgagtt ttaacaaatt aaaaaatcaa atcatgtaca tttgaaaata 4860
tttgcacaca tttaaaaata aatgtaaagt tgtcttttaa actactcgga tgtgtccttt 4920
ctgaacaaaa 4930

<210> 232
<211> 790
<212> PRT
<213> Homo sapiens

<400> 232

Met Ala Ala Pro Gly Gly Arg Ser Glu Pro Pro Gln Leu Pro Glu Tyr
1               5                   10                  15

Ser Cys Ser Tyr Met Val Ser Arg Pro Val Tyr Ser Glu Leu Ala Phe
            20                  25                  30

Gln Gln Gln His Glu Arg Arg Leu Gln Glu Arg Lys Thr Leu Arg Glu
        35                  40                  45

Ser Leu Ala Lys Cys Cys Ser Cys Ser Arg Lys Arg Ala Phe Gly Val
    50                  55                  60

Leu Lys Thr Leu Val Pro Ile Leu Glu Trp Leu Pro Lys Tyr Arg Val
65                  70                  75                  80

Lys Glu Trp Leu Leu Ser Asp Val Ile Ser Gly Val Ser Thr Gly Leu
                85                  90                  95

Val Ala Thr Leu Gln Gly Met Ala Tyr Ala Leu Leu Ala Ala Val Pro
            100                 105                 110

Val Gly Tyr Gly Leu Tyr Ser Ala Phe Phe Pro Ile Leu Thr Tyr Phe
        115                 120                 125

Ile Phe Gly Thr Ser Arg His Ile Ser Val Gly Pro Phe Pro Val Val
    130                 135                 140

Ser Leu Met Val Gly Ser Val Val Leu Ser Met Ala Pro Asp Glu His
145                 150                 155                 160

Phe Leu Val Ser Ser Ser Asn Gly Thr Val Leu Asn Thr Thr Met Ile
                165                 170                 175

Asp Thr Ala Ala Arg Asp Thr Ala Arg Val Leu Ile Ala Ser Ala Leu
            180                 185                 190

Thr Leu Leu Val Gly Ile Ile Gln Leu Ile Phe Gly Gly Leu Gln Ile
        195                 200                 205

Gly Phe Ile Val Arg Tyr Leu Ala Asp Pro Leu Val Gly Gly Phe Thr
    210                 215                 220

Thr Ala Ala Ala Phe Gln Val Leu Val Ser Gln Leu Lys Ile Val Leu
225                 230                 235                 240

Asn Val Ser Thr Lys Asn Tyr Asn Gly Val Leu Ser Ile Ile Tyr Thr
                245                 250                 255

Leu Val Glu Ile Phe Gln Asn Ile Gly Asp Thr Asn Leu Ala Asp Phe
            260                 265                 270

Thr Ala Gly Leu Leu Thr Ile Val Val Cys Met Ala Val Lys Glu Leu
        275                 280                 285

Asn Asp Arg Phe Arg His Lys Ile Pro Val Pro Ile Pro Ile Glu Val
    290                 295                 300

Ile Val Thr Ile Ile Ala Thr Ala Ile Ser Tyr Gly Ala Asn Leu Glu
305                 310                 315                 320

Lys Asn Tyr Asn Ala Gly Ile Val Lys Ser Ile Pro Arg Gly Phe Leu
                325                 330                 335

Pro Pro Glu Leu Pro Pro Val Ser Leu Phe Ser Glu Met Leu Ala Ala
            340                 345                 350

Ser Phe Ser Ile Ala Val Val Ala Tyr Ala Ile Ala Val Ser Val Gly

        355                 360                 365

Lys Val Tyr Ala Thr Lys Tyr Asp Tyr Thr Ile Asp Gly Asn Gln Glu
    370                 375                 380

Phe Ile Ala Phe Gly Ile Ser Asn Ile Phe Ser Gly Phe Phe Ser Cys
385                 390                 395                 400

Phe Val Ala Thr Thr Ala Leu Ser Arg Thr Ala Val Gln Glu Ser Thr
                405                 410                 415

Gly Gly Lys Thr Gln Val Ala Gly Ile Ile Ser Ala Ala Ile Val Met
            420                 425                 430

Ile Ala Ile Leu Ala Leu Gly Lys Leu Leu Glu Pro Leu Gln Lys Ser
        435                 440                 445

Val Leu Ala Ala Val Val Ile Ala Asn Leu Lys Gly Met Phe Met Gln
    450                 455                 460

Leu Cys Asp Ile Pro Arg Leu Trp Arg Gln Asn Lys Ile Asp Ala Val
465                 470                 475                 480

Ile Trp Val Phe Thr Cys Ile Val Ser Ile Ile Leu Gly Leu Asp Leu
                485                 490                 495

Gly Leu Leu Ala Gly Leu Ile Phe Gly Leu Leu Thr Val Val Leu Arg
            500                 505                 510

Val Gln Phe Pro Ser Trp Asn Gly Leu Gly Ser Ile Pro Ser Thr Asp
        515                 520                 525

Ile Tyr Lys Ser Thr Lys Asn Tyr Lys Asn Ile Glu Glu Pro Gln Gly
    530                 535                 540

Val Lys Ile Leu Arg Phe Ser Ser Pro Ile Phe Tyr Gly Asn Val Asp
545                 550                 555                 560

Gly Phe Lys Lys Cys Ile Lys Ser Thr Val Gly Phe Asp Ala Ile Arg
                565                 570                 575

Val Tyr Asn Lys Arg Leu Lys Ala Leu Arg Lys Ile Gln Lys Leu Ile
            580                 585                 590

Lys Ser Gly Gln Leu Arg Ala Thr Lys Asn Gly Ile Ile Ser Asp Ala
        595                 600                 605

Val Ser Thr Asn Asn Ala Phe Glu Pro Asp Glu Asp Ile Glu Asp Leu
    610                 615                 620

Glu Glu Leu Asp Ile Pro Thr Lys Glu Ile Glu Ile Gln Val Asp Trp
625                 630                 635                 640

Asn Ser Glu Leu Pro Val Lys Val Asn Val Pro Lys Val Pro Ile His
                645                 650                 655

Ser Leu Val Leu Asp Cys Gly Ala Ile Ser Phe Leu Asp Val Val Gly
            660                 665                 670

Val Arg Ser Leu Arg Val Ile Val Lys Glu Phe Gln Arg Ile Asp Val
        675                 680                 685

Asn Val Tyr Phe Ala Ser Leu Gln Asp Tyr Val Ile Glu Lys Leu Glu
    690                 695                 700

Gln Cys Gly Phe Phe Asp Asp Asn Ile Arg Lys Asp Thr Phe Phe Leu
705                 710                 715                 720

Thr Val His Asp Ala Ile Leu Tyr Leu Gln Asn Gln Val Lys Ser Gln
                725                 730                 735

Glu Gly Gln Gly Ser Ile Leu Glu Thr Ile Thr Leu Ile Gln Asp Cys
            740                 745                 750

Lys Asp Thr Leu Glu Leu Ile Glu Thr Glu Leu Thr Glu Glu Glu Leu
        755                 760                 765

Asp Val Gln Asp Glu Ala Met Arg Thr Leu Ala Ser Gln Asp Glu Ala
    770                 775                 780

Met Arg Thr Leu Ala Ser
785                 790

<210>  233
<211>  1282
<212>  DNA
<213>  Homo sapiens

<400>  233
ctgcgatccc gcagggcagc gacgcgactc tggtgcgggc cgtcttcttc cccccgagct      60

gggcgtgcgc ggccgcaatg aactgggagc tgctgctgtg gctgctggtg ctgtgcgcgc     120

tgctcctgct cttggtgcag ctgctgcgct tcctgagggc tgacggcgac ctgacgctac     180

tatgggccga gtggcaggga cgacgcccag aatgggagct gactgatatg gtggtgtggg     240

tgactggagc ctcgagtgga attggtgagg agctggctta ccagttgtct aaactaggag     300

tttctcttgt gctgtcagcc agaagagtgc atgagctgga aagggtgaaa agaagatgcc     360

tagagaatgg caatttaaaa gaaaaagata tacttgtttt gccccttgac ctgaccgaca     420

ctggttccca tgaagcggct accaaagctg ttctccagga gtttggtaga atcgacattc     480

tggtcaacaa tggtggaatg tcccagcgtt ctctgtgcat ggataccagc ttggatgtct     540

acagaaagct aatagagctt aactacttag ggacggtgtc cttgacaaaa tgtgttctgc     600

ctcacatgat cgagaggaag caaggaaaga ttgttactgt gaatagcatc ctgggtatca     660

tatctgtacc tctttccatt ggatactgtg ctagcaagca tgctctccgg ggttttttta      720

atggccttcg aacagaactt gccacatacc caggtataat agtttctaac atttgcccag     780

gacctgtgca atcaaatatt gtggagaatt ccctagctgg agaagtcaca aagactatag     840

gcaataatgg agaccagtcc cacaagatga caaccagtcg ttgtgtgcgg ctgatgttaa     900

tcagcatggc caatgatttg aaagaagttt ggatctcaga acaacctttc ttgttagtaa     960

catatttgtg gcaatacatg ccaacctggg cctggtggat aaccaacaag atggggaaga    1020

aaaggattga gaactttaag agtggtgtgg atgcagactc ttcttatttt aaaatcttta    1080

agacaaaaca tgactgaaaa gagcacctgt acttttcaag ccactggagg gagaaatgga    1140

aaacatgaaa acagcaatct tcttatgctt ctgaataatc aaagactaat ttgtgatttt    1200

actttttaat agatatgact ttgcttccaa catggaatga aataaaaaat aaataataaa    1260

agattgccat gaatcttgca aa                                              1282

<210>  234
<211>  339
<212>  PRT

<213>  Homo sapiens

<400>  234

Met Asn Trp Glu Leu Leu Leu Trp Leu Leu Val Leu Cys Ala Leu Leu
1               5                   10                  15

Leu Leu Leu Val Gln Leu Leu Arg Phe Leu Arg Ala Asp Gly Asp Leu
            20                  25                  30

Thr Leu Leu Trp Ala Glu Trp Gln Gly Arg Arg Pro Glu Trp Glu Leu
        35                  40                  45

Thr Asp Met Val Val Trp Val Thr Gly Ala Ser Ser Gly Ile Gly Glu
    50                  55                  60

Glu Leu Ala Tyr Gln Leu Ser Lys Leu Gly Val Ser Leu Val Leu Ser
65                  70                  75                  80

Ala Arg Arg Val His Glu Leu Glu Arg Val Lys Arg Arg Cys Leu Glu
                85                  90                  95

Asn Gly Asn Leu Lys Glu Lys Asp Ile Leu Val Leu Pro Leu Asp Leu
            100                 105                 110

Thr Asp Thr Gly Ser His Glu Ala Ala Thr Lys Ala Val Leu Gln Glu
        115                 120                 125

Phe Gly Arg Ile Asp Ile Leu Val Asn Asn Gly Gly Met Ser Gln Arg
    130                 135                 140

Ser Leu Cys Met Asp Thr Ser Leu Asp Val Tyr Arg Lys Leu Ile Glu
145                 150                 155                 160

Leu Asn Tyr Leu Gly Thr Val Ser Leu Thr Lys Cys Val Leu Pro His
                165                 170                 175

Met Ile Glu Arg Lys Gln Gly Lys Ile Val Thr Val Asn Ser Ile Leu
            180                 185                 190

Gly Ile Ile Ser Val Pro Leu Ser Ile Gly Tyr Cys Ala Ser Lys His
        195                 200                 205

Ala Leu Arg Gly Phe Phe Asn Gly Leu Arg Thr Glu Leu Ala Thr Tyr
    210                 215                 220

Pro Gly Ile Ile Val Ser Asn Ile Cys Pro Gly Pro Val Gln Ser Asn
225                 230                 235                 240

Ile Val Glu Asn Ser Leu Ala Gly Glu Val Thr Lys Thr Ile Gly Asn
                245                 250                 255

Asn Gly Asp Gln Ser His Lys Met Thr Thr Ser Arg Cys Val Arg Leu
            260                 265                 270

Met Leu Ile Ser Met Ala Asn Asp Leu Lys Glu Val Trp Ile Ser Glu
        275                 280                 285

Gln Pro Phe Leu Leu Val Thr Tyr Leu Trp Gln Tyr Met Pro Thr Trp
    290                 295                 300

Ala Trp Trp Ile Thr Asn Lys Met Gly Lys Lys Arg Ile Glu Asn Phe
305                 310                 315                 320

Lys Ser Gly Val Asp Ala Asp Ser Ser Tyr Phe Lys Ile Phe Lys Thr
                325                 330                 335

Lys His Asp

<210> 235
<211> 1833
<212> DNA
<213> Homo sapiens

<400> 235
atgattgctt cacagtttct ctcagctctc actttggtgc ttctcattaa agagagtgga 60

gcctggtctt acaacacctc cacggaagct atgacttatg atgaggccag tgcttattgt 120

cagcaaaggt acacacacct ggttgcaatt caaaacaaag aagagattga gtacctaaac 180

tccatattga gctattcacc aagttattac tggattggaa tcagaaaagt caacaatgtg 240

tgggtctggg taggaaccca gaaacctctg acagaagaag ccaagaactg ggctccaggt 300

gaacccaaca ataggcaaaa agatgaggac tgcgtggaga tctacatcaa gagagaaaaa 360

gatgtgggca tgtggaatga tgagaggtgc agcaagaaga agcttgccct atgctacaca 420

gctgcctgta ccaatacatc ctgcagtggc cacggtgaat gtgtagagac catcaataat 480

tacacttgca agtgtgaccc tggcttcagt ggactcaagt gtgagcaaat tgtgaactgt 540

acagccctgg aatcccctga gcatggaagc ctggtttgca gtcacccact gggaaacttc 600

agctacaatt cttcctgctc tatcagctgt gataggggtt acctgccaag cagcatggag 660

accatgcagt gtatgtcctc tggagaatgg agtgctccta ttccagcctg caatgtggtt 720

gagtgtgatg ctgtgacaaa tccagccaat gggttcgtgg aatgtttcca aaaccctgga 780

agcttcccat ggaacacaac ctgtacattt gactgtgaag aaggatttga actaatggga 840

gcccagagcc ttcagtgtac ctcatctggg aattgggaca acgagaagcc aacgtgtaaa 900

gctgtgacat gcagggccgt ccgccagcct cagaatggct ctgtgaggtg cagccattcc 960

cctgctggag agttcacctt caaatcatcc tgcaacttca cctgtgagga aggcttcatg 1020

ttgcagggac cagcccaggt tgaatgcacc actcaagggc agtggacaca gcaaatccca 1080

gtttgtgaag ctttccagtg cacagccttg tccaaccccg agcgaggcta catgaattgt 1140

cttcctagtg cttctggcag tttccgttat gggtccagct gtgagttctc ctgtgagcag 1200

ggttttgtgt tgaagggatc caaaaggctc caatgtggcc ccacagggga gtgggacaac 1260

gagaagccca catgtgaagc tgtgagatgc gatgctgtcc accagccccc gaagggtttg 1320

gtgaggtgtg ctcattcccc tattggagaa ttcacctaca agtcctcttg tgccttcagc 1380

tgtgaggagg gatttgaatt atatggatca actcaacttg agtgcacatc tcagggacaa 1440

tggacagaag aggttccttc ctgccaagtg gtaaaatgtt caagcctggc agttccggga 1500

aagatcaaca tgagctgcag tggggagccc gtgtttggca ctgtgtgcaa gttcgcctgt 1560

cctgaaggat ggacgctcaa tggctctgca gctcggacat gtggagccac aggacactgg 1620

tctggcctgc tacctacctg tgaagctccc actgagtcca acattccctt ggtagctgga 1680

ctttctgctg ctggactctc cctcctgaca ttagcaccat ttctcctctg gcttcggaaa 1740

tgcttacgga aagcaaagaa atttgttcct gccagcagct gccaaagcct tgaatcagac 1800

ggaagctacc aaaagccttc ttacatcctt taa                                    1833

<210>  236
<211>  610
<212>  PRT
<213>  Homo sapiens

<400>  236

Met Ile Ala Ser Gln Phe Leu Ser Ala Leu Thr Leu Val Leu Leu Ile
1               5                   10                  15

Lys Glu Ser Gly Ala Trp Ser Tyr Asn Thr Ser Thr Glu Ala Met Thr
            20                  25                  30

Tyr Asp Glu Ala Ser Ala Tyr Cys Gln Gln Arg Tyr Thr His Leu Val
        35                  40                  45

Ala Ile Gln Asn Lys Glu Glu Ile Glu Tyr Leu Asn Ser Ile Leu Ser
    50                  55                  60

Tyr Ser Pro Ser Tyr Tyr Trp Ile Gly Ile Arg Lys Val Asn Asn Val
65                  70                  75                  80

Trp Val Trp Val Gly Thr Gln Lys Pro Leu Thr Glu Glu Ala Lys Asn
                85                  90                  95

Trp Ala Pro Gly Glu Pro Asn Asn Arg Gln Lys Asp Glu Asp Cys Val
            100                 105                 110

Glu Ile Tyr Ile Lys Arg Glu Lys Asp Val Gly Met Trp Asn Asp Glu
        115                 120                 125

Arg Cys Ser Lys Lys Lys Leu Ala Leu Cys Tyr Thr Ala Ala Cys Thr
    130                 135                 140

Asn Thr Ser Cys Ser Gly His Gly Glu Cys Val Glu Thr Ile Asn Asn
145                 150                 155                 160

Tyr Thr Cys Lys Cys Asp Pro Gly Phe Ser Gly Leu Lys Cys Glu Gln
                165                 170                 175

Ile Val Asn Cys Thr Ala Leu Glu Ser Pro Glu His Gly Ser Leu Val
            180                 185                 190

Cys Ser His Pro Leu Gly Asn Phe Ser Tyr Asn Ser Ser Cys Ser Ile
        195                 200                 205

Ser Cys Asp Arg Gly Tyr Leu Pro Ser Ser Met Glu Thr Met Gln Cys
    210                 215                 220

Met Ser Ser Gly Glu Trp Ser Ala Pro Ile Pro Ala Cys Asn Val Val
225                 230                 235                 240

Glu Cys Asp Ala Val Thr Asn Pro Ala Asn Gly Phe Val Glu Cys Phe
                245                 250                 255

Gln Asn Pro Gly Ser Phe Pro Trp Asn Thr Thr Cys Thr Phe Asp Cys
            260                 265                 270

Glu Glu Gly Phe Glu Leu Met Gly Ala Gln Ser Leu Gln Cys Thr Ser
        275                 280                 285

Ser Gly Asn Trp Asp Asn Glu Lys Pro Thr Cys Lys Ala Val Thr Cys
    290                 295                 300

Arg Ala Val Arg Gln Pro Gln Asn Gly Ser Val Arg Cys Ser His Ser
305                 310                 315                 320

Pro Ala Gly Glu Phe Thr Phe Lys Ser Ser Cys Asn Phe Thr Cys Glu
                325                 330                 335

Glu Gly Phe Met Leu Gln Gly Pro Ala Gln Val Glu Cys Thr Thr Gln
            340                 345                 350

Gly Gln Trp Thr Gln Gln Ile Pro Val Cys Glu Ala Phe Gln Cys Thr
        355                 360                 365

Ala Leu Ser Asn Pro Glu Arg Gly Tyr Met Asn Cys Leu Pro Ser Ala
    370                 375                 380

Ser Gly Ser Phe Arg Tyr Gly Ser Ser Cys Glu Phe Ser Cys Glu Gln
385                 390                 395                 400

Gly Phe Val Leu Lys Gly Ser Lys Arg Leu Gln Cys Gly Pro Thr Gly
                405                 410                 415

Glu Trp Asp Asn Glu Lys Pro Thr Cys Glu Ala Val Arg Cys Asp Ala
            420                 425                 430

Val His Gln Pro Pro Lys Gly Leu Val Arg Cys Ala His Ser Pro Ile
        435                 440                 445

Gly Glu Phe Thr Tyr Lys Ser Ser Cys Ala Phe Ser Cys Glu Glu Gly
    450                 455                 460

Phe Glu Leu Tyr Gly Ser Thr Gln Leu Glu Cys Thr Ser Gln Gly Gln
465                 470                 475                 480

Trp Thr Glu Glu Val Pro Ser Cys Gln Val Val Lys Cys Ser Ser Leu
                485                 490                 495

Ala Val Pro Gly Lys Ile Asn Met Ser Cys Ser Gly Glu Pro Val Phe
            500                 505                 510

Gly Thr Val Cys Lys Phe Ala Cys Pro Glu Gly Trp Thr Leu Asn Gly
        515                 520                 525

Ser Ala Ala Arg Thr Cys Gly Ala Thr Gly His Trp Ser Gly Leu Leu
    530                 535                 540

Pro Thr Cys Glu Ala Pro Thr Glu Ser Asn Ile Pro Leu Val Ala Gly
545                 550                 555                 560

Leu Ser Ala Ala Gly Leu Ser Leu Leu Thr Leu Ala Pro Phe Leu Leu
                565                 570                 575

Trp Leu Arg Lys Cys Leu Arg Lys Ala Lys Lys Phe Val Pro Ala Ser
            580                 585                 590

Ser Cys Gln Ser Leu Glu Ser Asp Gly Ser Tyr Gln Lys Pro Ser Tyr
        595                 600                 605

Ile Leu
    610

<210>  237
<211>  3793
<212>  DNA
<213>  Homo sapiens

<400>  237
atgatgtgtg aagtgatgcc cacgattaat gaggacaccc caatgagcca aagggggtcc      60

caaagcagtg gctcggactc agactcccat tttgagcagc tgatggtgaa tatgctagat     120

gaaagggatc gtcttctaga cacccttcgg gagacccagg aaagcctctc acttgcccag 180
caaagacttc aggatgtcat ctatgaccga gactcactcc agagacagct caattcagcc 240
ctgccacagg atatcgaatc cctaacagga gggctggctg gttctaaggg ggctgatcca 300
ccggaatttg ctgcactgac aaaagaatta aatgcctgca gggaacaact tctagaaaag 360
gaagaagaaa tctctgaact taaagctgaa agaaacaaca caagactatt actggagcat 420
ttggagtgcc ttgtgtcacg acatgaaaga tcactaagaa tgacggtggt aaaacggcaa 480
gcccagtctc cctcaggagt atccagtgaa gttgaagttc tcaaggcact gaaatctttg 540
tttgagcacc acaaggcctt ggatgaaaag gtaagggagc gactgagggt ttctttagaa 600
agagtctctg cactggaaga agaactagct gctgctaatc aggagattgt tgccttgcgt 660
gaacaaaatg ttcatataca aagaaaaatg gcatcaagcg agggatccac agagtcagaa 720
catcttgaag ggatggaacc tggacagaaa gtccatgaga agcgtttgtc caatggttct 780
atagactcaa ccgatgaaac tagtcaaata gttgaactac aagaattgct tgaaaagcaa 840
aactatgaaa tggcccagat gaaagaacgt ttagcagccc tttcttcccg agtgggagag 900
gtggaacagg aagcagagac agcaagaaag gatctcatta aaacagaaga aatgaacacc 960
aagtatcaaa gggacattag ggaggccatg gcacaaaagg aagatatgga agaaagaatt 1020
acaacccttg aaaagcgtta cctcagtgct cagagagaat ctacctccat acatgacatg 1080
aatgataaac tagaaaatga gttagcaaat aaagaagcta tcctacggca gatggaagag 1140
aaaaacagac agttacaaga acgtcttgag ctagctgaac aaaagttgca gcagaccatg 1200
agaaaggctg aaaccttgcc tgaagtagag gctgaactgg ctcagagaat tgcagcccta 1260
accaaggctg aagagagaca tggaaatatt gaagaacgta tgagacattt agagggtcaa 1320
cttgaagaga agaatcaaga acttcaaaga gctaggcaaa gagagaaaat gaatgaggag 1380
cataacaaga gattatcgga tacggttgat agacttctga ctgaatccaa tgaacgccta 1440
caactacact taaaggaaag aatggctgct ctagaagaaa agaatgtttt aattcaagaa 1500
tcagaaactt tcagaaagaa tcttgaagaa tctttacatg ataaggaaag attagcagaa 1560
gaaattgaaa agctgagatc tgaacttgac caattgaaaa tgagaactgg ctctttaatt 1620
gaacccacaa taccaagaac tcatctagac acctcagctg agttgcggta ctcagtggga 1680
tccctagtgg acagccagtc tgattacaga acaactaaag taataagaag accaaggaga 1740
ggccgcatgg gtgtgcgaag agatgagcca aaggtgaaat ctcttgggga tcacgagtgg 1800
aatagaactc aacagattgg agtactaagc agccaccctt ttgaaagtga cactgaaatg 1860
tctgatattg atgatgatga cagagaaaca atttttagct caatggatct tctctctcca 1920
agtggtcatt ccgatgccca gacgctagcc atgatgcttc aggaacaatt ggatgccatc 1980
aacaaagaaa tcaggctaat tcaggaagaa aaagaatcta cagagttgcg tgctgaagaa 2040
attgaaaata gagtggctag tgtgagcctc gaaggcctga atttggcaag ggtccaccca 2100
ggtacctcca ttactgcctc tgttacagct tcatcgctgg ccagttcatc tccccccagt 2160

ggacactcaa ctccaaagct cacccctcga agccctgcca gggaaatgga tcggatggga 2220

gtcatgacac tgccaagtga tctgaggaaa catcggagaa agattgcagt tgtggaagaa 2280

gatggtcgag aggacaaagc aacaattaaa tgtgaaactt ctcctcctcc tacccctaga 2340

gccctcagaa tgactcacac tctcccttct tcctaccaca atgatgctcg aagtagttta 2400

tctgtctctc ttgagccaga aagcctcggg cttggtagtg ccaacagcag ccaagactct 2460

cttcacaaag cccccaagaa gaaaggaatc aagtcttcaa taggacgttt gtttggtaaa 2520

aaagaaaaag ctcgacttgg gcagctccga ggctttatgg agactgaagc tgcagctcag 2580

gagtccctgg ggttaggcaa actcggaact caagctgaga aggatcgaag actaaagaaa 2640

aagcatgaac ttcttgaaga agctcggaga aagggattac cttttgccca gtgggatggg 2700

ccaactgtgg tcgcatggct agagctttgg ttgggaatgc ctgcgtggta cgtggcagcc 2760

tgccgagcca acgtgaagag tggtgccatc atgtctgctt tatctgacac tgagatccag 2820

agagaaattg gaatcagcaa tccactgcat cgcttaaaac ttcgattagc aatccaggag 2880

atggtttccc taacaagtcc ttcagctcct ccaacatctc gaactccttc aggcaacgtt 2940

tgggtgactc atgaagaaat ggaaaatctt gcagctccag caaaaacgaa agaatctgag 3000

gaaggaagct gggcccagtg tccggttttt ctacagaccc tggcttatgg agatatgaat 3060

catgagtgga ttggaaatga atggcttccc agcttggggt tacctcagta cagaagttac 3120

tttatggaat gcttggtaga tgcaagaatg ttagatcacc taacaaaaaa agatctccgt 3180

gtccatttaa aaatggtgga tagtttccat cgaacaagtt tacaatatgg aattatgtgc 3240

ttaaagaggt tgaattatga cagaaaagaa ctagaaagaa gacgggaagc aagccaacat 3300

gaaataaaag acgtgttggt gtggagcaat gaccgaatta ttcgctggat acaagcaatt 3360

ggacttcgag aatatgcaaa taatatactt gagagcggtg tgcatggctc acttatagcc 3420

ctggatgaaa actttgacta cagcagctta actttattat tacagattcc aacacagaac 3480

acccaggcaa ggcagattct tgaaagagaa tacaataacc tcttggccct gggaactgaa 3540

aggcgactgg atgaaagtga tgacaagaac ttcagacgtg gatcaacctg gagaaggcag 3600

tttcctcctc gtgaagtaca tggaatcagc atgatgcctg ggtcctcaga aacattacca 3660

gctggattta ggttaaccac aacctctggg caatcaagaa aaatgacaac agatgttgct 3720

tcatcaagac tgcagaggtt agacaactcc actgttcgca catactcatg tctcgagtaa 3780

gcggccgctt taa 3793

<210> 238
<211> 1259
<212> PRT
<213> Homo sapiens

<400> 238

Met Met Cys Glu Val Met Pro Thr Ile Asn Glu Asp Thr Pro Met Ser
1               5                   10                  15

Gln Arg Gly Ser Gln Ser Ser Gly Ser Asp Ser Asp Ser His Phe Glu
            20                  25                  30

Gln Leu Met Val Asn Met Leu Asp Glu Arg Asp Arg Leu Leu Asp Thr
        35                  40                  45

Leu Arg Glu Thr Gln Glu Ser Leu Ser Leu Ala Gln Gln Arg Leu Gln
    50                  55                  60

Asp Val Ile Tyr Asp Arg Asp Ser Leu Gln Arg Gln Leu Asn Ser Ala
65                  70                  75                  80

Leu Pro Gln Asp Ile Glu Ser Leu Thr Gly Gly Leu Ala Gly Ser Lys
                85                  90                  95

Gly Ala Asp Pro Pro Glu Phe Ala Ala Leu Thr Lys Glu Leu Asn Ala
            100                 105                 110

Cys Arg Glu Gln Leu Leu Glu Lys Glu Glu Glu Ile Ser Glu Leu Lys
        115                 120                 125

Ala Glu Arg Asn Asn Thr Arg Leu Leu Leu Glu His Leu Glu Cys Leu
    130                 135                 140

Val Ser Arg His Glu Arg Ser Leu Arg Met Thr Val Val Lys Arg Gln
145                 150                 155                 160

Ala Gln Ser Pro Ser Gly Val Ser Ser Glu Val Glu Val Leu Lys Ala
                165                 170                 175

Leu Lys Ser Leu Phe Glu His His Lys Ala Leu Asp Glu Lys Val Arg
            180                 185                 190

Glu Arg Leu Arg Val Ser Leu Glu Arg Val Ser Ala Leu Glu Glu Glu
        195                 200                 205

Leu Ala Ala Ala Asn Gln Glu Ile Val Ala Leu Arg Glu Gln Asn Val
    210                 215                 220

His Ile Gln Arg Lys Met Ala Ser Ser Glu Gly Ser Thr Glu Ser Glu
225                 230                 235                 240

His Leu Glu Gly Met Glu Pro Gly Gln Lys Val His Glu Lys Arg Leu
                245                 250                 255

Ser Asn Gly Ser Ile Asp Ser Thr Asp Glu Thr Ser Gln Ile Val Glu
            260                 265                 270

Leu Gln Glu Leu Leu Glu Lys Gln Asn Tyr Glu Met Ala Gln Met Lys
        275                 280                 285

Glu Arg Leu Ala Ala Leu Ser Ser Arg Val Gly Glu Val Glu Gln Glu
    290                 295                 300

Ala Glu Thr Ala Arg Lys Asp Leu Ile Lys Thr Glu Glu Met Asn Thr
305                 310                 315                 320

Lys Tyr Gln Arg Asp Ile Arg Glu Ala Met Ala Gln Lys Glu Asp Met
                325                 330                 335

Glu Glu Arg Ile Thr Thr Leu Glu Lys Arg Tyr Leu Ser Ala Gln Arg
            340                 345                 350

Glu Ser Thr Ser Ile His Asp Met Asn Asp Lys Leu Glu Asn Glu Leu
        355                 360                 365

Ala Asn Lys Glu Ala Ile Leu Arg Gln Met Glu Glu Lys Asn Arg Gln
    370                 375                 380

Leu Gln Glu Arg Leu Glu Leu Ala Glu Gln Lys Leu Gln Gln Thr Met

385                 390                 395                 400

Arg Lys Ala Glu Thr Leu Pro Glu Val Glu Ala Glu Leu Ala Gln Arg
                405                 410                 415

Ile Ala Ala Leu Thr Lys Ala Glu Glu Arg His Gly Asn Ile Glu Glu
            420                 425                 430

Arg Met Arg His Leu Glu Gly Gln Leu Glu Glu Lys Asn Gln Glu Leu
        435                 440                 445

Gln Arg Ala Arg Gln Arg Glu Lys Met Asn Glu Glu His Asn Lys Arg
    450                 455                 460

Leu Ser Asp Thr Val Asp Arg Leu Leu Thr Glu Ser Asn Glu Arg Leu
465                 470                 475                 480

Gln Leu His Leu Lys Glu Arg Met Ala Ala Leu Glu Glu Lys Asn Val
                485                 490                 495

Leu Ile Gln Glu Ser Glu Thr Phe Arg Lys Asn Leu Glu Glu Ser Leu
            500                 505                 510

His Asp Lys Glu Arg Leu Ala Glu Glu Ile Glu Lys Leu Arg Ser Glu
        515                 520                 525

Leu Asp Gln Leu Lys Met Arg Thr Gly Ser Leu Ile Glu Pro Thr Ile
    530                 535                 540

Pro Arg Thr His Leu Asp Thr Ser Ala Glu Leu Arg Tyr Ser Val Gly
545                 550                 555                 560

Ser Leu Val Asp Ser Gln Ser Asp Tyr Arg Thr Thr Lys Val Ile Arg
                565                 570                 575

Arg Pro Arg Arg Gly Arg Met Gly Val Arg Arg Asp Glu Pro Lys Val
            580                 585                 590

Lys Ser Leu Gly Asp His Glu Trp Asn Arg Thr Gln Gln Ile Gly Val
        595                 600                 605

Leu Ser Ser His Pro Phe Glu Ser Asp Thr Glu Met Ser Asp Ile Asp
    610                 615                 620

Asp Asp Asp Arg Glu Thr Ile Phe Ser Ser Met Asp Leu Leu Ser Pro
625                 630                 635                 640

Ser Gly His Ser Asp Ala Gln Thr Leu Ala Met Met Leu Gln Glu Gln
                645                 650                 655

Leu Asp Ala Ile Asn Lys Glu Ile Arg Leu Ile Gln Glu Glu Lys Glu
            660                 665                 670

Ser Thr Glu Leu Arg Ala Glu Glu Ile Glu Asn Arg Val Ala Ser Val
        675                 680                 685

Ser Leu Glu Gly Leu Asn Leu Ala Arg Val His Pro Gly Thr Ser Ile
    690                 695                 700

Thr Ala Ser Val Thr Ala Ser Ser Leu Ala Ser Ser Ser Pro Pro Ser
705                 710                 715                 720

Gly His Ser Thr Pro Lys Leu Thr Pro Arg Ser Pro Ala Arg Glu Met
                725                 730                 735

Asp Arg Met Gly Val Met Thr Leu Pro Ser Asp Leu Arg Lys His Arg
            740                 745                 750

Arg Lys Ile Ala Val Val Glu Glu Asp Gly Arg Glu Asp Lys Ala Thr
        755                 760                 765

Ile Lys Cys Glu Thr Ser Pro Pro Pro Thr Pro Arg Ala Leu Arg Met
    770                 775                 780

Thr His Thr Leu Pro Ser Ser Tyr His Asn Asp Ala Arg Ser Ser Leu
785                 790                 795                 800

Ser Val Ser Leu Glu Pro Glu Ser Leu Gly Leu Gly Ser Ala Asn Ser
                805                 810                 815

Ser Gln Asp Ser Leu His Lys Ala Pro Lys Lys Lys Gly Ile Lys Ser
            820                 825                 830

Ser Ile Gly Arg Leu Phe Gly Lys Lys Glu Lys Ala Arg Leu Gly Gln
        835                 840                 845

Leu Arg Gly Phe Met Glu Thr Glu Ala Ala Ala Gln Glu Ser Leu Gly
    850                 855                 860

Leu Gly Lys Leu Gly Thr Gln Ala Glu Lys Asp Arg Arg Leu Lys Lys
865                 870                 875                 880

Lys His Glu Leu Leu Glu Glu Ala Arg Arg Lys Gly Leu Pro Phe Ala
                885                 890                 895

Gln Trp Asp Gly Pro Thr Val Val Ala Trp Leu Glu Leu Trp Leu Gly
            900                 905                 910

Met Pro Ala Trp Tyr Val Ala Ala Cys Arg Ala Asn Val Lys Ser Gly
        915                 920                 925

Ala Ile Met Ser Ala Leu Ser Asp Thr Glu Ile Gln Arg Glu Ile Gly
    930                 935                 940

Ile Ser Asn Pro Leu His Arg Leu Lys Leu Arg Leu Ala Ile Gln Glu
945                 950                 955                 960

Met Val Ser Leu Thr Ser Pro Ser Ala Pro Pro Thr Ser Arg Thr Pro
                965                 970                 975

Ser Gly Asn Val Trp Val Thr His Glu Glu Met Glu Asn Leu Ala Ala
            980                 985                 990

Pro Ala Lys Thr Lys Glu Ser Glu Glu Gly Ser Trp Ala Gln Cys Pro
        995                 1000                1005

Val Phe Leu Gln Thr Leu Ala Tyr Gly Asp Met Asn His Glu Trp
    1010                1015                1020

Ile Gly Asn Glu Trp Leu Pro Ser Leu Gly Leu Pro Gln Tyr Arg
    1025                1030                1035

Ser Tyr Phe Met Glu Cys Leu Val Asp Ala Arg Met Leu Asp His
    1040                1045                1050

Leu Thr Lys Lys Asp Leu Arg Val His Leu Lys Met Val Asp Ser
    1055                1060                1065

Phe His Arg Thr Ser Leu Gln Tyr Gly Ile Met Cys Leu Lys Arg
    1070                1075                1080

Leu Asn Tyr Asp Arg Lys Glu Leu Glu Arg Arg Arg Glu Ala Ser
    1085                1090                1095

Gln His Glu Ile Lys Asp Val Leu Val Trp Ser Asn Asp Arg Ile
    1100                1105                1110

Ile Arg Trp Ile Gln Ala Ile Gly Leu Arg Glu Tyr Ala Asn Asn
    1115                1120                1125

Ile Leu Glu Ser Gly Val His Gly Ser Leu Ile Ala Leu Asp Glu
    1130                1135                1140

Asn Phe Asp Tyr Ser Ser Leu Thr Leu Leu Leu Gln Ile Pro Thr
    1145                1150                1155

Gln Asn Thr Gln Ala Arg Gln Ile Leu Glu Arg Glu Tyr Asn Asn
    1160                1165                1170

Leu Leu Ala Leu Gly Thr Glu Arg Arg Leu Asp Glu Ser Asp Asp
    1175                1180                1185

Lys Asn Phe Arg Arg Gly Ser Thr Trp Arg Arg Gln Phe Pro Pro
    1190                1195                1200

Arg Glu Val His Gly Ile Ser Met Met Pro Gly Ser Ser Glu Thr
    1205                1210                1215

Leu Pro Ala Gly Phe Arg Leu Thr Thr Thr Ser Gly Gln Ser Arg
    1220                1225                1230

Lys Met Thr Thr Asp Val Ala Ser Ser Arg Leu Gln Arg Leu Asp
    1235                1240                1245

Asn Ser Thr Val Arg Thr Tyr Ser Cys Leu Glu
    1250                1255

<210> 239
<211> 1134
<212> DNA
<213> Homo sapiens

<400> 239
atgaggcgac tgaatcggaa aaaaacttta agtttggtaa aagagttgga tgcctttccg 60

aaggttcctg agagctatgt agagacttca gccagtggag gtacagtttc tctaatagca 120

tttacaacta tggctttatt aaccataatg gaattctcag tatatcaaga tacatggatg 180

aagtatgaat acgaagtaga caaggatttt tctagcaaat taagaattaa tatagatatt 240

actgttgcca tgaagtgtca atatgttgga gcggatgtat tggatttagc agaaacaatg 300

gttgcatctg cagatggttt agtttatgaa ccaacagtat ttgatctttc accacagcag 360

aaagagtggc agaggatgct gcagctgatt cagagtaggc tacaagaaga gcattcactt 420

caagatgtga tatttaaaag tgcttttaaa agtacatcaa cagctcttcc accaagagaa 480

gatgattcat cacagtctcc aaatgcatgc agaattcatg gccatctata tgtcaataaa 540

gtagcaggga attttcacat aacagtgggc aaggcaattc cacatcctcg tggtcatgca 600

catttggcag cacttgtcaa ccatgaatct tacaattttt ctcatagaat agatcatttg 660

tcttttggag agcttgttcc agcaattatt aatcctttag atggaactga aaaaattgct 720

atagatcaca accagatgtt ccaatatttt attacagttg tgccaacaaa actacataca 780

tataaaatat cagcagacac ccatcagttt tctgtgacag aaagggaacg tatcattaac 840

catgctgcag gcagccatgg agtctctggg atatttatga aatatgatct cagttctctt 900

atggtgacag ttactgagga gcacatgcca ttctggcagt tttttgtaag actctgtggt 960

attgttggag gaatcttttc aacaacaggc atgttacatg gaattggaaa atttatagtt 1020

gaaataattt gctgtcgttt cagacttgga tcctataaac ctgtcaattc tgttcctttt 1080

gaggatggcc acacagacaa ccacttacct cttttagaaa ataatacaca ttga 1134

<210> 240
<211> 377
<212> PRT
<213> Homo sapiens

<400> 240

Met Arg Arg Leu Asn Arg Lys Lys Thr Leu Ser Leu Val Lys Glu Leu
1               5                   10                  15

Asp Ala Phe Pro Lys Val Pro Glu Ser Tyr Val Glu Thr Ser Ala Ser
            20                  25                  30

Gly Gly Thr Val Ser Leu Ile Ala Phe Thr Thr Met Ala Leu Leu Thr
        35                  40                  45

Ile Met Glu Phe Ser Val Tyr Gln Asp Thr Trp Met Lys Tyr Glu Tyr
    50                  55                  60

Glu Val Asp Lys Asp Phe Ser Ser Lys Leu Arg Ile Asn Ile Asp Ile
65                  70                  75                  80

Thr Val Ala Met Lys Cys Gln Tyr Val Gly Ala Asp Val Leu Asp Leu
                85                  90                  95

Ala Glu Thr Met Val Ala Ser Ala Asp Gly Leu Val Tyr Glu Pro Thr
            100                 105                 110

Val Phe Asp Leu Ser Pro Gln Gln Lys Glu Trp Gln Arg Met Leu Gln
        115                 120                 125

Leu Ile Gln Ser Arg Leu Gln Glu Glu His Ser Leu Gln Asp Val Ile
    130                 135                 140

Phe Lys Ser Ala Phe Lys Ser Thr Ser Thr Ala Leu Pro Pro Arg Glu
145                 150                 155                 160

Asp Asp Ser Ser Gln Ser Pro Asn Ala Cys Arg Ile His Gly His Leu
                165                 170                 175

Tyr Val Asn Lys Val Ala Gly Asn Phe His Ile Thr Val Gly Lys Ala
            180                 185                 190

Ile Pro His Pro Arg Gly His Ala His Leu Ala Ala Leu Val Asn His
        195                 200                 205

Glu Ser Tyr Asn Phe Ser His Arg Ile Asp His Leu Ser Phe Gly Glu
    210                 215                 220

Leu Val Pro Ala Ile Ile Asn Pro Leu Asp Gly Thr Glu Lys Ile Ala
225                 230                 235                 240

Ile Asp His Asn Gln Met Phe Gln Tyr Phe Ile Thr Val Val Pro Thr
                245                 250                 255

Lys Leu His Thr Tyr Lys Ile Ser Ala Asp Thr His Gln Phe Ser Val
            260                 265                 270

Thr Glu Arg Glu Arg Ile Ile Asn His Ala Ala Gly Ser His Gly Val
        275                 280                 285

Ser Gly Ile Phe Met Lys Tyr Asp Leu Ser Ser Leu Met Val Thr Val
    290                 295                 300

Thr Glu Glu His Met Pro Phe Trp Gln Phe Phe Val Arg Leu Cys Gly
305                 310                 315                 320

Ile Val Gly Gly Ile Phe Ser Thr Thr Gly Met Leu His Gly Ile Gly
                325                 330                 335

Lys Phe Ile Val Glu Ile Ile Cys Cys Arg Phe Arg Leu Gly Ser Tyr
            340                 345                 350

Lys Pro Val Asn Ser Val Pro Phe Glu Asp Gly His Thr Asp Asn His
        355                 360                 365

Leu Pro Leu Leu Glu Asn Asn Thr His
    370                 375

<210>  241
<211>  4298
<212>  DNA
<213>  Homo sapiens

<400>  241
aattcgccct tgcttaatta agcatgttta ccttcctgtc atctgtcact gctgctgtca      60
gtggcctcct ggtgggttat gaacttggga tcatctctgg ggctcttctt cagatcaaaa     120
ccttattagc cctgagctgc catgagcagg aaatggttgt gagctccctc gtcattggag     180
ccctccttgc ctcactcacc ggaggggtcc tgatagacag atatggaaga aggacagcaa     240
tcatcttgtc atcctgcctg cttggactcg gaagcttagt cttgatcctc agtttatcct     300
acacggttct tatagtggga cgcattgcca taggggtttc catctccctc tcttccattg     360
ccacttgtgt ttacatcgca gagattgctc ctcaacacag aagaggcctt cttgtgtcac     420
tgaatgagct gatgattgtc atcggcattc tttctgccta tatttcaaat tacgcatttg     480
ccaatgtttt ccatggctgg aagtacatgt ttggtcttgt gattcccttg ggagttttgc     540
aagcaattgc aatgtatttt cttcctccaa gccctcggtt tctggtgatg aaaggacaag     600
agggagctgc tagcaaggtt cttggaaggt taagagcact ctcagataca actgaggaac     660
tcactgtgat caaatcctcc ctgaaagatg aatatcagta cagtttttgg gatctgtttc     720
gttcaaaaga caacatgcgg acccgaataa tgataggact aacactagta ttttttgtac      780
aaatcactgg ccaaccaaac atattgttct atgcatcaac tgttttgaag tcagttggat     840
ttcaaagcaa tgaggcagct agcctcgcct ccactggggt tggagtcgtc aaggtcatta     900
gcaccatccc tgccactctt cttgtagacc atgtcggcag caaaacattc ctctgcattg     960
gctcctctgt gatggcagct tcgttggtga ccatgggcat cgtaaatctc aacatccaca    1020
tgaacttcac ccatatctgc agaagccaca attctatcaa ccagtccttg gatgagtctg    1080
tgatttatgg accaggaaac ctgtcaacca acaacaatac tctcagagac cacttcaaag    1140
ggatttcttc ccatagcaga agctcactca tgcccctgag aaatgatgtg gataagagag    1200
gggagacgac ctcagcatcc ttgctaaatg ctggattaag ccacactgaa taccagatag    1260
tcacagaccc tggggacgtc ccagcttttt tgaaatggct gtccttagcc agcttgcttg    1320

tttatgttgc tgctttttca attggtctag gaccaatgcc ctggctggtg ctcagcgaga 1380
tctttcctgg tgggatcaga ggacgagcca tggctttaac ttctagcatg aactggggca 1440
tcaatctcct catctcgctg acatttttga ctgtaactga tcttattggc ctgccatggg 1500
tgtgctttat atatacaatc atgagtctag atcttattgg cctgccatgg gtgtgcttta 1560
tatatacaat catgagtcta gcatccctgc tttttgttgt tatgtttata cctgagacaa 1620
agggatgctc tttggaacaa atatcaatgg agctagcaaa agtgaactat gtgaaaaaca 1680
acatttgttt tatgagtcat caccaagaag aattagtgcc aaaacagcct caaaaaagaa 1740
aaccccagga gcagctcttg gagtgtaaca agctgtgtgg taggggccaa tccaggcagc 1800
tttctccaga gacctaatgg cctcaacacc ttctgaacgt ggatagtgcc agaacactta 1860
ggagggtgtc tttggaccaa tgcatagttg cgactcctgt gctctctttt cagtgtcatg 1920
gaactggttt tgaagagaca ctctgaaatg ataaagacag cctttaatcc ccctcctcmc 1980
cagaaggaac ctcaaaaggt agatgaggta caaggtccta agtgatctct ttttctgagc 2040
aggatatcag gttaaaaaaa aaaagttact ggctggttta atactttcta ccttcttcac 2100
agagcagcct ttgaatagac tatgtcctag tgaagacatc aacctccgcc ttaagctatg 2160
tatgtatgga ggccagtcgc agctttatta tgcagacaca caagtggtct ggacatgagg 2220
gtacagtttc tgcctaccaa gacactactt gcactggatc ttacgcaaaa aagaaccaga 2280
acacacagtg tggacaactg cccatatatt ctatctagat taggagaggg tcctggctag 2340
gattttagtg gtaattccta gttacattca acaagtataa agattataga gcttatttta 2400
tgaactataa actataattt aatgcaaaat atccttttat gaatttcatg ttaatattgt 2460
gaaatattaa aataattccr caatagttga gaaaaatgag catttttttc catttttaaa 2520
aaatgcatag aaaagacaat tttaaaatcc tgggaccata tttatttaga agtagctgtt 2580
agtaaaacat tagaaaagga gtcaggccat taggttattt atccaaatct ctaagcaatt 2640
aggttgaagt tattaagtca agcctagaaa agctgcctcc ttgtaaggct ttcatgacaa 2700
tgtatagtaa tccacagtgt ccaattcttc acactcctca ggaatatcac tacctcaggt 2760
tacggtacac aggctataat tgatgatgat gttcagataa ctgaagacac aataaatgac 2820
attcagacat caggamaaww ccctcatgtt cttttctatg atggccacct gtaccagcaa 2880
cgtgggtttc acccacacaa cgatgaactg ttctcttact tctccagttg attttaaaga 2940
cttgttaaga ggtcttacta ataaaatttg ggtatgatag aaaawccaca atcaaawctt 3000
gaaccaaata acatattaaa ttactaatat ttaagtgatg gaagacacac aaaaaactta 3060
aaagcacgaa caacctaact tgaaaaagaa ttttaaaata tgattaacct gaagaaaaga 3120
gaatcctaag agccaaagct cctttttatt tagcttggaa ttttcctatt ggttcctaac 3180
aaactgtccc aatgtcatat aaggaaacat gatctattac attcctttat aacaatgtgg 3240
agagactata aacctatgta agtagtaaaa ctatatyaga gactcaggag actgactaaa 3300
aggcctggat ctgcagtgta ttatctgtat aaaaattggc agggggaagc taaaaggaaa 3360

ggagattgga gatctcaatt ctatcatggt gtatttcata cgcaaatcag agcatgcatt 3420
gttttttgtt tttggaaaga gaagggaagt gtgttctgcc ccatgtttcc ttccgtgttt 3480
atagttcaaa ctctatatat acttcaggta ttttttgttt agcccttcat tataaatggg 3540
caggaaattg tttatcaacc tagccagttt attactagtg accttgactt cagtatcttg 3600
agcattcttt tatatttttc ttttattatc ctgagtctgt aactaaacaa ttttgtcttc 3660
aaatttttat ccaatatcca ttgcaccaca ccaaatcaag cttcttgatt ttcaaaaata 3720
aaaaggggga aatacttaca acttgtacat atatattcac agtttttatt tataaaaaaa 3780
atttacagta cttatggaga gccagcagaa gacatcagag cactcacttc ttcccatctt 3840
tgttaaggtt agcgaattac ccatggacac tgttaggtga ggctcattcg gcagccctga 3900
aaacaaacct ggtcacactg tctttaccct ctcccttcag ataaagcact tcgattatct 3960
attgatctgc ccagttttca agtcatgcga atactaaaaa ggttacatca tctggatctg 4020
taccttggct atataagcat gttttccccc tattctatgt ttcttttttt ggtgaacatt 4080
gaaaaacagg aggtgactta ttactgttaa ttaaaactaa atgaaaaatg tcaagtcttt 4140
aaaacagtga gcttgtaact ctttcatgta attttattct ctatgaattt ggctatccta 4200
ctgaatctta aaataaagga aataaacact tttttttwaa aaaaaaggaa aaatamaarw 4260
mwaaaaatct caatgaaata tttcacaaga aggaaaaa 4298

<210> 242
<211> 597
<212> PRT
<213> Homo sapiens

<400> 242

Met Phe Thr Phe Leu Ser Ser Val Thr Ala Ala Val Ser Gly Leu Leu
1               5                   10                  15

Val Gly Tyr Glu Leu Gly Ile Ile Ser Gly Ala Leu Leu Gln Ile Lys
            20                  25                  30

Thr Leu Leu Ala Leu Ser Cys His Glu Gln Glu Met Val Val Ser Ser
        35                  40                  45

Leu Val Ile Gly Ala Leu Leu Ala Ser Leu Thr Gly Gly Val Leu Ile
    50                  55                  60

Asp Arg Tyr Gly Arg Arg Thr Ala Ile Ile Leu Ser Ser Cys Leu Leu
65                  70                  75                  80

Gly Leu Gly Ser Leu Val Leu Ile Leu Ser Leu Ser Tyr Thr Val Leu
                85                  90                  95

Ile Val Gly Arg Ile Ala Ile Gly Val Ser Ile Ser Leu Ser Ser Ile
            100                 105                 110

Ala Thr Cys Val Tyr Ile Ala Glu Ile Ala Pro Gln His Arg Arg Gly
        115                 120                 125

Leu Leu Val Ser Leu Asn Glu Leu Met Ile Val Ile Gly Ile Leu Ser
    130                 135                 140

Ala Tyr Ile Ser Asn Tyr Ala Phe Ala Asn Val Phe His Gly Trp Lys

145                 150                 155                 160

Tyr Met Phe Gly Leu Val Ile Pro Leu Gly Val Leu Gln Ala Ile Ala
                165                 170                 175

Met Tyr Phe Leu Pro Pro Ser Pro Arg Phe Leu Val Met Lys Gly Gln
            180                 185                 190

Glu Gly Ala Ala Ser Lys Val Leu Gly Arg Leu Arg Ala Leu Ser Asp
        195                 200                 205

Thr Thr Glu Glu Leu Thr Val Ile Lys Ser Ser Leu Lys Asp Glu Tyr
    210                 215                 220

Gln Tyr Ser Phe Trp Asp Leu Phe Arg Ser Lys Asp Asn Met Arg Thr
225                 230                 235                 240

Arg Ile Met Ile Gly Leu Thr Leu Val Phe Phe Val Gln Ile Thr Gly
                245                 250                 255

Gln Pro Asn Ile Leu Phe Tyr Ala Ser Thr Val Leu Lys Ser Val Gly
            260                 265                 270

Phe Gln Ser Asn Glu Ala Ala Ser Leu Ala Ser Thr Gly Val Gly Val
        275                 280                 285

Val Lys Val Ile Ser Thr Ile Pro Ala Thr Leu Leu Val Asp His Val
    290                 295                 300

Gly Ser Lys Thr Phe Leu Cys Ile Gly Ser Ser Val Met Ala Ala Ser
305                 310                 315                 320

Leu Val Thr Met Gly Ile Val Asn Leu Asn Ile His Met Asn Phe Thr
                325                 330                 335

His Ile Cys Arg Ser His Asn Ser Ile Asn Gln Ser Leu Asp Glu Ser
            340                 345                 350

Val Ile Tyr Gly Pro Gly Asn Leu Ser Thr Asn Asn Asn Thr Leu Arg
        355                 360                 365

Asp His Phe Lys Gly Ile Ser Ser His Ser Arg Ser Ser Leu Met Pro
    370                 375                 380

Leu Arg Asn Asp Val Asp Lys Arg Gly Glu Thr Thr Ser Ala Ser Leu
385                 390                 395                 400

Leu Asn Ala Gly Leu Ser His Thr Glu Tyr Gln Ile Val Thr Asp Pro
                405                 410                 415

Gly Asp Val Pro Ala Phe Leu Lys Trp Leu Ser Leu Ala Ser Leu Leu
            420                 425                 430

Val Tyr Val Ala Ala Phe Ser Ile Gly Leu Gly Pro Met Pro Trp Leu
        435                 440                 445

Val Leu Ser Glu Ile Phe Pro Gly Gly Ile Arg Gly Arg Ala Met Ala
    450                 455                 460

Leu Thr Ser Ser Met Asn Trp Gly Ile Asn Leu Leu Ile Ser Leu Thr
465                 470                 475                 480

Phe Leu Thr Val Thr Asp Leu Ile Gly Leu Pro Trp Val Cys Phe Ile
                485                 490                 495

Tyr Thr Ile Met Ser Leu Asp Leu Ile Gly Leu Pro Trp Val Cys Phe
            500                 505                 510

Ile Tyr Thr Ile Met Ser Leu Ala Ser Leu Leu Phe Val Val Met Phe
        515                 520                 525

Ile Pro Glu Thr Lys Gly Cys Ser Leu Glu Gln Ile Ser Met Glu Leu
    530                 535                 540

Ala Lys Val Asn Tyr Val Lys Asn Asn Ile Cys Phe Met Ser His His
545                 550                 555                 560

Gln Glu Glu Leu Val Pro Lys Gln Pro Gln Lys Arg Lys Pro Gln Glu
                565                 570                 575

Gln Leu Leu Glu Cys Asn Lys Leu Cys Gly Arg Gly Gln Ser Arg Gln
            580                 585                 590

Leu Ser Pro Glu Thr
        595

<210>  243
<211>  979
<212>  DNA
<213>  Homo sapiens

<220>
<221>  misc_feature
<222>  (17)..(17)
<223>  n is a, c, g, or t

<220>
<221>  misc_feature
<222>  (229)..(230)
<223>  n is a, c, g, or t

<220>
<221>  misc_feature
<222>  (238)..(238)
<223>  n is a, c, g, or t

<400>  243
tttagccacc agagganttc tcttgaaata cccaaaatcc atcagtatct tgaatcatgc      60

tggattttga agaattctta agaagccatg taaagggggc tctctggcct tgaaatagtg     120

atgtttttta tacagaaagg agaatgcaga atggtcagac tatcatgcac tgttaaattt     180

gatttcaaga aattacagga aaactttcca aagttccatc tcacagaann ttattttncc     240

aagaattcca agataagttt agttttatgg aagactttta tgtggttttt actcactctt     300

catctcagac atcgacagat gattacatca cttatagttc tagtaaattt attaatataa     360

aactcagaga cattccaata tccacattgc ttacaccatt aggcatagat tcagtgtcag     420

ctatgacaat tgaaaatgag ctgttttgtg atttaaaggt ttaaatttct ctaaccaaac     480

tgcttgatcc agatgcagga ctgcaaatgt taatatttgt tctggaagaa caatcaaata     540

agacttaaga ggaaagggaa tggccacaat ccacctgaaa tttttttcta aaaagtgtgc     600

agcctactaa atcagaatga aaatagaagt acaagattat aaacaaaatg caatcaaact     660

tttcttaagc ttacctaaag ttatttcatc tgaaaatttc aagcaacttt gttcaacatt     720

aaattgacaa tctaaactaa caagtctttt gaatttatgc atggtagtaa acattctctc     780

tattaacttt attacctaag gctaaaccta aaatttttaa gcaaaattag aaaaatagtc     840

ttcactcatc aaaaaataaa gtttgttaca tttagtattt tcccaataaa attggtcgtt     900

cttggttttt tatttggaga gtctgtgcaa aatgtcacta aaaataaatt agcactagaa 960

attatttcta aataccaaa 979

<210> 244
<211> 2443
<212> DNA
<213> Homo sapiens

<400> 244
aaatggcgtg cccgtctctc cgccggcccc ctgcctcgca gtggtttctc ctgcagctcc 60

cctgggctcc gcggccagta gtgcagcccg tggagccgcg gctttgcccg tctcctctgg 120

gtggccccag tgcgcgggct gacactcatt cagccgggga aggtgaggcg agtagaggct 180

ggtgcggaac ttgccgcccc cagcagcgcc ggcgggctaa gcccagggcc gggcagacaa 240

aagaggccgc ccgcgtagga aggcacggcc ggcggcggcg gagcgcagcg atggccgggc 300

gagggggcag cgcgctgctg gctctgtgcg gggcactggc tgcctgcggg tggctcctgg 360

gcgccgaagc ccaggagccc ggggcgcccg cggcgggcat gaggcggcgc cggcggctgc 420

agcaagagga cggcatctcc ttcgagtacc accgctaccc cgagctgcgc gaggcgctcg 480

tgtccgtgtg gctgcagtgc accgccatca gcaggattta cacggtgggg cgcagcttcg 540

agggccggga gctcctggtc atcgagctgt ccgacaaccc tggcgtccat gagcctggtg 600

agcctgaatt taaatacatt gggaatatgc atgggaatga ggctgttgga cgagaactgc 660

tcattttctt ggcccagtac ctatgcaacg aataccagaa ggggaacgag acaattgtca 720

acctgatcca cagtacccgc attcacatca tgccttccct gaacccagat ggctttgaga 780

aggcagcgtc tcagcctggt gaactcaagg actggtttgt gggtcgaagc aatgcccagg 840

gaatagatct gaaccggaac tttccagacc tggataggat agtgtacgtg aatgagaaag 900

aaggtggtcc aaataatcat ctgttgaaaa atatgaagaa aattgtggat caaaacacaa 960

agcttgctcc tgagaccaag gctgtcattc attggattat ggatattcct tttgtgcttt 1020

ctgccaatct ccatggagga gaccttgtgg ccaattatcc atatgatgag acgcggagtg 1080

gtagtgctca cgaatacagc tcctccccag atgacgccat tttccaaagc ttggcccggg 1140

catactcttc tttcaacccg gccatgtctg accccaatcg gccaccatgt cgcaagaatg 1200

atgatgacag cagctttgta gatggaacca ccaacggtgg tgcttggtac agcgtacctg 1260

gagggatgca agacttcaat taccttagca gcaactgttt tgagatcacc gtggagctta 1320

gctgtgagaa gttcccacct gaagagactc tgaagaccta ctgggaggat aacaaaaact 1380

ccctcattag ctaccttgag cagatacacc gaggagttaa aggatttgtc cgagaccttc 1440

aaggtaaccc aattgcgaat gccaccatct ccgtggaagg aatagaccac gatgttacat 1500

ccgcaaagga tggtgattac tggagattgc ttatacctgg aaactataaa cttacagcct 1560

cagctccagg ctatctggca ataacaaaga aagtggcagt tccttacagc cctgctgctg 1620

gggttgattt tgaactggag tcattttctg aaaggaaaga agaggagaag gaagaattga 1680

tggaatggtg gaaaatgatg tcagaaactt taaattttta aaaaggcttc tagttagctg 1740

ctttaaatct atctatataa tgtagtatga tgtaatgtgg tctttttttt agattttgtg 1800

cagttaatac ttaacattga tttatttttt aatcatttaa atattaatca actttcctta 1860

aaataaatag cctcttaggt aaaaatataa gaacttgata tatttcattc tcttatatag 1920

tattcatttt cctacctata ttacacaaaa aagtatagaa aagatttaag taattttgcc 1980

atcctaggct taaatgcaat attcctggta ttatttacaa tgcagaattt tttgagtaat 2040

tctagctttc aaaaattagt gaagttcttt tactgtaatt ggtgacaatg tcacataatg 2100

aatgctattg aaaaggttaa cagatacagc tcggagttgt gagcactcta ctgcaagact 2160

taaatagttc agtataaatt gtcgtttttt tcttgtgctg actaactata agcatgatct 2220

tgttaatgca tttttgatgg gaagaaaagg tacatgttta caaagaggtt ttatgaaaag 2280

aataaaaatt gacttcttgc ttgtacatat aggagcaata ctattatatt atgtagtccg 2340

ttaacactac ttaaaagttt agggttttct cttggttgta gagtggccca gaattgcatt 2400

ctgaatgaat aaaggttaaa aaaaaatccc cagtgaaaaa aaa 2443

<210> 245
<211> 476
<212> PRT
<213> Homo sapiens

<400> 245

Met Ala Gly Arg Gly Gly Ser Ala Leu Leu Ala Leu Cys Gly Ala Leu
1               5                   10                  15

Ala Ala Cys Gly Trp Leu Leu Gly Ala Glu Ala Gln Glu Pro Gly Ala
            20                  25                  30

Pro Ala Ala Gly Met Arg Arg Arg Arg Arg Leu Gln Gln Glu Asp Gly
        35                  40                  45

Ile Ser Phe Glu Tyr His Arg Tyr Pro Glu Leu Arg Glu Ala Leu Val
    50                  55                  60

Ser Val Trp Leu Gln Cys Thr Ala Ile Ser Arg Ile Tyr Thr Val Gly
65                  70                  75                  80

Arg Ser Phe Glu Gly Arg Glu Leu Leu Val Ile Glu Leu Ser Asp Asn
                85                  90                  95

Pro Gly Val His Glu Pro Gly Glu Pro Glu Phe Lys Tyr Ile Gly Asn
            100                 105                 110

Met His Gly Asn Glu Ala Val Gly Arg Glu Leu Leu Ile Phe Leu Ala
        115                 120                 125

Gln Tyr Leu Cys Asn Glu Tyr Gln Lys Gly Asn Glu Thr Ile Val Asn
    130                 135                 140

Leu Ile His Ser Thr Arg Ile His Ile Met Pro Ser Leu Asn Pro Asp
145                 150                 155                 160

Gly Phe Glu Lys Ala Ala Ser Gln Pro Gly Glu Leu Lys Asp Trp Phe
                165                 170                 175

Val Gly Arg Ser Asn Ala Gln Gly Ile Asp Leu Asn Arg Asn Phe Pro
            180                 185                 190

Asp Leu Asp Arg Ile Val Tyr Val Asn Glu Lys Glu Gly Gly Pro Asn
        195                 200                 205

Asn His Leu Leu Lys Asn Met Lys Lys Ile Val Asp Gln Asn Thr Lys
    210                 215                 220

Leu Ala Pro Glu Thr Lys Ala Val Ile His Trp Ile Met Asp Ile Pro
225                 230                 235                 240

Phe Val Leu Ser Ala Asn Leu His Gly Gly Asp Leu Val Ala Asn Tyr
                245                 250                 255

Pro Tyr Asp Glu Thr Arg Ser Gly Ser Ala His Glu Tyr Ser Ser Ser
            260                 265                 270

Pro Asp Asp Ala Ile Phe Gln Ser Leu Ala Arg Ala Tyr Ser Ser Phe
        275                 280                 285

Asn Pro Ala Met Ser Asp Pro Asn Arg Pro Pro Cys Arg Lys Asn Asp
    290                 295                 300

Asp Asp Ser Ser Phe Val Asp Gly Thr Thr Asn Gly Gly Ala Trp Tyr
305                 310                 315                 320

Ser Val Pro Gly Gly Met Gln Asp Phe Asn Tyr Leu Ser Ser Asn Cys
                325                 330                 335

Phe Glu Ile Thr Val Glu Leu Ser Cys Glu Lys Phe Pro Pro Glu Glu
            340                 345                 350

Thr Leu Lys Thr Tyr Trp Glu Asp Asn Lys Asn Ser Leu Ile Ser Tyr
        355                 360                 365

Leu Glu Gln Ile His Arg Gly Val Lys Gly Phe Val Arg Asp Leu Gln
    370                 375                 380

Gly Asn Pro Ile Ala Asn Ala Thr Ile Ser Val Glu Gly Ile Asp His
385                 390                 395                 400

Asp Val Thr Ser Ala Lys Asp Gly Asp Tyr Trp Arg Leu Leu Ile Pro
                405                 410                 415

Gly Asn Tyr Lys Leu Thr Ala Ser Ala Pro Gly Tyr Leu Ala Ile Thr
            420                 425                 430

Lys Lys Val Ala Val Pro Tyr Ser Pro Ala Ala Gly Val Asp Phe Glu
        435                 440                 445

Leu Glu Ser Phe Ser Glu Arg Lys Glu Glu Glu Lys Glu Glu Leu Met
    450                 455                 460

Glu Trp Trp Lys Met Met Ser Glu Thr Leu Asn Phe
465                 470                 475

<210>  246
<211>  1926
<212>  DNA
<213>  Homo sapiens

<400>  246
ggggcgacgt gagcgcgcag gggggcggcg gcctcgcctc gtctctctct ctgcgcctgg      60

gtcgggtggg tgacgccgag agccagagag atgtcggatt tcgacagtaa cccgtttgcc     120

gacccggatc tcaacaatcc cttcaaggat ccatcagtta cacaagtgac aagaaatgtt     180

ccaccaggac ttgatgaata taatccattc tcggattcta gaacacctcc accaggcggt     240

gtgaagatgc ctaatgtacc caatacacaa ccagcaataa tgaaaccaac agaggaacat 300

ccagcttata cacagattgc aaaggaacat gcattggccc aagctgaact tcttaagcgc 360

caagaagaac tagaaagaaa agccgcagaa ttagatcgtc gggaacgaga aatgcaaaac 420

ctcagtcaac atggtagaaa aaatatttgg ccacctcttc ctagcaattt tcctgtcgga 480

ccttgtttct atcaggaatt ttctgtagac attcctgtag aattccaaaa gacagtaaag 540

cttatgtact acttgtggat gttccatgca gtaacactgt ttctaaatat cttcggatgc 600

ttggcttggt tttgtgttga ttctgcaaga gcggttgatt ttggattgag tatcctgtgg 660

ttcttgcttt ttactccttg ttcatttgtc tgttggtaca gaccacttta tggagctttc 720

aggagtgaca gttcatttag attctttgta ttcttcttcg tctatatttg tcagtttgct 780

gtacatgtac tccaagctgc aggatttcat aactggggca attgtggttg gatttcatcc 840

cttactggtc tcaaccaaaa tattcctgtt ggaatcatga tgataatcat agcagcactt 900

ttcacagcat cagcagtcat ctcactagtt atgttcaaaa aagtacatgg actatatcgc 960

acaacaggtg ctagttttga gaaggcccaa caggagtttg caacaggtgt gatgtccaac 1020

aaaactgtcc agaccgcagc tgcaaatgca gcttcaactg cagcatctag tgcagctcag 1080

aatgctttca agggtaacca gatttaagaa tcttcaaaca atacactgtt accttttgac 1140

tgtacctttt tctccagtta ctgtattcta caaatatttt tatgttcaaa acacacagta 1200

cagacagcat ggatatttcc tgttcacttg tgcatgggct aaaaccagga aacttccttt 1260

gtcttattac tttacctaat agtttcttaa tatttcagtg ccccttgcag aaaaaatatt 1320

acatgctaaa taaatattct ccatattttt gggggatgac attcagtgaa ttatttcagt 1380

ggtgacccac tgaaaattaa taatggtact tatgattaaa aacgcattta atactaactg 1440

cagtagttct ttcaagaatc tttagagata aggattgcac attggaaaag taaaccatgt 1500

ttcattcctt tttccctatt tatattgaaa gaaataggcc agcagagact tagggatttt 1560

aaattggctt gctttttagc tgtttcagtc accagtgaag agcctatgtg cattttgtag 1620

tagataatgt aaaatttgtc atctttttct tttctttttt ttagaatagc tgatattttg 1680

ataacaatct ctaatttgca tgggcaccac atttcttata ttaaaagaat tagtgttttg 1740

gcttctgtac tgcttatggt tgtaggattc aggggttaat ggaatcacag aaatgatatt 1800

ctgcaagaat ttcttttaaa taaaaagttt gggggtgcaa tataagaagt ttatataata 1860

tgcagtacat tatccaaaag agaaggtagt taatgcagta gaaagtagtg gtaataattc 1920

cttttt 1926

<210> 247
<211> 338
<212> PRT
<213> Homo sapiens

<400> 247

<210>  248
<211>  615
<212>  DNA
<213>  Homo sapiens

<400>  248
atgagagaca ataaatcgtg tgcttttttc atgggaaagt taaatgtttg ttttgaaggc      60

acagtaatag caggctattc agtgtttgcc actacctgca tcattcatct ggctgtagct     120

agtgcactac aatttcctaa aaagtcttct caccctcaca ggactgctct acatctggcc     180

tctgccaatg gaaattcaga agtagtaaaa ctcctgctgg acagacgatg tcaacttaat     240

atccttgaca acaaaaagag gacagctctg acaaaggccg tacaatgcca ggaagatgaa     300

tgtgcgttaa tgttgctgga acatggcact gatccgaata ttccagatga gtatggaaat     360

accgctctac actatgctat ctacaatgaa gataaattaa tggccaaagc actgctctta     420

tacggtgctg atatcgaatc aaaaaacaag catggcctca caccactgtt acttggtgta     480

catgagcaaa aacagcaagt ggtgaaattt ttaatcaaga aaaaagcaaa tttaaatgca     540

ctggatagat atggaaggtg tgtgaccttg ggaacgttat ttaccaccaa atatgttgtc     600

atatatgaaa agtag                                                      615

<210>  249
<211>  204
<212>  PRT
<213>  Homo sapiens

<400>  249

Met Arg Asp Asn Lys Ser Cys Ala Phe Phe Met Gly Lys Leu Asn Val
1               5                   10                  15

Cys Phe Glu Gly Thr Val Ile Ala Gly Tyr Ser Val Phe Ala Thr Thr
            20                  25                  30

Cys Ile Ile His Leu Ala Val Ala Ser Ala Leu Gln Phe Pro Lys Lys
        35                  40                  45

Ser Ser His Pro His Arg Thr Ala Leu His Leu Ala Ser Ala Asn Gly
    50                  55                  60

Asn Ser Glu Val Val Lys Leu Leu Leu Asp Arg Arg Cys Gln Leu Asn
65                  70                  75                  80

Ile Leu Asp Asn Lys Lys Arg Thr Ala Leu Thr Lys Ala Val Gln Cys
                85                  90                  95

Gln Glu Asp Glu Cys Ala Leu Met Leu Leu Glu His Gly Thr Asp Pro
            100                 105                 110

Asn Ile Pro Asp Glu Tyr Gly Asn Thr Ala Leu His Tyr Ala Ile Tyr
        115                 120                 125

Asn Glu Asp Lys Leu Met Ala Lys Ala Leu Leu Leu Tyr Gly Ala Asp
    130                 135                 140

Ile Glu Ser Lys Asn Lys His Gly Leu Thr Pro Leu Leu Leu Gly Val
145                 150                 155                 160

His Glu Gln Lys Gln Gln Val Val Lys Phe Leu Ile Lys Lys Lys Ala
                165                 170                 175

Asn Leu Asn Ala Leu Asp Arg Tyr Gly Arg Cys Val Thr Leu Gly Thr
            180                 185                 190

Leu Phe Thr Thr Lys Tyr Val Val Ile Tyr Glu Lys
        195                 200

<210> 250
<211> 3342
<212> DNA
<213> Homo sapiens

<400> 250
atggtgatca tctatctttc tttctgcaat tattacatgg agttctacag agaagagctt 60

ccccacattg actatttgat tgacattcag tttgcaacag gaaaggttac tcagccggga 120

gaggacactt cctaccatca atgcgctcag cttgaagcca gagacgaagg caccgacagt 180

ttattattaa acaatggcag cagcgccacg ctgaagacac gaacgcgctg ttatggaacc 240

cccagaggtc tcccccatcg tagcctgctc cagccgactc cgcccacatg taaaacgaag 300

atcaggagca gatttgaaga attacaaagt gaattggtgc cagtcagcat gtcagagaca 360

gaccacatag cctctacttc ctctgataaa aatgttggga aaacacctga attaaaggaa 420

gactcatgca acttgttttc tggcaatgaa agcagcaaat tagaaaatga gtccaaacta 480

ttgtcattaa acactgataa aactttatgt caacctaatg agcataataa tcgaattgaa 540

gcccaggaaa attatattcc agatcatggt ggaggtgagg attcttgtgc caaaacagac 600

acaggctcag aaaattctga acaaatagct aattttccta gtggaaattt tgctaaacat 660

atttcaaaaa caaatgaaac agaacagaaa gtaacacaaa tattggtgga attaaggtca 720

tctacatttc cagaatcagc taatgaaaag acttattcag aaagccccta tgatacagac 780

tgcaccaaga aatttatttc aaaaataaag agcgtttcag catcagagga tttgttggaa 840

gaaatagaat ctgagctctt atctacggag tttgcagaac atcgagtacc aaatggaatg 900

aataagggag aacatgcatt agttctgttt gaaaagtgtg tgcaagataa atatttgcag 960

caggaacata tcataaaaaa gttaattaaa gaaaataaga agcatcagga gctcttcgta 1020

gacatttgtt cagaaaaaga caatttaaga gaagaactaa agaaaagaac agaaactgag 1080

aagcagcata tgaacacaat taaacagtta gaatcaagaa tagaagaact taataaagaa 1140

gttaaagctt ccagagatca actaatagct caagacgtta cagctaaaaa tgcagttcag 1200

cagttacaca aagagatggc ccaacggatg gaacaggcca acaagaaatg tgaagaggca 1260

cgccaagaaa aagaagcaat ggtaatgaaa tatgtaagag gtgagaagga atctttagat 1320

cttcgaaagg aaaaagagac acttgagaaa aaacttagag atgcaaataa ggaacttgag 1380

aaaaacacta acaaaattaa gcagctttct caggagaaag gacggttgca ccagctgtat 1440

gaaactaagg aaggcgaaac gactagactc atcagagaaa tagacaaatt aaaggaagac 1500

attaactctc acgtcatcaa agtaaagtgg gcacaaaaca aattaaaagc tgaaatggat 1560

tcacacaagg aaaccaaaga taaactcaaa gaaacaacaa caaaattaac acaagcaaag 1620

gaagaagcag atcagatacg aaaaaactgt caggatatga taaaaacata tcaggagtca 1680

gaagaaatta aatcaaatga gcttgatgca aagcttagag tcacaaaagg agaacttgaa 1740

aaacaaatgc aagaaaaatc tgaccagcta gagatgcatc atgccaaaat aaaggaacta 1800

gaagatctga agagaacatt taaggagggt atggatgagt taagaacact gagaacaaag 1860

gtgaaatgtc tagaagatga acgattaaga acagaagatg aattatcaaa atataaggaa 1920
attattaatc gccaaaaagc tgaaattcag aatttattgg acaaggtgaa aactgcagat 1980
cagctacagg agcagcttca aagaggtaag caagaaattg aaaatttgaa agaagaagtg 2040
gaaagtctta attctttgat taatgaccta caaaaagaca tcgaaggcag taggaaaaga 2100
gaatctgagc tgctgctgtt tacagaaagg ctcactagta agaatgcaca gcttcagtct 2160
gaatccaatt ctttgcagtc acaatttgat aaagtttcct gtagtgaaag tcagttacaa 2220
agccagtgtg aacaaatgaa acagacaaat attaatttgg aaagtaggtt gttgaaagag 2280
gaagaactgc gaaaagagga agtccaaact ctgcaagctg aactcgcttg tagacaaaca 2340
gaagttaaag cattgagtac ccaggtagaa gaattaaaag atgagttagt aactcagaga 2400
cgtaaacatg cctctagtat caaggatctc accaaacaac ttcagcaagc acgaagaaaa 2460
ttagatcagg ttgagagtgg aagctatgac aaagaagtca gcagcatggg aagtcgttct 2520
agttcatcag ggtccctgaa tgctcgaagc agtgcagaag atcgatctcc agaaaatact 2580
gggtcctcag tagctgtgga taactttcca caagtagata aggccatgtt gattgagaga 2640
atagttaggc tgcaaaaagc acatgcccgg aaaaatgaaa agatagaatt tatggaggac 2700
cacatcaaac aactggtgga agaaattagg aaaaaaacaa aaataattca aagttatatt 2760
ttacgagaag aatcaggcac actttcttca gaggcatctg attttaacaa agttcattta 2820
agtagacggg gtggcatcat ggcatcttta tatacatccc atccagctga caatggatta 2880
acattggagc tctctttgga aatcaaccga aaattacagg ctgttttgga ggatacgtta 2940
ctaaaaaata ttactttgaa ggaaaatcta caaacacttg gaacagaaat agaacgtctt 3000
attaaacacc agcatgaact agaacagagg acaaagaaaa cctaaaacaa gcctcttgct 3060
cagtaaagag acaaaagcca cacaggagta ggtgccactg acctctattg ttggagactt 3120
tgttccactt tttgtttcag ccagtaaaaa tattgttttg cttcatctgt acacaaaaaa 3180
ataccctttt acaatatgaa tgcattgctg tatatactgt aagactgaaa gctttgatga 3240
aatttgtttt tgtatggtgc aatatgacag cctgtcattg aatctaaaca acttaatttg 3300
cttgtattca taagaagtgt tgaacattac aagggctttt at 3342

<210> 251
<211> 1014
<212> PRT
<213> Homo sapiens

<400> 251

Met Val Ile Ile Tyr Leu Ser Phe Cys Asn Tyr Tyr Met Glu Phe Tyr
1               5                   10                  15

Arg Glu Glu Leu Pro His Ile Asp Tyr Leu Ile Asp Ile Gln Phe Ala
            20                  25                  30

Thr Gly Lys Val Thr Gln Pro Gly Glu Asp Thr Ser Tyr His Gln Cys
        35                  40                  45

Ala Gln Leu Glu Ala Arg Asp Glu Gly Thr Asp Ser Leu Leu Leu Asn

50                  55                  60

Asn Gly Ser Ser Ala Thr Leu Lys Thr Arg Thr Arg Cys Tyr Gly Thr
65                  70                  75                  80

Pro Arg Gly Leu Pro His Arg Ser Leu Leu Gln Pro Thr Pro Pro Thr
                85                  90                  95

Cys Lys Thr Lys Ile Arg Ser Arg Phe Glu Glu Leu Gln Ser Glu Leu
            100                 105                 110

Val Pro Val Ser Met Ser Glu Thr Asp His Ile Ala Ser Thr Ser Ser
        115                 120                 125

Asp Lys Asn Val Gly Lys Thr Pro Glu Leu Lys Glu Asp Ser Cys Asn
    130                 135                 140

Leu Phe Ser Gly Asn Glu Ser Ser Lys Leu Glu Asn Glu Ser Lys Leu
145                 150                 155                 160

Leu Ser Leu Asn Thr Asp Lys Thr Leu Cys Gln Pro Asn Glu His Asn
                165                 170                 175

Asn Arg Ile Glu Ala Gln Glu Asn Tyr Ile Pro Asp His Gly Gly Gly
            180                 185                 190

Glu Asp Ser Cys Ala Lys Thr Asp Thr Gly Ser Glu Asn Ser Glu Gln
        195                 200                 205

Ile Ala Asn Phe Pro Ser Gly Asn Phe Ala Lys His Ile Ser Lys Thr
    210                 215                 220

Asn Glu Thr Glu Gln Lys Val Thr Gln Ile Leu Val Glu Leu Arg Ser
225                 230                 235                 240

Ser Thr Phe Pro Glu Ser Ala Asn Glu Lys Thr Tyr Ser Glu Ser Pro
                245                 250                 255

Tyr Asp Thr Asp Cys Thr Lys Lys Phe Ile Ser Lys Ile Lys Ser Val
            260                 265                 270

Ser Ala Ser Glu Asp Leu Leu Glu Glu Ile Glu Ser Glu Leu Leu Ser
        275                 280                 285

Thr Glu Phe Ala Glu His Arg Val Pro Asn Gly Met Asn Lys Gly Glu
    290                 295                 300

His Ala Leu Val Leu Phe Glu Lys Cys Val Gln Asp Lys Tyr Leu Gln
305                 310                 315                 320

Gln Glu His Ile Ile Lys Lys Leu Ile Lys Glu Asn Lys Lys His Gln
                325                 330                 335

Glu Leu Phe Val Asp Ile Cys Ser Glu Lys Asp Asn Leu Arg Glu Glu
            340                 345                 350

Leu Lys Lys Arg Thr Glu Thr Glu Lys Gln His Met Asn Thr Ile Lys
        355                 360                 365

Gln Leu Glu Ser Arg Ile Glu Glu Leu Asn Lys Glu Val Lys Ala Ser
    370                 375                 380

Arg Asp Gln Leu Ile Ala Gln Asp Val Thr Ala Lys Asn Ala Val Gln
385                 390                 395                 400

Gln Leu His Lys Glu Met Ala Gln Arg Met Glu Gln Ala Asn Lys Lys
                405                 410                 415

Cys Glu Glu Ala Arg Gln Glu Lys Glu Ala Met Val Met Lys Tyr Val
            420                 425                 430

Arg Gly Glu Lys Glu Ser Leu Asp Leu Arg Lys Glu Lys Glu Thr Leu
        435                 440                 445

Glu Lys Lys Leu Arg Asp Ala Asn Lys Glu Leu Glu Lys Asn Thr Asn
    450                 455                 460

Lys Ile Lys Gln Leu Ser Gln Glu Lys Gly Arg Leu His Gln Leu Tyr
465                 470                 475                 480

Glu Thr Lys Glu Gly Glu Thr Thr Arg Leu Ile Arg Glu Ile Asp Lys
                485                 490                 495

Leu Lys Glu Asp Ile Asn Ser His Val Ile Lys Val Lys Trp Ala Gln
            500                 505                 510

Asn Lys Leu Lys Ala Glu Met Asp Ser His Lys Glu Thr Lys Asp Lys
        515                 520                 525

Leu Lys Glu Thr Thr Thr Lys Leu Thr Gln Ala Lys Glu Glu Ala Asp
    530                 535                 540

Gln Ile Arg Lys Asn Cys Gln Asp Met Ile Lys Thr Tyr Gln Glu Ser
545                 550                 555                 560

Glu Glu Ile Lys Ser Asn Glu Leu Asp Ala Lys Leu Arg Val Thr Lys
                565                 570                 575

Gly Glu Leu Glu Lys Gln Met Gln Glu Lys Ser Asp Gln Leu Glu Met
            580                 585                 590

His His Ala Lys Ile Lys Glu Leu Glu Asp Leu Lys Arg Thr Phe Lys
        595                 600                 605

Glu Gly Met Asp Glu Leu Arg Thr Leu Arg Thr Lys Val Lys Cys Leu
    610                 615                 620

Glu Asp Glu Arg Leu Arg Thr Glu Asp Glu Leu Ser Lys Tyr Lys Glu
625                 630                 635                 640

Ile Ile Asn Arg Gln Lys Ala Glu Ile Gln Asn Leu Leu Asp Lys Val
                645                 650                 655

Lys Thr Ala Asp Gln Leu Gln Glu Gln Leu Gln Arg Gly Lys Gln Glu
            660                 665                 670

Ile Glu Asn Leu Lys Glu Glu Val Glu Ser Leu Asn Ser Leu Ile Asn
        675                 680                 685

Asp Leu Gln Lys Asp Ile Glu Gly Ser Arg Lys Arg Glu Ser Glu Leu
    690                 695                 700

Leu Leu Phe Thr Glu Arg Leu Thr Ser Lys Asn Ala Gln Leu Gln Ser
705                 710                 715                 720

Glu Ser Asn Ser Leu Gln Ser Gln Phe Asp Lys Val Ser Cys Ser Glu
                725                 730                 735

Ser Gln Leu Gln Ser Gln Cys Glu Gln Met Lys Gln Thr Asn Ile Asn
            740                 745                 750

Leu Glu Ser Arg Leu Leu Lys Glu Glu Glu Leu Arg Lys Glu Glu Val
        755                 760                 765

Gln Thr Leu Gln Ala Glu Leu Ala Cys Arg Gln Thr Glu Val Lys Ala

770                 775                 780

Leu Ser Thr Gln Val Glu Glu Leu Lys Asp Glu Leu Val Thr Gln Arg
785                 790                 795                 800

Arg Lys His Ala Ser Ser Ile Lys Asp Leu Thr Lys Gln Leu Gln Gln
                805                 810                 815

Ala Arg Arg Lys Leu Asp Gln Val Glu Ser Gly Ser Tyr Asp Lys Glu
            820                 825                 830

Val Ser Ser Met Gly Ser Arg Ser Ser Ser Ser Gly Ser Leu Asn Ala
        835                 840                 845

Arg Ser Ser Ala Glu Asp Arg Ser Pro Glu Asn Thr Gly Ser Ser Val
    850                 855                 860

Ala Val Asp Asn Phe Pro Gln Val Asp Lys Ala Met Leu Ile Glu Arg
865                 870                 875                 880

Ile Val Arg Leu Gln Lys Ala His Ala Arg Lys Asn Glu Lys Ile Glu
                885                 890                 895

Phe Met Glu Asp His Ile Lys Gln Leu Val Glu Glu Ile Arg Lys Lys
            900                 905                 910

Thr Lys Ile Ile Gln Ser Tyr Ile Leu Arg Glu Glu Ser Gly Thr Leu
        915                 920                 925

Ser Ser Glu Ala Ser Asp Phe Asn Lys Val His Leu Ser Arg Arg Gly
    930                 935                 940

Gly Ile Met Ala Ser Leu Tyr Thr Ser His Pro Ala Asp Asn Gly Leu
945                 950                 955                 960

Thr Leu Glu Leu Ser Leu Glu Ile Asn Arg Lys Leu Gln Ala Val Leu
                965                 970                 975

Glu Asp Thr Leu Leu Lys Asn Ile Thr Leu Lys Glu Asn Leu Gln Thr
            980                 985                 990

Leu Gly Thr Glu Ile Glu Arg Leu  Ile Lys His Gln His  Glu Leu Glu
        995                 1000                1005

Gln Arg  Thr Lys Lys Thr
    1010

<210>  252
<211>  1491
<212>  DNA
<213>  Homo sapiens

<400>  252
ttgccgtgaa gggctgtgcg gttcccgtgc gcgccggagc ctgctgtggc ctcttatgca      60

ctccaccacc cccatcagct ccctcttctc cttcaccagc cccgcagtga agagactgct     120

aggctggaag caaggagatg aagaggaaaa gtgggcagag aaggcagtgg actctctagt     180

gaagaagtta aagaagaaga agggagccat ggacgagctg gagagggctc tcagctgccc     240

ggggcagccc agcaaatgcg tcacgattcc ccgctccctg gacgggcggc tgcaggtgtc     300

ccaccgcaag ggcctgcccc atgtgattta ctgtcgcgtg tggcgctggc cggatctgca     360

gtcccaccac gagctgaagc cgctggagtg ctgtgagttc ccatttggct ccaagcagaa     420

agaagtgtgc attaaccctt accactaccg ccgggtggag actccagtac tgcctcctgt 480

gctcgtgcca agacacagtg aatataaccc ccagctcagc ctcctggcca agttccgcag 540

cgcctccctg cacagtgagc cactcatgcc acacaacgcc acctatcctg actctttcca 600

gcagcctccg tgctctgcac tccctccctc acccagccac gcgttctccc agtccccgtg 660

cacggccagc taccctcact ccccaggaag tccttctgag ccagagagtc cctatcaaca 720

ctcagttgac acaccacccc tgccttatca tgccacagaa gcctctgaga cccagagtgg 780

ccaacctgta gatgccacag ctgatagaca tgtagtgcta tcgataccaa atggagactt 840

tcgaccagtt tgttacgagg agccccagca ctggtgctcg gtcgcctact atgaactgaa 900

caaccgagtt ggggagacat tccaggcttc ctcccgaagt gtgctcatag atgggttcac 960

cgacccttca aataacagga acagattctg tcttggactt ctttctaatg taaacagaaa 1020

ctcaacgata gaaaatacca ggagacatat aggaaagggt gtgcacttgt actacgtcgg 1080

gggagaggtg tatgccgagt gcgtgagtga cagcagcatc tttgtgcaga gccggaactg 1140

caactatcaa cacggcttcc acccagctac cgtctgcaag atccccagcg gctgcagcct 1200

caaggtcttc aacaaccagc tcttcgctca gctcctggcc cagtcagttc accacggctt 1260

tgaagtcgtg tatgaactga ccaagatgtg tactatccgg atgagttttg ttaagggttg 1320

gggtgctgag tatcatcgcc aggatgtcac cagcaccccc tgctggattg agattcatct 1380

tcatgggcca ctgcagtggc tggacaaagt tctgactcag atgggctctc cacataaccc 1440

catttcttca gtgtcttaac agtcatgtct taagctgcat ttccatagga t 1491

<210> 253
<211> 467
<212> PRT
<213> Homo sapiens

<400> 253

Met His Ser Thr Thr Pro Ile Ser Ser Leu Phe Ser Phe Thr Ser Pro
1               5                   10                  15

Ala Val Lys Arg Leu Leu Gly Trp Lys Gln Gly Asp Glu Glu Glu Lys
            20                  25                  30

Trp Ala Glu Lys Ala Val Asp Ser Leu Val Lys Lys Leu Lys Lys Lys
        35                  40                  45

Lys Gly Ala Met Asp Glu Leu Glu Arg Ala Leu Ser Cys Pro Gly Gln
    50                  55                  60

Pro Ser Lys Cys Val Thr Ile Pro Arg Ser Leu Asp Gly Arg Leu Gln
65                  70                  75                  80

Val Ser His Arg Lys Gly Leu Pro His Val Ile Tyr Cys Arg Val Trp
                85                  90                  95

Arg Trp Pro Asp Leu Gln Ser His His Glu Leu Lys Pro Leu Glu Cys
            100                 105                 110

Cys Glu Phe Pro Phe Gly Ser Lys Gln Lys Glu Val Cys Ile Asn Pro
        115                 120                 125

Tyr His Tyr Arg Arg Val Glu Thr Pro Val Leu Pro Pro Val Leu Val

130                 135                 140

Pro Arg His Ser Glu Tyr Asn Pro Gln Leu Ser Leu Leu Ala Lys Phe
145                 150                 155                 160

Arg Ser Ala Ser Leu His Ser Glu Pro Leu Met Pro His Asn Ala Thr
                165                 170                 175

Tyr Pro Asp Ser Phe Gln Gln Pro Pro Cys Ser Ala Leu Pro Pro Ser
            180                 185                 190

Pro Ser His Ala Phe Ser Gln Ser Pro Cys Thr Ala Ser Tyr Pro His
        195                 200                 205

Ser Pro Gly Ser Pro Ser Glu Pro Glu Ser Pro Tyr Gln His Ser Val
    210                 215                 220

Asp Thr Pro Pro Leu Pro Tyr His Ala Thr Glu Ala Ser Glu Thr Gln
225                 230                 235                 240

Ser Gly Gln Pro Val Asp Ala Thr Ala Asp Arg His Val Val Leu Ser
                245                 250                 255

Ile Pro Asn Gly Asp Phe Arg Pro Val Cys Tyr Glu Glu Pro Gln His
            260                 265                 270

Trp Cys Ser Val Ala Tyr Tyr Glu Leu Asn Asn Arg Val Gly Glu Thr
        275                 280                 285

Phe Gln Ala Ser Ser Arg Ser Val Leu Ile Asp Gly Phe Thr Asp Pro
    290                 295                 300

Ser Asn Asn Arg Asn Arg Phe Cys Leu Gly Leu Leu Ser Asn Val Asn
305                 310                 315                 320

Arg Asn Ser Thr Ile Glu Asn Thr Arg Arg His Ile Gly Lys Gly Val
                325                 330                 335

His Leu Tyr Tyr Val Gly Gly Glu Val Tyr Ala Glu Cys Val Ser Asp
            340                 345                 350

Ser Ser Ile Phe Val Gln Ser Arg Asn Cys Asn Tyr Gln His Gly Phe
        355                 360                 365

His Pro Ala Thr Val Cys Lys Ile Pro Ser Gly Cys Ser Leu Lys Val
    370                 375                 380

Phe Asn Asn Gln Leu Phe Ala Gln Leu Leu Ala Gln Ser Val His His
385                 390                 395                 400

Gly Phe Glu Val Val Tyr Glu Leu Thr Lys Met Cys Thr Ile Arg Met
                405                 410                 415

Ser Phe Val Lys Gly Trp Gly Ala Glu Tyr His Arg Gln Asp Val Thr
            420                 425                 430

Ser Thr Pro Cys Trp Ile Glu Ile His Leu His Gly Pro Leu Gln Trp
        435                 440                 445

Leu Asp Lys Val Leu Thr Gln Met Gly Ser Pro His Asn Pro Ile Ser
    450                 455                 460

Ser Val Ser
465

<210>  254
<211>  5857
<212>  DNA

<213>  Homo sapiens

<400>  254
tgcaggtttg cagggtctga gattacttgg gcttttcctg cctttttctt ttgcttaagg      60

gatggacaag gagctgagat ttatgaccct tattagagaa aaaaatgtgc cttgctaggg     120

tggggacact tggttgatgc agtctctctc tctctttctc ggtgtttata acaaaacaaa     180

accaaaatga actgaggggt ttgtaatggt agtttgtttg ttgctggaga atgctacttt     240

gcatgctttt tttctcttgc agggtatgtt ctgtcttgtg ctttttcttt tagaagctac     300

taaagggtgt tggggatgct tctgactatt atgaaggcca aaaggcctgt tgactggggc     360

tgcttttaac cctttcctat ttgctgagaa tgcagccgtg tgacagtaac tgaacattgg     420

[…]

tcgacattca gccagtttag cccgatctcc agtgctgaag agtttgatga cgacgagaag     900

attgaggtgg atgacccccc tgacaaggag gacatgcgat caagcttcag gtcgaatgtg     960

ttgacggggt cggctcccca gcaggactac gataagctga aggcactcgg aggggaaaac    1020

tccagcaaaa ctggactctc tacgtcaggc aatgtggaga aaaacaaagc tgttaagaga    1080

gaaacagaag ccagttctat aaacctgagt gtttatgaac cttttaaagt cagaaaagca    1140

gaggataaat tgaaggaaag ctctgacaag gtgctggaaa acagagtcct agatgggaag    1200

ctgagctccg agaagaatga caccagcctc cccagcgttg cgccatcaaa gacaaagtcg    1260

tcctccaagc tctcgtcctg catcgctgcc atcgcggctc tcagcgctaa aaaggcggct    1320

tcagactcct gcaaagaacc agtggccaat tcgagggaat cctccccgtt accaaaagaa    1380

gtaaatgaca gtccgagagc cgctgacaag tctcctgaat cccagaatct catcgacggg    1440

accaaaaaac catccctgaa gcaaccggat agtcccagaa gcatctcaag tgagaacagc    1500

agcaaaggat ccccgtcctc tcccgcaggg tccacaccag caatccccaa agtccgcata    1560

aaaaccatta agacatcttc tggggaaatc aagagaacag tgaccagggt attgccagaa    1620

gtggatcttg actctggaaa gaaaccttcc gagcagacag cgtccgtgat ggcctctgtg    1680

acatcccttc tgtcgtctcc agcatcagcc gccgtccttt cctctccccc cagggcgcct    1740

ctccagtctg cggtcgtgac caatgcagtt tcccctgcag agctcacccc caaacaggtc    1800

acaatcaagc ctgtggctac tgctttcctc ccagtgtctg ctgtgaagac ggcaggatcc    1860

caagtcatta atttgaagct cgctaacaac accacggtga aagccacggt catatctgct    1920

gcctctgtcc agagtgccag cagcgccatc attaaagctg ccaacgccat ccagcagcaa    1980

actgtcgtgg tgccggcatc cagcctggcc aatgccaaac tcgtgccaaa gactgtgcac 2040
cttgccaacc ttaacctttt gcctcagggt gcccaggcca cctctgaact ccgccaagtg 2100
ctaaccaaac ctcagcaaca aataaagcag gcaataatca atgcagcagc ctcgcaaccc 2160
cccaaaaagg tgtctcgagt ccaggtggtg tcgtccttgc agagttctgt ggtggaagct 2220
ttcaacaagg tgctgagcag tgtcaatcca gtccctgttt acatcccaaa cctcagtcct 2280
cccgccaatg cagggatcac gttaccgacg cgtgggtaca agtgcttgga gtgtggggac 2340
tcctttgcac ttgaaaagag tctgacccag cactacgaca gacggagcgt gcgcatcgaa 2400
gtaacgtgca accattgtac aaagaacctc gttttttaca acaaatgcag cctcctttcc 2460
catgcccgtg ggcataagga gaaaggggtg gtaatgcaat gctcccactt aattttaaag 2520
ccagtcccag cagatcaaat gatagtttct ccgtcaagca atacttccac ttcaacttcc 2580
actcttcaga gccctgtggg agctggcaca cacactgtca caaaaattca gtctggcata 2640
actgggacag tcatatcggc tccttcaagc actcccatca ccccagccat gcccctagat 2700
gaagacccct ccaaactgtg tagacatagt ctaaaatgtt tggagtgtaa tgaagtcttc 2760
caggacgaga catcactggc tacacatttc cagcaggctg cagatacgag tggacaaaag 2820
acttgcacta tctgccagat gctgcttcct aaccagtgca gttatgcatc acaccagaga 2880
atccatcagc acaaatctcc ctacacctgc cctgagtgtg gggccatctg caggtcggtg 2940
cacttccaga cccacgtcac caagaactgt ctgcactaca cgaggagagt tggttttcga 3000
tgtgtgcatt gcaatgttgt gtactctgat gtggctgctc tgaagtctca cattcaaggt 3060
tctcactgtg aagtcttcta caagtgtcct atttgtccaa tggcgtttaa gtctgcccca 3120
agcacacatt cccacgccta cacacagcat cctggcatca agataggaga accaaaaata 3180
atatataagt gttccatgtg cgacactgtg ttcaccctgc aaaccttgct gtatcgccac 3240
tttgaccaac acattgaaaa ccagaaggtg tctgttttca agtgtccaga ctgttctctt 3300
ttatatgcac agaagcaact tatgatggac catatcaagt ctatgcatgg aacattgaaa 3360
agtattgaag ggcctccaaa cttgggtata aacttgcctt tgagcattaa gcctgcaact 3420
caaaattcag caaatcagaa caaagaggac accaaatcca tgaatgggaa agagaaattg 3480
gaaaagaaat ctccatctcc tgtgaaaaaa tcaatggaaa ccaagaaagt ggccagtcct 3540
gggtggacgt gttgggagtg tgactgcctg ttcatgcaga gagatgtgta catatcccac 3600
gtgaggaagg agcacgggaa gcaaatgaag aaacacccct gccgccagtg tgacaagtct 3660
ttcagctcgt cccacagcct gtgccggcac aaccggatca agcacaaagg catcaggaaa 3720
gtgtacgcct gctcgcactg cccagactcc agacgtacct ttaccaaacg tttgatgctg 3780
gagaagcacg tccagctgat gcatggcatc aaggaccctg acctgaaaga aatgacagat 3840
gccaccaatg aggaggaaac agaaataaaa gaagacacta aggtccccag tcccaagcgg 3900
aagttggaag aaccagttct ggagttcagg cctccccgag gagcaatcac tcaaccactg 3960
aaaaagctga aaatcaatgt ttttaaggtt cacaagtgtg ccgtgtgtgg cttcaccacc 4020

gaaaacctgc tgcaattcca cgaacacatc cctcagcaca aatcggatgg ttcttcctac 4080
cagtgccggg agtgtggcct ctgctacacg tctcacgtct ctctgtccag gcacctcttc 4140
atcgtacaca agttaaagga acctcagcca gtgtccaagc aaaatggggc tggggaagat 4200
aaccaacagg agaacaaacc cagccacgag gatgaatccc ctgatggcgc cgtgtcagac 4260
agaaagtgca aagtgtgcgc aaaaactttt gaaactgaag ctgccttaaa tactcacatg 4320
cggacacacg gcatggcctt catcaaatcc aaaaggatga gctcagccga gaaatagcca 4380
cagatgctcc atgaggaaaa tccctgtcca cattggaata aaaaagacat ttttgttaca 4440
aagtttgcag tataatagag ttaacagtac tgtctaggct gttgcaatat attctctttc 4500
aatgtacctt ccttcacctc gtcgtatata tcctcgataa gtattaaaac agtatttgag 4560
tttaaaagag tttgtatata tttaaatgaa taacttttta tactctttgt tacatgtttg 4620
tatcagtatt tagtggaaaa ccatttgagt tgttttgggt tagaattttt ctttttgtac 4680
tgtttcttta aaacagagtt cttagtaaca ggggcagttc ctgaattcaa ataaaccatt 4740
ttgtatgttt ggattttgaa tgggttaact aattacaggc taaaataatg ccttttttag 4800
tgtttttaat ttttagaatt cactacataa attgtaagta attgtgggtc tcaaaaacac 4860
taggaacttt taagtgtctt agcacttcct cgatgtgcct gccctgaggg agtgagttca 4920
catttgagac aactgcactc cagtgtggac gtgcctttgt cttcaggcca tgccgaaggg 4980
tgtttaaagc agtcttgcag gtcgctcctt tcccagccgt ggataaaaac tgaagctagg 5040
aatctaataa ggaatgctga tttcctcagt tccattttga ggaatgggga aggctattct 5100
aaagaaaaaa atgggatttg ttttctcggc agatctgcaa ggctggcttt aagagcacaa 5160
ggagggaaag taacgaaagg gctggactac tataaaagtt acaaatacgt agttagacca 5220
atagatttat atagtcaggt ttttgtcatg taatttatta actaactatt acagaaacac 5280
agctaagaat atcaagtatt tctctggctc ttgacagaaa aaaatcagtt gacttaaccc 5340
tttgctgtca aaagagttgg cgtttcctgt tctgggtgct actgccaaac gttatggtac 5400
ttagagtcgg gatgcacaac ttcaaccacc gacttatcaa tgcagccgcc tgtgtattgc 5460
aattggccgt taccttaagc actgagccac ccgggtttag ttcagccatt tcaagaagta 5520
tatttaacgt cggtagttct gctttattaa aatgcagcag aggtactctt ctgtcccttc 5580
cgtttatagt tctctgagag agttctattt tttggttttg ttttgtgttt tcttttgcat 5640
tttgtatctt gtatttatcc ctgaacatgt tttgtacctt tttttttttt ttttttttaa 5700
gaaaaggaat tcttttgtgt atatatagat acttgcatga tatactgtag tcaatgttcg 5760
gttcctcaaa aggtcttgct gctgtcaggt gttatgcact ccatccatca taactgtatg 5820
aaacacattt catatgtaaa taaacgtggg acatttg 5857

<210> 255
<211> 1296
<212> PRT
<213> Homo sapiens

<400> 255

<210>  256
<211>  2788
<212>  DNA
<213>  Homo sapiens

<400>  256
ttttcgtcga ctcttaccgg ttggctgggc cagctgcgcc gcggctcaca gctgacgatg      60

ggggacccca gcaagcagga catcttgacc atcttcaagc gcctccgctc ggtgcccact     120

aacaaggtgt gttttgattg tggtgccaaa aatcccagct gggcaagcat aacctatgga     180

gtgttccttt gcattgattg ctcagggtcc caccggtcac ttggtgttca cttgagtttt     240

attcgatcta cagagttgga ttccaactgg tcatggtttc agttgcgatg catgcaagtc     300

ggaggaaacg ctagtgcatc ttcctttttt catcaacatg ggtgttccac caatgacacc     360

aatgccaagt acaacagtcg tgctgctcag ctctataggg agaaaatcaa atcgctcgcc     420

tctcaagcaa cacggaagca tggcactgat ctgtggcttg atagttgtgt ggttccacct     480

ttgtcccctc caccaaagga ggaagatttt tttgcctctc acgtttctcc tgaggtgagt 540
gacacagcgt gggcatcagc aatagcagaa ccatcttctt taacatcaag gcctgtggaa 600
accactttgg aaaataatga aggtggacaa gagcaaggac caagtgtgga aggtcttaat 660
gtaccaacaa aggctacttt agaggtatcc tctatcataa aaaagaaacc aaatcaagct 720
aaaaaaggcc ttggggccaa aaaaggaagt ttgggagctc agaaactggc aaacacatgc 780
tttaatgaaa ttgaaaaaca agctcaagct gcggataaaa tgaaggagca ggaagacctg 840
gccaaggtgg tatctaaaga agaatcaatt gtttcatcat tacgattagc ctataaggat 900
cttgaaattc aaatgaagaa agacgaaaag atgaacatta gtggcaaaaa aaatgttgac 960
tcagacagac tcggcatggg atttggaaat tgcagaagtg ttatttcaca ttcagtgact 1020
tcagatatgc agaccataga gcaggaatca cccattatgg caaaaccaag aaaaaagtat 1080
aatgatgaca gtgacgattc atattttact tccagctcaa gttactttga cgagccagtg 1140
gagttaagga gcagttcttt ctctagctgg gatgacagtt cagattccta ttggaaaaaa 1200
gagaccagca aagatactga aacagttctg aaaaccacag gctattcaga cagacctact 1260
gctcgccgca agccagatta tgagccagtt gaaaatacag atgaggccca gaagaagttt 1320
ggcaatgtca aggccatttc atcagatatg tattttggaa gacaatccca ggctgattat 1380
gagaccaggg cccgcctaga gaggctgtcg gcaagttcct ccataagctc ggctgatctg 1440
ttcgaggagc cgaggaagca gccagcaggg aactacagcc tgtccagtgt gctgcccaac 1500
gcccccgaca tggcgcagtt caagcaggga gtgagatcgg ttgctggaaa actctccgtc 1560
tttgctaatg gagtcgtgac ttcaattcag gatcgctacg gttcttaata ctgaagtcat 1620
gatgtgtatt tcctggagaa attcctcttt aaatgaacaa gtaaccacat ctcaggcggc 1680
agtgaagtcc agatagtttt gcagattgtt ttgctacttt ttcatatggt atatgtttct 1740
gatttttaat atttcttttg agaaattctg agttctgatg taggagcttt cctgtgattt 1800
ctgtttcacg ttccttcctg tcacaccctc ctttggcgtc tctgtgtata tccttgcttt 1860
attttcttgg aacctttgat ttcaacactg agggcctgga gacctcggct cctcctgctc 1920
ctgaaccagg aggcttcatg tgggggagga ggagaggtct ccatgtgaca catgggctca 1980
gggctgccag aatcagcgga tgctggatgg gcctgcagaa acaacactca ccacacacac 2040
ttccttcaaa agaccaaaag tgactggtgt ctcgtgtgac agattgcttc atttatgttt 2100
ctacatagta aggtgactgc caaataatat ttgaagtcat ctgtctcttt gtaaattatt 2160
ttatatgacc tataaattta aaaatgtttt tcagtgagtg cttttaacaa acttaagctt 2220
ctgccctgcc aagggaatta atgttatctt gtgaaaggtg ttgctgtttg aattgatgag 2280
aaatggaaga tgagaactcc ctaagagttc tcataataaa tcatctcatc acaaatcaat 2340
acggtataca gagttaaagt ggaatgaggt aagaagatac agctacagaa aatagttgcg 2400
tgtatgggag aacagtcatt gtaattgggt agttttgtta ataaatattt ttaaatcttg 2460
cttttcagaa attaccgaat gtgtataaac aaataaagaa aaataattta gctgtgtttt 2520

agacagcatt agaatatatt gttcagcaca gtaaaatata tttgaaattt gataagccaa 2580

aaatgtggtt ttgaatgaat attttgtgaa tctttcttaa aagctcaaat ttgtagactt 2640

ctaaatagaa taaacacttg cagcagaaaa aaaaaaaaaa aaaaaaaaaa aaaaaaaaaa 2700

aaaaaaaaaa aaaaaaaaaa aaaaaaaaaa aaaaaaaaaa aaaaaaaaaa aaaaaaaaaa 2760

aaaaaaaaaa aaaaaaaaaa aaaaaaaa 2788

<210> 257
<211> 516
<212> PRT
<213> Homo sapiens

<400> 257

Met Gly Asp Pro Ser Lys Gln Asp Ile Leu Thr Ile Phe Lys Arg Leu
1               5                   10                  15

Arg Ser Val Pro Thr Asn Lys Val Cys Phe Asp Cys Gly Ala Lys Asn
            20                  25                  30

Pro Ser Trp Ala Ser Ile Thr Tyr Gly Val Phe Leu Cys Ile Asp Cys
        35                  40                  45

Ser Gly Ser His Arg Ser Leu Gly Val His Leu Ser Phe Ile Arg Ser
    50                  55                  60

Thr Glu Leu Asp Ser Asn Trp Ser Trp Phe Gln Leu Arg Cys Met Gln
65                  70                  75                  80

Val Gly Gly Asn Ala Ser Ala Ser Ser Phe Phe His Gln His Gly Cys
                85                  90                  95

Ser Thr Asn Asp Thr Asn Ala Lys Tyr Asn Ser Arg Ala Ala Gln Leu
            100                 105                 110

Tyr Arg Glu Lys Ile Lys Ser Leu Ala Ser Gln Ala Thr Arg Lys His
        115                 120                 125

Gly Thr Asp Leu Trp Leu Asp Ser Cys Val Val Pro Pro Leu Ser Pro
    130                 135                 140

Pro Pro Lys Glu Glu Asp Phe Phe Ala Ser His Val Ser Pro Glu Val
145                 150                 155                 160

Ser Asp Thr Ala Trp Ala Ser Ala Ile Ala Glu Pro Ser Ser Leu Thr
                165                 170                 175

Ser Arg Pro Val Glu Thr Thr Leu Glu Asn Asn Glu Gly Gly Gln Glu
            180                 185                 190

Gln Gly Pro Ser Val Glu Gly Leu Asn Val Pro Thr Lys Ala Thr Leu
        195                 200                 205

Glu Val Ser Ser Ile Ile Lys Lys Lys Pro Asn Gln Ala Lys Lys Gly
    210                 215                 220

Leu Gly Ala Lys Lys Gly Ser Leu Gly Ala Gln Lys Leu Ala Asn Thr
225                 230                 235                 240

Cys Phe Asn Glu Ile Glu Lys Gln Ala Gln Ala Ala Asp Lys Met Lys
                245                 250                 255

Glu Gln Glu Asp Leu Ala Lys Val Val Ser Lys Glu Glu Ser Ile Val
            260                 265                 270

Ser Ser Leu Arg Leu Ala Tyr Lys Asp Leu Glu Ile Gln Met Lys Lys
        275                 280                 285

Asp Glu Lys Met Asn Ile Ser Gly Lys Lys Asn Val Asp Ser Asp Arg
    290                 295                 300

Leu Gly Met Gly Phe Gly Asn Cys Arg Ser Val Ile Ser His Ser Val
305                 310                 315                 320

Thr Ser Asp Met Gln Thr Ile Glu Gln Glu Ser Pro Ile Met Ala Lys
                325                 330                 335

Pro Arg Lys Lys Tyr Asn Asp Asp Ser Asp Asp Ser Tyr Phe Thr Ser
            340                 345                 350

Ser Ser Ser Tyr Phe Asp Glu Pro Val Glu Leu Arg Ser Ser Ser Phe
        355                 360                 365

Ser Ser Trp Asp Asp Ser Ser Asp Ser Tyr Trp Lys Lys Glu Thr Ser
    370                 375                 380

Lys Asp Thr Glu Thr Val Leu Lys Thr Thr Gly Tyr Ser Asp Arg Pro
385                 390                 395                 400

Thr Ala Arg Arg Lys Pro Asp Tyr Glu Pro Val Glu Asn Thr Asp Glu
                405                 410                 415

Ala Gln Lys Lys Phe Gly Asn Val Lys Ala Ile Ser Ser Asp Met Tyr
            420                 425                 430

Phe Gly Arg Gln Ser Gln Ala Asp Tyr Glu Thr Arg Ala Arg Leu Glu
        435                 440                 445

Arg Leu Ser Ala Ser Ser Ser Ile Ser Ser Ala Asp Leu Phe Glu Glu
    450                 455                 460

Pro Arg Lys Gln Pro Ala Gly Asn Tyr Ser Leu Ser Ser Val Leu Pro
465                 470                 475                 480

Asn Ala Pro Asp Met Ala Gln Phe Lys Gln Gly Val Arg Ser Val Ala
                485                 490                 495

Gly Lys Leu Ser Val Phe Ala Asn Gly Val Val Thr Ser Ile Gln Asp
            500                 505                 510

Arg Tyr Gly Ser
        515

<210>  258
<211>  5571
<212>  DNA
<213>  Homo sapiens

<400>  258
atggatactg tcatgaagca gacacatgct gacacacctg ttgatcattg tctatctggc      60

ataagaaagt gtagcagcac ctttaagctt aaaagtgaag tcaacaagca tgaaacagcc     120

cttgaaatgc agaatccaaa tttgaacaat aaagaatgtt gtttcacctt tacgttgaat     180

ggaaactcca gaaaattaga ccgtagtgtg tttacagcat atggtaaacc cagcgagagt     240

atctactcag ccctgagtgc taatgactat ttcagtgaaa ggataaagaa tcagtttaat     300

aagaacatta ttgtttatga agaaaagaca atagatggac atataaattt aggaatgcct     360

ctcaagtgcc tgcctagtga ttctcatttt aaaattacat ttggtcaaag aaagagtagc 420
aaagaagatg gacacatatt acgccaatgt gaaaatccaa acatggaatg cattcttttt 480
catgttgttg ctataggaag gacaagaaag aagattgtta agatcaacga acttcatgaa 540
aaaggaagta aactttgtat ttatgccttg aagggtgaga ctattgaagg agccttatgc 600
aaggatggcc gttttcggtc tgacataggt gaatttgaat ggaaactaaa ggaaggtcat 660
aagaaaattt atggaaaaca gtccatggtg gatgaagtat ctggaaaagt cttagaaatg 720
gacatttcaa aaaaaaaagc attacaacag aaagatatcc ataaaaaaat taaacagaat 780
gaaagtgcca ctgatgaaat taatcaccag agtctgatac agtctaagaa aaaagtccac 840
aaaccaaaga aagatggaga gaccaaagat gtagaacaca gcagagagca aattctccca 900
cctcaggatc taagccatta tattaaagat aaaactcgcc agacaattcc caggattaga 960
aattattact tttgtagttt gccccgaaaa tataggcaaa taaactcaca agttagacgg 1020
aggccgcatc tgggtaggcg gtatgctatt aatctggatg tccaaaagga ggcaattaat 1080
ctcttaaaga attatcaaac gttgaatgaa gccataatgc atcagtatcc gaattttaaa 1140
gaggaggcac agtgggtaag aaaatatttt cgggaagaac aaaagagaat gaatctttca 1200
ccagctaagc aattcaacat atataaaaag gacttcggaa aaatgactgc aaattctgtt 1260
tcagttgcaa cctgcgaaca gcttacatat tatagcaagt cagttgggtt catgcaatgg 1320
gacaataatg gaaacacagg taatgctact tgctttgtct tcaatggtgg ttatattttc 1380
acctgtcgac atgttgtaca tcttatggtg ggtaaaaaca cacatccaag tttgtggcca 1440
gatataatta gcaaatgtgc gaaggtaacc ttcacttata cagagttctg ccctactcct 1500
gacaattggt tttccattga gccatggctt aaagtgtcca atgaaaatct agattatgcc 1560
attttaaaac taaaagaaaa tggaaatgcg tttcctccag gactatggcg acagatttct 1620
cctcaaccat ctactggttt gatttattta attggtcatc ctgaaggcca gatcaagaaa 1680
atagatggtt gtactgtgat tcctctaaac gaacgattga aaaaatatcc aaacgattgt 1740
caagatgggt tggtagatct ctatgatacc accagtaatg tatactgtat gtttacccaa 1800
agaagtttcc tatcagaggt ttggaacaca cacacgctta gttatgatac ttgtttctct 1860
gatgggtcct caggctcccc agtgtttaat gcatctggca aattggttgc tttgcatacc 1920
tttgggcttt tttatcaacg aggatttaat gtgcatgccc ttattgaatt tggttattct 1980
atggattcta ttctttgtga tattaaaaag acaaatgaga gcttgtataa atcattaaat 2040
gatgagaaac ttgagaccta cgatgaagag aaagcccggc ccaggccagc ctaccggcga 2100
ctaggatgct ttcgctttcg ctctcgcttt ccaatactcg ggactgggga aaccgggaga 2160
atagaagcag gcaaggaccg ccgtgggcac ggggtcagtg agacagggtc ctgctcgcgg 2220
cgtcaaggag gagcgctgtg ggtgtcccca gcgcagccaa tcggcttccg aagtagctgg 2280
agctctggag cctttgcttc ctcaaatacg agcgggaact gcgttgagcg ctggattcca 2340
ggccgagtgc tggcgaggcg cgcagtctct aaagagcaac agaataattg cagtacttct 2400

ctaatgagga tggagtctag aggagaccca agagccacaa ctaataccca ggctcaaaga 2460
ttccattcac ctaagaaaaa tccagaagac cagaccatgc cccaaaatag gacaatatat 2520
gttaccttga aggctgtcag aaaagagata gaaactcacc aaggccaaga aatgcttgtg 2580
cgtggcacag aaggaatcaa agagtacata aaccttggaa tgcccctcag ttgtttccct 2640
gaaggtggcc aggtggtcat tacattttcc caaagtaaaa gtaagcagaa ggaagataac 2700
cacatatttg gcaggcagga caaagcatcg actgaatgtg tcaaatttta cattcatgca 2760
attggaattg ggaagtgtaa aagaaggatt gttaaatgtg ggaagcttca caaaaagggg 2820
cgcaaactct gtgtttatgc tttcaaagga gaaaccatca aggatgcact gtgcaaggat 2880
ggcagatttc tttcctttct ggagaatgat gattggaaac tcattgaaaa caatgacacc 2940
attttagaaa gcacccagcc agttgatgaa ttagaaggca gatactttca ggttgaggtt 3000
gagaaaagaa tggtccccag tgcagcagct tctcagaatc ctgagtcaga gaaaagaaac 3060
acctgtgtgt tgagagaaca aatcgtggct cagtacccca gtttgaaaag agaaagtgaa 3120
aaaatcattg aaaacttcaa gaaaaaaatg aaagtaaaaa atggggaaac attatttgaa 3180
ttgcatagaa caacgtttgg gaaagtaaca aaaaattctt cttcgattaa agtagtgaaa 3240
cttcttgtac gtctcagtga ctcagttggg tacttattct gggacagtgc aactacgggt 3300
tacgccacct gctttgtttt taaaggattg ttcattttaa cttgtcggca tgtaatagat 3360
agcattgtgg gagacggaat agagccaagt aagtgggcaa ccataattgg tcaatgtgta 3420
agggtgacat ttggttatga agagctaaaa gacaaggaaa caaactactt ttttgttgaa 3480
ccttggtttg agatacataa tgaagagctt gactatgctg tcctgaaact gaaggaaaat 3540
ggacaacaag tacctatgga actatataat ggaattactc ctgtgccact tagtgggttg 3600
atacatatta ttggccatcc atatggagaa aaaaagcaga ttgatgcttg tgctgtgatc 3660
cctcagggtc agcgagcaaa gaaatgtcag gaacgtgttc agtctaaaaa agcagaaagt 3720
ccagagtatg tccatatgta tactcaaaga agtttccaga aaatagttca caaccctgat 3780
gtgattacct atgacactga atttttcttt ggggcttccg gctcccctgt gtttgattca 3840
aaaggttcat tggtggccat gcatgctgct ggctttgctt atacttacca aaatgagact 3900
cgtagtatca ttgagtttgg ctctaccatg gaatccatcc tccttgatat taagcaaaga 3960
cataaaccat ggtatgaaga agtatttgta aatcagcagg atgtagaaat gatgagtgat 4020
gaggacttgt gagaattcag tctactggat ttaaggaaat ggcttatgga gttgttattt 4080
cgtaggcatt gaaaatggtt ttctaaactc caaaatggtc atcttatcaa taataataat 4140
attgaccatt tcctatctgc caggcatttt tctaagcaca tgaagaaatt agtcctaaca 4200
acactatgag atggactata acttgcccaa attttttttt tttttgagac tgagtctcac 4260
tctgtcgcct gggctggagt acagtggtgc gatctcagct cactgcaact tccacctccc 4320
aggttcaagc gattcttatg cctcagtctc ctgagcagct gggattacag gcaaacgcca 4380
ccacacccag ctaaattttt tttttttttt tgtattttta gtagagacag ggtttcacca 4440

tgttggtcag gcgggtctcg aactcctgac ctcgtgatcc acctgcctcg gccttccaaa 4500
gtgctgggat tacaagtttg agccactgca cctggctaac ttgccctatt ttaaagtcaa 4560
gcaatgggaa gaataacaag attatatagt aatcagtttc atgacactaa aagtcatata 4620
gtcatagggt tttttcatct ttcatatctt tgcctaaatt catttgctac agtgcaggaa 4680
ccaaaacttg ttcatctcat gattccctac atctgacata aggaaagtaa gtgctcagaa 4740
aaatgtgcag gtcaataagt tgcaaaagtt ggggctgcaa ttaatgctaa cataagagct 4800
aaatgcttga ttagaaatga tctcaaaacc ttttagaatt tccaaaatct tcatattact 4860
gaaactgtcg gaatatatgg gtcctgaaat tcagaagatg atagtcactc ttcccatatt 4920
tataggctat taaggcaagg gatatcttaa acatcatatt actttattta gatttctact 4980
actccaatta ttaatgttat gtatttctca ttgttttact tcttcatggt attatgaaga 5040
ctatatagat gattcaacca agcctgcaaa tctccctctt gtggaattcc actggaccca 5100
atctgttttc catttccatt gcaatactac taaagccata caatatcaag caccctccct 5160
ctaggtccag ggactatcac agaagaagca ggcatgtaag attttaagga ctggtttcga 5220
ggggtcgagt gtaggaaaac agcctgttgc attgtaagag tgatgtcacc ttgaagagca 5280
gctggcatga tgactgctgt ttgactcctg cataccaaga tattctgcag caatgtcttt 5340
aaacagtgcc ggtagtacag ataacccctc ataaagatgc ttatctaacc tccccagtgt 5400
tcaggtgttt cacaagaaag tctgagatat gactagctac acgttttgcc aaaaatgctt 5460
gttatataaa gggtactttt gggagggtga gtgccgccat ttagtggctg ctagaaacat 5520
tgcttctgtt tgtaagttcc tattaaatgt tctttctgag aaaaaaaaaa a 5571

<210> 259
<211> 1343
<212> PRT
<213> Homo sapiens

<400> 259

<210>  260
<211>  2829
<212>  DNA
<213>  Homo sapiens

<400>  260
acatttcaaa aaaaatacat agactgatgt ttcagacttg tgcagcataa gcctacaggg      60

tacgaagaat gaactctgag aatgtttgga gaatgtttca tcattactaa caggatattc     120

ctcatgacat tgctgtctga tctttgacca tcagtctgtg acctgcccct tctctttaca     180

tgcagccgct ctctgctccc tgccccaatg aacatctgca ctaggcccaa gccttggagt     240

aatttacctg aagagtgaca ccattgattt tgaaactact gaagaaaccc aagacagctg     300

aaaaccagaa ggcatctgag gagaatgaga ttactcagcc gggtggatcc agcgccaagc     360

cgggccttcc ctgcctgaac tttgaagctg ttttgtctcc agacccagcc ctcatccact     420

caacacattc actgacaaac tctcacgctc acaccgggtc atctgattgt gacatcagtt     480

gcaaggggat gaccgagcgc attcacagca tcaaccttca caacttcagc aattccgtgc     540

tcgagaccct caacgagcag cgcaaccgtg gccacttctg tgacgtaacg gtgcgcatcc     600

acgggagcat gctgcgcgca caccgctgcg tgctggcagc cggcagcccc ttcttccagg     660

acaaactgct gcttggctac agcgacatcg agatcccgtc ggtggtgtca gtgcagtcag     720

tgcaaaagct cattgacttc atgtacagcg gcgtgctacg ggtctcgcag tcggaagctc     780

tgcagatcct cacggccgcc agcatcctgc agatcaaaac agtcatcgac gagtgcacgc     840

gcatcgtgtc acagaacgtg ggcgatgtgt tcccggggat ccaggactcg ggccaggaca     900

cgccgcgggg cactcccgag tcaggcacgt caggccagag cagcgacacg gagtcgggct 960
acctgcagag ccacccacag cacagcgtgg acaggatcta ctcggcactc tacgcgtgct 1020
ccatgcagaa tggcagcggc gagcgctctt tttacagcgg cgcagtggtc agccaccacg 1080
agactgcgct cggcctgccc cgcgaccacc acatggaaga ccccagctgg atcacacgca 1140
tccatgagcg ctcgcagcag atggagcgct acctgtccac cacccccgag accacgcact 1200
gccgcaagca gccccggcct gtgcgcatcc agaccctagt gggcaacatc cacatcaagc 1260
aggagatgga ggacgattac gactactacg ggcagcaaag ggtgcagatc ctggaacgca 1320
acgaatccga ggagtgcacg gaagacacag accaggccga gggcaccgag agtgagccca 1380
aaggtgaaag cttcgactcg ggcgtcagct cctccatagg caccgagcct gactcggtgg 1440
agcagcagtt tgggcctggg gcggcgcggg acagccaggc tgaacccacc caacccgagc 1500
aggctgcaga agcccccgct gagggtggtc cgcagacaaa ccagctagaa acaggtgctt 1560
cctctccgga gagaagcaat gaagtggaga tggacagcac tgttatcact gtcagcaaca 1620
gctccgacaa gagcgtccta caacagcctt cggtcaacac gtccatcggg cagccattgc 1680
caagtaccca gctctactta cgccagacag aaaccctcac cagcaacctg aggatgcctc 1740
tgaccttgac cagcaacacg caggtcattg gcacagctgg caacacctac ctgccagccc 1800
tcttcactac ccagcccgcg ggcagtggcc ccaagccttt cctcttcagc ctgccacagc 1860
ccctggcagg ccagcagacc cagtttgtga cagtgtccca gcccggtctg tcgaccttta 1920
ctgcacagct gccagcgcca cagcccctgg cctcatccgc aggccacagc acagccagtg 1980
ggcaaggcga aaaaaagcct tatgagtgca ctctctgcaa caagactttc accgccaaac 2040
agaactacgt caagcacatg ttcgtacaca caggtgagaa gccccaccaa tgcagcatct 2100
gttggcgctc cttctcctta aaggattacc ttatcaagca catggtgaca cacacaggag 2160
tgagggcata ccagtgtagt atctgcaaca agcgcttcac ccagaagagc tccctcaacg 2220
tgcacatgcg cctccaccgg ggagagaagt cctacgagtg ctacatctgc aaaaagaagt 2280
tctctcacaa gaccctcctg gagcgacacg tggccctgca cagtgccagc aatgggaccc 2340
cccctgcagg cacaccccca ggtgcccgcg ctggcccccc aggcgtggtg gcctgcacgg 2400
aggggaccac ttacgtctgc tccgtctgcc cagcaaagtt tgaccaaatc gagcagttca 2460
acgaccacat gaggatgcat gtgtctgacg gataagtagt atctttctct ctttcttatg 2520
aacaaaacaa aacaacaaca aaaaacaaac aaacaaaaaa gctatggcac tagaatttaa 2580
gaaatgtttt ggtttcattt ttactttctg tttttgtttt tgtttcgttt cattttgtac 2640
tacatgaaga actgtttttt gcctgctggt acattacatt tccggaggct tgggtgaata 2700
atagttttcc cagtctccct cggatggtgg ccttaaggcc tggtagtgct tcaagaggtc 2760
cactggttgg atctctagct actggcctct aaatacaacc cttctttaca aaaaaaaaaa 2820
aaaaaaaaa 2829
<210> 261

<211>  668
<212>  PRT
<213>  Homo sapiens

<400>  261

Met Thr Glu Arg Ile His Ser Ile Asn Leu His Asn Phe Ser Asn Ser
1               5                   10                  15

Val Leu Glu Thr Leu Asn Glu Gln Arg Asn Arg Gly His Phe Cys Asp
            20                  25                  30

Val Thr Val Arg Ile His Gly Ser Met Leu Arg Ala His Arg Cys Val
        35                  40                  45

Leu Ala Ala Gly Ser Pro Phe Phe Gln Asp Lys Leu Leu Leu Gly Tyr
    50                  55                  60

Ser Asp Ile Glu Ile Pro Ser Val Val Ser Val Gln Ser Val Gln Lys
65                  70                  75                  80

Leu Ile Asp Phe Met Tyr Ser Gly Val Leu Arg Val Ser Gln Ser Glu
                85                  90                  95

Ala Leu Gln Ile Leu Thr Ala Ala Ser Ile Leu Gln Ile Lys Thr Val
            100                 105                 110

Ile Asp Glu Cys Thr Arg Ile Val Ser Gln Asn Val Gly Asp Val Phe
        115                 120                 125

Pro Gly Ile Gln Asp Ser Gly Gln Asp Thr Pro Arg Gly Thr Pro Glu
    130                 135                 140

Ser Gly Thr Ser Gly Gln Ser Ser Asp Thr Glu Ser Gly Tyr Leu Gln
145                 150                 155                 160

Ser His Pro Gln His Ser Val Asp Arg Ile Tyr Ser Ala Leu Tyr Ala
                165                 170                 175

Cys Ser Met Gln Asn Gly Ser Gly Glu Arg Ser Phe Tyr Ser Gly Ala
            180                 185                 190

Val Val Ser His His Glu Thr Ala Leu Gly Leu Pro Arg Asp His His
        195                 200                 205

Met Glu Asp Pro Ser Trp Ile Thr Arg Ile His Glu Arg Ser Gln Gln
    210                 215                 220

Met Glu Arg Tyr Leu Ser Thr Thr Pro Glu Thr Thr His Cys Arg Lys
225                 230                 235                 240

Gln Pro Arg Pro Val Arg Ile Gln Thr Leu Val Gly Asn Ile His Ile
                245                 250                 255

Lys Gln Glu Met Glu Asp Asp Tyr Asp Tyr Tyr Gly Gln Gln Arg Val
            260                 265                 270

Gln Ile Leu Glu Arg Asn Glu Ser Glu Glu Cys Thr Glu Asp Thr Asp
        275                 280                 285

Gln Ala Glu Gly Thr Glu Ser Glu Pro Lys Gly Glu Ser Phe Asp Ser
    290                 295                 300

Gly Val Ser Ser Ser Ile Gly Thr Glu Pro Asp Ser Val Glu Gln Gln
305                 310                 315                 320

Phe Gly Pro Gly Ala Ala Arg Asp Ser Gln Ala Glu Pro Thr Gln Pro
                325                 330                 335

Glu Gln Ala Ala Glu Ala Pro Ala Glu Gly Gly Pro Gln Thr Asn Gln
            340                 345                 350

Leu Glu Thr Gly Ala Ser Ser Pro Glu Arg Ser Asn Glu Val Glu Met
        355                 360                 365

Asp Ser Thr Val Ile Thr Val Ser Asn Ser Ser Asp Lys Ser Val Leu
    370                 375                 380

Gln Gln Pro Ser Val Asn Thr Ser Ile Gly Gln Pro Leu Pro Ser Thr
385                 390                 395                 400

Gln Leu Tyr Leu Arg Gln Thr Glu Thr Leu Thr Ser Asn Leu Arg Met
                405                 410                 415

Pro Leu Thr Leu Thr Ser Asn Thr Gln Val Ile Gly Thr Ala Gly Asn
            420                 425                 430

Thr Tyr Leu Pro Ala Leu Phe Thr Thr Gln Pro Ala Gly Ser Gly Pro
        435                 440                 445

Lys Pro Phe Leu Phe Ser Leu Pro Gln Pro Leu Ala Gly Gln Gln Thr
    450                 455                 460

Gln Phe Val Thr Val Ser Gln Pro Gly Leu Ser Thr Phe Thr Ala Gln
465                 470                 475                 480

Leu Pro Ala Pro Gln Pro Leu Ala Ser Ser Ala Gly His Ser Thr Ala
                485                 490                 495

Ser Gly Gln Gly Glu Lys Lys Pro Tyr Glu Cys Thr Leu Cys Asn Lys
            500                 505                 510

Thr Phe Thr Ala Lys Gln Asn Tyr Val Lys His Met Phe Val His Thr
        515                 520                 525

Gly Glu Lys Pro His Gln Cys Ser Ile Cys Trp Arg Ser Phe Ser Leu
    530                 535                 540

Lys Asp Tyr Leu Ile Lys His Met Val Thr His Thr Gly Val Arg Ala
545                 550                 555                 560

Tyr Gln Cys Ser Ile Cys Asn Lys Arg Phe Thr Gln Lys Ser Ser Leu
                565                 570                 575

Asn Val His Met Arg Leu His Arg Gly Glu Lys Ser Tyr Glu Cys Tyr
            580                 585                 590

Ile Cys Lys Lys Lys Phe Ser His Lys Thr Leu Leu Glu Arg His Val
        595                 600                 605

Ala Leu His Ser Ala Ser Asn Gly Thr Pro Pro Ala Gly Thr Pro Pro
    610                 615                 620

Gly Ala Arg Ala Gly Pro Pro Gly Val Val Ala Cys Thr Glu Gly Thr
625                 630                 635                 640

Thr Tyr Val Cys Ser Val Cys Pro Ala Lys Phe Asp Gln Ile Glu Gln
                645                 650                 655

Phe Asn Asp His Met Arg Met His Val Ser Asp Gly
            660                 665

<210>  262
<211>  912
<212>  DNA
<213>  Homo sapiens

<400>  262
   atggtggaag aggaaacagg catatcttac atggtggcag acaagggaca cccttctaca      60
   aactctacca cttctgcgcc gtcgtttcga ccatataaaa acgacctatg cgaactgcgt     120
   cggaaaactc cctcacgatg taaaacgaag atcaggagca gatttgaaga attacaaagt     180
   gaattggtgc cagtcagcat gtcagagaca gaccacatag cctctacttc ctctgataaa     240
   aatgttggga aaacacctga attaaaggaa gactcatgca acttgttttc tggcaatgaa     300
   agcagcaaat tagaaaatga gtccaaacta ttgtcattaa acactgataa aactttatgt     360
   caacctaatg agcataataa tcgaattgaa gcccaggaaa attatattcc agatcatggt     420
   ggaggtgagg attcttgtgc caaaacagac acaggctcag aaaattctga acaaatagct     480
   aatttcccta gtggaaattt tgctaaacat atttcaaaaa caaatgaaac agaacagaaa     540
   gtaacacaaa tattggtgga attaaggtca tctacatttc cagaatcagc taatgaaaag     600
   acttattcag aaagccccta tgatacagac tgcaccaaga aatttatttc aaaaataaag     660
   agcgtttcag catcagagga tttgttggaa gaaatagaat ctgagctctt atctacggag     720
   tttgcagaac atcgagtacc aaatggaatg aataagggag aacatgcatt agttctgttt     780
   gaaaagtgtg tgcaagataa atatttgcag caggaacata tcataaaaaa ggccagactt     840
   ggtctctgtt atttgccatc aagaacctca attgacacgt taattccgtt tatcccaaat     900
   ttatatagat aa                                                         912

<210>  263
<211>  219
<212>  PRT
<213>  Homo sapiens

<400>  263

Met Glu Pro Lys Glu Ala Thr Gly Lys Glu Asn Met Val Thr Lys Lys
1               5                   10                  15

Lys Asn Leu Ala Phe Leu Arg Ser Arg Leu Tyr Met Leu Glu Arg Arg
            20                  25                  30

Lys Thr Asp Thr Val Val Glu Ser Ser Val Ser Gly Asp His Ser Gly
        35                  40                  45

Thr Leu Arg Arg Ser Gln Ser Asp Arg Thr Glu Tyr Asn Gln Lys Leu
    50                  55                  60

Gln Glu Lys Met Thr Pro Gln Gly Glu Cys Ser Val Ala Glu Thr Leu
65                  70                  75                  80

Thr Pro Glu Glu Glu His His Met Lys Arg Met Met Ala Lys Arg Glu
                85                  90                  95

Lys Ile Ile Lys Glu Leu Ile Gln Thr Glu Lys Asp Tyr Leu Asn Asp
            100                 105                 110

Leu Glu Leu Cys Val Arg Glu Val Val Gln Pro Leu Arg Asn Lys Lys
        115                 120                 125

Thr Asp Arg Leu Asp Val Asp Ser Leu Phe Ser Asn Ile Glu Ser Val
    130                 135                 140

His Gln Ile Ser Ala Lys Leu Leu Ser Leu Leu Glu Glu Ala Thr Thr
145                 150                 155                 160

Asp Val Glu Pro Ala Met Gln Val Ile Gly Glu Val Phe Leu Gln Ile
                165                 170                 175

Lys Gly Pro Leu Glu Asp Ile Tyr Lys Ile Tyr Cys Tyr His His Asp
            180                 185                 190

Glu Ala His Ser Ile Leu Glu Ser Tyr Glu Lys Glu Glu Glu Leu Lys
        195                 200                 205

Glu His Leu Ser His Cys Ile Gln Ser Leu Lys
    210                 215

<210>  264
<211>  3812
<212>  DNA
<213>  Homo sapiens

<400>  264
gggcctactc tgccgccgcc gccgcccgcc cgctccagcc gccgccgccg ccgccaccgc      60

cctccaggct ccgggacccg gcccgcgcca ccgcccccgt gcgcgccccg ccgccgccgc     120

cttcgccttc gccttttgtt tcctccgctc cggcgccccc gccccggctc gcgctttgca     180

ggggacgcag cgcgcgcccc cagcgggccc gggaaaagcc gcggcgcgcg cgcgcgcctg     240

cgcggcggac ccctccttct cctccccgcg tgcgcgtgcc cttcttggct gcgcgccggc     300

gccgcctggc gggcgggagg ggaggtggca ggcgcgtttg caggaggggc gcacctcttc     360

gctcgcgcac ccccccggaa ggtagaccgg gaaggggagg cgggcgggcg gagaggagag     420

agtggcgcgc agtccagcga gggcgggggt tggctatgtg gggggtggtg caccccgcag     480

tctagacagt ctgatccggg ctgggggcgt gtacactcgg cgcacctgcg agactacaga     540

gcctcgggcc ggcacgtgtg gggagtgtgg acacgtctgc tgcgccccgc ttctcgctgc     600

tgaggggaag ggagggggcg ggcaggtgca gcggccgggc tagtgggagg gggcggcggc     660

catggagcgg gtgaacgacg cttcgtgcgg cccgtctggc tgctacacat accaggtgag     720

cagacacagc acggagatgc tgcacaacct gaaccagcag cgcaaaaacg gcgggcgctt     780

ctgcgacgtg ctcttgcggg taggcgacga gagcttccca gcgcaccgcg ccgtgctggc     840

cgcctgcagc gagtactttg agtcggtgtt cagcgcccag ttgggcgacg gcggagctgc     900

ggacgggggt ccggctgatg tagggggcgc gacggcagca ccaggcggcg gggccggggg     960

cagccgggag ctggagatgc acactatcag ctccaaggta tttggggaca ttctggactt    1020

cgcctacact tcccgcatcg tggtgcgctt ggagagcttt cccgaactca tgacggccgc    1080

caagttcctg ctgatgaggt cggttatcga gatctgccag gaagtcatca aacagtccaa    1140

cgtacagatc ctggtacccc ctgcccgcgc cgatataatg ctctttcgcc cccctgggac    1200

ctcggacttg ggcttccctt tggacatgac caacggggca gccttggcag ccaacagcaa    1260

tggcatcgcc ggcagcatgc agccagagga ggaggcagct cgggcggctg gtgcagccat    1320

tgcaggccaa gcctctttgc ctgtgttacc tggggtggac cgcttgccca tggtggctgg    1380

acccctatcc ccccaactgc tgacttcccc attccccagt gtggcatcca gtgcccctcc    1440

cctgactggc aagcgaggcc ggggccgccc aaggaaggcc aacctgctgg actcaatgtt 1500
tgggtcccca gggggcctga gggaggcagg catccttcca tgcggtctat gtggtaaggt 1560
gttcactgat gccaaccggc tccggcagca cgaggcccag cacggtgtca ccagcctcca 1620
gctgggctac atcgaccttc ctcctccgag gctgggtgag aatgggctac ccatctctga 1680
agaccccgac ggcccccgaa agaggagccg gaccaggaag caggtggctt gtgagatctg 1740
cggcaagatc ttccgtgatg tgtatcatct taaccggcac aagctgtccc actctgggga 1800
gaagccctac tcctgccctg tgtgtgggtt gcggttcaag agaaaagacc gcatgtccta 1860
ccatgtgcgg tcccatgatg ggtccgtggg caagccttac atctgccaga gctgtgggaa 1920
aggcttctcc aggcctgatc acttgaacgg acatatcaag caggtgcaca cttctgagcg 1980
gcctcacaag tgtcagacct gcaatgcttc ttttgccacc cgagaccgtc tgcgctccca 2040
cctggcctgt catgaagaca aggtgccctg ccaggtgtgt gggaagtact tgcgggcagc 2100
atacatggca gaccacctga agaagcacag cgaggggccc agcaacttct gcagtatctg 2160
taaccgaggt ttctcctctg cctcctactt aaaggtccat gttaaaaccc accacggtgt 2220
tccccttccc caggtctcca ggcaccagga gcccatcctg aatgggggag cagcgttcca 2280
ctgcgccagg acctatggca acaaagaagg ccagaaatgc tcacatcagg atccgattga 2340
gagctctgac tcctatggtg acctctcaga tgccagcgac ctgaagacgc cagagaagca 2400
gagtgccaat ggctctttct cctgcgacat ggcagtcccc aaaaacaaaa tggagtctga 2460
tggggagaag aagtacccat gccctgaatg tgggagcttc ttccgctcta agtcctactt 2520
gaacaaacac atccagaagg tgcatgtccg ggctctcggg ggccccctgg gggacctggg 2580
ccctgccctt ggctcacctt tctctcctca gcagaacatg tctctcctcg agtcctttgg 2640
gtttcagatt gttcagtcgg catttgcgtc atctttagta gatcctgagg ttgaccagca 2700
gcccatgggg cctgaaggga aatgaggcag ctgctgtgtc cccacggaaa caaccatctg 2760
gggactgctg ggaaatgctg tgaatgcgga gggaagtgat gtttgggttc tgtagctgag 2820
agatttttat tcatttttaa ctgcccccca accccactcc aactccttct ccaccaccca 2880
ttctcccaat ggtctttaga aatagatttt catctgatat tctgcagaaa tatcaatgag 2940
acttggtatg ggacaggggc agaaaacact acataggcct ccaaggcaaa accagtccca 3000
gtttctttaa tgggaagaag ctggaattcc tggtgctcaa ttcttagtga ccccaatcct 3060
atacccaaat ctatgatatt ctgggacctc agtgattttg gtcccctccc acttctctag 3120
ttcgtcatcc tcccttccca tatccttcaa aagaaccaca ctagggtctc cacctactta 3180
tacaatgcgg atgcccaact gtttttaagg aagccagaag catcccatgg accatggggt 3240
gagtgtcctc caagagcccc ctgagctcag ccctctgcct ggagggctcc agacctttct 3300
gagccctgct tggaggcgag cattttcact gctaggacaa gctcagctgt tgaggacacc 3360
cccaccccaa atttcagttc ttacgtgatt ttaaccattc aacatgctgt tgggttttaa 3420
ttctctaatt attattatta ttgttattat tttttaggac cagttgtagt gaattgctac 3480

tgaaagctat cccaggtgat acagagctct ttgtaaaccg cagtcacaca ttagggttag 3540

tattaaactt tgtttagatg taccataatt aacttggcta gttgattgtt tgaagtctat 3600

ggaagaaata gttttatgca aaattttaaa aaatgccagt ctggtcaggg aagtaggggg 3660

tttcaatgct gttgggaacc aggaaggtgg gacagccggc aggtagggac attgtgtacc 3720

tcagttgtgt cacatgtgag caagcccagg ttgaccttgt gatgtgaatt gatctgatca 3780

gactgtatta aaaatgttag tacattactc ta 3812

<210> 265
<211> 641
<212> PRT
<213> Homo sapiens

<400> 265

Met Glu Arg Val Asn Asp Ala Ser Cys Gly Pro Ser Gly Cys Tyr Thr
1               5                   10                  15

Tyr Gln Val Ser Arg His Ser Thr Glu Met Leu His Asn Leu Asn Gln
            20                  25                  30

Gln Arg Lys Asn Gly Gly Arg Phe Cys Asp Val Leu Leu Arg Val Gly
        35                  40                  45

Asp Glu Ser Phe Pro Ala His Arg Ala Val Leu Ala Ala Cys Ser Glu
    50                  55                  60

Tyr Phe Glu Ser Val Phe Ser Ala Gln Leu Gly Asp Gly Gly Ala Ala
65                  70                  75                  80

Asp Gly Gly Pro Ala Asp Val Gly Gly Ala Thr Ala Ala Pro Gly Gly
                85                  90                  95

Gly Ala Gly Gly Ser Arg Glu Leu Glu Met His Thr Ile Ser Ser Lys
            100                 105                 110

Val Phe Gly Asp Ile Leu Asp Phe Ala Tyr Thr Ser Arg Ile Val Val
        115                 120                 125

Arg Leu Glu Ser Phe Pro Glu Leu Met Thr Ala Ala Lys Phe Leu Leu
    130                 135                 140

Met Arg Ser Val Ile Glu Ile Cys Gln Glu Val Ile Lys Gln Ser Asn
145                 150                 155                 160

Val Gln Ile Leu Val Pro Pro Ala Arg Ala Asp Ile Met Leu Phe Arg
                165                 170                 175

Pro Pro Gly Thr Ser Asp Leu Gly Phe Pro Leu Asp Met Thr Asn Gly
            180                 185                 190

Ala Ala Leu Ala Ala Asn Ser Asn Gly Ile Ala Gly Ser Met Gln Pro
        195                 200                 205

Glu Glu Glu Ala Ala Arg Ala Ala Gly Ala Ala Ile Ala Gly Gln Ala
    210                 215                 220

Ser Leu Pro Val Leu Pro Gly Val Asp Arg Leu Pro Met Val Ala Gly
225                 230                 235                 240

Pro Leu Ser Pro Gln Leu Leu Thr Ser Pro Phe Pro Ser Val Ala Ser
                245                 250                 255

Ser Ala Pro Pro Leu Thr Gly Lys Arg Gly Arg Gly Arg Pro Arg Lys
            260                 265                 270

Ala Asn Leu Leu Asp Ser Met Phe Gly Ser Pro Gly Gly Leu Arg Glu
        275                 280                 285

Ala Gly Ile Leu Pro Cys Gly Leu Cys Gly Lys Val Phe Thr Asp Ala
    290                 295                 300

Asn Arg Leu Arg Gln His Glu Ala Gln His Gly Val Thr Ser Leu Gln
305                 310                 315                 320

Leu Gly Tyr Ile Asp Leu Pro Pro Pro Arg Leu Gly Glu Asn Gly Leu
                325                 330                 335

Pro Ile Ser Glu Asp Pro Asp Gly Pro Arg Lys Arg Ser Arg Thr Arg
            340                 345                 350

Lys Gln Val Ala Cys Glu Ile Cys Gly Lys Ile Phe Arg Asp Val Tyr
        355                 360                 365

His Leu Asn Arg His Lys Leu Ser His Ser Gly Glu Lys Pro Tyr Ser
    370                 375                 380

Cys Pro Val Cys Gly Leu Arg Phe Lys Arg Lys Asp Arg Met Ser Tyr
385                 390                 395                 400

His Val Arg Ser His Asp Gly Ser Val Gly Lys Pro Tyr Ile Cys Gln
                405                 410                 415

Ser Cys Gly Lys Gly Phe Ser Arg Pro Asp His Leu Asn Gly His Ile
            420                 425                 430

Lys Gln Val His Thr Ser Glu Arg Pro His Lys Cys Gln Thr Cys Asn
        435                 440                 445

Ala Ser Phe Ala Thr Arg Asp Arg Leu Arg Ser His Leu Ala Cys His
    450                 455                 460

Glu Asp Lys Val Pro Cys Gln Val Cys Gly Lys Tyr Leu Arg Ala Ala
465                 470                 475                 480

Tyr Met Ala Asp His Leu Lys Lys His Ser Glu Gly Pro Ser Asn Phe
                485                 490                 495

Cys Ser Ile Cys Asn Arg Glu Gly Gln Lys Cys Ser His Gln Asp Pro
            500                 505                 510

Ile Glu Ser Ser Asp Ser Tyr Gly Asp Leu Ser Asp Ala Ser Asp Leu
        515                 520                 525

Lys Thr Pro Glu Lys Gln Ser Ala Asn Gly Ser Phe Ser Cys Asp Met
    530                 535                 540

Ala Val Pro Lys Asn Lys Met Glu Ser Asp Gly Glu Lys Lys Tyr Pro
545                 550                 555                 560

Cys Pro Glu Cys Gly Ser Phe Phe Arg Ser Lys Ser Tyr Leu Asn Lys
                565                 570                 575

His Ile Gln Lys Val His Val Arg Ala Leu Gly Gly Pro Leu Gly Asp
            580                 585                 590

Leu Gly Pro Ala Leu Gly Ser Pro Phe Ser Pro Gln Gln Asn Met Ser
        595                 600                 605

Leu Leu Glu Ser Phe Gly Phe Gln Ile Val Gln Ser Ala Phe Ala Ser
    610                 615                 620

Ser Leu Val Asp Pro Glu Val Asp Gln Gln Pro Met Gly Pro Glu Gly
625                 630                 635                 640

Lys

<210>  266
<211>  2818
<212>  DNA
<213>  Homo sapiens

<400>  266
ccctactccg cctctcggga tcctttaaga ggcggggctt ggctgccagc tccgcggccc      60
gggcaaaagg ctgggacttt actccgggtg gcggcgagga cgagtctgtg ctccatcagc     120
tgccgcaccc gccgcctccc gcccccaaac cccatccccg cggttgagcc acgatgagcg     180
gcagagtcgg cgatctgagc cccaggcaga aggaggcatt ggccaagttt cgggagaatg     240
tccaggatgt gctgccggcc ctgccgaatc cagatgacta ttttctcctg cgttggctcc     300
gagccagaag cttcgacctg cagaagtcgg aggccatgct ccggaagcat gtggagttcc     360
gaaagcaaaa ggacattgac aacatcatta gctggcagcc tccagaggtg atccaacagt     420
atctgtcagg gggtatgtgt ggctatgacc tggatggctg cccagtctgg tacgacataa     480
ttggacctct ggatgccaag ggtctgctgt tctcagcctc caaacaggac ctgctgagga     540
ccaagatgcg ggagtgtgag ctgcttctgc aagagtgtgc ccaccagacc acaaagttgg     600
ggaggaaggt ggagaccatc accataattt atgactgcga ggggcttggc ctcaagcatc     660
tctggaagcc tgctgtggag gcctatggag agtttctctg catgtttgag gaaaattatc     720
ccgaaacact gaagcgtctt tttgttgtta aagcccccaa actgtttcct gtggcctata     780
acctcatcaa acccttcctg agtgaggaca ctcgtaagaa gatcatggtc ctgggagcaa     840
attggaagga ggttttactg aaacatatca gccctgacca ggtgcctgtg gagtatgggg     900
gcaccatgac tgaccctgat ggaaacccca agtgcaaatc caagatcaac tacgggggtg     960
acatccccag gaagtattat gtgcgagacc aggtgaaaca gcagtatgaa cacagcgtgc    1020
agatttcccg tggctcctcc caccaagtgg agtatgagat cctcttccct ggctgtgtcc    1080
tcaggtggca gtttatgtca gatggagcgg atgttggttt tgggattttc ctgaagacca    1140
agatgggaga gaggcagcgg gcaggggaga tgacagaggt gctgcccaac cagaggtaca    1200
actcccacct ggtccctgaa gatgggaccc tcacctgcag tgatcctggc atctatgtcc    1260
tgcggtttga caacacctac agcttcattc atgccaagaa ggtcaatttc actgtggagg    1320
tcctgcttcc agacaaagcc tcagaagaga agatgaaaca gctgggggca ggcaccccga    1380
aataacacct tctcctatag caggcctggc cccctcagtg tctccctgtc aatttctacc    1440
ccttgtagca gtcattttcg cacaaccctg aagcccaaag aaactgggct ggaggacaga    1500
cctcaggagc tttcatttca gttaggcaga ggaagagcga ctgcagtggg tctccgtgtc    1560
tatcaaatac ctaaggagtc cccaggagct ggctggccat cgtgatagga tctgtctgtc    1620
ctgtaaactg tgccaacttc acctgtccag ggacagcgaa gctgggggtg gcggggggca     1680

tgtaccacag ggtggcagca gggaaaaaaa ttagaaaagg gtgaaagatt gggacttaac 1740
acttcaggga agtcagctgc cggggagaaa cttgctccta aatgaacaca taagtttaga 1800
tcgcaatgag gagtagcagg gtagctggtt gctagagtta cggtggggat cagaaactct 1860
tccaaacatt ttagcactga ggctggggta gcttttggct tttcccaggt ctcaggaggt 1920
ggcctgagtc agcacacatc ttcccactcg gtagacaggc tggcctctcc ctcactttga 1980
gactttggca actcctgggc cacacggcct gcctctttga ttactaatga ttgtcagtga 2040
ctcagagctt cctgggactt cgggtaccca cccgctgttc tccatgcaaa caaagcgcca 2100
gggaaatgac ccacagggat cgcagctgca gggagggcca gggaggttgg gggtgggagt 2160
gaatgctaaa agcagatcgt ccagtgccct tttcagtgct accggcctct caccaagcag 2220
tcctccatgt gagcaacccc gagacaaaaa tgctaagtgg gatcaagaga gcagcactcg 2280
gagagggtgt ttgccagtct gagtgtcccg cggtgcccgc caacccgctt cctgactgac 2340
ctgagcaagg tcttactaag cagtcccatc tctgtgggag gcatgcaacg cgtgcaggga 2400
gttcaggtgc cggtcggcgt agccaggcct ggaggccccc caggcaggag gccgcccaaa 2460
ggcggggccg gcgtctcgca gactaggggc tgggggcggc cacagacggc ctcgaaacca 2520
cagcccttac cccaatccca cgagccccgc caacgaacca caggtgctgg gctttagaga 2580
acatgggaag gcggccccag acctggcggg aacgcctttc cctcagagcc aggccccggc 2640
cccgtctggg aagctcatct tgcgaagctg agggagctca gggcaaaggc caggctagcg 2700
cggaccggaa ggggccgagg ctgcacgggc ctctgccaga acgctcagga catcccggcc 2760
tgggtttaca acgctgttag gaaaattaac caatgaataa agcaacgttc agtgcgca 2818

<210> 267
<211> 403
<212> PRT
<213> Homo sapiens

<400> 267

Met Ser Gly Arg Val Gly Asp Leu Ser Pro Arg Gln Lys Glu Ala Leu
1               5                   10                  15

Ala Lys Phe Arg Glu Asn Val Gln Asp Val Leu Pro Ala Leu Pro Asn
            20                  25                  30

Pro Asp Asp Tyr Phe Leu Leu Arg Trp Leu Arg Ala Arg Ser Phe Asp
        35                  40                  45

Leu Gln Lys Ser Glu Ala Met Leu Arg Lys His Val Glu Phe Arg Lys
    50                  55                  60

Gln Lys Asp Ile Asp Asn Ile Ile Ser Trp Gln Pro Pro Glu Val Ile
65                  70                  75                  80

Gln Gln Tyr Leu Ser Gly Gly Met Cys Gly Tyr Asp Leu Asp Gly Cys
                85                  90                  95

Pro Val Trp Tyr Asp Ile Ile Gly Pro Leu Asp Ala Lys Gly Leu Leu
            100                 105                 110

Phe Ser Ala Ser Lys Gln Asp Leu Leu Arg Thr Lys Met Arg Glu Cys
        115                 120                 125

Glu Leu Leu Leu Gln Glu Cys Ala His Gln Thr Thr Lys Leu Gly Arg
    130                 135                 140

Lys Val Glu Thr Ile Thr Ile Ile Tyr Asp Cys Glu Gly Leu Gly Leu
145                 150                 155                 160

Lys His Leu Trp Lys Pro Ala Val Glu Ala Tyr Gly Glu Phe Leu Cys
                165                 170                 175

Met Phe Glu Glu Asn Tyr Pro Glu Thr Leu Lys Arg Leu Phe Val Val
            180                 185                 190

Lys Ala Pro Lys Leu Phe Pro Val Ala Tyr Asn Leu Ile Lys Pro Phe
        195                 200                 205

Leu Ser Glu Asp Thr Arg Lys Lys Ile Met Val Leu Gly Ala Asn Trp
    210                 215                 220

Lys Glu Val Leu Leu Lys His Ile Ser Pro Asp Gln Val Pro Val Glu
225                 230                 235                 240

Tyr Gly Gly Thr Met Thr Asp Pro Asp Gly Asn Pro Lys Cys Lys Ser
                245                 250                 255

Lys Ile Asn Tyr Gly Gly Asp Ile Pro Arg Lys Tyr Tyr Val Arg Asp
            260                 265                 270

Gln Val Lys Gln Gln Tyr Glu His Ser Val Gln Ile Ser Arg Gly Ser
        275                 280                 285

Ser His Gln Val Glu Tyr Glu Ile Leu Phe Pro Gly Cys Val Leu Arg
    290                 295                 300

Trp Gln Phe Met Ser Asp Gly Ala Asp Val Gly Phe Gly Ile Phe Leu
305                 310                 315                 320

Lys Thr Lys Met Gly Glu Arg Gln Arg Ala Gly Glu Met Thr Glu Val
                325                 330                 335

Leu Pro Asn Gln Arg Tyr Asn Ser His Leu Val Pro Glu Asp Gly Thr
            340                 345                 350

Leu Thr Cys Ser Asp Pro Gly Ile Tyr Val Leu Arg Phe Asp Asn Thr
        355                 360                 365

Tyr Ser Phe Ile His Ala Lys Lys Val Asn Phe Thr Val Glu Val Leu
    370                 375                 380

Leu Pro Asp Lys Ala Ser Glu Glu Lys Met Lys Gln Leu Gly Ala Gly
385                 390                 395                 400

Thr Pro Lys

<210>  268
<211>  1464
<212>  DNA
<213>  Homo sapiens

<400>  268
gtggggacag ccgagccgcg ccgggcccct ggacggcgtc gccaaggagc tgggatcgca      60

cttgctgcag actttggatg gatttgtttt tgtggtagca tctgatggca aaatcatgta     120

tatatccgag accgcttctg tccatttagg cttatcccag gtggagctca cgggcaacag     180

tatttatgaa tacatccatc cttctgacca cgatgagatg accgctgtcc tcacggccca 240
ccagccgctg caccaccacc tgctccaagg tatgagatag agaggtcgtt ctttcttcga 300
atgaaatgtg tcttggcgaa aaggaacgcg ggcctgacct gcagcggata caaggtcatc 360
cactgcagtg gctacttgaa gatcaggcag tatatgctgg acatgtccct gtacgactcc 420
tgctaccaga ttgtggggct ggtggccgtg ggccagtcgc tgccacccag tgccatcacc 480
gagatcaagc tgtacagtaa catgttcatg ttcagggcca gccttgacct gaagctgata 540
ttcctggatt ccagggtgac cgaggtgacg gggtacgagc cgcaggacct gatcgagaag 600
accctatacc atcacgtgca cggctgcgac gtgttccacc tccgctacgc acaccacctc 660
ctgttggtga agggccaggt caccaccaag tactaccggc tgctgtccaa gcggggcggc 720
tgggtgtggg tgcagagcta cgccaccgtg gtgcacaaca gccgctcgtc ccggccccac 780
tgcatcgtga gtgtcaatta tgtactcacg gagattgaat acaaggaact tcagctgtcc 840
ctggagcagg tgtccactgc caagtcccag gactcctgga ggaccgcctt gtctacctca 900
caagaaacta ggaaattagt gaaacccaaa aataccaaga tgaagacaaa gctgagaaca 960
aacccttacc ccccacagca atacagctcg ttccaaatgg acaaactgga atgcggccag 1020
ctcggaaact ggagagccag tccccctgca agcgctgctg ctcctccaga actgcagccc 1080
cactcagaaa gcagtgacct tctgtacacg ccatcctaca gcctgccctt ctcctaccat 1140
tacggacact tccctctgga ctctcacgtc ttcagcagca aaaagccaat gttgccggcc 1200
aagttcgggc agccccaagg atccccttgt gaggtggcac gctttttcct gagcacactg 1260
ccagccagcg gtgaatgcca gtggcattat gccaaccccc tagtgcctag cagctcgtct 1320
ccagctaaaa atcctccaga gccaccggcg aacactgcta ggcacagcct ggtgccaagc 1380
tacgaaggca agcagatgtc ctctgcggag ataccgccag ctccccagga cgcagactga 1440
ctcctgtttg ctcgctggac caac 1464

<210> 269
<211> 667
<212> PRT
<213> Homo sapiens

<400> 269

Met Lys Glu Lys Ser Lys Asn Ala Ala Lys Thr Arg Arg Glu Lys Glu
1               5                   10                  15

Asn Gly Glu Phe Tyr Glu Leu Ala Lys Leu Leu Pro Leu Pro Ser Ala
            20                  25                  30

Ile Thr Ser Gln Leu Asp Lys Ala Ser Ile Ile Arg Leu Thr Thr Ser
        35                  40                  45

Tyr Leu Lys Met Arg Ala Val Phe Pro Glu Gly Leu Gly Asp Ala Trp
    50                  55                  60

Gly Gln Pro Ser Arg Ala Gly Pro Leu Asp Gly Val Ala Lys Glu Leu
65                  70                  75                  80

Gly Ser His Leu Leu Gln Thr Leu Asp Gly Phe Val Phe Val Val Ala

                85                  90                  95

Ser Asp Gly Lys Ile Met Tyr Ile Ser Glu Thr Ala Ser Val His Leu
            100                 105                 110

Gly Leu Ser Gln Val Glu Leu Thr Gly Asn Ser Ile Tyr Glu Tyr Ile
        115                 120                 125

His Pro Ser Asp His Asp Glu Met Thr Ala Val Leu Thr Ala His Gln
    130                 135                 140

Pro Leu His His His Leu Leu Gln Glu Tyr Glu Ile Glu Arg Ser Phe
145                 150                 155                 160

Phe Leu Arg Met Lys Cys Val Leu Ala Lys Arg Asn Ala Gly Leu Thr
                165                 170                 175

Cys Ser Gly Tyr Lys Val Ile His Cys Ser Gly Tyr Leu Lys Ile Arg
            180                 185                 190

Gln Tyr Met Leu Asp Met Ser Leu Tyr Asp Ser Cys Tyr Gln Ile Val
        195                 200                 205

Gly Leu Val Ala Val Gly Gln Ser Leu Pro Pro Ser Ala Ile Thr Glu
    210                 215                 220

Ile Lys Leu Tyr Ser Asn Met Phe Met Phe Arg Ala Ser Leu Asp Leu
225                 230                 235                 240

Lys Leu Ile Phe Leu Asp Ser Arg Val Thr Glu Val Thr Gly Tyr Glu
                245                 250                 255

Pro Gln Asp Leu Ile Glu Lys Thr Leu Tyr His His Val His Gly Cys
            260                 265                 270

Asp Val Phe His Leu Arg Tyr Ala His His Leu Leu Leu Val Lys Gly
        275                 280                 285

Gln Val Thr Thr Lys Tyr Tyr Arg Leu Leu Ser Lys Arg Gly Gly Trp
    290                 295                 300

Val Trp Val Gln Ser Tyr Ala Thr Val Val His Asn Ser Arg Ser Ser
305                 310                 315                 320

Arg Pro His Cys Ile Val Ser Val Asn Tyr Val Leu Thr Glu Ile Glu
                325                 330                 335

Tyr Lys Glu Leu Gln Leu Ser Leu Glu Gln Val Ser Thr Ala Lys Ser
            340                 345                 350

Gln Asp Ser Trp Arg Thr Ala Leu Ser Thr Ser Gln Glu Thr Arg Lys
        355                 360                 365

Leu Val Lys Pro Lys Asn Thr Lys Met Lys Thr Lys Leu Arg Thr Asn
    370                 375                 380

Pro Tyr Pro Pro Gln Gln Tyr Ser Ser Phe Gln Met Asp Lys Leu Glu
385                 390                 395                 400

Cys Gly Gln Leu Gly Asn Trp Arg Ala Ser Pro Pro Ala Ser Ala Ala
                405                 410                 415

Ala Pro Pro Glu Leu Gln Pro His Ser Glu Ser Ser Asp Leu Leu Tyr
            420                 425                 430

Thr Pro Ser Tyr Ser Leu Pro Phe Ser Tyr His Tyr Gly His Phe Pro
        435                 440                 445

Leu Asp Ser His Val Phe Ser Ser Lys Lys Pro Met Leu Pro Ala Lys
    450                 455                 460

Phe Gly Gln Pro Gln Gly Ser Pro Cys Glu Val Ala Arg Phe Phe Leu
465                 470                 475                 480

Ser Thr Leu Pro Ala Ser Gly Glu Cys Gln Trp His Tyr Ala Asn Pro
                485                 490                 495

Leu Val Pro Ser Ser Ser Ser Pro Ala Lys Asn Pro Pro Glu Pro Pro
            500                 505                 510

Ala Asn Thr Ala Arg His Ser Leu Val Pro Ser Tyr Glu Ala Pro Ala
        515                 520                 525

Ala Ala Val Arg Arg Phe Gly Glu Asp Thr Ala Pro Pro Ser Phe Pro
    530                 535                 540

Ser Cys Gly His Tyr Arg Glu Glu Pro Ala Leu Gly Pro Ala Lys Ala
545                 550                 555                 560

Ala Arg Gln Ala Ala Arg Asp Gly Ala Arg Leu Ala Leu Ala Arg Ala
                565                 570                 575

Ala Pro Glu Cys Cys Ala Pro Pro Thr Pro Glu Ala Pro Gly Ala Pro
            580                 585                 590

Ala Gln Leu Pro Phe Val Leu Leu Asn Tyr His Arg Val Leu Ala Arg
        595                 600                 605

Arg Gly Pro Leu Gly Gly Ala Ala Pro Ala Ala Ser Gly Leu Ala Cys
    610                 615                 620

Ala Pro Gly Gly Pro Glu Ala Ala Thr Gly Ala Leu Arg Leu Arg His
625                 630                 635                 640

Pro Ser Pro Ala Ala Thr Ser Pro Pro Gly Ala Pro Leu Pro His Tyr
                645                 650                 655

Leu Gly Ala Ser Val Ile Ile Thr Asn Gly Arg
            660                 665

<210>  270
<211>  1192
<212>  DNA
<213>  Homo sapiens

<220>
<221>  misc_feature
<222>  (1122)..(1122)
<223>  n is a, c, g, or t

<400>  270
ggcacgagga gaagatgtgg cttgctcatg cttgacttct gccatggttg tgaggcctcc      60

ccagccatgt ggaactgttt tcaggtgctg gttccatggc tcttcctgag ccgaaaataa     120

ggaaactcca tagaccttgt ccactggaac tcgttcccat ctaccctcca ctctatccag     180

ggtgatggat ctctgcagta agtggaagag ttcttcatgg cccccaaggt tatatccatc     240

tagaacttca gcacgtaatt tcatctggaa atagtgcctt tgtggatata agttaggtaa     300

aactgaagat gagatcatac tggattagga tgggatctaa atccaatgaa aatgtcttca     360

taaaaaacag gaaagaaccc atagaaacac aaggaagaag gtcatgtgaa gatggaggca     420

gagattggag ggatgcagcc accggcccag gaatgccagc agccacccag aagctggaag 480
gaaatgaggg attctctcct agaaccttta gagagracat ggtcctgtga acagcttgat 540
tttggacttg cccatagctt gtatactctt actttggata caattttatc caaacttggc 600
taaacagttt ctcagcctat ggaaaattta aaatggagaa gattcaactc gattcttaca 660
gattcaaagc aagaaaatga tgggaacata ggaggagacc aagaaagcct ataaaaagca 720
aaaatatgaa gtgaacattg tggtagcttt aagatgttta gtgtagctgc aggcacccta 780
tacacatgaa aacccccaag gggaatcccc atatcacagt gtagtgtgat atttgacatt 840
ygtgatcaty tagagatgta cagaaaaggt gaatctgtgt tctgtatatt ctgcctaagg 900
caaagaaatg tttagctytc tttaaaatag ttccataatt ttttytaaaa agctttgctt 960
gaaaactgta agcttcccat atctggagca tttcacttta aatatttgga taaatatgtt 1020
atcttcttac ttggacattt catgtgttta gggattgtyt tytaaattct tcctaattca 1080
tatagctgct aacacttccc gcagagctaa accattacag antatgaaat aaagacccta 1140
ttgatttgaa cttaaaaaaa aaaamamaaa aaaaaaaaaa aaaaaaaaat ga 1192

<210> 271
<211> 1365
<212> DNA
<213> Homo sapiens

<400> 271
atggaatcaa tctctatgat gggaagccct aagagcctta gtgaaacttg tttacctaat 60
ggcataaatg gtatcaaaga tgcaaggaag gtcactgtag gtgtgattgg aagtggagat 120
tttgccaaat ccttgaccat tcgacttatt agatgcggct atcatgtggt cataggaagt 180
agaaatccta agtttgcttc tgaatttttt cctcatgtgg tagatgtcac tcatcatgaa 240
gatgctctca caaaaacaaa tataatattt gttgctatac acagagaaca ttatacctcc 300
ctgtgggacc tgagacatct gcttgtgggt aaaatcctga ttgatgtgag caataacatg 360
aggataaacc agtacccaga atccaatgct gaatatttgg cttcattatt cccagattct 420
ttgattgtca aaggatttaa tgttgtctca gcttgggcac ttcagttagg acctaaggat 480
gccagccggc aggtttatat atgcagcaac aatattcaag cgcgacaaca ggttattgaa 540
cttgcccgcc agttgaattt cattcccatt gacttgggat ccttatcatc agccagagag 600
attgaaaatt tacccctacg actctttact ctctggagag ggccagtggt ggtagctata 660
agcttggcca catttttttt cctttattcc tttgtcagag atgtgattca tccatatgct 720
agaaaccaac agagtgactt ttacaaaatt cctatagaga ttgtgaataa aaccttacct 780
atagttgcca ttactttgct ctccctagta tacctcgcag gtcttctggc agctgcttat 840
caactttatt acggcaccaa gtataggaga tttccacctt ggttggaaac ctggttacag 900
tgtagaaaac agcttggatt actaagtttt ttcttcgcta tggtccatgt tgcctacagc 960
ctctgcttac cgatgagaag gtcagagaga tatttgtttc tcaacatggc ttatcagcag 1020
gttcatgcaa atattgaaaa ctcttggaat gaggaagaag tttggagaat tgaaatgtat 1080

atctcctttg gcataatgag ccttggctta ctttccctcc tggcagtcac ttctatccct    1140

tcagtgagca atgctttaaa ctggagagaa ttcagtttta ttcagtctac acttggatat    1200

gtcgctctgc tcataagtac tttccatgtt ttaatttatg gatggaaacg agcttttgag    1260

gaagagtact acagatttta tacaccacca aactttgttc ttgctcttgt tttgccctca    1320

attgtaattc tggatctttt gcagctttgc agatacccag actga                    1365

<210>  272
<211>  454
<212>  PRT
<213>  Homo sapiens

<400>  272
   Met Glu Ser Ile Ser Met Met Gly Ser Pro Lys Ser Leu Ser Glu Thr
   1               5                   10                  15
   Cys Leu Pro Asn Gly Ile Asn Gly Ile Lys Asp Ala Arg Lys Val Thr
               20                  25                  30
   Val Gly Val Ile Gly Ser Gly Asp Phe Ala Lys Ser Leu Thr Ile Arg
           35                  40                  45
   Leu Ile Arg Cys Gly Tyr His Val Val Ile Gly Ser Arg Asn Pro Lys
       50                  55                  60
   Phe Ala Ser Glu Phe Phe Pro His Val Val Asp Val Thr His His Glu
   65                  70                  75                  80
   Asp Ala Leu Thr Lys Thr Asn Ile Ile Phe Val Ala Ile His Arg Glu
                   85                  90                  95
   His Tyr Thr Ser Leu Trp Asp Leu Arg His Leu Leu Val Gly Lys Ile
               100                 105                 110
   Leu Ile Asp Val Ser Asn Asn Met Arg Ile Asn Gln Tyr Pro Glu Ser
           115                 120                 125
   Asn Ala Glu Tyr Leu Ala Ser Leu Phe Pro Asp Ser Leu Ile Val Lys
       130                 135                 140
   Gly Phe Asn Val Val Ser Ala Trp Ala Leu Gln Leu Gly Pro Lys Asp
   145                 150                 155                 160
   Ala Ser Arg Gln Val Tyr Ile Cys Ser Asn Asn Ile Gln Ala Arg Gln
                   165                 170                 175
   Gln Val Ile Glu Leu Ala Arg Gln Leu Asn Phe Ile Pro Ile Asp Leu
               180                 185                 190
   Gly Ser Leu Ser Ser Ala Arg Glu Ile Glu Asn Leu Pro Leu Arg Leu
           195                 200                 205
   Phe Thr Leu Trp Arg Gly Pro Val Val Val Ala Ile Ser Leu Ala Thr
       210                 215                 220
   Phe Phe Phe Leu Tyr Ser Phe Val Arg Asp Val Ile His Pro Tyr Ala
   225                 230                 235                 240
   Arg Asn Gln Gln Ser Asp Phe Tyr Lys Ile Pro Ile Glu Ile Val Asn
                   245                 250                 255
   Lys Thr Leu Pro Ile Val Ala Ile Thr Leu Leu Ser Leu Val Tyr Leu
               260                 265                 270
   Ala Gly Leu Leu Ala Ala Ala Tyr Gln Leu Tyr Tyr Gly Thr Lys Tyr
           275                 280                 285
   Arg Arg Phe Pro Pro Trp Leu Glu Thr Trp Leu Gln Cys Arg Lys Gln
       290                 295                 300
   Leu Gly Leu Leu Ser Phe Phe Phe Ala Met Val His Val Ala Tyr Ser
   305                 310                 315                 320
   Leu Cys Leu Pro Met Arg Arg Ser Glu Arg Tyr Leu Phe Leu Asn Met
                   325                 330                 335
   Ala Tyr Gln Gln Val His Ala Asn Ile Glu Asn Ser Trp Asn Glu Glu
               340                 345                 350
   Glu Val Trp Arg Ile Glu Met Tyr Ile Ser Phe Gly Ile Met Ser Leu
           355                 360                 365
   Gly Leu Leu Ser Leu Leu Ala Val Thr Ser Ile Pro Ser Val Ser Asn
       370                 375                 380
   Ala Leu Asn Trp Arg Glu Phe Ser Phe Ile Gln Ser Thr Leu Gly Tyr
   385                 390                 395                 400
   Val Ala Leu Leu Ile Ser Thr Phe His Val Leu Ile Tyr Gly Trp Lys
                   405                 410                 415
   Arg Ala Phe Glu Glu Glu Tyr Tyr Arg Phe Tyr Thr Pro Pro Asn Phe
               420                 425                 430
   Val Leu Ala Leu Val Leu Pro Ser Ile Val Ile Leu Asp Leu Leu Gln
           435                 440                 445
   Leu Cys Arg Tyr Pro Asp
       450

<210> 273
<211> 1933
<212> DNA
<213> Homo sapiens

<400> 273
ccgccgcctt ctactccgcc gcgggggtcg cagcggctgc cgcgccgtcc tcgagtttcc 60

agcgtgagga ggaggctgag ggcggagagg cgcatcgtgt tcgaggcgga gaccgagggg 120

gagccccgcg cgcggcgtcg ctcattgcta tggacagtgc tatcaccctg tggcagttcc 180

ttcttcagct cctgcagaag cctcagaaca agcacatgat ctgttggacc tctaatgatg 240

ggcagtttaa gcttttgcag gcagaagagg tggctcgtct ctgggggatt cgcaagaaca 300

agcctaacat gaattatgac aaactcagcc gagccctcag atactattat gtaaagaata 360

tcatcaaaaa agtgaatggt cagaagtttg tgtacaagtt tgtctcttat ccagagattt 420

tgaacatgga tccaatgaca gtgggcagga ttgagggtga ctgtgaaagt ttaaacttca 480

gtgaagtcag cagcagttcc aaagatgtgg agaatggagg gaaagataaa ccacctcagc 540

ctggtgccaa gacctctagc cgcaatgact acatacactc tggcttatat tcttcattta 600

ctctcaactc tttgaactcc tccaatgtaa agcttttcaa attgataaag actgagaatc 660

cagccgagaa actggcagag aaaaaatctc ctcaggagcc cacaccatct gtcatcaaat 720

ttgtcacgac accttccaaa aagccaccag ttgaacctgt tgctgccacc atttcaattg 780

gcccaagtat ttctccatct tcagaagaaa ctatccaagc tttggagaca ttggtttccc 840

caaaactgcc ttccctggaa gccccaacct ctgcctctaa cgtaatgact gcttttgcca 900

ccacaccacc catttcgtcc ataccccctt tgcaggaacc tcccagaaca ccttcaccac 960

cactgagttc tcacccagac atcgacacag acattgattc agtggcttct cagccaatgg 1020

aacttccaga gaatttgtct ctggagccta aagaccagga ttcagtcttg ctagaaaagg 1080

acaaagtaaa taattcatca agatccaaga aacccaaagg gttaggactg gcacccaccc 1140

ttgtgatcac gagcagtgat ccaagcccac tgggaatact gagcccatct ctccctacag 1200

cttctcttac accagcattt ttttcacaga cacccatcat actgactcca agccccttgc 1260

tctccagtat ccacttctgg agtactctca gtcctgttgc tcccctaagt ccagccagac 1320

tgcaaggtgc taacacactt ttccagtttc cttctgtact gaacagtcat gggccattca 1380

ctctgtctgg gctggatgga ccttccaccc ctggcccatt ttccccagac ctacagaaga 1440

cataacctat gcacttgtgg aatgagagaa ccgaggaacg aagaaacaga cattcaacat 1500

gattgcattt gaagtgagca attgatagtt ctacaatgct gataatagac tattgtgatt 1560

tttgccattc cccattgaaa acatcttttt aggattctct ttgaatagga ctcaagttgg 1620

actatatgta taaaaatgcc ttaattggag tctaaactcc acctccctct gtcttttcct 1680

tttctttttc tttccttcct tccttttctt ttctccttta aaaatatttt gagctttgtg 1740

ctgaagaagt ttttggtggg ctttagtgac tgtgctttgc aaaagcaatt aagaacaaag 1800

ttactccttc tggctattgg gaccctttgg ccaggaaaaa ttatgcttag aatctattat 1860

ttaaagaagt atttgtgaaa tgaaaaaaaa aaaaaaaaaa aaaaaaaaaa aaaaaaaaaa 1920

aaaaaaaaaa aaa 1933

<210> 274
<211> 431
<212> PRT
<213> Homo sapiens

<400> 274

Met Asp Ser Ala Ile Thr Leu Trp Gln Phe Leu Leu Gln Leu Leu Gln
1               5                   10                  15

Lys Pro Gln Asn Lys His Met Ile Cys Trp Thr Ser Asn Asp Gly Gln
            20                  25                  30

Phe Lys Leu Leu Gln Ala Glu Glu Val Ala Arg Leu Trp Gly Ile Arg
        35                  40                  45

Lys Asn Lys Pro Asn Met Asn Tyr Asp Lys Leu Ser Arg Ala Leu Arg
    50                  55                  60

Tyr Tyr Tyr Val Lys Asn Ile Ile Lys Lys Val Asn Gly Gln Lys Phe
65                  70                  75                  80

Val Tyr Lys Phe Val Ser Tyr Pro Glu Ile Leu Asn Met Asp Pro Met
                85                  90                  95

Thr Val Gly Arg Ile Glu Gly Asp Cys Glu Ser Leu Asn Phe Ser Glu
            100                 105                 110

Val Ser Ser Ser Ser Lys Asp Val Glu Asn Gly Gly Lys Asp Lys Pro
        115                 120                 125

Pro Gln Pro Gly Ala Lys Thr Ser Ser Arg Asn Asp Tyr Ile His Ser
    130                 135                 140

Gly Leu Tyr Ser Ser Phe Thr Leu Asn Ser Leu Asn Ser Ser Asn Val
145                 150                 155                 160

Lys Leu Phe Lys Leu Ile Lys Thr Glu Asn Pro Ala Glu Lys Leu Ala
                165                 170                 175

Glu Lys Lys Ser Pro Gln Glu Pro Thr Pro Ser Val Ile Lys Phe Val
            180                 185                 190

Thr Thr Pro Ser Lys Lys Pro Pro Val Glu Pro Val Ala Ala Thr Ile
        195                 200                 205

Ser Ile Gly Pro Ser Ile Ser Pro Ser Ser Glu Glu Thr Ile Gln Ala
    210                 215                 220

Leu Glu Thr Leu Val Ser Pro Lys Leu Pro Ser Leu Glu Ala Pro Thr
225                 230                 235                 240

Ser Ala Ser Asn Val Met Thr Ala Phe Ala Thr Thr Pro Pro Ile Ser
                245                 250                 255

Ser Ile Pro Pro Leu Gln Glu Pro Pro Arg Thr Pro Ser Pro Pro Leu
            260                 265                 270

Ser Ser His Pro Asp Ile Asp Thr Asp Ile Asp Ser Val Ala Ser Gln
        275                 280                 285

Pro Met Glu Leu Pro Glu Asn Leu Ser Leu Glu Pro Lys Asp Gln Asp
    290                 295                 300

Ser Val Leu Leu Glu Lys Asp Lys Val Asn Asn Ser Ser Arg Ser Lys
305                 310                 315                 320

Lys Pro Lys Gly Leu Gly Leu Ala Pro Thr Leu Val Ile Thr Ser Ser
                325                 330                 335

Asp Pro Ser Pro Leu Gly Ile Leu Ser Pro Ser Leu Pro Thr Ala Ser
            340                 345                 350

Leu Thr Pro Ala Phe Phe Ser Gln Thr Pro Ile Ile Leu Thr Pro Ser
        355                 360                 365

Pro Leu Leu Ser Ser Ile His Phe Trp Ser Thr Leu Ser Pro Val Ala
    370                 375                 380

Pro Leu Ser Pro Ala Arg Leu Gln Gly Ala Asn Thr Leu Phe Gln Phe
385                 390                 395                 400

Pro Ser Val Leu Asn Ser His Gly Pro Phe Thr Leu Ser Gly Leu Asp
                405                 410                 415

Gly Pro Ser Thr Pro Gly Pro Phe Ser Pro Asp Leu Gln Lys Thr
            420                 425                 430

<210>  275
<211>  3060
<212>  DNA
<213>  Homo sapiens

<400>  275
aatcaggaac agatcatata ttgaccgaga ttctgagtat ctcttgcaag aaaatgaacc      60

agatggaact ttagaccaaa aattattgga agatttacaa aagaaaaaaa atgaccttcg 120
gtatattgaa atgcagcatt tcagagaaaa gctgccttcg tatggaatgc aaaaggaatt 180
ggtaaattta attgataacc atcaggtaac agtaataagt ggtgaaactg gttgtggcaa 240
aaccactcaa gttactcagt tcattttgga taactacatt gaaagaggaa aaggatctgc 300
ttgcagaata gtttgtactc agccaagaag aattagtgcc atttcagttg cggaaagagt 360
agctgcagaa agggcagaat cttgtggcag tggtaatagt actggatatc aaattcgtct 420
ccagagtcgg ttgccaagga aacagggttc tatcttatac tgtacaacag gaatcatcct 480
tcagtggctc cagtcagacc cgtatttgtc cagtgttagt catatcgtac ttgatgaaat 540
ccatgaaaga aatctgcagt cagatgtttt aatgactgtt gttaaagacc ttctcaattt 600
tcgatctgac ttgaaagtaa tattgatgag tgcaacattg aatgcagaaa agttttcaga 660
atattttggt aactgtccaa tgatacatat acctggtttt acctttccgg ttgtggaata 720
tcttttggaa gatgtaattg aaaaaataag gtatgttcca gaacaaaaag aacacagatc 780
ccagtttaag aggggtttca tgcaagggca tgtaaataga caagaaaaag aagaaaaaga 840
agcaatatat aaagaacgtt ggccagatta tgtaagggaa ctgcgaagaa ggtattctgc 900
aagtactgta gatgttatag aaatgatgga ggatgataaa gttgatctga atttgattgt 960
tgccctcatc cgatacattg ttttggaaga agaggatggt gcgatactgg tctttctgcc 1020
aggctgggac aatatcagca ctttacatga tctcttgatg tcacaagtaa tgtttaaatc 1080
agataaattt ttaattatac ctttacattc actgatgcct acagttaacc agacacaggt 1140
gtttaaaaga acccctcctg gtgttcggaa aatagtaatt gctaccaaca ttgcggagac 1200
tagcattacc atagatgatg tcgtttatgt gatagatgga ggaaaaataa aagagacgca 1260
ttttgatact cagaacaata tcagtacaat gtccgctgag tgggttagta aagctaatgc 1320
caaacagaga aaaggtcgag ctggaagagt tcaacctggt cattgctatc atctgtataa 1380
tggtcttaga gcaagtcttc tagatgacta tcaactgcca gaaattttga gaactccttt 1440
ggaagaactt tgtttacaaa taaagatttt aaggctaggt ggaattgctt attttctgag 1500
tagattaatg gacccaccat caaatgaggc agtgttactc tccataagac acctgatgga 1560
gctgaacgct ttggataaac aagaagaatt gacacctctt ggagtccact tggcacgatt 1620
acccgttgag ccacatattg gaaaaatgat tctttttgga gcactgttct gctgcttaga 1680
cccagtactc actattgctg ctagtctcag tttcaaagat ccatttgtca ttccactggg 1740
aaaagaaaag attgcagatg caagaagaaa ggaattggca aaggatacta gaagtgatca 1800
cttaacagtt gtgaatgcgt ttgagggctg ggaagaggct aggcgacgtg gtttcagata 1860
cgaaaaggac tattgctggg aatattttct gtcttcaaac acactgcaga tgctgcataa 1920
catgaaagga cagtttgctg agcatcttct tggagctgga tttgtaagca gtagaaatcc 1980
taaagatcca gaatctaata taaattcaga taatgagaag ataattaaag ctgtcatctg 2040
tgctggttta tatcccaaag ttgctaaaat tcgactaaat ttgggtaaaa aaagaaaaat 2100

ggtaaaagtt tacacaaaaa ccgatggcct ggttgctgtt catcctaaat ctgttaatgt 2160
ggagcaaaca gactttcact acaactggct tatctatcac ctaaagatga gaacaagcag 2220
tatatacttg tatgactgca cagaggtttc cccatactgt ctcttgtttt ttggaggtga 2280
catttccatc cagaaggata acgatcagga aactattgct gtagatgagt ggattgtatt 2340
tcagtctcca gcaagaattg cccatcttgt taaggaatta agaaaggaac tagatattct 2400
tctgcaagag aagattgaaa gtcctcatcc tgtagactgg aatgacacta aatccagaga 2460
ctgtgcagta ctgtcagcta ttatagactt gatcaaaaca caggaaaagg caactcccag 2520
gaactttccg ccacgattcc aggatggata ttacagctga cagcttttca ggggtggtct 2580
gaaaagccag tttgacagcc attcttcatc attgtttaaa ttttggctgg atgccaaacc 2640
ctgggacatg aacaattttc atgtgtaagg tagaagcctt cagtaggtag taaagactta 2700
atgtgcatga cttgatgtta tatgtagaga tatatatata tatatatata ccataaaagc 2760
aatatgttct ctgatcatat actctgctgt ggtcatgccc actctttggg agtatattcc 2820
ctttatatat attgagtatt gtaccacttg agaaattcct ttgttctgtt atacaaaatt 2880
aatctttctg ctcataatga ttgatgatac caccagtaaa aataggatgt ttaccccaaa 2940
acaagtgtca attaagaatt tgaacacaac cacatttttt aaaatgaaac ttctatcgga 3000
agtaaattaa tttgttgtaa taaagtccag tatttaataa aatgtacaat gttaaatctc 3060

<210> 276
<211> 852
<212> PRT
<213> Homo sapiens

<400> 276

Ile Arg Asn Arg Ser Tyr Ile Asp Arg Asp Ser Glu Tyr Leu Leu Gln
1               5                   10                  15

Glu Asn Glu Pro Asp Gly Thr Leu Asp Gln Lys Leu Leu Glu Asp Leu
            20                  25                  30

Gln Lys Lys Lys Asn Asp Leu Arg Tyr Ile Glu Met Gln His Phe Arg
        35                  40                  45

Glu Lys Leu Pro Ser Tyr Gly Met Gln Lys Glu Leu Val Asn Leu Ile
    50                  55                  60

Asp Asn His Gln Val Thr Val Ile Ser Gly Glu Thr Gly Cys Gly Lys
65                  70                  75                  80

Thr Thr Gln Val Thr Gln Phe Ile Leu Asp Asn Tyr Ile Glu Arg Gly
                85                  90                  95

Lys Gly Ser Ala Cys Arg Ile Val Cys Thr Gln Pro Arg Arg Ile Ser
            100                 105                 110

Ala Ile Ser Val Ala Glu Arg Val Ala Ala Glu Arg Ala Glu Ser Cys
        115                 120                 125

Gly Ser Gly Asn Ser Thr Gly Tyr Gln Ile Arg Leu Gln Ser Arg Leu
    130                 135                 140

Pro Arg Lys Gln Gly Ser Ile Leu Tyr Cys Thr Thr Gly Ile Ile Leu

145                 150                 155                 160

Gln Trp Leu Gln Ser Asp Pro Tyr Leu Ser Ser Val Ser His Ile Val
                165                 170                 175

Leu Asp Glu Ile His Glu Arg Asn Leu Gln Ser Asp Val Leu Met Thr
            180                 185                 190

Val Val Lys Asp Leu Leu Asn Phe Arg Ser Asp Leu Lys Val Ile Leu
        195                 200                 205

Met Ser Ala Thr Leu Asn Ala Glu Lys Phe Ser Glu Tyr Phe Gly Asn
    210                 215                 220

Cys Pro Met Ile His Ile Pro Gly Phe Thr Phe Pro Val Val Glu Tyr
225                 230                 235                 240

Leu Leu Glu Asp Val Ile Glu Lys Ile Arg Tyr Val Pro Glu Gln Lys
                245                 250                 255

Glu His Arg Ser Gln Phe Lys Arg Gly Phe Met Gln Gly His Val Asn
            260                 265                 270

Arg Gln Glu Lys Glu Glu Lys Glu Ala Ile Tyr Lys Glu Arg Trp Pro
        275                 280                 285

Asp Tyr Val Arg Glu Leu Arg Arg Arg Tyr Ser Ala Ser Thr Val Asp
    290                 295                 300

Val Ile Glu Met Met Glu Asp Asp Lys Val Asp Leu Asn Leu Ile Val
305                 310                 315                 320

Ala Leu Ile Arg Tyr Ile Val Leu Glu Glu Glu Asp Gly Ala Ile Leu
                325                 330                 335

Val Phe Leu Pro Gly Trp Asp Asn Ile Ser Thr Leu His Asp Leu Leu
            340                 345                 350

Met Ser Gln Val Met Phe Lys Ser Asp Lys Phe Leu Ile Ile Pro Leu
        355                 360                 365

His Ser Leu Met Pro Thr Val Asn Gln Thr Gln Val Phe Lys Arg Thr
    370                 375                 380

Pro Pro Gly Val Arg Lys Ile Val Ile Ala Thr Asn Ile Ala Glu Thr
385                 390                 395                 400

Ser Ile Thr Ile Asp Asp Val Val Tyr Val Ile Asp Gly Gly Lys Ile
                405                 410                 415

Lys Glu Thr His Phe Asp Thr Gln Asn Asn Ile Ser Thr Met Ser Ala
            420                 425                 430

Glu Trp Val Ser Lys Ala Asn Ala Lys Gln Arg Lys Gly Arg Ala Gly
        435                 440                 445

Arg Val Gln Pro Gly His Cys Tyr His Leu Tyr Asn Gly Leu Arg Ala
    450                 455                 460

Ser Leu Leu Asp Asp Tyr Gln Leu Pro Glu Ile Leu Arg Thr Pro Leu
465                 470                 475                 480

Glu Glu Leu Cys Leu Gln Ile Lys Ile Leu Arg Leu Gly Gly Ile Ala
                485                 490                 495

Tyr Phe Leu Ser Arg Leu Met Asp Pro Pro Ser Asn Glu Ala Val Leu
            500                 505                 510

Leu Ser Ile Arg His Leu Met Glu Leu Asn Ala Leu Asp Lys Gln Glu
        515                 520                 525

Glu Leu Thr Pro Leu Gly Val His Leu Ala Arg Leu Pro Val Glu Pro
    530                 535                 540

His Ile Gly Lys Met Ile Leu Phe Gly Ala Leu Phe Cys Cys Leu Asp
545                 550                 555                 560

Pro Val Leu Thr Ile Ala Ala Ser Leu Ser Phe Lys Asp Pro Phe Val
                565                 570                 575

Ile Pro Leu Gly Lys Glu Lys Ile Ala Asp Ala Arg Arg Lys Glu Leu
            580                 585                 590

Ala Lys Asp Thr Arg Ser Asp His Leu Thr Val Val Asn Ala Phe Glu
        595                 600                 605

Gly Trp Glu Glu Ala Arg Arg Arg Gly Phe Arg Tyr Glu Lys Asp Tyr
    610                 615                 620

Cys Trp Glu Tyr Phe Leu Ser Ser Asn Thr Leu Gln Met Leu His Asn
625                 630                 635                 640

Met Lys Gly Gln Phe Ala Glu His Leu Leu Gly Ala Gly Phe Val Ser
                645                 650                 655

Ser Arg Asn Pro Lys Asp Pro Glu Ser Asn Ile Asn Ser Asp Asn Glu
            660                 665                 670

Lys Ile Ile Lys Ala Val Ile Cys Ala Gly Leu Tyr Pro Lys Val Ala
        675                 680                 685

Lys Ile Arg Leu Asn Leu Gly Lys Lys Arg Lys Met Val Lys Val Tyr
    690                 695                 700

Thr Lys Thr Asp Gly Leu Val Ala Val His Pro Lys Ser Val Asn Val
705                 710                 715                 720

Glu Gln Thr Asp Phe His Tyr Asn Trp Leu Ile Tyr His Leu Lys Met
                725                 730                 735

Arg Thr Ser Ser Ile Tyr Leu Tyr Asp Cys Thr Glu Val Ser Pro Tyr
            740                 745                 750

Cys Leu Leu Phe Phe Gly Gly Asp Ile Ser Ile Gln Lys Asp Asn Asp
        755                 760                 765

Gln Glu Thr Ile Ala Val Asp Glu Trp Ile Val Phe Gln Ser Pro Ala
    770                 775                 780

Arg Ile Ala His Leu Val Lys Glu Leu Arg Lys Glu Leu Asp Ile Leu
785                 790                 795                 800

Leu Gln Glu Lys Ile Glu Ser Pro His Pro Val Asp Trp Asn Asp Thr
                805                 810                 815

Lys Ser Arg Asp Cys Ala Val Leu Ser Ala Ile Ile Asp Leu Ile Lys
            820                 825                 830

Thr Gln Glu Lys Ala Thr Pro Arg Asn Phe Pro Pro Arg Phe Gln Asp
        835                 840                 845

Gly Tyr Tyr Ser
    850

<210>  277
<211>  1671

<212>  DNA
<213>  Homo sapiens

<400>  277
gattttatcc tggaacatta cagtgaagat ggctatttat atgaagatga aattgcagat      60

cttatggatc tgagacaagc ttgtcggacg cctagccggg atgaggccgg ggtggaactg     120

ctgatgacat acttcatcca gctgggcttt gtcgagagtc gattcttccc gcccacacgg     180

cagatgggac tcctgttcac ctggtatgac tctctcaccg gggttccggt cagccagcag     240

aacctgctgc tggagaaggc cagtgtcctg ttcaacactg gggccctcta cacccagatt     300

gggacccggt gtgatcggca gacgcaggct gggctggaga gtgccataga tgcctttcag     360

agagccgcag gggttttaaa ttacctgaaa gacacattta cccatactcc aagttacgac     420

atgagccctg ccatgctcag cgtgctcgtc aaaatgatgc ttgcacaagc ccaagaaagc     480

gtgtttgaga aaatcagcct tcctgggatc cggaatgaat tcttcatgct ggtgaaggtg     540

gctcaggagg ctgctaaggt gggagaggtc taccaacagc tacacgcagc catgagccag     600

gcgccggtga aagagaacat cccctactcc tgggccagct tagcctgcgt gaaggcccac     660

cactacgcgg ccctggccca ctacttcact gccatcctcc tcatcgacca ccaggtgaag     720

ccaggcacgg atctggacca ccaggagaag tgcctgtccc agctctacga ccacatgcca     780

gaggggctga cacccttggc cacactgaag aatgatcagc agcgccgaca gctggggaag     840

tcccacttgc gcagagccat ggctcatcac gaggagtcgg tgcgggaggc cagcctctgc     900

aagaagctgc ggagcattga ggtgctacag aaggtgctgt gtgccgcaca ggaacgctcc     960

cggctcacgt acgcccagca ccaggaggag gatgacctgc tgaacctgat cgacgccccc    1020

agtgttgttg ctaaaactga gcaagaggtt gacattatat tgccccagtt ctccaagctg    1080

acagtcacgg acttcttcca gaagctgggc cccttatctg tgttttcggc taacaagcgg    1140

tggacgcctc ctcgaagcat ccgcttcact gcagaagaag gggacttggg gttcaccttg    1200

agagggaacg cccccgttca ggttcacttc ctggatcctt actgctctgc ctcggtggca    1260

ggagcccggg aaggagatta tattgtctcc attcagcttg tggattgtaa gtggctgacg    1320

ctgagtgagg ttatgaagct gctgaagagc tttggcgagg acgagatcga gatgaaagtc    1380

gtgagcctcc tggactccac atcatccatg cataataaga gtgccacata ctccgtggga    1440

atgcagaaaa cgtactccat gatctgctta gccattgatg atgacgacaa aactgataaa    1500

accaagaaaa tctccaagaa gctttccttc ctgagttggg gcaccaacaa gaacagacag    1560

aagtcagcca gcaccttgtg cctcccatcg gtcggggctg cacggcctca ggtcaagaag    1620

aagctgccct cccctttcag ccttctcaac tcagacagtt cttggtacta a             1671

<210>  278
<211>  556
<212>  PRT
<213>  Homo sapiens

<400>  278

Asp Phe Ile Leu Glu His Tyr Ser Glu Asp Gly Tyr Leu Tyr Glu Asp

1               5                   10                  15

Glu Ile Ala Asp Leu Met Asp Leu Arg Gln Ala Cys Arg Thr Pro Ser
            20                  25                  30

Arg Asp Glu Ala Gly Val Glu Leu Leu Met Thr Tyr Phe Ile Gln Leu
        35                  40                  45

Gly Phe Val Glu Ser Arg Phe Phe Pro Pro Thr Arg Gln Met Gly Leu
    50                  55                  60

Leu Phe Thr Trp Tyr Asp Ser Leu Thr Gly Val Pro Val Ser Gln Gln
65                  70                  75                  80

Asn Leu Leu Leu Glu Lys Ala Ser Val Leu Phe Asn Thr Gly Ala Leu
                85                  90                  95

Tyr Thr Gln Ile Gly Thr Arg Cys Asp Arg Gln Thr Gln Ala Gly Leu
            100                 105                 110

Glu Ser Ala Ile Asp Ala Phe Gln Arg Ala Ala Gly Val Leu Asn Tyr
        115                 120                 125

Leu Lys Asp Thr Phe Thr His Thr Pro Ser Tyr Asp Met Ser Pro Ala
    130                 135                 140

Met Leu Ser Val Leu Val Lys Met Met Leu Ala Gln Ala Gln Glu Ser
145                 150                 155                 160

Val Phe Glu Lys Ile Ser Leu Pro Gly Ile Arg Asn Glu Phe Phe Met
                165                 170                 175

Leu Val Lys Val Ala Gln Glu Ala Ala Lys Val Gly Glu Val Tyr Gln
            180                 185                 190

Gln Leu His Ala Ala Met Ser Gln Ala Pro Val Lys Glu Asn Ile Pro
        195                 200                 205

Tyr Ser Trp Ala Ser Leu Ala Cys Val Lys Ala His His Tyr Ala Ala
    210                 215                 220

Leu Ala His Tyr Phe Thr Ala Ile Leu Leu Ile Asp His Gln Val Lys
225                 230                 235                 240

Pro Gly Thr Asp Leu Asp His Gln Glu Lys Cys Leu Ser Gln Leu Tyr
                245                 250                 255

Asp His Met Pro Glu Gly Leu Thr Pro Leu Ala Thr Leu Lys Asn Asp
            260                 265                 270

Gln Gln Arg Arg Gln Leu Gly Lys Ser His Leu Arg Arg Ala Met Ala
        275                 280                 285

His His Glu Glu Ser Val Arg Glu Ala Ser Leu Cys Lys Lys Leu Arg
    290                 295                 300

Ser Ile Glu Val Leu Gln Lys Val Leu Cys Ala Ala Gln Glu Arg Ser
305                 310                 315                 320

Arg Leu Thr Tyr Ala Gln His Gln Glu Glu Asp Asp Leu Leu Asn Leu
                325                 330                 335

Ile Asp Ala Pro Ser Val Val Ala Lys Thr Glu Gln Glu Val Asp Ile
            340                 345                 350

Ile Leu Pro Gln Phe Ser Lys Leu Thr Val Thr Asp Phe Phe Gln Lys
        355                 360                 365

Leu Gly Pro Leu Ser Val Phe Ser Ala Asn Lys Arg Trp Thr Pro Pro
    370                 375                 380

Arg Ser Ile Arg Phe Thr Ala Glu Glu Gly Asp Leu Gly Phe Thr Leu
385                 390                 395                 400

Arg Gly Asn Ala Pro Val Gln Val His Phe Leu Asp Pro Tyr Cys Ser
                405                 410                 415

Ala Ser Val Ala Gly Ala Arg Glu Gly Asp Tyr Ile Val Ser Ile Gln
            420                 425                 430

Leu Val Asp Cys Lys Trp Leu Thr Leu Ser Glu Val Met Lys Leu Leu
        435                 440                 445

Lys Ser Phe Gly Glu Asp Glu Ile Glu Met Lys Val Val Ser Leu Leu
    450                 455                 460

Asp Ser Thr Ser Ser Met His Asn Lys Ser Ala Thr Tyr Ser Val Gly
465                 470                 475                 480

Met Gln Lys Thr Tyr Ser Met Ile Cys Leu Ala Ile Asp Asp Asp Asp
                485                 490                 495

Lys Thr Asp Lys Thr Lys Lys Ile Ser Lys Lys Leu Ser Phe Leu Ser
            500                 505                 510

Trp Gly Thr Asn Lys Asn Arg Gln Lys Ser Ala Ser Thr Leu Cys Leu
        515                 520                 525

Pro Ser Val Gly Ala Ala Arg Pro Gln Val Lys Lys Lys Leu Pro Ser
    530                 535                 540

Pro Phe Ser Leu Leu Asn Ser Asp Ser Ser Trp Tyr
545                 550                 555

<210>  279
<211>  2674
<212>  DNA
<213>  Homo sapiens

<400>  279
gaattcggca cgagccttgt tggaggttct ggggcgcaga accgctactg ctgcttcggt      60

ctctccttgg gaaaaaataa aatttgaacc ttttggagct gtgtgctaaa tcttcagtgg     120

gacaatgggt tcagacaaaa gagtgagtag aacagagcgt agtggaagat acggttccat     180

catagacagg gatgaccgtg atgagcgtga atcccgaagc aggcggaggg actcagatta     240

caaaagatct agtgatgatc ggaggggtga tagatatgat gactaccgag actatgacag     300

tccagagaga gagcgtgaaa gaaggaacag tgaccgatcc gaagatggct accattcaga     360

tggtgactat ggtgagcacg actataggca tgacatcagt gacgagaggg agagcaagac     420

catcatgctg cgcggccttc ccatcaccat cacagagagc gatattcgag aaatgatgga     480

gtccttcgaa ggccctcagc ctgcggatgt gaggctgatg aagaggaaaa caggtgagag     540

cttgcttagt tcctgatatt attgttctct tccccattcc cacctcagtc cctaaagaac     600

atcctgattc ccccagtctt caagcacatg aattcagaat gaaaggtttg ccatggctaa     660

ggaatgtgac tctttgaaaa ccatgttagc atctgaggaa cttttttaaa ctttgtttta     720

gggacttttt tttccttagg taagtaatga tttataaact cctttttttt tttgactata     780

gtcggttgca tggttacttt aagcgtggaa tcaaatggag tggcatttag ttcaggcggc 840
ttgttccttg ccatggcaaa gtatcaagaa gatccccaag tcaagtcaca tttgtaaagc 900
tgcttcccaa ttggctttgt cacgcagtgt tgaagcagtg ggagagagat tcacctgtta 960
taaaggaact gactaacaca agtatcccgt ctatatctga atgctgtctc taggtgtaag 1020
ccgtggtttc gccttcgtgg agttttatca cttgcaagat gctaccagct ggatggaagc 1080
caatcaggtt gcttcactca ccaagtctag atattcatga aaatggaaca agtctgtaca 1140
attttaaaaa aaggttgaag gagtggtttg ttccaaagga gtgacttttt tttaaaaaaa 1200
aagctttgta tatattaaaa ttgatgttac tagaataagt acagtaccaa ggacttcatt 1260
atagaatttg ttctgccttt aaacatggct acctacctgg cagggctttg ttaactactg 1320
aatacctgtc tggtaatcac taaaacatct ttatgtttcc cttttttcta gtttgttata 1380
ttcctattat gtccattgag agtaagctta gtatatcaaa ctctccattt gacagtgaag 1440
agaacatagt gaaagtctgt ggcggcattt ttataagtaa ttccttattt ctgcctgaag 1500
accacaaagc ctcctggagg cgtaactgct cagaccggtc ttcagggaat atttaaggac 1560
ttagtggaat ttatgaacaa taagtctgat gagattagcc tgggagtggt gtcctgcagc 1620
tgtctaatct agagtggcat taacattcta atctccttga gaatgccttt tatagtctgt 1680
tcaaagcaag tcattgatgg ttcttcgagg tagtgttaac tgaagtgttc ttcagtttgt 1740
caagataatg ttcagtgctt ggcacttaaa taacattttt tgcaagaact ccaaggcaca 1800
ttattgaatg cctttaacca agtgcattct gggaagtttg cttgactcat tatcttgctt 1860
ttctgcagca ttctgtgatt tgagtcatcc atgaatccat gaataaaagt tacattcttt 1920
gattggtaat attgccattt ataacaagac tcactaatga gggtatcact ttgactgact 1980
gatttgttaa agtttttaag cctctcattt tcctaaccca gaaatcacag cctgatttta 2040
ttaaaagtag agcttcattc atttcatacc atagatacca tcctagtaaa tccagaacat 2100
atacaaggtt catgtgagtc tgctttcttg acatgatagc attgtttgat gcagtggata 2160
tgtcagaatg actaacctag gagtttgaaa ctcctaagaa actaaaacct gtaagacatt 2220
taaaagtctc cacaatttta atgtatacaa agctatgtta ctgtgtaaca cattacagtt 2280
caaattcact ccagaaataa aaggccagta ggattaggga ctcactggta gtttggagtc 2340
tcccagcaca catccctcct agtgggatga tctattcaca tatctcccag cttttttatt 2400
tttgcttctg tatatcacag tgagtggatg gcccttcagc tttttctctc ctggccagac 2460
atgcagtctt gcctttagat atcgcagaga caaaattcac agcatgtctt aaatcttcca 2520
ggatttgcaa gaaccaaatt gctcaacagt atgtatgttt agaggggtta gactcctttt 2580
taaaatctgg atatctaacc acctacttaa atctgtttga tagtgtcaaa ccacccccac 2640
ccttgatcct cccaccccca aaaaaaaaaa aaaa 2674

<210> 280
<211> 143
<212> PRT
<213> Homo sapiens

<400> 280

Met Gly Ser Asp Lys Arg Val Ser Arg Thr Glu Arg Ser Gly Arg Tyr
1               5                   10                  15

Gly Ser Ile Ile Asp Arg Asp Asp Arg Asp Glu Arg Glu Ser Arg Ser
            20                  25                  30

Arg Arg Arg Asp Ser Asp Tyr Lys Arg Ser Ser Asp Asp Arg Arg Gly
        35                  40                  45

Asp Arg Tyr Asp Asp Tyr Arg Asp Tyr Asp Ser Pro Glu Arg Glu Arg
    50                  55                  60

Glu Arg Arg Asn Ser Asp Arg Ser Glu Asp Gly Tyr His Ser Asp Gly
65                  70                  75                  80

Asp Tyr Gly Glu His Asp Tyr Arg His Asp Ile Ser Asp Glu Arg Glu
                85                  90                  95

Ser Lys Thr Ile Met Leu Arg Gly Leu Pro Ile Thr Ile Thr Glu Ser
            100                 105                 110

Asp Ile Arg Glu Met Met Glu Ser Phe Glu Gly Pro Gln Pro Ala Asp
        115                 120                 125

Val Arg Leu Met Lys Arg Lys Thr Gly Glu Ser Leu Leu Ser Ser
    130                 135                 140

<210>  281
<211>  4000
<212>  DNA
<213>  Homo sapiens

<400>  281
cggccgcttt tttctcaaga tggcagattc ccactgaggc tgagggggcc gagctcgcgc      60

gccgcgttcc cttctccgtt gccatgaacc gcggacaccc cggccccgat ggcccccgtg     120

tacgaaggta tggcctcaca tgtgcaagtt ttctcccctc acacccttca atcaagtgcc     180

ttctgtagtg tgaagaaact aaaagtagag ccaagttcca actgggacat gactgggtac     240

ggctcccaca gcaaagtgta cagccagagc aagaacatac caccttctca gccagcctcc     300

acaaccgtca gcacctcctt gccggtccca aacccaagcc taccttacga gcagaccatc     360

gtcttcccag gaagcaccgg gcacatcgtg gtcacctcag caagcagcac ttctgtcacc     420

gggcaagtcc tcggcggacc acacaaccta atgcgtcgaa gcactgtgag cctccttgat     480

acctaccaaa aatgtggact caagcgtaag agcgaggaga tcgagaacac aagcagcgtg     540

cagatcatcg aggagcatcc acccatgatt cagaataatg caagcggggc cactgtcgcc     600

actgccacca cgtctactgc cacctccaaa aacagcggct ccaacagcga gggcgactat     660

cagctggtgc agcatgaggt gctgtgctcc atgaccaaca cctacgaggt cttagagttc     720

ttgggccgag ggacgtttgg acaagtggtc aagtgctgga aacggggcac caatgagatc     780

gtagccatca agatcctgaa gaaccgccca tcctatgccc gacaaggtca gattgaagtg     840

agcatcctgg cccggttgag cacggagagt gccgatgact ataacttcgt ccgggcctac     900

gaatgcttcc agcacaagaa ccacacgtgc ttggtcttcg agatgttgga gcagaacctc     960

tatgactttc tgaagcaaaa caagtttagc cccttgcccc tcaaatacat tcgcccagtt 1020
ctccagcagg tagccacagc cctgatgaaa ctcaaaagcc taggtcttat ccacgctgac 1080
ctcaaaccag aaaacatcat gctggtggat ccatctagac aaccatacag agtcaaggtc 1140
atcgactttg gttcagccag ccacgtctcc aaggctgtgt gctccaccta cttgcagtcc 1200
agatattaca gggcccctga gatcatcctt ggtttaccat tttgtgaggc aattgacatg 1260
tggtccctgg gctgtgttat tgcagaattg ttcctgggtt ggccgttata tccaggagct 1320
tcggagtatg atcagattcg gtatatttca caaacacagg gtttgcctgc tgaatattta 1380
ttaagcgccg ggacaaagac aactaggttt ttcaaccgtg acacggactc accatatcct 1440
ttgtggagac tgaagacacc agatgaccat gaagcagaga cagggattaa gtcaaaagaa 1500
gcaagaaagt acattttcaa ctgtttagat gatatggccc aggtgaacat gacgacagat 1560
ttggaaggga gcgacatgtt ggtagaaaag gctgaccggc gggagttcat tgacctgttg 1620
aagaagatgc tgaccattga tgctgacaag agaatcactc caatcgaaac cctgaaccat 1680
ccctttgtca ccatgacaca cttactcgat tttccccaca gcacacacgt caaatcatgt 1740
ttccagaaca tggagatctg caagcgtcgg gtgaatatgt atgacacggt gaaccagagc 1800
aaaacccctt tcatcacgca cgtggccccc agcacgtcca ccaacctgac catgaccttt 1860
aacaaccagc tgaccactgt ccacaaccag gctccctcct ctaccagtgc cactatttcc 1920
ttagccaatc ccgaagtctc catactaaac tacccatcta cactctacca gccctcagcg 1980
gcatccatgg ctgcagtggc ccagcggagc atgcccctgc agacaggaac agcccagatt 2040
tgtgcccggc ctgacccgtt ccagcaagct ctcatcgtgt gtcccccggg cttccaaggc 2100
ttgcaggcct ctccctctaa gcacgctggc tactcggtgc gaatggaaaa tgcagttccc 2160
atcgtcactc aagccccagg agctcagcct cttcagatcc aaccaggtct gcttgcccag 2220
caggcttggc caagtgggac ccagcagatc ctgcttcccc cagcatggca gcaactgact 2280
ggagtggcca cccacacatc agtgcagcat gccaccgtga ttcccgagac catggcaggc 2340
acccagcagc tggcggactg gagaaatacg catgctcacg gaagccatta taatcccatc 2400
atgcagcagc ctgcactatt gaccggtcat gtgacccttc cagcagcaca gcccttaaat 2460
gtgggtgtgg cccacgtgat gcggcagcag ccaaccagca ccacctcctc ccggaagagt 2520
aagcagcacc agtcatctgt gagaaatgtc tccacctgtg aggtgtcctc ctctcaggcc 2580
atcagctccc cacagcgatc caagcgtgtc aaggagaaca cacctccccg ctgtgccatg 2640
gtgcacagta gcccggcctg cagcacctcg gtcacctgtg ggtggggcga cgtggcctcc 2700
agcaccaccc gggaacggca gcggcagaca attgtcattc ccgacactcc cagccccacg 2760
gtcagcgtca tcaccatcag cagtgacacg gacgaggagg aggaacagaa acacgccccc 2820
accagcactg tctccaagca aagaaaaaac gtcatcagct gtgtcacagt ccacgactcc 2880
ccctactccg actcctccag caacaccagc ccctactccg tgcagcagcg tgctgggcac 2940
aacaatgcca atgcctttga caccaagggg agcctggaga atcactgcac ggggaacccc 3000

cgaaccatca tcgtgccacc cctgaaaacc caggccagcg aagtattggt ggagtgtgat 3060
agcctggtgc cagtcaacac cagtcaccac tcgtcctcct acaagtccaa gtcctccagc 3120
aacgtgacct ccaccagcgg tcactcttca gggagctcat ctggagccat cacctaccgg 3180
cagcagcggc cgggccccca cttccagcag cagcagccac tcaatctcag ccaggctcag 3240
cagcacatca ccacggaccg cactgggagc caccgaaggc agcaggccta catcactccc 3300
accatggccc aggctccgta ctccttcccg cacaacagcc ccagccacgg cactgtgcac 3360
ccgcatctgg ctgcagccgc tgccgctgcc cacctcccca cccagcccca cctctacacc 3420
tacactgcgc cggcggccct gggctccacc ggcaccgtgg cccacctggt ggcctcgcaa 3480
ggctctgcgc gccacaccgt gcagcacact gcctacccag ccagcatcgt ccaccaggtc 3540
cccgtgagca tgggcccccg ggtcctgccc tcgcccacca tccacccgag tcagtatcca 3600
gcccaatttg cccaccagac ctacatcagc gcctcgccag cctccaccgt ctacactgga 3660
tacccactga gccccgccaa ggtcaaccag tacccttaca tataaacact ggaggggagg 3720
gagggaggga gggagggaga gaatggcccg agggaggagg gagagaagga gggaggcgct 3780
cctgggaccg tgggcgctgg ccttttatac tgaagatgcc gcacacaaac aatgcaaacg 3840
gggcaggggc gggggggggg ggggcagagg gcagggggac gggtcgggac accagtgaaa 3900
cttgaaccgg gaagtgggag gacgtagagc agagaagaga acatttttaa aaggaaggga 3960
ttaaagaggg tgggaaatct atggttttta ttttaaaaaa 4000

<210> 282
<211> 1198
<212> PRT
<213> Homo sapiens

<400> 282

Met Ala Pro Val Tyr Glu Gly Met Ala Ser His Val Gln Val Phe Ser
1               5                   10                  15

Pro His Thr Leu Gln Ser Ser Ala Phe Cys Ser Val Lys Lys Leu Lys
            20                  25                  30

Val Glu Pro Ser Ser Asn Trp Asp Met Thr Gly Tyr Gly Ser His Ser
        35                  40                  45

Lys Val Tyr Ser Gln Ser Lys Asn Ile Pro Pro Ser Gln Pro Ala Ser
    50                  55                  60

Thr Thr Val Ser Thr Ser Leu Pro Val Pro Asn Pro Ser Leu Pro Tyr
65                  70                  75                  80

Glu Gln Thr Ile Val Phe Pro Gly Ser Thr Gly His Ile Val Val Thr
                85                  90                  95

Ser Ala Ser Ser Thr Ser Val Thr Gly Gln Val Leu Gly Gly Pro His
            100                 105                 110

Asn Leu Met Arg Arg Ser Thr Val Ser Leu Leu Asp Thr Tyr Gln Lys
        115                 120                 125

Cys Gly Leu Lys Arg Lys Ser Glu Glu Ile Glu Asn Thr Ser Ser Val
    130                 135                 140

Gln Ile Ile Glu Glu His Pro Pro Met Ile Gln Asn Asn Ala Ser Gly
145                 150                 155                 160

Ala Thr Val Ala Thr Ala Thr Thr Ser Thr Ala Thr Ser Lys Asn Ser
                165                 170                 175

Gly Ser Asn Ser Glu Gly Asp Tyr Gln Leu Val Gln His Glu Val Leu
            180                 185                 190

Cys Ser Met Thr Asn Thr Tyr Glu Val Leu Glu Phe Leu Gly Arg Gly
        195                 200                 205

Thr Phe Gly Gln Val Val Lys Cys Trp Lys Arg Gly Thr Asn Glu Ile
    210                 215                 220

Val Ala Ile Lys Ile Leu Lys Asn Arg Pro Ser Tyr Ala Arg Gln Gly
225                 230                 235                 240

Gln Ile Glu Val Ser Ile Leu Ala Arg Leu Ser Thr Glu Ser Ala Asp
                245                 250                 255

Asp Tyr Asn Phe Val Arg Ala Tyr Glu Cys Phe Gln His Lys Asn His
            260                 265                 270

Thr Cys Leu Val Phe Glu Met Leu Glu Gln Asn Leu Tyr Asp Phe Leu
        275                 280                 285

Lys Gln Asn Lys Phe Ser Pro Leu Pro Leu Lys Tyr Ile Arg Pro Val
    290                 295                 300

Leu Gln Gln Val Ala Thr Ala Leu Met Lys Leu Lys Ser Leu Gly Leu
305                 310                 315                 320

Ile His Ala Asp Leu Lys Pro Glu Asn Ile Met Leu Val Asp Pro Ser
                325                 330                 335

Arg Gln Pro Tyr Arg Val Lys Val Ile Asp Phe Gly Ser Ala Ser His
            340                 345                 350

Val Ser Lys Ala Val Cys Ser Thr Tyr Leu Gln Ser Arg Tyr Tyr Arg
        355                 360                 365

Ala Pro Glu Ile Ile Leu Gly Leu Pro Phe Cys Glu Ala Ile Asp Met
    370                 375                 380

Trp Ser Leu Gly Cys Val Ile Ala Glu Leu Phe Leu Gly Trp Pro Leu
385                 390                 395                 400

Tyr Pro Gly Ala Ser Glu Tyr Asp Gln Ile Arg Tyr Ile Ser Gln Thr
                405                 410                 415

Gln Gly Leu Pro Ala Glu Tyr Leu Leu Ser Ala Gly Thr Lys Thr Thr
            420                 425                 430

Arg Phe Phe Asn Arg Asp Thr Asp Ser Pro Tyr Pro Leu Trp Arg Leu
        435                 440                 445

Lys Thr Pro Asp Asp His Glu Ala Glu Thr Gly Ile Lys Ser Lys Glu
    450                 455                 460

Ala Arg Lys Tyr Ile Phe Asn Cys Leu Asp Asp Met Ala Gln Val Asn
465                 470                 475                 480

Met Thr Thr Asp Leu Glu Gly Ser Asp Met Leu Val Glu Lys Ala Asp
                485                 490                 495

Arg Arg Glu Phe Ile Asp Leu Leu Lys Lys Met Leu Thr Ile Asp Ala
            500                 505                 510

Asp Lys Arg Ile Thr Pro Ile Glu Thr Leu Asn His Pro Phe Val Thr
        515                 520                 525

Met Thr His Leu Leu Asp Phe Pro His Ser Thr His Val Lys Ser Cys
    530                 535                 540

Phe Gln Asn Met Glu Ile Cys Lys Arg Arg Val Asn Met Tyr Asp Thr
545                 550                 555                 560

Val Asn Gln Ser Lys Thr Pro Phe Ile Thr His Val Ala Pro Ser Thr
                565                 570                 575

Ser Thr Asn Leu Thr Met Thr Phe Asn Asn Gln Leu Thr Thr Val His
            580                 585                 590

Asn Gln Ala Pro Ser Ser Thr Ser Ala Thr Ile Ser Leu Ala Asn Pro
        595                 600                 605

Glu Val Ser Ile Leu Asn Tyr Pro Ser Thr Leu Tyr Gln Pro Ser Ala
    610                 615                 620

Ala Ser Met Ala Ala Val Ala Gln Arg Ser Met Pro Leu Gln Thr Gly
625                 630                 635                 640

Thr Ala Gln Ile Cys Ala Arg Pro Asp Pro Phe Gln Gln Ala Leu Ile
                645                 650                 655

Val Cys Pro Pro Gly Phe Gln Gly Leu Gln Ala Ser Pro Ser Lys His
            660                 665                 670

Ala Gly Tyr Ser Val Arg Met Glu Asn Ala Val Pro Ile Val Thr Gln
        675                 680                 685

Ala Pro Gly Ala Gln Pro Leu Gln Ile Gln Pro Gly Leu Leu Ala Gln
    690                 695                 700

Gln Ala Trp Pro Ser Gly Thr Gln Gln Ile Leu Leu Pro Pro Ala Trp
705                 710                 715                 720

Gln Gln Leu Thr Gly Val Ala Thr His Thr Ser Val Gln His Ala Thr
                725                 730                 735

Val Ile Pro Glu Thr Met Ala Gly Thr Gln Gln Leu Ala Asp Trp Arg
            740                 745                 750

Asn Thr His Ala His Gly Ser His Tyr Asn Pro Ile Met Gln Gln Pro
        755                 760                 765

Ala Leu Leu Thr Gly His Val Thr Leu Pro Ala Ala Gln Pro Leu Asn
    770                 775                 780

Val Gly Val Ala His Val Met Arg Gln Gln Pro Thr Ser Thr Thr Ser
785                 790                 795                 800

Ser Arg Lys Ser Lys Gln His Gln Ser Ser Val Arg Asn Val Ser Thr
                805                 810                 815

Cys Glu Val Ser Ser Ser Gln Ala Ile Ser Ser Pro Gln Arg Ser Lys
            820                 825                 830

Arg Val Lys Glu Asn Thr Pro Pro Arg Cys Ala Met Val His Ser Ser
        835                 840                 845

Pro Ala Cys Ser Thr Ser Val Thr Cys Gly Trp Gly Asp Val Ala Ser
    850                 855                 860

Ser Thr Thr Arg Glu Arg Gln Arg Gln Thr Ile Val Ile Pro Asp Thr

865                 870                 875                 880

Pro Ser Pro Thr Val Ser Val Ile Thr Ile Ser Ser Asp Thr Asp Glu
                885                 890                 895

Glu Glu Glu Gln Lys His Ala Pro Thr Ser Thr Val Ser Lys Gln Arg
            900                 905                 910

Lys Asn Val Ile Ser Cys Val Thr Val His Asp Ser Pro Tyr Ser Asp
        915                 920                 925

Ser Ser Ser Asn Thr Ser Pro Tyr Ser Val Gln Gln Arg Ala Gly His
    930                 935                 940

Asn Asn Ala Asn Ala Phe Asp Thr Lys Gly Ser Leu Glu Asn His Cys
945                 950                 955                 960

Thr Gly Asn Pro Arg Thr Ile Ile Val Pro Pro Leu Lys Thr Gln Ala
                965                 970                 975

Ser Glu Val Leu Val Glu Cys Asp Ser Leu Val Pro Val Asn Thr Ser
            980                 985                 990

His His Ser Ser Ser Tyr Lys Ser  Lys Ser Ser Ser Asn  Val Thr Ser
        995                 1000                1005

Thr Ser  Gly His Ser Ser Gly  Ser Ser Ser Gly Ala  Ile Thr Tyr
    1010                1015                1020

Arg Gln  Gln Arg Pro Gly Pro  His Phe Gln Gln Gln  Gln Pro Leu
    1025                1030                1035

Asn Leu  Ser Gln Ala Gln Gln  His Ile Thr Thr Asp  Arg Thr Gly
    1040                1045                1050

Ser His  Arg Arg Gln Gln Ala  Tyr Ile Thr Pro Thr  Met Ala Gln
    1055                1060                1065

Ala Pro  Tyr Ser Phe Pro His  Asn Ser Pro Ser His  Gly Thr Val
    1070                1075                1080

His Pro  His Leu Ala Ala Ala  Ala Ala Ala Ala His  Leu Pro Thr
    1085                1090                1095

Gln Pro  His Leu Tyr Thr Tyr  Thr Ala Pro Ala Ala  Leu Gly Ser
    1100                1105                1110

Thr Gly  Thr Val Ala His Leu  Val Ala Ser Gln Gly  Ser Ala Arg
    1115                1120                1125

His Thr  Val Gln His Thr Ala  Tyr Pro Ala Ser Ile  Val His Gln
    1130                1135                1140

Val Pro  Val Ser Met Gly Pro  Arg Val Leu Pro Ser  Pro Thr Ile
    1145                1150                1155

His Pro  Ser Gln Tyr Pro Ala  Gln Phe Ala His Gln  Thr Tyr Ile
    1160                1165                1170

Ser Ala  Ser Pro Ala Ser Thr  Val Tyr Thr Gly Tyr  Pro Leu Ser
    1175                1180                1185

Pro Ala  Lys Val Asn Gln Tyr  Pro Tyr Ile
    1190                1195

<210>  283
<211>  1464
<212>  DNA

<213>  Homo sapiens

<400>  283
agtcacagcc aggtaaccct ggagtgaagc ggtttagtta gaagggagca gataaactcg      60

tcactctagt agctttaacc ctcaccctga ggcaccttag caatcagcca ttgcctgcaa     120

gcctccaaag cttgtctttg cctaatatgg agcccaaaga agccactggg aaagaaaaca     180

tggtcaccaa gaaaaagaat ctggccttct tgaggtctag actctatatg ctggagagaa     240

ggaagactga cactgtggtt gagagcagtg tttctgggga ccactctggc accttgagga     300

ggagccaatc tgacaggacc gaatacaacc agaaattaca agaaaagatg actccacagg     360

gtgagtgttc tgtagctgag accttaaccc cagaggaaga gcatcatatg aagaggatga     420

tggcaaagcg ggaaaagatc attaaggagc tgatacagac agaaaaggat tatctcaatg     480

atctagagct gtgtgttagg gaagtggttc agcccctgag aaataaaaag actgataggc     540

tggatgtgga tagcttgttt agcaacattg agtccgtgca tcagatatca gccaagctgc     600

tgtcattgtt ggaagaggcc acaacagacg tggaaccggc catgcaagta attggagaag     660

tattcttgca gattaaaggg ccactggaag atatttataa aatctactgc tatcaccatg     720

atgaagcaca tagtatactg gagtcctatg aaaaggaaga agagctgaag gaacatttga     780

gccactgtat ccagtcctta aagtaaggcc ttttcaaatg atgattccca tctcctctca     840

gttgcctagc agggaacatt ttaaatggat gtagatgaaa ggtctcacat aaatcctatg     900

ttttatgaga cttgctggga gctctgcttt gcattccctt tataaaaagc tgacatgcca     960

gaagccctga ttgacttttt ttccccctgc gagaatgact aaaaataaca tggaagaaga    1020

tttagagctc tgcagcgatt gaaaaatgca atatcaaaat ataaaatgtg gaagaaaagc    1080

ctcttcttaa agctattgta acttgcctgg ccccacgtag ttcaaggatt atgtgagata    1140

acacgtggcc ccatgaccac tggagcacat gggttaatgg agttagggga atggcctaca    1200

actctgcatg gccgtcttct ttccccaaac tcactgtggg gagatgggtg aagacaagtc    1260

aggccttgtt aaagttagtt tcagaacaat tactcatgcc ttcctttctc atccctaaaa    1320

cattggtggg ggagctacac aatgtacttt ttcttttcta gaggaagtat ctattcactg    1380

tgaaaatctg aaaaatataa caaagtatgt gtaagataaa aaccccttgc tatttcaaaa    1440

aaaaaaaaaa aaaaaaaaaa aaaa                                            1464

<210>  284
<211>  219
<212>  PRT
<213>  Homo sapiens

<400>  284
   Met Glu Pro Lys Glu Ala Thr Gly Lys Glu Asn Met Val Thr Lys Lys
   1               5                   10                  15
   Lys Asn Leu Ala Phe Leu Arg Ser Arg Leu Tyr Met Leu Glu Arg Arg
               20                  25                  30
   Lys Thr Asp Thr Val Val Glu Ser Ser Val Ser Gly Asp His Ser Gly
           35                  40                  45
   Thr Leu Arg Arg Ser Gln Ser Asp Arg Thr Glu Tyr Asn Gln Lys Leu
       50                  55                  60
   Gln Glu Lys Met Thr Pro Gln Gly Glu Cys Ser Val Ala Glu Thr Leu
   65                  70                  75                  80
   Thr Pro Glu Glu Glu His His Met Lys Arg Met Met Ala Lys Arg Glu
                   85                  90                  95
   Lys Ile Ile Lys Glu Leu Ile Gln Thr Glu Lys Asp Tyr Leu Asn Asp
               100                 105                 110
   Leu Glu Leu Cys Val Arg Glu Val Val Gln Pro Leu Arg Asn Lys Lys
           115                 120                 125
   Thr Asp Arg Leu Asp Val Asp Ser Leu Phe Ser Asn Ile Glu Ser Val
       130                 135                 140
   His Gln Ile Ser Ala Lys Leu Leu Ser Leu Leu Glu Glu Ala Thr Thr
   145                 150                 155                 160
   Asp Val Glu Pro Ala Met Gln Val Ile Gly Glu Val Phe Leu Gln Ile
                   165                 170                 175
   Lys Gly Pro Leu Glu Asp Ile Tyr Lys Ile Tyr Cys Tyr His His Asp
               180                 185                 190
   Glu Ala His Ser Ile Leu Glu Ser Tyr Glu Lys Glu Glu Glu Leu Lys
           195                 200                 205
   Glu His Leu Ser His Cys Ile Gln Ser Leu Lys
       210                 215

<210>  285
<211>  3004
<212>  DNA
<213>  Homo sapiens

<400>  285
cccttatggc gattgggcgg ctgcagagac caggactcag ttcccctgcc ctagtctgag      60

cctagtgggt gggactcagc tcagagtcag ttttcagaag caggtttcag ttgcagagtt     120

ttcctacact tttcctgcgc tagagcagcg agcagcctgg aacagaccca ggcggaggac     180

acctgtgggg gagggagcgc ctggaggagc ttagagaccc cagccgggcg tgatctcacc     240

atgtgcggat ttgcgaggcg cgccctggag ctgctagaga tccggaagca cagccccgag     300

gtgtgcgaag ccaccaagac tgcggctctt ggagaaagcg tgagcagggg gccaccgcgg     360

tctccggcct gtctgcaccc tgtcgcctga gctgcctgac agtgacaatg acatcccagt     420

taccagtgtc cttgaattga tagtggcttc tgtttgtcag tctcatataa gaactacagc     480

tcatcaggag gagatcgcag cagggtaaga gacaccaaca ccatgttctg cacgaagctc     540

aaggatctca agatcacagg agagtgtcct ttctccttac tggcaccagg tcaagttcct     600

aacgagtctt cagaggaggc agcaggaagc tcagagagct gcaaagcaac cgtgcccatc     660

tgtcaagaca ttcctgagaa gaacatacaa gaaagtcttc ctcaaagaaa aaccagtcgg     720

agccgagtct atcttcacac tttggcagag agtatttgca aactgatttt cccagagttt     780

gaacggctga atgttgcact tcagagaaca ttggcaaagc acaaaataaa agaaagcagg     840

aaatctttgg aaagagaaga ctttgaaaaa acaattgcag agcaagcagt gcagcagagt 900
ccagtggagt tatcaaagaa tctcttggtg aagaggtttt taaaatatgt tacgaggaag 960
atgaaaacat ccttggggtg gttggaggca cccttaaaga tttttaaaca gcttcagtac 1020
ccttctgaaa cagagcagcc attgccaaga agcaggaaaa aggggcagct tgaggacgcc 1080
tccattctat gcctggataa ggaggatgat tttctacatg tttactactt cttccctaag 1140
agaaccacct ccctgattct tcccggcatc ataaaggcag ctgctcacgt attatatgaa 1200
acggaagtgg aagtgtcgtt aatgcctccc tgcttccata atgattgcag cgagtttgtg 1260
aatcagccct acttgttgta ctccgttcac atgaaaagca ccaagccatc cctgtccccc 1320
agcaaacccc agtcctcgct ggtgattccc acatcgctat tctgcaagac atttccattc 1380
catttcatgt ttgacaaaga tatgacaatt ctgcaatttg gcaatggcat cagaaggctg 1440
atgaacagga gagactttca aggaaagcct aattttgaat actttgaaat tctgactcca 1500
aaaatcaacc agacctttag cgggatcatg actatgttga atatgcagtt tgttgtacga 1560
gtgaggagat gggacaactc tgtgaagaaa tcttcaaggg ttatggacct caaaggccaa 1620
atgatctaca ttgttgaatc cagtgcaatc ttgtttttgg ggtcaccctg tgtggacaga 1680
ttagaagatt ttacaggacg agggctctac ctctcagaca tcccaattca caatgcactg 1740
agggatgtgg tcttaatagg ggaacaagcc cgagctcaag atggcctgaa gaagaggctg 1800
gggaagctga aggctaccct tgagcaagcc caccaagccc tggaggagga gaagaaaaag 1860
acagtagacc ttctgtgctc catatttccc tgtgaggttg ctcagcagct gtggcaaggg 1920
caagttgtgc aagccaagaa gttcagtaat gtcaccatgc tcttctcaga catcgttggg 1980
ttcactgcca tctgctccca gtgctcaccg ctgcaggtca tcaccatgct caatgcactg 2040
tacactcgct tcgaccagca gtgtggagag ctggatgtct acaaggtgga gaccattgcg 2100
atgcctattg tgtggcttgg gggattacac aaagagagtg atactcatgc tgttcagata 2160
gcgctgatgg ccctgaagat gatggagctc tctgatgaag ttatgtctcc ccatggagaa 2220
cctatcaaga tgcgaattgg actgcactct ggatcagttt ttgctggcgt cgttggagtt 2280
aaaatgcccc gttactgtct ttttggaaac aatgtcactc tggctaacaa atttgagtcc 2340
tgcagtgtac cacgaaaaat caatgtcagc ccaacaactt acagattact caaagactgt 2400
cctggtttcg tgtttacccc tcgatcaagg gaggaacttc caccaaactt ccctagtgaa 2460
atccccggaa tctgccattt tctggatgct taccaacaag gaacaaactc aaaaccatgc 2520
ttccaaaaga aagatgtgga agatgcaagc caatttttta ggcaaagcat caggaataga 2580
ttagcaacct atatacctat ttataagtct ttggggtttg actcattgaa gatgtgtaga 2640
gcctctgaaa gcactttagg gattgtagat ggctaacaag cagtattaaa atttcaggag 2700
ccaagtcaca atctttctcc tgtttaacat gacaaaatgt actcacttca gtacttcagc 2760
tcttcaagaa aaaaaaaaaa accttaaaaa gctacttttg tgggagtatt tctattatat 2820
aaccagcact tactacctgt actcaaaatt cagcaccttg tacatatatc agataattgt 2880

agtcaattgt acaaactgat ggagtcacct gcaatctcat atcctggtgg aatgccatgg 2940

ttattaaagt gtgtttgtga tagttgtcgt caaaaaaaaa aaaaaaaaaa aaaaaaaaaa 3000

aaaa 3004

<210> 286
<211> 690
<212> PRT
<213> Homo sapiens

<400> 286

Met Phe Cys Thr Lys Leu Lys Asp Leu Lys Ile Thr Gly Glu Cys Pro
1               5                   10                  15

Phe Ser Leu Leu Ala Pro Gly Gln Val Pro Asn Glu Ser Ser Glu Glu
            20                  25                  30

Ala Ala Gly Ser Ser Glu Ser Cys Lys Ala Thr Val Pro Ile Cys Gln
        35                  40                  45

Asp Ile Pro Glu Lys Asn Ile Gln Glu Ser Leu Pro Gln Arg Lys Thr
    50                  55                  60

Ser Arg Ser Arg Val Tyr Leu His Thr Leu Ala Glu Ser Ile Cys Lys
65                  70                  75                  80

Leu Ile Phe Pro Glu Phe Glu Arg Leu Asn Val Ala Leu Gln Arg Thr
                85                  90                  95

Leu Ala Lys His Lys Ile Lys Glu Ser Arg Lys Ser Leu Glu Arg Glu
            100                 105                 110

Asp Phe Glu Lys Thr Ile Ala Glu Gln Ala Val Ala Ala Gly Val Pro
        115                 120                 125

Val Glu Val Ile Lys Glu Ser Leu Gly Glu Glu Val Phe Lys Ile Cys
    130                 135                 140

Tyr Glu Glu Asp Glu Asn Ile Leu Gly Val Val Gly Gly Thr Leu Lys
145                 150                 155                 160

Asp Phe Leu Asn Ser Phe Ser Thr Leu Leu Lys Gln Ser Ser His Cys
                165                 170                 175

Gln Glu Ala Gly Lys Arg Gly Arg Leu Glu Asp Ala Ser Ile Leu Cys
            180                 185                 190

Leu Asp Lys Glu Asp Asp Phe Leu His Val Tyr Tyr Phe Phe Pro Lys
        195                 200                 205

Arg Thr Thr Ser Leu Ile Leu Pro Gly Ile Ile Lys Ala Ala Ala His
    210                 215                 220

Val Leu Tyr Glu Thr Glu Val Glu Val Ser Leu Met Pro Pro Cys Phe
225                 230                 235                 240

His Asn Asp Cys Ser Glu Phe Val Asn Gln Pro Tyr Leu Leu Tyr Ser
                245                 250                 255

Val His Met Lys Ser Thr Lys Pro Ser Leu Ser Pro Ser Lys Pro Gln
            260                 265                 270

Ser Ser Leu Val Ile Pro Thr Ser Leu Phe Cys Lys Thr Phe Pro Phe

        275                 280                 285

His Phe Met Phe Asp Lys Asp Met Thr Ile Leu Gln Phe Gly Asn Gly

290                 295                 300

Ile Arg Arg Leu Met Asn Arg Arg Asp Phe Gln Gly Lys Pro Asn Phe
305                 310                 315                 320

Glu Glu Tyr Phe Glu Ile Leu Thr Pro Lys Ile Asn Gln Thr Phe Ser
                325                 330                 335

Gly Ile Met Thr Met Leu Asn Met Gln Phe Val Val Arg Val Arg Arg
            340                 345                 350

Trp Asp Asn Ser Val Lys Lys Ser Ser Arg Val Met Asp Leu Lys Gly
        355                 360                 365

Gln Met Ile Tyr Ile Val Glu Ser Ser Ala Ile Leu Phe Leu Gly Ser
    370                 375                 380

Pro Cys Val Asp Arg Leu Glu Asp Phe Thr Gly Arg Gly Leu Tyr Leu
385                 390                 395                 400

Ser Asp Ile Pro Ile His Asn Ala Leu Arg Asp Val Val Leu Ile Gly
                405                 410                 415

Glu Gln Ala Arg Ala Gln Asp Gly Leu Lys Lys Arg Leu Gly Lys Leu
            420                 425                 430

Lys Ala Thr Leu Glu Gln Ala His Gln Ala Leu Glu Glu Glu Lys Lys
        435                 440                 445

Lys Thr Val Asp Leu Leu Cys Ser Ile Phe Pro Cys Glu Val Ala Gln
    450                 455                 460

Gln Leu Trp Gln Gly Gln Val Val Gln Ala Lys Lys Phe Ser Asn Val
465                 470                 475                 480

Thr Met Leu Phe Ser Asp Ile Val Gly Phe Thr Ala Ile Cys Ser Gln
                485                 490                 495

Cys Ser Pro Leu Gln Val Ile Thr Met Leu Asn Ala Leu Tyr Thr Arg
            500                 505                 510

Phe Asp Gln Gln Cys Gly Glu Leu Asp Val Tyr Lys Val Glu Thr Ile
        515                 520                 525

Gly Asp Ala Tyr Cys Val Ala Gly Gly Leu His Lys Glu Ser Asp Thr
    530                 535                 540

His Ala Val Gln Ile Ala Leu Met Ala Leu Lys Met Met Glu Leu Ser
545                 550                 555                 560

Asp Glu Val Met Ser Pro His Gly Glu Pro Ile Lys Met Arg Ile Gly
                565                 570                 575

Leu His Ser Gly Ser Val Phe Ala Gly Val Val Gly Val Lys Met Pro
            580                 585                 590

Arg Tyr Cys Leu Phe Gly Asn Asn Val Thr Leu Ala Asn Lys Phe Glu
        595                 600                 605

Ser Cys Ser Val Pro Arg Lys Ile Asn Val Ser Pro Thr Thr Tyr Arg
    610                 615                 620

Leu Leu Lys Asp Cys Pro Gly Phe Val Phe Thr Pro Arg Ser Arg Glu
625                 630                 635                 640

Glu Leu Pro Pro Asn Phe Pro Ser Glu Ile Pro Gly Ile Cys His Phe
                645                 650                 655

Leu Asp Ala Tyr Gln Gln Gly Thr Asn Ser Lys Pro Cys Phe Gln Lys
            660                 665                 670

Lys Asp Val Glu Asp Gly Asn Ala Asn Phe Leu Gly Lys Ala Ser Gly
        675                 680                 685

Ile Asp
    690

<210>  287
<211>  7193
<212>  DNA
<213>  Homo sapiens

<400>  287
agaataaggg cagggaccgc ggctcctatc tcttggtgat ccccttcccc attccgcccc      60

cgcctcaacg cccagcacag tgccctgcac acagtagtcg ctcaataaat gttcgtggat     120

gatgatgatg atgatgatga aaaaaatgca gcatcaacgg cagcagcaag cggaccacgc     180

gaacgaggca aactatgcaa gaggcaccag acttcctctt tctggtgaag gaccaacttc     240

tcagccgaat agctccaagc aaactgtcct gtcttggcaa gctgcaatcg atgctgctag     300

acaggccaag gctgcccaaa ctatgagcac ctctgcaccc ccacctgtag gatctctctc     360

ccaaagaaaa cgtcagcaat acgccaagag caaaaaacag ggtaactcgt ccaacagccg     420

acctgcccgc gcccttttct gtttatcact caataacccc atccgaagag cctgcattag     480

tatagtggaa tggaaaccat ttgacatatt tatattattg gctatttttg ccaattgtgt     540

ggccttagct atttacatcc cattccctga agatgattct aattcaacaa atcataactt     600

ggaaaaagta gaatatgcct tcctgattat ttttacagtc gagacatttt tgaagattat     660

agcgtatgga ttattgctac atcctaatgc ttatgttagg aatggatgga atttactgga     720

ttttgttata gtaatagtag gattgtttag tgtaattttg gaacaattaa ccaaagaaac     780

agaaggcggg aaccactcaa gcggcaaatc tggaggcttt gatgtcaaag ccctccgtgc     840

ctttcgagtg ttgcgaccac ttcgactagt gtcaggggtg cccagtttac aagttgtcct     900

gaactccatt ataaaagcca tggttcccct ccttcacata gcccttttgg tattatttgt     960

aatcataatc tatgctatta taggattgga actttttatt ggaaaaatgc acaaaacatg    1020

tttttttgct gactcagata tcgtagctga agaggaccca gctccatgtg cgttctcagg    1080

gaatggacgc cagtgtactg ccaatggcac ggaatgtagg agtggctggg ttggcccgaa    1140

cggaggcatc accaactttg ataactttgc ctttgccatg cttactgtgt ttcagtgcat    1200

caccatggag ggctggacag acgtgctcta ctgggtaaat gatgcgatag gatgggaatg    1260

gccatgggtg tattttgtta gtctgatcat ccttggctca tttttcgtcc ttaacctggt    1320

tcttggtgtc cttagtggag aattctcaaa ggaaagagag aaggcaaaag cacggggaga    1380

tttccagaag ctccgggaga agcagcagct ggaggaggat ctaaagggct acttggattg    1440

gatcacccaa gctgaggaca tcgatccgga gaatgaggaa gaaggaggag aggaaggcaa    1500

acgaaatact agcatgccca ccagcgagac tgagtctgtg aacacagaga acgtcagcgg    1560

tgaaggcgag aaccgaggct gctgtggaag tctctggtgc tggtggagac ggagaggcgc    1620

ggccaaggcg gggccctctg ggtgtcggcg gtggggtcaa gccatctcaa aatccaaact 1680
cagccgacgc tggcgtcgct ggaaccgatt caatcgcaga agatgtaggg ccgccgtgaa 1740
gtctgtcacg ttttactggc tggttatcgt cctggtgttt ctgaacacct taaccatttc 1800
ctctgagcac tacaatcagc cagattggtt gacacagatt caagatattg ccaacaaagt 1860
cctcttggct ctgttcacct gcgagatgct ggtaaaaatg tacagcttgg gcctccaagc 1920
atatttcgtc tctcttttca accggtttga ttgcttcgtg gtgtgtggtg gaatcactga 1980
gacgatcctg gtggaactgg aaatcatgtc tcccctgggg atctctgtgt ttcggtgtgt 2040
gcgcctctta agaatcttca aagtgaccag gcactggact tccctgagca acttagtggc 2100
atccttatta aactccatga agtccatcgc ttcgctgttg cttctgcttt ttctcttcat 2160
tatcatcttt tccttgcttg ggatgcagct gtttggcggc aagtttaatt ttgatgaaac 2220
gcaaaccaag cggagcacct ttgacaattt ccctcaagca cttctcacag tgttccagat 2280
cctgacaggc gaagactgga atgctgtgat gtacgatggc atcatggctt acgggggccc 2340
atcctcttca ggaatgatcg tctgcatcta cttcatcatc ctcttcattt gtggtaacta 2400
tattctactg aatgtcttct tggccatcgc tgtagacaat ttggctgatg ctgaaagtct 2460
gaacactgct cagaaagaag aagcggaaga aaaggagagg aaaaagattg ccagaaaaga 2520
gagcctagaa aataaaaaga acaacaaacc agaagtcaac cagatagcca acagtgacaa 2580
caaggttaca attgatgact atagagaaga ggatgaagac aaggacccct atccgccttg 2640
cgatgtgcca gtaggggaag aggaagagga agaggaggag gatgaacctg aggttcctgc 2700
cggaccccgt cctcgaagga tctcggagtt gaacatgaag gaaaaaattg cccccatccc 2760
tgaagggagc gctttcttca ttcttagcaa gaccaacccg atccgcgtag gctgccacaa 2820
gctcatcaac caccacatct tcaccaacct catccttgtc ttcatcatgc tgagcagcgc 2880
tgccctggcc gcagaggacc ccatccgcag ccactccttc cggaacacga tactgggtta 2940
ctttgactat gccttcacag ccatctttac tgttgagatc ctgttgaaga tgacaacttt 3000
tggagctttc ctccacaaag gggccttctg caggaactac ttcaatttgc tggatatgct 3060
ggtggttggg gtgtctctgg tgtcatttgg gattcaatcc agtgccatct ccgttgtgaa 3120
gattctgagg gtcttaaggg tcctgcgtcc cctcaggccc atcaacagag caaaaggact 3180
taagcacgtg gtccagtgcg tcttcgtggc catccggacc atcggcaaca tcatgatcgt 3240
cactaccctc ctgcagttca tgtttgcctg tatcggggtc cagttgttca aggggaagtt 3300
ctatcgctgt acggatgaag ccaaaagtaa ccctgaagaa tgcaggggac ttttcatcct 3360
ctacaaggat ggggatgttg acagtcctgt ggtccgtgaa cggatctggc aaaacagtga 3420
tttcaacttc gacaacgtcc tctctgctat gatggcgctc ttcacagtct ccacgtttga 3480
gggctggcct gcgttgctgt ataaagccat cgactcgaat ggagagaaca tcggcccaat 3540
ctacaaccac cgcgtggaga tctccatctt cttcatcatc tacatcatca ttgtagcttt 3600
cttcatgatg aacatctttg tgggctttgt catcgttaca tttcaggaac aaggagaaaa 3660

agagtataag aactgtgagc tggacaaaaa tcagcgtcag tgtgttgaat acgccttgaa 3720
agcacgtccc ttgcggagat acatccccaa aaacccctac cagtacaagt tctggtacgt 3780
ggtgaactct tcgcctttcg aatacatgat gtttgtcctc atcatgctca acacactctg 3840
cttggccatg cagcactacg agcagtccaa gatgttcaat gatgccatgg acattctgaa 3900
catggtcttc accggggtgt tcaccgtcga gatggttttg aaagtcatcg catttaagcc 3960
taaggggtat tttagtgacg cctggaacac gtttgactcc ctcatcgtaa tcggcagcat 4020
tatagacgtg gccctcagcg aagcggaccc aactgaaagt gaaaatgtcc ctgtcccaac 4080
tgctacacct gggaactctg aagagagcaa tagaatctcc atcacctttt tccgtctttt 4140
ccgagtgatg cgattggtga agcttctcag caggggggaa ggcatccgga cattgctgtg 4200
gacttttatt aagtcctttc aggcgctccc gtatgtggcc ctcctcatag ccatgctgtt 4260
cttcatctat gcggtcattg gcatgcagat gtttgggaaa gttgccatga gagataacaa 4320
ccagatcaat aggaacaata acttccagac gtttccccag gcggtgctgc tgctcttcag 4380
gtgtgcaaca ggtgaggcct ggcaggagat catgctggcc tgtctcccag ggaagctctg 4440
tgaccctgag tcagattaca accccgggga ggagtataca tgtgggagca actttgccat 4500
tgtctatttc atcagttttt acatgctctg tgcatttctg atcatcaatc tgtttgtggc 4560
tgtcatcatg gataatttcg actatctgac ccgggactgg tctattttgg ggcctcacca 4620
tttagatgaa ttcaaaagaa tatggtcaga atatgaccct gaggcaaagg gaaggataaa 4680
acaccttgat gtggtcactc tgcttcgacg catccagcct cccctggggt ttgggaagtt 4740
atgtccacac agggtagcgt gcaagagatt agttgccatg aacatgcctc tcaacagtga 4800
cgggacagtc atgtttaatg caaccctgtt tgctttggtt cgaacggctc ttaagatcaa 4860
gaccgaaggg aacctggagc aagctaatga agaacttcgg gctgtgataa agaaaatttg 4920
gaagaaaacc agcatgaaat tacttgacca agttgtccct ccagctggtg atgatgaggt 4980
aaccgtgggg aagttctatg ccactttcct gatacaggac tactttagga aattcaagaa 5040
acggaaagaa caaggactgg tgggaaagta ccctgcgaag aacaccacaa ttgccctaca 5100
ggcgggatta aggacactgc atgacattgg gccagaaatc cggcgtgcta tatcgtgtga 5160
tttgcaagat gacgagcctg aggaaacaaa acgagaagaa gaagatgatg tgttcaaaag 5220
aaatggtgcc ctgcttggaa accatgtcaa tcatgttaat agtgatagga gagattccct 5280
tcagcagacc aataccaccc accgtcccct gcatgtccaa aggccttcaa ttccacctgc 5340
aagtgatact gagaaaccgc tgtttcctcc agcaggaaat tcggtgtgtc ataaccatca 5400
taaccataat tccataggaa agcaagttcc cacctcaaca aatgccaatc tcaataatgc 5460
caatatgtcc aaagctgccc atggaaagcg gcccagcatt gggaaccttg agcatgtgtc 5520
tgaaaatggg catcattctt cccacaagca tgaccgggag cctcagagaa ggtccagtgt 5580
gaaaagaacc cgctattatg aaacttacat taggtccgac tcaggagatg aacagctccc 5640
aactatttgc cgggaagacc cagagataca tggctatttc agggaccccc actgcttggg 5700

ggagcaggag tatttcagta gtgaggaatg ctacgaggat gacagctcgc ccacctggag 5760

caggcaaaac tatggctact acagcagata cccaggcaga aacatcgact ctgagaggcc 5820

ccgaggctac catcatcccc aaggattctt ggaggacgat gactcgcccg tttgctatga 5880

ttcacggaga tctccaagga gacgcctact acctcccacc ccagcatccc accggagatc 5940

ctccttcaac tttgagtgcc tgcgccggca gagcagccag gaagaggtcc cgtcgtctcc 6000

catcttcccc catcgcacgg ccctgcctct gcatctaatg cagcaacaga tcatggcagt 6060

tgccggccta gattcaagta aagcccagaa gtactcaccg agtcactcga cccggtcgtg 6120

ggccacccct ccagcaaccc ctccctaccg ggactggaca ccgtgctaca cccccctgat 6180

ccaagtggag cagtcagagg ccctggacca ggtgaacggc agcctgccgt ccctgcaccg 6240

cagctcctgg tacacagacg agcccgacat ctcctaccgg actttcacac cagccagcct 6300

gactgtcccc agcagcttcc ggaacaaaaa cagcgacaag cagaggagtg cggacagctt 6360

ggtggaggca gtcctgatat ccgaaggctt gggacgctat gcaagggacc caaaatttgt 6420

gtcagcaaca aaacacgaaa tcgctgatgc ctgtgacctc accatcgacg agatggagag 6480

tgcagccagc accctgctta atgggaacgt gcgtccccga gccaacgggg atgtgggccc 6540

cctctcacac cggcaggact atgagctaca ggactttggt cctggctaca gcgacgaaga 6600

gccagaccct gggagggatg aggaggacct ggcggatgaa atgatatgca tcaccacctt 6660

gtagccccca gcgaggggca gactggctct ggcctcaggt ggggcgcagg agagccaggg 6720

gaaaagtgcc tcatagttag gaaagtttag gcactagttg ggagtaatat tcaattaatt 6780

agacttttgt ataagagatg tcatgcctca agaaagccat aaacctggta ggaacaggtc 6840

ccaagcggtt gagcctggca gagtaccatg cgctcggccc cagctgcagg aaacagcagg 6900

ccccgccctc tcacagagga tgggtgagga ggccagacct gccctgcccc attgtccaga 6960

tgggcactgc tgtggagtct gcttctccca tgtaccaggg caccaggccc acccaactga 7020

aggcatggcg gcggggtgca ggggaaagtt aaaggtgatg acgatcatca cacctcgtgt 7080

cgttacctca gccatcggtc tagcatatca gtcactgggc ccaacatatc catttttaaa 7140

ccctttcccc caaatacact gcgtcctggt tcctgtttag ctgttctgaa ata 7193

<210> 288
<211> 2181
<212> PRT
<213> Homo sapiens

<400> 288

<210>  289
<211>  4187
<212>  DNA
<213>  Homo sapiens

<400>  289
aactcccgcc tcgggacgcc tcggggtcgg gctccggctg cggctgctgc tgcggcgccc      60

gcgctccggt gcgtccgcct cctgtgcccg ccgcggagca gtctgcggcc cgccgtgcgc     120

cctcagctcc ttttcctgag cccgccgcga tgggagctgc gcggggatcc ccggccagac     180

cccgccggtt gcctctgctc agcgtcctgc tgctgccgct gctgggcggt acccagacag     240

ccattgtctt catcaagcag ccgtcctccc aggatgcact gcaggggcgc cgggcgctgc     300

ttcgctgtga ggttgaggct ccgggcccgg tacatgtgta ctggctgctc gatggggccc     360

ctgtccagga cacggagcgg cgtttcgccc agggcagcag cctgagcttt gcagctgtgg     420

accggctgca ggactctggc accttccagt gtgtggctcg ggatgatgtc actggagaag     480

aagcccgcag tgccaacgcc tccttcaaca tcaaatggat tgaggcaggt cctgtggtcc     540

tgaagcatcc agcctcggaa gctgagatcc agccacagac ccaggtcaca cttcgttgcc     600

acattgatgg gcaccctcgg cccacctacc aatggttccg agatgggacc cccctttctg     660

atggtcagag caaccacaca gtcagcagca aggagcggaa cctgacgctc cggccagctg     720

gtcctgagca tagtgggctg tattcctgct gcgcccacag tgcttttggc caggcttgca     780

gcagccagaa cttcaccttg agcattgctg atgaaagctt tgccagggtg gtgctggcac     840

cccaggacgt ggtagtagcg aggtatgagg aggccatgtt ccattgccag ttctcagccc     900

agccaccccc gagcctgcag tggctctttg aggatgagac tcccatcact aaccgcagtc     960

gcccccacac cctccgcaga gccacagtgt ttgccaacgg gtctctgctg ctgacccagg    1020

tccggccacg caatgcaggg atctaccgct gcattggcca ggggcagagg ggcccaccca    1080

tcatcctgga agccacactt cacctagcag agattgaaga catgccgcta tttgagccac    1140

gggtgtttac agctggcagc gaggagcgtg tgacctgcct tccccccaag ggtctgccag    1200

agcccagcgt gtggtgggag cacgcgggag tccggctgcc cacccatggc agggtctacc    1260

agaagggcca cgagctggtg ttggccaata ttgctgaaag tgatgctggt gtctacacct    1320

gccacgcggc caacctggct ggtcagcgga gacaggatgt caacatcact gtggccactg    1380

tgccctcctg gctgaagaag ccccaagaca gccagctgga ggagggcaaa cccggctact    1440

tggattgcct gacccaggcc acaccaaaac ctacagttgt ctggtacaga aaccagatgc    1500

tcatctcaga ggactcacgg ttcgaggtct tcaagaatgg gaccttgcgc atcaacagcg    1560

tggaggtgta tgatgggaca tggtaccgtt gtatgagcag caccccagcc ggcagcatcg    1620

aggcgcaagc ccgtgtccaa gtgctggaaa agctcaagtt cacaccacca ccccagccac 1680
agcagtgcat ggagtttgac aaggaggcca cggtgccctg ttcagccaca ggccgagaga 1740
agcccactat taagtgggaa cgggcagatg ggagcagcct cccagagtgg gtgacagaca 1800
acgctgggac cctgcatttt gcccgggtga ctcgagatga cgctggcaac tacacttgca 1860
ttgcctccaa cgggccgcag ggccagattc gtgcccatgt ccagctcact gtggcagttt 1920
ttatcacctt caaagtggaa ccagagcgta cgactgtgta ccagggccac acagccctac 1980
tgcagtgcga ggcccagggg gaccccaagc cgctgattca gtggaaaggc aaggaccgca 2040
tcctggaccc caccaagctg ggacccagga tgcacatctt ccagaatggc tccctggtga 2100
tccatgacgt ggcccctgag gactcaggcc gctacacctg cattgcaggc aacagctgca 2160
acatcaagca cacggaggcc cccctctatg tcgtggacaa gcctgtgccg gaggagtcgg 2220
agggccctgg cagccctccc ccctacaaga tgatccagac cattgggttg tcggtgggtg 2280
ccgctgtggc ctacatcatt gccgtgctgg gcctcatgtt ctactgcaag aagcgctgca 2340
aagccaagcg gctgcagaag cagcccgagg gcgaggagcc agagatggaa tgcctcaacg 2400
gagggccttt gcagaacggg cagccctcag cagagatcca agaagaagtg gccttgacca 2460
gcttgggctc cggccccgcg gccaccaaca aacgccacag cacaagtgat aagatgcact 2520
tcccacggtc tagcctgcag cccatcacca cgctggggaa gagtgagttt ggggaggtgt 2580
tcctggcaaa ggctcagggc ttggaggagg gagtggcaga gaccctggta cttgtgaaga 2640
gcctgcagac gaaggatgag cagcagcagc tggacttccg gagggagttg gagatgtttg 2700
ggaagctgaa ccacgccaac gtggtgcggc tcctggggct gtgccgggag gctgagcccc 2760
actacatggt gctggaatat gtggatctgg gagacctcaa gcagttcctg aggatttcca 2820
agagcaagga tgaaaaattg aagtcacagc ccctcagcac caagcagaag gtggccctat 2880
gcacccaggt agccctgggc atggagcacc tgtccaacaa ccgctttgtg cataaggact 2940
tggctgcgcg taactgcctg gtcagtgccc agagacaagt gaaggtgtct gccctgggcc 3000
tcagcaagga tgtgtacaac agtgagtact accacttccg ccaggcctgg gtgccgctgc 3060
gctggatgtc ccccgaggcc atcctggagg gtgacttctc taccaagtct gatgtctggg 3120
ccttcggtgt gctgatgtgg gaagtgttta cacatggaga gatgccccat ggtgggcagg 3180
cagatgatga agtactggca gatttgcagg ctgggaaggc tagacttcct cagcccgagg 3240
gctgcccttc caaactctat cggctgatgc agcgctgctg ggccctcagc cccaaggacc 3300
ggccctcctt cagtgagatt gccagcgccc tgggagacag caccgtggac agcaagccgt 3360
gaggagggag cccgctcagg atggcctggg caggggagga catctctaga gggaagctca 3420
cagcatgatg ggcaagatcc ctgtcctcct gggccctgag gtgccctagt gcaacaggca 3480
ttgctgaggt ctgagcaggg cctggccttt cctcctcttc ctcaccctca tcctttggga 3540
ggctgacttg gacccaaact gggcgactag ggctttgagc tgggcagttt cccctgccac 3600
ctcttcctct atcagggaca gtgtgggtgc cacaggtaac cccaatttct ggccttcaac 3660

ttctcccctt gaccgggtcc aactctgcca ctcatctgcc aactttgcct ggggagggct 3720

aggcttggga tgagctgggt ttgtggggag ttccttaata ttctcaagtt ctgggcacac 3780

agggttaatg agtctcttgc ccactggtcc acttgggggt ctagaccagg attatagagg 3840

acacagcaag tgagtcctcc ccactctggg cttgtgcaca ctgacccaga cccacgtctt 3900

ccccaccctt ctctcctttc ctcatcctaa gtgcctggca gatgaaggag ttttcaggag 3960

cttttgacac tatataaacc gccctttttg tatgcaccac gggcggcttt tatatgtaat 4020

tgcagcgtgg ggtgggtggg catgggaggt aggggtgggc cctggagatg aggagggtgg 4080

gccatcctta ccccacactt ttattgttgt cgttttttgt ttgttttgtt tttttgtttt 4140

tgtttttgtt tttacactcg ctgctctcaa taaataagcc tttttta 4187

<210> 290
<211> 1070
<212> PRT
<213> Homo sapiens

<400> 290

<210> 291
<211> 3150
<212> DNA
<213> Homo sapiens

<400> 291
   atggggagcc ggacgccaga gtcccctctc cacgccgtgc agctgcgctg gggcccccgg 60
   cgccgacccc cgctsstgcc gctgctgttg ctgctsstgc cgccgccacc cagggtcggg 120
   ggcttcaact tagacgcgga ggccccagca gtactctcgg ggcccccggg ctccttcttc 180
   ggattctcag tggagtttta ccggccggga acagacgggg tcagtgtgct ggtgggagca 240
   cccaaggcta ataccagcca gccaggagtg ctgcagggtg gtgctgtcta cctctgtcct 300
   tggggtgcca gccccacaca gtgcaccccc attgaatttg acagcaaagg ctctcggctc 360
   ctggagtcct cactgtccag ctcagaggga gaggagcctg tggagtacaa gtccttgcag 420
   tggttcgggg caacagttcg agcccatggc tcctccatct tggcatgcgc tccactgtac 480
   agctggcgca cagagaagga gccactgagc gaccccgtgg gcacctgcta cctctccaca 540
   gataacttca cccgaattct ggagtatgca ccctgccgct cagatttcag ctgggcagca 600
   ggacagggtt actgccaagg aggcttcagt gccgagttca ccaagactgg ccgtgtggtt 660
   ttaggtggac caggaagcta tttctggcaa ggccagatcc tgtctgccac tcaggagcag 720
   attgcagaat cttattaccc cgagtacctg atcaacctgg ttcaggggca gctgcagact 780
   cgccaggcca gttccatcta tgatgacagc tacctaggat actctgtggc tgttggtgaa 840
   ttcagtggtg atgacacaga agactttgtt gctggtgtgc ccaaagggaa cctcacttac 900
   ggctatgtca ccatccttaa tggctcagac attcgatccc tctacaactt ctcaggggaa 960
   cagatggcct cctactttgg ctatgcagtg gccgccacag acgtcaatgg ggacgggctg 1020
   gatgacttgc tggtgggggc acccctgctc atggatcgga cccctgacgg gcggcctcag 1080
   gaggtgggca gggtctacgt ctacctgcag cacccagccg gcatagagcc cacgcccacc 1140
   cttaccctca ctggccatga tgagtttggc cgatttggca gctccttgac ccccctgggg 1200
   gacctggacc aggatggcta caatgatgtg gccatcgggg ctccctttgg tggggagacc 1260
   cagcagggag tagtgtttgt atttcctggg ggcccaggag ggctgggctc taagccttcc 1320
   caggttctgc agcccctgtg ggcagccagc cacaccccag acttctttgg ctctgccctt 1380
   cgaggaggcc gagacctgga tggcaatgga tatcctgatc tgattgtggg gtcctttggt 1440
   gtggacaagg ctgtggtata caggggccgc cccatcgtgt ccgctagtgc ctccctcacc 1500
   atcttccccg ccatgttcaa cccagaggag cggagctgca gcttagaggg gaaccctgtg 1560
   gcctgcatca accttagctt ctgcctcaat gcttctggaa aacacgttgc tgactccatt 1620
   ggtttcacag tggaacttca gctggactgg cagaagcaga agggaggggt acggcgggca 1680
   ctgttcctgg cctccaggca ggcaaccctg acccagaccc tgctcatcca gaatggggct 1740
   cgagaggatt gcagagagat gaagatctac ctcaggaacg agtcagaatt tcgagacaaa 1800
   ctctcgccga ttcacatcgc tctcaacttc tccttggacc cccaagcccc agtggacagc 1860
   cacggcctca ggccagccct acattatcag agcaagagcc ggatagagga caaggctcag 1920
   atcttgctgg actgtggaga agacaacatc tgtgtgcctg acctgcagct ggaagtgttt 1980
   ggggagcaga accatgtgta cctgggtgac aagaatgccc tgaacctcac tttccatgcc 2040
   cagaatgtgg gtgagggtgg cgcctatgag gctgagcttc gggtcaccgc ccctccagag 2100
   gctgagtact caggactcgt cagacaccca gggaacttct ccagcctgag ctgtgactac 2160
   tttgccgtga accagagccg cctgctggtg tgtgacctgg gcaaccccat gaaggcagga 2220
   gccagtctgt ggggtggcct tcggtttaca gtccctcatc tccgggacac taagaaaacc 2280
   atccagtttg acttccagat cctcagcaag aatctcaaca actcgcaaag cgacgtggtt 2340
   tcctttcggc tctccgtgga ggctcaggcc caggtcaccc tgaacggtgt ctccaagcct 2400
   gaggcagtgc tattcccagt aagcgactgg catccccgag accagcctca gaaggaggag 2460
   gacctgggac ctgctgtcca ccatgtctat gagctcatca accaaggccc cagctccatt 2520
   agccagggtg tgctggaact cagctgtccc caggctctgg aaggtcagca gctcctatat 2580
   gtgaccagag ttacgggact caactgcacc accaatcacc ccattaaccc aaagggcctg 2640
   gagttggatc ccgagggttc cctgcaccac cagcaaaaac gggaagctcc aagccgcagc 2700
   tctgcttcct cgggacctca gatcctgaaa tgcccggagg ctgagtgttt caggctgcgc 2760
   tgtgagctcg ggcccctgca ccaacaagag agccaaagtc tgcagttgca tttccgagtc 2820
   tgggccaaga ctttcttgca gcgggagcac cagccattta gcctgcagtg tgaggctgtg 2880
   tacaaagccc tgaagatgcc ctaccgaatc ctgcctcggc agctgcccca aaaagagcgt 2940
   caggtggcca cagctgtgca atggaccaag gcagaaggca gctatggcgt cccactgtgg 3000
   atcatcatcc tagccatcct gtttggcctc ctgctcctag gtctactcat ctacatcctc 3060
   tacaagcttg gattcttcaa acgctccctc ccatatggca ccgccatgga aaaagctcag 3120
   ctcaagcctc cagccacctc tgatgcctga 3150

<210>  292
<211>  1049
<212>  PRT
<213>  Homo sapiens

<400>  292

Met Gly Ser Arg Thr Pro Glu Ser Pro Leu His Ala Val Gln Leu Arg
1               5                   10                  15

Trp Gly Pro Arg Arg Arg Pro Pro Leu Leu Pro Leu Leu Leu Leu Leu
            20                  25                  30

Leu Pro Pro Pro Pro Arg Val Gly Gly Phe Asn Leu Asp Ala Glu Ala
        35                  40                  45

Pro Ala Val Leu Ser Gly Pro Pro Gly Ser Phe Phe Gly Phe Ser Val
    50                  55                  60

Glu Phe Tyr Arg Pro Gly Thr Asp Gly Val Ser Val Leu Val Gly Ala
65                  70                  75                  80

Pro Lys Ala Asn Thr Ser Gln Pro Gly Val Leu Gln Gly Gly Ala Val
                85                  90                  95

Tyr Leu Cys Pro Trp Gly Ala Ser Pro Thr Gln Cys Thr Pro Ile Glu
            100                 105                 110

Phe Asp Ser Lys Gly Ser Arg Leu Leu Glu Ser Ser Leu Ser Ser Ser
        115                 120                 125

Glu Gly Glu Glu Pro Val Glu Tyr Lys Ser Leu Gln Trp Phe Gly Ala
    130                 135                 140

Thr Val Arg Ala His Gly Ser Ser Ile Leu Ala Cys Ala Pro Leu Tyr
145                 150                 155                 160

Ser Trp Arg Thr Glu Lys Glu Pro Leu Ser Asp Pro Val Gly Thr Cys
                165                 170                 175

Tyr Leu Ser Thr Asp Asn Phe Thr Arg Ile Leu Glu Tyr Ala Pro Cys
            180                 185                 190

Arg Ser Asp Phe Ser Trp Ala Ala Gly Gln Gly Tyr Cys Gln Gly Gly
        195                 200                 205

Phe Ser Ala Glu Phe Thr Lys Thr Gly Arg Val Val Leu Gly Gly Pro
    210                 215                 220

Gly Ser Tyr Phe Trp Gln Gly Gln Ile Leu Ser Ala Thr Gln Glu Gln
225                 230                 235                 240

Ile Ala Glu Ser Tyr Tyr Pro Glu Tyr Leu Ile Asn Leu Val Gln Gly
                245                 250                 255

Gln Leu Gln Thr Arg Gln Ala Ser Ser Ile Tyr Asp Asp Ser Tyr Leu
            260                 265                 270

Gly Tyr Ser Val Ala Val Gly Glu Phe Ser Gly Asp Asp Thr Glu Asp
        275                 280                 285

Phe Val Ala Gly Val Pro Lys Gly Asn Leu Thr Tyr Gly Tyr Val Thr
    290                 295                 300

Ile Leu Asn Gly Ser Asp Ile Arg Ser Leu Tyr Asn Phe Ser Gly Glu
305                 310                 315                 320

Gln Met Ala Ser Tyr Phe Gly Tyr Ala Val Ala Ala Thr Asp Val Asn
                325                 330                 335

Gly Asp Gly Leu Asp Asp Leu Leu Val Gly Ala Pro Leu Leu Met Asp
            340                 345                 350

Arg Thr Pro Asp Gly Arg Pro Gln Glu Val Gly Arg Val Tyr Val Tyr
        355                 360                 365

Leu Gln His Pro Ala Gly Ile Glu Pro Thr Pro Thr Leu Thr Leu Thr
    370                 375                 380

Gly His Asp Glu Phe Gly Arg Phe Gly Ser Ser Leu Thr Pro Leu Gly
385                 390                 395                 400

Asp Leu Asp Gln Asp Gly Tyr Asn Asp Val Ala Ile Gly Ala Pro Phe
                405                 410                 415

Gly Gly Glu Thr Gln Gln Gly Val Val Phe Val Phe Pro Gly Gly Pro
            420                 425                 430

Gly Gly Leu Gly Ser Lys Pro Ser Gln Val Leu Gln Pro Leu Trp Ala
        435                 440                 445

Ala Ser His Thr Pro Asp Phe Phe Gly Ser Ala Leu Arg Gly Gly Arg
    450                 455                 460

Asp Leu Asp Gly Asn Gly Tyr Pro Asp Leu Ile Val Gly Ser Phe Gly
465                 470                 475                 480

Val Asp Lys Ala Val Val Tyr Arg Gly Arg Pro Ile Val Ser Ala Ser
                485                 490                 495

Ala Ser Leu Thr Ile Phe Pro Ala Met Phe Asn Pro Glu Glu Arg Ser
            500                 505                 510

Cys Ser Leu Glu Gly Asn Pro Val Ala Cys Ile Asn Leu Ser Phe Cys
        515                 520                 525

Leu Asn Ala Ser Gly Lys His Val Ala Asp Ser Ile Gly Phe Thr Val
    530                 535                 540

Glu Leu Gln Leu Asp Trp Gln Lys Gln Lys Gly Gly Val Arg Arg Ala
545                 550                 555                 560

Leu Phe Leu Ala Ser Arg Gln Ala Thr Leu Thr Gln Thr Leu Leu Ile
                565                 570                 575

Gln Asn Gly Ala Arg Glu Asp Cys Arg Glu Met Lys Ile Tyr Leu Arg
            580                 585                 590

Asn Glu Ser Glu Phe Arg Asp Lys Leu Ser Pro Ile His Ile Ala Leu
        595                 600                 605

Asn Phe Ser Leu Asp Pro Gln Ala Pro Val Asp Ser His Gly Leu Arg
    610                 615                 620

Pro Ala Leu His Tyr Gln Ser Lys Ser Arg Ile Glu Asp Lys Ala Gln
625                 630                 635                 640

Ile Leu Leu Asp Cys Gly Glu Asp Asn Ile Cys Val Pro Asp Leu Gln
                645                 650                 655

Leu Glu Val Phe Gly Glu Gln Asn His Val Tyr Leu Gly Asp Lys Asn
            660                 665                 670

Ala Leu Asn Leu Thr Phe His Ala Gln Asn Val Gly Glu Gly Gly Ala
        675                 680                 685

Tyr Glu Ala Glu Leu Arg Val Thr Ala Pro Pro Glu Ala Glu Tyr Ser
    690                 695                 700

Gly Leu Val Arg His Pro Gly Asn Phe Ser Ser Leu Ser Cys Asp Tyr
705                 710                 715                 720

Phe Ala Val Asn Gln Ser Arg Leu Leu Val Cys Asp Leu Gly Asn Pro
                725                 730                 735

Met Lys Ala Gly Ala Ser Leu Trp Gly Gly Leu Arg Phe Thr Val Pro
            740                 745                 750

His Leu Arg Asp Thr Lys Lys Thr Ile Gln Phe Asp Phe Gln Ile Leu
        755                 760                 765

Ser Lys Asn Leu Asn Asn Ser Gln Ser Asp Val Val Ser Phe Arg Leu
    770                 775                 780

Ser Val Glu Ala Gln Ala Gln Val Thr Leu Asn Gly Val Ser Lys Pro
785                 790                 795                 800

Glu Ala Val Leu Phe Pro Val Ser Asp Trp His Pro Arg Asp Gln Pro
                805                 810                 815

Gln Lys Glu Glu Asp Leu Gly Pro Ala Val His His Val Tyr Glu Leu
            820                 825                 830

Ile Asn Gln Gly Pro Ser Ser Ile Ser Gln Gly Val Leu Glu Leu Ser
        835                 840                 845

Cys Pro Gln Ala Leu Glu Gly Gln Gln Leu Leu Tyr Val Thr Arg Val
    850                 855                 860

Thr Gly Leu Asn Cys Thr Thr Asn His Pro Ile Asn Pro Lys Gly Leu
865                 870                 875                 880

Glu Leu Asp Pro Glu Gly Ser Leu His His Gln Gln Lys Arg Glu Ala
                885                 890                 895

Pro Ser Arg Ser Ser Ala Ser Ser Gly Pro Gln Ile Leu Lys Cys Pro
            900                 905                 910

Glu Ala Glu Cys Phe Arg Leu Arg Cys Glu Leu Gly Pro Leu His Gln
        915                 920                 925

Gln Glu Ser Gln Ser Leu Gln Leu His Phe Arg Val Trp Ala Lys Thr
    930                 935                 940

Phe Leu Gln Arg Glu His Gln Pro Phe Ser Leu Gln Cys Glu Ala Val
945                 950                 955                 960

Tyr Lys Ala Leu Lys Met Pro Tyr Arg Ile Leu Pro Arg Gln Leu Pro
                965                 970                 975

Gln Lys Glu Arg Gln Val Ala Thr Ala Val Gln Trp Thr Lys Ala Glu
            980                 985                 990

Gly Ser Tyr Gly Val Pro Leu Trp  Ile Ile Ile Leu Ala  Ile Leu Phe
        995                 1000                 1005

Gly Leu  Leu Leu Leu Gly Leu  Leu Ile Tyr Ile Leu  Tyr Lys Leu
    1010                 1015                 1020

Gly Phe  Phe Lys Arg Ser Leu  Pro Tyr Gly Thr Ala  Met Glu Lys
    1025                 1030                 1035

Ala Gln  Leu Lys Pro Pro Ala  Thr Ser Asp Ala

1040                1045

<210>  293
<211>  1373
<212>  DNA
<213>  Homo sapiens

<400>  293
gggggcgccg cgcgctgacc ctccctgggc accgctgggg acgatggcgc tgctcgcctt      60

gctgctggtc gtggccctac cgcgggtgtg gacagacgcc aacctgactg cgagacaacg     120

agatccagag gactcccagc gaacggacga gggtgacaat agagtgtggt gtcatgtttg     180

tgagagagaa aacactttcg agtgccagaa cccaaggagg tgcaaatgga cagagccata     240

ctgcgttata gcggccgtga aaatatttcc acgttttttc atggttgcga agcagtgctc     300

cgctggttgt gcagcgatgg agagacccaa gccagaggag aagcggtttc tcctggaaga     360

gcccatgccc ttcttttacc tcaagtgttg taaaattcgc tactgcaatt tagaggggcc     420

acctatcaac tcatcagtgt tcaaagaata tgctgggagc atgggtgaga gctgtggtgg     480

gctgtggctg gccatcctcc tgctgctggc ctccattgca gccggcctca gcctgtcttg     540

agccacggga ctgccacaga ctgagccttc cggagcatgg actcgctcca gaccgttgtc     600

acctgttgca ttaaacttgt tttctgttga ttacctcttg gtttgacttc ccagggtctt     660

gggatgggag agtggggatc aggtgcagtt ggctcttaac cctcaagggt tctttaactc     720

acattcagag gaagtccaga tctcctgagt agtgattttg gtgacaagtt tttctctttg     780

aaatcaaacc ttgtaactca tttattgctg atggccactc ttttccttga ctcccctctg     840

cctctgaggg cttcagtatt gatggggagg gaggcctaag taccactcat ggagagtatg     900

tgctgagatg cttccgacct ttcaggtgac gcaggaacac tgggggagtc tgaatgattg     960

gggtgaagac atccctggag tgaaggactc ctcagcatgg ggggcagtgg ggcacacgtt    1020

agggctgccc ccattccagt ggtggaggcg ctgtggatgg ctgcttttcc tcaacctttc    1080

ctaccagatt ccaggaggca gaagataact aattgtgttg aagaaactta gacttcaccc    1140

accagctggc acaggtgcac agattcataa attcccacac gtgtgtgttc aacatctgaa    1200

acttaggcca agtagagagc atcagggtaa atggcgttca tttctctgtt aagatgcagc    1260

catccatggg gagctgagaa atcagactca aagttccacc aaaaacaaat acaaggggac    1320

ttcaaaagtt cacgaaaaaa aaaaaaaaaa aaaaaaaaaa aaaaaaaaaa aaa           1373

<210>  294
<211>  165
<212>  PRT
<213>  Homo sapiens

<400>  294

Met Ala Leu Leu Ala Leu Leu Leu Val Val Ala Leu Pro Arg Val Trp
1               5                   10                  15

Thr Asp Ala Asn Leu Thr Ala Arg Gln Arg Asp Pro Glu Asp Ser Gln
            20                  25                  30

Arg Thr Asp Glu Gly Asp Asn Arg Val Trp Cys His Val Cys Glu Arg

35                  40                  45

Glu Asn Thr Phe Glu Cys Gln Asn Pro Arg Arg Cys Lys Trp Thr Glu
    50                  55                  60

Pro Tyr Cys Val Ile Ala Ala Val Lys Ile Phe Pro Arg Phe Phe Met
65                  70                  75                  80

Val Ala Lys Gln Cys Ser Ala Gly Cys Ala Ala Met Glu Arg Pro Lys
                85                  90                  95

Pro Glu Glu Lys Arg Phe Leu Leu Glu Glu Pro Met Pro Phe Phe Tyr
            100                 105                 110

Leu Lys Cys Cys Lys Ile Arg Tyr Cys Asn Leu Glu Gly Pro Pro Ile
        115                 120                 125

Asn Ser Ser Val Phe Lys Glu Tyr Ala Gly Ser Met Gly Glu Ser Cys
    130                 135                 140

Gly Gly Leu Trp Leu Ala Ile Leu Leu Leu Leu Ala Ser Ile Ala Ala
145                 150                 155                 160

Gly Leu Ser Leu Ser
                165

<210>  295
<211>  6
<212>  PRT
<213>  Artificial Sequence HIS6 epitope tag

<220>
<221>  source
<223>  /note= "Description of artificial sequence: HIS6 epitope tag"

<400>  295

His His His His His His
1               5

<210>  296
<211>  24
<212>  DNA
<213>  Artificial Sequence T7-T24 oligo

<220>
<221>  source
<223>  /note= "Description of artificial sequence: T7-T24 oligo"

<220>
<221>  misc_feature
<222>  (8)..(24)
<223>  t at positions 8-24 may be present or absent

<400>  296
tttttttttt tttttttttt tttt                                              24